(19) Europäisches Patentamt
European Patent Office
Office européen des brevets

(11) EP 1 947 112 B1

(12) **EUROPEAN PATENT SPECIFICATION**

(45) Date of publication and mention of the grant of the patent:
**16.05.2012 Bulletin 2012/20**

(21) Application number: **08075174.6**

(22) Date of filing: **07.04.2000**

(51) Int Cl.:
***C07K 14/435*** *(2006.01)* ***C12N 15/12*** *(2006.01)*
***C12N 5/10*** *(2006.01)* ***C07K 16/18*** *(2006.01)*
***G01N 33/50*** *(2006.01)* ***A61K 48/00*** *(2006.01)*
***A61K 38/17*** *(2006.01)*

(54) **Flea head, nerve cord, hindgut and malpighian tubule nucleic acid molecules, proteins and uses thereof**

Nukleinsäuremoleküle und Proteine des Kopfs, Nervenstrangs, Hinterdarms und malpighischen Gefäßes des Flohs sowie deren Verwendung

Molécules d'acides nucléiques de tête, cordon nerveux, intestin postérieur et tube de Malpighi de puce et leurs utilisations

(84) Designated Contracting States:
**AT BE CH CY DE DK ES FI FR GB GR IE IT LI LU MC NL PT SE**

(30) Priority: **09.04.1999 US 128704 P**

(43) Date of publication of application:
**23.07.2008 Bulletin 2008/30**

(62) Document number(s) of the earlier application(s) in accordance with Art. 76 EPC:
**06010625.9 / 1 710 252**
**00918540.6 / 1 169 343**

(73) Proprietor: **Heska Corporation**
**Loveland, CO 80538 (US)**

(72) Inventors:
- **Brandt, Kevin S.**
**Longmont, CO 80503 (US)**
- **Stinchcomb, Dan T.**
**Fort Collins**
**Colorado 80528 (US)**
- **Gaines, Patrick J.**
**Westminster, CO 80234 (US)**
- **Wisnewski, Nancy**
**Fort Collins**
**Colorado 80526 (US)**

(74) Representative: **Thomson, Craig Richard**
**Murgitroyd & Company**
**Scotland House**
**165-169 Scotland Street**
**Glasgow G5 8PL (GB)**

(56) References cited:
**WO-A-97/37676** **WO-A-98/33818**
**WO-A-99/07828** **US-A- 5 418 137**
**US-A- 5 854 411**

- **DATABASE UniProt 15 July 1998 (1998-07-15), VALENZULA S.M. ET AL: "Human chloride intracellular channel protein 1 (CLIC1)" XP002484878 retrieved from EBI accession no. CLIC1_HUMAN Database accession no. O00299**
- **DATABASE UniProt 15 July 1998 (1998-07-15), HEISS N.S. ET AL: "Human chloride intracellular protein 2 (CLIC2)" XP002484879 retrieved from EBI accession no. CLIC2_HUMAN Database accession no. O15247**
- **DATABASE UniProt 1 June 1994 (1994-06-01), LANDRY D.W. ET AL: "Bovine chloride intracellular protein 5 (CICL5)" XP002484880 retrieved from EBI accession no. CLIC5_BOVIN Database accession no. P35526**
- **DATABASE EM-EST EMBL; Bombyx mori cDNA, clone wsooo21 19 January 1999 (1999-01-19), MITA K. ET AL: "Establishment of cDNA database of Bombyx mori", retrieved from EBI accession no. AU003320 Database accession no. AU003320**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 947 112 B1

## Description

### FIELD OF THE INVENTION

The present invention relates to nucleic acid molecules isolated from the head and nerve cord of a flea, nucleic acid molecules isolated from the hindgut and Malpighian tubule of a flea, proteins encoded by such nucleic acid molecules, antibodies raised against such proteins, and inhibitors of such proteins. The present invention also includes therapeutic compositions comprising such nucleic acid molecules, proteins, antibodies, and/or other inhibitors, as well as uses thereof.

### BACKGROUND OF THE INVENTION

Flea infestation of animals is a health and economic concern because fleas are known to cause and/or transmit a variety of diseases. Fleas directly cause a variety of diseases, including allergies, and also carry a variety of infectious agents including, but not limited to, endoparasites (e.g., nematodes, cestodes, trematodes and protozoa), bacteria and viruses. In particular, the bites of fleas are a problem for animals maintained as pets because the infestation becomes a source of annoyance not only for the pet but also for the pet owner who may find his or her home generally contaminated with insects. As such, fleas are a problem not only when they are on an animal but also when they are in the general environment of the animal.

Bites from fleas are a particular problem because they not only can lead to disease transmission but also can cause a hypersensitive response in animals which is manifested as disease. For example, bites from fleas can cause an allergic disease called flea allergic (or allergy) dermatitis (FAD). A hypersensitive response in animals typically results in localized tissue inflammation and damage, causing substantial discomfort to the animal.

The medical importance of flea infestation has prompted the development of reagents capable of controlling flea infestation. Commonly encountered methods to control flea infestation are generally focused on use of insecticides. While some of these products are efficacious, most, at best, offer protection of a very limited duration. Furthermore, many of the methods are often not successful in reducing flea populations. In particular, insecticides have been used to prevent flea infestation of animals by adding such insecticides to shampoos, powders, collars, sprays, spot-on formulations foggers and liquid bath treatments (i.e., dips). Reduction of flea infestation on the pet has been unsuccessful for one or more of the following reasons: failure of owner compliance (frequent administration is required); behavioral of physiological intolerance of the pet to the pesticide product or means of administration; and the emergence of flea populations resistant to the prescribed dose of pesticide.

There exists prior art such as US 5,854,411 which describes a human chloride channel protein (HCCP) and polynucleotides which encode those proteins, vectors, hosts, antibodies, pharmaceutical compositions and the like.

WO 98/33818 describes a novel human anion channel (NANCH) and polynucleotides which encode those proteins, vectors, hosts, antibodies, pharmaceutical compositions and the like.

Molecular Cloning and Expression of a Chloride Ion Channel of Cell Nuclei (1997) J Biol Chem 272:12575-12582 discloses a Human CLIC-1 protein and nucleic acids encoding same.

Genomics 45: 224-228, Heiss N S et al (1997) discloses Human CLIC-2 protein and nucleic acids encoding same.

(1993) J Biol Chem 268:14948-14955, Landry D W et al discloses Bovine CLIC-5 protein and nucleic acids encoding same.

EBI accession no. AU003320 discloses Bombyx mori cDNA, clone ws00021 19 Jan 1999, MITA K et al "Establishment of cDNA database of Bombyx mori".

However, there remains a need to develop a reagent and a method to protect animals from flea infestation.

### SUMMARY OF THE INVENTION

The present invention relates to a novel product and process for protection of animals from flea infestation, as claimed hereafter. Preferred embodiments of the invention are set forth in the dependent claims.

The present invention also includes methods to obtain such proteins, and antibodies. The present invention also includes the use of proteins to identify such inhibitory compounds as well as assay kits to identify such inhibitory compounds. Also included in the present invention are therapeutic compositions.

### DETAILED DESCRIPTION OF THE INVENTION

There are disclosed nucleic acid molecules isolated from the head and/or nerve cord of a flea, nucleic acid molecules isolated from the hindgut and/or Malpighian tubule of a flea, proteins encoded by such nucleic acid molecules, antibodies raised against such proteins, and inhibitors of such proteins. As used herein, nucleic acid molecules isolated from the head and/or nerve cord of a flea and proteins encoded by such nucleic acid molecules are also referred to as flea HNC, or HNC, nucleic acid molecules and proteins respectively; and nucleic molecules isolated from the hindgut and/or Malpighian tubules of a flea and proteins encoded by such nucleic acid molecules are referred to as flea HMT or HMT, nucleic acid molecules and proteins respectively. HNC nucleic acid molecules and HMT nucleic acid molecules described herein are nucleic acid molecules that are primarily expressed in flea HNC tissues and HMT tissues respectively, but which may be expressed in cells derived from flea tissue other than HNC and HMT. HNC and HMT nucleic acid molecules and proteins as described herein can be isolated from a flea or prepared recombinantly or synthetically. The HMT and HNC nucleic acid molecules described can be RNA or DNA; examples of nucleic acid molecules include, but are not limited to, complementary DNA (cDNA) molecules, genomic DNA molecules, synthetic DNA molecules, DNA molecules which are specific tags for messenger RNA derived from HMT and HNC tissues, and corresponding mRNA molecules. As used herein, the phrases "HMT and/or HNC protein" and "HMT and HNC protein" refer to a protein expressed by a flea HMT tissue, by a flea HNC tissue, or by both flea HMT and HNC tissues. As used herein, the phrases "HMT and/or HNC nucleic acid molecule" and "HMT and HNC nucleic acid molecule" refer to a nucleic acid molecule that can be isolated from a HMT cDNA library, from a HNC cDNA library, or from both libraries, or a gene corresponding thereto.

Also discussed are nucleic acid molecules containing partial or full-length coding regions that encode one or more of the following flea proteins: an allantoinase (ALN) protein, a chitin-binding protein (CBP) protein, a sodium/potassium ATPase beta subunit (NKAH) protein, a ligand-gated chloride channel (LGIC) protein, an ANON/23DA (ANON) protein, a malvolio (MALV) protein, an odorant-binding protein-like (OS-D) protein, a N-methyl-D-aspartate receptor associated (NMDA) protein, a chemical sense related lipophilic ligand binding protein-like (CLBP) protein, a Sodium/Hydrogen Transporter-like (NAH) protein, a Chloride Intracellular Channel-like (CLIC) protein, aPeritrophin-like (PL2) protein, aPeritrophin-like (PL3) protein, aPeritrophin-like (PL4) protein, a synaptic vesicle 2B-like (SVP) protein, a voltage-gated Chloride-like (VGCC) protein, an anoxia upregulated protein-like (AUP) protein, and a neuroendocrine specific 7B2-like (7B2) protein. Such nucleic acid molecules are referred to as ALN nucleic acid molecules, CBP nucleic acid molecules, NKAB nucleic acid molecules, LGIC nucleic acid molecules, ANON nucleic acid molecules, MALV nucleic acid molecules, OS-D nucleic acid molecules, NMDA nucleic acid molecules, CLBP nucleic acid molecules, NAH nucleic acid molecules, CLIC nucleic acid molecules, PL2 nucleic acid molecules, PL3 nucleic acid molecules, PL4 nucleic acid molecules, SVP nucleic acid molecules, VGCC nucleic acid molecules, AUP nucleic acid molecules, and 7B2 nucleic acid molecules respectively and are described herein in detail below.

Allantoinase is involved in the catalysis of the reaction converting allantoin to allantoic acid. This is a middle step in purine catabolism, which in insects results in the secretion of urea as the end product. The enzyme is located in the peroxisomes of the liver and kidney in amphibians. There is no known mammalian homologue to allantoinase, as mammals secrete uric acid, a precursor to.allantoin. As such, flea allantoinase represents a novel target for anti-flea vaccines and chemotherapeutic drugs.

The function of chitin binding protein is largely unknown. A chitinase-like protein of *Bombyx mori* (GenBank accession # 1841851) is reported to have weak similarity with the chitin-binding domain of insect chitinases; however, it has no significant similarity to the catalytic regions of known chitinases, and therefore is not expected to have chitinase activity. The chitinase-like protein of *B. mori* is also similar to the peritrophin family of proteins located in the peritrophic matrix of insects. These proteins contain putative chitin-binding domains but have no other apparent homology to any known proteins. Without being bound by theory, it is believed that these proteins bind chitin and are a structural component of the peritrophic matrix. As such, flea chitin binding protein represents a novel target for anti-flea vaccines and chemotherapeutic drugs.

Na+/K+ATPase is involved in the hydrolysis of ATP to power the transport of Na+ out of and K+ into cells. It is responsible for establishing the Na+ gradient across plasma membranes, which is then used by cells for a number of functions including sugar and amino acid transport, diuresis and nerve cell signaling. The Na+/K+ ATPase pump is a trimer of a 100-kilodalton (kDa) alpha ($\alpha$) subunit, a 40-kDa beta ($\beta$) subunit, and a 6-kDa gamma ($\gamma$) subunit. Most insects express three isotypes of the P subunit, each being expressed in a tissue and cell-type dependent manner. The $\alpha$ subunit has 8 transmembrane domains whereas the $\beta$ and $\gamma$ subunits have just one. The a subunit mediates ATPase and ion transporting activities and together with the $\gamma$ subunit comprises the site for cardiac glycoside (ouabain) binding. The $\beta$ subunit is required for detectable pump activity, and is thought to have roles in stability, localization, and determining cation specificity. As such, a flea NKAB protein represents a novel target for anti-flea vaccines and chemotherapeutic drugs.

Ligand-gated ion channel family proteins have been shown to transmit neural signals in response to binding neurotransmitters such as GABA, glycine, and glutamate. GABA and glycine receptors transmit inhibitory signals whereas glutamate receptors transmit excitatory signals. This family of proteins is the target for many drugs affecting neural signaling, and also for several families of insecticides including cyclodienes, pyrethroids, and phenyl pyrazoles. Northern blot analysis indicates that the mRNA corresponding to a LGIC nucleic acid molecule as described is only expressed in HMT tissue, which suggests a role in the regulation or mediation of diuresis. Without being bound by theory, assuming protein expression correlates with the mRNA expression, flea LGIC may represent the first of this family of receptors shown to be exclusively expressed in renal tissue. Sequence analysis shows that a flea LGIC protein is distinct from other subfamilies of ligand-gated ion channels, and thus may represent a new subfamily. As such, a flea LGIC protein as described represents a novel target for anti-flea vaccines and chemotherapeutic drugs.

The function of ANON/23DA protein largely unknown. The ANON/23DA gene is reported to be linked to the MAD gene in *Drosophila,* though it is not known if ANON/23DA and MAD are functionally related. ANON/23DA may also have functional similarity to human probable membrane receptor protein pHPS 1-2, which is similar to rhodopsin/ beta-adrenergic receptor which plays an important role in kidney function. As such, a flea ANON/23DA protein as described represents a novel target for anti-flea vaccines and chemotherapeutic drugs.

*Drosophila* malvolio shows high sequence homology to mammalian natural resistance associated proteins (NRAMPs) and to yeast Smfl, which are proteins that transport divalent cations, specifically Mn++, Zn++, and Fe++. NRAMPs have also been shown be similar to ATPase transporters and use ATP as an energy source. There are two types of NRAMP proteins, NRAMP1 and NRAMP2. NRAMP1 is expressed exclusively on macrophages and is responsible for preventing intracellular replication of microbes. NRAMP2 is expressed in several cell and tissue types, including mouse intestinal epithelia. Flea malvolio proteins as described appear to be most similar to NRAMP1. As such, a flea malvolio protein as described represents a novel target for and-flea vaccines and chemotherapeutic drugs.

The function of OS-D proteins is largely unknown. An OS-D nucleic acid molecule isolated from a *Drosophila melanogaster* antenna cDNA library encodes a protein that shares features common to vertebrate odorant-binding proteins, but has a primary structure unlike odorant-binding proteins. The encoded protein is also homologous to a family of soluble chemosensory proteins from the chemosensory organ of the desert locust, *Schistocerca gregaria.* As such, a flea OS-D protein as described represents a novel target for anti-flea vaccines and chemotherapeutic drugs.

NMDA receptors are a subtype of glutamate-gated ion channels. All glutamate-gated ion channels transmit Na+ and K+ when stimulated, resulting in a depolarization of the membrane potential. NMDA receptors also transport Ca++ into cells upon stimulation, which distinguishes NMDA receptors from the other glutamate-gated ion channels. NMDA receptors play an important role in glutamate excitotoxicity, which has been linked to a number of neurodegenerative disorders such as focal cerebral ischemia (stroke), Parkinson's disease, Huntington's chorea, Alzheimer's disease, schizophrenia and epilepsy. It is thought that the Ca++ influx in open NMDA channels is the mediator for these diseases, since the increase in intracellular Ca++ concentration leads to the induction of metabolic changes in the cell, including the activation of Ca++ dependent proteases and production of free-oxygen radicals. As such, a flea NMDA protein as described represents a novel target for anti-flea vaccines and chemotherapeutic drugs.

CLBP proteins as described appear to fall into the family of PBP/GOBP proteins (pheromone binding protein/ general odorant binding protein) based on sequence homology with members of this family (30% identity with PBPRP-2, pheromone binding protein related protein #2 of *Drosophila melanogaster,* and approximately the same identity with CSRLLBP, chemical sense related lipophilic ligand binding protein of *Phormia regina*). Without being bound by theory, it is believed that these proteins are involved in the perception of odors or pheromones, such as the ability to sense the presence of a host or mate. As such, a flea CLBP protein as described represents a novel target for anti-flea vaccines and chemotherapeutic drugs.

Peritrophins, including flea PL2, PL3 and PL4 proteins as described are a family of putative chitin-binding proteins that comprise a structural component of the peritrophic matrix, an acellular membrane composed of proteins and sugars, most commonly chitin which forms a barrier between the contents of an ingested meal and the gut epithelia. Peritrophin-like proteins have also been shown to be present in the trachea of *Drosophila* embryos, indicating that such proteins may have additional roles outside the midgut. The function of the peritrophin-like proteins in adult fleas is not clear, since adult fleas do not produce a peritrophic matrix in the gut. Peritrophins have been investigated as targets for immunological control of hematophagous insects including the sheep blowfly, *Lucilia cuprina.* It has been shown in this insect that ingestion of antibodies against peritrophins inhibits the growth of larvae and can result in increased larval mortality. It has also been shown that the ingestion of antibodies against peritrophins reduces the permeability of the peritrophic matrix in *L cuprina* larvae. This in turn may inhibit the movement of digested food across the peritrophic matrix to the gut epithelium, resulting in starvation. As such, a flea peritrophin as described represents a novel target for anti-flea vaccines and chemotherapeutic drugs.

In general, voltage-gated chloride channels (VGCC) maintain resting epithelial and neural membrane potentials and prevent hyperexcitability (sustained contraction) in muscle cells. In *Drosophila* Malpighian tubules, the diuretic hormone leukokinin has been shown to stimulate voltage-gated chloride channels in the stellate cells by increasing intracellular calcium levels. The flea VGCC protein sequence as described contains an EF-hand calcium binding motif, indicating potential regulation by calcium ions, and thus a possible link to leukokinins and diuresis. Chloride channels are critical for diuresis since chloride is the primary anion driving diuresis and is required to help neutralize the sodium and potassium cations that are secreted into the lumen in response to diuretic peptide. The mRNA for the VGCC as described has been shown to be HMT-specific in adult fleas, indicating a potential role in diuresis. As such, a flea VGCC as described represents a novel target for anti-flea vaccines and chemotherapeutic drugs.

The CLIC family of chloride channels are voltage-gated chloride channels that are expressed on a variety of vesicles and are thought to act in concert with the V-ATPase pump to regulate the pH of the vesicle interior. Members of the CLIC family have also been shown to be expressed on the plasma membrane, again, in association with the V-ATPase pump. In humans, a homologous protein has been shown to be expressed on the plasma membrane in epithelial tissues, suggesting a possible role in transepithelial chloride transport and in cows, an antibody against a homologous channel has been shown to inhibit all chloride conductance in kidney microsomes. If the CLIC gene product is indeed involved in transepithelial chloride transport in HMT tissues, it likely plays a critical role in mediating diuresis. As such, a flea CLIC of the present invention represents a novel target for anti-flea vaccines and chemotherapeutic drugs.

The NAH exchanger uses the proton gradient in the lumen of the Malpighian tubule to power the transport of sodium ions across the apical membrane into the lumen. The transport of sodium ions across the Malpighian tubule epithelia is induced by diuretic peptide and is a critical step in the induction of diuresis. The Northern blot analysis described herein indicates that NAH mRNA is upregulated within 15 minutes of feeding in adults, which is consistent with a molecule having a role in diuresis. In many insects, sodium has been shown to be the principle ion driving diuresis. The NAH exchanger has been shown to be located on the apical membrane in the Malpighian tubules, but may also be located in the hindgut and rectum. If located in the hindgut and rectum, it could be accessible to antibody attack on either the basolateral or apical membranes. As such, a flea NAH as described represents a novel target for anti-flea vaccines and chemotherapeutic drugs.

SVP proteins have structural and sequence conservation with a bacterial family of proton co-transporters, with the mammalian proton/glucose transporter, and with organic ion transporters. SVP has 12 putative transmembrane regions that arise from an internal duplication. In mammals, it is located on neural and endocrine vesicles and is thought to function in the uptake of neurotransmitters into vesicles utilizing the proton gradient. Neurotransmitters in turn regulate the activity the ion channels on these membranes. In the Malpighian tubules, the activity of the ion channels determines the rate of diuresis, or fluid secretion from the hemolymph into the lumen. Thus, inhibiting the transport of neurotransmitters in the HMT tissues may have significant effects on the functions of these tissues. As such, a flea SVP as described represents a novel target for anti-flea vaccines and chemotherapeutic drugs.

The function of flea AUP proteins is largely unknown. C. felis AUP shares some homology to Drosophila melanogaster anoxia-regulated gene product fau. The Drosophila melanogaster fau gene has no homology to previously described database entries, but localizes to laminal and cortical neurons of the Drosophila CNS by in situ hybridization, and plays and important role in response to 02 deprivation as measured by impaired recovery time of transgenic flies over-expressing fau to anoxia. As such, a flea AUP as described represents a novel target for anti-flea vaccines and chemotherapeutic drugs.

A flea 7B2 protein has some BLAST homology to the neuroendocrine protein 7B2 from various organisms, including *Drosophila, C. elegant,* the pond snail *Lymnaea stagnalis*, and humans. 7B2 has been implicated in activation of prohormone convertase 2 (PC2) an important neuroendocrine precursor processing endoprotease. Additionally, 7B2 was found to be critical in islet hormone processing in mice using null mutants which displayed hypoglycemia, hyperproinsulinemia and hypoglucagonemia. As such, a flea 7B2 as described represents a novel target for anti-flea vaccines and chemotherapeutic drugs.

Flea allantoinase nucleic acid molecules of known length isolated from *C. felis* are denoted "$nCfALN_{\#}$", for example $nCfALN_{2057}$, wherein "#" refers to the number of nucleotides in that molecule, and allantoinase proteins of known length are denoted "$PCfALN_{\#}$" (for example $PCfALN_{384}$) wherein "#" refers to the number of amino acid residues in that molecule. Similarly, *C. felis* CBP nucleic acid molecules and proteins of known length are denoted "$nCfCBP_{\#}$" and "$PCEBP_{\#}$", respectively; *C. felis* NKAB nucleic acid molecules and proteins of known length are denoted "$nCfNKAB_{\#}$" and "$PCfNKAB_{\#}$", respectively; *C. felis* LGIC nucleic acid molecules and proteins of known length are denoted "$nCfLGIC_{\#}$" and "$PCfLGIC_{\#}$", respectively, *C. felis* ANON nucleic acid molecules and proteins of known length are denoted "nCfANON," and "$PCfANON_{\#}$", respectively; *C. felis* MALV nucleic acid molecules and proteins of known length are denoted "$nCfMALV_{\#}$" and "$PCBAALV_{\#}$" respectively, *C. felis* OS-D nucleic acid molecules and proteins of known length are denoted "$nCfOSD_{\#}$" and "$PCfOSD_{\#}$ respectively, *C. felis* NMDA nucleic acid molecules and proteins of known length are denoted "$nCflVMDA_{\#}$" and "$PCfNMDA_{\#}$ respectively; *C. felis* CLBP nucleic acid molecules and proteins of known length are denoted "$nCfCLBP_{\#}$" and "$PCfCLBP_{\#}$ respectively, *C. felis* NAH nucleic acid molecules and proteins of known length are denoted "$nCfNAH_{\#}$" and "$PCfNAH_{\#}$ respectively, *C. felis* CLIC nucleic acid molecules and proteins of known length are denoted "$nCfCLIC_{\#}$" and "$PCfCLIC_{\#}$ respectively, *C. felis* PL2 nucleic acid molecules and proteins of known length are denoted "$nCfpL2_{\#}$" and "$PCfPL2_{\#}$ respectively, *C. felis* PL3 nucleic acid molecules and proteins of known length are denoted "$nCfPL3_{\#}$" and "$PCfPL3_{\#}$ respectively, *C. felis* PLA nucleic acid molecules and proteins of known length are denoted "$nCfPL4_{\#}$" and "$PCfPL4_{\#}$ respectively, *C. felis* SVP nucleic acid molecules and proteins of known length are denoted "$nCfSVP_{\#}$" and "$PCfSVP_{\#}$ respectively, *C. felis* VGCC nucleic acid molecules and proteins of known length are denoted "$nCfVGCC_{\#}$" and "PCfVGCC, respectively, *C. felis* AUP nucleic acid molecules and proteins of known length are denoted "nCAUP $f_{\#}$" and "$PCfAUP_{\#}$ respectively, and *C. felis* 7B2 nucleic acid molecules and proteins of known length are denoted "$nCf7B2_{\#}$" and "$PCf7B2_{\#}$ respectively.

Also described are HMT and HNC DNA molecules that are specific tags for messenger RNA molecules derived from HMT and HNC tissues. Such DNA molecules can correspond to an entire or partial sequence of a messenger RNA, and therefore, a DNA molecule corresponding to such a messenger RNA molecule (i.e. a cDNA molecule), can encode a full-length or partial-length protein. A nucleic acid molecule encoding a partial-length protein can be used directly as a probe or indirectly to generate primers to identify and/or isolate a cDNA nucleic acid molecule encoding a corresponding, or structurally related, full-length protein. Such a partial cDNA nucleic acid molecule can also be used in a similar manner to identify a genomic nucleic acid molecule, such as a nucleic acid molecule that contains the complete gene including regulatory regions, exons and introns. Methods for using partial HMT and HNC cDNA molecules and sequences to isolate full-length transcripts and corresponding cDNA molecules are described in the examples herein below.

The proteins and nucleic acid molecules of the present invention can be obtained from their natural source, or can be produced using, for example, recombinant nucleic acid technology or chemical synthesis. Also included in the present invention is the use of these proteins and nucleic acid molecules as well as antibodies as therapeutic compositions to protect animals from flea infestation as well as in other applications, such as those disclosed below.

Flea HMT and HNC proteins and nucleic acid molecules as described herein have utility because they represent novel targets for anti-arthropod vaccines and chemotherapeutic drugs. The products and processes described herein including those of the present invention are advantageous because they enable the inhibition of arthropod development, metamorphosis, feeding, digestion and/or reproduction processes that involve HMT and/or HNC proteins.

The head and nerve cord of the flea, including antennae, brain, corpora cardiacum, corpora allata, and subesophageal and abdominal ganglion tissues are of interest as such tissues are highly enriched for transcripts that encode neuronal and endocrine targets, as well as targets involved in chemosensory and mechanosensory reception. By sequencing cDNA fragments from a library enriched in flea head and nerve cord nucleic acid sequences (referred to herein as HNC nucleic acid sequences), genes, and their respective full-length coding regions, integrally involved with flea neuronal and endocrine function are identified. Once identified, these genes can be further characterized and specific interference strategies are designed. As such, flea HNC proteins and nucleic acid molecules of the present invention have utility because they represent novel targets for anti-arthropod vaccines and chemotherapeutic drugs.

Blood-feeding insects such as fleas ingest large quantities of blood relative to their body weight and, as such, are adapted to reduce the volume of the ingested blood meal through the rapid elimination of water. In addition, the concentrations of sodium, potassium, and chloride ions in the blood meal are greater than in the hemolymph of fleas, necessitating the excretion of excessive amounts of these ions. The active transport of these ions from the hemolymph into the lumens of the Malpighian tubules and the hindgut drives the passive transport of water and other hemolymph contents into these organs as well. While passing through these organs, waste products from the hemolymph are excreted and needed nutrients, water, and salts are reabsorbed. As such, interfering with these essential processes is an important strategy for developing a product for controlling flea populations. By sequencing cDNA fragments from a library enriched in hindgut and Malpighian tubule nucleic acid sequences (referred to herein as HMT nucleic acid sequences), genes integrally involved with these processes, and their respective full-length coding regions, are identified. Once identified, these genes are further characterized and specific interference strategies can be designed. As such, flea HMT proteins and nucleic acid molecules of the present invention have utility because they represent novel targets for anti-arthropod vaccines and chemothempeutic drugs.

There is provided an isolated protein that includes a flea HMT and/or HNC protein. It is to be noted that the term "a" or "an" entity refers to one or more of that entity; for example, a protein, a nucleic acid molecule, an antibody and a therapeutic composition refers to "one or more" or "at least one" protein, nucleic acid molecule, antibody and therapeutic composition respectively. As such, the terms "a" (or "an"), "one or more" and "at least one" can be used interchangeably herein. It is also to be noted that the terms "comprising", "including", and "having" can be used interchangeably. According to the present invention, an isolated, or biologically pure, protein, is a protein that has been removed from its natural milieu. As such, "isolated" and "biologically pure" do not necessarily reflect the extent to which the protein has been purified. An isolated protein as described can be obtained from its natural source, can be produced using recombinant DNA technology, or can be produced by chemical synthesis.

As used herein, isolated flea HMT and/or HNC proteins can be full-length proteins or any homologue of such proteins. An isolated protein described herein including a homologue, can be identified in a straight-forward manner by the protein's ability to elicit an immune response against a flea HMT and/or HNC protein or by the protein's HMT and/or HNC activity. Examples of flea HMT and HNC homologue proteins include flea HMT and HNC proteins in which amino acids have been deleted (e.g., a truncated version of the protein, such as a peptide), inserted, inverted, substituted and/or derivarized (e.g., by glycosylation, phosphorylation, acetylation, myristoylation, prenylation, palmitoylation, amidation and/or addition of glycerophosphatidyl inositol) such that the homologue includes at least one epitope capable of eliciting an immune response against a flea HMT or HNC protein, and/or of binding to an antibody directed against a flea HMT or HNC protein. That is, when the homologue is administered to an animal as an immunogen, using techniques known to those skilled in the art, the animal will produce an immune response against at least one epitope of a natural flea HMT or HNC protein. The ability of a protein to effect an immune response can be measured using techniques known to those skilled in the art. As used herein, the term "epitope" refers to the smallest portion of a protein or other antigen capable of selectively binding to the antigen binding site of an antibody or a T cell receptor. It is well accepted by those skilled in the art that the minimal size of a protein epitope is about four to six amino acids. As is appreciated by those skilled in the act, an epitope can include amino acids that naturally are contiguous to each other as well as amino acids that, due to the tertiary structure of the natural protein, are in sufficiently close proximity to form an epitope. According to the present invention, an epitope includes a portion of a protein comprising at least about 4 amino acids, at least about 5 amino acids, at least about 6 amino acids, at least about 10 amino acids, at least about 15 amino acids, at least about 20 amino acids, at least about 25 amino acids, at least about 30 amino acids, at least about 35 amino acids, at least about 40 amino acids or at least about 50 amino acids in length.

A flea homologue protein may have HMT or HNC activity, i.e. the homologue exhibits an activity similar to its natural counterpart. Examples of such activities are disclosed herein; e.g., all. Methods to detect and measure such activities are known to those skilled in the art. Examples of such activities are disclosed herein; e.g. allantoinase, chitin-binding protein, sodium/potassium ATPase, ligand-gated chloride channel, ANON/23DA, malvolio, odorant binding protein-like protein, N-methyl-D-aspartate receptor associated protein, chemical sense related lipophilic ligand binding protein, Sodium/Hydrogen Transporter-like protein, a Chloride Intracellular Channel-like protein, aPeritrophin-like protein, aPeritrophin-like protein, aPeritrophin-like protein, a synaptic vesicle 2B-like protein, a voltage-gated Chloride-like protein, an anoxia upregulated protein-like protein, and a neuroendocrine specific 7B2-like protein.

Flea HMT and/or HNC homologue proteins can be the result of natural allelic variation or natural mutation. Flea HMT and/or HNC protein homologues described herein can also be produced using techniques known in the art including, but not limited to, direct modifications to the protein or modifications to the gene encoding the protein using, for example, classic or recombinant DNA techniques to effect random or targeted mutagenesis.

Flea HMT and HNC proteins as described herein are encoded by flea HMT and HNC nucleic acid molecules, respectively. As used herein, flea HMT and HNC nucleic acid molecules include nucleic acid sequences related to natural flea HMT and HNC genes, and, preferably, to *Ctenocephalides felis* HMT and HNC genes. As used herein, flea HMT and HNC genes include all regions such as regulatory regions that control production of flea HMT and HNC proteins encoded by such genes (such as, but not limited to, transcription, translation or post-translation control regions) as well as the coding region itself, and any introns or non-translated coding regions. As used herein, a nucleic acid molecule that "includes" or "comprises" a sequence may include that sequence in one contiguous array, or may include the sequence as fragmented exons such as is often found for a flea gene. As used herein, the term "coding region" refers to a continuous linear array of nucleotides that translates into a protein. A full-length coding region is that coding region that is translated into a full-length, i.e., a complete protein as would be initially translated in its natural millieu, prior to any post-translational modifications.

There is described a *C. felis* ALN gene that includes the nucleic acid sequence SEQ ID NO:1 and/or SEQ ID NO:4, a *C. felis* CBP gene that includes the nucleic acid sequence SEQ ID NO:7 and/or SEQ ID NO:10, a *C. felis* NKAB gene that includes the nucleic acid sequence SEQ ID NO:13 and/or SEQ ID NO:16, a *C. felis* LGIC gene that includes the nucleic acid sequence SEQ ID NO:19, SEQ ID NO:22, SEQ ID NO:1861, and/or SEQ ID NO:1864, a *C. felis* ANON gene that includes the nucleic acid sequence SEQ ID NO:25 and/or SEQ ID NO:28, a *C. felis* MALV gene that includes the nucleic acid sequence SEQ ID NO:31 and/or SEQ ID NO:34, a C. *felis* OS-D gene that includes the nucleic acid sequence SEQ ID NO:37 and/or SEQ ID NO:40, a *C. felis* NMDA gene that includes the nucleic acid sequence SEQ ID NO: 43 and/or SEQ ID NO:46, a *C. felis* CLBP gene that includes the nucleic acid sequence SEQ ID NO: 153, SEQ ID NO:156, SEQ ID NO:159, SEQ ID NO: 162, SEQ ID NO:165, and/or SEQ ID NO:168, a *C. felis* NAH gene that includes the nucleic acid sequence SEQ ID NO:1867 and/or SEQ ID NO:1870, a *C. felis* CLIC gene according to the invention that includes the nucleic acid sequence SEQ ID NO: 1872 and/or SEQ ID NO: 1875, a *C. felis* PL2 gene that includes the nucleic acid sequence SEQ ID NO:1877, SEQ ID NO:1878, SEQ ID NO:1880, SEQ ID NO:1882 and/or SEQ ID NO:1885, a *C. felis* PL3 gene that includes the nucleic acid sequence SEQ ID NO:1887 and/or SEQ ID NO: 1890, a *C. felis* PL4 gene that includes the nucleic acid sequence SEQ ID NO:1896 and/or SEQ ID NO:1899, a *C. felis* SVP gene that includes the nucleic acid sequence SEQ ID NO:1901 and/or SEQ ID NO:1904, a *C. felis* VGCC gene that includes the nucleic acid sequence SEQ ID NO:1914 and/or SEQ ID NO:1917, a *C. felis* AUP gene that includes the nucleic acid sequence SEQ ID NO:1919 and/or SEQ ID NO:1922, a *C. felis* 7B2 gene that includes the nucleic acid sequence SEQ ID NO:1924 and/or SEQ ID NO:1927, a *C. felis* gene that includes a nucleic acid sequence of Table I, Table II, Table III and/or Table IV; as well as the complements of any of these nucleic acid sequences. These nucleic acid sequences are further described herein. For example, nucleic acid sequence SEQ ID NO:1 represents the deduced sequence of the coding strand of a *C. felis* cDNA denoted herein as *C. felis* ALN nucleic acid molecule $nCfALN_{2057}$, the production of which is disclosed in the Examples. Nucleic acid molecule SEQ ID NO:1 comprises an apparently full-length coding region. The complement of SEQ ID NO:1 (represented herein by SEQ ID NO:3) refers to the nucleic acid sequence of the strand fully complementary to the strand having SEQ ID NO:1, which can easily be determined by those skilled in the art. Likewise, a nucleic acid sequence complement of any nucleic acid sequence of the present invention refers to the nucleic acid sequence of the nucleic acid strand that is fully complementary to (i.e., can form a complete double helix with) the strand for which the sequence is cited. For example, the complements of SEQ ID NOs: 4, 7, 10, 13, 16, 19, 22, 25, 28, 31, 34, 37, 40, 43, 46, 153, 156, 159, 162, 165, and 168 are SEQ ID NOs: 6, 9, 12, 15, 18, 21, 24, 27, 30, 33, 36, 39, 42, 45, 48, 155, 158, 161, 164, 167, and 170, respectively. It should be noted that since nucleic acid sequencing technology is not entirely error-free, SEQ ID NO:1 (as well as other nucleic acid and protein sequences presented herein) represents an apparent nucleic acid sequence of the nucleic acid molecule encoding an ALN protein.

Translation of SEQ ID NO:1, the coding strand of $nCfALN_{2057}$, as well as translation of SEQ ID NO:4, the coding strand of $nCfALN_{1152}$, which represents the coding region of SEQ ID NO:1, each yields a protein of about 384 amino acids, denoted herein as $PCfALN_{384}$, the amino acid sequence of which is presented in SEQ ID NO:2, assuming a first in-frame codon extending from nucleotide 1 to nucleotide 3 of SEQ ID NO:4.

Translation of SEQ ID NO:7, the coding strand of $nCfCBP_{1128}$, as well as translation of SEQ ID NO:10, the coding strand of $nCfCBP_{1128}$, which represents the coding region of SEQ ID NO:7, each yields a protein of about 272 amino acids, denoted herein as $PCfCBP_{272}$, the amino acid sequence of which is presented in SEQ ID NO:8, assuming a first in-frame codon extending from nucleotide I to nucleotide 3 of SEQ ID NO:10.

Translation of SEQ ID NO: 13, the coding strand of $nCfNKAB_{1714}$, as well as translation of SEQ ID NO:16, the coding strand of $nCfNKAB_{978}$, which represents the coding region of SEQ ID NO:13, each yields a protein of about 326 amino acids, denoted herein as $PCfNKAB_{326}$, the amino acid sequence of which is presented in SEQ ID NO:14, assuming a first in-frame codon extending from nucleotide 1 to nucleotide 3 of SEQ ID NO: 16.

Translation of SEQ ID NO:19, the coding strand of $nCfLGIC_{2240}$, as well as translation of SEQ ID NO:22, the coding strand of $nCfLGIC_{1707}$, which represents the coding region of SEQ ID NO:19, each yields a protein of about 569 amino acids, denoted herein as $PCfLGIC_{569}$, the amino acid sequence of which is presented in SEQ ID NO:20, assuming a first in-frame codon extending from nucleotide 1 to nucleotide 3 of SEQ ID NO:22.

Translation of SEQ ID NO:25, the coding strand of $nCfANON_{1429}$, as well as translation of SEQ ID NO:28, the coding strand of $nCfANON_{1194}$, which represents the coding region of SEQ ID NO:25, each yields a protein of about 398 amino acids, denoted herein as $PCfANON_{398}$, the amino acid sequence of which is presented in SEQ ID NO:26, assuming a first in-frame codon extending from nucleotide 1 to nucleotide 3 of SEQ ID NO:28.

Translation of SEQ ID NO:31, the coding strand of $nCfMALV_{765}$, as well as translation of SEQ ID NO:34, the coding strand of $nCfMALV_{762}$, which represents the coding region of SEQ ID NO:31, each yields a protein of about 327 amino acids, denoted herein as $PCfMALV_{254}$, the amino acid sequence of which is presented in SEQ ID NO:32, assuming a first in-frame codon extending from nucleotide 1 to nucleotide 3 of SEQ ID NO:34.

Translation of SEQ ID NO:37, the coding strand of $nCfOSD_{604}$, as well as translation of SEQ ID NO:40, the coding strand of $nCfOSD_{405}$, which represents the coding region of SEQ ID NO:37, each yields a protein of about 135 amino acids, denoted herein as $PCfOSD_{135}$, the amino acid sequence of which is presented in SEQ ID NO:38, assuming a first in-frame codon extending from nucleotide 1 to nucleotide 3 of SEQ ID NO:40.

Translation of SEQ ID NO:43, the coding strand $ofNMDA_{1227}$, as well as translation of SEQ ID NO:46, the coding strand of $nCfNMDA_{738}$, which represents the coding region of SEQ ID NO:43, each yields a protein of about 246 amino acids, denoted herein as $PCfNMDA_{246}$, the amino acid sequence of which is presented in SEQ ID NO:44, assuming a first in-frame codon extending from nucleotide 1 to nucleotide 3 of SEQ ID NO:46.

Translation of SEQ ID NO:153, the coding strand of $nCfCLBP1A_{633}$, as well as translation of SEQ ID NO: 156, the coding strand of $nCfCLBP1A_{441}$, which represents the coding region of SEQ ID NO: 153, each yields a protein of about 147 amino acids, denoted herein as $PCfCLBP_{147}$, the amino acid sequence of which is presented in SEQ ID NO: 154, assuming a first in-frame codon extending from nucleotide 1 to nucleotide 3 of SEQ ID NO: 156.

Translation of SEQ ID NO:162, the coding strand of $nCfCLBP2A_{631}$, as well as translation of SEQ ID NO:165, the coding strand of $nCfCLBP2A_{441}$, which represents the coding region of SEQ ID NO:162, each yields a protein of about 147 amino acids, denoted herein as $PCfCLBP2A_{147}$, the amino acid sequence of which is presented in SEQ ID NO:163, assuming a first in-frame codon extending from nucleotide 1 to nucleotide 3 of SEQ ID NO:165.

Translation of SEQ ID NO:1861, the coding strand of $nCfLGIC_{2739}$, as well as translation of SEQ ID NO: 1864, the coding strand of $nCfLGIC_{2016}$, which represents the coding region of SEQ ID NO:1861, each yields a protein of about 672 amino acids, denoted herein as $PCfLGIC_{672}$, the amino acid sequence of which is presented in SEQ ID NO: 1862, assuming a first in-frame codon extending from nucleotide 1 to nucleotide 3 of SEQ ID NO:1864.

Translation of SEQ ID NO:1867, the coding strand of $nCfNAH_{2080}$ as well as translation of SEQ ID NO:1870, the coding strand of $nCfNAH_{1824}$, which represents the coding region of SEQ ID NO:1867, each yields a protein of about 608 amino acids, denoted herein as $PCfNAH_{608}$, the amino acid sequence of which is presented in SEQ ID NO:1868, assuming a first in-frame codon extending from nucleotides 1 to nucleotide 3 of SEQ ID NO:1870.

According to the present invention translation of SEQ m NO: 1872. the coding strand of $nCfCLIC_{2283}$, as well as translation of SEQ ID NO:1875, the coding strand of $nCfCLIC_{786}$, which represents the coding region of SEQ m NO: 1872, each yields a protein of about 262 amino acids, denoted herein as $PCfCLIC_{262}$, the amino acid sequence of which is presented in SEQ ID NO:1873, assuming a first in-frame codon extending from nucleotide 1 to nucleotide 3 of SEQ ID NO: 1875.

Translation of SEQ ID NO:1882, the coding strand of $nCfPL2_{1477}$, as well as translation of SEQ ID NO:1885, the coding strand of $nCfPL2_{1359}$, which represents the coding region of SEQ ID NO:1882, each yields a protein of about 453 amino acids, denoted herein as $PCfPL2_{453}$, the amino acid sequence of which is presented in SEQ ID NO:1883, assuming a first in-frame codon extending from nucleotide 1 to nucleotide 3 of SEQ ID NO: 1885.

Translation of SEQ ID NO:1887, the coding strand of $nCfPL3_{406}$, as well as translation of SEQ m NO: 1890, the coding strand of $nCfPL3_{243}$, which represents the coding region of SEQ ID NO:1887, each yields a protein of about 81 amino acids, denoted herein as $PCfPL3_{81}$, the amino acid sequence of which is presented in SEQ ID NO:1888, assuming a first in-frame codon extending from nucleotide 1 to nucleotide 3 of SEQ ID NO:1890.

Translation of SEQ ID NO:1896, the coding strand of $nCfPL4_{1062}$, as well as translation of SEQ ID NO:1899, the coding strand of $nCfPL4_{855}$, which represents the coding region of SEQ ID NO:1896, each yields a protein of about 285 amino acids, denoted herein as $PCfPL4_{285}$, the amino acid sequence of which is presented in SEQ ID NO:1897, assuming a first in-frame codon extending from nucleotide 1 to nucleotide 3 of SEQ ID NO:1899.

Translation of SEQ ID NO:1901, the coding strand of $nCfSVP_{1875}$, as well as translation of SEQ ID NO:1904, the coding strand of $nCfSVP_{1590}$, which represents the coding region of SEQ ID NO:1901, each yields a protein of about 530 amino acids, denoted herein as $PCfSVP_{530}$, the amino acid sequence of which is presented in SEQ ID NO:1902, assuming a first in-frame codon extending from nucleotide I to nucleotide 3 of SEQ ID NO:1904.

Translation of SEQ ID NO:1914, the coding strand of $nCfVGCC_{3126}$, as well as translation of SEQ ID NO:1917, the coding strand of $nCfVGCC_{2533}$, which represents the coding region of SEQ ID NO:1914, each yields a protein of about 851 amino acids, denoted herein as $PCfVGCC_{851}$, the amino acid sequence of which is presented in SEQ ID NO: 1915, assuming a first in-frame codon extending from nucleotide 1 to nucleotide 3 of SEQ ID NO:1917.

Translation of SEQ ID NO:919, the coding strand of $nCfAUP_{1181}$, as well as translation of SEQ ID NO:1922, the coding strand of $nCfAUP_{306}$, which represents the coding region of SEQ ID NO:1919, each yields a protein of about 102 amino acids, denoted herein as $PCfAUP_{102}$, the amino acid sequence of which is presented in SEQ ID NO:1920, assuming a first in-frame codon extending from nucleotide I to nucleotide 3 of SEQ ID NO: 1922.

Translation of SEQ ID NO:1924, the coding strand of $nCf7B2_{2161}$, as well as translation of SEQ ID NO:1927, the coding strand of $nCf7B2_{801}$, which represents the coding region of SEQ ID NO:1924, each yields a protein of about 267 amino acids, denoted herein as $PCf7B2_{267}$, the amino acid sequence of which is presented in SEQ ID NO:1925, assuming a first in-frame codon extending from nucleotide I to nucleotide 3 of SEQ ID NO:1927.

Table I represents a variety of flea HNC nucleic acid molecules described herein. Also cited in Table I are nucleic acid molecules from other organisms which share the closest sequence identity with the cited HNC sequences of the present invention, as determined by submitting each HNC sequence for a search through the National Center for Biotechnology Information (NCBI), National Library of Medicine, National Institute of Health, Baltimore, MD, using the BLAST network. This database includes SwissProt + PIR + SPupdate + GenPept + GPUpdate + PDB databases. The search was conducted using the xBLAST function using default parameters.

TABLE I

| SEQ ID NO | Name | Genbank Homology | *Organism* |
|---|---|---|---|
| 63 | 2096-46 | ATPase 6 | *D. melanogaster* |
| 64 | 2098-25 | ATP synthase delta chain | *Sus scrofa* |
| 65 | 2098-34 | F1-ATPase epsilon-subunit | *Ipomoea batatas* |
| 66 | 2110-19 | ATP synthase beta subunit | *Drosophila pseudoobscura* |
| 67 | 2113-15 | ATP synthase delta chain, | *Sus scrofa* |
| 68 | 2180-31 | ATP synthase alpha subunit precursor | *Rattus rattus* |
| 69 | 2224-50 | oligomysin sensitivity conferring protein | *D. melanogaster* |
| 70 | 2116-51 | cysteine dioxygenase | *Homo sapiens* |
| 71 | 2116-55 | pyrroline-5-carboxylate dehydrogenase (P5CDh) | *Homo sapiens* |
| 72 | 2124-17 | AMP deaminase | *Homo sapiens* |
| 73 | 2138-38 | ubiquitin | *Mus musculus* |
| 74 | 2184-59 | manganese superoxide dismutase | *Homo sapiens* |
| 75 | 2096-24 | muscle LIM protein 1 | *D. melanogaster* |
| 76 | 2140-53 | F25H5.1a | *C. elegans* |

(continued)

| SEQ ID NO | Name | Genbank Homology | *Organism* |
|---|---|---|---|
| 77 | 2176-41 | Frazzled | *D. melanogaster* |
| 78 | 2223-11 | LIMm domain-containing protein | *C. elegans* |
| 79 | 2223-53 | deleted in split hand/split foot 1 (DSS1) | *Homo sapiens* |
| 80 | 2225-28 | stranded-at-second | *D. melanogaster* |
| 81 | 2099-61 | histone H3 | *Spisula solidissima* |
| 82 | 2114-21 | STE12 | *S. cereviciae* |
| 83 | 2117-4 | Rad51 homolog | *Bombyx mori* |
| 84 | 2138-46 | heat shock protein p27 | *D. immitis* |
| 85 | 2182-37 | heat shock protein 70 | *D. immitis* |
| 86 | 2211-32 | BTB-II protein domain gene | *D. melanogaster* |
| 87 | 2223-7 | heat shock protein . ... | *D, melanogaster* |
| 88 | 2224-17 | heat shock protein 86 | *Homo sapiens* |
| 89 | 2225-16 | POU domain protein | *D. melanogaster* |
| 90 | 2225-18 | nucleolin | *Xenopus laevis* |
| 91 | 2212-85 | thyroid hormone receptor-associated protein complex component TRAP220 | *Homo sapiens* |
| 92 | 2211-21 | T03D8.3 | *C. elegans* |
| 93 | 2223-67 | hepatoma derived growth factor (HDGF) | *Mus musculus* |
| 94 | 2225-61 | tyrosine hydroxylase type 1 (neuronal form) | *D. melanogaster* |
| 95 | 2097-7 | sarco/endoplasmic reticulum-type Ca-2+-ATPase | *D. melanogaster* |
| 96 | 2098-27 | calcium-transporting ATPase | *D. melanogaster* |
| 97 | 2099-19 | calcium channel alpha-1 subunit | *Aplysia californica* |
| 98 | 2120-5 | P-type voltage-gated calcium channel alpha 1 subunit homolog | *Homo sapiens* |
| 99 | 2124-2 | sarco/endoplasmic reticulum Ca2+-ATPase (SERCA) | *Procambarus clarkii* |
| 100 | 2182-43 | sulfonylurea receptor 2b | *Mus musculus* |
| 101 | 2223-18 | Sodium-Potassium-Chloride cotransporter | *D. melanogaster* |
| 102 | 2223-63 | sarco/endoplasmic reticulum-type Ca2(+)-ATPase | *D. melanogaster* |
| 103 | 2224-13 | similar to ABC transporters | *C. elegans* |
| 104 | 2098-3 | Camguk | *D. melanogaster* |
| 105 | 2101-9 | UNC-89 | *C. elegans* |
| 106 | 2132-31 | arginine kinase | *Homarus gammarus* |
| 107 | 2141-51 | casein kinase-II beta | *Oryctolagus cuniculus* |
| 108 | 2178-18 | diacylglycerol kinase eta | *Cricetinae* |
| 109 | 2180-32 | retinoid- and fatty acid-binding glycoprotein | *D. melanogaser* |
| 110 | 2137-23 | vitellogenin | *Aedes aegypti* |
| 111 | 2144-14 | nuclear localization signal spot 1 | *Mus musculus* |
| 112 | 2212-13 | putative n-terminal acetyltransferase | *S. cereviciae* |

(continued)

| SEQ ID NO | Name | Genbank Homology | *Organism* |
|---|---|---|---|
| 113 | 2212-27 | clathrin associated protein AP47 | *Drosophila grimshawi* |
| 114 | 2223-28 | O1 chloroquine-resistance protein | *Plasmodium falciparans* |
| 115 | 2224-14 | vitellogenin | *Athalia rosae* |
| 116 | 2224-15 | antigen NY-CO-3 | *Homo sapiens* |
| 117 | 2225-24 | carbonic anhydrase | *C. elegans* |
| 118 | 2225-58 | yk500f6.3 | *C. elegans* |
| 119 | 2225-76 | unknown | *Homo sapiens* |
| 120 | 2224-86 | BmP109 (cerebroside sulfate activator protein family) | *Bombyx mori* |
| 121 | 2225-23 | intersectin | *Homo sapiens* |
| 122 | 2170-16 | chemical-sense-related lipophilic-ligand-binding protein | *Phormia regina* |
| 123 | 2176-2 | olfactory receptor protein 2.4 | *Danio rerio* |
| 124 | 2212-63 | olfactory receptor | *Xenopus laevis* |
| 125 | 2224-77 | inner mitochondrial membrane translocase Tim23 | *Homo sapiens* |
| 126 | 2225-12 | sodium-dependent multi-vitamin transporter | *Rattus norvegicus* |
| 127 | 2225-42 | ribophorin I | *Rattus norvegicus* |
| 128 | 2101-59 | phosphate carrier protein | *C. elegans* |
| 129 | 2132-38 | proteinase inhibitor | *Locusta migratoria* |
| 130 | 2174-72 | HE4 protein | *Homo sapiens* |
| 131 | 2211-48 | spermatogenic cell/sperm-associated Tat-binding homologue | *Rattus norvegicus* |
| 132 | 2110-23 | Gcap1 gene product | *Mus musculus* |
| 133 | 2116-64 | toll protein | *D. melanogaster* |
| 134 | 2124-3 | tuberin (TSC2) gene | *Homo sapiens* |
| 135 | 2178-55 | RAS-like protein | *Gallus gallus* |
| 136 | 2223-35 | Rho1 gene product | *D. melanogaster* |
| 137 | 2224-82 | paxillin | *Homo sapiens* |
| 138 | 2225-44 | adenylyl cyclase-associated protein (CAP) | *Homo sapiens* |
| 139 | 2225-80 | adenylate kinase | *Gallus gallus* |
| 140 | 2110-52 | hydroxyproline-rich glycoprotein | *Phaseolus vulgaris* |
| 141 | 2115-49 | mitogen inducible gene mig-2 | *Homo sapiens* |
| 142 | 2116-5 | F52H3.5 | *C. elegans* |
| 143 | 2172-89 | 12D3 antigen | *Babesia bovis* |
| 144 | 2178-20 | frameshift | *P. falciparum* |
| 145 | 2178-81 | KIAA0066 | *Homo sapeins* |
| 146 | 2182-16 | Y57G11C.4 | *C. elegans* |
| 147 | 2182-53 | C16C10.5 | *C. elegans* |
| 148 | 2211-8 | Unknown | *Homo sapiens* |

(continued)

| SEQ ID NO | Name | Genbank Homology | *Organism* |
|---|---|---|---|
| 149 | 2211-31 | hopothetical protein | *Arabidopsis thaliana* |
| 150 | 2223-54 | ORF YNL207w | *S. cereviciae* |
| 151 | 2224-94 | 14.3 kDa perchloric acid soluble protein | *Capra hircus* |
| 152 | 2225-36 | EST clone | *C. elegans* |
| 1719 | 2228-2 | BIGH3 | *H. sapiens* |
| 1720 | 2228-5 | H protein | *H. sapiens* |
| 1721 | 2228-8 | ubiquinol-cytochrome c reductase | *Schizosaccharomyces pombe* |
| 1722 | 2228-11 | similar to mitochondrial ATPase inhibitors | *C. elegans* |
| 1723 | 2228-16 | Putative enzyme | *E. coli* |
| 1724 | 2228-18 | Ribosomal protein L7A | *Drosophila* |
| 1725 | 2228-22 | Troponin-I wings up A | *Drosophila* |
| 1726 | 2228-25 | tls gene product | *E. coli* |
| 1727 | 2228-27 | YCR521 gene product | *Saccharomyces cerevisiae* |
| 1728 | 2228-28 | putative transport system permease protein | *E. coli* |
| 1729 | 2228-32 | SapA protein | *E. coli* |
| 1730 | 2228-34 | Putative protein | *Arabidopsis thaliana* |
| 1731 | 2228-37 | Ada | *E. coli* |
| 1732 | 2228-39 | Titin | *H. sapiens* |
| 1733 | 2228-42 | adenylosuccinate synthetase | *Mus musculus* |
| 1734 | 2228-43 | transfer RNA-Ala synthetase | *B. mori* |
| 1735 | 2228-44 | C4 zinc finger DNA-binding protein | *Drosophila* |
| 1736 | 2228-48 | heme A: farnesyltransferase | *H. sapiens* |
| 1737 | 222B-51 | URF 4L (aa 1-96) | *Drosophila* |
| 1738 | 2228-53 | DOLICHOL-PHOSPHATE MANNOSYLTRANSFERASE | *E. coli* |
| 1739 | 2228-58 | troponin-T | *Drosophila* |
| 1740 | 2228-59 | protein disulfide isomerase | *Drosophila* |
| 1741 | 2228-63 | orf, hypothetical protein | *E. coli* |
| 1742 | 2228-66 | ilvl polypeptide | *E. coli* |
| 1743 | 2228-68 | orf, hypothetical protein | *E. coli* |
| 1744 | 2228-72 | Respiratory nitrate reductase 1 alpha chain | *E. coli* |
| 1745 | 2228-77 | homolog of virulence factor | *E. coli* |
| 1746 | 2228-84 | ORF o164 | *E. coli* |
| 1747 | 2228-91 | nuclear protein E3-3 orf1 | *Rattus norvegicus* |
| 1748 | 2245-66 | Troponin C | *Drosophila* |
| 1749 | 2245-70 | Predicted secreted protein | *Plasmodium falciparum* |
| 1750 | 2245-72 | Cytochrome C-1 | *H. sapiens* |
| 1751 | 2245-75 | rpoB | *Plasmodium falciparum* |

(continued)

| SEQ ID NO | Name | Genbank Homology | *Organism* |
|---|---|---|---|
| 1752 | 2245-78 | sarco(endo)plasmic reticulum-type calcium ATPase | *Helliothis virescens* |
| 1753 | 2246-31 | Ras-related GTP-binding protein | *H. sapiens* |
| 1754 | 2246-57 | Similar to inositol 1,4,5-triphosphate receptor | *C. elegans* |
| 1755 | 2246-61 | reverse transcriptase-like protein | *Aedes aegypti* |
| 1756 | 2247-13 | polyprotein | *Drosophila* |
| 1757 | 2247-14 | ORF2 for putative reverse transcriptase | *Drosophila* |
| 1758 | 2247-42 | Asparaginyl tRNA Synthetase | *H. sapiens* |
| 1759 | 2247-44 | calcium binding protein | *Drosophila* |
| 1760 | 2247-58 | similar to Fibronectin type III domain | *C. elegans* |
| 1761 | 2247-62 | reverse transcriptase | *Drosophilia* |
| 1762 | 2247-65 | gag-like protein | *Culex pipiens* |
| 1763 | 2247-79 | L-3-phosphoserine phosphatase | *H. sapiens* |
| 1764 | 2247-80 | esterase E4 | *Myzus persicae* |
| 1765 | 2247-89 | Similar to aldehyde dehydrogenase | *C. elegans* |
| 1766 | 2248-76 | O-44 protein | *Rattus sp.* |
| 1767 | 2248-85 | cDNA isolated for this protein using a monoclonal antibody directed against the p27k prosomal protein | *H. sapiens* |
| 1768 | 2249-3 | Projectin | *Drosophila* |
| 1769 | 2249-5 | ORF_ID:o312#14 | *E. coli* |
| 1770 | 2249-9 | Heat shock protein 60 | *Culicoides variipennis* |
| 1771 | 2249-11 | enigma protein | *H. sapiens* |
| 1772 | 2249-12 | alpha,alpha-trehalose glucohydrolase | *Oryctolagus cuniculus* |
| 1773 | 2249-13 | small GTP binding protein | *Drosophila* |
| 1774 | 2249-14 | Spermidine/putrescine transport system permease | *E. coli* |
| 1775 | 2249-19 | nueroendocrine-specific protein C | *H. sapiens* |
| 1776 | 2249-21 | a-agglutinin core subunit | *Saccharomyces cerevisiae* |
| 1777 | 2249-24 | KIAA0337 | *H. sapiens* |
| 1778 | 2249-34 | su(wa) protein | *Drosophila* |
| 1779 | 2249-42 | regulator of kdp operon | *E. coli* |
| 1780 | 2249-59 | No definition line found | *C. elegans* |
| 1781 | 2249-60 | proline oxidase | *Drosophila* |
| 1782 | 2249-62 | Formate acetyltransferase | *E. coli* |
| 1783 | 2249-70 | similar to HECT-domain | *C. elegans* |
| 1784 | 2249-75 | PHOSPHORIBOSYLFORMYLGLYCINAM *E.* IDINE CYCLO-LIGASE | *coli* |
| 1785 | 2249-77 | Hypothetical 38.5 kd protein in agal-mtr intergenic region precursor | *E. coli* |
| 1786 | 2249-85 | D4L | *Variola virus* |
| 1787 | 2249-87 | similar to isocitrate dehydrogenase | *C. elegans* |

(continued)

| SEQ ID NO | Name | Genbank Homology | *Organism* |
|---|---|---|---|
| 1788 | 2250-6 | Fii (head-tail joining;117) | *Bacteriophage Lambda* |
| 1789 | 2250-7 | possible NAGC-like transcriptional regulator | *E. coli* |
| 1790 | 2250-10 | cysteine string protein | *Bos taurus* |
| 1791 | 2250-13 | Tol B protein | *E. coli* |
| 1792 | 2250-14 | 6-phosphogluconate dehydratase | *E. coli* |
| 1793 | 2250-15 | 6-phosphogluconate dehydratase | *E. coli* |
| 1794 | 2250-22 | PSST subunit of the NADH: ubiquinone oxidoreductase | *Bos taurus* |
| 1795 | 2250-30 | sol i 3 antigen | *Solenopsis invicta* |
| 1796 | 2250-36 | predicted using Genefinder; similar to tRNA synthetases class I (E and Q | *C. elegans* |
| 1797 | 2250-37 | PNP | *H. sapiens* |
| 1798 | 2250-42 | ORF_ID:o331#2 | *E. coli* |
| 1799 | 2250-44 | Extensin | *E. coli* |
| 1800 | 2250-47 | OAF o654 | *E. coli* |
| 1801 | 2250-48 | Gcap1 gene product | *Mus musculus* |
| 1802 | 2250-52 | similar to human MLH1 on chromosome 3p21 | *Mus musculus* |
| 1803 | 2250-53 | Hypothetical 27.6 kd protein in hpt-panD intergenic region. | *E. coli* |
| 1804 | 2250-58 | UmuC protein | *E. coli* |
| 1805 | 2250-61 | dJ134E15.1 (Blimp-1 | *H. sapiens* |
| 1806 | 2250-63 | ribosomal protein L23-related product homolog | *Rattus rattus* |
| 1807 | 2250-65 | hypothetical protein MJ1143 | *E. coli* |
| 1808 | 2250-68 | HI0025 homology | *E. coli* |
| 1809 | 2250-77 | R34094_1 | *H. sapiens* |
| 1810 | 2250-78 | erythrocyte binding protein | *Plasmodium yoelii* |
| 1811 | 2250-79 | fosmidomycin resistance protein | *E. coli* |
| 1812 | 2250-81 | cyclophilin 1 | *Drosophila* |
| 1813 | 2250-83 | putative glutamine synthetase | *E. coli* |
| 1814 | 2251-3 | J (tail:host specificity;1132) | *Bacteriophage Lambda* |
| 1815 | 2251-5 | Molybdopterin biosynthesis MoeB protein | *E. coli* |
| 1816 | 2251-6 | Fo-ATP synthase subunit b | *Drosophila* |
| 1817 | 2251-9 | citrate lyase alpha chain | *E. coli* |
| 1818 | 2251-10 | cuticle protein ACP65A | *Drosophila* |
| 1819 | 2251-13 | H repeat-associated protein in rhsC 3'region (orf-h3 | *E. coli* |
| 1820 | 2251-20 | glycine-rich protein | *Arabadopsis thaliana* |
| 1821 | 2251-23 | 2-oxoglutarate dehydrogenase precursor | *H. sapiens* |
| 1822 | 2251-29 | NFX1 | *H. sapiens* |
| 1823 | 2251-32 | ebgR product, repressor | *E. coli* |

(continued)

| SEQ ID NO | Name | Genbank Homology | *Organism* |
|---|---|---|---|
| 1824 | 2251-41 | neural protein | *Drosophila* |
| 1825 | 2251-45 | similar to unidentified ORF | *E. coli* |
| 1826 | 2251-46 | NADH:ubiquinone oxidoreductase b17.2 subunit | *Bos taurus* |
| 1827 | 2251-49 | tyrosine kinase | *Drosophila* |
| 1828 | 2251-50 | coded for by C. elegans cDNA yk89e9.5 | *C. elegans* |
| 1829 | 2251-57 | H (tail component;853) | *Bacteriophage Lambda* |
| 1830 | 2251-60 | Lysyl tRNA Synthetase | *Drosophila* |
| 1831 | 2251-62 | 7,8-diamino-pelargonic acid aminotransferase | *E. coli* |
| 1832 | 2251-64 | actin related protein | *Drosophila* |
| 1833 | 2252-6 | discs-large tumor suppressor | *Drosophila* |
| 1834 | 2252-16 | S-adenosylmethionine decarboxylase | *E. coli* |
| 1835 | 2252-17 | F52H3.5 | *E. coli* |
| 1836 | 2252-21 | translationally controlled tumor protein | *Oryctolagus cuniculus* |
| 1837 | 2252-31 | GTP binding protein | *Rattus rattus* |
| 1838 | 2252-34 | mitochondrial porin transcript 1 | *Drosophila* |
| 1839 | 2252-38 | cuticle protein | *Manduca sexta* |
| 1840 | 2252-39 | Similarity to Rat CD63 antigen | *C. elegans* |
| 1841 | 2252-41 | similar to S. cerevisiae Lpg20p | *E. coli* |
| 1842 | 2252-48 | cut E | *E. coli* |
| 1843 | 2252-61 | Histone H3 | *Spisula solidissima* |
| 1844 | 2252-66 | ea10 (ssb;122) | *Bacteriophage Lambda* |
| 1845 | 2252-71 | Mao C protein | *E. coli* |
| 1846 | 2252-72 | miniparomyosin | *Drosophila* |
| 1847 | 2252-73 | pherophorin-S | *Volvox carteri* |
| 1848 | 2252-80 | cyclophilin | *Mus musculus* |
| 1849 | 2252-84 | alternate gene name yhhG | *E. coli* |
| 1850 | 2222-20 | nucleoporin Nup98 | *rat* |
| 1851 | 2222-21 | hypothetical protein | *Escherichia coli* |
| 1852 | 2222-36 | ribosomal protein S11 | *human* |
| 1853 | 2222-39 | hypothetical protein PFB0315w | *Plasmodium-falciparans* |
| 1854 | 2222-50 | serine/threonine-specific protein k. | *Plasmodium falciparans* |
| 1855 | 2222-58 | hypothetical protein C25E10.9 | *C. elegans* |
| 1856 | 2222-64 | transporting ATP synthase | *bovine* |
| 1857 | 2222-94 | tricarboxylate carrier | *rat* |
| 1858 | 2218-95 | anoxia upregulated protein | *Drosophila melanogaster* |

Table II represents a variety of flea HMT nucleic acid molecules described herein. Also cited in Table II are nucleic acid molecules from other organisms which share the closest sequence identity with the cited HMT sequences of the present invention, as determined by a search through the BLAST network as described above.

TABLE II

| SEQ ID NO: | Name: | GenBank Homology | *Organism* |
|---|---|---|---|
| 171 | 2094-23 | mitochondrian ATP synthase, alpha subunit | *Drosophila melanogaster* |
| 172 | 2104-20 | mitochondrial ATP synthase | *Drosophila melanogaster* |
| 173 | 2105-14 | ATP synthase gamma-subunit | *Homo sapiens* |
| 174 | 2167-72 | oligomysin sensitivity conferring protein | *Drosophila melanogaster* |
| 175 | 2179-20 | ATPase 6 | *Drosophila melanogaster* |
| 176 | 2193-60 | ATP synthase subunit B | *Schizaphis graminum* |
| 177 | 2229-41 | ATP synthase alpha subunit | *D. melanogaster* |
| 178 | 2231-35 | 9 kD basic protein | *D. melanogaster* |
| 179 | 2231-47 | ATP synthase alpha-subunit | *Bos taurus* |
| 180 | 2232-95 | mitochondrial ATP synthase subunit, 9 | *Homo sapiens* |
| 181 | 2084-56 | Late embryogenesis abundant protein | *Picea glauca* |
| 182 | 2084-36 | TGF-beta masking protein/stranded at second | *Drosophila melanogaster* |
| 183 | 2086-2 | Argonaute protein | *Arabidopsis thaliana* |
| 184 | 2196-92 | like Drosophila HMPB homeotic proboscipedia protein | *C. elegans* |
| 185 | 2092-27 | DMDHEM2 | *Drosophila melanogaster* |
| 186 | 2094-21 | SelD protein | *Drosophila melanogaster* |
| 187 | 2106-11 | Unr | *Rattus norvegicus* |
| 188 | 2231-15 | cno (canoe) | *D. melanogaster* |
| 189 | 2230-79 | ALR homologue | *D. melanogaster* |
| 190 | 2232-42 | saxophone serine-threonine kinase receptor | *D. melanogaster* |
| 191 | 2232-68 | selenophosphate synthetase | *D. melanogaster* |
| 192 | 2088-11 | MMTAX107, TAX responsive element binding protein | *Mus musculus* |
| 193 | 2089-2 | cs Dna J-1 | *Cucumis sativus* |
| 194 | 2090-7 | Lethal (2) TID | *Drosophila melanogaster* |
| 195 | 2102-33 | monocytic leukaemia zinc finger protein | *homo sapiens* |
| 196 | 2105-26 | orf1 5' of EpoR | *Mus musculus* |
| 197 | 2106-6 | contains similarity to EGF-1 | *C. elegans* |
| 198 | 2106-9 | HSP70 protein | *Ceratitis capitata* |
| 199 | 2084-60 | 82 kD heat shock protein | *Drosophila pseudobscura* |
| 200 | 2108-59 | PAR domain protein | *Drosophila melanogaster* |
| 201 | 2156-34 | yk29g12.3 | *C. elegans* |
| 202 | 2161-17 | segmentation protein | *Drosophila melanogaster* |
| 203 | 2162-28 | heat shock protein 70, hsp70A2 | *Anopheles albimanus* |
| 204 | 2187-18 | Heat shock protein 70 | *Anopheles albimanus* |

(continued)

| SEQ ID NO: | Name: | GenBank Homology | Organism |
|---|---|---|---|
| 205 | 2173-77 | Heat shock protein hsp70 | *D. melanogaster* |
| 206 | 2165-30 | nucleolar protein | *Drosophila melanogaster* |
| 207 | 2165-59 | contains similarity to C4-type zinc fingers | *C. elegans* |
| 208 | 2177-80 | zinc finger protein | *Mus musculus* |
| 209 | 2181-45 | PAR domain protein 1 | *Drosophila melanogster* |
| 210 | 2185-9 | Heat shock protein-70 | *Anopheles albimanus* |
| 211 | 2185-82 | segmentation protein | *Drosophila melanogaster* |
| 212 | 2188-33 | transcriptional repressor protein | *Drosophila melanogaster* |
| 213 | 2203-18 | Mastermind | *Drosophila virilis* |
| 214 | 2205-82 | high mobility group protein 1a | *Chironomus tentans* |
| 215 | 2230-26 | DNA repair protein | *D. melanogaster* |
| 216 | 2230-71 | homologue of seven in absentia | *Homo sapiens* |
| 217 | 2230-89 | nuclear speckle-type protein, SPOP | *Homo sapiens* |
| 218 | 2230-96 | heat shock protein | *D. melanogaster* |
| 219 | 2231-7 | hypothetical protein | *S.pombe* |
| 220 | 2231-38 | Rad51 homolog | *Bombyx mori* |
| 221 | 2231-81 | DNA repair protein | *D. melanogaster* |
| 222 | 2232-2 | cellular nucleic acid binding protein | *Xenopus laevis* |
| 223 | 2234-63 | heat shock protein 70 | *Trichoplusia ni* |
| 224 | 2232-77 | actin-binding double-zinc-finger protein (abLIM) | *Homo sapiens* |
| 225 | 2234-78 | DNA-binding protein isoform I | *D. melanogaster* |
| 226 | 2084-48 | Allantoinase . | *Rana catesbeiana* |
| 227 | 2085-22 | beta-glucuronidase | *E. coli* |
| 228 | 2094-24 | prolidase = peptidaseD/imidopeptidase | *Mus musculus* |
| 229 | 2088-43 | branched chain alpha-keto acid dehydrogenase E1-beta subunit | *Bos taurus* |
| 230 | 2086-29 | 3-hydroxyisobutyrate dehydrogenase | *Dictyostelium discoideum* |
| 231 | 2088-5 | Rab 5c protein | *Canis familiaris* |
| 232 | 2095-17 | cytochrome P-450 | *Heliothis virescens* |
| 233 | 2102-16 | carbamoyl phosphate synthetase II | *Plasmodium falciparans* |
| 234 | 2102-48 | NADPH cytochrome P450 reductase | *Musca domestica* |
| 235 | 2104-15 | branched chain alpha-keto acid dehydrogenase | *Rattus norvegicus* |
| 236 | 2106-5 | Metallothionein | *Strongylocentrotus purpuratus* |
| 237 | 2106-47 | peroxidoxin-1 | *Dirofilaria immitis* |
| 238 | 2107-17 | tetracycline transporter-like protein | *Mus musculus* |

(continued)

| SEQ ID NO: | Name: | GenBank Homology | *Organism* |
|---|---|---|---|
| 239 | 2107-58 | allergen Bla g 5 (glutathione-S-transferase) | *Blattella germanica* |
| 240 | 2156-58 | HAL-3 homologue | *Arabidopsis thaliana* |
| 241 | 2195-90 | aminoacyclase-1 | *Homo sapiens* |
| 242 | 2171-55 | NADPH-ferrihemoprotein reductase | *Drosophila melanogaster* |
| 243 | 2169-30 | hypothetical protein | *Synechocystis sp* |
| 244 | 2169-52 | insulin degrading enzyme | *Drosophila melanogaster* |
| 245 | 2177-64 | 3-hydroxyisobutyrate dehydrogenase | *Rattus norvegicus* |
| 246 | 2181-69 | Endonexin | *Bos taurus* |
| 247 | 2138-25 | glutamate dehydrogenase | *Drosophila melanogaster* |
| 248 | 2230-28 | glutathione -S-transferase | *Anopheles gambiae* |
| 249 | 2191-8 | lactase-phlorizin hydrolase | *Rattus rattus* |
| 250 | 2193-52 | cytochrome P450 | *Heloithis virescens* |
| 251 | 2202-35 | gtutathione-S-transferaso | *Anopheles gambiae* |
| 252 | 2229-77 | glutathione-S-transferase | *Anopheles gambiae* |
| 253 | 2229-81 | urate oxidase | *D. melanogaster* |
| 254 | 2231-42 | superoxide dismutase | *Cervus elaphus* |
| 255 | 2232-74 | allergen Bla g 5 | *Blattella germanica* |
| 256 | 2234-42 | glutathione reductase family | *Musca domestica* |
| 257 | 2087-8 | cystic fibrosis transmembrane conductance regulator | *Homo sapiens* |
| 258 | 2087-23 | Nervous system antigen 2 | *Drosophila melanogaster* |
| 259 | 2091-56 | adenosine triphosphatase | *Homo sapiens* |
| 260 | 2094-20 | sodium pump, alpha subunit | *Ctenocephalides felis* |
| 261 | 2095-51 | similar to Hrs | *C. elegans* |
| 262 | 2103-24 | N-methyl-D-aspartate receptor-associated protein | *Drosophila melanogaster* |
| 263 | 2105-55 | inward rectifying K channel | *Sus scrofa* |
| 264 | 2105-63 | EF-hand Ca2+ binding protein p22 | *Rattus norvegicua* |
| 265 | 2106-62 | Dents disease candidate gene product | *Homo sapiens* |
| 266 | 2167-50 | PKD1 (polycystic kidney disease 1) | *Fugu rubripes* |
| 267 | 2185-37 | copper-transporting ATPase | *Archaeoglobus fulgidus* |
| 268 | 2193-29 | TrkG Potassium transport protein | *E. coli* |
| 269 | 2195-33 | silicon transporter | *Cylindrotheca fusiformis* |
| 270 | 2202-16 | similarity to human sulfate anion transporter | *C. elegans* |
| 271 | 2230-2 | sulfate transporter | *Arabidopsis thaliana* |
| 272 | 2230-69 | mitochondrial porin | *D. melanogaster* |
| 273 | 2231-22 | muscarinic acetylcholine receptor | *D. metanogaster* |

(continued)

| SEQ ID NO: | Name: | GenBank Homology | *Organism* |
|---|---|---|---|
| 274 | 2231-24 | p97 subunit of 15S Mg(2+)- ATPase | *Xenopus laevis* |
| 275 | 2231-32 | anion transporting ATPase | *Aquifex aeolicus* |
| 276 | 2231-70 | sulfate permease | *Schizosaccharomyces pombe* |
| 277 | 2231-94 | putative Na/H exchanger | *S.pombe* |
| 278 | 2233-6 | plasma membrane Ca2+-ATPase 2 | *Mus musculus .* |
| 279 | 2233-24 | chloride channel gene, CLIC2 | *Homo sapiens* |
| 280 | 2085-61 | beta-type protein kinase C | *Bos taurus* |
| 281 | 2089-20 | cGMP-dependent protein kinase | *Drosophila melanogaster* |
| 282 | 2092-12 | Btk | *Homo sapiens* |
| 283 | 2093-64 | Receptor-like protein tyrosine phosphatase | *Drosophila melanogaster* |
| 284 | 2095-31 | frt (fms-related tyrosine kinase gene) | *Homo sapiens* |
| 285 | 2094-58 | casein kinase II beta | *Oryctolagus cuniculus* |
| 286 | 2103-54 | ORF YGL084c | *Saccharomyces cerevisiae* |
| 287 | 2106-42 | protein phosphatase epsilon subunit | *Homo sapiens* |
| 288 | 2156-5 | serine/threonine kinase | *Rattus norvegicus* |
| 289 | 2157-95 | cGMP-dependent protein kinase | *Drosophila melanogaster* |
| 290 | 2165-80 | ABL gene product | *Gallus gallus* |
| 291 | 2165-63 | diadenosine tetraphosphatase | *Homo sapiens* |
| 292 | 2167-17 | adenylate cyclase | *S. cereviciae* |
| 293 | 2177-44 | serine/threonine kinase | *C. elegans* |
| 294 | 2188-16 | weakly similar to serine/threonine kinase | *C. elegans* |
| 295 | 2191-60 | carbohydrate kinase, pfkB family | *Archaeoglobus fulgidus* |
| 296 | 2195-22 | protein kinase | *Drosophila melanogaster* |
| 297 | 2196-30 | calcium-dependent protein kinase | *A. thaliana* |
| 298 | 2205-83 | protein kinase/endoribonulcease (IAE1) | *Homo sapiens* |
| 299 | 2205-87 | receptor tyrosine phosphatase | *Hirudo medicinalis* |
| 300 | 2229-11 | magnesium-dependent calcium inhibitable phosphatase | *Bos taurus* |
| 301 | 2229-29 | phosphoglycerate kinase | *Schistosoma mansoni* |
| 302 | 2229-74 | pyruvate kinase | *D. melanogaster* |
| 303 | 2230-55 | serine/threonine specific protein phosphatase 4 | *D. melanogaster* |
| 304 | 2230-57 | stress activated MAP kinase kinase 3 | *D. melanogaster* |
| 305 | 2231-64 | alkaline phosphatase | *D. melanogaster* |
| 306 | 2231-91 | olynucleotide phosphorylase | *Yersinia enterocolitica* |
| 307 | 2232-43 | protein kinase PkwA | *Thermomonospora curvata* |
| 308 | 2234-94 | serine/threonine kinase ULK1 | *Homo sapiens* |

(continued)

| SEQ ID NO: | Name: | GenBank Homology | *Organism* |
|---|---|---|---|
| 309 | 2085-18 | Pyridoxamine phosphate oxidase | *C. elegans* |
| 310 | 2094-13 | sphingomyelin phosphodiesterase | *Mus musculus* |
| 311 | 2105-47 | apolipoprotein E receptor 2 | *Homo sapiens.* |
| 312 | 2092-38 | squalene synthetase | *Homo sapiens* |
| 313 | 2094-25 | fatty acid synthetase | *Rattus norvegicus* |
| 314 | 2089-32 | coproporphyrinogen oxidase | *Homo sapiens* |
| 315 | 2085-46 | HADHB mitochondrial trifunctional protein beta subunit | *Homo sapiens* |
| 316 | 2104-56 | pyridoxal kinase | *Homo sapiens* |
| 317 | 2107-30 | Phosphomevalonate kinase | *Homo sapiens* |
| 318 | 2154-70 | very-long chain acyl-CoA dehydrogenase | *Mus musculus* |
| 319 | 2191-85 | stearyl-CoA desaturase | *Cyprinus carpio* |
| 320 | 2192-44 | very-long-chain Acyl-CoA dehydrogenase | *Rattus norvegicus* |
| 321 | 2195-55 | Similar to LDL receptor-related protein | *C. elegans* |
| 322 | 2229-82 | lipase-3 | *D. melanogaster* |
| 323 | 2231-59 | Phosphatidylethanotamine-binding protein | *Macaca fascicularis* |
| 324 | 2233-25 | similarity to yeast ethanolaminephosphotransferase | *C. elegans* |
| 325 | 2233-41 | cellular retinoic acid binding protein (mCRABP) | *Manduca sexta* |
| 326 | 2087-61 | 1 allergen | *Lepidoglyphus destructor* |
| 327 | 2087-41 | chloroquine resistance candidate protein | *Plasmodium falciparum* |
| 328 | 2089-51 | Xenopus Bf B | *Xenopus laevis* |
| 329 | 2086-58 | repeat organellar protein | *Plasmodium falciparum* |
| 330 | 2090-45 | heat shock cognate protein | *Drosophila melanogaster* |
| 331 | 2104-23 | 40 kDa heat shock chaperone protein | *Deinococcus* |
| 332 | 2107-26 | Luciferase | *Photuris pennsylvanica* |
| 333 | 2162-46 | F20D1.9 | *C. elegans* |
| 334 | 2162-49 | PKR inhibitor P58 | *Bos taurus* |
| 335 | 2162-93 | GroES homologue | *Ricketsia* |
| 336 | 2171-46 | NH2 terminus uncertain | *Leishmania tarentolae* |
| 337 | 2089-10 | beta adaptin | *Drosophila melanogaster* |
| 338 | 2229-24 | non-functional folate binding protein | *Homo sapiens* |
| 339 | 2229-25 | calmodulin B | *Halocynthia roretzi* |
| 340 | 2229-31 | putative T1/ST2receptor binding protein | *C. elegans* |
| 341 | 2229-36 | alpha-crystallin cognate protein 25 - | *Plodia interpunctella* |

(continued)

| SEQ ID NO: | Name: | GenBank Homology | *Organism* |
|---|---|---|---|
| 342 | 2229-40 | Defensin | *Apis mellifera* |
| 343 | 2229-86 | glutamate-ammonia ligase | *D. melanogaster* |
| 344 | 2231-49 | melanoma-associated antigen ME491 | *Homo sapiens* |
| 345 | 2231-76 | histone C | *Drosophila virilis* |
| 34G, | 2232-65 | translationally controlled tumor protein | *Oryctolagus cuniculus* |
| 347 | 2232-84 | Apyrase . | *Aedes aegypti* |
| 348 | 2232-85 | KIAA0124 | *Homo sapiens* |
| 349 | 2233-59 | Glutamine-dependent carbamoyl-phosphate synthase | *C. elegans* |
| 350 | 2233-86 | ANG12 precursor | *Anopheles gambiae* |
| 351 | 2234-11 | tissue specific secretory protein | *Pan troglodytes* |
| 352 | 2234-76 | methionine adenosyltransferase | *D. melanogaster* |
| 353 | 2089-13 | Synaptic vessicle protein 2 form B | *Rattus norvegicus* |
| 354 | 2159-52 | glycoprotein 56 | *Rattus norvegicus* |
| 355 | 2084-6 | CLN3; homologue of the gene underlying Batten disease | *Mus musculus* |
| 356 | 2085-10 | Amphiphysin | *Gallus gallus* |
| 357 | 2156-39 | glycoprotein 55 | *Rattus norvegicus* |
| 358 | 2104-59 | Transmembrane transporter | *Discopyge ommata* |
| 359 | 2105-9 | insect intestinal mucin II | *Trichoplusia ni* |
| 360 | 2106-14 | kinesin-like protein | *D. melanogaster* |
| 361 | 2107-45 | Lazarillo precursor | *Schistocerca americana* |
| 362 | 2156-3 | clathrin-associated protein | *Mus musculus* |
| 363 | 2161-46 | neural variant mena+ protein | *Mus musculus* |
| 364 | 2171-92 | Malvolio | *Drosophila melanogaster* |
| 365 | 2175-18 | homolog of SYT - synaptotagmin | *Mus musculus* |
| 366 | 2177-10 GABA | receptor subunit (Rdl) | *Aedes aegypti* |
| 367 | 2181-10 | neurexin IV | *Drosophila melanogaster* |
| 368 | 2191-92 | synaptic vessicle protein 2B | *Rattus norvegicus* |
| 369 | 2229-18 | Synaptic vessicle protein 2A | *Rattus norvegicus* |
| 370 | 2194-38 | gamma-sub.unit of mouse nerve growth factor | *Mus musculus* |
| 371 | 2230-60 | lin-7-C | *Rattus norvegicus* |
| 372 | 2230-81 | PDZ domain protein | *Homo sapiens* |
| 373 | 2234-5 | Gcap1 gene product | *Mus musculus* |
| 374 | 2234-55 | Gcap1 gene product | *Mus musculus* |
| 375 | 2234-71 | Gcap1 gene product | *Mus musculus* |
| 376 | 2085-34 | Liver-specific transport protein | *Rattus norvegicus* |
| 377 | 2087-15 | polyspecific organic cation transporter | *Homo sapiens* |

(continued)

| SEQ ID NO: | Name: | GenBank Homology | *Organism* |
|---|---|---|---|
| 378 | 2204-80 | transmembrane transporter | *Discopyge ommatta* |
| 379 | 2093-39 | liver-specific transport protein | *Rattus norvegicus* |
| 380 | 2093-46 | similar to monocarboxylate transporter family | *C. elegans* |
| 381 | 2092-22 | similar to matrin F/G | *C. elegans* |
| 382 | 2103-50 | Unknown | *Drosophila melanogaster* |
| 383 | 2103-51 | organic cation transporter | *Ratttus norvegicus* |
| 384 | 2197-35 | renal organic cation transporter | *Oryctolagus cuniculus* |
| 385 | 2156-17 | sulfate anion transporter | *Manduca sexta* |
| 386 | 2166-84 | LX1 | *Mus musculus* |
| 387 | 2167-94 | MCT (monocarboxylate transporter) | *Homo sapiens* |
| 388 | 2196-83 | renal organic cation transporter | *Oryctolagus cuniculus* |
| 389 | 2229-83 | similarity to monocarboxylate transporter 1 | *C. elegans* |
| 390 | 2231-89 | Golgi 4-transmembrane spanning transporter MTP | *Mus musculus* |
| 391 | 2158-8 | phosphate carrier protein | *C. elegans* |
| 392 | 2085-14 | ADP/ATP translocase | *Drosophila melanogaster* |
| 393 | 2085-17 | Na+-dependent inorganic phosphatase cotransporter | *Drosophila melanogaster* |
| 394 | 2088-38 | ADP/ATP translocase | *Bos taurus* |
| 395 | 2092-50 | ADP/ATP translocase | *Drosophila melanogaster* |
| 396 | 2104-21 | Na(+)-dependent inorganic phosphate cotransporter | *Drosophila melanogaster* |
| 397 | 2121-55 | phosphate carrier protein | *C. elegans* |
| 398 | 2105-64 | phosphate carrier protein | *Homo sapiens* |
| 399 | 2102-6 | ZK512.6 | *C. elegans* |
| 400 | 2108-27 | mitochondrial phosphate carrier protein | *Homo sapiens* |
| 401 | 2194-63 | mitochondrial phosphate transporter | *Rattus norvegicus* |
| 402 | 2196-14 | phosphateltriose-phosphate translocator precursor | *C. elegans* |
| 403 | 2204-11 | EST clone | *D. melanogaster* |
| 404 | 2085-16 | Chymotrypsin I | *Anopheles gambiae* |
| 405 | 2085-54 | Chymotrypsin II | *Anopheles gambiae* |
| 406 | 2086-12 | Plasminogen | *Homo sapiens* |
| 407 | 2086-18 | Trypsin eta | *Drosophila melanogaster* |
| 408 | 2090-21 | Trypsin | *Manduca sexta* |
| 409 | 2092-15 | Alp1 | *Cochliobolus carbonum* |
| 410 | 2102-11 | vitellin-degrading protease | *Bombyx mori* |
| 411 | 2102-17 | Chymotrypsin II | *Anopheles gambiae* |

(continued)

| SEQ ID NO: | Name: | GenBank Homology | *Organism* |
|---|---|---|---|
| 412 | 2102-51 | chymotrypsin -like protease | *Anopheles gambiae* |
| 413 | 2103-31 | Beta trypsin | *Drosophila erecta* |
| 414 | 2107-22 | Factor IX | *Rattus norvegicus* |
| 415 | 2108-29 | Trypsin | *Anopheles stephensi* |
| 416 | 2157-15 | Trypsin | *Choristoneura fumiferana* |
| 417 | 2160-34 | Aminopeptidase | *Synechocystis* |
| 418 | 2160-36 | E01 G6.1 | *C. elegans* |
| 419 | 2103-62 | plasminogen activator inhibitor 2 | *Mus musculus* |
| 420 | 2167-36 | factor IX | *Oryctolagus cuniculus* |
| 421 | 2167-67 | Alp1 | *Cochliobolus carbonum* |
| 422 | 2169-51 | Trypsin | *Aedes aegypti* |
| 423 | 2181-27 | Chymotrypsin BII | *Penaeus vannamei* |
| 424 | 2185-69 | plasma prekallikrein | *Homo sapeins* |
| 425 | 2187-20 | pre-procathepsin L | *Paragonimus westermani* |
| 426 | 2188-45 | vitellin-degrading protease | *Bombyx mori* |
| 427 | 2192-91 | late trypsin precourser | *Culex pipiens quinquefasciatus* |
| 428 | 2196-10 | SPC2 | *Branchiostoma califomiensis* |
| 429 | 2196-88 | Trypsin | *Anopheles stephensi* |
| 430 | 2204-9 | camitine/choline acetyltransferase | *C. elegans* |
| 431 | 2229-7 | iota trypsin | *D. melanogaster* |
| 432 | 2229-22 | Trypsin | *Anopheles gambiae* |
| 433 | 2229-89 | Trypsin | *Anopheles gambiae* |
| 434 | 2229-94 | late trypsin precourser | *Culex pipiens quinquefasciatus* |
| 435 | 2230-59 | Chymotrypsin 1 | *Anopheles gambiae* |
| 436 | 2230-67 | carboxypeptidase A | *Drosophila heteroneura* |
| 437 | 2231-62 | aminopeptidase N | *Sus scxrofa* |
| 438 | 2231-74 | limulus factor C serine protease | *Tachypleus tridentatus* |
| 439 | 2232-15 | cysteine proteinase | *Sitophilus zeamais* |
| 440 | 2232-25 | Carboxypeptidase - | *Simulium vitatum* |
| 441 | 2232-33 | putative aspartic protease | *Brassica oleracea* |
| 442 | 2233-46 | aminopeptidase N | *Pleuronectes americanus* |
| 443 | 2233-85 | chymotrypsin 1 | *Anopheles gambiae* |
| 444 | 2233-90 | Trypsin | *Anopheles stephensi* |
| 445 | 2233-94 | preprechymotrypsin 1 | *Penaeus vannamei* |
| 446 | 2234-29 | chymotrypsin-like protease precursor | *Aedes aegypti* |
| 447 | 2234-58 | Putative | *C. elegans* |
| 448 | 2234-61 | carboxyl esterase precursor | *Aphis gossypii* |
| 449 | 2234-68 | serine protease inhibitor I | *Schistocerca gregaria* |

(continued)

| SEQ ID NO: | Name: | GenBank Homology | *Organism* |
|---|---|---|---|
| 450 | 2084-35 | Integral membrane protein | *Mus musculus* |
| 451 | 2086-45 | similar to beta-ureidopropionase of Rat | *C. elegans* |
| 452 | 2087-54 | Cyclin | *Mus musculus* |
| 453 | 2088-22 | Esp 8 | *Mus musculus* |
| 454 | 2091-16 | contains similarity to EGF-like domains | *C. elegans* |
| 455 | 2091-29 | multiple exostosis-like protein | *Homo sapiens* |
| 456 | 2091-30 | apoptosis 1 inhibitor | *Drosophila melanogaster* |
| 457 | 2092-33 | KIAA0023 (putitive oncogene) | *Homo sapiens* |
| 458 | 2095-35 | G coupled receptor | *C. elegans* |
| 459 | 2095-3 | Go (heterotrimeric guanyl nucleotide binding protein alpha subunit) | *Manduca sexta* |
| 460 | 2085-4 | gp 150 protein | *Drosophila melanogaster* |
| 461 | 2103-28 | leukotriene A4 hydrolase | *Rattus sp.* |
| 462 | 2105-62 | putitive orf | *Homo sapiens* |
| 463 | 2107-6 | activator protein | *Drosophila melanogaster* |
| 464 | 2107-28 | platelet-endothelial tetraspan antigen 3 | *Homo sapiens* |
| 465 | 2189-3 | oligopeptidase A (prlC) | *Haemopholis influenzea* |
| 466 | 2156-54 | fibroblast growth factor receptor | *Xenopus laevis* |
| 467 | 2160-92 | contains similarity to EGF-like domains | *C. elegans* . |
| 468 | 2160-65 | weak similarity to the drosophila hyperplastic disc protein | *C. elegans* |
| 469 | 2165-53 | inositol triphosphate receptor | *Rattus norvegicus* |
| 470 | 2166-22 | placental protein 11 | *Homo sapiens* |
| 471 | 2166-92 | elongation factor 1 alpha-like | *Drosophila melanogaster* |
| 472 | 2181-34 | DSch | *Drosophila melanogaster* |
| 473 | 2192-65 | STAM, signal transducing adaptor molecule | *Homo sapiens* |
| 474 | 2194-24 | ATPases associated with various cellular activities (AAA family) | *Arabidopsis thaliana* |
| 475 | 2196-75 | similar to cell division control protein | *C. elegans* |
| 476 | 2230-38 | EST clone | *S. cereviciae* |
| 477 | 2230-39 | NTPase | *D. melanogaster* |
| 478 | 2230-66 | adenylyl cyclase aggregation protein | *Dictyostelium discoideum* |
| 479 | 22230-80 | sphingomyelin phosphodiesterase | *C. elegans* |
| 480 | 2231-29 | nuclear antigen H731 | *Homo sapiens* |
| 481 | 2231-40 | suppressor of actin mutation 2 | *Homo sapiens* |
| 482 | 2231-66 | DET1 | *Arabidopsis thaliana* |
| 483 | 2232-7 | Calreticulin | *D. melanogaster* |
| 484 | 2232-38 | activator protein | *D. melanogaster* |

(continued)

| SEQ ID NO: | Name: | GenBank Homology | *Organism* |
|---|---|---|---|
| 485 | 2232-69 | ornithine decarboxylase | *Gallus gallus* |
| 486 | 2233-32 | similar bHLH-PAS | *D. melanogaster* |
| 487 | 2233-45 | rab1 | *D. melanogaster* |
| 488 | 2234-2 | C10A gene product | *Mus musculus* |
| 489 | 2234-72 | QM homolog | *D. melanogaster* |
| 490 | 2084-17 | Integral membrane protein | *Herpesvirus-2* |
| 491 | 2091-4 | endomembrane protien EMP70 precourser isolog | *Arabidopsis thaliana* |
| 492 | 2102-45 | Ylr251wp | *Saccharomyces cerevisiae* |
| 493 | 2162-68 | 220 kDa silk protein | *Chironomus thummi* |
| 494 | 2160-47 | precursor HT7 protein | *Gallus gallus* |
| 495 | 2161-12 | peritrophin 95 precourser | *Lucilia cuprina* |
| 496 | 2161-15 | yk86g11.5 | *C. elegans* |
| 497 | 2171-12 | 51 A surface protein | *Paramecium tetraurelia* |
| 498 | 2173-18 | hypothetical - mitochondrial membrane transport protein | *Schizosaccharomyces pombe* |
| 499 | 2087-32 | est sequence | *C. elegans* |
| 500 | 2091-19 | Similar to P. aeruginosa hypothetical protein | *C. elegans* |
| 501 | 2192-86 | tyrosine kinase | *Drosophila melangaster* |
| 502 | 2086-42 | M04B2.4 | *C. elegans* |
| 503 | 2088-16 | glycoprotein 330 | *C.* elegans/Human |
| 504 | 2088-39 | EST sequence | *Arabidopsis thaliana* |
| 505 | 2088-57 | Yer 126cp | *Saccharomyces cereviciae* |
| 506 | 2089-25 | similar to S. cereviciae hypothetical protein YKL166 | *C. elegans* |
| 507 | 2090-3 | EST sequence | *Saccharomyces cereviciae* |
| 508 | 2090-53 | EST sequence | *C. elegans* |
| 509 | 2095-20 | Chloroplast ORF | *Marchantia polymorpha* |
| 510 | 2102-28 | similar to S. cerevisiae hypothetical protein YKL166 | *C. elegans* |
| 511 | 2102-55 | D1054.3 | *C. elegans* |
| 512 | 2102-58 | ZC513.5 gene product | *C. elegans* |
| 513 | 2105-44 | E 1087 protein | *Saccharomyces cerevisiae* |
| 514 | 2109-24 | F11C1.5 | *C. elegans* |
| 515 | 2154-21 | disulfide-like protein | *Acanthamoeba castellanii* |
| 516 | 2156-6 | ZK470.1 | *C. elegans* |
| 517 | 2156-18 | BIIIA3 | *Ovis aries* |
| 518 | 2156-27 | AFR1 | *S. cereviciae* |

(continued)

| SEQ ID NO: | Name: | GenBank Homology | *Organism* |
|---|---|---|---|
| 519 | 2165-94 | COS41.8 | *Ciona intestinalis* |
| 520 | 2167-65 | EST sequence, function unknown | *C. elegans* |
| 521 | 2171-93 | -<IAA0160 | *Homo sapiens* |
| 522 | 2175-45 | ORF YJR83.18 | *S. cereviciae* |
| 523 | 2185-66 | rps4 | *Plasmodium falciparum* |
| 524 | 2195-40 | C27C12.4 | *C. elegans* |
| 525 | 2196-20 | glycoprotein A | *Pneumocystis carinii* |
| 526 | 2205-89 | KRF1 encodes EBNA-1 protein | *Epstein Barr virus* |
| 527 | 2229-19 | D4L | *Variola virus* |
| 528 | 2230-35 | KIAA0747 | *Homo sapiens* |
| 529 | 2231-8 | 3 | *Mus musculus* |
| 530 | 2231-78 | unknown protein | *Arabidopsis thaliana* |
| 531 | 2232-49 | Similarity to Yeast hypothetical 52.9 KD protein | *C. elegans* |
| 532 | 2232-52 | tetratricopeptide repeat protein (tpr2) | *Homo sapiens* |
| 533 | 2233-5 | similar to Saccharomyces cerevisiae SCD6 protein | *C. elegans* |
| 534 | 2233-22 | cDNA EST yk486b9.3 | *C. elegans* |
| 535 | 2233-93 | CDC27Dm | *D. melanogaster* |
| 536 | 2084-34 | Immune suppressorN-ATPase 115 kDa subunit | *Mus musculus* |
| 537 | 2086-30 | V-ATPase A-subunit | *Aedes aegypti* |
| 538 | 2087-45 | H+ ATPase | *Drosophila melanogaster* |
| 539 | 2088-55 | 40-kDa-V-ATPase subunit | *Manduca sexta* |
| 540 | 2088-62 | vacuolar ATPase subunit A | *Drosophila melanogaster* |
| 541 | 2091-26 | proton-A TPase-like protein | *Homo sapiens* |
| 542 | 2091-31 | vacuolar ATPase subunit A | *Drosophila melanogaster* |
| 543 | 2092-20 | vacuolar ATPase 115 kDa subunit | *Homo sapiens* |
| 544 | 2095-18 | similar to S. cereviciae vacuolar H (+)-ATPase 54 kD subunit | *C. elegans* |
| 545 | 2095-54 | H (+)-transporting ATPase subunit B | *Manduca sexta* |
| 546 | 2108-8 | similar to *S. cereviciae* 54 kDa V-ATPase subunit | *C. elegans* |
| 547 | 2154-36 | V-ATPase subunit E | *Drosophila melanogaster* |
| 548 | 2154-76 | V-ATPase subunit A (new fragment) | *Aedes aegypti* |
| 549 | 2166-32 | V-ATPase C subunit it | *Drosophila melanogaster* |
| 550 | 2166-33 | vacuolar (V-type) H(+)-ATPase B subunit | *Helicoveipa virescens* |
| 551 | 2166-90 | beta subunit of ATPase | *Schizaphis graminum* |
| 552 | 2161-5 | ATPase I | *Plasmodium falciparum* |

(continued)

| SEQ ID NO: | Name: | GenBank Homology | Organism |
|---|---|---|---|
| 553 | 2171-24 | similar to V-ATPase 116kd subunit | *C. elegans* |
| 554 | 2169-82 | V-ATPase subunit E | *Drosophila melanogaster* |
| 555 | 2187-36 | V-ATPase membrane sector associated protein M8-9 | *Homo sapiens* |
| 556 | 2188-91 | V-ATPase subunit A | *Candida tropicalis* |
| 557 | 2230-88 | vacuolar ATPase G subunit | *Manduca sexta* |
| 558 | 2232-61 | rf-ATPase subunit C | *D. melanogaster* |
| 559 | 2086-52 | Penelope transposable element ORF | *Drosophila virilis* |
| 560 | 2103-2 | genome polyprotein gene product | *Plum pox virus* |
| 561 | 2106-8 | pol protein | *Human T-cell lymphotropic virus type 2* |
| 562 | 2108-41 | reverse transcriptase, Doc retroposon | *Drosophila melanogaster* |
| 563 | 2202-28 | Polyprotein | *Hepatitis virus C* |
| 564 | 2165-95 | DNA polymerase | *Choristoneura biennis entomopoxvirus* |
| 565 | 2169-81 | reverse transcriptase | *Drosophila melanogaster* |
| 566 | 2181-36 | reverse transcriptase | *Anopheles gambiae* |
| 1416 | 2240-4 | alpha-L-tucosidase precursor | *Homo sapiens* |
| 1417 | 2240-11 | estrogen related receptor alpha | *Mus musculus* |
| 1418 | 2240-14 | NADH:ubiquinone oxidoreductase 51-kD subunit | *Homo sapiens* |
| 1419 | 2240-17 | peritrophin 1 | *Anopheles gambiae* |
| 1420 | 2240-19 | small GTPase rac1b | *Homo sapiens* |
| 1421 | 2240-23 | Symplekin | *Homo sapiens* |
| 1422 | 2240-26 | ribosomal protein L30 | *Bos taurus* |
| 1423 | 2240-28 | fi0S Ribosomal Protein RPL10A | *Homo sapiens* |
| 1424 | 2240-29 | KIN17 protein | *D. melanogaster* |
| 1425 | 2240-31 | eukaryotic initiation factor 4 gamma | *Homo sapiens* |
| 1426 | 2240-38 | ornithine decarboxylase antizyme | *D. melanogaster* |
| 1427 | 2240-44 | electron transfer flavoprotein | *Rattus norvegicus .* |
| 1428 | 2240-53 | EST clone | *C. elegans* |
| 1429 | 2240-55 | glutathione reductase family | *Musca domestica* |
| 1430 | 2240-58 | chymotrypsin-like serine protease | *C. felis* |
| 1431 | 2240-63 | ferritin subunit 1 | *D. melanogaster* |
| 1432 | 2240-64 | vacuolar ATPase subunit B | *D. melanogaster* |
| 1433 | 2240-66 | chaperonin containing TCP-1 delta | *Fugu rubripes* |
| 1434 | 2240-70 | 1-acyl-glycerol-3-phosphate acyltransferase | *Zea mays* |
| 1435 | 2240-71 | EST clone AL021106 | *D. melanogaster* |
| 1436 | 2240-72 | 376aa long hypothetical dehydrogenase | *Pyrococcus horikoshii* |

(continued)

| SEQ ID NO: | Name: | GenBank Homology | Organism |
|---|---|---|---|
| 1437 | 2240-77 | chymotrypsin-like serine protease | *C. felis* |
| 1438 | 2240-80 | EST clone | *C. elegans* |
| 1439 | 2240-83 | chymotrypsin-like serine protease | *C. felis* |
| 1440 | 2240-90 | cytochrome P450 | *D. melanogaster* |
| 1441 | 2240-93 | enhancer-trap-locus-1 | *Mus musculus* |
| 1442 | 2240-94 | glycerol-3-phosphate dehydrogenase | *Ceratitis capitata* |
| | | | |
| 1443 | 2241-3 | FS-H precourser | *Ctenocephalides felis* |
| 1444 | 2241-5 | trypsin-like serine protease | *Ctenocephalides felis* |
| 1445 | 2241-7 | myospheroid protein | *D. melanogaster* |
| 1446 | 2241-10 | Sam50 | *D. melanogaster* |
| 1447 | 2241-12 | NADH dehydrogenase subunit 2 | *Chorthippus parallelus* |
| 1448 | 2241-15 | putative protein | *Arabidopsis thaliana* |
| 1449 | 2241-16 | contains EGF-like repeats | *C. elegans* |
| 1450 | 2241-20 | Gcap1 gene product | *Mus musculus* |
| 1451 | 2241-25 | Na(+)-dependent inorganic phosphate cotransporter | *D. melanogaster* |
| 1452 | 2241-31 | D4L | *Variola virus* |
| 1453 | 2241-36 | plenty-of-prolines-101; POP101; SH3-philo-protein | *Mus musculus* |
| 1454 | 2241-40 | EF-1-alpha | *D. melanogaster* |
| 1455 | 2241-44 | F1-ATP synthase epsilon-subunit | *Ipomoea batatas* |
| 1456 | 2241-54 | ribosomal protein S28 | *Homo sapiens* |
| 1457 | 2241-55 | Y-box protein | *D. melanogaster* |
| 1458 | 2241-56 | short-chain alcohol dehydrogenase | *Homo sapiens* |
| 1459 | 2241-59 | contains 3 cysteine rich repeats | *C. elegans* |
| 1460 | 2241-60 | muscle type phosphofructokinase | *Canis familiaris* |
| 1461 | 2241-61 | Heat shock protein 82 | *Mus musculus* |
| 1462 | 2241-65 | chymotrypsin-like protease | *C. felis* |
| 1463 | 2241-66 | Oligosaccharyltransferase subunit | *D. melanogaster* |
| 1464 | 2241-70 | EST clone | *D. melanogaster* |
| 1465 | 2241-72 | failed axon connections protein | *D. melanogaster* |
| 1466 | 2241-74 | Enolase | *Hymenolepis diminuta* |
| 1467 | 2241-78 | multiple exostosis 2 protein | *Mus musculus* |
| 1468 | 2241-80 | Protein on Ecdysone Puffs | *D. melanogaster* |
| 1469 | 2241-82 | paramyosin | *D. melanogaster* |
| 1470 | 2241-83 | beta-tubulin | *Bombyx mori* |
| 1471 | 2241-84 | natural killer cell enhancing factor | *Cyprinus carpio* |

(continued)

| SEQ ID NO: | Name: | GenBank Homology | *Organism* |
|---|---|---|---|
| 1472 | 2241-86 | similar to MYOTUBULARIN-RELATED PROTEIN | *Homo sapiens* |
| 1473 | 2241-87 | Renin | *Rattus norvegicus* |
| 1474 | 2241-90 | Myophilin | *Echinococcus multilocularis* |
| | | | |
| 1475 | 2243-10 | alpha-actinin | *D. melanogater* |
| 1476 | 2243-11 | monocarboxylate transporter | *Homo sapiens* |
| 1477 | 2243-13 | yk278a10.3 | *C. elegans* |
| 1478 | 2243-15 | selenium donor protein | *Homo sapiens* |
| 1479 | 2243-18 | acetyl-CoA synthetase | *D. melanogater* |
| 1480 | 2243-20 | cytochrome P450 CYP12A3 | *Musca domestica* |
| 1481 | 2243-22 | NADH dehydrogenase subunit 4 | *Anopheles arabiensis* . |
| 1482 | 2243-27 | Polyubiquitin | *Cricetulus griseus* |
| 1483 | 2243-28 | Moesin | *D. melanogater* |
| 1484 | 2243-31 | QM protein | *Bombyx mandarina* |
| 1485 | 2243-32 | Sec23 protein | *Homo sapiens* |
| 1486 | 2243-37 | truncated protein | *S. cereviciae* |
| 1487 | 2243-38 | Projectin | *D. melanogater* |
| 1488 | 2243-39 | Unknown | *Homo sapiens* |
| 1489 | 2243-41 | similar to enoyl-CoA hydratase | *C. elegans* |
| 1490 | 2243-45 | similar to dehydrogenase | *C. elegans* |
| 1491 | 2243-46 | trypsin-like serine protease | *C. felis* |
| 1492 | 2243-48 | Merlin | *Rattus norvegicus* |
| 1493 | 2243-52 | GTP-specific suxinyl-CoA synthetase beta subunit | *Homo sapiens* |
| 1494 | 2243-53 | sod protein (superoxide dismutase) | *Drosophila virilis* |
| 1495 | 2243-54 | trypsin-like serine protease | *C. felis* |
| 1496 | 2243-61 | chymotrypsin-like serine protease | *C. felis* |
| 1497 | 2243-66 | Tag B | *Dictyostelium discoideum* |
| 1498 | 2243-67 | hypothetical protien | *Arabidopsis thaliana* |
| 1499 | 2243-68 | heat shock cognate protein 70 | *Trichoplusia ni* |
| 1500 | 2243-72 | TRIP-1 homologue | *D. melanogater* |
| 1501 | 2243-73 | cytosolic NADP-dependent isocitrate dehydrogenase | *Microtis mexicanis* |
| 1502 | 2243-86 | progesterone-induced protein | *Oryctolagus cuniculus* |
| 1503 | 2243-87 | Bmsqd-2 | *Bombyx mori* |
| 1504 | 2243-91 | sodiumfiodide symporter | *Homo sapiens* |
| 1505 | 2243-92 | ORF2 | *Acidianus ambivalens* |
| 1506 | 2243-94 | lysosomal beta-galactosidase | *Felis cattus* |

(continued)

| SEQ ID NO: | Name: | GenBank Homology | *Organism* |
|---|---|---|---|
| 1507 | 2244-12 | tropomyosin isoform 127 | *D. melanogaster* |
| 1508 | 2244-19 | KIAA0181 | *Homo sapiens* |
| 1509 | 2244-23 | plasma membrane calcium ATPase isoform 1 | *Homo sapiens* |
| 1510 | 2244-29 | NADH dehydrogenase | *Bos taurus* |
| 1511 | 2244-44 | glutamate dehydrogenase | *D. melanogaster* |
| 1512 | 2244-54 | spliceosomal protein | *D. melanogaster* |
| 1513 | 2244-59 | ciliary body glutathione peroxidase | *Bos taurus* |
| 1514 | 2244-61 | pyridoxal-phoshate-dependent aminotransferases | *C. elegans* |
| 1515 | 2244-64 | Unknown | *Rattus norvegicus* |
| 1516 | 2244-69 | trypsin-like serine protease | *C. felis* |
| 1517 | 2244-71 | peritrophin 1 | *Anopheles gambiae* |
| 1518 | 2244-75 | NADH dehydrogenase subunit 5 | *Anopheles gambiae* |
| 1519 | 2244-84 | microsomal epoxide hydrolase | *Rattus norvegicus* |
| 1520 | 2244-86 | C54G7.2 gene product | *C. elegans* |
| 1521 | 2244-91 | Aminopeptidase N | *Plutella xylostella* |
| 1522 | 2253-2 | cytochrome C oxidase | *H. sapiens* |
| 1523 | 2253-13 | Initiation factor 5A | *Gallus gallus* |
| 1524 | 2253-14 | protein phosphatase type 2A catalytic subunit | *Bos taurus* |
| 1525 | 2253-16 | myosin light chain 2 | *D. melanogaster* |
| 1526 | 2253-18 | cDNA EST yk462d1.5 | *C. elegans* |
| 1527 | 2253-19 | ribosomal protein S10 | *H. sapiens* |
| 1528 | 2253-24 | aspartyl(asparaginyl)beta-hydroxylase, HAAH | *H. sapiens* |
| 1529 | 2253-27 | larval and adult myosin heavy chain | *D. melanogaster* |
| 1530 | 2253-33 | nervous system antigen 2 | *D. melanogaster* |
| 1531 | 2253-36 | dJ366N23.2 | *H. sapiens* |
| 1532 | 2253-40 | hrp48.1 | *D. melanogaster* |
| 1533 | 2253-42 | ZnT-1 | *Mus musculus* |
| 1534 | 2253-43 | aminopeptidase N | *Manduca sexta* |
|  | 1535.2253-56 | Profilin | *D. melanogaster* |
| 1536 | 2253-59 | T26A5 *H.* | *sapiens* |
| 1537 | 2253-68 | NADH-ubiquinone oxidoreductase 42 kDa subunit | *D. melanogaster* |
| 1538 | 2253-78 | glycine-rich protein |  |
| 1539 | 2253-81 | 5'-nucleotidase | *H. sapiens* |
| 1540 | 2253-86 | glutathione S-transferase | *Anopheles gambiae* |

(continued)

| SEQ ID NO: | Name: | GenBank Homology | *Organism* |
|---|---|---|---|
| 1541 | 2253-87 | ferritin subunit 1 | *D. melanogaster* |
| 1542 | 2253-92 | myosin light chain 2 | *D. melanogaster* |
| 1543 | 2253-94 | xylose-proton symport | *E. coli* |
| 1544 | 2254-4 | mature-parasite-infected erythrocyte surface antigen | *P. falciparum* |
| 1545 | 2254-6 | Fo-ATP synthase subunit b | *D. melanogaster* |
| 1546 | 2254-13 | similar to Arabidopsis thaliana male sterility protein 2 | *C. elegans* |
| 1547 | 2254-17 | CLN3 protein | *H. sapiens* |
| 1548 | 2254-21 | YbgG | *B. subtilis* |
| 1549 | 2254-25 | peroxisomal protein | *Synechocystis sp* |
| 1550 | 2254-27 | Glutaminase | *Rattus norvegicus* |
| 1551 | 2254-30 | tartan protein | *D. melanogaster* |
| 1552 | 2254-33 | leucine zipper-EF-hand containing transmembrane protein 1 | *H. sapiens* |
| 1553 | 2254-39 | similar to helicase | *C. elegans* |
| 1554 | 2254-43 | muscle myosin heavy chain | *D. melanogaster* |
| 1555 | 2254-45 | putative nicotinate phosphoribosyltransferase | *N. tabacum* |
| 1556 | 2254-51 | 60S ribosomal protein | *Mus musculus* |
| 1557 | 2254-54 | small nuclear riboprotein Sm-D | *H. sapiens* |
| 1558 | 2254-55 | nucleoside diphosphate kinase | *Salmo salar* |
| 1559 | 2254-60 | serine protease | *C. felis* |
| 1560 | 2254-63 | myospheroid protein | *D. melanogaster* |
| 1561 | 2254-65 | Carboxylesterase | *Anisopteromalus calandrae* |
| 1562 | 2254-66 | siah binding protein 1 | *H. sapiens* |
| 1563 | 2254-70 | vacuolar ATPase, subunit M9.7 | *Manduca sexta* |
| 1564 | 2254-83 | Fumarylacetoacetate hydrolase | *Rattus norvegicus* |
| 1565 | 2254-84 | metalloproteinase 1 | *Hydra vulgaris* |
| 1566 | 2254-88 | alpha-spectrin | *D. melanogaster* |
| 1567 | 2254-93 | NADH dehydrogenase subunit, 6 | *D. melanogaster* |
| 1568 | 2254-96 | cyclophilin isoform 5 | *C. elegans* |
| 1569 | 2255-5 | similar to mitochondrial ATPase inhibitors | *C. elegans* |
| 1570 | 2255-8 | yk391f12.5 | *C. elegans* |
| 1571 | 2255-12 | Unknown | *H. sapiens* |
| 1572 | 2255-17 | ribonucleotide reductase subunit M1 | M. *musculus* |
| 1573 | 2255-19 | docking protein | *H. sapiens* |
| 1574 | 2255-22 | Similar to rat 5E5 antigen | *H. sapiens* |

(continued)

| SEQ ID NO: | Name: | GenBank Homology | *Organism* |
|---|---|---|---|
| 1575 | 2255-23 | ribosomal protein S31 | *D., melanogaster* |
| 1576 | 2255-25 | Similar to acyl-CoA dehydrogenase | *C. elegans* |
| 1577 | 2255-28 | Arginine tyrosine kinase | *H. sapiens* |
| 1578 | 2255-32 | ribosomal protein L7a | *D., melanogaster* |
| 1579 | 2255-33 | chS-Rex-s | *G. gallus* |
| 1580 | 2255-39 | Phosphoacetylglucosamine mutase | *C. elegans* |
| 1581 | 2255-41 | NADH dehydrogenase subunit 6 | *D., melanogaster* |
| 1582 | 2255-45 | tRNA-glutamine synthetase | *C. elegans* |
| 1583 | 2255-46 | p68 | *M. musculus* |
| 1584 | 2255-49 | ABC8 | *M. musculus* |
| 1585 | 2255-50 | kynurenine aminotransferase | *R. rattus* |
| 1586 | 2255-51 | SmD homolog {Gly-Arg repeat} | *M. musculus* |
| 1587 | 2255-56 | epoxide hydrolase | *S. scrofa* |
| 1588 | 2255-60 | Sec23 protein | *H. sapiens* |
| 1589 | 2255-62 | HMG CoA synthase | *M. musculus* |
| 1590 | 2255-63 | dipeptidyl aminopeptidase-like protein 6 | *M. musculus* |
| 1591 | 2255-66 | retinal rod Na+/Ca+, K+ exchanger | *H. sapiens* |
| 1592 | 2255-67 | 4-hydroxybutyrate coenzyme A transferase | *C. elegans* |
| 1593 | 2255-70 | hD54+ins2 isoform | *H. sapiens* |
| 1594 | 2255-73 | chromaffin granule ATPase II homolog | *M. musculus* |
| 1595 | 2255-77 | 40S ribosomal protein S10 | *H. sapiens* |
| 1596 | 2255-79 | 34/67 kD laminin binding protein | *S. purpuratus* |
| 1597 | 2255-82 | RNA-binding protein lark | *D., melanogaster* |
| 1598 | 2255-86 | thiol-specific antioxidant protein | *R. norvegicus* |
| 1599 | 2256-7 | Similar to Human estrogen-responsive finger protein | *H. sapiens* |
| 1600 | 2256-11 | Trypsin | *C. felis* |
| 1601 | 2256-12 | CEV14 | *H. sapiens* |
| 1602 | 2256-16 | AL021475 | *C. elegans* |
| 1603 | 2256-21 | Heterogenous Nuclear Ribonucleoprotein C1 | *H. sapiens* |
| 1604 | 2256-22 | b4 integrin interactor | *H. sapiens* |
| 1605 | 2256-28 | testis enhanced gene transcript protein | *H. sapiens* |
| 1606 | 2256-31 | synaptic vesicle protein 2B | *R. norvegicus* |
| 1607 | 2256-40 | TNF-alpha stimulated ABC protein | *H. sapiens* |
| 1608 | 2256-42 | carboxypeptidase A | *H. armigera* |
| 1609 | 2256-46 | pherophorin S | *V. carteri* |
| 1610 | 2256-52 | Fo-ATP synthase subunit b | *D. melanogaster* |

(continued)

| SEQ ID NO: | Name: | GenBank Homology | *Organism* |
|---|---|---|---|
| 1611 | 2256-54 | PDGF associated protein | *H. sapiens* |
| 1612 | 2256-58 | S20 ribosomal protein | *D. melanogaster* |
| 1613 | 2256-64 | ribosomal protein S9 | *H. sapiens* |
| 1614 | 2256-69 | elongation factor 1-gamma | *Artemia sp* |
| 1615 | 2256-70 | conserved hypothetical protein | *S. pombe* |
| 1616 | 2256-72 | fructose 1,6 bisphosphate-aldolase 4C | *D. melanogaster* |
| 1617 | 2256-73 | troponin-T | *D. melanogaster* |
| 1618 | 2256-80 | SRP14 | *C. familiaris* |
| 1619 | 2256-82 | succinyl-CoA synthetase alpha subunit | *S. scrofa* |
| 1620 | 2256-89 | Csa-19 | *H. sapiens* |
| 1621 | 2256-92 | Sacm21 | *M. musculus* |
| 1622 | 2256-94 | apoptosis inhibitor | *Cydia pomonella granulosis virus* |
| 1623 | 2256-96 | ribosomal protein L22 | *D. melanogaster* |

Table III represents a variety of flea HNC nucleic acid molecules described herein.

Table III

| SEQ ID NO: | Name |
|---|---|
| 567 | 2096-19NB.HNC |
| 568 | 2096-25NB.HNC |
| 569 | 2096-48NB.HNC |
| 570 | 2096-50NB.HNC |
| 571 | 2096-52NB.HNC |
| 572 | 2096-55NB.HNC |
| 573 | 2097-09NB.HNC |
| 574 | 2097-15NB.HNC |
| 575 | 2097-20NB.HNC |
| 576 | 2097-22NB.HNC |
| 577 | 2097-32NB.HNC |
| 578 | 2097-45NB.HNC |
| 579 | 2097-46NB.HNC |
| 580 | 2097-47NB.HNC |
| 581 | 2097-56NB.HNC |
| 582 | 2097-64NB.HNC |
| 583 | 2098-04NB.HNC |
| 584 | 2098-40NB.HNC |
| 585 | 2098-43NB.HNC |
| 586 | 2099-9NB.HNC |
| 587 | 2100-10NB.HNC |
| 588 | 2100-45NB.HNC |
| 589 | 2100-47NB.HNC |
| 590 | 2100-56NB.HNC |
| 591 | 2100-63NB.HNC |
| 592 | 2110-41NB.HNC |
| 593 | 2110-53NB.HNC |
| 594 | 2112-12NB.HNC |
| 595 | 2112-35NB.HNC |
| 596 | 2113-17NB.HNC |
| 597 | 2115-16NB.HNC |
| 598 | 2115-22NB.HNC |
| 599 | 2115-3NB.HNC |
| 600 | 2116-19NB.HNC |
| 601 | 2116-24NB.HNC |

| SEQ ID NO: | Name |
|---|---|
| 602 | 2116-27NB.HNC |
| 603 | 2116-41NB.HNC |
| 604 | 2116-59NB.HNC |
| 605 | 2116-64NB.HNC |
| 606 | 2117-05NB.HNC |
| 607 | 2117-09NB.HNC |
| 608 | 2117-11NB.HNC |
| 609 | 2117-53NB.HNC |
| 610 | 2118-03NB.HNC |
| 611 | 2122-39NB.HNC |
| 612 | 2123-25NB.HNC |
| 613 | 2124-40NB.HNC |
| 614 | 2124-62NB.HNC |
| 615 | 2131-22NB.HNC |
| 616 | 2131-32NB.HNC |
| 617 | 2132-15NB.HNC |
| 618 | 2132-28NB.HNC |
| 619 | 2132-63NB.HNC |
| 620 | 2132-9NB.HNC |
| 621 | 2137-19NB.HNC |
| 622 | 2137-24NB.HNC |
| 623 | 2138-05NB.HNC |
| 624 | 2138-51NB.HNC |
| 625 | 2139-31NB.HNC |
| 626 | 2139-41NB.HNC |
| 627 | 2139-60NB.HNC |
| 628 | 2140-13NB.HNC |
| 629 | 2140-15NB.HNC |
| 630 | 2140-18NB.HNC |
| 631 | 2140-54NB.HNC |
| 632 | 2141-16NB.HNC |
| 633 | 2141-59NB.HNC |
| 634 | 2142-16NB.HNC |
| 635 | 2142-18NB.HNC |
| 636 | 2143-06NB.HNC |
| 637 | 2143-07NB.HNC |

Table III (cont'd)

| SEQ ID NO: | Name |
|---|---|
| 638 | 2143-33NB.HNC |
| 639 | 2143-54NB.HNC |
| 640 | 2168-06NB.HNC |
| 641 | 2168-09NB.HNC |
| 642 | 2168-42NB.HNC |
| 643 | 2168-79NB.HNC |
| 644 | 2168-82NB.HNC |
| 645 | 2170-04NB.HNC |
| 646 | 2170-08NB.HNC |
| 647 | 2170-82NB.HNC |
| 648 | 2172-39NB.HNC |
| 649 | 2172-59NB.HNC |
| 650 | 2172-60NB.HNC |
| 651 | 2172-77NB.HNC |
| 652 | 2174-14NB.HNC |
| 653 | 2174-17NB.HNC |
| 654 | 2174-41NB.HNC |
| 655 | 2174-49NB.HNC |
| 656 | 2174-59NB.HNC |
| 657 | 2174-68NB.HNC |
| 658 | 2176-21NB.HNC |
| 659 | 2176-34NB.HNC |
| 660 | 2176-47NB.HNC |
| 661 | 2176-56NB.HNC |
| 662 | 2176-62NB.HNC |
| 663 | 2176-63NB.HNC |
| 664 | 2176-64NB.HNC |
| 665 | 2176-65NB.HNC |
| 666 | 2176-75NB.HNC |
| 667 | 2178-05NB.HNC |
| 668 | 2178-13NB.HNC |
| 669 | 2178-23NB.HNC |
| 670 | 2178-25NB.HNC |
| 671 | 2178-41NB.HNC |
| 672 | 2178-56NB.HNC |
| 673 | 2178-57NB.HNC |
| 674 | 2178-58NB.HNC |
| 675 | 2178-67NB.HNC |
| 676 | 2178-72NB.HNC |

| SEQ ID NO: | Name |
|---|---|
| 677 | 2178-78NB.HNC |
| 678 | 2178-80NB.HNC |
| 679 | 2178-90NB.HNC |
| 680 | 2178-91NB.HNC |
| 681 | 2178-95NB.HNC |
| 682 | 2180-05NB.HNC |
| 683 | 2180-18NB.HNC |
| 684 | 2180-20NB.HNC |
| 685 | 2180-32NB.HNC |
| 686 | 2180-59NB.HNC |
| 687 | 2180-62NB.HNC |
| 688 | 2180-74NB.HNC |
| 689 | 2180-78NB.HNC |
| 690 | 2180-79NB.HNC |
| 691 | 2180-88NB.HNC |
| 692 | 2180-90NB.HNC |
| 693 | 2182-07NB.HNC |
| 694 | 2182-12NB.HNC |
| 695 | 2182-13NB.HNC |
| 696 | 2182-27NB.HNC |
| 697 | 2182-2NB.HNC |
| 698 | 2182-46NB.HNC |
| 699 | 2182-55NB.HNC |
| 700 | 2182-57NB.HNC |
| 701 | 2182-63NB.HNC |
| 702 | 2182-64NB.HNC |
| 703 | 2182-83NB.HNC |
| 704 | 2182-86NB.HNC |
| 705 | 2182-88NB.HNC |
| 706 | 2182-90NB.HNC |
| 707 | 2182-92NB.HNC |
| 708 | 2182-94NB.HNC |
| 709 | 2184-15NB.HNC |
| 710 | 2184-37NB.HNC |
| 711 | 2184-65NB.HNC |
| 712 | 2186-14NB.HNC |
| 713 | 2186-45NB.HNC |
| 714 | 2186-50NB.HNC |
| 715 | 2186-52NB.HNC |

Table III (cont'd)

| SEQ ID NO: | Name |
|---|---|
| 716 | 2186-60NB.HNC |
| 717 | 2186-62NB.HNC |
| 718 | 2186-63NB.HNC |
| 719 | 2186-68NB.HNC |
| 720 | 2186-69NB.HNC |
| 721 | 2211-19NB.HNC |
| 722 | 2211-23NB.HNC |
| 723 | 2211-29NB.HNC |
| 724 | 2211-30NB.HNC |
| 725 | 2211-43NB.HNC |
| 726 | 2211-52NB.HNC |
| 727 | 2211-64NB.HNC |
| 728 | 2212-30NB.HNC |
| 729 | 2212-31NB.HNC |
| 730 | 2212-71NB.HNC |
| 731 | 2212-72NB.HNC |
| 732 | 2212-73NB.HNC |
| 733 | 2212-81NB.HNC |
| 734 | 2212-85NB.HNC |
| 735 | 2212-87NB.HNC |
| 736 | 2212-91NB.HNC |
| 737 | 2212-96NB.HNC |
| 738 | 2212-9NB.HNC |
| 739 | 2213-08NB.HNC |
| 740 | 2213-09NB.HNC |
| 741 | 2213-11NB.HNC |
| 742 | 2213-12NB.HNC |
| 743 | 2213-18NB.HNC |
| 744 | 2213-34NB.HNC |
| 745 | 2213-53NB.HNC |
| 746 | 2213-58NB.HNC |
| 747 | 2213-67NB.HNC |
| 748 | 2213-79NB.HNC |
| 749 | 2214-02NB.HNC |
| 750 | 2214-03NB.HNC |
| 751 | 2214-05NB.HNC |
| 752 | 2214-07NB.HNC |
| 753 | 2214-15NB.HNC |
| 754 | 2214-23NB.HNC |

| SEQ ID NO: | Name |
|---|---|
| 755 | 2214-30NB.HNC |
| 756 | 2214-36NB.HNC |
| 757 | 2214-37NB.HNC |
| 758 | 2214-40NB.HNC |
| 759 | 2214-43NB.HNC |
| 760 | 2214-53NB.HNC |
| 761 | 2214-57NB.HNC |
| 762 | 2214-60NB.HNC |
| 763 | 2214-61NB.HNC |
| 764 | 2214-73NB.HNC |
| 765 | 2214-76NB.HNC |
| 766 | 2214-80NB.HNC |
| 767 | 2215-07NB.HNC |
| 768 | 2215-15NB.HNC |
| 769 | 2215-31NB.HNC |
| 770 | 2215-41NB.HNC |
| 771 | 2215-51NB.HNC |
| 772 | 2215-80NB.HNC |
| 773 | 2215-85NB.HNC |
| 774 | 2215-91NB.HNC |
| 775 | 2217-14NB.HNC |
| 776 | 2217-16NB.HNC |
| 777 | 2217-33NB.HNC |
| 778 | 2217-39NB.HNC |
| 779 | 2217-78NB.HNC |
| 780 | 2217-92NB.HNC |
| 781 | 2218-15NB.HNC |
| 782 | 2218-19NB.HNC |
| 783 | 2218-26NB.HNC |
| 784 | 2218-36NB.HNC |
| 785 | 2218-41NB.HNC |
| 786 | 2218-56NB.HNC |
| 787 | 2218-58NB.HNC |
| 788 | 2218-69NB.HNC |
| 789 | 2218-71NB.HNC |
| 790 | 2218-76NB.HNC |
| 791 | 2218-77NB.HNC |
| 792 | 2218-84NB.HNC |
| 793 | 2218-96NB.HNC |

Table III (cont'd)

| SEQ ID NO: | Name |
|---|---|
| 794 | 2219-11NB.HNC |
| 795 | 2219-13NB.HNC |
| 796 | 2219-17NB.HNC |
| 797 | 2219-19NB.HNC |
| 798 | 2219-20NB.HNC |
| 799 | 2219-22NB.HNC |
| 800 | 2219-23NB.HNC |
| 801 | 2219-32NB.HNC |
| 802 | 2219-45NB.HNC |
| 803 | 2219-49NB.HNC |
| 804 | 2219-51NB.HNC |
| 805 | 2219-72NB.HNC |
| 806 | 2219-80NB.HNC |
| 807 | 2219-952122-39NB.HNC |
|  | 2220-02NB.HNC |
| 808 | 2220-02NB.HNC |
| 809 | 2220-27NB.HNC |
| 810 | 2220-32NB.HNC |
| 811 | 2220-53NB.HNC |
| 812 | 2220-60NB.HNC |
| 813 | 2220-66NB.HNC |
| 814 | 2221-06NB.HNC |
| 815 | 2221-15NB.HNC |
| 816 | 2221-18NB.HNC |
| 817 | 2221-20NB.HNC |
| 818 | 2221-24NB.HNC |
| 819 | 2221-45NB.HNC |
| 820 | 2221-46NB.HNC |
| 821 | 2221-48NB.HNC |
| 822 | 2221-54NB.HNC |
| 823 | 2221-55NB.HNC |
| 824 | 2221-59NB.HNC |
| 825 | 2221-61NB.HNC |
| 826 | 2221-62NB.HNC |
| 827 | 2221-70NB.HNC |
| 828 | 2221-86NB.HNC |
| 829 | 2221-87NB.HNC |
| 830 | 2221-95NB.HNC |
| 831 | 2223u-18NB.HNC |

| SEQ ID NO: | Name |
|---|---|
| 832 | 2223u-22NB.HNC |
| 833 | 2223u-23NB.HNC |
| 834 | 2223u-31NB.HNC |
| 835 | 2223u-33NB.HNC |
| 836 | 2223u-36NB.HNC |
| 837 | 2223u-67NB.HNC |
| 838 | 2223u-85NB.HNC |
| 839 | 2224u-05NB.HNC |
| 840 | 2224u-07NB.HNC |
| 841 | 2224u-10NB.HNC |
| 842 | 2224u-11NB.HNC |
| 843 | 2224u-15NB.HNC |
| 844 | 2224u-25NB.HNC |
| 845 | 2224u-27NB.HNC |
| 846 | 2224u-44NB.HNC |
| 847 | 2224u-52NB.HNC |
| 848 | 2224u-62NB.HNC |
| 849 | 2224u-70NB.HNC |
| 850 | 2224u-71NB.HNC |
| 851 | 2224u-79NB.HNC |
| 852 | 2225u-11NB.HNC |
| 853 | 2225u-20NB.HNC |
| 854 | 2225u-23NB.HNC |
| 855 | 2225u-28NB.HNC |
| 856 | 2225u-55NB.HNC |
| 857 | 2225u-59NB.HNC |
| 858 | 2225u-64NB.HNC |
| 859 | 2225u-77NB.HNC |
| 860 | 2225u-95NB.HNC |
| 861 | 2226-932122-39NB.HNC |
| 862 | 2226u-07NB.HNC |
| 863 | 2226u-19NB.HNC |
| 864 | 2226u-39NB.HNC |
| 865 | 2226u-45NB.HNC |
| 866 | 2226u-49NB.HNC |
| 867 | 2226u-54NB.HNC |
| 868 | 2226u-71NB.HNC |
| 869 | 2226u-77NB.HNC |

Table III (cont'd)

| SEQ ID NO: | Name |
|---|---|
| 870 | 2226u-83NB.HNC |
| 871 | 2226u-91NB.HNC |
| 872 | 2227u-12NB.HNC |
| 873 | 2227u-13NB.HNC |
| 874 | 2227u-23NB.HNC |
| 875 | 2227u-26NB.HNC |
| 876 | 2227u-30NB.HNC |
| 877 | 2227u-31NB.HNC |
| 878 | 2227u-33NB.HNC |
| 879 | 2227u-43NB.HNC |
| 880 | 2227u-51NB.HNC |
| 881 | 2227u-60NB.HNC |
| 882 | 2227u-93NB.HNC |
| 883 | 2228u-04NB.HNC |
| 884 | 2228u-09NB.HNC |
| 885 | 2228u-12NB.HNC |
| 886 | 2228u-21NB.HNC |
| 887 | 2228u-26NB.HNC |
| 888 | 2228u-49NB.HNC |
| 889 | 2228u-54NB.HNC |
| 890 | 2228u-55NB.HNC |
| 891 | 2228u-61NB.HNC |
| 892 | 2228u-65NB.HNC |
| 893 | 2228u-79NB.HNC |
| 894 | 2228u-90NB.HNC |
| 1624 | 2222-7 |
| 1625 | 2222-16 |
| 1626 | 2222-19 |
| 1627 | 2222-39 |
| 1628 | 2222-56 |
| 1629 | 2222-59 |
| 1630 | 2222-79 |
| 1631 | 2222-89 |
| 1632 | 2228-4 |
| 1633 | 2228-9 |
| 1634 | 2228-12 |
| 1635 | 2228-21 |
| 1636 | 2228-26 |
| 1637 | 2228-49 |
| 1638 | 2228-54 |
| 1639 | 2228-61 |
| 1640 | 2228-65 |

| SEQ ID NO: | Name |
|---|---|
| 1641 | 2228-79 |
| 1642 | 2228-90 |
| 1643 | 2245-5 |
| 1644 | 2245-7 |
| 1645 | 2245-15 |
| 1646 | 2245-16 |
| 1647 | 2245-17 |
| 1648 | 2245-20 |
| 1649 | 2245-35 |
| 1650 | 2245-38 |
| 1651 | 2245-39 |
| 1652 | 2245-51 |
| 1653 | 2245-52 |
| 1654 | 2245-57 |
| 1655 | 2246-13 |
| 1656 | 2246-19 |
| 1657 | 2246-25 |
| 1658 | 2246-27 |
| 1659 | 2246-29 |
| 1660 | 2246-40 |
| 1661 | 2246-45 |
| 1662 | 2246-52 |
| 1663 | 2246-64 |
| 1664 | 2246-66 |
| 1665 | 2246-74 |
| 1666 | 2246-82 |
| 1667 | 2247-6 |
| 1668 | 2247-17 |
| 1669 | 2247-29 |
| 1670 | 2247-31 |
| 1671 | 2247-36 |
| 1672 | 2247-40 |
| 1673 | 2247-46 |
| 1674 | 2247-50 |
| 1675 | 2247-54 |
| 1676 | 2247-63 |
| 1677 | 2247-66 |
| 1678 | 2247-68 |
| 1679 | 2247-69 |
| 1680 | 2247-81 |
| 1681 | 2247-82 |
| 1682 | 2247-95 |
| 1683 | 2248-7 |
| 1684 | 2248-18 |
| 1685 | 2248-32 |
| 1686 | 2248-41 |

Table III (cont'd)

| SEQ ID NO: | Name |
|---|---|
| 1687 | 2248-50 |
| 1688 | 2248-54 |
| 1689 | 2248-60 |
| 1690 | 2248-62 |
| 1691 | 2248-65 |
| 1692 | 2248-86 |
| 1693 | 2248-94 |
| 1694 | 2249-6 |
| 1695 | 2249-30 |
| 1696 | 2249-35 |
| 1697 | 2249-36 |
| 1698 | 2249-68 |
| 1699 | 2249-74 |
| 1700 | 2249-79 |
| 1701 | 2250-20 |
| 1702 | 2250-24 |
| 1703 | 2251-7 |
| 1704 | 2251-21 |
| 1705 | 2251-25 |
| 1706 | 2251-38 |
| 1707 | 2251-58 |
| 1708 | 2252-7 |
| 1709 | 2252-15 |
| 1710 | 2252-19 |
| 1711 | 2252-24 |
| 1712 | 2252-26 |
| 1713 | 2252-27 |
| 1714 | 2252-32 |
| 1715 | 2252-36 |
| 1716 | 2252-37 |
| 1717 | 2252-69 |
| 1718 | 2252-78 |

Table IV represents a variety of flea HMT nucleic acid molecules described herein.

Table IV

| SEQ ID NO: | Name |
|---|---|
| 895 | 2084-02.HMTNB |
| 896 | 2084-05.HMTNB |
| 897 | 2084-07.HMTNB |
| 898 | 2084-09.HMTNB |
| 899 | 2084-15.HMTNB |
| 900 | 2084-17.HMTNB |
| 901 | 2084-18.HMTNB |
| 902 | 2084-21.HMTNB |
| 903 | 2084-22.HMTNB |
| 904 | 2084-30.HMTNB |
| 905 | 2084-33.HMTNB |
| 906 | 2084-36.HMTNB |
| 907 | 2084-37.HMTNB |
| 908 | 2084-38.HMTNB |
| 909 | 2084-39.HMTNB |
| 910 | 2084-43.HMTNB |
| 911 | 2084-50.HMTNB |
| 912 | 2084-54.HMTNB |
| 913 | 2084-56.HMTNB |
| 914 | 2084-59.HMTNB |
| 915 | 2085-03.HMTNB |
| 916 | 2085-13.HMTNB |
| 917 | 2085-35.HMTNB |
| 918 | 2085-38.HMTNB |
| 919 | 2085-39.HMTNB |
| 920 | 2085-49.HMTNB |
| 921 | 2085-53.HMTNB |
| 922 | 2085-58.HMTNB |
| 923 | 2085-61.HMTNB |
| 924 | 2086-05.HMTNB |
| 925 | 2086-10.HMTNB |
| 926 | 2086-13.HMTNB |
| 927 | 2086-15.HMTNB |
| 928 | 2086-20.HMTNB |
| 929 | 2086-25.HMTNB |
| 930 | 2086-32.HMTNB |
| 931 | 2086-33.HMTNB |

| SEQ ID NO: | Name |
|---|---|
| 932 | 2086-34.HMTNB |
| 933 | 2086-37.HMTNB |
| 934 | 2086-41.HMTNB |
| 935 | 2086-43.HMTNB |
| 936 | 2086-44.HMTNB |
| 937 | 2086-54.HMTNB |
| 938 | 2086-55.HMTNB |
| 939 | 2086-58.HMTNB |
| 940 | 2087-09.HMTNB |
| 941 | 2087-17.HMTNB |
| 942 | 2087-28.HMTNB |
| 943 | 2087-33.HMTNB |
| 944 | 2087-35.HMTNB |
| 945 | 2087-51.HMTNB |
| 946 | 2087-54.HMTNB |
| 947 | 2088-07.HMTNB |
| 948 | 2088-17.HMTNB |
| 949 | 2088-35.HMTNB |
| 950 | 2088-52.HMTNB |
| 951 | 2088-59.HMTNB |
| 952 | 2089-12.HMTNB |
| 953 | 2089-14.HMTNB |
| 954 | 2089-33.HMTNB |
| 955 | 2089-36.HMTNB |
| 956 | 2089-51.HMTNB |
| 957 | 2089-60.HMTNB |
| 958 | 2090-11.HMTNB |
| 959 | 2090-27.HMTNB |
| 960 | 2090-33.HMTNB |
| 961 | 2090-44.HMTNB |
| 962 | 2090-57.HMTNB |
| 963 | 2091-11.HMTNB |
| 964 | 2091-22.HMTNB |
| 965 | 2091-23.HMTNB |
| 966 | 2091-35.HMTNB |
| 967 | 2091-63.HMTNB |
| 968 | 2092-11.HMTNB |

Table IV (cont'd)

| SEQ ID NO: | Name |
|---|---|
| 969 | 2092-16.HMTNB |
| 970 | 2092-40.HMTNB |
| 971 | 2092-42.HMTNB |
| 972 | 2092-46.HMTNB |
| 973 | 2092-60.HMTNB |
| 974 | 2093-20.HMTNB |
| 975 | 2093-23.HMTNB |
| 976 | 2093-43.HMTNB |
| 977 | 2093-48.HMTNB |
| 978 | 2093-50.HMTNB |
| 979 | 2093-62.HMTNB |
| 980 | 2093-63.HMTNB |
| 981 | 2094-08.HMTNB |
| 982 | 2094-26.HMTNB |
| 983 | 2094-33.HMTNB |
| 984 | 2094-47.HMTNB |
| 985 | 2094-50.HMTNB |
| 986 | 2094-62.HMTNB |
| 987 | 2095-04.HMTNB |
| 988 | 2095-10.HMTNB |
| 989 | 2095-12.HMTNB |
| 990 | 2095-13.HMTNB |
| 991 | 2095-15.HMTNB |
| 992 | 2095-20.HMTNB |
| 993 | 2095-22.HMTNB |
| 994 | 2095-31.HMTNB |
| 995 | 2095-33.HMTNB |
| 996 | 2095-34.HMTNB |
| 997 | 2095-36.HMTNB |
| 998 | 2095-40.HMTNB |
| 999 | 2095-48.HMTNB |
| 1000 | 2102-12.HMTNB |
| 1001 | 2102-16.HMTNB |
| 1002 | 2102-18.HMTNB |
| 1003 | 2102-19.HMTNB |
| 1004 | 2102-20.HMTNB |
| 1005 | 2102-29.HMTNB |
| 1006 | 2102-35.HMTNB |
| 1007 | 2102-37.HMTNB |
| 1008 | 2102-38.HMTNB |
| 1009 | 2102-41.HMTNB |
| 1010 | 2102-47.HMTNB |
| 1011 | 2103-02.HMTNB |
| 1012 | 2103-09.HMTNB |
| 1013 | 2103-45.HMTNB |
| 1014 | 2103-56.HMTNB |
| 1015 | 2103-58.HMTNB |
| 1016 | 2104-58.HMTNB |

| SEQ ID NO: | Name |
|---|---|
| 1017 | 2104-60.HMTNB |
| 1018 | 2104-61.HMTNB |
| 1019 | 2105-02.HMTNB |
| 1020 | 2105-20.HMTNB |
| 1021 | 2105-35.HMTNB |
| 1022 | 2105-42.HMTNB |
| 1023 | 2105-44.HMTNB |
| 1024 | 2106-05.HMTNB |
| 1025 | 2106-27.HMTNB |
| 1026 | 2106-29.HMTNB |
| 1027 | 2106-34.HMTNB |
| 1028 | 2106-48.HMTNB |
| 1029 | 2106-50.HMTNB |
| 1030 | 2106-64.HMTNB |
| 1031 | 2107-02.HMTNB |
| 1032 | 2107-10.HMTNB |
| 1033 | 2107-37.HMTNB |
| 1034 | 2108-03.HMTNB |
| 1035 | 2108-23.HMTNB |
| 1036 | 2108-46.HMTNB |
| 1037 | 2108-47.HMTNB |
| 1038 | 2108-48.HMTNB |
| 1039 | 2108-49.HMTNB |
| 1040 | 2108-63.HMTNB |
| 1041 | 2109-04.HMTNB |
| 1042 | 2109-06.HMTNB |
| 1043 | 2109-37.HMTNB |
| 1044 | 2109-38.HMTNB |
| 1045 | 2109-44.HMTNB |
| 1046 | 2154-08.HMTNB |
| 1047 | 2154-09.HMTNB |
| 1048 | 2154-10.HMTNB |
| 1049 | 2154-28.HMTNB |
| 1050 | 2154-30.HMTNB |
| 1051 | 2154-45.HMTNB |
| 1052 | 2154-46.HMTNB |
| 1053 | 2154-61.HMTNB |
| 1054 | 2154-71.HMTNB |
| 1055 | 2154-81.HMTNB |
| 1056 | 2154-83.HMTNB |
| 1057 | 2156-02.HMTNB |
| 1058 | 2156-06.HMTNB |
| 1059 | 2156-18.HMTNB |
| 1060 | 2156-27.HMTNB |
| 1061 | 2156-43.HMTNB |
| 1062 | 2156-48.HMTNB |
| 1063 | 2156-50.HMTNB |
| 1064 | 2157-16.HMTNB |

Table IV (cont'd)

| SEQ ID NO: | Name |
|---|---|
| 1065 | 2157-34.HMTNB |
| 1066 | 2157-45.HMTNB |
| 1067 | 2157-70.HMTNB |
| 1068 | 2157-75.HMTNB |
| 1069 | 2157-79.HMTNB |
| 1070 | 2157-86.HMTNB |
| 1071 | 2158-02.HMTNB |
| 1072 | 2158-14.HMTNB |
| 1073 | 2158-19.HMTNB |
| 1074 | 2158-22.HMTNB |
| 1075 | 2158-27.HMTNB |
| 1076 | 2158-34.HMTNB |
| 1077 | 2158-37.HMTNB |
| 1078 | 2158-39.HMTNB |
| 1079 | 2159-07.HMTNB |
| 1080 | 2159-09.HMTNB |
| 1081 | 2159-17.HMTNB |
| 1082 | 2159-34.HMTNB |
| 1083 | 2159-35.HMTNB |
| 1084 | 2159-60.HMTNB |
| 1085 | 2160-16.HMTNB |
| 1086 | 2160-17.HMTNB |
| 1087 | 2160-29.HMTNB |
| 1088 | 2160-30.HMTNB |
| 1089 | 2160-32.HMTNB |
| 1090 | 2160-39.HMTNB |
| 1091 | 2160-49.HMTNB |
| 1092 | 2160-53.HMTNB |
| 1093 | 2160-54.HMTNB |
| 1094 | 2160-55.HMTNB |
| 1095 | 2160-77.HMTNB |
| 1096 | 2160-82.HMTNB |
| 1097 | 2160-89.HMTNB |
| 1098 | 2160-91.HMTNB |
| 1099 | 2161-13.HMTNB |
| 1100 | 2161-19.HMTNB |
| 1101 | 2161-45.HMTNB |
| 1102 | 2161-57.HMTNB |
| 1103 | 2161-60.HMTNB |
| 1104 | 2161-79.HMTNB |
| 1105 | 2161-83.HMTNB |
| 1106 | 2161-90.HMTNB |
| 1107 | 2161-94.HMTNB |
| 1108 | 2162-05.HMTNB |
| 1109 | 2162-12.HMTNB |
| 1110 | 2162-13.HMTNB |
| 1111 | 2162-18.HMTNB |
| 1112 | 2162-35.HMTNB |

| SEQ ID NO: | Name |
|---|---|
| 1113 | 2162-41.HMTNB |
| 1114 | 2162-50.HMTNB |
| 1115 | 2162-59.HMTNB |
| 1116 | 2162-63.HMTNB |
| 1117 | 2162-71.HMTNB |
| 1118 | 2162-75.HMTNB |
| 1119 | 2162-78.HMTNB |
| 1120 | 2163-07.HMTNB |
| 1121 | 2163-11.HMTNB |
| 1122 | 2163-18.HMTNB |
| 1123 | 2163-23.HMTNB |
| 1124 | 2163-25.HMTNB |
| 1125 | 2163-43.HMTNB |
| 1126 | 2163-50.HMTNB |
| 1127 | 2163-61.HMTNB |
| 1128 | 2163-65.HMTNB |
| 1129 | 2163-73.HMTNB |
| 1130 | 2163-77.HMTNB |
| 1131 | 2163-87.HMTNB |
| 1132 | 2163-93.HMTNB |
| 1133 | 2163-95.HMTNB |
| 1134 | 2165-04.HMTNB |
| 1135 | 2165-06.HMTNB |
| 1136 | 2165-24.HMTNB |
| 1137 | 2165-45.HMTNB |
| 1138 | 2165-59.HMTNB |
| 1139 | 2165-65.HMTNB |
| 1140 | 2166-02.HMTNB |
| 1141 | 2166-12.HMTNB |
| 1142 | 2166-42.HMTNB |
| 1143 | 2166-46.HMTNB |
| 1144 | 2166-47.HMTNB |
| 1145 | 2167-07.HMTNB |
| 1146 | 2167-16.HMTNB |
| 1147 | 2167-42.HMTNB |
| 1148 | 2167-65.HMTNB |
| 1149 | 2167-66.HMTNB |
| 1150 | 2167-79.HMTNB |
| 1151 | 2167-90.HMTNB |
| 1152 | 2167-94.HMTNB |
| 1153 | 2169-05.HMTNB |
| 1154 | 2169-12.HMTNB |
| 1155 | 2169-16.HMTNB |
| 1156 | 2169-17.HMTNB |
| 1157 | 2169-19.HMTNB |
| 1158 | 2169-22.HMTNB |
| 1159 | 2169-26.HMTNB |
| 1160 | 2169-33.HMTNB |

Table IV (cont'd)

| SEQ ID NO: | Name | SEQ ID NO: | Name |
|---|---|---|---|
| 1161 | 2169-42.HMTNB | 1209 | 2181-84.HMTNB |
| 1162 | 2169-46.HMTNB | 1210 | 2183-05.HMTNB |
| 1163 | 2169-47.HMTNB | 1211 | 2183-13.HMTNB |
| 1164 | 2169-57.HMTNB | 1212 | 2183-17.HMTNB |
| 1165 | 2169-69.HMTNB | 1213 | 2183-28.HMTNB |
| 1166 | 2171-06.HMTNB | 1214 | 2183-45.HMTNB |
| 1167 | 2171-09.HMTNB | 1215 | 2183-50.HMTNB |
| 1168 | 2171-11.HMTNB | 1216 | 2183-51.HMTNB |
| 1169 | 2171-29.HMTNB | 1217 | 2183-70.HMTNB |
| 1170 | 2171-33.HMTNB | 1218 | 2185-05.HMTNB |
| 1171 | 2171-35.HMTNB | 1219 | 2185-10.HMTNB |
| 1172 | 2171-41.HMTNB | 1220 | 2185-12.HMTNB |
| 1173 | 2171-54.HMTNB | 1221 | 2185-18.HMTNB |
| 1174 | 2171-57.HMTNB | 1222 | 2185-43.HMTNB |
| 1175 | 2171-69.HMTNB | 1223 | 2185-49.HMTNB |
| 1176 | 2171-82.HMTNB | 1224 | 2185-54.HMTNB |
| 1177 | 2171-84.HMTNB | 1225 | 2185-82.HMTNB |
| 1178 | 2171-85.HMTNB | 1226 | 2187-21.HMTNB |
| 1179 | 2173-12.HMTNB | 1227 | 2187-37.HMTNB |
| 1180 | 2173-34.HMTNB | 1228 | 2187-47.HMTNB |
| 1181 | 2173-42.HMTNB | 1229 | 2187-93.HMTNB |
| 1182 | 2173-48.HMTNB | 1230 | 2188-22.HMTNB |
| 1183 | 2173-54.HMTNB | 1231 | 2188-29.HMTNB |
| 1184 | 2173-57.HMTNB | 1232 | 2188-32.HMTNB |
| 1185 | 2173-75.HMTNB | 1233 | 2188-52.HMTNB |
| 1186 | 2173-86.HMTNB | 1234 | 2188-54.HMTNB |
| 1187 | 2173-91.HMTNB | 1235 | 2188-72.HMTNB |
| 1188 | 2175-06.HMTNB | 1236 | 2188-92.HMTNB |
| 1189 | 2175-15.HMTNB | 1237 | 2189-31.HMTNB |
| 1190 | 2175-20.HMTNB | 1238 | 2189-56.HMTNB |
| 1191 | 2175-58.HMTNB | 1239 | 2189-75.HMTNB |
| 1192 | 2175-96.HMTNB | 1240 | 2189-84.HMTNB |
| 1193 | 2177-16.HMTNB | 1241 | 2191-23.HMTNB |
| 1194 | 2177-70.HMTNB | 1242 | 2191-38.HMTNB |
| 1195 | 2177-86.HMTNB | 1243 | 2191-58.HMTNB |
| 1196 | 2179-02.HMTNB | 1244 | 2191-73.HMTNB |
| 1197 | 2179-03.HMTNB | 1245 | 2191-77.HMTNB |
| 1198 | 2179-19.HMTNB | 1246 | 2191-90.HMTNB |
| 1199 | 2179-22.HMTNB | 1247 | 2191-94.HMTNB |
| 1200 | 2179-29.HMTNB | 1248 | 2191-96.HMTNB |
| 1201 | 2179-39.HMTNB | 1249 | 2192-03.HMTNB |
| 1202 | 2179-63.HMTNB | 1250 | 2192-14.HMTNB |
| 1203 | 2181-04.HMTNB | 1251 | 2192-36.HMTNB |
| 1204 | 2181-24.HMTNB | 1252 | 2192-46.HMTNB |
| 1205 | 2181-35.HMTNB | 1253 | 2192-88.HMTNB |
| 1206 | 2181-66.HMTNB | 1254 | 2194-07.HMTNB |
| 1207 | 2181-75.HMTNB | 1255 | 2194-13.HMTNB |
| 1208 | 2181-76.HMTNB | 1256 | 2194-16.HMTNB |

Table IV (cont'd)

| SEQ ID NO: | Name |
|---|---|
| 1257 | 2194-18.HMTNB |
| 1258 | 2194-28.HMTNB |
| 1259 | 2195-06.HMTNB |
| 1260 | 2195-47.HMTNB |
| 1261 | 2195-60.HMTNB |
| 1262 | 2196-18.HMTNB |
| 1263 | 2196-30.HMTNB |
| 1264 | 2196-53.HMTNB |
| 1265 | 2196-65.HMTNB |
| 1266 | 2196-76.HMTNB |
| 1267 | 2197-28.HMTNB |
| 1268 | 2197-46.HMTNB |
| 1269 | 2197-51.HMTNB |
| 1270 | 2197-59.HMTNB |
| 1271 | 2202-96.HMTNB |
| 1272 | 2203-36.HMTNB |
| 1273 | 2204-09.HMTNB |
| 1274 | 2205-11.HMTNB |
| 1275 | 2205-33.HMTNB |
| 1276 | 2205-43.HMTNB |
| 1277 | 2205-85.HMTNB |
| 1278 | 2229-08u.HMTNB |
| 1279 | 2229-10u.HMTNB |
| 1280 | 2229-12u.HMTNB |
| 1281 | 2229-14u.HMTNB |
| 1282 | 2229-27u.HMTNB |
| 1283 | 2229-40u.HMTNB |
| 1284 | 2229-45u.HMTNB |
| 1285 | 2229-48u.HMTNB |
| 1286 | 2229-50u.HMTNB |
| 1287 | 2229-54u.HMTNB |
| 1288 | 2229-56u.HMTNB |
| 1289 | 2229-57u.HMTNB |
| 1290 | 2229-59u.HMTNB |
| 1291 | 2229-70u.HMTNB |
| 1292 | 2229-87u.HMTNB |
| 1293 | 2229-91u.HMTNB |
| 1294 | 2229-95u.HMTNB |
| 1295 | 2230-07u.HMTNB |
| 1296 | 2230-11u.HMTNB |
| 1297 | 2230-19u.HMTNB |
| 1298 | 2230-27u.HMTNB |
| 1299 | 2230-33u.HMTNB |
| 1300 | 2230-41u.HMTNB |
| 1301 | 2230-51u.HMTNB |
| 1302 | 2230-56u.HMTNB |
| 1303 | 2230-66u.HMTNB |
| 1304 | 2230-71u.HMTNB |

| SEQ ID NO: | Name |
|---|---|
| 1305 | 2230-75.HMTNB |
| 1306 | 2230-81u.HMTNB |
| 1307 | 2230-84u.HMTNB |
| 1308 | 2230-93u.HMTNB |
| 1309 | 2231-23u.HMTNB |
| 1310 | 2231-26u.HMTNB |
| 1311 | 2231-32u.HMTNB |
| 1312 | 2231-37u.HMTNB |
| 1313 | 2231-44u.HMTNB |
| 1314 | 2231-50u.HMTNB |
| 1315 | 2231-51u.HMTNB |
| 1316 | 2231-63u.HMTNB |
| 1317 | 2231-68u.HMTNB |
| 1318 | 2231-74u.HMTNB |
| 1319 | 2231-82u.HMTNB |
| 1320 | 2231-85u.HMTNB |
| 1321 | 2231-88u.HMTNB |
| 1322 | 2231-94u.HMTNB |
| 1323 | 2231-95u.HMTNB |
| 1324 | 2232-03u.HMTNB |
| 1325 | 2232-11u.HMTNB |
| 1326 | 2232-19u.HMTNB |
| 1327 | 2232-25u.HMTNB |
| 1328 | 2232-30u.HMTNB |
| 1329 | 2232-44u.HMTNB |
| 1330 | 2232-50u.HMTNB |
| 1331 | 2232-56u.HMTNB |
| 1332 | 2232-60u.HMTNB |
| 1333 | 2232-64u.HMTNB |
| 1334 | 2232-71u.HMTNB |
| 1335 | 2232-73u.HMTNB |
| 1336 | 2232-80u.HMTNB |
| 1337 | 2232-83u.HMTNB |
| 1338 | 2233-02u.HMTNB |
| 1339 | 2233-53u.HMTNB |
| 1340 | 2233-57u.HMTNB |
| 1341 | 2233-58u.HMTNB |
| 1342 | 2233-80u.HMTNB |
| 1343 | 2233-81u.HMTNB |
| 1344 | 2233-83u.HMTNB |
| 1345 | 2234-02u.HMTNB |
| 1346 | 2234-03u.HMTNB |
| 1347 | 2234-05u.HMTNB |
| 1348 | 2234-06u.HMTNB |
| 1349 | 2234-09u.HMTNB |
| 1350 | 2234-12u.HMTNB |
| 1351 | 2234-23u.HMTNB |
| 1352 | 2234-26u.HMTNB |

Table IV (cont'd)

| SEQ ID NO: | Name |
|---|---|
| 1353 | 2234-46u.HMTNB |
| 1354 | 2234-66u.HMTNB |
| 1355 | 2234-67u.HMTNB |
| 1356 | 2234-70u.HMTNB |
| 1357 | 2234-74u.HMTNB |
| 1358 | 2234-77u.HMTNB |
| 1359 | 2234-82u.HMTNB |
| 1360 | 2234-88u.HMTNB |
| 1361 | 2234-89u.HMTNB |
| 1362 | 2234-90u.HMTNB |
| 1363 | 2234-93u.HMTNB |
| 1364 | 2240-39 |
| 1365 | 2240-40 |
| 1366 | 2240-49 |
| 1367 | 2240-51 |
| 1368 | 2240-57 |
| 1369 | 2240-61 |
| 1370 | 2240-62 |
| 1371 | 2241-2 |
| 1372 | 2241-3 |
| 1373 | 2241-8 |
| 1374 | 2241-9 |
| 1375 | 2241-13 |
| 1376 | 2241-21 |
| 1377 | 2241-29 |
| 1378 | 2241-38 |
| 1379 | 2241-45 |
| 1380 | 2241-49 |
| 1381 | 2241-51 |
| 1382 | 2241-57 |
| 1383 | 2241-63 |
| 1384 | 2241-68 |
| 1385 | 2241-89 |
| 1386 | 2241-91 |
| 1387 | 2243-2 |
| 1388 | 2243-3 |
| 1389 | 2243-12 |
| 1390 | 2243-14 |
| 1391 | 2243-19 |
| 1392 | 2243-24 |
| 1393 | 2243-25 |
| 1394 | 2243-33 |
| 1395 | 2243-49 |
| 1396 | 2243-50 |
| 1397 | 2243-51 |
| 1398 | 2243-59 |
| 1399 | 2243-63 |
| 1400 | 2243-69 |
| 1401 | 2243-74 |
| 1402 | 2243-75 |
| 1403 | 2243-77 |
| 1404 | 2244-19 |
| 1405 | 2244-26 |
| 1406 | 2244-35 |
| 1407 | 2244-38 |
| 1408 | 2244-40 |
| 1409 | 2244-47 |
| 1410 | 2244-52 |
| 1411 | 2244-57 |
| 1412 | 2244-63 |
| 1413 | 2244-68 |
| 1414 | 2244-77 |
| 1415 | 2244-80 |

In one embodiment, a gene or other nucleic acid molecule of the present invention can be an allelic variant selected from the group consisting of SEQ ID NO:1872, SEQ ID NO:1874, SEQ ID NO:1875 and SEQ ID NO:1876.

Isolated HMT and HNC proteins described herein may be encoded by nucleic acid molecules that hybridize under stringent hybridization conditions to genes or other nucleic acid molecules encoding flea HMT and HNC proteins, respectively. The minimal size of HMT and HNC proteins described is a size sufficient to be encoded by a nucleic acid molecule capable of forming a stable hybrid (i.e., hybridizing under stringent hybridization conditions) with the complementary sequence of a nucleic acid molecule encoding the corresponding natural protein. The size of a nucleic acid molecule encoding such a protein is dependent on the nucleic acid composition and the percent homology between the flea HMT or HNC nucleic acid molecule and the complementary nucleic acid sequence. It can easily be understood that the extent of homology required to form a stable hybrid under stringent conditions can vary depending on whether the homologous sequences are interspersed throughout a given nucleic acid molecule or are clustered (i.e., localized) in distinct regions on a given nucleic acid molecule.

The minimal size of a nucleic acid molecule capable of forming a stable hybrid with a gene encoding a flea HMT or HNC protein is typically at least about 12 to about 15 nucleotides in length if the nucleic acid molecule is GC-rich and at least about 15 to about 17 bases in length if it is AT-rich. The minimal size of a nucleic acid molecule used to encode an HMT or HNC protein homologue of the present invention is from about 12 to about 18 nucleotides in length. Thus, the minimal size of HMT or HNC protein homologues described herein is from about 4 to about 6 amino acids in length. There is no limit, other than a practical limit, on the maximal size of a nucleic acid molecule encoding a flea HMT or HNC protein of the present invention because a nucleic acid molecule as described herein can include a portion of a gene, an entire gene, or multiple genes. The preferred size of a protein encoded by a nucleic acid molecule as described herein depends on whether a full-length, fusion, multivalent, or functional portion of such a protein is desired.

Stringent hybridization conditions are determined based on defined physical properties of the gene to which the nucleic acid molecule is being hybridized, and can be defined mathematically. Stringent hybridization conditions are those experimental parameters that allow an individual skilled in the art to identify significant similarities between heterologous nucleic acid molecules. These conditions are well known to those skilled in the art. See, for example, Sambrook, et al., 1989, Molecular Cloning: A Laboratory Manual, Cold Spring Harbor Labs Press, and Meinkoth, et al., 1984, Anal. Biochem. 138, 267-284. As explained in detail in the cited references, the determination of hybridization conditions involves the manipulation of a set of variables including the ionic strength (M, in moles/liter), the hybridization temperature (°C), the concentration of nucleic acid helix destabilizing agents (such as formamide), the average length of the shortest hybrid duplex (n), and the percent G + C composition of the fragment to which an unknown nucleic acid molecule is being hybridized. For nucleic acid molecules of at least about 150 nucleotides, these variables are inserted into a standard mathematical formula to calculate the melting temperature, or $T_m$, of a given nucleic acid molecule. As defined in the formula below, $T_m$ is the temperature at which two complementary nucleic acid molecule strands will disassociate, assuming 100% complementarity between the two strands:

$$T_m = 81.5°C + 16.6 \log M + 0.41(\%G + C) - 500/n - 0.61(\%formamide).$$

For nucleic acid molecules smaller than about 50 nucleotides, hybrid stability is defined by the dissociation temperature ($T_d$), which is defined as the temperature at which 50% of the duplexes dissociate. For these smaller molecules, the stability at a standard ionic strength is defined by the following equation:

$$T_d = 4(G + C) + 2(A + T).$$

A temperature of 5°C below $T_d$ is used to detect hybridization between perfectly matched molecules.

Also well known to those skilled in the art is how base pair mismatch, i.e. differences between two nucleic acid molecules being compared, including non-complementarity of bases at a given location, and gaps due to insertion or deletion of one or more bases at a given location on either of the nucleic acid molecules being compared, will affect $T_m$ or $T_d$ for nucleic acid molecules of different sizes. For example, $T_m$ decreases about 1°C for each 1% of mismatched base pairs for hybrids greater than about 150 bp, and $T_d$ decreases about 5°C for each mismatched base pair for hybrids below about 50 bp. Conditions for hybrids between about 50 and about 150 base pairs can be determined empirically and without undue experimentation using standard laboratory procedures well known to those skilled in the art. These simple procedures allow one skilled in the art to set the hybridization conditions (by altering, for example, the salt concentration, the formamide concentration or the temperature) so that only nucleic acid hybrids with greater than a specified % base pair mismatch will hybridize. Stringent hybridization conditions are commonly understood by those skilled in the art to be those experimental conditions that will allow less than or equal to about 30% base pair mismatch (i.e., at least about 70% identity). Because one skilled in the art can easily determine whether a given nucleic acid molecule to be tested is less than or greater than about 50 nucleotides, and can therefore choose the appropriate formula for determining hybridization conditions, he or she can determine whether the nucleic acid molecule will hybridize with a given gene under stringent hybridization conditions and similarly whether the nucleic acid molecule will hybridize under conditions designed to allow a desired amount of base pair mismatch.

Hybridization reactions are often carried out by attaching the nucleic acid molecule to be hybridized to a solid support such as a membrane, and then hybridizing with a labeled nucleic acid molecule, typically referred to as a probe, suspended in a hybridization solution. Examples of common hybridization reaction techniques include, but are not limited to, the well-known Southern and northern blotting procedures. Typically, the actual hybridization reaction is done under non-stringent conditions, i.e., at a lower temperature and/or a higher salt concentration, and then high stringency is achieved by washing the membrane in a solution with a higher temperature and/or lower salt concentration in order to achieve the desired stringency.

For example, if the skilled artisan wished to identify a nucleic acid molecule that hybridizes under conditions that would allow less than or equal to 30% pair mismatch with a flea nucleic acid molecule of about 150 bp in length or greater, the following conditions could preferably be used. The average G + C content of flea DNA is about 37%, as calculated from known flea nucleic acid sequences. The unknown nucleic acid molecules would be attached to a support membrane, and the 150 bp probe would be labeled, e.g. with a radioactive tag. The hybridization reaction could be carried out in a solution comprising 2X SSC and 0% formamide, at a temperature of about 37°C (low stringency conditions). Solutions of differing concentrations of SSC can be made by one of skill in the art by diluting a stock solution of 20X SSC (175.3 gram NaCl and about 88.2 gram sodium citrate in 1 liter of water, pH 7) to obtain the desired concentration of SSC. The skilled artisan would calculate the washing conditions required to allow up to 30% base pair mismatch. For example, in a wash solution comprising 1X SSC and 0% formamide, the $T_m$ of perfect hybrids would be about 77°C:

$$81.5°C + 16.6 \log(.15M) + (0.41 \times 0.37) - (500/150) - (0.61 \times 0) = 77.5°C.$$

Thus, to achieve hybridization with nucleic acid molecules having about 30% base pair mismatch, hybridization washes would be carried out at a temperature of less than or equal to 47.5°C. It is thus within the skill of one in the art to calculate additional hybridization temperatures based on the desired percentage base pair mismatch, formulae and G/C content disclosed herein. For example, it is appreciated by one skilled in the art that as the nucleic acid molecule to be tested for hybridization against nucleic acid molecules of the present invention having sequences specified herein becomes longer than 150 nucleotides, the $T_m$ for a hybridization reaction allowing up to 30% base pair mismatch will not vary significantly from 47.5°C.

Furthermore, it is known in the art that there are commercially available computer programs for determining the degree of similarity between two nucleic acid sequences. These computer programs include various known methods to determine the percentage identity and the number and length of gaps between hybrid nucleic acid molecules. Preferred methods to determine the percent identity among amino acid sequences and also among nucleic acid sequences include analysis using one or more of the commercially available computer programs designed to compare and analyze nucleic acid or amino acid sequences. These computer programs include, but are not limited to, the Wisconsin Package Version 9.0 sequence analysis software, available from Genetics Computer Group (GCG™), Madison, WI, DNAsis™, available from Hitachi Software, San Bruno, CA, and Mac Vector™, available from the Eastman Kodak Company, New Haven, CT. A preferred method to determine percent identity among amino acid sequences and also among nucleic acid sequences includes using the GAP program with pair-wise comparisons within the GCG™ Wisconsin Package Version 9.0 sequence analysis software, hereinafter referred to as default parameters.

Flea ALN, CBP, NKAB, LGIC, ANON, MALV, OS-D, NMDA CLBP NAH, CLIC, PL2, PL3, PL4, SVP, VGCC, AUP, and 7B2 proteins are described. A preferred flea ALN protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:3 and SEQ ID NO:6.

A preferred flea CBP protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:9 and SEQ ID NO: 12.

A preferred flea NKAB protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:15 and SEQ ID NO:18.

A preferred flea LGIC protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:21 and SEQ ID NO:24, SEQ ID NO:1860, SEQ ID NO: 1863, and SEQ ID NO:1866.

A preferred flea ANON protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:27 and SEQ ID NO:30.

A preferred flea MALV protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:33 and SEQ ID NO:36.

A preferred flea OS-D protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:39 and SEQ ID NO:42.

A preferred flea NMDA protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:45 and SEQ ID NO:48.

A preferred flea CLBP protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:155, SEQ m NO:158, SEQ ID NO:161, SEQ ID NO: 164, SEQ ID NO:167 and SEQ ID NO:170.

A preferred flea NAH protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1869 and SEQ ID NO:1871.

A preferred flea CLIC protein of the present invention includes a protein encoded by a nucleic acid molecule that hybridizes under conditions that preferably allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1874 and SEQ ID NO: 1876.

A preferred flea PL2 protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1879, SEQ ID NO:1881, SEQ ID NO:1884, and SEQ ID NO:1886.

A preferred flea CPL3 protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1889 and SEQ ID NO:1891.

A preferred flea PL4 protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO: 1893, SEQ ID NO:1895, SEQ ID NO:1898, and SEQ ID NO:1900.

A preferred flea SVP protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO: 1903 and SEQ ID NO:1905.

A preferred flea VGCC protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1907, SEQ ID NO:1909, SEQ ID NO:1911, SEQ ID NO:1913, SEQ ID NO:1916, and SEQ ID NO:1918.

A preferred flea AUP protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1921 and SEQ ID NO:1923.

A preferred flea 7B2 protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO: 1926, SEQ ID NO:1928, and SEQ ID NO:1931.

A preferred flea HMT and/or HNC protein includes a protein encoded by a nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of a nucleic acid sequence complementary to a nucleic acid sequence of Table I, Table II, Table III and/or Table IV.

Also described is a flea ALN protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:3 and SEQ ID NO:6.

Also described is a flea CBP protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:9 and SEQ ID NO:13.

Also described is a flea NKAB protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO: 15 and SEQ ID NO: 18.

Also described is a flea LGIC protein encoded by a nucleic acid molecule that hybridizes under conditions comprising (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:21, SEQ ID NO:24, SEQ ID NO:1860, SEQ ID NO: 1863, and SEQ ID NO:1866.

Also described is a flea ANON protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:27 and SEQ ID NO:30.

Also described is a flea MALV protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:33 and SEQ ID NO:36.

Also described is a flea OS-D protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:39 and SEQ ID NO:42.

Also described is a flea NMDA protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:45 and SEQ ID NO:48.

Also described is a flea CLBP protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:155, SEQ ID NO:158, SEQ ID NO:161, SEQ ID NO: 164, SEQ ID NO:167 and SEQ ID NO:170.

Also described is a flea NAH protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1869 and SEQ ID NO:1871.

Also described is a flea CLIC protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1874 and SEQ ID NO:1876.

Also described is a flea PL2 protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1879, SEQ ID NO:1881, SEQ ID NO:1884 and SEQ ID NO:1886.

Also described is a flea PL3 protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1889 and SEQ ID NO:1891.

Also described is a flea PL4 protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1893, SEQ ID NO:1895, SEQ ID NO:1898, and SEQ ID NO:1900.

Also described is a flea SVP protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1903 and SEQ ID NO:

Also described is a flea VGCC protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1907, SEQ ID NO:1909, SEQ ID NO:1911, SEQ ID NO:1913, SEQ ID NO:1916, and SEQ ID NO:1918.

Also described is a flea AUP protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1921 and SEQ m NO: 1923.

Also described is a flea 7B2 protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of SEQ ID NO:1926, SEQ ID NO:1928, and SEQ ID NO:1931.

Also described is a flea HMT and/or HNC protein encoded by a nucleic acid molecule that hybridizes under conditions comprising, (a) hybridizing in a solution comprising 1X SSC and 0% formamide, at a temperature of about 37°C and (b) washing in a solution comprising 1X SSC and 0% formamide, at a temperature of about 47.5°C, to an isolated nucleic acid molecule selected from the group consisting of a nucleic acid sequence complementary to a nucleic acid sequence of Table I, Table II, Table III and/or Table IV.

Another preferred flea ALN protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1 and/or SEQ ID NO:4; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea CBP protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:7 and/or SEQ m NO:10; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea NKAB protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:13 and/or SEQ ID NO:16; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea LGIC protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:19, SEQ ID NO:22, SEQ ID NO:1861, and/or SEQ ID NO:1864; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea ANON protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:25 and/or SEQ ID NO:28; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea MALV protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:31 and/or SEQ ID NO:34; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea OS-D protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:37 and/or SEQ ID NO:40; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea NMDA protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ ID NO:43 and/or SEQ ID NO:46; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea CLBP protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having the nucleic acid sequence SEQ m NO:153, SEQ ID NO:156, SEQ ID NO:159, SEQ ID NO:162, SEQ ID NO:165 and/or SEQ ID NO:168; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea NAH protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1867 and/or SEQ ID NO:1870; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea CLIC protein of the present invention includes a protein that is encoded by a nucleic acid molecule that is preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1872 and/or SEQ ID NO:1875; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea PL2 protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1877, SEQ ID NO: 1878, SEQ ID NO:1880, SEQ ID NO:1882, and/or SEQ ID NO:1885; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea PL3 protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1887 and/or SEQ ID NO:1890; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea PL4 protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1892, SEQ ID NO: 1894, SEQ ID NO:1896 and/or SEQ ID NO:1899; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea SVP protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1901 and/or SEQ ID NO:1904; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea VGCC protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1906, SEQ ID NO:1908, SEQ ID NO:1910, SEQ ID NO:1912, SEQ ID NO:1914 and/or SEQ ID NO:1917; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea AUP protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1919 and/or SEQ m NO:1922; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea 7B2 protein includes a protein that is encoded by a nucleic acid molecule that is preferably at least about 70% identical, more preferably at least about 75% identical, more preferably at least about 80% identical, more preferably about at least 85% identical, more preferably about at least 90% identical, and even more preferably about at least 95% identical to a nucleic acid molecule having the nucleic acid sequence SEQ ID NO:1924, SEQ ID NO: 1927 and/or SEQ ID NO:1929; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Another preferred flea HMT and/or HNC protein includes a protein that is encoded by a nucleic acid molecule that is preferably at least about 70% identical, more preferably at least about 75% identical, more preferably at least about 80% identical, more preferably at least about 85% identical, more preferably at least about 90% identical, and even more preferably at least about 95% identical to a nucleic acid molecule having a nucleic acid sequence of Table I, Table II, Table III and/or Table IV; also preferred are fragments (i.e. portions) of such proteins encoded by nucleic acid molecules that are at least about 18 nucleotides. Percent identity as used herein is determined using the Compare function by maximum matching within the program DNAsis Version 2.1 using default parameters.

Additional preferred flea ALN proteins include proteins having the amino acid sequence SEQ ID NO:2 or SEQ ID NO:5, and proteins comprising homologues of a protein having the amino acid sequence SEQ ID NO:2 or SEQ ID NO:5, wherein such a homologue comprises at least one epitope that elicits an immune response against a protein having an amino acid sequence SEQ ID NO:2 or SEQ ID NO:5. Likewise, also preferred are proteins encoded by nucleic acid molecules comprising nucleic acid sequence SEQ ID NO:1 and/or SEQ ID NO:4, or by homologues thereof.

Additional preferred flea CBP proteins include proteins having the amino acid sequence SEQ m NO:8 or SEQ ID NO:11, and proteins comprising homologues of a protein having the amino acid sequence SEQ ID NO:8 or SEQ m NO:11, wherein such a homologue comprises at least one epitope that elicits an immune response against a protein having an amino acid sequence SEQ ID NO:8 or SEQ ID NO:11. Likewise, also preferred are proteins encoded by nucleic acid molecules comprising nucleic acid sequence SEQ ID NO:7 and/or SEQ ID NO:10, or by homologues thereof.

Additional preferred flea NKAB proteins include proteins having the amino acid sequence SEQ ID NO:14 or SEQ ID NO:17; and proteins comprising homologues of a protein having the amino acid sequence SEQ ID NO:14 or SEQ ID NO:17, wherein such a homologue comprises at least one epitope that elicits an immune response against a protein having an amino acid sequence SEQ ID NO:14 or SEQ ID NO:17. Likewise, also preferred are proteins encoded by nucleic acid molecules comprising nucleic acid sequence SEQ ID NO:13 and/or SEQ ID NO:16, or by homologues thereof.

Additional preferred flea LGIC proteins include proteins having the amino acid sequence SEQ ID NO:20, SEQ ID NO:23 or SEQ ID NO:1862, and proteins comprising homologues of a protein having the amino acid sequence SEQ ID NO:20, SEQ ID NO:23 or SEQ ID NO:1862, wherein such a homologue comprises at least one epitope that elicits an immune response against a protein having an amino acid sequence SEQ ID NO:20, SEQ ID NO:23 or SEQ ID NO: 1862. Likewise, also preferred are proteins encoded by nucleic acid molecules comprising nucleic acid sequence SEQ ID NO:19, SEQ ID NO:22, SEQ ID NO:1859, SEQ ID NO:1861 and/or SEQ ID NO:1864 or by homologues thereof.

Additional preferred flea ANON proteins include proteins having the amino acid sequence SEQ ID NO:26 or SEQ ID NO:29, and proteins comprising homologues of a protein having the amino acid sequence SEQ ID NO:26 or SEQ ID NO:29, wherein such a homologue comprises at least one epitope that elicits an immune response against a protein having an amino acid sequence SEQ ID NO:26 or SEQ ID NO:29. Likewise, also preferred are proteins encoded by nucleic acid molecules comprising nucleic acid sequence SEQ ID NO:25 and/or SEQ ID NO:28, or by homologues thereof.

Additional preferred flea MALV proteins include proteins having the amino acid sequence SEQ ID NO:32 or SEQ ID NO:35, and proteins comprising homologues of a protein having the amino acid sequence SEQ ID NO:32 or SEQ ID NO:35, wherein such a homologue comprises at least one epitope that elicits an immune response against a protein having an amino acid sequence SEQ ID NO:32 or SEQ ID NO:35. Likewise, also preferred are proteins encoded by nucleic acid molecules comprising nucleic acid sequence SEQ ID NO:31 and/or SEQ ID NO:34, or by homologues thereof.

Additional preferred flea OS-D proteins include proteins having the amino acid sequence SEQ ID NO:38 or SEQ ID NO:41, and proteins comprising homologues of a protein having the amino acid sequence SEQ ID NO:38 or SEQ ID NO:41, wherein such a homologue comprises at least one epitope that elicits an immune response against a protein having an amino acid sequence SEQ ID NO:38 or SEQ ID NO:41. Likewise, also preferred are proteins encoded by nucleic acid molecules comprising nucleic acid sequence SEQ ID NO:37 and/or SEQ ID NO:40, or by homologues thereof.

Additional preferred flea NMDA proteins include proteins having the amino acid sequence SEQ ID NO:44 or SEQ ID NO:47, and proteins comprising homologues of a protein having the amino acid sequence SEQ ID NO:44 or SEQ ID NO:47, wherein such a homologue comprises at least one epitope that elicits an immune response against a protein having an amino acid sequence SEQ ID NO:44 or SEQ ID NO:47. Likewise, also preferred are proteins encoded by nucleic acid molecules comprising nucleic acid sequence SEQ ID NO:43 and/or SEQ ID NO:46, or by homologues thereof.

Additional preferred flea CLBP proteins include proteins having the amino acid sequence SEQ ID NO:154, SEQ ID NO:157, SEQ ID NO: 160, SEQ ID NO:163, SEQ ID NO:166 or SEQ ID NO:169, and proteins comprising homologues of a protein having the amino acid sequence SEQ ID NO:154, SEQ ID NO:157, SEQ ID NO:160, SEQ ID NO:163, SEQ ID NO:166 or SEQ ID NO:169, wherein such a homologue comprises at least one epitope that elicits an immune response against a protein having an amino acid sequence SEQ ID NO:154, SEQ ID NO:157, SEQ ID NO:160, SEQ ID NO:163, SEQ ID NO:166 or SEQ ID NO:169. Likewise, also preferred are proteins encoded by nucleic acid molecules comprising nucleic acid sequence SEQ ID NO:153, SEQ ID NO:156, SEQ ID NO:159, SEQ ID NO:162, SEQ ID NO:165 and/or SEQ ID NO:168, or by homologues thereof.

Additional preferred flea NAH proteins include proteins having the amino acid sequence SEQ ID NO:1868, and proteins comprising homologues of a protein having the amino acid sequence SEQ ID NO:1868, wherein such a homologue comprises at least one epitope that elicits an immune response against a protein having an amino acid sequence SEQ ID NO:1868. Likewise, also preferred are proteins encoded by nucleic acid molecules comprising nucleic acid sequence SEQ ID NO:1867 and/or SEQ ID NO:1870, or by homologues thereof.

Additional preferred flea CLIC proteins of the present invention include proteins having the amino acid sequence SEQ ID NO:1873, and proteins comprising homologues of a protein having the amino acid sequence SEQ ID NO:1873, wherein such a homologue comprises at least one epitope that elicits an immune response against a protein having an amino acid sequence SEQ ID NO:1873. Likewise, also preferred are proteins encoded by nucleic acid molecules comprising nucleic acid sequence SEQ ID NO:1872 and/or SEQ ID NO:1875, or by homologues thereof.

Additional preferred flea PL2 proteins include proteins having the amino acid sequence SEQ ID NO:1883, and proteins comprising homologues of a protein having the amino acid sequence SEQ ID NO:1883, wherein such a homologue comprises at least one epitope that elicits an immune response against a protein having an amino acid sequence SEQ ID NO:1883. Likewise, also preferred are proteins encoded by nucleic acid molecules comprising nucleic acid sequence SEQ ID NO:1877, SEQ ID NO:1878, SEQ ID NO:1880, SEQ ID NO:1882 and/or SEQ ID NO:1885, or by homologues thereof.

Additional preferred flea PL3 proteins include proteins having the amino acid sequence SEQ ID NO:1888, and proteins comprising homologues of a protein having the amino acid sequence SEQ ID NO:1888, wherein such a homologue comprises at least one epitope that elicits an immune response against a protein having an amino acid sequence SEQ ID NO:1888. Likewise, also preferred are proteins encoded by nucleic acid molecules comprising nucleic acid sequence SEQ ID NO:1887 and/or SEQ ID NO:1890, or by homologues thereof.

Additional preferred flea PL4 proteins include proteins having the amino acid sequence SEQ ID NO:1897, and proteins comprising homologues of a protein having the amino acid sequence SEQ ID NO:1897, wherein such a homologue comprises at least one epitope that elicits an immune response against a protein having an amino acid sequence SEQ ID NO:1897. Likewise, also preferred are proteins encoded by nucleic acid molecules comprising nucleic acid sequence SEQ ID NO:1892, SEQ ID NO:1894, SEQ ID NO:1896 and/or SEQ ID NO:1899, or by homologues thereof.

Additional preferred flea SVP proteins include proteins having the amino acid sequence SEQ ID NO:1902, and proteins comprising homologues of a protein having the amino acid sequence SEQ ID NO:1902, wherein such a homologue comprises at least one epitope that elicits an immune response against a protein having an amino acid sequence SEQ ID NO:1902. Likewise, also preferred are proteins encoded by nucleic acid molecules comprising nucleic acid sequence SEQ ID NO:1901 and/or SEQ ID NO:1904, or by homologues thereof.

Additional preferred flea VGCC proteins include proteins having the amino acid sequence SEQ m NO:1915, and proteins comprising homologues of a protein having the amino acid sequence SEQ ID NO:1915, wherein such a homologue comprises at least one epitope that elicits an immune response against a protein having an amino acid sequence SEQ ID NO:1915. Likewise, also preferred are proteins encoded by nucleic acid molecules comprising nucleic acid sequence SEQ ID NO:1906, SEQ ID NO:1908, SEQ ID NO:1910, SEQ ID NO:1912, SEQ ID NO:1914 and/or SEQ ID NO:1917, or by homologues thereof.

Additional preferred flea AUP proteins include proteins having the amino acid sequence SEQ ID NO:1920, and proteins comprising homologues of a protein having the amino acid sequence SEQ ID NO:1920, wherein such a homologue comprises at least one epitope that elicits an immune response against a protein having an amino acid sequence SEQ ID NO:1920. Likewise, also preferred are proteins encoded by nucleic acid molecules comprising nucleic acid sequence SEQ ID NO:1919 and/or SEQ ID NO:1922, or by homologues thereof.

Additional preferred flea 7B2 proteins include proteins having the amino acid sequence SEQ ID NO:1925 or SEQ ID NO:1930, and proteins comprising homologues of a protein having the amino acid sequence SEQ ID NO:1925 or SEQ ID NO:1930, wherein such a homologue comprises at least one epitope that elicits an immune response against a protein having an amino acid sequence SEQ ID NO:1925 or SEQ ID NO:1930. Likewise, also preferred are proteins encoded by nucleic acid molecules comprising nucleic acid sequence SEQ ID NO:1924, SEQ ID NO:1927 and/or SEQ ID NO:1929, or by homologues thereof.

Additional preferred flea HMT and/or HNC proteins include proteins having an amino acid sequence encoded by a nucleic acid sequence of Table I, Table II, Table III and/or Table IV, and proteins comprising homologues of a protein encoded by a nucleic acid sequence of Table I, Table II, Table III and/or Table IV, wherein such a homologue comprises at least one epitope that elicits an immune response against a protein encoded by a nucleic acid sequence of Table I, Table II, Table III and/or Table IV.

A preferred isolated protein is a protein encoded by at least one of the following nucleic acid molecules: $nCfALN_{2057}$, $nCfALN_{1152}$, $nCfCBP_{1128}$, $nCfCBP_{816}$, $nCtNKAB_{1714}$, $nCfNKAB_{978}$, $nCfLGIC_{2240}$, $nCfLGIC_{1707}$, $nCfANON_{1429}$, $nCfANON_{1194}$, $nCfMALV_{765}$, $nCfMALV_{762}$, $nCfDSD_{604}$, $nCfOSD_{405}$, $nCfNMDA_{1227}$, $nCfNMDA_{738}$, $nCfCLBP1A_{633}$, $nCfCLBP1A_{441}$, $nCfCLBP2A_{631}$, $nCfCLBP2A_{441}$, $nCfLGIC_{2739}$, $nCfLGIC_{2016}$, $nCfNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPL4_{1043}$, $nCfPL4_{1062}$, $nCfPL4_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7B2_{801}$, $nCf7B2_{741}$, or allelic variants of any of these nucleic acid molecules. A preferred isolated protein according to the invention is encoded by a nucleic acid molecule having nucleic acid sequence SEQ ID NO:1872, SEQ ID NO:1875, or a protein encoded by an allelic variant of any of these listed nucleic acid molecules.

Also described are proteins that are at least about 70%, preferably at least about 80%, more preferably at least about 85%, even more preferably at least about 90%, even more preferably at least about 95%, and even more preferably about 100% identical to $PCfALN_{384}$, $PCfCBP_{272}$, $PCfCBP_{272}$, $PCfLGIC_{569}$, $PCfANON_{398}$, $PCfMALV_{254}$, $PCfOSD_{135}$, $PCfNMDA_{246}$, $PCfCLBP1A_{147}$ or $PCfCLBP2A_{147}$. Additionally preferred are proteins encoded by allelic variants of a nucleic acid molecules encoding proteins $PCfALN_{384}$, $PCfCBP_{272}$, $PCfNKAB_{326}$, $PCfLGIC_{569}$, $PCfANON_{398}$, $PCfMALV_{254}$, $PCfOSD_{135}$, $PCfNMDA_{246}$, $PCfCLBP1A_{147}$, $PCfCLBP2A_{147}$, $PCfLGIC_{672}$, $PCfNAH_{608}$, $PCtCLIC_{262}$, $PCfPL_{391}$, $PCfPL3_{81}$, $PCfPL4_{285}$, $PCfSVP_{530}$, $PCfVGCC_{851}$, $PCfAUP_{102}$, $PCf7B2_{267}$, $PCf7B2_{247}$. Also preferred are fragments thereof having at least about 6 amino acid residues.

Other preferred HMT and HNC proteins of the present invention include proteins having amino acid sequences that are at least about 75%, preferably at least about 80%, more preferably at least about 85%, even more preferably at least about 90%, even more preferably at least about 95%, and even more preferably about 100% identical to amino acid sequence; and proteins encoded by allelic variants of nucleic acid molecules encoding HMT and HNC proteins having amino acid sequences SEQ ID NO: 1873. Also preferred are fragments thereof having at least about 6 amino acid residues.

Also described are *C. felis* HMT and HNC proteins which comprise amino acid sequence SEQ ID NO:2, SEQ ID NO:8, SEQ ID NO:14, SEQ ID NO:20, SEQ ID NO:26, SEQ ID NO:32, SEQ ID NO:38, SEQ ID NO:44, SEQ ID NO: 154, SEQ ID NO:160, SEQ ID NO:163, SEQ ID NO:169, SEQ ID NO:1862, SEQ ID NO:1868, SEQ ID NO:1873, SEQ ID NO:1879, SEQ ID NO:1883, SEQ ID NO:1888, SEQ ID NO:1897, SEQ ID NO:1902, SEQ ID NO:1915, SEQ ID NO: 1920, SEQ ID NO:1925, and/or SEQ ID NO:1930 (including, but not limited to, the proteins consisting of amino acid sequence SEQ ID NO:2, SEQ ID NO:8, SEQ ID NO:14, SEQ ID NO:20, SEQ NO:26, SEQ ID NO:32, SEQ ID NO:38, SEQ ID NO:44, SEQ ID NO:154, SEQ ID NO:160, SEQ ID NO:163, SEQ ID NO:169, SEQ ID NO:1862, SEQ ID N0: 1868, SEQ ID NO:1873, SEQ ID NO:1879, SEQ ID NO:1883, SEQ ID NO:1888, SEQ ID NO:1897, SEQ ID NO:1902, SEQ ID NO:1915, SEQ ID NO:1920, SEQ ID NO:1925, and/or SEQ ID NO:1930, fusion proteins and multivalent proteins), and proteins encoded by allelic variants of nucleic acid molecules encoding proteins having amino acid sequence SEQ ID NO:2, SEQ ID NO:8, SEQ ID NO:14, SEQ ID NO:20, SEQ ID NO:26, SEQ ID NO:32, SEQ ID NO:38, SEQ ID NO: 44, SEQ ID NO:154, SEQ ID NO:160, SEQ ID NO:163, SEQ ID NO:169, SEQ ID NO: 1862, SEQ ID NO:1868, SEQ ID NO:1873, SEQ ID NO:1879, SEQ ID NO:1883, SEQ ID NO:1888, SEQ ID NO:1897, SEQ ID NO:1902, SEQ ID NO: 1915, SEQ ID NO:1920, SEQ ID NO:1925, and/or SEQ ID NO:1930.

A preferred flea HMT or HNC protein comprises an amino acid sequence of at least about 35 amino acids, preferably at least about 50 amino acids, more preferably at least about 100 amino acids, more preferably at least about 200 amino acids, more preferably at least about 250 amino acids, more preferably at least about 300 amino acids, more preferably at least about 350 amino acids, more preferably at least about 400 amino acids, more preferably at least about 450 amino acids, more preferably at least about 500 amino acids, even more preferably at least about 550 amino acids, and even more preferably at least about 575 amino acids. In another embodiment, preferred flea HMT and HNC proteins comprise full-length proteins, i.e., proteins encoded by full-length coding regions, or post-translationally modified proteins thereof, such as mature proteins from which initiating methionine and/or signal sequences or "pro" sequences have been removed.

A fragment of an HMT and/or HNC protein may comprise at least about 5 amino acids, more preferably at least about 10 amino acids, more preferably at least about 15 amino acids, more preferably at least about 20 amino acids, more preferably at least about 25 amino acids, more preferably at least about 30 amino acids, more preferably at least about 35 amino acids, more preferably at least about 40 amino acids, more preferably at least about 45 amino acids, more preferably at least about 50 amino acids, more preferably at least about 55 amino acids, more preferably at least about 60 amino acids, more preferably at least about 65 amino acids, more preferably at least about 70 amino acids, more preferably at least about 75 amino acids, more preferably at least about 80 amino acids, more preferably at least about 85 amino acids, more preferably at least about 90 amino acids, more preferably at least about 95 amino acids, and even more preferably at least about 100 amino acids in length.

Additional preferred HMT and HNC proteins include proteins encoded by nucleic acid molecules comprising at least a portion of $nCfALN_{2057}$, $nCfALN_{1152}$, $nCfCBP_{1128}$, $nCfCBP_{816}$, $nCfNKAB_{1714}$, $nCfNKAB_{978}$, $nCtLGIC_{2240}$, $nCfLGIC_{1707}$, $nCfANON_{1429}$, $nCfANON_{1194}$, $nCfMALV_{765}$, $nCfMALV_{762}$, $nCfOSD_{604}$, $nCfDSD_{405}$, $nCfNMDA_{1227}$, $nCfNMDA_{738}$, $nCfCLBP1A_{633}$, $nCfCLBP1A_{441}$, $nCfCLBP2A_{631}$, $nCfCLBP2A_{441}$, $nCfLGIC_{2739}$, $nCfLGIC_{2016}$, $nCfNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPLA_{1043}$, $nCfPL4_{1062}$, $nCfPLA_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7B2_{801}$, $nCf7B2_{741}$ as well as HMT and HNC proteins encoded by allelic variants of such nucleic acid molecules. A portion of such HMT and HNC nucleic acid molecule is preferably at least 18 nucleotides in length.

Also preferred are HMT and HNC proteins encoded by nucleic acid molecules having nucleic acid sequences comprising at least a portion of SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO: 37, SEQ ID NO:40, SEQ ID NO:43, SEQ ID NO:46, SEQ ID NO:153, SEQ ID NO:156, SEQ ID NO:159, SEQ ID NO: 162, SEQ ID NO:165, SEQ ID NO:168, SEQ ID NO:1859, SEQ ID NO:1861, SEQ ID NO:1864, SEQ ID NO:1867, SEQ ID NO:1870, SEQ ID NO:1872, SEQ ID NO:1875, SEQ ID NO:1877, SEQ ID NO:1878, SEQ ID NO:1881, SEQ ID NO: 1882, SEQ ID NO:1885, SEQ ID NO:1887, SEQ ID NO:1890, SEQ ID NO:1892, SEQ ID NO:1894, SEQ ID NO:1896, SEQ ID NO:1899, SEQ ID NO:1901, SEQ ID NO:1904, SEQ ID NO:1906, SEQ ID NO:1908, SEQ ID NO:1910, SEQ ID NO:1912, SEQ ID NO:1914, SEQ ID NO:1917, SEQ ID NO:1919, SEQ ID NO:1922, SEQ ID NO:1924, SEQ ID NO: 1927, and/or SEQ ID NO:1929, as well as allelic variants of these nucleic acid molecules. A portion of such HMT and HNC nucleic acid molecule is preferably at least 18 nucleotides in length.

Also described is preferred flea HMT and/or HNC protein encoded by a nucleic acid molecule comprising at least about 15 nucleotides, more preferably at least about 18 nucleotides, more preferably at least about 20 nucleotides, more preferably at least about 25 nucleotides, more preferably at least about 30 nucleotides, more preferably at least about 40 nucleotides, more preferably at least about 50 nucleotides, more preferably at least about 100 nucleotides, more preferably at least about 150 nucleotides, more preferably at least about 350 nucleotides, more preferably at least about 450 nucleotides, more preferably at least about 550 nucleotides, more preferably at least about 650 nucleotides, more preferably at least about 750 nucleotides, more preferably at least about 1000 nucleotides, more preferably at least about 1500 nucleotides, more preferably at least about 1750 nucleotides more preferably at least about 2000 nucleotides, and even more preferably at least about 2250 nucleotides in length. Within this example is a HMT protein encoded by at least a portion of $nCfALN_{2057}$, $nCfALN_{1152}$, $nCfCBP_{1128}$, $nCfCBP_{816}$, $nCfNKAB_{1714}$, $nCfNKAB_{978}$ $nCfLGIC_{2240}$, $nCfLGIC_{1707}$, $nCfANON_{1429}$, $nCfANON_{1194}$, $nCfMALV_{765}$, $nCfMALV_{762}$, $nCfOSD_{604}$, $nCfOSD_{405}$, $nCfNMDA_{1227}$, $nCfNMDA_{738}$, $nCfCLBP1A_{633}$, $nCfCLBP1A_{441}$, $nCfCLBP2A_{631}$, $nCfCLBP2A_{441}$, $nCfLGIC_{2739}$, $nCfLGIC_{2016}$, $nCfNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPL4_{1043}$, $nCfPL4_{1062}$, $nCfPL4_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7B2_{801}$, $nCf7B2_{741}$ or by an allelic variant of any of these nucleic acid molecules. In yet another embodiment, preferred flea HMT and HNC proteins are encoded by nucleic acid molecules comprising apparently full-length HMT or HNC coding regions respectively, i.e., nucleic acid molecules encoding an apparently full-length HMT or HNC proteins.

Preferred flea HMT and HNC proteins of the present invention can be used to develop inhibitors that, when administered to an animal in an effective manner, are capable of protecting that animal from flea infestation. In accordance with the present invention, the ability of an inhibitor to protect an animal from flea infestation refers to the ability of that protein to, for example, treat, ameliorate and/or prevent infestation caused by fleas. In particular, the phrase "to protect an animal from flea infestation" refers to reducing the potential for flea population expansion on and around the animal (i.e., reducing the flea burden). Preferably, the flea population size is decreased, optimally to an extent that the animal is no longer bothered by fleas. A host animal, as used herein, is an animal from which fleas can feed by attaching to and feeding through the skin of the animal. Fleas, and other ectoparasites, can live on a host animal for an extended period of time or can attach temporarily to an animal in order to feed. At any given time, a certain percentage of a flea population can be on a host animal whereas the remainder can be in the environment of the animal. Such an environment can include not only adult fleas, but also flea eggs and/or flea larvae. The environment can be of any size such that fleas in the environment are able to jump onto and off of a host animal. For example, the environment of an animal can include plants, such as crops, from which fleas infest an animal. As such, it is desirable not only to reduce the flea burden on an animal per se, but also to reduce the flea burden in the environment of the animal. Suitable fleas to target include any flea that is essentially incapable of causing disease in an animal administered an inhibitor. As such, fleas to target include any flea that produces a protein that can be targeted by an inhibitory compound that inhibits a flea HMT or HNC protein function, thereby resulting in the decreased ability of the parasite to cause disease in an animal. Preferred fleas to target include fleas of the following genera: *Ctenocephalides, Cyopsyllus, Diamanus (Oropsylla), Echidnophaga, Nosopsyllus, Pulex, Tunga,* and *Xenopsylla,* with those of the species *Ctenocephalides canis, Ctenocephalides felis, Diamanus montanus, Echidnophaga gallinacea, Nosopsyllus faciatus, Pulex irritans, Pulex simulans, Tunga penetrans* and *Xenopsylla cheopis* being more preferred, with *C. felis* being even more preferred. Such fleas are also preferred for the isolation of proteins or nucleic acid molecules of the present invention.

There is described a flea HMT and/or HNC protein which is a fusion protein that includes a flea HMT and/or HNC protein-containing domain attached to one or more fusion segments. Suitable fusion segments for use with the present invention include, but are not limited to, segments that can: enhance a protein's stability; act as an immunopotentiator to enhance an immune response against a flea HMT and/or HNC protein; and/or assist in purification of a flea HMT and/or HNC protein (e.g., by affinity chromatography). A suitable fusion segment can be a domain of any size that has the desired function (e.g., imparts increased stability, imparts increased immunogenicity to a protein, and/or simplifies purification of a protein). Fusion segments can be joined to amino and/or carboxyl termini of the flea HMT-containing and/or HNC-containing domain of the protein and can be susceptible to cleavage in order to enable straight-forward recovery of a flea HMT and/or HNC protein. Fusion proteins are preferably produced by culturing a recombinant cell transformed with a fusion nucleic acid molecule that encodes a protein including the fusion segment attached to either the carboxyl and/or amino terminal end of an HMT-containing and/or HNC-containing domain. Preferred fusion segments include a metal binding domain (e.g., a poly-histidine segment); an immunoglobulin binding domain (e.g., Protein A;

Protein G; T cell; B cell; Fc receptor or complement protein antibody-binding domains); a sugar binding domain (e.g., a maltose binding domain); and/or a "tag" domain (e.g., at least a portion of β-galactosidase, a strep tag peptide, a T7 tag peptide, a Flag™ peptide, or other domains that can be purified using compounds that bind to the domain, such as monoclonal antibodies). More preferred fusion segments include metal binding domains, such as a poly-histidine segment; a maltose binding domain; a strep tag peptide, such as that available from Biometra in Tampa, FL; and an S10 peptide.

Also discussed are mimetopes of flea HMT and/or HNC proteins of the present invention. As used herein, a mimetope of a flea HMT and/or HNC protein refers to any compound that is able to mimic the activity of such an HMT and/or HNC protein, often because the mimetope has a structure that mimics the particular HMT and/or HNC protein. Mimetopes can be, but are not limited to: peptides that have been modified to decrease their susceptibility to degradation such as all-D retro peptides; anti-idiotypic and/or catalytic antibodies, or fragments thereof; non-proteinaceous immunogenic portions of an isolated protein (e.g., carbohydrate structures); and synthetic or natural organic molecules, including nucleic acids. Such mimetopes can be designed using computer-generated structures of proteins described herein. Mimetopes can also be obtained by generating random samples of molecules, such as oligonucleotides, peptides or other organic molecules, and screening such samples by affinity chromatography techniques using the corresponding binding partner.

Also described is an isolated nucleic acid molecule comprising a flea HMT and/or HNC nucleic acid molecule, i.e. a nucleic acid molecule that can be isolated from a HMT cDNA library, from a HNC cDNA library, or from both libraries. As used herein, HMT and HNC nucleic acid molecules has the same meaning as HMT and/or HNC nucleic acid molecule. The identifying characteristics of such nucleic acid molecules are heretofore described. A nucleic acid molecule can include an isolated natural flea HMT and/or HNC gene or a homologue thereof, the latter of which is described in more detail below. A nucleic acid molecule as described can include one or more regulatory regions, full-length or partial coding regions, or combinations thereof. The minimal size of a nucleic acid molecule as described is a size sufficient to allow the formation of a stable hybrid (i.e., hybridization under stringent hybridization conditions) with the complementary sequence of another nucleic acid molecule. As such, the minimal size of a HMT and/or HNC nucleic acid molecule as described is from about 12 to about 18 nucleotides in length. Suitable and preferred fleas from which to isolate nucleic acid molecules are disclosed herein. Particularly preferred HMT and/or HNC nucleic acid molecules include *C. felis* HMT and/or HNC nucleic acid molecules.

In accordance herewith, an isolated nucleic acid molecule is a nucleic acid molecule that has been removed from its natural milieu (i.e., that has been subjected to human manipulation) and can include DNA, RNA, or derivatives of either DNA or RNA. As such, "isolated" does not reflect the extent to which the nucleic acid molecule has been purified. Isolated flea HMT and/or HNC nucleic acid molecules as described or homologues thereof, can be isolated from a natural source or produced using recombinant DNA technology (e.g., polymerase chain reaction (PCR) amplification or cloning) or chemical synthesis. Isolated flea HMT and/or HNC nucleic acid molecules, and homologues thereof, can include, for example, natural allelic variants and nucleic acid molecules modified by nucleotide insertions, deletions, substitutions, and/or inversions in a manner such that the modifications do not substantially interfere with the nucleic acid molecule's ability to encode a HMT and/or HNC protein as described herein

A flea HMT and/or HNC nucleic acid molecule homologue can be produced using a number of methods known to those skilled in the art, see, for example, Sambrook et al., *ibid.* For example, nucleic acid molecules can be modified using a variety of techniques including but not limited to, classic mutagenesis and recombinant DNA techniques such as site-directed mutagenesis, chemical treatment, restriction enzyme cleavage, ligation of nucleic acid fragments, PCR amplification, synthesis of oligonucleotide mixtures and ligation of mixture groups to "build" a mixture of nucleic acid molecules, and combinations thereof. Nucleic acid molecule homologues can be selected by hybridization with flea HMT and/or HNC nucleic acid molecules or by screening the function of a protein encoded by the nucleic acid molecule (e.g., ability to elicit an immune response against at least one epitope of a flea HMT or HNC protein or to effect HMT or HNC activity).

An isolated nucleic acid molecule of the present invention can include a nucleic acid sequence that encodes at least one flea HMT or HNC protein of the present invention, examples of such proteins being disclosed herein. Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a flea HMT or HNC protein.

A preferred nucleic acid molecule when administered to an animal, is capable of protecting that animal from flea infestation. As will be disclosed in more detail below, such a nucleic acid molecule can be, or encode, an antisense RNA, a molecule capable of triple helix formation, a ribozyme, or other nucleic acid-based drug compound. Additionally a nucleic acid molecule can encode a protective protein (e.g., an HMT or HNC protein of the present invention), the nucleic acid molecule being delivered to the animal, for example, by direct injection (i.e, as a genetic vaccine) or in a vehicle such as a recombinant virus vaccine or a recombinant cell vaccine.

A preferred flea HMT and/or HNC nucleic acid molecule may include an isolated nucleic acid molecule that hybridizes under conditions that preferably allow less than or equal to about 30% base pair mismatch, more preferably under conditions that allow less than or equal to about 25% base pair mismatch, more preferably under conditions that allow less than or equal to about 20% base pair mismatch, more preferably under conditions that allow less than or equal to about 15% base pair mismatch, more preferably under conditions that allow less than or equal to about 10% base pair mismatch and even more preferably under conditions that allow less than or equal to about 5% base pair mismatch with a nucleic acid molecule selected from the group consisting of SEQ ID NO:1, SEQ ID NO:3, SEQ ID NO: 4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, SEQ ID NO:19, SEQ ID NO:21, SEQ ID NO:22, SEQ ID NO:24, SEQ ID NO:25, SEQ ID NO: 27, SEQ ID NO:28, SEQ ID NO:30, SEQ ID NO:31, SEQ ID NO:33, SEQ ID NO:34, SEQ ID NO:36, SEQ ID NO:37, SEQ ID NO:39, SEQ ID NO:40, SEQ ID NO:42, SEQ ID NO:43, SEQ ID NO:45, SEQ ID NO:46, SEQ ID NO:48, SEQ ID NO:153, SEQ ID NO:155, SEQ ID NO:156, SEQ ID NO:158, SEQ ID NO:159, SEQ ID NO:161, SEQ ID NO:162, SEQ ID NO:164, SEQ ID NO:165, SEQ ID NO:167, SEQ ID NO:168, SEQ ID NO:170, SEQ ID NO:1859, SEQ ID NO: 1860, SEQ ID NO:1861, SEQ ID NO:1863, SEQ ID NO:1864, SEQ ID NO:1866, SEQ ID NO:1867, SEQ ID NO:1869, SEQ ID NO:1870, SEQ ID NO:1871, SEQ ID NO:1872, SEQ ID NO:1874, SEQ ID NO:1875, SEQ ID NO:1876, SEQ ID NO:1877, SEQ ID NO:1878, SEQ ID NO:1880, SEQ ID NO:1881, SEQ ID NO:1882, SEQ ID NO:1884, SEQ ID NO: 1885, SEQ ID NO:1886, SEQ ID NO:1887, SEQ ID NO:1889, SEQ ID NO:1890, SEQ ID NO:1891, SEQ ID NO:1892, SEQ ID NO:1893, SEQ ID NO:1894, SEQ ID NO:1895, SEQ ID NO:1896, SEQ ID NO:1898, SEQ ID NO:1899, SEQ ID NO:1900, SEQ ID NO:1901, SEQ ID NO:1903, SEQ ID NO:1904, SEQ ID NO:1905, SEQ ID NO:1906, SEQ ID NO: 1907, SEQ ID NO:1908, SEQ ID NO:1909, SEQ ID NO:1910, SEQ ID NO:1911, SEQ ID NO:1912, SEQ ID NO:1913, SEQ ID NO:1914, SEQ ID NO:1916, SEQ ID NO:1917, SEQ ID NO:1918, SEQ ID NO:1919, SEQ ID NO:1921, SEQ ID NO:1922, SEQ ID NO:1923, SEQ ID NO:1924, SEQ ID NO:1926, SEQ ID NO:1927, SEQ ID NO:1928, SEQ ID NO: 1929, and/or SEQ ID NO:1931, a nucleic acid molecule of Table I, Table II, Table III or Table IV and/or a nucleic acid molecule that is complementary to a nucleic acid molecule of Table I, Table II, Table III or Table IV.

Another example may include a HMT and/or HNC nucleic acid molecule, wherein said nucleic acid molecule hybridizes, in a solution comprising 1X SSC and 0% formamide, at a temperature of about 47.5°C, to an isolated nucleic acid molecule selected from the group consisting of SEQ ID NO:1, SEQ ID NO:3, SEQ ID NO:4, SEQ ID NO:6, SEQ ID NO:7, SEQ ID NO:9, SEQ ID NO: 10, SEQ ID NO:12, SEQ ID NO:13, SEQ ID NO:15, SEQ ID NO:16, SEQ ID NO:18, SEQ ID NO: 19, SEQ ID NO:21, SEQ ID NO:22, SEQ ID NO:24, SEQ ID NO:25, SEQ ID NO:27, SEQ ID NO:28, SEQ ID NO:30, SEQ ID NO:31, SEQ ID NO:33, SEQ ID NO:34, SEQ ID NO:36, SEQ ID NO:37, SEQ ID NO:39, SEQ ID NO: 40, SEQ ID NO:42, SEQ ID NO:43, SEQ ID NO:45, SEQ ID NO:46, SEQ ID NO:48, SEQ ID NO:153, SEQ ID NO:155, SEQ ID NO:156, SEQ ID NO:158, SEQ ID NO:159, SEQ ID NO:161, SEQ ID NO:162, SEQ ID NO:164, SEQ ID NO: 165, SEQ ID NO:167, SEQ ID NO:168, SEQ ID NO:170, SEQ ID NO:1859, SEQ ID NO:1860, SEQ ID NO:1861, SEQ ID NO:1863, SEQ ID NO:1864, SEQ ID NO:1866, SEQ ID NO:1867, SEQ ID NO:1869, SEQ ID NO:1870, SEQ ID NO: 1871, SEQ ID NO:1872, SEQ ID NO:1874, SEQ ID NO:1875, SEQ ID NO:1876, SEQ ID NO:1877, SEQ ID NO:1878, SEQ ID NO:1880, SEQ ID NO:1881, SEQ ID NO:1882, SEQ ID NO:1884, SEQ ID NO:1885, SEQ ID NO:1886, SEQ ID NO:1887, SEQ ID NO:1889, SEQ ID NO:1890, SEQ ID NO:1891, SEQ ID NO:1892, SEQ ID NO:1893, SEQ ID NO: 1894, SEQ ID NO:1895, SEQ ID NO:1896, SEQ ID NO:1898, SEQ ID NO:1899, SEQ ID NO:1900, SEQ ID NO:1901, SEQ ID NO: 1903, SEQ ID NO: 1904, SEQ ID NO: 1905, SEQ ID NO: 1906, SEQ ID NO:1907, SEQ ID NO:1908, SEQ ID NO:1909, SEQ ID NO:1910, SEQ ID NO:1911, SEQ ID NO:1912, SEQ ID NO:1913, SEQ ID NO:1914, SEQ ID NO: 1916, SEQ ID NO:1917, SEQ ID NO:1918, SEQ ID NO:1919, SEQ ID NO:1921, SEQ ID NO:1922, SEQ ID NO: 1923, SEQ ID NO:1924, SEQ ID NO:1926, SEQ ID NO:1927, SEQ ID NO: 1928, SEQ ID NO: 1929, and/or SEQ ID NO: 1931, a nucleic acid molecule of Table I, Table II, Table III or Table IV and/or a nucleic acid molecule that is complementary to a nucleic acid molecule of Table I, Table II, Table III or Table IV. Additional preferred nucleic acid molecules include oligonucleotides of an isolated nucleic acid molecule, wherein said nucleic acid molecule hybridizes, in a solution comprising 1X SSC and 0% formamide, at a temperature of about 47.5°C, to an isolated nucleic acid molecule selected from the group consisting of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, SEQ ID NO:19, SEQ ID NO: 21, SEQ ID NO:22, SEQ ID NO:24, SEQ ID NO:25, SEQ ID NO:27, SEQ ID NO:28, SEQ ID NO:30, SEQ ID NO:31, SEQ ID NO:33, SEQ ID NO:34, SEQ ID NO:36, SEQ ID NO:37, SEQ ID NO:39, SEQ ID NO:40, SEQ ID NO:42, SEQ ID NO:43, SEQ ID NO:45, SEQ ID NO:46, SEQ ID NO:48, SEQ ID NO: 153, SEQ ID NO:155, SEQ ID NO:156, SEQ ID NO:158, SEQ ID NO:159, SEQ ID NO:161, SEQ ID NO:162, SEQ ID NO:164, SEQ ID NO:165. SEQ ID NO:167, SEQ ID NO:168, SEQ ID NO:170, SEQ ID NO:1859, SEQ ID NO:1860, SEQ ID NO:1861, SEQ ID NO:1863, SEQ ID NO:1864, SEQ ID NO:1866, SEQ ID NO:1867, SEQ ID NO:1869, SEQ ID NO:1870, SEQ ID NO:1871, SEQ ID NO: 1872, SEQ ID NO:1874, SEQ ID NO:1875, SEQ ID NO:1876, SEQ ID NO:1877, SEQ ID NO:1878, SEQ ID NO:1880, SEQ ID NO:1881, SEQ ID NO:1882, SEQ ID NO:1884, SEQ ID NO:1885, SEQ ID NO:1886, SEQ ID NO:1887, SEQ ID NO:1889, SEQ ID NO:1890, SEQ ID NO:1891, SEQ ID NO: 1892, SEQ ID NO:1893, SEQ ID NO:1894, SEQ ID NO: 1895, SEQ ID NO:1896, SEQ ID NO: 1898, SEQ ID NO:1899, SEQ ID NO:1900, SEQ ID NO:1901, SEQ ID NO:1903, SEQ ID NO:1904, SEQ ID NO:1905, SEQ ID NO:1906, SEQ ID NO:1907, SEQ ID NO:1908, SEQ ID NO:1909, SEQ ID NO:1910, SEQ ID NO:1911, SEQ ID NO:1912, SEQ ID NO:1913, SEQ ID NO:1914, SEQ ID NO:1916, SEQ ID NO: 1917, SEQ ID NO:1918, SEQ ID NO:1919, SEQ ID NO:1921, SEQ ID NO:1922, SEQ ID NO:1923, SEQ ID NO:1924, SEQ ID NO:1926, SEQ ID NO:1927, SEQ ID NO:1928, SEQ ID NO:1929, and/or SEQ ID NO:1931, a nucleic acid molecule of Table I, Table II, Table III or Table IV and/or a nucleic acid molecule that is complementary to a nucleic acid molecule of Table I, Table II, Table III or Table IV, wherein said oligonucleotide comprises at least about 18 nucleotides.

Additional preferred flea HMT and/or HNC nucleic acid molecules may include nucleic acid molecules comprising a nucleic acid sequence that is preferably at least about 70%, more preferably at least about 75%, more preferably at least about 80% more preferably at least about 85%, more preferably at least about 90%, and even more preferably at least about 95% identical to a nucleic acid sequence selected from the group consisting of SEQ ID NO:1, SEQ ID NO: 3, SEQ ID NO:4, SEQ ID NO:6, SEQ ID NO:7, SEQ ID NO:9, SEQ ID NO:10, SEQ ID NO:12, SEQ ID NO: 13, SEQ ID NO:15, SEQ ID NO:16, SEQ ID NO:18, SEQ ID NO:19, SEQ ID NO:21, SEQ ID NO:22, SEQ ID NO:24, SEQ ID NO: 25, SEQ ID NO:27, SEQ ID NO:28, SEQ ID NO:30, SEQ ID NO:31, SEQ ID NO:33, SEQ ID NO:34, SEQ ID NO:36, SEQ ID NO:37, SEQ ID NO:39, SEQ ID NO:40, SEQ ID NO:42, SEQ ID NO:43, SEQ ID NO:45, SEQ ID NO:46, SEQ ID NO:48; SEQ ID NO:153, SEQ ID NO:155, SEQ ID NO:156, SEQ ID NO:158, SEQ ID NO:159, SEQ ID NO:161, SEQ ID NO:162, SEQ ID NO:164, SEQ ID NO:165, SEQ ID NO:167, SEQ ID NO:168, SEQ ID NO:170, SEQ ID NO:1859, SEQ ID NO:1860, SEQ ID NO:1861, SEQ ID NO:1863, SEQ ID NO:1864, SEQ ID NO:1866, SEQ ID NO:1867, SEQ ID NO:1869, SEQ ID NO:1870, SEQ ID NO:1871, SEQ ID NO:1872, SEQ ID NO:1874, SEQ ID NO:1875, SEQ ID NO: 1876, SEQ ID NO:1877, SEQ ID NO:1878, SEQ ID NO:1880, SEQ ID NO:1881, SEQ ID NO:1882, SEQ ID NO:1884, SEQ ID NO:1885, SEQ ID NO:1886, SEQ ID NO:1887, SEQ ID NO:1889, SEQ ID NO:1890, SEQ ID NO:1891, SEQ ID NO:1892, SEQ ID NO:1893, SEQ ID NO:1894, SEQ ID NO:1895, SEQ ID NO:1896, SEQ ID NO:1898, SEQ ID NO: 1899, SEQ ID NO:1900, SEQ ID NO:1901, SEQ ID NO:1903, SEQ ID NO:1904, SEQ ID NO:1905, SEQ ID NO:1906, SEQ ID NO:1907, SEQ ID NO:1908, SEQ ID NO:1909, SEQ ID NO:1910, SEQ ID NO:1911, SEQ ID NO:1912, SEQ ID NO:1913, SEQ ID NO:1914, SEQ ID NO:1916, SEQ ID NO:1917, SEQ ID NO:1918, SEQ ID NO:1919, SEQ ID NO: 1921, SEQ ID NO:1922, SEQ ID NO:1923, SEQ ID NO:1924, SEQ ID NO:1926, SEQ ID NO:1927, SEQ ID NO:1928, SEQ ID NO:1929, and/or SEQ ID NO:1931, a nucleic acid molecule of Table I, Table II, Table **III** or Table IV and/or a nucleic acid molecule that is complementary to a nucleic acid molecule of Table I, Table II, Table III or Table IV. Also preferred are oligonucleotides of any of such nucleic acid molecules. Percent identity may be determined using the GCG™ Wisconsin Package Version 9.0 sequence analysis software, using default parameters.

Further described is a nucleic acid molecule comprising all or part of nucleic acid molecules $nCfALN_{2057}$, $nCfALN_{1152}$, $nCfCBP_{1128}$, $nCfCBP_{816}$, $nClNKAB_{1714}$, $nCfNKAB_{978}$, $nCfLGIC_{2240}$, $nCfLGIC_{1707}$, $nCfANON_{1429}$, $nCfANON_{1194}$, $nCfMALV_{765}$, $nCfMALV_{762}$, $nCrOSD_{604}$, $nCfOSD_{405}$, $nCfNMDA_{1227}$, $nCfNMDA_{738}$, $nCfCLBP1A_{633}$, $nCfCLBP1A_{441}$, $nCfCLBP2A_{631}$, nCfCLBP2A441, $nCfLGIC_{2739}$, $nCfLGIC_{2016}$, $nCtNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPL4_{1043}$, $nCfPL4_{1062}$, $nCfPL4_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7H2_{801}$, $nCf7B2_{741}$, or allelic variants of these nucleic acid molecules. Another preferred nucleic acid molecule includes at least a portion of nucleic acid sequence SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, SEQ ID NO:19, SEQ ID NO:21, SEQ ID NO:22, SEQ ID NO:24, SEQ ID NO:25, SEQ ID NO:27, SEQ ID NO:28, SEQ ID NO:30, SEQ ID NO:31, SEQ ID NO:33, SEQ ID NO:34, SEQ ID NO:36, SEQ ID NO: 37, SEQ ID NO:39, SEQ ID NO:40, SEQ ID NO:42, SEQ ID NO:43, SEQ ID NO:45, SEQ ID NO:46, SEQ ID NO:48, SEQ ID NO:153, SEQ ID NO:155, SEQ ID NO:156, SEQ ID NO:158, SEQ ID NO:159, SEQ ID NO:161, SEQ ID NO: 162, SEQ ID NO:164, SEQ ID NO:165, SEQ ID NO:167, SEQ ID NO:168, SEQ ID NO:170, SEQ ID NO:1859, SEQ ID NO:1860, SEQ ID NO:1861, SEQ ID NO:1863, SEQ ID NO:1864, SEQ ID NO:1866, SEQ ID NO:1867, SEQ ID NO: 1869, SEQ ID NO:1870, SEQ ID NO:1871, SEQ ID NO:1872, SEQ ID NO:1874, SEQ ID NO:1875, SEQ m NO:1876, SEQ ID NO:1877, SEQ ID NO:1878, SEQ ID NO:1880, SEQ ID NO:1881, SEQ ID NO:1882, SEQ ID NO:1884, SEQ ID NO:1885, SEQ ID NO:1886, SEQ ID NO:1887, SEQ ID NO:1889, SEQ ID NO:1890, SEQ ID NO:1891, SEQ ID NO: 1892, SEQ ID NO:1893, SEQ ID NO:1894, SEQ ID NO:1895, SEQ ID NO:1896, SEQ ID NO:1898, SEQ ID NO:1899, SEQ ID NO:1900, SEQ ID NO:1901, SEQ ID NO:1903, SEQ ID NO:1904, SEQ ID NO:1905, SEQ ID NO:1906, SEQ ID NO:1907, SEQ ID NO:1908, SEQ ID NO:1909, SEQ ID NO:1910, SEQ ID NO:1911, SEQ ID NO:1912, SEQ ID NO: 1913, SEQ ID NO:1914, SEQ ID NO:1916, SEQ ID NO:1917, SEQ ID NO:1918, SEQ ID NO:1919, SEQ ID NO:1921, SEQ ID NO:1922, SEQ ID NO:1923, SEQ ID NO:1924, SEQ ID NO:1926, SEQ ID NO:1927, SEQ ID NO:1928, SEQ ID NO:1929, and/or SEQ ID NO:1931, and/or a nucleic acid molecule of Table I, Table B, Table III or Table IV, as well as allelic variants of nucleic acid molecules having these nucleic acid sequences and homologues of nucleic acid molecules having these nucleic acid sequences; preferably such a homologue encodes or is complementary to a nucleic acid molecule that encodes at least one epitope that elicits an immune response against a protein having an amino acid sequence SEQ ID NO:2, SEQ ID NO:8, SEQ ID NO:14, SEQ ID NO:20, SEQ ID NO:26, SEQ ID NO:32, SEQ ID NO: 38, SEQ ID NO:44, SEQ ID NO: 154, SEQ ID NO:160, SEQ ID NO:163, SEQ ID NO:169, SEQ ID NO:1862, SEQ ID NO:1868, SEQ ID NO:1873, SEQ ID NO:1879, SEQ ID NO:1883, SEQ ID NO:1888, SEQ ID NO:1897, SEQ ID NO: 1902, SEQ ID NO:1915, SEQ ID NO:1920, SEQ ID NO:1925, and/or SEQ ID NO:1930. Such nucleic acid molecules can include nucleotides in addition to those included in the SEQ ID NOs, such as, but not limited to, a full-length gene, a full-length coding region, a nucleic acid molecule encoding a fusion protein, or a nucleic acid molecule encoding a multivalent protective compound.

HMT and/or HNC nucleic acid molecules may encode a protein that is at least about 70%, preferably at least about 75%, more preferably at least about 80%, even more preferably at least about 85%, even more preferably at least about 90%, even more preferably at least about 95%, even more preferably at least about 98%, and even more preferably at least about 100% identical to $PCfALN_{384}$, $PCfCBP_{272}$, $PCfNKAB_{326}$, $PCfLGIC_{569}$, $PCfANON_{398}$, $PCfMALV_{254}$, $PCfOSD_{135}$, $PCfNMDA_{246}$, $PCfCLBP1A_{147}$, $PCfCLBP2A_{147}$, $PCfLGIC_{672}$, $PCfNAH_{608}$, $PCfCLIC_{262}$, $PCfPL2_{391}$, $PCfPL3_{81}$, $PCfPLA_{285}$, $PCfSVP_{530}$, $PCfVGCC_{851}$, $PCfAUP_{102}$, $PCf7B2_{267}$, $PCf7B2_{247}$ and/or a protein encoded by a nucleic acid molecule having a sequence of Table I, Table II, Table III and/or Table IV.

A HMT and/or HNC nucleic acid molecule may encode a protein having an amino acid sequence that is at least about 70%, preferably at least about 75%, more preferably at least about 80%, even more preferably at least about 85%, even more preferably at least about 90%, even more preferably at least about 95%, even more preferably at least about 98%, and even more preferably at least about 100% identical to SEQ ID NO:2, SEQ ID NO:8, SEQ ID NO:14, SEQ ID NO:20, SEQ ID NO:26, SEQ ID NO:32, SEQ ID NO:38, SEQ ID NO:44, SEQ ID NO:154, SEQ ID NO:160, SEQ ID NO: 163, SEQ ID NO:169, SEQ ID NO:1862, SEQ ID NO: 1868, SEQ ID NO: 1873, SEQ ID NO: 1879, SEQ ID NO: 1883, SEQ ID NO:1888, SEQ ID NO:1897, SEQ ID NO:1902, SEQ ID NO:1915, SEQ ID NO:1920, SEQ ID NO:1925, and/or SEQ ID NO:1930 and/or a protein encoded by a nucleic acid molecule having a sequence of Table I, Table II, Table III and/or Table IV. Also described is a HMT and/or HNC nucleic acid molecule encoding a protein having at least a portion of SEQ ID NO:2, SEQ ID NO:8, SEQ ID NO:14, SEQ ID NO:20, SEQ ID NO:26, SEQ ID NO:32, SEQ ID NO:38, SEQ ID NO:44, SEQ ID NO:154, SEQ ID NO:160, SEQ ID NO:163, SEQ ID NO:169, SEQ ID NO:1862, SEQ ID NO:1868, SEQ ID NO:1873, SEQ ID NO:1879, SEQ ID NO: 1883, SEQ ID NO:1888, SEQ ID NO:1897, SEQ ID NO:1902, SEQ ID NO:1915, SEQ ID NO:1920, SEQ ID NO:1925, and/or SEQ ID NO:1930 and/or a protein encoded by a nucleic acid molecule having a sequence of Table I, Table II, Table III and/or Table IV, as well as allelic variants of a nucleic acid molecule encoding a protein having these sequences, including nucleic acid molecules that have been modified to accommodate codon usage properties of the cells in which such nucleic acid molecules are to be expressed.

A preferred flea HMT and/or HNC nucleic acid molecule comprises a nucleic acid molecule comprising at least about 15 nucleotides, more preferably at least about 18 nucleotides, more preferably at least about 20 nucleotides, more preferably at least about 25 nucleotides, more preferably at least about 30 nucleotides, more preferably at least about 40 nucleotides, more preferably at least about 50 nucleotides, more preferably at least about 100 nucleotides, more preferably at least about 150 nucleotides, more preferably at least about 350 nucleotides, more preferably at least about 450 nucleotides, more preferably at least about 550 nucleotides, more preferably at least about 650 nucleotides, more preferably at least about 750 nucleotides, more preferably at least about 1000 nucleotides, more preferably at least about 1500 nucleotides, more preferably at least about 1750 nucleotides more preferably at least about 2000 nucleotides, and even more preferably at least about 2250 nucleotides in length.

An alternative preferred flea HMT and/or HNC nucleic acid molecule encodes a protein comprising at least about 5 amino acids, preferably at least about 6 amino acids, more preferably at least about 10 amino acids, more preferably at least about 15 amino acids, more preferably at least about 20 amino acids, more preferably at least about 25 amino acids, more preferably at least about 30 amino acids, more preferably at least about 40 amino acids, more preferably at least about 50 amino acids, more preferably at least about 100 amino acids, more preferably at least about 150 amino acids, more preferably at least about 200 amino acids, more preferably at least about 300 amino acids, more preferably at least about 400 amino acids, more preferably at least about 500 amino acids, even more preferably at least about 560 amino acids in length.

A further preferred flea HMT and/or HNC nucleic acid molecule comprises an apparently full-length HMT and/or HNC coding region, i.e., the preferred nucleic acid molecule encodes an apparently full-length HMT and/or HNC protein, or a post-translationally modified protein thereof. The preferred HMT and/or HNC nucleic acid molecule may encode a mature protein.

A further preferred flea HMT and/or HNC nucleic acid molecule may comprise a nucleic acid molecule comprising SEQ ID NO:1, SEQ ID NO:3, SEQ ID NO:4, SEQ ID NO:6, SEQ ID NO:7, SEQ ID NO:9, SEQ ID NO:10, SEQ ID NO: 12, SEQ ID NO:13, SEQ ID NO:15, SEQ ID NO:16, SEQ ID NO:18, SEQ ID NO:19, SEQ ID NO:21, SEQ ID NO:22, SEQ ID NO:24, SEQ ID NO:25, SEQ ID NO:27, SEQ ID NO:28, SEQ ID NO:30, SEQ ID NO:31, SEQ ID NO:33, SEQ ID NO:34, SEQ ID NO:36, SEQ ID NO:37, SEQ ID NO:39, SEQ ID NO:40, SEQ ID NO:42, SEQ ID NO:43, SEQ ID NO: 45, SEQ ID NO:46, SEQ ID NO:49, SEQ ID NO:153, SEQ ID NO:155, SEQ ID NO:156, SEQ ID NO:158, SEQ ID NO: 159, SEQ ID NO:161, SEQ ID NO:162, SEQ ID NO:164, SEQ ID NO:165, SEQ ID NO:167, SEQ ID NO:168, SEQ ID NO:170, SEQ ID NO:1859, SEQ ID NO:1860, SEQ ID NO:1861, SEQ ID NO:1863, SEQ ID NO:1864, SEQ ID NO:1866, SEQ ID NO:1867, SEQ ID NO:1869, SEQ ID NO:1870, SEQ ID NO:1871, SEQ ID NO:1872, SEQ ID NO:1874, SEQ ID NO:1875, SEQ ID NO:1876, SEQ ID NO:1877, SEQ ID NO:1878, SEQ ID NO:1880, SEQ ID NO:1881, SEQ ID NO: 1882, SEQ ID NO: 1884, SEQ ID NO:1885, SEQ ID NO:1886, SEQ ID NO:1887, SEQ ID NO:1889, SEQ ID NO:1890, SEQ ID NO:1891, SEQ ID NO:1892, SEQ ID NO:1893, SEQ ID NO:1894, SEQ ID NO:1895, SEQ ID NO:1896, SEQ ID NO:1898, SEQ ID NO:1899, SEQ ID NO:1900, SEQ ID NO:1901, SEQ ID NO:1903, SEQ ID NO:1904, SEQ ID NO: 1905, SEQ ID NO:1906, SEQ ID NO:1907, SEQ ID NO:1908, SEQ m NO:1909, SEQ ID NO:1910, SEQ ID NO:1911, SEQ ID NO:1912, SEQ ID NO:1913, SEQ ID NO:1914, SEQ ID NO:1916, SEQ ID NO:1917, SEQ ID NO:1918, SEQ ID NO:1919, SEQ ID NO:1921, SEQ ID NO:1922, SEQ ID NO:1923, SEQ ID NO:1924, SEQ ID NO:1926, SEQ ID NO: 1927, SEQ ID NO:1928, SEQ ID NO:1929, and/or SEQ ID NO: 1931.

Knowing the nucleic acid sequences of certain flea HMT and/or HNC nucleic acid molecules described herein allows one skilled in the art to, for example, (a) make copies of those nucleic acid molecules, (b) obtain nucleic acid molecules including at least a portion of such nucleic acid molecules (e.g., nucleic acid molecules including full-length genes, full-length coding regions, regulatory control sequences, truncated coding regions), and (c) obtain other flea HMT and/or HNC nucleic acid molecules. Such nucleic acid molecules can be obtained in a variety of ways including screening appropriate expression libraries with antibodies as herein described; traditional cloning techniques using oligonucleotide probes as described herein to screen appropriate libraries; and PCR amplification of appropriate libraries or DNA using oligonucleotide primers as described herein. Preferred libraries to screen or from which to amplify nucleic acid molecules include flea 1st instar larvae; 3rd instar larvae, wandering larvae, prepupal larvae, pupae and whole adult flea cDNA libraries as well as genomic DNA libraries. Similarly, preferred DNA sources to screen or from which to amplify nucleic acid molecules include flea prepupal cDNA, adult cDNA and genomic DNA. Techniques to clone and amplify genes are disclosed, for example, in Sambrook et al., *ibid.*

Also described herein are nucleic acid molecules that are oligonucleotides capable of hybridizing, under stringent hybridization conditions, with complementary regions of other, preferably longer, nucleic acid molecules such as those comprising *C. felis* HMT and/or HNC nucleic acid molecules or other flea HMT and/or HNC nucleic acid molecules. Oligonucleotides herein can be RNA, DNA, or derivatives of either. The minimum size of such oligonucleotides is the size required for formation of a stable hybrid between an oligonucleotide and a complementary sequence on a nucleic acid molecule described herein. A preferred oligonucleotide has a maximum size of preferably about 100 to 200 nucleotides. Also described are oligonucleotides that can be used as, for example, probes to identify nucleic acid molecules, primers to produce nucleic acid molecules, or therapeutic reagents to inhibit flea HMT and/or HNC protein production or activity (e.g., as antisense-, triplex formation-, ribozyme- and/or RNA drug-based reagents). Also described is the use of such oligonucleotides to protect animals from disease using one or more of such technologies. Appropriate oligonucleotide-containing therapeutic compositions can be administered to an animal using techniques known to those skilled in the art.

Also described is a recombinant vector, which includes at least one isolated nucleic acid molecule of the present invention, inserted into any vector capable of delivering the nucleic acid molecule into a host cell. Such a vector contains heterologous nucleic acid sequences, that is nucleic acid sequences that are not naturally found adjacent to nucleic acid molecules of the present invention and that preferably are derived from a species other than the species from which the nucleic acid molecule(s) are derived. The vector can be either RNA or DNA, either prokaryotic or eukaryotic, and typically is a virus or a plasmid. Recombinant vectors can be used in the cloning, sequencing, and/or otherwise manipulating of flea HMT and/or HNC nucleic acid molecules as described herein.

One type of recombinant vector, referred to herein as a recombinant molecule, comprises a nucleic acid molecule as described herein operatively linked to an expression vector. The phrase operatively linked refers to insertion of a nucleic acid molecule into an expression vector in a manner such that the molecule is able to be expressed when transformed into a host cell. As used herein, an expression vector is a DNA or RNA vector that is capable of transforming a host cell and of effecting expression of a specified nucleic acid molecule. Preferably, the expression vector is also capable of replicating within the host cell. Expression vectors can be either prokaryotic or eukaryotic, and are typically viruses or plasmids. Expression vectors of the present invention include any vectors that function (i.e., direct gene expression) in recombinant cells of the present invention, including in bacterial, fungal, parasite, insect, other animal, and plant cells. Preferred expression vectors of the present invention can direct gene expression in bacterial, yeast, insect and mammalian cells, and more preferably in the cell types disclosed herein.

In particular, expression vectors of the present invention contain regulatory sequences such as transcription control sequences, translation control sequences, origins of replication, and other regulatory sequences that are compatible with the recombinant cell and that control the expression of nucleic acid molecules as described herein. In particular, recombinant molecules as described herein include transcription control sequences. Transcription control sequences are sequences that control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the recombinant cells of the present invention. A variety of such transcription control sequences are known to those skilled in the art. Preferred transcription control sequences include those that function in bacterial, yeast, or insect and mammalian cells, such as, but not limited to, *tac, lac, trp, trc,* oxy-pro, omp/lpp, rmB, bacteriophage lambda (such as lambda $p_L$ and lambda $p_R$ and fusions that include such promoters), bacteriophage T7, T7*lac*, bacteriophage T3, bacteriophage SP6, bacteriophage SP01, metallothionein, alpha-mating factor, *Pichia* alcohol oxidase, alphavirus subgenomic promoter, antibiotic resistance gene, baculovirus, *Heliothis zea* insect virus, vaccinia virus, herpesvirus, raccoon poxvirus, other poxvirus, adenovirus, cytomegalovirus (such as immediate early promoter), simian virus 40, retrovirus, actin, retroviral long terminal repeat, Rous sarcoma virus, heat shock, phosphate and nitrate transcription control sequences as well as other sequences capable of controlling gene expression in prokaryotic or eukaryotic cells. Additional suitable transcription control sequences include tissue-specific promoters and enhancers as well as lymphokine-inducible promoters (e.g., promoters inducible by interferons or interleukins). Transcription control sequences of the present invention can also include naturally occurring transcription control sequences naturally associated with fleas, such as *C. felis* transcription control sequences.

Suitable and preferred nucleic acid molecules to include in recombinant vectors as described are as disclosed herein. Preferred nucleic acid molecules to include in recombinant vectors, and particularly in recombinant molecules, include nucleic acid molecules having a sequence of Table I, Table II, Table III and/or Table IV. Particularly preferred nucleic acid molecules to include in recombinant vectors, and particularly in recombinant molecules, include $nCfALN_{2057}$, $nCfALN_{1152}$, $nCfCBP_{1128}$, $nCfCBP_{816}$, $nCfNKAB_{1714}$, $nCfNKAB_{978}$ $nCfLGIC_{2240}$, $nCfLGIC_{1707}$, $nCfANON_{1429}$, $nCfANON_{1194}$, $nCfMALV_{765}$, $nCfMALV_{762}$, $nCfOSD_{604}$, $nCfOSD_{405}$, $nCfNMDA_{1227}$, $nCfNMDA_{738}$, $nCfCLBP1A_{633}$, $nCfCLBP1A_{441}$, $nCfCLBP2A_{631}$, $nCfCLBP2A_{441}$, $nCfLGIC_{2739}$, $nCfLGIC_{2016}$, $nCfNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPL4_{1043}$, $nCfPL4_{1062}$, $nCfPL4_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7B2_{801}$, $nCf7B2_{741}$.

Recombinant molecules as described may also (a) contain secretory signals (i.e., signal segment nucleic acid sequences) to enable an expressed flea protein of the present invention to be secreted from the cell that produces the protein and/or (b) contain fusion sequences which lead to the expression of nucleic acid molecules as described herein as fusion proteins. Examples of suitable signal segments include any signal segment capable of directing the secretion of a protein as described herein. Preferred signal segments include, but are not limited to, tissue plasminogen activator (t-PA), interferon, interleukin, growth hormone, histocompatibility and viral envelope glycoprotein signal segments. Suitable fusion segments encoded by fusion segment nucleic acids are disclosed herein. In addition, a nucleic acid molecule of the present invention can be joined to a fusion segment that directs the encoded protein to the proteosome, such as a ubiquitin fusion segment. Eukaryotic recombinant molecules may also include intervening and/or untranslated sequences surrounding and/or within the nucleic acid sequences of nucleic acid molecules as described herein.

Also disclosed is a recombinant cell comprising a host cell transformed with one or more recombinant molecules as described. Transformation of a nucleic acid molecule into a cell can be accomplished by any method by which a nucleic acid molecule can be inserted into the cell. Transformation techniques include, but are not limited to, transfection, electroporation, microinjection, lipofection, adsorption, and protoplast fusion. A recombinant cell may remain unicellular or may grow into a tissue, organ or a multicellular organism. It is to be noted that a cell line refers to any recombinant cell described herein that is not a transgenic animal. Transformed nucleic acid molecules as described can remain extrachromosomal or can integrate into one or more sites within a chromosome of the transformed (i.e., recombinant) cell in such a manner that their ability to be expressed is retained. Preferred nucleic acid molecules with which to transform a cell include *C. felis* HMT and HNC nucleic acid molecules disclosed herein. Preferred nucleic acid molecules with which to transform a cell include nucleic acid molecules having a sequence of Table I, Table **II,** Table III and/or Table IV. Particularly preferred nucleic acid molecules with which to transform a cell include $nCfALN_{2057}$, $nCfALN_{1152}$, $nCfCBP_{1128}$, $nCfCBP_{816}$, $nCfNKAB_{1714}$, $nCfNKAB_{978}$ $nCfLGIC_{2240}$, $nCfLGIC_{1707}$, $nCfANON_{1429}$, $nCfANON_{1194}$, $nCfMALV_{765}$, $nCfMALV_{762}$, $nCfOSD_{604}$, $nCfOSD_{405}$, $nCfNMDA_{1227}$, $nCfNMDA_{738}$, $nCfCLBP1A_{633}$, $nCfCLBP1A_{441}$, $nCfCLBP2A_{631}$, $nCfCLBP2A_{441}$, $nCfLGIC_{2739}$, $nCfLGIC_{2016}$, $nCfNAH_{2080}$, $nCfNAH_{1824}$, $nCfCLIC_{2283}$, $nCfCLIC_{786}$, $nCfPL2_{1291}$, $nCfPL2_{1173}$, $nCfPL3_{406}$, $nCfPL3_{243}$, $nCfPL4_{974}$, $nCfPL4_{1043}$, $nCfPL4_{1062}$, $nCfPL4_{855}$, $nCfSVP_{1875}$, $nCfSVP_{1590}$, $nCfVGCC_{381}$, $nCfVGCC_{2191}$, $nCfVGCC_{1968}$, $nCfVGCC_{673}$, $nCfVGCC_{3126}$, $nCfVGCC_{2553}$, $nCfAUP_{1181}$, $nCfAUP_{306}$, $nCf7B2_{2161}$, $nCf7B2_{801}$, or $nCf7B2_{741}$.

Suitable host cells to transform include any cell that can be transformed with a nucleic acid molecule as described herein. Host cells can be either untransformed cells or cells that are already transformed with at least one nucleic acid molecule (e.g., nucleic acid molecules encoding one or more proteins as described herein and/or other proteins useful in the production of multivalent vaccines). Host cells either can be endogenously (i.e., naturally) capable of producing flea HMT and/or HNC proteins as described herein or can be capable of producing such proteins after being transformed with at least one nucleic acid molecule as described herein. Host cells as discussed herein can be any cell capable of producing at least one protein of the present invention, and include bacterial, fungal (including yeast), parasite (including helminth, protozoa and ectoparasite), other insect, other animal and plant cells. Preferred host cells include bacterial, mycobacterial, yeast, insect and mammalian cells. More preferred host cells include *Salmonella, Escherichia, Bacillus, Caulobacter, Listeria, Saccharomyces, Pichia, Spodoptera, Mycobacteria, Trichoplusia,* BHK (baby hamster kidney) cells, MDCK cells (Madin-Darby canine kidney cell line), CRFK cells (Crandell feline kidney cell line), CV-1 cells (African monkey kidney cell line used, for example, to culture raccoon poxvirus), COS (e.g., COS-7) cells, and Vero cells. Particularly preferred host cells are *Escherichia coli,* including *E. coli* K-12 derivatives; *Salmonella typhi; Salmonella typhimurium,* including attenuated strains such as UK-1 $_{x}$3987 and SR-11 $_{x}$4072; *Caulobacter; Pichia; Spodoptera frugiperda; Trichoplusia ni;* BHK cells; MDCK cells; CRFK cells; CV-1 cells; COS cells; Vero cells; and non-tumorigenic mouse myoblast G8 cells (e.g., ATCC CRL 1246). Additional appropriate mammalian cell hosts include other kidney cell lines, other fibroblast cell lines (e.g., human, murine or chicken embryo fibroblast cell lines), myeloma cell lines, Chinese hamster ovary cells, mouse NIH/3T3 cells, LMTK$^{31}$ cells and/or HeLa cells. In one embodiment, the proteins may be expressed as heterologous proteins in myeloma cell lines employing immunoglobulin promoters.

A recombinant cell is preferably produced by transforming a host cell with one or more recombinant molecules, each comprising one or more nucleic acid molecules of the present invention operatively linked to an expression vector containing one or more transcription control sequences, examples of which are disclosed herein. The phrase operatively linked refers to insertion of a nucleic acid molecule into an expression vector in a manner such that the molecule is able to be expressed when transformed into a host cell.

A recombinant cell as described herein includes any cell transformed with at least one of any nucleic acid molecule as described herein. Suitable and preferred nucleic acid molecules as well as suitable and preferred recombinant molecules with which to transfer cells are disclosed herein.

Recombinant cells as described herein can also be co-transformed with one or more recombinant molecules including flea HMT and/or HNC nucleic acid molecules encoding one or more proteins as described herein and one or more other nucleic acid molecules encoding other protective compounds, as disclosed herein (e.g., to produce multivalent vaccines).

Recombinant DNA technologies can be used to improve expression of transformed nucleic acid molecules by manipulating, for example, the number of copies of the nucleic acid molecules within a host cell, the efficiency with which those nucleic acid molecules are transcribed, the efficiency with which the resultant transcripts are translated, and the efficiency of post-translational modifications. Recombinant techniques useful for increasing the expression of nucleic acid molecules of the present invention include, but are not limited to, operatively linking nucleic acid molecules to high-copy number plasmids, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of nucleic acid molecules of the present invention to correspond to the codon usage of the host cell, deletion of sequences that destabilize transcripts, and use of control signals that temporally separate recombinant cell growth from recombinant enzyme production during fermentation. The activity of an expressed recombinant protein of the present invention may be improved by fragmenting, modifying, or derivatizing nucleic acid molecules encoding such a protein.

Isolated flea HMT and/or HNC proteins as described herein can be produced in a variety of ways, including production and recovery of natural proteins, production and recovery of recombinant proteins, and chemical synthesis of the proteins. For example, an isolated protein can be produced by culturing a cell capable of expressing the protein under conditions effective to produce the protein, and , recovering the protein. A preferred cell to culture is a recombinant cell described herein. Effective culture conditions include, but are not limited to, effective media, bioreactor, temperature, pH and oxygen conditions that permit protein production. An effective, medium refers to any medium in which a cell is cultured to produce a flea HMT and/or HNC protein as described herein. Such medium typically comprises an aqueous medium having assimilable carbon, nitrogen and phosphate sources, and appropriate salts, minerals, metals and other nutrients, such as vitamins. Cells of the present invention can be cultured in conventional fermentation bioreactors, shake flasks, test tubes, microtiter dishes, and petri plates. Culturing can be carried out at a temperature, pH and oxygen content appropriate for a recombinant cell Such culturing conditions are within the expertise of one of ordinary skill in the art. Examples of suitable conditions are included in the Examples section.

Depending on the vector and host system used for production, resultant proteins of the present invention may either remain within the recombinant cell; be secreted into the fermentation medium; be secreted into a space between two cellular membranes, such as the periplasmic space in *E. coli*; or be retained on the outer surface of a cell or viral membrane.

The phrase "recovering the protein", as well as similar phrases, refers to collecting the whole fermentation medium containing the protein and need not imply additional steps of separation or purification. Proteins as described herein can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization. Proteins as described are preferably retrieved in "substantially pure" form. As used herein, "substantially pure" refers to a purity that allows for the effective use of the protein as a therapeutic composition or diagnostic. A therapeutic composition for animals, for example, should exhibit no substantial toxicity and preferably should be capable of stimulating the production of antibodies in a treated animal.

Also described are isolated (i.e., removed from their natural milieu) antibodies that selectively bind to a flea HMT and/or HNC protein of the present invention or a mimetope thereof (e.g., anti-*C. felis* HMT or HNC antibodies). As used herein, the term "selectively binds to" an HMT and/or HNC protein refers to the ability of antibodies as described herein to preferentially bind to specified proteins and mimetopes thereof. Binding can be measured using a variety of methods standard in the art including enzyme immunoassays (e.g., ELISA), immunoblot assays, etc.; see, for example, Sambrook et al., *ibid.,* and Harlow, et al., 1988, *Antibodies, a Laboratory Manual,* Cold Spring Harbor Labs Press; Harlow et al., *ibid.* An anti-HMT or anti-HNC antibody may preferably selectively bind to a flea HMT or HNC protein respectively in such a way as to inhibit the function of that protein.

Isolated antibodies as described herein can include antibodies in serum, or antibodies that have been purified to varying degrees. Antibodies of the present invention can be polyclonal or monoclonal, or can be functional equivalents such as antibody fragments and genetically-engineered antibodies, including single chain antibodies or chimeric antibodies that can bind to one or more epitopes.

A preferred method to produce antibodies as described herein includes (a) administering to an animal an effective amount of a protein, peptide or mimetope thereof of the present invention to produce the antibodies and (b) recovering the antibodies. In another method, antibodies are produced recombinantly using techniques as heretofore disclosed to produce HMT and/or HNC proteins as described herein. Antibodies raised against defined proteins or mimetopes can be advantageous because such antibodies are not substantially contaminated with antibodies against other substances that might otherwise cause interference in a diagnostic assay or side effects if used in a therapeutic composition.

Antibodies as described herein have a variety of potential uses that are within the scope of the present invention. For example, such antibodies can be used (a) as therapeutic compounds to passively immunize an animal in order to protect the animal from fleas susceptible to treatment by such antibodies and/or (b) as tools to screen expression libraries and/or to recover desired proteins as described herein from a mixture of proteins and other contaminants. Furthermore, antibodies of the present invention can be used to target cytotoxic agents to fleas in order to directly kill such fleas. Targeting can be accomplished by conjugating (i.e., stably joining) such antibodies to the cytotoxic agents using techniques known to those skilled in the art. Suitable cytotoxic agents are known to those skilled in the art.

Also described is a therapeutic composition that, when administered to an animal susceptible to flea infestation, is capable of protecting that animal from flea infestation. Therapeutic compositions as described herein include at least one of the following protective molecules: an isolated flea HMT and/or HNC protein; a mimetope of an isolated flea HMT and/or HNC protein; an isolated flea HMT and/or HNC nucleic acid molecule; and/or a compound derived from said isolated flea HMT and/or HNC protein that inhibits HMT and/or HNC protein activity. A therapeutic composition as described herein can further comprise a component selected from the group of an excipient, a carrier, and/or an adjuvant; these components are described further herein. As used herein, a protective molecule or protective compound refers to a compound that, when administered to an animal in an effective manner, is able to treat, ameliorate, and/or prevent flea infestation. Preferred fleas to target are heretofore disclosed One example of a protective molecule is a vaccine, such as, but not limited to, a naked nucleic acid vaccine, a recombinant virus vaccine, a recombinant cell vaccine, and a recombinant protein vaccine. Another example of a protective molecule is a compound that inhibits HMT and/or HNC protein activity, such as an isolated antibody that selectively binds to a flea HMT and/or HNC protein, a substrate analog of a flea HMT and/or HNC protein, anti-sense-, triplex formation-, ribozyme-, and/or RNA drug-based compounds, or other inorganic or organic molecules that inhibit HMT and/or HNC protein activity. Inhibiting flea HMT and/or HNC protein activity can refer to the ability of a compound to reduce the activity of flea HMT and/or HNC proteins. Inhibiting flea HMT and/or HNC protein activity can also refer to the ability of a compound to reduce the amount of flea HMT and/or HNC protein in a flea.

Also described is a method to reduce a flea infestation in an animal susceptible to flea infestation. Such a method includes the step of administering to the animal a therapeutic molecule comprising a protective compound selected from the group consisting of (a) an isolated flea HMT and/or HNC protein; (b) a mimetope of an isolated flea HMT and/or HNC protein; (c) an isolated flea HMT and/or HNC nucleic acid molecule; and (d) a compound derived from an isolated flea HMT and/or HNC protein that inhibits HMT and/or HNC protein activity.

Therapeutic compositions as described herein can be administered to any animal susceptible to flea infestation, preferably to mammals, and more preferably to dogs, cats, humans, ferrets, horses, cattle, sheep, and other pets, economic food animals, work animals and/or zoo animals. Preferred animals to protect against flea infestation include dogs, cats, humans, and ferrets, with dogs and cats being particularly preferred.

As used herein, the term derived, or the term derived from, refers to a peptide, antibody, mimetope, nucleic acid molecule, or other compound that was obtained from a flea HMT and/or HNC protein or nucleic acid molecule as described herein. Methods to obtain derivatives from a HMT and/or HNC molecule as described herein are known in the art, and as such include, but are not limited to molecular modeling of HMT and/or HNC proteins to determine active sites, i.e. sites that interact with other molecules, and predicting from these active sites smaller fragments and/or mimetopes that retain and/or mimic these active sites, thereby inhibiting HMT and/or HNC protein activity; screening of peptide or small chemical compound libraries against HMT and/or HNC proteins; and screening of polyclonal or monoclonal antibodies to find antibodies that specifically bind HMT and/or HNC proteins as described herein.

A HMT and/or HNC protein inhibitor herein is identified by its ability to bind to, modify, or otherwise interact with, a flea HMT and/or HNC protein, thereby inhibiting the activity of HMT and/or HNC proteins. Suitable inhibitors of HMT and/or HNC protein activity are compounds that inhibit HMT and/or HNC protein activity in at least one of a variety of ways: (a) by binding to or otherwise interacting with or otherwise modifying HMT and/or HNC protein sites; (b) by binding to or otherwise interacting with or otherwise modifying the HMT and/or HNC protein active site; (c) by binding to the HMT and/or HNC protein and thus reducing the availability of the HMT and/or HNC protein in solution; and (d) by interacting with other regions of the HMT and/or HNC protein to inhibit HMT and/or HNC protein activity, for example, by allosteric interaction.

Flea HMT and/or HNC protein inhibitors can be used directly as compounds in compositions of the present invention to treat animals as long as such compounds are not harmful to host animals being treated. Preferred HMT and/or HNC protein inhibitors as described herein include, but are not limited to, flea HMT and/or HNC protein substrate analogs, and other molecules that bind to a flea HMT and/or HNC proteins (e.g., to an allosteric site) in such a manner that the activity of the flea HMT and/or HNC protein is inhibited. A HMT and/or HNC protein substrate analog refers to a compound that interacts with (e.g., binds to, associates with, modifies) the active site of a HMT and/or HNC protein. A preferred HMT and/or HNC protein substrate analog inhibits HMT and/or HNC protein activity. HMT and/or HNC protein substrate analogs can be of any inorganic or organic composition. HMT and/or HNC protein substrate analogs can be, but need not be, structurally similar to a HMT and/or HNC protein natural substrate as long as they can interact with the active site of that HMT and/or HNC protein. HMT and/or HNC protein substrate analogs can be designed using computer-generated structures of HMT and/or HNC proteins as described or computer structures of HMT and/or HNC protein's natural substrates. Preferred sites to model include one or more of the active sites of HMT and/or HNC protein. Substrate analogs can also be obtained by generating random samples of molecules, such as oligonucleotides, peptides, peptidomimetic compounds, or other inorganic or organic molecules, and screening such samples for their ability to interfere with interaction between HMT and/or HNC proteins and their substrates, e.g. by affinity chromatography techniques. A preferred HMT and/or HNC protein substrate analog is a HMT and/or HNC protein mimetic compound, i.e., a compound that is structurally and/or functionally similar to a natural substrate of a HMT and/or HNC protein as described, particularly to the region of the substrate that interacts with the HMT and/or HNC protein active site, but that inhibits HMT and/or HNC protein activity upon interacting with the HMT and/or HNC protein active site.

Also discussed is a therapeutic composition comprising at least one protective molecule as described herein in combination with at least one additional compound protective against one or more infectious agents.

A therapeutic composition as described herein can be used to protect an animal from flea infestation by administering such composition to a flea in order to prevent infestation. Such administration to the flea and/or animal could be oral, or by application to the animal's body surface (e.g. topical spot-on, or spraying onto the animal), or by application to the environment (e.g., spraying). Examples of such compositions include, but are not limited to, transgenic vectors capable of producing at least one therapeutic composition. In another embodiment a flea can ingest therapeutic compositions, or products thereof, present on the surface of or in the blood of a host animal that has been administered a therapeutic composition.

As discussed herein, a host animal (i.e., an animal that is or is capable of being infested with fleas) is treated by administering to the animal a therapeutic composition in such a manner that the composition itself (e.g., a HMT and/or HNC protein inhibitor, a HMT and/or HNC protein synthesis suppressor (i.e., a compound that decreases the production or half-life of a HMT and/or HNC protein in fleas), a HMT and/or HNC protein mimetope, or a anti-HMT and/or HNC antibody) or a product generated by the animal in response to administration of the composition (e.g., antibodies produced in response to administration of a flea HMT and/or HNC protein or nucleic acid molecule, or conversion of an inactive inhibitor "prodrug" to an active HMT and/or HNC protein inhibitor) ultimately enters the flea. A host animal is preferably treated in such a way that the compound or product thereof is present on the body surface of the animal or enters the blood stream of the animal. Fleas are then exposed to the composition or product when they feed from the animal. For example, flea HMT and/or HNC protein inhibitors administered to an animal are administered in such a way that the inhibitors enter the blood stream of the animal, where they can be taken up by feeding fleas.

Also discussed is the ability to reduce larval flea infestation in that when fleas feed from a host animal that has been administered a therapeutic composition of the present invention, at least a portion of compounds of the present invention, or products thereof, in the blood taken up by the fleas are excreted by the fleas in feces, which is subsequently ingested by flea larvae. In particular, it is of note that flea larvae obtain most, if not all, of their nutrition from flea feces.

As disclosed herein, reducing HMT and/or HNC protein activity in a flea can lead to a number of outcomes that reduce flea burden on treated animals and their surrounding environments. Such outcomes include, but are not limited to, (a) reducing the viability of fleas that feed from the treated animal, (b) reducing the fecundity of female fleas that feed from the treated animal, (c) reducing the reproductive capacity of male fleas that feed from the treated animal, (d) reducing the viability of eggs laid by female fleas that feed from the treated animal, (e) altering the blood feeding behavior of fleas that feed from the treated animal (e.g., fleas take up less volume per feeding or feed less frequently), (f) reducing the viability of flea larvae, for example due to the feeding of larvae from feces of fleas that feed from the treated animal, (g) altering the development of flea larvae (e.g., by decreasing feeding behavior, inhibiting growth, inhibiting (e.g., slowing or blocking) molting, and/or otherwise inhibiting maturation to adults), and/or (h) altering or decreasing the ability of fleas or flea larvae to digest a blood meal.

In order to protect an animal from flea infestation, a therapeutic composition of the present invention is administered to the animal in an effective manner such that the composition is capable of protecting that animal from flea infestation. Therapeutic compositions of the present invention can be administered to animals prior to infestation in order to prevent infestation (i.e., as a preventative vaccine) and/or can be administered to animals after infestation. For example, proteins, mimetopes thereof, and antibodies thereof can be used as immunotherapeutic agents.

Therapeutic compositions as described herein can be formulated in an excipient that the animal to be treated can tolerate. Examples of such excipients include water, saline, Ringer's solution, dextrose solution, Hank's solution, and other aqueous physiologically balanced salt solutions. Nonaqueous vehicles, such as fixed oils, sesame oil, ethyl oleate, or triglycerides may also be used. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer, while examples of preservatives include thimerosal, or o-cresol, formalin and benzyl alcohol. Standard formulations can either be liquid injectables or solids which can be taken up in a suitable liquid as a suspension or solution for injection. Thus, in a non-liquid formulation, the excipient can comprise dextrose, human serum albumin, preservatives, etc., to which sterile water or saline can be added prior to administration.

A therapeutic composition as described herein can include an adjuvant. Adjuvants are agents that are capable of enhancing the immune response of an animal to a specific antigen. Suitable adjuvants include, but are not limited to, cytokines, chemokines, and compounds that induce the production of cytokines and chemokines (e.g., granulocyte macrophage colony stimulating factor (GM-CSF), Flt-3 ligand, granulocyte colony stimulating factor (G-CSF), macrophage colony stimulating factor (M-CSF), colony stimulating factor (CSF), erythropoietin (EPO), interleukin 2 (IL-2), interleukin-3 (IL-3), interleukin 4 (IL-4), interleukin 5 (IL-5), interleukin 6 (IL-6), interleukin 7 (IL-7), interleukin 8 (IL-8), interleukin 10 (1L-10), interleukin 12 (IL-12), interferon gamma, interferon gamma inducing factor I (IGIF), transforming growth factor beta, RANTES (regulated upon activation, normal T cell expressed and presumably secreted), macrophage inflammatory proteins (e.g., MIP-1 alpha and MIP-1 beta), and Leishmania elongation initiating factor (LEIF)); bacterial components (e.g., endotoxins, in particular superantigens, exotoxins and cell wall components); aluminum-based salts; calcium-based salts; silica; polynucleotides; toxoids; serum proteins, viral coat proteins; block copolymer adjuvants (e.g., Hunter's Titermax™ adjuvant (Vaxcel™, Inc. Norcross, GA), Ribi adjuvants (Ribi ImmunoChem Research, Inc., Hamilton, MT); and saponins and their derivatives (e.g., Quil A (Superfos Biosector A/S, Denmark). Protein adjuvants described herein can be delivered in the form of the protein themselves or of nucleic acid molecules encoding such proteins using the methods described herein.

There is described a therapeutic composition can include a carrier. Carriers include compounds that increase the half-life of a therapeutic composition in the treated animal. Suitable carriers include, but are not limited to, polymeric controlled release vehicles, biodegradable implants, liposomes, bacteria, viruses, other cells, oils, esters, and glycols.

There is also described a controlled release formulation that is capable of slowly releasing a composition of the present invention into an animal. As used herein, a controlled release formulation comprises a composition of the present invention in a controlled release vehicle. Suitable controlled release vehicles include, but are not limited to, biocompatible polymers, other polymeric matrices, capsules, microcapsules, microparticles, bolus preparations, osmotic pumps, diffusion devices, liposomes, lipospheres, and transdermal delivery systems. Other controlled release formulations of the present invention include liquids that, upon administration to an animal, form a solid or a gel *in situ.* Preferred controlled release formulations are biodegradable (i.e., bioerodible).

A preferred controlled release formulation is capable of releasing a composition as described herein into the blood of the treated animal at a constant rate sufficient to attain therapeutic dose levels of the composition to protect an animal from flea infestation. The therapeutic composition is preferably released over a period of time ranging from about 1 to about 12 months. A controlled release formulation as described is capable of effecting a treatment preferably for at least about 1 month, more preferably for at least about 3 months, even more preferably for at least about 6 months, even more preferably for at least about 9 months, and even more preferably for at least about 12 months.

Acceptable protocols to administer therapeutic compositions in an effective manner include individual dose size, number of doses, frequency of dose administration, and mode of administration. Determination of such protocols can be accomplished by those skilled in the art. A suitable single dose is a dose that is capable of protecting an animal from disease when administered one or more times over a suitable time period. For example, a preferred single dose of a protein, mimetope or antibody therapeutic composition, including a recombinant protein vaccine, is from about 1 microgram ($\mu$g) to about 10 milligrams (mg) of the therapeutic composition per kilogram body weight of the animal.

Booster vaccinations can be administered from about 2 weeks to several years after the original administration. Booster administrations preferably are administered when the immune response of the animal becomes insufficient to protect the animal from disease. A preferred administration schedule is one in which from about 10 μg to about 1 mg of the therapeutic composition per kg body weight of the animal is administered from about one to about two times over a time period of from about 2 weeks to about 12 months. Modes of administration can include, but are not limited to, subcutaneous, intradermal, intravenous, intranasal, oral, transdermal, intraocular, intranasal, conjunctival, and intramuscular routes. Methods of administration for other therapeutic compounds can be determined by one skilled in the art, and may include administration of a therapeutic composition one or more times, on a daily, weekly, monthly or yearly regimen; routes of administration can be determined by one skilled in the art, and may include any route. A preferred route of administration of an inhibitory compound when administering to fleas is a topical, or "spot-on" formulation administered to the body surface of the animal, so that a flea would encounter the inhibitory compound when attached to the animal; another preferred route of administration of an inhibitory compound is an oral formulation that, when fed to an animal, would enter the bloodstream of the animal, which would then be transferred to a flea while feeding from the animal.

A recombinant protein vaccine comprises a recombinantly-produced flea HMT and/or HNC protein as described herein that is administered to an animal according to a protocol that results in the animal producing a sufficient immune response to protect itself from a flea infestation. Such protocols can be determined by those skilled in the art.

According to one embodiment, a nucleic acid molecule as described can be administered to an animal in a fashion to enable expression of that nucleic acid molecule into a protective protein or protective RNA (e.g., antisense RNA, ribozyme, triple helix forms or RNA drug) in the animal. Nucleic acid molecules can be delivered to an animal in a variety of methods including, but not limited to, (a) administering a naked (i.e., not packaged in a viral coat or cellular membrane) nucleic acid as a genetic vaccine (e.g., as naked DNA or RNA molecules, such as is taught, for example in Wolff et al., 1990, Science 247, 1465-1468) or (b) administering a nucleic acid molecule packaged as a recombinant virus vaccine or as a recombinant cell vaccine (i.e., the nucleic acid molecule is delivered by a viral or cellular vehicle).

A genetic (i.e., naked nucleic acid) vaccine includes a nucleic acid molecule as described herein and preferably includes a recombinant molecule as described herein that preferably is replication, or otherwise amplification, competent. A genetic vaccine as described can comprise one or more nucleic acid molecules as described in the form of, for example, a dicistronic recombinant molecule. Preferred genetic vaccines include at least a portion of a viral genome, i.e., a viral vector. Preferred viral vectors include those based on alphaviruses, poxviruses, adenoviruses, herpesviruses, picornaviruses, and retroviruses, with those based on alphaviruses, such as sindbis or Semliki forest virus, species-specific herpesviruses and poxviruses being particularly preferred. Any suitable transcription control sequence can be used, including those disclosed as suitable for protein production. Particularly preferred transcription control sequences include cytomegalovirus immediate early (preferably in conjunction with Intron-A), Rous sarcoma virus long terminal repeat, and tissue-specific transcription control sequences, as well as transcription control sequences endogenous to viral vectors if viral vectors are used. The incorporation of a "strong" polyadenylation signal is also preferred.

Genetic vaccines as described herein can be administered in a variety of ways, with intramuscular, subcutaneous, intradermal, transdermal, conjunctival, intraocular, intranasal and oral routes of administration being preferred. A preferred single dose of a genetic vaccine ranges from about 1 nanogram (ng) to about 600 μg, depending on the route of administration and/or method of delivery, as can be determined by those skilled in the art. Suitable delivery methods include, for example, by injection, as drops, aerosolized and/or topically. Genetic vaccines can be contained in an aqueous excipient (e.g., phosphate buffered saline) alone or in a carrier (e.g., lipid-based vehicles).

A recombinant virus vaccine includes a recombinant molecule as described herein that is packaged in a viral coat and that can be expressed in an animal after administration. Preferably, the recombinant molecule is packaging- or replication-deficient and/or encodes an attenuated virus. A number of recombinant viruses can be used, including, but not limited to, those based on alphaviruses, poxviruses, adenoviruses, herpesviruses, picornaviruses, and retroviruses. Preferred recombinant virus vaccines are those based on alphaviruses (such as Sindbis virus), raccoon poxviruses, species-specific herpesviruses and species-specific poxviruses. An example of methods to produce and use alphavirus recombinant virus vaccines are disclosed in U.S. Patent No. 5,766,602 to Xiong and Grieve.

When administered to an animal, a recombinant virus vaccine as described infects cells within the immunized animal and directs the production of a protective protein or RNA nucleic acid molecule that is capable of protecting the animal from flea infestation as disclosed herein. For example, a recombinant virus vaccine comprising a flea HMT and/or HNC nucleic acid molecule described herein is administered according to a protocol that results in the animal producing a sufficient immune response to protect itself from flea infestation. A preferred single dose of a recombinant virus vaccine is from about $1 \times 10^4$ to about $1 \times 10^8$ virus plaque forming units (pfu) per kilogram body weight of the animal. Administration protocols are similar to those described herein for protein-based vaccines, with subcutaneous, intramuscular, intranasal, intraocular, conjunctival, and oral administration routes being preferred.

A recombinant cell vaccine as described herein includes recombinant cells as described herein that express at least one protein described herein. Preferred recombinant cells for this embodiment include *Salmonella, E. coli, Listeria, Mycobacterium, S. frugiperda,* yeast, (including *Saccharomyces cerevisiae and Pichia pastoris*), BHK, CV-1, myoblast G8, COS (e.g., COS-7), Vero, MDCK and CRFK recombinant cells. Recombinant cell vaccines as described can be administered in a variety of ways but have the advantage that they can be administered orally, preferably at doses ranging from about $10^8$ to about $10^{12}$ cells per kilogram body weight. Administration protocols are similar to those described herein for protein-based vaccines. Recombinant cell vaccines can comprise whole cells, cells stripped of cell walls or cell lysates.

The efficacy of a therapeutic composition described herein to protect an animal from flea infestation can be tested in a variety of ways including, but not limited to, detection of protective antibodies (using, for example, proteins or mimetopes of the present invention), detection of cellular immunity within the treated animal, or challenge of the treated animal with the fleas to determine whether the treated animal is resistant to infestation. Challenge studies can include direct administration of fleas to the treated animal. Therapeutic compositions can be tested in animal models such as mice. Such techniques are known to those skilled in the art.

One therapeutic composition described herein includes an inhibitor of flea HMT and/or HNC protein activity, i.e., a compound capable of substantially interfering with the function of a flea HMT and/or HNC protein susceptible to inhibition by an inhibitor of flea HMT and/or HNC protein activity. An inhibitor of HMT and/or HNC protein activity can be identified using flea HMT and/or HNC proteins described herein. An inhibitor of HMT and/or HNC protein function can be identified using flea HMT and/or HNC proteins of the present invention. A preferred inhibitor of HMT and/or HNC protein function is a compound capable of substantially interfering with the function of a flea HMT and/or HNC protein and which does not substantially interfere with host animal proteins. As used herein, a compound that does not substantially inhibit or interfere with host animal proteins is one that, when administered to a host animal, the host animal shows no significant adverse effects attributable to the compound and which, when administered to an animal in an effective manner, is capable of protecting that animal from flea infestation.

Also described is a method to identify a compound capable of inhibiting HMT and/or HNC protein activity of a flea. Such a method includes the steps of (a) contacting (e.g., combining, mixing) an isolated flea HMT and/or HNC protein, preferably a *C. felis* HMT and/or HNC protein of the present invention, with a putative inhibitory compound under conditions in which, in the absence of the compound, the protein has HMT and/or HNC protein activity, and (b) determining if the putative inhibitory compound inhibits the activity. HMT and/or HNC protein activity can be determined in a variety of ways known in the art, including but not limited to determining the ability of HMT and/or HNC protein to bind to or otherwise interact with a substrate. Such conditions under which a HMT and/or HNC protein has HMT and/or HNC protein activity include conditions in which a HMT and/or HNC protein has a correct three-dimensionally folded structure under physiologic conditions, i.e. physiologic pH, physiologic ionic concentrations, and physiologic temperatures.

Putative inhibitory compounds to screen include antibodies (including fragments and mimetopes thereof), putative substrate analogs, and other, preferably small, organic or inorganic molecules. Methods to determine HMT and/or HNC protein activity are known to those skilled in the art; see, for example, the Examples section of the present application. Methods to determine binding of a putative inhibitory compound to a HMT and/or HNC protein as described are known to those of skill in the art and include, for example, determining changes in molecular mass using surface plasmon resonance (e.g., determining light scatter by an inhibitor of a HMT and/or HNC protein, before and after contacting the inhibitor or protein with a HMT and/or HNC protein or inhibitor, respectively) or screening for compounds that inhibit interaction between a HMT and/or HNC protein and a substrate.

A preferred method to identify a compound capable of inhibiting HMT and/or HNC protein activity includes contacting an isolated flea HMT and/or HNC protein having an amino acid sequence selected from the group consisting of: (a) a protein comprising an amino acid sequence selected from the group consisting of SEQ ID NO:2, SEQ ID NO:8, SEQ ID NO:14, SEQ ID NO:20, SEQ ID NO:26, SEQ ID NO:32, SEQ ID NO:38, SEQ ID NO:44, SEQ ID NO:154, SEQ ID NO:160, SEQ ID NO:163, SEQ ID NO:169, SEQ ID NO:1862, SEQ ID NO:1868, SEQ ID NO:1873, SEQ ID NO:1879, SEQ ID NO:1883, SEQ ID NO:1888, SEQ ID NO:1897, SEQ ID NO:1902, SEQ ID NO:1915, SEQ ID NO:1920, SEQ ID NO:1925, and/or SEQ ID NO:1930, and/or a protein encoded by a nucleic acid molecule of Table I, Table II, Table III and/or Table IV; (b) a protein comprising an at least 25 consecutive amino acid portion identical in sequence to a consecutive amino acid portion of a sequence as set forth in (a), wherein the protein has HMT and/or HNC protein activity; (c) a protein comprising a fragment of a protein as set forth in (a), wherein the fragment has an activity selected from the group consisting of binding to a HMT and/or HNC molecule and hydrolyzing a HMT and/or HNC protein substrate; and (d) a protein encoded by an allelic variant of a nucleic acid molecule that encodes any protein of (a), (b), or (c), with a putative inhibitory compound under conditions in which, in the absence of the compound, the protein has HMT and/or HNC protein activity; and determining if the putative inhibitory compound inhibits the activity.

Also described is an assay kit to identify an inhibitor of a flea HMT and/or HNC protein of the present invention. This kit comprises an isolated flea HMT and/or HNC protein of the present invention, and a means for determining inhibition of an activity of flea HMT and/or HNC protein, where the means enables detection of inhibition. Detection of inhibition of flea HMT and/or HNC protein identifies a putative inhibitor to be an inhibitor of flea HMT and/or HNC protein.

Means for determining inhibition of flea HMT and/or HNC protein include an assay system that detects binding of a putative inhibitor to a flea HMT and/or HNC molecule, and an assay system that detects interference by a putative inhibitor of the ability of flea HMT and/or HNC protein to hydrolyze a substrate. Means and methods are described herein and are known to those skilled in the art.

The following examples are provided for the purposes of illustration and are not intended to limit the scope of the present invention. The following examples include a number of recombinant DNA and protein chemistry techniques known to those skilled in the art; see, for example, Sambrook et al., *ibid.*

Example 1

This Example describes the isolation of RNA from the hindgut and Malpighian tubules (HMT) of *Ctenocephalides felis* and the use of isolated RNA to construct subtracted and unsubtracted cDNA libraries.

Approximately 10,000 hindguts and Malpighian tubules were dissected from equal numbers of cat blood fed and unfed adult *C. felis* with a male to female ratio of 1 to 4, and total RNA was extracted using a guanidine isothiocyanate lysis buffer and the standard procedure described by Sambrook et al. Poly-A enriched mRNA was purified from total RNA above using a mRNA Purification Kit, available from Pharmacia Biotech, Piscataway, NJ, following the manufacturer's protocol. The same procedures were used to extract total RNA and isolate poly-A enriched mRNA from the dissected *C. felis* bodies following removal of HMT, referred to hereinafter as "non-HMT mRNA".

Poly-A enriched RNA was used to construct a cDNA library using subtractive hybridization and suppression PCR as follows. Subtractive hybridization and suppression PCR was conducted using a PCR-Select™ cDNA Subtraction Kit, available from Clontech Laboratories, Inc., Palo Alto, CA according to the manufacturer's instructions. Briefly, this kit uses subtractive hybridization and suppression PCR to specifically amplify cDNA sequences that are present in the tester cDNA and absent in the driver cDNA, thus enriching for tester-specific sequences. The efficiency of the subtraction process can be assessed by semi-quantitative PCR and by comparing the ethidium bromide staining patterns of the subtracted and unsubtracted samples on agarose gels as described in section V.D. of the manufacturer's protocol. For the semi-quantitative PCR, three genes with mRNAs known to be expressed outside of the HMT tissue were used to test for specific subtraction. These genes encoded putative actin, N-aminopeptidase, and serine protease proteins.

Subtractive hybridization and suppression PCR was conducted under the following conditions. Two micrograms (μg) of HMT mRNA was used as the template for synthesis of the tester material and 2 μg of non-HMT mRNA was used as template for synthesis of the driver material in this reaction. The number of cycles used in the selective amplification steps was optimized using the manufacturer's protocols. Optimization resulted in the use of 24 rather than the standard 27 cycles of primary PCR in combination with 15 cycles of secondary PCR rather than the standard 12 cycles.

The products from the suppressive PCR reaction were ligated into the pCR®2.1 vector, available from Invitrogen, Carlsbad, CA, using an Original TA Cloning® Kit, available from Invitrogen. The ligation reaction was then used to transform INVαF' One Shot™ competent cells, available from Invitrogen, which were plated on Luria broth (LB) agar with 50 micrograms per milliliter (μg/ml) ampicillin, available from Sigma-Aldrich Co., St. Louis, MO, and 50 μg/ml 5-bromo-4-chloro-3-indoyl β-D-galactopyranoside (X-Gal), available from Fisher Biotech, Fair Lawn, NJ. Transformed colonies were amplified and the DNA isolated using the standard alkaline lysis procedure described by Sambrook et al., *ibid.*

Automated cycle sequencing of DNA samples was performed using an ABI PRISM™ Model 377, available from Perkins Elmer, with XL upgrade DNA Sequencer, available from PE Applied Biosystems, Foster City, CA, after reactions were carried out using the PRISM™ Dye Terminator Cycle Sequencing Ready Reaction Kit or the PRISM™ dRhodamine Terminator Cycle Sequencing Ready Reaction Kit or the PRISM™ BigDye™ Terminator Cycle sequencing Ready Reaction Kit, available from PE Applied Biosystems, following the manufacturer's protocol, hereinafter "standard sequencing methods". Sequence analysis was performed using the MacVector™ sequence analysis software, available from International Biotechnologies Inc., New Haven, CT, and the Wisconsin Package Version 9.0 sequence analysis software, available from Genetics Computer Group (GCG), Madison, WI, hereinafter referred to as GCG version 9.0, using default parameters. Each sequence read was trimmed of vector sequence at either end and submitted for a search through the National Center for Biotechnology Information (NCBI), National Library of Medicine, National Institute of Health, Baltimore, MD, using the BLAST network. This database includes SwissProt + PIR + SPupdate + GenPept + GPUpdate + PDB databases. The search was conducted using the xBLAST function, which compares the translated sequences in all 6 reading frames to the protein sequences contained in the database. Clones with significant homology to sequences in the GenBank database were grouped according to proposed function and are listed in Table II. Clones with no significant homology to sequences in the GenBank database were searched manually for open reading frames and are listed in Table IV.

An unsubtracted HMT cDNA library was constructed as follows. Approximately 10,000 HMT tissues were dissected from equal numbers of unfed and cat blood-fed adult *C. felis* with a male to female ratio of 1:4. Total RNA was extracted using a guanidine isothiocyanate lysis buffer and procedures described in Sambrook et al., followed by isolation using a mRNA purification kit, available from Pharmacia, according to the manufacturer's protocols. The library was constructed with 5 μg of isolated mRNA using a ZAP-cDNA® cDNA synthesis kit, and packaged using a ZAP-cDNA® Gigapack® gold cloning kit, both available from Stratagene, La Jolla, CA. The resultant HMT library was amplified to a titer of about 5 x $10^9$ plaque forming units per milliliter (pfu/ml). Single clone excisions were performed using the Ex-Assist™ helper phage, available from Stratagene, and used to create double stranded plasmid template for sequencing using the manufacturer's protocols with the following exceptions. Following incubation of the SOLR cells with the cleared phage lysate, the mixture was used to inoculate LB broth, and the mix was incubated overnight and then subjected to mini-prep plasmid preparation and sequencing as described for the subtracted HMT library above.

Example 2

This Example describes the isolation of RNA from the head and nerve cord (HNC) of *Ctenocephalides felis* and the use of isolated RNA to construct subtracted and unsubtracted cDNA libraries.

Approximately 4,000 heads and attached nerve cords, including the terminal abdominal ganglia were dissected from equal numbers of cat blood-fed and unfed adult *C. felis* with a male to female ratio of 1 to 4, and total RNA was extracted using a guanidine isothiocyanate lysis buffer and the standard procedure described by Sambrook et al. Approximately 618 μg of total RNA was recovered. Poly-A enriched mRNA was purified from total RNA above using a mRNA Purification Kit, available from Pharmacia, following the manufacturer's protocol. Approximately 13 μg of mRNA was isolated. The same procedures were used to extract total RNA and isolate poly-A enriched mRNA from the dissected *C. felis* bodies following removal of HNC tissues, referred to hereinafter as "non-HNC mRNA".

Suppression subtractive PCR was conducted as described in Example 1 using a PCR-Select™ cDNA Subtraction kit, available from Clontech, under the following conditions. Two micrograms (μg) of HNC mRNA was used as the template for synthesis of the tester material and 2 μg of non-HMT mRNA was used as template for synthesis of the driver material in this reaction. The number of cycles used in the selective amplification steps was optimized using the manufacturer's protocols. Optimization resulted in the use of 24 rather than the standard 27 cycles of primary PCR in combination with either 12 or 15 cycles of secondary PCR.

cDNA pools from various PCR cycling combinations were ligated into the TA vector using a TA cloning kit, available from Invitrogen. Aliquots of ligation reaction were transformed into Ultramax DH5∝™ bacteria, available from Gibco-BRL, Gaithersburg, MD. Portions of the transformation mixes were used to inoculate LB broth cultures containing 100 μg/ml of ampicillin. The overnight cultures were plated to generate discreet colonies which were used individually for overnight cultures for plasmid preps. Transformed colonies were amplified and the DNA isolated using the standard alkaline lysis procedure described by Sambrook et al., *ibid.*

Automated cycle sequencing of DNA samples was performed using the standard sequencing methods described in Example 1. Sequence analysis was performed using the MacVector™ sequence analysis software, available from International Biotechnologies Inc., New Haven, CT, and the Wisconsin Package Version 9.0 sequence analysis software, available from Genetics Computer Group (GCG), Madison, WI, hereinafter referred to as GCG version 9.0, using default parameters. Each sequence read was trimmed of vector sequence at either end and submitted for a xBLAST search as described in Example 1. Clones with significant homology to sequences in the GenBank database were grouped according to proposed function and are listed in Table I. Clones with no significant homology to sequences in the GenBank database were searched manually for open reading frames and are listed in Table III.

An unsubtracted cDNA library was constructed as follows. Approximately 6400 head and nerve cords were dissected from *C. felis* and poly-A RNA was isolated as described above. About seven μg of HNC poly-A RNA was used to construct a cDNA library using Stratagene's λZAP-cDNA Synthesis Kit and protocol. The resultant HNC library was amplified to a titer of about 5 x $10^9$ plaque forming units per milliliter (pfu/ml). Single clone excisions were performed using the Ex-Assist helper phage, available from Stratagene, and used to create double stranded plasmid template for sequencing using the manufacturer's protocols with the following exceptions. Following incubation of the SOLR cells with the cleared phage lysate, the mixture was used to inoculate LB broth, and the mix was incubated overnight and then subjected to mini-prep plasmid preparation and sequencing as described for the subtracted library above.

Example 3

This example describes the production of a *C. felis* cDNA pool by Rapid Amplification of cDNA Ends (RACE cDNA pool).

Total RNA was extracted from adult fed and unfed fleas as follows. Approximately 1000 adult fed fleas and 1000 adult unfed fleas were frozen on dry ice and separately ground into powder using a mortar and pestle and total RNA was extracted from each powder as follows. Ten ml of solution D (4 M guanidine isothiocyanate, 25 mM Sodium Citrate pH 7.0, 1.5% Sarcosyl, 0.5 M 2-mercaptoethanol) were added to the powder and the suspension was mixed by shaking. One ml of 2M sodium acetate, pH 4.0 and 3 ml of pH 4.7 phenol/chloroform/isoamyl alcohol (125:24:1), available from Sigma, were added and the suspension was mixed on a vortex shaker then incubated on ice for 15 minutes. Following incubation, the mixture was centrifuged at 10,000 X g for 20 minutes and the supernatant was removed and extracted twice with pH 4.7 phenol/chloroform/isoamyl alcohol. Next, an equal volume of isopropanol was added to the supernatant and incubated at -20°C for 2 hours followed by centrifugation at 10,000 X g for 20 minutes. Following centrifugation, the supernatant was removed and discarded and the pellet was washed in 70% ethanol and allowed to dry at room temperature. The pellet was resuspended in 10 mM Tris 1 mM EDTA pH 8.0. Spectrophotometer analysis indicated that the yield of total RNA from unfed fleas was 1140 μg and the yield from fed fleas was 1500 μg.

Six-hundred μg from each of the fed and unfed adult flea total RNA extractions were combined and mRNA was then extracted using a mRNA Purification Kit, available from Amersham Pharmacia Biotech, Piscataway, NJ, using the manufacture's protocol. Approximately 15-25 μg of mRNA were isolated based on spectrophotometer analysis and ethidium bromide staining. One μg of purified mRNA was used as template to construct a RACE cDNA pool using a Marathon cDNA Amplification Kit, available from Clontech Laboratories, Inc., Palo Alto, CA, according to the manufacture's instructions.

Example 4 (Reference)

This example describes the cloning, sequencing, recombinant protein expression and purification of a *C. felis* allantoinase nucleic acid molecule. This example also describes the expression of allantoinase mRNA in a variety of flea tissues.

A TA clone from the HMT EST library described in Example 1 was sequenced using standard sequencing methods and shown to have significant homology to allantoinase genes. This clone was digested with EcoRI to excise an insert 682 nucleotides in length, referred to as flea nucleic acid molecule $nCfALN_{682}$. The insert was isolated by gel purification using a Gel Purification kit, available from Qiagen, Chatsworth, CA. Approximately 50 nanograms (ng) of purified $nCfALN_{682}$ was used to construct a $^{32}P$ α-dATP labeled DNA probe using a Megaprime DNA labeling kit, available from Amersham, Arlington Heights, IL, using the manufacturer's protocols.

The $^{32}P$ α-dATP labeled probe was used in a standard plaque lift hybridization procedure to isolate a clone from the HMT lambda-ZAP unsubtracted cDNA library described in Example 1. The following hybridization conditions were used, hereinafter referred to as "standard hybridization conditions". Filters were hybridized with about 1 X $10^6$ counts per minute (cpm) per ml of the probe in 5X SSPE, (see Sambrook et al., *ibid.*), 1.2% sodium dodecyl sulfate (SDS), 0.1 mg/ml salmon sperm DNA and 5X Denhardt's reagent, (see Sambrook et al., *ibid.*), at 55°C for about 14 hours. The filters were washed as follows: (a) 10 minutes with 5X SSPE and 1% SDS, (b) 10 minutes with 2X SSPE and 1% SDS, (c) 10 minutes with 1X SSPE and 0.5% SDS, and (d) 10 minutes with 0.5X SSPE and 1% SDS. All washes were conducted at 55°C. Plaques that hybridized strongly to the probe were isolated and subjected to *in vivo* excision. *In vivo* excision was performed using the Stratagene Ex-Assist™ helper phage system and protocols, to convert a positive plaque to pBluescript™ plasmid DNA. Sequencing was conducted using standard sequencing methods following preparation of DNA with a Qiagen Qiaprep™ spin mini prep kit using the manufacturer's instructions and restriction enzyme digestion with about 1 μl of 20 U/μl each of *Eco*RI and *Xho*I, available from New England Biolabs, Beverly, MA. A clone was isolated from a primary plaque, containing a nucleic acid molecule of about 2057 base pairs, referred to herein as $nCfALN_{2057}$, having a nucleotide sequence denoted herein as SEQ ID NO:1. The complement of SEQ ID NO: 1 is represented herein as SEQ ID NO:3. Sequencing of $nCfALN_{682}$ indicates that $nCfALN_{682}$ shared 100% identity with nucleotides 855 through 1536 of SEQ ID NO:1.

Translation of SEQ ID NO:1 suggests that nucleic acid molecule $nCfALN_{2057}$ encodes a full-length allantoinase protein of 384 amino acids, referred to herein as $PCfALN_{384}$, having an amino acid sequence represented by SEQ ID NO:2, assuming the initiation codon spans from nucleotide 152 through nucleotide 154 of SEQ ID NO:1 and the termination codon spans from nucleotide 1304 through nucleotide 1306 of SEQ ID NO:1. The coding region encoding $PCfALN_{384}$, is represented by nucleic acid molecule $nCfALN_{1152}$, having a coding strand with the nucleic acid sequence represented by SEQ ID NO:4 and a complementary strand with nucleic acid sequence represented by SEQ ID NO:6. The amino acid sequence of $PCfALN_{384}$ also represented as SEQ ID NO:5, predicts that $PCfALN_{384}$ has an estimated molecular weight of about 42.2 kilodaltons (kDa) and an estimated isoelectric point (pI) of about 6.

Comparison of amino acid sequence SEQ ID NO:2 with amino acid sequences reported in GenBank indicates that SEQ ID NO:2 showed the most homology, i.e., about 48.6% identity, with a *Rana catesbeiana* (bullfrog) allantoinase protein, GenBank Accession No. 458126. Comparison of SEQ ID NO:4 with nucleic acid sequences reported in GenBank indicates that SEQ ID NO:4 showed the most homology, i.e., about 51% identity, with a *Rana catesbeiana* nucleic acid molecule, GenBank Accession number U03471. Percent identity calculations were performed using GCG version 9.0 using default parameters.

The coding region of $nCfALN_{2057}$, i.e. SEQ ID NO:4, was PCR amplified from the pBluescript™ clone described above as the template, using sense primer ALN-FE, having nucleotide sequence 5' **GCG GAT CCT** ATG CTG AAT TGC AAG AAC CTT G 3', having a *Bam*HI site indicated in bold, designated herein as SEQ ID NO:37, and anti-sense primer ALN-RE, having nucleotide sequence 5' CA**G GTA CCC** TCT TTT AGA AGC ACC GGT CCC 3', having a *KpnI* site indicated in bold, designated herein as SEQ ID NO:38. PCR reactions were performed using the following amplification cycles: (a) one cycle at 95°C for thirty seconds; (b) thirty cycles at 95°C for twenty seconds, 50°C for twenty seconds, and 72°C for two minutes; and (c) one cycle at 72°C for five minutes, hereinafter referred to as "standard thermocycling conditions", in reactions containing 2.5 mM $MgCl_2$, 0.2 mM dNTPs, 1 μM of each primer, 0.5 μl of 5U/μl *Taq* polymerase, 1 μl of 1 μg/μl template, and 3 μl of 10X Taq buffer, hereinafter referred to as "standard PCR reaction conditions". The PCR product was digested with *Bam*HI and *Kpn*I and ligated into the vector pTrcHisB, available from Invitrogen, that had been digested with *Bam*HI and *Kpn*I and treated with alkaline phosphatase. The resulting recombinant molecule, referred to herein as pTrc-$nCfALN_{1152}$, was transformed into *E. coli* strain BL21, available from Novagen Inc., Madison, WI, to form recombinant cell *E. coli*:pTrc-$nCfALN_{1152}$.

The recombinant cell was grown under standard conditions and then incubated in the presence of 0.5 μM isopropylthio-β-galactoside (IPTG) to induce expression of recombinant protein, predicted to be approximately 42.2 kDa. Expression was confirmed using Coomassie-blue-stained Tris-glycine gel and by Western blot using a T7 tag antibody, available from Novagen, which showed expression of an about 55-kDa protein. The protein product was purified by liquid chromatography using a HiTrap™ chelating column charged with $NiCl_2$, available from Pharmacia, and was shown to contain the His tag of the vector when subjected to automated protein sequencing by Edman degradation.

A Northern Blot analysis was conducted as follows to determine whether allantoinase is expressed exclusively in HMT tissues. HMT tissues were dissected from 1000 adult cat blood-fed *C. felis* having a male to female ratio of 1: 4. Total RNA was separately extracted from HMT tissues and the HMT-less carcasses that resulted from these dissections as follows. The tissues were frozen at -80°C, ground into a powder with a mortar and pestle, and the powders were equally divided into four 2-ml eppendorf tubes each containing 1 ml of lysis buffer. The lysis buffer contained 4 M guanidinium thiocyanate, 25 mM sodium citrate, pH 7.0, 3% sarcosyl, 0.5M 2-mercaptoethanol, 0.1% antifoam, and 1 mM aurintricarboxylic acid, all available from Sigma Chemical Corporation, St. Louis, MO. After mixing, the tubes were spun at 14,000 rpm for 2 minutes and the supernatants were transferred to separate 2 ml eppendorf tubes containing 250 μl of phenol, available from Aldrich, Milwaukee, WI. After mixing, the tubes were spun at 14,000 rpm for 5 minutes and the supernatants were transferred to new 2-ml tubes. This process was repeated 3 times until no proteinaceous matter was visible at the phenol/lysis buffer interface, then 250 μl of chloroform was added to each tube and the contents mixed and spun at 14,000 rpm for 5 minutes followed by transferring the supernatant to a new tube. A volume of isopropanol equal to the volume of the supernatant was added to each tube and the tubes placed on ice for 5 minutes. The tubes were then spun at 14,000 rpm at room temperature for 15 minutes, the supernatants were removed and discarded and the remaining RNA pellets were washed with 70% ethanol and dried. The RNA pellets were resuspended in 100 μl of TE (10 mM Tris, 1 mM ethylenediaminetetraacetic acid (EDTA)). The quantity of RNA in each tube was then determined using a spectrophotometer.

Approximately 10 μg of each RNA was added to separate tubes containing 18.75 μl of loading buffer, which consists of 50% formamide, 16% formaldehyde, 17% water, 7% glycerol, 1 X MOPS buffer (a 1:20 dilution of 0.4 M 93-[N-morpholino]propanesulfonic acid (MOPS), 0.1 M sodium acetate, and 20 mM EDTA), 10 μl ethidium bromide, and 10 μl bromophenol blue dye, all available from Sigma. The tubes were heated to 95°C for 2 minutes then placed on ice. The RNA samples were separated by gel electrophoresis on a 1.5% agarose gel with 3.2% formaldehyde and 1 X MOPS buffer; the gel was then soaked in water for 30 minutes prior to transfer to remove excess formaldehyde. The gel was then transferred using standard techniques, described by Sambrook *et al., ibid,* with 10 X SSPE as the transfer buffer onto Nytran® nylon membrane, available from Schleicher and Schuell Inc., Keene, NH. The membrane was UV cross-linked using the Stratalinker®, available from Stratagene, then prehybridized at 42°C in 50% formamide, 5X SSPE, 1.2% SDS, 5X Denhardt's reagent, 2.5 mM EDTA, and 100 μg/ml salmon sperm DNA. A probe comprising the allantoinase EST nucleic acid molecule, $nCfALN_{682}$ was labeled with α-$^{32}$P-ATP using a DNA labeling kit, available from Amersham and added to the buffer at a concentration of approximately 1 x $10^6$ cpm/ml, and allowed to hybridize for 18 hours at 42°C. The blot was then washed as follows: 10 minutes at 42°C in 4X SSPE and 1% SDS; 10 minutes at 42°C in 2X SSPE and 1% SDS; 10 minutes at 42°C with 0.5X SSPE and 0.5X SDS; and 10 minutes at 42°C with 0.25X SSPE and 0.25% SDS. The blot was then exposed to film for 1 hour, and the film was developed using standard procedures. Analysis of the developed film revealed that allantoinase mRNA was present in HMT tissues but was not present in non-HMT tissues.

Northern Blot analysis was also conducted to determine whether allantoinase mRNA is expressed only in certain stages of the flea life cycle and whether allantoinase mRNA expression is influenced by feeding. Total RNA was extracted as described above from 1000 fleas at each of the following flea life stages; eggs, first instar larvae, third instar larvae, wandering larvae and pupae and from 1000 adult fleas under the following feeding conditions; unfed, fed on cat blood for 15 minutes, fed on cat blood for 2 hours, fed on cat blood for 8 hours, and fed on cat blood for 24 hours.

Each RNA sample was separated by gel electrophoresis, transferred to nylon membrane and hybridized with α-$^{32}$P-ATP labeled $nCfALN_{682}$ probe as described above. Analysis of the developed film revealed that allantoinase mRNA was expressed in all adult fleas tested regardless of feeding conditions and was expressed by all life stages except for eggs and pupae, the two life stages which do not feed or excrete urine.

Example 5 (Reference)

This example describes the cloning, sequencing, recombinant protein expression and purification of a *C. felis* chitin-binding protein nucleic acid molecule. This example also describes the expression of chitin-binding protein mRNA in a variety of flea tissues.

A TA clone from the HMT EST library described in Example 1 was sequenced using standard sequencing methods and shown to have homology to a chitinase-like gene from *Bombyx mori* (silkworm). This clone was digested with *EcoRI* to excise an insert about 429 nucleotides in length, referred to as chitin-binding protein (CBP) nucleic acid molecule $nCfCBP_{429}$. The insert was isolated by gel purification using a Gel Purification kit, available from Qiagen. Approximately 50 ng of purified $nCfCBP_{429}$ was used to construct a $^{32}P$ $\alpha$-dATP labeled DNA probe using a Megaprime DNA labeling kit, available from Amersham, using the manufacturer's protocols.

The $^{32}P$ $\alpha$-dATP labeled probe was used in a plaque lift hybridization procedure to isolate a clone from the HMT lambda-ZAP unsubtracted cDNA library described in Example 1, using standard hybridization conditions described in Example 4. Plaques that hybridized strongly to the probe were isolated and subjected to *in vivo* excision. In *vivo* excision was performed using the Stratagene Ex-Assist™ helper phage system and protocols, to convert a positive plaque to pBluescript™ plasmid DNA, and sequencing was conducted following preparation of DNA with a Qiagen Qiaprep™ spin mini prep kit using the manufacturer's instructions and restriction enzyme digestion with about 1 $\mu$l of 20 U/$\mu$l each of *Eco*RI and *Xho*I, available from New England Biolabs. A clone was isolated from a primary plaque, containing a nucleic acid molecule of about 1128 base pairs, referred to herein as $nCfCBP_{1128}$, having a nucleotide sequence denoted herein as SEQ ID NO:7. The complement of SEQ ID NO:7 is represented herein as SEQ ID NO:9. Sequencing of $nCfCBP_{429}$ indicated that $nCfCBP_{429}$ shares 100% identity with nucleotides 148 through 576 of SEQ ID NO:7.

Translation of SEQ ID NO:7 suggests that nucleic acid molecule $nCfCBP_{1128}$ encodes a full-length chitin-binding protein of 272 amino acids, referred to herein as $PCfCfCBP_{272}$, having an amino acid sequence represented by SEQ ID NO:8, assuming the initiation codon spans from nucleotide 6 through nucleotide 8 of SEQ ID NO:7 and the termination codon spans from nucleotide 822 through nucleotide 824 of SEQ ID NO:7. The coding region encoding $PCfCBP_{272}$, is represented by nucleic acid molecule $nCfCBP_{816}$, having a coding strand with the nucleic acid sequence represented by SEQ ID NO:10 and a complementary strand with nucleic acid sequence represented by SEQ ID NO:12. The amino acid sequence of $PCfCBP_{272}$, also represented as SEQ ID NO:11, predicts that $PCfCBP_{272}$ has an estimated molecular weight of about 30.6 kDa and an estimated pI of about 7.3.

Comparison of amino acid sequence SEQ ID NO:8 with amino acid sequences reported in GenBank indicates that SEQ ID NO:8 showed the most homology, i.e., about 26% identity with a *Lucilia cuprina* peritrophin-44 protein, GenBank Accession No. 407976. Comparison of SEQ ID NO:10 with nucleic acid sequences reported in GenBank indicates that SEQ ID NO:10 showed the most homology, i.e., about 40% with a *Lucilia cuprina* peritrophin-44 nucleic acid molecule, GenBank Accession number L25106. Percent identity calculations were performed using GCG version 9.0 using default parameters.

A nucleic acid molecule comprising nucleotides 59 through 827 of SEQ ID NO:7, encoding a predicted mature flea chitin-binding protein, was PCR amplified from the pBluescript™ clone described above as the template, using sense primer CBP-FE, having nucleotide sequence 5' CGC GAT CCT GCT GAC AGG AAT TCG CCC AC 3', having a *Bam*HI site indicated in bold, designated herein as SEQ ID NO:39, and anti-sense primer CBP-RE, having nucleotide sequence 5' CAT GGT ACC CCT GGT TTA AGC CTT ACT TAG C 3', having a *Kpn*I site indicated in bold, designated herein as SEQ ID NO:38. PCR reactions were performed using standard PCR reaction and thermocycling conditions described in Example 4. The PCR product was digested with *BamHI* and *Kpn*I and ligated into the vector pTrcHisB, available from Invitrogen, that had been digested with *BamHI* and *KpnI* and treated with alkaline phosphatase. The resulting recombinant molecule, referred to herein as pTrc-$nCfCBP_{769}$, was transformed into *E. coli* strain BL21, available from Novagen, to form recombinant cell *E. coli*:pTrc-$nCfCBP_{769}$. The recombinant cell was grown under standard conditions and then incubated in the presence of 0.5 $\mu$M IPTG to induce expression of recombinant protein, predicted to be a protein of approximately 32 kDa. Expression of protein was confirmed using Coomassie-blue-stained Tris-glycine gel and by Western blot using a T7 tag antibody which showed expression of an about 32-kDa protein. The protein product was purified by liquid chromatography using a HiTrap™ chelating column charged with $NiCl_2$, available from Pharmacia, and was shown to contain the His tag of the vector when subjected to automated protein sequencing by Edman degradation.

Northern Blot analysis was conducted as described in Example 4 to determine whether CBP mRNA is expressed in only HMT tissue, only in certain stages of the flea life cycle and whether CBP mRNA expression is influenced by feeding. Total RNA was extracted from flea tissues, life stages and feeding conditions as described in Example 4. Each RNA sample was separated by gel electrophoresis, transferred to a nylon membrane and hybridized with $\alpha$-$^{32}P$-ATP labeled $nCfCBP_{429}$ under the Northern Blotting conditions described in Example 4. Analysis of the developed film revealed that CBP mRNA was expressed in HMT tissues but not in non-HMT tissues. CBP mRNA was also detected in all adult fleas tested regardless of feeding conditions but was not detected in any of the non-adult life stages.

Example 6 (Reference)

This example describes the cloning and sequencing of a *C. felis* sodium/potassium ATPase, beta subunit nucleic acid molecule.

A TA clone from the HMT EST library described in Example 1 was sequenced using standard sequencing methods and shown to have homology to the nervous system antigen 1 gene from *Drosophila melanogaster.* This clone was digested with *EcoRI* to excise an insert about 439 nucleotides in length, referred to as flea NKAB nucleic acid molecule $nCfNKAB_{439}$. The insert was isolated by gel purification using a Gel Purification kit, available from Qiagen. Approximately 50 ng of purified $nCfNKAB_{439}$ was used to construct a $^{32}P$ $\alpha$-dATP labeled DNA probe using a Megaprime DNA labeling kit, available from Amersham, using the manufacturer's protocols.

The $^{32}P$ $\alpha$-dATP labeled probe was used in a plaque lift hybridization procedure to isolate a clone from the HMT lambda-ZAP unsubtracted cDNA library described in Example 1, using standard hybridization conditions described in Example 4. Plaques that hybridized strongly to the probe were isolated and subjected to *in vivo* excision. In *vivo* excision was performed using the Stratagene Ex-Assist™ helper phage system and protocols, to convert a positive plaque to pBluescript™ plasmid DNA, and sequencing was conducted following preparation of DNA with a Qiagen Qiaprep™ spin mini prep kit using the manufacturer's instructions and restriction enzyme digestion with about 1 $\mu$l of 20 U/$\mu$l each of *Eco*RI and *Xho*I, available from New England Biolabs. A clone was isolated from a secondary plaque, containing a nucleic acid molecule of about 1714 base pairs, referred to herein as $nCfNKAB_{1714}$, having a nucleotide sequence denoted herein as SEQ ID NO:13. The complement of SEQ ID NO:13 is represented herein as SEQ ID NO: 15. Sequencing of $nCfNKAB_{439}$ indicates that $nCfNKAB_{439}$ shared 100% identity with nucleotides 907 through 1345 of SEQ ID NO:13.

Translation of SEQ ID NO:13 suggests that nucleic acid molecule $nCfNKAB_{1714}$ encodes a full-length NKAB protein of 326 amino acids, referred to herein as $PCfNKAB_{326}$, having an amino acid sequence represented by SEQ ID NO:14, assuming the initiation codon spans from nucleotide 294 through nucleotide 296 of SEQ ID NO:13 and the termination codon spans from nucleotide 1272 through nucleotide 1274 of SEQ ID NO:13. The coding region encoding $PCfNKAB_{326}$ is represented by nucleic acid molecule $nCfNKAB_{978}$, having a coding strand with the nucleic acid sequence represented by SEQ ID NO:16 and a complementary strand with nucleic acid sequence represented by SEQ ID NO:18. The amino acid sequence of $PCfNKAB_{326}$, also represented by SEQ ID NO:17 predicts that $PCfNKAB_{326}$ has an estimated molecular weight of about 37.7 kDa and an estimated pI of about 5.

Comparison of amino acid sequence SEQ ID NO:14 with amino acid sequences reported in GenBank indicates that SEQ ID NO:14 showed the most homology, i.e., about 46% identity, with a *Drosophila melanogaster* nervous system antigen 2 protein, GenBank Accession No. 881344. Comparison of SEQ ID NO:16 with nucleic acid sequences reported in GenBank indicates that SEQ ID NO:16 showed the most homology, i.e., about 52% identity, with a *Drosophila melanogaster* nervous system antigen 2 nucleic acid molecule, GenBank Accession number U22440. Percent identity calculations were performed using GCG version 9.0 using default parameters.

Example 7 (Reference)

This example describes the cloning and sequencing of a *C. felis* ligand-gated ion channel nucleic acid molecule. This example also describes the expression of ligand-gated ion channel mRNA in a variety of flea tissues.

A TA clone from the HMT EST library described in Example 1 was sequenced using standard sequencing methods and shown to have homology to a human ligand-gated chloride channel nucleic acid molecule. The clone was digested with *EcoRI* to excise an insert about 376 nucleotides in length, referred to as flea LGIC nucleic acid molecule $nCfLGIC_{376}$. The insert was isolated by gel purification using a Gel Purification kit, available from Qiagen. Approximately 50 ng of purified $nCfLGIC_{376}$ was used to construct a $^{32}P$ $\alpha$-dATP labeled DNA probe using a Megaprime DNA labeling kit available from Amersham, using the manufacturer's protocols.

The $^{32}P$ $\alpha$-dATP labeled probe was used in a plaque lift hybridization procedure to isolate a clone from the HMT lambda-ZAP unsubtracted cDNA library described in Example 1, using standard hybridization conditions described in Example 4. Plaques that hybridized strongly to the probe were isolated and subjected to in *vivo* excision. *In vivo* excision was performed using the Stratagene Ex-Assist™ helper phage system and protocols, to convert a positive plaque to pBluescript™ plasmid DNA and sequencing was conducted following preparation of DNA with a Qiagen Qiaprep™ spin mini prep kit using the manufacturer's instructions and restriction enzyme digestion with about i $\mu$l of 20 U/$\mu$l each of EcoRI and *Xho*I, available from New England Biolabs. A clone was isolated from a secondary plaque, containing a nucleic acid molecule of about 2240 base pairs, referred to herein as n $nCfLGIC_{2240}$, having a nucleotide sequence denoted herein as SEQ ID NO:19. The complement of SEQ ID NO:19 is represented herein as SEQ ID NO: 21. Sequencing of $nCfLGIC_{376}$ indicates that $nCfLGIC_{376}$ shared 100% identity with nucleotides 763 through 1138 of SEQ ID NO:19.

Translation of SEQ ID NO:19 suggests that nucleic acid molecule $nCfLGIC_{2240}$ encodes a partial-length LGIC protein of 569 amino acids, referred to herein as $PCfLGIC_{569}$, having an amino acid sequence represented by SEQ ID NO:20, assuming the initiation codon spans from nucleotide 1 through nucleotide 3 of SEQ ID NO:19 and the termination codon spans from nucleotide 1708 through nucleotide 1710 of SEQ ID NO:19. The coding region encoding $PCfLGIC_{569}$, is represented by nucleic acid molecule $nCfLGIC_{1707}$, having a coding strand with the nucleic acid sequence represented by SEQ ID NO:22 and a complementary strand with nucleic acid sequence represented by SEQ ID NO:24. The amino acid sequence of $PCfLGIC_{569}$, also represented as SEQ ID NO:23, predicts that $PCfLGIC_{569}$ has an estimated molecular weight of about 64 kDa and an estimated pI of about 6.6.

Comparison of amino acid sequence SEQ ID NO:20 with amino acid sequences reported in GenBank indicates that SEQ ID NO:20 showed the most homology, i.e., about 23% identity, with a *Rattus norvegicus* glycine receptor alpha-3 chain precursor protein, GenBank Accession No. 121580. Comparison of SEQ ID NO:22 with nucleic acid sequences reported in GenBank indicates that SEQ ID NO:22 showed the most homology, i.e., about 38% identity, with a human glycine receptor alpha-3 subunit nucleic acid molecule, GenBank Accession number AF017715. Percent identity calculations were performed using GCG version 9.0 using default parameters.

Northern Blot analysis was conducted as described in Example 4 to determine whether LGIC mRNA is expressed in only HMT tissue. Total RNA was extracted from HMT tissues and non-HMT tissues as described in Example 4. Each RNA sample was separated by gel electrophoresis, transferred to nylon membranes and hybridized with $\alpha$-$^{32}$P-ATP labeled $nCfLGIC_{376}$ under the Northern Blotting conditions described in Example 4. Analysis of the developed film revealed that LGIC mRNA was expressed in HMT tissues but not in non-HMT tissues.

Additional nucleic acid sequence corresponding to the coding regions at the 5' end of the LGIC cDNA described above was isolated by PCR using the RACE cDNA pool prepared as described in Example 3 as the template. A first PCR reaction was conducted using reverse primer LGIC-R4, which is complementary to nucleotides 200-223 of SEQ ID NO:19, having a nucleic acid sequence 5' GCG ATA CTG GTG GTA CTG GTG AAG 3', denoted herein as SEQ ID NO:1932 was used with the forward linker primer Adapter Primer 1, having a nucleic acid sequence 5'CCA TCC TAA TAC GAC TCA CTA TAG GGC 3', denoted herein as SEQ ID NO:1933 using standard PCR reaction conditions and the following thermocycling conditions: (1) 94°C for 30 seconds, (2) 5 cycles of 94°C for 10 seconds then 72°C for 4 minutes, (3) 5 cycles of 94°C for 10 seconds then 70°C for 4 minutes, and (4) 25 cycles of 94°C for 10 seconds then 68°C for 4 minutes. The reaction product was separated on a 1.5% agarose gel and stained by ethidium bromide, but no clear bands were seen. The first PCR reaction product was diluted 1:50 in water and used as template for a second PCR reaction using reverse primer LGIC-R5, which is complementary to nucleotides 88-110 of SEQ ID NO:19, having a nucleic acid sequence 5' GAG GTG GTT GTC TTC AGT GGT TG 3', denoted herein as SEQ ID NO:1934 and forward Adapter Primer 2, having a nucleic acid sequence 5' ACT CAC TAT AGG GCT CGA GCG GC 3', denoted herein as SEQ ID NO:1935 under the same reaction conditions described for the first PCR reaction. The reaction product was separated by electrophoresis on a 1.5% agarose gel and stained with ethidium bromide revealing an approximately 700 bp band. This band was cut from the gel and purified using the QIAquick Gel Extraction Kit, then ligated into the pCR II TA Cloning vector, available from Invitrogen Corporation, Carlsbad, CA, using the manufacture's protocol. This clone, referred to herein as $nCfLGIC_{613}$ and having a coding sequence denoted SEQ ID NO:1859, and a complementary strand denoted herein as SEQ ID NO:1860 was sequenced using an ABI PRISM 377 automatic DNA Sequencer, available from Perkin Elmer, Branchburg, NJ. Sequence analysis revealed that nucleotides 503-613 of $nCfLGIC_{613}$ had 100% identity with nucleotides 1-110 of SEQ ID NO:19. The two sequences were aligned to form a 2739 nucleotide contiguous sequence, referred to herein as $nCfLGIC_{2739}$, having a coding strand denoted herein as SEQ ID NO:1861 and a complementary strand denoted herein as SEQ ID NO:1863. Translation of SEQ ID NO:1861 suggests that nucleic acid molecule $nCfLGIC_{2739}$ encodes a full-length LGIC protein of 672 amino acids, referred to herein as $PCfLGIC_{672}$, having an amino acid sequence represented by SEQ ID NO:1862, assuming the initiation codon spans from nucleotide 191 through nucleotide 193 of SEQ ID NO:1861 and the termination codon spans from nucleotide 2207 through nucleotide 2209 of SEQ ID NO:1861. The coding region encoding $PCfLGIC_{672}$, is represented by nucleic acid molecule $nCfLGIC_{2016}$, having a coding strand with the nucleic acid sequence represented by SEQ ID NO:1864 and a complementary strand with nucleic acid sequence represented by SEQ ID NO:1866. The amino acid sequence of $PCfLGIC_{672}$, i.e. SEQ ID NO:1862, predicts that $PCfLGIC_{672}$ has an estimated molecular weight of about 75.5 kDa and an estimated pI of about 5.89.

Comparison of amino acid sequence SEQ ID NO:1862 with amino acid sequences reported in GenBank indicates that SEQ ID NO:1862 showed the most homology, i.e., 31.4% identity with glycine receptor Alpha 3 chain precursor cDNA from *Rattus norvegicus* (Accession # P24524). Comparison of SEQ ID NO:1864 with nucleic acid sequences reported in GenBank indicates that SEQ ID NO:1864 showed the most homology, i.e., about 43.1% identity with the *Homo sapiens* glycine receptor, alpha3 cDNA (Accession # NP006520). Percent identity calculations were performed using GCG version 9.0 using default parameters.

A LGIC nucleic acid molecule for recombinant expression of the predicted extracellular domain was produced as follows. In order to ligate the region encoding the predicted extracellular domain of the LGIC cDNA into the Insect-Select™ expression vector pIB/V5-His, two separate but overlapping DNA fragments were generated to be used as the template in the PCR overlap extension. To generate a 3' DNA fragment, a first PCR reaction was conducted using forward primer LGIC-ECD-D2F, which corresponds to nucleotides 2-25 of SEQ ID NO:19, having a nucleic acid sequence 5' CAA TTT TAA ACG CAT CCA CGA CCG 3', denoted herein as SEQ ID NO:1936, and reverse primer LGIC-ECD-RE, which is complementary to nucleotides 937-961 of SEQ ID NO:19, having a nucleic acid sequence 5'CCG **CTC GAG** CGA CCC ATT TCA CGA CTT ATT TGA ATC G 3', denoted herein as SEQ ID NO:1937 and having a *Xho*I site indicated in bold, to amplify nucleotides 2-963 from SEQ ID NO:19 which was used as template under standard PCR reaction conditions and the following thermocycling conditions: (1) 94°C for 30 seconds, (2) 25 cycles of 94°C for 10 seconds, 55°C for 10 seconds, and 72°C for 3 minutes. The products of this reaction were separated on a 1.5% agarose gel, and a band corresponding to an approximately 960 nucleotide molecule was cut from the gel and purified using the QIAquick Gel Extraction Kit as described above. To generate a 5' cDNA fragment, a second PCR reaction was conducted using reverse primer LGIC-R5 (SEQ ID NO:1934) and forward primer LGIC-ECD-FE, which corresponds to nucleotides 188-215 of SEQ ID NO:1859, having a nucleic acid sequence **5' GGA ATT** CTA AAA TGC ACA ACA AAA TCC TGG TCC TGG 3', denoted herein as SEQ ID NO:1938, and having an *Eco*RI site indicated in bold, using SEQ ID NO:1859 as the template under the thermocycling conditions described for generating the 3' fragment. The products of this reaction were separated on a 1.5% agarose gel, and a band corresponding to an approximately 425 nucleotide molecule was cut from the gel and purified using the QIAquick Gel Extraction Kit as described above.

For the PCR overlap extension reaction, the 5' and 3' cDNA fragments described above were used as the template in a PCR reaction with forward primer LGIC-ECD-FE and reverse primer LGIC-ECD-RE under the thermocycling conditions described for generating the 5' and 3' fragments. The products of this reaction were separated on a 1.5% agarose gel, and a band corresponding to an approximately 1300 nucleotide molecule, as visualized by agarose gel electrophoresis and ethidium bromide staining, referred to herein as $nCfLGIC_{1300}$, was cut from the gel and purified using the QIAquick Gel Extraction Kit as described above.

The product of the PCR overlap extension reaction was the digested with *Eco*RI and *Xho*I restriction endonucleases, available from New England BioLabs, Inc., Beverly, MA, for 18 hours at 37'. The digestion product was purified using the QIAquick Nucleotide Removal Kit, available from Qiagen, and ligated into the vector pIB/V5-His which had also been digested with EcoRI and *XhoI* and treated with shrimp alkaline phosphatase, available from New England BioLabs, Inc. for 30 minutes at 37'. Following standard transformation procedures, a bacterial clone containing the plasmid pIB/V5-His-$nCfLGIC_{1300}$ was isolated. DNA sequence analysis of pIB/V5-His-$nCfLGIC_{1300}$ confirmed that nucleotides 188-1464 of SEQ ID NO:1861 had been successfully ligated into the pIB/V5-His expression vector in frame with the C-terminal V5 epitope encoded by the vector.

<u>Example 8</u> (Reference)

This example describes the cloning and sequencing of a *C. felis* ANON/23DA nucleic acid molecule. This example also describes the expression of ANON/23DA mRNA in a variety of flea tissues.

A TA clone from the HMT EST library described in Example 1 was sequenced using standard sequencing methods and shown to have homology to an ANON/23DA gene from *Drosophila melanogaster.* This clone was digested with *EcoRI* to excise an insert about 177 nucleotides in length, referred to as flea ANON nucleic acid molecule $nCfANON_{177}$. The insert was isolated by gel purification using a Gel Purification kit, available from Qiagen. Approximately 50 ng of purified $nCfANON_{177}$ was used to construct a $^{32}P$ $\alpha$-dATP labeled DNA probe using a Megaprime DNA labeling kit, available from Amersham, using the manufacturer's protocols.

The $^{32}P$ $\alpha$-dATP labeled probe was used in a plaque lift hybridization procedure to isolate a clone from the HMT lambda-ZAP unsubtracted cDNA library described in Example 1, using standard hybridization conditions described in Example 4. Plaques that hybridized strongly to the probe were isolated and subjected to *in vivo* excision. *In vivo* excision was performed using the Stratagene Ex-Assist™ helper phage system and protocols, to convert a positive plaque to pBluescript™ plasmid DNA and sequencing of DNA was conducted following preparation with a Qiagen Qiaprep™ spin mini prep kit using the manufacturer's instructions and restriction enzyme digestion with about 1 $\mu$l of 20 U/$\mu$l each of *EcoRI* and *Xho*I, available from New England Biolabs. A clone was isolated from a secondary plaque, containing a nucleic acid molecule of about 1429 base pairs, referred to herein as $nCfANON_{1429}$, having a nucleotide sequence denoted herein as SEQ ID NO:25. The complement of SEQ ID NO:25 is represented herein as SEQ ID NO: 27. Sequencing of $nCfANON_{177}$ indicates that $nCfANON_{177}$ shared 100% identity with nucleotides 279 through 455 of SEQ ID NO:25.

Translation of SEQ ID NO:25 suggests that nucleic acid molecule $nCfANON_{1429}$ encodes a full-length ANON protein of 398 amino acids, referred to herein as $PCfANON_{398}$, having an amino acid sequence represented by SEQ ID NO:26, assuming the initiation codon spans from nucleotide 18 through nucleotide 20 of SEQ ID NO:25 and the termination codon spans from nucleotide 1212 through nucleotide 1214 of SEQ ID NO:25. The coding region encoding $PCfANON_{398}$,

is represented by nucleic acid molecule $nCfANON_{1194}$, having a coding strand with the nucleic acid sequence represented by SEQ ID NO:28 and a complementary strand with nucleic acid sequence represented by SEQ ID NO:30. The amino acid sequence of $PCfANON_{398}$, also represented as SEQ ID NO:29, predicts that $PCfANON_{398}$ has an estimated molecular weight of about 45 kDa and an estimated pI of about 8.8.

Comparison of amino acid sequence SEQ ID NO:26 with amino acid sequences reported in GenBank indicates that SEQ ID NO:26 showed the most homology, i.e., about 65% identity, with a *Drosophila melanogaster* ANON/23DA protein, GenBank Accession No. 924937. Comparison of SEQ ID NO:28 with nucleic acid sequences reported in GenBank indicates that SEQ ID NO:28 showed the most homology, i.e., about 60% identity, with a *Drosophila melanogaster* ANON/23DA nucleic acid molecule, GenBank Accession number U29170. Percent identity calculations were performed using GCG version 9.0 using default parameters.

Northern Blot analysis was conducted as described in Example 4 to determine whether ANON mRNA is expressed in only HMT tissue, only in certain stages of the flea life cycle and whether ANON mRNA expression is influenced by feeding. Total RNA was extracted from flea tissues, life stages and feeding conditions as described in Example 4. Each RNA sample was separated by gel electrophoresis, transferred to nylon membranes and hybridized with $\alpha$-$^{32}$P-ATP labeled $nCfANON_{177}$ under the Northern Blotting conditions described in Example 4. Analysis of the developed film revealed that ANON mRNA was expressed in non-HMT tissues but not in HMT tissues. ANON mRNA was also detected in all adult fleas tested regardless of feeding conditions and in the wandering larvae and pupal life stages.

Example 9 (Reference)

This example describes the cloning and sequencing of a *C. felis* malvolio nucleic acid molecule.

A TA clone from the HMT EST library described in Example 1 was digested with EcoRI to excise an insert about 432 nucleotides in length, referred to as $nCfMALV_{432}$. The insert was isolated by gel purification using a Gel Purification kit, available from Qiagen and sequenced using standard sequencing methods and shown to have homology to a malvolio gene from *Drosophila melanogaster,* hereinafter referred to as a flea MALV nucleic acid molecule.

Sequence information from $nCfMALV_{432}$ was used to design PCR primers to amplify a *C. felis* MALV nucleic acid molecule from the HMT unsubtracted library described in Example 1 using a nested PCR as follows. Sense primer MALV R1, having the nucleotide sequence 5'CCA TTA TTA ACC TGG TCG ACC AC 3', designated SEQ ID NO:41 and corresponding to nucleotides 365-387 of $nCfMALV_{432}$ and reverse primer M13 Reverse, having the nucleotide sequence 5' GGA AAC AGT ATG ACC ATG 3', designated SEQ ID NO:42 were used in a first PCR reaction using HMT unsubtracted library as the template using standard PCR reaction and thermocycling conditions, with the exception that 2 $\mu$l of template was used. The reaction product from the first PCR reaction was diluted 1:50 and used as the template in a second PCR reaction as follows. Reverse primer malvolio R2, having a nucleotide sequence 5' CGC TAT AGT CGG TAG GGT CGC 3', designated SEQ ID NO:43 and corresponding to nucleotides 239-259 of $nCfMALV_{432}$ and forward primer T3, having a nucleotide sequence 5' AAT TAA CCC TCA CTA AAG GG 3' were used in a second PCR reaction under standard PCR reaction and thermocycling conditions.

The second PCR reaction resulted in an approximately 1000 bp PCR product which was separated by electrophoresis on a 1.5% agarose gel, excised and purified using a Gel Purification Kit, available from Qiagen. The purified PCR product was ligated into the pCRII™, Original TA cloning vector, available from Invitrogen. The ligation reaction was then used to transform INV$\alpha$F' One Shot™ competent cells, available from Invitrogen, which were plated on LB agar with 50 micrograms per milliliter ($\mu$g/ml) ampicillin, available from Sigma-Aldrich Co., and 50 $\mu$g/ml X-Gal, available from Fisher Biotech. A clone was isolated from the ligation mix containing a nucleic acid molecule of about 765 base pairs, referred to herein as $nCfMALV_{765}$, having a nucleotide sequence denoted herein as SEQ ID NO:31. The complement of SEQ ID NO:31 is represented herein as SEQ ID NO:33.

Translation of SEQ ID NO:31 suggests that nucleic acid molecule $nCfMALV_{765}$, encodes a partial-length MALV protein of 254 amino acids, referred to herein as $PCfMALV_{254}$, having an amino acid sequence represented by SEQ ID NO:32, assuming the initiation codon spans from nucleotide 2 through nucleotide 4 of SEQ ID NO:31 and the last codon spans from nucleotide 761 through nucleotide 763 of SEQ ID NO:31. The coding region encoding $PCfMALV_{254}$, is represented by nucleic acid molecule $nCfMALV_{762}$, having a coding strand with the nucleic acid sequence represented by SEQ ID NO:34 and a complementary strand with nucleic acid sequence represented by SEQ ID NO:36. The amino acid sequence of $PCfMALV_{254}$, also represented as SEQ ID NO:35, predicts that $PCfMALV_{254}$ has an estimated molecular weight of about 36 kDa and an estimated pI of about 4.9.

Comparison of amino acid sequence SEQ ID NO:32 with amino acid sequences reported in GenBank indicates that SEQ ID NO:32 showed the most homology, i.e., about 71% identity, with a *Drosophila melanogaster* malvolio protein, GenBank Accession No. 780776. Comparison of SEQ ID NO:34 with nucleic acid sequences reported in GenBank indicates that SEQ ID NO:34 showed the most homology, i.e., about 63% identity, with a *Drosophila melanogaster* malvolio nucleic acid molecule, GenBank Accession number U23948. Percent identity calculations were performed using GCG version 9.0 using default parameters.

Example 10 (Reference)

This example describes the cloning, sequencing, and recombinant expression of a *C. felis* odorant-binding protein-like (OS-D) nucleic acid molecule. This example also describes the expression of OS-D mRNA in a variety of flea tissues.

A *C. felis* OS-D nucleic acid molecule of about 311 nucleotides was isolated from a cat blood-fed adult flea cDNA library, prepared as described in example 8 of PCT publication WO 96/11706 by Grieve et al., published April 25, 1996, by PCR amplification as follows. Sense primer 5'newBsaI5', having a nucleotide sequence 5' CAA AAC TGG TCT CCC CGC TC 3', denoted SEQ ID NO:57 was used in combination with vector primer T7, having a nucleic acid sequence 5' TAA TAC GAC TCA CTA TAG GG 3', denoted SEQ ID NO:58, in a first PCR reaction using the cat blood-fed adult flea cDNA library as the template under standard PCR reaction and thermocycling conditions. A 311-nucleotide fragment, denoted $nCfOSD_{311}$ was isolated and shown to encode a partial length protein of 45 amino acids having a sequence similar to *Drosophila melanogaster* OS-D protein. Since primer 5'newBsaI5' was designed to be specific for the *C. felis* serpin constant region, $nCfOSD_{311}$ is believed to have been fortuitously amplified in this PCR reaction.

To isolate a flea OS-D nucleic acid molecule encoding a full-length OS-D protein, nucleic acid molecule $nCfOSD_{311}$ was used to design primers for a nested PCR as follows. Sense primer OSD-R1, having a nucleotide sequence 5' GGT TCG CCT CTC TTC ACT TG 3', which is complementary in sequence to nucleotides 108-127 of $nCfOSD_{311}$, denoted SEQ ID NO:59, was used in combination with M13 reverse primer, SEQ ID NO:54, in a first PCR reaction using the cat blood-fed adult *C. felis* cDNA library as the template. The product of the first reaction was diluted 1:50 and used as the template for a second PCR reaction using reverse primer OSD-R2, having a nucleotide sequence 5' CGG TTG GAT CGT AAA CTG CAG 3', which is complementary in sequence to nucleotides 52-72 of $nCfOSD_{311}$, denoted SEQ ID NO:60, and forward primer T3, SEQ ID NO:56. Each PCR reaction was conducted under standard PCR reaction and thermocycling conditions with the exception that an annealing temperature of 55°C was used rather than 50°C.

A DNA fragment of about 365 nucleotides, referred to herein as $nCfOSD_{365}$, was isolated from the second PCR product and purified using a Gel Purification Kit, available from Qiagen. The purified fragment was ligated into the pCRII™ TA cloning vector, available from Invitrogen, and sequenced using standard sequencing methods. Sequencing revealed that nucleotides 294-365 of $nCfOSD_{365}$ match nucleotides 1-72 of molecule $nCfOSD_{311}$ described above. The sequences from the partial length clones described were aligned to produce a sequence including a full-length coding region of 604 nucleotides, referred to as $nCfOSD_{604}$, denoted herein as SEQ ID NO:37, where $nCfOSD_{311}$ is identical in sequence to nucleotides 294-604 of SEQ ID NO:37 and $nCfOSD_{365}$ is identical in sequence to nucleotides 1-365 of SEQ ID NO:37. The complement of SEQ ID NO:37 is represented herein as SEQ ID NO:39.

Translation of SEQ ID NO:37 suggests that nucleic acid molecule $nCfOSD_{604}$ encodes a full-length OS-D protein of 135 amino acids, referred to herein as $PCfOSD_{135}$, having an amino acid sequence represented by SEQ ID NO:38, assuming the initiation codon spans from nucleotide 26 through nucleotide 28 of SEQ ID NO:37 and the termination codon spans from nucleotide 431 through nucleotide 433 of SEQ ID NO:37. The coding region encoding $PCfOSD_{135}$, is represented by nucleic acid molecule $nCfOSD_{405}$, having a coding strand with the nucleic acid sequence represented by SEQ ID NO:40 and a complementary strand with nucleic acid sequence represented by SEQ ID NO:42. The amino acid sequence of $PCfOSD_{135}$, also represented as SEQ ID NO:41, predicts that $PCfOSD_{135}$ has an estimated molecular weight of about 15 kDa and an estimated pI of about 8.6. Analysis of SEQ ID NO:38 suggests the presence of a signal peptide encoded by a stretch of amino acids spanning from about amino acid 1 through about amino acid 20. The proposed mature protein, denoted herein as $PCfOSD_{115}$, contains about 115 amino acids corresponding to amino acids 21 through 135 of SEQ ID NO:38. The predicted pI of the mature protein (i.e. the protein with the signal peptide removed) is 6.6.

Comparison of amino acid sequence SEQ ID NO:38 with amino acid sequences reported in GenBank indicates that SEQ ID NO:38 showed the most homology, i.e., about 60% identity, with a *Schistocerca gregaria* chemosensory protein CSP-sg4, GenBank Accession No. 3283938. Comparison of SEQ ID NO:40 with nucleic acid sequences reported in GenBank indicates that SEQ ID NO:40 showed the most homology, i.e., about 58% identity, with a *Schistocerca gregaria* chemosensory protein CSP-sg4 nucleic acid molecule, GenBank Accession number AF070964. Comparison of SEQ ID NO:40 with nucleic acid molecules sequenced when screening the HNC subtracted and unsubtracted libraries described in Example 2 revealed that OS-D (i.e.

SEQ ID NO:40) is expressed in each of these libraries. Additional sequence analysis revealed that there are four cysteines present in *C. felis* OS-D which are conserved in sequence alignments with the four cysteines of OS-D-like molecules of other insects, including *D. melanogaser* OS-D protein GenBank Accession No. U02546, *S. gregaria* chemosensory protein CSP-sg4, GenBank Accession number AF070964, and cockroach leg regenerative protein, GenBank Accession No. AF030340. Percent identity calculations and additional sequence analysis was performed using GCG version 9.0 using default parameters.

A nucleic acid molecule comprising nucleotides 91 through 447 of SEQ ID NO:37, encoding a predicted mature flea OS-D protein, was PCR amplified using the pBluescript™ clone described above as the template, using sense primer OSD-FE, having nucleotide sequence 5'CGC GGA TCC AGA AGA TAA ATA TAC TAG CAA ATT TGA TAA C 3', having a *Bam*HI site indicated in bold, designated herein as SEQ ID NO:61, and anti-sense primer OSD-RE, having nucleotide sequence 5' GAG GAA TTC CTC TTT TTG GAA ATT TAA ACT GTA ACG G 3', having an *Eco*RI site indicated in bold, designated herein as SEQ ID NO:62. PCR reactions were performed using standard PCR reaction and thermocycling conditions described in Example 4; the product was separated by agarose gel electrophoresis, and a fragment was excised and purified using a Gel Purification Kit, available from Qiagen. The fragment was digested with *Bam*HI and *Eco*RI and ligated into the vector pTrcHisB, available from Invitrogen, that had been digested with *Bam*HI and *Eco*RI and treated with alkaline phosphatase. The resulting recombinant molecule, referred to herein as pTrc-nCfOSD$_{357}$, was transformed into *E. coli* strain BL21, available from Novagen, to form recombinant cell *E. coli*:pTrc-nCfOSD$_{357}$.

The recombinant cell was grown under standard conditions then incubated in the presence of 0.5 mM IPTG to induce expression of recombinant protein, predicted to be approximately 17-kDa. Expression of protein was confirmed using Coomassie-blue-stained Tris-glycine gel and by Western blot using a T7 tag antibody which showed expression of an about 17 kDa protein.

A Northern Blot analysis was conducted as follows to determine whether OS-D mRNA is expressed exclusively in HNC tissues. HNC tissues were dissected from 1500 adult cat blood-fed *C. felis* having a male to female ratio of 1: 4. Total RNA was separately extracted from HNC tissues and the HNC-less carcasses that resulted from these dissections using a standard guanidine lysis method, described by Sambrook et al., *ibid.*

Approximately 15 μg of each RNA were separated by electrophoresis on either Glyoxal gels with RNA prepared according to Bumett, Biotechniques, 22:4, pp. 668-671, 1997, or formaldehyde gels with RNA prepared according to Sambrook et al., *ibid.* Following electrophoresis, RNA was blotted to Hybond N nylon membranes, available from Amersham, according to the protocols described in Burnett and Sambrook et el. *ibid.* The membrane was UV cross-linked using the Stratalinker®, available from Stratagene, and placed in approximately 30 ml of hybridization buffer consisting of 5X SSPE, 1% Sarcosyl, 50% formamide, 5X Denhardt's reagent and 25 mM EDTA at 42°C for approximately 3 to 6 hours. A probe comprising the flea OS-D nucleic acid molecule nCfOSD$_{357}$ was labeled with α-$^{32}$P-ATP using a DNA labeling kit, available from Amersham and added to the buffer at a concentration of approximately 1 x $10^6$ cpm/ml, and allowed to hybridize for about 14 to 18 hours at 42°C. The blot was then washed twice for 10 minutes per wash in 0.5X SSPE and 0.1% sarcosyl at 55°C and exposed to film for autoradiography. Analysis of the developed film showed that there was greater expression of OS-D mRNA in HNC tissues compared to non-HNC tissues, indicating possible upregulation of OS-D in flea head and nerve cords.

Example 11 (Reference)

This example describes the cloning and sequencing of a *C. felis* N-methyl-D-aspartate receptor associated (NMDA) nucleic acid molecule.

A TA clone from the HMT EST library described in Example 1 was sequenced using standard sequencing methods and shown to have significant homology to NMDA genes. This clone was digested with *Eco*RI to excise an insert 279 nucleotides in length, referred to as flea NMDA nucleic acid molecule nCfNMDA$_{279}$. The insert was isolated by gel purification using a Gel Purification kit, available from Qiagen. Approximately 50 ng of purified nCfNMDA$_{279}$ was used to construct a $^{32}$P α-dATP labeled DNA probe using a Megaprime DNA labeling kit, available from Amersham, using the manufacturer's protocols.

The $^{32}$P α-dATP labeled probe was used in a plaque lift hybridization procedure to isolate a clone from the HMT lambda-ZAP unsubtracted cDNA library described in Example 1, using standard hybridization conditions described in Example 4. Plaques that hybridized strongly to the probe were isolated and subjected to *in vivo* excision. *In vivo* excision was performed using the Stratagene Ex-Assist™ helper phage system and protocols, to convert a positive plaque to pBluescript™ plasmid DNA and sequencing was conducted following preparation of DNA with a Qiagen Qiaprep™ spin mini prep kit using the manufacturer's instructions and restriction enzyme digestion with about 1 μl of 20 U/μl each of *Eco*RI and *Xho*I, available from New England Biolabs. A clone was isolated from a secondary plaque, containing a nucleic acid molecule of about 1227 base pairs, referred to herein as nCfNMDA$_{1227}$, having a nucleotide sequence denoted herein as SEQ ID NO:43. The complement of SEQ ID NO:43 is represented herein as SEQ ID NO: 45. Sequencing of nCfNMDA$_{279}$ indicates that nCfNMDA$_{279}$ shared 100% identity with nucleotides 709 through 987 of SEQ ID NO:43.

Translation of SEQ ID NO:43 suggests that nucleic acid molecule nCfNMDA$_{1227}$ encodes a full-length NMDA protein of 246 amino acids, referred to herein as PCfNMDA$_{246}$, having an amino acid sequence represented by SEQ ID NO:44, assuming the initiation codon spans from nucleotide 312 through nucleotide 314 of SEQ ID NO:43 and the termination codon spans from nucleotide 1050 through nucleotide 1052 of SEQ ID NO:43. The coding region encoding PCfNMDA$_{246}$, is represented by nucleic acid molecule nCfNMDA$_{738}$, having a coding strand with the nucleic acid sequence represented by SEQ ID NO:46 and a complementary strand with nucleic acid sequence represented by SEQ ID NO:48. The amino acid sequence of $PCfNMDA_{246}$, also represented as SEQ ID NO:47 predicts that $PCfNMDA_{246}$ has an estimated molecular weight of about 27 kDa and an estimated pI of about 5.6.

Comparison of amino acid sequence SEQ ID NO:44 with amino acid sequences reported in GenBank indicates that SEQ ID NO:44 showed the most homology, i.e., about 34% identity, with a *Emericella nidulans* negative-acting regulatory protein, GenBank Accession No. 3676056. Comparison of SEQ ID NO:46 with nucleic acid sequences reported in GenBank indicates that SEQ ID NO:46 showed the most homology, i.e., about 45% identity, with a *Drosophila melanogaster* NMDA nucleic acid molecule, GenBank Accession number L37377. Percent identity calculations were performed using GCG version 9.0 using default parameters.

Example 12 (Reference)

This example describes the cloning and sequencing of *C, felis* chemical sense related lipophilic ligand binding protein nucleic acid molecule. This example also describes the expression of chemical sense related lipophilic ligand binding protein mRNA in a variety of flea tissues.

A TA clone from the HNC EST library described in Example 2 was sequenced using standard sequencing methods and shown to have significant homology to chemical sense related lipophilic ligand binding protein (CLBP) genes. This clone was digested with *Eco*RI to excise an insert 339 nucleotides in length, referred to as flea CLBP nucleic acid molecule $nCfCLBP_{339}$. The insert was isolated by gel purification using a Gel Purification kit, available from Qiagen, Chatsworth, CA. Approximately 50 ng of purified $nCfCLBP_{339}$ was used to construct a $^{32}P$ $\alpha$-dATP labeled DNA probe using a Megaprime DNA labeling kit, available from Amersham, using the manufacturer's protocols.

The $^{32}P$ $\alpha$-dATP labeled probe was used in a standard plaque lift hybridization procedure to isolate a clone from the HNC lambda-ZAP unsubtracted cDNA library described in Example 2. The following hybridization conditions were used. Filters were hybridized with about 5 X $10^7$ counts per minute (cpm) per ml of the probe in 100 ml of buffer (5X SSPE, 1 % Sarcosyl, 0.1 mg/ml BLOTTO) at 45°C for about 14 hours. The filters were washed twice for 20 minutes per wash in 500 ml of 0.5X SSPE and 0.1% Sarcosyl at 55°C and subjected to autoradiography. Two plaques that hybridized strongly to the probe were isolated and subjected to *in vivo* excision using the Stratagene Ex-Assist™ helper phage system and protocols. Miniprep DNA was prepared from each positive clone using a Quantum Prep mini prep kit, available from BioRad, Hercules, CA, and sequenced using standard sequencing procedures. Sequencing revealed that the two positive clones share 97% amino acid identity to each other. The first clone contained a nucleic acid molecule of about 633 nucleotides, referred to herein as $nCfCLBP1A_{633}$, having a nucleotide sequence denoted herein as SEQ ID NO:153. The complement of SEQ ID NO:153 is represented herein as SEQ ID NO:155. The second clone contained a nucleic acid molecule of about 631 nucleotides, referred to herein as $nCfCLBP2A_{631}$, having a nucleotide sequence denoted herein as SEQ ID NO:162. The complement of SEQ ID NO:162 is represented herein as SEQ ID NO:164. Sequencing of $nCfCLBP_{340}$ indicated that $nCfCLBP_{339}$ shared 100% identity with nucleotides 1 through 339 of SEQ ID NO:153 and shared 100% identity with nucleotides 2 through 339 of SEQ ID NO: 162.

Translation of SEQ ID NO:153 suggests that nucleic acid molecule $nCfCLBP1A_{633}$ encodes a full-length CLBP protein of 147 amino acids, referred to herein as $PCfCLBP1A_{147}$, having an amino acid sequence represented by SEQ ID NO:154, assuming the initiation codon spans from nucleotide 67 through nucleotide 69 of SEQ ID NO:153 and the termination codon spans from nucleotide 511 through nucleotide 513 of SEQ ID NO:153. The coding region encoding $PCfCLBP1A_{147,}$ is represented by nucleic acid molecule $nCfCLBP1A_{441}$, having a coding strand with the nucleic acid sequence represented by SEQ ID NO:156 and a complementary strand with nucleic acid sequence represented by SEQ ID NO:158. The amino acid sequence of $PCfCLBP1A_{147}$, also represented by SEQ ID NO:157, predicts that $PCfCLBP1A_{147}$ has an estimated molecular weight of about 15 kDa and an estimated pI of about 5.

Analysis of SEQ ID NO:154 suggests the presence of a signal peptide encoded by a stretch of amino acids spanning from about amino acid 1 through about amino acid 19. The proposed mature protein, denoted herein as $PCfCLBP1A_{128}$, contains 128 amino acids which is represented herein as SEQ ID NO:160. $PCfCLBP1A_{128}$ is encoded by a nucleic acid molecule denoted $nCfCLBP1A_{384}$ having a coding strand with nucleic acid sequence SEQ ID NO:159 and a complementary strand with SEQ ID NO:161.

Translation of SEQ ID NO:162 suggests that nucleic acid molecule $nCfCLBP2A_{631}$ encodes a full-length CLBP protein of 147 amino acids, referred to herein as $PCfCLBP2A_{147}$, having an amino acid sequence represented by SEQ ID NO:163, assuming the initiation codon spans from nucleotide 65 through nucleotide 67 of SEQ ID NO:162 and the termination codon spans from nucleotide 509 through nucleotide 511 of SEQ ID NO:162. The coding region encoding $PCfCLBP2A_{147}$ is represented by nucleic acid molecule $nCfCLBP2A_{441}$, having a coding strand with the nucleic acid sequence represented by SEQ ID NO:165 and a complementary strand with nucleic acid sequence represented by SEQ ID NO:167. The amino acid sequence of $PCfCLBP2A_{147}$ predicts that $PCfCLBP2A_{147}$ has an estimated molecular weight of about 15 kDa and an estimated pI of about 5.

Analysis of SEQ ID NO:163 suggests the presence of a signal peptide encoded by a stretch of amino acids spanning from about amino acid 1 through about amino acid 19. The proposed mature protein, denoted herein as

PCfCLBP2A$_{128}$, contains about 128 amino acids which is represented herein as SEQ ID NO:169. PCfCLBP2A$_{128}$ is encoded by a nucleic acid molecule denoted nCfCLBP2A$_{384}$ having a coding strand with nucleic acid sequence SEQ ID NO:168 and a complementary strand with SEQ ID NO:170.

Comparison of amino acid sequences SEQ ID NO:154 and SEQ ID NO:163 with amino acid sequences reported in GenBank indicates that each sequence showed the most homology, i.e., about 29% identity, with a *Drosophila melanogaster* pheromone binding protein related protein 2 (PBPRP-2), GenBank Accession No. 1709595. Percent identity calculations were performed using GCG version 9.0 using default parameters. Blast comparison of nucleic acid sequences SEQ ID NO:156 and SEQ ID NO:165 with nucleic acid sequences reported in GenBank indicates that each sequence showed the most homology to a human Xp22 PAC PRCI1-5G11 nucleic acid molecule, GenBank Accession number AC002369. Pairwise identity could not be performed as the human clone in GenBank is too large to load into GCG version 9.0. Blast comparison performed using default parameters showed an insignificant level of identity of 0.87. Additional sequence analysis revealed that there are six cysteines present in C. *felis* CLBP which are conserved in sequence alignments with the six cysteines of neuronausense-related molecules in the PBP/GOBP family, including *D. melanogaser* PBPRP-2, GenBank Accession No. 1709595, and PBPRP-5, GenBank Accession No. P54195, proteins, and *Phormia regina* chemical sense related lipophilic ligand binding protein (CSRLLBP), GenBank Accession No. S65458.

A Northern Blot analysis was conducted to determine whether CLBP mRNA is expressed exclusively in HNC tissues. HNC tissues were dissected, total RNA was isolated and separated by electrophoresis as described in Example 10.

Following electrophoresis, RNA was blotted as described in Example 10 and a probe comprising clone nCfCLBP$_{340}$ labeled with $\alpha$-$^{32}$P-ATP was added to the buffer at a concentration of approximately 1 x $10^6$ cpm/ml and allowed to hybridize for about 14 to 18 hours. The blot was then washed as described in Example 10 and exposed to film for autoradiography. Analysis of the developed film showed that there was greater expression of CLBP mRNA in HNC tissues compared to non-HNC tissues, indicating possible upregulation of CLBP in flea head and nerve cords.

The coding region of nCfCLBP2A$_{631}$, i.e. SEQ ID NO:162, was PCR amplified from the pBluescript™ clone described above as the template, using sense primer 2A1 BamSen having nucleotide sequence 5' ATG GAT CCG GCA AAA TAT ACC AAA GAA GAA G 3', having a *Bam*HI site indicated in bold, designated herein as SEQ ID NO:1952, and anti-sense primer 2A1antiR1, having nucleotide sequence 5' ATG AAT TCT TAT ATT GGT ATC GCG TCC ATT 3', having a *Eco*RI site indicated in bold, designated herein as SEQ ID NO:1953. PCR reactions were performed using the following thermocycling conditions: (a) one cycle at 95°C for one minute; (b) five cycles at 94°C for ten seconds, 49°C for twenty-five seconds, and 69°C for one minute; and (c) twenty-three cycles at 94°C for ten seconds, 53°C for twenty seconds, and 69°C for seventy-five seconds, in reactions containing 0.2 mM dNTPs, 1 $\mu$M of each primer, 0.5 $\mu$l of 5U/$\mu$l *KlenTaq* Advantage polymerase, available from Clontech, 1 $\mu$l of 1 $\mu$g/$\mu$l template, and 1X *KlenTaq* buffer, hereinafter referred to as "standard PCR conditions". The PCR product was digested with *Bam*HI and *Eco*RI and ligated into the vector pTrcHisB, available from Invitrogen, that had been digested with *Bam*HI and *Eco*RI. The resulting recombinant molecule, referred to herein as pTrc- nCfCLBP2A$_{441}$, was transformed into *E. coli* strain BL21, available from Novagen Inc., Madison, WI, to form recombinant cell *E. coli*:pTrc- nCfCLBP2A$_{441}$.

The recombinant cell was grown under standard conditions and then incubated in the presence of 0.5 $\mu$M isopropylthio-$\beta$-galactoside (IPTG) to induce expression of recombinant protein. Expression was confirmed using Coomassie-blue-stained Tris-glycine gel and by Western blot using a T7 tag antibody, available from Novagen, which showed expression of an about 18 kDa protein. The protein product was purified as follows. The recombinant cells were collected by centrifugation, the supernatant was discarded and the pellets were resuspended and homogenized in 60ml (total) of 50mM Tris pH8.0 containing 50mM NaCl and 1 mM phenylmethylsulfonyl fluoride(PMSF). The sample was then passed through the microfluidizer five times, rocked at 4°C for 20 minutes, and centrifuged at 20,000 x G for 30minutes. The supernatant was collected and filtered through a 0.45um filter then run over a HiTrap Chelating column, available from Amersham Pharmacia, in 50mM Tris pH8 containing 50mM NaCl and 10mM immidazole and eluted with an increasing imidazole gradient. The recombinant protein was eluted at approximately 150mM imidazole. Fractions containing recombinant protein were pooled and concentrated using a Centricon Plus-20 (Amicon), and diafiltered into PBS. Quantification of the protein was performed by densitometry against a known standard.

<u>Example 13</u> (Reference)

This Example describes the further characterization and expression of a Sodium/Hydrogen Transporter-like cDNA isolated by EST sequencing described in Example 1.

A cDNA designated clone 2231-94 was isolated from the unsubtracted HMT library as described in Example 1. Analysis of clone 2231-94 indicated that the cDNA, denoted nCfNAH$_{2080}$, is about 2080 nucleotides in length, having a coding strand with nucleic acid sequence SEQ ID NO:1867 and a complementary sequence having SEQ ID NO:1869. Translation of SEQ ID NO:1867 suggests that nucleic acid molecule nCfNAH$_{2080}$ encodes a full-length Sodium/Hydrogen Transporter-like protein of 608 amino acids, referred to herein as $PCfNAH_{608}$, having an amino acid sequence represented by SEQ ID NO:1868, assuming the initiation codon spans from nucleotide 45 through nucleotide 47 of SEQ ID NO:1867 and the termination codon spans from nucleotide 1869 through nucleotide 1871 of SEQ ID NO:1867. The coding region encoding $PCfNAH_{608}$, is represented by nucleic acid molecule $nCfNAH_{1824}$, having a coding strand with the nucleic acid sequence represented by SEQ ID NO:1870 and a complementary strand with nucleic acid sequence represented by SEQ ID NO:1871. The amino acid sequence of SEQ ID NO:1868, predicts that $PCfNAH_{608}$ has an estimated molecular weight of about 67.9 kDa and an estimated isoelectric point (pI) of about 6.47.

Comparison of amino acid sequence SEQ ID NO:1868 with amino acid sequences reported in GenBank indicates that SEQ ID NO:1868 showed the most homology, i.e., about 67.7% identity, with a sodium hydrogen exchanger NHE1 (Accession # AAD32689.1). Comparison of SEQ ID NO:1867 with nucleic acid sequences reported in GenBank indicates that SEQ ID NO:1867 showed the most homology, i.e., about 59.5% identity, with a *Drosophila melanogaster* sodium hydrogen exchanger NHE1 (Accession # AF142676). Percent identity calculations were performed using GCG version 9.0 using default parameters.

In order to express the full-length putative NAH protein, the entire coding region was amplified by PCR and then ligated into the InsectSelect™ expression vector pIB/V5-His, available from Invitrogen, as follows. Forward primer NAH-IS-FE, which corresponds to nucleotides 42-74 of SEQ ID NO:1867, having the sequence 5'**GAC TAG T**AA AAT GGG CGT TAA AAA TAT ATA TTT ATA CTG C 3', denoted SEQ ID NO:1939 and having a *Spe*I site indicated in bold, was used in conjunction with reverse primer NAH-IS-RE, which is complementary to nucleotides 1845-1867 of SEQ ID NO:1867, having the sequence 5' CCG **CTC GAG** GTA CTG CAC GTA CTA ACG TCA TC 3', denoted SEQ ID NO: 140 and having a *Xho*I restriction site indicated in bold, in a PCR reaction using SEQ ID NO:1867 as the template. Standard PCR reaction conditions were used with the following thermocycling conditions: (1) 94°C for 30 seconds, (2) 25 cycles of 94°C for 10 seconds, 55°C for 10 seconds and 72°C for 3 minutes. The products of this reaction were separated on a 1.5% agarose gel, and a band corresponding to an approximately 1825 nucleotide molecule was cut from the gel and purified using the QIAquick Gel Extraction Kit as described above. The PCR product was then digested with *Spe*I and *Xho*I restriction endonucleases for 18 hours at 37°. The digestion product was purified using the QIAquick Nucleotide Removal Kit, available from Qiagen, and ligated into the vector pIB/V5-His which had also been digested with *Spe*I and *Xho*I and treated with shrimp alkaline phosphatase, available from New England BioLabs, Inc., for 30 minutes at 37°. Following Standard transformation procedures, a bacterial clone containing the plasmid pIB/V5- His-NAH was isolated. DNA sequence analysis of the clone confirmed that nucleotides 42-1867 of SEQ ID NO:1867, referred to herein as $nCfNAH_{1826}$, had been successfully ligated into the pIB/V5-His expression vector in frame with the C-terminal V5 epitope encoded by the vector.

A Northern Blot analysis was conducted as described in Example 4 to determine whether NAH mRNA is expressed only in certain life stages of the flea life cycle and whether NAH mRNA is expressed only in HMT tissue. Total RNA was extracted from eggs, first, third, and wandering larvae, pupae, unfed adults, and adults fed on cat blood for 0.25, 2, 8, and 24 hours. In addition, total RNA was extracted from hindguts and Malpighian tubules extracted from 24 hour cat blood-fed adult fleas, and from the remaining body parts following the removal of hindguts and Malpighian tubules. Each RNA sample was separated by gel electrophoresis, transferred to nylon membranes and hybridized with $\alpha$-$^{32}$P-ATP labeled $nCfNAH_{1826}$ under the Northern Blotting conditions described in Example 4. Analysis of the developed film revealed that NAH mRNA was expressed in the 0.25, 2, and 8 hour adult fed timepoints only.

## Example 14

This Example, according to the present invention, describes the further characterization of a Chloride Intracellular Channel-like cDNA isolated by EST sequencing described in Example 1.

A cDNA designated clone 2233-24 was isolated from the unsubtracted HMT library as described in Example 1. Analysis of clone 2233-24 indicated that the cDNA, denoted $nCfCLIC_{2283}$ is about 2283 nucleotides in length, having a coding strand with nucleic acid sequence SEQ ID NO:1872 and a complementary sequence having SEQ ID NO:1874. Translation of SEQ ID NO:1872 suggests that nucleic acid molecule $nCfCLIC_{2283}$ encodes a full-length Chloride Intracellular Channel-like protein of 262 amino acids, referred to herein as $PCfCLIC_{262}$, having an amino acid sequence represented by SEQ ID NO:1873, assuming the initiation codon spans from nucleotide 60 through nucleotide 62 of SEQ ID NO:1872 and the termination codon spans from nucleotide 846 through nucleotide 848 of SEQ ID NO:1872. The coding region encoding $PCfCLIC_{262}$, is represented by nucleic acid molecule $nCfCLIC_{786}$, having a coding strand with the nucleic acid sequence represented by SEQ ID NO:1875 and a complementary strand with nucleic acid sequence represented by SEQ ID NO:1876. The amino acid sequence of SEQ ID NO:1873, predicts that $PCfCLIC_{262}$ has an estimated molecular weight of about 30.2 kDa and an estimated isoelectric point (pI) of about 6.02.

Comparison of amino acid sequence SEQ ID NO:1873 with amino acid sequences reported in GenBank indicates that SEQ ID NO:1873 showed the most homology, i.e., about 37.8% identity, with a *Homo sapiens* chloride intracellular channel 2 (Accession # NP001280.1). Comparison of SEQ ID NO:1872 with nucleic acid sequences reported in GenBank indicates that SEQ ID NO:1872 showed the most homology, i.e., about 37.5% identity with a *Homo sapiens* chloride intracellular channel 2 (Accession # NM001289). Percent identity calculations were performed using GCG version 9.0 using default parameters.

Example 15 (Reference)

This Example describes the further characterization of a Peritrophin-like DNA, referred to herein as PL2, isolated by EST sequencing described in Example 1.

A cDNA designated clone 2232-23 was isolated from the unsubtracted HMT library as described in Example 1, denoted herein as SEQ ID NO:1877. Analysis of clone 2232-23 indicated that the cDNA, denoted $nCfPL2_{457}$ is about 457 nucleotides in length. Translation of the coding strand of $nCfPL2_{457}$ suggests that nucleic acid molecule $nCfPL2_{457}$ encodes a partial-length Peritrophin-like protein of 113 amino acids, referred to herein as $PCfPL2_{113}$, assuming a stop coding at nucleotides 342-344 of $nCfPL2_{457}$.

Additional coding sequence corresponding to the 5' end of $nCfPL2_{457}$ was isolated by PCR performed using a RACE cDNA pool prepared as described in Example 3 as template. A first PCR reaction was performed using reverse primer PL2-R1, which is complementary to nucleotides 167-187 of the $nCfPL2_{457}$ cDNA, having a nucleic acid sequence 5' GTC TGG AAG CTC AGG AAG AGG 3', denoted herein as SEQ ID NO:1941, in conjunction with forward Adapter Primer 1, SEQ ID NO:1933, described above under the following thermocycling conditions: (1) 94°C for 30 seconds, (2) 5 cycles of 94°C for 10 seconds and 72°C for 4 minutes, (3) 5 cycles of 94°C for 10 seconds and 70°C for 4 minutes, and (4) 25 cycles of 94°C for 10 seconds then 68°C for 4 minutes. The product of this reaction was diluted 1:50 and used as template for a second PCR reaction as follows. Forward adapter primer 2, SEQ ID NO: 1935, was used with reverse primer PL2-R2, which is complementary to nucleotides 29-52 of the $nCfPL2_{457}$ cDNA, having a nucleic acid sequence 5' GTA ATA TGC GTG ACA ATC GTG TGG 3', denoted herein as SEQ ID NO: 1942, using the thermocycling conditions described for the first PCR reaction. The resulting product was gel purified as described above to reveal a distinct band corresponding to nucleic acid molecule of approximately 900 bp in length. The fragment was then ligated into the pCR II TA Cloning vector, available from Qiagen and sequenced using an ABI PRISM 377 automatic DNA Sequencer. Sequencing revealed that nucleotides 791-835 of the fragment had 100% identity with nucleotides 1-45 of the $nCfPL2_{457}$ cDNA. The two sequences were aligned to form a contiguous sequence, denoted $nCfPL2_{1291}$, which is about 1291 nucleotides in length, having a coding strand with nucleic acid sequence SEQ ID NO:1878 and a complementary sequence having SEQ ID NO: 1879. Translation of SEQ ID NO: 1878 suggests that nucleic acid molecule $nCfPL2_{1291}$ encodes a non full-length Peritrophin-like protein of 391 amino acids.

In order to isolate the additional sequence corresponding to the 5' end of SEQ ID NO: 1878, nested PCR reactions were performed using the RACE cDNA pool as template. For the first PCR, forward adapter primer AP 1 was used with reverse primer PL2-R1 under standard PCR reaction conditions and the following thermocycling conditions: (1) 94° C for 1 minute, (2) 5 cycles of 94° C for 20 seconds and 70° C for 1 minute, (3) 5 cycles of 94° C for 20 seconds and 68° C for 1 minute, (4) 10 cycles of 94° C for 20 seconds and 66° C for 1 minute. The products of this reaction were diluted 1:50 in water and used as template for the second, nested PCR. The second PCR reaction used forward adapter primer AP2 in conjunction with reverse primer PL2-R5, which is complementary to nucleotides 70-93 of SEQ ID NO: 1878, having a nucleotide sequence 5' CGG TGC AAG TTA TAG AAC CTT CCG 3', denoted herein as SEQ ID NO: 1943 under standard PCR reaction conditions using the following thermocycling conditions: (1) 94° C for 1 minute, (2) 5 cycles of 94° C for 20 seconds and 70° C for 1 minute, (3) 5 cycles of 94° C for 20 seconds and 68° C for 1 minute, (4) 40 cycles of 94' C for 20 seconds and 66° C for 1 minute. The products of this reaction were separated by agarose gel electrophoresis and a band approximately 279 nucleotides in length was excised from the gel and purified as described above. The fragment, referred to as $nCfPL2_{279}$, having a coding nucleic acid sequence designated SEQ ID NO:1880 and a complementary sequence designated SEQ ID NO:1881, was then ligated into the pCROII TA Cloning vector, available from Qiagen, and sequenced as described above. Sequencing revealed that nucleotides 228-279 of $nCfPL2_{279}$ were identical to nucleotides 42-93 of SEQ ID NO: 1878, however, nucleotides 186-228 of $nCfPL2_{279}$ had no significant similarity to SEQ ID NO: 1878. This discrepancy may be the result of alternative RNA splicing or may be an artifact of the cDNA pool. To determine the reason for this discrepancy, additional fragments corresponding to this region were isolated by PCR from flea cDNA libraries from adult midguts, hindgut and Malpighian tubules and mixed instar larvae using techniques described herein. Sequence analysis of fragments obtained from these libraries revealed that these fragments were identical in sequence to the sequence of $nCfPL2_{279}$, therefore, the region of SEQ ID NO:1878 which did not align to $nCfPL2_{279}$ was deemed to be an artifact and was not used in subsequent alignments.

The PL2 sequences described above were aligned to form a contiguous sequence, denoted $nCfPL2_{1477}$, which is about 1477 nucleotides in length, having a coding strand with nucleic acid sequence SEQ ID NO:1882 and a complementary sequence having SEQ ID NO: 1884. Translation of SEQ ID NO: 1882 suggests that nucleic acid molecule $nCfPL2_{1477}$ encodes a full-length Peritrophin-like protein of 453 amino acids, referred to herein as $PCfPL2_{453}$, having an amino acid sequence represented by SEQ ID NO:1883, assuming an initiation codon spanning from nucleotide 3 through nucleotide 5 of SEQ ID NO:1882 and a termination codon spanning from nucleotide 1362 through nucleotide 1364 of SEQ ID NO: 1882. The coding region encoding $PCfPL2_{453}$, is represented by nucleic acid molecule $nCfPL2_{1359}$, having a coding strand with the nucleic acid sequence represented by SEQ ID NO:1885 and a complementary strand with nucleic acid sequence represented by SEQ ID NO: 1886. The amino acid sequence of SEQ ID NO: 1883, predicts that $PCfPL2_{453}$ has an estimated molecular weight of about 49 kDa and an estimated isoelectric point (pI) of about 4.7.

Comparison of amino acid sequence SEQ ID NO:1883 with amino acid sequences reported in GenBank indicates that SEQ ID NO:1883 showed the most homology, i.e., about 28% identity, with a *Drosophila melanogaster* locus AE003474 protein (Accession # AAF47629). Comparison of SEQ ID NO:1882 with nucleic acid sequences reported in GenBank indicates that SEQ ID NO:1882 showed the most homology, i.e., about 50% identity, *Penaeus semisulcatus* (a crustacean) peritrophin-like protein 1 cDNA (Accession # AF095580). Percent identity calculations were performed using GCG version 9.0 using default parameters.

Example 16 (Reference)

This Example describes the further characterization and expression of a Peritrophin-like sequence cDNA, referred to herein as PL3, isolated by EST sequencing described in Example 1.

A cDNA designated clone 2240-17 was isolated from the unsubtracted HMT library as described in Example 1. Analysis of clone 2240-17 indicated that the cDNA, denoted $nCfPL3_{406}$, is about 406 nucleotides in length, having a coding strand with nucleic acid sequence SEQ ID NO:1887 and a complementary sequence having SEQ ID NO:1889. Translation of SEQ ID NO:1887 suggests that nucleic acid molecule $nCfPL3_{406}$ encodes a full-length Peritrophin-like protein of 81 amino acids, referred to herein as $PCfPL3_{81}$, having an amino acid sequence represented by SEQ ID NO: 1888, assuming the initiation cordon spans from nucleotide 20 through nucleotide 22 of SEQ ID NO:1887 and the termination codon spans from nucleotide 263 through nucleotide 265 of SEQ m NO:1887. The coding region encoding $PCfPL3_{81}$,is represented by nucleic acid molecule $nCfPL3_{243}$, having a coding strand with the nucleic acid sequence represented by SEQ ID NO:1890 and a complementary strand with nucleic acid sequence represented by SEQ ID NO: 891. The amino acid sequence of SEQ ID NO: 1888, predicts that $PCfPL3_{81}$ has an estimated molecular weight of about 9.1 kDa and an estimated isoelectric point (pI) of about 3.64.

Comparison of amino acid sequence SEQ ID NO:1888 with amino acid sequences reported in GenBank indicates that SEQ ID NO:1888 showed the most homology, i.e., about 34.2% identity, with a *Anopheles gambiae* peritrophin 1 protein (Accession # AAC39127). Comparison of SEQ ID NO:187 with nucleic acid sequences reported in GenBank indicates that SEQ ID NO:1887 showed the most homology, i.e., about 37% identity, with a *Anopheles gambiae* chloride intracellular channel 2 (Accession # AF030431). Percent identity calculations were performed using GCG version 9.0 using default parameters.

In order to express the full-length putative PL3 protein, the entire coding region was amplified by PCR and then ligated into the *E. coli* expression vector pTrcHisB, available from Invitrogen, as follows. Forward primer PL3FE, which corresponds to nucleotides 70-93 of SEQ ID NO:1887, having the sequence 5' CGG **GAT** CCC GAA TAT GCT GAC GTA GAT GTG TG 3', denoted SEQ ID NO:1944, and having a *Bam*HI restriction endonuclease site indicated in bold, was used in conjunction with reverse primer PL3RE, which is complementary to nucleotides 245-269 of SEQ ID NO: 1887, having the sequence 5' G**GA ATT CTG** TTT TAT TCT GGT TGG TAA CAT TC 3', denoted herein as SEQ ID NO:1945 and having an *EcoRI* restriction endonuclease site indicated in bold, in a PCR reaction using SEQ ID NO:1887 as the template under standard PCR reaction conditions and the following thermocycling conditions: (1) 94°C for 30 seconds, (2) 25 cycles of 94°C for 10 seconds, 55°C for 10 seconds and 72°C for 3 minutes. The reaction product was separated on a 1.5% agarose gel, and a band corresponding to an approximately 200 nucleotide molecule, as visualized by agarose gel electrophoresis and ethidium bromide staining, was cut from the gel and purified using the QIAquick Gel Extraction Kit as described above.

The product of the PCR reaction was the digested with *Bam*HI and *EcoRI* restriction endonucleases, available from New England BioLabs, Inc. for 18 hours at 37° C, purified using the QIAquick Nucleotide Removal Kit, available from Qiagen, and ligated into the vector pTrcHisB which had been similarly digested, treated with shrimp alkaline phosphatase, available from New England BioLabs, Inc., for 30 minutes at 37°C, and purified. Following standard transformation procedures into *E. coli* BL-21 competent cells, a bacterial clone containing the plasmid pTrcHisB-PL3 was isolated. DNA sequence analysis of the clone confirmed that 70-269 of SEQ ID NO:1887 had been successfully ligated into the pTrcHisB expression vector in frame with the N-terminal T7 Tag epitope encoded by the vector. The recombinant protein encoded thereby is predicted to be 97 amino acids in length and have a molecular mass of 10.9 kDa, including the T7 Tag and have a pI of 4.08.

A recombinant PL3 protein was expressed as follows. Five mls of Luria broth were innoculated with a glycerol stock of *E. coli* BL-21 competent cells, available from Novagen, Madison, WI, that had been transformed with the pTrcHisB-PL3 plasmid prepared as described above and allowed to grow overnight at 37°C under selection with 100 μg/ml ampicillin. A 1 ml aliquot of this culture was then used to inoculate 10 mls of fresh Luria broth containing 100 μg/ml

ampicillin and the culture was allowed to grow to an approximate OD reading of 0.5. A 1 ml aliquot of the culture was removed, the cells were pelleted by centrifugation and the supernatant discarded. The cells were resuspended in a solution of 100 μl PBS and 100 μl of 2X SDS-PAGE loading buffer (100 mM Tris pH 6.8, 4% SDS, 20% glycerol, 0.02% bromophenol blue, and 10% 2-mercaptoethanol). Following removal of the 1 ml aliquot described above, IPTG was added to the remaining 9 ml culture to a final concentration of 5 mM of IPTG, the culture was incubated at 37°C for an additional 60 minutes, 1 ml was removed and the OD measured at approximately 0.6. The cells in this 1 ml sample were then pelleted by centrifugation and resuspended in a solution of 120 μl of PBS and 120 μl of SDS-PAGE loading buffer. Equal volumes of the IPTG-induced and uninduced lysates were loaded onto a 14% Tris-Glycine SDS-PAGE gel, available from Novex, San Diego, CA. Following electrophoresis, the proteins were transferred from the SDS-PAGE gel to a nitrocellulose membrane and a Western blot analysis was performed using the T7 tag antibody, available from Novagen, which revealed an approximately 18 kDa protein was induced by IPTG. The fact that the recombinant PL3 protein ran at a higher molecular weight than predicted is consistent with previous published results for other peritrophin proteins, and is thought to be due in part to the characteristically low pI of these proteins (Tellam et al., (1999) Peritrophic Matrix Proteins, Insect Biochemistry and Molecular Biology, 29:87-101). Sequence analysis of this protein indicates that it contained the N-terminal T7 Tag encoded by the vector.

Four flasks, each containing 1 liter of Luria broth with 100 μg/ml ampicillin were inoculated with a starter culture of 5 ml of *E. coli* BL-21 cells transformed with the pTrcHisB-PL3 plasmid as described above. The cultures were allowed to grow at 37°C until the optical density reached approximately 0.500, at which time a 1 ml aliquot was removed from each flask as the pre-induction sample. IPTG was added to each 1 liter flask to a final concentration of 0.5 mM and the cultures allowed to grow at 37°C for 135 additional minutes, at which time a 1 ml aliquot was removed from each flask as the post-induction sample. The 1 ml aliquots were centrifuged, the supernatants were discarded and the pellets were resuspended in 100μl 2X SDS-PAGE loading buffer per each 0.5 optical density units measured. The pre-induction and post induction samples were then tested for recombinant PL3 protein expression using the standard Western blot techniques and the T7 Tag antibody described above. A protein running at approximately 18 kDa was detected in the post-induced but not in the pre-induced samples.

The cells from the remaining 4 liters of culture were centrifuged, the supernatants were discarded and the cell pellets were combined and resuspended in 120 mls of buffer A (50 mM Tris, PH 8.0, 20 mM NaCl, 1 mM phenylmethylsulfonyl fluoride (PMSF)). The sample was then passed through a microfluidizer five times then rocked at 4°C for 20 minutes. The sample was then centrifuged for 30 minutes and the supernatant collected. Western blot analysis of the supernatant showed that the recombinant PL3 protein was soluble in the first buffer A extraction. The buffer A supernatant containing the recombinant PL3 protein was then further purified by a nickel column, a Q2 anion exchange chromatography column, and cation exchange chromatography, using techniques well known to those of skill in the art.

Example 17 (Reference)

This Example describes the further characterization of a Peritrophin-like sequence cDNA, referred to herein as PL4, isolated by EST sequencing described in Example 1.

A cDNA designated clone 2244-71 was isolated from the unsubtracted HMT library as described in Example 1. Analysis of clone 2244-71 indicated that the cDNA, denoted $nCfPL4_{974}$, is about 974 nucleotides in length, having a coding strand with nucleic acid sequence SEQ ID NO:1892 and a complementary sequence having SEQ ID NO: 1893. Translation of SEQ ID NO: 1892 suggests that nucleic acid molecule $nCfPL4_{974}$ encodes a partial-length Peritrophin-like protein of 285 amino acids. Additional sequence corresponding to the 5' end was isolated by PCR using the RACE cDNA pool described in Example 3 as the template, as follows. Adapter Primer 1, i.e. SEQ ID NO: 1933, was used as the forward primer in conjunction with reverse primer PL4-R1, which is complementary to nucleotides 229-251 of SEQ ID NO:1892, having a nucleic acid sequence 5' GAT ATC CAC TTT GAT CAG CGC AC 3', denoted herein as SEQ ID NO:1946 in a PCR reaction under standard PCR reaction conditions and the following thermocycling conditions: (1) 94°C for 30 seconds, (2) 5 cycles of 94°C for 10 seconds and 72°C for 4 minutes, (3) 5 cycles of 94°C for 10 seconds and 70°C for 4 minutes, (4) 25 cycles of 94°C for 10 seconds then 68°C for 4 minutes. The products of this reaction were diluted 1:50 and used as template in a second PCR reaction using Adapter Primer 2, i.e. SEQ ID NO:1935 as the forward primer and reverse primer PL4-R2, which is complementary to nucleotides 58-78 of SEQ ID NO:1892, having a nucleic acid sequence 5' GGT ACT ACT CCT GGT GCG GGC 3', denoted herein as SEQ ID NO: 1947, using the thermocycling conditions described for the first PCR reaction. The products of this reaction were gel purified as previously described and the fragment was ligated into the pCR II TA Cloning vector, available from Qiagen, and sequenced to reveal of fragment of approximately 150 nucleotides in length. Sequence analysis revealed that nucleotides 68-146 of the fragment had 100% identity with nucleotides 1-79 of $nCfPLA_{974}$. The two sequences were aligned to form a contiguous sequence of about 1043 nucleotides in length, referred to as $nCfPL4_{1043}$, having a coding strand with SEQ ID NO:1894 and a complementary strand having SEQ ID NO: 1895. However, the contiguous sequence does not appear to encode a starting methionine in the predicted protein sequence, thus, a second attempt to isolate the remaining coding sequences at the 5' end was performed as follows. A first PCR reaction was performed with Adapter Primer 1 as the forward primer and PL4-R2 as the reverse primer using the RACE cDNA pool as the template under the thermocycling conditions described above. The products of this reaction were diluted 1:50 and used as the template in a second PCR reaction which used Adapter Primer 2 as the forward primer and reverse primer PL4-R4, which is complementary to nucleotides 58-80 of SEQ ID NO:1894, having the nucleic acid sequence 5'CCG TCG ACA TTA AAC TCA CCA TC 3', denoted SEQ ID NO: 1948, under the thermocycling conditions described for the first PCR reaction. The products of this reaction were gel purified as previously described and the fragment was ligated into the pCR II TA Cloning vector, available from Qiagen, and sequenced to reveal of fragment of approximately 100 nucleotides in length. Sequence analysis revealed that nucleotides 21-101 of the fragment had 100% identity with nucleotides 1-81 of SEQ ID NO: 1892. The two sequences were aligned to form a contiguous sequence that is 1062 nucleotides in length, referred to herein as $nCfPL4_{1062}$, having a coding strand with SEQ ID NO:1896 and a complementary strand with SEQ ID NO: 1898. Translation of SEQ ID NO: 1896 suggests that nucleic acid molecule $nCfPL4_{1062}$ encodes a full-length Peritrophin-like protein of 285 amino acids, referred to herein as $PCfPLA_{285}$, having an amino acid sequence represented by SEQ ID NO:1897, assuming the initiation codon spans from nucleotide 19 through nucleotide 21 of SEQ ID NO:1896 and the termination codon spans from nucleotide 874 through nucleotide 876 of SEQ ID NO: 1896. The coding region encoding $PCfPL4_{285}$, is represented by nucleic acid molecule $nCfPL4_{855}$, having a coding strand with the nucleic acid sequence represented by SEQ ID NO: 1899 and a complementary strand with nucleic acid sequence represented by SEQ ID NO: 1900. The amino acid sequence of SEQ ID NO: 1897, predicts that $PCfPL4_{285}$ has an estimated molecular weight of about 31.4 kDa and an estimated isoelectric point (pI) of about 6.99.

Comparison of amino acid sequence SEQ ID NO:1897 with amino acid sequences reported in GenBank indicates that SEQ ID NO:1897 showed the most homology, i.e., about 31.5% identity, with a *Drosophila melanogaster* Gasp precourser (Accession # AAD09748). Comparison of SEQ ID NO:1896 with nucleic acid sequences reported in GenBank indicates that SEQ ID NO:1896 showed the most homology, i.e., about 39.4% identity, with a *Drosophila melanogaster* Gasp precourser (Accession #AF070734). Percent identity calculations were performed using GCG version 9.0 using default parameters.

A Northern Blot analysis was conducted as described in Example 4 to determine whether PL4 mRNA is expressed only in certain life stages of the flea life cycle and whether PL4 mRNA is expressed only in HMT tissue. Total RNA was extracted from eggs, first, third, and wandering larvae, pupae, unfed adults, and adults fed on cat blood for 0.25, 2, 8, and 24 hours. In addition, total RNA was extracted from hindguts and Malpighian tubules extracted from 24 hour cat blood-fed adult fleas, and from the remaining body parts following the removal of hindguts and Malpighian tubules. Each RNA sample was separated by gel electrophoresis, transferred to nylon membranes and hybridized with $\alpha$-$^{32}$P-ATP labeled $nCfPL4_{974}$ under the Northern Blotting conditions described in Example 4.

The results of the Northern blot assay are complex. Although stringent conditions were used, several bands with distinct expression patterns were seen. An approximately 1600 bp message was detected in the egg, first instar, third instar and wandering larval stages only. An approximately 1500 bp message was detected in all lifestages and adult fed timepoints, but with the strongest signals in the egg, first instar larval, and unfed adult stages. A third message, which ran approximately 1200 bp, was detected in the egg, first instar larval, pupal, and adult lifestages, including all unfed and fed adult timepoints. All three of the messages detected were seen only in the HMT tissues, and were not detected in the carcass tissues.

The detection of three mRNAs instead of one may be the result of the expression of three highly homologous transcripts. It has been reported in the literature that peritrophin gene families have been found that consist of a number of highly related genes (See Schorderet et al., 1998, cDNA and deduced amino acid sequences of a peritrophic membrane glycoprotein, 'peritrophin-48', from the larvae of Lucilia cuprina, Insect Biochemistry and Molecular Biology 28, 99-111). It is possible that these transcripts represent the products of such a family or that the messages are the RNA products of alternative splicing of a single gene locus.

Example 18 (Reference)

This Example describes the further characterization of a synaptic vesicle 2B-like sequence cDNA, isolated by EST sequencing described in Example 1.

A cDNA designated clone 2104-59 was isolated from the subtracted HMT library as described in Example 1, denoted herein as SEQ ID NO:358. DNA from clone 2104-59 was purified, and the insert used for plaque hybridization screening of the unsubtracted HMT cDNA library as follows. The insert from clone 2104-59 was excised by digestion with *Eco*RI, separated by agarose gel electrophoresis and purified using the QiaQuick Gel Extraction kit, available from Qiagen. A Megaprime DNA labeling kit, available from Amersham Pharmacia, was used to incorporate $\alpha$-$^{32}$P-labeled dATP into the random-primed probe mix. Hybridization and plaque purification were performed as previously described which resulted in the isolation of a clone containing an about 1875 nucleotide synaptic vesicle 2B-like sequence, referred to herein as $nCfSVP_{1875}$, having a coding strand with nucleic acid sequence SEQ ID NO:1901 and a complementary sequence having SEQ ID NO:1903. Translation of SEQ ID NO:1901 suggests that nucleic acid molecule $nCfSVP_{1875}$ encodes a full-length synaptic vesicle 2B-like protein of 530 amino acids, referred to herein as $PCfSVP_{530}$, having an ammo acid sequence represented by SEQ ID NO:1902, assuming the initiation codon spans from nucleotide 44 through nucleotide 46 of SEQ ID NO:1901 and the termination codon spans from nucleotide 1634 through nucleotide 1636 of SEQ ID NO:1901. The coding region encoding $PCfSVP_{530}$, is represented by nucleic acid molecule $nCfSVP_{1590}$, having a coding strand with the nucleic acid sequence represented by SEQ ID NO:1904 and a complementary strand with nucleic acid sequence represented by SEQ ID NO: 1905. The amino acid sequence of SEQ ID NO: 1902, predicts that $PCfSVP_{530}$ has an estimated molecular weight of about 58.7 kDa and an pI of about 7.61.

Comparison of amino acid sequence SEQ ID NO:1902 with amino acid sequences reported in GenBank indicates that SEQ ID NO:1902 showed the most homology, i.e., about 32% identity, with a *Drosophila melanogaster* BACR7A4.y (Accession # CAB51685). Comparison of SEQ ID NO:1901 with nucleic acid sequences reported in GenBank indicates that SEQ ID NO:1901 showed the most homology, i.e., about 39% identity, with a *Rattus norvegicus* synaptic vesicle protein 2B (SVP2B) mRNA (Accession # L10362). Percent identity calculations were performed using GCG version 9.0 using default parameters.

Example 19 (Reference)

This Example describes the further characterization of a Voltage-Gated Chloride Channel-like sequence cDNA, isolated by EST sequencing described in Example 1.

A cDNA designated clone 2108-09 was isolated from the unsubtracted HMT library as described in Example 1. Analysis of clone 2108-09 indicated that the cDNA, denoted $nCfVGCC_{381}$, is about 381 nucleotides in length, having a coding strand with nucleic acid sequence SEQ ID NO:1906 and a complementary sequence having SEQ ID NO: 1907. Translation of SEQ ID NO: 1906 suggests that nucleic acid molecule $nCfVGCC_{381}$ encodes a partial-length Voltage-Gated Chloride Channel-like protein of 126 amino acids. Additional sequence corresponding to the 5' end was isolated by hybridization and PCR as follows.

The insert from clone 2108-09 was excised by digestion with *Eco*RI, separated by agarose gel electrophoresis and purified using the QiaQuick Gel Extraction kit, available from Qiagen. A Megaprime DNA labeling kit, available from Amersham Pharmacia, was used to incorporate $\alpha$-$^{32}$P-labeled dATP into the random-primed probe mix. Hybridization and plaque purification were performed on the unsubtracted HMT cDNA library as previously described which resulted in the isolation of a clone containing an about 2191 nucleotide VGCC-like sequence, referred to herein as $nCfVGCC_{2191}$, having a coding strand with nucleic acid sequence SEQ ID NO:1908 and a complementary sequence having SEQ ID NO: 1909. Translation of SEQ ID NO: 1908 suggests that nucleic acid molecule $nCfVGCC_{2191}$ encodes a partial s VGCC-like protein of 595 amino acids.

In order to isolate the remaining coding regions at the 5' end, a PCR was performed using the RACE cDNA pool, prepared as described in Example 3, as the template as follows. Adapter Primer 1 was used as the forward primer in conjunction with reverse primer VGCC-R1, which is complementary to the nucleotides 1482-1503 of SEQ ID NO: 1908, having a nucleic acid sequence 5' CGA TCA TGC GTC TAG CAT TGG C 3', denoted herein as SEQ ID NO:1949 under standard PCR reaction conditions and the following thermocycling conditions: (1) 94°C for 30 seconds, (2) 5 cycles of 94°C for 10 seconds and 72°C for 4 minutes, (3) 5 cycles of 94°C for 10 seconds and 70°C for 4 minutes, (4) 25 cycles of 94°C for 10 seconds and 68°C for 4 minutes. The reaction products were separated on an agarose gel and a band corresponding to an approximately 1970 nucleotide molecule was isolated, purified using a Gel Purification Kit, available from Qiagen, ligated into the pCR II TA cloning vector, available from Invitrogen, and sequenced using an ABI PRISM 377 automatic DNA Sequencer. Sequence analysis revealed an approximately 1968 nucleotide fragment, referred to as $nCfVGCC_{1968}$, having a coding strand with SEQ ID NO:1910 and a complementary strand with SEQ ID NO:1911. Sequence analysis also revealed that nucleic acid molecule $nCfVGCC_{1968}$ does not encode a start codon, thus, a second 5' RACE PCR was performed as follows in order to isolate additional sequence. Adapter Primer 1 was used as the forward primer in conjunction with reverse primer VGCC-R4 primer which is complementary to nucleotides 350-372 of SEQ ID NO:1910, having a nucleic acid sequence 5' CCC GCC CCA GTT CTA GGT TGT CC 3', denoted herein as SEQ ID NO:1950, using the RACE cDNA pool prepared as described in Example 3 as the template, and the PCR reaction and thermocycling conditions as described for the first PCR reaction. The products of this reaction were then diluted 1: 50 in water and used as the template in a second PCR reaction with Adapter Primer 2 as the forward primer in conjunction with reverse primer VGCC-R2, which is complementary to nucleotides 134-153 of SEQ ID NO:1910, having a nucleic acid sequence 5' CAC ACC CAA CCT GAC CAG GC 3', denoted herein as SEQ ID NO:1951, under the PCR reaction and thermocycling conditions as described for the first PCR reaction.

The products of this reaction were gel purified as previously described and the fragment was ligated into the pCR II TA Cloning vector, available from Qiagen, and sequenced to reveal of fragment of approximately 673 nucleotides in length, referred to herein as $nCfVGCC_{673}$, having a coding strand with SEQ ID NO:1912 and a complementary strand with SEQ ID NO:1913. Sequence analysis revealed that nucleotides 520-673 of the fragment had 100% identity with nucleotides 1-154 of SEQ ID NO:1910. The VGCC fragments were aligned to form a contiguous sequence that is 3126 nucleotides in length, referred to herein as $nCfVGCC_{3126}$, having a coding strand with SEQ ID NO:1914 and a complementary strand with SEQ ID NO:1916. Translation of SEQ ID NO:1914 suggests that nucleic acid molecule $nCfVGCC_{3126}$ encodes a full-length VGCC-like protein of 851 amino acids, referred to herein as $PCfVGCC_{851}$, having an amino acid sequence represented by SEQ ID NO:1915, assuming the initiation codon spans from nucleotide 168 through nucleotide 170 of SEQ ID NO:1914 and the termination codon spans from nucleotide 2721 through nucleotide 2723 of SEQ ID NO: 1914. The coding region encoding $PCfVGCC_{851}$ is represented by nucleic acid molecule $nCfVGCC_{2553}$, having a coding strand with the nucleic acid sequence represented by SEQ ID NO:1917 and a complementary strand with nucleic acid sequence represented by SEQ ID NO:1918. The amino acid sequence of SEQ ID NO:1915, predicts that $PCfVGCC_{851}$ has an estimated molecular weight of about 93.4 kDa and an estimated pI of about 7.35. Comparison of amino acid sequence SEQ ID NO:1915 with amino acid sequences reported in GenBank indicates that SEQ ID NO:1915 showed the most homology, i.e., about 63.1 % identity, with a *Oryctolagus cuniculus* (rabbit) chloride channel protein 3 (CLCN3) (Accession # AAB95163). Comparison of SEQ ID NO:1914 with nucleic acid sequences reported in GenBank indicates that SEQ ID NO:1914 showed the most homology, i.e., about 61.3% identity, with a *Oryctolagus cuniculus* chloride channel protein 3 (CLCN3) mRNA (Accession # AF029348). Percent identity calculations were performed using GCG version 9.0 using default parameters.

A Northern Blot analysis was conducted as described in Example 4 to determine whether VGCC mRNA is expressed only in certain life stages of the flea life cycle and whether VGCC mRNA is expressed only in HMT tissue. Total RNA was extracted from eggs, first, third, and wandering larvae, pupae, unfed adults, and adults fed on cat blood for 0.25, 2, 8, and 24 hours. In addition, total RNA was extracted from hindguts and Malpighian tubules extracted from 24 hour cat blood-fed adult fleas, and from the remaining body parts following the removal of hindguts and Malpighian tubules. Each RNA sample was separated by gel electrophoresis, transferred to nylon membranes and hybridized with $\alpha$-$^{32}$P-ATP labeled $nCfVGCC_{381}$ under the Northern Blotting conditions described in Example 4. An approximately 3 kB band was detected in all lifestages and adult unfed and fed timepoints, however, the intensity of the signal did vary between stages with the strongest signals seen in the egg, unfed adult, and 0.25 hour fed adult stages, and the weakest signals seen in the 3rd instar larval and pupal stages. A strong signal was detectable in the 24 hour fed adult HMT tissues, but only a very weak signal was present in the carcass tissues.

Example 20 (Reference)

This Example describes the further characterization and expression of an Intersectin-like cDNA isolated by EST sequencing described in Example 2.

A cDNA designated clone 2225-23 was isolated from the unsubtracted HNC library as described in Example 2, denoted herein as SEQ ID NO:121. A Northern Blot analysis was conducted as described in Example 10 to determine whether clone 2225-23 mRNA is expressed exclusively in HNC tissues. For the hybridization step, a probe comprising the flea clone 2225-23 nucleic acid molecule was labeled with $\alpha$-$^{32}$P-ATP using a DNA labeling kit, available from Amersham and added to the buffer at a concentration of approximately $1 \times 10^6$ cpm/ml, and allowed to hybridize for about 14 to 18 hours at 42°C. The blot was then washed twice for 10 minutes per wash in 0.5X SSPE and 0.1% sarcosyl at 55°C and exposed to film for autoradiography. Analysis of the developed film showed that there was greater expression of clone 2225-23 mRNA in HNC tissues compared to non-HNC tissues, indicating possible upregulation of clone 2225-23 in flea head and nerve cords.

Example 21 (Reference)

This Example describes the further characterization and expression of an Neuroendocrine Specific Protein C-like cDNA isolated by EST sequencing described in Example 2.

A cDNA designated clone 2249-19 was isolated from the unsubtracted HNC library as described in Example 2, denoted herein as SEQ ID NO: 1775. A Northern Blot analysis was conducted as described in Example 10 to determine whether clone 2249-19 mRNA is expressed exclusively in HNC tissues. For the hybridization step, a probe having the nucleic acid sequence of clone 2249-19 was generated as follows. A PCR reaction was conducted using forward primer 2249-19for, having a nucleotide sequence 5' AGT CGC ATA GTG CAC TTC TGA ATG 3', denoted herein as SEQ ID NO:1954, and reverse primer 2249-19rev, having a nucleotide sequence 5' CTG ACA TCT GTT TCC ACA GCT C 3', denoted herein as SEQ ID NO:1955, using the HNC cDNA library prepared as described in Example 2 as the template under standard PCR reaction conditions and the following thermocycling conditions: (1) one minute at 95°C, (2) two cycles of 94°C for 10 seconds, 50°C for 20 seconds, and 72°C for 20 seconds, (3) thirty cycles of 94°C for 10 sec, 53°C for 20 sec, 72°C for 40 sec. The PCR product was ligated into the TA vector using a TA cloning kit, available from Invitrogen and the clone was digested with *Eco*R1 enzyme, and purified from an agarose gel. The purified nucleic acid molecule was labeled with $\alpha$-$^{32}$P-ATP using a DNA labeling kit, available from Amersham and added to the buffer at a concentration of approximately 1 $\times$ $10^6$ cpm/ml, and allowed to hybridize, for about 14 to 18 hours at 42°C. The blot was then washed twice for 10 minutes per wash in 0.5X SSPE and 0.1% sarcosyl at 55°C and exposed to film for autoradiography. Analysis of the developed film showed that there was expression of clone 2249-19 mRNA in HNC tissues and non-HNC tissues with 2 bands evident; one at approximately 1.5 Kb and one at approximately 2.5 Kb.

Example 22 (Reference)

This Example describes the further characterization and expression of an anoxia upregulated protein-like cDNA isolated by EST sequencing described in Example 2.

A TA clone from the HNC EST library described in Example 2 designated clone 2218-95, denoted herein as SEQ ID NO:1858 was sequenced using standard sequencing methods and shown to contain a non-full length nucleic acid molecule having significant homology to anoxia upregulated protein (AUP) genes. Additional sequence encoding an AUP gene was isolated as follows. A hybridization probe containing the nucleic acid sequence of SEQ ID NO:1858 was constructed as follows. A PCR reaction was conducted using forward primer 2218-95for, having a nucleotide sequence 5' AAT AGT GAT GTT GTA AGA GTT AGG 3', denoted herein as SEQ ID NO: 1956, and reverse primer 2218-95rev, having a nucleotide sequence 5' GTT TAA TAT TGC ATG TTT ATT CAT TAA AA 3', denoted herein as SEQ ID NO:1957, using the HNC cDNA library prepared as described in Example 2 as the template under standard PCR reaction conditions and the following thermocycling conditions: (1) one minute at 95°C, (2) thirty cycles of 94°C for 10 sec, 55°C for 20 sec, 72°C for 20 sec. The PCR product was ligated into the TA vector using a TA cloning kit, available from Invitrogen and the clone was digested with *Eco*R1 enzyme, and purified from an agarose gel. The purified nucleic acid molecule was labeled with $\alpha$-$^{32}$P-ATP using a DNA labeling kit, available from Amersham.

The $^{32}$P $\alpha$-dATP labeled probe was used in a standard plaque lift hybridization procedure to isolate a clone from the HNC lambda-ZAP unsubtracted cDNA library described in Example 2. Hybridization was conducted as described in Example 12 and a plaque that hybridized strongly to the probe was isolated, purified and sequenced as described in Example 12. Sequencing revealed that the clone contained a nucleic acid molecule of about 1181 nucleotides, referred to herein as $nCfAUP_{1181}$, having a nucleotide sequence denoted herein as SEQ ID NO:1919. The complement of SEQ ID NO:1919 is represented herein as SEQ ID NO:1921.

Translation of SEQ ID NO:1919 suggests that nucleic acid molecule $nCfAUP_{1181}$ encodes a full-length AUP protein of 102 amino acids, referred to herein as $PCfAUP_{102}$, having an amino acid sequence represented by SEQ ID NO: 1920, assuming the initiation codon spans from nucleotide 127 through nucleotide 129 of SEQ ID NO:1919 and the termination codon spans from nucleotide 433 through nucleotide 435 of SEQ ID NO:1919. The coding region encoding $PCfAUP_{102}$, is represented by nucleic acid molecule $nCfAUP_{306}$, having a coding strand with the nucleic acid sequence represented by SEQ ID NO:1922 and a complementary strand with nucleic acid sequence represented by SEQ ID NO: 1923. The amino acid sequence of $PCfAUP_{102}$, predicts that $PCfAUP_{102}$ has an estimated molecular weight of about 11.9 kDa and an estimated pI of about 10.5.

Comparison of amino acid sequence SEQ ID NO:1920 with amino acid sequences reported in GenBank indicates that SEQ ID NO:1920 showed the most homology, i.e., about 52% identity, with a *Drosophila melanogaster* anoxia upregulated protein, GenBank Accession No. AAD38397. Percent identity calculations were performed using GCG version 9.0 using default parameters. Blast comparison of nucleic acid sequence SEQ ID NO:1919 with nucleic acid sequences reported in GenBank indicates that SEQ ID NO:1919 showed the most homology to a clone from human chromosome 14q31 region containing gene for neurexin III, GenBank #AC007056. Pairwise identity could not be performed as the human clone in GenBank is too large to load into GCG version 9.0.

Example 23 (Reference)

This Example describes the further characterization of a neuroendocrine specific 7B2 polypeptide, isolated by EST sequencing described in Example 2.

A cDNA designated clone 2211-21 was isolated from the subtracted HNC library as described in Example 2, denoted herein as SEQ ID NO:92. DNA from clone 2211-21 was purified, and the insert used for plaque hybridization screening of the unsubtracted HMT cDNA library as follows. The insert from clone 2211-21 was excised by digestion with *Eco*RI, separated by agarose gel electrophoresis and purified using the QiaQuick Gel Extraction kit, available from Qiagen. A Megaprime DNA labeling kit, available from Amersham Pharmacia, was used to incorporate $\alpha$-$^{32}$P-labeled dATP into the random-primed probe mix. The $^{32}$P $\alpha$-dATP labeled probe was used in a standard plaque lift hybridization procedure to isolate a clone from the HNC lambda- ZAP unsubtracted cDNA library, prepared as described in Example 2. The following hybridization conditions were used. Hybond-N filters, available from Amersham, were hybridized with about 2 X $10^6$ counts per minute (cpm) per ml of the probe in 50 ml of hybridization solution (5X SSPE, 25mM EDTA pH 8.0, 5X Denhardt's reagent, 1.2% SDS, 0.020 mg/mL salmon sperm DNA) at 55°C for about 48 hours. The filters were washed once in 50mL 4X SSPE, 1% SDS for 15 minutes at 55°C, once in 50 mL 2X . SSPE, 1% SDS for 10 minutes at 55°C, and washed twice in 50 mL 0.5X SSPE, 0.5% SDS for 10 minutes at 55°C. The filters were then subjected to autoradiography. One plaque that hybridized strongly to the probe was isolated and subjected to in vivo excision using the Stratagene Ex-Assist™ helper phage system and protocols. Miniprep DNA was prepared from the positive clone using a Miniprep kit and protocol, available from Qiagen, Chatsworth, CA, and sequenced using standard sequencing procedures. The clone, referred to as $nCf7B2_{2161}$ contains a nucleic acid molecule of about 2161 nucleotides in length, having a coding strand with nucleic acid sequence SEQ ID NO:1924 and a complementary sequence having SEQ ID NO: 1926.

Translation of SEQ ID NO: 1924 suggests that nucleic acid molecule $nCf7B2_{2161}$ encodes a full-length 7B2-like protein of 267 amino acids, referred to herein as $PCf7B2_{267}$, having an amino acid sequence represented by SEQ ID NO: 1925, assuming the initiation codon spans from nucleotide 107 through nucleotide 109 of SEQ ID NO:1924 and the termination codon spans from nucleotide 908 through nucleotide 910 of SEQ ID NO: 1924. The coding region encoding $PCf7B2_{267}$, is represented by nucleic acid molecule $nCf7B2_{801}$, having a coding strand with the nucleic acid sequence represented by SEQ ID NO:1927 and a complementary strand with nucleic acid sequence represented by SEQ ID NO: 1928. The amino acid sequence of SEQ ID NO:1925, predicts that $PCf7B2_{267}$ has an estimated molecular weight of about 31 kDa and an estimated pI of about 5. Analysis of $PCf7B2_{267}$ suggests the presence of a signal peptide encoded by a stretch of amino acids spanning from about amino acid 1 through amino acid 20. The proposed mature protein, referred to herein as $PCf7B2_{247}$ contains 247 amino acids, designated SEQ ID NO:1930, and is encoded by a nucleic acid molecule referred to as $nCf7B2_{741}$, having a coding strand with SEQ ID NO:1929 and a complementary strand with SEQ ID NO:1931.

Comparison of amino acid sequence SEQ ID NO:1925 with amino acid sequences reported in GenBank indicates that SEQ ID NO:1925 showed the most homology, i.e., about 39% identity, with a *Drosophila melanogaster* protein, GenBank Accession No. AAF52036. Percent identity calculations were performed using GCG version 9.0 using default parameters. Blast comparison of nucleic acid sequence SEQ ID NO:1924 with nucleic acid sequences reported in GenBank indicates that SEQ ID NO:1924 showed the most homology to a human chromosome 19, cosmid R28204 clone, GenBank #Accession No. AC006132. Pairwise identity could not be performed as the human clone in GenBank is too large to load into GCG version 9.0, however, the BLAST score was 0.20, which is not considered to be significant level of identity.

A Northern Blot analysis was conducted as described in Example 10 to determine whether 7B2 mRNA is expressed exclusively in HNC tissues. For the hybridization step, a probe having the nucleic acid sequence of clone 2211-21 was generated as follows. A PCR reaction was conducted using forward primer 2211-21 for, having a nucleotide sequence 5' GCG CCA TGA AGA TTT CAG GCG 3', denoted herein as SEQ ID NO: 1958, and reverse primer 2211-21rev, having a nucleotide sequence 5' AAG TGC AAT GAA TCA TCA GCA AG 3', denoted herein as SEQ ID NO: 1959, using the HNC cDNA library prepared as described in Example 2 as the template under standard PCR reaction conditions and the following thermocycling conditions: (1) one minute at 95°C, (2) five cycles of 94°C for 10 seconds, 50°C for 20 seconds, and 72°C for 20 seconds, (3) thirty cycles of 94°C for 10 sec, 53°C for 20 sec, 72°C for 40 sec. The PCR product was ligated into the TA vector using a TA cloning kit, available from Invitrogen and the clone was digested with *Eco*RI enzyme, and purified from an agarose gel. The purified nucleic acid molecule was labeled with $\alpha$-$^{32}$P-ATP using a DNA labeling kit, available from Amersham and added to the buffer at a concentration of approximately $1 \times 10^6$ cpm/ml, and allowed to hybridize for about 14 to 18 hours at 42°C. The blot was then washed twice for 10 minutes per wash in 0.5X SSPE and 0. 1% sarcosyl at 55°C and exposed to film for autoradiography. Analysis of the developed film showed that after 2.5 days of exposure clone 2211-21 mRNA was expressed exclusively in HNC tissue.

While various embodiments of the present invention have been described in detail, it is apparent that modifications and adaptations of those embodiments will occur to those skilled in the art.

**Claims**

1. An isolated nucleic acid molecule comprising a nucleic acid sequence selected from the group consisting of:

   (a) a nucleic acid sequence at least 75% identical to a polynucleotide sequence selected from the group consisting of SEQ ID NO:1872 and SEQ ID NO:1875; wherein the nucleic acid sequence encodes a protein having the voltage-gated chloride channel activity of a protein consisting of the amino acid sequence of SEQ ID NO: 1873; and
   (b) a nucleic acid sequence fully complementary to the nucleic acid sequence of (a).

2. The nucleic acid molecule of claim 1, wherein said nucleic acid sequence is selected from the group consisting of:

   (a) a nucleic acid sequence selected from the group consisting of SEQ ID NO:1872, SEQ ID NO:1874, SEQ ID NO:1875, SEQ ID NO:1876; and,
   (b) a nucleic acid sequence that is an allelic variant of sequence selected from the group consisting of SEQ ID NO:1872, SEQ ID NO:1874, and SEQ ID NO:1875 and SEQ ID NO:1876.

3. The nucleic acid molecule of claim 1, wherein said nucleic acid sequence encodes a protein comprising an amino acid sequence that is at least 75% identical to SEQ ID NO:1873.

4. The nucleic acid molecule of claim 1, wherein said nucleic acid sequence encodes a protein comprising SEQ ID NO:1873.

5. A recombinant molecule comprising a nucleic acid molecule as set forth in claim 1 operatively linked to a transcription control sequence.

6. A method to produce a protein comprising:

   (a) transforming a cell with a nucleic acid molecule comprising a nucleic acid sequence encoding said protein, wherein said protein comprises an amino acid sequence at least 75% identical to SEQ ID NO:1873, and wherein said protein has the voltage-gated chloride channel activity of a protein consisting of the amino acid sequence of SEQ ID NO:1873; and b) culturing said cell under conditions suitable for expression of said protein.

7. The method of claim 6, wherein said nucleic acid sequence is selected from the group consisting of:

   (a) a nucleic acid sequence comprising a sequence at least 80% identical to SEQ ID NO:1872 or SEQ ID NO: 1875; and,
   (b) a nucleic acid sequence that is an allelic variant of SEQ ID NO:1872 or SEQ ID NO:1875.

8. The method of claim 6, wherein said nucleic acid sequence is selected from SEQ ID NO:1872 or SEQ ID NO:1875.

9. An isolated protein encoded by a nucleic acid sequence at least 75% identical to a nucleic acid sequence selected from the group consisting of SEQ ID NO:1872 and SEQ ID NO:1875, and wherein the protein has the voltage-gated chloride channel activity of a protein consisting of the amino acid sequence of SEQ ID NO:1873.

**10.** The protein of claim 9, wherein said nucleic acid sequence is selected from the group consisting of:

(a) nucleic acid sequence selected from the group consisting of SEQ ID NO:1872 and SEQ ID NO:1875; and
(b) a nucleic acid sequence that is an allelic variant of a nucleic acid sequence selected from the group consisting of SEQ ID NO:1872 and SEQ ID NO:1875.

**11.** The protein of claim 9, wherein said protein comprises an amino acid sequence that is at least 75% identical to SEQ ID NO:1873.

**12.** The protein of claim 9, wherein said protein comprises SEQ ID NO:1873.

**13.** An isolated antibody that selectively binds to a protein as set forth in claim 9.

**14.** A method to identify a compound capable of inhibiting the activity of an isolated protein of claim 9, said method comprising contacting an isolated protein of claim 9 with a putative inhibitory compound under conditions in which, in the absence of said compound, said protein has activity; and determining if said putative inhibitory compound inhibits said activity.

**15.** A kit to identify a compound capable of inhibiting activity of an isolated protein of claim 9, said test kit comprising an isolated protein of claim 9 and a means for determining the extent of inhibition of said activity in the presence of a putative inhibitory compound.

**16.** A composition comprising an excipient and an isolated antibody that selectively binds to a protein that comprises an amino acid sequence that is at least 75% identical to SEQ ID NO:1873, and wherein the protein has the voltage-gated chloride channel activity of a protein consisting of the amino acid sequence of SEQ ID NO:1873.

**17.** The composition of claim 16, wherein said composition further comprises a component selected from the group consisting of an adjuvant and a carrier.

**18.** A composition according to claim 16, for use in the treatment of protection from flea infestation in an animal.

**Patentansprüche**

**1.** Ein isoliertes Nukleinsäuremolekül, das eine Nukleinsäuresequenz beinhaltet, die aus der Gruppe ausgewählt ist, die aus Folgendem besteht:

(a) einer Nukleinsäuresequenz, die mit einer Polynucleotidsequenz, ausgewählt aus der Gruppe, bestehend aus SEQ ID NO:1872 und SEQ ID NO:1875, mindestens zu 75 % identisch ist; wobei die Nukleinsäuresequenz ein Protein codiert, das die spannungsgesteuerte Chloridkanalaktivität eines Proteins, das aus der Aminosäuresequenz von SEQ ID NO:1873 besteht, aufweist; und
(b) einer Nukleinsäuresequenz, die zu der Nukleinsäuresequenz aus (a) völlig komplementär ist.

**2.** Nukleinsäuremolekül gemäß Anspruch 1, wobei die Nukleinsäuresequenz aus der Gruppe ausgewählt ist, die aus Folgendem besteht:

(a) einer Nukleinsäuresequenz, die aus der Gruppe ausgewählt ist, die aus Folgendem besteht: SEQ ID NO: 1872, SEQ ID NO:1874, SEQ ID NO:1875, SEQ ID NO:1876; und
(b) einer Nukleinsäuresequenz, die eine allele Variante der Sequenz, ausgewählt aus der Gruppe, bestehend aus SEQ ID NO:1872, SEQ ID NO:1874 und SEQ ID NO:1875 und SEQ ID NO:1876, ist.

**3.** Nukleinsäuremolekül gemäß Anspruch 1, wobei die Nukleinsäuresequenz ein Protein codiert, das eine Aminosäuresequenz beinhaltet, die mit SEQ ID NO:1873 mindestens zu 75 % identisch ist.

**4.** Nukleinsäuremolekül gemäß Anspruch 1, wobei die Nukleinsäuresequenz ein Protein codiert, das SEQ ID NO:1873 beinhaltet.

**5.** Ein rekombinantes Molekül, das ein Nukleinsäuremolekül gemäß Anspruch 1, das operativ mit einer Transkriptionssteuersequenz verknüpft ist, beinhaltet.

6. Ein Verfahren zum Produzieren eines Proteins, das Folgendes beinhaltet:

(a) Transformieren einer Zelle mit einem Nukleinsäuremolekül, das eine Nukleinsäuresequenz beinhaltet, die das Protein codiert, wobei das Protein eine Aminosäuresequenz beinhaltet, die mit SEQ ID NO:1873 mindestens zu 75 % identisch ist, und wobei das Protein die spannungsgesteuerte Chloridkanalaktivität eines Proteins, das aus der Aminosäuresequenz von SEQ ID NO:1873 besteht, aufweist; und
(b) Kultivieren der Zelle unter Bedingungen, die zur Expression des Proteins geeignet sind.

7. Verfahren gemäß Anspruch 6, wobei die Nukleinsäuresequenz aus der Gruppe ausgewählt ist, die aus Folgendem besteht:

(a) einer Nukleinsäuresequenz, die eine Sequenz beinhaltet, die mit SEQ ID NO:1872 oder SEQ ID NO:1875 mindestens zu 80 % identisch ist; und
(b) einer Nukleinsäuresequenz, die eine allele Variante von SEQ ID NO:1872 oder SEQ ID NO: 1875 ist.

8. Verfahren gemäß Anspruch 6, wobei die Nukleinsäuresequenz aus SEQ ID NO:1872 oder SEQ ID NO:1875 ausgewählt ist.

9. Ein isoliertes Protein, das durch eine Nukleinsäuresequenz codiert ist, die mit einer Nukleinsäuresequenz, ausgewählt aus der Gruppe, bestehend aus SEQ ID NO:1872 und SEQ ID NO:1875, mindestens zu 75 % identisch ist, und wobei das Protein die spannungsgesteuerte Chloridkanalaktivität eines Proteins, das aus der Aminosäuresequenz von SEQ ID NO:1873 besteht, aufweist.

10. Protein gemäß Anspruch 9, wobei die Nukleinsäuresequenz aus der Gruppe ausgewählt ist, die aus Folgendem besteht:

(a) einer Nukleinsäuresequenz, die aus der Gruppe ausgewählt ist, die aus SEQ ID NO:1872 und SEQ ID NO: 1875 besteht; und
(b) einer Nukleinsäuresequenz, die eine allele Variante einer Nukleinsäuresequenz, ausgewählt aus der Gruppe, bestehend aus SEQ ID NO:1872 und SEQ ID NO:1875, ist.

11. Protein gemäß Anspruch 9, wobei das Protein eine Aminosäuresequenz beinhaltet, die mit SEQ ID NO:1873 mindestens zu 75 % identisch ist.

12. Protein gemäß Anspruch 9, wobei das Protein SEQ ID NO:1873 beinhaltet.

13. Ein isolierter Antikörper, der selektiv an ein Protein gemäß Anspruch 9 bindet.

14. Ein Verfahren zum Identifizieren einer Verbindung, die zum Hemmen der Aktivität eines isolierten Proteins gemäß Anspruch 9 fähig ist, wobei das Verfahren das In-Kontakt-Bringen eines isolierten Proteins gemäß Anspruch 9 mit einer putativen hemmenden Verbindung unter Bedingungen, unter denen das Protein in Abwesenheit der Verbindung Aktivität aufweist; und Bestimmen, ob die putative hemmende Verbindung die Aktivität hemmt, beinhaltet.

15. Ein Kit zum Identifizieren einer Verbindung, die zum Hemmen der Aktivität eines isolierten Proteins gemäß Anspruch 9 fähig ist, wobei das Testkit ein isoliertes Protein gemäß Anspruch 9 und ein Mittel zum Bestimmen des Ausmaßes von Hemmung der Aktivität in der Gegenwart einer putativen hemmenden Verbindung beinhaltet.

16. Eine Zusammensetzung, die einen Hilfsstoff und einen isolierten Antikörper, der selektiv an ein Protein bindet, das eine Aminosäuresequenz beinhaltet, die mit SEQ ID NO:1873 mindestens zu 75 % identisch ist, beinhaltet, und wobei das Protein die spannungsgesteuerte Chloridkanalaktivität eines Proteins, das aus der Aminosäuresequenz von SEQ ID NO:1873 besteht, aufweist.

17. Zusammensetzung gemäß Anspruch 16, wobei die Zusammensetzung ferner eine Komponente beinhaltet, die aus der Gruppe ausgewählt ist, die aus einem Hilfsmittel und einem Träger besteht.

18. Zusammensetzung gemäß Anspruch 16 zur Verwendung bei der Behandlung zum Schutz vor Flohbefall bei einem Tier.

## Revendications

1. Une molécule d'acide nucléique isolée comprenant une séquence d'acide nucléique sélectionnée dans le groupe consistant en :

   (a) une séquence d'acide nucléique identique à au moins 75 % à une séquence de polynucléotide sélectionnée dans le groupe consistant en SEQ ID NO : 1872 et SEQ ID NO : 1875 ; dans laquelle la séquence d'acide nucléique code pour une protéine présentant l'activité de canal chlorure dépendant d'un potentiel d'action d'une protéine consistant en la séquence d'acides aminés de SEQ ID NO : 1873; et
   (b) une séquence d'acide nucléique totalement complémentaire à la séquence d'acide nucléique de (a).

2. La molécule d'acide nucléique de la revendication 1, dans laquelle ladite séquence d'acide nucléique est sélectionnée dans le groupe consistant en :

   (a) une séquence d'acide nucléique sélectionnée dans le groupe consistant en SEQ ID NO : 1872, SEQ ID NO : 1874, SEQ ID NO : 1875, SEQ ID NO : 1876 ; et,
   (b) une séquence d'acide nucléique qui est un variant allélique de séquence sélectionnée dans le groupe consistant en SEQ ID NO : 1872, SEQ ID NO : 1874, et SEQ ID NO : 1875 et SEQ ID NO : 1876.

3. La molécule d'acide nucléique de la revendication 1, dans laquelle ladite séquence d'acide nucléique code pour une protéine comprenant une séquence d'acides aminés qui est identique à au moins 75 % à SEQ ID NO : 1873.

4. La molécule d'acide nucléique de la revendication 1, dans laquelle ladite séquence d'acide nucléique code pour une protéine comprenant SEQ ID NO : 1873.

5. Une molécule recombinée comprenant une molécule d'acide nucléique telle qu'énoncée dans la revendication 1 liée de façon opérationnelle à une séquence de contrôle de transcription.

6. Une méthode pour produire une protéine comprenant :

   (a) transformer une cellule avec une molécule d'acide nucléique comprenant une séquence d'acide nucléique codant pour ladite protéine, ladite protéine comprenant une séquence d'acides aminés identique à au moins 75 % à SEQ ID NO : 1873, et dans laquelle ladite protéine présente l'activité de canal chlorure dépendante d'un potentiel d'action d'une protéine consistant en la séquence d'acides aminés de SEQ ID NO : 1873 ; et
   (b) cultiver ladite cellule dans des conditions adéquates pour l'expression de ladite protéine.

7. La méthode de la revendication 6, dans laquelle ladite séquence d'acide nucléique est sélectionnée dans le groupe consistant en :

   (a) une séquence d'acide nucléique comprenant une séquence identique à au moins 80 % à SEQ ID NO : 1872 ou SEQ ID NO : 1875 ; et,
   (b) une séquence d'acide nucléique qui est un variant allélique de SEQ ID NO : 1872 ou SEQ ID NO : 1875.

8. La méthode de la revendication 6, dans laquelle ladite séquence d'acide nucléique est sélectionnée parmi SEQ ID NO : 1872 ou SEQ ID NO : 1875.

9. Une protéine isolée codée par une séquence d'acide nucléique identique à au moins 75 % à une séquence d'acide nucléique sélectionnée dans le groupe consistant en SEQ ID NO : 1872 et SEQ ID NO : 1875, la protéine présentant l'activité de canal chlorure dépendant d'un potentiel d'action d'une protéine consistant en la séquence d'acides aminés de SEQ ID NO : 1873.

10. La protéine de la revendication 9, dans laquelle ladite séquence d'acide nucléique est sélectionnée dans le groupe consistant en :

    (a) séquence d'acide nucléique sélectionnée dans le groupe consistant en SEQ ID NO : 1872 et SEQ ID NO :

1875 ; et
(b) une séquence d'acide nucléique qui est un variant allélique d'une séquence d'acide nucléique sélectionnée dans le groupe consistant en SEQ ID NO : 1872 et SEQ ID NO : 1875.

**11.** La protéine de la revendication 9, dans laquelle ladite protéine comprend une séquence d'acides aminés qui est identique à au moins 75 % à SEQ ID NO : 1873.

**12.** La protéine de la revendication 9, dans laquelle ladite protéine comprend SEQ ID NO : 1873.

**13.** Un anticorps isolé qui se lie de façon sélective à une protéine telle qu'énoncée dans la revendication 9.

**14.** Une méthode pour identifier un composé capable d'inhiber l'activité d'une protéine isolée de la revendication 9, ladite méthode comprenant mettre en contact une protéine isolée de la revendication 9 avec un composé inhibiteur putatif dans des conditions selon lesquelles, en l'absence dudit composé, ladite protéine présente une activité ; et déterminer si ledit composé inhibiteur putatif inhibe ladite activité.

**15.** Un kit pour identifier un composé capable d'inhiber l'activité d'une protéine isolée de la revendication 9, ledit kit de test comprenant une protéine isolée de la revendication 9 et un moyen pour déterminer l'étendue d'inhibition de ladite activité en présence d'un composé inhibiteur putatif.

**16.** Une composition comprenant un excipient et un anticorps isolé qui se lie de façon sélective à une protéine qui comprend une séquence d'acides aminés qui est identique à au moins 75 % à SEQ ID NO : 1873, et dans laquelle la protéine présente l'activité de canal chlorure dépendant d'un potentiel d'action d'une protéine consistant en la séquence d'acides aminés de SEQ ID NO : 1873.

**17.** La composition de la revendication 16, dans laquelle ladite composition comprend en outre un composé sélectionné dans le groupe consistant en un adjuvant et un excipient.

**18.** Une composition selon la revendication 16, destinée à être utilisée dans le traitement pour la protection contre une infestation de puces chez un animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5854411 A
- WO 9833818 A
- US 5766602 A, Xiong and Grieve
- WO 9611706 A, Grieve


### Non-patent literature cited in the description


- Molecular Cloning and Expression of a Chloride Ion Channel of Cell Nuclei. *J Biol Chem,* 1997, vol. 272, 12575-12582
- **Heiss N S et al.** *Genomics,* 1997, vol. 45, 224-228
- **Landry D W.** *J Biol Chem,* 1993, vol. 268, 14948-14955
- **Sambrook et al.** Molecular Cloning: A Laboratory Manual. Cold Spring Harbor Labs Press, 1989
- **Meinkoth et al.** *Anal. Biochem.,* 1984, vol. 138, 267-284
- **Wolff et al.** *Science,* 1990, vol. 247, 1465-1468
- **Bumett.** *Biotechniques,* 1997, vol. 22 (4), 668-671
- **Tellam et al.** *Peritrophic Matrix Proteins, Insect Biochemistry and Molecular Biology,* 1999, vol. 29, 87-101
- **Schorderet et al.** cDNA and deduced amino acid sequences of a peritrophic membrane glycoprotein, 'peritrophin-48', from the larvae of Lucilia cuprina. *Insect Biochemistry and Molecular Biology,* 1998, vol. 28, 99-111

SEQUENCE LISTING

<110> Brandt, Kevin S.
Gaines, Patrick J.
Stinchcomb, Dan T.
Wisnewski, Nancy

<120> FLEA HEAD, NERVE CORD, HINDGUT AND MALPIGHIAN TUBULE NUCLEIC ACID MOLECULES, PROTEINS AND USES THEREOF

<130> FC-6-C1-PCT

<140> not yet assigned
<141> 2000-04-07

<150> 60/128,704
<151> 1999-04-09

<160> 1959

<170> PatentIn Ver. 2.1

<210> 1
<211> 2057
<212> DNA
<213> Ctenocephalides felis

<220>
<221> CDS
<222> (152)..(1303)

<400> 1

```
aacataataa taacttaata aaattttgtg atcagatttc taatatccag aacaaagcca 60

gtaattataa gaaccaagcc tatttcatgt gaaggttact tctccacagt attattatct 120

atctcaagaa gtaatctatt actgaatcaa a atg aaa agc agt acc tgt att   172
                                   Met Lys Ser Ser Thr Cys Ile
                                     1               5

ttt ctt ctg gtc att atg ctg aat tgc aag aac ctt gtt aat gct gcg   220
Phe Leu Leu Val Ile Met Leu Asn Cys Lys Asn Leu Val Asn Ala Ala
         10                  15                  20

tgc acc aac aac gcg cct cca atg aag ata ttc cgt agc cga aga gtt   268
Cys Thr Asn Asn Ala Pro Pro Met Lys Ile Phe Arg Ser Arg Arg Val
     25                  30                  35

ctt ctc ggt gat ggt act gaa aga gat gct ggc att gta gtt gat tcc   316
Leu Leu Gly Asp Gly Thr Glu Arg Asp Ala Gly Ile Val Val Asp Ser
 40                  45                  50                  55

tcc gga aga ata aaa agt ata att tca gga gaa gaa gtg gaa agg ata   364
Ser Gly Arg Ile Lys Ser Ile Ile Ser Gly Glu Glu Val Glu Arg Ile
                 60                  65                  70

gct aac gaa act aaa gtt gag gtg ttg gac tac ggt caa ttt tca ata   412
```

```
ctt ctc ggt gat ggt act gaa aga gat gct ggc att gta gtt gat tcc   316
Leu Leu Gly Asp Gly Thr Glu Arg Asp Ala Gly Ile Val Val Asp Ser
 40                  45                  50                  55

tcc gga aga ata aaa agt ata att tca gga gaa gaa gtg gaa agg ata   364
Ser Gly Arg Ile Lys Ser Ile Ile Ser Gly Glu Glu Val Glu Arg Ile
                 60                  65                  70

gct aac gaa act aaa gtt gag gtg ttg gac tac ggt caa ttt tca ata   412
Ala Asn Glu Thr Lys Val Glu Val Leu Asp Tyr Gly Gln Phe Ser Ile
             75                  80                  85

tgg cca ggt gtg ata gac tct cat gtg cac gtc aac gaa cca gga aga   460
Trp Pro Gly Val Ile Asp Ser His Val His Val Asn Glu Pro Gly Arg
         90                  95                 100

gaa tcc tgg gaa gga tac acc aca gct act aaa gca gca gct tgg ggc   508
Glu Ser Trp Glu Gly Tyr Thr Thr Ala Thr Lys Ala Ala Ala Trp Gly
    105                 110                 115

ggg att acc aca ata gta gac atg cct ttg aat tcc atc cca cct aca   556
Gly Ile Thr Thr Ile Val Asp Met Pro Leu Asn Ser Ile Pro Pro Thr
120                 125                 130                 135

act act gta gag aat ttg aga aca aaa gtg aat tca gcc tgt ggt aaa   604
Thr Thr Val Glu Asn Leu Arg Thr Lys Val Asn Ser Ala Cys Gly Lys
                140                 145                 150

acg cat gtt gat gtc gct ttc tgg gga ggc gtg att cct ggc aat gcg   652
Thr His Val Asp Val Ala Phe Trp Gly Gly Val Ile Pro Gly Asn Ala
            155                 160                 165

cac gaa ttg ttg cca ctt atc aac gcc gga gta aga gga ttc aaa tgt   700
His Glu Leu Leu Pro Leu Ile Asn Ala Gly Val Arg Gly Phe Lys Cys
        170                 175                 180

ttt aca agt gaa agt ggt gtc gat gag ttt cca cag gtt act aaa aat   748
Phe Thr Ser Glu Ser Gly Val Asp Glu Phe Pro Gln Val Thr Lys Asn
    185                 190                 195

gat ctg gaa atg gct cta aaa gag ctc cag aaa gca aat tcc gta ctt   796
Asp Leu Glu Met Ala Leu Lys Glu Leu Gln Lys Ala Asn Ser Val Leu
200                 205                 210                 215

ctg tac cat gcc gaa tta ccc gct cct caa gaa aat gtt aca agc aat   844
Leu Tyr His Ala Glu Leu Pro Ala Pro Gln Glu Asn Val Thr Ser Asn
                220                 225                 230
```

```
gaa act gaa aag tac atg act tac ctg aaa aca cga cct cca agt atg    892
Glu Thr Glu Lys Tyr Met Thr Tyr Leu Lys Thr Arg Pro Pro Ser Met
                235                 240                 245

gaa gta aat gct att gat atg att ata gac ctc aca aaa aaa tat aaa    940
Glu Val Asn Ala Ile Asp Met Ile Ile Asp Leu Thr Lys Lys Tyr Lys
            250                 255                 260

gtt agg tct cac ata gtg cat cta tca gca gca ggt gct tta ccg caa    988
Val Arg Ser His Ile Val His Leu Ser Ala Ala Gly Ala Leu Pro Gln
        265                 270                 275

ttg aaa aaa gcg cgc tca gag aac gtt cca ctt tcg att gaa act tgt    1036
Leu Lys Lys Ala Arg Ser Glu Asn Val Pro Leu Ser Ile Glu Thr Cys
280                 285                 290                 295

cat cat tac tta acc ttt gct gct gaa gat gtt cca gat gga cat act    1084
His His Tyr Leu Thr Phe Ala Ala Glu Asp Val Pro Asp Gly His Thr
                    300                 305                 310

gaa tac aaa tgc gct cca cca att aga gaa gaa agt aat caa gaa aaa    1132
Glu Tyr Lys Cys Ala Pro Pro Ile Arg Glu Glu Ser Asn Gln Glu Lys
                315                 320                 325

tta tgg caa gct ttg gaa aac aga gat att gat atg gta gtc agt gat    1180
Leu Trp Gln Ala Leu Glu Asn Arg Asp Ile Asp Met Val Val Ser Asp
            330                 335                 340

cat tct cca tca cct gct gca ctg aaa ggc ctg tgc aat ggt tgt cat    1228
His Ser Pro Ser Pro Ala Ala Leu Lys Gly Leu Cys Asn Gly Cys His
        345                 350                 355

cct gat ttc cta aaa gct tgg ggt gga att gct ggt atg cag ttt gga    1276
Pro Asp Phe Leu Lys Ala Trp Gly Gly Ile Ala Gly Met Gln Phe Gly
360                 365                 370                 375

tta tct tta ata agg gac cgg tgc ttc taaaagaggc tttaaagctc         1323
Leu Ser Leu Ile Arg Asp Arg Cys Phe
                    380

atgatgtatc tcgtttatta tctgcgggac ctgcgaaatt aactggactg gatggcataa 1383

aaggacaaat caaagaaggc ttggatgctg atttagtaat ttgggatcct gaggaagaat 1443

ttaaggtcac taaagacata atccaacaca agaataaaga aacaccatac ttaggaatga 1503

cgttgaaggg caaagttcat gcaactgttg tacgaggaga ctttgtttac cgtaatggac 1563
```

```
aaccattcga aattccaaaa ggaaatttac ttattgaatg attaaatgta atagattaat 1623

caaattttag atgattaaaa ttgttttatt actacaatag caacctctgc ctgaaaatta 1683

accgaacaaa cttctaacat ccttattaat gtatagattt tgaataataa catagaaatt 1743

atactatttt tttgatgact ctaataaaaa aaatgtataa atggccatgc ctgatatatt 1803

tttgataacc ttaatgaaaa aatgtttaaa tggccatgtc tgaaaagatt tctatgtgta 1863

ttttttttgtt aacattttat tgttgaatgg ataaaagata aatacaattt tataagctgt 1923

ttggataaat taattttgaa taaatccata atcatagaat atgttaagta gcaaattaaa 1983

atatggacca caaaccacaa aatgtatacg aaatataact tatatgatat atgaaaaaaa 2043

aaaaaaaaaa aaaa                                                   2057
```

<210> 2
<211> 384
<212> PRT
<213> Ctenocephalides felis

<400> 2

```
Met Lys Ser Ser Thr Cys Ile Phe Leu Leu Val Ile Met Leu Asn Cys
1               5                   10                  15

Lys Asn Leu Val Asn Ala Ala Cys Thr Asn Asn Ala Pro Pro Met Lys
            20                  25                  30

Ile Phe Arg Ser Arg Arg Val Leu Leu Gly Asp Gly Thr Glu Arg Asp
        35                  40                  45

Ala Gly Ile Val Val Asp Ser Ser Gly Arg Ile Lys Ser Ile Ile Ser
    50                  55                  60

Gly Glu Glu Val Glu Arg Ile Ala Asn Glu Thr Lys Val Glu Val Leu
65                  70                  75                  80

Asp Tyr Gly Gln Phe Ser Ile Trp Pro Gly Val Ile Asp Ser His Val
                85                  90                  95

His Val Asn Glu Pro Gly Arg Glu Ser Trp Glu Gly Tyr Thr Thr Ala
            100                 105                 110

Thr Lys Ala Ala Ala Trp Gly Gly Ile Thr Thr Ile Val Asp Met Pro
        115                 120                 125
```

```
Leu Asn Ser Ile Pro Pro Thr Thr Thr Val Glu Asn Leu Arg Thr Lys
    130                 135                 140

Val Asn Ser Ala Cys Gly Lys Thr His Val Asp Val Ala Phe Trp Gly
145                 150                 155                 160

Gly Val Ile Pro Gly Asn Ala His Glu Leu Leu Pro Leu Ile Asn Ala
                165                 170                 175

Gly Val Arg Gly Phe Lys Cys Phe Thr Ser Glu Ser Gly Val Asp Glu
            180                 185                 190

Phe Pro Gln Val Thr Lys Asn Asp Leu Glu Met Ala Leu Lys Glu Leu
        195                 200                 205

Gln Lys Ala Asn Ser Val Leu Leu Tyr His Ala Glu Leu Pro Ala Pro
    210                 215                 220

Gln Glu Asn Val Thr Ser Asn Glu Thr Glu Lys Tyr Met Thr Tyr Leu
225                 230                 235                 240

Lys Thr Arg Pro Pro Ser Met Glu Val Asn Ala Ile Asp Met Ile Ile
                245                 250                 255

Asp Leu Thr Lys Lys Tyr Lys Val Arg Ser His Ile Val His Leu Ser
            260                 265                 270

Ala Ala Gly Ala Leu Pro Gln Leu Lys Lys Ala Arg Ser Glu Asn Val
        275                 280                 285

Pro Leu Ser Ile Glu Thr Cys His His Tyr Leu Thr Phe Ala Ala Glu
    290                 295                 300

Asp Val Pro Asp Gly His Thr Glu Tyr Lys Cys Ala Pro Pro Ile Arg
305                 310                 315                 320

Glu Glu Ser Asn Gln Glu Lys Leu Trp Gln Ala Leu Glu Asn Arg Asp
                325                 330                 335

Ile Asp Met Val Val Ser Asp His Ser Pro Ser Pro Ala Ala Leu Lys
            340                 345                 350

Gly Leu Cys Asn Gly Cys His Pro Asp Phe Leu Lys Ala Trp Gly Gly
        355                 360                 365

Ile Ala Gly Met Gln Phe Gly Leu Ser Leu Ile Arg Asp Arg Cys Phe
    370                 375                 380
```

<210> 3
<211> 2057

<212> DNA
<213> Ctenocephalides felis

<400> 3

```
tttttttttt tttttttttt tcatatatca tataagttat atttcgtata cattttgtgg 60
tttgtggtcc atattttaat ttgctactta acatattcta tgattatgga tttattcaaa 120
attaatttat ccaaacagct tataaaattg tatttatctt ttatccattc aacaataaaa 180
tgttaacaaa aaaatacaca tagaaatctt ttcagacatg gccatttaaa cattttttca 240
ttaaggttat caaaaatata tcaggcatgg ccatttatac atttttttta ttagagtcat 300
caaaaaaata gtataatttc tatgttatta ttcaaaatct atacattaat aaggatgtta 360
gaagtttgtt cggttaattt tcaggcagag gttgctattg tagtaataaa acaattttaa 420
tcatctaaaa tttgattaat ctattacatt taatcattca ataagtaaat ttccttttgg 480
aatttcgaat ggttgtccat tacggtaaac aaagtctcct cgtacaacag ttgcatgaac 540
tttgcccttc aacgtcattc ctaagtatgg tgtttcttta ttcttgtgtt ggattatgtc 600
tttagtgacc ttaaattctt cctcaggatc ccaaattact aaatcagcat ccaagccttc 660
tttgatttgt ccttttatgc catccagtcc agttaatttc gcaggtcccg cagataataa 720
acgagataca tcatgagctt taaagcctct tttagaagca ccggtccctt attaaagata 780
atccaaactg cataccagca attccacccc aagcttttag gaaatcagga tgacaaccat 840
tgcacaggcc tttcagtgca gcaggtgatg gagaatgatc actgactacc atatcaatat 900
ctctgtttc caaagcttgc cataattttt cttgattact ttcttctcta attggtggag 960
cgcatttgta ttcagtatgt ccatctggaa catcttcagc agcaaaggtt aagtaatgat 1020
gacaagtttc aatcgaaagt ggaacgttct ctgagcgcgc ttttttcaat tgcggtaaag 1080
cacctgctgc tgatagatgc actatgtgag acctaacttt atattttttt gtgaggtcta 1140
taatcatatc aatagcattt acttccatac ttggaggtcg tgttttcagg taagtcatgt 1200
acttttcagt ttcattgctt gtaacatttt cttgaggagc gggtaattcg gcatggtaca 1260
gaagtacgga atttgctttc tggagctctt ttagagccat ttccagatca tttttagtaa 1320
cctgtggaaa ctcatcgaca ccactttcac ttgtaaaaca tttgaatcct cttactccgg 1380
cgttgataag tggcaacaat tcgtgcgcat tgccaggaat cacgcctccc cagaaagcga 1440
catcaacatg cgttttacca caggctgaat tcacttttgt tctcaaattc tctacagtag 1500
ttgtaggtgg gatggaattc aaaggcatgt ctactattgt ggtaatcccg ccccaagctg 1560
ctgctttagt agctgtggtg tatccttccc aggattctct tcctggttcg ttgacgtgca 1620
catgagagtc tatcacacct ggccatattg aaaattgacc gtagtccaac acctcaactt 1680
tagtttcgtt agctatcctt tccacttctt ctcctgaaat tatacttttt attcttccgg 1740
aggaatcaac tacaatgcca gcatctcttt cagtaccatc accgagaaga actcttcggc 1800
tacggaatat cttcattgga ggcgcgttgt tggtgcacgc agcattaaca aggttcttgc 1860
aattcagcat aatgaccaga agaaaaatac aggtactgct tttcattttg attcagtaat 1920
agattacttc ttgagataga taataatact gtggagaagt aaccttcaca tgaaataggc 1980
ttggttctta taattactgg ctttgttctg gatattagaa atctgatcac aaaattttat 2040
taagttatta ttatgtt                                                2057
```

<210> 4
<211> 1152
<212> DNA
<213> Ctenocephalides felis

<220>
<221> CDS
<222> (1)..(1152)

<400> 4

```
atg aaa agc agt acc tgt att ttt ctt ctg gtc att atg ctg aat tgc    48
Met Lys Ser Ser Thr Cys Ile Phe Leu Leu Val Ile Met Leu Asn Cys
 1               5                  10                  15

aag aac ctt gtt aat gct gcg tgc acc aac aac gcg cct cca atg aag    96
Lys Asn Leu Val Asn Ala Ala Cys Thr Asn Asn Ala Pro Pro Met Lys
            20                  25                  30

ata ttc cgt agc cga aga gtt ctt ctc ggt gat ggt act gaa aga gat   144
Ile Phe Arg Ser Arg Arg Val Leu Leu Gly Asp Gly Thr Glu Arg Asp
        35                  40                  45

gct ggc att gta gtt gat tcc tcc gga aga ata aaa agt ata att tca   192
Ala Gly Ile Val Val Asp Ser Ser Gly Arg Ile Lys Ser Ile Ile Ser
    50                  55                  60

gga gaa gaa gtg gaa agg ata gct aac gaa act aaa gtt gag gtg ttg   240
Gly Glu Glu Val Glu Arg Ile Ala Asn Glu Thr Lys Val Glu Val Leu
65                  70                  75                  80

gac tac ggt caa ttt tca ata tgg cca ggt gtg ata gac tct cat gtg   288
Asp Tyr Gly Gln Phe Ser Ile Trp Pro Gly Val Ile Asp Ser His Val
                85                  90                  95

cac gtc aac gaa cca gga aga gaa tcc tgg gaa gga tac acc aca gct   336
His Val Asn Glu Pro Gly Arg Glu Ser Trp Glu Gly Tyr Thr Thr Ala
            100                 105                 110

act aaa gca gca gct tgg ggc ggg att acc aca ata gta gac atg cct   384
Thr Lys Ala Ala Ala Trp Gly Gly Ile Thr Thr Ile Val Asp Met Pro
        115                 120                 125

ttg aat tcc atc cca cct aca act act gta gag aat ttg aga aca aaa   432
Leu Asn Ser Ile Pro Pro Thr Thr Thr Val Glu Asn Leu Arg Thr Lys
    130                 135                 140

gtg aat tca gcc tgt ggt aaa acg cat gtt gat gtc gct ttc tgg gga   480
Val Asn Ser Ala Cys Gly Lys Thr His Val Asp Val Ala Phe Trp Gly
145                 150                 155                 160
```

```
ggc gtg att cct ggc aat gcg cac gaa ttg ttg cca ctt atc aac gcc    528
Gly Val Ile Pro Gly Asn Ala His Glu Leu Leu Pro Leu Ile Asn Ala
                165                 170                 175

gga gta aga gga ttc aaa tgt ttt aca agt gaa agt ggt gtc gat gag    576
Gly Val Arg Gly Phe Lys Cys Phe Thr Ser Glu Ser Gly Val Asp Glu
            180                 185                 190

ttt cca cag gtt act aaa aat gat ctg gaa atg gct cta aaa gag ctc    624
Phe Pro Gln Val Thr Lys Asn Asp Leu Glu Met Ala Leu Lys Glu Leu
        195                 200                 205

cag aaa gca aat tcc gta ctt ctg tac cat gcc gaa tta ccc gct cct    672
Gln Lys Ala Asn Ser Val Leu Leu Tyr His Ala Glu Leu Pro Ala Pro
    210                 215                 220

caa gaa aat gtt aca agc aat gaa act gaa aag tac atg act tac ctg    720
Gln Glu Asn Val Thr Ser Asn Glu Thr Glu Lys Tyr Met Thr Tyr Leu
225                 230                 235                 240

aaa aca cga cct cca agt atg gaa gta aat gct att gat atg att ata    768
Lys Thr Arg Pro Pro Ser Met Glu Val Asn Ala Ile Asp Met Ile Ile
                245                 250                 255

gac ctc aca aaa aaa tat aaa gtt agg tct cac ata gtg cat cta tca    816
Asp Leu Thr Lys Lys Tyr Lys Val Arg Ser His Ile Val His Leu Ser
            260                 265                 270

gca gca ggt gct tta ccg caa ttg aaa aaa gcg cgc tca gag aac gtt    864
Ala Ala Gly Ala Leu Pro Gln Leu Lys Lys Ala Arg Ser Glu Asn Val
        275                 280                 285

cca ctt tcg att gaa act tgt cat cat tac tta acc ttt gct gct gaa    912
Pro Leu Ser Ile Glu Thr Cys His His Tyr Leu Thr Phe Ala Ala Glu
    290                 295                 300

gat gtt cca gat gga cat act gaa tac aaa tgc gct cca cca att aga    960
Asp Val Pro Asp Gly His Thr Glu Tyr Lys Cys Ala Pro Pro Ile Arg
305                 310                 315                 320

gaa gaa agt aat caa gaa aaa tta tgg caa gct ttg gaa aac aga gat    1008
Glu Glu Ser Asn Gln Glu Lys Leu Trp Gln Ala Leu Glu Asn Arg Asp
                325                 330                 335

att gat atg gta gtc agt gat cat tct cca tca cct gct gca ctg aaa    1056
Ile Asp Met Val Val Ser Asp His Ser Pro Ser Pro Ala Ala Leu Lys
            340                 345                 350
```

```
ggc ctg tgc aat ggt tgt cat cct gat ttc cta aaa gct tgg ggt gga   1104
Gly Leu Cys Asn Gly Cys His Pro Asp Phe Leu Lys Ala Trp Gly Gly
        355                 360                 365

att gct ggt atg cag ttt gga tta tct tta ata agg gac cgg tgc ttc   1152
Ile Ala Gly Met Gln Phe Gly Leu Ser Leu Ile Arg Asp Arg Cys Phe
    370                 375                 380
```

<210> 5
<211> 384
<212> PRT
<213> Ctenocephalides felis

<400> 5

```
Met Lys Ser Ser Thr Cys Ile Phe Leu Leu Val Ile Met Leu Asn Cys
1               5                   10                  15

Lys Asn Leu Val Asn Ala Ala Cys Thr Asn Asn Ala Pro Pro Met Lys
            20                  25                  30

Ile Phe Arg Ser Arg Arg Val Leu Leu Gly Asp Gly Thr Glu Arg Asp
        35                  40                  45

Ala Gly Ile Val Val Asp Ser Ser Gly Arg Ile Lys Ser Ile Ile Ser
    50                  55                  60

Gly Glu Glu Val Glu Arg Ile Ala Asn Glu Thr Lys Val Glu Val Leu
65                  70                  75                  80

Asp Tyr Gly Gln Phe Ser Ile Trp Pro Gly Val Ile Asp Ser His Val
                85                  90                  95

His Val Asn Glu Pro Gly Arg Glu Ser Trp Glu Gly Tyr Thr Thr Ala
            100                 105                 110

Thr Lys Ala Ala Ala Trp Gly Gly Ile Thr Thr Ile Val Asp Met Pro
        115                 120                 125

Leu Asn Ser Ile Pro Pro Thr Thr Thr Val Glu Asn Leu Arg Thr Lys
    130                 135                 140

Val Asn Ser Ala Cys Gly Lys Thr His Val Asp Val Ala Phe Trp Gly
145                 150                 155                 160

Gly Val Ile Pro Gly Asn Ala His Glu Leu Leu Pro Leu Ile Asn Ala
                165                 170                 175
```

Gly Val Arg Gly Phe Lys Cys Phe Thr Ser Glu Ser Gly Val Asp Glu
180 185 190

Phe Pro Gln Val Thr Lys Asn Asp Leu Glu Met Ala Leu Lys Glu Leu
195 200 205

Gln Lys Ala Asn Ser Val Leu Leu Tyr His Ala Glu Leu Pro Ala Pro
210 215 220

Gln Glu Asn Val Thr Ser Asn Glu Thr Glu Lys Tyr Met Thr Tyr Leu
225 230 235 240

Lys Thr Arg Pro Pro Ser Met Glu Val Asn Ala Ile Asp Met Ile Ile
245 250 255

Asp Leu Thr Lys Lys Tyr Lys Val Arg Ser His Ile Val His Leu Ser
260 265 270

Ala Ala Gly Ala Leu Pro Gln Leu Lys Lys Ala Arg Ser Glu Asn Val
275 280 285

Pro Leu Ser Ile Glu Thr Cys His His Tyr Leu Thr Phe Ala Ala Glu
290 295 300

Asp Val Pro Asp Gly His Thr Glu Tyr Lys Cys Ala Pro Pro Ile Arg
305 310 315 320

Glu Glu Ser Asn Gln Glu Lys Leu Trp Gln Ala Leu Glu Asn Arg Asp
325 330 335

Ile Asp Met Val Val Ser Asp His Ser Pro Ser Pro Ala Ala Leu Lys
340 345 350

Gly Leu Cys Asn Gly Cys His Pro Asp Phe Leu Lys Ala Trp Gly Gly
355 360 365

Ile Ala Gly Met Gln Phe Gly Leu Ser Leu Ile Arg Asp Arg Cys Phe
370 375 380

<210> 6
<211> 1152
<212> DNA
<213> Ctenocephalides felis

<400> 6

gaagcaccgg tcccttatta aagataatcc aaactgcata ccagcaattc caccccaagc 60

```
ttttaggaaa tcaggatgac aaccattgca caggcctttc agtgcagcag gtgatggaga 120
atgatcactg actaccatat caatatctct gttttccaaa gcttgccata atttttcttg 180
attactttct tctctaattg gtggagcgca tttgtattca gtatgtccat ctggaacatc 240
ttcagcagca aaggttaagt aatgatgaca agtttcaatc gaaagtggaa cgttctctga 300
gcgcgctttt ttcaattgcg gtaaagcacc tgctgctgat agatgcacta tgtgagacct 360
aactttatat ttttttgtga ggtctataat catatcaata gcatttactt ccatacttgg 420
aggtcgtgtt ttcaggtaag tcatgtactt ttcagtttca ttgcttgtaa catttcttg 480
aggagcgggt aattcggcat ggtacagaag tacggaattt gctttctgga gctcttttag 540
agccatttcc agatcatttt tagtaacctg tggaaactca tcgacaccac tttcacttgt 600
aaaacatttg aatcctctta ctccggcgtt gataagtggc aacaattcgt gcgcattgcc 660
aggaatcacg cctccccaga aagcgacatc aacatgcgtt ttaccacagg ctgaattcac 720
ttttgttctc aaattctcta cagtagttgt aggtgggatg gaattcaaag gcatgtctac 780
tattgtggta atcccgcccc aagctgctgc tttagtagct gtggtgtatc cttcccagga 840
ttctcttcct ggttcgttga cgtgcacatg agagtctatc acacctggcc atattgaaaa 900
ttgaccgtag tccaacacct caactttagt ttcgttagct atcctttcca cttcttctcc 960
tgaaattata ctttttattc ttccggagga atcaactaca atgccagcat ctctttcagt 1020
accatcaccg agaagaactc ttcggctacg gaatatcttc attggaggcg cgttgttggt 1080
gcacgcagca ttaacaaggt tcttgcaatt cagcataatg accagaagaa aaatacaggt 1140
actgcttttc at                                                     1152
```

<210> 7
<211> 1128
<212> DNA
<213> Ctenocephalides felis

<220>
<221> CDS
<222> (6)..(821)

<400> 7

```
tcaca atg aag ttc tta gga gct tta ttg gtt gca gtg ttt gcc ttg ggt 50
      Met Lys Phe Leu Gly Ala Leu Leu Val Ala Val Phe Ala Leu Gly
        1               5                  10                  15

gct gtg gct gct gac agg aat tcg ccc aca tat gtc cgc ggt ttc cca   98
Ala Val Ala Ala Asp Arg Asn Ser Pro Thr Tyr Val Arg Gly Phe Pro
                20                  25                  30

gtg gga aga tcc aga gca cga aca aca ttt ggc aat gaa gaa ata aag   146
Val Gly Arg Ser Arg Ala Arg Thr Thr Phe Gly Asn Glu Glu Ile Lys
            35                  40                  45

tgt act aat aag cag ttg gga aca ttt tgt cac gat tgt tct act ttg   194
Cys Thr Asn Lys Gln Leu Gly Thr Phe Cys His Asp Cys Ser Thr Leu
        50                  55                  60

aag ttg tgc gct gga caa gaa acc cca att aca aca atc aat tgc aga   242
```

```
Lys Leu Cys Ala Gly Gln Glu Thr Pro Ile Thr Thr Ile Asn Cys Arg
     65                  70                  75

gac tca aat tcc gat gct cca ttt tgt gta gat gat atg tgc tca tca     290
Asp Ser Asn Ser Asp Ala Pro Phe Cys Val Asp Asp Met Cys Ser Ser
 80                  85                  90                  95

aaa cct ggg gaa aac tgt aag acg gca gaa act aca tgc gcc gtt gta     338
Lys Pro Gly Glu Asn Cys Lys Thr Ala Glu Thr Thr Cys Ala Val Val
                100                 105                 110

gga tat cag cca gat ccg aaa gac tgc aca aga tac tta ttc tgc aaa     386
Gly Tyr Gln Pro Asp Pro Lys Asp Cys Thr Arg Tyr Leu Phe Cys Lys
            115                 120                 125

gat ggt aaa ggt cag gtt ttc gaa tgc cca cct aac tat gta tat gat     434
Asp Gly Lys Gly Gln Val Phe Glu Cys Pro Pro Asn Tyr Val Tyr Asp
        130                 135                 140

cat tct aaa aat atg tgt aaa aag aaa tcg tca gaa gct gat tgc acc     482
His Ser Lys Asn Met Cys Lys Lys Lys Ser Ser Glu Ala Asp Cys Thr
    145                 150                 155

gtc atg aaa tgc aca aat ccc aat tct ttt ata acc tat gca ccg gac     530
Val Met Lys Cys Thr Asn Pro Asn Ser Phe Ile Thr Tyr Ala Pro Asp
160                 165                 170                 175

cca tca att tat gct tgg tgc aat gac aaa ttg caa ccg atc gta ctg     578
Pro Ser Ile Tyr Ala Trp Cys Asn Asp Lys Leu Gln Pro Ile Val Leu
                180                 185                 190

aaa tgt gaa gac gac gtc aac gaa tgg ttt gac cca aaa tct ttc tcg     626
Lys Cys Glu Asp Asp Val Asn Glu Trp Phe Asp Pro Lys Ser Phe Ser
            195                 200                 205

tgc aga act gca tgc aaa agt gaa aac gtt ttt tcc gat cga aga gat     674
Cys Arg Thr Ala Cys Lys Ser Glu Asn Val Phe Ser Asp Arg Arg Asp
        210                 215                 220

tgt aaa aaa tat tat caa tgt ttc ttg gtt aac aac aaa tgg caa ata     722
Cys Lys Lys Tyr Tyr Gln Cys Phe Leu Val Asn Asn Lys Trp Gln Ile
    225                 230                 235

aaa cat tat gat tgt cca aat ggc ttg cac ttt gat aaa acg gag ttg     770
Lys His Tyr Asp Cys Pro Asn Gly Leu His Phe Asp Lys Thr Glu Leu
240                 245                 250                 255

cga tgc ata ccc acg cca ccc ggc gaa gaa tgc aaa agt gag att gct     818
```

```
Arg Cys Ile Pro Thr Pro Pro Gly Glu Glu Cys Lys Ser Glu Ile Ala
                260                 265                 270

aag taaggcttaa accaggaaaa caatcttgaa tagactaatt aggattcaaa       871
Lys

ttatcataaa gtagtcaatt aatataataa atacacaaat gatctgtgca attaaatata 931

aaaaatatgt ttaaaaatta aaatgtataa aattgtattt tatgtaagga gcacaaacaa 991

aatgtcctta actatagtaa tttctgatta tttaaaatat ataaatatag aagctttatg 1051

aaattacatg tatcttttta ataaaaataa atcgtttggg ccgttnnaaa aaaaaaaaaa 1111

aaaaaaaaan aaaaaaa                                                1128
```

<210> 8
<211> 272
<212> PRT
<213> Ctenocephalides felis

<400> 8

```
Met Lys Phe Leu Gly Ala Leu Leu Val Ala Val Phe Ala Leu Gly Ala
1               5                   10                  15

Val Ala Ala Asp Arg Asn Ser Pro Thr Tyr Val Arg Gly Phe Pro Val
            20                  25                  30

Gly Arg Ser Arg Ala Arg Thr Thr Phe Gly Asn Glu Glu Ile Lys Cys
        35                  40                  45

Thr Asn Lys Gln Leu Gly Thr Phe Cys His Asp Cys Ser Thr Leu Lys
    50                  55                  60

Leu Cys Ala Gly Gln Glu Thr Pro Ile Thr Thr Ile Asn Cys Arg Asp
65                  70                  75                  80

Ser Asn Ser Asp Ala Pro Phe Cys Val Asp Asp Met Cys Ser Ser Lys
                85                  90                  95

Pro Gly Glu Asn Cys Lys Thr Ala Glu Thr Thr Cys Ala Val Val Gly
            100                 105                 110

Tyr Gln Pro Asp Pro Lys Asp Cys Thr Arg Tyr Leu Phe Cys Lys Asp
        115                 120                 125

Gly Lys Gly Gln Val Phe Glu Cys Pro Pro Asn Tyr Val Tyr Asp His
```

130 135 140

Ser Lys Asn Met Cys Lys Lys Lys Ser Ser Glu Ala Asp Cys Thr Val
145 150 155 160

Met Lys Cys Thr Asn Pro Asn Ser Phe Ile Thr Tyr Ala Pro Asp Pro
165 170 175

Ser Ile Tyr Ala Trp Cys Asn Asp Lys Leu Gln Pro Ile Val Leu Lys
180 185 190

Cys Glu Asp Asp Val Asn Glu Trp Phe Asp Pro Lys Ser Phe Ser Cys
195 200 205

Arg Thr Ala Cys Lys Ser Glu Asn Val Phe Ser Asp Arg Arg Asp Cys
210 215 220

Lys Lys Tyr Tyr Gln Cys Phe Leu Val Asn Asn Lys Trp Gln Ile Lys
225 230 235 240

His Tyr Asp Cys Pro Asn Gly Leu His Phe Asp Lys Thr Glu Leu Arg
245 250 255

Cys Ile Pro Thr Pro Pro Gly Glu Glu Cys Lys Ser Glu Ile Ala Lys
260 265 270

<210> 9
<211> 1128
<212> DNA
<213> Ctenocephalides felis

<400> 9

```
tttttttntt ttttttttt ttttttttt nnaacggccc aaacgattta tttttattaa 60
aaagatacat gtaatttcat aaagcttcta tatttatata ttttaaataa tcagaaatta 120
ctatagttaa ggacattttg tttgtgctcc ttacataaaa tacaatttta tacattttaa 180
tttttaaaca tattttttat atttaattgc acagatcatt tgtgtattta ttatattaat 240
tgactacttt atgataattt gaatcctaat tagtctattc aagattgttt tcctggttta 300
agccttactt agcaatctca cttttgcatt cttcgccggg tggcgtgggt atgcatcgca 360
actccgtttt atcaaagtgc aagccatttg gacaatcata atgttttatt tgccatttgt 420
tgttaaccaa gaaacattga taatattttt tacaatctct tcgatcggaa aaaacgtttt 480
cacttttgca tgcagttctg cacgagaaag attttgggtc aaaccattcg ttgacgtcgt 540
cttcacattt cagtacgatc ggttgcaatt tgtcattgca ccaagcataa attgatgggt 600
ccggtgcata ggttataaaa gaattgggat ttgtgcattt catgacggtg caatcagctt 660
ctgacgattt ctttttacac atatttttag aatgatcata tacatagtta ggtgggcatt 720
cgaaaacctg acctttacca tctttgcaga ataagtatct tgtgcagtct ttcggatctg 780
gctgatatcc tacaacggcg catgtagttt ctgccgtctt acagttttcc ccaggttttg 840
```

```
atgagcacat atcatctaca caaaatggag catcggaatt tgagtctctg caattgattg 900
ttgtaattgg ggtttcttgt ccagcgcaca acttcaaagt agaacaatcg tgacaaaatg 960
ttcccaactg cttattagta cactttattt cttcattgcc aaatgttgtt cgtgctctgg 1020
atcttcccac tgggaaaccg cggacatatg tgggcgaatt cctgtcagca gccacagcac 1080
ccaaggcaaa cactgcaacc aataaagctc ctaagaactt cattgtga             1128
```

<210> 10
<211> 816
<212> DNA
<213> Ctenocephalides felis

<220>
<221> CDS
<222> (1)..(816)

<400> 10

```
atg aag ttc tta gga gct tta ttg gtt gca gtg ttt gcc ttg ggt gct    48
Met Lys Phe Leu Gly Ala Leu Leu Val Ala Val Phe Ala Leu Gly Ala
1               5                   10                  15

gtg gct gct gac agg aat tcg ccc aca tat gtc cgc ggt ttc cca gtg    96
Val Ala Ala Asp Arg Asn Ser Pro Thr Tyr Val Arg Gly Phe Pro Val
            20                  25                  30

gga aga tcc aga gca cga aca aca ttt ggc aat gaa gaa ata aag tgt    144
Gly Arg Ser Arg Ala Arg Thr Thr Phe Gly Asn Glu Glu Ile Lys Cys
        35                  40                  45

act aat aag cag ttg gga aca ttt tgt cac gat tgt tct act ttg aag    192
Thr Asn Lys Gln Leu Gly Thr Phe Cys His Asp Cys Ser Thr Leu Lys
    50                  55                  60

ttg tgc gct gga caa gaa acc cca att aca aca atc aat tgc aga gac    240
Leu Cys Ala Gly Gln Glu Thr Pro Ile Thr Thr Ile Asn Cys Arg Asp
65                  70                  75                  80

tca aat tcc gat gct cca ttt tgt gta gat gat atg tgc tca tca aaa    288
Ser Asn Ser Asp Ala Pro Phe Cys Val Asp Asp Met Cys Ser Ser Lys
                85                  90                  95

cct ggg gaa aac tgt aag acg gca gaa act aca tgc gcc gtt gta gga    336
Pro Gly Glu Asn Cys Lys Thr Ala Glu Thr Thr Cys Ala Val Val Gly
            100                 105                 110

tat cag cca gat ccg aaa gac tgc aca aga tac tta ttc tgc aaa gat    384
Tyr Gln Pro Asp Pro Lys Asp Cys Thr Arg Tyr Leu Phe Cys Lys Asp
        115                 120                 125
```

```
ggt aaa ggt cag gtt ttc gaa tgc cca cct aac tat gta tat gat cat      432
Gly Lys Gly Gln Val Phe Glu Cys Pro Pro Asn Tyr Val Tyr Asp His
    130                 135                 140

tct aaa aat atg tgt aaa aag aaa tcg tca gaa gct gat tgc acc gtc      480
Ser Lys Asn Met Cys Lys Lys Lys Ser Ser Glu Ala Asp Cys Thr Val
145                 150                 155                 160

atg aaa tgc aca aat ccc aat tct ttt ata acc tat gca ccg gac cca      528
Met Lys Cys Thr Asn Pro Asn Ser Phe Ile Thr Tyr Ala Pro Asp Pro
                165                 170                 175

tca att tat gct tgg tgc aat gac aaa ttg caa ccg atc gta ctg aaa      576
Ser Ile Tyr Ala Trp Cys Asn Asp Lys Leu Gln Pro Ile Val Leu Lys
            180                 185                 190

tgt gaa gac gac gtc aac gaa tgg ttt gac cca aaa tct ttc tcg tgc      624
Cys Glu Asp Asp Val Asn Glu Trp Phe Asp Pro Lys Ser Phe Ser Cys
        195                 200                 205

aga act gca tgc aaa agt gaa aac gtt ttt tcc gat cga aga gat tgt      672
Arg Thr Ala Cys Lys Ser Glu Asn Val Phe Ser Asp Arg Arg Asp Cys
    210                 215                 220

aaa aaa tat tat caa tgt ttc ttg gtt aac aac aaa tgg caa ata aaa      720
Lys Lys Tyr Tyr Gln Cys Phe Leu Val Asn Asn Lys Trp Gln Ile Lys
225                 230                 235                 240

cat tat gat tgt cca aat ggc ttg cac ttt gat aaa acg gag ttg cga      768
His Tyr Asp Cys Pro Asn Gly Leu His Phe Asp Lys Thr Glu Leu Arg
                245                 250                 255

tgc ata ccc acg cca ccc ggc gaa gaa tgc aaa agt gag att gct aag      816
Cys Ile Pro Thr Pro Pro Gly Glu Glu Cys Lys Ser Glu Ile Ala Lys
            260                 265                 270
```

<210> 11

<211> 272
<212> PRT
<213> Ctenocephalides felis

<400> 11

```
Met Lys Phe Leu Gly Ala Leu Leu Val Ala Val Phe Ala Leu Gly Ala
1               5                   10                  15

Val Ala Ala Asp Arg Asn Ser Pro Thr Tyr Val Arg Gly Phe Pro Val
```

```
            20                  25                  30

Gly Arg Ser Arg Ala Arg Thr Thr Phe Gly Asn Glu Glu Ile Lys Cys
        35                  40                  45

Thr Asn Lys Gln Leu Gly Thr Phe Cys His Asp Cys Ser Thr Leu Lys
    50                  55                  60

Leu Cys Ala Gly Gln Glu Thr Pro Ile Thr Thr Ile Asn Cys Arg Asp
65                  70                  75                  80

Ser Asn Ser Asp Ala Pro Phe Cys Val Asp Asp Met Cys Ser Ser Lys
                85                  90                  95

Pro Gly Glu Asn Cys Lys Thr Ala Glu Thr Thr Cys Ala Val Val Gly
            100                 105                 110

Tyr Gln Pro Asp Pro Lys Asp Cys Thr Arg Tyr Leu Phe Cys Lys Asp
        115                 120                 125

Gly Lys Gly Gln Val Phe Glu Cys Pro Pro Asn Tyr Val Tyr Asp His
    130                 135                 140

Ser Lys Asn Met Cys Lys Lys Lys Ser Ser Glu Ala Asp Cys Thr Val
145                 150                 155                 160

Met Lys Cys Thr Asn Pro Asn Ser Phe Ile Thr Tyr Ala Pro Asp Pro
                165                 170                 175

Ser Ile Tyr Ala Trp Cys Asn Asp Lys Leu Gln Pro Ile Val Leu Lys
            180                 185                 190

Cys Glu Asp Asp Val Asn Glu Trp Phe Asp Pro Lys Ser Phe Ser Cys
        195                 200                 205

Arg Thr Ala Cys Lys Ser Glu Asn Val Phe Ser Asp Arg Arg Asp Cys
    210                 215                 220

Lys Lys Tyr Tyr Gln Cys Phe Leu Val Asn Asn Lys Trp Gln Ile Lys
225                 230                 235                 240

His Tyr Asp Cys Pro Asn Gly Leu His Phe Asp Lys Thr Glu Leu Arg
                245                 250                 255

Cys Ile Pro Thr Pro Pro Gly Glu Glu Cys Lys Ser Glu Ile Ala Lys
            260                 265                 270
```

<210> 12
<211> 816

<212> DNA
<213> Ctenocephalides felis

<400> 12

```
cttagcaatc tcacttttgc attcttcgcc gggtggcgtg ggtatgcatc gcaactccgt 60
tttatcaaag tgcaagccat ttggacaatc ataatgtttt atttgccatt tgttgttaac 120
caagaaacat tgataatatt ttttacaatc tcttcgatcg gaaaaaacgt tttcactttt 180
gcatgcagtt ctgcacgaga aagattttgg gtcaaaccat tcgttgacgt cgtcttcaca 240
tttcagtacg atcggttgca atttgtcatt gcaccaagca taaattgatg ggtccggtgc 300
ataggttata aaagaattgg gatttgtgca tttcatgacg gtgcaatcag cttctgacga 360
tttcttttta cacatatttt tagaatgatc atatacatag ttaggtgggc attcgaaaac 420
ctgaccttta ccatctttgc agaataagta tcttgtgcag tctttcggat ctggctgata 480
tcctacaacg gcgcatgtag tttctgccgt cttacagttt tccccaggtt ttgatgagca 540
catatcatct acacaaaatg gagcatcgga atttgagtct ctgcaattga ttgttgtaat 600
tggggtttct tgtccagcgc acaacttcaa agtagaacaa tcgtgacaaa atgttcccaa 660
ctgcttatta gtacacttta tttcttcatt gccaaatgtt gttcgtgctc tggatcttcc 720
cactgggaaa ccgcggacat atgtgggcga attcctgtca gcagccacag cacccaaggc 780
aaacactgca accaataaag ctcctaagaa cttcat                           816
```

<210> 13
<211> 1714
<212> DNA
<213> Ctenocephalides felis

<220>
<221> CDS
<222> (294)..(1271)

<400> 13

```
atcgcaagta tcgggtgccg cgttcaaatt tacacccggg gcctcttcac gatttttctc 60

cgtgacacaa attatgacca gtgatccgaa acaaatctct acctgaacta cccacatgtt 120

attcagtgaa ctaaacaaat ttccttaccc agaatacaac ataagaacta acgatttgaa 180

ctgtttataa ttcataatat aaccgcatct tttatttcta attttatctt ttagtgaata 240

aatttatttg ttgttgaata aattaataat tgtgtacgtt caatttgttc gtg atg   296
                                                            Met
                                                            1

gag aaa ata gtt gga cgc gat gga aca gaa gtc atc aca tac gag ttt   344
Glu Lys Ile Val Gly Arg Asp Gly Thr Glu Val Ile Thr Tyr Glu Phe
            5                   10                  15
```

```
cca tat atg agg aga gcg gat aag cgg act aac tgg gaa aag ttc cgg     392
Pro Tyr Met Arg Arg Ala Asp Lys Arg Thr Asn Trp Glu Lys Phe Arg
            20                  25                  30

cag gga tgc tac aat ccg gac gag ggc agt ttt ttg ggc agg caa cca     440
Gln Gly Cys Tyr Asn Pro Asp Glu Gly Ser Phe Leu Gly Arg Gln Pro
        35                  40                  45

tca gca tgg gct cgc gta tcg cta ttt tac ttg gta ttc tac aca gtt     488
Ser Ala Trp Ala Arg Val Ser Leu Phe Tyr Leu Val Phe Tyr Thr Val
    50                  55                  60                  65

ttg gca tcc cta ttc aca ata tgc atg tac aca atg cta tct acg ata     536
Leu Ala Ser Leu Phe Thr Ile Cys Met Tyr Thr Met Leu Ser Thr Ile
                    70                  75                  80

gac aag gaa tac cca aaa tgg cag ctt gag gat tca ata ata gga act     584
Asp Lys Glu Tyr Pro Lys Trp Gln Leu Glu Asp Ser Ile Ile Gly Thr
                85                  90                  95

aat cct gga ctg gga ttt agg cca ata gca gat aac aca gaa gag gga     632
Asn Pro Gly Leu Gly Phe Arg Pro Ile Ala Asp Asn Thr Glu Glu Gly
            100                 105                 110

tct cta ata tgg ttc gac gcc aaa aat gaa act gaa gtt gcg aaa tgg     680
Ser Leu Ile Trp Phe Asp Ala Lys Asn Glu Thr Glu Val Ala Lys Trp
        115                 120                 125

aca aca ata att gac gaa ttt tta gct cct tac aaa aat cgg tct caa     728
Thr Thr Ile Ile Asp Glu Phe Leu Ala Pro Tyr Lys Asn Arg Ser Gln
    130                 135                 140                 145

ttg cca agc cac ggt gaa aat caa atg ttc tgc gac tac gaa acg ggg     776
Leu Pro Ser His Gly Glu Asn Gln Met Phe Cys Asp Tyr Glu Thr Gly
                    150                 155                 160

ccc aac act gca aat cgt gtt tgt gcc gta gcc gtc gag aag tgg ggc     824
Pro Asn Thr Ala Asn Arg Val Cys Ala Val Ala Val Glu Lys Trp Gly
                165                 170                 175

tca tgc aca tca cag gct aac tac ggc ttt gga caa tcc gca cct tgt     872
Ser Cys Thr Ser Gln Ala Asn Tyr Gly Phe Gly Gln Ser Ala Pro Cys
            180                 185                 190

gtc ttt ctt aag ctt aac agg ata tat aat tgg gta cca gat tat tat     920
Val Phe Leu Lys Leu Asn Arg Ile Tyr Asn Trp Val Pro Asp Tyr Tyr
        195                 200                 205
```

```
gat gat gtg gcg acg ctg cct gaa gat atg cct atg gaa ttg aag gat    968
Asp Asp Val Ala Thr Leu Pro Glu Asp Met Pro Met Glu Leu Lys Asp
210                 215                 220                 225

cac ata caa agt ctc aag ccg gat gag aga aaa caa att tgg gtt tcg   1016
His Ile Gln Ser Leu Lys Pro Asp Glu Arg Lys Gln Ile Trp Val Ser
                230                 235                 240

tgt caa gga gaa aat cca gtt gat cga gaa aat ttg ggc cca gtt gaa   1064
Cys Gln Gly Glu Asn Pro Val Asp Arg Glu Asn Leu Gly Pro Val Glu
            245                 250                 255

atg tat cca agc atg gga ttt gct gga tat tat tat cca ttc aga aac   1112
Met Tyr Pro Ser Met Gly Phe Ala Gly Tyr Tyr Tyr Pro Phe Arg Asn
        260                 265                 270

caa cga gat tat ctt agt cca tta gtt gct gtt caa ttc aaa aga cct   1160
Gln Arg Asp Tyr Leu Ser Pro Leu Val Ala Val Gln Phe Lys Arg Pro
    275                 280                 285

aca gtg gga cgt ttg atc aac gtg gaa tgt cgt gcc tgg gcc agg aac   1208
Thr Val Gly Arg Leu Ile Asn Val Glu Cys Arg Ala Trp Ala Arg Asn
290                 295                 300                 305

atc atc tat cgt ggt ggc aac aag gat cga caa gga tcc gtc cat ttc   1256
Ile Ile Tyr Arg Gly Gly Asn Lys Asp Arg Gln Gly Ser Val His Phe
                310                 315                 320

gaa ctg atg att gat tagaatcgac attattagtg ttaattttac tttattgata   1311
Glu Leu Met Ile Asp
            325

tcctaagcat tatcgttctg tgttatcgcg ccttgtacat cgttgcaaaa tagctcgtac 1371

gtcgatgttg tgaatagaat ttaagtttta attttaagta tgataattaa tgaagtgttt 1431

aataaatcaa aatgaacttt gagtataata gactttatat ttatatctaa ataaagttta 1491

cgcggttttg ttatcattaa aggtgtaaga ttttaatatt tataattgtt tatatattag 1551

ctataaatgt gtaaatatac gttatttaat atagtacaaa acaagttgat ttatttaatg 1611

cctattgtga aatatgttag tgtagtataa aatgcttata ttttattatg tatttagaaa 1671

atatattaca tttacttatt actttaaaaa aaaaaaaaaa aaa                   1714
```

<210> 14
<211> 326

<212> PRT
<213> Ctenocephalides felis

<400> 14

```
Met Glu Lys Ile Val Gly Arg Asp Gly Thr Glu Val Ile Thr Tyr Glu
 1               5                  10                  15

Phe Pro Tyr Met Arg Arg Ala Asp Lys Arg Thr Asn Trp Glu Lys Phe
            20                  25                  30

Arg Gln Gly Cys Tyr Asn Pro Asp Glu Gly Ser Phe Leu Gly Arg Gln
        35                  40                  45

Pro Ser Ala Trp Ala Arg Val Ser Leu Phe Tyr Leu Val Phe Tyr Thr
    50                  55                  60

Val Leu Ala Ser Leu Phe Thr Ile Cys Met Tyr Thr Met Leu Ser Thr
65                  70                  75                  80

Ile Asp Lys Glu Tyr Pro Lys Trp Gln Leu Glu Asp Ser Ile Ile Gly
                85                  90                  95

Thr Asn Pro Gly Leu Gly Phe Arg Pro Ile Ala Asp Asn Thr Glu Glu
            100                 105                 110

Gly Ser Leu Ile Trp Phe Asp Ala Lys Asn Glu Thr Glu Val Ala Lys
        115                 120                 125

Trp Thr Thr Ile Ile Asp Glu Phe Leu Ala Pro Tyr Lys Asn Arg Ser
    130                 135                 140

Gln Leu Pro Ser His Gly Glu Asn Gln Met Phe Cys Asp Tyr Glu Thr
145                 150                 155                 160

Gly Pro Asn Thr Ala Asn Arg Val Cys Ala Val Ala Val Glu Lys Trp
                165                 170                 175

Gly Ser Cys Thr Ser Gln Ala Asn Tyr Gly Phe Gly Gln Ser Ala Pro
            180                 185                 190

Cys Val Phe Leu Lys Leu Asn Arg Ile Tyr Asn Trp Val Pro Asp Tyr
        195                 200                 205

Tyr Asp Asp Val Ala Thr Leu Pro Glu Asp Met Pro Met Glu Leu Lys
    210                 215                 220
```

```
Asp His Ile Gln Ser Leu Lys Pro Asp Glu Arg Lys Gln Ile Trp Val
225                 230                 235                 240

Ser Cys Gln Gly Glu Asn Pro Val Asp Arg Glu Asn Leu Gly Pro Val
                245                 250                 255

Glu Met Tyr Pro Ser Met Gly Phe Ala Gly Tyr Tyr Tyr Pro Phe Arg
            260                 265                 270

Asn Gln Arg Asp Tyr Leu Ser Pro Leu Val Ala Val Gln Phe Lys Arg
        275                 280                 285

Pro Thr Val Gly Arg Leu Ile Asn Val Glu Cys Arg Ala Trp Ala Arg
    290                 295                 300

Asn Ile Ile Tyr Arg Gly Gly Asn Lys Asp Arg Gln Gly Ser Val His
305                 310                 315                 320

Phe Glu Leu Met Ile Asp
                325
```

<210> 15
<211> 1714
<212> DNA
<213> Ctenocephalides felis

<400> 15

```
ttttttttt tttttttaa agtaataagt aaatgtaata tattttctaa atacataata 60
aaatataagc attttatact acactaacat atttcacaat aggcattaaa taaatcaact 120
tgttttgtac tatattaaat aacgtatatt tacacattta tagctaatat ataaacaatt 180
ataaatatta aaatcttaca cctttaatga taacaaaacc gcgtaaactt tatttagata 240
taaatataaa gtctattata ctcaaagttc attttgattt attaaacact tcattaatta 300
tcatacttaa aattaaaact taaattctat tcacaacatc gacgtacgag ctattttgca 360
acgatgtaca aggcgcgata acacagaacg ataatgctta ggatatcaat aaagtaaaat 420
taacactaat aatgtcgatt ctaatcaatc atcagttcga aatggacgga tccttgtcga 480
tccttgttgc caccacgata gatgatgttc ctggcccagg cacgacattc cacgttgatc 540
aaacgtccca ctgtaggtct tttgaattga acagcaacta atggactaag ataatctcgt 600
tggtttctga atggataata atatccagca aatcccatgc ttggatacat ttcaactggg 660
cccaaatttt ctcgatcaac tggattttct ccttgacacg aaacccaaat ttgttttctc 720
tcatccggct tgagactttg tatgtgatcc ttcaattcca taggcatatc ttcaggcagc 780
gtcgccacat catcataata atctggtacc caattatata tcctgttaag cttaagaaag 840
acacaaggtg cggattgtcc aaagccgtag ttagcctgtg atgtgcatga gccccacttc 900
tcgacggcta cggcacaaac acgatttgca gtgttgggcc ccgtttcgta gtcgcagaac 960
atttgatttt caccgtggct tggcaattga gaccgatttt tgtaaggagc taaaaattcg 1020
tcaattattg ttgtccattt cgcaacttca gtttcatttt tggcgtcgaa ccatattaga 1080
gatccctctt ctgtgttatc tgctattggc ctaaatccca gtccaggatt agttcctatt 1140
```

```
attgaatcct caagctgcca ttttgggtat tccttgtcta tcgtagatag cattgtgtac 1200
atgcatattg tgaataggga tgccaaaact gtgtagaata ccaagtaaaa tagcgatacg 1260
cgagcccatg ctgatggttg cctgcccaaa aaactgccct cgtccggatt gtagcatccc 1320
tgccggaact tttcccagtt agtccgctta tccgctctcc tcatatatgg aaactcgtat 1380
gtgatgactt ctgttccatc gcgtccaact attttctcca tcacgaacaa attgaacgta 1440
cacaattatt aatttattca acaacaaata aatttattca ctaaaagata aaattagaaa 1500
taaaagatgc ggttatatta tgaattataa acagttcaaa tcgttagttc ttatgttgta 1560
ttctgggtaa ggaaatttgt ttagttcact gaataacatg tgggtagttc aggtagagat 1620
ttgtttcgga tcactggtca taatttgtgt cacggagaaa aatcgtgaag aggccccggg 1680
tgtaaatttg aacgcggcac ccgatacttg cgat                             1714
```

<210> 16
<211> 978
<212> DNA
<213> Ctenocephalides felis

<220>
<221> CDS
<222> (1)..(978)

<400> 16

```
atg gag aaa ata gtt gga cgc gat gga aca gaa gtc atc aca tac gag     48
Met Glu Lys Ile Val Gly Arg Asp Gly Thr Glu Val Ile Thr Tyr Glu
1               5                   10                  15

ttt cca tat atg agg aga gcg gat aag cgg act aac tgg gaa aag ttc     96
Phe Pro Tyr Met Arg Arg Ala Asp Lys Arg Thr Asn Trp Glu Lys Phe
            20                  25                  30

cgg cag gga tgc tac aat ccg gac gag ggc agt ttt ttg ggc agg caa     144
Arg Gln Gly Cys Tyr Asn Pro Asp Glu Gly Ser Phe Leu Gly Arg Gln
        35                  40                  45

cca tca gca tgg gct cgc gta tcg cta ttt tac ttg gta ttc tac aca     192
Pro Ser Ala Trp Ala Arg Val Ser Leu Phe Tyr Leu Val Phe Tyr Thr
    50                  55                  60

gtt ttg gca tcc cta ttc aca ata tgc atg tac aca atg cta tct acg     240
Val Leu Ala Ser Leu Phe Thr Ile Cys Met Tyr Thr Met Leu Ser Thr
65                  70                  75                  80

ata gac aag gaa tac cca aaa tgg cag ctt gag gat tca ata ata gga     288
Ile Asp Lys Glu Tyr Pro Lys Trp Gln Leu Glu Asp Ser Ile Ile Gly
                85                  90                  95

act aat cct gga ctg gga ttt agg cca ata gca gat aac aca gaa gag     336
Thr Asn Pro Gly Leu Gly Phe Arg Pro Ile Ala Asp Asn Thr Glu Glu
```

```
            100                 105                 110

gga tct cta ata tgg ttc gac gcc aaa aat gaa act gaa gtt gcg aaa     384
Gly Ser Leu Ile Trp Phe Asp Ala Lys Asn Glu Thr Glu Val Ala Lys
        115                 120                 125

tgg aca aca ata att gac gaa ttt tta gct cct tac aaa aat cgg tct     432
Trp Thr Thr Ile Ile Asp Glu Phe Leu Ala Pro Tyr Lys Asn Arg Ser
    130                 135                 140

caa ttg cca agc cac ggt gaa aat caa atg ttc tgc gac tac gaa acg     480
Gln Leu Pro Ser His Gly Glu Asn Gln Met Phe Cys Asp Tyr Glu Thr
145                 150                 155                 160

ggg ccc aac act gca aat cgt gtt tgt gcc gta gcc gtc gag aag tgg     528
Gly Pro Asn Thr Ala Asn Arg Val Cys Ala Val Ala Val Glu Lys Trp
                165                 170                 175

ggc tca tgc aca tca cag gct aac tac ggc ttt gga caa tcc gca cct     576
Gly Ser Cys Thr Ser Gln Ala Asn Tyr Gly Phe Gly Gln Ser Ala Pro
            180                 185                 190

tgt gtc ttt ctt aag ctt aac agg ata tat aat tgg gta cca gat tat     624
Cys Val Phe Leu Lys Leu Asn Arg Ile Tyr Asn Trp Val Pro Asp Tyr
        195                 200                 205

tat gat gat gtg gcg acg ctg cct gaa gat atg cct atg gaa ttg aag     672
Tyr Asp Asp Val Ala Thr Leu Pro Glu Asp Met Pro Met Glu Leu Lys
    210                 215                 220

gat cac ata caa agt ctc aag ccg gat gag aga aaa caa att tgg gtt     720
Asp His Ile Gln Ser Leu Lys Pro Asp Glu Arg Lys Gln Ile Trp Val
225                 230                 235                 240

tcg tgt caa gga gaa aat cca gtt gat cga gaa aat ttg ggc cca gtt     768
Ser Cys Gln Gly Glu Asn Pro Val Asp Arg Glu Asn Leu Gly Pro Val
                245                 250                 255

gaa atg tat cca agc atg gga ttt gct gga tat tat tat cca ttc aga     816
Glu Met Tyr Pro Ser Met Gly Phe Ala Gly Tyr Tyr Tyr Pro Phe Arg
            260                 265                 270

aac caa cga gat tat ctt agt cca tta gtt gct gtt caa ttc aaa aga     864
Asn Gln Arg Asp Tyr Leu Ser Pro Leu Val Ala Val Gln Phe Lys Arg
        275                 280                 285

cct aca gtg gga cgt ttg atc aac gtg gaa tgt cgt gcc tgg gcc agg     912
Pro Thr Val Gly Arg Leu Ile Asn Val Glu Cys Arg Ala Trp Ala Arg
```

```
    290                 295                 300

aac atc atc tat cgt ggt ggc aac aag gat cga caa gga tcc gtc cat     960
Asn Ile Ile Tyr Arg Gly Gly Asn Lys Asp Arg Gln Gly Ser Val His
305                 310                 315                 320

ttc gaa ctg atg att gat                                             978
Phe Glu Leu Met Ile Asp
                325
```

<210> 17
<211> 326
<212> PRT
<213> Ctenocephalides felis

<400> 17

```
Met Glu Lys Ile Val Gly Arg Asp Gly Thr Glu Val Ile Thr Tyr Glu
1               5                   10                  15

Phe Pro Tyr Met Arg Arg Ala Asp Lys Arg Thr Asn Trp Glu Lys Phe
            20                  25                  30

Arg Gln Gly Cys Tyr Asn Pro Asp Glu Gly Ser Phe Leu Gly Arg Gln
        35                  40                  45

Pro Ser Ala Trp Ala Arg Val Ser Leu Phe Tyr Leu Val Phe Tyr Thr
    50                  55                  60

Val Leu Ala Ser Leu Phe Thr Ile Cys Met Tyr Thr Met Leu Ser Thr
65                  70                  75                  80

Ile Asp Lys Glu Tyr Pro Lys Trp Gln Leu Glu Asp Ser Ile Ile Gly
                85                  90                  95

Thr Asn Pro Gly Leu Gly Phe Arg Pro Ile Ala Asp Asn Thr Glu Glu
            100                 105                 110

Gly Ser Leu Ile Trp Phe Asp Ala Lys Asn Glu Thr Glu Val Ala Lys
        115                 120                 125

Trp Thr Thr Ile Ile Asp Glu Phe Leu Ala Pro Tyr Lys Asn Arg Ser
    130                 135                 140

Gln Leu Pro Ser His Gly Glu Asn Gln Met Phe Cys Asp Tyr Glu Thr
145                 150                 155                 160

Gly Pro Asn Thr Ala Asn Arg Val Cys Ala Val Ala Val Glu Lys Trp
```

```
            165                 170                 175

Gly Ser Cys Thr Ser Gln Ala Asn Tyr Gly Phe Gly Gln Ser Ala Pro
        180                 185                 190

Cys Val Phe Leu Lys Leu Asn Arg Ile Tyr Asn Trp Val Pro Asp Tyr
    195                 200                 205

Tyr Asp Asp Val Ala Thr Leu Pro Glu Asp Met Pro Met Glu Leu Lys
    210                 215                 220

Asp His Ile Gln Ser Leu Lys Pro Asp Glu Arg Lys Gln Ile Trp Val
225                 230                 235                 240

Ser Cys Gln Gly Glu Asn Pro Val Asp Arg Glu Asn Leu Gly Pro Val
                245                 250                 255

Glu Met Tyr Pro Ser Met Gly Phe Ala Gly Tyr Tyr Tyr Pro Phe Arg
            260                 265                 270

Asn Gln Arg Asp Tyr Leu Ser Pro Leu Val Ala Val Gln Phe Lys Arg
        275                 280                 285

Pro Thr Val Gly Arg Leu Ile Asn Val Glu Cys Arg Ala Trp Ala Arg
    290                 295                 300

Asn Ile Ile Tyr Arg Gly Gly Asn Lys Asp Arg Gln Gly Ser Val His
305                 310                 315                 320

Phe Glu Leu Met Ile Asp
                325
```

<210> 18
<211> 978
<212> DNA
<213> Ctenocephalides felis

<400> 18

```
atcaatcatc agttcgaaat ggacggatcc ttgtcgatcc ttgttgccac cacgatagat 60
gatgttcctg gcccaggcac gacattccac gttgatcaaa cgtcccactg taggtctttt 120
gaattgaaca gcaactaatg gactaagata atctcgttgg tttctgaatg gataataata 180
tccagcaaat cccatgcttg gatacatttc aactgggccc aaattttctc gatcaactgg 240
attttctcct tgacacgaaa cccaaatttg ttttctctca tccggcttga gactttgtat 300
gtgatccttc aattccatag gcatatcttc aggcagcgtc gccacatcat cataataatc 360
tggtacccaa ttatatatcc tgttaagctt aagaaagaca caaggtgcgg attgtccaaa 420
gccgtagtta gcctgtgatg tgcatgagcc ccacttctcg acggctacgg cacaaacacg 480
```

```
atttgcagtg ttgggccccg tttcgtagtc gcagaacatt tgattttcac cgtggcttgg 540
caattgagac cgatttttgt aaggagctaa aaattcgtca attattgttg tccatttcgc 600
aacttcagtt tcatttttgg cgtcgaacca tattagagat ccctcttctg tgttatctgc 660
tattggccta aatcccagtc caggattagt tcctattatt gaatcctcaa gctgccattt 720
tgggtattcc ttgtctatcg tagatagcat tgtgtacatg catattgtga atagggatgc 780
caaaactgtg tagaatacca agtaaaatag cgatacgcga gcccatgctg atggttgcct 840
gcccaaaaaa ctgccctcgt ccggattgta gcatccctgc cggaactttt cccagttagt 900
ccgcttatcc gctctcctca tatatggaaa ctcgtatgtg atgacttctg ttccatcgcg 960
tccaactatt ttctccat                                               978
```

<210> 19
<211> 2240
<212> DNA
<213> Ctenocephalides felis

<220>
<221> CDS <221> CDS
<222> (1)..(1707)

<400> 19

```
aca att tta aac gca tcc acg acc gtt gaa aaa aat cct gga cat caa    48
Thr Ile Leu Asn Ala Ser Thr Thr Val Glu Lys Asn Pro Gly His Gln
 1               5                  10                  15

acc agt att tca gaa gaa tct acc aca aaa ttg gta aaa aca acc act    96
Thr Ser Ile Ser Glu Glu Ser Thr Thr Lys Leu Val Lys Thr Thr Thr
            20                  25                  30

gaa gac aac cac ctc ggt gta aag agc ctg aat gaa cct ggt gat gaa   144
Glu Asp Asn His Leu Gly Val Lys Ser Leu Asn Glu Pro Gly Asp Glu
        35                  40                  45

caa gaa tta aaa aaa cca tca tca cat ggt aag gag cat att tct tta   192
Gln Glu Leu Lys Lys Pro Ser Ser His Gly Lys Glu His Ile Ser Leu
    50                  55                  60

cca gtg gct tca cca gta cca cca gta tcg cat atc ttc cag gct aca   240
Pro Val Ala Ser Pro Val Pro Pro Val Ser His Ile Phe Gln Ala Thr
65                  70                  75                  80

cca gga gac ctt tgt cca gcc ttc gac gat gca gat cgc ttc acc cag   288
Pro Gly Asp Leu Cys Pro Ala Phe Asp Asp Ala Asp Arg Phe Thr Gln
                85                  90                  95

aca gaa ctt ttg tcc agg ctg aca aac gat tgc agg tac gat aag ctg   336
Thr Glu Leu Leu Ser Arg Leu Thr Asn Asp Cys Arg Tyr Asp Lys Leu
            100                 105                 110
```

```
gag cgc cct ttg ggg cct cac aat ggt gca ggg ccg ctc ccg gtg gcc    384
Glu Arg Pro Leu Gly Pro His Asn Gly Ala Gly Pro Leu Pro Val Ala
        115                 120                 125

gcc aga att tac gtg tat ttt ata caa aat acg gac gcg cac gaa ttg    432
Ala Arg Ile Tyr Val Tyr Phe Ile Gln Asn Thr Asp Ala His Glu Leu
    130                 135                 140

tca ttt tcc gtg acc gtc ctc ctc caa ttt cgt tac cca gga cgc cag    480
Ser Phe Ser Val Thr Val Leu Leu Gln Phe Arg Tyr Pro Gly Arg Gln
145                 150                 155                 160

att ggc cta caa aaa agt ggc acc cac cca gga cgg tca tca tgg gcg    528
Ile Gly Leu Gln Lys Ser Gly Thr His Pro Gly Arg Ser Ser Trp Ala
                165                 170                 175

aat cgc agc tca ggg aca aaa tct ggg tac cca cat gta ttc gtt gcc    576
Asn Arg Ser Ser Gly Thr Lys Ser Gly Tyr Pro His Val Phe Val Ala
            180                 185                 190

aac gag aga tct tcc cag gtt atg ggc aca gat gcc caa tct aag gac    624
Asn Glu Arg Ser Ser Gln Val Met Gly Thr Asp Ala Gln Ser Lys Asp
        195                 200                 205

atg ttg gtg tca gta gct cct gat ggt aca gtc gtc ttt tcg gtc agg    672
Met Leu Val Ser Val Ala Pro Asp Gly Thr Val Val Phe Ser Val Arg
    210                 215                 220

atg aag gca act ttg tac tgt tgg atg aat tta agg aaa ttt cct ttt    720
Met Lys Ala Thr Leu Tyr Cys Trp Met Asn Leu Arg Lys Phe Pro Phe
225                 230                 235                 240

gat gaa caa cag tgt cag atg atg ttg gaa agt tgg aag tac aat aca    768
Asp Glu Gln Gln Cys Gln Met Met Leu Glu Ser Trp Lys Tyr Asn Thr
                245                 250                 255

agt gaa ctc cta ttg act tgg gaa cca act gca cca gta act tta gca    816
Ser Glu Leu Leu Leu Thr Trp Glu Pro Thr Ala Pro Val Thr Leu Ala
            260                 265                 270

cca gaa cta cat ttg acc gaa tat gtc ctt act gac atg tgg gta aat    864
Pro Glu Leu His Leu Thr Glu Tyr Val Leu Thr Asp Met Trp Val Asn
        275                 280                 285

gaa aca gtt gtc aag gct gat ttg gat gac ctg aga cac gga gca ttt    912
Glu Thr Val Val Lys Ala Asp Leu Asp Asp Leu Arg His Gly Ala Phe
    290                 295                 300
```

```
ggt ggg aca tac agt gcc tta agt ttc acg att caa ata agt cgt gaa    960
Gly Gly Thr Tyr Ser Ala Leu Ser Phe Thr Ile Gln Ile Ser Arg Glu
305                 310                 315                 320

atg ggt tac tat tta atg gat tac ttt ttg cca tca gta atg atc gtg   1008
Met Gly Tyr Tyr Leu Met Asp Tyr Phe Leu Pro Ser Val Met Ile Val
                325                 330                 335

tcg tgt tcc tgg gta agt ttt tgg ctg gca gca gac caa tca gca ccc   1056
Ser Cys Ser Trp Val Ser Phe Trp Leu Ala Ala Asp Gln Ser Ala Pro
            340                 345                 350

aga gtc acc tta ggt aca agc acc atg tta tca ttt atc act tta gca   1104
Arg Val Thr Leu Gly Thr Ser Thr Met Leu Ser Phe Ile Thr Leu Ala
        355                 360                 365

agt gcc caa gga aaa act tta ccc aaa gta tcg tac atc aaa gct tca   1152
Ser Ala Gln Gly Lys Thr Leu Pro Lys Val Ser Tyr Ile Lys Ala Ser
    370                 375                 380

gaa atc tgg ttt tta ggt tgc acc ggg ttt att ttt ggg agt tta gtg   1200
Glu Ile Trp Phe Leu Gly Cys Thr Gly Phe Ile Phe Gly Ser Leu Val
385                 390                 395                 400

gaa ttc gcg ttt gtc aac aca att tgg aga cga agg aaa aat gtg gaa   1248
Glu Phe Ala Phe Val Asn Thr Ile Trp Arg Arg Arg Lys Asn Val Glu
                405                 410                 415

ttg aaa aaa gtc aac agc aag tat att ttg aag tca act ttg acg ccg   1296
Leu Lys Lys Val Asn Ser Lys Tyr Ile Leu Lys Ser Thr Leu Thr Pro
            420                 425                 430

agg ttg gcc cgg aag gag ttt cat gct tcg ttt aat tcg aat cct gga   1344
Arg Leu Ala Arg Lys Glu Phe His Ala Ser Phe Asn Ser Asn Pro Gly
        435                 440                 445

ggt ggt aat aag gat gat cag gat ttg gga aga ggg att agg gtc ttt   1392
Gly Gly Asn Lys Asp Asp Gln Asp Leu Gly Arg Gly Ile Arg Val Phe
    450                 455                 460

ccg ccg cct ttg gtc aag gct agg tct tgt tcc agt ctg gat agg agt   1440
Pro Pro Pro Leu Val Lys Ala Arg Ser Cys Ser Ser Leu Asp Arg Ser
465                 470                 475                 480

aat gga tcc ggg aat ttt ttg agc gtc cat gga aat gat cac aaa gtt   1488
Asn Gly Ser Gly Asn Phe Leu Ser Val His Gly Asn Asp His Lys Val
                485                 490                 495
```

```
cca aca ata aca gca caa tgt gca gac gat gcc gca agt gac cag att   1536
Pro Thr Ile Thr Ala Gln Cys Ala Asp Asp Ala Ala Ser Asp Gln Ile
            500                 505                 510

tca gtt tgt gtc gat ggg gaa aac gaa gaa cct gca caa att gtt cac   1584
Ser Val Cys Val Asp Gly Glu Asn Glu Glu Pro Ala Gln Ile Val His
        515                 520                 525

cac acc tgg acg acg atg aca cct caa gaa att tcc atg tgg att gac   1632
His Thr Trp Thr Thr Met Thr Pro Gln Glu Ile Ser Met Trp Ile Asp
    530                 535                 540

aaa agg tcc aga att tgt ttc ccg ata gct ttt gct ata ttt aac ttt   1680
Lys Arg Ser Arg Ile Cys Phe Pro Ile Ala Phe Ala Ile Phe Asn Phe
545                 550                 555                 560

ttt tat tgg ata ttt gtt tat tat tta taaacacact taatatactt         1727
Phe Tyr Trp Ile Phe Val Tyr Tyr Leu
                565

atagttttaa taattaataa atttataaaa taattaaaaa taaatatatg taaaatttaa 1787

aggaaacgtg aatagaatca aaagagattc ttattggatt attccattat taataggatt 1847

cttactagac aatattaatg attttatatt atatatcact tataactttt gaacggtttg 1907

ttaaaaatga atacaatatt tgacaaattt atataaaatt aaacaattta taatattgtc 1967

gaacatctta ccaccctaca gcgactcagt atactcgaaa atcgctattg aaatatctta 2027

cacaatttag tcattcctat ttcacatata atagttaata attaaaattg aaattttaaa 2087

ttaaaaaata atgatactgg aaattttaat tttaattatt aattattata tgaataatta 2147

attttactgc atagttataa ttataattat aaatattaaa tttttagaat aaatactcag 2207

ctggtctgaa aaaaaaaaaa aaaaaaaaaa aaa                              2240
```

<210> 20
<211> 569
<212> PRT
<213> Ctenocephalides felis

<400> 20

```
Thr Ile Leu Asn Ala Ser Thr Thr Val Glu Lys Asn Pro Gly His Gln
1               5                   10                  15
```

Thr Ser Ile Ser Glu Glu Ser Thr Thr Lys Leu Val Lys Thr Thr Thr
20 25 30

Glu Asp Asn His Leu Gly Val Lys Ser Leu Asn Glu Pro Gly Asp Glu
35 40 45

Gln Glu Leu Lys Lys Pro Ser Ser His Gly Lys Glu His Ile Ser Leu
50 55 60

Pro Val Ala Ser Pro Val Pro Pro Val Ser His Ile Phe Gln Ala Thr
65 70 75 80

Pro Gly Asp Leu Cys Pro Ala Phe Asp Asp Ala Asp Arg Phe Thr Gln
85 90 95

Thr Glu Leu Leu Ser Arg Leu Thr Asn Asp Cys Arg Tyr Asp Lys Leu
100 105 110

Glu Arg Pro Leu Gly Pro His Asn Gly Ala Gly Pro Leu Pro Val Ala
115 120 125

Ala Arg Ile Tyr Val Tyr Phe Ile Gln Asn Thr Asp Ala His Glu Leu
130 135 140

Ser Phe Ser Val Thr Val Leu Leu Gln Phe Arg Tyr Pro Gly Arg Gln
145 150 155 160

Ile Gly Leu Gln Lys Ser Gly Thr His Pro Gly Arg Ser Ser Trp Ala
165 170 175

Asn Arg Ser Ser Gly Thr Lys Ser Gly Tyr Pro His Val Phe Val Ala
180 185 190

Asn Glu Arg Ser Ser Gln Val Met Gly Thr Asp Ala Gln Ser Lys Asp
195 200 205

Met Leu Val Ser Val Ala Pro Asp Gly Thr Val Val Phe Ser Val Arg
210 215 220

Met Lys Ala Thr Leu Tyr Cys Trp Met Asn Leu Arg Lys Phe Pro Phe
225 230 235 240

Asp Glu Gln Gln Cys Gln Met Met Leu Glu Ser Trp Lys Tyr Asn Thr
245 250 255

Ser Glu Leu Leu Leu Thr Trp Glu Pro Thr Ala Pro Val Thr Leu Ala
260 265 270

```
Pro Glu Leu His Leu Thr Glu Tyr Val Leu Thr Asp Met Trp Val Asn
        275                 280                 285

Glu Thr Val Val Lys Ala Asp Leu Asp Asp Leu Arg His Gly Ala Phe
    290                 295                 300

Gly Gly Thr Tyr Ser Ala Leu Ser Phe Thr Ile Gln Ile Ser Arg Glu
305                 310                 315                 320

Met Gly Tyr Tyr Leu Met Asp Tyr Phe Leu Pro Ser Val Met Ile Val
                325                 330                 335

Ser Cys Ser Trp Val Ser Phe Trp Leu Ala Ala Asp Gln Ser Ala Pro
            340                 345                 350

Arg Val Thr Leu Gly Thr Ser Thr Met Leu Ser Phe Ile Thr Leu Ala
        355                 360                 365

Ser Ala Gln Gly Lys Thr Leu Pro Lys Val Ser Tyr Ile Lys Ala Ser
    370                 375                 380

Glu Ile Trp Phe Leu Gly Cys Thr Gly Phe Ile Phe Gly Ser Leu Val
385                 390                 395                 400

Glu Phe Ala Phe Val Asn Thr Ile Trp Arg Arg Arg Lys Asn Val Glu
                405                 410                 415

Leu Lys Lys Val Asn Ser Lys Tyr Ile Leu Lys Ser Thr Leu Thr Pro
            420                 425                 430

Arg Leu Ala Arg Lys Glu Phe His Ala Ser Phe Asn Ser Asn Pro Gly
        435                 440                 445

Gly Gly Asn Lys Asp Asp Gln Asp Leu Gly Arg Gly Ile Arg Val Phe
    450                 455                 460

Pro Pro Pro Leu Val Lys Ala Arg Ser Cys Ser Ser Leu Asp Arg Ser
465                 470                 475                 480

Asn Gly Ser Gly Asn Phe Leu Ser Val His Gly Asn Asp His Lys Val
                485                 490                 495

Pro Thr Ile Thr Ala Gln Cys Ala Asp Asp Ala Ala Ser Asp Gln Ile
            500                 505                 510

Ser Val Cys Val Asp Gly Glu Asn Glu Glu Pro Ala Gln Ile Val His
        515                 520                 525
```

His Thr Trp Thr Thr Met Thr Pro Gln Glu Ile Ser Met Trp Ile Asp
    530                 535                 540

Lys Arg Ser Arg Ile Cys Phe Pro Ile Ala Phe Ala Ile Phe Asn Phe
545                 550                 555                 560

Phe Tyr Trp Ile Phe Val Tyr Tyr Leu
                565

<210> 21
<211> 2240
<212> DNA
<213> Ctenocephalides felis

<400> 21

tttttttttt tttttttttt tttttcagac cagctgagta tttattctaa aaatttaata 60
tttataatta taattataac tatgcagtaa aattaattat tcatataata attaataatt 120
aaaattaaaa tttccagtat cattattttt taatttaaaa tttcaatttt aattattaac 180
tattatatgt gaaataggaa tgactaaatt gtgtaagata tttcaatagc gattttcgag 240
tatactgagt cgctgtaggg tggtaagatg ttcgacaata ttataaattg tttaatttta 300
tataaatttg tcaaatattg tattcatttt taacaaaccg ttcaaaagtt ataagtgata 360
tataatataa aatcattaat attgtctagt aagaatccta ttaataatgg aataatccaa 420
taagaatctc ttttgattct attcacgttt cctttaaatt ttacatatat ttatttttaa 480
ttattttata aatttattaa ttattaaaac tataagtata ttaagtgtgt ttataaataa 540
taaacaaata tccaataaaa aaagttaaat atagcaaaag ctatcgggaa acaaattctg 600
gacctttgt caatccacat ggaaatttct tgaggtgtca tcgtcgtcca ggtgtggtga 660
acaatttgtg caggttcttc gttttcccca tcgacacaaa ctgaaatctg gtcacttgcg 720
gcatcgtctg cacattgtgc tgttattgtt ggaactttgt gatcatttcc atggacgctc 780
aaaaaattcc cggatccatt actcctatcc agactggaac aagacctagc cttgaccaaa 840
ggcggcggaa agaccctaat ccctcttccc aaatcctgat catccttatt accacctcca 900
ggattcgaat taaacgaagc atgaaactcc ttccgggcca acctcggcgt caaagttgac 960
ttcaaaatat acttgctgtt gactttttc aattccacat ttttccttcg tctccaaatt 1020
gtgttgacaa acgcgaattc cactaaactc ccaaaaataa acccggtgca acctaaaaac 1080
cagatttctg aagctttgat gtacgatact ttgggtaaag tttttccttg ggcacttgct 1140
aaagtgataa atgataacat ggtgcttgta cctaaggtga ctctgggtgc tgattggtct 1200
gctgccagcc aaaaacttac ccaggaacac gacacgatca ttactgatgg caaaaagtaa 1260
tccattaaat agtaacccat ttcacgactt atttgaatcg tgaaacttaa ggcactgtat 1320
gtcccaccaa atgctccgtg tctcaggtca tccaaatcag ccttgacaac tgtttcattt 1380
acccacatgt cagtaaggac atattcggtc aaatgtagtt ctggtgctaa agttactggt 1440
gcagttggtt cccaagtcaa taggagttca cttgtattgt acttccaact ttccaacatc 1500
atctgacact gttgttcatc aaaaggaaat ttccttaaat tcatccaaca gtacaaagtt 1560
gccttcatcc tgaccgaaaa gacgactgta ccatcaggag ctactgacac caacatgtcc 1620
ttagattggg catctgtgcc cataacctgg gaagatctct cgttggcaac gaatacatgt 1680
gggtacccag attttgtccc tgagctgcga ttcgcccatg atgaccgtcc tgggtgggtg 1740
ccactttttt gtaggccaat ctggcgtcct gggtaacgaa attggaggag gacggtcacg 1800

```
gaaaatgaca attcgtgcgc gtccgtattt tgtataaaat acacgtaaat tctggcggcc 1860
accgggagcg gccctgcacc attgtgaggc cccaaagggc gctccagctt atcgtacctg 1920
caatcgtttg tcagcctgga caaaagttct gtctgggtga agcgatctgc atcgtcgaag 1980
gctggacaaa ggtctcctgg tgtagcctgg aagatatgcg atactggtgg tactggtgaa 2040
gccactggta aagaaatatg ctccttacca tgtgatgatg gttttttaa ttcttgttca 2100
tcaccaggtt cattcaggct ctttacaccg aggtggttgt cttcagtggt tgtttttacc 2160
aattttgtgg tagattcttc tgaaatactg gtttgatgtc caggattttt ttcaacggtc 2220
gtggatgcgt ttaaaattgt                                             2240
```

<210> 22
<211> 1707
<212> DNA
<213> Ctenocephalides felis

<220>
<221> CDS
<222> (1)..(1707)

<400> 22

```
aca att tta aac gca tcc acg acc gtt gaa aaa aat cct gga cat caa     48
Thr Ile Leu Asn Ala Ser Thr Thr Val Glu Lys Asn Pro Gly His Gln
1               5                   10                  15

acc agt att tca gaa gaa tct acc aca aaa ttg gta aaa aca acc act     96
Thr Ser Ile Ser Glu Glu Ser Thr Thr Lys Leu Val Lys Thr Thr Thr
            20                  25                  30

gaa gac aac cac ctc ggt gta aag agc ctg aat gaa cct ggt gat gaa     144
Glu Asp Asn His Leu Gly Val Lys Ser Leu Asn Glu Pro Gly Asp Glu
        35                  40                  45

caa gaa tta aaa aaa cca tca tca cat ggt aag gag cat att tct tta     192
Gln Glu Leu Lys Lys Pro Ser Ser His Gly Lys Glu His Ile Ser Leu
    50                  55                  60

cca gtg gct tca cca gta cca cca gta tcg cat atc ttc cag gct aca     240
Pro Val Ala Ser Pro Val Pro Pro Val Ser His Ile Phe Gln Ala Thr
65                  70                  75                  80

cca gga gac ctt tgt cca gcc ttc gac gat gca gat cgc ttc acc cag     288
Pro Gly Asp Leu Cys Pro Ala Phe Asp Asp Ala Asp Arg Phe Thr Gln
                85                  90                  95

aca gaa ctt ttg tcc agg ctg aca aac gat tgc agg tac gat aag ctg     336
Thr Glu Leu Leu Ser Arg Leu Thr Asn Asp Cys Arg Tyr Asp Lys Leu
            100                 105                 110
```

```
gag cgc cct ttg ggg cct cac aat ggt gca ggg ccg ctc ccg gtg gcc    384
Glu Arg Pro Leu Gly Pro His Asn Gly Ala Gly Pro Leu Pro Val Ala
        115                 120                 125

gcc aga att tac gtg tat ttt ata caa aat acg gac gcg cac gaa ttg    432
Ala Arg Ile Tyr Val Tyr Phe Ile Gln Asn Thr Asp Ala His Glu Leu
    130                 135                 140

tca ttt tcc gtg acc gtc ctc ctc caa ttt cgt tac cca gga cgc cag    480
Ser Phe Ser Val Thr Val Leu Leu Gln Phe Arg Tyr Pro Gly Arg Gln
145                 150                 155                 160

att ggc cta caa aaa agt ggc acc cac cca gga cgg tca tca tgg gcg    528
Ile Gly Leu Gln Lys Ser Gly Thr His Pro Gly Arg Ser Ser Trp Ala
                165                 170                 175

aat cgc agc tca ggg aca aaa tct ggg tac cca cat gta ttc gtt gcc    576
Asn Arg Ser Ser Gly Thr Lys Ser Gly Tyr Pro His Val Phe Val Ala
            180                 185                 190

aac gag aga tct tcc cag gtt atg ggc aca gat gcc caa tct aag gac    624
Asn Glu Arg Ser Ser Gln Val Met Gly Thr Asp Ala Gln Ser Lys Asp
        195                 200                 205

atg ttg gtg tca gta gct cct gat ggt aca gtc gtc ttt tcg gtc agg    672
Met Leu Val Ser Val Ala Pro Asp Gly Thr Val Val Phe Ser Val Arg
    210                 215                 220

atg aag gca act ttg tac tgt tgg atg aat tta agg aaa ttt cct ttt    720
Met Lys Ala Thr Leu Tyr Cys Trp Met Asn Leu Arg Lys Phe Pro Phe
225                 230                 235                 240

gat gaa caa cag tgt cag atg atg ttg gaa agt tgg aag tac aat aca    768
Asp Glu Gln Gln Cys Gln Met Met Leu Glu Ser Trp Lys Tyr Asn Thr
                245                 250                 255

agt gaa ctc cta ttg act tgg gaa cca act gca cca gta act tta gca    816
Ser Glu Leu Leu Leu Thr Trp Glu Pro Thr Ala Pro Val Thr Leu Ala
            260                 265                 270

cca gaa cta cat ttg acc gaa tat gtc ctt act gac atg tgg gta aat    864
Pro Glu Leu His Leu Thr Glu Tyr Val Leu Thr Asp Met Trp Val Asn
        275                 280                 285

gaa aca gtt gtc aag gct gat ttg gat gac ctg aga cac gga gca ttt    912
Glu Thr Val Val Lys Ala Asp Leu Asp Asp Leu Arg His Gly Ala Phe
    290                 295                 300
```

```
ggt ggg aca tac agt gcc tta agt ttc acg att caa ata agt cgt gaa    960
Gly Gly Thr Tyr Ser Ala Leu Ser Phe Thr Ile Gln Ile Ser Arg Glu
305                 310                 315                 320

atg ggt tac tat tta atg gat tac ttt ttg cca tca gta atg atc gtg    1008
Met Gly Tyr Tyr Leu Met Asp Tyr Phe Leu Pro Ser Val Met Ile Val
                325                 330                 335

tcg tgt tcc tgg gta agt ttt tgg ctg gca gca gac caa tca gca ccc    1056
Ser Cys Ser Trp Val Ser Phe Trp Leu Ala Ala Asp Gln Ser Ala Pro
            340                 345                 350

aga gtc acc tta ggt aca agc acc atg tta tca ttt atc act tta gca    1104
Arg Val Thr Leu Gly Thr Ser Thr Met Leu Ser Phe Ile Thr Leu Ala
        355                 360                 365

agt gcc caa gga aaa act tta ccc aaa gta tcg tac atc aaa gct tca    1152
Ser Ala Gln Gly Lys Thr Leu Pro Lys Val Ser Tyr Ile Lys Ala Ser
    370                 375                 380

gaa atc tgg ttt tta ggt tgc acc ggg ttt att ttt ggg agt tta gtg    1200
Glu Ile Trp Phe Leu Gly Cys Thr Gly Phe Ile Phe Gly Ser Leu Val
385                 390                 395                 400

gaa ttc gcg ttt gtc aac aca att tgg aga cga agg aaa aat gtg gaa    1248
Glu Phe Ala Phe Val Asn Thr Ile Trp Arg Arg Arg Lys Asn Val Glu
                405                 410                 415

ttg aaa aaa gtc aac agc aag tat att ttg aag tca act ttg acg ccg    1296
Leu Lys Lys Val Asn Ser Lys Tyr Ile Leu Lys Ser Thr Leu Thr Pro
            420                 425                 430

agg ttg gcc cgg aag gag ttt cat gct tcg ttt aat tcg aat cct gga    1344
Arg Leu Ala Arg Lys Glu Phe His Ala Ser Phe Asn Ser Asn Pro Gly
        435                 440                 445

ggt ggt aat aag gat gat cag gat ttg gga aga ggg att agg gtc ttt    1392
Gly Gly Asn Lys Asp Asp Gln Asp Leu Gly Arg Gly Ile Arg Val Phe
    450                 455                 460

ccg ccg cct ttg gtc aag gct agg tct tgt tcc agt ctg gat agg agt    1440
Pro Pro Pro Leu Val Lys Ala Arg Ser Cys Ser Ser Leu Asp Arg Ser
465                 470                 475                 480

aat gga tcc ggg aat ttt ttg agc gtc cat gga aat gat cac aaa gtt    1488
Asn Gly Ser Gly Asn Phe Leu Ser Val His Gly Asn Asp His Lys Val
                485                 490                 495
```

```
cca aca ata aca gca caa tgt gca gac gat gcc gca agt gac cag att   1536
Pro Thr Ile Thr Ala Gln Cys Ala Asp Asp Ala Ala Ser Asp Gln Ile
            500                 505                 510

tca gtt tgt gtc gat ggg gaa aac gaa gaa cct gca caa att gtt cac   1584
Ser Val Cys Val Asp Gly Glu Asn Glu Glu Pro Ala Gln Ile Val His
        515                 520                 525

cac acc tgg acg acg atg aca cct caa gaa att tcc atg tgg att gac   1632
His Thr Trp Thr Thr Met Thr Pro Gln Glu Ile Ser Met Trp Ile Asp
    530                 535                 540

aaa agg tcc aga att tgt ttc ccg ata gct ttt gct ata ttt aac ttt   1680
Lys Arg Ser Arg Ile Cys Phe Pro Ile Ala Phe Ala Ile Phe Asn Phe
545                 550                 555                 560

ttt tat tgg ata ttt gtt tat tat tta                               1707
Phe Tyr Trp Ile Phe Val Tyr Tyr Leu
                565
```

<210> 23
<211> 569
<212> PRT
<213> Ctenocephalides felis

<400> 23

```
Thr Ile Leu Asn Ala Ser Thr Thr Val Glu Lys Asn Pro Gly His Gln
1               5                   10                  15

Thr Ser Ile Ser Glu Glu Ser Thr Thr Lys Leu Val Lys Thr Thr Thr
            20                  25                  30

Glu Asp Asn His Leu Gly Val Lys Ser Leu Asn Glu Pro Gly Asp Glu
        35                  40                  45

Gln Glu Leu Lys Lys Pro Ser Ser His Gly Lys Glu His Ile Ser Leu
    50                  55                  60

Pro Val Ala Ser Pro Val Pro Pro Val Ser His Ile Phe Gln Ala Thr
65                  70                  75                  80

Pro Gly Asp Leu Cys Pro Ala Phe Asp Asp Ala Asp Arg Phe Thr Gln
                85                  90                  95

Thr Glu Leu Leu Ser Arg Leu Thr Asn Asp Cys Arg Tyr Asp Lys Leu
            100                 105                 110
```

Glu Arg Pro Leu Gly Pro His Asn Gly Ala Gly Pro Leu Pro Val Ala
115 120 125

Ala Arg Ile Tyr Val Tyr Phe Ile Gln Asn Thr Asp Ala His Glu Leu
130 135 140

Ser Phe Ser Val Thr Val Leu Leu Gln Phe Arg Tyr Pro Gly Arg Gln
145 150 155 160

Ile Gly Leu Gln Lys Ser Gly Thr His Pro Gly Arg Ser Ser Trp Ala
165 170 175

Asn Arg Ser Ser Gly Thr Lys Ser Gly Tyr Pro His Val Phe Val Ala
180 185 190

Asn Glu Arg Ser Ser Gln Val Met Gly Thr Asp Ala Gln Ser Lys Asp
195 200 205

Met Leu Val Ser Val Ala Pro Asp Gly Thr Val Val Phe Ser Val Arg
210 215 220

Met Lys Ala Thr Leu Tyr Cys Trp Met Asn Leu Arg Lys Phe Pro Phe
225 230 235 240

Asp Glu Gln Gln Cys Gln Met Met Leu Glu Ser Trp Lys Tyr Asn Thr
245 250 255

Ser Glu Leu Leu Leu Thr Trp Glu Pro Thr Ala Pro Val Thr Leu Ala
260 265 270

Pro Glu Leu His Leu Thr Glu Tyr Val Leu Thr Asp Met Trp Val Asn
275 280 285

Glu Thr Val Val Lys Ala Asp Leu Asp Asp Leu Arg His Gly Ala Phe
290 295 300

Gly Gly Thr Tyr Ser Ala Leu Ser Phe Thr Ile Gln Ile Ser Arg Glu
305 310 315 320

Met Gly Tyr Tyr Leu Met Asp Tyr Phe Leu Pro Ser Val Met Ile Val
325 330 335

Ser Cys Ser Trp Val Ser Phe Trp Leu Ala Ala Asp Gln Ser Ala Pro
340 345 350

Arg Val Thr Leu Gly Thr Ser Thr Met Leu Ser Phe Ile Thr Leu Ala
355 360 365

Ser Ala Gln Gly Lys Thr Leu Pro Lys Val Ser Tyr Ile Lys Ala Ser
370 375 380

Glu Ile Trp Phe Leu Gly Cys Thr Gly Phe Ile Phe Gly Ser Leu Val
385 390 395 400

Glu Phe Ala Phe Val Asn Thr Ile Trp Arg Arg Arg Lys Asn Val Glu
405 410 415

Leu Lys Lys Val Asn Ser Lys Tyr Ile Leu Lys Ser Thr Leu Thr Pro
420 425 430

Arg Leu Ala Arg Lys Glu Phe His Ala Ser Phe Asn Ser Asn Pro Gly
435 440 445

Gly Gly Asn Lys Asp Asp Gln Asp Leu Gly Arg Gly Ile Arg Val Phe
450 455 460

Pro Pro Pro Leu Val Lys Ala Arg Ser Cys Ser Ser Leu Asp Arg Ser
465 470 475 480

Asn Gly Ser Gly Asn Phe Leu Ser Val His Gly Asn Asp His Lys Val
485 490 495

Pro Thr Ile Thr Ala Gln Cys Ala Asp Asp Ala Ala Ser Asp Gln Ile
500 505 510

Ser Val Cys Val Asp Gly Glu Asn Glu Glu Pro Ala Gln Ile Val His
515 520 525

His Thr Trp Thr Thr Met Thr Pro Gln Glu Ile Ser Met Trp Ile Asp
530 535 540

Lys Arg Ser Arg Ile Cys Phe Pro Ile Ala Phe Ala Ile Phe Asn Phe
545 550 555 560

Phe Tyr Trp Ile Phe Val Tyr Tyr Leu
565

<210> 24
<211> 1707
<212> DNA
<213> Ctenocephalides felis

<400> 24

taaataataa acaaatatcc aataaaaaaa gttaaatata gcaaaagcta tcgggaaaca 60

```
aattctggac cttttgtcaa tccacatgga aatttcttga ggtgtcatcg tcgtccaggt 120
gtggtgaaca atttgtgcag gttcttcgtt ttccccatcg acacaaactg aaatctggtc 180
acttgcggca tcgtctgcac attgtgctgt tattgttgga actttgtgat catttccatg 240
gacgctcaaa aaattcccgg atccattact cctatccaga ctggaacaag acctagcctt 300
gaccaaaggc ggcggaaaga ccctaatccc tcttcccaaa tcctgatcat ccttattacc 360
acctccagga ttcgaattaa acgaagcatg aaactccttc cgggccaacc tcggcgtcaa 420
agttgacttc aaaatatact tgctgttgac tttttcaat tccacatttt tccttcgtct 480
ccaaattgtg ttgacaaacg cgaattccac taaactccca aaaataaacc cggtgcaacc 540
taaaaaccag atttctgaag ctttgatgta cgatactttg ggtaaagttt ttccttgggc 600
acttgctaaa gtgataaatg ataacatggt gcttgtacct aaggtgactc tgggtgctga 660
ttggtctgct gccagccaaa aacttaccca ggaacacgac acgatcatta ctgatggcaa 720
aaagtaatcc attaaatagt aacccatttc acgacttatt tgaatcgtga aacttaaggc 780
actgtatgtc ccaccaaatg ctccgtgtct caggtcatcc aaatcagcct tgacaactgt 840
ttcatttacc cacatgtcag taaggacata ttcggtcaaa tgtagttctg gtgctaaagt 900
tactggtgca gttggttccc aagtcaatag gagttcactt gtattgtact tccaactttc 960
caacatcatc tgacactgtt gttcatcaaa aggaaatttc cttaaattca tccaacagta 1020
caaagttgcc ttcatcctga ccgaaaagac gactgtacca tcaggagcta ctgacaccaa 1080
catgtcctta gattgggcat ctgtgcccat aacctgggaa gatctctcgt tggcaacgaa 1140
tacatgtggg tacccagatt ttgtccctga gctgcgattc gcccatgatg accgtcctgg 1200
gtgggtgcca ctttttgta ggccaatctg gcgtcctggg taacgaaatt ggaggaggac 1260
ggtcacggaa aatgacaatt cgtgcgcgtc cgtattttgt ataaaataca cgtaaattct 1320
ggcggccacc gggagcggcc ctgcaccatt gtgaggcccc aaagggcgct ccagcttatc 1380
gtacctgcaa tcgtttgtca gcctggacaa aagttctgtc tgggtgaagc gatctgcatc 1440
gtcgaaggct ggacaaaggt ctcctggtgt agcctggaag atatgcgata ctggtggtac 1500
tggtgaagcc actggtaaag aaatatgctc cttaccatgt gatgatggtt tttttaattc 1560
ttgttcatca ccaggttcat tcaggctctt tacaccgagg tggttgtctt cagtggttgt 1620
ttttaccaat tttgtggtag attcttctga aatactggtt tgatgtccag gattttttc 1680
aacggtcgtg gatgcgttta aaattgt                                     1707
```

<210> 25
<211> 1429
<212> DNA
<213> Ctenocephalides felis

<220>
<221> CDS
<222> (18)..(1211)

<400> 25

```
gtttttgtgt gagcgtg atg tct ccg gct ctt tta gcg gtt att gct gtg    50
                   Met Ser Pro Ala Leu Leu Ala Val Ile Ala Val
                   1               5                   10

att ttg tgc att tta ttt aga att tta aat gta aat aca caa ccg gga   98
Ile Leu Cys Ile Leu Phe Arg Ile Leu Asn Val Asn Thr Gln Pro Gly
        15                  20                  25
```

```
acg cct aaa ata tgg tgc aaa gat gag acg ttt ctt gag gcc att tac     146
Thr Pro Lys Ile Trp Cys Lys Asp Glu Thr Phe Leu Glu Ala Ile Tyr
                30                  35                  40

aaa att gct ccg ctt tta cga gag cct tat gtg cct cct aga ctc tgg     194
Lys Ile Ala Pro Leu Leu Arg Glu Pro Tyr Val Pro Pro Arg Leu Trp
            45                  50                  55

gga ttc agc ggg cac gtg cag acg atc gtg cac agc ata gtg ggc cgc     242
Gly Phe Ser Gly His Val Gln Thr Ile Val His Ser Ile Val Gly Arg
 60                  65                  70                  75

gtc aag tgc cca ctg cca ctc gga gag agg gtg tac ctg tca ctg gct     290
Val Lys Cys Pro Leu Pro Leu Gly Glu Arg Val Tyr Leu Ser Leu Ala
                    80                  85                  90

gat ggg tcg acg ctc act tac gat cta tac aaa gct ctt aat ccg gat     338
Asp Gly Ser Thr Leu Thr Tyr Asp Leu Tyr Lys Ala Leu Asn Pro Asp
                95                  100                 105

aaa cat gaa gat gag gta act ctg gca gtg tgc cct ggc ata agt aac     386
Lys His Glu Asp Glu Val Thr Leu Ala Val Cys Pro Gly Ile Ser Asn
            110                 115                 120

tcc tcg gag tcg gtc tac att cgc aca ttt gtc cat tac gca caa tat     434
Ser Ser Glu Ser Val Tyr Ile Arg Thr Phe Val His Tyr Ala Gln Tyr
        125                 130                 135

tac gga tac aga tgt gcc gta ctt aat cat att ggt gcc tta tct gga     482
Tyr Gly Tyr Arg Cys Ala Val Leu Asn His Ile Gly Ala Leu Ser Gly
140                 145                 150                 155

gtg cct gtc act aac tct aga aat ttc agt tat ggt cat acc gat gat     530
Val Pro Val Thr Asn Ser Arg Asn Phe Ser Tyr Gly His Thr Asp Asp
                    160                 165                 170

tat aat gaa atg att cga cat ctg caa tca cag ttt cct cct tct aaa     578
Tyr Asn Glu Met Ile Arg His Leu Gln Ser Gln Phe Pro Pro Ser Lys
                175                 180                 185

ata att tgt gtg ggc tac agt tta aga ggc aat atc atc acc aaa tat     626
Ile Ile Cys Val Gly Tyr Ser Leu Arg Gly Asn Ile Ile Thr Lys Tyr
            190                 195                 200

ctt ggt gaa aag aca aaa att aaa aat ggt aat ata att gga gga att     674
Leu Gly Glu Lys Thr Lys Ile Lys Asn Gly Asn Ile Ile Gly Gly Ile
        205                 210                 215
```

```
tca ata tgc caa gga tac aac gcc att gag ggt acg aaa tgg cta ctg    722
Ser Ile Cys Gln Gly Tyr Asn Ala Ile Glu Gly Thr Lys Trp Leu Leu
220                 225                 230                 235

aat tgg caa aat ttc cgt cgt ttc tac ttg tat gtt tta aca gaa agt    770
Asn Trp Gln Asn Phe Arg Arg Phe Tyr Leu Tyr Val Leu Thr Glu Ser
                240                 245                 250

gta aag aca ata att ttg aaa cac aga cat att ctc ctg tcc gat gaa    818
Val Lys Thr Ile Ile Leu Lys His Arg His Ile Leu Leu Ser Asp Glu
            255                 260                 265

atg aaa tta aaa tgc caa ttg aat gag aga gat ata gca tcg gca gcc    866
Met Lys Leu Lys Cys Gln Leu Asn Glu Arg Asp Ile Ala Ser Ala Ala
        270                 275                 280

act ttg ccg gaa ttg gat gac gcc tat acg aga aaa gtt cac aag ttt    914
Thr Leu Pro Glu Leu Asp Asp Ala Tyr Thr Arg Lys Val His Lys Phe
    285                 290                 295

cca tct gta aac gct ttg tac aaa tgg agt tcc tgc ata aac tac atc    962
Pro Ser Val Asn Ala Leu Tyr Lys Trp Ser Ser Cys Ile Asn Tyr Ile
300                 305                 310                 315

cag gac att gaa act cca atg gtg ttc ata aat gct aaa gat gat cct    1010
Gln Asp Ile Glu Thr Pro Met Val Phe Ile Asn Ala Lys Asp Asp Pro
                320                 325                 330

cta ctc cat gat acg ctt cta gac cct ata aga aaa att gct ggt tct    1058
Leu Leu His Asp Thr Leu Leu Asp Pro Ile Arg Lys Ile Ala Gly Ser
            335                 340                 345

tct aga aga atg atc tac gta gaa ctt tct cat gga ggt cat cca aga    1106
Ser Arg Arg Met Ile Tyr Val Glu Leu Ser His Gly Gly His Pro Arg
        350                 355                 360

ttc ttt gaa agg ggt ctc ata tac ccc aat ccc gtt acc tgg ata gat    1154
Phe Phe Glu Arg Gly Leu Ile Tyr Pro Asn Pro Val Thr Trp Ile Asp
    365                 370                 375

agg gca gtg ata agc ttg gtt ggn ggt ctc ctg ctt gca cat aat gaa    1202
Arg Ala Val Ile Ser Leu Val Xaa Gly Leu Leu Leu Ala His Asn Glu
380                 385                 390                 395

aag agc tat taaaccaatt tagatttata attattattt ataaaaattt            1251
Lys Ser Tyr

atgaaatatt tttttgttat aaattgtgga tttatttttt tatttgtgct gtcttttgca 1311
```

tcttgtgctc agttattcga tgttattgaa gttattttct aaatttatat atacgcggat 1371

gtgaagatca atatatgtca taaagttagg ggatttaggg gaaaaaaaaa aaaaaaaa 1429

<210> 26
<211> 398
<212> PRT
<213> Ctenocephalides felis

<400> 26

```
Met Ser Pro Ala Leu Leu Ala Val Ile Ala Val Ile Leu Cys Ile Leu
1               5                   10                  15

Phe Arg Ile Leu Asn Val Asn Thr Gln Pro Gly Thr Pro Lys Ile Trp
            20                  25                  30

Cys Lys Asp Glu Thr Phe Leu Glu Ala Ile Tyr Lys Ile Ala Pro Leu
        35                  40                  45

Leu Arg Glu Pro Tyr Val Pro Pro Arg Leu Trp Gly Phe Ser Gly His
    50                  55                  60

Val Gln Thr Ile Val His Ser Ile Val Gly Arg Val Lys Cys Pro Leu
65                  70                  75                  80

Pro Leu Gly Glu Arg Val Tyr Leu Ser Leu Ala Asp Gly Ser Thr Leu
                85                  90                  95

Thr Tyr Asp Leu Tyr Lys Ala Leu Asn Pro Asp Lys His Glu Asp Glu
            100                 105                 110

Val Thr Leu Ala Val Cys Pro Gly Ile Ser Asn Ser Ser Glu Ser Val
        115                 120                 125

Tyr Ile Arg Thr Phe Val His Tyr Ala Gln Tyr Tyr Gly Tyr Arg Cys
    130                 135                 140

Ala Val Leu Asn His Ile Gly Ala Leu Ser Gly Val Pro Val Thr Asn
145                 150                 155                 160

Ser Arg Asn Phe Ser Tyr Gly His Thr Asp Asp Tyr Asn Glu Met Ile
                165                 170                 175

Arg His Leu Gln Ser Gln Phe Pro Pro Ser Lys Ile Ile Cys Val Gly
            180                 185                 190
```

```
Tyr Ser Leu Arg Gly Asn Ile Ile Thr Lys Tyr Leu Gly Glu Lys Thr
        195                 200                 205

Lys Ile Lys Asn Gly Asn Ile Ile Gly Gly Ile Ser Ile Cys Gln Gly
    210                 215                 220

Tyr Asn Ala Ile Glu Gly Thr Lys Trp Leu Leu Asn Trp Gln Asn Phe
225                 230                 235                 240

Arg Arg Phe Tyr Leu Tyr Val Leu Thr Glu Ser Val Lys Thr Ile Ile
                245                 250                 255

Leu Lys His Arg His Ile Leu Leu Ser Asp Glu Met Lys Leu Lys Cys
            260                 265                 270

Gln Leu Asn Glu Arg Asp Ile Ala Ser Ala Ala Thr Leu Pro Glu Leu
        275                 280                 285

Asp Asp Ala Tyr Thr Arg Lys Val His Lys Phe Pro Ser Val Asn Ala
    290                 295                 300

Leu Tyr Lys Trp Ser Ser Cys Ile Asn Tyr Ile Gln Asp Ile Glu Thr
305                 310                 315                 320

Pro Met Val Phe Ile Asn Ala Lys Asp Asp Pro Leu Leu His Asp Thr
                325                 330                 335

Leu Leu Asp Pro Ile Arg Lys Ile Ala Gly Ser Ser Arg Arg Met Ile
            340                 345                 350

Tyr Val Glu Leu Ser His Gly Gly His Pro Arg Phe Phe Glu Arg Gly
        355                 360                 365

Leu Ile Tyr Pro Asn Pro Val Thr Trp Ile Asp Arg Ala Val Ile Ser
    370                 375                 380

Leu Val Xaa Gly Leu Leu Leu Ala His Asn Glu Lys Ser Tyr
385                 390                 395
```

<210> 27
<211> 1429
<212> DNA
<213> Ctenocephalides felis

<400> 27

```
ttttttttt tttttttccc ctaaatcccc taactttatg acatatattg atcttcacat 60
```

```
ccgcgtatat ataaatttag aaaataactt caataacatc gaataactga gcacaagatg 120
caaaagacag cacaaataaa aaaataaatc cacaatttat aacaaaaaaa tatttcataa 180
atttttataa ataataatta taaatctaaa ttggtttaat agctcttttc attatgtgca 240
agcaggagac cnccaaccaa gcttatcact gccctatcta tccaggtaac gggattgggg 300
tatatgagac ccctttcaaa gaatcttgga tgacctccat gagaaagttc tacgtagatc 360
attcttctag aagaaccagc aatttttctt atagggtcta gaagcgtatc atggagtaga 420
ggatcatctt tagcatttat gaacaccatt ggagtttcaa tgtcctggat gtagtttatg 480
caggaactcc atttgtacaa agcgtttaca gatggaaact tgtgaacttt tctcgtatag 540
gcgtcatcca attccggcaa agtggctgcc gatgctatat ctctctcatt caattggcat 600
tttaatttca tttcatcgga caggagaata tgtctgtgtt tcaaaattat tgtctttaca 660
ctttctgtta aaacatacaa gtagaaacga cggaaatttt gccaattcag tagccatttc 720
gtaccctcaa tggcgttgta tccttggcat attgaaattc ctccaattat attaccattt 780
ttaatttttg tcttttcacc aagatatttg gtgatgatat tgcctcttaa actgtagccc 840
acacaaatta ttttagaagg aggaaactgt gattgcagat gtcgaatcat ttcattataa 900
tcatcggtat gaccataact gaaatttcta gagttagtga caggcactcc agataaggca 960
ccaatatgat taagtacggc acatctgtat ccgtaatatt gtgcgtaatg gacaaatgtg 1020
cgaatgtaga ccgactccga ggagttactt atgccagggc acactgccag agttacctca 1080
tcttcatgtt tatccggatt aagagctttg tatagatcgt aagtgagcgt cgacccatca 1140
gccagtgaca ggtacaccct ctctccgagt ggcagtgggc acttgacgcg gcccactatg 1200
ctgtgcacga tcgtctgcac gtgcccgctg aatccccaga gtctaggagg cacataaggc 1260
tctcgtaaaa gcggagcaat tttgtaaatg gcctcaagaa acgtctcatc tttgcaccat 1320
attttaggcg ttcccggttg tgtatttaca tttaaaattc taaataaaat gcacaaaatc 1380
acagcaataa ccgctaaaag agccggagac atcacgctca cacaaaaac            1429
```

<210> 28
<211> 1194
<212> DNA
<213> Ctenocephalides felis

<220>
<221> CDS
<222> (1)..(1194)

<400> 28

```
atg tct ccg gct ctt tta gcg gtt att gct gtg att ttg tgc att tta    48
Met Ser Pro Ala Leu Leu Ala Val Ile Ala Val Ile Leu Cys Ile Leu
1               5                   10                  15

ttt aga att tta aat gta aat aca caa ccg gga acg cct aaa ata tgg    96
Phe Arg Ile Leu Asn Val Asn Thr Gln Pro Gly Thr Pro Lys Ile Trp
            20                  25                  30

tgc aaa gat gag acg ttt ctt gag gcc att tac aaa att gct ccg ctt   144
Cys Lys Asp Glu Thr Phe Leu Glu Ala Ile Tyr Lys Ile Ala Pro Leu
        35                  40                  45

tta cga gag cct tat gtg cct cct aga ctc tgg gga ttc agc ggg cac   192
```

```
Leu Arg Glu Pro Tyr Val Pro Pro Arg Leu Trp Gly Phe Ser Gly His
     50                  55                  60

gtg cag acg atc gtg cac agc ata gtg ggc cgc gtc aag tgc cca ctg    240
Val Gln Thr Ile Val His Ser Ile Val Gly Arg Val Lys Cys Pro Leu
 65                  70                  75                  80

cca ctc gga gag agg gtg tac ctg tca ctg gct gat ggg tcg acg ctc    288
Pro Leu Gly Glu Arg Val Tyr Leu Ser Leu Ala Asp Gly Ser Thr Leu
                 85                  90                  95

act tac gat cta tac aaa gct ctt aat ccg gat aaa cat gaa gat gag    336
Thr Tyr Asp Leu Tyr Lys Ala Leu Asn Pro Asp Lys His Glu Asp Glu
            100                 105                 110

gta act ctg gca gtg tgc cct ggc ata agt aac tcc tcg gag tcg gtc    384
Val Thr Leu Ala Val Cys Pro Gly Ile Ser Asn Ser Ser Glu Ser Val
        115                 120                 125

tac att cgc aca ttt gtc cat tac gca caa tat tac gga tac aga tgt    432
Tyr Ile Arg Thr Phe Val His Tyr Ala Gln Tyr Tyr Gly Tyr Arg Cys
    130                 135                 140

gcc gta ctt aat cat att ggt gcc tta tct gga gtg cct gtc act aac    480
Ala Val Leu Asn His Ile Gly Ala Leu Ser Gly Val Pro Val Thr Asn
145                 150                 155                 160

tct aga aat ttc agt tat ggt cat acc gat gat tat aat gaa atg att    528
Ser Arg Asn Phe Ser Tyr Gly His Thr Asp Asp Tyr Asn Glu Met Ile
                165                 170                 175

cga cat ctg caa tca cag ttt cct cct tct aaa ata att tgt gtg ggc    576
Arg His Leu Gln Ser Gln Phe Pro Pro Ser Lys Ile Ile Cys Val Gly
            180                 185                 190

tac agt tta aga ggc aat atc atc acc aaa tat ctt ggt gaa aag aca    624
Tyr Ser Leu Arg Gly Asn Ile Ile Thr Lys Tyr Leu Gly Glu Lys Thr
        195                 200                 205

aaa att aaa aat ggt aat ata att gga gga att tca ata tgc caa gga    672
Lys Ile Lys Asn Gly Asn Ile Ile Gly Gly Ile Ser Ile Cys Gln Gly
    210                 215                 220

tac aac gcc att gag ggt acg aaa tgg cta ctg aat tgg caa aat ttc    720
Tyr Asn Ala Ile Glu Gly Thr Lys Trp Leu Leu Asn Trp Gln Asn Phe
225                 230                 235                 240

cgt cgt ttc tac ttg tat gtt tta aca gaa agt gta aag aca ata att    768
```

```
Arg Arg Phe Tyr Leu Tyr Val Leu Thr Glu Ser Val Lys Thr Ile Ile
                245                 250                 255

ttg aaa cac aga cat att ctc ctg tcc gat gaa atg aaa tta aaa tgc      816
Leu Lys His Arg His Ile Leu Leu Ser Asp Glu Met Lys Leu Lys Cys
            260                 265                 270

caa ttg aat gag aga gat ata gca tcg gca gcc act ttg ccg gaa ttg      864
Gln Leu Asn Glu Arg Asp Ile Ala Ser Ala Ala Thr Leu Pro Glu Leu
        275                 280                 285

gat gac gcc tat acg aga aaa gtt cac aag ttt cca tct gta aac gct      912
Asp Asp Ala Tyr Thr Arg Lys Val His Lys Phe Pro Ser Val Asn Ala
    290                 295                 300

ttg tac aaa tgg agt tcc tgc ata aac tac atc cag gac att gaa act      960
Leu Tyr Lys Trp Ser Ser Cys Ile Asn Tyr Ile Gln Asp Ile Glu Thr
305                 310                 315                 320

cca atg gtg ttc ata aat gct aaa gat gat cct cta ctc cat gat acg      1008
Pro Met Val Phe Ile Asn Ala Lys Asp Asp Pro Leu Leu His Asp Thr
                325                 330                 335

ctt cta gac cct ata aga aaa att gct ggt tct tct aga aga atg atc      1056
Leu Leu Asp Pro Ile Arg Lys Ile Ala Gly Ser Ser Arg Arg Met Ile
            340                 345                 350

tac gta gaa ctt tct cat gga ggt cat cca aga ttc ttt gaa agg ggt      1104
Tyr Val Glu Leu Ser His Gly Gly His Pro Arg Phe Phe Glu Arg Gly
        355                 360                 365

ctc ata tac ccc aat ccc gtt acc tgg ata gat agg gca gtg ata agc      1152
Leu Ile Tyr Pro Asn Pro Val Thr Trp Ile Asp Arg Ala Val Ile Ser
    370                 375                 380

ttg gtt ggn ggt ctc ctg ctt gca cat aat gaa aag agc tat              1194
Leu Val Xaa Gly Leu Leu Leu Ala His Asn Glu Lys Ser Tyr
385                 390                 395
```

<210> 29
<211> 398
<212> PRT
<213> Ctenocephalides felis

<400> 29

```
Met Ser Pro Ala Leu Leu Ala Val Ile Ala Val Ile Leu Cys Ile Leu
 1               5                  10                  15
```

```
Phe Arg Ile Leu Asn Val Asn Thr Gln Pro Gly Thr Pro Lys Ile Trp
                20                  25                  30

Cys Lys Asp Glu Thr Phe Leu Glu Ala Ile Tyr Lys Ile Ala Pro Leu
            35                  40                  45

Leu Arg Glu Pro Tyr Val Pro Pro Arg Leu Trp Gly Phe Ser Gly His
    50                  55                  60

Val Gln Thr Ile Val His Ser Ile Val Gly Arg Val Lys Cys Pro Leu
65                  70                  75                  80

Pro Leu Gly Glu Arg Val Tyr Leu Ser Leu Ala Asp Gly Ser Thr Leu
                85                  90                  95

Thr Tyr Asp Leu Tyr Lys Ala Leu Asn Pro Asp Lys His Glu Asp Glu
            100                 105                 110

Val Thr Leu Ala Val Cys Pro Gly Ile Ser Asn Ser Ser Glu Ser Val
        115                 120                 125

Tyr Ile Arg Thr Phe Val His Tyr Ala Gln Tyr Tyr Gly Tyr Arg Cys
    130                 135                 140

Ala Val Leu Asn His Ile Gly Ala Leu Ser Gly Val Pro Val Thr Asn
145                 150                 155                 160

Ser Arg Asn Phe Ser Tyr Gly His Thr Asp Asp Tyr Asn Glu Met Ile
                165                 170                 175

Arg His Leu Gln Ser Gln Phe Pro Pro Ser Lys Ile Ile Cys Val Gly
            180                 185                 190

Tyr Ser Leu Arg Gly Asn Ile Ile Thr Lys Tyr Leu Gly Glu Lys Thr
        195                 200                 205

Lys Ile Lys Asn Gly Asn Ile Ile Gly Gly Ile Ser Ile Cys Gln Gly
    210                 215                 220

Tyr Asn Ala Ile Glu Gly Thr Lys Trp Leu Leu Asn Trp Gln Asn Phe
225                 230                 235                 240

Arg Arg Phe Tyr Leu Tyr Val Leu Thr Glu Ser Val Lys Thr Ile Ile
                245                 250                 255

Leu Lys His Arg His Ile Leu Leu Ser Asp Glu Met Lys Leu Lys Cys
            260                 265                 270
```

Gln Leu Asn Glu Arg Asp Ile Ala Ser Ala Ala Thr Leu Pro Glu Leu
275 280 285

Asp Asp Ala Tyr Thr Arg Lys Val His Lys Phe Pro Ser Val Asn Ala
290 295 300

Leu Tyr Lys Trp Ser Ser Cys Ile Asn Tyr Ile Gln Asp Ile Glu Thr
305 310 315 320

Pro Met Val Phe Ile Asn Ala Lys Asp Asp Pro Leu Leu His Asp Thr
325 330 335

Leu Leu Asp Pro Ile Arg Lys Ile Ala Gly Ser Ser Arg Arg Met Ile
340 345 350

Tyr Val Glu Leu Ser His Gly Gly His Pro Arg Phe Phe Glu Arg Gly
355 360 365

Leu Ile Tyr Pro Asn Pro Val Thr Trp Ile Asp Arg Ala Val Ile Ser
370 375 380

Leu Val Xaa Gly Leu Leu Leu Ala His Asn Glu Lys Ser Tyr
385 390 395

<210> 30
<211> 1194
<212> DNA
<213> Ctenocephalides felis

<400> 30

atagctcttt tcattatgtg caagcaggag accnccaacc aagcttatca ctgccctatc 60
tatccaggta acgggattgg ggtatatgag accccttca aagaatcttg gatgacctcc 120
atgagaaagt tctacgtaga tcattcttct agaagaacca gcaatttttc ttatagggtc 180
tagaagcgta tcatggagta gaggatcatc tttagcattt atgaacacca ttggagtttc 240
aatgtcctgg atgtagttta tgcaggaact ccatttgtac aaagcgttta cagatggaaa 300
cttgtgaact tttctcgtat aggcgtcatc caattccggc aaagtggctg ccgatgctat 360
atctctctca ttcaattggc attttaattt catttcatcg gacaggagaa tatgtctgtg 420
tttcaaaatt attgtcttta cactttctgt taaaacatac aagtagaaac gacggaaatt 480
ttgccaattc agtagccatt tcgtaccctc aatggcgttg tatccttggc atattgaaat 540
tcctccaatt atattaccat ttttaatttt tgtcttttca ccaagatatt tggtgatgat 600
attgcctctt aaactgtagc ccacacaaat tattttagaa ggaggaaact gtgattgcag 660
atgtcgaatc atttcattat aatcatcggt atgaccataa ctgaaatttc tagagttagt 720
gacaggcact ccagataagg caccaatatg attaagtacg gcacatctgt atccgtaata 780
ttgtgcgtaa tggacaaatg tgcgaatgta gaccgactcc gaggagttac ttatgccagg 840
gcacactgcc agagttacct catcttcatg tttatccgga ttaagagctt tgtatagatc 900

```
gtaagtgagc gtcgacccat cagccagtga caggtacacc ctctctccga gtggcagtgg 960
gcacttgacg cggcccacta tgctgtgcac gatcgtctgc acgtgcccgc tgaatcccca 1020
gagtctagga ggcacataag gctctcgtaa aagcggagca attttgtaaa tggcctcaag 1080
aaacgtctca tctttgcacc atattttagg cgttcccggt tgtgtattta catttaaaat 1140
tctaaataaa atgcacaaaa tcacagcaat aaccgctaaa agagccggag acat       1194
```

<210> 31
<211> 765
<212> DNA
<213> Ctenocephalides felis

<220>
<221> CDS
<222> (2)..(763)

<400> 31

```
t cac aat ttg tat ttg cat agt gcc ctt gta aag tcg aga gat gtc gac 49
  His Asn Leu Tyr Leu His Ser Ala Leu Val Lys Ser Arg Asp Val Asp
  1               5                   10                  15

agg aga aac ccg gag aaa gtg aga gac gct aat tat tat ttc ttt atc   97
Arg Arg Asn Pro Glu Lys Val Arg Asp Ala Asn Tyr Tyr Phe Phe Ile
            20                  25                  30

gaa gca gcg att gca cta ttt ata tct ttc ata ata aat gtg ttc gta   145
Glu Ala Ala Ile Ala Leu Phe Ile Ser Phe Ile Ile Asn Val Phe Val
        35                  40                  45

gtt gct gtg ttt gca cat ggt tta ttt aaa act act aac caa gaa atc   193
Val Ala Val Phe Ala His Gly Leu Phe Lys Thr Thr Asn Gln Glu Ile
    50                  55                  60

tta gac act tgc aaa agt tca gcc cca tat atc cga gag gaa gcc cta   241
Leu Asp Thr Cys Lys Ser Ser Ala Pro Tyr Ile Arg Glu Glu Ala Leu
65                  70                  75                  80

ata gtc ttt aac aac aat act gaa att gta gaa gcc gat ttg tac aaa   289
Ile Val Phe Asn Asn Asn Thr Glu Ile Val Glu Ala Asp Leu Tyr Lys
                85                  90                  95

gga ggc att tat tta ggc tgt gcc ttc ggg gct gca gcc gtg tac att   337
Gly Gly Ile Tyr Leu Gly Cys Ala Phe Gly Ala Ala Ala Val Tyr Ile
            100                 105                 110

tgg gct gtc gga ata ttg gca gcc ggt caa agt tct aca atg act ggg   385
Trp Ala Val Gly Ile Leu Ala Ala Gly Gln Ser Ser Thr Met Thr Gly
        115                 120                 125
```

```
act tat gct ggt caa ttc gcc atg gag ggt ttc ctc aac cta caa tgg    433
Thr Tyr Ala Gly Gln Phe Ala Met Glu Gly Phe Leu Asn Leu Gln Trp
    130                 135                 140

tct cgc tgg aag agg atc cta ttc acc cga atg att gcc atc ata cca    481
Ser Arg Trp Lys Arg Ile Leu Phe Thr Arg Met Ile Ala Ile Ile Pro
145                 150                 155                 160

aca ttt ctg atg gca ttt ttc aat agc atc gaa gac cta tcg ggt atg    529
Thr Phe Leu Met Ala Phe Phe Asn Ser Ile Glu Asp Leu Ser Gly Met
                165                 170                 175

aac gac ctt ctg aat gca gtg atg tcc tta caa cta cct ttt gcg acc    577
Asn Asp Leu Leu Asn Ala Val Met Ser Leu Gln Leu Pro Phe Ala Thr
            180                 185                 190

cta ccg act ata gcg ttt acc agc aat gct gct atc atg gga gaa ttc    625
Leu Pro Thr Ile Ala Phe Thr Ser Asn Ala Ala Ile Met Gly Glu Phe
        195                 200                 205

gtt aat gga gcg gtt aat tca gtc gtt gca atc ctt cta tcg att tta    673
Val Asn Gly Ala Val Asn Ser Val Val Ala Ile Leu Leu Ser Ile Leu
    210                 215                 220

gta att gca atc aat att tat ttt gtg gtc gac cag gtt aat aat gga    721
Val Ile Ala Ile Asn Ile Tyr Phe Val Val Asp Gln Val Asn Asn Gly
225                 230                 235                 240

gac ctg acg gaa ggc tat tta gct ctt ata gtg ata ttt gga at         765
Asp Leu Thr Glu Gly Tyr Leu Ala Leu Ile Val Ile Phe Gly
                245                 250
```

<210> 32
<211> 254
<212> PRT
<213> Ctenocephalides felis

<400> 32

```
His Asn Leu Tyr Leu His Ser Ala Leu Val Lys Ser Arg Asp Val Asp
1               5                   10                  15

Arg Arg Asn Pro Glu Lys Val Arg Asp Ala Asn Tyr Tyr Phe Phe Ile
            20                  25                  30

Glu Ala Ala Ile Ala Leu Phe Ile Ser Phe Ile Ile Asn Val Phe Val
        35                  40                  45
```

```
Val Ala Val Phe Ala His Gly Leu Phe Lys Thr Thr Asn Gln Glu Ile
    50                  55                  60

Leu Asp Thr Cys Lys Ser Ser Ala Pro Tyr Ile Arg Glu Glu Ala Leu
65                  70                  75                  80

Ile Val Phe Asn Asn Asn Thr Glu Ile Val Glu Ala Asp Leu Tyr Lys
                85                  90                  95

Gly Gly Ile Tyr Leu Gly Cys Ala Phe Gly Ala Ala Ala Val Tyr Ile
            100                 105                 110

Trp Ala Val Gly Ile Leu Ala Ala Gly Gln Ser Ser Thr Met Thr Gly
        115                 120                 125

Thr Tyr Ala Gly Gln Phe Ala Met Glu Gly Phe Leu Asn Leu Gln Trp
    130                 135                 140

Ser Arg Trp Lys Arg Ile Leu Phe Thr Arg Met Ile Ala Ile Ile Pro
145                 150                 155                 160

Thr Phe Leu Met Ala Phe Phe Asn Ser Ile Glu Asp Leu Ser Gly Met
                165                 170                 175

Asn Asp Leu Leu Asn Ala Val Met Ser Leu Gln Leu Pro Phe Ala Thr
            180                 185                 190

Leu Pro Thr Ile Ala Phe Thr Ser Asn Ala Ala Ile Met Gly Glu Phe
        195                 200                 205

Val Asn Gly Ala Val Asn Ser Val Val Ala Ile Leu Leu Ser Ile Leu
    210                 215                 220

Val Ile Ala Ile Asn Ile Tyr Phe Val Val Asp Gln Val Asn Asn Gly
225                 230                 235                 240

Asp Leu Thr Glu Gly Tyr Leu Ala Leu Ile Val Ile Phe Gly
                245                 250
```

<210> 33
<211> 765
<212> DNA
<213> Ctenocephalides felis

<400> 33

```
attccaaata tcactataag agctaaatag ccttccgtca ggtctccatt attaacctgg 60
tcgaccacaa aataaatatt gattgcaatt actaaaatcg atagaaggat tgcaacgact 120
gaattaaccg ctccattaac gaattctccc atgatagcag cattgctggt aaacgctata 180
gtcggtaggg tcgcaaaagg tagttgtaag gacatcactg cattcagaag gtcgttcata 240
cccgataggt cttcgatgct attgaaaaat gccatcagaa atgttggtat gatggcaatc 300
attcgggtga ataggatcct cttccagcga gaccattgta ggttgaggaa accctccatg 360
gcgaattgac cagcataagt cccagtcatt gtagaacttt gaccggctgc caatattccg 420
acagcccaaa tgtacacggc tgcagccccg aaggcacagc ctaaataaat gcctcctttg 480
tacaaatcgg cttctacaat ttcagtattg ttgttaaaga ctattagggc ttcctctcgg 540
atatatgggg ctgaactttt gcaagtgtct aagatttctt ggttagtagt tttaaataaa 600
ccatgtgcaa acacagcaac tacgaacaca tttattatga aagatataaa tagtgcaatc 660
gctgcttcga taaagaaata ataattagcg tctctcactt tctccgggtt tctcctgtcg 720
acatctctcg actttacaag ggcactatgc aaatacaaat tgtga                765
```

<210> 34
<211> 762
<212> DNA
<213> Ctenocephalides felis

<220>
<221> CDS
<222> (1)..(762)

<400> 34

```
cac aat ttg tat ttg cat agt gcc ctt gta aag tcg aga gat gtc gac     48
His Asn Leu Tyr Leu His Ser Ala Leu Val Lys Ser Arg Asp Val Asp
1               5                   10                  15

agg aga aac ccg gag aaa gtg aga gac gct aat tat tat ttc ttt atc     96
Arg Arg Asn Pro Glu Lys Val Arg Asp Ala Asn Tyr Tyr Phe Phe Ile
            20                  25                  30

gaa gca gcg att gca cta ttt ata tct ttc ata ata aat gtg ttc gta     144
Glu Ala Ala Ile Ala Leu Phe Ile Ser Phe Ile Ile Asn Val Phe Val
        35                  40                  45

gtt gct gtg ttt gca cat ggt tta ttt aaa act act aac caa gaa atc     192
Val Ala Val Phe Ala His Gly Leu Phe Lys Thr Thr Asn Gln Glu Ile
    50                  55                  60

tta gac act tgc aaa agt tca gcc cca tat atc cga gag gaa gcc cta     240
Leu Asp Thr Cys Lys Ser Ser Ala Pro Tyr Ile Arg Glu Glu Ala Leu
65                  70                  75                  80

ata gtc ttt aac aac aat act gaa att gta gaa gcc gat ttg tac aaa     288
Ile Val Phe Asn Asn Asn Thr Glu Ile Val Glu Ala Asp Leu Tyr Lys
                85                  90                  95
```

```
gga ggc att tat tta ggc tgt gcc ttc ggg gct gca gcc gtg tac att     336
Gly Gly Ile Tyr Leu Gly Cys Ala Phe Gly Ala Ala Ala Val Tyr Ile
                100                 105                 110

tgg gct gtc gga ata ttg gca gcc ggt caa agt tct aca atg act ggg     384
Trp Ala Val Gly Ile Leu Ala Ala Gly Gln Ser Ser Thr Met Thr Gly
        115                 120                 125

act tat gct ggt caa ttc gcc atg gag ggt ttc ctc aac cta caa tgg     432
Thr Tyr Ala Gly Gln Phe Ala Met Glu Gly Phe Leu Asn Leu Gln Trp
    130                 135                 140

tct cgc tgg aag agg atc cta ttc acc cga atg att gcc atc ata cca     480
Ser Arg Trp Lys Arg Ile Leu Phe Thr Arg Met Ile Ala Ile Ile Pro
145                 150                 155                 160

aca ttt ctg atg gca ttt ttc aat agc atc gaa gac cta tcg ggt atg     528
Thr Phe Leu Met Ala Phe Phe Asn Ser Ile Glu Asp Leu Ser Gly Met
                165                 170                 175

aac gac ctt ctg aat gca gtg atg tcc tta caa cta cct ttt gcg acc     576
Asn Asp Leu Leu Asn Ala Val Met Ser Leu Gln Leu Pro Phe Ala Thr
            180                 185                 190

cta ccg act ata gcg ttt acc agc aat gct gct atc atg gga gaa ttc     624
Leu Pro Thr Ile Ala Phe Thr Ser Asn Ala Ala Ile Met Gly Glu Phe
        195                 200                 205

gtt aat gga gcg gtt aat tca gtc gtt gca atc ctt cta tcg att tta     672
Val Asn Gly Ala Val Asn Ser Val Val Ala Ile Leu Leu Ser Ile Leu
    210                 215                 220

gta att gca atc aat att tat ttt gtg gtc gac cag gtt aat aat gga     720
Val Ile Ala Ile Asn Ile Tyr Phe Val Val Asp Gln Val Asn Asn Gly
225                 230                 235                 240

gac ctg acg gaa ggc tat tta gct ctt ata gtg ata ttt gga             762
Asp Leu Thr Glu Gly Tyr Leu Ala Leu Ile Val Ile Phe Gly
                245                 250
```

<210> 35
<211> 254
<212> PRT
<213> Ctenocephalides felis

<400> 35

```
His Asn Leu Tyr Leu His Ser Ala Leu Val Lys Ser Arg Asp Val Asp
 1               5                  10                  15

Arg Arg Asn Pro Glu Lys Val Arg Asp Ala Asn Tyr Tyr Phe Phe Ile
            20                  25                  30

Glu Ala Ala Ile Ala Leu Phe Ile Ser Phe Ile Ile Asn Val Phe Val
        35                  40                  45

Val Ala Val Phe Ala His Gly Leu Phe Lys Thr Thr Asn Gln Glu Ile
    50                  55                  60

Leu Asp Thr Cys Lys Ser Ser Ala Pro Tyr Ile Arg Glu Glu Ala Leu
65                  70                  75                  80

Ile Val Phe Asn Asn Asn Thr Glu Ile Val Glu Ala Asp Leu Tyr Lys
                85                  90                  95

Gly Gly Ile Tyr Leu Gly Cys Ala Phe Gly Ala Ala Ala Val Tyr Ile
            100                 105                 110

Trp Ala Val Gly Ile Leu Ala Ala Gly Gln Ser Ser Thr Met Thr Gly
        115                 120                 125

Thr Tyr Ala Gly Gln Phe Ala Met Glu Gly Phe Leu Asn Leu Gln Trp
    130                 135                 140

Ser Arg Trp Lys Arg Ile Leu Phe Thr Arg Met Ile Ala Ile Ile Pro
145                 150                 155                 160

Thr Phe Leu Met Ala Phe Phe Asn Ser Ile Glu Asp Leu Ser Gly Met
                165                 170                 175

Asn Asp Leu Leu Asn Ala Val Met Ser Leu Gln Leu Pro Phe Ala Thr
            180                 185                 190

Leu Pro Thr Ile Ala Phe Thr Ser Asn Ala Ala Ile Met Gly Glu Phe
        195                 200                 205

Val Asn Gly Ala Val Asn Ser Val Val Ala Ile Leu Leu Ser Ile Leu
    210                 215                 220

Val Ile Ala Ile Asn Ile Tyr Phe Val Val Asp Gln Val Asn Asn Gly
225                 230                 235                 240

Asp Leu Thr Glu Gly Tyr Leu Ala Leu Ile Val Ile Phe Gly
                245                 250
```

<210> 36
<211> 762

<212> DNA
<213> Ctenocephalides felis

<400> 36

```
tccaaatatc actataagag ctaaatagcc ttccgtcagg tctccattat taacctggtc 60
gaccacaaaa taaatattga ttgcaattac taaaatcgat agaaggattg caacgactga 120
attaaccgct ccattaacga attctcccat gatagcagca ttgctggtaa acgctatagt 180
cggtagggtc gcaaaaggta gttgtaagga catcactgca ttcagaaggt cgttcatacc 240
cgataggtct tcgatgctat tgaaaaatgc catcagaaat gttggtatga tggcaatcat 300
tcgggtgaat aggatcctct tccagcgaga ccattgtagg ttgaggaaac cctccatggc 360
gaattgacca gcataagtcc cagtcattgt agaactttga ccggctgcca atattccgac 420
agcccaaatg tacacggctg cagccccgaa ggcacagcct aaataaatgc ctcctttgta 480
caaatcggct tctacaattt cagtattgtt gttaaagact attagggctt cctctcggat 540
atatggggct gaacttttgc aagtgtctaa gatttcttgg ttagtagttt taaataaacc 600
atgtgcaaac acagcaacta cgaacacatt tattatgaaa gatataaata gtgcaatcgc 660
tgcttcgata aagaaataat aattagcgtc tctcactttc tccgggtttc tcctgtcgac 720
atctctcgac tttacaaggg cactatgcaa atacaaattg tg                    762
```

<210> 37
<211> 604
<212> DNA
<213> Ctenocephalides felis

<220>
<221> CDS
<222> (26)..(430)

<400> 37

```
tcaatactct tacttacaga tcaaa atg aga tcc ttc ctc ctc gct aca ttc   52
                            Met Arg Ser Phe Leu Leu Ala Thr Phe
                             1               5

gca gcc ttg ttg gtt tgc tct gtt ttt gct aga cct caa gaa gat aaa   100
Ala Ala Leu Leu Val Cys Ser Val Phe Ala Arg Pro Gln Glu Asp Lys
 10                  15                  20                  25

tat act agc aaa ttt gat aac atc aat tta gat gaa att ttg caa agc   148
Tyr Thr Ser Lys Phe Asp Asn Ile Asn Leu Asp Glu Ile Leu Gln Ser
                30                  35                  40

aat aga ttg ctc aac aac tat gta aac tgc ctt ctc gac aaa ggc agc   196
Asn Arg Leu Leu Asn Asn Tyr Val Asn Cys Leu Leu Asp Lys Gly Ser
            45                  50                  55
```

```
tgc aca gca gaa gga aaa gaa ttg aaa aaa gtc tta cct gat gcc tta     244
Cys Thr Ala Glu Gly Lys Glu Leu Lys Lys Val Leu Pro Asp Ala Leu
        60                  65                  70

tcc aac gag tgc gct aaa tgt agc gag aaa caa aga gaa gga gct gag     292
Ser Asn Glu Cys Ala Lys Cys Ser Glu Lys Gln Arg Glu Gly Ala Glu
    75                  80                  85

aaa gta atc aga ttt ttc gtc aac aac aaa cca gaa gag tgg aag aaa     340
Lys Val Ile Arg Phe Phe Val Asn Asn Lys Pro Glu Glu Trp Lys Lys
90                  95                  100                 105

ctt tct gca gtt tac gat cca acc ggc gag tac aca aag aaa tat agc     388
Leu Ser Ala Val Tyr Asp Pro Thr Gly Glu Tyr Thr Lys Lys Tyr Ser
                110                 115                 120

acc caa att gaa caa gtg aag aga ggc gaa ccc gtt aca gtt             430
Thr Gln Ile Glu Gln Val Lys Arg Gly Glu Pro Val Thr Val
            125                 130                 135

taaatttcca aaaagagatt tctcaaaata ttggcaagtc atttaggaat cgtagtgtta 490

ttttcacctg tagatatttc tgtttttaat aataaaaaaa atgtagttgt aggacaaata 550

gagcatattt aaataaaaaa aaaaaaaaaa aaaaaaaaaa aaaaaaaaaa aaaa       604
```

<210> 38
<211> 135
<212> PRT
<213> Ctenocephalides felis

<400> 38

```
Met Arg Ser Phe Leu Leu Ala Thr Phe Ala Ala Leu Leu Val Cys Ser
1               5                   10                  15

Val Phe Ala Arg Pro Gln Glu Asp Lys Tyr Thr Ser Lys Phe Asp Asn
            20                  25                  30

Ile Asn Leu Asp Glu Ile Leu Gln Ser Asn Arg Leu Leu Asn Asn Tyr
        35                  40                  45

Val Asn Cys Leu Leu Asp Lys Gly Ser Cys Thr Ala Glu Gly Lys Glu
    50                  55                  60

Leu Lys Lys Val Leu Pro Asp Ala Leu Ser Asn Glu Cys Ala Lys Cys
65                  70                  75                  80
```

```
Ser Glu Lys Gln Arg Glu Gly Ala Glu Lys Val Ile Arg Phe Phe Val
                85                  90                  95

Asn Asn Lys Pro Glu Glu Trp Lys Lys Leu Ser Ala Val Tyr Asp Pro
            100                 105                 110

Thr Gly Glu Tyr Thr Lys Lys Tyr Ser Thr Gln Ile Glu Gln Val Lys
        115                 120                 125

Arg Gly Glu Pro Val Thr Val
    130                 135
```

<210> 39
<211> 604
<212> DNA
<213> Ctenocephalides felis

<400> 39

```
ttttttttt ttttttttt ttttttttt ttttttttt atttaaatat gctctatttg 60
tcctacaact acattttttt tattattaaa aacagaaata tctacaggtg aaaataacac 120
tacgattcct aaatgacttg ccaatatttt gagaaatctc tttttggaaa tttaaactgt 180
aacgggttcg cctctcttca cttgttcaat ttgggtgcta tatttctttg tgtactcgcc 240
ggttggatcg taaactgcag aaagtttctt ccactcttct ggtttgttgt tgacgaaaaa 300
tctgattact ttctcagctc cttctctttg tttctcgcta catttagcgc actcgttgga 360
taaggcatca ggtaagactt ttttcaattc ttttccttct gctgtgcagc tgcctttgtc 420
gagaaggcag tttacatagt tgttgagcaa tctattgctt tgcaaaattt catctaaatt 480
gatgttatca aatttgctag tatatttatc ttcttgaggt ctagcaaaaa cagagcaaac 540
caacaaggct gcgaatgtag cgaggaggaa ggatctcatt ttgatctgta agtaagagta 600
ttga                                                              604
```

<210> 40
<211> 405
<212> DNA
<213> Ctenocephalides felis

<220>
<221> CDS
<222> (1)..(405)

<400> 40

```
atg aga tcc ttc ctc ctc gct aca ttc gca gcc ttg ttg gtt tgc tct    48
Met Arg Ser Phe Leu Leu Ala Thr Phe Ala Ala Leu Leu Val Cys Ser
  1               5                  10                  15
```

```
gtt ttt gct aga cct caa gaa gat aaa tat act agc aaa ttt gat aac     96
Val Phe Ala Arg Pro Gln Glu Asp Lys Tyr Thr Ser Lys Phe Asp Asn
            20                  25                  30

atc aat tta gat gaa att ttg caa agc aat aga ttg ctc aac aac tat     144
Ile Asn Leu Asp Glu Ile Leu Gln Ser Asn Arg Leu Leu Asn Asn Tyr
        35                  40                  45

gta aac tgc ctt ctc gac aaa ggc agc tgc aca gca gaa gga aaa gaa     192
Val Asn Cys Leu Leu Asp Lys Gly Ser Cys Thr Ala Glu Gly Lys Glu
    50                  55                  60

ttg aaa aaa gtc tta cct gat gcc tta tcc aac gag tgc gct aaa tgt     240
Leu Lys Lys Val Leu Pro Asp Ala Leu Ser Asn Glu Cys Ala Lys Cys
65                  70                  75                  80

agc gag aaa caa aga gaa gga gct gag aaa gta atc aga ttt ttc gtc     288
Ser Glu Lys Gln Arg Glu Gly Ala Glu Lys Val Ile Arg Phe Phe Val
                85                  90                  95

aac aac aaa cca gaa gag tgg aag aaa ctt tct gca gtt tac gat cca     336
Asn Asn Lys Pro Glu Glu Trp Lys Lys Leu Ser Ala Val Tyr Asp Pro
            100                 105                 110

acc ggc gag tac aca aag aaa tat agc acc caa att gaa caa gtg aag     384
Thr Gly Glu Tyr Thr Lys Lys Tyr Ser Thr Gln Ile Glu Gln Val Lys
        115                 120                 125

aga ggc gaa ccc gtt aca gtt                                         405
Arg Gly Glu Pro Val Thr Val
    130                 135
```

<210> 41
<211> 135
<212> PRT
<213> Ctenocephalides felis

<400> 41

```
Met Arg Ser Phe Leu Leu Ala Thr Phe Ala Ala Leu Leu Val Cys Ser
1               5                   10                  15

Val Phe Ala Arg Pro Gln Glu Asp Lys Tyr Thr Ser Lys Phe Asp Asn
            20                  25                  30

Ile Asn Leu Asp Glu Ile Leu Gln Ser Asn Arg Leu Leu Asn Asn Tyr
        35                  40                  45
```

```
Val Asn Cys Leu Leu Asp Lys Gly Ser Cys Thr Ala Glu Gly Lys Glu
     50                  55                  60

Leu Lys Lys Val Leu Pro Asp Ala Leu Ser Asn Glu Cys Ala Lys Cys
 65                  70                  75                  80

Ser Glu Lys Gln Arg Glu Gly Ala Glu Lys Val Ile Arg Phe Phe Val
                 85                  90                  95

Asn Asn Lys Pro Glu Glu Trp Lys Lys Leu Ser Ala Val Tyr Asp Pro
            100                 105                 110

Thr Gly Glu Tyr Thr Lys Lys Tyr Ser Thr Gln Ile Glu Gln Val Lys
        115                 120                 125

Arg Gly Glu Pro Val Thr Val
    130                 135
```

<210> 42
<211> 405
<212> DNA
<213> Ctenocephalides felis

<400> 42

```
aactgtaacg ggttcgcctc tcttcacttg ttcaatttgg gtgctatatt tctttgtgta 60
ctcgccggtt ggatcgtaaa ctgcagaaag tttcttccac tcttctggtt tgttgttgac 120
gaaaaatctg attactttct cagctccttc tctttgtttc tcgctacatt tagcgcactc 180
gttggataag gcatcaggta agactttttt caattctttt ccttctgctg tgcagctgcc 240
tttgtcgaga aggcagttta catagttgtt gagcaatcta ttgctttgca aaatttcatc 300
taaattgatg ttatcaaatt tgctagtata tttatcttct tgaggtctag caaaaacaga 360
gcaaaccaac aaggctgcga atgtagcgag gaggaaggat ctcat                405
```

<210> 43
<211> 1227
<212> DNA
<213> Ctenocephalides felis

<220>
<221> CDS
<222> (312)..(1049)

<400> 43

```
gcgttttcta aaacaaactt ctttataatg taaataaata gaaatgaaat gtgaataaat 60

ttgagtatga aaacaaatta atcaacaaca caatttaatt tccttatttc atttataatg 120
```

```
ttggcattct aataaattaa gttattatga gaaaacttca ttacagtgat gtgtcaagct 180

cctgtcaaaa taaattgaaa atctgtttgt gataaaaatt gtttttcta taaaaatagc 240

ataatttagg aaactatatc gaaaaaataa taaattttta agtggccctt taatatcttc 300

tagaattaaa t atg gca acc gta ccc ctt atg ttt gct gaa gat gac cta  350
             Met Ala Thr Val Pro Leu Met Phe Ala Glu Asp Asp Leu
              1               5                  10

gaa ggt ggt gga aaa gaa ggt tca ata gag aat gac ttt gca tat aac   398
Glu Gly Gly Gly Lys Glu Gly Ser Ile Glu Asn Asp Phe Ala Tyr Asn
    15                  20                  25

aat aac gtt att aat gca tct gtt cgt gtg aga ctt gga ttc att cga   446
Asn Asn Val Ile Asn Ala Ser Val Arg Val Arg Leu Gly Phe Ile Arg
30                  35                  40                  45

aaa gtc tat gga cta ctt aca gtt cag ttg tta ttg agc ttg ctg gtg   494
Lys Val Tyr Gly Leu Leu Thr Val Gln Leu Leu Leu Ser Leu Leu Val
                50                  55                  60

ggc ata gcc tgc caa att gag cct gta caa gga att gtt aaa gca aat   542
Gly Ile Ala Cys Gln Ile Glu Pro Val Gln Gly Ile Val Lys Ala Asn
            65                  70                  75

gac tgg ctc gta tta gtc tgc atg atc agt agc att ggt gtg ctg att   590
Asp Trp Leu Val Leu Val Cys Met Ile Ser Ser Ile Gly Val Leu Ile
        80                  85                  90

gct ctt cac atc aag aga aag gaa aca cca act aat ttt att ctt tta   638
Ala Leu His Ile Lys Arg Lys Glu Thr Pro Thr Asn Phe Ile Leu Leu
    95                  100                 105

aca att ttc aca att aca aac tcc atc agt gtg ggt gtg cta gta aca   686
Thr Ile Phe Thr Ile Thr Asn Ser Ile Ser Val Gly Val Leu Val Thr
110                 115                 120                 125

cat ttt aaa gct agt tta gta ctt caa gct att gca att act ttg tgt   734
His Phe Lys Ala Ser Leu Val Leu Gln Ala Ile Ala Ile Thr Leu Cys
                130                 135                 140

gtt gtt att ggt ata aca ctc ttt aca tta caa aac aaa ctg gat tta   782
Val Val Ile Gly Ile Thr Leu Phe Thr Leu Gln Asn Lys Leu Asp Leu
            145                 150                 155

tca atg ctc cca gca gca ttg ttt act gga ctt tgc tgt tta ttg gta   830
```

```
Ser Met Leu Pro Ala Ala Leu Phe Thr Gly Leu Cys Cys Leu Leu Val
        160                 165                 170

ggt ggt atc att cag ata ttc act cat tca acc att ttt gaa tta gtg    878
Gly Gly Ile Ile Gln Ile Phe Thr His Ser Thr Ile Phe Glu Leu Val
    175                 180                 185

tta tgc agt ttt ggt gca cta ata ttc agc ttg ttt ttg ctt tat gac    926
Leu Cys Ser Phe Gly Ala Leu Ile Phe Ser Leu Phe Leu Leu Tyr Asp
190                 195                 200                 205

acg cat gtt atg atg acg aca tta tca cca gaa gag tat att ttg gcc    974
Thr His Val Met Met Thr Thr Leu Ser Pro Glu Glu Tyr Ile Leu Ala
                210                 215                 220

aca att aac ttg tac tta gat att gtc aat cta ttc ata tat att tta    1022
Thr Ile Asn Leu Tyr Leu Asp Ile Val Asn Leu Phe Ile Tyr Ile Leu
            225                 230                 235

aga att ctg caa gca gca gac agg ggt taaatcatct gtgataaaat          1069
Arg Ile Leu Gln Ala Ala Asp Arg Gly
        240                 245

ataaatggtt caaaattcta tattgtatta tttatatatt taaaaatgcc ttgcttattt 1129

atattgtatg tttccattta ttgtatatag tttattttgt tattttatgg ccaagattaa 1189

taaattcgaa attaatatgc aaaaaaaaaa aaaaaaaa                         1227
```

<210> 44
<211> 246
<212> PRT
<213> Ctenocephalides felis

<400> 44

```
Met Ala Thr Val Pro Leu Met Phe Ala Glu Asp Asp Leu Glu Gly Gly
 1               5                  10                  15

Gly Lys Glu Gly Ser Ile Glu Asn Asp Phe Ala Tyr Asn Asn Asn Val
            20                  25                  30

Ile Asn Ala Ser Val Arg Val Arg Leu Gly Phe Ile Arg Lys Val Tyr
        35                  40                  45

Gly Leu Leu Thr Val Gln Leu Leu Leu Ser Leu Leu Val Gly Ile Ala
    50                  55                  60
```

```
Cys Gln Ile Glu Pro Val Gln Gly Ile Val Lys Ala Asn Asp Trp Leu
 65                  70                  75                  80

Val Leu Val Cys Met Ile Ser Ser Ile Gly Val Leu Ile Ala Leu His
                 85                  90                  95

Ile Lys Arg Lys Glu Thr Pro Thr Asn Phe Ile Leu Leu Thr Ile Phe
            100                 105                 110

Thr Ile Thr Asn Ser Ile Ser Val Gly Val Leu Val Thr His Phe Lys
        115                 120                 125

Ala Ser Leu Val Leu Gln Ala Ile Ala Ile Thr Leu Cys Val Val Ile
    130                 135                 140

Gly Ile Thr Leu Phe Thr Leu Gln Asn Lys Leu Asp Leu Ser Met Leu
145                 150                 155                 160

Pro Ala Ala Leu Phe Thr Gly Leu Cys Cys Leu Leu Val Gly Gly Ile
                165                 170                 175

Ile Gln Ile Phe Thr His Ser Thr Ile Phe Glu Leu Val Leu Cys Ser
            180                 185                 190

Phe Gly Ala Leu Ile Phe Ser Leu Phe Leu Leu Tyr Asp Thr His Val
        195                 200                 205

Met Met Thr Thr Leu Ser Pro Glu Glu Tyr Ile Leu Ala Thr Ile Asn
    210                 215                 220

Leu Tyr Leu Asp Ile Val Asn Leu Phe Ile Tyr Ile Leu Arg Ile Leu
225                 230                 235                 240

Gln Ala Ala Asp Arg Gly
                245
```

<210> 45
<211> 1227
<212> DNA
<213> Ctenocephalides felis

<400> 45

```
ttttttttt tttttttgc atattaattt cgaatttatt aatcttggcc ataaaataac 60
aaaataaact atatacaata aatggaaaca tacaatataa ataagcaagg catttttaaa 120
tatataaata atacaatata gaattttgaa ccatttatat tttatcacag atgatttaac 180
ccctgtctgc tgcttgcaga attcttaaaa tatatatgaa tagattgaca atatctaagt 240
```

```
acaagttaat tgtggccaaa atatactctt ctggtgataa tgtcgtcatc ataacatgcg 300
tgtcataaag caaaaacaag ctgaatatta gtgcaccaaa actgcataac actaattcaa 360
aaatggttga atgagtgaat atctgaatga taccacctac caataaacag caaagtccag 420
taaacaatgc tgctgggagc attgataaat ccagtttgtt ttgtaatgta aagagtgtta 480
taccaataac aacacacaaa gtaattgcaa tagcttgaag tactaaacta gctttaaaat 540
gtgttactag cacacccaca ctgatggagt ttgtaattgt gaaaattgtt aaaagaataa 600
aattagttgg tgtttccttt ctcttgatgt gaagagcaat cagcacacca atgctactga 660
tcatgcagac taatacgagc cagtcatttg ctttaacaat tccttgtaca ggctcaattt 720
ggcaggctat gcccaccagc aagctcaata acaactgaac tgtaagtagt ccatagactt 780
ttcgaatgaa tccaagtctc acacgaacag atgcattaat aacgttattg ttatatgcaa 840
agtcattctc tattgaacct tcttttccac caccttctag gtcatcttca gcaaacataa 900
ggggtacggt tgccatattt aattctagaa gatattaaag ggccacttaa aaatttatta 960
tttttcgat atagtttcct aaattatgct atttttatag aaaaaacaat tttatcaca 1020
aacagatttt caatttattt tgacaggagc ttgacacatc actgtaatga agttttctca 1080
taataactta atttattaga atgccaacat tataaatgaa ataaggaaat taaattgtgt 1140
tgttgattaa tttgttttca tactcaaatt tattcacatt tcatttctat ttatttacat 1200
tataaagaag tttgttttag aaaacgc                                    1227
```

<210> 46
<211> 738
<212> DNA
<213> Ctenocephalides felis

<220>
<221> CDS
<222> (1)..(738)

<400> 46

```
atg gca acc gta ccc ctt atg ttt gct gaa gat gac cta gaa ggt ggt    48
Met Ala Thr Val Pro Leu Met Phe Ala Glu Asp Asp Leu Glu Gly Gly
 1               5                  10                  15

gga aaa gaa ggt tca ata gag aat gac ttt gca tat aac aat aac gtt    96
Gly Lys Glu Gly Ser Ile Glu Asn Asp Phe Ala Tyr Asn Asn Asn Val
            20                  25                  30

att aat gca tct gtt cgt gtg aga ctt gga ttc att cga aaa gtc tat   144
Ile Asn Ala Ser Val Arg Val Arg Leu Gly Phe Ile Arg Lys Val Tyr
        35                  40                  45

gga cta ctt aca gtt cag ttg tta ttg agc ttg ctg gtg ggc ata gcc   192
Gly Leu Leu Thr Val Gln Leu Leu Leu Ser Leu Leu Val Gly Ile Ala
    50                  55                  60

tgc caa att gag cct gta caa gga att gtt aaa gca aat gac tgg ctc   240
Cys Gln Ile Glu Pro Val Gln Gly Ile Val Lys Ala Asn Asp Trp Leu
65                  70                  75                  80
```

```
gta tta gtc tgc atg atc agt agc att ggt gtg ctg att gct ctt cac    288
Val Leu Val Cys Met Ile Ser Ser Ile Gly Val Leu Ile Ala Leu His
                85                  90                  95

atc aag aga aag gaa aca cca act aat ttt att ctt tta aca att ttc    336
Ile Lys Arg Lys Glu Thr Pro Thr Asn Phe Ile Leu Leu Thr Ile Phe
            100                 105                 110

aca att aca aac tcc atc agt gtg ggt gtg cta gta aca cat ttt aaa    384
Thr Ile Thr Asn Ser Ile Ser Val Gly Val Leu Val Thr His Phe Lys
        115                 120                 125

gct agt tta gta ctt caa gct att gca att act ttg tgt gtt gtt att    432
Ala Ser Leu Val Leu Gln Ala Ile Ala Ile Thr Leu Cys Val Val Ile
    130                 135                 140

ggt ata aca ctc ttt aca tta caa aac aaa ctg gat tta tca atg ctc    480
Gly Ile Thr Leu Phe Thr Leu Gln Asn Lys Leu Asp Leu Ser Met Leu
145                 150                 155                 160

cca gca gca ttg ttt act gga ctt tgc tgt tta ttg gta ggt ggt atc    528
Pro Ala Ala Leu Phe Thr Gly Leu Cys Cys Leu Leu Val Gly Gly Ile
                165                 170                 175

att cag ata ttc act cat tca acc att ttt gaa tta gtg tta tgc agt    576
Ile Gln Ile Phe Thr His Ser Thr Ile Phe Glu Leu Val Leu Cys Ser
            180                 185                 190

ttt ggt gca cta ata ttc agc ttg ttt ttg ctt tat gac acg cat gtt    624
Phe Gly Ala Leu Ile Phe Ser Leu Phe Leu Leu Tyr Asp Thr His Val
        195                 200                 205

atg atg acg aca tta tca cca gaa gag tat att ttg gcc aca att aac    672
Met Met Thr Thr Leu Ser Pro Glu Glu Tyr Ile Leu Ala Thr Ile Asn
    210                 215                 220

ttg tac tta gat att gtc aat cta ttc ata tat att tta aga att ctg    720
Leu Tyr Leu Asp Ile Val Asn Leu Phe Ile Tyr Ile Leu Arg Ile Leu
225                 230                 235                 240

caa gca gca gac agg ggt                                            738
Gln Ala Ala Asp Arg Gly
                245
```

<210> 47
<211> 246
<212> PRT
<213> Ctenocephalides felis <400> 47

```
Met Ala Thr Val Pro Leu Met Phe Ala Glu Asp Asp Leu Glu Gly Gly
 1              5                   10                  15

Gly Lys Glu Gly Ser Ile Glu Asn Asp Phe Ala Tyr Asn Asn Asn Val
            20                  25                  30

Ile Asn Ala Ser Val Arg Val Arg Leu Gly Phe Ile Arg Lys Val Tyr
        35                  40                  45

Gly Leu Leu Thr Val Gln Leu Leu Leu Ser Leu Leu Val Gly Ile Ala
    50                  55                  60

Cys Gln Ile Glu Pro Val Gln Gly Ile Val Lys Ala Asn Asp Trp Leu
65                  70                  75                  80

Val Leu Val Cys Met Ile Ser Ser Ile Gly Val Leu Ile Ala Leu His
                85                  90                  95

Ile Lys Arg Lys Glu Thr Pro Thr Asn Phe Ile Leu Leu Thr Ile Phe
            100                 105                 110

Thr Ile Thr Asn Ser Ile Ser Val Gly Val Leu Val Thr His Phe Lys
        115                 120                 125

Ala Ser Leu Val Leu Gln Ala Ile Ala Ile Thr Leu Cys Val Val Ile
    130                 135                 140

Gly Ile Thr Leu Phe Thr Leu Gln Asn Lys Leu Asp Leu Ser Met Leu
145                 150                 155                 160

Pro Ala Ala Leu Phe Thr Gly Leu Cys Cys Leu Leu Val Gly Gly Ile
                165                 170                 175

Ile Gln Ile Phe Thr His Ser Thr Ile Phe Glu Leu Val Leu Cys Ser
            180                 185                 190

Phe Gly Ala Leu Ile Phe Ser Leu Phe Leu Leu Tyr Asp Thr His Val
        195                 200                 205

Met Met Thr Thr Leu Ser Pro Glu Glu Tyr Ile Leu Ala Thr Ile Asn
    210                 215                 220

Leu Tyr Leu Asp Ile Val Asn Leu Phe Ile Tyr Ile Leu Arg Ile Leu
225                 230                 235                 240

Gln Ala Ala Asp Arg Gly
                245
```

<210> 48
<211> 738

<212> DNA
<213> Ctenocephalides felis

<400> 48

```
acccctgtct gctgcttgca gaattcttaa aatatatatg aatagattga caatatctaa 60
gtacaagtta attgtggcca aaatatactc ttctggtgat aatgtcgtca tcataacatg 120
cgtgtcataa agcaaaaaca agctgaatat tagtgcacca aaactgcata acactaattc 180
aaaaatggtt gaatgagtga atatctgaat gataccacct accaataaac agcaaagtcc 240
agtaaacaat gctgctggga gcattgataa atccagtttg ttttgtaatg taaagagtgt 300
tataccaata acaacacaca aagtaattgc aatagcttga agtactaaac tagctttaaa 360
atgtgttact agcacaccca cactgatgga gtttgtaatt gtgaaaattg ttaaaagaat 420
aaaattagtt ggtgtttcct ttctcttgat gtgaagagca atcagcacac caatgctact 480
gatcatgcag actaatacga gccagtcatt tgctttaaca attccttgta caggctcaat 540
ttggcaggct atgcccacca gcaagctcaa taacaactga actgtaagta gtccatagac 600
ttttcgaatg aatccaagtc tcacacgaac agatgcatta ataacgttat tgttatatgc 660
aaagtcattc tctattgaac cttcttttcc accaccttct aggtcatctt cagcaaacat 720
aaggggtacg gttgccat                                               738
```

<210> 49
<211> 31
<212> DNA
<213> Artificial Sequence

<220>
<223> Description of Artificial Sequence: Synthetic Primer

<400> 49
gcggatccta tgctgaattg caagaacctt g 31

<210> 50
<211> 30
<212> DNA
<213> Artificial Sequence

<220>
<223> Description of Artificial Sequence: Synthetic Primer

<400> 50
caggtaccct cttttagaag caccggtccc 30

<210> 51
<211> 29
<212> DNA
<213> Artificial Sequence

<220>
<223> Description of Artificial Sequence: Synthetic Primer

<400> 51
cgggatcctg ctgacaggaa ttcgcccac 29

<210> 52
<211> 31
<212> DNA

<213> Artificial Sequence

<220>
<223> Description of Artificial Sequence: Synthetic Primer

<400> 52
catggtaccc ctggtttaag ccttacttag c 31

<210> 53
<211> 23
<212> DNA
<213> Artificial Sequence

<220>
<223> Description of Artificial Sequence: Synthetic Primer

<400> 53
ccattattaa cctggtcgac cac 23

<210> 54
<211> 18
<212> DNA
<213> Artificial Sequence

<220>
<223> Description of Artificial Sequence: Synthetic Primer

<400> 54
ggaaacagta tgaccatg 18

<210> 55
<211> 21
<212> DNA
<213> Artificial Sequence

<220>
<223> Description of Artificial Sequence: Synthetic Primer

<400> 55
cgctatagtc ggtagggtcg c 21

<210> 56
<211> 20
<212> DNA
<213> Artificial Sequence

<220>
<223> Description of Artificial Sequence: Synthetic Primer

<400> 56
aattaaccct cactaaaggg 20

<210> 57
<211> 20
<212> DNA
<213> Artificial Sequence

<220>

<223> Description of Artificial Sequence: Synthetic Primer

<400> 57
caaaactggt ctccccgctc 20

<210> 58
<211> 20
<212> DNA
<213> Artificial Sequence

<220>
<223> Description of Artificial Sequence: Synthetic Primer

<400> 58
taatacgact cactataggg 20

<210> 59
<211> 20
<212> DNA
<213> Artificial Sequence

<220>
<223> Description of Artificial Sequence: Synthetic Primer

<400> 59
ggttcgcctc tcttcacttg 20

<210> 60
<211> 21
<212> DNA
<213> Artificial Sequence

<220>
<223> Description of Artificial Sequence: Synthetic Primer

<400> 60
cggttggatc gtaaactgca g 21

<210> 61
<211> 40
<212> DNA
<213> Artificial Sequence

<220>
<223> Description of Artificial Sequence: Synthetic Primer

<400> 61
cgcggatcca gaagataaat atactagcaa atttgataac 40

<210> 62
<211> 37
<212> DNA
<213> Artificial Sequence

<220>
<223> Description of Artificial Sequence: Synthetic Primer

<400> 62

gaggaattcc tctttttgga aatttaaact gtaacgg 37

<210> 63
<211> 500
<212> DNA
<213> Ctenocephalides felis

<400> 63

```
accaggncga ataatatttc taattgtttc aatacaaact ataaanggta ttaaaattat 60
tggagttcct tgaggcacta gatgactaaa tatatggtta taattattaa ttcatcctan 120
taatataaat cttaatcata aaggaagtct taatcttaaa gttaaagtta aatgactagt 180
tcttgtanaa atgtaattaa ataatcctaa aaaattatta aataaaataa atgaaaataa 240
tctaataaat ataaaagttc ttcctatagg attatatttt ataagtaatt taaattcatt 300
atgaagtgtt ataataattt ttaatcaaat tatgttgtat cgggacggaa ttattcanaa 360
tatatttgga ataaataata ntcctaatac ctgccggnnn tttgtntnnt ctctccnnct 420
ntccannctn atcncntncc cntcnnnnnt ganatncntn tnnnnnctnt ctcccctncn 480
tacnctgnnn ctccnnnttc                                             500
```

<210> 64
<211> 164
<212> DNA
<213> Ctenocephalides felis

<400> 64

```
gcagctaaca agaatgtatt aactgcattc caaagaagaa attattctga tgaattatct 60
ttgacatttg ctgctgccaa taaggtattc tatgactcag tagatgtaaa gcaggtggat 120
gttccatctt tcagtggtgc ttttggtatc ttagctaaac acgt                  164
```

<210> 65
<211> 337
<212> DNA <213> Ctenocephalides felis

<400> 65

```
cgcttccagt tgacgtttcg tctcatgcaa taattaatta aacttgtttg ttagaggtgc 60
aaaataaaat taaattaaaa tgactgcctg gagacaagct ggtttaaact acattaactt 120
ttcaacaatt gctgcccgaa tggtccgcca agctttgaaa tctgatctaa aaaatgaggc 180
tttgaaacgg gacgtatcta gcattaaatt cacaccctgg aaggacggaa aagcgatcac 240
tggaaaaccg gaataaaatc aaatactcat ctataaaagt gaaaccaagt aatcacaaga 300
tggaataata gacaattcac tcaaattaat aatgtgt                           337
```

<210> 66
<211> 201
<212> DNA
<213> Ctenocephalides felis

<400> 66

acaggccatt tatgtgccag ctgatgactt gacagatcct gcccctgcca ctacattcgc 60
tcacttggac gccaccactg tattgtcccg tgccattgct gaattaggta tctacccagc 120
tgtggatcct ttggattcta catcccgtat tatggacccc aacatcattg gagctgaaca 180
ttacaacatt gcccgtggcg t 201

<210> 67
<211> 179
<212> DNA
<213> Ctenocephalides felis

<400> 67

accttgagac ttttggcagc taacaagaat gtattaactg cattccaaag aagaaattat 60
tctgatgaat tatctttgac atttgctgct gccaataagg tattctatga ctcagtagat 120
gtaaagcagg tggatgttcc atctttcagt ggtgcttttg gtatcttagc taaacacgt 179

<210> 68
<211> 500
<212> DNA
<213> Ctenocephalides felis

<400> 68

acaccatcat tgtatcagcc actgcttctg atgctgcccc tcttcaatat cttgctccat 60
actctggatg tgctatgggt gaattcttcc gtgacaatgg aaaacatgct ttgatcatct 120
atgatgattt atccaaacaa gctgttgctt atcgtcaaat gtctctattg ttacgtcgtc 180
caccaggtcg tgaggcttat ccaggtgatg tcttctacct tcactcacgt ctacttgaac 240
gtgccgctaa aatgtctgaa gctcatggag gtggctcttt gactgctttg ccagttattg 300
aaacacaagc tggtgacgta tcagcttata ttccaactaa tgtcatttcc attactgatg 360
gcaaatcttc ttggaaactg aattgttcta caagggtatt cgaccagcca tcaatgtagg 420
tttatctgta tctcgtgtag gtctgctgca caaaccaaag ccatgaaaca ggttgccggt 480

tcatgaaact ggaattagct 500

<210> 69
<211> 549
<212> DNA
<213> Ctenocephalides felis

<400> 69

tacataatgg catctttgaa gttaaatatc gtaaccagga gcttaagctc atctgcagtc 60
gcgtctcaaa tggttaaacc acccgtacaa gtatatggaa ttgagggtag atacgcaact 120
gccttgtact caggagccag taagaataaa gtactcgacg ccgtcgagaa agatttggtc 180
aaaatacaga ataacttaaa gaccgatgta aaattccgcg atttcattgc caatccaaca 240
ttcaagcgct caattaaatc aaatgcattg aaggaagcca gcagcaagct gcaaatggct 300
ccagctactt ctaacttgtt ggagttactt gctgagaatg gacgtttaaa taaactggaa 360
ggtgttatca acgcttataa agttatgatg tctgctcatc gtggagaggt tccatgtgaa 420
gttacaacag ctaaaccttt agatgagaat caacgaaaac aacttgaaag cctttaagag 480
gattcttaaa acccaaagaa aacttattac taccottaag taatcctcat cttggtggaa 540
tgtcgntca 549

<210> 70
<211> 238
<212> DNA
<213> Ctenocephalides felis

<400> 70

actgctgatc cctgtccctc gccccagcat aataccatta ggttgaaacg tccatttcct 60
gcgtccacca gattgcgagt gtaacggtaa cgatcaaatt tggcataccg cctccactcg 120
gctggatccg acttgtagct cagcatcaga tgattgacca actcgatgtc tacatcatct 180
tcagcaaatg cttcgtgcaa ctgttcaact agatctttta gagaagtaac accttggt 238

<210> 71
<211> 500
<212> DNA
<213> Ctenocephalides felis

<400> 71

actaatgaca aggctggtgg tccccattat gtcttgcgct ggactactcc tcaatctatc 60
aaggagacat ttgttccaat cacatgtgtc gactatccat acatgagaga ataagctgcc 120
aaatcccccc caaagcaccc acaccataac aataattcaa tggacactaa accaaagatg 180
cgtgttacaa atactctgtt caagatctcg atcataaaat ttgcacacag tccggttcac 240
ttttgatttc tgttattcag taactatttt atctttaccg cgcacttgga aaataacagg 300
tgaatcaaag aaattgattt tagtaattat ttttcttgtt atataaataa aagaatatta 360
tagtaacatt ttgccattaa aaatattaat tttacctagg aagcaatatc aagtattcat 420
tcaagtcaaa tttgaagaca tttattaaaa atcgatgttg ccattttatt aaatagatta 480
tgaaaattat gggggtatta 500

<210> 72
<211> 500
<212> DNA
<213> Ctenocephalides felis

<400> 72

acatataata ttgataatat gcatagggcg gattttcagt gtctgtccat tcttctggtg 60
tcggaacatc cttatcgaat aatggatttt cgggtttact ttcatcatcc accgagtcaa 120
aacctatcac gaactgaaga aacttgtgta gttcaggatg cgattgtgga tcattcgtaa 180
cttcaaataa tggcaaatag atatttgtaa gaatttcttg gaaattgttc atcagcttat 240
ttaatttgaa aatatcgtat aatcgtggta tttgaataag ccagcgcaca ttatcactat 300
aaacattaga ttctattgcc cattttgcaa gtttatccca ctcttcagga cttttaccat 360
aaattgaaag tctcaattcg caattttgat atttgctttc ttctaaatcc gaactaactt 420
cattgataat tcttgcaaaa tattttccgt tcaagtagtt atcggtttta agaaacttct 480
ctcaatctgc tttcaccaat 500

<210> 73
<211> 500
<212> DNA
<213> Ctenocephalides felis

<400> 73

actaattaat tttaaaaaat taaggataga aaccaacctg gcttaaaccg gtttgaactc 60
agannatgta agaattaatg gtcgaacaga ccaaatttta aaacttctgc attttaaaat 120
tatcttaatc caacatcgag gtcgcaatct attttgtcga tatgttctct taaaaataat 180
tacgctgtta tcccttaagt aacttaatct tttaatcata atttatggat caattattca 240
attatttatg ttttaataaa aaaaaagttt tataaatttt cctatcaccc caataaaata 300
tattaatata aataaattta ataatattct taaaattaat ctatatttat atataaaact 360
ttaaagggtc ttctcgtcct ttaataatat ttacgctttt taacataaaa attaaattct 420
ataacaattt tattaagaca ggtaatattc attcaatcat tcattccagc ttcaattaaa 480
aaactatgga ttatgctacc 500

<210> 74
<211> 500
<212> DNA
<213> Ctenocephalides felis

<400> 74

ccttatgata ttcataaatc taaaccacag tnnntttttc ttgcactttg tgcaaaacta 60
tattccagtg ttgtctgcct ttgacacagc tcaacatttt gttgcatttt gcaatctttc 120
actaatatct gcccagttgg caacatcgaa aattgctttt acatagtcag ccctgacatt 180
tttatattga aggtagtatg catgttccca gacgtcaatt ccaaatagtg gaaccagacc 240
agtggttgct tccaatggat cttgatttgc gcatgtagct atttgtaact ttttagcagt 300
tttattataa gccagccatc cccatcctga gccttgaaca cctacagctg ctgtagacaa 360
agcagttttc atcttatcca tagatccaaa atcacattca atcattttct gtagctggtc 420
acttggttta cccccttcg gggaaaggtt tttccaaaag atagaatgat tgatgtgcct 480
cctccattga atttcaatgc 500

<210> 75
<211> 348
<212> DNA
<213> Ctenocephalides felis

<400> 75

cagtgacgaa aagcaagtgc cattatcgtt ctctccgtcg atttacgaaa ctacacttca 60
attccaaaaa ggataactgg gactcgcatt ccgactcact acccaacgac tacgatttaa 120
ttagctaaag gccaaattaa agcttcaaga tgccgttcaa gccagtagaa aacccaaaat 180
gcccaaaatg cggcaaatca gtatatgccg ccgaagagcg tgttgccggt ggacttaagt 240
ggcacaaaat gtgtttcaaa tgcggtatgt gcagcaaatt gttggactcc accaactgca 300
ctgagcacga aggtgagttg ttctgcaaga actgccacgc ccgcaagt 348

<210> 76
<211> 451
<212> DNA
<213> Ctenocephalides felis

<400> 76

actatatata atatatatcg attttctata ttagtcaaat atatgtgtta agcatttttt 60
tagagcattc ctatttaaaa ataaaatgtt acgtcaaatt tgaaaattcc aagtaaaaaa 120
atagtttttt tctatatttg aattttgatc gttgtattac tacgaatgtt gccttgagat 180
gtgcgttcta ttttgtagaa taaatatcat tcgtaatgaa ttaattcgcg tttaagccct 240
gtttttggcg cagtccgggc atataatgtc ggcgccgtct gtgatgaagc cacggccgac 300
tagagaagct ttgcatgacg cgcaggtgaa gcagtcattg tgccaatggc gatcttcaaa 360
cgagatgaag cgagtgccac caattcctgt aatgggtttg gtgcaagccg tgcatctctt 420
agcaaacaat tcaccgaaca ttcagcacag t 451

<210> 77
<211> 500
<212> DNA
<213> Ctenocephalides felis

<400> 77

accacatatg acttcaatgg gacttggtaa tggaggctct tcactcactt cggggcaaaa 60
catatcttct gngggatcac tgccaaattc tattgaaact tgtaaatcca atctacctca 120
caataattct gcacaaacac cagaaaatcc ttacgtttat gatacagtaa cttctaatta 180
tagccaacca ccagtaactt catcaccata tgcaccagta gaacctaaga gagcgcaggg 240
acatccatta aaaagtttca gtgttccggc accacccaca tcatcaactc caaatactcc 300
taacaccaag cataatgctt cccaaggtat taatcgacca caaaatgcat caccatataa 360
aaaaccttta atgattaata gactgcaagc aggaccatca gtttgtcatt catcagatga 420

agttcaacga ctggctccga gccctcaact gaggaattgc atcaagaaat ggctatttgg 480
aaggcttaat gaaagactaa 500

<210> 78
<211> 550
<212> DNA
<213> Ctenocephalides felis

<400> 78

```
ggagaacgga caccaacagc aaggtggcga gtacaccaag gccatgaaca aggactggca 60
ctctggtcac ttctgctgct ggcaatgcga cgagtcactg accggccaac gctacgtact 120
ccgcgacgaa catccttact gcatcaagtg ctacgagagc gtcttttcca acacctgcga 180
ggaatgcagc aagatcattg gcattgattc caaggactta tcttacaaag aaaagcattg 240
gcatgaggca tgtttcttgt gcagtaaatg ccgcgtatct ctcgtcgata aacagttcgg 300
aagtaaattg gacaaaatct actgtggaaa ctgctatgat gcccaattcg cttccaggtg 360
tgatggttgc ggcgaaatct tccgtgcggg tactaaaaaa atggagtaca aaactcgtca 420
atggcatgaa aagtgtttct gctgctgtgt gtgcaagact gtatcggaac caaaagcttt 480
attctcgtga gcaggaaatt attgccagct gtatgaggaa agttcgcacc agatcattaa 540
atgcataaga                                                         550
```

<210> 79
<211> 550
<212> DNA
<213> Ctenocephalides felis

<400> 79

```
ttatttttgt gtgatttaat aaattctagt gaactgttca ttgctaaata tcacatagaa 60
atgtctataa ataacgataa aactaatttg gatttgttgg aagaagacga cgagtttgag 120
gagtttcctt gcgacgattg ggcatcacac gacgaagatg cagatgatgt tacagtttgg 180
gaagataatt gggatgatga taatgttaaa gatgacttca gccagcaatt aaggtctcaa 240
atgagcaatc ctaaagaggc atctaaaaaa agttaagact atgtatatat tagaatataa 300
tgtaatttca aaaaacataa ttaataaact gattttttaa atnttaaaaa aaaaaaaaaa 360
aaaaaaaaaa aaccctnggg gggggcccg gccccaattc nccctatngg gagtcgnttc 420
aattcactgn ccgngttttc acgtcgngac tgggaaaccc tgcgttaccc acttaatccc 480
ttgagcacat cccctttcc cagtgggnaa taggaaaagg ccccccgntc cccttccaca 540
ttgcncacct                                                         550
```

<210> 80
<211> 549
<212> DNA
<213> Ctenocephalides felis

<400> 80

```
gctatcagtc cacctaaaag caaaaccagc ccattaagtg tgtcttcaaa aggaagggcc 60
atagattttt caaatcagtt tgacgttggc gaaaagcaga aaacaaaaat agacgacatg 120
```

```
aatgacatga tgtcgacaaa aaacatcatc gccgataagg ataaaacgaa aatcgacagc 180
aaaggtcttg atgatgtaag catggatgat gacgatgacg acgatgtgat atcagcaggc 240
gacgtttcga aaagtaaatc agaacaatca ctggctcgaa aaccaatact gaccacaaat 300
gattcgccaa atatgcaagt gcattgtatc ttcaatggaa caacatataa gccaggacat 360
tcgttagata aacactgtga aggcatgtgc aaatgttccg aagaaggtct ttggagatgt 420
gagcccaggt gtgaagctct tatgtcacaa gactcctgat ggcaccctaa atgatgtcac 480
caccaaaaat gaaaggggtg ccgcgaaatg gccacccaac aannatgctg cctgtctggt 540
tggcancgt                                                          549
```

<210> 81
<211> 500
<212> DNA
<213> Ctenocephalides felis

<400> 81

```
actgttgctc ttcgcgaaat ccgtcgttat cagaaatcca ctgaattgtt gatccgaaaa 60
ttgccattcc aacgtttggt gagagaaatt gcccaggatt tcaagactga tctacgtttc 120
cagtcagctg ctattggtgc tctacaggaa gccagtgagg cttatctcgt tggcttattt 180
gaagatacaa atttgtgcgc cattcatgcc aagagggtaa caattatgcc taaagatatc 240
cagttagcgc ggcgaattcg tggtgaacgt gcttaaaatt cgggttatca agaagccaga 300
tatcccacat gcacatctcg atatttacct attataaata tacacatata tgtggaaatc 360
gtgacattta tgtttaagca ttcactttta taacaaatca tctttacatc ttangacgta 420
gtcaaaaatt tggtaacaat attttgcatt tgaatatgaa attttagctt taatcatatt 480
tatattatca tttttgtgta                                             500
```

<210> 82
<211> 238
<212> DNA
<213> Ctenocephalides felis

<400> 82

```
aaacaattcc gagattaacg gggctcgacc cggcgaaatc ggtgcttgcg tatcgagcgc 60
aataaaaaca ttatataaca caaacaatgc agattattcg gttaacgaaa ttataagtga 120
aaaaaagtca ttaggaaaca caaaaattaa acataaaatc aaacctagca ttagcaaaaa 180
tgccgaaaaa aatattaaaa aaaatactga cattattcca gaaatgttaa aatctggt   238
```

<210> 83
<211> 500
<212> DNA
<213> Ctenocephalides felis

<400> 83

```
acagggnaaa acacaattat gccatacatt agctgtaaat tgccaggtaa acatatttca 60
caaagaatta tcagtattat aattaactgc attatctata acaaaaatat cttatattcc 120
ataagaattt taaaatagat ttcattactt ttcaaataat tagttattag ttaaaatatt 180
ttctagattt attttaaata tatttttgtg ttaaattcaa taacatcaaa atattaatgt 240
tttctcacaa tctcgtaaca gaataattat taattaaata ttcttaaatt aattctttgc 300
agttacctat agatcaaaat ggaggtgaag gcaaatgttt atatatagac actgagggaa 360
catttcgacc tgacaggtta ttggctgttg ctgccgttac aaattatctg gtagcgatgt 420
gttagataat attgcatatg cacgagcata taatacagat catcaaacac aacttttaat 480
atatgctctg caatgatgtc                                             500
```

<210> 84

<211> 500
<212> DNA
<213> Ctenocephalides felis

<400> 84

```
actcgcggta tacgctcttg gtgtcagatt tttcttcgtg tttggcatgg accaatagtt 60
tattgtcgac tgtcttgaca acaatttctt ctggtgcata ttgactaaca tcaaacctca 120
atttcagaac tttgccatcg gcgtcctcac ggatcaatgg tgaattgagg tcgtccaacc 180
acgtgctgac ttgtctgcct ggtgcgccca agtttgagct ggacagcctt tggtcagtgg 240
aagtttgttg tgttgtgctg cttgtgcttt tgaagaagtt attactttct ctattcataa 300
gctcggatct gaatttagac atttcctctt ccattttctt catttcggca tcaaatcttt 360
ccctgatgct gctgaattct gtatcaatta cactgaaatc tccaagcttg attgggatat 420
cgngtttaaa tccactatca nncattttta taaaanttta tttaanattc acatcacaca 480
naattaatta attggtaaat                                             500
```

<210> 85
<211> 413
<212> DNA
<213> Ctenocephalides felis

<400> 85

```
tgcagactgc ttctaattct ttttgnttgn gttcgtattc ttccttatcg gccaattggt 60
tagcatccaa ccatttaata acatcattgc atttgtccat aattacagtc ttatcggttt 120
cggcaagttt atccttcaat ttttcatctt ccatggtgga tttcatgttg aagcaagtaa 180
gattctaacg agttcttagc agcaattgta gacttttgtt tctcatcttc atttctgtat 240
ttctcagcat cgttaaccat cctttcaatg tcttcttgct aagacgacct ttgtcatttg 300
taatggtgat tttattttcc ttgntggttg atttttcaat tgctgtgaca ttaagaatac 360
cgttagcatc aatatcgaaa gttacctcaa tttgtgggac accacgaggt gca        413
```

<210> 86
<211> 500
<212> DNA
<213> Ctenocephalides felis

<400> 86

```
actgcaatct tcatcagatt tcgttgcagt gtttgaaaga tttttgaatg ctgaatctgc 60
accagataga cctaacaaaa tgtatatcat atataagtat aagtttttga taaagatttg 120

tgatactata taactaattt atttaattta ggaagaggaa tttatgaaaa tattgaaaca 180
aaaaactatt ttttattcaa gcataatatt taattattaa ttagatcgat aattaacaaa 240
caatcaaaat agttccaatt caaatagaac taacctctta tctgcagtgt ctctgcacat 300
tttaaaagat cttgtagccc ttcttgtgta acatttactt ccccttata catgaaatca 360
accaacgctt gtaattccca aaacctaaca tctttcaaaa ttataattgg atgctggcaa 420
ggattttctc ccagtagact ttcaaaaaat ccagaacaag cggccaaaac taacctatga 480
caagttagtg acgccttcca                                             500
```

<210> 87

<211> 550
<212> DNA
<213> Ctenocephalides felis

<400> 87

```
atttttctga caacagactt taaaataaca tttaaactgt ccgattcaat tatttgataa 60
aaatgaagac aattatagtt ttcgcactaa ttaccattgc agcttgcaaa gggcaatcta 120
cttgccctaa ctttaacgat acacaacatg acactggcag aatcgctctt atgaagaaat 180
agttgatttg ccacagaaac cagaagtttt acttattgat gttcgtcaac cggaggaatt 240
ggagcaggaa ggaaaaattc cgacggctat aaacattcca ttacgtgaat tggaaaatgc 300
tctcaagaac atgtctcctg aagaattcaa aaccaaattc ggaagagata aaccaacatt 360
cgatactgaa atcattttta gttgccgttc cggaaaacga gcaaaggaag ctatggaaac 420
agcattggga ttgtntacaa gaaatcaaga tctacgaagg tagcttttta gaatgggcac 480
aagcagaaga acagtgaatt gcaggtaatc aatttttat gtcacattta ttacaattga 540
ccaactagtt                                                        550
```

<210> 88
<211> 549
<212> DNA
<213> Ctenocephalides felis

<400> 88

```
gatagagtgt tcgtctttgg tttaggaagt tacatataat tattcaagat gcctgaagat 60
acacaaaacg ctggtgatgt cgagacattc gccttccaag ctgaaattgc tcagcttatg 120
tctttaatta ttaatacatt ctactctaac aaagaaatct tcttgcgaga attgatctca 180
aattcatcag atgctttgga taaaatccgc tatgaatccc ttactgatgc atctcgcttg 240
gacagcggca aagatctcca catcaagatc attcctaata aaagtgaggg cacgctcacc 300
attattgaca caggcattgg catgacgaaa gctgatcttg tgaacaattt gggtacaatt 360
gcgaagtctg gaactaaagc cttcatggaa gccttacaag ctggagccga tattagtatg 420
attggtcaat ttggtgtggt tctactcggc tattcgtgtg ataaagtcac ggcacatcta 480
agcacaatga tgatgaacaa tcctttggaa tcttccgcag aggtccttac agtaagactg 540
cacttgacc                                                         549
```

<210> 89
<211> 549
<212> DNA
<213> Ctenocephalides felis

<400> 89

```
agtcgttcga gtctggttct gtaacaggcg gcaaaaagag aaacgcatga cgcccccgaa 60
cacgatgggc agcgacgtct ccgaaagtct cgtgtacaac gcggcctacg accagaactt 120
gggccacagc ctggtccaca agtacgagga catgcccggc ggaggcggag gcgcaaggca 180
cttggatgac acgggacgac acctggaaca cggcgtgtcg cacctgggcc acgaaatggc 240
caacaagttc gaagggcacg acatggaca gaacctggga cacaatttag gccacaatct 300
agttcataaa ttcgaagacg gcagcaggca catagagcag aacctcgagc ctagttattc 360
ggaagcgcag agtcctggca attagcatag gacgtgtaaa tacgtgagtt agggacataa 420
attcaagtga ctgattagtg actgaacgat gttataaatg acaacgtgag tgcgaaaaca 480
aaacgtcttg acgaagaaat atcgttaaag taaaaaaaaa acaaacaaat gaaataacca 540
ggggtaaaa                                                         549
```

<210> 90
<211> 549
<212> DNA
<213> Ctenocephalides felis

<400> 90

```
acaagttccc gaccggtcgc ttcgcaaaac ggattctaat taaaatttag tgttttaaag 60
caagtgttac gtgttcttgt gagtgtttat tgtgtgcaaa aatcgcgccg agatgtccga 120
caacaaagga gagaacgcca catcggaatc accgaagggc aaaccgggca gacgaaacag 180
agctttacaa agaatgaaag aagagggtga agcactaatt aagtctctag gtggaacccc 240
tgaaattgaa ggcagaaggc gtactaggtc ttcactaaaa acgccagcaa cacccccagt 300
tacgccccca actcccacga aaaaggcaaa atctacacca gcacccaaag gcacaaaggg 360
acgcgggagg ggaagaaaaa gtgaaaaggt ggaggaggcc gaagaaaagc aagaatcaac 420
agaacaagaa gacgaagtag acgaatccaa tgcacttact aaattcacct ccgaagacaa 480
ggccaagtag aacaaaatgt aaaacagaag accttcagaa gaaccgcaaa ctgatgactg 540
ttaatccga                                                         549
```

<210> 91
<211> 251
<212> DNA
<213> Ctenocephalides felis

<400> 91

```
actcttccca cagtgagaga aaaagtttaa gttctattca tcggtcgtct aaggaaacca 60
ctcatcagca gattgaaact caatctaatg cacgaaataa acaccaagta tcatcatcta 120
acgctaccta cgttattgaa cgcccgcaga aaaccgttcg acgcgataat ctgttaactg 180
gcggtgaatt ttatggtcaa aaagattcaa ggtatggtaa tttttctaat tgtgaacaaa 240
gtctaagaag t                                                      251
```

<210> 92
<211> 375
<212> DNA
<213> Ctenocephalides felis

<400> 92

```
actccctttg cgccatgaag atttcaggcg catttcgagt atcaagtccg ccttccatgt 60
ccagaaggtc ctggtgttcg tcctgttcgt ctgattcgga tgagtcgtcc agttcgtctc 120
cgtctaatga ggacggacaa cggaacatat gctcaccgtc gcacatgcat tgctgagccg 180
cctggtagtc gcggctgaag gctgcggtgt tctcgaagtt ctcggtgcaa ccatgctctt 240
ctgttaatcc aacaggacag ggattaggtg gattacaata agcgggcaaa ttatccgttt 300
ttacttgctg atgattcatt gcacttncat ctggtttgag cctttgttgg ccttcaccag 360
cgcctncact tacgt                                                  375
```

<210> 93
<211> 550
<212> DNA
<213> Ctenocephalides felis

<400> 93

```
gcaaaagcaa tgactgtatc aaatggcaat ttagtagatg ttagattttt tggagcacat 60
gataaagcat ggatacctat gaaggattgc ttgctttata gtgagaaaga tccaaatttc 120
agtgataaag gaaaacgctc tgatttcatc gaatcactta gggagttagc tatatatgtg 180
aaaaatcttg agcaaaaatt tggaaaattt tgtcatgcac cattcaaaac tccatatcct 240
aatgatcaag cagctattta tagtataatg ttaccttcat ataaattcaa atcagatatt 300
gcaaataaaa aaataataac aaaacaaaaa gtttgtgaca taacggataa actactagag 360
gacacaaaag gtaacatgaa aataaataat tcgttagatg aggatagtta tattgaagga 420
tatgatactg aagatgagga agcactaaaa gatgtatcaa atgaatctgt gatatgtaat 480
gatatgaaaa ttaaacaaac ccttgcgtgc cgtgttagaa tgagagnggt aaataattca 540
caggaaaata                                                        550
```

<210> 94
<211> 549
<212> DNA
<213> Ctenocephalides felis

<400> 94

```
aacacaccaa gagtacaaca ttcgtcgtgc tggatatttg acattattgt atacaaatat 60
agcggcacac gatcgtattt attcgcgaag ttttgacaca ttcagcagat tttggatac 120
atacagcaat tcaggagtta tccgctgagg aaatcaattt gagcgcaatt agacgatcag 180
atcgtatacg aatcaaaaat caattaattg gcagtgattt tgtcaggcca ttttagttaa 240
cggcgcgcgt ttgttatact tttaactttg aaagttttct ccatcgatag ttttcgttaa 300
tcaacgtgag gaaaagttct aatcaagatg gcagtagcag cagcacaaaa gaaccgcgaa 360
atgttcgcta tcaagaaatc ctacagtatc gagaacgggt atccatccag gcgccggtcg 420
ctggtcgacg acgcgcgttt cgagaccctc gtagtcaaac agaccaaaca gtctgtcttg 480
aagaagccgc cagagagcac gattcgagtc tcgatggcaa gaccatggac agatgtatat 540
gatgacaag                                                         549
```

<210> 95
<211> 240
<212> DNA
<213> Ctenocephalides felis

<400> 95

acttgtaaca attgagatgt taaacgcaat gaacagtttg tctgaaaatc aatctcttct 60
tgctatgccc ccatggcaaa acatgtggtt ggtaggatcc atggctctct ccttcactct 120
tcacttcgtc attttacatg tagaagtttt atcagctgtt ttccaagtaa ctccattgtc 180
tcctgatgaa tggatcactg tgatgaaatt ctctattcct gttatattgc ttgatgaagt 240

<210> 96
<211> 431
<212> DNA
<213> Ctenocephalides felis

<400> 96

acaataggaa gacatagatt tacgatcgcg agagaattcg agagtgaact ctttttttcca 60
tttggtttca atttcctggc ggacggcgat ggcagcagaa cgacggtcta gtccttgttt 120
agaaacattg aatggattca tttttcagc caatacaatc aaggcggttt cggtagcttc 180
accgactttt tcgaaagctt gtttgaattc gttgaaatca atagcggaat cattgcacat 240
gatacagatt gttcctaatt catgcagggt gtcaaattcg ctggccttaa ctttagcacc 300
tttcaagtat acatcaccaa ttggttcata agtggatcca gtgatttcga attcagtgaa 360
actgctatcg ctaccttcaa ttttgtcaaa gacaaacata cgagatcaga catctgatta 420
gtggtcaaag t 431

<210> 97
<211> 500
<212> DNA
<213> Ctenocephalides felis

<400> 97

actgcgagac actaacaaaa ctcaaaaaag ccattcaaaa tcgccggaga agaagaccga 60
gcaagggcgt ggcggcgaag ttcttcgaag cgggttttca aaagtgaaaa actctttaga 120
aaaaatgcga tgaaagatct ggcgactatg tagaaaaata atttgaaata caagctttcc 180
gatggtatac acatttattg caataaaaat gcctcaaccc tgaaaaaaat gtgggaacct 240
tactttttgc atgaccctcg tatgatttca aaaaattgag aatgtctatc gatttgttac 300
ttagacgtgt cctttgttta caatatttcc caaaatagaa aatactctct tacttgcgac 360
cgaacttgat tgttgttaaa gcctgatata ctaattctat atacactggt ataaaaccta 420
aattaatata tacattagac aaaggataac acaaaacttt tctggaagcc atagcagact 480
gataagattc caaatgctga 500

<210> 98
<211> 350
<212> DNA
<213> Ctenocephalides felis

<400> 98

```
acgcgtaata ataaaaagag agaaaatatt taaaataaaa atatttaaat ttaaaaaaga 60
aaagaaaaac nttatcaaaa ttattaaaac caattcatta ttgcatcatc ttacaaaaag 120
acccgaaaat acacgacgtt aaaatatgaa ataagaaaaa aaaactctac taatactacc 180
aaccaattac tctataacta tttattatac aacaatccag ctggtcagat atgaatagca 240
tgcctatgtt tcaactattt gaaaaaaagt aagtccttgt taaactaaca aatccgattc 300
ggcactgtct gcctggctca aaaaacttcg gctctctgca cgcgtttcgt           350
```

<210> 99
<211> 200
<212> DNA
<213> Ctenocephalides felis

<400> 99

```
actgctgtag ctaaatctgc ttctgaaatg gtgttagctg atgataactt ctcttctatt 60
gtcgctgctg ttgaagaagg tcgcgctatt tataacaaca tgaaacaatt catccgatac 120
ttgatttctt ccaacgttgg tgaggttgtt tcaatcttct tgactgcggc tcttggtctt 180
cctgaagctt taatccctgt                                             200
```

<210> 100
<211> 273
<212> DNA
<213> Ctenocephalides felis

<400> 100

```
catcttacat ctaccgggac atcatagcta caaaaaattt cttnangata acaaacaatt 60
attagtgtgt aaggcagata aaggaaacat ttcagtggtt ttgttaaaag aggaatatga 120
taaagaagca aagaaaattc tcagtgatga atctctatat gaagaattgg catcggatcc 180
gaacaactat caccatagac aagtttttaa atttataaat ttattagaga aaaagggaca 240
tattaataat accatatcat taaagttaaa agt                              273
```

<210> 101
<211> 550
<212> DNA
<213> Ctenocephalides felis

<400> 101

```
aagaatatga acaaatagta gtagcaccag ttcaccctgg tttagatgta caggaagcac 60
aactaaatga agataatgag gatttcgcat caaggcgtcg ataccatcaa tcagctactg 120
tgcatggaca ttacgtaaac attgacggat agttgtttta attaatgatc acctaataca 180
tatttgacca gtattgcaaa tttttgagtc acaaagctat tgatttagat ttttatatat 240
ccttataaaa gctatttcta tggtataatt tatttaattt aacaaaaatt tgcaatatta 300
```

```
gcttgtattt taaaaagctg attaaaattt attgtgaagt atctaattta ttaaaaaaaa 360
tctaatataa tgaataatat agaaatgaat gaaaaccgac tcgagtgcag tcaacattac 420
tgataatgtg atttgatgca ttttgcttta ttaaaggcta aattagttca aaaaggccag 480
tgtttaattt aatatttatc acttatttaa ttcaaataat taatcactcc agttgtatta 540
taaataatgt                                                        550
```

<210> 102
<211> 550
<212> DNA
<213> Ctenocephalides felis

<400> 102

```
agcaaccgac gctggcgtcg tttgaacaag acggttttaa acgattttta tattcaccta 60
aagatttcta taaaccattc tatgaatggt tagtaaagca accacattcc aaaattcaag 120
tacagccgaa cgaagtttga tttgttaatt agtgtttttg tgcacattga ttggattgaa 180
aagaacgccg ccaagagcgt aagaagcaat catggaggac gcacatgcga aatccgtgga 240
cgaagtctta ggatatttca gtacagatcc tgaaagggga ttgtctactg atcaaattaa 300
aaggaatcaa gctaaatatg gacctaatga acttccaacg gaagagggta aatccatctg 360
gcaattagta cttgaacaat tcgatgatct tctagttaaa attttactgt tagctgctat 420
tatatctttc gttctcgccc tttttgaaga gcacgaagat tctttcactg cttcgttgaa 480
cctttcgtca ttntactgat ttgatcgcta cgctatgncg gtgttggcan gaaagaacgc 540
tgaatcgcta                                                        550
```

<210> 103
<211> 549
<212> DNA
<213> Ctenocephalides felis

<400> 103

```
gcttctattc ttaaggttta aattcagatc aattctatat ttaaatttca gaacaatctt 60
gagcctttat catgccttct gatgccaaaa aacgtgaaca gcaacgcaag aaggagcaag 120
cgaaagccag acaggctggc aaaaaagttg caactaaaaa tggtgaagaa aatgacaaag 180
aacaatctcc agcccccaac caaactaatg gagtgaaaag taatggaact acagagctct 240
ctgcagaaga aattctttgt gcgaaactgg aagcagaagc aaaattgaat tctgatgcca 300
ggtcttgcac aggatcttta gctgtccatc cacgctcaag ggatattaaa atagctaatt 360
tttctgtaac tttctatggt tgtgaattgc ttcaagacac tcttttagaa ttaaattgtg 420
ggaggagatt ggtcttttag gccttaatgg aagcggcaaa tcatcactat tgctgtcttg 480
gtatcgtgaa gtaccaattc tgacatatag acatctttca ttaactagag aaatgctgta 540
ggntaattg                                                         549
```

<210> 104
<211> 500
<212> DNA
<213> Ctenocephalides felis

<400> 104

acacaaccaa actgttaatt ccctgcagcg cctgctgagg gacgccagag gttctataac 60
tttcaaaatc gttccatcat acagaagtgc accgcctcct tgtgaggtaa gcgccgcatc 120
tgatgcaatg cgtttattcc ggattaggcc tacacctgna ttagtatttg ngcgtgccca 180
gtttgactat gatcctttgg aagatgattt aataccatgt gctcaagctg gtatttcctt 240
caaagttggg gatatattgc agattatcaa taaggatgac tatcactggt ggcaagcaag 300
aaaggatgct gtcgaagggt ctgctggatt aataccttct cctgaacttc aagagtggcg 360
aatagcaaat gcggctcttg aaaagaataa gaacgaacaa gttaattgct ctatatttgg 420
aaaaaagaat taaaaatgcc gagataaata tcttgcaaaa catatgctgt tttgatggat 480
ggatcttgnt acctatgagg 500

<210> 105
<211> 248
<212> DNA
<213> Ctenocephalides felis

<400> 105

accaagtaac ttttaccttc ttgtcagaag ttttcaaact gcacataaac tctgccgatt 60
taccttcttc gatagtaaca tcaacaagtc ctttaacaat ttgtggtttt tcgccttttt 120
ctggttcagg atccggtaaa tctaagatcg ttacttcttg tgatgttgcc tcgccctttt 180
cattctttac aaccaatttg taagtacctg cccnctncnc ttcncnnanc anngntgcca 240
tnantcan 248

<210> 106
<211> 494
<212> DNA
<213> Ctenocephalides felis

<400> 106

accgtcgtct tgtaaatgct gtgaatgata ttgagaagcg tattcctttc tctcaccacg 60
atagattagg tttcctcact ttctgtccca ctaacttggg aactactgtc cgtgcctctg 120
tgcacatcaa ggtccctaag ttggctgcta accgcgccaa gttggaagaa gttgctggac 180
gttacaatct ccaagttcgt ggaactcgtg gtgaacacac tgaagctgaa ggtggtgtct 240
acgatatctc caacaagagg cgcatgggcc tgactgaata ccaagctgtc aaggagatgc 300
acgatggcat tgctgaactc attaagatgg agaaagaaat gtaaacattt cacttttatc 360
atcagactat tttttgtgat caaataaatg gtcatgcgat agatattggc aaagattcta 420
tcagtatttc tattttaaat aataatttat attatattgn tacaactttt tttaataatt 480
taatttttat ttgt 494

<210> 107
<211> 445
<212> DNA
<213> Ctenocephalides felis

<400> 107

acgtaaacat ccatacattt agggcagtag gactttacca tagcctctcc aggcacatca 60
gaaagaccta atggcagcat aggctgacta tcgcagtaaa ctctagggca atatccgaag 120
tctccagatt ggtatttttc tatcatttga gctatacctc tatttgttaa aatatatctg 180
gcgtgaatta gaccatataa catctctgca gcctgttcta ttgcatctga ctgatttgga 240
ttatcatcta tttcatcatc aggttctaaa tctaatatca tatccaaggc ttgtctatat 300
cgtggaattt gctcattaag gcctgttaga ttgaatttat cctgtatata gtcttcatcc 360
acctcgcaaa agaattcatt tcctcgtaga tcacaaaacc aagatatcca tgagacctcc 420
tcagaactgc tcattttcct tttgg 445

<210> 108
<211> 500
<212> DNA
<213> Ctenocephalides felis

<400> 108

acaatcgggc acaaccaaag cataatacaa caaactttca tcgtaataat taaatgatta 60
tttctatcac ttaccgaggc atttttcgca taaaacagag tgtgtccttt cagcctgaaa 120
tatcgccttc tccatcgctg taaggaccag gtctgcttca tgagaaatcc ctctctggtt 180
gcggcctgcc ggaaattaac agaaatcctt gtaatacaga aatgtgacaa gaatatttaa 240
attggagttg acagtatcgt aatgaaatat cttgggcatg aaaagcattc gaagatagtc 300
tataatgggt ttaattcaat cagtttcaaa aaataagaat cttgatatcg atataaaatg 360
acaaaagctc tactaagatt ttttcttcag tataaaaaaa taatataaaa ttgttagaat 420
actttacata tatcgcataa ttcttaacac aatataaaaa tgaataatcc aaggnaacga 480
acgtatttaa tctcttacac 500

<210> 109
<211> 343
<212> DNA
<213> Ctenocephalides felis

<400> 109

caaaantgcg tctgagggac tgnntcttaa tttattcata atatcaaaat agttattnat 60
ttgcaaattg ntggtaattc actcgcgttt gtttatttat aattatatct ggntttcttc 120
tcgatttgct atttaacctn tagcagcctt ttgcactttt gctgntggta ataatttcct 180
atccgtaaca acacaatggg aaactgggaa taatccgtcc ttaagcacac atacgcaatc 240
actgtgcatt tcagttctac taatttggtc taccaaactg tttgatacta cactgatgaa 300
gttcttgttt tgcttttggg ctttagcgtg ttggaagntc tgt 343

<210> 110
<211> 491
<212> DNA
<213> Ctenocephalides felis

<400> 110

actcctatcg tttgtaacat ttacattccc ctttacttta ttattttcat ctcctgctac 60

gatctgcgat tcatttgaat cttcaatatt taaattcgct ggagcatttt cgatttcttc 120
aagagccgtc aacgctcgca gttcttcttc tatcaatttc tttctagcag ctattgcttc 180
aaccgagctt atactttccc tttttcaca ctcgccgctt ttccttgaac gaaaactgcg 240
tctctttcga ctacccgagc ctcttttcac ggttctggaa atcccatcca aatttcttaa 300
aaattcctcc tgaggcgttg caccaggtgt agaccaggtt gaccaggatt tgacgtcctc 360
ctcttcgtct ccggaatctt ccacaacttc cgcctttttt agcttttctc ggacactcct 420
cgtgtaatcg gacaagtccg gacttgctcg accgcaaatc gaaccaacac acagacaact 480
cgcctttcaa c 491

<210> 111
<211> 500
<212> DNA
<213> Ctenocephalides felis

<400> 111

acggaaccca tgggaatgta aacaaaattt atacttattg ttaaaatctc tctaaaatat 60
ctgataattt gatgtgcata tgtctaatga tcatttttga ccgccaaatg gcacattttt 120
ttactttttc ctccatatct ccaaagtcgt tggacctttc caaaattttg aacagttctt 180
catttagtca atacaaataa aatgtttttt aaattattca aatcggacgc tccgttctct 240
taaaaactga gttaccgttt tcggcacttt ttgccccgta tcttcggaac ggctagacct 300
acctttgcca aaaactaatc agcacgtctt cttatcaata tgaatcgaat gttttttaaa 360
ttattcaaat cggttgattc gtgttcccga aatcgtcgac gaaaatttgt atccgcacat 420
acatacatac acacacatac acacacacac acacacacat ccatncattt ttctaaggat 480
gccaaaatgt cagaaacctt 500

<210> 112
<211> 500
<212> DNA
<213> Ctenocephalides felis

<400> 112

acggtaaaca atgtatccaa gattcttata catatttatt gctacttcat tactaactcg 60
aacaaacagg tcaacaaaat atgtattttt tctttctgat acatcttcta agaaattcat 120
taatttagct gctaagccaa gtctacgaaa atctggggaa acagttaggg ctgtgacatg 180
tccatgccaa ttttctccat gaccttctgc tttgcccatt atatatccca ttatttctcc 240
gtttggggac tcagcaactt gaaaatactc tggccaatgc gctagatatt gcatgtagaa 300
tgaaagtcca tatgtttctg ttagtggatc taaatttaca ttattaaaat taaacatatc 360
attgcaagta aacggtctta atgtagtcat attaattnta ttgcaaaaga tatcagaagg 420
aatttaatta acaaaagtca cgcctttaat gaagtaaaat attcagagaa aaaataaaac 480
gagttactaa tatttctact 500

<210> 113
<211> 256
<212> DNA
<213> Ctenocephalides felis

<400> 113

cgatcatttt caaatctaga taatcgcaca cattggtgaa acttcacatc ctcaagttcc 60
acagatnttg acttacccct gcctgtgctt tcaaatagaa ctttatcatt taagcctaat 120
cttaattctg gcattcctga tagatatact ctcattttga tagcaccgac aatttcactt 180
cttagaacat tgccatttac atttgccaaa agatttacgg attctattac atctaaaaat 240
acttcatttt ttcggt 256

<210> 114
<211> 550
<212> DNA
<213> Ctenocephalides felis

<400> 114

ataaggataa aaagaaaagg aaaaaggaga agaaagagaa gaaattgaaa aaattaaaga 60
aaaaattgaa gaaagaaaaa ctcaagaata agaaaacaga agattcagac aaaaagaata 120
aagatacgaa aagtctcaaa aatgtggtat cctcatcctc ttcatcatct tcggaaagct 180
ctgattcaga tgatgagaaa tcatgtatcg gtccagtgca aaaacaaagt gctggtctta 240
gtcataagga ctttggacat gcattgttac ctggtgaagg agctgctatg gctgcatttg 300
ttgctgaggg aaaacgtata cctagacgtg gtgaaattgg tctcacgtct gatgaaatcg 360
cgcagtatga aagcgtcggt tatgttatga gtggaagcag gcatcgtcgt atggaagctg 420
tcgtatccgt aaagaaaatc aaatttattc cgcggatgaa aaacgtgctc ttgctatgtt 480
cagtaaagaa gaaagacaaa acgtgagcat aggatcttag ncagttaaag agatattaat 540
tcaagtatcc 550

<210> 115
<211> 549
<212> DNA
<213> Ctenocephalides felis

<400> 115

attnagagta gcagcttcaa agatcaggcg ttncacagaa ggtacttcga cgtcttggaa 60
gatccagaag ccaaatcagg agtagtgcga gcgcgcaatt caccagtcat tttgaccaaa 120
gtgggagtct tcaaagagta gacgaagggc ataccagttt ccaaggggaa ggcaatggtg 180
ataacatcgc ggttgtacaa tttagtgaag tcgaaagatt ttccttgacg gagagattcg 240
gcggcggagc ggatggcttt gggcaaagcg ttcaaggttt ggttgtcgaa agcaaagaat 300
ctcttggtgt tcaacatgcg agtggaaatg acagcttcca atttttcagc ttcttcaggt 360
ttcatgttca acattttggc gatttttca acagaccatt tgtcttcaga agagtggtcg 420
ctttcgtgtt cagattcgcg gttcttgaat tgagcagaaa ctacttcaac caatctttca 480
acactggaga ccatggcgta gacatcggag tatctttgtt tgtatccggc gaagtcagct 540
tcaagcttg 549

<210> 116
<211> 549
<212> DNA
<213> Ctenocephalides felis

<400> 116

gcgaagagag ttagatcact gtagaagaca gatttcagaa cagtccggtc aaatatctcg 60
tttacaatct gagttaagta ttagccaaaa aaatgaagga caatatacaa caaaattagc 120
tacagctttg gaaacagttg aaaaaaatat ggcacggagc aataaaaggg caatagatgc 180
tgaaagtaca gttgcaaagc ttaagaaaca gatttcacaa atgacgtcag agatgatggt 240
tcttcgaaat gaaaatacat cactgcgcta tggtccagct gcaaatgatt ccaatagcat 300
gatgagatta tcaaatgagt tgcgaactgc agctagtact gcagagtcgt cactgaggca 360
actattaacg ggtgttgata atttaaggac tcttgtagtt ctttagaaag ctctaaccga 420
atatttgaac cttctgatga caatttctgc gaaaatgaag atgaagatgc cggcctgact 480
ataatgtgta gtgaataaat ttntcattca aatgtcttgt attaaaataa atattctagt 540
ttatatgct 549

<210> 117
<211> 549
<212> DNA
<213> Ctenocephalides felis

<400> 117

gaaatataga gaagttacta agaaaactat ggttaaacag tttgaatgtg ttaaggatca 60
cccgacgcca aaagctgtgt tctttacttg tatggacagt agaatgatac ctacaagatt 120
tacagaaact aatgttggtg atatgtttgt tgttcgaaat gctggtaatt tggtacctca 180
ttcccaacat tttttggatg aatatataag tgctgaacct gctgctttag agttaggttg 240
tgtagtaaat gacattcggc atataattgt ttgcggtcac agcgattgta aagcaatgaa 300
cctgctatat aaacttcagg ataatgcttt cgcttctcag gataatagga gaatatcacc 360
actacgagca tggttatgtt cgcatgccca aagcagtctg gataaatttc aacaacttgc 420
tcttagtgat tataaaacac ctcttatttt tactgctgag actcctttaa gaaaatttgt 480
ggttatatag atcctgaaga tagttttctg tgaagacaaa tatcacaggt aaatctctgc 540
acaactcaa 549

<210> 118
<211> 549
<212> DNA
<213> Ctenocephalides felis

<400> 118

cacaacgttg agttatcctt gtaattgcaa gtgcatttaa aaatataatt tgtttggcat 60
agaatttgtg aaaaaacagg agcataaata tgctttcaaa cattcggatt caaataaaaa 120
ttgcccgtgt acgagaatta aaagatctgc gttatttttc ttcttctaca acaaatttcg 180
ctgaacacaa atgtcgggta ttggtggtcg gaggcggctc tggaggatgc accatggcat 240
caaaactttg ttcacatttg cgacaagatg atgtcattgt attagagcct agtgatgtcc 300
attattatca gccaatgttc acaatgattg gaggtggcat gaaaactttg gaacaatctc 360
gaaggccaat gtcttcagtt ttacccaaga aggctcgctg gctaaaagat tcggcgaaaa 420
cattcaaacc gatcgaaaat tcagttttaa catcatctgg tgacaagata acatacacta 480
tctcgtgtgc tgtggttgaa acttnttacg caagatcctg gttgtaaaag ctttgctcac 540

caaatggca 549

<210> 119

<211> 549
<212> DNA
<213> Ctenocephalides felis

<400> 119

```
aatatgtgtt tactatttaa tgatactgta agaaaaaacg atacgtacgt aattctgaca 60
ttgactgtca ttaataatgg gatatgactt tcgaggttat tattgagaaa tgaaaacgta 120
gcatttactt atagtacgtt atatggtttt tgattggttt aaataaacta aataaaatga 180
agtgcaacaa taacgatacg aatggccatt caaaaaggca tttggaagcg aagatctccg 240
aagctgaaga ggtaatagaa aaatctttat ctcaatgtga ggcagatgaa atttttatgt 300
ctttcaatgg gggcaaggat tgcactgttt tattacatat actccaaaga gtttataaac 360
ttaaatatgg ttctgatgca cctccattac tctgtttata tgtgagacct aatgatcctt 420
ttcctgagat agaaagtttt gtgctgagtg caaaaatctg tccccataaa tctaatacat 480
ttnctcttcc ttaaaactgc tttaaaagat ctgcagtgcg nacctcactc aaactagttc 540
atggatncg                                                         549
```

<210> 120
<211> 549
<212> DNA
<213> Ctenocephalides felis

<400> 120

```
gactaccaaa tggtgttcaa gctcaatgtc gtgcctttgt tgatacatat ggagatgcct 60
ttattgccat atttgtgcaa gaattagatc catcgcaggt gtgtcccaag ttatctttat 120
gtccaaataa agaagttgaa gtgtttgaac aagaagatac cagagacaaa ccaacttgtc 180
ctatgtgtct aatggctatg tttgaattag aagagaagct taaggaggat aaaaccaagg 240
cagctgttga gcaagcacta tctggattat gcaatcatct ttctgatcat ctgaaacctg 300
cttgcttaac cttagtaaat acttattata atcaattagt tgaaatgctg atggctgatt 360
ttaaaccaca agaaatttgc gtgtacctaa gattatgcca tgataagaat cccgacttaa 420
gtgaattgga tattcttgtg cagtaccaat taataagacc catgattaat gcaaatgaaa 480
tattggacac cacaattaat ggcaaaccat caggtgtgtc aaacaactta ttctgaaaat 540
tttgccaac                                                         549
```

<210> 121
<211> 549
<212> DNA
<213> Ctenocephalides felis

<400> 121

```
gcataatgat aatccacatg tagaaaaaca aattgttgag ttaatatatg atgccgtact 60
atttgaaaac ttgtcaaaaa attataaaaa taccgcattg tggcaagaaa tttttgtttt 120
gccattctac tccctatata aggagaattt gataaaccta atagacattt tacatataga 180
```

tacaagatat gcaattgaaa ataaattaca ccaagcagcc agggcaaagg cactgtattg 240
gactcatgca ctttattatt ttgagaagga gttaaagtac tcgtcaaatt ctccaacgtc 300
actaagtttt gatgtagaaa aattatataa aagggttctt tgtaaagaga attcagatac 360
aaccgatctt ggctggatac atatcacaca gatattcaaa aatttacccg cagaatgcat 420
atctgtaaaa tttgatggca aaatgattca tggataagtt ccgctggaac caactgtgcc 480
gccagatgta tgtcagagtc cgaattacgc gatgattctg tactcnccgt cgagataatg 540
atgtctctg 549

<210> 122
<211> 339
<212> DNA
<213> Ctenocephalides felis

<400> 122

accatggctg atacttcaat tccggcatca ggatggtatt ttccatcttt catcactcca 60
agtttcttgn aaacgcaatt gatcatgcat tttcctgctt ttgaatctgg tatgttcttc 120
tgcaataatt tttcgatatc atctgaagat gctcctgttt ctacagcaca atctttgcct 180
atctgcaaaa gtttttcctt tgcttcttct ttggtatagc tctgccgcag aatataaagc 240
gaccagtgtt ccaatcacca agaatatctt cattttgatt ttcttctact tcaaatttat 300
aaaacaattt gnttataatt ttcaaatgta gtatttact 339

<210> 123
<211> 500
<212> DNA
<213> Ctenocephalides felis

<400> 123

cccgcacacc acacatacat caatttttc tgtgggtgcc aaatattcag aaaactcaaa 60
aacgtaaaga tatataaaat ttttcatttt cgattttttg cgattactat aacttgcccc 120
atgggaagtt aataatattg tgtagttatt tatgatcaat actaatatta tgtgtagtaa 180
tccataatca agacttttta tcattttaa ctttccgtgg ggcaagttat agttatcgca 240
aaataatcga aaatgaaaat ttgatatatc tttacgtttc aaaattttct gaatattttg 300
gtataccttaa gaaaaaatgg atgtatgtgt gtatatgtgt atgtgtattt ttttatagag 360
gtgcgaaaaa aaatctccat aagcagacaa cgctagacgg tagtgtgaag atttttgcga 420
cgccttcttc ttactactgg ccctttctnc ggtttattct tcaaaggtgc tgtgcatatg 480
ctgctaagct gccagtttct 500

<210> 124
<211> 489
<212> DNA
<213> Ctenocephalides felis

<400> 124

acttaaacta tgagctattc tgtaactcat tttttttatc tttattttgt aaaccatctt 60
gntcataaaa tttgctaact acgcatttac tttttttttg acctatatat tttatctaaa 120

tactattcct aactctattc tatctgggcg tgcattaatt gtataaaaga gaaaaaaaaa 180
acagtctaaa caagtaattt ctattatata ataaatctcg caaaagaata aagaaacaac 240
agatgtaata cataataatt atatcaaaaa tagtcttact gttattataa aacatttgta 300
aagcgaatcg ggcattgaat ttaaaaaaaa gaacaattat taaataatct cactatttaa 360
cctatgtgtt atacggtaac tcgccatttt tttttctcgc tttactttca ctttgcaaac 420
tatctctttc ctaaatccta gcgacttatg cattcttcgc tttcgcttat ttcatttatt 480
acatctagt 489

<210> 125
<211> 549
<212> DNA
<213> Ctenocephalides felis

<400> 125

gattagtttg tagttaataa ttattaaaat gaacgactac ttatcttctc ctttaggatc 60
agattctagt aatttagatg tgccagctcc tattaaaact ggaaagactg catatcaact 120
ttctccgtat ctaaattata atcctgttta cctgcctgca agccaacccg aatttatttt 180
ccctgaaggg gctagtagac aaaggggtcg ttttgaatta gccttttcgc aaattggatc 240
atcatgtatg ataggagctg ctttgggagg catggctggt acatacaatg gtctgaaggc 300
tacaacattg ttgggtcaaa ctggaaagct gcgaagaaca caaatgttaa atcacattat 360
gaaacaaggt tcagctacgg caaatacact aggaacgata gcagttatgt attctggact 420
tggtgtgcta ctgcatggct tcgaggagaa gatgacgaaa taaatacttt aggtgctgca 480
cagcncagga cacttataaa tcacagctga cttaggaatg tncattggtg gaggagtaga 540
tttctatac 549

<210> 126
<211> 549
<212> DNA
<213> Ctenocephalides felis

<400> 126

ccggataaaa gatatttatt acaatggatg attcctcgcc agatttccat ttcaccacga 60
tcgattattg tgtttttggc ctggccctgt tcgtttcggc cctgacaggg ttgtactacg 120
gatgtaggaa atcaggggat gaatctgatg aaaacaatca gcagaacagc aataaaagaa 180
cagaagagtt tctaaatgga aactccaatt ttaggcctct gccagtggct gcctctcttg 240
tcgccagtta tgtgtctggt gtcacgattt tgggaacacc ttcggagata tttcggtacg 300
gaacccaata ttggataata gtgttgccaa tcgctctgat gagcctagtg gtggccaatg 360
tttttcttcc gatgttctgc aagttgcaag ttcagagttc ctatgagtat ttagaaatga 420
gatttaatcc gtggtgagga cgatagcatc cgtcatgttt gtcatagacg agctttgttc 480
taccaattgt atatatgtgc cagctttagc ttttaatcaa gtcaccggtg cgacgtcatg 540
gattgtacc 549

<210> 127
<211> 549
<212> DNA
<213> Ctenocephalides felis

<400> 127

```
agattttaag gaacctttac aacctgcgaa aactgtcaat attcagatcg acgcggtatt 60
cagtaaagtt ttagtacctt atccatcaag cataattcaa ctagagcgtc aacttgtact 120
ctatcatgga aatcattact tttactctgc ctatcgtact attaaacaac aaacaactgt 180
acaacttgct tctaagaaca tcgaaagctt ctctaaactg aaaccatttt cacaaagtga 240
taccacaata acttatggct catatgaaaa cataccagca tttactcatg acaaaatgac 300
gattcattat gagaatcata caccgttttt aacagttaca aaattggaaa gaacaattga 360
agtatcacat tggggaaata ttgcggttga agaaacaatt gacatggtgc attctggtgc 420
attactgaaa ggtcgtttcc agatatgaat tcaaaaagat tcacgaacgg ttggaagtgt 480
aaatcatata aaacttgctt ccagcttctg attggggtac tccggatcta tggaatattc 540
tcatcaata                                                         549
```

<210> 128
<211> 307
<212> DNA
<213> Ctenocephalides felis

<400> 128

```
accattgtgg atgaggcaaa tcccatatac catgatgaag tttgcttgtt tcgaaagaac 60
agttgaattg ttatacactc atgtggttcc caaacccaga gcagagtgca ctaaaggtga 120
acaattggtt gtcacctttg ctgctggtta cattgccggt gtattctgtg cagtagtttc 180
tcatcctgca gacacagttg tttccaagct aaatcaagac aaaggagcaa cagccattga 240
cgctgccaaa aaacttggct ttgctggttt atggaaggga ttaggaccta ggatcatcat 300
gattggt                                                           307
```

<210> 129
<211> 440
<212> DNA
<213> Ctenocephalides felis

<400> 129

```
nctcctggcc aagaaacaag attggattgc attncatgca aatgcgccag cgatggaaca 60
gggtactttt gcacccgcca agcatgtgct ccagttcacc atcataaacg atcagctgaa 120
gaagtgaaag aagtaaccac aacttctttg gcaacgactc catgcactcc tggagagaag 180
actcaaattg attgtaatac ttgcacctgt gccagggatg gctctggata cgcctgcact 240
cgcaaaatgt gtttgccagc cacccacgat cgtcgacgta gagaagctga aactgaagaa 300
gtcaaagaag tcacaactga tactttggca accactccat gcaaagcagg agagcaaaga 360
caagtggact gcaatacctg cacctgtgcc gcggatggaa ctggatatca atgcacccgn 420
caagcttgtg agtttgcagt                                             440
```

<210> 130
<211> 500
<212> DNA
<213> Ctenocephalides felis

<400> 130

```
acatatgatg ctacatccat tttgacagca tttcgctgga ccccgacatt gagcgtctgt 60
atggcattct cgagaacatc tatctttctc atgtccagca acaaaactga ctggacaaga 120
tcctggtttg ttttctaatg aaataattat taaaatagat ttattcttct acatttattc 180
taaataaaaa tatttttatt ttaataaaga catagcctat ggaacgaaac ttaccgagcc 240
gacattctcc aatgaaaact gttctaccgg aaacttcatc tcgttgcaag tctattccgc 300
atgaacttcc ttcgtgacag cggatatcag aacaaggatc aaagcatgag cattgaacac 360
aattattttt atccacagat ttatgaattc cgtaaggaca acgtaattct tcacacgatc 420
gatcgtcatt gatgcaattg ttaataatat tctctaaatg aaaaaaacaa attagcaatc 480
attaaatctt cttaaaaata                                             500
```

<210> 131
<211> 376
<212> DNA
<213> Ctenocephalides felis

<400> 131

```
actaattgtg gccccgccaa tttcaaaaat gttgatttag tttgagctgc acatgcacga 60
gccaataatg tttttcctgt cccgggtggc ccataaagta aaacgccttt gggtggatgt 120
atccctaaat ttatgaactt ctctttgtgc gtcattggca atacaaccgc ttcaattagt 180
tcttggattt gcttgtctaa gccaccaatg tcagagtatt gttctgtagg ccgctcatca 240
acctccattg ctttgactcg agcatcatat tcagcaggta atgtttccaa aattaaatat 300
gagtctttgt tgacacctac taaatctcca ggcttcaatt gttctggatc tactaaacct 360
ataacaggta gaaagt                                                 376
```

<210> 132
<211> 500
<212> DNA
<213> Ctenocephalides felis

<400> 132

```
acggaaccca tgggaatgta aacaaaattt atacttattg ttaaaatctc tctaaaatat 60
ctgataattt gatgtgcata tgtctaatga tcatttttga ccgccaaatg gcacattttt 120
ttactttttc ctccatatct ccaaagtcgt tggacctttc caaaattttg aacagttctt 180
catttagtca atacaaataa aatgtttttt aaattattca aatcggacgc tccgttctct 240
taaaaactga gttaccgttt tcggcacttt ttgcccgtat cttcggaacg gctagaccta 300
cctttgccaa aaactaatca gcacgtcttc ttatcaatat gaatcgaatg ttttttaaat 360
tattcaaatc ggttgattcg tgttcccgaa atcgtcgacg aaaatttgta tccgcacata 420
catacataca cacacataca cacacacaca cacacacata catccatttt ttctaaggta 480
tgccaaaatg gtcagaacct                                             500
```

<210> 133
<211> 235
<212> DNA
<213> Ctenocephalides felis

<400> 133

```
accaccccccc aaaacaccat taatctcacg gaacgtttct ctagcggatt tgcctttaac 60
gaaggaaaac tttaaaatag cgcgaatttc ggcgtaagtg aactccatgt ttacacgtct 120
ataactgtta aacgcaatat ccaaactaat catgcatagc atcgttttgt aggttatgtc 180
aagacctttc aaattatgta tagtattgcc agatacgagc tctgtagcgc tttgt      235
```

<210> 134
<211> 500
<212> DNA
<213> Ctenocephalides felis

<400> 134

```
acatgtctat atttgctata ttattaccct acacaaatcc ttttcgatat aatcattaca 60
tagtatcact agctcatcat gttatagctg cttggttttt aaaatgcagg ctgcctttca 120
ggagagattt tgtaaaattt atcaccacgg tatgtgtgaa atataacaat tgtgttaact 180
tggtagtaaa caaaatatat taatcaattc atgacgtata gggcctaaaa tcaaattgca 240
ttgcaccatt tgaggaanga aggatattaa ttaaaccaga ggttacaact ttgaatgaag 300
aatcatctta cngaaaaaga aagtctaggt taacagaacc agntaggtat ttggaggttt 360
tcataagaaa aatgggtgaa atggtaaatg ncaccccaag ttttattggc ngccaaaaaa 420
aaatgntttg canattancc taacntccaa attttgggta ntgnataatt tttaatttac 480
cataatctan gggttnaaaa                                             500
```

<210> 135
<211> 500
<212> DNA
<213> Ctenocephalides felis

<400> 135

```
ctacgatatt tctgncaagt cgaactacaa ttttgaaaaa ccattcctgt ggttggctcg 60
caagcttatt ggagatccca acttagagtt cgttgcaatg ccagcattgg tgcctccaga 120
ggtcaccatg gacccagaat ggaaacagaa gatagagaag gatctgaagg aggctcaaga 180
aaccgctttg ccggaagatg atgaagactt ataagttact ttgttataga caacatgaat 240
gttttgtata ttttgttaat gaaaatggtg ggtttgaatt tgaataatag agaatgggtt 300
ctgcaaaatg tcgtgaaata attattttaa cgccagatgt atttttttat tcttaatcat 360
ctgnaaagtg aaaaatttaa aaatgtgagt gctgtaatca tgaatgctga gtgtaatata 420
actttttaaa gnaactcgcc taattttaa ttataattta atataatggt aaccagttgc 480
atccaagttt cagtggtatg                                             500
```

<210> 136
<211> 550
<212> DNA
<213> Ctenocephalides felis

<400> 136

```
atatagtggt ctattaaaga ataactgctg aataataaat taaacagaac caatggctgc 60
aattcgaaaa aagttagtga ttgtcggaga cggagcgtgc ggtaaaactt gtttgctgat 120
tgtatttagc aaagatcaat ttccagaagt atatgtccca actgtttttg aaaactatgt 180
ggctgacatc gaagtcgatg gaaaacaagt tgaacttgcc ctttgggata ccgccggaca 240
agaagactac gatcgcttgc gacctctcag ttatcctgat actgatgtca ttctgatgtg 300
tttttctgtc gactcacctg attcattaga aaatattcca gaaaaatgga ctccagaggt 360
gaaacacttt tgtccaaatg tacctattat tcttgttgga aataagaaag atttacgcaa 420
tgacccaaac acaatcaaag agttaagtaa gatgaaacag gacctgtgaa gccacaggaa 480
ggcgtgcctg gccgagaaga taaatgcttt gatatttaaa tgtctgttaa tcaaaagaag 540
agtccagagt                                                        550
```

<210> 137
<211> 549
<212> DNA
<213> Ctenocephalides felis

<400> 137

```
attaccaagt accacctgaa ggaaggatcc ttgtgggaca taaacaatta cctcgagctc 60
tcaccagcac caaacggtga cagaatcgag caaatcagtg caaacgatca cccacaaaga 120
tcaattagat tctatgttag gaaacttaca agcagacatg tccagacaag gtgttaatac 180
tgctcaaaaa ggaagctgtg gtgcttgtga caaagccatt gttggccaag ttataactgc 240
tcttggcaaa acatggcatc ctgaacattt cgtttgcaac cattgcaacc aagaattggg 300
aacaagaaac ttcttcgaaa gagatggaca cccatactgc gaacctgatt accataattt 360
gttcagccca agatgcgcgt attgcaatgg agctattttg gataaatgcg taacagcctt 420
agaaaaaact tggcacacag agcacttctt ctgcgcccaa tgtggtcaac aatttggtga 480
aaaggttcac gaaaaagatg gtaaccntat tgcgcatgct atttcgattg ttgtcccaat 540
gtggagatg                                                         549
```

<210> 138
<211> 549
<212> DNA
<213> Ctenocephalides felis

<400> 138

```
gaacgcccaa cgagtgcaag atttattttc gtattttatt tcggtttatt gtgcaaacat 60
ttcgatactt ttgaagtatt gagaactgtg aatagtgttt ctgatttatt atttatattc 120
atcagcgctt gatccaagaa aagccttcca aaaatgagtg tcgctcgcta tgaagatatt 180
gttgcaggcc ctttggccaa atttttgagc ctttcaaaaa gtattggcgg agatgttgcg 240
cagcaaactg tttttgtgga gaatgccttt aaggcacaat tagcttttat cacaactgct 300
agcactgcat cacaacctgc tcctgatgtc ttacaacaat tattgcaacc gaccagccaa 360
cagatattag cggctcaaga atttcgagaa aagcatcgtt catcgaactt cgtgaatcat 420
ttagcggcga ttagcgagag cattgcgcac tcggatgggt ttgatttccc taccctgtgt 480
gcatgtgaaa gaaatgcatg atgctgacgt tctcactgcc ggtcttgaag agaaaaggct 540
actgccaat                                                         549
```

<210> 139
<211> 549
<212> DNA
<213> Ctenocephalides felis

<400> 139

```
gccagtgccg ccatgtctga gagcttgaag caactgccag taatctgggt tttaggtgga 60
cctggatcgg gcaaaggaac ccaatgtgac aaaattgttg ctaaatatgg attcacccac 120
ttgtctactg gtgatttgct ccgagctgag gttcaaagtg ggtcagatag aggcaagaac 180
ctcacggcta tcatggagag aggagaacta gtacccatgg acatagtact tgacttatta 240
aaagaagcta tgactaaggc cttgcccaca tctaaaggat ttttaattga tggatatccc 300
cgtgaaaaag accaaggtgt ggctttcgaa aatcaagtga ctcccgtaaa cacaatttta 360
tatttcgaat gtaagcctga aacattggtc gaacgtcttt tgggacgtgc aaaaacttct 420
ggccgagctg atgacaatga agagaccatc aaattgcgtt tgatcttcat gccaataatg 480
accaagtttt ggcctatccc agacagactg aagagataac gcgaaaggca gtgataatct 540
ttgcggaga                                                         549
```

<210> 140
<211> 500
<212> DNA
<213> Ctenocephalides felis

<400> 140

```
gttttatata taaatatagg attaatttta aggaatatta ttaaatttat ttatattaat 60
atattttact ccncggccgc gannangcta gcngtntccc tgntcattgg cggnngcccc 120
ccnnnctctn cnnncncccc tcnctccccn cctcncnncc cccnncntct acntctctcc 180
ccnccncncc nncctttntc tccccncccc ctcccnnnnn ctcntctctc ttccngncnc 240
ncnncccntc ncncctcnnc cctcttccct ccntcncccn nccnttcccn ccctcncccc 300
tntncccncg ccccnnccnn ctcccntccc tntnctnccn ngtcnncnnn ccnnctccnn 360
ncctccnncc cccncntctc ctcccctcnc nnnnncnccc ctccccccctt cnccnctccn 420
ntcgnnctcn nctctntncc cntncccnnn ncncnccnct ntncctcngc ctcccctttc 480
ntnnctcccn cctcccnctc                                             500
```

<210> 141
<211> 500
<212> DNA
<213> Ctenocephalides felis

<400> 141

```
acaagtttga ttacaatgta atttctaagc atttgataaa agtttatatt gctagttttt 60
taattataat tacaaataac aacataaata tataacacat atgttttata tgttaacaac 120
attaaacttt ataaataagt attaatatat aagccattaa tatttgcttt agggtaaaaa 180
cttaatacaa ctccataaaa tataaggcct tgattattca caattatctg atttaaggca 240
ttaaggtaat tgttgaagtt tatgaattat aaaacttttt actttataat cataatataa 300

gttaattgac tgaataaata atttgtttgt ataggtaact atgaccagcc acccgttaac 360
ttatggaaca tttcttgatt caactgttga tttgcatctt tagatctcat tgacataaat 420
atgtaacctc cgataaactc atggatcacc ttgcaatctg ctgataagca tgaaaatatt 480
atgntttctt cttcgaatga                                             500
```

<210> 142

<211> 285
<212> DNA
<213> Ctenocephalides felis

<400> 142

```
acctgngtat ttaattcttc ttattataac atgtttnagt atcatatatt naatatacct 60
gaattacgga tatatnacta taaacaattt ctaaacataa aatcatattt acacaaatat 120
tttttatcta ttatttcaat ttagcaaaaa cttctcccaa catttgatta cataaagcag 180
cataaggatt catttccacc aactgatttt ttgcaaaggt ggagcctaag cttgcggctt 240
tgttgaaatc ttcccgagct aaatcgtctt caccagtttt tctgt                 285
```

<210> 143
<211> 198
<212> DNA
<213> Ctenocephalides felis

<400> 143

```
accacaggca ttgacacaag ggttaacaca actattgtcg atacaagctt ggtcttgtct 60
gcattcttca cttcctatgc attcgtatcg aaaacactgc ctcaaaggat ctcctttatg 120
atttggcagg catgaacaaa caggtaaccc ctcatggctt ctagtgcatt gcgcatttac 180
accacaggtg tgataggt                                               198
```

<210> 144
<211> 500
<212> DNA
<213> Ctenocephalides felis

<400> 144

```
ataaaataat tttaaaaaaa atatttttaa atttattagt tatgtaatnt aaaatgaaat 60
antaataata ataatagtag attagtattg taaaagaaaa attaaaatng tgtataagta 120
aatttaattt attgtatctn gtgtatcaga gttaatttaa taaaagatat gaagaatatt 180
nttctcgaat ttaaaagggg aatttattat ttaatttaat gnaatataat tatttaaaat 240
aataaattag taatgaaatg ttattcgntt ttaaagntat ctagnttttt tagaaataaa 300
tttaatttat tatatttaaa tatttatatt aatttattaa atatagatat tttaaatata 360
aaatattttt agggataagc ttaaaaataa attattataa attaataaat tatttataaa 420
tttatagggg tataaatatt cattaatata aaaangtata atttatttat aaataaatta 480
aaattagtag attttaaatt                                             500
```

<210> 145
<211> 474
<212> DNA
<213> Ctenocephalides felis

<400> 145

acaaaaacgc aggaaacccc acggntgcat ttctataaaa taatatcata gatttgaaaa 60
ctatccgaat ccaaaagttt taacgcctaa tatttatatc aaaactttcc caacattatc 120
cctagattct cttaataaaa ctttcatttc attattagaa ccactcgaac tcaagtttaa 180
taaaaccatt tcaacattta ttaataattt caaaaacttt atatatcacc attatcccca 240
gcagaagtat cggagcagtc ataaaattct tcatcagaat cttccggact ttcggactta 300
ttttcaggat ttgttgaagc agcattgtcc cgttcttgct gccgagcaac gcagcaattc 360
agcaattgta attgttggtg aagaatacac gtgcgaggat cgggaaatcc gcctccaact 420
cccggtattt tgtggtatta tcaatcctgt atctcaattc ttccacaaat tcgt 474

<210> 146
<211> 500
<212> DNA
<213> Ctenocephalides felis

<400> 146

ttaaaactaa ctcattcgng taatgttaac atttaaacca ttatacattg caacaagatc 60
ctnggnggtn ggctccaaag cattttcaca atacgcttct tattttattg atcctgtttt 120
caaaccacaa aaacaaaatg aattagctca agaaggtgan atccaaaact tgcacacgtg 180
cctattaaag ctgctcgaaa taatgatact agctctgtat tccatgatga tctacttagc 240
aaattcacaa attatgttat gaaaggcgga caaaaggttt tggctagaaa tttaatagat 300
gaagcttttg tgaatattaa aagaatacaa attgaaaaat atcacaaggc taaagaggaa 360
aataagagta atattatact aaatccaaaa gtgatattgc acaatgctat tacaaattgc 420
aggccaggtc taacacttac accaatcaaa agaggaggtg ntcgtatnag ttcctatccc 480
gacactgaga agcactcata 500

<210> 147
<211> 347
<212> DNA
<213> Ctenocephalides felis

<400> 147

caattatgtt atttcacctt taaaacttgt attatagcat aatagacaga attatacttc 60
atctattcat cgtttccgca aataaaccaa ttcgaaaatt acaatatatt ttgcataata 120
aaattacata ggaaaaataa aacatctatt ctaattaact tactccaatt tttgaggcta 180
ttttatcagc acatatttca gcaatatctc gacctagcac gccataatat aacccataaa 240
atagtaacaa aaaacctaag tccatccaag aatgcggcct ttgattgaag attaaattga 300
ctccagcaaa tgttgccatc accattccat aaccaattat accaagt 347

<210> 148
<211> 500
<212> DNA
<213> Ctenocephalides felis

<400> 148

atttttaggc tttaaataac ccaatagatt gtaaaagatg taaaataaaa acactatctc 60
tgatgcttcc acacaaatac ctatttagta aaataaaact aaatgaaatt tattagtatt 120
ttccaaactc atagctcagg caatattata attaatttgg tagaggcagt cttaataata 180
gcatgcaaaa acaaaaataa atattttgta aaaaaactat taaagtgaag aaactgatca 240
atacagttaa actataaaaa aaaggtaaca ccttataact aacagccgaa caaaataaga 300
aagaagcact tcattaaaca cttacaggta tctggttaaa tcctctatat caaccagcat 360
gctatcttgg ctcatatgaa gttcatcacg attcattaat aagcccacca actgttcatt 420
aaatatttca acctgtgtat ggagtcggca aacaacaacc tgcaattgag caatcgtcat 480
gctggtcaac aaacgccggg 500

<210> 149
<211> 500
<212> DNA
<213> Ctenocephalides felis

<400> 149

acttacaact gcctaaatgg tccggtatgt cttntatgtt cggtaaaggg tatgaatcac 60
ccaggggcgt aacgtgcctt ggcggggccc catatcaaaa tcggtttggg ggctccccta 120
aagatttttc ataatttaaa gaaacataaa gagattaata aattcattta ataaaattaa 180
aagaagattt atttatcaaa ttttcaaata tacatcgaat attattttta atattttaca 240
agtaactaaa attaaatatt cactctcctt gcctttttat cagcaaactg atttattaag 300
tcatttatag ctgatgttgt ttttagtttt tctaatgttt ttgcctctat ggacaataga 360
gacaggtctg atagacgttc ctgttttatt gatgttctga gataattttg gattaatttt 420
aattttgaaa aactcgtttc agcagttgcg gttgtaactg gaagggcaag aataaaatgc 480
aagctgttat tacatccgga 500

<210> 150
<211> 550
<212> DNA
<213> Ctenocephalides felis

<400> 150

gctaaaccac gtgtttatta atgtataaat tattattaaa caagttttcc ggaaatatat 60
tgtagatcaa actaaacagg aataaaattg aataatacac aatgggaaaa ctgaacgtat 120
caatattacg ttatttagac aaagaagact ttcgcgtttt gacggccatt gagatgggca 180
tgaaaaatca tgaactggtg ccgggatctc ttacagctct gatcgcaaat ttgcgacatg 240
gtggtgttca caaaatttta agagaacttt gcaaacatcg gttgctgagc tacgaacgtg 300
gaaaacatta tgatggatac aggctgacaa atatgggcta tgattatctg gccttgaaag 360
cattaactat gagaaacgtt gttgagtcat ttggaaatca aattggtgtt ggtaaggaat 420
cgaatattta tgttgtagca gatgaagaag gtgaagcctt atgtcttaaa ctcccagatt 480
ggccgaacat gctttcgagt gtaagcaaat agagatatca tcacatagca tagagctctg 540

gnttattatc 550

<210> 151
<211> 549
<212> DNA

<213> Ctenocephalides felis

<400> 151

```
ggaacgtgca acatatttag ttatttaaat aatttgataa tttaacaaaa atggcaacca 60
ttgttcgcaa aatcatcagc accaaagctg cagccaagcc agtggcccca tataatcagg 120
caattttggt cgatcgcact ctctacgtat cgggatgtct aggattggac gcctgcacaa 180
tgaaattggt cgcaggagga gctgccgccg aagcaagaca agctttgacc aaccttggac 240
atatcttggc cgctggtgac tcatcatatg accgtgttgt gaaaaccacc gtcttacttg 300
ccgatttagc tgaccttgca gctgtaaatg aagtttatgg acaagtgttt acacatgacc 360
accctgccag atcctccttc aagttggagc actaccaatg aatgctaagg tcgaaattga 420
agtcgttgca gtttcaggag acgtccgaac tattccggag tggaatgcta agaaagtaag 480
aataatatca taattaatag tgttcagtaa attatagttt atatgtaaat aattaactac 540
catagtttt                                                         549
```

<210> 152
<211> 549
<212> DNA
<213> Ctenocephalides felis

<400> 152

```
acgaaagatt tcgatggggc ggagcgggcg agatggagga cagctacgaa gcggctttac 60
gacgaggact gccctatccc gtccttacgg tcgctgaata cttctcgctg ggacaagaag 120
gattcgcttg gggaggacaa tatagagctg cgggatatta tgcttcgatt ttacttggta 180
cggcattggc gtgttggctg cttatgaacc tgctcctggt agctgtcccg cgatatggag 240
cctatttgat gtttacgact gggcttttat tggctgccac ggatttaggc tactatctga 300
tgttgccagc aaggcctcta cgaattgtac ttgaaggcgg agcgctcgat tttcgattag 360
gatggtgctt ctggctggtc ttagttgctg gaagtatatg ctcattttcc ggattagtaa 420
taacgtgcgt ggatttggca tttctcaccg attttcgact gtcttgaggt gactatgatc 480
tccntacgat cgnacgttat attgaagaga cccgacacgc ctcaggaaac gcacagtggc 540
gnaacngcg                                                         549
```

<210> 153
<211> 633
<212> DNA
<213> Ctenocephalides felis

<220>
<221> CDS
<222> (67)..(507)

<400> 153

```
agtaaatact acatttgaaa attataaaca aattgtttta taaatttgaa gtagaagaaa 60

atcaaa atg aag ata ttc ttg gtg att gga aca ctg gtc gct tta tat    108
       Met Lys Ile Phe Leu Val Ile Gly Thr Leu Val Ala Leu Tyr
        1               5                  10

tct gcg gca gag gct gct aaa tat act aaa gaa gaa gca aaa gaa aaa   156
Ser Ala Ala Glu Ala Ala Lys Tyr Thr Lys Glu Glu Ala Lys Glu Lys
 15                  20                  25                  30

ctt ttg cag att ggt aag gat tgt gct gta gaa aca gga gct tct tca   204
Leu Leu Gln Ile Gly Lys Asp Cys Ala Val Glu Thr Gly Ala Ser Ser
                 35                  40                  45

gat gat att gaa aaa tta ctg caa aag aac ata cca gat tca aag cct   252
Asp Asp Ile Glu Lys Leu Leu Gln Lys Asn Ile Pro Asp Ser Lys Pro
             50                  55                  60

gga aaa tgc atg att aat tgc gtt tac aag aaa ctt gga gtg atg aaa   300
Gly Lys Cys Met Ile Asn Cys Val Tyr Lys Lys Leu Gly Val Met Lys
         65                  70                  75

gat gga aaa tac cat cct gat gcc gga att gaa gta tca gcc atg gta   348
Asp Gly Lys Tyr His Pro Asp Ala Gly Ile Glu Val Ser Ala Met Val
     80                  85                  90

cac gaa cac gat tca gaa tta atg gaa aaa gtt aag aaa atc gca acc   396
His Glu His Asp Ser Glu Leu Met Glu Lys Val Lys Lys Ile Ala Thr
 95                 100                 105                 110

gaa tgt gac agc gaa gcc aaa gga gaa gac gag tgc gaa att gct gcc   444
Glu Cys Asp Ser Glu Ala Lys Gly Glu Asp Glu Cys Glu Ile Ala Ala
                115                 120                 125

aaa gca atg gag tgc ggc gtg agg atg gcc aaa gaa cac aac cta atg   492
Lys Ala Met Glu Cys Gly Val Arg Met Ala Lys Glu His Asn Leu Met
            130                 135                 140

gac gcg ata cca ata taatagttaa aaatttaata ttaggtcggt tgaagacctg   547
Asp Ala Ile Pro Ile
        145

tcaaaatgta gtcgttaaat tgtagtgtga agttgtcacg atgtgggcgt aggaataaaa 607

tgctatttaa aaaaaaaaaa aaaaaa                                      633
```

<210> 154
<211> 147
<212> PRT
<213> Ctenocephalides felis

<400> 154

```
Met Lys Ile Phe Leu Val Ile Gly Thr Leu Val Ala Leu Tyr Ser Ala
 1               5                  10                  15

Ala Glu Ala Ala Lys Tyr Thr Lys Glu Glu Ala Lys Glu Lys Leu Leu
            20                  25                  30

Gln Ile Gly Lys Asp Cys Ala Val Glu Thr Gly Ala Ser Ser Asp Asp
        35                  40                  45

Ile Glu Lys Leu Leu Gln Lys Asn Ile Pro Asp Ser Lys Pro Gly Lys
    50                  55                  60

Cys Met Ile Asn Cys Val Tyr Lys Lys Leu Gly Val Met Lys Asp Gly
65                  70                  75                  80

Lys Tyr His Pro Asp Ala Gly Ile Glu Val Ser Ala Met Val His Glu
                85                  90                  95

His Asp Ser Glu Leu Met Glu Lys Val Lys Lys Ile Ala Thr Glu Cys
            100                 105                 110

Asp Ser Glu Ala Lys Gly Glu Asp Glu Cys Glu Ile Ala Ala Lys Ala
        115                 120                 125

Met Glu Cys Gly Val Arg Met Ala Lys Glu His Asn Leu Met Asp Ala
    130                 135                 140

Ile Pro Ile
145
```

<210> 155
<211> 633
<212> DNA
<213> Ctenocephalides felis

<400> 155

```
tttttttttt tttttttaa atagcatttt attcctacgc ccacatcgtg acaacttcac 60
actacaattt aacgactaca ttttgacagg tcttcaaccg acctaatatt aaatttttaa 120
ctattatatt ggtatcgcgt ccattaggtt gtgttctttg gccatcctca cgccgcactc 180
cattgctttg gcagcaattt cgcactcgtc ttctcctttg gcttcgctgt cacattcggt 240
```

```
tgcgattttc ttaacttttt ccattaattc tgaatcgtgt tcgtgtacca tggctgatac 300
ttcaattccg gcatcaggat ggtattttcc atctttcatc actccaagtt tcttgtaaac 360
gcaattaatc atgcattttc caggctttga atctggtatg ttcttttgca gtaatttttc 420
aatatcatct gaagaagctc ctgtttctac agcacaatcc ttaccaatct gcaaaagttt 480
ttcttttgct tcttctttag tatatttagc agcctctgcc gcagaatata aagcgaccag 540
tgttccaatc accaagaata tcttcatttt gattttcttc tacttcaaat ttataaaaca 600
atttgtttat aattttcaaa tgtagtattt act                              633
```

<210> 156
<211> 441
<212> DNA
<213> Ctenocephalides felis

<220>
<221> CDS
<222> (1)..(441)

<400> 156

```
atg aag ata ttc ttg gtg att gga aca ctg gtc gct tta tat tct gcg    48
Met Lys Ile Phe Leu Val Ile Gly Thr Leu Val Ala Leu Tyr Ser Ala
 1               5                  10                  15

gca gag gct gct aaa tat act aaa gaa gaa gca aaa gaa aaa ctt ttg    96
Ala Glu Ala Ala Lys Tyr Thr Lys Glu Glu Ala Lys Glu Lys Leu Leu
            20                  25                  30

cag att ggt aag gat tgt gct gta gaa aca gga gct tct tca gat gat   144
Gln Ile Gly Lys Asp Cys Ala Val Glu Thr Gly Ala Ser Ser Asp Asp
        35                  40                  45

att gaa aaa tta ctg caa aag aac ata cca gat tca aag cct gga aaa   192
Ile Glu Lys Leu Leu Gln Lys Asn Ile Pro Asp Ser Lys Pro Gly Lys
    50                  55                  60

tgc atg att aat tgc gtt tac aag aaa ctt gga gtg atg aaa gat gga   240
Cys Met Ile Asn Cys Val Tyr Lys Lys Leu Gly Val Met Lys Asp Gly
65                  70                  75                  80

aaa tac cat cct gat gcc gga att gaa gta tca gcc atg gta cac gaa   288
Lys Tyr His Pro Asp Ala Gly Ile Glu Val Ser Ala Met Val His Glu
                85                  90                  95

cac gat tca gaa tta atg gaa aaa gtt aag aaa atc gca acc gaa tgt   336
His Asp Ser Glu Leu Met Glu Lys Val Lys Lys Ile Ala Thr Glu Cys
            100                 105                 110

gac agc gaa gcc aaa gga gaa gac gag tgc gaa att gct gcc aaa gca   384
```

```
Asp Ser Glu Ala Lys Gly Glu Asp Glu Cys Glu Ile Ala Ala Lys Ala
        115                 120                 125

atg gag tgc ggc gtg agg atg gcc aaa gaa cac aac cta atg gac gcg     432
Met Glu Cys Gly Val Arg Met Ala Lys Glu His Asn Leu Met Asp Ala
    130                 135                 140

ata cca ata                                                         441
Ile Pro Ile
145
```

<210> 157
<211> 147
<212> PRT
<213> Ctenocephalides felis

<400> 157

```
Met Lys Ile Phe Leu Val Ile Gly Thr Leu Val Ala Leu Tyr Ser Ala
  1               5                  10                  15

Ala Glu Ala Ala Lys Tyr Thr Lys Glu Glu Ala Lys Glu Lys Leu Leu
            20                  25                  30

Gln Ile Gly Lys Asp Cys Ala Val Glu Thr Gly Ala Ser Ser Asp Asp
        35                  40                  45

Ile Glu Lys Leu Leu Gln Lys Asn Ile Pro Asp Ser Lys Pro Gly Lys
    50                  55                  60

Cys Met Ile Asn Cys Val Tyr Lys Lys Leu Gly Val Met Lys Asp Gly
65                  70                  75                  80

Lys Tyr His Pro Asp Ala Gly Ile Glu Val Ser Ala Met Val His Glu
                85                  90                  95

His Asp Ser Glu Leu Met Glu Lys Val Lys Lys Ile Ala Thr Glu Cys
            100                 105                 110

Asp Ser Glu Ala Lys Gly Glu Asp Glu Cys Glu Ile Ala Ala Lys Ala
        115                 120                 125

Met Glu Cys Gly Val Arg Met Ala Lys Glu His Asn Leu Met Asp Ala
    130                 135                 140

Ile Pro Ile
145
```

<210> 158
<211> 441

<212> DNA
<213> Ctenocephalides felis

<400> 158

```
tattggtatc gcgtccatta ggttgtgttc tttggccatc ctcacgccgc actccattgc 60
tttggcagca atttcgcact cgtcttctcc tttggcttcg ctgtcacatt cggttgcgat 120
tttcttaact ttttccatta attctgaatc gtgttcgtgt accatggctg atacttcaat 180
tccggcatca ggatggtatt ttccatcttt catcactcca agtttcttgt aaacgcaatt 240
aatcatgcat tttccaggct ttgaatctgg tatgttcttt tgcagtaatt tttcaatatc 300
atctgaagaa gctcctgttt ctacagcaca atccttacca atctgcaaaa gtttttcttt 360
tgcttcttct ttagtatatt tagcagcctc tgccgcagaa tataaagcga ccagtgttcc 420
aatcaccaag aatatcttca t                                           441
```

<210> 159
<211> 384
<212> DNA
<213> Ctenocephalides felis

<220>
<221> CDS
<222> (1)..(384)

<400> 159

```
gct aaa tat act aaa gaa gaa gca aaa gaa aaa ctt ttg cag att ggt      48
Ala Lys Tyr Thr Lys Glu Glu Ala Lys Glu Lys Leu Leu Gln Ile Gly
1               5                   10                  15

aag gat tgt gct gta gaa aca gga gct tct tca gat gat att gaa aaa      96
Lys Asp Cys Ala Val Glu Thr Gly Ala Ser Ser Asp Asp Ile Glu Lys
            20                  25                  30

tta ctg caa aag aac ata cca gat tca aag cct gga aaa tgc atg att      144
Leu Leu Gln Lys Asn Ile Pro Asp Ser Lys Pro Gly Lys Cys Met Ile
        35                  40                  45

aat tgc gtt tac aag aaa ctt gga gtg atg aaa gat gga aaa tac cat      192
Asn Cys Val Tyr Lys Lys Leu Gly Val Met Lys Asp Gly Lys Tyr His
    50                  55                  60

cct gat gcc gga att gaa gta tca gcc atg gta cac gaa cac gat tca      240
Pro Asp Ala Gly Ile Glu Val Ser Ala Met Val His Glu His Asp Ser
65                  70                  75                  80
```

```
gaa tta atg gaa aaa gtt aag aaa atc gca acc gaa tgt gac agc gaa    288
Glu Leu Met Glu Lys Val Lys Lys Ile Ala Thr Glu Cys Asp Ser Glu
                85                  90                  95

gcc aaa gga gaa gac gag tgc gaa att gct gcc aaa gca atg gag tgc    336
Ala Lys Gly Glu Asp Glu Cys Glu Ile Ala Ala Lys Ala Met Glu Cys
            100                 105                 110

ggc gtg agg atg gcc aaa gaa cac aac cta atg gac gcg ata cca ata    384
Gly Val Arg Met Ala Lys Glu His Asn Leu Met Asp Ala Ile Pro Ile
        115                 120                 125
```

<210> 160
<211> 128
<212> PRT
<213> Ctenocephalides felis

<400> 160

```
Ala Lys Tyr Thr Lys Glu Glu Ala Lys Glu Lys Leu Leu Gln Ile Gly
1               5                   10                  15

Lys Asp Cys Ala Val Glu Thr Gly Ala Ser Ser Asp Asp Ile Glu Lys
            20                  25                  30

Leu Leu Gln Lys Asn Ile Pro Asp Ser Lys Pro Gly Lys Cys Met Ile
        35                  40                  45

Asn Cys Val Tyr Lys Lys Leu Gly Val Met Lys Asp Gly Lys Tyr His
    50                  55                  60

Pro Asp Ala Gly Ile Glu Val Ser Ala Met Val His Glu His Asp Ser
65                  70                  75                  80

Glu Leu Met Glu Lys Val Lys Lys Ile Ala Thr Glu Cys Asp Ser Glu
                85                  90                  95

Ala Lys Gly Glu Asp Glu Cys Glu Ile Ala Ala Lys Ala Met Glu Cys
            100                 105                 110

Gly Val Arg Met Ala Lys Glu His Asn Leu Met Asp Ala Ile Pro Ile
        115                 120                 125
```

<210> 161
<211> 384
<212> DNA
<213> Ctenocephalides felis

<400> 161

```
tattggtatc gcgtccatta ggttgtgttc tttggccatc ctcacgccgc actccattgc 60
tttggcagca atttcgcact cgtcttctcc tttggcttcg ctgtcacatt cggttgcgat 120
tttcttaact ttttccatta attctgaatc gtgttcgtgt accatggctg atacttcaat 180
tccggcatca ggatggtatt ttccatcttt catcactcca agtttcttgt aaacgcaatt 240
aatcatgcat tttccaggct ttgaatctgg tatgttcttt tgcagtaatt tttcaatatc 300
atctgaagaa gctcctgttt ctacagcaca atccttacca atctgcaaaa gtttttcttt 360
tgcttcttct ttagtatatt tagc                                        384
```

<210> 162
<211> 631
<212> DNA
<213> Ctenocephalides felis

<220>
<221> CDS
<222> (65)..(505)

<400> 162

```
gtaaatacta tattggaaaa ttataaaaaa gtttatttat aaatttaaag tagaagaaat 60

caaa atg aag ata ttc ttg gtg att gga gca ctg gtt gct tta tat tct  109
     Met Lys Ile Phe Leu Val Ile Gly Ala Leu Val Ala Leu Tyr Ser
      1               5                  10                  15

gtg gca gag gct gca aaa tat acc aaa gaa gaa gca aag gaa aaa ctt   157
Val Ala Glu Ala Ala Lys Tyr Thr Lys Glu Glu Ala Lys Glu Lys Leu
                20                  25                  30

ttg cag ata ggc aaa gat tgt gct gta gaa aca gga gca tct tca gat   205
Leu Gln Ile Gly Lys Asp Cys Ala Val Glu Thr Gly Ala Ser Ser Asp
            35                  40                  45

gat atc gaa aaa tta ttg cag aag aac ata cca gat tca aaa gca gga   253
Asp Ile Glu Lys Leu Leu Gln Lys Asn Ile Pro Asp Ser Lys Ala Gly
        50                  55                  60

aaa tgc atg atc aat tgc gtt tac aag aaa ctt gga gtg atg aaa gat   301
Lys Cys Met Ile Asn Cys Val Tyr Lys Lys Leu Gly Val Met Lys Asp
    65                  70                  75

gga aaa tac cat cct gat gcc gga att gaa gta tca gcc atg gta cac   349
Gly Lys Tyr His Pro Asp Ala Gly Ile Glu Val Ser Ala Met Val His
 80                  85                  90                  95
```

```
gaa cac gat tca gaa tta atg gaa aaa gtt aag aaa atc gca acc gaa    397
Glu His Asp Ser Glu Leu Met Glu Lys Val Lys Lys Ile Ala Thr Glu
                100                 105                 110

tgt gac agc gag gcc aaa gga gaa gac gag tgc gaa att gct gcc aaa    445
Cys Asp Ser Glu Ala Lys Gly Glu Asp Glu Cys Glu Ile Ala Ala Lys
            115                 120                 125

gca atg gcg tgc ggc gtg agg atg gcc aaa gaa cac aac tta atg gac    493
Ala Met Ala Cys Gly Val Arg Met Ala Lys Glu His Asn Leu Met Asp
        130                 135                 140

gcg ata cca ata taatagtaaa aaaatctttg ttaggtcggt tgaagacctg        545
Ala Ile Pro Ile
    145

tcaaaatgta gtcgttaaat tgtagtgtga agttgtcacg atgtgggcgt aggaataaaa 605

tgttatttaa aaaaaaaaaa aaaaaa                                       631
```

<210> 163
<211> 147

<212> PRT
<213> Ctenocephalides felis

<400> 163

```
Met Lys Ile Phe Leu Val Ile Gly Ala Leu Val Ala Leu Tyr Ser Val
  1               5                  10                  15

Ala Glu Ala Ala Lys Tyr Thr Lys Glu Glu Ala Lys Glu Lys Leu Leu
             20                  25                  30

Gln Ile Gly Lys Asp Cys Ala Val Glu Thr Gly Ala Ser Ser Asp Asp
         35                  40                  45

Ile Glu Lys Leu Leu Gln Lys Asn Ile Pro Asp Ser Lys Ala Gly Lys
     50                  55                  60

Cys Met Ile Asn Cys Val Tyr Lys Lys Leu Gly Val Met Lys Asp Gly
 65                  70                  75                  80

Lys Tyr His Pro Asp Ala Gly Ile Glu Val Ser Ala Met Val His Glu
                 85                  90                  95

His Asp Ser Glu Leu Met Glu Lys Val Lys Lys Ile Ala Thr Glu Cys
            100                 105                 110
```

```
Asp Ser Glu Ala Lys Gly Glu Asp Glu Cys Glu Ile Ala Ala Lys Ala
        115                 120                 125

Met Ala Cys Gly Val Arg Met Ala Lys Glu His Asn Leu Met Asp Ala
    130                 135                 140

Ile Pro Ile
145
```

<210> 164
<211> 631
<212> DNA
<213> Ctenocephalides felis

<400> 164

```
tttttttttt ttttttttaa ataacatttt attcctacgc ccacatcgtg acaacttcac 60
actacaattt aacgactaca ttttgacagg tcttcaaccg acctaacaaa gattttttta 120
ctattatatt ggtatcgcgt ccattaagtt gtgttctttg gccatcctca cgccgcacgc 180
cattgctttg gcagcaattt cgcactcgtc ttctcctttg gcctcgctgt cacattcggt 240
tgcgattttc ttaacttttt ccattaattc tgaatcgtgt tcgtgtacca tggctgatac 300
ttcaattccg gcatcaggat ggtattttcc atctttcatc actccaagtt tcttgtaaac 360
gcaattgatc atgcattttc ctgcttttga atctggtatg ttcttctgca ataatttttc 420
gatatcatct gaagatgctc ctgtttctac agcacaatct ttgcctatct gcaaaagttt 480
ttcctttgct tcttctttgg tatattttgc agcctctgcc acagaatata aagcaaccag 540
tgctccaatc accaagaata tcttcatttt gatttcttct actttaaatt tataaataaa 600
ctttttttata attttccaat atagtattta c                                631
```

<210> 165
<211> 441
<212> DNA
<213> Ctenocephalides felis

<220>
<221> CDS
<222> (1)..(441)

<400> 165

```
atg aag ata ttc ttg gtg att gga gca ctg gtt gct tta tat tct gtg     48
Met Lys Ile Phe Leu Val Ile Gly Ala Leu Val Ala Leu Tyr Ser Val
  1               5                  10                  15

gca gag gct gca aaa tat acc aaa gaa gaa gca aag gaa aaa ctt ttg     96
Ala Glu Ala Ala Lys Tyr Thr Lys Glu Glu Ala Lys Glu Lys Leu Leu
             20                  25                  30
```

```
cag ata ggc aaa gat tgt gct gta gaa aca gga gca tct tca gat gat    144
Gln Ile Gly Lys Asp Cys Ala Val Glu Thr Gly Ala Ser Ser Asp Asp
            35                  40                  45

atc gaa aaa tta ttg cag aag aac ata cca gat tca aaa gca gga aaa    192
Ile Glu Lys Leu Leu Gln Lys Asn Ile Pro Asp Ser Lys Ala Gly Lys
        50                  55                  60

tgc atg atc aat tgc gtt tac aag aaa ctt gga gtg atg aaa gat gga    240
Cys Met Ile Asn Cys Val Tyr Lys Lys Leu Gly Val Met Lys Asp Gly
65                  70                  75                  80

aaa tac cat cct gat gcc gga att gaa gta tca gcc atg gta cac gaa    288
Lys Tyr His Pro Asp Ala Gly Ile Glu Val Ser Ala Met Val His Glu
                85                  90                  95

cac gat tca gaa tta atg gaa aaa gtt aag aaa atc gca acc gaa tgt    336
His Asp Ser Glu Leu Met Glu Lys Val Lys Lys Ile Ala Thr Glu Cys
            100                 105                 110

gac agc gag gcc aaa gga gaa gac gag tgc gaa att gct gcc aaa gca    384
Asp Ser Glu Ala Lys Gly Glu Asp Glu Cys Glu Ile Ala Ala Lys Ala
        115                 120                 125

atg gcg tgc ggc gtg agg atg gcc aaa gaa cac aac tta atg gac gcg    432
Met Ala Cys Gly Val Arg Met Ala Lys Glu His Asn Leu Met Asp Ala
    130                 135                 140

ata cca ata                                                        441
Ile Pro Ile
145
```

<210> 166
<211> 147
<212> PRT
<213> Ctenocephalides felis

<400> 166

```
Met Lys Ile Phe Leu Val Ile Gly Ala Leu Val Ala Leu Tyr Ser Val
1               5                   10                  15

Ala Glu Ala Ala Lys Tyr Thr Lys Glu Glu Ala Lys Glu Lys Leu Leu
            20                  25                  30

Gln Ile Gly Lys Asp Cys Ala Val Glu Thr Gly Ala Ser Ser Asp Asp
        35                  40                  45
```

```
Ile Glu Lys Leu Leu Gln Lys Asn Ile Pro Asp Ser Lys Ala Gly Lys
    50                  55                  60

Cys Met Ile Asn Cys Val Tyr Lys Lys Leu Gly Val Met Lys Asp Gly
65                  70                  75                  80

Lys Tyr His Pro Asp Ala Gly Ile Glu Val Ser Ala Met Val His Glu
                85                  90                  95

His Asp Ser Glu Leu Met Glu Lys Val Lys Lys Ile Ala Thr Glu Cys
            100                 105                 110

Asp Ser Glu Ala Lys Gly Glu Asp Glu Cys Glu Ile Ala Ala Lys Ala
        115                 120                 125

Met Ala Cys Gly Val Arg Met Ala Lys Glu His Asn Leu Met Asp Ala
    130                 135                 140

Ile Pro Ile
145
```

<210> 167
<211> 441
<212> DNA
<213> Ctenocephalides felis

<400> 167

```
tattggtatc gcgtccatta agttgtgttc tttggccatc ctcacgccgc acgccattgc 60
tttggcagca atttcgcact cgtcttctcc tttggcctcg ctgtcacatt cggttgcgat 120
tttcttaact ttttccatta attctgaatc gtgttcgtgt accatggctg atacttcaat 180
tccggcatca ggatggtatt ttccatcttt catcactcca agtttcttgt aaacgcaatt 240
gatcatgcat tttcctgctt ttgaatctgg tatgttcttc tgcaataatt tttcgatatc 300
atctgaagat gctcctgttt ctacagcaca atctttgcct atctgcaaaa gtttttcctt 360
tgcttcttct ttggtatatt ttgcagcctc tgccacagaa tataaagcaa ccagtgctcc 420
aatcaccaag aatatcttca t                                           441
```

<210> 168
<211> 384
<212> DNA
<213> Ctenocephalides felis

<220>
<221> CDS

<222> (1)..(384)

<400> 168

```
gca aaa tat acc aaa gaa gaa gca aag gaa aaa ctt ttg cag ata ggc     48
Ala Lys Tyr Thr Lys Glu Glu Ala Lys Glu Lys Leu Leu Gln Ile Gly
 1               5                  10                  15

aaa gat tgt gct gta gaa aca gga gca tct tca gat gat atc gaa aaa     96
Lys Asp Cys Ala Val Glu Thr Gly Ala Ser Ser Asp Asp Ile Glu Lys
            20                  25                  30

tta ttg cag aag aac ata cca gat tca aaa gca gga aaa tgc atg atc    144
Leu Leu Gln Lys Asn Ile Pro Asp Ser Lys Ala Gly Lys Cys Met Ile
        35                  40                  45

aat tgc gtt tac aag aaa ctt gga gtg atg aaa gat gga aaa tac cat    192
Asn Cys Val Tyr Lys Lys Leu Gly Val Met Lys Asp Gly Lys Tyr His
    50                  55                  60

cct gat gcc gga att gaa gta tca gcc atg gta cac gaa cac gat tca    240
Pro Asp Ala Gly Ile Glu Val Ser Ala Met Val His Glu His Asp Ser
65                  70                  75                  80

gaa tta atg gaa aaa gtt aag aaa atc gca acc gaa tgt gac agc gag    288
Glu Leu Met Glu Lys Val Lys Lys Ile Ala Thr Glu Cys Asp Ser Glu
                85                  90                  95

gcc aaa gga gaa gac gag tgc gaa att gct gcc aaa gca atg gcg tgc    336
Ala Lys Gly Glu Asp Glu Cys Glu Ile Ala Ala Lys Ala Met Ala Cys
            100                 105                 110

ggc gtg agg atg gcc aaa gaa cac aac tta atg gac gcg ata cca ata    384
Gly Val Arg Met Ala Lys Glu His Asn Leu Met Asp Ala Ile Pro Ile
        115                 120                 125
```

<210> 169
<211> 128
<212> PRT
<213> Ctenocephalides felis

<400> 169

```
Ala Lys Tyr Thr Lys Glu Glu Ala Lys Glu Lys Leu Leu Gln Ile Gly
 1               5                  10                  15

Lys Asp Cys Ala Val Glu Thr Gly Ala Ser Ser Asp Asp Ile Glu Lys
            20                  25                  30

Leu Leu Gln Lys Asn Ile Pro Asp Ser Lys Ala Gly Lys Cys Met Ile
        35                  40                  45
```

```
Asn Cys Val Tyr Lys Lys Leu Gly Val Met Lys Asp Gly Lys Tyr His
     50                  55                  60

Pro Asp Ala Gly Ile Glu Val Ser Ala Met Val His Glu His Asp Ser
 65                  70                  75                  80

Glu Leu Met Glu Lys Val Lys Lys Ile Ala Thr Glu Cys Asp Ser Glu
                 85                  90                  95

Ala Lys Gly Glu Asp Glu Cys Glu Ile Ala Ala Lys Ala Met Ala Cys
            100                 105                 110

Gly Val Arg Met Ala Lys Glu His Asn Leu Met Asp Ala Ile Pro Ile
        115                 120                 125
```

<210> 170
<211> 384
<212> DNA
<213> Ctenocephalides felis

<400> 170

```
tattggtatc gcgtccatta agttgtgttc tttggccatc ctcacgccgc acgccattgc 60
tttggcagca atttcgcact cgtcttctcc tttggcctcg ctgtcacatt cggttgcgat 120
tttcttaact ttttccatta attctgaatc gtgttcgtgt accatggctg atacttcaat 180
tccggcatca ggatggtatt ttccatcttt catcactcca agtttcttgt aaacgcaatt 240
gatcatgcat ttttcctgctt ttgaatctgg tatgttcttc tgcaataatt tttcgatatc 300
atctgaagat gctcctgttt ctacagcaca atctttgcct atctgcaaaa gtttttcctt 360
tgcttcttct ttggtatatt ttgc                                        384
```

<210> 171
<211> 133
<212> DNA
<213> Ctenocephalides felis

<400> 171

```
ctgtangtga agaattatta ggcagagtag ttgatgcttt aggaaatgcc attgatggca 60
aaggtgcttt acaaagcaaa accagattcc gtgtaggaac taaagctccc ggtatcattc 120
cacgtgtctc tgt                                                    133
```

<210> 172
<211> 498
<212> DNA
<213> Ctenocephalides felis

<400> 172

gctgtaaatt gggcggctct tgccgagcgt gttccacctg cccaaaaacc caatttaatg 60
gccttcaaag taaaaagtga cggatatatg aggagggttg cggcgaatcc ggaaaatcca 120
ccagctttag attggaactt ttataagaag tttgtgagtg tgcctggaat ggtagccgaa 180
tttcaaaaac aatgcgagtc tctgaaagtc ccatatccag ccgataatta cacttctaaa 240
gtcgatgaac aagaacggca agtcaaggct gaaatagaaa ccttcaaaaa ggagtctaat 300
gatcgtatta caaaatatca agctgatatg gaaaggctta aggctttgtt gccatatgaa 360
agcatgacgt tggaagactt ccatgatgca catcctgact tggctttgga tgctgttaac 420
aaaccaacgt tctggcctna cacttcagag gaacaattgg gataccaatc caaagatcca 480
gtagaagctc cttctcat 498

<210> 173
<211> 557

<212> DNA
<213> Ctenocephalides felis

<400> 173

tccaaaaata ttcttatggt agccaatgag gttggacgta aaccaccaac tttccaagat 60
gcagctaaat taacaaatgc tgtattgaat tcaggttatg atttcgcatc cggaaaaata 120
atttacaata aatttaaatc tgttgtctcc tacagttctg ctgaattacc attgtttagc 180
ctgggagctg ttgagtctgc cccaaaattg ggtgtatacg attccttaga tgctgatgtc 240
atccaaagct acttggaatt ttcaatggct tcattattat tctacacaat gaaggaagga 300
gcttgctcgg agcagtcatc ccgtatgact gctatggaca atgctagcaa gaatgcagga 360
gaaatgattg acaaattgac attaacattc aacagaacta gacaggctgt catcaccaga 420
gaacttattg aaattatttc tggagctgct tctttggatt aaatttcata cnaatatttt 480
aatatgtggc ttcatctaag tagctcaatt tattacaata tctttaatac tttggttatt 540
tttaatttga atcttgg 557

<210> 174
<211> 498
<212> DNA
<213> Ctenocephalides felis

<400> 174

aatcaattat ttattgacta tataaataat atacagatgt tagttaaaat tgcatacaac 60
atgaacattt cattttcttc tatttttgac aaagtttatt ttaaacaggt gttgaaataa 120
tttcagtgta cagctttact ttactggcaa cactcatatc aacataacga tctccaattg 180
atacgaccat tccacccaag attgagggat taactttaag ggttagtaat aagttttctt 240
tgggttttaa gaatcctctt aaagtgcttt caagttgttt ttgttgattc tcatctaaag 300
gtttagctgt tgtaacttca catggaacct ctccacgatg agcagacatc ataactttat 360
aagcgttgat aacaccttcc agtttattta aacgtccatt ctcagcaagt aactccagca 420
agttggatgt agctggagcc atttgcagct tgctgctggc ttccttcaat gcatttgatt 480
taatggagcg cttgaaag 498

<210> 175
<211> 236
<212> DNA
<213> Ctenocephalides felis

<400> 175

```
tttttttttt tttttttttt ttttttttgg naaatttctc tagangaaag aggnnttaaa 60
acagcaaata cataagattg aataatagat acagcanttt ntaaaattaa taaaattaat 120
tgagagaaaa ttaaaattca aattaatcat atagataggg agtttcctgt attgccaagt 180
aaagttagaa ttaaatgtcc agcaattatn tttgctgaga gtcggatggc taaagt     236
```

<210> 176
<211> 161
<212> DNA
<213> Ctenocephalides felis

<400> 176

```
atcttgtcct tcctggtcac ggaagtattc agntacagtt aatccagtca aagcgacacg 60
ggcacgggcg cctgggggtt cgttcatctg tccgtagaca agagctacct tggaggtctt 120
gtctttcaag gaaatgacac caccttcaat catttcattg t                     161
```

<210> 177
<211> 549
<212> DNA
<213> Ctenocephalides felis

<400> 177

```
gacangttat gttatttatt tcatctgatt ttatcacata aaaacaaaaa taagtagcat 60
ggatttcata cttttaatta taaaatgcag tacagtttaa aatttgtcaa aaagacagtg 120
ttacttattt aataatacat gaaattatgc aacttacatt ttcattttgg ataacgtatt 180
tctaatccaa tactcactta ttttattaaa ttatgcatta aaagtagcta aaaagtcagc 240
tacaattttc tttaattggg catcagatgc ttcactaatc gttccttcct tggcaatggt 300
ttgcaaaagg gatgaatggt tggctttgat gtgagcgtta aattcctttt caaatgctgt 360
gatcttggtt gggtccattt tgtccaagtg tcctcggaca ccacagtaga taattgcaac 420
ttgttcttca atagccattg ggacatattg ccttgcttca ataattcagt caaacgaaca 480
cctctgttaa caattgtgtg tggtgatcaa gactgaccga attggcaaaa gcagcaccta 540
cgatttgac                                                         549
```

<210> 178
<211> 400
<212> DNA
<213> Ctenocephalides felis

<400> 178

```
gatncttgaa agtccgaaaa ttattaatat cattaattaa aatggaccag ggaaaagcac 60
```

cagttcgcgt ttcacctctg atcaaattcg gaaggtggag ttttctcgtt gtcggaatat 120
tatatggagc agctcaccaa agcaggctgg caaaacgcga agtaggaatt agagaagtcg 180
aagctaaaca taaagcaatt cgggatgcaa aattagctga ggaaaagaaa cgagctcaag 240
aagaggaaaa caaatacttc gcttcacttt aaacagatta gcattattaa ataggaaatg 300
cagtaacatt caccataagg cattagatgt gctctgtaaa ttattcggat tttatgtgaa 360
ataaaaagtt attatacata caaaaaaaaa aaaaaaaaaa 400

<210> 179
<211> 549
<212> DNA
<213> Ctenocephalides felis

<400> 179

atcagccact gcttctgatg ctgcccctct tcaatatctt gctccatact ctggatgtgc 60
tatgggtgaa ttcttccgtg acaatggaaa acatgctttg atcatctatg atgatttatc 120
caaacaagct gttgcttatc gtcaaatgtc tctattgtta cgtcgtccac caggtcgtga 180
ggcttatcca ggtgatgtct tctaccttca ctcacgtcta cttgaacgtg ccgctaaaat 240
gtctgaagct catggaggtg gctctttgac tgctttgcca gttattgaaa cacaagctgg 300
tgacgtatca gcttatattc caactaatgt catttccatt actgatggtc aaatcttctt 360
ggaaactgaa ttgttctaca agggtattcg accagccatc aatgtaggtt tatctgtatc 420
tcgtgtaggt tctgctgcac aaaccaaagc catgaaacag gttgccggtc catgaaactg 480
gaatagctca tatcgtgagg tggctgtttt gccaattcgg ctagatcttg atgcancacc 540
aacaatggt 549

<210> 180
<211> 550
<212> DNA
<213> Ctenocephalides felis

<400> 180

gttaatatag gattcgttgt ttctagaacc acaacaaatt aaaacacacc aagatgttct 60
cagcagcaaa attcatcgcc ccagtcgcca aatccgcatt tgtcaatgga tcaaaggcat 120
atctgcgacc aatctcaagt gcagtattga ctcaaagctc tactatcaat gtattacctg 180
cagccgctca atctagcatt ttaccacaag ttcgttgtct gcaaactaca gcagtaacga 240
aagacattga ttcagcagct aaatttattg gtgctggagc agccaccgta ggagtagcag 300
gatcaggagc tggtattggt tcagtctttg gatcattaat cattggttat gcacgtaatc 360
catcccttaa acaacaacta ttctcatatg ccattcttgg atttgcattg tcagaagcta 420
tgggactttt ctgcttatga tggctttctt actgtattcg cattctaatt tggttttcat 480
ttgcccgaag agggtctaga agagtgctna cgtatcacca ggactgtatt aatcattcaa 540
atccattant 550

<210> 181
<211> 498
<212> DNA
<213> Ctenocephalides felis

<400> 181

```
caagttatct ctgaattcct tcacggctcc tcttgcgcat ttgccgactt cacgaaccaa 60
gttgcccaat tgggaccagg tttctttgaa agccttggcc aagtccttgg ctccttcacg 120
ggcagcttga aggacatctt tcaagcaagc tctggcttca tcgacatcgg ctgcggtaac 180
aacgcactcc ttggcttgtg ctctgagggc ttcggcgttc tccctggctt ctctccaggc 240
ggccaagcca ttttggcgaa cttggttgaa ttcctcacgt ttatcatccc tgcaagcctt 300
gaccttagca cgagcttctt gtgcaaaacc tagagctttt tggtgttggc ctttaaggca 360
ttcctttgcg gcttcactca cttttttgct aagtttcctt tcaacttctt tgttgaacct 420
ttccaaatct ctatcgactt cangggcaaa tccttgtttt tcgaattcag cagcaatcat 480
ttaattttgc tatcntgg                                              498
```

<210> 182
<211> 498
<212> DNA
<213> Ctenocephalides felis

<400> 182

```
ggaagcacct taggctgtgc aggacatgta actgtatagc attttgtgcc cagagccata 60
ggtccgcaat tatgacgtaa cggatcatat gcaaaatttg cggggcaata atattgggtt 120
cctatactat tttcgtcaca ataataatag ctttgacaat catttatatt tggataaaat 180
ctcgatggtg acggacattt gaaaccttgt actttgcana ctgtactttc tttagtggtg 240
cactgtagat atccactttg atcagcgcac acttgatctg ggctacagga ttccttaacc 300
ggatctgctc ctatttccgg acaatactgc atttcgggtg aactgcacga attcacgcaa 360
ctaaatttta ctttttttaca tttttgctcg ggtttcggta cangtgtacg ttcangagta 420
gtactactcc tggtgcgggc tcatccaatg atgcaaatac ttctttattt actgttaaan 480
gcgttccgtc gacattaa                                              498
```

<210> 183
<211> 424
<212> DNA
<213> Ctenocephalides felis

<400> 183

```
ccagcatttg atggagtaaa gaatgtatat gcttcttcac cccttcctaa agtgggtgtt 60
aatgtccttc aaggagaggt ttctattata aaccctgata atgaactgga aaaaaaatat 120
aaagtaacta ttaaatttgc ttcttatgtt gacatgactt ccctttcaaa atatatggaa 180
aatggatcat ctattgagac accgcaggaa gccctgcaat gtattgacat catatttagg 240
catgcactct ccaaacaatt tgttcaggtg ggaaagtcct tctttacacc acccagtggt 300
cgaattgttt ctcttggtga tggaatggat ctatggtatg ggttattttc tagttgtgtt 360
ttangatgga aaaatgtaga tgttgcacat aaaaggattt caactaaaca actantncag 420
aant                                                             424
```

<210> 184
<211> 313
<212> DNA
<213> Ctenocephalides felis

<400> 184

aattcttggc tataagttcg ttgcatgttt tgcaagtgat ttgtttattg catttattcc 60
cgtggcatat tttacatgaa tcagagtttg cattacaatc gttaccctga tctgatttgc 120
atcctcgagt aatgacacct ttttcaattt ttgaatagca gtcgtccttt ggattcatac 180
aaactttggt cttcgtcttt cctgcatctt tgaaacattc gtttcttagt tctggaagtt 240
tttgaacgtt acaacccgat ttctcgcaac acatttttc tcggttagtc gagtcacaac 300
tttctttaac cgt 313

<210> 185
<211> 498
<212> DNA
<213> Ctenocephalides felis

<400> 185

tatttgatct aaaagcagat aaacactttc tctgtttttg actgcttctt tatcggcctc 60
ttgtcctaat cttagtaaac ttgatgatgt tagtgctaaa aattccttca tcctatcttc 120
aatatcaccc tgtccacaag tagtaaacaa agcaccaaaa atattattaa tggctaaagc 180
catacaatgc atattgttag tatggccttc caaacttgct cggtanaaag attgatcgct 240
tcttgctaat tttggaatag aaacagctac aaaaaccatc aataagcata ctaacaaatg 300
ttcatcctcc tcgtgttctg acttctgctg cctcaaagca tttgctanag ttggatctac 360
tttacaagtc aaacctgctg ccgatgacat ctcaagaaac aattttcatt gggtctccac 420
ttggcaatga tgtttaatgt ccttggatan aacttataaa aatggtatac gctgttctaa 480
cacgtctact anagatct 498

<210> 186
<211> 498
<212> DNA
<213> Ctenocephalides felis

<400> 186

aaaacttcaa agaacattcc atccaatgga ttttcaaaca ttctcatcat acggtagttt 60
gatataggaa aattaacaaa tacaataaca atatattaaa ctattcatat tagttatact 120
atttatttct taatccacaa gttcagagac ttcagtaatt gtagcatctg ctaacatgcc 180
ttttttgtca ccagttatta catgaccaac aataaaagct ttttgctttt caagctcata 240
aatctcttta atatatcctt ctgcatctgc ttttggtaaa gctattaata ggcctcctga 300
agtttctggc aaatatcctt ctgctagctt aaacattttg ctgaagattt tggcaacttc 360
ggaacatttg taaaaaactg gtagtttatt caatacaaat gaaacattat ttttctgatt 420
ctgggccaaa ttatcagcat gaccgagaat tccaaagcct gtgacatcag tagcatcatg 480
agcattatat ttatgcaa 498

<210> 187
<211> 498
<212> DNA
<213> Ctenocephalides felis

<400> 187

cattcagcaa aggaccggtg ccttctggca gtggtattga tccacgagca atagttctaa 60
cattttctgc agaacagcaa ctaccagaac cgctacctcc attggaacca cgaggagcta 120
atatgtattc tacttcttga cctaaatcta ggtttgatgt gtcgccttca aaattgctga 180
aatggaaaaa cacttcttta tcatgcgaaa tggtctcgat aaaaccgaaa ccatccttca 240
aggctgctac aaatccttgg cacaactggc cgttgctcaa agactgacgt ccatttttgg 300
agacaccatt gcgcaatgag gtgcttgatt gtgaagttga actactgctg cttgacgaga 360
cagttgaacc gttaaagctg tggtttgatg actggttact aatggcagtt actacaacat 420
ctgntgctat cagttcttta ttacgcttac ctgactaatg ttgaaatcaa ctttatcacc 480
cattctaggt tgccgatc 498

<210> 188
<211> 549
<212> DNA
<213> Ctenocephalides felis

<400> 188

gcacactggt gctgttgtaa cattggaagt tgcaaaacag ggagctattt atcatggtct 60
ggctacatta cttcaacaac cgagtccact tttgaatcaa ggcccaagaa gaatgtcgga 120
acgcgatttg ccgtcacgtg ttagtgcaga gggctcggtt aaatctattc tgccatctag 180
taaatcagta ccagcactgc accatggtgg ttcttcgatg caggaaatgt cagtgcaatc 240
agctaattta aaatctcata gcacccataa cttaacacaa aatacaacac cacagcaaga 300
tcaaggtttt tatcaaaacc taagtgtata tagaggaaat tcatcacagc caaatttaga 360
tcgtggatca ggtttaaggt ccccacagaa tatggttcaa caaaatgtgc atcagagttc 420
taggccagct tcagcatact ttcctaacca atctaggtgc caatcaaact tcaaccaacc 480
tgttctaatc ttagatctca aagcactaaa gatattgaaa cttttgcgtg aaatcttgnc 540
caacagtta 549

<210> 189
<211> 549
<212> DNA
<213> Ctenocephalides felis

<400> 189

gacaacccta tcagtccagc gcagagttta tataaatagg gatgaaaata ggcaagttgc 60
ctccgtgatg caccattctg atctgaataa tttgttgaga gttggcagtt tgatattctt 120
aaatgtcggt caattgctgc cgcatcagct gcagtcgttc cacacgccga attacatcta 180
tccgattggg tatcagattg tcagatttta ctggtcgatg aggaggccga ataaacggtg 240
cagatatatt tgttcaatag ctgatgttgc tggacgtcca gaatttcgag tacgcgttca 300
agaacctcaa caggatgata ttgaactaag agatgctaca ccaagggctg tttggaatcg 360
aattctggag ccattggccg ctatgagaag ggaattgggc gattcagttc gtctgtttcc 420
caagtacgtc actggtgaag atttgtttgg acttactgag ccagctgttg ttcgagtact 480
cgaagtttac caggaattga gacattaacc gatatcgctt caagtatgga aggacccctt 540

attgagctc 549

<210> 190
<211> 550

<212> DNA
<213> Ctenocephalides felis
<400> 190

```
gctgtacgca gttatggnta ttgacacatt accatccatt aggctctttg tacgancacc 60
tgaacagaac aaccttgact cataaccaaa tggcacatat ttgtctttca attgcagntg 120
gattggttca cttacattca gaaatttttg gaacagaggg cgaaggtaaa ccagctatgg 180
ctcatcgtga tatcaaatca aagaatattc ttgtgagagt aaatggaaca tgtgttatag 240
ctgattttgg tttagcagtt acacatacac aatctacagg agctctagat gttgcatcta 300
atccaagagt tggaactaga cggtatatgg ctcctgaggt tcttgatgaa agcatcaaca 360
tgcaatgctt cgaagccttc aggcgcgccg atatctacgc tctgggtctt gtctatggga 420
ggtcgctaac agaactcttt cgaatggcat tgtgaagaat taggccacct tactacgacg 480
tcgtgccttc tgatccaagc ttgaggatat gcgcaaagtg tctgcgtgcc acacagacca 540
gtacacctac                                                        550
```

<210> 191
<211> 492
<212> DNA
<213> Ctenocephalides felis

<400> 191

```
gcatgtggta acatgtttca actgctgcaa ggacattcag cagaaacttc tggaggtctt 60
ttgatctgcc tcccaagaga acaggctgca gcttattgca aagacattga gaaacaagaa 120
ggttaccaag cttggatcat tggaattgtt gaaaaaggaa atcgcacagc aagaataatt 180
gacaaaccta gagttattga agttccagca aaagattaga atgataatga tatgattatt 240
taattacaga atgaaataaa gggtgtatgt ctgtgtcaac attatgctag ttaaacaatg 300
gcatctcatt cctgctattt gcaatgcttc aatatttttt tgaaacaaat tgttctatta 360
atgcaaatgt agattttaag aatatatata gataagaatt attgttttaa gagtaatttt 420
gaatcatgaa gaaatttctt gatttttcaa ttcttaataa agtcaaaagt aaaaaaaaaa 480
aaaaaaaaaa aa                                                     492
```

<210> 192
<211> 479
<212> DNA
<213> Ctenocephalides felis

<400> 192

```
tttttttttt ttgggaagtt caatttattt tatgaatata gtatttagaa cttcatacga 60
tgtggatatt gggaagattt caaaccaaac atagctgaca aatatgtata caaaagtttg 120
tggtctgggt gcttcttgat tactcctaaa accattttgt ctacaagctt ttggtcattc 180
ttcctttgct cagttggttt ataagcctct ttcttggcag caaaaatatc accttcctct 240

ttctttgcac gcttgttctt ttgccttttg aaataatcat cattgatgtg ttctggtaat 300
ttaactccac taacgtctaa tcgggtagag gtggcaatga catagttttg ggaaatgcga 360
cgcaatgggc atgcattgat taagaangga cctgtaacaa gtaacaatcc agagtccaac 420
tgtttaagaa gaactacacg ttttccntta tgttgtccag ctaaaacaat gcaaatcgt  479
```

<210> 193

<211> 528
<212> DNA
<213> Ctenocephalides felis

<400> 193

```
agtttctggt tacattcccc aacgttattc ctccagaaat ggttgcagct ttagagagat 60
gtcttccacc tcgaccagta gttgagattc ctcctggtgc tgaagaatgt atgcttattg 120
acatggaccc agaacaagag gcacgaagac gtagtcataa aaatgcatat gatgaagatg 180
atgagggagg acctggagca aacagagttc aatgtgccac gagttaaatc gtagtaactt 240
agggcattgt tttaaagtat tgtctaacac tatttatata attttcctac gatgataaca 300
tgagttttat gtcatctatc atatttatca tattgatact tggttagggt tttacattgc 360
acaattattt gtgactttgn aattacacac gatnttatgc tccacngtaa cggatgggac 420
acccaantgg nctaanggta ccatttttc ntttttagat agacaatctt tattttttgt 480
atgtttcata gctgagaaat gcctggccta tgtgatattt gttccagt          528
```

<210> 194
<211> 370
<212> DNA
<213> Ctenocephalides felis

<400> 194

```
ttctgataac aaaaggccct gtggataaag tctccatccc tgttccgtta caatgtgtac 60
atttgacagg ctttgtgcca ggctcacacc tggaacctct gcatttctga caagtatcta 120
ctatgttgac attaatatct ttattaactc ctcgtgcagc ttgagagaat gttagtttca 180
tagatacttc ttttgcacca ccaaatccaa aactggattc agcaaagtca tcaaaacctc 240
cagttttaaa tcctgcatca ccaaatattt tgtgaaatag ttcttcaggg tcgattgtcg 300
attgatactt ccaactctgt gagaagcctt gagggcctgc tccccccatg ccgcccatct 360
gttcagaggt                                                    370
```

<210> 195
<211> 343
<212> DNA
<213> Ctenocephalides felis

<400> 195

```
aggtggcggc gtcatcgtat tacaaggact ggtctggttc atttctaatc cgtttgcaag 60
ctgttgaagt ttagctaaac tgaaactagt attagctcca gtttgctcta caggattgcc 120
ttggggagta ggagtcgctg tcggggtcgg tgtctgcggt gcagacggtc ctggtgtatg 180
tgaatgtgga tgcgtgcctg attgaatata aaaatttgta gcagtggaaa ctgcacatga 240

aggcgatgat ccatgccttt gatgtggact cggcagagga tgctgagctg tgggaatact 300
accttgattt gacatcctat gttgtatcac agaactcatc ggt               343
```

<210> 196
<211> 749

<212> DNA
<213> Ctenocephalides felis

<400> 196

```
ttttttttt ttttttttt tttttttag actatagttc tatttattct ttagcatttt 60
ttgatgttac atttttacaa taataccaag taaaataatg acttcattca tataataaag 120
ggtttaatac gccgatttaa aaacagacat cttatacaaa attaaaatgg ctgctgntaa 180
attatagaat atcttttgat ttaaacaaaa tctaaatagt acataaaact ggacattact 240
tccattatat atatagatat atacatatgt atgttaatgt atatatacat aaatacatat 300
agaatcaaac aatatcacaa actgttgatt tgaataagtt gctattcata aaattatgtt 360
ttcgcattta gtacattttt tgtatgtgct taagatgtat ataaacacat atttgntaat 420
tagtaaaaat taacataagc ttgagacatt attaggtgct actaaaattg ataaatattt 480
acataaatta aaacaatttc aattcttata tgtgtggnag taacttcaaa atctgnggaa 540
atattggtta aattggtact ggnctgnaaa taatttacta aacagaaatt atattgactc 600
ctatctggca ttggcttctt aaccatttta aatttaattt acttaatctc ttcagaaata 660
gcttaataag gngagacatc ccagganaat tggtggataa atattggtaa aagctcaaca 720
ggaacattgn gacctgaatg gctggcaaa 749
```

<210> 197
<211> 210
<212> DNA
<213> Ctenocephalides felis

<400> 197

```
ggaagcacct taggctgtgc aggacatgta actgtatagc attttgtgcc cagagccata 60
ggtccgcaat tatgacgtaa cggatcatat gcaaaatttg cggggcaata atattgggtt 120
cctatactat tttcgtcaca ataataatag ctttgacaat catttatatt tggataaaat 180
ctcgatggtg acggacattt gaaaccttgt 210
```

<210> 198
<211> 185
<212> DNA
<213> Ctenocephalides felis

<400> 198

```
accacgaggt gcaggcggaa ttccagttaa atcaaaggtt cctaaacgat tgttatcttt 60
ggtcataacc ctttctcctt catagacttg aattgttact gcaggctgat tgtctgcata 120
agtgctgaag gtttgtgttc ttttacatgg aattcttgca ttgcgttcaa taatcttagc 180
cattg 185
```

<210> 199
<211> 223
<212> DNA
<213> Ctenocephalides felis

<400> 199

cacggccgag gggttcagag ttgtcagtct taactgtgaa ggaacctcct gcggaagatt 60
cccaaaggta ttgttcatca tcattgtgct tagatgtgac cgtgacttta tcagcaacga 120
gataggccga gtagaaacca acaccaaatt gaccaatcat actaatatcg gctccagctt 180
gtaaggcttc catgaaggct ttagttccag acttcgcaat tgt 223

<210> 200
<211> 465
<212> DNA
<213> Ctenocephalides felis

<400> 200

cgaaagataa ggactccgat tgttctgaga gcgggctttg gggcaatgtg gaggcgcagg 60
ccgccttcct ggggcctaat atatgggata agacattacc ctatgatgct gatctcaagt 120
atgtggatct agatgagttc ctttctgaga atggaatccc tgtggatgga atgggtcaca 180
gcggtggatt ggggtccatg agtcacttag gtggcttagg aggatctcat aggtctgagg 240
ctttaggtca ccctttgagt ggtatgccaa cacatctaac aaagattgaa agatcacctt 300
caccttccga atgcatgagc ccggatacta tgaatcctgc ttctccagct gattcaacat 360
tctcaatggc ttcttctggg cgagattttg atcctcgaac acgggctttt tcggatgaag 420
aactaaagcc tcaacctatg atcaagaaaa gtagaaagca gtttg 465

<210> 201
<211> 312
<212> DNA
<213> Ctenocephalides felis

<400> 201

attcttggct ataagttcgt tgcatgtttt gcaagtgatt tgtttattgc atttattccc 60
gtggcatatt ttacatgaat cagagtttgc attacaatcg ttaccctgat ctgatttgca 120
tcctcgagta atgacacctt tttcaatttt tgaatagcag tcgtcctttg gattcataca 180
aactttggtc ttcgtctttc ctgcatcttt gaaacattcg tttcttagtt ctggaagttt 240
ttgaacgtta caacccgatt tctcgcaaca catttttctc cggttagtcg agtcacaact 300
ttctttaacc gt 312

<210> 202
<211> 209
<212> DNA
<213> Ctenocephalides felis

<400> 202

attcagggct cttgctcttt tctcatctct tgtcaaatgt tcttcaggac tgtctccatt 60
cttccgttgc ttgtctttct tgtccctcgt ttgaggtctt ggagtagcac ctgccatgtg 120
gggtgacatg tggtaggtgt gattatgggc tataggatcg taagctgccc taatgctctg 180
ctgacgagca aagtccattg tgcaggagt 209

<210> 203
<211> 293

<212> DNA

<213> Ctenocephalides felis

<400> 203

```
ccggagnctg gggtggaagt ggagcttttg aaggatgggt attggtttct gcccaaaatt 60
acaagagcaa gatttgaaga attatgctct gatttattcc gctctacttt acgcccagtt 120
gaacaagcgt taagagatgc aaagttagac aaaggagcca tacatgatgt tgtattggtg 180
ggaggttcca caagaatacc taaaattcga tctctgctac aagaattctt tgcaggaaag 240
accttaaatt cttccataaa tccagatgaa gctgtggctt atggtgctgc agt       293
```

<210> 204
<211> 377
<212> DNA
<213> Ctenocephalides felis

<400> 204

```
aaaaaggata taagaaccga tnttaggnnt atgagacgat tacgaactgc tgnagagcgt 60
gcaaaaagaa ctctaccaac tagtacggaa gcatgtgtgg aagttgaagc tttgaaggat 120
ggtattgatt tctgcaccaa aattacaaga gcaagatttg aagaattatg ctctgattta 180
ttccgctcta ctttacgccc agttgaacaa gcgttaagag atgcaaagtt agacaaagga 240
gccatacatg atgttgtatt ggtgggaggt tccacaagaa tacctaaaat tcgatctctg 300
ctacaagaat tctttgcagg aaagacctta aattcttcca taaatccaga tgaagctgtg 360
gcttatggtg ctgcagt                                                377
```

<210> 205
<211> 452
<212> DNA
<213> Ctenocephalides felis

<400> 205

```
tttcttttaa attcgtccat gagatgatct actagacgat tgtcaaaatc ttcaccgcct 60
agatgagtat caccggcagt tgctctgact tcaaatagag atccttcatc aattgttaaa 120
acagatacat caaatgtgcc tccaccgagg tcaaatatta acacattcct ttctccttgt 180
aggtgtttat ctaagccata tgccaaagca gcagctgttg gttcgtttat aacccttaaa 240
acattcaaac ctgcaatgac accagcatct tttgtagctt gccgttgaga gtcattgaaa 300
taagcaggaa cagtaataac tgcatctttc actttctctc caagatagtt ttcagctgtt 360
tctttcatct taactagcac cattgcacta atttcttctg gtgaaaagcg tttcagttgg 420

ttcttgtatt caacttcaat tttaggtgtt gt                               452
```

<210> 206
<211> 500
<212> DNA
<213> Ctenocephalides felis

<400> 206

```
tnnttttttt ttttttttt tttttttttc tgacgttgac acatccattg cttcaccact 60
tcctgacgca tcaccagaac ttaacttacg ttttttgta ggttcttcag acccgttagt 120
agcagattga gaagcctctt gttcatcccc cggttctttc ttaatactgn aatctacgta 180
tccagtaagc cattcctttg gtgtgttttc atttggctct ccgtgtttgt ctagtttgcc 240
tgcggctatc aaagacttct tttgtgatgc cttaggtcct aagccccatt ttctgggata 300
tgtatntcgc tccattatca cacgttttaa tttagcacag actccatggt cgcatgacat 360
cattgttgct gnggtcatta gagcgattgc taatgcaata gcttctccct tagtagtaac 420
aataacaatt tcctgattca tctcgattcc atcttcatac ctcaagacgc cggnaacata 480
actttngacc gnnacaaaca                                              500
```

<210> 207
<211> 264
<212> DNA
<213> Ctenocephalides felis

<400> 207

```
acgtggtaga aattctagaa gttgccaatt tcttctgtcg actagttcga atggtgtgtt 60
ggttacccat tcgtgatcat actggttcag aagataacaa ccgacagtca tgcctcccaa 120
aaagatcaat ccaatggctc cgatggagaa aaatcttcct ttacatttgc agctggtttc 180
agtatacaaa tattgacctt ctatatcttc tgattttttt aaaagtggtt gttcttggtt 240
gccttctgat ggtggttgtg ttgt                                         264
```

<210> 208
<211> 498
<212> DNA
<213> Ctenocephalides felis

<400> 208

```
atttccttgt gctaattcaa ttgttcgggg atgaagtgcc agctctggaa ccccttgaac 60
aacttcagaa accaaatcag caactctagc ctgacttgtc tgtgaaccag nttgtccagg 120
tgtgttagca gaactttgaa tgataagatt ttggttggca gcaagactct gtccacacat 180
atttctccgt ttatgagatg ctaaatcatt actttgggca aaagctctac cacaaaccat 240
acaagcgtag ggcttttcac ctgtatgtgt cctcatatgt atagctaact tatcagatcg 300
tgcaaatcct ctgttacaaa cactgcatac aaatggnttt tctcctgnat gttttctcat 360
atgcaatgna agatctgtag ttcttggata acctttacca cagattttgc aaaaatttgt 420
catttgacca gtatgtctcc gcatatggac ggntaaatta cttcttgact gnaactttta 480
ccacaaacac tgnataat                                                498
```

<210> 209
<211> 470
<212> DNA
<213> Ctenocephalides felis

<400> 209

```
aatcagatta cagcttattt cacaaaaaat gagaaatnaa gaatccacaa ctaaatacaa 60
tatgngtaaa caagataaaa atataactat aaacaaaata taaatcaagt taatcttgaa 120
cataattaaa taaacttttt gtcaaaaact acagtaaaaa taataaaaac atttcatata 180
aatgtaaaca aaattgatga tcattgataa ttcttgttaa atgtgctttt ttaaagcaaa 240
tttatttaca tattaaacat ctgtgaattt tgataattta tctcgtagca tcatgttttc 300
tttcttcatt cgctcaagct cttggcgaag accaatattc tccttctcca agtatcctgc 360
acgtaaggct atttgatttt ctttcatgcg tcgagcatct cgagatcgct tggctgccat 420
gttattcttt ctcctctagc ccaatcttat catccttcaa attatcaggt            470
```

<210> 210
<211> 500
<212> DNA
<213> Ctenocephalides felis

<400> 210

```
ttnnnnnntt tttttttnnn ntnnttttgg acctgaaaac tatttattat cacatttatt 60
naattacata atgattacna cttaacaact attcttaaat tctagtcaga tatcagncat 120
ataataaata cttaagcttt tataagaaaa aatgcatcac tagataattt tttcagccct 180
tattcgatta attttaatgg ctttgtttaa tcaacttctt ctatcgttgg cccttgcctt 240
ccaccaaagc ctccggcttg ctgcccacaa gtttgtgctt gtgctccact tgaattggct 300
tgatgtagct tcatcataag cggcgtacaa atctgttgga actctttctc tttttgttca 360
tattcatggc attctgcttg gggattctga tctatccaag anaatatatc atcacatgcc 420
ttgcgngctg gttcacaatc ttgctgngga agcctgcttc cacatcggcc agagcttggt 480
ttacttgnaa gcatatcctc                                             500
```

<210> 211
<211> 263
<212> DNA
<213> Ctenocephalides felis

<400> 211

```
ttcatttcag gcataaattc tgttgttttg tgaccattaa taacatcaac ccctggtaac 60
atatgaccag gcccagtgta gtgccctggt tcatattcaa atttcacagg tgtgctcaca 120
tgtggagcta aaggaggagc cagagatctg agatgatttc cattagagga tggtggactg 180
ttttgttctt ggaagtatct tctggcaaac atatggtgtt tcttctgagc aacaggaggc 240
tggcgcgagg atatcgaggc ggt                                         263
```

<210> 212
<211> 244
<212> DNA
<213> Ctenocephalides felis

<400> 212

```
atggtgcggt gaaagcaaat taccattaaa atatgtattt cccacacaaa atgggaaaaa 60
ggaattttgc tcggagacct gcattgcaga attcaggaag gcttatagca agggagcctg 120
cctacaatgt gataatgtaa tccgaggaaa ctcatctacc agtcgagagt tttgttctac 180
cttttgtatg aacaaatatc aaaaaaagaa tgataagaat ctcataaatt tacccgttac 240
gagt                                                              244
```

<210> 213
<211> 418
<212> DNA
<213> Ctenocephalides felis

<400> 213

```
atcatcagca aaaccagcag tttaataatc agttaaatca acaaaatcaa caagtaaatc 60
atcagcaaaa ccaacaattt aataatcagc tgaatcagca attgaataat caacaaaatc 120
agcaatataa taatcagaat caacaattaa ataatcagca aaaccagcaa ataaatagta 180
atcagataaa tcaacaacaa aataatcagc atgttaacac acaacaacaa aatatatctc 240
aaggaagtaa gcaagtaggc cagggcaatc aaattccaca ggtgcagcaa cagcaggtgc 300
cgaccatgta gcttctgtga atcttaacaa taatattcca cataaatctg gggaaattca 360
aaacagtgtg tagacagtat acaactcctg agactctcta ttccaagcaa gacccgt    418
```

<210> 214
<211> 498
<212> DNA
<213> Ctenocephalides felis
<400> 214

```
aacgtaaaga caaaggtaaa tctcgtttca cagcttatat gctctgggct aaaggaatta 60
gaaataaaat cgtaaaagaa aatccttcaa ttgatttttc atctatatca aaaaaattag 120
gtgaactttg ggcaaatgtg cctaatggag agaagtataa ttggcgtcga agggctaaac 180
gtatggctat gaaagtagcg agagatgaag aaataaaaac atctgataat aaagcacaat 240
ttataaataa aagcaaatct aataagccct cccaattctc cacttcacca acagtttcta 300
agaaacttga tatagaagat ttagaagtga tgcctaattc ccaacaaaat tctgataatt 360
taatgttgag ccccaaatca tcagcaatga gcagcggttt atcatctcaa ggcatgtata 420
aggtaacagg aagttcccca attgacatag ctgcttatct aaagttactt ggcgagaggt 480
tgagtattat tggnggac                                               498
```

<210> 215
<211> 398
<212> DNA
<213> Ctenocephalides felis

<400> 215

gccattgcag ccactactga agttgaattc aaagaagcta ccacaattaa ggaatacatt 60
aaggatccca gcaagtttgc tgctgctact gctgccgctc cagctgctgc tgctgcagca 120
cctgctgaat ccaagaaaga agaaaagaaa gaggaatctg agtctgaaga tgatgacatg 180
ggtttcggtc tctttgatta agaacttgtt ccacaatatt aacattttgg gaaatccata 240
tttatatgaa tatttacata ttcaagtctg tttgtgatta ttatctgtaa atacttgtct 300
gaactttgcg cagttctgtg gcaatttaca agattttatt tgtaagcata actttgtata 360
ataaaatatg gatgaggata aaaaaaaaaa aaaaaaa 398

<210> 216
<211> 549
<212> DNA
<213> Ctenocephalides felis

<400> 216

gagccatctt atcaaacata tttaatataa atttataata aatcgtgcaa agttttcatg 60
ttttaattaa aataccgatt gaggtaatac gaagtacaat ggtggataat aaattagcag 120
gattaacaga ggaaaagcta cgtgttttag taaaacaatg taacaaatgc ccgaaatgta 180
atgaaatttg tctagaggat tttcccgttg ttcaatgtag tttgaaccac agactttgca 240
agacgtgctt tttggcttcc ataaatgatc cttgcttcca gtgcactaag ggcagcaaac 300
catccgctaa taaaaaagat cggccaaagc agccaaatgc cccagacaat tctttcccga 360
aggtaaactg caaatatgcc agtgacggat gcaaaatctc aaaaaagaag gacaaaatta 420
gatttcacga atcggaatgt gtgtttcaac cacaagaatg tctggaaaat tcactgttta 480
tttaattgta ctggccggta tttcaacgca tgctaccttt gtgagaacat cattaaatgg 540
aacacatga 549

<210> 217
<211> 549
<212> DNA
<213> Ctenocephalides felis

<400> 217

caaacgtaca taagaatggg agcgattatt ttaagttcag attaaattgt atatactttt 60
gattcgatgt acgggcgcaa tcatcacact cggagattgc agagagcttc aactgctgca 120
cttctcgaaa acaccaacga aaggttgccg gtggcgagcg actgcagcac aggccctagc 180
gtgcgttcgt ccagctcgaa cccgttgcaa gccagcgcga gcactagtgc tactatgatg 240
gcttccagcc gagtgcccag tccgcctctg caggaggtca acacacccgt agctgagaac 300
tggtgttaca cacaggttaa ggtagttaaa ttcagttata tgtggaccat taataacttt 360
agtttttgcc gggaagaaat gggcgaagtg ttaaagtctt caacattctc agcgggcgcg 420
aacgacaaat taaagtggtg tctacgtgta aatccaaaag ggctagatga agaaagtaaa 480
gactcctcta ctgattacta cttgatctgt acaaatctga agtcggccaa ttaattctcg 540
atttaatgc 549

<210> 218
<211> 547
<212> DNA
<213> Ctenocephalides felis

<400> 218

```
tttaacgata cacaacatga cactggcaga atcgctactt atgaagaaat agttgatttg 60
ccgcagaaac cagaagtttt acttattgat gttcgtcaac cggaggaatt ggagcaggaa 120
ggaaaaattc cgacggctat aaacattcca ttacgtgaat tggaaaatgc tctcaagaac 180
atgtctcctg aagaattcaa aaccaaattc ggaagagata aaccaacatt cgatactgaa 240
atcattttta gttgccgttc cggaaaacga gcaaaggaag ctatggaaac agcattggga 300
ttgtgttaca agaaatcaag atactacgaa ggtagctttt tagaatggag cagcaagcag 360
aagaaacagt gaaattgcag ggttaatcaa atattttata tgatacacat attatttaca 420
gatatgatca aactaagtat tcttgnggng ntatattagc atattgatat tttcttacga 480
aatgttctta agatgattta aaaatattgt gaataaattg ttacttttag attnncctaa 540
taaaata                                                           547
```

<210> 219
<211> 549
<212> DNA
<213> Ctenocephalides felis

<400> 219

```
gtgatgtcat ttgtgcaaaa gaaacaaact gaatgggaac cggagtggtc tggagatgag 60
agtataatgg cattaatgaa tggtggagaa ttatttattt atgaaataga taaatgtgag 120
tttggaacat catgggatgg aaagccaacc aaaaaaattg ggggaggtcg aaatggaaga 180
ataagcatat caccatctaa tactacaacc actttagctt tttatgtacc tggtgcgcaa 240
gctcagccat cgacttgcaa attatttgca tatcctaata tcagtcaacc agtagcttca 300
aaaagctttt tccaggctga cagggttgat tttttgtgga ataatcgtgg aacaaatcta 360
cttcttctaa cagcaactga tgttgaccaa actggtgctt catattatgg aaaacaatct 420
ttgcagtata tgagttgcaa gggtgatact gnttggtcag tgccaaagaa gggctgtcat 480
gcagttgcat ggagtcctaa gattagagtt tgngtggtac ggcattgcct gtaaactcan 540
tatcaatct                                                         549
```

<210> 220
<211> 549
<212> DNA
<213> Ctenocephalides felis

<400> 220

```
gctntttaga cgttggaatc taaataatta aataatgata ccgaaaatat tattatttag 60
taactaattt acgaaaaaag tattttttaa attaagtaac atgtcagcat cagcaagcag 120
atcggaattt gcaatcacag aatctaacca acaagaagat gaatgtggac cacaattaat 180
ttctaagata gagggtaatg gtattacaag tggggatatt aagaagttac aagaagctgg 240
atactatact gtagaatcaa tagcattcgc accaaaaaag agtcttataa ctataaaagg 300
aatatccgaa gctaaagctg ataaactatt agccgaagct gctaagcttg tgcctatggg 360

gtttacaaca gcaacagaat ttcatcaaaa acgatcagaa ataatattgt taacaacagg 420
ttcaaaggag ctagataaac tattgggcgg aggtttgaaa ctggatcaat aacggaagtt 480
tttggtgaat ttcgtcaggg aaaacacatt atgcctacat tagctgnaat tgcagtacct 540
atagatcaa                                                         549
```

<210> 221

<211> 549
<212> DNA
<213> Ctenocephalides felis

<400> 221

```
cttgaataga ttatttactt tgtccttaaa tttcaaagaa gtccctacgc gcaccaagat 60
gggtagggag gacaaagcca cctggaagtc aaactacttt actaagctcg tccaattgtt 120
ggatgaatac ccaaaatgtt ttattgtggg agccgacaat gtgggttcca agcaaatgca 180
acaaattcgt atgtctttgc gtggaagcgc cgttgtcctt atgggcaaaa acaccatgat 240
gcgaaaagct atcaaaggtc atgttgagaa caaccaggct cttgaaaagc ttcttcctca 300
cattcgcgga aatgtaggat ttgtatttac tcgcggagat cttgttgata ttcgtgataa 360
attgttggaa aacaaagtgc gtgctccagc tcgtgctggt gccattgctc cattgccgtt 420
attattccag ctcaaaatac tggtctagga cccgaaaaga catctttctt ccaagctctg 480
ncatccaaca aaatttcaaa aggactattg aatcatcaat gatgtcatat cttaaaacct 540
ggggataaa                                                         549
```

<210> 222
<211> 550
<212> DNA
<213> Ctenocephalides felis

<400> 222

```
gcgaggcgga nattgtnagt ncttttaagt attaacaatt taattaagtt tttatttaat 60
ttcgttattt ttttttaagt ttttacgtga aagactttga ggagtgattg nttatcagca 120
accaacanaa aatggggtcc gagatgaaaa gtacatgcta caaatgcaat cgcgtangac 180
actttgccag agaatgcacc caaggaggcg gaggtatggg aggtggccgg gaccgcgatg 240
gcggtcacag ggactcgggc cgcgtgcgtg agaaatgcta caagtgtaat agatttggcc 300
actttgccag agactgcaag gaggaggctg accgatgcta cagatgcaat ggcactggac 360
acattgctcg cgcttgtctg caaagcccag atgaccatct gttacaactg taacaagcca 420
ggacacattg cgaggaattg tctgagagcc gtggtntgac tccagntagg tcaaccaacg 480
tgttcaactg cacaaaactg gtcatatttc cgtactgcca gaaatgctag acttgtatgc 540
tgtggaaagc                                                        550
```

<210> 223
<211> 549

<212> DNA
<213> Ctenocephalides felis

<400> 223

```
caagttttgt ctgcaatata taaaaattta agatggctaa agcaccagca gttggtatag 60
atttgggtac tacgtactcc tgcgtgggtg ttttccaaca cggaaaagta gaaattattg 120
caaatgacca aggaaacagg actactcctt catatgtcgc gtttaccgat acagagcgtc 180
tcatcggaga cgccgccaag aatcaagtgg ccatgaaccc caataacaca atttttgatg 240
ccaaacgtct tattgggcgt aaattcgagg accaaacagt ccaagctgat atgaaacatt 300
ggcccttcga ggttgtcagc gatggaggta aaccaaaaat tagagtatcg tacaaaggag 360
aatccaaaac cttcttccct gaagaagtca gttccatggt gttgactaaa atgaaggaaa 420
ccgctgaagc ttacttaggc aaaactgtga ccaatgctgt cgtctgacct gctacttcaa 480
tgactcacaa cgtcaagccc caaggattcg ggactatctc cgtctgatgg tgagatataa 540
cggcccccg                                                         549
```

<210> 224
<211> 550
<212> DNA
<213> Ctenocephalides felis

<400> 224

```
atacgatagg catttgtgcg agataattga ttattgtgtg aaatttatgt gaataaatct 60
gtgaaccgct ctgtaggtta cgatttgtgt tcaatgttaa aagaaatgtg caaacagtga 120
attcagatct gtacaagtga catttgcatt taacaaatat ggggaagaca tactgtcaat 180
cttgtcgcaa aaaatgttca ggtgaagttt tacgagtctc cgacaaatac tttcatactc 240
aatgcttcca gtgcgtgcag tgcaataaaa gtctcgccca aggggggcttc tttcacaaag 300
acgataaata ttattgtaca ggagactatc aaaaattatt tggtacaaaa tgcgcagtgt 360
gccagcaata tgttgaaggc gaagttgtat cagctctagg aaatacttac catcaaaaat 420
gtttcacatg tctagttgtc gtcaagctgt gccgccggag aaaaagtaac atacaccgga 480
aaagaagtcc tctgccagaa atggttcaaa tccagcagac aagtcaacag tcacagaaag 540
tccntagtca                                                        550
```

<210> 225
<211> 549
<212> DNA
<213> Ctenocephalides felis

<400> 225

```
ttttcgtgct tgtgttattt tgctacatgn ngctgtctgt ctggttgcac gaccatacct 60
acagtaaggg ctattcggag ttgacaaaaa actcagatgt cgaattcatt gtagaagaac 120
attattcgga atccgattcc gatgcatcgg tgcactgcga aagagataat aaaatcggtt 180
cgtcgcaaag acaggaacaa catgtgataa taatccaaaa atctgctgat gacatacacc 240
gatgcgaact gtgcaataag acgtttaaat ttgcaacaaa tcttaaggct catatggtta 300
tgcacagcgg cgaacggccc tatgtctgtg ccatatgcag taaagctttt ccacgaattg 360
ccaccttgaa ccgacacatg caagtgcata ctgaatttaa accatttcag tgtgaatttt 420
gtgagaagag atttcgacaa gatgtnactt tgaagaacca tatcangact cataccggtg 480
agagattaat tggcatggtg tgaaaatctt ttaccgccta ccactctgaa cacattacga 540
tcacatgag                                                         549
```

<210> 226
<211> 498

<212> DNA
<213> Ctenocephalides felis

<400> 226

```
tgaaagagat gcaggcattg tggttgattc ctccggaaga ataaaaagtg taatttcagg 60
agaagaagtg gaaaggatag ctaacgaaac taaagttgag gtgttggact acggtcaatt 120
ttcaatatgg ccaggtgtga tagactctca tgtgcacgtc aacgaaccag gaagagaatc 180
ctgggaagga tacaccacag ctactaaagc agcagcttgg ggcggaatta ccacaatagt 240
agacatgcct ttgaattcca tcccacctac aactactgta gagaatttga gaacaaaagt 300
gaattcagcc tgtggtaaaa cgcatgttga tgtcgctttc tggggaggcg tgattcctgg 360
caatgcgcac gaattgttgc cacttatcaa cgccggagta agaggattca aatgttttac 420
aagtgaaagt ggtgtcgatg agtttccaca ggttactaaa aatgatctgg aaatggctct 480
aaaagagctc cagaaagc                                               498
```

<210> 227
<211> 498
<212> DNA
<213> Ctenocephalides felis

<400> 227

```
cagccgttat agcggttgaa actgataata tccaaatgtt gtccaacttt atcttcatcg 60
actgccctcg acaatgctat tgttactggc cgtgaaacat ctaaactttt cacgtgatca 120
acgatagccg aaaaataatc atccgatgcc gtaatctgag ttctaggttc atttgcaatg 180
gaccacatta tcactcctgg gcgattctta tcacggcgaa ttagctcgct taacgacatc 240
ttgtgcttag caagcaattt tgaattgtaa ttctctgtat ctacacttgg acattcattg 300
attatcatga tgccgttttt gtccgccagg tccataactt cttccgaata aggataatgt 360
gatgttctat atgagttggc cccaatccat tttattaagt tgtaatcttt tgcaataata 420
gccaaatcga gaccttttca cgtatgtcag agtcttcatg tctaccaaaa cctcttagat 480
ataatttcct gtgattaa                                               498
```

<210> 228
<211> 498
<212> DNA
<213> Ctenocephalides felis

<400> 228

```
atgtccttga agtcctcgag ccgatgcaag ggtcccatcc agaccgcata ttcctccggc 60
aatctcggaa caaaaagcgt gcttcgacct gttccgacct caacggcccc gaacagatcg 120
ggttccttag cgccaaacat gtattggaag taggattcct gtttaaaaat gtagcccaca 180
tccgtgtcgt tccagctgac ctcttcacct ccttgcagaa ggataatgga tttttttaag 240
gaacaacttt gtgccgataa agcatcgcac accttcttcc tgttctcggc gaataaactc 300
atcggaactt tcagcgtttt tggtcccatg gagaagaccc gctgctgacc cgatccgttg 360
gagcacttgc atgttgattc tgccattatt cttttaaact ataacactaa gcgatttaca 420
caatttaatt aacaataatt actaaaaaaa ttaaattaaa cacatataac caaaaatttt 480

ctgtcactga acataagt                                               498
```

<210> 229
<211> 498
<212> DNA
<213> Ctenocephalides felis

<400> 229

```
aacagttgca tgaactttgc ccttcaacgt cattcctaag tatggtgttt ctttattctt 60
gtgttggatt atgtctttag tgaccttaaa ttcttcctcg ggatcccaaa ttactaaatc 120
agcatccaag ccttctttga tttgtccttt tatgccatcc agtccagtta atcgagcggc 180
cgcccgggca ggtacttgag ttccccatcc aatcaatgtt actgcatcac ctttcttgat 240
aatatcagct tttcccaaag gaactgtgaa atcatcgaca ggaacttctt ctacagctgc 300
tctgtaaagc gtttttggtt ccagtagcaa acatggatct ttttctctga tcatggataa 360
tagaagacct ttggccatat ttggacctct tggtatagca atcttcaatc ctggagtatg 420
tgcaaaatat gcctcanggc tttgggaatg ataacaagcc ccgtgtccga ctgcatacaa 480
ngtgctcgca ctgttaaa                                                498
```

<210> 230
<211> 237
<212> DNA
<213> Ctenocephalides felis

<400> 230

```
ctgctgctgc accgggaaca ccccctgata ctggcgtatc acaaaattga gaattatgtt 60
ggttcaccaa ttgtttaact tctttggcaa ctgctggatc gattgtgctg gaatctaaga 120
acaatgtatc ttttttggca gatgccacca ttcctttgta acagtctaat actatggcgt 180
tgtttggcaa cattgtgacc acaaagtcgg atttttggc aacttcggca gccgagg    237
```

<210> 231
<211> 171
<212> DNA
<213> Ctenocephalides felis

<400> 231

```
tgcttggaga aagtgcagtt ggaaaaagtt ctttggtgtt aagatttgtc aaggggcagt 60
ttcatgaata tcaagagtct actataggtg ctgcgttttt aactcaaact gtgtgtctgg 120
atgacacaac ggttaaattt gaaatttggg atacggctgg acaagaaagg t          171
```

<210> 232
<211> 315
<212> DNA
<213> Ctenocephalides felis

<400> 232

tgatagagtg catggccaat atgaatatcc aaaaatatta taggcctctc attacgatca 60
tcatattgcc caaaagtggg tccgtggtct cgtcgatgaa aaaattatgg tccacaaaat 120
gatcaaaatc tttcacgccc atttgtctga ccagctctgg atctcgtatc agcaatacag 180
gttttaaaac ttcatagaaa cctgaaaatc tgctatcagg aaaattctca taagctcttc 240
cgtaggtata aatctgattt tccaactgga aaaagtttct ccaggtgctt ccaaggatcg 300
gcacaggttt caggt 315

<210> 233
<211> 247
<212> DNA
<213> Ctenocephalides felis

<400> 233

agaactactt gcatcttttt taggatgtct tttgccacac attctgcgga acatcacttg 60
agcaataaat attcataatt ttgcgaattc cttgatttta aacaagcgca gacataatgt 120
atgtatatat gtatatatat atatatatat attgaatggt agagtcttgg atttgtgtat 180
attttgtaga gtttcgaaca agaatccaaa aataatacaa aaaaaaaaaa aaaaaaaaaa 240
aaaaaaa 247

<210> 234
<211> 330
<212> DNA
<213> Ctenocephalides felis

<400> 234

tggtgaagaa tttgccggtc gcctagccaa agaaggaata cgatataaat taaagggaat 60
ggtagcagac ccagaagaat gtgatatgga agaattggtt agcatgaagt ctataccaaa 120
ttcacttgcg gtgttctgct tagctacgta tggtgaagga gatccaacag ataacgccat 180
ggagttcttc gaatggatcc aaaacggtga cgccgatctc acgggtctta attatgccgt 240
ttttggactt ggaaataaaa cctacgaaca ttacaatgaa gttgcaattt atgttgataa 300
acgattggaa gagttgggag caactagagt 330

<210> 235
<211> 417

<212> DNA
<213> Ctenocephalides felis

<400> 235

aattaaatgc aattatatca attctggcat aaatccaagg cactattttg actattttct 60
aattgcaaaa tatcaagtgt ttaataatgc aaaattttct taacagcatc aaagcaacgc 120
catttgtcgg gtagataaaa cggttcaaag acatgaggga atggtgtatc gtggccggtc 180
actctttgaa taggagcctc gagatgcaaa aagcattcct cctgaatgga agcagccagt 240
tcagccccaa agcctccagt gtatggagct tcatgtgcaa taatgcaacg accagtcttc 300
tttactgaat tacaaacagt atcataatcc catggtagta tcgaaacaag atcgataact 360

tcacaattag catcaagttt ttcttttaca atctcagcta cttctcgtaa aacatgt 417

<210> 236
<211> 112
<212> DNA
<213> Ctenocephalides felis

<400> 236

ctggtttgca agtcattcca ctcggacatg tttccaatgt tgtttctaag aaatcagngc 60
caatcgcaat gcaaaggttt gctgtctcac aatcctggca ctttatttct cc 112

<210> 237
<211> 325
<212> DNA
<213> Ctenocephalides felis

<400> 237

actgtagtct cgtgaaattt gtttattaaa atccgaaagt aatggataat tcaaaccacc 60
taagccgcct acttttctat ccatgttaat ccatgctaag tggctaaaat gtgaatccgt 120
tgagcatcca acaacctcag caccgatttc ccgaaactgg cctattgcat cactataagc 180
acgaatctcc gttggacaga caaaagtaaa atctaagggg tagaagaaca ataccaaata 240
tttgccttta taatctgtta agctgatctt cttaaaatca ccattgacaa ctgccatacc 300
ttcaaaatga ggggctggac tttgt 325

<210> 238
<211> 500
<212> DNA
<213> Ctenocephalides felis
<400> 238

tgaagccctn tgtgccggat gcgaggactg tgcaacggct taggtcctgc catgtttggt 60
gttatatttt atctttttca tgttgatttg aatgaagatc atcttggaaa taagatagaa 120
agaaatgaaa ctgctggtgc agatgattat atacaattag ttcctgggcc tccatttgtt 180
tttggagcat tgcttgtaat atgtgcactt ttagtagcag ttttcatacc agaagagcac 240
aaagatataa gccgacgttc ttctggtgtt tctttagata cacattttga aattgaacgt 300
ggcagaaaag ttgcaagtcc tctaccacca ttgattcata ccgattctgc acagctataa 360
acatcatata ccatcaccac ctatgcaata caattttatt aataatgaat aattaattta 420
aaactagttt gaaacccaat ggttaatgat aatgaaataa ccagatacaa attacagata 480
ttttaaatga gatgtcatac 500

<210> 239
<211> 252
<212> DNA
<213> Ctenocephalides felis

<400> 239

```
acgatacact catcccattt tatacagaaa aacttgataa aatagcgaag gctaataatg 60
gccatttagc tcttggaaga ctaacttggg ccgactttgt tttcgccgga gttatcgaat 120
atatgagttt catatctgga acagatttcc ttggaaaata tgcaggattc aagagtgtct 180
ttaataatgt tgcaaatttg ccgaacgtga aggaatggat cgccaagagg ccaaaaactg 240
atttgtaatt gt                                                     252
```

<210> 240
<211> 500
<212> DNA
<213> Ctenocephalides felis

<400> 240

```
tttttttttt tttttttttt tttttttta ttcaactcat caattacaac atcaacaata 60
gtattaacat cagccatagc accattacca gtatcaccgc acaccaaagt cttagaaatg 120
caaggcactt ntatataccc ccaatctttt aaagttttta cttgtggagc ggttatgggg 180
tgatcccaca ttctcgtatt cattgctgga caaaacagga ggggcttgga tgtgttccag 240
gctcgaactg tgcaggttag gaggttgtca cataaaccat ttgatatttt agccaaagtg 300
ttggcgtcca acggtgcaat gcacatgatg tctgcccatt tagttaaatc gatgtgtaaa 360
acagggtcac cacgtttgtt ccaaaatttc cattcatggt catctgtgta tattttgacg 420
gtttggggta tttctgattg gtcgaagaag tgttttgcat attctgttac tataactgaa 480
tttctatatg tatattggag                                             500
```

<210> 241
<211> 498
<212> DNA
<213> Ctenocephalides felis

<400> 241

```
aattttattg tatttatata cagataaaaa ttgatatcaa gttagataac aaaatataaa 60
atttaaaaat caaaacatat ttataaattt ttttggtctt atttctagaa tttattcagc 120
attagaaaca ttttgaataa ttttcttgaa tattgtgata cctcgaagga aagtttctgt 180
atctaaatat tcatcatggt catgcagcaa tactggcgtg ttattcatgg gagaaaaacc 240
aaaagccggc agtccaattt ctcttatata tctgctatct gttgcagctg gaaaaacttg 300
tggttcaaga acaagtccca tttcatcagt agctctttta aatgctgtcc aaaatttatt 360
gctttcattc agttcagtgt ttgctatcag tggttctttt tgtcaaattc tatttcaaca 420
tccttgccag cttctttgca ccattttcgt atcttttctt ccaattcctt gtggctaagg 480
atggtgtaat acgtatat                                               498
```

<210> 242
<211> 500
<212> DNA
<213> Ctenocephalides felis

<400> 242

```
atagtcttgc ttacatgaaa atttgatttt ataaaaatat ttgctattat gcattagcaa 60
gtagcatttt tgcacataaa tcatacatat tttaccatat gttggttata ttacattatt 120
aaagcgttaa ctccatacat ctgagctata tcttttctgt gcttccattt tcttgatgta 180
agcttgagct tggtgtcatt catgttgcct ttggacataa tcactttcaa aactatgttg 240
cgaacatcac ttgccatagt ccgggcatct ccgcaaatat ataagtggcc attattttca 300
ccaatgacat tccataactc atcaccattt ctctctagta aatgagtgac atattccttt 360
tcagctttat cccttgagaa tgcaacatga attttaagag cgcccttatt aatatattct 420
tgaagntcct cttcatataa gtaatcttca gacttcttgc gcaaccaaag tataagatag 480
tatctccaac agttttatct                                             500
```

<210> 243
<211> 364
<212> DNA
<213> Ctenocephalides felis

<400> 243

```
tttttttttt tttttttttt ttttgggagt aaagcacttt taatgcctgn tttctttagt 60
ncttctatta agtctccaga ttgatgcatt tgcaacatga tgtcacaacc ccctacaaat 120
tctccagaaa tataaacttg tggaatagtc ggccaattgg agaaatcctt aataccttgt 180
cttaaaacat catccttcaa tacgtcatga ctctcatatt ccacaccgtg catcctcata 240
atttgaacca ctgcattgct gaaaccacat ttgggggctt caggaacacc cttcatgaaa 300
actaccactt ttttgtcttt aacaagcttg tttattctat ctgagatcgc tgttgtgctg 360
cagt                                                              364
```

<210> 244
<211> 535
<212> DNA
<213> Ctenocephalides felis

<400> 244

```
rchsncnamt ydnachcksm cratndatst randsncatt tgtcttcaaa ctgtttggag 60
atgacagtta ctcggacatt ctcaggtata aattcatcaa caattgactc tataagttta 120
gggtcccagt gtgtcattag cgaaggaccg caaagaacct cttcaaattt aaaatgcatc 180
agattcttga ctagataaac aacataactt cttggttgtt ctttatctct gaacttaaag 240
ttcatatcgn taattgcctt tgattcatca aatatccatt tttgtggtgt ttccttttttc 300
agcaaattaa tatactgaaa aactagtctg actatatcat caacatggtt tacgccctct 360
tctgttaaat ctgcatctag tttgaagaaa ccaaatcccc tcctctgaac agcaccgcaa 420
gataagcttg aactccaacc acgtgctttc aaacatgata agagactgcc gggtccctca 480
tgcccaaaaa gatgacctaa ataatttcct ggtgcagatt ttctaaaagg ctcaa      535
```

<210> 245
<211> 497
<212> DNA
<213> Ctenocephalides felis

<400> 245

```
ccgactgcac ctgcttctca caattatgat ggtggtttcc aaaccaaatt gatgcacaaa 60
gacttaggct tggcctccgg agtggcatca gcaaccttga caccaattcc tttgggaact 120
ttggcacatc aacttttcac aactgtcatc gcccatggtc ttggggacaa agatttctcc 180
ataatttacg acttcatcca ggaacaagga aagaagaagg cataaattga aaaataaaaa 240
aaaaattgga tactgttgaa ccagaaccag aaccaatttt ctcctaaact aacataatat 300
ggtgcctttg attaaatcaa taccaatggt aatagaaaat ttaaaaaaaa ttccagagat 360
ccaaaattaa ttaatccatt agagtgcctt cgataaaatc agtgccaatt gatgcaaatt 420
caaaatttaa atatgttttt caggataaaa taaaaaaatt actngttata aaaaaaaaaa 480
aaaaaaaaaa aaaaaaa                                                497
```

<210> 246
<211> 306
<212> DNA
<213> Ctenocephalides felis

<400> 246

```
ggaaagagtt tggagagctg gatctccggc gatncatcag gagactacaa gaaagttctt 60
ctggacgctg gtcaattaat taaataacaa aaacaaacaa taataggata aactcactta 120
aatgcaaaat cagtgtaatt aaatccatcg atcaatatta ataataatat ataataaaac 180
acaacacata atcagataaa ggacantttt gttaatcgag tagaaaattg aatcantttt 240
ctattccaac antttatttt tattatgtgt tgtgttttta attgttagtc tttttgcaca 300
aaaagt                                                            306
```

<210> 247
<211> 498
<212> DNA
<213> Ctenocephalides felis

<400> 247

```
attctggctt ggactacaca atggaaaggt ctgcaagagc tatcatgaag accgcaatga 60
aattcaattt gggattggac ttgagaacag cagcatatgt aaactctatt gaaaagatct 120
tcacaactta tgctgaagcc ggtttagcct tttaagatta attatagcat tttacttata 180
gataacgata ccagtgaaaa atcattacat caataagctc atagtgcaaa gttccttgcc 240
agtttaattt gttttacata cttaaatcta ttaatctgta catgaaaaaa tcactcatta 300
gaacataatg taaatgttat aatatttgca caatcaaagt aataatgtaa gcacgcaata 360
tgtgaagaac gtgaatggta ataatcttta tacatttatt catttaatat aaatgaataa 420
atgtgtaagt ttgaattatg gtactttaat atgnattcaa gcataaattt cagcgtgata 480
attttagttc gcgcgtcg                                               498
```

<210> 248
<211> 549
<212> DNA
<213> Ctenocephalides felis

<400> 248

ctnagctcga ataatttcag tattatcacc caaaagttaa tttaaaatgc cgacatacaa 60
gttgacatac ttcaacgtta aggctttggc tgaaccattg cgcttgttgt tgtcttatgg 120
aggagccgat tttgaagacg tcaggttgga gaaggaaaac tggccagcag ttaaaaacac 180
attcccattc ggacaagtcc cagttttgga aatcgacggt aaacaaataa accaaagctt 240
ggcaattgct cggtatcttg gcaaacaatt caacttggga ggcaaagatg ccttagaaga 300
tttggaaatt gatgccatcg ttgattcaat gaacgatttc agactaaagg ctgctgtcgt 360
tatttatgaa caagatgaag cagtgaaagc caagaaagtc gaacaattga ccaaagaagt 420
agtaccattc tactttgaaa aattcgaggc tatcgccaaa aagacaatgg catttagctt 480
tgggaaaatt ncctgggcag attcgtgctg ctgtcaattc gaacatggac ttatggccga 540
ctgttattc 549

<210> 249
<211> 498
<212> DNA
<213> Ctenocephalides felis

<400> 249

ttattatttt gttatatgtt cttgatgatt cttttggtat tctggttctt tctgganccg 60
tgatgtttac gtaatatata ccaaaacgct ctgtgaatcc acgaagccat tcaaaattgt 120
ctataatgct ccatagggca tagcctttaa cattgcagtt atcaataatt atggctttga 180
gcatttcatt caagtaacta caaatgtaat gaactcnatc atgatcatcc aattgaccat 240
gatctgagta tccgttttcg gtaactatta tttcaattcc agggtattca ttttgaaccc 300
attttaaaag ttttcgaaat ccttctggaa caacttttaa ccaagatgat gctgcactgg 360
gccatgaggg gtccgattct aattttactt tctgatcagt ataccaggtg ttcggctttc 420
ctgaaattgc attggatgct aggcgagatg tataatggtt taatccaaga aaatcagcaa 480
gtgcctttaa tatgatca 498

<210> 250
<211> 498
<212> DNA
<213> Ctenocephalides felis

<400> 250

ttccgatctt tngtttatga caccatatca tctcgagaag ttacaggaac tgcaagatat 60
gatatgatgc agttgttaat tcaagctaaa aaaggaactc ttcaagacga tgatttaggg 120
gtaactgctg cacgaaataa agaattaaca ggcgatgata taacatccca agctttantt 180
ttcctcataa ctggagtaat aacttccact tcaacaatgg gtttcggggc ctatgaaata 240
gcaaacaacg aagaaattca gaaaaaattg atagaggaag tagatgaggt tagaaagaag 300
cataaaggtg aattatctta tgagataatt gataagatgg attatttagg cagagtaata 360
tcagaaactt tgcggaaatg gccgccggga attattgcta ggaattgtgt gcaaccttat 420
acaatcacgg ataataaaaa tagaatcaca tttaattgcg atccaggagc tgtnatatat 480
gttcccacaa tagccatt 498

<210> 251
<211> 175
<212> DNA
<213> Ctenocephalides felis

<400> 251

```
cattctactt tgaaaaattc gaggctatcg ccaaaaagaa caatggacat ttagctttgg 60
gaaaattgac ctgggcagat ttcgtgctgg ctggtcaatt cgaagcatgg aactttatgg 120
cccgcactga tttattcgcc aacacccccct cattgaaggc tgttatcaac aatgt     175
```

<210> 252
<211> 549
<212> DNA
<213> Ctenocephalides felis

<400> 252

```
ctttgacctt atatcatttt gcaccaagtt ttccgtcacg agcagcgctg ttaactatta 60
gagcactaaa tcttaaggtt gatataaaag aagtaaatct gtttgctaaa gagcaattta 120
aaccagaatt tttgaagatt aacccacaac attgtgttcc aacattagat gacaatggtt 180
ttgttctttg ggaaagtcgg gctatagcta cttacttggt ccaggcttat gggggtgaaa 240
aatacagttc cttgtatcct caagctgcta aagaaaaagc agttgttgat cagagattat 300
actttgatgc tggagtttta tttcctagaa ttcgagccat ttgctttcct attctatttt 360
tgggagaaac tacgatttca caagacaaaa aagaccaact aaatgaagca tttggttatc 420
tagatggttt tcttgcnaaa caaagtgggt agctggagac aactttaccg tacagataat 480
gcattctggc ttctgttcta gcatacaagc tgtggttcga tatttcnaat ccctctgtgc 540
tctgggaag                                                         549
```

<210> 253
<211> 549
<212> DNA
<213> Ctenocephalides felis

<400> 253

```
atanattaga aaaatgttag caagaccctt actgccacaa aatccaaaac aagccccttc 60
acattctgaa tttgtgatca gtgaccgtgg gtatggcaaa gattttgtaa aattattaca 120
tgttaaacga gatggagaaa cacatcatat tagagaattt gaagttggaa ctcatttgaa 180
acttgcttcg gatgtagatt acctaaaggg tgacaatgtc gatatagtag ccacagactc 240
tcaaaaaaat acagtgtacc ttttggctaa ccaacatggt gtgaataccc ccgaagaatt 300
tggcttgtta ttatgcagac actttttgca cacctacccc catgttctag aatgcagtgc 360
cactgtggag atgtaccctt gggaaagaat taagggtagt ggtcaaccag aaaggcagca 420
taagcatgct tttattttta acccttcggc agtcagacat tgtgtggtac acagaaaaaa 480
tacgaactcc ggcgtcgaag ttgtttgaag gatctcgtgt ctcaaaacac gcatccgatt 540
cctggntcg                                                         549
```

<210> 254
<211> 549
<212> DNA
<213> Ctenocephalides felis

<400> 254

gaattattat catttgcatt cgctttactt ggcttgcata agtacgattg atttggtgct 60
aattcgattc agttttgcat cagtcacagt caaagtcggt aataaaatat ggttactaaa 120
gcagtttgcg ttttaaacgg ggaagttaag ggaaccattt acttcgatca aagcggtcca 180
gaagcacctg tcacactaac aggatgcgtt agtggtttaa gcaagggtga tcacggtttc 240
cacatccacg aattcggtga cagcacaaat ggatgtattt cagccgggcc acattttaat 300
ccccacggta aagaccatgg aggacctgat tctgctatca gacatgtcgg cgacttggga 360
aatcttgtag ctgatgccga tggaaacgct aaagtgaaaa taaccgacag tcaaatttcc 420
ttacaaggtc tatgagcgtt ataggcagaa cattggtgtc atgctgatcc cgatgatctt 480
ggcttangtg gcatgaactt acaagaccct ggnatgctgg actcgatggn tgngtgtatt 540
ggattgcaa 549

<210> 255
<211> 502
<212> DNA
<213> Ctenocephalides felis

<400> 255

tgatgtttgt tttatgatgg atactcattg ataccgctaa taatcaaatt agcttgttga 60
ttcagtccag ttcagtcgat tcatttgtcg gcctgtgtga ccttttattt ttcgatacat 120
tgctacatat taaagattgt taacaatgcc agcctacaag ttaacttatt tccctgtgaa 180
ggctctngct gaacctttac gtatgctaat ttcttatgga ggagaagatt ttgaagatta 240
ccgattcaat cgtgaagatt ggccttcaat caaaccaact atgccttttg gtcaagtgcc 300
agttcttgaa tgggatggcc gtaaaatgaa ccaaagtgtt gccttgtgtc gttacctagg 360
aaagaaatac aatcttgcag gaagtactga tcttgagaat cttgaaattg atgctattgt 420
ggacacagtt catgatttta gagcaaagct agctgcagca cattacgaag cagatgaggc 480
agtaaaaaaa aaaaaaaaaa aa 502

<210> 256
<211> 549
<212> DNA
<213> Ctenocephalides felis

<400> 256

gtttaagaag tagtagaaac agatttgtta aagcagttac aaatggcacc tattggagaa 60
ccagaatatg atcttgtggt tatcggagga ggctctggcg gattagcttg tgccaaagaa 120
gcagtaaatt tgggagctaa agtagcagtc ttagactatg tagttccttc accacaagga 180
acaaaatggg gcttgggtgg cacatgtgta aatgtgggat gcatacctaa aaaacttatg 240
catcaagcat ctttacttgg agatgctata tttgattctt ccttttatgg atggaaattt 300
gataatcctg agaacacaaa acatgattgg ttttcattaa cagaagctgt tcaaaatcat 360
atcaaaagtg tcaactgggt cactagagta gatctacgag acaagaaaat cgaatatatt 420
aatggctagg atactttaaa gatcaaaatc tattatagct gattaaagaa taaaagtgaa 480

aaaatattgc tgcaaaaatt tgtatcgcag tggaggacgg ctactatccg atttcctgga 540
cactagagt 549

<210> 257
<211> 441

<212> DNA
<213> Ctenocephalides felis

<400> 257

```
catgcaccac tgtggcggtc ttgatgtgaa tcaaattcac tcgttaattc actttgggct 60
ccaaaggccc tcacagtcga caagccagct aacgatgccg caaggtgaga atatgcggga 120
cttcgggtta ttccttcaag acgtttaata ttttttgatg tttttaagta tattcttctc 180
acaaatatga aaactactcc aagaacgccg acagctatca aaaataatgg atttacagtt 240
gccgttaata ttaaagcgcc aactatagtc agtattatct gtgatgaatc aagcagggct 300
ttaggtaaaa attcatcaat agcgcccatg tcttttgaaa atctgttcat aatccgtcct 360
gaagggtttg tatcaaaaaa tcgcatagtt gtttgtaaaa ttcctttgaa catagaatca 420
tgtaatcntt gagaaacccg t                                           441
```

<210> 258
<211> 438
<212> DNA
<213> Ctenocephalides felis

<400> 258

```
aaggcgcgat aacacagaac gataatgctt aggatatcaa taaagtaaaa ttaacactaa 60
taatgtcgat tctaatcaat catcagttcg aaatggacgg atccttgtcg atccttgttg 120
ccaccacgat agatgatgtt cctggcccag gcacgacatt ccacgttgat caaacgtccc 180
actgtaggtc ttttgaattg aacagcaact aatggactaa gataatctcg ttggtttctg 240
aatggataat aatatccagc aaatcccatg cttggataca tttcaactgg gcccaaattt 300
tctcgatcaa ctggattttc tccttgacac gaaacccaaa tttgttttct ctcatccggc 360
ttgagacttt gtatgtgatc cttcaattcc ataggcatat cttcaggcag cgtcgccaca 420
tcatcataat aatccggt                                               438
```

<210> 259
<211> 323
<212> DNA
<213> Ctenocephalides felis

<400> 259

```
catcgcctgt gactgccacg acttctctgc tttcactgac attgctgtcg ataatacctt 60
tgaccaatgt gtatttgtca gtgggcgaag atcttgccaa aacacgtaat ttaggccaca 120
ctttatccag cagatgttgt tggacatcac cgttgctgtc tctaatacgc ctattgaatt 180
cttttccttc taaaatcaga aaatcttctt ggggtttcaa tattccacat ttcgtagcaa 240
tagaccttgc agtgtttata ttatcaccag ttaccatacg aacagttatt cctgcttttt 300
gacatttacg aatagcatca ggt                                         323
```

<210> 260
<211> 475
<212> DNA
<213> Ctenocephalides felis

<400> 260

```
ggcagctcta ggaggatcaa tcatagacat gagtccaaca aatctaaggt tttcaattgg 60
gaagttggga tcatcgctgt cgaatttgaa acctaagggg aatttgtctg taggcaacat 120
gagatcacaa aagcccaata cacgctctcc aagaccgccc aattccagat atgcattatt 180
gaaagcttct ttcatctctt catccagtag tttttccttt cctccaatga agatggtgga 240
acatttttct aagattcttt caggagctcc tttcataacc attacatgac gggggtctga 300
cgcatcttca gtttcgtgaa tggaaacctg gtatttgttt gtggagttaa atggaatttc 360
acagactttc ttatttcgtt ttcgaataga cataacatct cctaaagcca gttccataca 420
tttgagaaga gcagcttcag atgcatcanc actgacttct tttttcaaga tgggt      475
```

<210> 261
<211> 498
<212> DNA
<213> Ctenocephalides felis

<400> 261

```
acaaaacaaa atttgatcac aagccttgcc actttttttg tatcacacag cctgaaaata 60
tgctgattta gatatatgaa agataaatag ataatttttt tcattcatga taatctgcct 120
tttttatatc tagaatatta tttattaatt ttgattcgga atatggctta gattttaaac 180
ttgtcacctt ttttacagtt catgctcaat ggctaaacat gttcatgtct tttagattat 240
ttttttacat gcttattcag actttctatt ataacatgcg tcacaaaccc ttttaggttc 300
atgccaatcc cgttctggaa caggccttct tttcaaagag catttgttgc aaacagcttg 360
accgcaagca cgacaatggt gccttctttg atatgttaaa aacgtccgtt gctggtctga 420
tgttgcagcc atcattgggc ctgttgtatt taaatcacta ttaatgcttg tagtagtana 480
attacattca gaatcgga                                               498
```

<210> 262
<211> 279
<212> DNA
<213> Ctenocephalides felis

<400> 262

```
ttcaagctat tgcaattact ttgtgtgttg ttattggtat aacactcttt acattacaaa 60
acaaactgga tttatcaatg cttccagcag cattgtttac tggactttgc tgtttattgg 120
taggtggtat cattcagata ttcactcatt caaccatttt tgaattagtg ttatgcagtt 180
ttggtgcact aatattcagc ttgtttttgc tttatgacac gcatgttatg atgacgacat 240
tatcaccaga agagtatatt ttggccacaa ttaacttgt                         279
```

<210> 263
<211> 344
<212> DNA
<213> Ctenocephalides felis

<400> 263

aacctaaaaa taagcaattt acatacaaga tcgacacaag tcatgcaacg ggaaatattt 60
taagaactta taaccgaaaa agtagtgggt tgcgaaatag gccactgtgg gcactcacca 120
tttgccctgt attcgtcctg gactttgtag aattctgcta attccttacc cgagcacaaa 180
ggagtatcga cttgggtagt ttcgttaaat ctcgaataat cgacttcgta gcattgtctt 240
tctttattgt agttgacgac tgggtcgaac ctgtggcccc acaatatttc cgtattaatg 300
taacttgatc gagcttgtgt ggtctgtcct gtagattcaa tagt 344

<210> 264
<211> 477
<212> DNA
<213> Ctenocephalides felis

<400> 264

attaatttaa gaatctaatg gacatctttt gttcaacatc tgtgcgttcc aaagctttac 60
aaaaatcatc aaatgagatc atttggtcac cattttgatc agcttctaga atagttcttt 120
cggcaatgct tgacaattgt tcttcactta tatttgcacc caccatcatg tgtaaaatgg 180
caagaagttc atcccgagat atcatgtcat cattgtccaa gtcatacatt ttgaatgcaa 240
atctcaattt ttcttccctg ctgttcaatt tattttcccg attcttctta atgggtctga 300
aatgtgctaa gacttgcata aactgtaaga aattcaccct gtcatcatga ctttgagcaa 360
aaaatgcatg gacaatccta tcacctaagg gatttattgc gagttcgggt attctcaaaa 420
aatcatctcg tgaaagagtt ccacagtctc cacgatctag agatgtgaat cttgagt 477

<210> 265
<211> 377
<212> DNA
<213> Ctenocephalides felis

<400> 265

cnaactttgt cctgttatcc catcgatcat gcgtctagca ttggctatgg ctagatttaa 60
gtccctcctc anaacaaatc cnacaagata ctgagattct ctggaaacaa caactggata 120
tccattgtgc tcagntnctt tcantaaacc ttcaacatca tccacagtca tcgagtcttg 180
agtgattaca cttaatgttt cattcctctt gggttgcatg acatctgcag ctaaagatgt 240
atgtgcaaat tcatctttac tgccaagaat ggatatccat taagctgtat atgggcatca 300
tatataccct gtctgccaaa gcatcaccaa cccatttgga agccatagct gctgccatta 360
agggcacgat ataacgt 377

<210> 266
<211> 222
<212> DNA
<213> Ctenocephalides felis

<400> 266

tttctttagt ggtgcactgt agatatccac tttgatcagc gcacacttga tctgggctac 60
aggattcctt aaccggatct gctcctattt ccggacaata ctgcatttcg ggtgaactgc 120
acgaattcac gcaactaaat tttacttttt tacatttttg ctcgggtttc ggtacaaaat 180
gctatacagt tacatgtcct gcacagccta aggtgcttcc gt 222

<210> 267
<211> 209
<212> DNA
<213> Ctenocephalides felis

<400> 267

```
cnaaagaggn ttcaggaagc tcttcttcgt cncctgattt tgganccega gttggttcaa 60
ccaattcacc aggatcgtga atctaatgaa catgaagaac aattcaacaa gcaccatnat 120
nacaagcanc acaaagctga ttatatgaat cacatgagag tcaccgacac ttcngcaatc 180
atggctggtt cttttgctgt gatagcggt                                  209
```

<210> 268
<211> 178
<212> DNA
<213> Ctenocephalides felis

<400> 268

```
aaccaactag agctaagaaa aaagccagcg atagcataga actgctatgt tcccctccaa 60
ataccactgt tgttttatca tataaaaatg ccatangtat ggagctaatg catccaatca 120
ccatcaatat atatatatat atgaagagtc ttttatcctt cctgggtaaa gtttctgt   178
```

<210> 269
<211> 238
<212> DNA
<213> Ctenocephalides felis

<400> 269

```
tttgttttac tttatgtgtt atataaaaaa atattatggt tgaacacagg ctcgcaaata 60
tgataaggca tttaagaatt ttacaattta gatttttta aatccatgaa tatatttgtt 120
ctaatcaaaa ttattcattt tacgcttaat tttattggtt gaactaatag atagatagat 180
aaaagatata gagttaatat aaaaatgaag aaaaaaaaaa aaaaaaaaaa aaaaaaaa   238
```

<210> 270
<211> 326
<212> DNA
<213> Ctenocephalides felis

<400> 270

```
cgcaaggtat ggcatatgct ttgttagcaa atgttattcc aacagcagga ttatatgtgg 60
ctttctttcc tgcattggtt tatgtagttt ttgggtcatc cagacatgta tccatgggaa 120
catttgctgt agttagtata atggtaggaa aaattgttca agaatacgcc tattttccag 180
acggtgtaga gaaaaataca ggagatacga ccacaattcc tgaccctgat gtgcaatacn 240
atcctacaga agtggcaacc gcagttacat ttctggctgg aatttatctg cttatcatgt 300
ggatttttcg tctaggggct cttagt                                      326
```

<210> 271
<211> 549

<212> DNA
<213> Ctenocephalides felis

<400> 271

```
tcgcaagaaa tgttcactct aggaatatgt aatatatttg gcagtttcgt gaagtcgatc 60
ccaacttgtg gtgctttcac cagatcagca gtcagcaatg caagtggtgt tcgctctcca 120
atggcaggct tgtactcagg tactatgacg cttttagcgt taagttttct cactccatac 180
ttttactata ttccgagagc aactctttca tcagtgttga tatgtgctgt tatgttcatg 240
tttgattggc aaatagtgat gccaatgtgg cggactaata aattggacgt gttatttatg 300
gggtgtacat ttgctgcatc tttatataaa ggagtagaat ttggcttaag tgttggtgta 360
attctgactt tatgtccttt gctctatttg tgggcaaggc ccgaaattaa tcacatatct 420
aagtggacac ccgaaggtct tgaatatcgc gtgtatgccc agaccaggtc tttactttcc 480
ttcggttgac tttttgaaan gagaattgcc aaagttcttt aaattttcng gtccntgatt 540
tagactgtc                                                         549
```

<210> 272
<211> 549
<212> DNA
<213> Ctenocephalides felis

<400> 272

```
gtnttttcca ttgagtagcg aatttgcccg tcttacaata ttaaacgtga attcgttttg 60
ccgggtcgta gtaaccaatt taatataatg gctcctccac catacgcaga cttgggaaaa 120
caagctaggg aggtatttaa cagtggctat catttttggtc ttttcaaatt gaatttgaaa 180
actaaaactg cctctggggt tgaattcact tcaggaggaa cttctgaaca tgaaactggc 240
aaggtatttg gatctttgga gacaaaatac aaagtaagtg attacggtct cactttttct 300
gaaaaatgga acacagacaa tactttagct acagaagttt ccatacaaga tcaaatagct 360
aaaggtttga aagtatcatt cgactgctct ttcgcaccac aaacgggaag caaaactggt 420
gttttgaaaa ctgcttctta catgatagtg ttgcagtaaa tgctgatgta aatttgaatt 480
atcaggacct ttgatcaatg ccagcgcagt agtggttatc aaggtggtgg ccggtatnaa 540
ctgatttnc                                                         549
```

<210> 273
<211> 549
<212> DNA
<213> Ctenocephalides felis

<400> 273

```
ggcaaaagga tctgccaaac ctgcaagccg gcaagaaaga tagttcgaaa cgatccaatt 60
ccagcgatga agcgacgcat gtaaatcata ccggggaatg ggcgaggccg cgcagcgagt 120
cggcgggcga ccctgacgac ggctgcggga acacgaccga ctcgggagga acgtacgtca 180
aacgcacggg gttagcgtta tttctgtggt acgcctcatg cggaggagtt cgcgagtggt 240
gcgtagcttg gtggcactca ggcagagagg aatactatga accagatccg gaagaaccgt 300
cagatcctgg atacgcgact ccggtctcta tcgagacgcc gttgcagagc tcagtgtcga 360
gatgcacttc cctgaatgtg atacgcgacc catacatgac gacaacggaa gggcgaacct 420
cgcgctgccg ccgacttacc tcttcacgcc tcgtcgcgca actactcaag attcggttta 480
caccatctga tcaggctccc ggggaccaga caaagacgga caagatcgca cccttcataa 540
aatgatatc                                                         549
```

<210> 274
<211> 549
<212> DNA
<213> Ctenocephalides felis

<400> 274

```
ggctcttggt cgaagaggct gttaacgatg ataacagtgt ggtggctttg tctcaagcta 60
aaatggacga actgcagcta ttcagaggcg atacagtatt attaaaaggc aaacgcagga 120
aggagacagt ttgcatcgtt ctctctgatg atacttgccc cgatgaaaaa atccgtatga 180
acagagttgt acgtaacaac ttgcgtgtcc gcttgtctga tgttgtttct gtgcagcctt 240
gtccagacgt gaagtatgga aagcgaattc atgtgctgcc cattgatgat tctgttgaag 300
gcctaacagg aaatctgttc gaggtctacc tcaagcccta cttcttagaa gcctatcggc 360
caattcacaa ggacgacaca tttattgtcc gtggcggtat gagagcaata gaattcaaag 420
tagtcgaaac agatctgctt cttattgatc gtggtcctgc acagtcattc attgcgaagg 480
ggatccaatc aagcgtgagg aagagaagag ctcnatgctg cggtatgatg atattgtggt 540
gagaaacat                                                         549
```

<210> 275
<211> 549
<212> DNA
<213> Ctenocephalides felis

<400> 275

```
ttgacaagtt tcgatttggt tagttcttgt acttaatatg gaatcaatct catctgaact 60
gactgaacca caaacaaatt ctaattcatt taccgttgat caaatagaaa ttgacatatt 120
gcccactata tatgatatca tacgaagtgt tgaaagagat ccacatgata gcgctggcaa 180
aaccagagaa tcacaagatt gcagtgtgaa ggtattagac ttacaaaaga agttagaaaa 240
aattcgaagt caagttactc agctacctgg aattgattat aataaagagg aacaacttca 300
atatttagaa acacttagga aacaattaaa acttaagcaa gagcttttgc acaaatacag 360
gactatgtac acatttgatt caatgaaaat ataaattgtt taaaatgcct ctgcgatctc 420
tcatgaatta tttgctgaat aacgacgttt agttcagaag ttgctgaatc ttatccagtc 480

gaagagctgg cagttagcca tttcgctatg atagatcaaa atcaatttag ggacacactg 540
agaagctgg                                                         549
```

<210> 276
<211> 549
<212> DNA
<213> Ctenocephalides felis

<400> 276

ggctcgatat cgctggaaaa actttattat cgatgtgatt tatcaatggt ttcgtgagaa 60
tattctattt gctttgacga aaacaaaaaa ctgattagtt tattaaataa atagtatatg 120
ttaatatttg ctgattggcg atgacggcag agaaagcgga cttttccgac agtcttctga 180
gtcggacggg agagcaggtc cacagaccaa catttcgctc tcatgccaaa gactgtgcta 240
gtgcttgttg caccagaaaa accttagaaa ggcatctgcc tatagtaaat tggttaccga 300
aatacacctg ggaaaaatta ggcagagatg ccattgcagg tttgacagta ggtttgacag 360
caataccaca aggtattgca tatgctgtag ttgcaggact agaacctcag tatggtttat 420
atgctggttt catgggatgt tcgtgtacat attcctagga ggatgcaaag atgtgacata 480
ggcccacggn catatggctc tatggtgcac gctatgtcaa gactgggcct gatttgcaat 540
ctgccctttt 549

<210> 277
<211> 549
<212> DNA
<213> Ctenocephalides felis

<400> 277

aaagtgatgc aacaattatt ttcaaaatac aaaagtgtta aaaaatgggc gttaaaaata 60
tatatttata ctgcattctg atatgcctgc tacattatgc atcttatacc aaaactgaat 120
ctattaccaa caattctttg gaagaattgt acacaaacac ttctgccaaa acagattcca 180
ttactctttt atcaaaaacc agtctaccgc ctgatcaaaa tgccacgatt gaaaatcctg 240
atccagtgct tcctgaaaag ggctccgctg aacaagaaca acacagctcg atgtctatat 300
tcttcgtgct ttgtgtgctg gctttaggga ttcttttaat tcatttcatg ttacaaacag 360
ggtttcagta tttacctgaa agtattgttg tagttttctt aggtgcttta atcggcttga 420
taattaattt aatgtcgtct aaaaatattg caaattggag aatgaagaac cttttcaccc 480
acagcgtttt cttagtgctc tccgctataa tattgaatcc ggtatattgc ataaggnatt 540
ttttcaaat 549

<210> 278
<211> 549
<212> DNA
<213> Ctenocephalides felis

<400> 278

cttgcttaga aaaccatacg gaagaaccaa acctctcata tctcgtacca tgatgaaaaa 60
tatacttggg caatctgtat atcaactaac agttatattc acactgcttt ttgttggtga 120

taagctctta gacattgact caggaagagg agcagactac ggttcattgc caactcaaca 180
ttttacagta atttttaatg cttttgtact aatgacttta tttaatgaat ttaatgctcg 240
taagattcat ggacagcgta atgtttttga aggcattttt acaaatccaa ttttttacac 300
tatttgggta ggaaccgctg ttgcacatgt tgtcattgta caatacggag atctggcatt 360
ttcaacaaaa gggctgaatc tcgagcaatg gcttggtgct gttttcggct tggtcactat 420
tatggggaca aattgtaccc agtnctacaa gaaagattcc taaaattctt cgtagtntac 480
ttnttatttg atacacttca atgcaagaat aactagttta gttctantca taagaatgca 540
natttagtg 549

<210> 279

<211> 549
<212> DNA
<213> Ctenocephalides felis

<400> 279

```
aattcttgtg tgtagaataa aaaacatatt tgaaacgttc atataataaa aagtgcaaca 60
tgtcagatga aatgcaagaa aacggaacta ttaatggtga agtgccagaa attgaactta 120
tcattaaggc atccaccata gatggtcgac gtaaaggagc ttgtttattt tgtcaagaat 180
attttatgga tttgtacctg ctagcagaac ttaaaactat cagtttaaag gttacaacag 240
tagacatgca aaaacctcca ccggatttcc gtacaaattt tgaagcgacg ccgccgccaa 300
ttctaatcga caatggcctg ccgtgctaga aaacgacaaa atcgaacgtc acatcatgaa 360
gagtgtccct ggaggacaca atctttttgt tcaggataaa gaagtggcaa cactcatcga 420
gaatttgtct ctaaattgaa ntggtttagt caaaaaggat gcgtaaaaag caatagtctg 480
tgacccctga ggaaaatcaa cgccatttgg gcggcgcgga cgagattcta cggcgaccca 540
tgtntgntc                                                         549
```

<210> 280
<211> 269
<212> DNA
<213> Ctenocephalides felis

<400> 280

```
agatctacta atgccgcgtt taccactgtc acagcaattc cgcaaaccag caaccacaaa 60
tataaatatg atgctattgt taaatgttca ataaatgcac aaattattcc acaaatcccg 120
catgagacca tcacgaatac aagaattgga agctttccca cagagttaat aatagcacct 180
attatgggaa atccaatcgc atatccagct tccaacatta aagaatgcat aaatgcctgt 240
tcttccattg tgtctttaca ctcggtcgt                                   269
```

<210> 281
<211> 489

<212> DNA
<213> Ctenocephalides felis

<400> 281

```
catcaaatat tgaaaggaat cgatcatgtt caaatgcctg gaaaagttag cagaaaagcc 60

caagagttga ttagaaaact ctgtagacct gcacctgcgg agagactagg atatcaaaaa 120
aatggattgc aggatgttaa aaaccattca tggtattcat cagtttcatt cgactgggtg 180
agtctcaaat ctcaagaaat gcctgcgcca ctggtccgca cagtagaaaa ttcaacagat 240
atgagaaact ttgacaaatt cccgaagaac agagaatttc cacctgatga attatcaggt 300
ttcgatataa acttttaaaa acactaattt gcacacctga taatgttaca taaaatctac 360
gtggctgcag cgctgatata agtaattggt attataaata cagatgtcat tcttgaatat 420
aagaaattat ttatatattt gtaacgataa tattgaaata aattgtttta tatttcgata 480
atataaaaa                                                         489
```

<210> 282

<211> 498

<212> DNA
<213> Ctenocephalides felis

<400> 282

```
aacatgccct cagcgatttc cttanaaata cgatacatta atgatttatc taaatgatca 60
agtcccttga gatagtttaa angacttcct ccttttacgt aacttgtaat cgtccaaatt 120
tcttcagaan tggtggatat accgaaaaaa catgacaaac gatgatgatc tagtgttttc 180
atgatcatag cttccgattt aattatatct aatgaattca cacgataaat gcttatttta 240
tgaactgtca catcgatttt cttcttttc catagtgctt gcgaaacagg attataagat 300
cctctactca agttagactt anactcaata tcttcaactg gtataattat ttcatcacca 360
tgactattat aatcgtcctg cttagngcaa angatttgtc aatttaatgc ccactgtatc 420
cancgggtta tgtntgtnnn ttncacacaa gcgcangcat actggggaaa actatgtnga 480
attcantaga gtaccgtc                                               498
```

<210> 283
<211> 498
<212> DNA
<213> Ctenocephalides felis

<400> 283

```
atctttagca ccaacattcg attccaacca caattgatta agaccgcaat cactcaaatc 60
caaccagttg agaagactga gaggaaggaa tactttagct tcaacggtct tcaatggatt 120
gcctgataac aaaagtttgt ttaaactttt actattttta aatattctag tatcgagttt 180
gttgatttta ttattactca aatctaagct gattaaattg cgcatatgcc agaaaatatt 240
atattttaaa tctttgatcg agttctgagc taaattcagt tgagccatgg aaggcatttc 300
agcaaatgta tgtttgtcta agctaagtaa tccgcaattt actgcttcaa acatataagt 360
ggtaaacgat gacaaatttg aatgaaatct aggcaaattc atgagattcg ggttgttaga 420
aattttaata acatccaaat gatcattatt ttggaagacg ttgtcaggta agaaagtaag 480
tagattatgt gtcaagtc                                               498
```

<210> 284
<211> 129
<212> DNA
<213> Ctenocephalides felis

<400> 284

```
atcgatcttt ggcggctaaa catatcttgc tgagcgaagc cggccgtata gaaatatgtg 60
gctttaagga aatgagagtt cttcctgagg gccaaaatta tataaaagaa aatctcgaca 120
atcctaagt                                                         129
```

<210> 285
<211> 424
<212> DNA
<213> Ctenocephalides felis

<400> 285

gtaaacatcc atacatttag ggcagtagga ttttaccata gcctctccag gcacatcaga 60
aagacctaat ggcagcatag gctgactatc gcagtaaact ctagggcaat atccgaagtc 120
tccagattgg tatttttcta tcatttgagc tatacctcta tttgttaaaa tatatctggc 180
gtgaattaga ccatataaca tctctgcagc ttgttctatt gcatctgact gatttggatt 240
atcatctatt tcatcatcag gtttaaatct aatatcatat ccaaggcttg tctatatcgt 300
ggaatttgct cattaaggcc tgttagattg aatttatcct gtatatagtc ttcatccacc 360
tcgcaaaaga attcatttcc tcgtagacca caaaaccaag atatccatga gacctcctca 420
gaac 424

<210> 286
<211> 204
<212> DNA
<213> Ctenocephalides felis

<400> 286

ggacggccga ctcgtccgaa ttatgctcgg cacgtgttcg attcaaatga tcccgctacg 60
ttcgacatcc aaatgataaa cactatctaa tcaagtgatg ttgtttaatt aagcaagtgt 120
tagttcgaat tcatttttgt gttttgtgta tattataata aatggacccc gaaattcctc 180
tgaaaggtgt gactccgggt ttgt 204

<210> 287
<211> 446
<212> DNA
<213> Ctenocephalides felis

<400> 287

acacgcacga gcactataat cactttgaaa cttcatacac cgattgatgt catgtcgatg 60
tgtggtaaat gtcatgacag atcacagtag cagcaggaag gttataggtt gctcagattg 120
tatcctggtt cttgacacat gtattactga tgtttcactt ggttttctgt ggcaattgat 180
ggtgggttac ggctgtcgtt acagcattct gcaactgtgg tcctgatgag cttgaggact 240
ctatctgctt tcggtgactt atttccaaat ctgctaaacg tttccataat tcatctcgtt 300
cgcgctcgcg tttcttttct ttctgcgttc tcctttataa cttgcagtca attcatcaaa 360
tagtttacta ttcatttcca tgaatgnttt taagacatta taaactaaag cacaattgct 420

gattccaagc tcttttgaaa tgcggt 446

<210> 288
<211> 268

<212> DNA
<213> Ctenocephalides felis

<400> 288

```
ccacaggaaa ggaggtagct atcaaaatta ttgacaaaac acagctcaat cctggttcgc 60
ttcaaaaact tttccgagag gtgcgcatca tgaaaatgtt ggatcaccca aatattgtaa 120
aactgtttca agtgattgag acagaaaaaa ctttatatct agtaatggaa tatgcatcag 180
gtggtgaagt gtttgattat cttgttcttc atggtaggat gaaagaaaag gaagctagag 240
ccaaatttag gcaaattgtt agcgctgt                                    268
```

<210> 289
<211> 465
<212> DNA
<213> Ctenocephalides felis

<400> 289

```
cggcaaatgt ccaagctttt ttgcctggat ccacacgttt ggcaaatcca aagtcgacca 60
gtttgatgta tcctttagta tctagcatga gattttcagg ttttaaatca cggaagatca 120
tatttttgtc gtgtaaatat tcgaaagctt caataacaca agcggttata aatctagaag 180
ttttttcatc aaaacatttt gctttatgca acgccgtcca gacatcacct cccagacatg 240
cttccattag gaaatacaag tatttggtat ctttatatgt gttgtataat ctgcatataa 300
atgggctgtc acaactagcc ataacatgct tctcattgta aacgtgctgc tgttgttgct 360
gctgaacaat atcaactttc tggagacatt ttagagcaaa tgtcttgtct ggtgtggatg 420
gatgactgac ccagttccac gcgaccaaat ccgcaacgcc caaag                 465
```

<210> 290
<211> 294
<212> DNA
<213> Ctenocephalides felis

<400> 290

```
ctatattctg gctttctaaa tactccatgc cgtcaataat ctgccgacaa tatgtaggga 60
aaagttcttc gtctataata atattctctt tctctcttaa ataatgcaac aaaatgtagt 120
ctagtttttc agtaatgatg taatgttttg gagtttcgat ccaggcaaga acttgctgta 180
tatttggatg tttcagtgat ttacttaaat ttatctctct tttaaaattt tcatgttctt 240
gactttccac gccatctatt gtggttgtta ccaagagtgg ctttctcgaa atgt       294
```

<210> 291
<211> 203
<212> DNA
<213> Ctenocephalides felis

<400> 291

```
gaaaagcagc aggatttgtt gttttccgac gactgtctga cacaattcaa tatctactac 60
tacaagcttc ttacgcaaat tttcattgga gtccaccaaa aggccattta aaacgcaacg 120
aaaatgaatt tgatgcggca cttcgagaaa cattagagga aacaggtctt tgcaaaagcg 180
atattaaaat atttaaagat tgt                                         203
```

<210> 292
<211> 283
<212> DNA
<213> Ctenocephalides felis

<400> 292

```
catcactgag aagaactctt cggctacgga atatcttcat tggaggcgcg ttgttggtgc 60
acgcagcatt aacaaggttc ttgcaattca gcataatgac cagaagaaaa atacaggtac 120
tgcttttcat tttgattcag taatagatta cttcttgaga tagataataa tactgtggag 180
aagtaacctt cacatgaaat aggcttgggt cttataatta ctggctttgt tctggatatt 240
agaaatctga tcacaaaatt ttattaagtt attattatgt taa                   283
```

<210> 293
<211> 500
<212> DNA
<213> Ctenocephalides felis

<400> 293

```
agnnttgcgt annaanccct nannaagctt cntcgagngc nngaattcgg cttagcgtgg 60
tcgcgggccg gggnacaagt atcaaacatg ggctacgggg gacccgtaga ttcccaacat 120
tctacaaggg gcgncnttct ttantaaaat atcatccaga ttttcaaaac gaccgatgga 180
ttngcccggn tnaaaaccag gcacacctgg aactaacgct ttagttagcg gnggaggngg 240
cacaatgcag gatgaccagg tcaaaccgag gnccttgagg gtcacatggn ccatgaagac 300
cacaagcagt agggaccccn atgagataat gtntgaaatt cgaaaagttt tggatgccaa 360
tggatgcgat tatgaacaac gtgaaagggn tttactactt tgtgtcacgg agatctatca 420
cggatagttt agtacaatgg gaaatagaag tctgnaaatt ggcaaacttt ccttggatgg 480
aggtcgattc aaacgcattt                                             500
```

<210> 294
<211> 302
<212> DNA
<213> Ctenocephalides felis

<400> 294

```
actgtcatca tcgcttaact ccagtaatat attatctgat ttcagatctc tatgtgcaat 60
tccataagct gataaatggg caatcgcttc caaaagttga gcaaacaaaa ttatggacgt 120
cctcattgat attttattac tactaagata atcatgaaga ctgcaatcgt atctcttcat 180
taacaaaaac aatgacatat ttctaccata accatcactg ttaattcttt gtggtaaagc 240
tgatggaaac aaactggctg ctagtggtaa accagttgga attctatcag caaacacgct 300
gt                                                                302
```

<210> 295
<211> 498
<212> DNA
<213> Ctenocephalides felis

<400> 295

aagctttctt tttttttttt tttttttttt tttaaatcgg tccaaaacca tcattggntt 60
attcattngt agaaatngag atgttaaaat tgactccaat cctnttaata atatcggctg 120
ctaccctact tccaaattct gaacaagttt gtaaagtatt gtcctgcaag aatttatata 180
agaagccagc gaggaatgca tcacctgcac ctgttgtatn ttttataagc gccgatggaa 240
ttttctcgat ttcaacagac cattttgcaa atcttttcat cttgttggta aaatatggta 300
actggttttg aagcatcagt tgctacaact atcttacttt tgaagctagt gcatttcttc 360
tttagaggct tgtaaacttt aatcaaatgc tccataacac tatccaagtt attgaaatta 420
taagccttgc ctaattctaa aaattgctca gcggnttcca aagactacat ctgcagattc 480
tgctagcgnt tttacatt 498

<210> 296
<211> 227
<212> DNA
<213> Ctenocephalides felis

<400> 296

cactacctat tgatcctgct atgtttccaa cgtggcctgc aaaaagtgaa cttggaacaa 60
gacgtgcact tgccagtagt cctaaaccac cttcaggagg tgaccagttt aaaacattgg 120
gggaaggtga aggagccgat gcgtattggg gtgaacgagc atctttgttg gatacaagaa 180
acattccaat gggtgcaggc ttctcactca agttttaata aattcgt 227

<210> 297
<211> 452
<212> DNA
<213> Ctenocephalides felis

<400> 297

ggcacgaata tttgtcttca taatcattac ccgctcttgt atattgagtt ttagggatca 60
acttcttcac ataattctca tttataaaac gttggaacaa aactccaaat gttgttttcg 120
ctttcgaatg tcgaaatagt gcataaaatt catcgaagtc caaaaaccgt gaatgattag 180
agtcacccaa actcataaga tactgagtcg cagattctgg aaactcatta ttatattcac 240
ttctctctac aattctttgt aattctctga cagatatcaa gttatcatta tctgtgtcat 300
actttcaaaa tacaattaat tattgttaac gtgcatacat aaactttgat ttattataat 360
tttaccttcc cgaataggta tctgatatat tgatcagttt ccctcaacgg tatattatat 420
tccatctcta attgtgatat ccttctctga gt 452

<210> 298
<211> 138
<212> DNA
<213> Ctenocephalides felis

<400> 298

ttagatacaa gtatttctga tgatcttagg aagtatagaa cctactctgg aagtaatgtt 60
agagatttat taagagctat aaggaataag aagcatcatt atcatcaact ctcacctgat 120
gcattgaaag ttttgggt 138

<210> 299
<211> 498
<212> DNA
<213> Ctenocephalides felis

<400> 299

```
aatcattctc tctgatatgt gtatcaaaat ggcgctatca actggccaaa ttgatgtcct 60
tcacaatctg cacaaaatta gagaacaaag ggccaacttg gtggacaaca ctaatcaatt 120
caaaatagtt catatgcttg ttttggaatg cttatttgga gagtctacaa gtattccatg 180
tgaacatttg ctaaaaactg tagccgaaat gaaaagtcgt aatgaaatgt tcaaaatgtg 240
gaaaaaaata gacgatgtcg catggaaaga tgacttcgtt aaaggaatag acagtgaacc 300
atctcaagaa gatattgcga gaaaagagaa tagaaataaa attgtgccag gaagacgagg 360
tcgtgtattt ttgtcaagat ttccattgac aaaagcagat tcggattata taaatgcaat 420
atttgtagat ggatttcaaa caaaacgtca atttatcggt acgcattttn ctttacatca 480
tacagttgct gatttctg                                               498
```

<210> 300
<211> 549
<212> DNA
<213> Ctenocephalides felis

<400> 300

```
cttntgttta aatattgtat tttattanat tattcgttgn tattaacgct gngactatag 60
ataatatttt taacaattta taattcaatg ggtgttagaa ctgcatgcag gntatttaat 120
taagaatggg cgcttattta tccaagcctg ttgtggaaaa gcattcgagc gatgaatcga 180
ataacatatt aacttgtgca gntagtagca tgcanggatg gagaataact caagaggatg 240
cacataacct gatattaaac tttgacaagg atacatcact ctttgctgtt tatgatgggc 300
atgggggtgc cgaagtggct aaatactgtg ctgaaaagtt gcctgattcc atcaaggaaa 360
ctcaagctta taaaaatggg gatntancac aaggtcttaa agatgctttc cttagttntg 420
atgctccatt gcagaanaaa aagtcattga aatactcaaa aattatccac tttgataatg 480
acattaacan gcaagtcagn tgaatggact gangatgacg gtattanatg atcctcagaa 540
tctgngaag                                                         549
```

<210> 301
<211> 547
<212> DNA
<213> Ctenocephalides felis

<400> 301

agtttctact tttgacatct tcattcattg tgccgacaaa atgttgtaaa atgctgtttt 60
ataagtgaat tttatacgaa taacttagaa ttatatttta ttttattgag tgcatgtgtg 120
tgttcgagaa gtctcaaagg tcaatttgat aaaagtacaa gagtacctgc aagatttttg 180
taaagaaatt atataataag tgttttatta atatatttta tacaatggct ttgaacaaat 240
tgagtatcga gagtgtggat ttggaaggca agaaagttct tatgagggtt gatttcaacg 300
ttccgttaaa aaacggtgtt atcacgaaca atcaaagaat agtggcagct ttggatacga 360
ttaagtcgct ttgaataaaa atgctttgag tgttattctg atgagccatt taggacgtcc 420
tgatggcttc taaaaaggaa tacagtttga gacctgtgcg aggaattgaa gaactattaa 480
acagggatgt gcatttttgg aagactgtgt gccccaaagt agacaagagt gcataatgca 540
agcaagg 547

<210> 302
<211> 549
<212> DNA
<213> Ctenocephalides felis

<400> 302

aagcnggtac caatgccgaa attgaattaa aacgcggcga aaccatcaaa ctgaccaccg 60
acagagccta ttccgaacga tgcaccgatc aaattttata cttggattat gaaaacattg 120
ttagagtggt taaaccagga acaaggattt tcatcgatga tggcttaatt agtgtcatag 180
tgcaaagtgt agcaacaaat accttgattt gccaaattga aaatggtggc cttctaggaa 240
gccgtaaagg tgtcaatcta ccaggagttg aagtcgattt accagcactc tccgaaaaag 300
acaagcaaga ccttcgtttt ggaattgagc acgacgttga tatgattttt gcttctttca 360
ttcgagatgc caacgcttta gatgaaatca gagcagtatt aggagaaaga ggacgccgaa 420
ttaaagttat ctctaaaata gaaaacaaac aaggagtagc taatgcagat gagatcatta 480
gggcttccga tggttcatgg tgccgaggtg nttgggaata gaaatccgca gagaaattgt 540
cttgacaaa 549

<210> 303
<211> 547
<212> DNA
<213> Ctenocephalides felis

<400> 303

aatataataa tgatggtata cgtttgatag gttctactgc tcagttttta atacagtatt 60
atattatcga attctcataa tttcatctaa ttttgagctg gatagagtgc tgattgaaat 120
atccaaattt cagccttgtc acaatcagaa tgactgataa tagcgattta gatagacaaa 180
ttgaacaatt aaaaagatgt gagattatta aagaagctga agtaaaggct ctttgtgcta 240
aagccagaga aattttggta gaagaaagca atgttcaacg agttgattca cccgtcacag 300
tttgtgggga tattcatgga cagttctatg atttgaagga gcttttcaaa gtgggtggtg 360

atgttcccga aacaaactat ttgtttatgg gtgattttgt ggatagagga ttttacagtg 420
tggaaacatt tttattgttg ttagctttaa aagttcgata tcctgatcga attcattaat 480
aagaggaaat catgaatcaa gacaatacac aagtatatgg atttatgatg aatgcttaga 540
aatatgg 547

<210> 304

<211> 549
<212> DNA
<213> Ctenocephalides felis

<400> 304

```
aaanaattga tcccaatgga gatccatcac attatgatat aagatctgat gtctggtcac 60
ttggtatttc tttattggaa ttggcaactg gtagatttcc ttacaataca tggggaacac 120
cttttgaaca actaaaacag gttgtgaaag atgatcctcc tcgtttacca gccggtgtat 180
atagtgaaca atttgaaaac ttgatagaac aatgtttaca aaaacaattt gaacgtagac 240
ccaattatca acagttattg cagcatgaat tttgtgtgac ccatcgagat aaaccaacag 300
atgttgcatc atttgtgaaa gatatactaa cgtttgatac agtacaataa atttgttctt 360
actaatattt agtattaaac taataaatta taataatgta gttaacataa cttgttgctg 420
ttagattttt tgaagttata atcctgaaac ttgcaagatt tgtttcaaat gtaaaattat 480
atataaaatc atatttaaca tcatttacat catatacatg atattttgtt aaataaacct 540
caattctcg                                                         549
```

<210> 305
<211> 549
<212> DNA
<213> Ctenocephalides felis

<400> 305

```
aactcacgca actccagccg cgctctacgc gagaagcgtt caccgggatt gggaatgcga 60
tgtgtccgtg cccgaaaatt tcagggagat gagcggctta aaggacatcg ccaggcaact 120
tgtgcaagaa gcaccaggca ataaattgaa cgtaattttg gcgggtggaa gcgacatgat 180
gggcagcagc cgcaacccgg agtcggcgtg ccaacgcgga gatggacaag atttggtagc 240
agaatggctt cattcgagga ctagcttgaa ctcccagggc gtctatgtta atacgactgg 300
tggacttgaa aaggcgaaag tgaatgagat cgattacttg atgggtatat ttgcagcaga 360
tcatttgccc tacaatgcag tgagggataa gggtcctaat ggaactccgt ctttagcaag 420
gatgaccaaa caagcattag gaatattaca aaggccagat aaaggattcg tttaatggtg 480
aaggaggcgt attgatcacg ccatcataaa attttnacaa ttagctcttg cgaactgctg 540
aatttgcga                                                         549
```

<210> 306
<211> 549
<212> DNA
<213> Ctenocephalides felis

<400> 306

```
gaaaaataga acattatcga cgtttaaatt tctccaaaat tgttaggtta tgcaaaaact 60
ttgtccacaa agactctgct actacaccag agattgatac cattttctca aatggacgca 120
atttaactat atcaactggc aaatacgctc gattttgcga tggatcagct gttgccaaaa 180
ttggtgatac ttcagttatg gttacggctg tgtcaaaacc aaagtctcat aatgttggga 240
attttcttcc acttgttgtt gattacaaac aaaaatcagc tgcggcagga cgcattccta 300
cgaatttctt aagaagagaa ctaggtccaa cagaaactga tatattaaca tccaggttaa 360
tagatagatg cctcaggcca ttatttccac ctaactatct caatgaaacc caattagttt 420
gtaattcgtt agccgtgatt caatatacaa tgctgatgtg ccagctatta atgctgttca 480
gctgtttgcg taagtgatta ccatggaatg gccaattgng ctgtaggatc ggttaaaaga 540
ttcgactga                                                         549
```

<210> 307
<211> 550
<212> DNA
<213> Ctenocephalides felis

<400> 307

```
ctcagtttta gttcaacagc aggcgaagtt atcgtttccg gagcagaaga cggtaaatat 60
cgtgtttgga acttgcaagg ccgacaactg tatgggggca atgcacacca tcaagccgcc 120
gtgacttctc ttgcttgggc tccatccgga gcttattttg ctctcggctc ttacaacgga 180
attcgcctgt gcgacagcgc tgggtggtcg catgctttga ataaagccga aactggttct 240
ctgtacagca ttgcatggtc acaagacgga actcgtattg cagcagcatg tgccaatgga 300
catgttttat tcggaaatat tatagaaaag gaattgtgca aatacagcta taacattgtt 360
ctaactacag ctagtacttt gtcagtgagc agtataattg atacaacaat aaatgaagtg 420
cttgaattta cagatcgagt aacaaatttt gatataaact ctgagcatct gtggtcacta 480
caccaactca atgcntatat ataaaattga cgatttattc acccacagtg ttgagttgaa 540
ggatggtgta                                                        550
```

<210> 308
<211> 549
<212> DNA
<213> Ctenocephalides felis

<400> 308

```
aaaatctttc atcatgtagc gtataggaga aatttatttg cctaaaatct gcgttattga 60
tctttggctt actaatcaaa agtgatcgac ttctttcttg tagttttgta gtagtgttta 120
aatcatttgt taataatgga aattgtgggt gatttcgagt ataacagcaa ggatttaata 180
ggacatggag ccttcgctgt ggtctttaaa ggaaggcata gaaagaagac gcatcttgtg 240
gtggcaatta aaagcataac aaaaaagtct ttggccaaat ctcaaaattt gttaggaaag 300
gaaatcaaga ttctcaagga acttactgaa ttgcaccacg aaaacgtagt cgccctcctg 360
gactgcaaag aatctgctca caatgtatat ctcgtaatgg agtactgcaa tgggggtgat 420
ctagctgact atctcagtgc taaagccctt aagcgaagat caattagact tttcttgcca 480
ttagcggcgc atgcganttn atgccaaagc tcgactcgga ctgaaccgag attctctgcc 540
atccgcagc                                                         549
```

<210> 309
<211> 308
<212> DNA
<213> Ctenocephalides felis

<400> 309

```
ttataaataa agaacaagaa ctgaaagaaa aatacaaaga tgatcaaccg attgaatgtc 60
catactgggg cgggtatata gtaatacctc gatctattga gttctggcaa ggccaaagtg 120
atcgcttaca tgatagaata aaattccgaa agttgcttcc aaatgaacaa atagatttga 180
atttaacaca tgaagcagat aatggatggg tttacgaaag attatcacct taaacttaaa 240
attatttata tttttatgca ggaaagccaa aataaaaata gttcaataat ttgaacttat 300
aattatgt                                                          308
```

<210> 310
<211> 437
<212> DNA
<213> Ctenocephalides felis

<400> 310

```
cacgattcaa tatcgacgat ttcaaaattt gctgcgttga ctttataaat tttataattc 60
ggatttcggt taacgtaagt tgtaatactt cctccattga atgcaacatt aataggatgt 120
gatgaattca tagaatcata gaaaatctta aattcgtcat tatgtgtgtg gccattgaat 180
tgtcctgtta ttatatgcga aaatctttgg atgatgcgcc tatattctct gtcccaagta 240
atgaagttag tgggttcacc aggaggaaca tgtcccaaaa tgtgcacttt ttcattagtt 300
ctttctgctt ctaacagagt atcatgtaac cattgcaatt gctttttggg gaatgcagga 360
tcatacagta accacaaatt ataaatatag gccacattat tattcaacgc tatgactcgt 420
agtccaagtt taggtgt                                                437
```

<210> 311
<211> 173
<212> DNA
<213> Ctenocephalides felis

<400> 311

```
cgaaacccga gcaaaaatgt aaaaaagtaa aatttagttg cgtgaattcg tgcagttcac 60
ccgaaatgca gtattgtccg gaaataggag cagatccggt taaggaatcc tgtagcccag 120
atcaagtgtg cgctgatcaa agtggatatc tacagtgcac cactaaagaa agt        173
```

<210> 312
<211> 337
<212> DNA
<213> Ctenocephalides felis

<400> 312

```
cgtcaagtcc aatccaaaag tggaagcttt ttcaggaaag ttactaagag aggtgtcatt 60
```

tccaccagtg ggatcgagaa ataatcgcac aattagctgt gttcgtgccc tggaccaata 120
tgtaaatggc caaggaggct atgccactct tgtcaatggt ggagttggct ggaaaaatgt 180
aactttactc ctatcttctc agactggcaa aggatttaac ttcttagttg aaatttgggg 240
atattagatt agaaatataa tgaaaatgtg aatatagaaa aaaaattaaa tatacaatag 300
tattttagaa aaaaaaaaaa aaaaaaaaaa aaaaaaa 337

<210> 313
<211> 498

<212> DNA
<213> Ctenocephalides felis

<400> 313

attgaagacc gtacacaggc ctgttcaatc tggaagctaa gggttctaag ggtgctacaa 60
ctccttcaat cgcatgaact aagaacaaag gtttcttatc actcttgtca tcctttgatg 120
tcaacctcaa caatactttt tctggcatga gttcgttact ggcaaagctg acttgtttgt 180
cttcgctggt gactgcacca ttggtcgttg cagaagtatc gccaccacct gcacctaatt 240
ctttcagttt accaaaggtg agtgatctga tttcttgggc attcagtact aagtcgtaat 300
ttctttctaa agtttgttta atttcagctc ccatcagcga gtccatacca agatcagaga 360
gggtagcaga ggcctgtgta tttttggtgc ccttaagacc taatatattt gcaactgcat 420
caactaaact aacacctcct gcaccagcac cagcgtctgc tttcctctta tcagctaata 480
ccatggaggc caagacan 498

<210> 314
<211> 457
<212> DNA
<213> Ctenocephalides felis

<400> 314

tgnggttggc attatcanca accccaaaga caagcangtt gaaatgtctg tttcaaaaat 60
aaactgcaca nngattttta gctgacccta taacacctgt aanaagattt acaanancct 120
cnaaactcca tgcgaacaaa aatggaatta cttgtgctac gaatacangg ggaatttgtt 180
atntgcattg gaaaatgagg aaaattttga ttccaaattt cngattgata catggaatag 240
gcaanatggt aaatggggtg gaattacttg tgttttacag gatggagatg tgtttgaaaa 300
agcgggagtg aatatcacag taatgactgg tgaactanaa ccccaggcca ttcaacaaat 360
gaaaagtcgt ggaaaacant tcatccantg aaggtggacc actanaattc nttgcggcan 420
gtgttagtgc antaantcat cccaaaaatc ctcatgt 457

<210> 315
<211> 498
<212> DNA
<213> Ctenocephalides felis

<400> 315

tggcgaaact atgggtcact ctgctgatag attggctgct gctttcaaag catctcgaga 60
agaacaagat gcatatgctt tgaaatctca tacatatgct aaaaatgcac aggagaaagg 120

```
atatttcaca gacattgttc catttcaagt tccaggtgtg tcaaagatgg tagaaactga 180
caatggtatt cgtgtcacat cattagaaag tctggccaaa ttgaaacctg cctttgtgaa 240
accccatggc acaattacag ctgctaatgc atcattcttg actgatggtg cttcagcttg 300
cctcattatg actgaagcca aagccaagga attaggtttg acaccaaaag cttatctaag 360
agaattttg tatgttgccc aagacccagt cgaccagttg ctgcttgggt cctgcttatg 420
ttacaccaaa gatcttagaa cgtatggtct gagttaaaag acatagatgt ctgggaaatg 480
catgaagctt ttgctggt                                               498
```

<210> 316
<211> 465
<212> DNA
<213> Ctenocephalides felis

<400> 316

```
cactcgtgtt cttttcccat cttcattttg actgcccaac ataagtataa tgtctttatc 60
ccctagattg ctgcttgaaa caacaactgt atgcacatga cggctatgaa accattctgt 120
gcatttccat gcatcgtcaa tgttatgaat tttgcaacca gtgagcaact ccacctcaaa 180
ttgatttgga gtaacaatat cagccaatgg aataattagt tctctataaa ttggtaataa 240
tgattctgga acatacattt ttccattatc acctaacact ggatcgcaaa cgtatgtcaa 300
ttttggattt ttttctctca attccttaac gacgttcgca attgttttaa gaaaatctgg 360
attagctaca taacccgtta ataagtgact gtagacattg ataccattca atgctaaccc 420
ttctgccagc tctcctaatt ccttgtccgt tagaacttgc ctcgt                 465
```

<210> 317
<211> 500
<212> DNA
<213> Ctenocephalides felis

<400> 317

```
tttttttttt tttttttttt tttttttttt tcttgaatnc ataataaaat ttattattag 60
ntnattataa aaaaaatact taaaaaatat attatatgct taaatngttt catgtagnca 120
tatatttatt acatganctt tttaactaaa tctataangg gttgaatcaa ttcctccgtt 180
gnagttncat tgttattaac aagancaaaa tcccagtttt cataattatn taaatcacat 240
tcanangcaa catcatcaac accttcngta aatttccaac ctcganattn tcgaatgcat 300
ttactgcaat taattctaat ngttataact ttagcaccat atttttcaat aaaccatttt 360
atatctgngt tcctgcggat atcactaact atgcaaatag gttttgaaat tgcttggcaa 420
gccctttaca aaaataacca taatcttcat ttcaaactcg gtcgctccat tctatcatac 480
tttctgagct ttctttanat                                             500
```

<210> 318
<211> 585
<212> DNA
<213> Ctenocephalides felis
<400> 318

nctgacaatt tatcttaata gaatagcaca agcaacattc gacaactttt caatggctgg 60
ggtctatttc gtgcatcgaa gtcgttaaag gaaaattcgc ctactgccaa acatgaagaa 120
ctcatggcaa aagcatggtg ttttgaggct tcagaacgcc tagaggcaaa tttaaaacat 180
gtagtaagcg gtaagcattt agatttatat accaaattag ctacaatagg caaaaattca 240
tgcgctgcca atggagttcc gcaaaataat ccattaaatt tgtaataaat attaagtatg 300
tgtaactaag cttaagttca tttaatttat tttattgata aatactttaa tttaacagaa 360
tgtaaatatt tcattttatg taaataatta gggggnattt gttatcaaga tcgtgcgcca 420
aataagcctt gtcatacaga ttattttcaa ttttgataat aagtaataca atgcgcattg 480
gtacattttg naatctgaaa tattgncaat ggcctgatat atttaaatat aaaatgaaaa 540
aaattaaaaa ttaaannnnn nnnnnnnnnn nnnnnnnngg cttgg 585

<210> 319
<211> 363
<212> DNA
<213> Ctenocephalides felis

<400> 319

cctcctagtt cacctgtttt ataatcatnt ggaaaaacat gatgataatt gtgccaacct 60
tcaccaaggg ctgcaattgc aacccctaag ttttccactg gacttatatt tttgtcataa 120
ggtttctggc cccacatatg tgcaacactg ttcacaaaaa atgcaatatt tagggtcaaa 180
cagaacctaa aattaaaatt gacccaaaaa gaaacccata ggtcttcttg ccaaaaatac 240
caaggcgcca aaaccggtaa acctatggcc aatagggcaa atagagggat gtaaaacctt 300
ttttgccaca tgactactgc atcggcttcc aagtcactca tatcaacctg tttcctttta 360
agt 363

<210> 320
<211> 223
<212> DNA
<213> Ctenocephalides felis

<400> 320

ttcatcagaa gtgtttccac agtctggcca anttaagcta taccccttggc agctagttgt 60
gacgattctc ctaaattagg atggactaaa tgagccatgt tcggtgctcc accaatgcct 120
atgctacgga atgcacgttt tgaaacttca tcgaaaatca tacccaaatt tgctgcaggg 180
tttttaaatg ctttttgtaa ttctttcaaa tgtgcaccag cgt 223

<210> 321
<211> 337
<212> DNA
<213> Ctenocephalides felis

<400> 321

ttttataagc agaaccacca ggtgaaagat aaacgttcca ggatcctgca ttattgaaaa 60
ctttcagaaa attaccagat aatttatttt gtatacaaaa cgtgtctgta tatgcagcag 120
cctggatgga agatggtaac ttttgtatta gtttggtaac ttttttcttt tcatctggtt 180

gaaaattgga tcgttcatac actttttga cgcgttgcac aaaagtgaaa aaatcatcgt 240
aattattggt ttgcaataaa tcattcacta caaataaccc atgaatgctc agtggtaagt 300
tactgttgta actaacaaaa tctatgagag aaagtgt 337

<210> 322
<211> 549
<212> DNA
<213> Ctenocephalides felis

<400> 322

gaaccaacaa attccatcta caaccaaatt taatattaaa atttgaaatc atttttaaaa 60
aatgatcgtc agaagtgttt tgggcctttg tgtcctggtt gggctcctgg tgtctgcaaa 120
agcagattta acagatgacg tcatggctag gatgcctgat gacttcagga gggagtattt 180
gtaccagaaa aagttgacag tggtggatct gattgagcaa aatggttacc cctgtgaaac 240
ccaccaggtg acaacagaag atggctacat cctgaccgtc tacagaattc ctcacaacag 300
aaacaacgac accattacca ggggagcagt cttcgtaatg cacggactcc tgtccagtgc 360
tgctgactgg gtcgtcctcg ggccacatca aggactacca tatttgcttt ccgaccaagg 420
ctacgatgtt tggctaggaa atgcaagagg caacacactg tcaggaatca cacacattga 480
gtgtgaagag tggtgaatct ggaattagtg gaacgagatt gttatacgat ttgctgcatg 540
atcgatacg 549

<210> 323
<211> 369
<212> DNA
<213> Ctenocephalides felis

<400> 323

ggatgtgatg tgtctaaagg ggaaactttg tctgaatata ttggtagtgg accaccacag 60
ggcacaggat tacatagata cgtttttctg ctctacaagc aaccggataa aataaaattt 120
gatgaattga gactgactaa cagaagtggc gataatcgtg ggcagttcag tattgcaaaa 180
tttgccatga aatataattt aggtcaacct attgctggaa atttatacca agctcaatgg 240
gatgattatg ttccagaact ctataaacag ttgggagctt agctggctaa aatatagttc 300
aataataata tgaacaatgt ttgaatttta ataaaactat tatttgttga taaaaaaaaa 360
aaaaaaaaa 369

<210> 324
<211> 549
<212> DNA
<213> Ctenocephalides felis

<400> 324

gcatattatt tatttttata tatgtatatc cagctagata agttaaacat gcactcattg 60
agtttcatca tcttttagta gtggcatttc gtcttcttcc ccttcttgac gaattttgat 120
gtgggcattt ttgtcagttt gtgattgcgg tgcttgtgta gaaccaacac tttttggtgg 180
atatggtatt ctgaacaatt gaattcggag atgatcacat atttctaagc agacttgtgc 240

acagtatctc atcagatcga agatggacaa agccaaacaa aggcagagaa cataatattc 300
atttacaaag ttgttgaaat actgatttag gaataataaa gatggtccta atagagccca 360
gtctaaatat tccatttcac tttttgtcat atgcgcgacc actaatctgt ttgtgacctt 420
tgcagctcca ttccaaaggc gagaatatac aaagcgggat gattctcaaa tatattgctg 480
gactcttctg gcaaataatg aatgcaggaa tacaaccaaa ctgaatggat tattggtaca 540
aacctgtcc 549

<210> 325
<211> 549
<212> DNA
<213> Ctenocephalides felis

<400> 325

gacaaatccg agaacttcga tgattacatg aaagccttag gtgtcagtct agtcacccgt 60
aaattgggca accaagtgag cccagtggta gagctgacta agaatggaga tacctatacc 120
ttgtcatcca ctagcacctt caaaaattcc atcatcacat tcaaacttgg tgaagaattt 180
gatgaagaga ctcctgatgg ccgaaaggta aaatcggtag tcaccttgga tggtgataaa 240
ctgactcatg aacaaaaggg agataagccc accaaaatag tccgtgagtt tggaccaact 300
gaaatgaaag cagttatgac tgttgatgat gtggtctgca cgagaactta caaagcattg 360
taatttcaac actacggttt tctatttttg ccttaagtta tatgcatact cgtatggaat 420
ctgttataat acagactaat tgactaatta tggcattgta ggatgctgct tgttcatctg 480
ntaaagtact gtttaacttt tttgttttat cgaangatga aattaanctt aaaaaaaaaa 540
aaaaaaact 549

<210> 326
<211> 298
<212> DNA
<213> Ctenocephalides felis

<400> 326

aagctaccac catcgcaaaa tgcgataatg gagctccttt gccaaccagt gttgacgtcg 60
aaggatgtga caaattacct tgtccattag ttcgtggtag cacttctttg actgatgtta 120
aatttactgt ccctgccgat tctgctactc tgaaaccaga ggtaaaagcc aaagttgccg 180
gtgtcaccgt tccttaccca ttacccccag agctaagtga tgcttgccaa tttcttaagg 240
aaggatcatg ccctttgaaa aaagacgata aagtcacata caatctaaaa gttccagt 298

<210> 327
<211> 598
<212> DNA
<213> Ctenocephalides felis

<400> 327

gaaatcaata agacattatg aaactaataa acagtatgaa agtaataaac aatatgaagc 60
tagtaaacaa tacgaaggcc ataaacaatt tgaacctagt cgaccttttg aagctaataa 120
acaattcgaa gctagtaaac aatttgaagc taacaaacaa ttcgaagcta ataaacaatt 180

cgaagctaat aaacaattcg aagctaataa acaattcgaa gctaataaac aattcgaagc 240
taataaacaa tttgaagcta gtaaacaata tgaagctaat aagcaatatg gagctaacaa 300
acaatatgaa gctaataaac aatatgaaag taataaacca tatgaaacca ataaacaata 360
ccattcaatg agcgaacgtc atcatgaatt tagcaaatca ggaggatatg gaggctcgaa 420
tttcgatcaa aatcaggcan atgcaanaaa ctttganaga aacggaaaat ttgaaagcag 480
cggaagctat caatatcaca ntgaacaaat ggcgagaaat ctactgattc aaataagcct 540
tactcctaaa catatcgatg agctaataga aacgttatct gatagttcaa tgtactcg 598

<210> 328
<211> 221
<212> DNA
<213> Ctenocephalides felis

<400> 328

gaacaatata gataccaaaa tcattttgaa cgagatggaa cttgcgcaag agctcacatg 60
gaatctttag ttgatggtaa aataaaattc agacatgtca tggaagaaaa tggaaaaaaa 120
gttgaattta gtggacaact cagacgtaat gatgaacatt ccggtaatgg atatctgaga 180
atcagttatg aagatacaaa tcgagaatca gattatatag t 221

<210> 329
<211> 489
<212> DNA
<213> Ctenocephalides felis

<400> 329

ttgatagaca gatcagtcgc atcaattgaa tcttgagcta ttttgctcaa ttgactattt 60
tgctcaattg gaactgactg aatagacgga attccttcaa tactttgact tccaattcca 120
tctccaagaa ttttctctaa tttttgggtg aataaggaac ttctaattga cgctgagtca 180
gaatttttat ctctgagctt tacagaacta gatcctgtat tagttttatc agtttcgggt 240
ttataacaat catcacattg agaattcacc agtcctgcag gatcaactat ttgaatattg 300
tcattacagt gagctttatc atcgttgtct ttgaattctt catttttgat atcgatttca 360
tttttagttt cgggaaaatc tttcagcgct ccgtcacgta gtaagctaga ttcagtggaa 420
gatattgaac tacttctaat tgatttaatg gatgagttat tgctttttga agagcttttt 480
cgaagttca 489

<210> 330
<211> 352
<212> DNA
<213> Ctenocephalides felis

<400> 330

cagtttgtcc aattactcgg aatggccaat gcttcgaatc ttgctgcact gcaggttctt 60
cccattttct accaattaat cgtttagcat caaatacagt attttttgga ttcatagcgg 120
cctggcccct agctgcgtct ccaaccaaac gttctgattc agtaaaagca acatagcttg 180
gcgtggtgcg atttccctga tcatttgcaa taatctccac ttttccctgc tgccatactc 240

cgacacagga gtatgtagtt cccaaatcta ttccaatagc tggcattttt ttctatgtta 300
gactgtttga ttgttaatca aaattaaatc caattgaact cgaactttgc gt 352

<210> 331
<211> 265
<212> DNA
<213> Ctenocephalides felis

<400> 331

ttttttttt ttttttttt ttttttttt ttctgcatca gataatacct cataagcngg 60
tcctagatct tgaaacttcg aggctgcatc tggatcgttt ttattcttat caggatgcaa 120
ttcctttgct tgtttcctat aagctttctt aatttcattt aacgtcgcac tacgagatac 180
ttttaatatt gaatagaaat ctcgtccagc gctggctagt aaaaaataaa tacttaagtt 240
aactaacact aaataacaca gtttg 265

<210> 332
<211> 498
<212> DNA
<213> Ctenocephalides felis

<400> 332

acatagtgat cttcatcata ataaccgatg tcaccagttt tgaaccaacc agtttctgga 60
tccaaaactt cagaagttgc ttcaggacga ccataatacc ctaacattgt agggccacgt 120
agatacatct caccttgccg atttggccct aaagtttctc ctgtagaagt atccacaatc 180
ttaagttcag tgttcattaa aacgtgtcca acagttcctg gtctatattc tgcgcaagtt 240
atagttgtag ttccacccat acattcggtc aaaccataag aaaccaccaa tttaccattt 300
gtcaaatgtt tttgcatagt ctcgacctgt tgtggtgaca atttggaacc aacagcaaac 360
agcaacatta catctccaaa tggttctgca agttcttcct tatcttgtaa atacaaattc 420
atcagagaca attggtaaga tgatagtagc atcaggcctg cttgtattcg cgcataattc 480
tcaataagtt tcttcgtt 498

<210> 333
<211> 500
<212> DNA
<213> Ctenocephalides felis

<400> 333

tccgaagcct tngccgttan tntttactca tcactccaga gaaggcaata aaattggcag 60
caaacgactn tttcagacac catttgaagg acaaaaatgg taatttacca ataacaaatc 120
aaatggtggc tggaggggtt agctggatta tgtcaaatag taataactac gccaatggaa 180
ttgttgaaaa tacaaatgcg ggatgcagga cggatagctg ctcaaaataa aaaagctgga 240
atagccactc ctaagatacc agctactgaa ttaacttttg gacttattag agacaaaggt 300
attttgggtc tatataaagg aactggtgcc actatgctgc gagatgtttc attttctgnt 360
gtttactttc ctttgttcgc tactcttaat gcactgggtc caagaaaaag tgcggattct 420
aatgaancaa gtttttggng ctcctnttat ctgctgcgct gttggatcaa tggcggcatt 480

agttgtcaat ccatttgacg 500

<210> 334
<211> 500

<212> DNA
<213> Ctenocephalides felis

<400> 334

```
ctcctgcacg ctttcaaatt tcacactgat ctattaatac agncttgaat cacatacaga 60
ctcctcatat gtataatcac atataatgnt tcataagcct taattgaaat ggaatttaaa 120
ttgaaatggt gatccatgat ggaagtgatg gaacggtgaa ccaccgcccc ctctgtgatt 180
atttgcagat tctggatcta aaggatcttc accgttgtca aactgagctc ttttctcagg 240
atcagtaaga acctctttag cagcagcaat atctataaat ttattctctg ctattttctt 300
ttcatcatct ttgaaattat caggatgcca tttttgtgct gcttttcgat aagctttaat 360
gatctcttgc tttgtagctg ntctttttac acctagaaat ttataataan ctcttctctc 420
acttgntttg taaccctgag ccttcaaaag cccatctttc gctctctgac atgctcattt 480
atttccaaag ctgatttata 500
```

<210> 335
<211> 500
<212> DNA
<213> Ctenocephalides felis

<400> 335

```
naagacgttt tgaaacctnc ntttaaacca tttgggttgg gggctggggt aanttttaag 60
gaaacccttn caaaaatggg cacctcaact ggtgcaaaac gtttttggct tttnttgggn 120
ccgtggtctt tagacaaacg tgccagaggg cttttcacga aaaccaaggg gagggcattg 180
tcattcctga gaagggcgca agcaaaaagt tctcaagggc agagttattg cagttggccc 240
ggcaaagaaa ccaactggtg aacatgtgcc cctaggaatc aaaagtggtg acatggtttg 300
ctgccgaatc cgaggcccaa aagttgagct cgaagagaac aagaattcct tattcaaaga 360
atccgatatn cttgccaagt tggatatcga cgaataaatt gccnaaaatt taccatcttt 420
cacaatttnc ccaacacaaa ctgnattcga agctgttgct tnttatnaaa gtagcaatgg 480
tttttnnttg ngggtctctt 500
```

<210> 336
<211> 482
<212> DNA
<213> Ctenocephalides felis

<400> 336

```
ttatataata caatttatta taaaattaac gntctaatng tatcccgtgt aattaattat 60
tttaataacg atttttttact tttttataat atatatgtat tatattccct attatataat 120
acaatttatt ataaaattaa ctatctaata tatcccgtgt aattaattat tttaataacg 180
atttttttta ctttttataa tatatatgta ttatattccc tattatataa tacaatttat 240
tataaaatta actatctaat atatcccgtg taattaatta ttttaataac gattttttac 300
```

```
tttttataa tatatatgta ttatattgca aaaatgtaaa aaagtaaaat ttagttgcgt 360
gaattcgtgc agttcacccg aaatgcanta ttgccggaaa taggagcaga tcccggtaaa 420
gaatnctgta gcccagacaa gtggcgctgt caaagtggat atctcagtgc ccactaagaa 480
ag                                                                 482
```

<210> 337
<211> 418
<212> DNA
<213> Ctenocephalides felis

<400> 337

```
ttgacaagca aaataaaata catcaacgtt gtttttacg gccacttgca agttatttaa 60
tggttccatt ttttgaacaa ctccatttgt tccaagaacc aatgatgttt catatgtttg 120
tgatggttgt aaattcggaa cttgtaatgg tgcaccaggt gccagaccaa agctgttttt 180
attcaactgt atggcaaaac cactcatagc ttgcattgct ttgttggtgt agctcatgtc 240
catgtaaatc tgtccacttc tcctggagaa tgttccatat atttccaaac ctttgccctt 300
ttcagctggc aaccacaatg cttttggtat aacaaaaggg ccagctgatc ccacagagaa 360
tatttctccc agaagtccag agttaccgat gttattaagt anaattgaca tctgatgt   418
```

<210> 338
<211> 549
<212> DNA
<213> Ctenocephalides felis

<400> 338

```
gtcntattcc attattccat gcacacaata ttcaggcgaa tgagcctgct ttaagcactc 60
taatttgttc aaagtaaacg taccggccca cctcgacact cggtgaagag caccgtggta 120
ggattttgag ttgggccgcc ttttgacagg ctaagcccac cggtaggacg tcccacagac 180
atgccagttg aacaccgcga gcggtgaacc gacagtgtgg gacacagatt caactacgag 240
ctttttaacc gcaacaactt taatatacgc tattagagct ggaattaccg cggctgctgg 300
caccagactg gccctctaat agatcctcgt taaaggattt aaagtgtact cattccgatt 360
acggggcctc ggatgagtcc cgtatcgtta tttttcgtca ctacctcccc gtgccgggag 420
tgggtaattt gcgcgcctgc tgcttccttg gatgtggtag ccgttctcag gctccctctc 480
cggaatcgaa ccctgattcc ccgtcccgta acaaccatgg agtcgcagaa ctacctcgac 540
agtgataag                                                         549
```

<210> 339
<211> 549
<212> DNA
<213> Ctenocephalides felis

<400> 339

```
aatctctgaa actaattacg agtgcagtag caattctttt tttactaaaa gaaaaataac 60
tttggaacaa aatggcaagc acaagacccc acagggagaa catgacagac gaacagattg 120
cagaattccg tgaagctttt gctttgtacg acaaagacgg agatggtgcg atatcagccg 180
```

```
ccgaattagg aactgtcatg agggctttgg gtcaaactcc ttccgaagcc gaacttaaag 240
gatacgtcaa ggataatagc gtggccatga ccgtagattt tccaacattt ttgacaatga 300
tggctcgtca gatgcaggaa ggcagcagtg ttgatgaaat ccgggaagcc ttccgggttt 360
ttgataaaga tggtaatggc cgaatgtctg ttgcggaatt gagacacatt ttaacatctc 420
ttggagaacg cttaacagat aatgaggtgg cgcaatgatc cgagaagcag acgtagataa 480
tgatgggatg tagattatga gcaatttatt caagcatgcg atgagttcat aatataaaaa 540
taataataa                                                         549
```

<210> 340
<211> 549
<212> DNA
<213> Ctenocephalides felis

<400> 340

```
gacnntataa caaaccgaga ttatttttga aatnaaaggt attaggaaaa attattgata 60
ataacataaa agtctaaatg cttttataat atgacagcaa tgcgtactgc aatgaatgtt 120
gtgtatacag ttttgtggat tctattaatt tcacaaggta cccaaagcgt tgaattaact 180
tttgaactac cggataatgc aaaagaatgc ttttaccaag atattcaaaa aaatacaagc 240
gtcaccttag agtttcaggt cgtcacgggc ggtcagtatg atgttgatgt aacattagaa 300
agcccaaata agcaaattat atatagtcaa gtgaaaaccc aatttgattc gcatcatttc 360
actgcaccga taagtggtgt ttacgttgct tgtttcagta atgaattttc cacgttctca 420
cacaaattgg ttatatggac tttcaagttg gtgatgaaca gctttacccg gtgtcgggga 480
gcatgctaca gtcttaccca acttgaatct ttgccaagag attcatcgaa gtttgccagt 540
ttctcaatt                                                         549
```

<210> 341
<211> 549
<212> DNA
<213> Ctenocephalides felis

<400> 341

```
ataccaaata aagtttgttt gtgagaaaaa gtatttgcat aatttggatt atttccacta 60
tatcagatta ctcatttgaa ccatgtctct cgtaccattg ttgtttcacg actggtggga 120
ggattacgac cacccaatgc gtcttatgga ccaacatttt ggaatgggcc taaatcgtaa 180
tgatcttatt actaatctaa gggccactcc atcactcttc cgtggcggtt attacagacc 240
ttggaggaat gaaattactg ctgacgattc ttcatcaact atcgttgctg acaaagataa 300
attccaagtg actttagacg ttcaacaatt caaaccaaaa gaaatcaccg taaaaacaaa 360
ggacaattgc gtaatcgtcg aaggcaaaca cgaagaaaaa caagatgaac acggatacat 420
ttcccgtcat tttgttcgac gatatgtctt gccagaaatc acgatgctgc cgatgtagta 480
tcgagtttgc ctcggatgga gtattgccat acagcgccaa gaaggccttc agtgagcaga 540
cgagtggcc                                                         549
```

<210> 342
<211> 383
<212> DNA
<213> Ctenocephalides felis

<400> 342

```
agcnttaaat aacaatttca aattcaatat gaggaattta gtggttttcg ggttagtgtt 60
ggtagtttta tttgttgtta caatggcaga agacacacca gatgaaaatg agaaattcga 120
agtgggaatg tcagaggttt ctttgaatga tgtagagcca gcaccacgtg tagtatgcca 180
acttggagga aacagattat gcaatgctcg gtgcatatct ctaggaaaaa gaggaggctc 240
gtgcaaaaaa ggaacttgtt actgcagaaa ttgaagaaat ttaatatagc ataatatatt 300
agataaactt tgaataaaac cgtgttaaaa attttgcgca aaatatataa tatacctaca 360
aattaaaaaa aaaaaaaaaa aaa                                         383
```

<210> 343
<211> 549
<212> DNA
<213> Ctenocephalides felis

<400> 343

```
aacagctgtt tgtgccgtac ggtggtcccc gcgtgtgtag cattgtagca gtatcaaagc 60
gaagctcgat cgatccggtc gagacgtgaa gcccgcgcga tacccccgct tcagagacaa 120
tttacagact ccggagttaa tatcacgaaa ccactttgtg ttcgacttct cagtacgaca 180
gtgtcggtca tttcaaacgc gcgttgtgtg tgtccatcta tcatttacaa cggggttcct 240
gatttttcga aggttcagta ataattttcg tatttcgtta tggcgatgag atgtgtagga 300
ttgctcgtca agagcaccat ggtccagtcc ggacagcgca ggctgatatc ccaatcagct 360
gttgctatga acaggatgct tcaggattca ccaaacgctc acctagataa atcaatcttg 420
caaaggtaca ctcgtcttct caagacgatc ggtcacgcag gtcacttacg tgtggatcga 480
tggacggtga gtacttgccg ctaaggacgt gtcttgaatt aacctccaaa ccagaaactg 540
caaatggac                                                         549
```

<210> 344
<211> 549
<212> DNA
<213> Ctenocephalides felis

<400> 344

```
tatcaactta aataccagca tcaaaatggt gtcagctggc atgacgtgcg tgaagtattt 60
attgttctgc ttcaatttaa tatttgctct ttcaggacta accattctta ttgttggagc 120
cctaatccaa tcatcttttc accactattc tgaatttgta aatgctagtg tctggtcagc 180
tcctgtcttg cttattgtaa ttggcgcaat tgcgtttgtt atcgcattct tcggatgctg 240
tggtgctgtg aaggagagca attgcatgat ctataccttt gcagtatttc tcattggtat 300
atttatattg gaattatctg ctggaatagc tggctatata aagcatggtg aacttgctga 360
aaccttggaa aataatttta ataccagtat gaattcctat attgatgata agcaaacacg 420
tgcaacatgg gacgttatcc aggaagatct cgattgctgt ggtatgaatg gccaagtgac 480
tggaaaaaag tttttaataa tgaccaattc caaaatcctg tgtgatgagc tccaaagtga 540
tttgaatgc                                                         549
```

<210> 345
<211> 549
<212> DNA
<213> Ctenocephalides felis

<400> 345

```
ggcttattga cgttagtata acctcgtttc taattcatac tgaatttctc aatttatcag 60
tataattaaa gatattgttc atctgctttc caggtgtacg tcttcagatc tgtaagtaaa 120
aatattttac aagccgtccg agttcattgt gttcattcga gctgtgttag cattgcactt 180
caattcctca agtttcatac ataattcaaa atggctcgta ctaagcaaac tgctcgtaag 240
tcaaccggag gaaaagctcc acgaaaacaa ttagccacaa aggctgcgcg taaaagtgct 300
ccatccactg gaggcgtcaa gaaaccccat cgttatcgtc caggtactgt cgcccttcgt 360
gaaatccgtc gttatcagaa atctactgaa ttgtgatccg taaattacct ttccaacggt 420
ggtgagagaa attnccagga tttcaagaca gatttgcgtt ttcaatcact gctattgcgc 480
tctgcaggaa gcagtgaagc ttcctctagn ttgttgaaga caccatttgg tgccttcatg 540
ctagaggta                                                         549
```

<210> 346
<211> 550
<212> DNA
<213> Ctenocephalides felis

<400> 346

```
gacatcatta ctggtgatga gatgttctca gacacatata aaataaagtt ggtcgatgaa 60
gttttgtacg aagtgaccgg caaattggtt tcaaggtctc aaggggatat ccaaattgaa 120
ggtttcaacc catctgctga agaggctgat gaaggaactg aaacagccac ggaatctggt 180
gttgatgtgg tcttaaatca ccgcctttgt gaaacttttg ccttctcaga taaaaaatca 240
tacactcttt atttaaaaga ttatatgaaa aaattggtgg cgaaattaga ggagaaatca 300
ccagaacaag ttgaggtatt caaaacaaac atgaacaaag tcatgaaaga aatattaagc 360
cgttttaaag aaatgcaaat gttcactggt gaatcaatgg attgtgatgg catggttgct 420
cttatggaat atcgtgaaat agatggtgaa tctgtccaat tctgatgttc tttaaacatg 480
gctagaagaa gagaaatttg aacaatacac tatttattat gtgaaactca tcttaatata 540
ctgattttgn                                                        550
```

<210> 347
<211> 550
<212> DNA
<213> Ctenocephalides felis

<400> 347

```
agaaaatcaa cttgtgtcac ggcaactggg acagtatcac ctatgaaaaa tgaatttgga 60
aaagttgttt gtggtgatcc tgtgtctgtg cttggttgaa agttcaattt gtgccactgt 120
catctcaaaa gatgatcagg atttatatga gctatctatc atacatctga atgattttca 180
cgccagattt gaagagataa cacctcaatc aacagcttgt aataataaag aagaatgtat 240
cggtggaata gcgcgtgtct ataccgaagt aaagcgtctg caaaatgaaa ggacaaatcc 300
aattttcttg aacgcaggcg acaattttca gggcactctt tggtacaaca tacatcgatg 360
gaatgtcacg cagtactttt taaataaatt taagactgat gctgtgacat tgggtaatca 420
tgaatttgac cataagattg aaggtgtggt tccattcatg ggttccatcg aagcacctat 480
cgtagtgtgc aacattgatg actcgcaaga ccacatttca gggcaaatca aaaagacatt 540
gtttagacgc                                                        550
```

<210> 348
<211> 550
<212> DNA
<213> Ctenocephalides felis

<400> 348

```
ggaacgtgca aagaaagatc cccaatttta tatgctttgg tctgctgatg atcagcctga 60
acatatgcgt agaattcata aacacattgc ggcgccgaaa agacacttgc caggtcatgc 120
tgaaagttac aacccacctc cagaatatct atttgacaag aaagaattga agcaatggaa 180
taaacaaaag gacacaccat ggaagcgtaa actacatttt gtacctgaaa aatataattc 240
actacgcgaa gtaccatcat attcaagata cattaaggaa cgtttcttac gttgtcttga 300
cctttactta tgtcccagag ctataaaaat gagattgact attgaaccgg aagctttggt 360
accacaacta ccaagtccta aagatttgca gccattccct actgtcaaag tctcgtctat 420
aagggacata aagacatgat aaggtgcatg acaatagatt gcttggacaa tatctggcta 480
cagggtctga tgacatgcag taaaagttgg gaagtttgca ccgccgtgtt gccacaatat 540
atgtccggga                                                        550
```

<210> 349
<211> 549
<212> DNA
<213> Ctenocephalides felis

<400> 349

```
ggaacattta ttcgtgaagc tagcactagt catgcattgt ttggtaaaca tattctcact 60
gtagacatgt tcaacaaaga acaattaaat gatattttca atttggctga aaatatcaaa 120
gctcgcgttg tcaaagatcg accagtagat gaaattttgc gcggtaaggt aatggcttct 180
atcttttatg aagtgagtac tcgtaccagc tgcagctttg ctgctgcaat gcagaggctc 240
ggaggccgag tgatccatat agatgaaact agctcatctg ctaagaaagg cgaaactcta 300
gaagattctg tttctgtcat ggctggttac tcggatgtaa ttgtacttcg ccatccagaa 360
ccaggagcag tagctaaagt ggctgcgcac tgtagaaagc cattaataaa tgctggcgat 420
ggagttggcg aacatccaac tcagcgtgct tgatatattt actattcgtg aagaaatggt 480
ccgttatggt tgactatact atggncggac ttgaaaatgc gtctgacctc attgntcgtt 540
ggtgcgtat                                                         549
```

<210> 350
<211> 536
<212> DNA
<213> Ctenocephalides felis

<400> 350

```
cgattccgaa gtgcaagagg catacgaata tctgaaatcg gatgaatttg ccaaagcctg 60
gaagtatgct gtcgaacatc cggatatttt ggaaattctg gattatttac aggaaagcgg 120
cctggacatt gtagagcttt tgaacaaaat tgctgactat ttaggtctcc agaccttgga 180
accaagatcc atcaattaca acgatgaaat tccaatttcc acaggtggtc tcaaggaatt 240
ggtgaataaa attaaggaca tgttaccttt aaccgatttt atgatcttat tcttcgacaa 300
aatggacaat agcgatgact tccagaatct aatgacccgc attcaatcta ctgattttca 360
aaaaattatc gattttgtag aaaactctcc agaaattttg gctctaattg ataaattgga 420
aaatttaggc tttgatgttg acacaataat cgatttcatc aaaagcttct tcggtggctt 480
aataaaccgg atgtgatgaa ataaatatag aatattcgta aaaaaaaaaa aaaaaa     536
```

<210> 351
<211> 284
<212> DNA
<213> Ctenocephalides felis

<400> 351

```
cctncctcct catttaaaca atgcttgtga tcatttgaac gatggcgccc aatgcccctt 60
gaagaaaggt gaccaagtta cttacaatct taaagttcca gtactgcaat cttacccttc 120
aataaacttg gacttgatgg tgtcacttgt ggatgacagt aatgagtcag tagtatgttt 180
caagatcccg tgcaaggttg tataagtagc caaaataatt tttgatttgt tattgatgga 240
acaaaattaa aataataaaa ttgaaaaaaa aaaaaaaaaa aaaa                  284
```

<210> 352
<211> 549
<212> DNA
<213> Ctenocephalides felis

<400> 352

```
cttaagtgtt cagaatgcca caatcaatgc atgtaaatgg ctatgccaac ggccacagcg 60
gttccttcga tatggaggat ggatctgttt tcttgtttac ttcggaatct gttggcgaag 120
gacatccaga taaaatgtgt gatcagatca gtgatgctgt attagatgct cacttgaagc 180
aagatccaaa tgctaaagtt gcttgtgaaa ctgttactaa aactggaatg gttctcctct 240
gtggagaaat aacttccaat gctgtagttg attatcaaaa agtggttcgt gacacggtga 300
aacacattgg ttatgatgat tcatctaaag ggtttgactg gcgtaccctt aatcttctgg 360
ttgcattgga acagcagagt ccagacattg cgggtggtgt acacatgaat aggcaagaac 420
atgacatagg tgctggggat caggtttcga ctataggacc tgcaatgtct acttgcattg 480
atctcagcac ctatatagca gctggagtca tgacatcgaa ggatgaaaaa tgggcangaa 540
acagggtta                                                         549
```

<210> 353
<211> 550
<212> DNA
<213> Ctenocephalides felis

<400> 353

```
ttttacatca ggagtgtgtg cagcctgcat gttctttgtt tacaataaac gtcacaattt 60
aatcgtgtct gcagtcttca gctctgttat atcttgtgga aatgcagcgt tggactgtct 120
tatcaccgag gtgtttccaa caaatttgag ggctactggt gtggcaatat ctatggtagc 180
tgctcgactt ggaggcataa ttggcaatgt ggtaattgca actttattag acatgtattg 240
tccagccccg acatttattg tagcactact attggctgga ggtggtctga tgtgtctatt 300
tttgcctaac acgaccaggg aaccactttc ataagaaaat ctcatcgtat aacagtcggg 360
cgatataata aatatattta ttcttctgct ccatcccaaa aacttgattt tgaatttaaa 420
tacttataaa ataatgagtc ttttcttata aaaatgtata taaataatta tcctanagtc 480
gttcattaat tanttcatta atgatgactt cgttattaat taataactan taccnancat 540
cnanaaaaaa                                                        550
```

<210> 354
<211> 498
<212> DNA
<213> Ctenocephalides felis

<400> 354

```
taatgtcgta gaaaggtgaa aaactgcgag ttgtttgcac agttgtaggc aaacctacac 60
caacagtgtc atggaaagtt attaatgaga cttatgacga atcatccgat cgaatcaaat 120
tgttagatca tgacaatata ccaaattctg ctttggaaat tgatttggct gaaaaaagcg 180
atcgaggaga atatacatgt attgctacta atcaaggcat tggaattact gttaattcta 240
caaccttggt tcgagttcaa gataaattag ctgctttgtg gccatttttg ggtatctgtg 300
ctgaagtaat aattttatgc gcgattattc tcatttatga aaagaaacgc aacaaagctg 360
aaatggagga gagtgataca gatcaaagtn cagatcgaaa aaatactcct gtcacatgaa 420
ggcactgtgt gaggcatagg aagtaancaa aanttaatcn gttgaataaa agttatgccc 480
ngagtgaagt atcaaagn                                               498
```

<210> 355
<211> 498
<212> DNA
<213> Ctenocephalides felis

<400> 355

```
tattgttgga tggatggtga ctttcatcag ctgctttgag gatatcgtga gcggcactta 60
acataactac atatccatag ttgttgcata atccaaggat ccagtaggcc accaggtccc 120
gccagaggcc tctgtctttt aaaggagtgg attcgtgagg cgattctgga tttttcgttg 180
gagtcatcat tttaaaaatc aagtaaaaat cacagatgta tgcttacagg ttaatttaat 240
ttatggagca gaggttctcc accaaaaatc gaagatactt taaatattgt aaacaagacc 300
acacttcaag tctggttaaa atttaaccaa tgtatgatgt cctgaatgta gatttctgct 360
agtccaaata atgtttcaat aaattgtaat tcagcacaaa ctattctaag ttcactaggc 420
ttctccaaca atctaaatcc aactcattat cttcttctta atatgcacca agatcagtct 480
tttggtcatt tttagcta                                               498
```

<210> 356
<211> 269
<212> DNA
<213> Ctenocephalides felis

<400> 356

```
gtgttgagtg gtatcaaaac ttggtcaccc agtttatgag agaagtcttg ccacaacatt 60
tccaaagcat ttgtctggtg tagcaaaaca tcgtgcccac tccatgggct ttcgtagact 120
tcagatactg cttccattaa agacttggaa gcactttgca cagctctaat acatcttatg 180
tagttattaa attccttttg cagtctatta gcactatttt gctgcctggt gaagttttgt 240
aaatgctcat caaaaatatc atctgcggt                                  269
```

<210> 357
<211> 498
<212> DNA
<213> Ctenocephalides felis

<400> 357

```
taatgtcgta gaaggtgaaa aactgcgagt tgtttgcaca gttgtaggca aacctacacc 60
aacagtgtca tggaaagtta ttaatgagac ttatgacgaa tcatccgatc gaatcaaatt 120
gttagatcat gacaatatac caaattctgc tttggaaatt gatttggctg aaaaaagcga 180
tcgaggagaa tatacatgta ttgctactaa tcaaggcatt ggaattactg ttaattctac 240
aaccttggtt cgagttcaag ataaattagc tgctttgtgg ccatttttgg gtatctgtgc 300
tgaagtaata attttatgcg cgattattct catttatgaa aagaaacgca acaaagctga 360
aatggaggag agtgatacag atcaaagtcc agatcgaaaa aatactcctg atcacatgaa 420
ggacactgat gtgaggcata ggaagtaaac caaaaattta atctagttga ataaaagtta 480
atgcaccaga gtaggaag                                              498
```

<210> 358
<211> 498
<212> DNA
<213> Ctenocephalides felis

<400> 358

```
cttggcatct agttttgtaa attcagtttg gctgtttatt ttgctgcgat atttcaatgg 60
atttttcgta tctggaggaa gtgcaacaat atatgcatat ttaggagaat ttcataatcc 120
taggcatcgc agcagggcta ttatgggagc gtcaagcatc ttcggatttg cgtgtcttgc 180
attaccgacg gttgcatggt taattataaa tcagaaatgg tcattctata ttgacttttt 240
gggatataca tacaagccct ggaggttgta tatggttgca tgtggtttgc catcactgct 300
ttgttgtttt gctttgtgga aattaccaga aagtcccaaa tttttgatga atcagggaag 360
aaacgaagaa gctcgtcaaa ttattgccaa aatgtataga attaatactg gtaaaccaga 420
aagtgaattc cccgtatcat caatcttaga tgaatatcca ggagtggatg gtgaaaatac 480
aaataaaaca aagaaatc                                              498
```

<210> 359
<211> 749
<212> DNA
<213> Ctenocephalides felis

<400> 359

ccatttgcaa ggaattatca gtgtaatcca aaaacaatcc tgtccgcacg caagcaaagg 60
gttgcgatgg tcctggtcct ggtggcggtg gtggaggtgc agctgtcgcc aagcactcgg 120
ataaaggcgc ttgcacgcat tgttggtaaa gcgcgctgaa gtatgtctgt ccggcgcatg 180
taaagcgacc cacgttgaaa tttcctccag agagtctcac acattcaaaa tatactttgc 240
atgttttgtc tgctggatct gcaaatgagc cgctctgcac gcagttgtat atgggcggcg 300
gcgtaggtgg ttgccaaggc gctggaggat ttcttaggca ctccgaaagg gaagcgacca 360
cacattgctg atacgtgctg ctgaagtaag tagaaccagg acaattgtaa cgcgctacac 420
tgaatcctcc gcctgctttc aaagcacatt tgtagtatcc tttgcaagtg ctgtcatagg 480
gatctataaa catccctttc tgcacgcatg aaaaaagaag cggcggtgct ggtgtaattg 540
gccgtagtcg tagtaatcgg acaattggag gangcanang tgtaattaaa cattccgaat 600
atgtagggcc cacacatntt tggtaaaact cacttatatc caagttncat tggcgntaaa 660
aaatgancgg ttttgcanag caccctttta aaccaagntt gnacttggag tacctttggg 720
cgggacacnc ttaacccaaa tnttgcnga 749

<210> 360
<211> 450
<212> DNA
<213> Ctenocephalides felis

<400> 360

agaattagaa ttgacacaaa atgctttagt gaangaaatg aaattaaaat tgatgattat 60
tgatngattt tataccaaat gaaatcaaag ataaatttta tgcaaatgca aagtttgatg 120
aagatttgga catatggaca gttgcaataa ctaaagaaat gttgccacca agacgaccga 180
tatccaaacc aggacggcgc aggcctattt ctgaggtctc tttggttaaa agacattacc 240
ctggcttggc aggtatacgt cacagaggtg agaatattat tgagtatgac ttgggcatgc 300
catttcgcac cacatttgaa tacgaagccc caacagtgtc cccagcgtta caatcagtat 360
tagaagatgc tttacaacca gaaggagatt tagaaatcac ttcaccccgt caaccgtctt 420
cagactgccc aaatcatgaa aagacctagt 450

<210> 361
<211> 426
<212> DNA
<213> Ctenocephalides felis

<400> 361

cttgatagaa gaatttatta ttaatacata ttatatatat tgaatataga aagcaaatct 60
attataaaaa tattaatgca cttatgtcaa tagtttcata tagtattgcc aggaaaagct 120
aagtattaag tataatatat ttagttattc ttaaacatat gttgttgttg ttttgataaa 180
gctgtgtctt aaaatgactt tatatacagc aattatcttc aataatcctt tccacaattt 240
tgctcggtgt gttttagttt gctcaaatct atgggtgtat tgttttccac atatgtctta 300
atttctttca tcaattccga tgatgggttt tgctcacgtg tgagaatcca agctaattcc 360
atttttcctc gtggatgaat tgtgcagctc cacacaatag caaatttatc aaaatcgctt 420
tttagt 426

<210> 362
<211> 420
<212> DNA
<213> Ctenocephalides felis

<400> 362

ctgaattcca gatgtagtga agtaaggaat ctcaaatttc acttgtattg ggggttttcc 60
ttccgtatct tcacattcca cacttggcaa tccaaaatgt gctctcatta ggtattcttt 120
accacctgga aatgatttta tggaccaagt aaatgcattc aaatcaggag catatttaac 180
acttccaatt gttgttttaa acttcgggga atctgcatca gctggtacag gaatcagtat 240
ctccacatta tttgcagttg accgtctttt aaattgtgac ttagctttga tcatgtattc 300
tacacggctg tgtgcgtgcc tttcaattac tgattcaatc catatcaagg gttttacatg 360
tgtgttaagt cgataagaca ttaactcaaa ctctccatca ggaggaataa atgatatggt 420

<210> 363
<211> 218
<212> DNA
<213> Ctenocephalides felis

<400> 363

cctggtggtt tntggtagtg ggggttttgg tggttctaat gggaggattt ggtggaggat 60
tttggcttan gggntgggaa tcggganggt tccattggtt tcattcangt ttgaacttgg 120
agcanttgng gatgggctta tttcatcatt cgttgantgn ggntgggcgt ctttaacgtc 180
actggtaaat aaacgcctgg gcccanggtc aagtctgt 218

<210> 364
<211> 432
<212> DNA
<213> Ctenocephalides felis

<400> 364

attccaaata tcactataag agctaaatag ccttccgtca ggtctccatt attaacctgg 60
tcgaccacaa aataaatatt gattgcaatt actaaaatcg atagaaggat tgcaacgact 120
gaattaaccg ctccattaac gaattctccc atgatagcag cattgctggt aaacgctata 180
gtcggtaggg tcgcaaaagg tagttgtaag gacatcactg cattcagaag gtcgttcata 240
cccgataggt cttcgatgct attgaaaaat gccatcagaa atgttggtat gatggcaatc 300
attcgggtga ataggatcct cttccagcga gaccattgta ggttgaggaa accctccatg 360
gcgaattgac cagcacaagt cccagtcatt gtagaacttt gaccggctgn caatatttcg 420
acagnccaaa tg 432

<210> 365
<211> 390
<212> DNA
<213> Ctenocephalides felis

<400> 365

aaatctccgc cctgcaatct tccccaaacc aaccaaccga tcaaatagga agccaactcc 60
aagctgtcca agttctccca acaaacctcg gtttgcacac acacagacac caaaacaact 120
atgctgtacc ccaacaaacc aactttggat cctttcacca tcaccaacaa atcgcaccca 180
caaatattgg gggctactac tccccccacg gaattctacc aatctactac gataatgggc 240
acggattgtt gaattttaat cctttgaagc ctcaaaacta ctacgcccag tattatcctt 300
actaccagaa cacgagacag tattatcctt acaattttgg atattattat catcacagtt 360
tgccgaattc gcactactac aggcagcagt 390

<210> 366
<211> 376
<212> DNA
<213> Ctenocephalides felis

<400> 366

aatacaagtg aactcctatt gacttgggaa ccaactgcac cagtaactnn agcaccagag 60
ctacatttga ccgaatatgt ccttactgac atgtgggtaa atgaaacagt tgtcaaggct 120
gatttggatg acctgagaca cggagcattt ggtgggacat acagtgcctt aagtttcacg 180
attcaaataa gtcgtgaaat gggttactat ttaatggatt actttttgcc atcagtaatg 240
atcgtgtcgt gttcctgggt aagtttttgg ctggcagcag accaatcagc acccagagtc 300
accttaggca caagcaccat gttgtcattt atcacattag caagtaccca aggaaaaact 360
ttacccaaag tatcgt 376

<210> 367
<211> 377
<212> DNA
<213> Ctenocephalides felis

<400> 367

nctccacagt tccctgcatt gggccccatt cttacatggc gatgggacac aagaaggtct 60
acantcnttt atgatttctg ataaatgggt tgacatataa ctataaatat ccaatatttc 120
accattgacg accaaaccgn ggaagcagcc gatcaaccct tgctttatag ttgatttttt 180
tagatgatcc gccggagctc accgatgaac atgctttctt caaatggcca aactgctcct 240
ctgctccaaa tcaaccatca tataatctgt atttatcatg aatctgcatg atattcgttg 300
naatcgatcc aaattttatg ccattctccg ccatttaatt tctatttgaa tttatttcaa 360
tttcttctga cttcctg 377

<210> 368
<211> 498
<212> DNA
<213> Ctenocephalides felis

<400> 368

atatatctta catcgaatct aataacattn aagagttatt aattttcatg tcgatgnntt 60
agaaaatgat tcagtagcaa cttctgaatt ttctttgtaa tcgataatac tttctcaaag 120
tataattgca tttcacaaca cacgacggaa atatggttat ggtccatatg aagcaatgct 180
cgttcttcta tccacttgct tctgataaat gttcggtata aagaatgcaa tcactccaca 240
tgctatcaga gatgttcctg atattaaaaa tgttaaatcg caattataat ctagaattat 300
tcctacaaca ttgcttccaa caacacttcc taatcgaccc atcattaaag atatacatac 360
tgccattgcc ctgagttgtg tagggtacag atctactaat gccgcgttta ccactgtcac 420
agcaattccg caaaccagca accacaaata taaatatgat gctattgtta aatgttcaat 480
aaatgcacaa attattcc 498

<210> 369
<211> 549
<212> DNA
<213> Ctenocephalides felis

<400> 369

aacaaaacgg tttaattgaa tctgtagttt ggaaattaat aaatatggac agtaacacgg 60
ggattcaaat aatagcttcc aaagaaccaa aaccaaggca gtttgaagat gcgttggcac 120
tcacaggttt tggaaaattc aattaccttc ttctggtcgt gagtggatgc gtattagtat 180
gtgttttgat ggaaactctt ggaatgagtt ttgtcgttcc ttcagcacaa tgtgatctgg 240
aattaacaac aaaacaaaaa ggaatattaa gcgctatagc ttttataggt attataagca 300
gttcacattt atggggattt ttagccgata cgagagggag gcggaaagtg attatgccta 360
cacttcttct tgcatttttt tgtaccttgg catctagttt tgtaaattca gtttggctgt 420
tattttgctg cgatatttca atggattttt cgtatctgga ggaagtgcac aatatatgca 480
tatttaggag aattcataat cctaggcatc gcacagggct attatggggc gtcaagcatc 540
ttcgatttg 549

<210> 370
<211> 498
<212> DNA
<213> Ctenocephalides felis

<400> 370

cctttaagat tcgatgataa tatacaancg gccacagtgg ctgattatgg acaacaacca 60
ctcgctggca catcagcagt agtcgcagga tgggaaagat tcgagacagg actagacatg 120
gcaggtgatt tgagaaaaat caacgtttca atagtcgacg agtttgattg cttcctattt 180
tacatagaag aacaagaatt tacaaaatat cagctttgtg cctcctcttc taaaaaacat 240
atgggcgctt gtaaaggtga tattgnatca ccattagccg tagatggtct tatagtcgga 300
ttatattctt ggtcaggaaa atgtggcgac cccgagaaac cagaagttta ttcaaattta 360
gctgaatatt tcatgtggat cgaccattct ataaaaatat tcacctaatt taataactat 420
atgctttgca atcatttatt tatttgacct nggncgggac acccttagcc gnatttgnag 480
atattcatca cacttggg 498

<210> 371
<211> 549
<212> DNA
<213> Ctenocephalides felis

<400> 371

```
tataaattgt atatatgagc aatgaatcaa ttttttaaa cattttgatt ttcctgggcc 60
ctgtagcaaa cttgatattt gacaactgaa ccatcaaagc tatatataaa tattaatcga 120
atttctataa atcttaaata catattttga attaaagata gtaaattaac aatggcagcg 180
ggggctgagc ctttgtcact agctaaagat gtcaaaagag cctgcgagct ccttgataaa 240
ttacaaatga ctggagaaat accagccaca aaaatagctg ccttacaaaa agttttacaa 300
tccgattttt ttactgccgt tcgcgaagtt tatgaacata tttatgaaac agtagatatt 360
caaggttctg aagacataag agcatcagca acagcaaagg ctactgttgc agcttttgca 420
gccagtgagg ccatgccatc cacgagttgt tgaattccta agacagatga aggcttaggg 480
tttaatgtat gggtggaaaa gaacaaaatt ctccatatca tatcaagaat atccagtggt 540
gagctgtcg                                                         549
```

<210> 372

<211> 549
<212> DNA
<213> Ctenocephalides felis

<400> 372

```
gcttatgttt gtttttattt tgacgttgct aaaattttag ctttaatgtt tgttttcttg 60
tgataaatta gttttatttt aaaggctagc gcataataat aatgatgcat ctgagcgctg 120
acatatctag tgcacttcag caacttgaga gcatcaagac agcaatagat gactcccatg 180
atccaaaact tcagctcagt actaatgaag atttggatat gataataagc ctattgcaag 240
atccagtttt tcgaagcatt gttactactc aagattcact aggtgaattg aattcccaaa 300
taacacaaca tccatcaata ttaccaggag attttgatat aactacttca ggtgatctaa 360
ttctgcggtg ccccccttctc ttgatttata tgataatgag tacactgatg aacaaagagt 420
accctctgac aattaagtcc aggtagccct cagaggttag gtatagcatc ggtnggggca 480
gtcangggaa cattacattc atgaagggat caatntngca atgaggcaac cntgatggat 540
attcccaca                                                         549
```

<210> 373
<211> 549
<212> DNA
<213> Ctenocephalides felis

<400> 373

```
gcaaaatatg atcaatactg tatatttaac atacacgcgc acatatgcat atatatatgt 60
atatatatat atatatatat atatacttat atgcatataa agcaatatca acatctattt 120
agttttcgat attctgcaaa taataatgat cttaaattac aaatagaata ttacatttaa 180
cagctaagaa tttgcagttt cacaaatact gccagtcacc aaatggtatt cagaatatca 240
ttcaaaacaa tttagccaat aatgtaagat gaaatacaga tgattataga aacattcaga 300
atttatacac tataaatatg aacattaata ctatgtacca cttaaaatgt gaaatctgaa 360
tacgtcattt gatgactgcg tgtaacaaga acttgttata gattataaca attataattt 420
aatatatctt tttcattttg taaccccaaa agagcattcc tcgcgtcttg cacgacttgn 480
```

ggggtgtggt agtgtgatga tgatcatgta gatggtgatc cttatatcac ggttgggtga 540
cgagattat 549

<210> 374
<211> 549
<212> DNA
<213> Ctenocephalides felis

<400> 374

atcaaacaaa gaaaatgaaa accagtgttg taaaacatga aactgaggat actataaaaa 60
gtgaacaaag cgatcaatca attattgaaa tagatgatta ataatttgga aaactatttg 120
aagacgatgt aacaatataa gatttgtgac gttgaatcac atatatgtcg gtaaatgatg 180
ttttcctgtt tcggaaaaac tcaactagta tttatttgcc atcatttaca aagccgtggg 240
aatagctaaa cctaatagag tctccattcc atttgtaagc aatttataca tacccacata 300
tatatgcaaa tatatatata tatatatata tatatatata tatatatata tattattttt 360
aaatgctgga ccatgtttct tctaaatntg agtttaatgt ngcattcttt cgatatcgaa 420
tcattagttt tatgttttta taaggtgtat aattatttca taagactgtc actggagaaa 480
tatgttattt attaatttaa tattaattaa atatatacat tacccactat gtataaatgt 540
tcataaatc 549

<210> 375

<211> 549
<212> DNA
<213> Ctenocephalides felis

<400> 375

cttaggaatt gtggcagcaa gtccagccat atatagtgca tttacacatt gtattcgcaa 60
atcgtattag tgtgtaagtt ctgtattttt tttctatatt tgaattatta ggaatgatca 120
ccaacattgt gtatattcat gtcaaatata ttgaaaatgt tctatttaga tgtgaaactt 180
gacatgattt aatttaattt aaatatgtat tttactttgt tcatgataaa ggcatttata 240
tttaaaaaaa tatattttac tatgtgtatg taagtataca tgtacatgca tacatacata 300
tatatatata tatatatata tatatatata tgatggtaga ggcttggatt tgtaaatatt 360
ttgaagagct tcgaacaaag aatccaaagg taataaaaaa ccacaaagtt tgttgaaaca 420
atttttttat ttactgtcgc gtttcggaac taagtatggt cacctgcagg acattgcaag 480
aacatcatct taaacaagca atatacttag tagaataaaa tgctggattt tataaatgac 540
aaattgnga 549

<210> 376
<211> 498
<212> DNA
<213> Ctenocephalides felis

<400> 376

atggaatagt gctaaccaaa gcaaagataa accaatctcg cgtcgcccac atcagtaacg 60
gcataagaat cattccactg gtccacccga tggactgcat catggcaatg tgtgctctat 120

```
cttcacttct ggatatttcc atcgcaatta tcaaagggct ttgaaaaaca gatgtagatg 180
ttagtgaacc aagaaaagcc gctgttataa aaacaataaa ccaactggaa gtaaatgctg 240
taattagtct tccggtgacg attgtgatga gacttaagta aaatactggt cttcttccaa 300
tcctatctcc taattgtcca aaaataaatg tgcctatgac ttcgcctgca cgtccaatag 360
caaatgtatt agttacatag agttctctat cacaaaccca gtcttgatct gacggagccg 420
ttgataaata catgttctgt catattcata accatgctga caaggtatta tttgactttc 480
aatcttattt gtctcttc                                                498
```

<210> 377
<211> 598
<212> DNA
<213> Ctenocephalides felis

<400> 377

```
agtaagaagt aacttaaacc gcaaacgacc cagcatatca ataacattat tgtgttcttg 60
gcgagcgtcc agcttgtgaa caaactcatc attccaaaaa catgttcagg ttcagacgaa 120
taatctttta gcatttctaa tgcatcatca ttcaatgttg caccattaac tttggcaatt 180
gtgcgtaaag tttttcaca tttcttcatt tttcctctgc ttgctagcca tcttggtgat 240
tcaatcatat atttgttatt aattaaaaat attgcaaatg gcaatgaaga aactagaaca 300
aaatcaaacc aatttctcaa ccaccacatc aaaagaggca ttgtgcaaat tccaaacgtc 360
cacccaattc cttgtaacat agcgacgtgt gagttttcat cacttgtcga tatttccatt 420
cctatgacaa gaacagtctg gtatagagac atctgcgggn agtgcagtca ccacatatcc 480
aantacatac caataatatg aactagtgag aaataagtta aatatttttc ctacataatc 540
aacaccatgc tgagaaaaaa tactggttcc ttccaataca tccctaattg tcaaatac   598
```

<210> 378
<211> 498
<212> DNA
<213> Ctenocephalides felis

<400> 378

```
cttggcatct agttttgtaa attcagtttn gctgtttatt ttgctgcgat atttcaangg 60
atttttcgta tctggaggaa gtgcaacaat atatgcatat ttaggagaat ttcataatcc 120
taggcatcgc agcagggcta ttatgggagc gtcaagcatc ttcggatttg cgtgtcttgc 180
attaccgacg gttgcatggt taattataaa tcagaaatgg tcattctata ttgacttttt 240
gggatataca tacaagccct ggaggttgta tatggttgca tgtggtttgc catcactgct 300
ttgttgtttt gctttgtgga aattaccaga aagtcccaaa tttttgatga atcagggaag 360
aaacgaagaa gctcgtcaaa ttattgccaa aatgtataga attaatactg gtaaaccaga 420
aagtgaattc cccgtatcat caatcttaga tgaatatcca ggagtggatg gtgaaaatac 480
aaataaaaca aagaaatc                                                498
```

<210> 379
<211> 451
<212> DNA
<213> Ctenocephalides felis

<400> 379

ttctgttcga agtaggaaat agttcgagtg tatgcaaaat aactactgca aatactgcac 60
tcatgaataa acgaccaata agtgaaatta ccattaatgt ataagcataa tctaaactga 120
aagatagaat tggtatgaaa cacgccgtag cagacataaa taatagaaag tttatagttc 180
ggcgtccaca aaatttcagt agcggcaaag gtatgaaata acttatacac tcaagcgatt 240
cactcataat gccgtatatc attttttcga caggcaaatt aatatcgttt attgccatag 300
catagtaacc gagagcactt gaaaaccaag taacaaggca aataagtaat ctatgtctca 360
tttctgtatg cttaagtata tcagttatct ccacaaaact atttttaact ttctgccagc 420
aagatggttt tttaccacca tctgcttgag t 451

<210> 380
<211> 401
<212> DNA
<213> Ctenocephalides felis

<400> 380

agtccactca ataaagtagc gacacctccc atcgtcagtg ttatgttatt aatccataaa 60
acatttacgc tagtatttga agccaaaacc ccgcatatca ccctagtcaa tgtgttagat 120
ataccaatag ttgataacaa ccaagctgtt tcatcatcgg tcattccgct ggttttgttt 180
ctctgagcaa tatataggaa tggtattaag taacccatca tagtaaagaa accactgaag 240
gcgagcaaaa ggaatgacgg cgaacgcatc aagttcatat ctaacatggt agcgagagtt 300
cgacggaacg cttctgggca aagcttgcaa gaagactgtt cttcgacatc cgcccgagta 360
ggtaatctag ttacggacat gtggtatcca atagaagtcg t 401

<210> 381
<211> 498
<212> DNA
<213> Ctenocephalides felis

<400> 381

ggccctggac aagatgccct tcgactcacc accgaatatg gagctgtcta cgatcaaaat 60
gccaccattg aagttcttaa taaacagaag aggaaaactt tgtgccacac tgatggcgaa 120
ggagttgaat gcgaaacaga agaagggagc atcgcacctc aaataatact atttgttgcc 180
caattaattt ctggagtcgg tggatcttta tactacactt taggtgtatc ctatatggac 240
gataacacca aaaaatctaa aacaccagca ctgatgagtt tttcttattt tcttcgtatg 300
ctcggacctg caaccggtta tgccttggct agcgtctgtc taaagttcta catttcgcca 360
acgttgacgc caacaattga taataatgat cctagatggt taggagcatg gtggttaggt 420
tggttgatac taggatcaac tttaatattt tttgcaacgt tgattggatt gtttcctaaa 480
atctgccaag agctgcaa 498

<210> 382
<211> 461
<212> DNA
<213> Ctenocephalides felis

<400> 382

atgaatccaa ttcggcaagg agcaatcact tcatcatttg aatgcccatc gtgatgctgg 60
gtcttccata cagaattcac agttgtattt ttaccctcag gacaaccttc agtcgacata 120
tttaggtaag ggaatttcaa attcccttga gcaatgttaa tttgggctcc acttaccatc 180
caactgacca ggattagact tgcagctgct cccaccaaaa ctcccttagt gtttgcttta 240
gggaacaaga ttcccaatgt aaacattccc aaaagtgttc ctgcagttac accagtaaca 300
ctgatgacaa ggtgcatgac gcttcctaat tgttctacta cgaagacaag ccccagacaa 360
attcctccaa ttactactac agtcaacttc ataatattac tggcagtctt ctcagtagtg 420
ctaaccggta accactcctt aataaaatct tcataaagtg t 461

<210> 383
<211> 498
<212> DNA
<213> Ctenocephalides felis

<400> 383

aataaatata attttattat ataagaaaca tatgtataac aatacaaatt ataactattt 60
aacagtcttc ttgtgataat ttcttgacgg ccgtatctgt agactttgat acaccttttt 120
tcggtaagct ccagaacacc tgatccctgc cgaatacgtg agcttcagtt agcgtttccg 180
gtaacttttg gtgtaaagtt tccggcaaga acattccgga agtcgcgccc accatcatca 240
ttacggaaag tacagcgtac ggatatctgg cgtcatatgt ggttcccaag tatacaatgt 300
atgggcctaa tactcctaat gcatttgaaa ctatagttcc aatggaaatt cctgtctgcc 360
tcaaacaggt tggatatgtt ttcattgctt gcagattcac aacataaaac gtgatgctta 420
tgcaaaattt cattaccacg gctaacagtg ggaccaaatt ctggagggat tcatcattgg 480
caatgcatat aagaatgg 498

<210> 384
<211> 500
<212> DNA
<213> Ctenocephalides felis

<400> 384

atcnnncnnn atangtggat ncnaagaatn ctangnatgg gcctaanact nctaatgcat 60
ttgaaactat nnnnccaatg gaaattcctg tctacctcaa actggttgga tatgtttcca 120
ttgcttgcag attcacaaca taaaacgtaa tgcttatgca aaatttcatt accacggcta 180
acagtgatac caaattctgg agggattcat cattggcaat gcatataaga atggaagatg 240
ctgccagagc aattccaaaa gaagctacag aggtccaacg tcttcctatt ctgtcgctgg 300
agaaacgtcc tagtagatat gctggtaatt ctactgccga ttgatataaa aagttgagaa 360
atggattacc tcccatattg ctcacgttca aaattaatgt aaaatacgtc acggaacata 420
caatccagca aagcacaata agtgtagtat ttctagctaa acgtaaacta gaaaataaac 480
tcatgattcc gtaaactttt 500

<210> 385
<211> 498
<212> DNA
<213> Ctenocephalides felis

<400> 385

```
cattaaagaa gtatctttag gaccaacaag tttagctgct ctgatggttt tggaatattg 60
tcatgaaaaa ccagtcgaat atgtagtttt actaggcttt ttagctggtt gcattgaact 120
tatgatggga ttactgaaac ttggattttt agtcgatttc ataagtgccc caatcgtatc 180
gggttttaca tccgcgatgt cgttaataat tatttgtgcg caggcaaaag gtttgctagg 240
gctgcattat acaggacatg gatttgtgga tacattgatg cagctaatac aaaggatatc 300
aaatgcgaga ttagctgatt ctatacttgc cttatgctgt atagtttttc ttttaacatt 360
aaggcaaata aaagatttga aagtctccag tcctgtttta aaaagaacct tatggtttat 420
ttcaactgga agaaatgcct tgatcggttt aattacagcg ttgcggctta cttttgggaa 480
angaattctt ggnaagcc                                               498
```

<210> 386
<211> 498
<212> DNA
<213> Ctenocephalides felis

<400> 386

```
aatattttta attcataata tgtatatgat tgaatcgcca agatggttgg nggagtcgat 60
gtaaaataga acgatgtgaa aatacattaa aaatcatttc cagcgtanat aagacaactc 120
ttaatgaaga tgcaattaga gtgttaagag aaaaatcctc aggaaaacca gaaaaagttt 180
acggaatcat gagtttattt tctagtttac gtttagctag aaatactaca cttattgtgc 240
tttgctggat tgtatgttcc gtgacgtatt ttacattaat tttgaacgtg agcaatatgg 300
gaggtaatcc atttctcaac tttttatatc aatcggcagt agaattacca gcatatctac 360
taggacgttt ctccagcgac agaataggaa gacgttggac ctctgtagct tcttttggaa 420
ttgctctggc agcatcttca ttcttatatg cattgccaat gatgaatccc tccagaattt 480
ggtcccactg tagccgcg                                               498
```

<210> 387
<211> 396
<212> DNA
<213> Ctenocephalides felis

<400> 387

```
atggattcat gtgcctttac ctttagtatt tgccgaatat ttttcacagg agagatttcc 60
ttcagcatac ggactgttca tgttcttgca aggaataatg acattggctt tgggtccaat 120
tgttggattt attcgagatg caacacacag ctacataata tgtttccatg ccttgactgt 180
gtgtttactc atttgctgta taccatggct tgccgagatg gcgtggttaa aaatgaaaaa 240
taagaaataa atttaagaat taagttaata ttaatggaaa aattatatat agtttatgtg 300
aattttatca cacgtgttat atatctttat aaaagtaatt tataaaggat tgtcacagaa 360
aatataaatg acaaaaaaaa tgttttnnaa aaaaaa                           396
```

<210> 388
<211> 203
<212> DNA
<213> Ctenocephalides felis

<400> 388

agagaacatt cggaagtgaa gatgcctgtg aacaagcatg atggatacca aatggacttg 60
gacagtattt tgaaagagtt gggacatttt ggaaaatttc aacttctgaa ttgcctgttt 120
atttgcatta caatattgct atttgctatg tatgcgatga gctatgtgtt cacagcggga 180
gtagttaatc atagatgttt agt 203

<210> 389
<211> 549
<212> DNA
<213> Ctenocephalides felis

<400> 389

gtcnntcggt cagtcggtct tgactcgccg tacaagatag tgattcgttt tagtgcgggt 60
gtttttgttg ttgaacttat gtgcctttta cttaaacttt tcaaatttat tcaatttcat 120
caagtttttg atttcgactg tgacgtacct ttaacaaata ctaaaaattt gaaaagataa 180
aaaattgaaa tcgaacaaaa taaattaaag tacaaaaaat ggagaaagat atggaaaata 240
atgaggacca aaaagagatc aaaatggaat ccggcgaaga aagcatgcga ccagttttaa 300
acacacctga tccagtcgcg actgccacag taatagttcc tcctgatgga ggatggggct 360
gggtcatagt agccgcatca ttcatgagca acatgatagt ggacggtatt gtgttctgat 420
cggaccaata attgaagaga tcaaatttca tttggtgcaa gtaagctaaa gtagccctta 480
tcagttcgct gtctccggat tctatctgat ggtggtcttt tgtagtggct tgcgaatcga 540
ntgggttcc 549

<210> 390
<211> 549
<212> DNA
<213> Ctenocephalides felis

<400> 390

gcctgtggta ggtcccagga gcaaggagtg gaaatgttgc ttctgtctcc acgttcggac 60
tgcaacaata tgtcttggca tttggcattt gatgctgcac ttggttgcac tatgtgttct 120
tgtgataatt ttgcattctc cgaacctgat aaaccaattg gagcacccag atcaatcaat 180
gatgggtgta ggtgttggtt ctatttctcg ttataacgga gacaatagct tgttaaacaa 240
ggttgatgat aatggaacac ctttgggaga aatggagcat cctccatatg catatagaga 300
ccactcactt acatatcatg atgtggacat gggtgctctg gttacaattt gtacactggc 360
cataacatta atgatgttat atggagcagt taaacaaaag cctgcacata ttttgccatt 420
cttctgcttg caactatttg attttgcata actacactta ctgcacagga tatttggtta 480
tttgcgcagt gtcatagatt gtatcagaaa gtcgcattac caatgagaga agacttttga 540
actaaccca 549

<210> 391
<211> 304
<212> DNA
<213> Ctenocephalides felis

<400> 391

cattgtggat gaggcaaatc ccatatacca tgatgaagtt tgcttgtttc gaaagaacag 60
ttgaattgtt atacactcat gtggttccca aacccagagc agaatgcact aaaggtgaac 120
aattggttgt cacctttgct gctggttaca ttgccggtgt attctgtgca gtagtttctc 180
atcctgcaga cacagttgtt tccaagctaa atcaagacaa aggagcaaca gccattgacg 240
ctgcaaaaaa cttggctttg ctggtttatg gaagggatta ggacctagga tcatcatgat 300
tggt 304

<210> 392
<211> 229
<212> DNA
<213> Ctenocephalides felis

<400> 392

cgtggttttg gagtatctgt tcaaggtatc atcatctacc gtgcagcata ctttggattc 60
tatgacaccg ctcgtggaat gttgccagac cccaagaaca ccccattagt tatcagctgg 120
gccattgcac aagccgtcac aactgttgct ggtattgtgt catatccatt cgacactgtc 180
cgtaggcgta tgatgatgca gtctggacgt gcaaagtctg aaatgttgt 229

<210> 393
<211> 408
<212> DNA
<213> Ctenocephalides felis

<400> 393

gaaaaatggc aactgctgtt tgttgcatag gacaaggcat tttcatggca ggtttagcat 60
tctctggtaa tgatcacatc gcggctattg tgttcatgac tctggcgact gcagttaatg 120
gagccgtttc aactgggcca ttagccagct ttgtggattt gagtcccaat tatgccagta 180
ttacgctggg attgagtggt atgatttctg taatgcctgg ttttatttcg cctgctatcg 240
ttggtatact aacatttgaa aaccaaacga tagagcaatg gcagaaagtt ttcctcctag 300
cgacagcaat gttggtcgtt tgtggtcttt tatatttggt gtttgcggac tctaatctac 360
aatcctggaa cagtccagat aaaatcgttc aagatccgaa gaaattgt 408

<210> 394
<211> 129
<212> DNA
<213> Ctenocephalides felis

<400> 394

aagcaagttt tcttgggtgg agttgacaag aagacccaat tctggcgtta cttcgcagga 60
aacttggcat ctggtggtgc tgctggagcc acatcattgt gcttcgtcta cccacttgat 120

ttcgcccgt 129

<210> 395
<211> 427

<212> DNA
<213> Ctenocephalides felis

<400> 395

```
catgattggc cgctgatgta ggtaaaggcg ctggtcaacg tgaattctct ggattgggca 60
actgcttgac caaaatcttc aagtctgatg gtctccctgg attgtaccgt ggttttggag 120
tatctgttca aggtatcatc atctaccgtg cagcatactt tggattctat gacaccgctc 180
gtggaatgtt gccagacccc aagaacaccc cattagttat cagctgggcc attgcacaag 240
ccgtcacaac tgttgctggt attgtgtcat atccattcga cactgtccgt aggcgtatga 300
tgatgcagtc tggacgtgca aagtctgaaa tgttgtacaa gggaacactg cactgctggg 360
ccaccattgc caagacagaa tgaagtggtg ccttcttcaa gggagctttc tccaatatcc 420
tccgtgg                                                           427
```

<210> 396
<211> 498
<212> DNA
<213> Ctenocephalides felis

<400> 396

```
atccaagaaa ctttaaaatg cacatctagg gaaactgatt taccaccaac accatggtgg 60
gcaatttgga catctgttcc attgtgggca cttatttgcg cccagattgg acacgattgg 120
ggtttcttca caatggtaac agatcttcca aaatacatga acgatatttt gaagttcaac 180
atttctgaaa atggattata ttctgctctt ccatatgtcg tcatgtggat cgtgtcgatt 240
ttatcagcaa tttggtgcga tcatatgttg aagaaaaaga tgttgagtgt caccaatgcc 300
aggaaattat tcacaacgat agcatctgtt ggtccagctt gttttattat tggagcatca 360
tttgctggtt gtgataaaac tcttgttgtt gccttgttca ccattggaat gggtttcatg 420
ggaactttct acgccggtat gaaaattaac gcattagatt taagtccaaa ttatgccggt 480
cattgatggc cattgcaa                                               498
```

<210> 397
<211> 305
<212> DNA
<213> Ctenocephalides felis

<400> 397

```
caatcatgat gatcctaggt cctaatccct tccataaacc agcaaagcca agtttttggg 60
cagcgtcaat ggctgttgct cctttgtctt gatttagctt ggaaacaact gtgtctgcag 120
gatgagaaac tactgcacag aatacaccgg caatgtaacc agcagcaaag gtgacaacca 180
attgttcacc tttagtgcat tctgctctgg gtttgggaac cacatgagtg tataacaatt 240
caactgttct ttcgaaacaa gcaaacttca tcatggtata tgggatttgc ctcatccaca 300
atggt                                                             305
```

<210> 398
<211> 342
<212> DNA
<213> Ctenocephalides felis

<400> 398

```
aatactttga atacttcata aagaccgaat ttgcaaagac cctgcatgga gtatccaaca 60
aaggttggag cccaaccttt agcaagacct ctagcaccat cttccgctag agttactttg 120
aatccattga atactgattt gtattttgca ggatctactt gaatacggca tttcactaaa 180
tcaagtggaa cgaccatggt gtgtgttaca ccgcaagaaa taattcctcc aaatccgcaa 240
agagcaaagt aatgaccaga tccaaaggca caggaatctc ctgttgcatc tgaggtggag 300
gctgccattg ttgccaatga tttagattca gcttcacatt gt                   342
```

<210> 399
<211> 500
<212> DNA
<213> Ctenocephalides felis

<400> 399

```
tttttttttt tttttttttt ttttttttta ttattgaaat tcatttattg aatataatta 60
aaatttcaca tatttatatt cataatttta ctacatttct aagccttcat tataataccc 120
gttgcctgca gctccactag ttttacttgc attagaatta atattgtttg gataatcgtc 180
atttttaata cttgtatttg tattagttaa atttttggcc catggctgca cttcgccgga 240
tgcaaaaaac caatatatca cagctcctaa caaatatatt ccacttgaaa tgtaaaatac 300
aatttgccac gatgtgagta aatcatctcc aacctttcca ttaacaatga aaccagtcaa 360
ttgagggctg attattcctg gcaatgttgc aaaagtattt gacaatccca tcagaacgct 420
agcatattgt ggtgcgatgt ccaaatgatt tacactgaat ccagaccaag caaaagcgcc 480
caaacccaca gcgactgcag                                             500
```

<210> 400
<211> 383
<212> DNA
<213> Ctenocephalides felis

<400> 400

```
cgctgcccaa tggtttattt atgatgccgt caaagtctgg ttgcgtatgc cacgaccacc 60
accaccagag atgccagaat ctctcaagaa gaaattagct gcccaacagt aaaaatagat 120
ctgtgatgat cctctcaata atgcatgtat cagcaatatt acaattgaaa ttgcaactaa 180
catctaaaat agcagtatca gtgatggact attcaattta gtaacaatgc tgtctaactg 240
gatcacgttt ttcattccaa attttaattt taaatgaaat aggcaattat aatagtatta 300
caatttcttt taacaaattt gtgaaaaacg tttctggttg atgtgtaaat aaaacaaaaa 360
aaaaaaaaaa aaaaaaaaaa aaa                                         383
```

<210> 401

<211> 188
<212> DNA
<213> Ctenocephalides felis

<400> 401

```
taagccctta tagaatccat taagtccttc atncttatag attttgggaa ctgcttcacg 60
catggtgtta gcaaatccag gcatagtctg gatacgaact ttagcagctt ccatgggagc 120
taaagcaatg tcagcaaaga attctgcact agcagaagcc gacaagtaaa gcgatgtcct 180
ccataagt                                                          188
```

<210> 402
<211> 500
<212> DNA
<213> Ctenocephalides felis

<400> 402

```
tttttttttt tttttttttt tttttttttt ggttatataa aaatatttgt actatagnta 60
gttctaaata tacatttgtt cacaataata taaatattat ataatcattt gtaaaaaaaa 120
ttaatataaa aatctataca aatatttatg ttataaatta ctataacaac ngttacaatg 180
aaagaaaaaa atagcactac tatacacaaa gtctttggtt atatgaaaaa tatttaaatt 240
caatctaatt aatttttttgc ttttgcatga tgtaagattt taccatgcct tttatatcag 300
tgaatataaa agtgcctata aatacaagtg cagtaccgat ccaatgtatg attgtaaatt 360
cattattaaa atatacaata gagaacaatn atgacacaaa ttttctaagt gtaataacta 420
aggncactgt taatgatgaa cattctgtag tcagcncatc nctgggcttt tgcccnggtt 480
tngggttatt ncntttcccg                                             500
```

<210> 403

<211> 487
<212> DNA
<213> Ctenocephalides felis

<400> 403

```
attattactt ttataaaaat ataaatctat ggatatctat ataagggaga ggtgtggnna 60
acctacatga ttaaagtatc acttttattt aattaattag ctattatgta taggtaattc 120
tatttagttt tgtttaatta attaattact cagccttgct tattattatc gatttcgata 180
cggaaagcca aatcctgcat cgagtcgtgc tttagaaggg acttcttttc gggatcttta 240
acttttctga ttggtttttt gtttttacca tatctatttg taacaccatc atcctcgtcc 300
atcatcctta aaatagttaa ttgatccgat gaatctaagg gggccacaga aatatcacga 360
acggctctga atttaccaca attattcaaa tgttcgtttt ccaaacggaa gaaattccat 420
acaaacctcc taaacacttc caatggtgac agtatagatg taagaatgtc cccgctcaca 480
aaattgt                                                           487
```

<210> 404
<211> 343
<212> DNA
<213> Ctenocephalides felis

<400> 404

```
tttttatgct gatttagggt taattaaatt agcagaagac atagagttta gtgataaggt 60
gcaagctgta aacattcacc aaagtgaaat ccaaggtggt gaggaatgca aagcaactgg 120
atggggtcga ttgggtgcca gtcaaccaat accaaatgcg ttgcaacagt tggcaaccac 180
tgctttaagt aatgagaagt gtaaagaagt tactggattc ttcgagccca catcgcaaat 240
atgtgtattc aaaggatctg gaaaaggagt ttgttttggc gattctggtg gacctttagt 300
ttacaatgga gaacaagttg gagttgcatc atttatattg ggt                   343
```

<210> 405
<211> 387
<212> DNA
<213> Ctenocephalides felis

<400> 405

```
ntgtcaacna tttgcaanta tttggnaatt tggcnaccat tanataatta cccccancna 60
cccanantgg anacggcccn aattggggta ccanaccttg ccanacnanc nggtttgnca 120
nanttngtgt anacaanatt tgangtantt tttaccaaac cnatntnatn gtggnangtg 180
tncgggttaa attggttatn aacnatnatt tgggcaactt tgtanccaac nccgccanca 240
tncaaantgt tggtnccaac nacaanaaga atancatttg ganatttatt taanacccaa 300
ttaccaccat taanaancca acnattgcng atnatggaac caccacanaa ttgtccttgg 360
ttggttctca aagacaccat aaaaggt                                      387
```

<210> 406
<211> 127
<212> DNA
<213> Ctenocephalides felis

<400> 406

```
aagagggcca ccggagtctc cagagcaagc gctaactcca ccagtcaagg gtccagtgca 60
caagtttgtt gggtgcaatg gagtagatcc ggctcctcct agagcctttt cacactcggg 120
gtatggt                                                            127
```

<210> 407
<211> 415
<212> DNA
<213> Ctenocephalides felis

<400> 407

```
cgaagggaac aactttgagt gtaactggat ggggcgccac gaaggaatgg gggccaattt 60
cgccaaagtt acaagaagtt aaagttaaag cttactcaag tcaagaatgc aagaacagtc 120
atgctattaa cagtgacatc atttctgaca gtatgatgtg cgctggtttt cctcaaggac 180
aaaaagatac ttgtcatggg gatagcggtg ggccacttgt agatgaaaaa caggttcaag 240
```

```
taggagttat atcctggagg cgaggatgcg cgcgacctgg atatcctggc gtatatacaa 300
aattgagcca cccggaaatc caacagttta ttaaaaacaa tgtaaaattt taaatcataa 360
aactgtatga ataaacaatt acgaaaaaaa aaaaaaannn aaaaaaaaaa aaaaa      415
```

<210> 408

<211> 445
<212> DNA
<213> Ctenocephalides felis

<400> 408

```
cccaggatac aatccctata agtttcctgt ttttgtcaac taatggacca ccataatcac 60
cgtgacaaaa accgccacct tttgcagaac cagcacataa cattctatca gtgattctgt 120
ctggttttcc tggttctgca taattacttt gacatactga agaatctatc anaggaatcc 180
aagcaccaag taattctgaa gaatcgtttc cttcggtctg gttagctccc catcctgtta 240
cataaactaa agatcctaca ggagtctcat attgctcatt agctaaattt acaggtcgac 300
tattacacac tgtaagttta ataggatttt taactttaac cagagcgaca tcataatcaa 360
aggtccttat attaaatttc ggatgtaaaa ctatgtcggt tacctcgtat acatttccat 420
ttgaattgtg gtaggaactt cctgt                                       445
```

<210> 409
<211> 445
<212> DNA
<213> Ctenocephalides felis

<400> 409

```
aggaagttcc taccacaatt caaatggaaa tgtatacgaa gtaaccgaca tagttttaca 60
tccgaaattt aatataagga cctttgacta tgatgtcgct ctggttaaag ttaaaaatcc 120
tattaaactt acagtgtgta atagtcgacc tgtaaattta gctaatgagc aatatgagac 180
tcctgtagga tctttagttt atgtaacagg atggggagct aaccagaccg aaggaaacga 240
ttcttcagaa ttacttggtg cttggattcc tctgatagat tcttcagcat gtcaaagtaa 300
ttatgcagaa ccaggaaaac cagacagaat cactgataga atgttatgtg ctggttctgc 360
aaaaggtgac ggtttttgtc acggtgatta tggtggtcca ttagttgaca aaaacaggaa 420
acttatangg attgtatcct ggggt                                       445
```

<210> 410
<211> 352
<212> DNA
<213> Ctenocephalides felis

<400> 410

gacaatctaa taagggccac atcatgttcg aaagatgtaa agttggtgtc aaagtcggga 60
tgtaataaat aaaaattggc atccacaatt ataccagcgt aataagccct aaagctgcca 120
actcgaactt gaacttgttt ttcgttgcgg acatttttga agcattgagc aacagtgagc 180
acaaaatatc tactaactat gactcctcca caaatatgat cacctctata taaaacagat 240
gccacgtaag gtaattcgct gatgtcagca ggctttcctc cgatcatacg agaatcggtc 300

aaatttttgc tttctgctcc agaaaataat acaataaaga aaattgcaaa gt 352

<210> 411
<211> 498
<212> DNA
<213> Ctenocephalides felis

<400> 411

tggtatagaa taaaattttt ttataatcag aaattatctt cgctttaact tttgatgcat 60
attattttat gagatatatt tataataaat attttattca gaattgttca ataaaacatt 120
gtccgtgttt ttgtgaatcc agtccaagaa gtgatatact cttgcataga cgtctggcac 180
tcctgtgcca catggaatac caaaagatac tactctggca acaactggtc catcaggttc 240
tcgaacgatc agaggaccac cggaatcacc catgcataca ccaatgccca gtttccggaa 300
agcacacagc attgtttcag ttatatcatt ttgtctagaa actttattct tgattggtcc 360
ttgattgaga aatttttggc aaatgcgact ttcgtatgag atacccatca tttttgtaa 420
atgatctggt atttgaccaa atggagtagt ttgtccccaa ccagaaactt caacagtctc 480
tccgccatgg tatcttct 498

<210> 412
<211> 386
<212> DNA
<213> Ctenocephalides felis

<400> 412

cttttatggt gtctttgaga accaaccaag gacacttctg tggtggttcc atcatcagca 60
atcgttggat tcttactgct gctacttgcg tgttaaataa atatccaaat gatattcttc 120
tcgtcgttgg aaccaacact ttgaatgctg gcggagtagg atacaaagtt gcccaaataa 180
tcgttcataa ccaatttaac cagaacacat accacaataa catcgctttg ctaaaaacta 240
catcaaatat tgtatacaca aactatgtca aaccagttgg tctggcaagc tatgatacac 300
caattggagc agtatccact ctggctggat ggggctactt atctaatggt ggcaaattcc 360
caactacttg caaatcgttg acacgt 386

<210> 413
<211> 348
<212> DNA
<213> Ctenocephalides felis

<400> 413

```
ctcatctacg gtcaatggac ctccagaatc tccactgcat tgtgctctgt cataatctgg 60
ataagctgcg caaatttgct ctttataaat gggtctaaag tgcaaatcac cacattcctt 120
ggatccgacg atctggtagt gcaattcttg cagatgtgtt tgcaaaactc cgtcggtagt 180
agaatttatt ccccatccga caagagtagc tggtgcattg tgtttagttt gttcatttgc 240
ttctggcaaa gtaactggtt gcgcaaattt actatatttt aagggttcag atagcttgag 300
aagtgcaatg tcgttgatat aactgttact aggattatat cctttgtg             348
```

<210> 414
<211> 147
<212> DNA
<213> Ctenocephalides felis

<400> 414

```
cattgccatc taatgagttt gatcccatat agacggattt taaaatgccg ggttttaggc 60
aatgtgcagc cgtcacaatc caacgtttgt tcaagatgga tccaccacag aaatgctcta 120
ggtctcgatt tctcaatgaa acttggt                                    147
```

<210> 415
<211> 467
<212> DNA
<213> Ctenocephalides felis

<400> 415

```
ctttacatat tgcagttgtt cggggtcgtc ttcatcttcc cctaaatttc cccagccagt 60
aacagtggnt aattctcctg atggcatgtc ttttccaaca tcagtaagtt tgactgttct 120
aacagntctg ttattcaacc ggaatggtct tcgaaccttg atgagggcga catccatatc 180
gatgtcagtt acattaccgt atgcaggatg cttgataatt tgtgccacag gatgaacgga 240
accacgtctt ccttggaaac tggtgccgac tcgaactgaa tacgtgaatt catcatatat 300
gcaatgagct gctgtgacaa tccaataatt attcaatatt gaagctccac agaaatgttc 360
gttaaatact tggagtgaag cttgatagcc atatttggca atatcagcat cttgnctcca 420
acaatgcgcc catctaatcc atcctttatc ttgnaagatg agacggc              467
```

<210> 416
<211> 346
<212> DNA
<213> Ctenocephalides felis

<400> 416

```
tcattacggt gtggaatggc tcgtccctca tcctcgttat gatagccgcg atcaaaatta 60
caatgtaggt ttaattatga taacaaagga tttcaatgaa actagaagaa gccgacctgc 120
caagctcgta gaggcaaatg tcgacttgcc tgtaggctcc tttgtcacag ctactggatg 180
gggatctgaa acgataccag gagcacctat gtcagaaaat cttagagcaa tatctttgca 240
cgtcattgat aatcaagaat gtcttgaaaa aaatcaagag ttgattgatg tcacagacaa 300
aatgttttgt gctggatcaa tagaagataa aggaaaatca gtttgt               346
```

<210> 417

<211> 312
<212> DNA
<213> Ctenocephalides felis

<400> 417

```
aattcttggc tataagttcg ttgcatgttt tgcaagtgat ttgnttattg catttattnc 60
cgtggcatat tttacatgaa tcagagtttg cattacaatc gttaccctga tctgatttgc 120
atcctcgagt aatgacacct ttttcaattt ttgaatagca gtcgcctttg gattcataca 180
aactttggtc ttcgtctttc ctgcatcttt gaaacattcg tttcttagtt ctggaagttt 240
ttgaacgtta caacccgatt tctcgcaaca cattttttct cggntagtcg agtcacaact 300
ttctttaacc gt                                                     312
```

<210> 418
<211> 315
<212> DNA
<213> Ctenocephalides felis

<400> 418

```
aattcttggc tataagttcg ntgcatgttt tgcaagtgat ttgnttattg catttattcc 60
cgngggatat tttacatgaa tcaagagttt gcattacaat cgttaccctg atctgatttg 120
catcctcgag taatgacacc tttttcaatt tttgaatagc aagtcgcctt tggattcata 180
caaactttgg ncttcgtctt tcctgcatct ttgaaacatt cgtttcttag ttctggaagg 240
ttttgaacgt tacaacccga tttctcgcaa cacatttttt cttgggtagg tcgagtcaca 300
actttntttta accgn                                                 315
```

<210> 419
<211> 387
<212> DNA
<213> Ctenocephalides felis

<400> 419

```
aaactggnnt atccctggtg tcaatgggag ctggnggnaa tactgncaca naaanagcng 60
cgggnttacg tcagcctnaa ncaaaagaaa aaatncaaga tgactaccat gcattgatga 120
acactctnaa tacacaaaaa ggngaaactc nggaaattgc caacaaagtt tacgttatgg 180
aaggctatac attgaaaccc accttcaaag aagnngccac caacaaatnc ttagctggag 240
cagaaaactt gaactttgcc caaaatgctg aaagcgctaa agttatcaac acttgggntg 300
aagaaaaaac tcatgacaaa attcatgagt ngatcaaagc cggtgatcta gaccaggatt 360
caagaanggn tcttgtcaat gcattgn                                     387
```

<210> 420
<211> 236
<212> DNA
<213> Ctenocephalides felis

<400> 420

```
aatatatttt ttgacatcat agtaacgacc tcctttgttt agtttgttgg atccgacgta 60
gatggaactc aaacggcctg cgactagaca atgtgcagca gtaagaaccc atttttcatt 120
aagaatcgat ccaccacaaa aatgtttcaa gaatttatct nttaaagata cttgaaatgg 180
agcagaaccg gggcggcgg tctgcnnctc cacaattctg gtgtggtcat tggaag     236
```

<210> 421
<211> 447
<212> DNA
<213> Ctenocephalides felis

<400> 421

```
tnnntttttt tttttttttt ttttttgatn attagngata tttatttgag tagtaatatg 60
gtttaatttg ttagttgagt atcaaaattt tgtaaaacat ctcgtcttag ttagtctcgt 120
cttagtcatt gaaattaagg aatatcaaaa aatatttgta atattataag ttaaaactct 180
tatattccag tgtgcatttt gataaattct cttattggtt ttgaagccac tcgggtatat 240
actcctggga tttcgggtcg tgcacatcct attccccaag acactattcc atgcagaact 300
cccttagagt tgacgagtgg gccaccgcta tctccttggc aagagtcctt accgccttca 360
ggataaccag cacaaatcat attttgggta attattagtc cttcatttcc atatatagtt 420
ttgcactgag tccagttaac aataggt                                     447
```

<210> 422
<211> 367
<212> DNA
<213> Ctenocephalides felis

<400> 422

```
tttnttttttn tttttttttt ctttttttagg ganttttttat atttatttta tgaacngctc 60
tgatttttaa attttaacat gtttactgat aaaatctcta acaattggtg atgaaattng 120
tgtgtatact ccgggactga ccgccccttgc acacccgtcg ccccaagata cgatccccac 180
aagagtntta ttttnntnga caagtgagcc cgcgntgttt ncttggcacg aatccttttn 240
ttcgtcaaga aatccagcnc aaagcatgtg ttntgtcaat gtataacgag cagcatacaa 300
tattttacag aaagaaaagt caatcactgg tatagaaacn ccgcgaaggt tttccgaaaa 360
aacttgt                                                           367
```

<210> 423
<211> 432
<212> DNA
<213> Ctenocephalides felis

<400> 423

```
cagatgtata tgcaagagta tactattatt tggattggat ccatcaacat actgataatg 60
nntctattga acaactcaga ataaattaat tgaaaaaata gctaagttat catagtaaag 120
atcttgacgt ntttataaga tttaataaat aaaaaacaat catagataaa aaatcataga 180
tagataaacc atagaatgct gttcctttgt atggcaaact gacaaattga ttttaatcac 240
taccaaatta ttttttgtaa ctatgataaa atattctata aaactattcc tactaattta 300
tgttatagat gaggtgatag tataggtcag tcagcatatg tgatatttac cagtatattt 360
aagttgaaac cattaaattc agtattacgt gaaataaatg caaaaaaaaa aaaaaaaaaa 420
aaaaaaaaaa aa                                                     432
```

<210> 424
<211> 354
<212> DNA
<213> Ctenocephalides felis

<400> 424

```
tacctcatct acggtcaatg ggaccctcca gaatctccac tgcattgtgc tctgtcataa 60
tctggataag ctgcgcaaat ttgctcttta taaatgggtc taaagtgcaa atcaccacat 120
tccttggatc cgacgatctg gtagtgcaat tcttggagat gtgtttgtaa aactccgtcg 180
gtagtagaat ttattcccca tccgacaaga gtagctgatg cattgtgttt agtttgttca 240
ttggcttctg gcaaagtaac tggttgcgca aatttactat attttaaggg ttcagatagc 300
ttgagaagtg caatgtcgtt gatataactg ttactaggat tataaccttt gtgt       354
```

<210> 425
<211> 498
<212> DNA
<213> Ctenocephalides felis

<400> 425

```
atacatatcc aaaattcaac aagnctatgc catactggat cataaaaaat tccnggggnc 60
cccgctgggg cgaacaagga tattaccgag tataccgngg tgatggaact tgcggagtgg 120
accaaatggc tacttcagcg gttttggata aaccagttgt taactagaca aaaatacacc 180
aagtgccaaa tatgatcctg ccaaaccttt ccatgtttat aagtagtgcc aaacaaatta 240
tcttcggcga ctgaaatatt ggacaacttg catgcatttt tgactatttt aaattgaaca 300
gttcagcatt atttatgtct ggcagaataa cagaggcaaa acaaacaaat gncgatcaaa 360
tattacaatt cgatgngctc ttaatttgca taaatataga tattatacta attcaaaaag 420
caatctgctt taaaatgaga atataaagga gcaagncgtc agttttcttt tacatggtaa 480
aatatcgaca acataaga                                               498
```

<210> 426
<211> 500
<212> DNA
<213> Ctenocephalides felis

<400> 426

ccagtagtat tagtagattt agccgaaagt ggaactgaag ttaaacctgg agcaatactt 60
agtgtcactg gatggggtgc aactaaggaa ggtggcggng gaactttgca actacaaggt 120
gtgaaagttc cagctatctc tcccaaagat tgtgctaagg ggtatccacc ttctggaggt 180
aaagacaaaa ttacagacag natgttatgt gctggtcttn ctgaaggagg taaagatttc 240
tgccaaggcg acagtggcgg tccactggta gatgaaaata gaaagcaagt aggagtggtt 300
tcttggggtc aaggatgtgc cagaccagga aaaccaggaa tttatgctaa agtgtcacac 360
cccgaaatca gaaaatttat tgaaaaatat gctaatgttt aagtggattt tatttcaata 420
taatgtgatt taagatactc tttaatggta tgtaataaat tgngataaat taaataataa 480
aaattggaga actggaaaaa 500

<210> 427
<211> 360
<212> DNA
<213> Ctenocephalides felis
<400> 427

aaaatcgaat cgttggtggc aatgatgtaa gtttttcaaa aaatgggtgg cangtatcag 60
tgnaaagtaa taaccaacat ttctgtggtg gttcaatcat tgctaaagat tgggtgctga 120
cttcttctca atgcgtcgtg gacaaacaaa gtccaccgaa ggatttaact gttcgtgttg 180
gaactagcac tcacaatgat ggaggaaaag tgtatgatgt tattgaaatt ataaaacatc 240
cgaaatataa taaagcagtg ccagatgatt ttgatgttgc acttttacgg atcaaagagc 300
caatatcatt tactccatgc acagtaactc ctgtaaaatt aatacaatcg ggaaaagaag 360

<210> 428
<211> 266
<212> DNA
<213> Ctenocephalides felis

<400> 428

ttttctacat ccttgtcaac ttcttgaatc tttttatcca acattttctc aatctttgta 60
agatcgtctt catcataagc gtctccgcaa atcttacgag ctacaacatt ccattcctct 120
attacatcat cttgtgggta gaactcagaa agatcagatg agtaaggctc tggttcacct 180
ggactgatgt tattgtctcc catatatgtg attaaatcac ggaagtcgac atcacattga 240
attggatttt gtgaaaaatc aatagt 266

<210> 429
<211> 328
<212> DNA
<213> Ctenocephalides felis

<400> 429

caacacaact ctgcacatat attgcanttt ggaaaaggtg catgtgctgg tgattctgga 60
agtcctttgg cagcaggtgg ccaattagta ggtattgttt cctggggtgt cccatgtgcc 120
actggtgtcc cagatgtcta caccagagtc tatgcttacc gcgattggat cagatattac 180
actggatttt aatctcctaa actcatctca tttgttatat tgtaaattat gtaaataaat 240
atgaaaaatg tataatgaaa atacttgtta aataaaagtt acttttatta agaaaaaaaa 300
aaaaaaaaaa aaaaaaaaaa aaaaaaaa 328

<210> 430
<211> 235
<212> DNA
<213> Ctenocephalides felis

<400> 430

```
ttttttttt ttagttttaa taattattgt agatcaacat gttatattac tttgcatagn 60
gngatatcgg ttactaaata ccagtatgtt tcttcacaaa ttctcttatt tctggatcag 120
ctagtcttgt aaatacattt ggatatggaa aacttgtgca atttctagtt gaaaaagctg 180
tcaagcctac taaaacccca ttttcgtcaa cgacgggtcc accaaaatca cctgt      235
```

<210> 431
<211> 549
<212> DNA
<213> Ctenocephalides felis

<400> 431

```
cggnggtgtt attttaaata aagaatatgt tcttactgcg gcacactgtt tcaatgatgt 60
aactcatcat tcggaaattc aagtcagagt tggaagtaca aatgcttatc acggaggaat 120
aatcgtggat gttgaggaca ttacagtaca cgaactttat aatgaaaagt tcacaaatta 180
tgatgtagct gttgtgaaat tagcttatcc attaagattt gataaaaata taaaagcagc 240
ggtactggca gaggatggat atgagccaga aataaattct aaggtcactg tatccggatg 300
gggtagtttg agctaccttg gtccataccc agaagagcta caacaggtag atttgcaggt 360
cgcagaccac gacgactgct cattgcttac atggcacacc tcgacctgcc gaaagtcaaa 420
tttgtgctcc gtcctggtgg agtcaaagac tctgccaggt gactctggtg gcccctgctg 480
agaatggcgt cgcgtagcat gtgtccttgg tgccgtgcgc ggaccagata tcagagttat 540
ctagattgg                                                         549
```

<210> 432
<211> 549
<212> DNA
<213> Ctenocephalides felis

<400> 432

```
gaaaatcaga ataaaatgca gaaattattg atactagttt catttttatt tcccttgatc 60
gtctgtaaag aaaaccgaat tattggtgga gaagaagcta atatagcaaa acatggctgg 120
caggtgtcac ttttattatt cggaaatcat tattgtggtg gagtaatcat tgacaaaaat 180
tggattttaa cagccgcaca ttgcattgaa aatgaaacaa atgccaataa aagatattca 240
gttcgagttg gaagtagtac acatgaaaag ggcggaaaag tatacaaagt caaagaggct 300
attttacacc cagaatatga tacttatacg gtggactttg atgtagctct gattcgccta 360
gccgaaccaa ttgcattcac cgcctgcaca gtgcgcccaa ttcaaatagt agatgaagga 420
gtaaaaacat tggatggggc aatgttaact gtaccggatg gggatccaga cgactggtgg 480
agattaacta cagaattaag aacgtaatgt ccattattaa taagaaaaat gtgatgaatt 540
tattctcca                                                         549
```

<210> 433
<211> 549
<212> DNA
<213> Ctenocephalides felis

<400> 433

gaagatatga agactttttt caaaatatcg gttttaatcg atctgtatat gttgacaaat 60

gttgctgcgg aaaaaaattc tggtcgaata gctggtggaa aaataataga tatatcaaaa 120
tgtggatggc aagtttcatt gcaaacattt gatcagcatc tttgtggtgg ttctataatt 180
aataatcatt ggatactgac agcagctcat ggaaatgctg atacttattc aattcgtgtt 240
ggaagctcta ggcacgactc cggtggtgtt gtatataatg tcacaaaaat tataagacat 300
cccaaacacg atgaacaaac atttgatttt gatgttgctc tggtacgtgt taacacacct 360
atcaagttta cagtatgtaa tagtaaatcc gtcaaaattg aagaaaaagg catcgaaaca 420
cctccaggaa aaatggtcca agtcacagga tggggtgcag aacaagctgg aggtcccgct 480
catatttcct gcaggaacat ggttctattg nagcaatgga tctgcaagaa atatgcagaa 540
gattaaaac 549

<210> 434
<211> 549
<212> DNA
<213> Ctenocephalides felis

<400> 434

ttcaacttgt ggatggcaag tttcgtttca caataggaaa ggacattttt gtggagggtc 60
catcattggc aaagaatgga ttctaactgc tgcgcattgt gtaaccaaat atgaaaacga 120
tatcgaaggt ttaaaagtta gggttggaag caatgagcat aacaaaggtg ggcgtttata 180
cgacattaaa gaaattaaaa aacatccaag atataacgat cgaaccagat acgattttga 240
tgtcgcttta ttacgcattg caaagccaat tgcatacact gcttgcactg ttgttcctgt 300
agcattggca gaaactggaa aagaagttcc agaaggcgca ctcgttagtg tcacaggatg 360
gggggctact atggtgggcg gccagcatca acgcatctaa aaggtgttaa gggtccaatc 420
gtgtcaaatg aagaatgcaa caaaaattat ccattcctgg aggtctggat gacaaaattt 480
cagacagcat gttttgcctg gttcctgaag gcggaaagga ctcgtgtcaa ggagangcgg 540
tggcctgta 549

<210> 435
<211> 465
<212> DNA
<213> Ctenocephalides felis

<400> 435

attgtattca gtgacaaagt tcaaccaatc aaaattagca aaagaaatat caaggatggt 60
gaaatctgca aggccactgg ttggggtcga ttggcggatg gggccccagt accaaacgaa 120
ttacaacaag tggaaaccac tgtaataaca aacgaaaagt gctacgaatt gtctcaattc 180
gttgaaccaa cttcgcaaat atgtacatta aaagaatttg gaagaggcat ttgctttggt 240
gattctggtg gaccactggt ttacaaagat gaactggttg gcgtttcttc gtttctcttg 300
tatacttgcg gagctggacg cccagatgtt tttgttaaag tgcgcgattt ccaatcctgg 360
atcaattctg aaattagaaa aaattaaata gatatcaatc ataatttctt gtaataaaaa 420
atggttaaat aaagacagca taatctaaaa aaaaaaaaaa aaaaa 465

<210> 436
<211> 549
<212> DNA
<213> Ctenocephalides felis

<400> 436

gcnttaggcc tatgcctagg tgaaaaagtt atattcaata actataaagt ttatagagta 60
attgctgaca acttcgaaca agttgaggtt ctgaagaatt tggaaaagga ctctgatgcg 120
tacaatttct ggacccatgt tggagcccca ggcaaaaatg tagacatcat ggtacccccca 180
cacaaacttg aggatttcga aagcacgatg caataccata gaataaacca cagtgtaatg 240
agcgatgacg tccagaaaga tatcgaccta gaagtttttg gtacaagtag agaagcttac 300
agttggacca agtatcaaga tcttgaaaca acctatgcat ggatggacag cttagccaag 360
gcacacccag gaaaagtcac tgttctcacc attggcaaaa cttttgaggg aagagacatc 420
aagggagtca agatttcatt cggaactggc aaaccaggcg tatttattga cgctggaatc 480
cacgcccgcg aatggatcac actgccactg cacttacatc ttaaacgaat tgtgactcca 540
aagacgccg 549

<210> 437
<211> 549
<212> DNA
<213> Ctenocephalides felis

<400> 437

gaaatataaa atttcttttg tatatattgg ttttatgaat gatcagatga gtggatttta 60
cagaagttct tacaccgagg acgggaaaac aaaatggatt gccaccactc agttccagcc 120
aactcatgcc cgaaaggctt tcccatgctt cgacgaacct ctttttaagg caactttcga 180
catttctata attcgaccaa aacatatggc aactttagga aacatgaaac atctgaggaa 240
agatgaagtg gttgcagacc atcctaatcg actgaaagac accttcaaaa ccacactcaa 300
aatgtcatct tacattgttg catttgttgt ttcggaattc aaaagtgttt cccaaaaacc 360
tgatcaggaa tttgatgttt gggcccgacc caatgcatat acgcagggtc aatacagtta 420
cgatattgga aaacaaactt tggtaaatta gaagagttac tggctataat tcgngccaag 480
gatggaaaaa tggnatggna gcttttctgt tttctgagcc ttggagaact gggcttntac 540
ttcaggaac 549

<210> 438
<211> 549
<212> DNA
<213> Ctenocephalides felis

<400> 438

aacaaacctt cgtggaggtt ttgtttaata tcatccagcg cgcagcaacc tcaaagaaca 60
tgcacagaat taaagtctga cactctggac atccaatgcc aaaggggatt tcgctcagtg 120
ccctgcacca gccgtatgca gcccgggacc agggcaaact tggcatgcaa accaggattc 180
cagttgctca aagaaccaga gttctcgcaa attaattgcg gaaatgatgg gatttgggat 240
aattgtttgt tttcttgcga accagaatgt ggaaatccaa caccaattga aactgttttt 300
aattcggacc cacctgtaac gtacttagca ggtcaatatc catggtatgc aatgttgttt 360
acccgaaggg aagatttatt caaaggacaa tttctattca gttgtggggg gtcaataatc 420
aactcacgaa tgatagttac aactgcttat gcgctcataa gccagaaatc gattggatga 480
tcagagagtg tgtggatcta gtgtattcgt ttaataaaca gagatnttat gctagcntac 540

gaatagaaa 549

<210> 439
<211> 550
<212> DNA
<213> Ctenocephalides felis

<400> 439

agcatttgtc agtggctgtg ttttaatcaa ctgggttatc gtcattgcga taacaaataa 60
ttagtataaa atcggccatt taataacact agccatcagt tttcaatcga acgttgaaca 120
agaaacatta aaggataaag gggtaaagga tattttggat ttttcaaaat gaagttgcta 180
gtattatttt taacattggt cgcctgcagt agcgccgtat ccttctttga tttggtgaag 240
gaagaatgga gttcattcaa gttggcccac aagaagcgct atgaaagtga aaccgaagaa 300
aagttccgtc tcaagatctt catggaaaac aaacacaaag ttgcaaaaca taaccaacga 360
tatgaaatgg gtttggaatc ttacaaacaa cgtatcaaca aatatgctga tatgttgcac 420
catgaattcg tccagacttt gaatggattc aacaagacca gatcaaatgc cttcgtcttg 480
tggtgtcgaa aaattgcgtg gagcactttc atctctctgc nacgtagaat tgcaaacatg 540
tgactgcgtg 550

<210> 440
<211> 550
<212> DNA
<213> Ctenocephalides felis

<400> 440

gttcnnncgc ccacanacat tttcaaaatg ctaaaagcan caacaataat ttttatcgct 60
ttcaattttg tgtctggtgg cgtttatgat ggttacaaac tttacgaaat aagaccccaa 120
acaaaatccg aggcttacga tttaatggaa tggcaagtaa aaccaggagt cgatttctgg 180
tccgaagcca ggatgctcaa tcaggctagc caggttatga tctcacctga acttcaggag 240
gaattcgaag gatatctggt caatggtaat tatacttgga aagttgctga ggataacata 300
gagagacttt tacaagattt tgaaagaagc agaaaaaagt caagtgcccc acgtgacgat 360
ggatttgatt tcaatgatta tcaaagatcg caaacgatca acttatacgt aaacaaattg 420
ccaaaacgta tccaaaatat gtgactgtta aggatgaagg aagaagtttt gacagcgaat 480
catcaaatct gtccaattac agatggatca attccaaaaa caagcgcgat ggtgatcgct 540
gtggtgccat 550

<210> 441
<211> 548
<212> DNA
<213> Ctenocephalides felis

<400> 441

```
ctcctccagg tcctaaggac aatgatacta tagcaattta taaattttta gatactgaat 60
tttatgctga ggttggaata ggccatcctg taaagtattt caaacttgtg gttgacactg 120
catgggcaga aacatgggtg gcctcgaaac aatgtggatt aaaatgtgtt ggatgttgga 180

atcttaataa atatgactct ttggcatcat caacatttca agaaaacggt aaagaatttt 240
cttttggctc aggcaaagaa gccataacag ggttcttttc aatagaaagt ttttatattg 300
gccacataaa tgttaaaaat cagacttttg ggaagtaac atgtttgcca tggcactact 360
tgttttcaaa agcagatgga gtattaggat tagcattcag cagtttatct attggcaaca 420
taatgccaat attttataat atggttatca acaattgatt aagaaaccta tattttctat 480
ttattgaata gagatccaac gcaaatcatc tggttcatca tgatcgggca tcaaatccta 540
acattata                                                          548
```

<210> 442
<211> 549
<212> DNA
<213> Ctenocephalides felis

<400> 442

```
tttggctgat cctgataagg aaagtcaggc tgaaccttca ccatacaatt accgatggga 60
aattccaata acgtacatca cgaatgttaa agacgattat aaacttgaat ggttcccgag 120
ggaagatgct caaaaaacta ttcaagtagc cgaagatgtc gaatggataa aactcaacaa 180
tgatcaaatc ggatactaca gagtcaatta ttctgaggaa atgtggcaaa aattgagcaa 240
tgctatgaag aaaaggataa ttagtttttc agcctcagac agagcccatt tattaaatga 300
tgccttttcc ttggccgaag caactttgtt gccctattca actgctttgg agatgacaac 360
ttatttggca aatgaaatgc attatgttcc atgggcagtt gcctctactg aattttattc 420
tttgaaaaaa ttgctatttg gaagtgaagt tatgagaaat ttacgaaata tgcactagaa 480
attcttcagc tgttatgata gaataaaatg ggatgtcatg atgatgaaaa cattngataa 540
catctcnag                                                         549
```

<210> 443
<211> 549
<212> DNA
<213> Ctenocephalides felis

<400> 443

```
ctttgtctca gccaatccgg ctggaaggat cgttggaggc gagaacgctg atgatgcctc 60
tgctccttat caggtttcac tacaatttaa aaacttccac ttttgtggag gttctatttt 120
gaacaaatac tggatcatta cagctgcaca ttgcatgggg agacgttttg aggtagtagt 180
cggtattaac agattagacc aggaaggcta tagataccaa gtagccgaaa tagtcacatt 240
gccattcgat tccgaaacaa ataattatga tttggcactt gtaaaagtta agaagccaat 300
taagttcaac tacagggtac aaccaattcc tttgggcgaa gaatatgtcg aaggaggtga 360
agaagctcgt cttacaggat ggggcagatt aggagctgat gaccctgcac caaacgaatt 420
gcaggaattg aacactttta ccatcagtca taaaatttgc aagaaagctc accanatgtg 480
gttacccaag tcagatatgt gcatttgaga aaaagaaaag ggctgctggt gacttgtggn 540
cattgncga                                                         549
```

<210> 444
<211> 549
<212> DNA
<213> Ctenocephalides felis

<400> 444

```
ggcatccaaa actttatcta ttttgtgctt ttnggtattg ttctacctag tttcggatac 60
cgaatccttt atcttaaaga ccaataaaac tgatgagaag atcgttggtg gtgaggaaat 120
aagcataaag aaagttcctt atcaagtatc attgctccat tttaatggac accgtgtgcg 180
gcggtgtgat tctgactaga caatttgtcc tgacagcggc tcattgcttc atgttcgtct 240
acagccacga agaagtcaaa gtacgtgttg gcagttctga aataaaccat ggaggaatga 300
tatttgatat tgaattctat gctcttcatc ctgactatcc agaagaccat gatgacacat 360
ctgattatga cgtggctctt gttaaacttg catatccgct taagtttagc gaagacatcc 420
aaccgatcat gatggctgaa aaggactacg aaccaccagc aggaaccaag gcttatgtgt 480
ctggatgggg cagaacatcg tcggtggcaa ttgctaaaaa tcttagagga gttgatagaa 540
ataatagac                                                         549
```

<210> 445
<211> 549
<212> DNA
<213> Ctenocephalides felis

<400> 445

```
aaatgaagtc tctattagca gtattgttgt tggttgttgc cacgtcggca acaggaattg 60
attggaaaaa tgtgaaacct atcgagcaac ctgccatcat gagcaatttg cctgcatgga 120
gaaaaactgg agaacgtatt gctggggggtg aagaagctac accacaccag ttcccattcc 180
aggtcgctgt tcttgttcac atggatgatg gcaaaagtgc attctgcgga ggttccttga 240
tttcccaaaa ctatgtgttg actgctgctc attgcgccga taaagcaaaa tctttcaccg 300
ttgttctcgg agctcacaat gtaaccgatg aaaacgaagc cggaactttg agagtagaga 360
cttccactaa agttgtccac aaggactgga acagtttctt attgagaaac gacattgcct 420
tgttaagctg catcaccagt tcaattgaat gatcgtgtca attatctcga ttgccgaaaa 480
aagccaagcc acacccttt gatattgacg cactgcttag gtggggaaga atggagatct 540
gtncacatt                                                         549
```

<210> 446
<211> 549
<212> DNA
<213> Ctenocephalides felis

<400> 446

```
gtancatcag ctgctccacc agccagtgaa ccccaagtat tgggttttcc cgatggattc 60
ccacgagttg taggtggaca cactgccaat gagcatcaat tcccatggca agtatccctc 120
caaagatttg gaagtcactt ctgcggtggt tccatcatca attctgaatg ggttcttact 180
gctgctcatt gcatcagtgg cacttccgga ttcgatgccg tagtaggaaa acacgatctt 240
tcaaaaactg aagctactga acagcgatct gccttcaaga gaaccattgt gcacaaatct 300
tatgctggag gcgtcaatcc ttatgacatc gctttgatcc aagttgccac accattcaag 360
ctgaacgaaa acgttaaagc tgttaagctt caactaaaga tgaggctcac tcaggacaag 420
ttacattgtc tggatgggga tctacttcta cttcagcttc ccagctccct aataaactac 480
agactgtgac aaaccaatcg accatacccc ggtgtgaaaa ggtctaggag gagccgatct 540
actcattga                                                         549
```

<210> 447
<211> 549
<212> DNA
<213> Ctenocephalides felis

<400> 447

```
ctgtagtcct agtattggcc ttagggctat tatgcagtga tgtttctggt ttcttcagtc 60
ttcggtcccg aaaaaccaga gaacctgtcg aatttgtccc aaagtccagg cataacgtcc 120
aaactttgtg ggtgactcaa aaattggacc acttcaatcc tcacgataat agaacttggg 180
aaatgaggta tatgtccaat gatgaacact tcaaagctgg tggaccaatt atcatataca 240
ttggaggtga atggacaatc agtgctggag ctttgattgg tggtcaacaa tatgatatcg 300
cagtccaaca taatggatat ttattctata cagagcatcg ttactacggt gaaagtcatc 360
caacaccaga tgcttctacc aagaatcttc agtacttgag cgtggatcaa tcactggctg 420
acttggctta ctttgtgatt atgtcaagag tcaaatcaca ggagccaaag acagcaaagt 480
aatcgtgtgg tggatcttac gccgtagtat ggtgttggtc cgctaaatat cctcccagtg 540
cgacattgc                                                         549
```

<210> 448
<211> 520
<212> DNA
<213> Ctenocephalides felis

<400> 448

```
atttggttgg cattcaggat gtaaaaggag cttgtcatgc cgatgaattg ggatatttat 60
tcaaaaatga gttgtcacag tttccaaagg aattggagag tgctgtggtg acacagaaga 120
ggttgttgag tttgtggaca aattttgcca aaaccgggaa tcctactcca tcaacaagca 180
atttgttacc agtcaagtgg ttaccagcta ccaaggacca actggtttat ttatcaattg 240
gtaaaaatct agaaataaaa gttaatccaa tgaaagaacg tatacaattt tgggaacgag 300
ccaccaagaa agattatttg tcacgtttgt aatggaatat ttttaaggaa aattacctat 360
agaacaaata ctactttatc agtaagttat gtatttcaac tatttaaaac cttgcatatc 420
ttgaattaac agtgatttga taactttttg catttttacg attttaatat tatagtaaat 480
ataaatatga attgtgtttt taaaaaaaaa aaaaaaaaaa                        520
```

<210> 449
<211> 549
<212> DNA
<213> Ctenocephalides felis

<400> 449

```
aattcggcac cagaagtgac cgagttagaa gttctgtcgt tttatttgtg atatttgtta 60
gaagtttatt gtaaaaaagt agaagcaata aacatgaaga gtttgctgct attcgtagca 120
gtttttgctg ctgcagcaca cgccttaccc acggcggaac caactacgac tgcagttcct 180
tgcacacctg gagaaaccaa acaagaggat tgcaatgaat gcatctgcaa agctgatggc 240
acaggatatc aatgcactga aagagaatgc aaacatgacc cagaatcaaa agcagacgat 300
catggaaaaa tttgcgaacc aggatcaacg aagaaagaag actgcaacac atgcacatgt 360
actcctgatg gtaaaaacta tatgtgcaca ttgatgatgt gtggacatca tcatgaaaag 420
agagaaactg aaattgaaga agtcaaagaa gtcaccattc aatcacttgc actacccatg 480
tctctggcca aaacaagatt ggattgnatc ttgcaatgcg cagggtngga caggtctttg 540
accgccagc                                                          549
```

<210> 450
<211> 154
<212> DNA
<213> Ctenocephalides felis

<400> 450

```
tgtgtgaaga atattaatga tgaacgagta tttgttgttc tctatgcttt gagtgctgtg 60
tattttgctg gtgtcatggt tcgactgatg ttaactctga ctccggttgt ctgtgtgctg 120
tcgggaatag cattctcctg tctgctggac ttgt                             154
```

<210> 451
<211> 215
<212> DNA
<213> Ctenocephalides felis

<400> 451

```
ctctagctcg ccatgaatct acaagagcct cgagagttgt tagaattgca gcagtgcaat 60
ttgcactacc cgaaggactc aacacgtgga ctcctgtggc tgaaatgaga gaggctttgt 120
atggaaaggc taggaatatt atacaagctg ctcatgataa taatgttaat gtgctttgct 180
tgcaggaagc atggacaatg ccttttgggt tttgt                            215
```

<210> 452
<211> 160
<212> DNA
<213> Ctenocephalides felis

<400> 452

```
ttatttccca atgcanatgc actgctggtg atttgactcg tatggctaca attgttgccg 60
acaaactagg tgcccaacct ggttgtccac caattacagc tttgaccttc ttgcgtccat 120
tacatgccat gtttangcaa agggctctgg aattgggggt                       160
```

<210> 453
<211> 322
<212> DNA
<213> Ctenocephalides felis

<400> 453

```
ggggagaatt tttagagatt ttagatgata gtagaaagtg gtggaaagct cgcaatattc 60
gcggtcaagt agctcacgtt ccacatacca tagtcacacc tcatgcttgt ggatgcgatg 120
atagttttca gagacaagac tctggcagat catctatagg cgccagtgag ggcccgtcag 180
gacccgtaca gagtccatcg tcagttgact ggattcgcaa ccagcatcag gtaaatggga 240
acgagcaaaa aattgatcct ccaccacctc cgccgttgcc agtaggagaa ttgcgctcca 300
gacaagaatc accggaaccg gt                                          322
```

<210> 454
<211> 210
<212> DNA
<213> Ctenocephalides felis

<400> 454

```
ggaagcacct taggctgtgc aggacatgta actgtatagc attttgtgcc cagagccata 60
ggtccgcaat tatgacgtaa cggatcatat gcaaaatttg cggggcaata atattgggtt 120
cctatactat tttcgtcaca ataataatag ctttgacaat catttatatt tggataaaat 180
ctcgatggtg acggacattt gaaaccttgt                                  210
```

<210> 455
<211> 464
<212> DNA
<213> Ctenocephalides felis

<400> 455

```
gnagnnnntn cngccgtcag tgtgctggga attcggcttc gagcggggcg cccggggcaa 60
ngtacggtgc acatatattg atgtggcatc tcaatgtcta nccataggta atatacaaca 120
taccctctgc acacttttgc ttcgagcaac atttcgaata agccgctgaa gaacggaagc 180
tggtgcacca ccagtaacag caccaagctg gcaataaatt actgctgtga atcccgcgct 240
tggtgaagct cctaaagcgt ccaggtagaa tggatatcgt gcccacgaat ctgagaatct 300
ggttaaagtc aaaagtgcgc caggattcnt attccatact agacccgatc gaacaggttc 360
tcctggtaaa cgctcacgga ttatctataa taagtaatat aagttgtaat gattanttaa 420
atttatataa ntaaatattg gntaacgtaa ttataaaact atat                  464
```

<210> 456
<211> 292
<212> DNA
<213> Ctenocephalides felis

<400> 456

```
gtcattttac aagtgcatta ttttttagta aaaccttcat tatagtaata gcttggaaaa 60
cataaatgtt acttcatttt tatatacacg gtgaatttgt tggaaatgga ctatataata 120
aaaatatatt tcttcaagcc gatcgaaggg tggttacaat taaaaaatat atttctatta 180
tatagtctaa gagaaaaata ctctcataac tcctgtgtat ggtcttcggc aaacggggca 240
ttcttttact gacagcgaac actttataca ggacacgaca tggccacaag gt         292
```

<210> 457
<211> 498
<212> DNA
<213> Ctenocephalides felis

<400> 457

```
aaaactcacc taaagataaa aatgttgctg ttcgaaaaca agaactccct gggcatgtaa 60
cttatcttgc tgtgaattgt gatcatacag ttttgtccat agtgttaact tctaatggaa 120
actttatatt acaattttat gatgttactt catattataa acagacaata gtattagtta 180
gcgaagtaag gctacccagc cctttgttac aaatgtcatg gaatccctgt atagcaaatg 240
tagtagctgc tactttanaa aatggcacat tgtggtcctg tgaatttggt aatggnctga 300
aaataaattc aacaggaaaa tgatgtgcaa gctttatcat tatcatggag cccgaangga 360
aaacanattg tcattgggac aaaatctggc acattatgcc aattcacacc agatttgaag 420
ccgtnaaaac atcagtgtnt ccnatatnaa agntccataa ttctgttcat ggtgagcatt 480
atcagtttga gctacatt                                               498
```

<210> 458
<211> 498
<212> DNA
<213> Ctenocephalides felis

<400> 458

```
tgaaatgtga agacgacgtc aacgaatggt ttgacccaaa atctttctcg tgcagaactg 60
catgcaaaag tgaaaacgtt ttttccgatc gaagagattg taaaaaatat tatcaatgtt 120
tcttggttaa caacaaatgg caaataaaac attatgattg tccaaatggc ttgcactttg 180
ataaaacgga gttgcgatgc atacccacgc cacccggcga agaatgcaaa agtgagattg 240
ctaagtaagg cttaaaccag gaaaacaatc ttgaatagac taattaggat tcaaattacc 300
ataaagtagt caattaatat aataaataca caaatgatct gtgcaattaa atataaaaaa 360
tatgtataaa aattaaaatg tataaaattg tattttatgt aaggagcaca aacaaaatgt 420
cattaactat agtaatttct gattatttaa aatatataaa tatagaagct ttataaaaaa 480
aaaanaaaan aaanaaaa                                               498
```

<210> 459
<211> 267
<212> DNA
<213> Ctenocephalides felis

<400> 459

```
cccgctgtcg gaccaaagtc gcttcatagc tgctaaaagt tcctcggaaa atgcttctgt 60
atcctccata cgttggatta cgtcaaagac cattttgccg tccgtctctc tttcattgtt 120
cccaaaacta atgcccaaat tgggcatggc ccgcagtatg gcaaccagtg attgaatggt 180
attactgtaa acaactggtc gatattgttt gaagtcttcg cttgtgaaac ccgactcatg 240
gataattttc atttgtttta cgatagt                                     267
```

<210> 460
<211> 351
<212> DNA
<213> Ctenocephalides felis

<400> 460

```
ttgcaacaat gatacgggag tgttggattg taatgcacgc caaattttaa ctgtttttac 60
tgatgatgaa tggaataaac taatggatga ctccaaaact aacatcagtg ttgtgcagat 120
gcaaaataat gaattgactc atttattacc gtttcctgca cttgatataa aagttttaga 180
cttgagtcta aacagaattt taagaattga gccagcaact ttcaaaaatc tccaaaattt 240
gacagagttg aacttaagta ataacaggtt gacatcaaag tttttaattc catcagtttt 300
tgagggtgat tattctccag atgcatatga gccattaaaa tcgatgaaag t          351
```

<210> 461
<211> 500
<212> DNA
<213> Ctenocephalides felis

<400> 461

ctttganttc cnncctcnga agctcntngt gtgctncnaa tttgcggaaa cacctcagat 60
tttagcggca gcggaagaaa tttgtggacc ttatgtctgg ggccgttatg atttacttgt 120
tttacctcct tcattccctt atggtggaat ggaaaatcca tgcttgacat ttgtaacacc 180
tactctattg gctggcgaca gatcgctagt gagtgttgtt gctcatgaaa tagcacatag 240
ctggactgga aatttagtca caaattgcag ttttgaacat ttctggctaa atgaaggttt 300
cactgtattt gtagaataca aaattcaggg aaaattgcat ggtgaagatg ttagagactt 360
tcattcgctt tgcggactca caacattgaa agaagaggtt caactattag gtgaaaccaa 420
tcaactgact gctctcgttg ttaacttaca aaatttaagt cctgacgatg cattttcttc 480
tataccttac atgaaaggct 500

<210> 462
<211> 176
<212> DNA
<213> Ctenocephalides felis

<400> 462

ctgtcactgg ctgatgggtc gacgctcacc tacgatctat acaaagctct taatccggat 60
aaacatgaag atgaggtaac tctggcagtg tgccctggca ttggtaactc ttcggagtca 120
gtctacattc gcacatttgt ccattacgca caatattacg gatacagatg tgccgt 176

<210> 463
<211> 498
<212> DNA
<213> Ctenocephalides felis

<400> 463

cttattccta aagctagtaa tgccgaaagt aaggttttct atcttaagat gaagggagat 60
tactacaggt atctagctga agtagcaaca ggagaaaccc gtaacaccgt cgtagatgat 120
tcacagaaag cttatcaaga tgcatttgag atcagcaagg ccaaaatgca gcctacacat 180
cctattagat tgggtcttgc acttaacttt tccgtatttt attatgaaat actaaattca 240
cctgataaag cgtgtcaact tgcgaaacag gcattcgatg atgcgatagc agagctcgat 300
accctaaatg aagattcata taaagattca actctcataa tgcaattgct gcgagataac 360
ttaacactgt ggacatcaga cacgcagggt gatggagatg agcctcagga aggcggggac 420
aactagtcct gaaaacctta cctatccact tccgtatcct taattcaccc ttatacaatc 480
ccttcgttat ttggtaat 498

<210> 464
<211> 246
<212> DNA
<213> Ctenocephalides felis

<400> 464

cagattcatg ttgtaaaaca gaaataccaa tgtgtgggaa aagtgaccat cccagcaata 60
ttccttatac gggttgtata cacagtatga ctgatagatt ggaacatcaa ctttggcttt 120
taggagcagt aggcctggga atgtgcgctt tgcccgtatt tggaatgata ttcaactgtt 180
gcctttatat aaaattgaaa gattttattg acgattgaaa aaaattattt gaaattattt 240
taacgt 246

<210> 465
<211> 477

<212> DNA
<213> Ctenocephalides felis

<400> 465

atctttatca gtgagccagt tagacataac gtgaccacat atacctacag catcccattc 60
aatgttagat agacctgata catcggagta agtagcctgc gaaagcaaat gctgtaaggc 120
gtgtccaaat ttgttgaata aaattgatac ctcttttaga gacagaagag aaggtatatt 180
gccctcagga gggatttaga cttaaaatta atgatgctaa tggtgttgta tccacaattt 240
tacttttatt gcgcatactc aacatccatc ctgaactctt ttggaatatc ttctcttctt 300
ctctagtata aggatctaaa taaaatcagc cagtggcaat tcagctgcag aatcatgtat 360
atcaaaatac cttacatctt tatgccaagn attcatattt gcctttcgat aattttnata 420
ccatcaactt ttnacacaac tggaatagcc ccgtcnaaaa cctttggaat nggaagn 477

<210> 466
<211> 395
<212> DNA
<213> Ctenocephalides felis

<400> 466

ttagcccaag tttcacagca ggtttggcag tcactataat agtatgctcc ctttcatttt 60
ctcctgaacc aactttgcat gtgtaagagc cagcatcatc ttgttcagtt ttttcaataa 120

tgaattgatg ttcttccttt attaattggt aacggtcctt gagatcattg atttcttcta 180
ctttttttctc atctttaaac cactgatcaa caggaccatc tttcaatgga caggttagaa 240
ccaatggact acgaatatca aaaagttttt gcgacgtggc ttcaccttct ctggcataaa 300
cactgctttg gaccaaaaat aataaaattg cactacacaa aaactgcttc atatttattg 360
aattttcttc ccctcgattg aaatagttca taagt 395

<210> 467
<211> 211
<212> DNA
<213> Ctenocephalides felis

<400> 467

```
ggaagcacct taggctgtgc agggacatgt aactgtatag cattttgtgc ccagagccat 60
aggtccgcaa ttatgacgta acggatcata tgcaaaattt gcggggcaat aatattgggt 120
tcctatacta ttttcgtcac aataataata gctttgacaa tcatttatat ttggataaaa 180
tctcgatggt gacggacatt tgaaaccttg t                                211
```

<210> 468
<211> 500
<212> DNA
<213> Ctenocephalides felis

<400> 468

```
ncantggcca tttttatcag ttctttcttt atcaactgat tgttgagttt ttgctctnag 60
tgtttcancn aattcttcan tagccgtctc caagaagacc cctcattcta taaacaactc 120
tcattgattc tccttcgcat tgnncagcca accacacctt tttataaact tctttcactg 180
gaagatccaa actcatgatt ttgttgntga ccagtaattc cataccattg ncatcttcta 240
ataatgctac taattcacaa tcttgacaaa tnttattttt aacatctctc attaatggcc 300
aagaccaggn tcattctgga atatggatta cccaacatgc gaccctgtaa aaagtcttct 360
tgttgaggat ccttctcaag agataagaaa aattccccaa tatcattttc ttctggataa 420
taattgagca aagctttaaa tataaataca ggagttcgac atcntcaatg gatattatta 480
ctgnttccat gccccagaca                                             500
```

<210> 469
<211> 251
<212> DNA
<213> Ctenocephalides felis

<400> 469

```
aataaatata attttattat ataagaaaca tatgtataac aatacaaatt ataactattt 60
aacagtcttc ttgtgataat ttcttgacgg ccgtatctgt agactttgat acaccttttt 120
tcggtaagct ccagaacacc tgatccctgc cgaatacgtg agcttcagtt agcgtttccg 180
gtaacttttg gtgtaaagtt tccggcaaga acattccgga agtcgcgccc accatcatca 240
ttacggaaag t                                                      251
```

<210> 470
<211> 498
<212> DNA
<213> Ctenocephalides felis

<400> 470

```
caataaatat tgaatttaca ggcttatgaa caccattaag agtaaacctc aatttgatca 60
cagcaccctt ctctcccaac tcaattttct tcgtataccc catgtaattg atatgattag 120
ccctttcctc ttcctcgaaa taaacccaat tatgtagacc tgttacttca caatttttta 180
attcagctaa gaaaacatgc tcaaatgctg aactaccgat tttgcccctc ctctggagta 240
taaattaaac caaatcgttt tcaataaatc cttatgagtt tggggatctc tagtcacaat 300
tccctttca ataaaaaaat tcataagatg tcgcatcagg ctggtggata acattgcatc 360
taacaaagca ttttcttcat ttctttcttg tgcagttaca tgttcattta ctgcagtatc 420
taattcataa ttattaaata atggtttcat aagcgctata gttttaatat caaaagctgc 480
tgattcacaa caagcaat                                                498
```

<210> 471
<211> 105
<212> DNA
<213> Ctenocephalides felis

<400> 471

```
agaggggtag cttttattcc atttattgat gacatgcctt cttttaatcg caaagtggat 60
ggaccattta taatgcctgt tgtagacaag tataaagaca tgggt                 105
```

<210> 472
<211> 496
<212> DNA
<213> Ctenocephalides felis

<400> 472

```
ttttttttt ttttttttt ttttgaacca ttttataaaa ttttataatc aantcantac 60
tataaattta taataatatc aataacaaaa gttattttat agtagtcata atatactttg 120
cattaatant tcatacacac acacacacat tacttgtctt ttcaattcca atttgttctt 180
attctcgtcc ttgttcaaat gtcgatgttc aatgttgtat taagcattct attgtgantt 240
tagtattgga tttcttaaca ttaaagcagc ttctgatgat attattggca acgcattgac 300
acaatgatan tttattaaat gtgaaatact ttcaaacatt ntatctctgg tgtcgaacca 360
ctcctttagg atcaataagt aataaatgct tcttattaac tccttgaata ccagtcagca 420
catattgcca ggacttgctc gtgactctng aactagaaaa tctncatnng ttttaccaaa 480
ccctnagaac aactnt                                                  496
```

<210> 473
<211> 500
<212> DNA
<213> Ctenocephalides felis

<400> 473

```
aaataattca ggagccccgc aaagtcagan ttctatatga ctttgaagca gcagaagang 60
gacganttga ccttcttagc tggtgaaata atccatatgc tagatgattc acatcctant 120
tggtggaaag gatataatca acatggagaa ggtttatttc ctgcaaactt tgtaactgct 180
gatttatcaa gttgaaccag aacantttag aattgatgct aacaagaaat ctgttcantt 240
ttctgatgct gttcangtca aaactatatc tcatgatgat gaaaccagtc ctgaaattaa 300
tgaagaaagt atagatactt tattaaattt attacatgaa gcaaatcctg aanatcctaa 360
tgatgataca gagcaaatgc tcaacttaga ngttcangta aataaaatgg gccctctgat 420
tgatgctgaa ttaagaaaat attgacagga aacatgctca acttacgcaa ttgagtggcg 480
atttagtaga agcacttant                                             500
```

<210> 474
<211> 500
<212> DNA
<213> Ctenocephalides felis

<400> 474

```
tttttttttt tttttttttt tttttttttt tctgacagac tcgagctagg ngnaaaaaac 60
tacattttta cagatgcgat taatgaaaat taaagtttgc tactaatcca catctatcct 120
tgattctgtt tttgctgcta atgtgccaca atgaaattta gatgctctca ttttnttaaa 180
actaagtgtt aaatcatcca ttgtaattgg tcgtagagca tcatgaaatt cctcttctgc 240
gtcttgtatg tgtggcacca aaggtgattc agngtgcata taatctctaa ctntatacac 300
agaagcattt cggcataatt cccttaagtc tgatcctgaa aatccctcag tgagtttaga 360
taattcatnt aaatcaactt catctgaaat aggttcattt tctaaaataa gctctagtat 420
cttgaatctt tgntgnctta tgggcatgcc aatatggaat gnggctggca tacnccgnaa 480
aaatgcttta tttnaatttn                                             500
```

<210> 475
<211> 462
<212> DNA
<213> Ctenocephalides felis

<400> 475

```
ttggtccaac tgtttggcat ttgtggatat attcaaaatc agccaatgct aatttttttt 60
nggtgttaca ttggcatttg ctacagcaca gattttttta gtcactgata tcttatttgc 120
ctatattaaa agagaatata ccttgaagag tggcttgaaa cgaatattga aaggcaaacc 180
tgcaaaatta gctttagaat aattttatta taatgtttga attgaaatgt ntttganttt 240
aaatatgant taagttttaa ataaaagctt ttttattttt acattataat attatgccat 300
tagcctatat tgttacacag ttaaatttaa angtatttaa taaatgggtt tacaactaaa 360
caaatcacaa acacacataa atataataaa aactggatga aaatanttga atatatatat 420
aantcgtnaa gaangtcaac cttctgnnat aatggagctt gt                    462
```

<210> 476
<211> 549
<212> DNA
<213> Ctenocephalides felis

<400> 476

```
gnttgactga cctgaaataa taaatatcat aactgcctgt gtaatttttt atttgtgtgt 60
tatacggtct ttaaataaat aaaggtttct taaaaatgcc gctaataatc atgacaggaa 120
tacccagtag tggaaaaact catcgaacac tagaaataaa aaaatatttc gaggaagaaa 180
gaaagaaaac agtacatgtg gtttctgaat ttgaagccgt cacaaaatca ggttattcaa 240
aaaatgatat ttatcttgat gcccaaaaag aaaaaatcgt tcggggcatt ctaaaatctg 300
aagtttttcg attattgacg aaggataatg ttgttattct agatggagga aattatataa 360
aaggatacag atacgaatta tattgtggga gcaaagctgc acgagttcct caatgcacaa 420
tttggacatc tatatctaaa gatgatgctt ggaagttcaa ccaaaattca ccttccatat 480
caaaagaagt tttgtgagct ttgttttcgg atgaagacct aatccaacaa atcgtgggga 540
tctnctttt                                                         549
```

<210> 477
<211> 549
<212> DNA
<213> Ctenocephalides felis

<400> 477

```
gatgacagta ccatcattca aaggcaatat aacagtcttc accaatagtt acttgggact 60
cggtttaaaa gctgccagac atgcagtttt tacgaaaaac aatggagact ccaaatcgtt 120
gtccagccct tgtattaatc ctatcatcaa aaacaaacct tgggtgtatg gcaacgttga 180
gtacagtata agtggcactc cgctagaatc aaagaaatta gaagtagatt ggccaaaatg 240
ccgcaaatta ttagcagaca ccctgttacc tctagtcgac ccaaaaccta taggtttaga 300
agtacaagat attgcagcgt tcagctattt ctttgacagg gctaccgggg ctggactgat 360
agatccattt ttgggcggcg aaataacagt tggggaattt gaaaaaactg caaaagccgt 420
ttgcaaaacc gcaaacaccg accagccctt catgtgtttc gatctaacat tcatatcggc 480
ttgctaaagg acggattcgg attaaaacct gaatcgaatt aaaatactaa aaaagatcgc 540
accacaaat                                                         549
```

<210> 478
<211> 417
<212> DNA
<213> Ctenocephalides felis

<400> 478

```
gttaagagct gtcaaattat tgtacaaata tttctaaatt aaatacgtat atataattta 60
ataaataata ttttactact atgaaaagag ccgcaaaaaa ccttctacta gaccgtctgc 120
acaaaggcgc cgtcatggcc tgcatgggca tcaccgtttt gggaacactc agtcttggat 180
tccgagttta tcaatacttt actgatataa aacctgaaat acaaagaaaa caaatattgg 240
caaagaacga gctgttaaaa gaaggagcct cggacatatc attatacgag agcaatatca 300
cgttaaagga ataactccta ggatagtaga tatatttagt actgattact ccaaaaatgt 360
atgttatata atgtaaataa gacttataat ttatttcaaa aaaaaaaaaa aaaaaaa    417
```

<210> 479
<211> 549
<212> DNA
<213> Ctenocephalides felis

<400> 479

gttaaccgaa atccgaatta taaaatttat aaagtcaacg cagcaaattt tgaaatcgtc 60
gatattgaat cgtggtacta cgatttagcc gaggcaaata aaaattctat tgtaaaccca 120
gaatggaaac aaatgtacgg ttcattcaag acagagtttg gcttaaattc attgaacagt 180
tcagagatgc acaggcttgt attgaatatg aaaaccaaca ataaacttgg aaaaaaatat 240
tttgaataca aagttaaacg tgctgatcct gaattaaaaa aaggctgtga taaaacctgt 300
cttaaaaatc atttgtgcgc aatagttacc acagttgtat cagatctcat ccagtgcaaa 360
aatatcatca aatcatcatc aagcattctt cagcatttga atatatatgt cctaggagct 420
attgtaattt ccaattattt attattataa agtcattttt gttaagttat tataaattag 480
aataaatcat tatgtggaaa tatgtaacgc ttgaaaagct agacaaatca atatttaga 540
aaaaaaaaa 549

<210> 480
<211> 549
<212> DNA
<213> Ctenocephalides felis

<400> 480

gcacatttgt gttaagtttt aagcaggaaa gttgaacata aaataacatn tnntnnttnt 60
tttgcgtgtc tttaacaatg gatgttgaaa acgtcgaagc atctggaagt gttccaaatg 120
ttgctggaga tgcatgtggg gattctatgg tcacggatgg gaacgaaacc cctgcaagtg 180
ccactggcgc agcaggttta gcccaagaaa gagttaaacg taaagctaaa agaattgtac 240
gacaaaatag tcgagaaaac gtggcctcag gagcggtgtt accacaacgt tcttggaaaa 300
acagccgcag acctagaaat ggtcatggga gagggctgcc caaaaaaggt ggcgcggggg 360
gtaaaggagt ctggggatta ccaggctcag agcttttaga agagtatgaa gatatcaatg 420
atccaaactt tgatactgaa tgtataagtc acaaagatat agagttgaag gccgtattcc 480
tgaagtttct gcagaagaat tcttgaaaaa ggctgacccg tattcttgat tttgacatgg 540
gatcccaag 549

<210> 481
<211> 549
<212> DNA
<213> Ctenocephalides felis

<400> 481

ggnttattta attagaagac tgttaagttt tagaagatat gactgtagtt taacagttta 60
tacgttttat aaatcagctt ctgaaacttt aatttcataa tgatttctgg cgattttcct 120
gaggacccag aaaaggagct gcagagttta gaagatgatg ttgttcaaga aattctcaaa 180
actggcactg atctgagaca atactcgaaa caaatagaaa aagaactgaa agatgtagaa 240

aataaatcta tacaggatta tattaaagaa agccaaaata tagctagctt gcacaatcaa 300
attggggctt gcgatgacat ccttgaaaga atggaagata tgttaatgag ttttcagagt 360
gttttaggta atatcagttc tgaaataacg tctctacaaa aaaaatctgt ttcaatgtcc 420
attcaattat caaataggca ggctgtcgag gagatctctc acagttatcg aagatatttc 480
tgtactcaaa gtctgtaccg gaattttgga tacccagtac tgagaaagat tataactcag 540
ttcaaatac 549

<210> 482
<211> 549
<212> DNA
<213> Ctenocephalides felis

<400> 482

```
gtgaaagaga ggctacaaaa agacttcttg cacagctacc aataagcgca caatcatata 60
gcagttctcc ttatctggat ttgtcattgt ttagctatga tgacaaatgg gtatcagtta 120
tggagagacc taaagcttgt ggggaatatc ctattagatt ttatgcacgt gattctggcc 180
ttctcaagtt tcgaatatat gcaggagctg ttgctaaaac accacctgca gccactagaa 240
gattggtagc ttttacattt catcctaatg aaccatttgc cattagtgtt cagaggacaa 300
attctgagta tattgtaaac tttcatgtca gacatgctag ctagttttta caaattattt 360
gcaaaagcta aaaaggttac atgatgtaat caattaggtg ggtgttgaga agatttgaag 420
gtaattttaa ctcaaaacat atctttataa tgaaattgna atattaacat gcgcatatgt 480
tgtgatcaat ttaaagtggt atagatgatg tgcctttgng cagggatgna gttgattatt 540
atagatatc                                                         549
```

<210> 483
<211> 550

<212> DNA
<213> Ctenocephalides felis

<400> 483

```
aagccgcatt tgttattgag aaatatatgc taaaatatat tgtctcagaa ttaatataat 60
gtgctgagtt tgcattaata ttgaaacgtt ttattcattc tgaggcatct atataaaata 120
aataaatatg aaagcaattt tgataacatt gatagtcgcc gcggctgtgt attccgtaag 180
gcctgaggtt ttcctggaag aaaacttcgt agacgatacg tggacaaata catgggttta 240
tagtgaacac cctggcaaag aattcggcaa attcgtgcac actgccggaa agttctataa 300
cgatgccgaa gcagacaaag gtttgcaaac aagtcaagat gctaggttct acgctctatc 360
tcataagttc aaacctttct caaataaaga caagacatta gttgtcaatt ttctgttaaa 420
catgaacaaa acattgactg tggaggtggt acttgaaggt gtcgattgaa gttgaatcaa 480
aaggacatgc atggtnaagt cctatgaaat atgtttggcc tgcatttggg ccaggaacta 540
aaaggtccgt                                                        550
```

<210> 484
<211> 550
<212> DNA
<213> Ctenocephalides felis

<400> 484

cgtgcaatta aattgtgtca aatttcaaat ctaccctgcc aaaagggcca cattgattat 60
ttaaagtccc tcgcactcac acgcacggca ccactatgtc tacggtcgac aaggaagaat 120
tggtgcaacg agccaaactg gcagagcagg ccgaaagata cgatgatatg gctgcggcga 180
tgaaggctgt tacggaaacc ggtgtggaat tatccaatga agaaaggaac cttttatctg 240
ttgcctataa aaatgtggtt ggggcgcggc gttcatcatg gcgtgttatc tcctccatcg 300
aacaaaaaac agagggttcc gaaagaaaac aacagatggc aaaggagtat cgggaaaaag 360
ttgaaaagga acttcgtgaa atttgttacg acgtactggg ccttcttgac aagtacctta 420
ttcctaaagc tagtaatgcc gaaagtaagg tttctatctt aagatgaagg gagatactac 480
aggtatctag ctgaagtagc aacaggagaa acccgtacan cgcgtagatg atcacagaag 540
ctatcaagat 550

<210> 485
<211> 550
<212> DNA
<213> Ctenocephalides felis

<400> 485

gtttcgggcg ccatgaagct aattgaagag aaaactgacc gcgtccacgt cctgaaccag 60
gcgtcggtgg acacctggag cgtggccaga gagattgcgg cagctagcac ggtagctgat 120
gaagaagatg cattttatgt gtgtgatatc ggggatatcg ttaagaagta tcaattgtgg 180
aaggagcata tgccgagagt gcgaccattt tatgccgtga aatgcaatga cagtccgatt 240
gtattggacg tactggccgc actcggaacc gggttcgatt gtgcgtccaa ggttgaaatt 300
aataaggtgc tgccaatggg tgtcaaacca gaggacatcg tgtttgcaaa cccttccaag 360
ccggcgagtc acatcaggca tgctgctcga cggggggtggc gaagatgacc tttgacaacg 420
aatacgaact tcataagatc aagagatttt acccaaatgc cagattgatt attcgcattc 480
gtgtgattct gaaattgcca atgccactcg gaatgaaatt ggctgcgatg cattcaatga 540
accccgcgct 550

<210> 486
<211> 549
<212> DNA
<213> Ctenocephalides felis

<400> 486

atcgcgaata aataaatgtg gcgatacatt aaatcttcat tattagtgaa caaggaactg 60
atttaatatg gaagataagc ctgtgaagcc atgtaaagag aagcgaagga acaatgagaa 120
gcgtaaggaa aaatcccgga atgctgcacg ataccgcaga tcacgggaga ccgagatttt 180
cactgagctg gcggaatgtt tgcctcttgc taaagaggac acggaccatc ttgacaagac 240
ctcaataatg cgactgacaa tctcgtattt gcgaatccga gctgccgtac ctcaaattgt 300
tccagaagag gatatttgct caacctcatt atcgaataag gacaatgaaa acttcttatt 360
acaggcgctt ggaggatttt tgatcatgat ctcacctgaa aacgatatcg tatacgtatc 420
aagcaatgtc aacgaatatc ttggaattac tcagatcgat ttaatgggtc aaagatgttc 480
gactcagtca tccttgtgat cataatgaat taaagaaccc ttccccaact caaccacaaa 540
agatgntac 549

<210> 487
<211> 549
<212> DNA

<213> Ctenocephalides felis

<400> 487

```
caccacatcc agttgaatta ttttaacatt tactttttgc caacatatgt caacgcgttc 60
tttaatctgt gtttgaagat caagtgatat aatttagaat aaagtagttt caattatagt 120
atgtccacaa tgaatccaga atatgattac ttgtttaaac tgctcttgat tggtgattca 180
ggtgttggaa aatcttgtct acttttacgg tttgcggatg atacttatac agaaagttat 240
ataagcacaa ttggtgtaga ttttaaaatt agaactatcg acttagatgg aaaaaccata 300
aaattacaaa tttgggatac agcaggtcaa gaacggtttc gaactataac ttcatcatat 360
tccgtggggc acatggaatc attgttgtat atgattgcac agatcaagag tctttcggaa 420
atgttaagca atggctcgaa gaaattgatc gctatgcttg tgatagtgna ataaatactt 480
gaggcaacca agagtgatta actccnaaaa agttgtagac tcactntgta agaattnctg 540
ccagntaga                                                        549
```

<210> 488
<211> 549
<212> DNA
<213> Ctenocephalides felis

<400> 488

```
cagcgatcag atttaccaga cacatctcat tggcaaagtt caatttccaa agatgaagct 60
gcntttgaaa tagactactg caatcctcaa tcaagcaatc aacattcgaa gncaactaaa 120
gataataaca atgacggtac tactgntcca gacgaagatt ttttttcgct cattatgaaa 180
atacaaagtg gaaggatgga tgaccagcga gcaagtataa atataaaacg agtaatatag 240
aactctactt taataattgt aataatattg tatatggatt attagattac ttttaatact 300
agaatatttc caatttttta atatcatttt ttgtggatta catacataga atagtctggc 360
tatcgattgg tactttgact atgaattgtt gtacctttga accgcaacaa tttctaatat 420
aaaatgagta gaaggtttat tagcgacata atagtgacat tgctataata tagcatttaa 480
atcaaacaaa ttaaaaatgt gattttatta ataggtacta tcataaagtc acaaaagccc 540
ttccggtac                                                        549
```

<210> 489
<211> 547
<212> DNA
<213> Ctenocephalides felis

<400> 489

```
tgacaaaggc aaaaatgggt cgtcgaccgg ccagatgtta tcgctattgc aaaaacaagc 60
cctaccccaa atctcggttc tgtcgtggtg tgccagacgc taaaattcgt atcttcgatt 120
tgggtaagaa gaaggcaggc gtagaagatt ttccactatg tgtgcatctt gtatctgatg 180
aatatgaaca attgagttct gaggcactgg aagcaggacg tatttgctgt aacaaatacc 240
```

```
tcgttaagaa ttgtggtaaa gatcaattcc acatcagaat gaggctgcat cctttccatg 300
ttatccgcat caataaaatg ttatcgtgtg ccggagctga taggctccaa actggaatgc 360
gtggtgcttt tggaaaacca caaggtactg ttgctagagt tcacatcggt caaccaatca 420
tgtctgttcg ttccagtgac agatacaagg ccgctgttgt aaagctctgc gtcgtgctaa 480
gttcaagtcc tgcagacaaa gactatgttt caagaatggg attactaatt tgacctgatg 540
ttatana                                                           547
```

<210> 490
<211> 353
<212> DNA
<213> Ctenocephalides felis

<400> 490

```
tgataaattg cctgcaagaa tgagtttcag ggaacgaaaa gaatcggcca aatgcatgca 60
aaaggttgca ttaaatatac aactagcagc agccagagtt tcaactgatg ttggcattaa 120
agtaggagaa gccctcagga atgtagtgga acttcgttta gatttgggaa aatgttcttc 180
agacaatttg aataaatggg aatccagatt ggaagaaatt aatgatgttg tggaaaattg 240
catcgtgtga aattgtaact tgaaataatt tttcttaact attagtttta tagatgaaca 300
aatacataat ctaataaacc agcaagtgaa aaaaaaaaaa aaaaaaaaaa aaa        353
```

<210> 491
<211> 373
<212> DNA
<213> Ctenocephalides felis

<400> 491

```
ntgnttccat ccatattcat cacccaaatc acgttccata tcatctattt cctcatcttt 60
actatanctt gcataatctt tgcgtantgt tctcancana atcatagaca caagtcctac 120
taaaantagt accancatan anctgttana tangctgaac caatgtatcc tatgttgaaa 180
gaagttanga tccaantact tgtcaaacct attttcaanc ttgacattgc ngggtttcca 240
tgtcacttcn naagtnaann tcanaacngc tccagtttta nngtagttct ttcttacnan 300
atganacant cacatctacn anttgcgtgn cantatatcn annnncaaat ttcttgtgtg 360
tgtagacnta gtt                                                    373
```

<210> 492
<211> 498
<212> DNA
<213> Ctenocephalides felis

<400> 492

```
attgaaaaag ttaaaattcc aagtaaagat gttgacaaaa taattttctg cctaaatttt 60
tataaagata tgagatttaa gaacaattga aaatttacta tatatacata ttacaaaaat 120
tcaaataatt gttattcttt gtcaaatacg ttgttctttt tcatgattac gatcattgtc 180
agctgggtca tgtttcacat gtgatgtgta tatatcatat cccaaggata ttgtattatc 240
atataacact ctaaacctat gaggtaatat ataaaagttt atgatttggg ctggcggcca 300
```

```
aactacccat tcagcagcgt aaagcctcca agccttttc ttaatttcct ctattaattc 360
atctttacta gtttttcta gtatagctaa tgtaataaag aacatagaaa tgcatattgg 420
tgagcataca atttgatcta tcacaacttt tttcatgact attcctattg tgcgtcctgg 480
caatcgttta tctagata                                                 498
```

<210> 493
<211> 308
<212> DNA
<213> Ctenocephalides felis

<400> 493

```
tgggaacagt taatttaaaa taacaaaatg aaaggaacat tattaatatt atcatgtcct 60
gtgatcatga taagtgccga atatgctgac gtagatgtgt gccaagattt ggacgatgga 120
acttttcttg ctgattcaaa caattgccaa aatttcttca tttgtgatgg aggccgagct 180
tggaaaatgt attgtccagg atcactttta tggaatgatc acgaaggaac atgtgattac 240
gcacaaaatg tagaatgtta ccaaccagaa taaaacattt taatatcaaa aaaaaaaaaa 300
aaaaaaaa                                                            308
```

<210> 494
<211> 500
<212> DNA
<213> Ctenocephalides felis

<400> 494

```
ctattacaac taggttaatt tgttagtaag ttttgttata actgttattg atattaaata 60
tgtattattt ttatatatgt aaacgaatta taatttttgc tctttgatta cttctactct 120
ggtgcattaa cttttattca actagattaa atttttggtt tacttcctat gcctcacatc 180
agtgtccttc atgtgatcag gagtattttt tcgatctgga ctttgatctg tatcactctc 240
ctccatttca gctttgttgc gtttcttttc ataaatgaga ataatcgcgc ataaaattat 300
tacttcagca cagatcccaa aaatggccac aaagcagcta atttatcttg aactcgaacc 360
aaggntgtag aattaacagt aattccaatg ccttgattag tagcaataca tgtatattct 420
cctcgatcgc tttttcagcc aaatcaattt ccaagcagaa tttggttatt gcatgatcaa 480
caattgatcg acggntgatc                                               500
```

<210> 495
<211> 244
<212> DNA
<213> Ctenocephalides felis

<400> 495

```
gatcggttgc aatttgtcat tgcaccaagc ataaattgat gggtccggtg cataggttat 60
aaaagaattg ggatttgtgc atttcatgac ggtgcaatca gcttctgacg atttcttttt 120
acacatattt ttagaatgat catatacata gttaggtggg cattcgaaaa cctgaccttt 180
accatctttg cagaataagt atcttgtgca gtctttcgga tctggctgat atcctacaac 240
cgcg                                                                244
```

<210> 496

<211> 500
<212> DNA
<213> Ctenocephalides felis

<400> 496

```
ccaatatcga agtgcgtgtt cggccgccac gctcaatgga gatccgttta gatgtgtttg 60
ccaataacaa acgcatttat ttcgacagac cttctctcaa gtttcaacac ttccaaggtg 120
ttactgtata tacaccaaca tacatcttga atcagtctga agttgttata atgtttgctt 180
ccggagccgg agtagaagtt gtagaaaatc aaggatttat gactgctaga gtttatttac 240
catggacatt tattaataaa actgctggtc tactcggaaa ttggagttgg gatatggcag 300
acgactttgt caaacctgat ggaacttttg tgcctgttaa tctcaacagt tttgaatctg 360
ttcataaaga tttcgcacgt cactggatgc tggcggatcg tgnaaatgaa cacctcggag 420
cngnactctt nacttcgnga atttggtcgc ncagccagtt attatgcaaa ttcctcattt 480
taccaaactg ggttaaagaa                                             500
```

<210> 497
<211> 411
<212> DNA
<213> Ctenocephalides felis

<400> 497

```
ttgtcattnt tgttctactt tgaagttgtg cgctggacaa gaaaccccaa ttacaacaat 60
caattgtaga gactcaaatt ccgatgctcc attttgtgta gatgatatgt gctcatcaaa 120
acctggggaa aactgtaaga cggcagaaac tacatgcgcg gntgnaggat atcagccaga 180
atccgaaaga ctgcacaaga tacttattct gcaaagatgg taaaggtcag gttttcgaat 240
gcccacctaa ctatgtatat gatcattcta aaaatatgtg taaaaagaaa tcgcagaagc 300
tgattgcacc gtcatgaaat gcacaaatcc aattctttta tacctatgca ccggcccatc 360
aatttatgct tggtgcaatg gcaaaatgga cccgatcggc ccctgaatgg g          411
```

<210> 498
<211> 498
<212> DNA
<213> Ctenocephalides felis

<400> 498

```
ccccatattt ctttgctcgt ttagttgngc aacagcagca tcttttaaag aatacaaatg 60
gggcatttcc atttcagttt ttgctaggca taatgctttt tcaaacattt caattgcacg 120
ctcaagattc cctcgttgaa cctcaatagt tcctaaagtt tcatagccaa attcacattt 180
gggatcaagt gaaatagctt cattaatcaa ttgaactgca gtatcaacat cacctgtcca 240
ttgcaacctt agtaatcctt tatgaacata aacaatagca ttattaggct caatttcaat 300
ggctttagag aaatatgaat ctgctttgga gtattgtctt tgatcacaaa gcacttgtgc 360
atataaagna tagcactcta tacagagagg aaattttta atggcattgn caaaggccca 420
attatttctt ttgccttatt catgtcntaa cagcaaaagc atatcgatgg tcgtatatat 480
tttttggaca taagccat                                               498
```

<210> 499
<211> 598
<212> DNA
<213> Ctenocephalides felis

<400> 499

```
tccaatataa agaaactctg tcacattgcc atagaaagga ttcaatacaa aatttatgcc 60
aacttataac tggagatgcc ttactctatt ctatgtttcn agaanaatca gttcccgtga 120
cttgcccttt gaaaggacct ttcacattca catataatan gggacatggc gaatgtatga 180
atccagtatc gaacattgaa agttgcactg aagacagtcg acttatttta acataccagg 240
catgtccgga tgttcatgga actgaaagtg cantggaaga actggagtgt ctagcaacat 300
ggaatgaagg taatgctcga tacctggttg gtaaaatgaa tcatcgacat gccataacca 360
gtgaagatcg atacagatgc tttgtttatg agaaaataac tggaattgga gataaagtaa 420
tggatacaaa attgcacaat catgagatgc tacttgcaat ggattgttta gtgctacaga 480
aggtcacgga caatgacttt aanacaagct gctattcctg agcggtgtcg tttccgaatt 540
ggttanctgc tggaccatca cactggcaaa cagttgataa tcacaantta ctggttca   598
```

<210> 500
<211> 462
<212> DNA
<213> Ctenocephalides felis

<400> 500

```
ccaaatattg cagttcctac acgaacaata ttgctgccca tttcaatcgc ttgttcaaag 60
tcatcagaca ttcccataga taaattaact tcttttggtt ctaaatttaa ttccttgcat 120
aattgctcac gacattgttt taaagtgaga aaatctgggt tgggtcctaa acttgtatca 180
tagccatatt ttccaatagt cattaaacca tcaacaagta ggtttgggca attttctttc 240
acatatttgt atanagttgt tgcttcattt gggtgaacac catgtttttc tatttcacca 300
cttgtantta tttgaatcat aactcntaat ttatcaccat gaattttatt agctgaatct 360
aattttgacc aancattctg cacactgtcg ggntaattta aatgagtgaa ttgtttcnac 420
cacnatatat cncaggtatg ttaacacctt aanaatttta nt                    462
```

<210> 501
<211> 216
<212> DNA
<213> Ctenocephalides felis

<400> 501

```
agactgaccc tgggcccagg cgtttgctta anttgtgacg ttaaagacga ccaaccacag 60
tgnacgaatg atgaaataag cccatccaca actgctccaa gttcaaacgt gaatgaaagc 120
aatggaaacc tcaccgaatc acaaccctta agccaaaata cttcaacaaa tactccatta 180
gaaacatcaa atacctcact agcagaaagc agcagt                          216
```

<210> 502
<211> 489
<212> DNA
<213> Ctenocephalides felis

<400> 502

ttgttgtatt ccatgcccac tgaatccagt tgctatatat gtattattgt gatatggatg 60
caatcctata atgccatttt catcaaaggt attgtattcg taataaccag cccaagcact 120
tttaacttta attgcttcaa aacatggaac acgatgtgct aaatgtggcc aaacgttttg 180
ttcaaaataa tccatatcta catctaagtt atcgactggc ggttctttgt caggatctgg 240
tgaacgacca cagatatatc tgccacctaa tccatctctt ctaaaatatg tatttgttgg 300
atcgattgtc agaggtgtat ttaaacctgg aggcgaatga tgttgacact caaagctata 360
cacatatctt tccacaggta aaggaattga cagcaatcct tctccagtac caattttagc 420
taatctggca acatgaccag actgangtcc tgcagctatg acacatattg caaactttat 480
tgggtataa 489

<210> 503
<211> 425
<212> DNA
<213> Ctenocephalides felis

<400> 503

gatcggttgc aatttgtcat tgcaccaagc ataaattgat gggtccggtg cataggttat 60
aaaagaattg ggatttgtgc atttcatgac ggtgcaatca gcttctgacg atttcttttt 120
acacatattt ttagaatgat catatacata gttaggtggg cattcgaaaa cctgaccttt 180
accatctttg cagaataagt atcttggtgc agtctttcgg atctgggctg atatcctaca 240
accgcgcatg tagtttctgc cgtcttacag ttttcccaaa ggttttgatg accacatatc 300
atctacacaa aatggancat cggaatttga gtctctgcan ttgatngttg taattggggt 360
ttcttgtcaa gcgcacanct tcaanntaga ccantcgtgn caaangttcc caactgctta 420
ttatt 425

<210> 504
<211> 203
<212> DNA
<213> Ctenocephalides felis

<400> 504

gaattgtttg agaattgctc agtgttaatc ctttgagatg tggatcgaag gattgacaaa 60
agtctctgaa ttctgtcagt gtggggccta atgttaagtc ggagtgcgtg caatttaaaa 120
gaagacttag aattgcttgg gttgcacatg cattgttgat cacctgtttt gcaaagaata 180
ttttatcgag tctaccatca tgt 203

<210> 505
<211> 317
<212> DNA
<213> Ctenocephalides felis

<400> 505

attggagaat ttggattttt cttaacacct atgataggca aacaaaaagt agcttttaat 60
tcaggatggg taacatgtgc ttttttgaac ctaagagcca taggtctgat aaatctttcg 120
aattttggag gtttacgtgt aaagccttca ccaacaaaag taacttttgt aaccattctc 180
ttccacgctt tccttctgga ctttcctgat gataaaactt tgaatacttc agcatctgcc 240
tgagctctga cttttggata ngcacatccc attttccggc cttctcttta cgtttctgtt 300
tgatcatatt agagagt 317

<210> 506
<211> 518
<212> DNA
<213> Ctenocephalides felis

<400> 506

```
ctctaanaat cattgacgct ttttcntttc tttcactgcc tttgttttan atggttgtcc 60
tcggttcana aaagacgtaa ngacttctga catttctggc atatcagaca tctttgtgaa 120
actgctgagt tgctccattt ccttttagt ttcaggatca ttcatcattt tcggtaaaan 180
cataattaat aataatggca anaccatcat taatatcata tgattgaata ggaaatcagt 240
gattttccat tgttccctta cttgaaaata tctgaatttt cctaatgtct ttaatcttaa 300
agggtatggc acttgtatga cttgagatgt ttgtaagtga ttcactttgc gtgctcngaa 360
ttttccttta nagttaattt ctactctaac aggctcatac atataanttg ngttaactgc 420
atccaanacg tangatccag atgggacatt anttataaca aangtgccgt ctcnttcaaa 480
aaacctctgt atnanccccc gttcaanant attttggt                         518
```

<210> 507
<211> 373
<212> DNA
<213> Ctenocephalides felis

<400> 507

```
tttttanct aantgtatgt cagaaaatgt cactaaagca acacctgtga tgcttaaaca 60
aactgcaatt aatttggata tagtaaatct atctccgata ttacaaggga ataaagcagc 120
taaaacaagt gtgaataaac ttgacgttga agataaaact gtaaccgtan cagcttctgt 180
ttgtgataat gccagctgaa atgtgtaatt agcagcaaac cacaataagc agaataacaa 240
agcaattttt gctattcttt gtgtgggcaa acgatttgcg gcccgccgtg cagcctcgct 300
tgcccttaaa cttgcgtggg aagataatct tgccaataat gcttctgttg ctcnattatc 360
acgcatttgc cgt                                                    373
```

<210> 508
<211> 430
<212> DNA
<213> Ctenocephalides felis

<400> 508

```
gtattcaatg gtatgaattg cttggtgant gaaaatttca ctcatgggtt cttcttcgtg 60
ctctccatga gctgcttttg gcggtgccnt tggtgcctct ggcataccgt tttcttggct 120
ggagacaacg tctgaagtca tttccattcc ttggttcaaa tcaccatttg atttagcana 180
atgaggaact tcatctttat ttttcttggt tgccatcana tatnatggtt tgcccaanga 240
gcatgagcgg natacagcag agcccaatta acacaaatat tttttgaatt tgctgttgtc 300
cttcanacat aaattcatcn caatgttctg ctggngatcc ttgcttgaat aacatcatgt 360
tnatgaatca gnatcaaaac tgatggtgng cagcatggtt canatctgat gtccgtacta 420
tttgcagagt                                                        430
```

<210> 509
<211> 498
<212> DNA
<213> Ctenocephalides felis

<400> 509

```
acactgtcag taatattgat aaaagtggtg agcaagacgg aaataatgtg cagcctactg 60
acattgcaga tgacttaaac tgtgttgatg tggatagtgt tgacactgaa acttactcga 120
aatgcgacaa aaacattaaa cttattgata aaccactcaa gaagcaaatt gtagttttat 180
cagaaaatga ttttgatgac ggctgtccaa aatcgaatac taataataat aacgacccca 240
aagaaatttc ttctcatatt tgctaccttc aagatagcga ttttaatacc agatcagatt 300
taagtcggat catgactccg aaacatattt caacaccaga aataccaaag tctaacgcaa 360
ataattatgc gactttagat cagagtttcc atttaggtca aaacgttcaa aatgcgcaaa 420
ttaacaaaaa taaatacatt tatatcgatc ctaataaaat tgaacaagat tttaacagca 480
atttaaataa tttaaaca                                                498
```

<210> 510
<211> 500
<212> DNA
<213> Ctenocephalides felis

<400> 510

```
tttttttttt tttttttttt ttttttttgt tttagatggt tgncctcggg tcaaaannga 60
cgtaatgact tctgacattt ctggcatatc agacatcttt gngaagctgc tgagttgctc 120
catttccttt ttagtttcag gatcattcat cattttcggt aaaaccatga ttaataataa 180
tggcaacacc atcattaaaa tcataggatt gaataggaaa tcagnaattt tccattggtc 240
ccttacttga aaatatctga attttcctaa tgnctttaat cttaaagggt atggcacttg 300
tatgacttga gatgtttgta tgtgattcac tttgcgtgct ctgaattttc ctttagagnt 360
aatttctact ctaacaggct catacatata atttgagtta actgcttcca atacgtaaga 420
tccagatgga acattactta taacaaaggn gccgtcttct ttnaaaaacc ctctatataa 480
gcccccgttc accaatattt                                              500
```

<210> 511
<211> 208
<212> DNA
<213> Ctenocephalides felis

<400> 511

```
tttttttttt tttttttttt tcgctttata aacattaata tatttgaaaa atgatattta 60
cataaaatga tttctctcaa tgaaatttaa aaatttctta atcgttttaa aaaaataaat 120
ttatgaatcc catttcttat actccattcc atctggngga cgaacctcaa ccttctcttt 180
gcccacctcg ttccaatttg ngctaagt                                     208
```

<210> 512
<211> 355
<212> DNA
<213> Ctenocephalides felis

<400> 512

tcaatatggt tcaccttaat aacattatct caatatcact ttatgttcta tttaagcagg 60
cctttaccaa atattttagc tctgccactt gtcttattag cagttaacca ttggttgaat 120
agtaaagaaa aatattttat catttattcg gcatctgcaa tattaatatt tcgggctgag 180
ttagctcttc tactgggatt atttctgcta tatgatctta ttcaaggaag agttcagatt 240
ttaagattaa ttaaaatatg cttgccaaca gcagctattt taatcacatt gactgtatta 300
gttgattcat tattctgggg acgactagtg tggccggaag cagaagtttt gtggt 355

<210> 513
<211> 518
<212> DNA
<213> Ctenocephalides felis

<400> 513

aattttggta atactttgaa atatattctt aaattaaatt ctttaacata tacataccac 60
tttattaatg gagttttatt tggcatgcta attcttccat taccttggtg attcattatg 120
tgttagattg gcgcctgtat ttattaggac gaagaaaatt gaacatgcca ttccttctat 180
tattttttat ggcatttggt gtggtattgt cactaccatt tggtgtatct gaagtttctc 240
ttgtgtcaga caaatgcaac aaagtcgcac gacctccatc taaacgacta ggcccccaac 300
ctgaagtgta tgcatattca ccagtatgcc ttcgcagact gtcattagca tgtgatccat 360
ttgctgaaag gctgtttggt tgagacctat gccctgcatc catttcatca tgagaaattg 420
tattccgatg ataatcaggg tttgttgatg ttctaattgg cacaggtgga ggcggtggtc 480
taacgctgac agtgacagta tttgaatact ggggtggt 518

<210> 514
<211> 382
<212> DNA
<213> Ctenocephalides felis

<400> 514

ttccggattt ttggtcaata ctttagctag acgttctgca ggaatgccgt atcttctcaa 60

atttgcatcn cttaaaacta cgacaattgc ctcatcacaa tcttctttag ccagggtatc 120
aatagctata tttgttgcgt ctaatgtatg atccccagac caacaaaatt gagcatgtgc 180
gtgcatcatt tttattgttt ccaatctagc tttttcattt ttaggtggtc ttgaatgatt 240
aacaaattct agagcatctg cttcaccgct gtgaccaaat atatcatact ggaatctatc 300
ttcgtgaccc tcaaaacttt ccatcagaag aactactgct tctaattgct atctaagcgt 360
ccatcatatc cattaaatct gt 382

<210> 515
<211> 489
<212> DNA
<213> Ctenocephalides felis

<400> 515

tcaaattaga ggagggaaac cggcaacagc ttgaagagaa cacaattcct tgntgcctct 60
aaagtgcagt caactttagc tacgttaatg ntagttgaac cgacaacctt tttagctaag 120
tcatcccaag taggtgctaa ccttttacaa tgtccacacc atggtgcaaa aaacttgaca 180
aaagtcacac cctttgaaat gccttgttca aagtttgatc ccacaagatt aaagacacct 240
tcttcttcat tagtgnctgg aatgcganta tcatctctat cttcatcatc aatttgtgaa 300
gcaccatgtt tcttctcaac ataagccttt aattcttcgt gattcctttg ccagagtatt 360
tttccacctt tttccatctt caatccataa taaagtagga taacctttta cttcaaattg 420
tgtgcaaatc gggcggnggt gagtgcagtc aattttagta atgcttacag agtcttcatg 480
ttcaaaagt 489

<210> 516
<211> 309
<212> DNA
<213> Ctenocephalides felis

<400> 516

ttttttttt ttttttttt ttttttttt ttaatgattt aattaattta ttntaagcca 60
ataattgata ttaattatgc attaatcatt gnatttatac tttcctagaa aactatacat 120
cacatgttga aacaaattaa ggttcatggc ttctcgcctt tccttagcgc ttgttttaaa 180
tttgatccat tctttccgaa tcttgttact ntttggtccc catttntcat tgggagtgaa 240
cacgtttttc aatgtatcat taatggaatg ttttctattt ttcaaatata tccaaattgc 300
ccaaagngg 309

<210> 517
<211> 215
<212> DNA
<213> Ctenocephalides felis

<400> 517

atctacaaca gcaccgataa cggcaacaac tttnccttgg gcacctgctg ctgctttggc 60
agcataactc ctgctgttcg ataaaatcga tgcgatttta cccgattctg ttttgctcag 120
ggttcgcaaa gtggaattga ttacggagtg catcttgtaa taaaatgtat agccagttga 180

gttgaattaa attgatctca cctgtaagct actgt 215

<210> 518
<211> 275
<212> DNA
<213> Ctenocephalides felis

<400> 518

agcaaaacgt ttccataact aggatatcct acttgaaagt gatggctatt gttggtactt 60
attgcatttg gtccaaaaat ctcaggctta taatattggc ccacagaaga atatcctggc 120
ctgtactgag aaaatgcgta aggtggtgga tggtaatcac agttcgccca ttggacaatc 180
gagatggcca ccaaaaatat tacagctttc attttagtca cttggtgaaa tatcacgaat 240
tgaaatattt caaccttcgc aaaaggagct tgtga 275

<210> 519
<211> 498

<212> DNA
<213> Ctenocephalides felis

<400> 519

agtattagca tcagcccgga caacaacgca gtaggatgtt agcaacatta ttcaaggatg 60
aaaggtgtca acatctacca gcatattcaa ttttggagaa aatgtattta gatcaaataa 120
ttcgcagatc tgacttgcag gagtttgaag cgcttttaca gcctcatcaa aaggcatcta 180
ctntggatgg gtcatnaatt ctggatcgng ctgngtttgg acataatttg ntttctggca 240
gcaaattgta taataatata acttttgaag aggtaggatc tttanttgga atnttagntn 300
gcaaagnngn naaaattncc gccaatgnta ttaaaagggn gaatgnatgg ggcntnaatn 360
anattgntna cattggcctt gggcgcgaac cccttaggcg aattttngga tattcantac 420
actgggggcg gttgngntnt gttttagggg ccaattcnct tatnggngnn gtttaaatac 480
tngcgggggt tacaannn 498

<210> 520
<211> 498
<212> DNA
<213> Ctenocephalides felis

<400> 520

atattttgta tagtttcttt tgttgaatac ttcatcaata tattaatata atttaggttt 60
cctctcgcaa gttcctgtgt cgcagctttg agtagcttgg ttaaatactg ttccaatcgg 120
acattgatca ggaactggct ctcctttagg acgtaaacac gtgaagaact tccggcatgt 180
tgcgtcgttc ttgaaagcaa attttccacg tgctcggcat tgcccgacac aggtttcgct 240
ccttggggaa aactcggcgg gttcttcgca ttgcaatacg gttcctcttc cggccataca 300
tacgacgtac aatgatttat caccaatgta cggaagcacc ttaggctgtg caggacatgt 360
aactgtatag cattttgtgc ccagagccat aggtccgcaa ttatgacgta acggatcata 420
tgcaaaattt gcggggcaat aatattgggt tcctatacta ttttcgtcac aataataata 480
gctttgacaa tcatttat 498

<210> 521
<211> 500
<212> DNA
<213> Ctenocephalides felis

<400> 521

ataacaatan tttgagacag caaactgaat cctgtatnga ttttctttgt ccttggtgca 60
gtctgaattg cggattatta tattcgctac tgaaacattt gaaactgtgc catgctagat 120
ttacctttac atatgtgcca ttaacacaat gtgctagaat agatgtagct ataaacgagt 180
tgtatgatgg ttcttatgtt ggggcacctc aagatttgct aggcccttct ggatttgcat 240
tttcagggaa tggaccgact cgaagaacta ttgttacaca tatattagtt tgccgaccaa 300
gaagaagtaa acccagttta tcagaattct tggaattgga tgaaaatgaa tgcgacagcc 360
aaagacctta cattacaggt cacaatcgct gtatcatcat acaataacat gcctaccgat 420
tcagctaaag aactagatgt tgattcagaa ngagaaagtg atcctttgtg gttaagnaaa 480
agacaatgtg atgatagacg 500

<210> 522
<211> 312
<212> DNA
<213> Ctenocephalides felis

<400> 522

tngaaaactt ttttaggccc ncaannngag cggncgccgg gcagggacca ggatattcat 60
ccatagcagc tttcaagtat tcttcaagat ctttttcaaa tggtgtattt tcaataatag 120
gcacacacaa ttcttcatca tatcttaaat ttctttttag tgaatagcta taaataccct 180
ttatcatttc taaatgagtg catttaaaaa cttttcagga cattttaatg taactaaaac 240
tgcagcagaa gaatgtgtat gtattacagc acccgcatta tggtttctgt atgcaagcat 300
aaataatggt gt 312

<210> 523
<211> 258
<212> DNA
<213> Ctenocephalides felis

<400> 523

atgaacgtat attatattga gtggcaataa tttaattcat gcaaatcata atgataaatt 60
tgtaattgtt tagtttcaag aaattgtaat attgtaattc atgtaaataa ttgattgcat 120
tccaaaatgt ttttattttt gtttttattt taatagtttt atttaaaatg ttgttgttgt 180
attatatatt cagtatttta aaaaataata aatttactcc cgttgctaaa aaaaaaaaaa 240
aaaaaaaaaa aaaaaaaa 258

<210> 524
<211> 204
<212> DNA
<213> Ctenocephalides felis

<400> 524

cagttaatga tctacaactt gttcaaaaag ccttaactga tggatcgaac gcgtgtggaa 60
gttcaatcga atccttccta aatgttattg aaactgaaga agcacctcca acctttaata 120
gaactaacaa attcactcaa ggtttccaaa acttgataga tgcctacgga gttgctagtt 180
acagagaagt aaatccagct ctgt 204

<210> 525

<211> 500
<212> DNA
<213> Ctenocephalides felis

<400> 525

```
tttttttttt ttttttttaa ttgataaatt ttatttaaaa cttgcaaaaa ttatttaaac 60
aatttacgca tagcacattt cgctcgtgca aatctcttcc tcgcattcca gacaagtttc 120
cttatcgcat ttgtaggtgt cggtgcagcc tctcttcact ttatctccgt caatatctaa 180
gtagcatttg cccatgcatt tggtcttcgt ctcgactttt tcagggtcgt caaggcaatt 240
ttcatcattt tcatcacaag aaacacattc gtgtttttg gcatcaccat tgcattcgtt 300
tttgtcacaa gttgggcaac gttctggttt tttctcgcac attttttca gattctggtt 360
cttgaacagg ttgctggtgc accctaattt tatggcatca ccttgtgcat ttaaaatact 420
caaacaagta tcttgnggct tgagacaaac ttccttggca tcatctttag cgttgcaaac 480
cctgcgattc ttgggaaaac                                              500
```

<210> 526
<211> 259
<212> DNA
<213> Ctenocephalides felis

<400> 526

```
cagcagctcc agcggctgca gcggctccag cagccgcaat tccgccagcg gcgttggctc 60
caccaccggc gccccatgct ccaccacctg ctccgctacc agctcctccc gctccagcgc 120
ctgctccgcc agcacttctc gcagaacggg cgagagtatc tccagccgcc tgttgggaac 180
tgaacgattg agcattactt ccgccatatc ctgaagaacc atatgcggat cctactggag 240
gcacatatgc ggtgttagt                                               259
```

<210> 527
<211> 549
<212> DNA
<213> Ctenocephalides felis

<400> 527

```
tgancgcccg cagaaaaccg ttcgacgcga taatctgtta actggcggtg aattttacgg 60
tcaaaaagat tcaaggtatg gtaatttttc taattgtgaa caaagtctaa gaagtaccaa 120
agttgaacat acaaggcggt cttctaatat ttctcatatt tcgtttggag aaggttcttt 180
tatatcatct acaacttaca ataagcatag gggcccttgc ccagctgctt tattggaaac 240
taataaagca ccatttaaat atacacgtca agtgaaggcg cataaatttt atgtgcctaa 300
agttgtcacg gaaaaatagg aagcctttaa aatatcattc agaatttatt tacttatcaa 360
tgtcttccat tactgtatat acttatatat atatatatat atatatatat atatatatat 420
atatatatat atatatatnn natanaggtn acaaatatnn cgcnnatnng agagngcgcg 480
cgcnnatatt gtntcntacn cnnatatntt gngntatana ganntntata tatntggcac 540
nttatatgc                                                          549
```

<210> 528

<211> 549
<212> DNA
<213> Ctenocephalides felis

<400> 528

```
agataattgt aacccagttt ttgatgaatc attcgaatac ataatttctc aaggagaatt 60
gtcgacgacg caattggaag tgacggtagc tacgcagaaa ggatttttat cgggaggtag 120
tcctgtgatc ggtcaagtga ttttagatct gaacgattac gatttatctc aagcttcaac 180
ccattggctg gatctatgcc cagaatttaa gtcataaatg tggatcattg ctttcgaatt 240
tcaatcaaaa catgcgtatg ctattaaatt aaatgtgtaa ttctaccttt aatttttgct 300
cctacagttt aaatttacgt ttgttatttt taggtataaa tatacgcttt attttgttat 360
tttataaatt atcaatattt aataatgctt ttatattaca aataatactt atgtgttgca 420
caaatttggt atatagatgt atatactgta ctaatattta ttttttcaac ataaatttct 480
tttgcatgtg ccttacattc tagatattca attatcttaa taagtcttaa tcttaaactt 540
attatgcgc                                                         549
```

<210> 529
<211> 441

<212> DNA
<213> Ctenocephalides felis

<400> 529

```
cagttaaata ataaggagaa gatggattac aataaagcac aaccacttca ccagcaatca 60
caatttggtg gaagacctca aactcaacaa ccaggaccac tcagagcgag tgcaatgcaa 120
tcaaaagcac tcctgcaagt tccattctca cctgcaaaca gttgtccaaa ttgtggtgtc 180
ggcttcgtca ctgacaatta ctcctgctgc gcggtctgcc taggagcatg ctgctttcct 240
ctgggacttc tgtgctgctg ggaatgaagg agagatcctg cgtcaactgt ggagcagctt 300
ttaactagga tatagagaaa attatgattt aatgtctttg taactgtcat ttttattact 360
tgattttaaa tattagatca gatgtttcat tttatgaaat acaaaaaata tatattaaga 420
agaaaaaaaa aaaaaaaaaa a                                           441
```

<210> 530
<211> 549
<212> DNA
<213> Ctenocephalides felis

<400> 530

```
gtttggcctt tgtctttcaa aatattaatt ttccaaaaac atataccaaa cgtatttgca 60
caaaatttat tgtacgtttt agaatagtat ataaaattca tgtgtccata aaatattata 120
aataagtaaa tttccaagtt ttcacacaaa ttattgagta agccgaagcc tctttaatgc 180
gccattgcgt tttaattata gtgttttaaa cagcaaaaat cttgattcta catcattaaa 240
atgagcttca acgataaaca gtcgaattca ttcccaaaag atgaatgtgc aaatcgatta 300
gaaggattgc atgtacagag atctgacatg aataaattga taatgaatta tcttgtcaca 360
gaaggcttta aagaggccgc tgaaaagttc caaatagagg ccggtgtagg tacttcgatg 420
gagttaaatt ctttagacga tagaatatta ataagggatg catacagtct ggacgtatca 480
agaagctcag tattagtaat cagcttatnc cgagttgtgg atacgcagna tcttattcat 540
tgcacactc                                                         549
```

<210> 531
<211> 550
<212> DNA
<213> Ctenocephalides felis

<400> 531

```
tattcattta taaataaaga aatatgtctt tgccagagcc acttcaaaag ttgttcagcc 60
acatcgacca gaataagaaa aggtacattg atgtattatc tgaagctgta gcaatcaaat 120
cagtgtcggc atgggcagac agtcgacaag aagttgttaa aatggttaaa tgggctgaac 180
aacgattgaa ggctctcggc gcaaccacag aattagcaga tgttggaaaa caaactcttc 240
cagacggcag agttattgac ttacctccag tattgctggg tcagttggga aatgatccta 300
aaaaacatat ggtatgtttg tatggacatt tagatgttca gccagctctg aaagaagatg 360
gttgggatac tgaaccattt gtattgactg agaaagatgg aaaattattt ggtagaggag 420
ctagtgatga caagggtccg ttatcggtgg attcatgcaa ttgaggctta tcaacagact 480
gacaagattt accagttaac atcaaattgt tttgaaggct ggaggaatct gtagtgaagg 540
atagatgatt                                                        550
```

<210> 532
<211> 550
<212> DNA
<213> Ctenocephalides felis

<400> 532

```
gtcaaacgtg acaatgacat catacataga tgatttttac ctggcagaag ccaaaaaaga 60
aattggaaat gatctataca aagagaaaaa ttatcatgga gccctacaac aatattcaaa 120
agccatcgtc ctatatccag attcttcatc ttactatgga aacagagccg cctgttatat 180
gatgcttttt caatataaaa atgctatgga agatgcaaaa aaagcagttg tgctcgatcc 240
aaattttgct aaagcatatc ttcgcattgc aaaatgcagc atattggtag atcaccggng 300
caaataacta acctgaagng gaatntgcat taaggcaagt ggnttaaatt ggccttgccn 360
cctttanant attnccnttg taaccaagaa nagaaaattt gggggggttnt tttggcntnt 420
tttttggggn aanaccacgt ttccccaaaa ataccccttt ataatttntg tngnggtngn 480
aaaaaanccc ccccccctt ccccggtttt tccccggngt tttttgggnc tcccgaaaaa 540
aaggggggc                                                         550
```

<210> 533
<211> 549

<212> DNA
<213> Ctenocephalides felis

<400> 533

```
gtttcacatt cagtcgatct gtgagtttaa tctgtaaaca tctgtttcat caaaaagttt 60
cagcgaaatt aaaataatgg caaatggaat gccggagttg ggctccaaga taagcctcat 120
atcgaaagca gatatcagat atgagggtcg ccttttcact gttgatcctc atgaatgtac 180
aattgcatta gcaaccgtac gttcatttgg aaccgaagat cgagataccc cgtttccagt 240
tgcaccgcaa acgcaaatat atgattatat tttgttccgc ggatctgata tcaaggacat 300
aagagttgtc aacaatgtca ataatcctgt gcccaatgac cctgcaatta tgcagttatc 360
ggtgcctcca agccttggcc aacctactta tcaacaaccc ggatataccc atccagttct 420
tggagctgta tggggcaatt tggaggtgct tatggaatgg nggttgccac aaagtatgcc 480
gctggatggc aatagaccag tggcaaacaa taaatgagtt gcgcgggagg atggcagcta 540
tcacacaga                                                        549
```

<210> 534
<211> 549
<212> DNA
<213> Ctenocephalides felis

<400> 534

```
ctncgaagtg ctcttactat atttgttata gggnatatat atttaagtat attgaagaat 60
tgaaccgatg gcaagcccag ttgtgcgaaa atcgaacaag gacgaagtaa ataaagaaat 120
tgtcagtatg gatgaagtag ctaaagatgc taaaaatttt attgaacgca ttcttggggg 180
atgtgagcaa aacctcagca actaaacaat tagtcattgg ttcattatca ggatggacaa 240
ctggatttgt tactatgagg attggtaaat tggctgctct tgccgttggc ggtggtattt 300
taatattaca agtagctaac cacaaagggt acatttctat tgactgggat aaagtaacta 360
aaaaggctga taaggtgaca gataaaattg aagaggctgt tactggagaa actcctaaat 420
taatggataa aattgagaga tttgttgata ggaaaattga caaagcagag gaattgctaa 480
agaaaaatca aagaaaagcc aaaaaatgga tcacggcttc aggtgaagaa gaattaaatt 540
gcaagaaat                                                        549
```

<210> 535
<211> 549
<212> DNA
<213> Ctenocephalides felis

<400> 535

```
gcagctctaa cagaagcaaa tgatcagaaa agnagtaaaa aacaaacctt aagcagccta 60
agagcacaaa tgggtttact aattagtcga aaagattcac ctgtagcagt ctcaaagcct 120
cctgtattca gccagagtgg taatgttagc aacacgacac cacaaaccac agttcctgga 180
tctaacatac attcagcaca agttaggagg agctctagac tttttacaaa taattactca 240
```

gttaaagaaa ataataaatc tccaaataga aataaatttg ccaccccaaa atctcctaga 300
aaaccaaagc aacgtttaac aaagactaat ttaggtaaaa caaattacga aataactgaa 360
aaaacagtag ataaagaaaa agtagaaact ataacgtctg atcaaaaagt tttgctgaat 420
aatagtataa actctgacaa acatcagcgc agcaacttta gttttgcaaa acaagtgtga 480
tggactctgt tcttttaaga cagtaggcaa gcttantatg ttaacccaat tcgtgcnaag 540
antgtgtgt 549

<210> 536
<211> 409
<212> DNA
<213> Ctenocephalides felis

<400> 536

cataatcata ctttggatct aaaactaact ctgtgttatt tagaactgta gactcattat 60
aattaatgct ccaaccactg ccgaaaatat ttatcgatat tgagaaaata tcattataaa 120
taaatccggt atacatggag aaaatgccca tcaacaacat aatatagcgt ccagaaaaaa 180
atatgttcca cgtgtcatca gttatgtttt tggctattaa ttttttctcg ttgatcacca 240
aaacagcggc aaatattaaa attataagac catgaccaat gtcgccgaac atgactccga 300
ataaaaatgg gaaggtgatg atggtgtata gtgcagggtt tgcttctcta tagctggcta 360
tgccataaga attgatgaga ttctggaagc cctgagtgaa tttatttgt 409

<210> 537
<211> 166
<212> DNA
<213> Ctenocephalides felis

<400> 537

atagctgatc ctgacatacg ttcagctgtt acgacaggac cagaaacggc gtatacatag 60
ccgaattttc cctctcgctc ttcatccgag attttattaa gttcttgagg cattttggct 120
cctttggtta tttgtcgttt taaaacacac tctgatcctg ttcgaa 166

<210> 538
<211> 135
<212> DNA
<213> Ctenocephalides felis

<400> 538

tgantgcggg tgctctggag gaaccaaaaa cctactcact ttataagggc ttcatacatc 60
tcggcgctgg tttggccgta ggattttccg gcttggcggc gggttttgcg attggaatcg 120
ttggtgatgc tggag 135

<210> 539
<211> 79
<212> DNA

<213> Ctenocephalides felis

<400> 539

```
tgatgattat gaacaagtta aagctgtagc tgaatattat gctgagtatt ctgctttatt 60
cgaaggctct ggcgatggt                                              79
```

<210> 540
<211> 140
<212> DNA
<213> Ctenocephalides felis

<400> 540

```
gtaggggcac aaattccacg aagtttttgt catagaagtc atccaaagca cgcatatatt 60
tactgtatga aatcaaccaa ttgattgaan ggaaatgttt cctttgagct aatttcntat 120
ccaaacccca naacacttgt                                             140
```

<210> 541
<211> 462
<212> DNA
<213> Ctenocephalides felis

<400> 541

```
ttttgatgtc atataaatgc cancaattaa accaantaga ccaatagcag aaccaaagat 60
ttcaacaatg agaattttga caaatagtgc agaattggca gcatcagcaa gagctgctcc 120
agatccaaca ataccaacag cgataccgca gaataaattc acaagaccaa cagccaaacc 180
agctccaaaa agaacataac cacccatcca atttgcttct ttatatcctg ctttttcaaa 240
aacagatttg tctgtgtatt ccgctaacag tcctgacaga acaattgctg taattaaacc 300
ataaatagca acagcttcac agaaaataac tgaaattaag tttttgtttt tgattctggg 360
cgctttcaca ccacctccta caatggatgt gcctgtggta tgaattccaa caagctgctc 420
caacanctga naatgctacg gntaaacaat tcctaaggga gc                   462
```

<210> 542
<211> 396
<212> DNA
<213> Ctenocephalides felis

<400> 542

```
aattggttgg taaagcctcc ttgggcggaa actgacaaga tcacacttga anttgccaag 60
ctcttgaaag acgatttcct acagcagaac agctactcat catatgatcg tttctgcccc 120
ttctataaaa cagttggtat gttacgtaac attattgctt tctatgatat ggcaaaatac 180
gcagttgaat ctactgcaca aagtgaaaac aagattacct ggaataccat tagagatgct 240
atgggcaata ttctatatca gttgtcctca atgaagttca aggacccagt caaggatggt 300
gaggcaaaga tcaagagtga ttttgatcaa ttacatgagg atttacagca agcattccgc 360
aacttggnag attagattat tgtgaatata atatgt                           396
```

<210> 543
<211> 283
<212> DNA
<213> Ctenocephalides felis

<400> 543

```
anacactttg tctttgttaa tttctgtttc tacgtagcgg agctttctct ccatctcatc 60
acaacgcctg acttcgttga cgaatttacg ttgaaatgaa ttaacatcaa cattcaagtc 120
tctaaattgg acggttccag cttctcccaa ttctgaaact gaagtgtaag cagcttcagg 180
ctgaataaac atctggcata aagccatctc ctcacttcga aacatagccc ccatgatgtg 240
gcctattcaa cacccgctcc ttgatacaaa ataagcgggt tgc                   283
```

<210> 544
<211> 346
<212> DNA
<213> Ctenocephalides felis

<400> 544

```
gacattttag aactttcaac aaagatatgt tcaacacaac aacagactat gtaaaaactg 60
tatacatata taatggaata ttttttgtat cataattaaa ttgtaaatct catgatttac 120
taagcgttga cattaacagt attagcagca gctcctttag tttcactggt ttggtccttc 180
tcgagttgct tgccaaggta ttcccaatta tgaaccatta atttctggac tgccaacaaa 240
gcttcatatc ttacatttgg ttcctcatat gccaataatt gcataaccag ttgtttgcca 300
ccaagctgtt cgatgatatt tttgccacgt ggataatgcc tgacgt                346
```

<210> 545
<211> 498
<212> DNA
<213> Ctenocephalides felis

<400> 545

```
atgtaacctg ggaaaccacg acgtccgggt acttcttcac gggcagctga tacctcacgc 60
aaagcttcag catatgagga catgtctgtt aagattacca acacgtgctt ttcacattgg 120
taggccaaga attcagcagc tgtcaaagcc agacgtgggg taatgatacg ttcaatagtt 180
ggatcattgg ccaagttcaa gaacaggcac acattctcca tggaaccatt ctcctcgaaa 240
tcttgtttga agaatctggc agtttccatg ttgacaccca tagcggcgaa cacaatggca 300
aagttatcct catggtcatc caaaactgat tttcctggga ttttaactaa acctgcttgc 360
ctacagatct gggcagcaat ttcattgtgg ggcagaccag ctgcagagaa aataggaatc 420
ttctgtccac gagcaatgga gttcatcaca tcaatagcag agataccagt ttggatattt 480
cctcangata gatacgag                                               498
```

<210> 546
<211> 393
<212> DNA
<213> Ctenocephalides felis

<400> 546

```
tctccaatat caaagcaggc tacagacaga acaaggggat cacgagagct ttcaagtaag 60
nggatcaana tacgcaataa ctcataattc ttctcattga gacgtggggc attctctctc 120
cagaacttag cagatttgtg aacaggtgac cattccaatc tgccggactt gatttctgta 180
gcatattcat caaatgagct aaggtcttga atggaagctt gtaatttttc ggtcaaatat 240
tcaacatcag caacgatatc ctcatcatct gaacgtcttt gttccaggat ggataattgt 300
ttcaatacct tgctttgcac cattgcaatg caatgctcct tggccacctg ttgatcttcc 360
actttctcaa ttaaattcct ataaacagcc agt                             393
```

<210> 547
<211> 649
<212> DNA
<213> Ctenocephalides felis

<400> 547

```
cgagtacaag gaatgttcca gcttatggag cccaaagtat tattgaaggt ccgcaaagct 60
gatttagatt tggtagaatc agttttaact gatgccatgg atcagtataa acaacagatg 120
gttaccaaag aagttgtcgc cactatcaat agggaagcat tttgccagt agaatgctgc 180
ggtggagttg aattgagtgg acttaatggc cgcattaagg tttcaaacac attggaatcc 240
cgtttggact tgattgctca acaattgatt ccagaaatcc gaactgcctt attcggaagc 300
aatgccaacc gtaaattcac agactaaata ttcatatcaa attacatgat taggatgcaa 360
agtgacctag attcgtatta gtaaaaagca tcaagatcaa aatgaatgca caatcagatt 420
cataatgagt gtttttgcat gatacacatt ttttcagaca atagttcata taattgatgc 480
ttccctttgc ttcatagtcc tatttcaaaa atgttaatag atgcacattc cgtagaagtt 540
atatagcatg ctataattga atgatgaata ttacatttga aaattttgaa tacttaattg 600
gnctaaatca taattttctg aaacatgcat tttattcaac acttttgct            649
```

<210> 548
<211> 360
<212> DNA
<213> Ctenocephalides felis

<400> 548

```
tgctcttgtc gcaaacacat ctaacatgcc tgtcgccgct cgtgaagctt ccatttacac 60
cggtatcacc ctcagtgaat acttccgtga tatgggttac aacgtatcta tgatggctga 120
tttcacttct cgttgggctg aagctttgag agaaatttca ggtcgtttgg ctgaaatgcc 180
tgccgattcc ggttatccag cgtacttggg agctaggttg gcctctttct acgaacgtgc 240
tggtcgtgta aaatgtttgg gtaatcccga acgtgaaggc tcagtatcaa ttgtaggagc 300
tgtatcaccg cccggtggtg acttctcaga tcccgtcact tcagctacat tgggtattgt 360
```

<210> 549
<211> 357
<212> DNA
<213> Ctenocephalides felis

<400> 549

ggattcattc gatcagcttc atcatcatat acatatatat gtttttgng ngngatttaa 60
taattggctt aaattttaca tcttgnttgt tttaaacttt gttgncaacc atatcgacgt 120
tcagtttata gtagacatat ggatagtaat caagcttggc caaaacccan accaggaata 180
tcaacgttct cagaagcacc ggagctttgt agagcactgc tgtcaagtgt ccgtacaatt 240
gattgagcac atctcgtaat ctcttgatgc tcttcttaga tggttgaagg caacttcagt 300
gaatgcttct gtgcatggat ccatgttaag gcgctacgag ttttcgttga gtcaagt 357

<210> 550
<211> 500
<212> DNA
<213> Ctenocephalides felis

<400> 550

cactnatttg gcccaattta tgaacgtgct ngacgtgttg aaggcagaaa tggttctatc 60
acccaaattc caattttgac tatgcccaac gatgatatta ctcaccctat tcctgatttg 120
actggttaca tcactgaagg acagatctac gtagacagac aactgcacaa caggcaaatc 180
tacccacctg taaacgtatt gncttccctg tcacgtttga tgaaatntgc cattggtgag 240
ggtttgacac gnagaganca ctctgatgtg tccaatcaat tgtatgcttg ctncgccntt 300
ggtaaggacg tgcaggctat gaaggctgtc gtangagagg aagctttgac acctgatgac 360
ttgttgnact tggaattctt gcgaaatttg agaagaactt tatctcacan ggtanttatg 420
agaaccgcac agantttgaa tctttggaca ttngctggca antggtggga tcttcccnag 480
gagatgttga agagaatacc 500

<210> 551
<211> 116
<212> DNA
<213> Ctenocephalides felis

<400> 551

ccatgtcagt ggccaaagtt ggttgataac ctacagcgga tggaatacga cccaacagag 60
cagatacttc agaaccggct tgagtgaaac ggaaaatgtt gtcaatgaaa agaagt 116

<210> 552
<211> 294
<212> DNA
<213> Ctenocephalides felis

<400> 552

caaaggctat caaaaaatgt cttttctaac aagtgtttac atatttgctt tgtatcgaat 60
actatttaaa aaatatttat tatacatgca tcaatgatac atattttaaa tttacaatac 120
ttaatacttt tagtaaggtc actatgattt gttgaataat ttaaataatt caaaatccat 180
tacaaataat aaatactgct aaacaattag caacatactg tatggataat acataaaatt 240

actcggagtt caaaaaaaaa aatnaaaaaa aaaaacaaaa aaataatcat gagt 294

<210> 553
<211> 436
<212> DNA
<213> Ctenocephalides felis

<400> 553

```
tttgcgtctn tgggccctgt cacttgctca cgcacaactt tcanacgtat tgnggaaaat 60
ggtgntgncc atgggattga gccaaacaac atatactggc tcgattatgc tctatgtgat 120
attctgcgng ngggcactat tcacaattgc catcctagtt atgatggaag gcctttctgc 180
gtcttgcaca cactgcgtct tcactgggtg gagttcatga gtaagttcta ctctggtttg 240
ggttatttgn tccaaccctt ctgcttcaaa actattttgg acgcggngga taaggnagca 300
gaataatcaa tttatctatt atttaaataa attaaaaaac aaattagtta taaagaaggt 360
ataaaaaagn aaaatattgg ntaaaaattg nttttaaaag nctgnaagng atttggaata 420
acacttaatt tgtagg                                                 436
```

<210> 554
<211> 223
<212> DNA
<213> Ctenocephalides felis

<400> 554

```
aataagtgtt gttaaaactt ctttgtattg ttccttatta cgtgtaactt ctcccaacct 60
tttgcgggct tcatccaaaa cattcctcac atgatcttca cgaactttca ataccttcaa 120
tcgagcttgg ttgagcatgt tggatgattg aattttcttt tgcaattcaa cttgtttttc 180
cttcttctca tagtattcca taatctttag tcgctgttgc tgt                   223
```

<210> 555
<211> 418
<212> DNA
<213> Ctenocephalides felis

<400> 555

```
aattcggtaa tactaggaat gtanaacana aatataatag aacaataatt cgatttcaac 60
aaagatattg tattttaatt ttaatttata ttgatcacaa attaataact cgttacattg 120
taataatact aaacaatcta tttacaatta aaaacactcc tttcgcaaac tttaattctc 180
cttcttgatg cgtgtggatg tcattctgta aataatggag tccctgccag ggtccattgt 240
ggagatcgag atacagatag caagtaacga gaagaaaaag gctactccga accataaaat 300
gatgttaaaa accaccgggt aggagtcgtt gtattcttta gctaggttga aatcgggttc 360
ttttggagca tcatctgctt gccttgctta cgtgttaaga cgtcattaga gggttagt   418
```

<210> 556
<211> 289
<212> DNA
<213> Ctenocephalides felis

<400> 556

cgtagcccag accagtaact gagaaattac ccgcaaacca cccattgttn actgggcaac 60
gtgtnttgga ttctctgttc ccatgtgttc agggaggaac cactgccatc cctggagctt 120
tcggttgtgg aaaaactgtn atttcacaag ctttgccaaa tactctaact ctgatgtcat 180
catttatgtt ggttgcggag aaagaggtaa tgaaatgtct gaagtacttc gtgatttccc 240
tgaattgagt gttgaaatcg acggcgtaac tgaatctatt atgaagcgt 289

<210> 557
<211> 549
<212> DNA
<213> Ctenocephalides felis

<400> 557

caaatccttt gacattagat aaacgtagta ctatttaagc acaaaaaggt ataatataat 60
aaaatggcta gccagactca gggaattcaa caacttcttg ccgctgagaa aagggctgct 120
gaaaaagtct ccgaggccag gaaacgcaaa gcacgcagac taaagcaagc taaggaagaa 180
gctcaagatg aaattgagaa atatcgtcag gaacgtgaga agcaattcaa agaatttgaa 240
gcaaagcata tgggctcacg ggaaggagtt gcggctagaa ttgatgctga cactcgtgtc 300
aaaatcgatc agatgaacaa agctgtatct gttcaaaagg atcccgtgat gtatgaaatt 360
ctgaagttgg tctatgacat caaaccagaa ttacacaaaa attatcgcaa agaataattt 420
attttattca gagctccagt gaaaaaataa tatatttaat aaagattgtt tatatccaca 480
tttttgctta tgtgaaaaaa tcttaataga tcaatctgat tttagaaatc tagaatttta 540
antagggtc 549

<210> 558
<211> 550
<212> DNA
<213> Ctenocephalides felis

<400> 558

ttttgattgt tattacttta ttttaacaaa ataccgtgaa aatcatttgg tataattaat 60
aaagaaccac atcaaaatga cagagtattg gctgatatct gcaccaggtg acaagacctg 120
ccagcagaca tgggagacga tgaacaatgt caccagcaaa caaaattcat tatctgtcaa 180
ctacaaattt catataccag accttaaggt cggtacatta gatcagctcg taggtctgtc 240
tgatgatcta ggcaagctcg atgtattcgt tgagcagatc actcgtaagg tagcaacata 300
tcttggtgag gttcttgagg accaacgtga taaacttcac gagaatttaa tggcaaacaa 360
cagcgatctg catcttacat aactcgcttc cagtgggaca tggcaagtac ccaatcaagc 420
aatctcttcg taacatctca gatatcatca gcaagcaagt aggacagatc gatgccgctt 480
gaaaaccaaa tctcagcgta cacaattgaa aggagtttgc agacttggag aagaacaaac 540
tggaanttgt 550

<210> 559
<211> 371
<212> DNA
<213> Ctenocephalides felis

<400> 559

tggtattggg agaattaatt tgtgttttta aaaaagttcg tctttttga ataactgaat 60
gaatgagatt ttttgggtaa caattttcaa ttagagttat ttttactagg gataaatttt 120
gtggatggaa agtttggtga gacaacaaaa tagcatggtc tgtaagggaa ataatagtgt 180
ttatcttata gtttctggga cagtatgagg aatagtgtaa gtatctaccc gaccatgtag 240
tttttctata ccaatttgtt cgaataaagt tattagtgtt gtaaacggat acatccaaga 300
aattaagcct gttgttattt tgtagttcat gtgtgaaaat tatattattg ttaaaattat 360
tgaagagatc t 371

<210> 560
<211> 228
<212> DNA
<213> Ctenocephalides felis

<400> 560

ctcgtaaatc acttggtcac cagtttgatt tgtggtagtc tcattttcta taggnataaa 60
catcagntgg ttcattgaaa gggttgtttg ggtgcttgtc ggcttcgtcg aagggattgg 120
tgcttgtttc gggttctggt tcgtcgaaag gatttgctgg aacgtcttgc ttggcgtaac 180
tggtgtcccc gtagacttgt ttcgaagatt caccaattgt tttgacgt 228

<210> 561
<211> 269
<212> DNA
<213> Ctenocephalides felis

<400> 561

agatctacta atgccgcgtt taccactgtc acagcaattc cgcaaaccag caaccacana 60
tataantatg atgctattgt taaatgntca ataaatgcac aaattattcc acaaatnccg 120
catgagacca tcacgaatac aagaattgga agctttccca cagagttaat aatagcacct 180
attatgggaa atccaatcgc atatccagct tccaacatta aagaatgcat aaatgcctgt 240
tcttccattg tgtctttaca ctcggtcgt 269

<210> 562
<211> 500
<212> DNA
<213> Ctenocephalides felis

<400> 562

aaaaatccaa cggatgtttc gtcatacaga cctatcagtt tgcttcctat aatttccaaa 60
atactggaaa anataatata caaaaaaatt aatcatgatt taccgacaga catctggatg 120
ccgtcacatc agttcgggtt caggcataag cactcaacga ctcaacagat ccataggatt 180

gttgaaaata ttaaaaaaac atttgaacaa aaggaatatt gcagtggcat tttcttggac 240
gttcgtcagg cctttgataa ggtctggcat cctgggctgt tatataaaat taaaaaacac 300
tttccagtta aatacttcaa gttattaaaa tcttatttag aaaacagatc tttccagatt 360
agagtgaacg ctgaactgtc agatttctgt aaaatagggt caggtgtccc gcagggcagc 420
atcctgggac cattattata tgnattattt accttagata tccaatacct tctaactcta 480
ttatagctac attgcagacg 500

<210> 563
<211> 270
<212> DNA
<213> Ctenocephalides felis

<400> 563

anatctacta atgccgcgtt taccactgtc acagcaattc cgcaaaccag caaccacggg 60
atataaatat gatgctattg ttaaatgttc aataaatgca caaattattc cacaaatccc 120
gcatgagacc atcacgaata caagaattgg aagctttccc acagagttaa taatagcacc 180
tattatggga aatccaatcg catatccagc ttccaacatt aaagaatgca taaatgcctg 240
ttcttccatt gtgtctttac actcggtcgt 270

<210> 564

<211> 210
<212> DNA
<213> Ctenocephalides felis

<400> 564

ttttnaagtg caacccaatc attagggaac cgctaacaga agttatnaca gtgacgttct 60
acatcggaga atggttagaa ttcaaaaaat aatcatgcaa cacggnctca ttattataac 120
attcacataa aaaatattta aaatatgtaa actagcttgc atgtttattg taagccagtc 180
tataatctat tattgnttgc actttactta 210

<210> 565
<211> 425
<212> DNA
<213> Ctenocephalides felis

<400> 565

ataagtcatt tgaaaataag catttctgta gtggtgtctt cctggatgtt cgacaagcat 60
tcgataaggt ttggcaccca ggacttttat ataaaataaa agcatatctt ccttcaagga 120
tttttcaagt aaaagtaaat caagtaacat ctgatttcca caaaataatg tcaggagttc 180
cacaaggtag tatttttgga cctttcttat atgttttata cacaaaggac ctcccattac 240
ttgaaaatct gacacttgct acattcgctg acgatattgc catactaagt agtaatcaca 300
gtgctgatca agcttcccga caaacgcaag aacatatcaa taaactacaa atgtggctta 360
ctaaatggaa gatttgcata aatgaaacca aatcagttca tatcacattt accctgagag 420
agggt 425

<210> 566

<211> 328
<212> DNA
<213> Ctenocephalides felis

<400> 566

```
gaaggatant ttatgtaaat taatggttga tattantttg gcnttttcaa gaaataatta 60
tgtggnggca ctcttttgcg atattaaagg ggcatatgat aatgtcgttc caagtataat 120
gttccaagaa ttaattaaaa taggattacc attccgattg gtgtcagcct taacatttaa 180
tatatatgaa aggaatataa tagtgaagaa agataacaaa atanttggtg aaagagtagt 240
gaataaagga ctaccacaag gaggtatact tagcccatta ttgtatgcta tatatgtaag 300
agatatagat aatatttggg taagaggt                                    328
```

<210> 567
<211> 284
<212> DNA
<213> Ctenocephalides felis

<400> 567

```
tgaaaacgaa ataattgtta aaatgtctga catagaagaa cattatgcca taaaattgtt 60
aacaaacaat ttcaatcgag aaccttcaat agttacaaaa tgggctatac ctgtatcgtt 120
cgcaggtttg gcttttgttg gaacttgtgt ggctaatatg ctgactaaaa aaccaatgat 180
gtcaggcatt cagaaacaca tccttctgac atctagtttt ggtggagttg gttatatcgc 240
agataaatat cgtaatgaat attatgcaga aagagatgct atgt                  284
```

<210> 568
<211> 500
<212> DNA
<213> Ctenocephalides felis

<400> 568

```
accaatgtat aaccgttaga ctgcaacgcg gcgtttacgc gctttgatat ttcatctcta 60
agcagctaac acaacgtaat tgctttgaaa aaagcatttc naaattttca acaaaatatt 120
tcaaattaaa aaagtcaatt tctctcactt ttcaaanttt tgnaaagctt tttnnnaaat 180
tannctangg gnccnnaagt nntnacaaat tngnccgnaa agcnagaagn cctgcacccn 240
caanggcact gncnccggta aaaanngggg ggctcncana ataattncnn tnnnccccca 300
gcntnggnca ccnacgnnnc nngttnnncn ntanntntcn ctccnncnac nnnntntcgt 360
ncnnacnacg cnntanncnc cncntcncnn nncccntnca ccnnntnncg cncgaccatn 420
nctntnnnaa ngcccnatnn cccctntagn nngccgtntc tnacttgntn nncnncnttt 480
cnntgancgt ntctcggnan                                              500
```

<210> 569
<211> 358
<212> DNA
<213> Ctenocephalides felis

<400> 569

acaattgtgt agtgcagtgg ctaactgtat tagatgatat agtcaattta aaattcagga 60
actaaactta aattagtatt acgttgctgt cttcacatta tgttctatgc tcatcagttt 120
taagttaaag ttggccacca ctcatatata gcaaatagat aatgactatt gcgaaagaag 180
tctagactat aaaaatacta atttatttat ttatgtagta tatttttagt attgtgagtg 240
aaagacgttt aaaatacatc taattcaaaa tagtcaaaat gattctaacc acattttgta 300
tgttttaagt atgcaagcat gtttgcttga gatttgttta agaacctaat tgtttagt 358

<210> 570
<211> 368

<212> DNA
<213> Ctenocephalides felis

<400> 570

acgtaatagt ctacttctac taggtgttca ctcgtcaaaa tcacgaattg aactggcgga 60
ttaggaattg ccaaatattt tatattttac tcgtaacaaa tattttttaa attgcaataa 120
aaaaaatctt gtaatttagg ctgtaaaact tcattgntta aatatagaat atgtaatatg 180
atnagttgan gttggnatat ctataaagtt ttttttngtt tnatgttnnc aannatcaat 240
caangttngc nttttnctga ngtatttgaa nattttgata caantattaa aatattttga 300
tttttagttt ttttttgcat atatngctga tcaaaatant ataatttttt aanacantat 360
gctggttg 368

<210> 571
<211> 255
<212> DNA
<213> Ctenocephalides felis

<400> 571

acaatgtttt acatcattaa cattaatttg cattcatcac aatgaacaaa actgatcttt 60
cgttcaatca ataacgtaat ctagaacaat tgtaatgaat tacttttatt attataagaa 120
tccaaacatg tattattcct taaccatttt ctaaagatct atggcatatt taaacatatt 180
aatatatttt gatactattt attttcatta aaataagcaa aattggcata ttaaaacaat 240
ttaaatagtt ttagt 255

<210> 572
<211> 500
<212> DNA
<213> Ctenocephalides felis

<400> 572

accactgtat ttcttgaagt caaaattaat atactcagaa atatcataaa aactgttggc 60
ttatttagta aattaatcaa aacgttgtgg aatttatgat tgctttgttg tcgtatttgt 120
aaaggtaata aattgaacag ttgaataccc aaaccatcaa ttgaattaaa agtagtatta 180

```
agcttaaaag tcgatcctct gatagttcta ttccttgtga catagtagag actagacatg 240
gaagataaat taaaatactg gggattattt tttataaaga gaagtgtaat aagtaggaat 300
atgttaggaa cggtaagaat attatacttc tgaaaaatat ttctacaact tgtcatagag 360
ttaagagaaa acatgattct tagtgctctt ttctgaagct tgaaaacata taatgaaaat 420
atcttctata atatgtatga aaatcaaatc atattttgnt atattcatca tcaaactttc 480
tcatattttg cttctttttc                                             500
```

<210> 573
<211> 341
<212> DNA
<213> Ctenocephalides felis

<400> 573

```
actgtttaat tgtatatgta acttttactc gttactctta tcacagagta atcaaattga 60
caagatgacg cgttcttaat taataaatga tttggacttc tattatttat tataagttgg 120
taaatccttg aaggcaacac agcaatttga tgatatcggc ctccgcctat ttttcctcaa 180
atgaataaca caaaaatcct atcgataatt aactaacaat agcacggact tataatacta 240
aaacgaatta tgatttagtt cacattatcc ttacggataa atgaagtgtt tgttacaact 300
tacaataaat gttgtaaaga gttgataaag ttacaaattg t                     341
```

<210> 574
<211> 359
<212> DNA
<213> Ctenocephalides felis

<400> 574

```
acatatacat atttatgatc cttctattag tagattatat aattaggttt ttgtctttga 60
actgattatc tgcaaataac gtaaattaga aaacgcacaa gagaaaatgt ttatatgatt 120
ccgatattta ctcactattt atttttcaca aagcatagaa ttagttttta tatgatgctt 180
gatcaataat ggtgtttgaa attttaaaat taatatacga agctatttaa tatcacgcaa 240
agaaactttg atgtttataa gacacctatg tttacacata catacacata tattgtaaat 300
attaatactt ctttataaaa tccatgcata tttgctttgt catattctta ttattctgt  359
```

<210> 575
<211> 353
<212> DNA
<213> Ctenocephalides felis

<400> 575

```
acataatata taagttcgtt ttatattaat tgaaatatat aaaatattcg aatttaaaat 60
taatgaataa ttaaaatgtt cgtttaaatt gatttaagcg taatggattt agtggatttg 120
tttgattttg atgctctact ctgtagtcgc ggctggttcg gtcttttcgg tcttttcgat 180
cttattggtc ttatctgtat tatcggtctt atccaccacc gattcgagct caagggcttt 240
gagttgaagt ggcctttttt ggatgtaata agataataaa tttacatcat tacggatttt 300
tccactttct gggtagtaag atttcctctt gttgtaggcc ttgtcgcctg cgt        353
```

<210> 576
<211> 399
<212> DNA
<213> Ctenocephalides felis

<400> 576

```
acacttgacc caatactgat gtcgtcaaat attcntattg taaagtcata tataaatcgt 60
caaacagttt ttgagttgtt tcaagtattt tgttttgata gtctagttca aaagatttta 120
aacacatcgc ccagcaaaat atattgcgtt ttcttcgtaa aaatcaaatt tcgtacactt 180
gactcaatac cacacacaaa tcattttatt tttctgtctt cgaaaacaga tattaatagg 240
caaatgttat gtttcagtaa tctctagtat ttttttttcat tgacatgttt atatacagat 300
tttaaatata ttgtgttttt tttacaatcc caacgtctct gttatgtctc tcagcactcg 360
acaatttttg aacctatcga cggggtgtcg tagtgccgt                        399
```

<210> 577
<211> 1000
<212> DNA
<213> Ctenocephalides felis

<400> 577

```
accgtaatta aatattggaa tgattttaat tgatttcaaa tagattatta tgaatattgt 60
aactttcatt attcatattt agtagatgaa atgtaagtag cttataaatt gaacgacggt 120
ctttatacat gtttatatga aaagtaaata aacaaacctc atagaaattt gataaactga 180
attgcaaagc tcaaattatt tatttcaagt atataagcga gctttagaat tttcgataga 240
attaaaatta aaaaatctga tgattttcaa tattaaaaan aaagaaaatt aaaaagaaag 300
tggaattttg agatgaaaaa aacaatttat ttctcaaaaa actaatcgat tctaagcatt 360
gtcaatgcaa gcaatatgtn tttttaaatc attggaattt agatgctgcg ttttcacaaa 420
aactagattt tagcactttt tgctcttcat ctaccaaacg ggtggacctg cctatgcaaa 480
aatctaacaa aaacgtctcg accgtaatta aatattggaa tgattttaat tgatttcaaa 540
tagattatta tgaatattgt aactttcatt attcatattt agtagatgaa atgtaagtag 600
cttataaatt gaacgacggt ctttatacat gtttatatga aaagtaaata aacaaacctc 660
atagaaattt gataaactga attgcaaagc tcaaattatt tatttcaagt atataagcga 720
gctttagaat tttcgataga attaaaatta aaaaatctga tgattttcaa tattaaaaan 780
aaagaaaatt aaaaagaaag tggaattttg agatgaaaaa aacaatttat ttctcaaaaa 840
actaatcgat tctaagcatt gtcaatgcaa gcaatatgtn tttttaaatc attggaattt 900
agatgctgcg ttttcacaaa aactagattt tagcactttt tgctcttcat ctaccaaacg 960
ggtggacctg cctatgcaaa aatctaacaa aaacgtctcg                       1000
```

<210> 578
<211> 500
<212> DNA
<213> Ctenocephalides felis

<400> 578

```
acattgagca ttcttgactt ttcgcattta aacagctgta ncttttaaat ttaattatta 60
ccaaaaaata ntattaacac ttttgttaag cgataaacaa caaatccatt aatccgtata 120
ggaatttatt gagacttttc attgaggagc tacatccgtt taactaaaaa tttgctcaac 180
tagctccgcc ctaccctact taactttata tntatccctt tggaatatat tgacaagacg 240
catttatntg ttattattta tgnnttaanc tttataacta aatactatga aataattcca 300
tatacattca aatcatttta tttagtttca gtaattatat ttgcatataa tataangatg 360
tattnnttta cttatttana aattntaang taaattttag tattnaataa atantgggag 420
gaaatctagg aactaggatg aattttgcaa ctcataaata tgataaacat ttatgaattn 480
tgcttatatt ctaatttgaa                                             500
```

<210> 579
<211> 500
<212> DNA
<213> Ctenocephalides felis

<400> 579

```
acaccgtaaa gctttcgctt ttcggtccga gatacgagac ctcgtttcaa aattgaaata 60
tgaatatttc attgtgtgtg agattgaaaa atctgctttc gctgatcttt cattttctta 120
aaacaacgca atgaagcgaa tgttcaaatt cgcacaattt gaataaaatg tgcagagtat 180
atagaatact aacgcaagcg tttactacaa tcgactttga aacactagac tttcatctag 240
cgcccaaaga attgattgag ctatgcgttt aaagtataat attcaaaaag aacgccgttt 300
tcacaacgaa cttcgtcatt cgctcaggca tctggcgata ccgagcagat atacatggca 360
gtgctctcac acaatgcata agagcgctaa gcggttgtaa accgcgtcac gctgcttttg 420
cacactgcgc aatattttgc gactagctcc ggtcaaacgt taatgaaacg aatgatccat 480
cgttgcgaga cgctttaaga                                             500
```

<210> 580
<211> 277
<212> DNA
<213> Ctenocephalides felis

<400> 580

```
acgagagcga catgttcggg aacccgtgcg ttatacctgc tcgagtcctt tggttttgag 60
acgtcccatt gcatattcat aggatattca taggatatcg cgatattagg agattgtgag 120
gacaagtggg acattccctg gcatgtcttg tgctgtctgg gaaggctgtc aaatgttatt 180
tcttcacaga ttgaatcaat aaccgaaact atgacacacc aaaatactaa ccgaaagcct 240
gcaaataatt ttatcgccaa ttcagataaa tatttgt                          277
```

<210> 581
<211> 500
<212> DNA
<213> Ctenocephalides felis

<400> 581

```
acacaaaatt agaaaaattt aattaaggat ctgcgataaa aaaaaaacaa tttttgacac 60
```

atgaaacaat tttctaaatc atttttctat ttcatgttca agagaacaga cggacagacg 120
aacagataga taaacaggtg gacacttaac acgttaatcg ccatgataat atcagaaaaa 180
aatttacatg cactatggta actttattag ttgaaaatgt atcgaaaact ccatgtagca 240
aagaaaaaaa gatattttga aaaattcctg cacattattt ttttatgcca catatatatg 300
actgatatat atgtcagaca taatttataa actagtcggn ttggtattta gccttgaggc 360
aagttacatg aacttcccga tatgtttaaa taatcaactt aatcaataaa aaataaatta 420
atttatgagt cattaaaagt attccgagtg gatttattat acataattga aaacgaacga 480
acactttgac tcngagtatt 500

<210> 582
<211> 469
<212> DNA
<213> Ctenocephalides felis

<400> 582

acaacgggga tttgattcca accacaacac caacgcaaac atttttgttc taggcatttt 60
tatacaaatt gaaatagcac aatttaaacc aaaacgaaac agaacaagta aaccgcgcaa 120
attatcaaat cactctaccg acgccgacgt cattttgcta ttctcgggat tttagtgtgt 180
cacaaaaacc agatgttgat aacaggttga ctgccgaccg tagtgttgtg ccattagtgt 240
ggaaatttat aaatatacat atgattcaga atatgagtaa cacttggtta tatttataac 300
gaagtttatt cacagaaaaa tatagaattg aatttagaaa ataacttttg tatacgaagt 360
gtaagttacg gtggttcaaa gtagagagag ttttgccggt ttctcctttc caaacccggc 420
gtcctgggtt gccagagcaa tcatgtttgt tcttccgaag cctcgccgt 469

<210> 583
<211> 500
<212> DNA
<213> Ctenocephalides felis

<400> 583

actgactgna tatccattgc gcatttngta taatngaatn ttnnnagcaa taaactttga 60
nggattgagg ggtatntcna atttggncat tgattcccat tactatgtga tcagtcattt 120
cgtaaattnt attttcctna ttnttcataa ccactatact gtgcgcgtnt gtnatgancg 180
nggcnatnat aacgaatttg ataaaacgna tncctaataa anatttgatt ttgtacacct 240
aggtcgngan cangcnaaga cnaataatgc agatgnncat cacagngggg ganngannaa 300
angtggatgt aganggtca tntttggcct atagngagtc gtattacaat tcantggcng 360
tnagtttaac aacgtcgtga ctgggaaaac cntggngata cccaactgaa ngacttgnag 420
cacatncacc tttnnggnag gtngggtaat ntncgaagaa ggnntgnacn gatnggcctn 480
ccaacagttg ngcannctga 500

<210> 584
<211> 500
<212> DNA
<213> Ctenocephalides felis

<400> 584

```
actgccttta atagaaacca tagaaagttg agtaatggaa aaatatatgg tggtcctatt 60
tttcgaatag atgaatgctt ctcagtatat acaataaaag tctcttgggc agtagaagca 120
ttatttcgac aaagatattg taaaactgct tgtgttatga acatatcaat ttctccatta 180
aatcctttct ctgtatgcaa ttcaactaac atagaagcaa aaccggaccc atccatggag 240
aacataaaat gatatcttgc ttgagcataa tttttttctt gccaatatgc ttgtgccaat 300
ttctggaatt atgtttatat ttaaatatgc aacaaacttt tcaaaccttc ctctgtccta 360
tatctaatta ttagcaatcc ttccataaac ctacatactc actttatgca attcaggatg 420
tccaagttta ttatcaccat ccttgaccat gttaaggaat ttgataaaaa tgtctctctc 480
tctggcacat agggcccaat                                             500
```

<210> 585
<211> 445
<212> DNA
<213> Ctenocephalides felis

<400> 585

```
acatatttac gcgcaacata gcggcactac gcgcaatatn caagccgnca gattaaaact 60
gtgattaaac tcataataaa actcgatggc acgatcattt tatgaaacat taaatatcag 120
ttattaaatg aacacttaaa cgggaatttc ataatgtaga taatatgaca aaatcgattt 180
tattttgctt ctgtttaaat tgaatatgcc cggtaattga caggttttaa ctgataaacg 240
gaatagctnc tgatcattaa tagccaaaat tgtgctaccg taatattatg attcagcttc 300
taatggatna ataataaatt atttcgaatg gtttcagtgg gcggcgtgta aataatatag 360
ttcatcactt gcaatgttag atttatgaga aaatgcattg ctttgctcgt taattaaaga 420
tttcatttaa atttatatat gtggg                                       445
```

<210> 586
<211> 500
<212> DNA
<213> Ctenocephalides felis

<400> 586

```
acaagaaaag agggcgatat agctcaatgt gttaaacgcg ccatcgaggg tctccgacca 60
atattcctcg aaggagacaa agctcgaaat ataccatcat tggaacccct cgaggtggga 120
gatttgctgg tgggcgacta cggtcgtagc aacggaggtc tacggatttc tgctacggaa 180
atcatggcta agggcgcctc caatttccgt attgataaaa taagcggaga tcttgataaa 240
ctagaatttt actttgaagt attactacct cgtcttgaga ctacaggaaa atatgctgta 300
natggaaatg tattattatt acctattaag ggtngtggac catttactgg aaattncact 360
gatagtgtag gaaaagcttg taatgcangg agaaaattgg tcgaaaagat ggccaaaaca 420
tattaaattc acngatttaa atatcangat caaagttgga aaaggtcgct tgcttttgaa 480
gaattatcng tggtgataaa                                             500
```

<210> 587
<211> 193
<212> DNA
<213> Ctenocephalides felis

<400> 587

actgaagttt gttgngcgta aagatcactg atgtgggana agaagncgac aaaacanagn 60
ggtaaacttt cgacaaggaa tacattacca acaaaattat aaaagtcatg aataaattaa 120
tatgnaaata ttatgataag gattatatac ttttganttc tttttgtaaa taaagattac 180
ggatttaaat tct 193

<210> 588
<211> 399
<212> DNA
<213> Ctenocephalides felis

<400> 588

accattttat atgagagacg gtatcctagc tttgccatat cattggtcta ttttaggaca 60
gaactggtcc ctatatatgg caatatccac caacattttt atgcaatatc taacaaacaa 120
tatactgtat ttttaccatt tatttaccaa cgagcaaagt tctctcgaat ctgatctcct 180
cgactagcga caactaagtt attagtctta actgaataat aacacgttgc ctgatatgat 240
aatattataa atatatacta gctcccagtt aagcgcttat atctgtcgct ttacaacact 300
ttacatatta tgcaaatcag gttcttcttt ctattgaagt tccagttgcc tgtaataagt 360
gcatccctat aatttcgatt gtctcaaaga tctcactgt 399

<210> 589
<211> 238
<212> DNA
<213> Ctenocephalides felis

<400> 589

aaacaattcc gagattaacg gggctcgacc cggcgaaatn ggtgcttgcg tatcgagcgc 60
aataaaaaca ttatataaca caaacaatgc agattattcg gttaacgaaa ttataagtga 120
aaaaaagtca ttaggaaaca caaaaattaa acataaaatc aaacctagca ttagcaaaaa 180
tgccgaaaaa aatattaaaa aaaatactga cattattcca gaaatgttaa aatctggt 238

<210> 590
<211> 500
<212> DNA
<213> Ctenocephalides felis

<400> 590

actcggtccg anacgctana tgcgaagagc gaacgaaatt tcttactatg agcggttgca 60
angctagttg natatttatt ataaaactat aataaaatta taantcnatc ctttaaaaat 120
attttngata cgtaaatccg tatatattac ataaatatat ggtatnttat naaagacgan 180
atgttttacg tnancatttn tgggtaacaa actattantt ttaatactat nttaccattt 240
atatatttng ctatcttagt atttattgcg tttngatant tcatatnant ntaatttnca 300
aatacaatgt gaaaaataag ctattacatt cttacangca attgaacgta gcttatanna 360

tcatatcatn atttatgtaa cnnactatta tgttgtnact aatttancac catanataat 420
annnnnngna taatgttnaa nacantcgaa ttgtcnangc nanantctag attgnatata 480
agtatgattn tntgtgatat 500

<210> 591
<211> 427
<212> DNA
<213> Ctenocephalides felis

<400> 591

acatatcggc ggtttcttgc attctatcat aanttgcagc canttcnnac taattagcta 60
ccaatnaagn ttaatngnaa tatnctcgtc catcgaacgc tntttaataa tctngaagac 120
aacgcgtgca cggnncttat taaatcgtct ataacgataa tnnattttta tatatacatn 180
catatatgta tactgtatat atncatatac agnaaaaccc cgatnagacg atctttnaan 240
aggctggacn aaaaacgcat cttacggcag aaaatantat taaaattaca ttaattgnta 300
aaacaatgaa aatattttca tttgcnagtt ttagtttatc anccattcac taaantagct 360
gtaagnggaa ttctgcctta aggcacnatg ncttaattgc cttaaggcaa atngctggaa 420
cttttng 427

<210> 592
<211> 307
<212> DNA
<213> Ctenocephalides felis

<400> 592

antcggtatt gccanctgnc cnngnggtnn gatngacgnt gnntnctatg tgcgccggca 60
ntncncgatt catngctaca gaaacggatc gttaaatttt ttgcanacta ttgaagngca 120
tgttttgnnt ctgatatnta cnatcanatt acantnncng gatgcggtat anttncangn 180
nncgatncga ngaaanctan ntgtcgaatg gcgntatgaa taaggcataa cancattcta 240
ggtattagat aattaccaat aacttatccg ctagactaat atctaatcta aatatatncg 300
ttttcgt 307

<210> 593
<211> 500
<212> DNA
<213> Ctenocephalides felis

<400> 593

ccanagnata ttatattggt aattncctat ggtacaaaca naanttaact aggtataaga 60
taaattntca tttcaagaca tctgttactt taagttgatt agcaagaatc tcaaattgtt 120
agctgttaat ttaaaaaaat ataaaagcgg cctttactaa aaatgtaaat gtgaaaatat 180
tttcaaaaat atnttttgaa tacattatgc ttaaaatcgg attaggattt tcagaaaaat 240
attaactgac aactggaaac ttannntntt tatnntttca ntatntatag cagnatannt 300
annnnatntn cattctnnnc naatnntgnt tnttntcntn tnnnnnntnn atttttntnc 360
cannctntnn gtcccnnnna ttntgccnnn ntnnnntnntn tttntnncnc acnttnnnnt 420

ncnnntntnt tcntttnnnc nnanttctnn ncatnttcct ttccnctnnc ntntnctncn 480
tacnaactnt nttnttnnna 500

<210> 594
<211> 500
<212> DNA
<213> Ctenocephalides felis

<400> 594

gnnnngnntn ntnnntttgg aancacngaa gctcttngtn agctccaatt cggtttgngg 60
aaaaccctcc aggtacggaa cccatgggaa tgtaaacaaa atttatactt attgttaaaa 120
tctctctaaa atatctgata atttgatgtg catatgtcta atgatcattt ttgaccgcca 180
aatggcacat ttttttactt tttcctccat atctccaaag tcgttggacc tttccaaaat 240
tttgaacagt tcttcattta gtcaatacaa ataaaatgtt ttttaaatta ttcaaatcgg 300
acgctccgtt ctcttaaaaa ctgagttacc gttttcggca ctttttgccc cgtatcttcg 360
gaacggctag acctaccttt gccaaaaact aatcagcacg tcttcttatc aatatgaatc 420
gaatgttttt taaattattc aaatcggttg attcgtgttc ccgaaatcgt cgacgaaaat 480
ttgnatccgn acatacatac 500

<210> 595
<211> 204
<212> DNA
<213> Ctenocephalides felis

<400> 595

accatgtaaa tcgcataatt agaccaaatc acgtatccaa caaatctttc aaaataacta 60
aaaatttcga atcggataat agggttcaga taatatcgca tcgttaaatc tgcgcccgta 120
aatctaccaa cggtttgata tattcaagcg atcgtgtggg cggcatgtct ggtgggatat 180
ttactttcac gtctcgcgac gggt 204

<210> 596
<211> 500
<212> DNA
<213> Ctenocephalides felis

<400> 596

actttgtttt tcttttaatt gatgcttgct catagaataa tgtaaattcc gggcaatctg 60
tagnggaaac caggaaatat aatccagcta aaacttgacc tttggtatca cgtgccagta 120
atgaacggaa gttgtatact aaattacgcc ttactgtcac atccactgca gtataataag 180
gtttatgcac atatactaga gataccacta gatttgtagt tgttcctcct tctttaatag 240
ttctgtgttg tcgttcatgc cattcaacat aatgagaaga ttgagtgcta taagcatagc 300
aatattcgcg tttgcttcca cctggccctt catcacattg tgtaaaccaa cccgtatgtg 360
gatatttttc atcagccatt actgtcacta ctcttgcgac gtgatatcct ggatcacaaa 420
ggactacacc ttttcttcca tttatgcaaa tacgaatagc accataacgt gttctttttc 480
taagcgccgc tggagccctg 500

<210> 597

<211> 428
<212> DNA
<213> Ctenocephalides felis

<400> 597

```
actgacttct tgtatttcac caccgtatac ttcttcctgc aaagctctaa aggtctcact 60
cttggcggga tcgtaaacaa cagtcggttt atatggtagg cttgttgtat gacgaatcgc 120
ttcggcgacg ttggcatcgg aatagaggcc aattggagaa ttgaattgtt tatgcacaac 180
cttgtttgct atgctatcac cagccactct gtgtaatact gaatcggcaa ccttttgctt 240
cattaaaact tcatgattta atgttgactg aacgggagcc ctcatggccg gattcgggtg 300
gtggcgcaga tagcaatcat tagcatttga gtcgcgcttc acctttgcac ctggtagcac 360
caacggggtc gttctgtatg gccaaagtgt tgcagaatca tccattttat ttccgtgatc 420
cgtggtgt                                                          428
```

<210> 598
<211> 500
<212> DNA
<213> Ctenocephalides felis

<400> 598

```
cgtttttcac gaaattatgc aacttgtatt aaatcgcttg ttaatatcac tgtgcatgtc 60
cgccaaaaaa atatcataac tcatcaagtt gtgaaactaa agattttcca ttattacata 120
ttacatacta tacatcataa tactgaacga agattattaa tcgaacgctt tatggagtct 180
gatatatttc tgtaatctag acgtataaga atctttccgc ttatcttgac ttatttattg 240
tttgttaatg attatacgca gcacaattga tattttatca cactttccaa taacaaattt 300
ttatggttta tgtgattttg tataaaatgc tgctaattat aaaattattc gactgttatt 360
acgtgttttg ttggactaaa ttctaagaaa attattaata tttatttaga tatatttgtt 420
tcgaattttt atttctaata taatgttgca ttctatttgn tacatttatt taaataaata 480
atctgctttg naaaaaaaaa                                             500
```

<210> 599
<211> 194
<212> DNA
<213> Ctenocephalides felis

<400> 599

```
cctgttgttt agacatcang taaacactaa atttntnaca accgantttn agncantaat 60
cattaatgtg cacnnaaaac taatatttag atgggaagga ttggatttta tcttaaaaac 120
taaagaaatg tgtcaaaacg tgcgcgttcc tcctcggcga acacgctagc cgaacacagt 180
ctcgtcggaa ctgg                                                   194
```

<210> 600
<211> 383
<212> DNA
<213> Ctenocephalides felis

<400> 600

actcaaaaaa taaataataa taaaatctgt aataagcttt taactgtatc tcattcggaa 60
taatacagaa aaagaaatgt tctttgctga ttattccact tttttcacaa ttgtagatta 120
tcatatttta tctataattt atataatttt tttaatacat ttagttttat atagtttaca 180
ttatattcct ttccataatt tttaatattc catgtaatat atgtaggaat attttgtaca 240
aggtgttaat attcaaagtt gcataaggct ttttgagctt taaaagtaaa agcttcaaaa 300
agtttggaac ttttcgtatt ttgatgtgaa aatcctaaat atcttttttt ttggtttatt 360
gttcatagca aattatacac agt 383

<210> 601
<211> 500
<212> DNA
<213> Ctenocephalides felis

<400> 601

actatactat ctaatacata ttaatacaac agaactgatg cttagcatta cattttttttt 60
cataatagat ctaaaatgca cttcataaaa caacaacaac attaacgtta attttaacgc 120
gttaaaaaaa tcacttttaa atatcagaag ttatagctaa aataatagca tttattatta 180
ttaaaatatt aactaaattc gtcccaaaat attaaacaaa cacaaaaaat cacaatgtag 240
taaacaatcc aattagatta gaatcaaatt agtatgaaac caattcgagt aaaatccata 300
catagatgtt atatagagcc attaagagta gaacataata attgtagtta aaatgcatag 360
attgcctcat caaaaatcaa cattaaatgt tgtatttccg atgatttgaa atcaattgca 420
ccacaaatta tctcaacaac aattcactta tacttatatt tatattgaca tatattcagg 480
tactacatct tatcatttac 500

<210> 602
<211> 500
<212> DNA
<213> Ctenocephalides felis

<400> 602

cataataaat gttacataca ttacaataaa atattataca tgtattttgt aaaactcatc 60
tttttcatcc aatttcttag cagtgcaatt aactctgtaa ttttgacgta tatactcgcc 120
atggattcga ttcacaagtt acaaaactgt acccactttg gagcccctaa atttaccacc 180
catcaacata tcgagagaat cgaatggacc ttatctaaaa gtaaccctta ggaatacaac 240
ggtttatgga gcttccaact tcattgtcac ggatttaaaa tccgatctca atactggata 300
tttccaattt aatttgactc ttccaaaact ggacgttgaa ggtgatttca aaatcaaatt 360
aaacctgttg ctcataaact atagcggtgc aggtcgaatt tacatcaaca tgactgatta 420
ccacgccagg atgcaaatac atggctacaa gaaagttgtc gacggaatcg aatccttcaa 480
gtcaaaccaa tcgacatgaa 500

<210> 603
<211> 220
<212> DNA
<213> Ctenocephalides felis

<400> 603

acgcccaatt aaaatgacga gtaaatcaat atcttgtcct attataattg cagtttttcc 60
agacattgat tcttgaatta cactttcaac aataaggaca tccgcatcat ctctggcttg 120
tttagtagtt atatttgcag cctttaaatt ttccattagc atgttaatga gcctaatttt 180
attattttga ttcgataaaa ataattcttg ggaaactggt 220

<210> 604
<211> 465
<212> DNA
<213> Ctenocephalides felis

<400> 604

acgtaacctg ggaggcacac agaagcatct atttcgcggg caagaacgta acggctgttt 60
cgttgcacac taatacaccg cggttacatt tttgctacgt atctaaattg aataaaaaag 120
cgtgtaggtg tcttttatgt tacatactta tttgattaaa tttcctgcaa atatagaata 180
ttaataataa tttcggatca ttagtaattt tattaaatat tatcatttac aatatacaaa 240
atttacatat tacatagtta acattagaaa actaaaattt gatagtccat aaaatgttag 300
cctataaata taattattta tttattcata atatgtatta tgtttaactc tcttggcaac 360
ttcagttaaa tcagacttgg caaagttaag ttttgcgtta aatatgttca actcacttgc 420
atctgttaat gtatctaaaa tagaaccaaa catcttagaa cattg 465

<210> 605
<211> 231

<212> DNA
<213> Ctenocephalides felis

<400> 605

accaccccc aaaacaccat taatctcacg gaacgtttct ctagcggatt tgcctttaac 60
gaaggaaaac tttaaaatag cgcgaatttc ggcgtaagtg aactccatgt ttacacgtct 120
ataactgtta aacgcaatat ccaaactaat catgcatagc atcgttttgt aggttatgtc 180
aagacctttc aaattatgta tagtattgcc agatacgagc tctgtagcgc t . 231

<210> 606
<211> 186
<212> DNA
<213> Ctenocephalides felis

<400> 606

acgtttggta atgtcacaga cgctctctcg cgggcattgt atatggtgtg attcattgcc 60
atagttccgc gcgtcgaaat attcaaaatg cattgcttcc gttttaacga cggcattcga 120

gtttctctct ttcctgcaca agcgatatgc tatttgcata cgggcacata taaatccgcg 180
ttcgcg 186

<210> 607
<211> 500

<212> DNA
<213> Ctenocephalides felis

<400> 607

```
actncntact atatgtatac tttttaaatg actgtaactc gaaaactatt tgagatatcg 60
atttgaaatt ttaatatgtt attttcaaag gtgtaattta ccgaaatata aaaaaaacaa 120
aaatcgattt tttcaaaatt tcacactagt tgtgcccctt aatccatctt ctattgctaa 180
agtgtaggac gtctactatt ctacacaaat cgcacaaata aaaaattccg acagaaataa 240
aatcccattt cttcaacatt ttaacttggc accatcaccg tccctctaaa tttcatatga 300
tatattgcaa attattccgg caaacgcgtt gcgcaacaat gggcccttgc tcaccgtaaa 360
cataaacagt tcttatcatt gacgtgcctt cgttctgtat tgtatacata tgtatgtata 420
gatacaccaa aaataggtat acatgatgaa atattctgca acgaatatat cgggaaatgc 480
atgtattatg cattaatcaa                                             500
```

<210> 608
<211> 269
<212> DNA
<213> Ctenocephalides felis

<400> 608

```
acgaatgtng cacaatcgat gtgatatata acaacgaccc aagcgacgtt tttcaaaacc 60
cgaatcaatt tcgcacaatt ccacaacgaa taaaataaac gtgactaaca aacgatatag 120
tggaaagata gtgccagcaa attcgaaggt tctgattctg attctcttct gcaattagtt 180
tttaaaatca aaacatttta ttatatttta tgaagttcaa ttaattgaag gttttattga 240
aaatttactg aatttatttc cctcagcgt                                   269
```

<210> 609
<211> 500
<212> DNA
<213> Ctenocephalides felis

<400> 609

```
acttattaac aaaattcaat caataaaact tattggtatt ttaacttact tttaagtatc 60
ttcagtatat ttttcagaca gcctcagaat gcttcagaca tctttggaat atttactaaa 120
tgctcgatat ttaatgagac atgccgcttt ttcatttgat ggccaccaat caaaatcact 180
tttacagtct taaaatgaca gtgatgatgt cagatgaaag catttgaaga aatataaaaa 240
cattgtaaac tgtgaaagca gacaaaaaaa taacccaaac aactaggccg attgttatac 300
aaatctaatc aacatacctt aaagcaatat gacactcaaa cctagttatt gtgttaatga 360
atgagtttcc tcagattatt aacttcaacc actgaacact agatttgtca gggcctccga 420
ctgatttcaa ttgcaaaatt atttccaaag cagcttcatt ttgctttcca atacgaataa 480

aacattggct gagcctgtaa                                             500
```

<210> 610
<211> 500

<212> DNA
<213> Ctenocephalides felis

<400> 610

```
acgttatcta ctcaacctga aactttgcct ttatcgcaac tgggaccagt aattagccct 60
gttgcaacca cattgggtcc tgatgcagaa ttgcatactt ttcgagaagc acttgccatg 120
agttctttga gtggacgacg tgctcgacat ggtcctgagt tatatgcctt tgtaactggt 180
gaaatagcaa gacactgtaa aagaccagcc agtcctggtg atgtcaatat ggagtctccc 240
acacatcata gtaagcgggt gcggnttatg taaactggta agttaacttt aacactaaag 300
naatttattt atgntaattt acatttacta ttggtcatgg actgaagaat attctaagat 360
tgccagtttg naatccaagt tttacctgga tattatattt tactaaattc gaggaatgaa 420
ctatgaatga tttcataggt ggattaangg aagtaattct ttaattttat gaccatacat 480
tggaatggac caaaagncct                                             500
```

<210> 611
<211> 140
<212> DNA
<213> Ctenocephalides felis

<400> 611

```
acaacatgcc acgggttcta ttcaataaac acattttaca catgatttgc cacttacgtt 60
tattatctaa gcccaaatca tacccctaga ccatcgagcc acgggttcta tccactgcgt 120
gtttgtgttg catccaatgt                                             140
```

<210> 612
<211> 500
<212> DNA
<213> Ctenocephalides felis

<400> 612

```
actcgtgata tcgtgctaaa aaaatataca aaagtgttgc catatgtgga acttgaattc 60
ggtcgcggca ttcatcgtag acacgaacag aatgaggtgt gaatctaaac tacattacac 120
ccatagatca gggcttctta aactatgggt tgcgacccca aatggggtca tgtaaaaaaa 180
ttttggggtc gcaaaagatt ttaatgccat tttattcatt ttattacatc acgccttgat 240
tttttatcaa caaattctta aacatatata ttttcatatt atatgtatat gtatattgtt 300
accaaataaa taaatcattt aaattttttt ttatttattt aaaatcatta aaataaatta 360
aaacatgttt ttatatgtgt agggtcgtca acaaactcgc aatcataaat gtgatcgtga 420
aggacagaag tttaagaacc ctgccataga taaataagca caaatgtttc cttatctatg 480
cataaatgat aaaatcattg                                             500
```

<210> 613
<211> 146
<212> DNA
<213> Ctenocephalides felis

<400> 613

ggtcaattga atctgagctc cagtgcttag caaagatcag tgttacgtat aaggtaattt 60
tacataatgg catctttgaa gttaaatatc gtaaccagga gcttaagctc atctgcagtc 120
gcgtctcaaa tggttaaacc acccgt 146

<210> 614
<211> 162
<212> DNA
<213> Ctenocephalides felis

<400> 614

cttaaaaatt gatgaattta ttctatattt tatgatttgt atattttatt aatgtttcaa 60
agtattatac gacatcttat gcaatcttgt ataaaatcat taatatcatc tagttgtata 120
ttatttgatt gttgactagg ttgtaaaatg ttttggttcg gt 162

<210> 615
<211> 274
<212> DNA
<213> Ctenocephalides felis

<400> 615

acataaacta cgagcctaca agtcttgtat ttgcgaacga ccttttcgaa caattaatta 60
tttattttta taattaataa gatagatttt atgaataaaa taccatggtg tttgtttcat 120
ttaaaattgc attgtgaaca tttaggcttt aataatgatt acgcttgaat tctgtattgg 180
gaaatttttg tgactcccat attgaataat ttagaaattt ttgataagtt cacaacaatt 240
tttgataagt tttattggaa tgataattta tggt 274

<210> 616
<211> 266
<212> DNA
<213> Ctenocephalides felis

<400> 616

acatcttcga tggtaaatca ttattagcat tttggaaatt ttaaacacat tatgcagaaa 60
atgtatagtt ctaagcattt ttttaagttt aattgtttaa gtctaagttg aaagattaga 120
tttagacaac tccaatcatt tgtaaaaaaa cactgcttgt tttgaaaaac aaaatttatc 180
taaaaattga cattataaaa gattcctaaa aattaaaggt atattgccca taaaacaatc 240
ggacgttatt tccatatcat aaaagt 266

<210> 617
<211> 173
<212> DNA
<213> Ctenocephalides felis

<400> 617

acgcggaacc gagaacatcg actcatttta tcttttgtta aaattatcac aaagccctct 60
caagagtaga catacgtgcg aacatttatt aacacctaca caggtggaaa tgtaacagag 120
gcattgtaat tttggaactg tttattattt ttaactaaat acgctaaatt ggt 173

<210> 618
<211> 500
<212> DNA
<213> Ctenocephalides felis

<400> 618

actatttgtc gttgctgaca tttttcctgt gacaagaatc ttttaaattg aatgatcgac 60
aatagcaagt taattaaatg cgtgtgtgct ttcgacgttt agttaaatag tttacggcct 120
cattctcgat attaattgaa ccaaatcagt tccagagaaa ccacggtgga gcagttcaaa 180
tattcattga agtttggaaa ttaaattcaa ccaattcagg tgtagatacg tagatagcac 240
tggaaacacc aaaccgcaat tcagcagaag ttttatcatt attttgttat tttttataaa 300
ttttaaaaac tttttgaaat gactcagatc tatagacgat atgaaatctt gtaatttata 360
tttaaaaatt acccggaccc cgggttaaat agaaaagcct tggtcaaatc ttgaccaata 420
atatgaggaa tggtccttga tactctatgc attaagttac taaaattcct tcgtacttct 480
catgacccct tattatcact 500

<210> 619
<211> 500
<212> DNA
<213> Ctenocephalides felis

<400> 619

gtntngaagc ccttanntna ngnttcttng tatttcccga ngnccntcca ggtacttgca 60
ctaaatgtag atcgtaaagt tttttatttg naaacaggta tgtccataaa tattgttcgn 120
atactgcatn nagaatatta ttaattatta aaatcacaaa atatattaca gaaaattata 180
aaacttttat aaaaatacat atgactggtt taaattagta tgtgattcat cacacacttg 240
attgtaaaat gttgataaaa aaatatgata agagattcaa tttaaaatgt aatagtaatc 300
tgtaacagtt acaacaatat ttatatacag tataacaata acaattaaaa ttgatactcg 360
tatctgcaca aaaagtttca attgttgaag aaataatcag catccaatct atgattaatt 420
tttggttatt ttccaatatg tatggacgga aaaataatcc ttgaaatatc ttttggcatt 480
cagttcatag ctcttcaact 500

<210> 620
<211> 299
<212> DNA
<213> Ctenocephalides felis

<400> 620

```
acatctatat ataagttctt atctatatta aantaacgaa gagaaaagtg aaagaaatat 60
tttntccttt ctttcttctt ttattccact aacgtggacc gtgtcgtatc tgtaattatt 120
tctaacttta cgctcaatct tataattaag gtctgataat ttgccaacta ttttataagg 180
cccgtgcaat ggatgttgtg gttagtctga ttggccttat ctacgaactt cggtgttccc 240
taacacccac tggcctacta taaaatttgg catttttgtg gagctctcac gatattttg  299
```

<210> 621
<211> 491
<212> DNA
<213> Ctenocephalides felis

<400> 621

```
gaaatgtcat tgcaaatctt gatgataaaa aggcaggtag taacgataac atttttgggt 60
ttttatttta ttttgagtta caactacctt tatacctgat ttgtagagca gaaaaaatga 120
gcaaaaagtt caatgtatta ttgatcacat aaagcaataa tacaaatttt tccaattaaa 180
tgcaacgcat tagtaaattc attgaaatgc aattcctaat gcattgcatg taaatagaca 240
actcactgct acaaacagtg cattaaattc tgttttaggc attagttttt gaaatccatt 300
ttcattttag atcattttttg ctatgtgcag tattcggtcc ttaggatcta aggtaaaaat 360
ttaatataga tataaagtca ctgtaagaat gtcatttctg aacttttact agaccatgta 420
gaatataacg agttcatgct tttgaagatg tgaaatctta attatgttta aattattttc 480
ttctttttct c                                                      491
```

<210> 622
<211> 121
<212> DNA
<213> Ctenocephalides felis

<400> 622

```
acatatacaa gcaatatatt aaaagtagtc tatttaacaa actttaaaat attataatat 60
tgtaaaacta aaaacatata taagaagaat tactaaagca aaattgtaat tagtaactag 120
t                                                                 121
```

<210> 623
<211> 500
<212> DNA
<213> Ctenocephalides felis

<400> 623

```
acgctatata aatgatagtt ttcggcatac ttacaaatca ctcataaatt cttatgaagt 60
ctggacaaga aaattataaa attgaataaa agctctatat gcaaacaaat ggaaagatga 120
ataacgaaac acttagcgac aatgccagtt ggctcccaga ctttgatgaa ttagtaatta 180
tgaataaggc agttaggggc ttgctgtaac attttttttt aattcatata tcttcagatc 240
```

```
cagtgattat atttctacaa aatagaagtg gatagtcgct gcaactattt ctctaccaaa 300
tcaagtaaag ataatttcac aacttttgtt aattatttca caataaatca gtttctagaa 360
aaagttcaga gttacatctt acctcgtgtt tggggcatgt tgtaatagct accggagcaa 420
gtagtagatc ggcaatnatg ctctgataaa tcattctcta ttattntata aaattaactt 480
gttctaagta attctgttat                                             500
```

<210> 624
<211> 409
<212> DNA
<213> Ctenocephalides felis

<400> 624

```
acaaattttc taaaaatatt agttatgttt tataaatagt ttaaaaatta catattattg 60
aacaagaaac aaactgtagt aaattaaaaa caagatccaa atttccaaag caatttgtta 120
atcaataact gaaaaacgac tcgtcgatta ttgacaaaat ttaatcagca agattctcaa 180
atggaaaacg tttgtaatgg tttttgaatt tttaaaatca attcactaat taaaaatttg 240
agactttttt gaaaattcta aaggcaaaac taagcagcac gagtgcttat cgaatttacg 300
aattttgacg atttttcaaa tgggattgtg catttttgag aaaaggccac taaagatgaa 360
attttgctca cgtaaaaaag cgccgctcat ttttgcaatc tttgatagt            409
```

<210> 625
<211> 600
<212> DNA
<213> Ctenocephalides felis

<400> 625

```
nccatnaaat tngtaccaaa antaantttg gatggggtcn anccgncccn ttanttggnc 60
caaaagtaan ctnnggcttt atcntantaa atanggccca gtttatttta aaccttaatt 120
ctaatcctta ttggttattt taaaaagccn tgagaccntt aagtaatatt gctgcgtgat 180
tcccttaatt gngcattcat caacattaat tttcggctaa gttttgngnt gngntcttgn 240
aagtaactaa taatatcggc ttgnttttca agaagttctg ctatatttct tccctgctgn 300
ggaaaaccaa cagcatcgct cctctgncct tctgacatgg nggccatttg ncttatccat 360
tcacttttca aaacatcggg tggnaaatgn ttcatattaa aatcaaaggc cttattaata 420
tatccccatt aacttccatt aaaactgntg gttcacaata ccaatatggt acatatccac 480
tggagaaaga atacttgctt cacaatggtt tggnaaatca tnctcataaa gtggttgctt 540
aaaggttgca tacccattat tcagcattcc atanggatct ccntnttacc gatatctatg 600
```

<210> 626
<211> 480
<212> DNA
<213> Ctenocephalides felis

<400> 626

```
caaatttatt gtagttgctg aatgatgaca cgcatctggt gtcgaaacgt ataaataaaa 60
gtttttaata ctttatacct tttttatttc gacgaatggg ctgctagtca ctttgaaata 120
```

tgtatatatg ttcgacattc ttctcttatt acctttgcta gcctgcatgc atttatttat 180
accccttacc ccgaagggtc ttggatcaaa taccaaaatc ctggtcaatt ccttacagca 240
gagtaaaaac caatgtcact actacacttc aatcttctga ttgaaatccc attgctctaa 300
ttactaatcc aaaccaaact ctatctatta tgtgtttttg aaaatcaata cgcctaaaac 360
tacccgtaaa ctagactgct aataactgga aacgaatggc acactcactt tatacgtcta 420
atctaaaata tttacttcgt gtaatataat atgggtaatg catgggagag gaaaggccgt 480

<210> 627
<211> 600
<212> DNA
<213> Ctenocephalides felis

<400> 627

acaaatatat tctttaatac ttactgtata gtgcattata gaacattatg atccgctaat 60
tatgttgatt taattaagtg ataacgtaaa tagtcaattg acgatttatt acttttgact 120
taagtatatg gctaaacaag ttagaacaat ttaaatgacc gacaaacttc atatttgtag 180
ttgatcattt caggagatca ttttaaattt ttttggttta agtttcaaaa tagtaatatt 240
ttgtttgtga ccctgggcaa tgcgcgaata taataatatg attttattta cataatgacc 300
gtattaattg aaataaattg acgtcgaaaa tgtaatagtt ttaattattt atgatcaaac 360
aactatcaaa acctaacatt attcatgcat tcaagtatta gtgaatgtgc caatttaaca 420
tgttccggaa aaaattgatt taagtttgga gcaatataaa ataaaaacga aacacaacga 480
tttttcatgc gaataggcta ctgataatat gttttattgc ttgccatttg gttaatttta 540
tcagaaaccg ttcgcataga tttaatcggg tttcgaaagc cagttaatnc agtgacgcat 600

<210> 628
<211> 500
<212> DNA
<213> Ctenocephalides felis

<400> 628

accattaact tatttttaga aaaggttcag gtgtttttac atattgtctt tctctttttt 60
agatgacttt tatccatttt taatttggga tgtgtgttat tttcgatatg tagttcacat 120
agaacacaaa aatatttaaa gtttggatct cctaaatatg ctggtgataa ggaacgtaaa 180
gctaaactgg gatcacactc aaagtgaagc catcttaaac acattccaca ttgttgccaa 240
gaactgctgg atggtattaa gtccccattg caattgcttt tgcaattaat aaacgcgtct 300
tgggaatttc ttagacatag tgaataactg taataataaa taaacaactt tatttgtaat 360
acaaacaaac ttgttaattc tgataattca aaattttgtg aaaagatatc tttatcagaa 420
ttctgataca aataagcaca tatatctaaa ttattatatt tacttacaga actccggctt 480
ctctacaaaa ttctaattag 500

<210> 629
<211> 111
<212> DNA
<213> Ctenocephalides felis

<400> 629

accctactaa gaatgtgaca gaattttaaa gtcgggacgt ttaatagttt tcgagatatg 60
cgtggtcgtt tggtcgctgg ctctacgacc aatactaata tttaaacagg t 111

<210> 630
<211> 103
<212> DNA
<213> Ctenocephalides felis

<400> 630

acacatcaaa ttgatttcaa atttaatgat aattatattt atttcatcat tctaagaata 60
aagacaagcc catgcatcac attattaaac aggtgctttt tgt 103

<210> 631
<211> 500
<212> DNA
<213> Ctenocephalides felis

<400> 631

actcacatct atctaaacat acatatatga tttgcagaaa ataacatttt caagtcatat 60
atgacttggn tttgntcant accnttntaa ntttggnaat ggncctanng gnnnannnca 120
nggcttattt ataatgaatg taaatgcnca aacatgtgaa taatattaat aggattagta 180
tgcacgaaaa aattaatanc aataaaaaag aaagttttgg attttagatt gagaattaaa 240
aattaatata ttagatgtaa tgattaaagt gcaaatgttt agctatattt taattgttaa 300
cagtggaatg tttattttgt attgaaatct gttattgcta atgtgataaa catttttttct 360
gtgttatgca catcaatttt tggtattact gtgttttaac tgtaaattat aatattgcaa 420
aaatacatta gtatattttt tcatgaagtt actaaaaata attttatcat tttcaaaatt 480
attgtgcgca tgtatttaag 500

<210> 632
<211> 500
<212> DNA
<213> Ctenocephalides felis

<400> 632

actaaatgtt aattgtgcga gaatggtgga tgaatttata aagaaacata tgggattcat 60
atngaattaa taaatgtaat gcaatgaaaa cattcgtatt aacaaattca ataagaaaca 120
acacgaactt aatacttgta acatttgtca atgcgaatgg ttcttgcaaa aaagccaata 180
caaaaaatgg caaatattat atatgtatcc ttgaattatt taattttttt caaacaattt 240
tccaaaattc cnaaaagaga gaatgtataa ttcagggcag tagcttaatg ataaaacgat 300
cgccggaagc attttagctt actatgacat gactattatg aaaataaaat tgatataaaa 360
tgtgaaacat ttgaactata aatattatat ttaataataa atttgntatt gntatatcna 420
caaaannaaa nanntngctg tnanaanaaa aaaggttgaa cttgggccgg anacccgnta 480
ggccgaattt tgganatttc 500

<210> 633
<211> 392
<212> DNA

<213> Ctenocephalides felis

<400> 633

```
acaatgtgat tcacggaagc caatttttct tccaatcaca caagatgacc ttgaccttgt 60
gcgtttgtgg tcacaattta gaacaaaaca catccaactc ttagcaaaat tacataaaca 120
gtaaaatact ttaaataaat aattagccgt caaaactccc accaaatttt atacgtctgt 180
ttttccgtgt ggaggatact ggcaatgaga actggtttca ctaaactcgg cgctcttcaa 240
acatttcgaa aatcttaggg agaacaaaat tacaaataat tagtcatatt acttacatta 300
tctttattta catcgtttag tcatctactt tttttaatta tttatagtta gaattacaaa 360
ttgatttcag acttaagctt ttcaaactgt gt                                392
```

<210> 634
<211> 413
<212> DNA
<213> Ctenocephalides felis

<400> 634

```
actacattgg aaacagccag tgatataagc aataagcctc caccgccatt aaagctgcta 60
atgaatccgt atggtaaagt tttagacata aatactgtgt ataaagaaac tggaaccgaa 120
ccgctcagtc cggatttaac cttcaatatc gtcaatgctc tgaacgcgag caaaggcaga 180
ggtgctgaat tgttcgctaa gcgccgcaag aaatctgaga agtggatcgt agatgaaaca 240
agaactactg aaaaaataat caacaaagaa aattctgttc agcagtattg gaaaccatca 300
caaagtccgt tatgccagac taatcgattg ggttcttggg aaaaaccaaa actgcaaagt 360
aataacaaag agtgccttta tacttctccg attcaatact accaatcgtg cgt        413
```

<210> 635
<211> 649
<212> DNA
<213> Ctenocephalides felis

<400> 635

```
acatgagnaa aaagtgtggt tatatatatt ttttatattt aataaatgat atattcaaat 60
gcattntttt ttaattctct atcatattca aatgcgctta tgatacaagt gcaaaaatta 120
catgcagttt tcataatcca aaagatttta taatcgtgta aatgtattta tagagattgc 180
actactaggt gggcgcaata ataatgctct tactaaataa tttaatacat atattagagg 240
caccactcta gccttatgct aattgatttg atctatattc agaaatgata aaaattaatt 300
cactgtagca gtattattca gttgttttaa ttagaatttt aatctaaatt gcaaaatttg 360
tatatgaaat aaaagaatag gtaatacact agaatacaat gaaataaatg caatgaatta 420
gatcacaaat cattgacttg nttttttata ttaaattcaa aagtttttat aaaacatttc 480
attgaattaa atcaataaag aaagatagaa atccaaatat tacaagatac attattgata 540
ttttataata atagtatgaa tccataactc atgtggcaca gccattatta acaattaaat 600
taacaatatg aaaatcctaa tattaaaact atatttaaga aagcacatt            649
```

<210> 636
<211> 500
<212> DNA
<213> Ctenocephalides felis

<400> 636

actcacatct atctaaacat acatatatga tttgcagaaa ataacatttt caagtcatat 60
atgacttgan tttgctcatt accatataat tttgagattg gcctcatgtg atgagacgag 120
gcttatttat aatgaatgta aatgcacaaa catgtgaata atattaatag gattagtatg 180
cacgaaaaaa ttaataacaa taaaaaagaa agttttggat tttagattga gaattaaaaa 240
ttaatatatt agatgtaatg attaaagtgc aaatgtttag ctatatttta attgntaaca 300
gtggaatgtt tattttggat tgaaatctgn tattgctaat gtgataaaca ttttttctgt 360
gttatgcaca tcaattttttg gtattactgn gttttaactg naaattataa tattgcaaaa 420
atcattagta tattttttca tgaagttcct aaaataattt atcatttcaa aatatgggcc 480
catgtttaag ggaaaatgaa 500

<210> 637
<211> 500
<212> DNA
<213> Ctenocephalides felis

<400> 637

actgaattat atcttaatta aattttggta tatttcgtag aaatataatc gaggatcttt 60
tcttcaagtn ccaaatgata ctgttacgac aagcccctag tcgtcctatt taatttcaat 120
ttttactcta attgtcgttt acaattgatt ttgagctgat tttccgtggc tttagacacg 180
tttttccatg gtttggcaac cttgttcgat tttttagttc ttttatattg aatcgtatga 240
tcgttgttcc tttaattttc tttttgaaac agtccttgtg tggtttgatt cactatttta 300
cttttaggct gcactcatag tcagtaatta atatacacga ttgctgattt catataaaag 360
taatgtaatg taaaactaga aatttcataa ataatcaata ctaaaaacta ggaacgaaat 420
taaagtctgn cacttncttt tgcatatagt ccttctaaaa ntagaaactc aagaagttta 480
actatgttgc aaagtttcag 500

<210> 638
<211> 500
<212> DNA
<213> Ctenocephalides felis

<400> 638

acanagctgn aattaatatt atattngtcg gagggcattt tcaatcnata tcctatagtc 60
gaagtgtcga tgtgangagt ccgcatttat tattcagact tctccatttc ccgctttaac 120
gtttcgatat aaaatcggtt atgatacctn ctaaattctt tacggcctta ttttagcttt 180
ttattgttcc gtgntttgta tactgaaata atgatgaatg cgcgaatatt taatttacag 240
tcttcaaagg atatctaaat gttgtcgttt tgncgntcgt ctgcctgtcc gtctattggg 300
cttttgtgaa ttaacaataa ataaatatgt attataacag tttagactat cgtatggtag 360

caaattattt tattcaacta attaaaaaaa caggcattat cgatttataa ttcttgaaaa 420
tctaaggtgt gataaanaaa actggtggtg tatggagagg agaccctatt ttntcaagta 480
aaaccgggac gataatttta 500

<210> 639
<211> 112
<212> DNA

<213> Ctenocephalides felis

<400> 639

```
aatttccata attataatac tataatttat ctttttgatc gctttatatg tgcaaataga 60
gcangccaag cgcgcatctt ngcagaattg cgaggaaggt attcggggcc ga         112
```

<210> 640
<211> 500
<212> DNA
<213> Ctenocephalides felis

<400> 640

```
acgaacaaag tttttcgta tcttttactg nttagctagt agagagcaat ctgngtgatt 60
gatcacttat tgaaatgcaa attacattta gattgcagca attattgaca atatttctat 120
attttcataa agaaaccata taaattataa tacattgnta aaatttcgta tggntctcca 180
gtcattcaat tctaataaca atcaccattt aggctaaaat cttgcatgtt ttctgccctc 240
gaacaaacgg ntattcacgg ncagcgtgtt tagatatagc agcagagccg catattcagt 300
tcagcaagcn ttcaagaata aaagaataaa ataatattta ttaaatatca actaatatat 360
aatcttttct cggcaatgaa tataaganat tattatttta ttctatacan tggattaaaa 420
aaaaaaaaac ataaatttat ttttattatt aatgctaaat tatttatcnt tcgtttattt 480
taataaanat aattgaanga                                             500
```

<210> 641
<211> 322
<212> DNA
<213> Ctenocephalides felis

<400> 641

```
nnnnnnttta gccccgntgt gnttcggcgn cgcccggcgg tcatattaaa acaaccggcg 60
ccgggttttt caactgaaag aaatgttttt ttaattcaat tatttataat taaatacaag 120
tgaataaaaa ttacatttaa gagtattatt attgcttaac aaaagagtat taaaaaagtc 180
tactgatcat aaatcaatag tataggaaaa gattggtaat atattggatg aaggtaaaat 240
acacaagaaa tcaataaaca aaaataaata ttgtgaaagt tatttacgcg tatataaatc 300
atcttttaac aagcatcatc gt                                          322
```

<210> 642
<211> 500
<212> DNA
<213> Ctenocephalides felis

<400> 642

actccgcgaa tctattgtca aacatgcttt taataccttt tatcatttct gaagaatgtt 60
gatcccaatt atcattattt aacctttcaa gataagattt gaagcttttg ttgccttctt 120
aaaaaatttt taagttctgc cattttaatt gtttttagaa ctcaagataa cttaatttaa 180
tttttgata gggaagcgca aaccgttaca aaattttacg tataacaggc gttatgcaat 240
atcagcggtt atatcagaat ttaaatctat aatcaattga ttatttaaca attctaattt 300
aataatatta gaattggagt ctagcacatc agtaacataa gttgnggngt taataaatta 360
acaaatatat aaaccaataa ttagtaatgn ttataataac taatataata tttcataaca 420
attaataacc taaggattta cgaaattggc ccaaaatctt agagcaagac caatttcaat 480
caataatatg gtcaggttta 500

<210> 643
<211> 127
<212> DNA
<213> Ctenocephalides felis
<400> 643

actaaaattg gttacaagtc gaagtccagt attataaaaa agtttttcc aaggaacata 60
caattcttta atgtatggca aacatattac accagaatga gtttctgagt caggtaaatc 120
gttatgt 127

<210> 644
<211> 393
<212> DNA
<213> Ctenocephalides felis

<400> 644

acaaaaaaat gtcccgggcg ttaaacttaa aaggcattgg tctctgaaac aaaatcttgc 60
ttgaataact aaatttttta atacaagaat taattattcg gaaaaagttt gagattgttt 120
taagcttaaa acacttgtga aaagttttat aaaggaaagt ttcatagttt tggagatatt 180
taattaattg tgcaaaaagg tcctgtgcgt caaacttata aggaatagtt ttcttgaaca 240
aaaattagtt tgaataacta attttttaa tataaaattt aattgttggg aattgtggtg 300
tatgcaaacg ttttagattt ttttatgctt aaaacacttg tgaaaagttt tatgaagaaa 360
agttttatag ttttggaaat attcaattaa ttg 393

<210> 645
<211> 394
<212> DNA
<213> Ctenocephalides felis

<400> 645

actttaactc aatgctattt atacaggtta caatattata tattaaccca tctgagtatt 60

ggaaaaaaat taatactgct tattataatt tgtgataaag cacaaaattc ttaatttgtc 120
actgtatttt tacatatgct taaattataa tttaatatta tttttagcat ttgttggtaa 180
cttttaattg tattctactg atatagattt ctatgtaagg aggtttaatg gtttttttac 240
aattcatgtg tggtattttg agaatcaaga aagcttttct ctaatatttt atacctctaa 300
actgaattga ttaactctat tttttcttaa tatttataac ctcctgctcc tccgcgctgg 360
gtgcgaggta ttgattgctt ggaccgcgcg aagt 394

<210> 646
<211> 435
<212> DNA
<213> Ctenocephalides felis

<400> 646

cgcgggctcc gatncnnaat gcacgacntn tanggcatgc gtgnaagtgc gctattaata 60
atacagttac aaagattant gnaacntana antngccaan cggaangnnt acgttgtaat 120
nanagggaaa acgtttttttc anncntttnt ntggantaag atngttaaat tgccgattnt 180
caacangnat nggnnnagta nnaaacnagg ctcaacggca aatgccntcc acctaaatta 240
ttttgtgaac ccaaanaaaa ctatccatac ttnattantn tngacggtgt cggtccatta 300
aagcatgana nttnnccgca tntntntcgg tttttgacan nangngtggn ntgcaaacnc 360
ngannnnang acanactnaa tncnagnatg agtgagtgng cgtgagttag agcatncnaa 420
anggatgcgc cgttn 435

<210> 647
<211> 492
<212> DNA
<213> Ctenocephalides felis

<400> 647

tcntacacta caaaaatgat ttttatattt aatgaaaata ctnatttata aaatatctct 60
tcaataagct atagttataa caggcncnnt taataattat gaattgnntt gaaaatgatt 120
tacaataaag ctatgtgaaa caagnntgtg tngtatataa catcatcgcg tnggncgggc 180
ttttccttgt tactatattt ttaaaaattt aataaaatta gctttgtagc aaatgctact 240
gtntgataat tgaattttca ngaatagtag gagttgtagc aggagtcttg ttagcttcac 300
gccatgcttc atcaaaattc tgaatagaat tagaacnaga tgatgaaact tttgtcttaa 360
cgttttcata ggctggccca tttttnttnt ngtgatttga aagantcaga atttctcatc 420
tgcccatttt ggtgnaaacc cccgtatttc ccttacaaan aagagttttt cggggnagaa 480
ttantactng gc 492

<210> 648
<211> 417

<212> DNA
<213> Ctenocephalides felis

<400> 648

acgccgttgg cgatttccgt gaaaatgttt gacaatgttt gttccagggt tggcttgaac 60

tcttggaaga atagtcttgc gttgtcgttc acagcctggc taagaattgt tccaagggtt 120
tgatcgttgt taaagagacc ggataggcgt attttattgt ttcccaaaat aattttcatg 180
tcgattggtt tgacttggag gcattcgatt ccgcgacaac tttcttgtag ccatgtattt 240
gcatcctggc gtggtaatca gctgaaatag taatataaat aaattactat aaatacgcaa 300
ttactagaaa tagtaatgta ataagttaac acgttcgcgg gtgtgaatgc tatagcattc 360
atcgtatata acgattaaaa tatgatgatt ttaatactac caatataata tgaatgt 417

<210> 649
<211> 500
<212> DNA
<213> Ctenocephalides felis

<400> 649

ttctaaaata atattgaata aaaaatagta gatacaatca gtggcttgta taatagggat 60
agctataagt agtgaaaatg gaagtgctta ttagaaatac ttttacaaaa cgaattgtaa 120
tgattcagta gcaacatatt aaaaaataaa attgtcaaaa tacttgcttc aaaattatgc 180
tagtattctt ctcatgaact aacattagct taattttata attaactaaa atttttgaat 240
atcctcacat tatcgcaaat ccttggtgac tacatcgtct tagcatatta cctagaaagc 300
atttcaccat caactgacaa atttattgga caattgatgc ctaacgtaat taccaatgtt 360
tacatcaagt aagatgctaa taattaacgt tatctatgtg cacgtaaata atgaaacaat 420
aaaatttcgt gagtttctta agcgtgcgtc cctcagaggt caaaaagtcg aaaagtcgta 480
gggtcatgat caacgaacgc 500

<210> 650
<211> 500

<212> DNA
<213> Ctenocephalides felis

<400> 650

acctactatc acagtttttt tttactaaca gtttagctat aagtaggatg gtgaaaactt 60
ttaaattttt tacatcttac tagcgatttt gataatactg attcattggt tataaatctt 120
caaagaaatt cttagttttc tacgtatact caaaacgtag tcaatacgta tatatccaaa 180
ttttacaccc catttgtgcg atttgctgcg cataattata tttacttcgt atattgagca 240
gttactaaaa ttgtatacta cgttttcctg aatattattg aacatgtgtt gaataaattt 300
acaccttacc tttattgaaa atatttgttt gtcgatagta atacgtggtc tttttacaac 360
aattttgatt gctgtcaatg tagttgaacc aacctttggg ttcaaaacaa aacctatcaa 420
tttgttgttt ggattccttc gtaaaaatct gctacacata gacattgagc aaaaatcaga 480
aaaccccttt gtatccgnag 500

<210> 651
<211> 483
<212> DNA
<213> Ctenocephalides felis

<400> 651

acacctatta aggagcaggt ttcaatatta ctcctaatga cctattgctt acacattgtt 60
atataaacat cactaaatat ataccatcaa aacttctaca agctaaagca gtaaatcatt 120
aattaacatc tagaatatac aaataagttt tcataaaatt ttcctattct gttctaaata 180
tatattttgt gcatcgcaat gaaaatacta agacgtatat attaatatta aaatatgtaa 240
taacttacaa ctgtcataaa gatagtgagt tataaatgaa aatatctcag atggtataga 300
taaatttcaa agttttaaat taaaaacttt ttcgaaggta acgcgaagtg caaacgaaag 360
tttcaccata aatagtttta gttcatttta ccataactcg cgataatact aaatcgatga 420
aaggctgaat tattaaagtt atcagaacaa tgaactgtta gcacacatat gcacatgtat 480
atg 483

<210> 652
<211> 500
<212> DNA
<213> Ctenocephalides felis

<400> 652

acctcatgag cagttgcatc aatcataagt cgttgctcgt agctcatcgc aagcaggtgc 60
ccgcaaatta tcgcatggtg attcttgagt ttctcgttaa ttctaaaatg taaaataatt 120
tacatgtaat atatacccaa tacactatta catatgaaaa cttattatat atttcttcaa 180
ttcaattatt attattatat tcaatatatc tagcaaataa cgatcgccca tctcttcgcg 240
ttcagtgttg atgtcaaacc tgttcaaagg agattggaaa cacgttgtat ttaattgtgg 300
tagatatggt gttatcctct tttggtgcga atagtattat ttttgactag cttattgaat 360
aatgacccat aagtatggtt tnaaaaantc ttagcntttc catgtaatta ttttgaaaga 420
catattgaca aatcgttgca caaaganctc ccatttngtt atgaaattca gatgnnttag 480
ttttagaagg ggctatttat 500

<210> 653
<211> 500
<212> DNA
<213> Ctenocephalides felis

<400> 653

acaatgtata ttgaataaga tgtgagcgat accttgnagg gatgtcgcat tgagaattta 60
ggtgtggaaa attgcaatag cattaaaata taccatttga attagatttt taaataatac 120
caaaacaaat cataaattaa attctttcgt agatattcat acataaatat tggctttcca 180
gaaagacaac tatgtagatt tcacattcac tccaaaatta atgagttatt atataacaaa 240
tttttaaata acttatatac aaacactatg cacaagtgtt gatgatatat gaccttaaaa 300
agtaattaaa tattgcgctt aaaaatcaaa tttacataca catttaccca ttctatctct 360
agaaaactag aacagatatt cttatgaaaa atttgctatt ttaaatatct caaaggtttg 420
gaccatcttc ataataaaat ccgtnctcgg cgngacacgc tagcgatctg ggatttcata 480
cctgnggcgg tcggctgctt 500

<210> 654
<211> 330
<212> DNA
<213> Ctenocephalides felis

<400> 654

nccgcancca aataaactcc tcttcataaa ttaatccntt atgttaacaa ttttttttaaa 60
tattganaac aacctttaaa natcgatgac cttaacatat taaagcctta cttaccgcaa 120
aatgaagatc aaattgtgga ttgtctttta ttttttacac attccaaact tttgtcaatg 180
acaatataat attaataatt ttcacttatt ttatgtcaca cttattatta ttattatgat 240
cgaaattaat tttaatttaa taattaatat tcgttgtaac ctcatggaag tatcggtctt 300
aactacgcgg ctttgtaacg ataacaatgt 330

<210> 655
<211> 500
<212> DNA
<213> Ctenocephalides felis

<400> 655

nttctagccc ttgggntttt attcccctaa cggggccncg ggcnngggac caaaatagan 60
cntaatattt ttaatacngg gntaaaagaa anggtaaaan tttgggaggn tttaaaatct 120
ctaacnaggg taaacccnaa acnaaaaaaa taatttaatc attactctct ttaactttat 180
ccntggcctt tataatactt atagataaaa tagaaaatag tttaaatttt nctnaacaat 240
gaattcattg ntttagagac gctctttact gntctatcct acaaaatact atacnagccn 300
agtatttttg aatatgaatt ggatatttct aataaagcta taaatacnta cngtattttt 360
gaatttaaac ntttctttat ggtatcttcc ncagatattt ggtttccata tctacnatcn 420
ccntgaggtt tcttctaatt tcatcaattc agaagacntt tctttttcaa ttttcattat 480
atttgccncn ggatttcaac 500

<210> 656
<211> 73
<212> DNA
<213> Ctenocephalides felis

<400> 656

acaaaaacat aacttcattg agaaaaagca attaaatcat tttcacactt tctaattaaa 60
atgaaatgat cgt 73

<210> 657
<211> 425
<212> DNA
<213> Ctenocephalides felis

<400> 657

acacccctga tattacaagt tgctaaccac tgtcataata ttagatttnt aacaagtcca 60
gactgaaatc ttagaaccct tgagctttag ctgatattaa cggttcatat taacttttct 120
cagactattg cgtagtagct gacattacac attctctcac gaaatcataa acctacatcg 180
acaaatctca gcaagatcat attatcgaca ttccgcctag ttaacaattt tcaccaagtt 240

tgcatgaata acattttgtt taatcggcta aattcattac tttgtccttt tcgttgcata 300
gaatattcga gacaaatccc cactacgttt caaaggattt tagttttaat aattatgaaa 360
taaatcagtc ttaatctgtc ggtcgccggg caaacacttc tctttcaata ccttcgggtt 420
ccttg 425

<210> 658
<211> 500
<212> DNA
<213> Ctenocephalides felis

<400> 658

gatagatccn tttcatgatc aacataataa tacaaaattt taactcaatt tcaccgaaat 60
gcttaaaatg taaaaccttg ccactcgctc gccaattaaa tcttgaacga aatagaaaac 120
gacgttgctc tttcaaatct tgcataatat tattcctaga acacttaata aaacaccgat 180
ttaataagat ttttattgnc attattttgn tcattacatg attttcgtta aaatatatat 240
tttaggtatt tgcaaaaaaa cgacgaaaaa cgacgaaaaa cgccaaaaat atatgaatct 300
ttagtagcca gtaacttgaa aagtatgaag ttttttttgaa aaactataaa atccttattt 360
ttaaaaaaat acgtaattaa acaattgaga agaacccgaa ttgntttcac ttttgaactt 420
ggtgtagatg aacctaattc ctaatttcat gcnattcggg acacagtaaa aaattcagac 480
acaaaacgct cgttactgcc 500

<210> 659
<211> 500
<212> DNA
<213> Ctenocephalides felis

<400> 659

acaacattgt tgtaaataat atgttctcga tccaaaatac aatactgtag ggaaataata 60
tgtgctcgaa tttaaagaaa tgtccgggct cgtgtaaact acatatatga tgaataatat 120
aatcatcaaa tactattggg tataaagaaa atgcattgaa tattccagag tataaataat 180
aactacttta cgctcccggg tgggctcgaa ccaccaacct ttcggttaac agccgagcgc 240
gctagccaat tgcgccacgg aggctcttat cgcttcttga taatagtatg aataaattat 300
ttatagcatg aacaataatt ataattattg aacaataatg agtataaaca acaattattc 360
ttatacccaa taaactacac taatctgtaa cccacggcgc aaccagtgcc caatacctgt 420
catacatcgc gctcgattca cttcacacac aaatagttaa catttacttc actcctataa 480
actatacaaa ctatcaaagt 500

<210> 660
<211> 500
<212> DNA
<213> Ctenocephalides felis

<400> 660

cctttagcga accgcgtcag aatttttcca ggattcctca atttgtttat aaaaatataa 60
taaattttca agtatttcaa ttttatttgt aaaactacat ttttgtatca ttattatcac 120

gagcaagtat tttgtgccgt tgtagaaaac agctggaagc acttaatgct tattgtgttc 180
atgttctcat tactcaggaa tatgagaatt gagaaatgtt tcatttgtaa gtccacactt 240
tttcattcat tagaaagctc tcgtcttgaa gtcgtttact caataattta catgtttatc 300
taaataaaga aatagtagaa acttaaatac aaattggaat aagagcatcg gtagaatata 360
gtataaacaa caaagcaatt ttaaatcatt aaattacaaa ataatcctca gtttctctat 420
tgtcaaaggg atgtgaaata ttttgatgnt taattttcaa aatcttttat ttaattaatc 480
aaaatattat tccagcgact 500

<210> 661
<211> 412
<212> DNA
<213> Ctenocephalides felis

<400> 661

actttttgat atttatgtaa tgattattta ttcaccgatg ttaacagctc aacgtctgaa 60
cgaatactcc aattaaaggt tggatcagcg aattattatt acaaatgaag atactgagga 120
atgactggag atagctgcaa caaactattt atgtaaatac aagcagtaaa ttacctgcaa 180
cagttttaca caaagtattg tattacgtaa tagccggtgg tgattttctg tttcaaaaag 240
ttataacttt tcaagactgc cagtatggtc aagactactg tataaaatta tcaacaaatt 300
atagaaatat ttacaataaa caaggtttta aattttaaat tatataaata aataaatagt 360
ttgatactat ttaacaatat ggttaattat attgctcttc gaatgatatt tg 412

<210> 662
<211> 500
<212> DNA
<213> Ctenocephalides felis

<400> 662

accatagtgg cgcttagact gtgcttagct tgggtaaatt tttaaaattt tgaatccaaa 60
attacataaa ttttttatgg aaaaataatt gtttatcctc aaaagcaatt gttttgtcaa 120
tacaagcaga aagtttagtc aaaattctag tagatacttt ataagaacaa aaattaggca 180
aaataatatt ttgatttttg gcaagtttca agtggtaaat agttacttaa tgcaattgac 240
tccattgtaa aaattggtga gggtaagtta tagtaatagc aaaaaaaagg agaatgaaat 300
atttcacatg tctttacatt gcaaaggttt ctgatcgttt taaaaaaaat gatgtatgaa 360
tgtgtgtgag aagtaatttt tctcattatg ttatatttaa taaaaagtaa aaaatcagaa 420
tttagggata aaagtagttt atgctgataa aaaatctata tgtaaaattt ttaggtatat 480
cggtccagta aaaggtgaga 500

<210> 663
<211> 500
<212> DNA
<213> Ctenocephalides felis

<400> 663

ncatnaatta cttgagccac aatgtataaa tntttcacta ttaagatnta taacttntct 60

ttttcaaatc ntattttggg ataaantaac ttatcataaa ataaaatact gggtaaaagt 120
atggataaaa ntnatatcan caataaattt tcatgaatct atntaataaa atggncaaca 180
tcgntnttta ataaatgggc ttcaaatttg actngaatga atacttgnta ttgcttccta 240
ggtaaaattn atatttttaa tggacaaaat gttactataa tattcttnna tttatataac 300
aagaaaaata attctaaaat caatttcttt gattcacctg ttattttcca ngtgcgcggt 360
aaaganaaaa tagttacnga ataatanaaa tcaaaagtga accggactgt gtgcaaattt 420
tatgatcgag atcttgaaca gagtntttgt aacacgcatc tntggtttag tgccattgaa 480
tantgttatg gtgggggtgc 500

<210> 664
<211> 295
<212> DNA
<213> Ctenocephalides felis

<400> 664

acttatgaca tatcatatta taagtttcta acaataatcc agtttcacta atnaatattc 60
cnggaactat gcttatgaaa aatgaattaa ataaaacatg aattgctaca tttattcaaa 120
atttttgaag cattaacatt agtatacagc atagaatgct acaattagcc agatagtcag 180
aaatcatttc aaaaattctc cgttcatggc tttgttaaca acaattattt taaacggatg 240
ccagttagat tgttttagaa taaaactttt gagagaaata taatataata atagt 295

<210> 665
<211> 310
<212> DNA
<213> Ctenocephalides felis

<400> 665

acttcaaaaa cgcgtataaa tagcgcgaag tctaattttc tagtttttat aattaattaa 60
ttttcacagg cctataatat tttaatatta tgattttgtt tatcatctct atcaccatgc 120
gacgtttcat aaattttgca gataagtata tctgaggttc tccttgtaag ttgtccgtgt 180
tatttaaaac aagtatgatt taatttagca agaaccaaaa ttagatactt ttaaaaaaaa 240
ttaaaaatgt aaaaaaaata tttgcaaaat tttccccata tactattcca cttaaaaatg 300
cccctgttgt 310

<210> 666
<211> 365
<212> DNA
<213> Ctenocephalides felis

<400> 666

acaaagcatc tacatatcgg agcctccagc aatattttcg cataatattt ttctgcgatc 60
gattttctgt caaacgctac tcataaatta taccgacaga acgaacgtag cgaacatgaa 120
aaatatctgc gcaaaatctg cgagctataa ttcaaacttt aaatatagtc ccgctttcac 180
aaacagaaga tcagatcaaa agcttcgata cggcgataaa tcggatatta taccgactca 240
aaacgtcgag aatggagaaa ataatacgat gctgtcgaat ccggtaatag atttgatagc 300

aaagtataaa gttgtgtaat tacaaaccca taaatcattt tctccataaa tatcaaaatc 360
tcggt 365

<210> 667
<211> 385
<212> DNA
<213> Ctenocephalides felis

<400> 667

tggngttttt antgggactt ttnaaanccc cgccgngcag tacctttatt attatgcttt 60
ggttcacgtc gggtataaaa tgttttgaac aagacaaagt ataatataaa taagnataaa 120
atgttttgaa caaaactaaa ttaatatata ccatttctgg gaaccattgc agactgataa 180
gatatcaaat gctattgaaa cgatatgatt tcaagaatca aaacgctttc atctatcttt 240
tatatttggc tatatttgtc attttccatt tatacagtga aagccgacta ttttcactaa 300
aaaaccggtt tttgaattcc acaatttccg caaaaaaccg gttatcaaaa ccggtttttg 360
aaaaccgaca aatcctactt acagt 385

<210> 668
<211> 160
<212> DNA
<213> Ctenocephalides felis

<400> 668

aggnntngng aacccttttna aancnagact tnctttaann ccccgnnagt tacatggntc 60
aaggagncta gccgccatca gggaaagttn cagacatacc gtttatgtcg aacaaatgaa 120
agatggcgaa ggngctgtca aactggaaat ttcanaaatgt 160

<210> 669
<211> 320
<212> DNA
<213> Ctenocephalides felis

<400> 669

atattgcttc tacaaatgca atgaaaagtc aatttgtntt gaatgtcacg taaactaata 60
aaggaactta tagataaatt atagcgacac aataatattt ttatttcata cggtataata 120
tcatcattag tataaaattt tccgaaatac tcgcataaac tgctaatata atgaaaacaa 180
ctcacgtatt ccgcaagggt gtccaatgca tggatcgaca catttatatt gagaacacgc 240
taaatggttg gggcattcac tatcgctaaa gcattcgccc tgaaacgaaa taaaaaattt 300
aatttaacat ttgactgagt 320

<210> 670
<211> 500
<212> DNA
<213> Ctenocephalides felis

<400> 670

```
acaacaagtt caaatcttct tgtttgtgct ataattttct atattcatta taatataacc 60
ttattttttt aaggnaagtg ttacacattt tccaatgaat cactatgtaa tgtgatactc 120
tatttttaca agttgtgtgt ttgttgagca aacatcataa tttttgaaga gacagttaaa 180
tataaataaa aattcttcac gtttagatat cgtatgaaat cgttcgaaaa ataaataaca 240
gtgataaatt tttataaaag caaattgata aaataaagtt tgataaagaa aatgcaattt 300
tagctaactg gactttcagt gtagtgatat catatcatga atttatcatt accgattgaa 360
tattttatca caataagttt tgtgcatttt atttgaaaag ggcttgctcg tggcttaatg 420
attatgacct tggctttcca acaagaaggn taaggcttga tcccatttga aaatcagatg 480
gaaatatatt tctagtgata                                             500
```

<210> 671
<211> 332
<212> DNA
<213> Ctenocephalides felis

<400> 671

```
acaaaaagat atgatttggg tcttatcatt attgagaact agattattaa atttatttga 60
nccaaagtaa atcngcaatc atcttgtgat caatttctac ctctgatatt tttctatgtt 120
agcatttttt atcataacaa atactattaa attacccatt gattattata tggtttcaaa 180
tggtttatgt tataataaat tacatataaa atatataatg atatatcgcg tgcaaaacaa 240
aagttatggt taaatcgcgt gcaaactcta caagtataaa cacctaatta agcaaatgtt 300
ttcaattaaa tgtaaaagca gttttcatat ag                               332
```

<210> 672
<211> 500

<212> DNA
<213> Ctenocephalides felis

<400> 672

```
actactcatg tggtttttct cttccatcca ttgtctattt cgaaaccttc actatcaaga 60
agcacgagag ccttccattt attttgagag ttgaccaaga attgcagtag cagccagctg 120
cagcatattt catagctgat gatgtttttc taatattctt tgttttgcat tgccggtatt 180
ctctgtaaaa aaaaaattat tacattaatt attcaaattt atataaaaaa ttaccgacga 240
atttttttaaa tcatttatat tatttgactg caatactcga gtaaattaaa tttgtaatgt 300
aaattcatca ctgataaaat ttttaaatat tatcaagcag tttaacaata acttacatat 360
atttgatata aagtctttga ttaggtagcc atttactagt aattctagga tttgcatcat 420
acatcctctg atttgcctgg ctttcttcca cagtctcgct tgacgtgttt agttattcaa 480
gccaatccat tgcccaaatg                                             500
```

<210> 673
<211> 500
<212> DNA
<213> Ctenocephalides felis

<400> 673

actatgaaga atgnttctat ataatttact gnattgnagt taactatatt taaatgctaa 60
cgtgcattta tatgttaagn ttagatttat tattgntaat attatatcta aaatgattcc 120
tacatataaa ataaataaaa ctttctgttg ttaaaacttt agaaatgtag ttattttaaa 180
tgttacatta ttgaattaat acaagtttta tatacaatga aatatgtttt aaaattgtat 240
attgntcatt atattttcat gcgccagtaa tagttgctgc aattccttca ccacaattga 300
agttttcttg tagttgttgc agtttgttta ccattcatca cagtagacaa tcatccaaaa 360
gtagattgnt aaccatttcc atgaagtaga aatcaccaag ttagacttcg aatgacatgg 420
cttaattctc ctctgacagt atacaaatct aactccatat gatttgttca ggaccaanga 480
cgcatttaac atgcctcctc 500

<210> 674
<211> 296
<212> DNA
<213> Ctenocephalides felis

<400> 674

acaaatttta gaatgtttaa atcattaagt ttgaataaat taccttaaag atgaaaattt 60
agctaacatt gtaaaagcat gttttgttgt aattttttaa ataacgtatt ttagatatac 120
ttttcgcttc ttaacaagcc aaattatgaa tgattctcat gtttagaaac aactaataaa 180
aaataaactc agtgcaaaca taaacattaa tatagacaga ttaagtagat atatcccttc 240
ataaattaat tgatgtcatt cattattcag ataacacccc tagtcaatag gtttgt 296

<210> 675
<211> 461
<212> DNA
<213> Ctenocephalides felis

<400> 675

cataataatc ttgcaaaagt aatatttgaa atgaactata ctacaaaacg aaacagcccc 60
cacttcctag aactaactcc atctgaaatc gacggaacat atcacttatc attgattagt 120
ttgttttatt atatttacaa atatagacaa ttatttttgt atggaatgaa acgagtttac 180
acatacgaag caaacatgct gaatattaaa aaatatttta tttaagttca ttttattaat 240
cttgctgatt ttgaagaaat gaatctctca cattcataat atgatttttc ataaataaca 300
acgaaataca tattgttgtt taataattat ttttaaccat agaaccacat ttagagttta 360
tttataattc ggcatctatg ttatgtcgaa attttaacac gcagaattta atgaaatcgt 420
aattaactgc tcgatgcctg acaatatcta gcgtctcaag t 461

<210> 676
<211> 500
<212> DNA
<213> Ctenocephalides felis

<400> 676

acttcccaaa agttgncagc gaaggatcct acaagggcga cagcagcttt ggagattacc 60
aaatcaagag ccgnggaatt ttcaacgtca ccatgtatga tgtgacagtc acttggaaaa 120
tcgaaggagc aactgaagaa cgcgatggag aaacttacat gcgcatcaaa cacttccgtg 180
tcagcccaaa agttggtgat atgaaaatct atgcaagtgg cttataccag atgaaggact 240
caataacgca gccgntgcct tcatgaacca atactggcaa cctgccttcc aggcactttt 300
accatacgca gaagaacacg gagaccaaat catgacaaac tttgtcaacg aaatgttctt 360
gagaatccca ttcaacaaat taatgccagn tgaataaagc caaaatttaa atatgtatat 420
aaaaaacata tagtaaataa gcaaaattat tttgatattt tttggngatt ccaaccaacc 480
aaattctatg taggttggat 500

<210> 677
<211> 500
<212> DNA
<213> Ctenocephalides felis

<400> 677

acgtatacta tactctatac agctgtatgt gccacacacc aataatgtat caatatgata 60
caaagaaccg atttgtaaca tttttgtagg gcctttccat gacgtcacgc acatgcattg 120
aatgtttttt ttgttcattt agaatagtta ggaaggtagc caaaaactcg aatccaaata 180
tttatacgta tccctttttt ttagtttgaa ttttgaattt gtatcaaatt gatactaggg 240
acggatgcgg gttaaacaaa aaagatcgga ttagtgatag tttcaaattt agcatggaat 300
cattcggatt atttcctact attaaagacc ctacgagaat aactaactct tcctcatatt 360
gtattgataa tttttcaca aatatatcta cggtaaattc atgtgtaatt aataccggtc 420
tttctgacca ttatggaatt tcgctctctt tgcctaatac tactaattct acttcccatg 480
tgnatcggna tataaaagaa 500

<210> 678
<211> 475
<212> DNA
<213> Ctenocephalides felis

<400> 678

acactatgat cagctcaaaa aataacttga acgcatatga taaactaaac cgnactcaaa 60
attttaatta attaattata gattattggg tagatcttta tctataattt aataaaatac 120
aacttatatt tctgaaagca tagcactgaa taattatctg acaatgcgtt acccatgtag 180
atattttact aatgggaaca atttaaatat ttttaatttc aaaattagta atgcaaatat 240
ttgcatgttt tgtgtttatt tttaatttgc gggattaata atcctggcag ttatttagac 300
aataatgctc aaaaaatatc taaattacaa cttgtttcat ttttttttta aacacatctc 360
tacataaatt aaatacgtaa aatataaatt acatattacg aatatatttt gtataaaacc 420
gacacgccac tgatggcatg ntggtaattc atgacattaa aatgcacccc ggggn 475

<210> 679
<211> 500
<212> DNA
<213> Ctenocephalides felis

<400> 679

acaaagntat taacataatt ggtttaacaa ataattntaa atatgatnat taaacagngc 60
agggnngcag ntaaaattat gccaagtgaa tggtaatttc gagttcataa aatttaataa 120
atgatgcngt catatttgaa gcatgtatga aataaaaggc ctttagagta aatacaaata 180
tcaaactttc tatataggnn acaaaattga aattaaatat tcaaggaata tttctactcc 240
tagtagatgc gtcattttaa caaaataaaa aacattatta attaataacc ataataattt 300
aaaccattat tctgatatcc taggntgcat tgcaatgtca aagctcttat cgctgatccc 360
cgagaaagct ctttaatata catatgagct tgatttctcc ctttgcgntt tataagnaaa 420
tttcaaagat ttgnttcgat attattaaca tttgcaatat ttatatttga tcacaaatta 480
gtgctaaagg tgaaattatt 500

<210> 680
<211> 475
<212> DNA
<213> Ctenocephalides felis

<400> 680

cagcatattt tatacgcaat gttacttaaa aaaatgttac atttttatat aagaagaaat 60
naacattatt atttattagt ctgatttcat ttatcttaat acgatattcc tttaaaaaga 120
aataattatt aattttaatg acaaatcaag ataatatttt gacacttgtt tatacaagaa 180
atcgacttaa gatgacattt attttcaggg attatgagat ttaaaattta tatggttccg 240
aaaaactcag actcaagaaa cattagtgta aattgaagtg attgcaaaaa aattaaaatt 300
gaaaatcttt gaattctgat attttggcat accttaggaa aaattgatgt atgtgtgtgt 360
atgtttgtat gcatacgcgg atacaactgt tcgttgacga ttcgcacgga tcaaccaatt 420
ttaattggcg gcaaaaacga ttagacattt gcacatcgag attttaataa tatgt 475

<210> 681
<211> 387
<212> DNA
<213> Ctenocephalides felis

<400> 681

acacaaaata tgacgagaca cgactttaat aaaaacatat tttgtgcaca tatttttatt 60
caaagattta ttttgaagtc attgttaatg agagtaattg atagtgaaga ttttagcttc 120
ttcataaaat cagataaaac cggtaaaatg aagtatcaag aattgaagaa atttttgtaa 180
ttggcaaatt caaactatgc actcaaattt tgtatttaaa aataaggcgg catcaaaatg 240
taattaaaaa tttcataaat gtttgtaaca tgagtgcatt ttccacagaa tgtcccatac 300
aaaaccggca tcacaaaaaa gacatgtaag acataactaa cataaaatac cnttttctat 360
gagacatcct cataactctc aactcaa 387

<210> 682
<211> 500
<212> DNA
<213> Ctenocephalides felis

<400> 682

```
ncctattaat gttaaaagaa taataatnan gggtgagttt taccgatggg tggagaaagt 60
gtataaatcg ttttngccga tggatttggt taataaaaag aaaactgcac cacctgccac 120
gtntttataa tanttctata atctgcaata aaaatcatct gccatgaaat atgctccaaa 180
ataaaaaacg agtatatcgt gaaaaaatat tttgaaagtt ataacaaatg cgttgcactt 240
tctagtttga ttagttcatg taaaaaataa ttattcacgg catttcaata catgctccca 300
atatatttca aattattcac ccaaataaga aacacnatcg tgacaattgc caaaccattt 360
tagataaaat atgtaaatat atcaaatgat cattatatgt cttaacaaag tttataagtt 420
atcaataaat aagaatgant ttatattatt atatctataa ataagtagac gtgtntatcc 480
accaatgaat ttcaattatt                                             500
```

<210> 683
<211> 500
<212> DNA
<213> Ctenocephalides felis

<400> 683

```
acttcccgta acgtaattaa aaattcgagg aatattacat gttttcaagt agattattat 60
aaatactgta acttatatat gatcacggaa aagtaaataa acaaaattaa tcaatcgaat 120
ttaaatctga ggacattgta taatatttat aagaattata taaactgaag tgcgaattat 180
tttgaatatt cagaacaaaa tttggcaata aaaaaaatcc aaaatcattt gtttcgtcaa 240
cacaagcaga aagttttctg aatcattcaa atcggatgct tagttctctc aaaaactgcc 300
atttctgca catttagttt ggtatctaca aatcggctgg acctgtcttt gccaaaaact 360
aatttctttc tatttctatt ttctgcactc ttttctccgt atcaccgaaa cggctggacc 420
gacttttgcg aaaaactaat cagcacattt ccctatcaat aggaatcgaa tgttttttga 480
acaattcaaa ttggtgatcc                                             500
```

<210> 684
<211> 500
<212> DNA
<213> Ctenocephalides felis

<400> 684

```
taagttagga gatttatttt gttaatgtca ggtagaagaa gatagatgta tgaaaggnng 60
taaggcttga acaatatcaa aaaaattgta attngtattt ttcagacaat agctaatctg 120
tgaaaaaaca ttttttaact ttcccgtagt taatacgata gcattcagat tcattttttt 180
tcatcttctt catcagatta tttagaggga tggccagatg ccanttttt gagaccttta 240
ggtctttagc tcacctttta caattaaatc tttatgactg tttttaantt ttttttctaa 300
attctcaaat caatcattaa taacttgcgt aaaatttatg tctcttacca aatactctct 360
aaaatactac tacaataatt tcatcttaaa aaagattgct tataaacttc gaaatttcag 420
atatatttta aagaactata tatataattg catatgttct gcttacgctt tgagcaaata 480
actaacatca gtaatattaa                                             500
```

<210> 685
<211> 343
<212> DNA
<213> Ctenocephalides felis

<400> 685

```
caaaantgcg tctgagggac tgnntcttaa tttattcata atatcaaaat agttattnat 60
ttgcaaattg ntggtaattc actcgcgttt gtttatttat aattatatct ggntttcttc 120
tcgatttgct atttaacctn tagcagcctt ttgcactttt gctgntggta ataatttcct 180
atccgtaaca acacaatggg aaactgggaa taatccgtcc ttaagcacac atacgcaatc 240
actgtgcatt tcagttctac taatttggtc taccaaactg tttgatacta cactgatgaa 300
gttcttgttt tgcttttggg ctttagcgtg ttggaagntc tgt                  343
```

<210> 686
<211> 436

<212> DNA
<213> Ctenocephalides felis

<400> 686

```
acttcgtaat gctcaattgt aaccgggttt gtattccatg ttagcatagc acttgtgctt 60
gtaacagact taattctaag atttatcacg tcagttttaa tattaggctc acttgtcgtt 120
atattaattt cagcgtaatc tacaacatca ctttctatac gctcggcagt atgattccat 180
aatcctattt catttttaat tctatagcta tttaaaatag ttttaggaat agatttagaa 240
aaaatacgaa ttctgtaagt cttcaaaggg tgtaaattcg ataaagatat tttagtatca 300
gtaaaatatt catcccttgg tttatataca gtattgcggt cgcaatcgaa acattttatg 360
ctataaacaa taatttcatc attcggtttt cttgaatctt ttgtagcatt agcatctttt 420
ggttgtccat gtgagt                                                 436
```

<210> 687
<211> 403
<212> DNA
<213> Ctenocephalides felis

<400> 687

```
accgttcagt gcgtttataa tgaatattat acagaagtag tattaagata gaatttttn 60
ccgcggaccg gaataatata ttaaggtaag gcaaggcata atatttataa gatacacaag 120
acattttgcc ttgaatatta atacatataa tataaagatc actatcaagg tgaggcatat 180
tatttattaa taacttatta ttattttaac gagtttttat gttttcgttt tagggtgtta 240
tagcgaaact ttttcaagtt atcaagttaa taatataaca atattaaggt gaagcatatt 300
aattatattt ataagataca aaataatttt tttacataat tatacttatc ctaatttagc 360
cttattaaat tttagttttt tatttttaga atatttcctt tgt                  403
```

<210> 688
<211> 500
<212> DNA
<213> Ctenocephalides felis

<400> 688

acacatctat atagtaatta gatctataat tataaaattt actcaattaa ataattatgt 60
aaatggtgca tattatcaag atataatagg tttaaattgt gctagagatt gtcggaatta 120
tactaattga aaagtcgatt actttttatt tttatccagg attaaaagta aagtttttcc 180
atagaatatt caaaaaactt ttgcctcaga ttacccttac aaaagacatt tgttaaacat 240
atatcgtttc ttccctaatt tatatattaa tagatcgatt tataataatt aataaatgca 300
atattggatc gagaactaac agttatagaa tttttactaa gtttctgtta gtattttata 360
atgtatattt cttataaatt caaactgata gtatctatta tttaataaaa gttgaaaaag 420
tgactaattt gcattgtgtg tggcatactc gcaggtgcaa aataaattta gtaaaatttc 480
aacacctgtt tatatatcaa 500

<210> 689
<211> 450
<212> DNA
<213> Ctenocephalides felis

<400> 689

actcaatata aatagaagca ttattgttca aaattaattg cactgagtgt tcaaccgcgc 60
gtcgaatttc gctctccgtt ctgctgatgg acgctattgt ggcttataat tctcgtggct 120
ataataaatg attattatat agaaattcag aagagtgctc cgattcgatg cgcatattac 180
tctttgagac ggtccaacgc gaccctttaa agtaatgcct ttcacaatag ggatgcatta 240
aactaattcg ggctgaataa aatattttaa cagtttggga aaataaacta ctaaccgata 300
attatctaca cactatgaaa tatttaaaaa aactaaattt ttggcacttg taaaatgaat 360
gcataattgt tcatttagtg ctttttttatg cttaataatt ttgaaatcta ttagtaatga 420
catgacattg tgctttcaga caaattgctg 450

<210> 690
<211> 351
<212> DNA
<213> Ctenocephalides felis

<400> 690

acattgccct tgatgttgcc gatgttgtga ttgacgtgga atttgggggt cgacagaggg 60
tctattgggg gtatctccag ggtttgtaa ccgtctttga tagctggcaa gatacttttc 120
attgattcct ttatgcattc gttcaaattg gggtcgtttc ttttgcaaat cttgaagaaa 180
tccggtaagg gtgcttcggg gtcgacgaga gctttcgctc ggcagagggc ggtcgtcgcc 240
aaaagcacaa ccatcgccgt catttgtccc attgtaaata aataattatt atgaatagtt 300
tcacggcaaa tcgtaatctc aattagcaat acnatctgct tcctggttgc t 351

<210> 691
<211> 500
<212> DNA
<213> Ctenocephalides felis

<400> 691

accaaaacct agataacata atttatcacg aataaattta ataaaatata aatataaata 60
aaacaataca atacaaatat ttaattaatg catttaatta tatttagtct tatttgtaat 120
gaaattagga aatttttatt atactctacg gaaaggtaat taccctatga gaagttaaac 180
tcctcgagtt cttcgaactt taccacacaa acttcatatc ttatgtttgt tcaaaaacta 240
ctatttcgca ataaaaatca ttttaaaaag aagccaaaaa cccaactcca attcgttgtt 300
tcaaaggtgt cgaaattata gtaatttatt cgccgcgtgt ggcacaaaac taagttttaa 360
aaaaataaaa cagattcttc catataaaaa ttggtctgga taatgacggg cgccgatctt 420
cctcttggta ataaaatctt ggtgaacttt tgccaagaaa gtcaataaaa cgccaattnt 480
tttcattcgt atcattatta 500

<210> 692
<211> 500
<212> DNA
<213> Ctenocephalides felis

<400> 692

acacaatggc aagtaattct tatacataat cagttttcat atatttacaa tttcgtatat 60
tgatagttat agatatttta gttaattcaa tccttttcag ttaaaaatca aatgaactag 120
tttattttag atcacatatt ttacaatatc acattcattt ttttatgttt ttcaaaacta 180
cactcataaa attataaaca ttcataaaat tgctagaata aaaattgtta aatatgtaga 240
taaatcctcg tttctaattt ttacatcttg attaaaataa aaatagttag ttcacaatta 300
tttgtaaatg gaattaaaat aattctaata acagaataaa aactttaggc taatataagt 360
cagtttacaa taaaagttaa aatgttatgt tgctatatta tattatatat attacatcgc 420
ataccataac tacatttaaa cacaaagtat tataatactt agtattaatg aatactaaag 480
tgnattatgc ataatatatt 500

<210> 693
<211> 500
<212> DNA
<213> Ctenocephalides felis

<400> 693

aatctttggt tttcatggta atatatctag gtgaaaattn nnngcaactt ttagacaatt 60
tttaattgac attgctaatt aattaaataa ataaaaatga cttaaagtta tattaataat 120
taataattac acaaaatagc gtattgagtc ttcttctgat ataactttcc aaggactaca 180
atattacatc ctaggatatg ataaaaatca gtgatgataa aatggttgtt tgatcaggaa 240
tcatagtttt gttttacatt ttcaagacat taaggtgggt gctagaatag taataataat 300
ccatagcaga aaataaaaat tatacaaatt tcttttagtt cttcttatga gtcttcaaat 360
agaaaccaat ttatcaaaac caaatttttc aattttcgtg tttacacacc gtatgagaag 420
tgacggtaag tggtcctcag gaagacgtnc agaagtagtg gagctgtagc tggatctgta 480
gctcctggtc atctcnagtc 500

<210> 694
<211> 434
<212> DNA
<213> Ctenocephalides felis

<400> 694

tgaataanta ctagtnaaac ttannttttc gttgttaccg aataaatacc tgggngagna 60
nntanngtaa ttacagactc ctttttatat aaaaaattaa aaatttagtc gaaatttatt 120
tatnttgtga natctatact tattttatgn cnnttttaaa tatttatntt tagaacgttt 180
gcataattat agttaccann ttggaattac ttataccaga antaanttaa aaaaaaacan 240
ttcagtgcct tatgcaagtg gnttataagg gngtttatgt aaacagnaaa atatgtgcaa 300
tatatcaaac taattatctg gcgtagagaa ntgaacagtn ttctgggcaa naattagtna 360
acntttgggt tgatatataa accaatagag gnttatcttt ttgtgtcata taggtggaga 420
ctcgcttgaa aagt 434

<210> 695
<211> 500
<212> DNA
<213> Ctenocephalides felis

<400> 695

tcttgaatta taagtgatca ttatganttt tattattgtc aatatttggg gggtataaat 60
tatgattaac catatgattt agaattttat tcctcggcgt atataaatgt atatttgngt 120
aaatnttgaa ttctaattta agaacaggtt aatantttta ttttattgtn ttcattaaaa 180
ttccntnaat tccgtataag catctttacg actttattca gcgattnttc ctattttttt 240
tatcagctta taaatgtcan ttcttttgaa aagtttatat gattactttg aagtgattta 300
tcaatatttt cttaaatcat caatttttct tttgaaaatc agggctggcg gaaggcggng 360
tctgctaggt ctgcaaagcc ttagggcatt atataaatat gttgacgagt gcatataaat 420
atgtctgaaa aaaatcactc tgcgtattaa ttaatatgga ttaaataact taataatttt 480
taacttccga tggnnagtta 500

<210> 696
<211> 500
<212> DNA
<213> Ctenocephalides felis

<400> 696

tanttcttat acataattag ttttcatata tttacanttt cgnatggngg ggngttatag 60
atattttagt tnatncaatc ctttcagnta aaaatcaaat gaactanttt attttanatc 120
acatatttta caatatcaca ttcntttctt tatgtttttc aaaactacac tcataaaatt 180
ataaacattc ataaaattgc tagaataaaa attgttaaat atgtagataa atcctcgttt 240
ctaattttta catcttgatt aaaataaaaa tagttagttc acanttattt gtnaatggaa 300
ttaaaatant tctantaaca gaataaaaac tttaggctaa tataagtcag cttacaataa 360
aagttaaaat gttatgttgc tatattatat tatatatatt acatcgcata ccataactac 420
atttaaacac aaagtatata atacttagta ttaatgaata ctaaagngta ttatgcataa 480
tatattttta atataaaaat 500

<210> 697
<211> 454
<212> DNA
<213> Ctenocephalides felis

<400> 697

```
acataattct taaataataa aatgtgtttt ntnttttaaa ccaanaaaaa ttttgggggg 60
gntttntata aaaaaaatna cacttatttt ttatttatta tttcaataat taattaatta 120
ataatatacc aataatattt atattttatc agaaattaaa taatttaatt aaatattgac 180
tataatctag atgtnataaa ncaaaattac nctttacaaa gtcaattntt taaaattgaa 240
aaattttnat ttcctagtaa taaaatataa aatcgtagaa ataaatgntt gatagtggat 300
catattggat aatatatgct cattatattt tattatatat gtgggctctt taggcaaatt 360
aaaattctgc ggcaaaanca tattcatcca gcaattcgcg agtttagaac ataatattag 420
aaattatata taacattttt aattcattca ttgt                             454
```

<210> 698
<211> 500
<212> DNA
<213> Ctenocephalides felis

<400> 698

```
gggggntgac aatggngtgt aaanaanagc atgtatatan agntnactaa ttngatctat 60
agngagnggg gnggngaaat ntactggnna gtcgccttan aacgtngnga ctgggaaaaa 120
ttctggngtt acncaattta anagcnntgg agcanaattn nacttttnat cagcntgggn 180
aaatancngn anaggtnccg nattnatatg nnccttccca atanntngcg nanatnttga 240
ttggtgaaat gngatgtgtn tntgtagcag gctcattaag cagcgngann nngtngtggn 300
nattnntnna ntnggatact cnttatactt tgaaaanant ccttanagca tnggtnntaa 360
nannnttctt tnattatntt tnaaggtnaa ggnttaaaaa gcttannnnc gtntatgttt 420
naaaanantt tgntttcntt annngagant aatttatgag tnttatnngn ttntagatnt 480
nttaaanann tnaattatgg                                             500
```

<210> 699
<211> 436
<212> DNA
<213> Ctenocephalides felis

<400> 699

```
attatatatn tgcatgttaa tttnaaataa cctgtctgca nttgnaaatt ngggnggttt 60
ttcgttttaa atggatagtt atttttgtta ctcaaatgtt ttatggagaa ttnctttttt 120
tggatatgtc gctttatcaa tggcatattg gaaaaaaatt taaaatcact tatgtttaga 180
gaagaactna gaaanaaaaa tattatgtta aacagcttct ctgaagtggg atataaattt 240
ttcaaaagca tngctgntta ttgaaacaga agtatcctat gactatgttg caagggattc 300
agttaattaa actcaggtca aggaatacga gagtctgaag atcaatttng aaaaattggg 360
caaaataaaa atatttcaaa agctagtaga aacatctgga agataataga aacgagctga 420
ataagcactg catagt                                                 436
```

<210> 700
<211> 225
<212> DNA
<213> Ctenocephalides felis

<400> 700

cttatttact gcagcatacc atacgttgaa gatttatatt attagttntg ccaaaatttt 60
gttagaagaa aacattttag tatcaggctc atccctacac tccttcaacc aattctttac 120
gaacattaaa tcccccaccc catccaatct caatactaat gtagtatatt ctgnttcttg 180
taatgactgt gcttttcaat atattggaca aacctcccaa tatgt 225

<210> 701
<211> 500
<212> DNA
<213> Ctenocephalides felis

<400> 701

ngctaaatgt gcaaaaatga cagagagaac tangctgtcc gatttgaatg attcaaagaa 60
ggggggggc ttgtttcaac aaaatanttg ctcttggaaa aaaaattaaa ntttttattt 120
tnaaaaaaaa tttttntttg tatatcgaaa atanttcaga ttttaatttt gatttttggc 180
taaagcnttt aaagctcact aatatacnnt ttttnatacc tttattcatt atattgtcaa 240
tcaagactta taaagtctta taatacaaaa ctgcttgctt agataataat aagaacaata 300
aaataaagta aacaaagcac gtnatangta gtagtantta agaaaccaaa cgaaanttat 360
aaantgaagt aaaaaatana aatataantg aaaaaagacg aatatacttt aaataaatan 420
attataactt tgcgattaaa nttaaatant tcttatatat attaaacaat ggtacagtat 480
ttatagtaat ctatttaaaa 500

<210> 702
<211> 494
<212> DNA
<213> Ctenocephalides felis

<400> 702

catggcatcg tgccctaatg caaattgctg gaacttcacg caccgtaatt aaaagggggg 60
ngaaataaaa ataatcaata actgttttaa ataatttcag aaagatcatc ttctacatat 120
ataaaaataa gtcgggtttt ccttcctatc gaaataactc cagaacgcac gaaccgattt 180
cgacaattta atatttcgct ggaaaggtaa caggctccgc aaggtttata gctaagaaaa 240
ttagttgtgc atggcatcgt gctttaatgc aagctgctgg aactcccgca ccgtaattaa 300
aaattcgagt aataaaaata atcaataact gttttaaata atttcagaaa gattatcttc 360
tacatatata aaaataagtc gggttttcct tcctgacgaa ataactccag aacgcacgaa 420
ccgatttcga cantttttgta ttccgttgga aaggtctncg gctncgcagg cttatagcta 480
agaaaattag ttgt 494

<210> 703
<211> 499
<212> DNA
<213> Ctenocephalides felis

<400> 703

caataaagaa ttggatttan attatttaca aggtcatttt ctctattcng gngtttgtat 60
gactccccgt aaacaagatc aagacctcct ccgcaggtat atcatgtatt gaagagaacg 120
tcctcaacat tctcgcagca tctttagttg agactagaga aaatgcaata tcagaaataa 180
gacatttatt gtaattaaaa tttattaaaa tctatagcat acaaagtatt aaaattaaaa 240
aagtttgacc agcaatggca tacacattac ataataaaat tgcgttaaaa ctgacaatca 300
aattaaatca attattcatt agcggagatg aagaactcca ccttttttagg tttgaaaact 360
tcaacacgaa atttgacgag accagtaaag aattcagact ggaaatttgt ttcagtcaaa 420
cagattttgt agttggcagc attttcaatt tcgtctcatc atccaagaaa tctccatttt 480
ggaagcacct gtgttgtaa 499

<210> 704
<211> 376
<212> DNA
<213> Ctenocephalides felis

<400> 704

aagtattgac gaagttaagc acaaattact caaaaatttg ttaagaaaca tattngtttg 60
taattttatt ccaaaagtat atacactgga gaatataatt tcaaaattat aattgtttat 120
cctgcatagt aagtcctgac gagctcccaa aatatgacga ttaatatatt acttaaatta 180
aatatacatc gngctgtcgg caaagttaag tacaaattac tcgaaaattt gttaagaaac 240
atattttttt gtaattttat tttgaaagta cacacaatga aaaagataat atcaaaactg 300
taattattta gtctacccgg gacgccctga agagctcctc aaaattcaca agtgcaatat 360
aacataaatt aatagt 376

<210> 705
<211> 118
<212> DNA
<213> Ctenocephalides felis

<400> 705

ttctgtataa gtaatttggt tgtaataatg ttcagtgtta acgaacnggg tgagctgcat 60
gagcaatgct ctacccaact gtaaaactct aaatgttgtt aattcgaact acgactgt 118

<210> 706
<211> 289
<212> DNA
<213> Ctenocephalides felis

<400> 706

taaagnttag tttagattta aattcattta acttgnattt caatgntatt attattgcaa 60

aataaattct tcaataatgn anatcaacaa atttcctttg tgttacaaaa tttgnatgta 120
ttttttaact ataataagtc ttcaatattt ccaaagcagt atgttctaca atgnaagtaa 180
tttaaaataa gtattggtgn aaaattaaaa taaatgaaat aattaaacaa taaatgcttt 240
atttttaatt aggaaatcan atcaccagta tcatctctaa gaaaaatgt 289

<210> 707
<211> 202
<212> DNA
<213> Ctenocephalides felis

<400> 707

aaataatatg cgctcgaatt gaaagaaatg cccgcgctcg tccgggattt gaacccggga 60
cctcccgcac cataaccgga aatcataccc ctaaaccaac ttttggattt gccacttacg 120
tttaatatct aagccgaaat cataacccta gaccaacaag ccacgagttt tatccagtgc 180
atgtttgtgt tggaaccaat gt 202

<210> 708
<211> 300
<212> DNA
<213> Ctenocephalides felis

<400> 708

ttggtatagc ctttccagaa ttactcatat actgngaaat acgngacngn tgtaaacttc 60
ttgcaacaaa gcatatatgg aaatatgttt tctctttaaa tgactcttta aattggcgtt 120
tgntgaacta tactttatct gctggntgca tactagttga ttttcaacta gttggttcaa 180
tgcccatcac tagttaaaat catttatgat attgaaaaag attaaaagtt gattatacta 240
aattattata tagccctcgg ctattttcgt gaatatttag tgagtaaaag cagcaaaagt 300

<210> 709
<211> 500
<212> DNA
<213> Ctenocephalides felis

<400> 709

acattgatac tactttgaca tgtcggaant nttttctcag tttgttgntc ttttgatngg 60
ggggngtcac aatatttact tattaaataa ttntgcgcta tatctaaatg tatctttaaa 120
attaatattt tgcaaattag caataactat caatgagtag atctgctcat agtaagtagt 180
atatattttt atatcgaatg ttagtgtaaa tgaccgtttt aaatttacag ttctagaata 240
ttaaacgagc tttgtgaaca agggtgaaag atcaaaataa catcactttt taaactgtaa 300
cgttgacaat attatgttga aatgatcaaa ttacataaat gagtcagcaa aatttgttgc 360
taatattttg ctcggatttt tttgaagaat ctagtntata tttggtatat atagatatat 420
agatgtgncg tatacctata tgtgcgtgcg tgtgtgccaa attaacaata tattngccaa 480
agatatgtat atgccagtct 500

<210> 710
<211> 425
<212> DNA
<213> Ctenocephalides felis

<400> 710

accaaaatat ggttaattat tatatattaa attttttta gttaaatagn ttttgtatgc 60
ggnggnaaac atgctttgtt nggaatgcna aaatagtgaa cacaaagctt cattcngcaa 120
acaaatagaa gttgtgtatt gacatatctt gatttcatct taccatttac ttaaatgttc 180
ataataatat ttgcaagata catagcgaaa aaaatcatta gctatagcac aattttcata 240
agttgttaat tcatgttatt tttattagaa ttcaaatttt atcataataa cacgattaaa 300
taaaataagc acaatgattt tatcatgtat accaaaatgt cgcttgaagg taatatctac 360
atcttaaact agaaaccctc attaaaaata taataattag atttagtatt tgttgaaata 420
gtagt 425

<210> 711
<211> 500
<212> DNA
<213> Ctenocephalides felis

<400> 711

cttcgtttac caaaaaatcg ctaattttnt ttttacaaac gtcaaaaaac tactcanggg 60
gntcaaactt tcataaattt tcaaaactaa actacttgtc gtaaggccgt aatatttcag 120
taagttctat aagaccatga tatataaagc caaaaaagta tcatcggctt aaattttttt 180
tcaacagtta attacaagaa aacaattaca tgaaatcttt aagcgctcat aacttttgaa 240
cgatttatta taggatattg atgttcccag gtgaaatatt ctaaatcaaa agatctttaa 300
tccagtataa aaagttttaa gatacctctt atcgttcgcg agatatacgc caaaaacggt 360
ttcaaatttt taagtttgaa ttagttaaac ggctatagct cgaaaaatat tcatgacgta 420
tatgaatgaa atatgcgtat ttaaaatatg tatctgatgg tgcaaaaagt ttgtaatagc 480
tctnccgttc cgnatatttt 500

<210> 712
<211> 499
<212> DNA
<213> Ctenocephalides felis

<400> 712

ctttgcttca aatatcgaag acatatttnt gaagaaaatc ttggcacaca ttgannngnc 60
nctgcacctc gcatgannnt cacttgaata ctcttgatga aagagtgaga aatagaaata 120
atcttcttgc aaangttcac tngcatggag aaacctgaaa tgcagtttat agaaatatat 180
tccacgcagt gaaaccantt gcacggacta ataaacgcaa cgntttcant tacgaccgca 240
nttcacaaaa aaccggcgct taaaantata ctaactattt atatgcctng gaacgntctc 300
gctttaagtg cgaancggca tttatcantt ggggcnctta aatgaaatct taatccagga 360
taagggtcac taattacgca tggnaacaaa acagcaaatc ggtgacgagc ntaataantt 420
aaaaaaaaac atnacaaccc ccggtatatg ttaancnaaa aatntanaaa agcctgaaan 480
ttggggganaa attaaaacc 499

<210> 713
<211> 500
<212> DNA
<213> Ctenocephalides felis

<400> 713

ntattggctt agaaagagga anttaaatnn tttnattact aantatatca attaagangg 60
ggngnnagna ggngtggtag naataaacat tataatcaag gcgtnttgaa ttaaaagctc 120
atattgntag aatgtannaa cgcgcnatac cttttctttt tttagacaaa aatttataac 180
gtaatatant tatnttaaaa atgcaaaaaa tatttaaagc gaaaaataaa caactaactt 240
catatacata tatgtatata taaggatctt gcnagggcaa aataataaag attctgcaat 300
aacaagcata gcttctgttc aataaccgca ttataaatgc cnntttgnta tttattacgc 360
natattagca aaaatcacta tgtgcgnnaa nntttcttaa ataattaact ggcgngcttg 420
cttaatatct ataatanntt taacatcaac aataatgnnt anttactnaa ganttctaaa 480
acanttatta gaatattcgt 500

<210> 714
<211> 500
<212> DNA
<213> Ctenocephalides felis

<400> 714

acgaattaca tttttgttat gtgtagaaag gtgtcaaaca nttcatctct tgatcagaaa 60
tannttttct ttctagtaaa tggatccaaa angngctaag aaagaagann ttcctgagat 120
tataaattct ctgaaaaaaa natacntttc cnttatttat ataatataaa ttatttagat 180
atatgggatg acttctagaa aaaattaaag tggtcaggaa tggngncatt gaaagaaatc 240
ttnctatcgg tatgntaatt acatcaaaga ttaaacataa ctncttaact aattcatact 300
cctatcaaaa tatcttttag ggatcgatat actggctctt ttctacngac atggttatct 360
tggaaattga tggaagtaaa ganatggatc taaataatat gggcctcggc cgggacaccc 420
taaccgnatc tgagatatca atacctggcg gcggtcgagc atgcattana nggnccaatc 480
gcctatagng ggcgnataca 500

<210> 715
<211> 500
<212> DNA
<213> Ctenocephalides felis

<400> 715

actcatttta tatattctcg gngggataaa aatataatta attantgtng gccttctacg 60
ggngtgcttt tagaaaaacc aaaatctggt ggggggggctt tttgacccnc cnttttgagg 120
ctttaaattt aattctttat tattatggaa aanataggtg gttnaaatca tgggnttaga 180
caacatcggc ggttcattta agtatattat acattttggn ccatcgcacn gatncagtga 240
ttaaaaaata cagtcacgct ttacaattaa atatcgcgag gtcgctaaaa ataaagccaa 300
caaagctcta cgtgaatgaa aagncgggac ctgagaagcc gcntttaagc ttggaagcgc 360

gctggagtta ctgggattgg accactacac ttaaagacac attgggaatc caagtagtcc 420
ccattagcta aacncttcaa aatgctacta taatggtgga nggtangctt ggctnggcgg 480
gacnccttag cgatntnnga 500

<210> 716
<211> 500
<212> DNA

<213> Ctenocephalides felis

<400> 716

```
actaaatgaa ttacaaatta tccgtatata tttanggaat attttacata tatggacang 60
tggggnntgg aagcttattt ttgntnttaa tattaactct tttatttaca actgtccagt 120
aaaggacatt tagtaaattt aagnnnttac aatagtccgc cgagaaggga cattgtatac 180
tacattgtca tgttggaaaa aacaactcag tantcgcnct tcctttctat ataaaattca 240
caatanttta ttattgaata attacttata aataatgatt aagttatttc tattnttttt 300
ctcaggagag ttgatagctt acatccattg gcgcttaata aatgataaaa atgnataatc 360
anttgatgaa ataacttcat acaattatta ttacannntt taatggaagc ttaatgaatg 420
ataaaaanta taataaantg atgaaatact tcatacnata ttattacaac cnctttattt 480
cattctacac tgggacgaac                                             500
```

<210> 717
<211> 363
<212> DNA
<213> Ctenocephalides felis

<400> 717

```
tattatatgt attaaatgaa accttttntt tnatacctan ntttagnggc aaatgggggg 60
nttngaaata aaaaggcttt ctataaatct atcgtaattg ggtcatcatg aaaaaaaatt 120
gattccatat atactacnac caantcgant ngcaatattc acatatagct ttcgatatgt 180
ccaaacaatc gaacatatat aatatttant tcatatttag gtgatttaat tatnggtaaa 240
aagggtgtgg catgtttgaa gagatatgta tatcgctata caattaaata tccacattat 300
tatataaata taattttttg ngcttattta tctttatcac tttcagcttt ctttccaatt 360
ggn                                                               363
```

<210> 718
<211> 500
<212> DNA
<213> Ctenocephalides felis

<400> 718

```
ggncanttta ttntcatttt tatggataag ntttntgaaa taaatagtca acagaaccnn 60
ggggngagnt annnccttta ttgcttggaa atctatctat acaatagatt taaatanntt 120
agttccngnt tataangtaa cngcnggncc gncttcaaaa atatagaata anttcgataa 180
ggactagcgn ttgtcanntt tgcacaacaa tcagccatct cngtaaatat ccgaaaagga 240
aaaaaangnn cgtctacgcn tnagcccaca nttctanatc gattttaaaa atacaaaacc 300

gttcgaaaga tattgggtgg gaatganatt gattancctt cngnaaanat ctcttttatt 360
gacagnttct tgngaataac tggcaaaaaa acncttgacg acnttatgaa actcctttcg 420
aananatatt ataggctcaa tggatatcta aaatanaggg cctcacanac ggttgcttaa 480
aanttataaa ggggaaaant                                             500
```

<210> 719
<211> 353
<212> DNA

<213> Ctenocephalides felis

<400> 719

```
aaacagaaaa atatcaaacc tttcatntnt ttcaatacat anttatctac aagngnnggg 60
ggggngaggg nngngactac tacaatacca tcacttttg cttttaattt tattccaant 120
acatactang agcngncttc nncgnctcaa tgcctcattg aatccttcgc acaaagttaa 180
atcagatcgn ccttgtgcgc attgnaagaa ttgcatgatt ncccatgcgc aagggccagt 240
agagttcttg gccttggtaa gcagnacttg cgnangcant ngntgagggg cttgggcagg 300
tgctgcttca ctggaggaac caccagagaa gaaccagtta taccggnttc tac       353
```

<210> 720
<211> 500
<212> DNA
<213> Ctenocephalides felis

<400> 720

```
aaaaagttca gaacttccaa aattttttna aataaaccct tcttcaaaan gcnggggggg 60
gaagaaaagt aanggggggnc tcaaaaatct ancnttttttc gaactaatan ttgcagaaga 120
ttcggcagca ttcacggccc ngcccncanc ctttcagacc aaaactgngt tcattactaa 180
aacantnatg nancttcacc atgttcaatt attctgaaaa tanttatgca ataaagaact 240
gcanttcatc cagattctat tacaatcagc tgctcctcgt ggaatctcaa taccataann 300
cttatgnggc agaatgaagc gcgctcgnan ataaatgaan ttacggccgg tatttacaan 360
tttaactcga cantaattaa aacggattca tgtnccgaga aacttggaat cttacatata 420
aanagcggnn ctgngtttat ttcgactctt aagnaacntc attgcgctaa ccctagagag 480
anatttatgg tgactnaana                                            500
```

<210> 721
<211> 500
<212> DNA
<213> Ctenocephalides felis

<400> 721

```
actttgaaaa tttccaactt atttcatttc ttgnggcata gttatcaaca gagagacttt 60
ctgggagaaa gagccgctcg gggcacgcag ctgttttcgc tgcttgatat tttctacatt 120
gtttgagtct cttatttcat taacaagcga gttcttcaaa ggaaattttc gcagcttgca 180
caattatatg atgtctcaac acacgacagt caataatctt cttgaaatta ttaacactat 240
tctatcaacc gaaaatatat atttatacat aatattatat gacatgatat aggaaaaaga 300
atttttaaat attttttcta atatagagta ggacggtgca caattcgaac ttaaggaact 360
acatgacaac acataacatg tatcaaacta tctactatcg aactgtgttc aataaaatgt 420
ttagttaatt tctaattata tcctttcata ctatttttct aaaatttatc atctgtcatt 480
tttatcacat aggctgagta                                            500
```

<210> 722
<211> 500
<212> DNA
<213> Ctenocephalides felis

<400> 722

```
acatcaattt gtcgctccaa cgagcaaata cnnacaagcc agcagtagac tcaatagaat 60
gtcgagtgat ttcagataca atacactttt gattgatttc caatagaatc caggtcgaac 120
attttgcaaa acaccattca atagaatttt gaattcgttt gccatcgagc caataatgag 180
ttcgctgatt ttgttttaat tatcgacgtt attgaggaat gttaagactg ttgacaaatg 240
tgattagaat cttaactgat atatagttaa actatatcga atattcatac tatttgtata 300
gacagtgatg tgctgacacc caggatttca cgaattttaa ttgtaaattt aagttgccaa 360
atatgcattt ttgtgggtaa ttttgagggt aatttattgc ttttgtttct atgcagagac 420
aagaataaaa tgattcaaag cgtaaacacc aataaaatgc tactatattc ttacttggat 480
attcttattt ctattattgc                                             500
```

<210> 723
<211> 151
<212> DNA
<213> Ctenocephalides felis

<400> 723

```
acaagtgtta ataagagcac gtttgatgga tcnactggtt aatagaaatg ttcgtgaaaa 60
tgttaatgtt tactaataaa tatatatttt actaatgcaa aacaaaatta tactaataaa 120
aaaataactt atgtatcact tgatataaag t                                151
```

<210> 724
<211> 167
<212> DNA
<213> Ctenocephalides felis

<400> 724

```
acgcattgtc taaattgtaa taaaaatgtt ntttaataga attgttttct tagacagaca 60
ttttaaatgt atttcgaaaa tattactgtc acatgtgaat ctgtgaaatg tgtcggtttc 120
attgccaaag tcacatgtaa tgtttgatac atttacagat tcctagt               167
```

<210> 725
<211> 381
<212> DNA
<213> Ctenocephalides felis

<400> 725

```
acaaccgttg gcaaatatca acttccacat catnaccaaa tgttttatcc caatcaggcg 60
atgaatccaa atgattgaat tcattacact gttcaggctg agtgtaaaat aatgatgttg 120
gactttgagg cggagtaaaa tgatttagtt ccactttatc ataaatgcgt tcaaattctc 180
gtaaaagact ctcagtgtct tgttgaataa aatctgtggt atcaaattgc tgaggaaatt 240
tggtttctcc gcttagagtt ttgttcatac cagttgtagt agacaaatcc tccagcagag 300
gcaaatccac tttctcctcc agccattgag aaaatgcttc attagtaaag ttatcttcca 360
gcaagcacga atcagcaaag t                                           381
```

<210> 726

<211> 424
<212> DNA
<213> Ctenocephalides felis

<400> 726

```
acaaaattta tattataatt caaataaatt taaaaaaata ataatctgaa cttttatatg 60
agcaatccca tgttggaatt atgttgcaac tatttgaaag aaggcagcta aattaagtta 120
agttaattga agtttggaat tacgttgaat ataattttaa taccttttaa gacaaaacgg 180
aaactttcac atgaaacatg ctaaatcgag taaaggactt gtaagtcctc tgagaagtgc 240
aagcagcctt tacaataaac taccagacga actaaaaaca atgacagacc taaacagttt 300
caaaattaaa ttgaaaaatt acataagaga caaatgaaac aaatgagtta aaatccctat 360
tttaaatcta tattanttnt gntntgtaaa atattaatca caattnagta tnagttgtat 420
gntg                                                              424
```

<210> 727
<211> 488
<212> DNA
<213> Ctenocephalides felis

<400> 727

```
acatacatac acatacacag acatccattt ttntgatgta tgccaaaatg ttcagaaacc 60
ttcaaaacaa aaaaagatcc tgaaaaatta tgaggaaaat cacacccaac aaattgatct 120
tttttatgat ttcaaaaatt aataatacaa tattaaatta tacactacct aacttatgtt 180
ttaattaagt atattttgtt ataggtaggt aatagttttc gatctttgac ctaaaataat 240
ttttgaatat ataaaaatta tagaacatat gtgtaaatct tttaaaattt tgaagacttt 300
tttaataaat cgcaaaaaag tggatattaa atttagttta taatctttta gttcttgttc 360
gaatgttaaa ttaaattttg gcaaagttaa gtttccaagt catttttatt atttattaat 420
gaaacaatat tagctttttt ggtaagaaaa tcggcacttt ttggtaggtc aacatttatt 480
cttgtgag                                                          488
```

<210> 728
<211> 290
<212> DNA
<213> Ctenocephalides felis

<400> 728

```
acttttcatg atttaattgt atattacctt antaactaat ataaatatat ttattagggc 60
gggtcaattt aaaggtcact taggcacttg tgattttcgg attctaggga tcaaaataag 120
atactttgct gaagaaacca cacctctaaa atgaattctg atgtccggtg cattgactca 180
ataagggttt aattttgaaa aacctctcag ggggggtccc agggtttgaa ccagaggcat 240
gagttgattg gccttctaaa gttagtcata catttggaag caattggggt            290
```

<210> 729
<211> 500
<212> DNA
<213> Ctenocephalides felis
<400> 729

accgaactat acataatcca agaataacac anagaaaaat ggcagaaatt ttccaagtca 60
tngttcaaac tgtttccatt tacaaacaat ttgaagatat atcaaaataa ggttgacaat 120
atctgacaat gagtgccatt atctgttttt tattgatttt ttgcagtgta ttatttctga 180
tcaaaggtta aaaaaagttt atcttgagaa tcttgttctt atactagcac gttgtagaca 240
aagtaaatat ttcattttta tttaacaata ttaaacctgt gagataagcc aattaaaaac 300
tgctcatata gataatttat gatttcacgt tttaacaagt ttaaccaatc tattcgaaat 360
caataatacc tttaaatgta aaaaaatata aaacaaagaa atgtaactgg cttgatttat 420
ttgctaaaaa cagctgataa acggcaaaat tcttcgaatt tgcacttgat ctatataaaa 480
atatttcggt tttagcaata 500

<210> 730
<211> 500
<212> DNA
<213> Ctenocephalides felis

<400> 730

acttcaagta taacccaacg attcacttcc ctntgtgcac tttggcataa atatccccta 60
aaatatgtaa aacaggaacc agggcaccgt gataatgtaa tcaaacgccg gaaaatgatt 120
tttgatcgta tccatacctt tatagcgaag tcgggaatcc ttgtgattat attttcctaa 180
aaaaggaaag aagttctcgc atactgaaaa gaaaagtttt gtctggtggt ccgatttcga 240
actttcaaac ttattcattc ggttaccgcg accgcgaatg aaatattatg atttttcttg 300
ctcttgtccg gaataagata gattgacttc gaatattacc ggaatataag ctggctgcga 360
gtaaattgtt tggatttgtg gcttttantt atcgataaaa tatgngcttn ttngagnatn 420
gttggaanct cnngggtant nttcaggtcc catggntacc nttagnctaa ttattaattt 480
ngtagnncca ccaaaccatn 500

<210> 731
<211> 256
<212> DNA
<213> Ctenocephalides felis

<400> 731

attaaagtaa attaaatata aatgagaaat tgtccttatt aaattattca ctatttttta 60
aatgnncttt ggacattctt ttataaaaat tttaacaccg tttagttcta gtattctaat 120
gtcagagaaa gtaaacaagc cgagtgctct ggatttttat ctttttagtg catcttgatg 180
taagttagct tcatctttta tctgtataat aaatgataca ctactgaaat atatttctat 240
gtaattattt atatgt 256

<210> 732
<211> 500
<212> DNA
<213> Ctenocephalides felis

<400> 732

```
acaacctggg ctgtgaaagg tgaacttgaa gaacgtgatg gtgaaaagta tatggttatt 60
aaggangctg atatgtcgcc agttccttca aatgttaata tttatgccac aaatattttc 120
cctgataaag cagtcaatga cgctttccta gtgaccatga atcaaaactg gcgctcctta 180
tacaaagagg cgctcccaat attgagcacc ctctggggac ccccaatcaa aacaggaatg 240
aactacgttt ttgaaaaaat acccttccga cgtctgttcc cagaatccta aataaaaact 300
gatagtgcaa actgaatgct tagaaacaaa tatgtgattg taatgatagt tattagaaca 360
tgatataata tgttagaata gtggttaata tattatgagt atataaattt ttacagatgt 420
taatataagt atatgttaat gttaacaatt taatataaat tttgtattta tatttatttt 480
gttatatacg tgtaaaaatc                                             500
```

<210> 733
<211> 500
<212> DNA
<213> Ctenocephalides felis

<400> 733

```
cgatcaggcg tatttcacat agaaaagcaa gaaagaatgt tatgataact gcatcattga 60
agattcttct tattataatt ttatttgaat atttttgatg caatattgta ttaatgcgat 120
tgtgatacct ttttacgtag aataattatg tgttgtctta atgtcattga tttaaaaagt 180
aaaaatcact gtcttattta aattagtttg attctactga tgacaaaatt actctcctct 240
gctaaaattt tatattactc tctgctgatt tttatattac tacttctatt ttatgattta 300
attttaattt ggttttaaat tagaaaagca acgacttagc ccaaaaatgg tctccactga 360
cgaacgaaat tactcttcgt caaatacaag tattgttaac actattttta ttctgagatt 420
atttttaaat ttgaatttaa ggtgataatg aactataccg acaagtttca gattcagatt 480
tcagttacaa tagaactcta                                             500
```

<210> 734
<211> 251
<212> DNA
<213> Ctenocephalides felis

<400> 734

```
actcttccca cagtgagaga aaaagtttaa gttctattca tcggtcgtct aaggaaacca 60
ctcatcagca gattgaaact caatctaatg cacgaaataa acaccaagta tcatcatcta 120
acgctaccta cgttattgaa cgcccgcaga aaaccgttcg acgcgataat ctgttaactg 180
gcggtgaatt ttatggtcaa aaagattcaa ggtatggtaa tttttctaat tgtgaacaaa 240
gtctaagaag t                                                      251
```

<210> 735
<211> 229
<212> DNA
<213> Ctenocephalides felis

<400> 735

accagtgtta tgttatggtt atgttatacc gtttaaaaga cttcaatttc caatatagtg 60
ggagaattga gactcatcat tttataaagc aattctgcat ttagcatccc ttatattata 120
aatatatatg tattttggtg taaagagtaa tttaaaaaac aataatcaat atttttaggt 180
aaacgataaa tattagtata ttacattata ttaaaaaaat gtagagggt 229

<210> 736
<211> 333
<212> DNA
<213> Ctenocephalides felis

<400> 736

acatacgagt atactaataa aatagatttt agaactaata gaatcttatt gcgatatata 60
tgtgtgattt tcattataat gcataaactt tgttataaac gagcatagtc atttagatag 120
tattcgtcat ataatcaaat ttcttctgaa aattgtctta agcgattcct ccccttaaca 180
agcgaaagtg ctcgaacgaa attactactt caaaacaaat ttaaaagata aggcatatag 240
tcgcagaagt tcgacaatga aaggtagatg aaatagaaat gatataggga aattaataaa 300
atcattaagt aatctttaaa gggtgacatt tgt 333

<210> 737
<211> 197
<212> DNA
<213> Ctenocephalides felis

<400> 737

acttctcaca tgcaaagcga gcgctctacc aatagagcta cgcccccgac aagtgacatc 60
attttattcc aagattctac gggttttata cttaacaatc actcacatta acatcacgaa 120
gacatgaaaa agtaagattt ggaagttacc aagatataaa atagtaatat ttggaagcac 180
cgggtatcga tcccggt 197

<210> 738
<211> 354
<212> DNA
<213> Ctenocephalides felis

<400> 738

acagaccttg agatacgggg gcaattcttc ggcttgaata atactgaaaa acgtcacaaa 60
cgttaaaatc actacaatat tcgtgatcgc cataacgatg ttgtttctgg atgtgtctgg 120
aagtcaaagt gcgtgatttg aatttttgtt gcgattttgg ttctgtatcc ttagataaaa 180
tctgcgccct tgaccgtaat gccaaattca agatgataaa ggacaaagat gtaagtcgaa 240
attatcactt ctagagcact tgacatccag ataaacaatg acaggcctgg aaagaatcgc 300
ggtttactta tatgagaagg atgcgtggct gatgtgtcac cttgtccttg gttg 354

<210> 739
<211> 500
<212> DNA
<213> Ctenocephalides felis

<400> 739

```
actcttgaaa gtaatgataa agtatcagtg agaaaaataa aaacacagca atcaatatta 60
agttacaagt gcaaattgaa gcctgaaatt agctgaccag tattgaaaat agggctcata 120
attttatgtc aagtcagtta agagaacaca taatctatta aataaaataa aattcaaaat 180
aaaggttaaa ttccaccctt cttttaacac tgctaatgtt agtttgggat cgactaatcc 240
acaacaaaag tttatgagta gcaaaaattg ccttttgata tttcacgaag aaaacatatc 300
taataataat tgtgcgtata gtttctaaag ctaaatacca tattctgtaa tcatgtatgt 360
aatatataaa aatattgttt tttaaaaacc attttttttt aattttgaat tcgaacgatc 420
tatataatca tagataaaca agtataaata ttggtgtttt ttcagaattt tatatttgcc 480
ccacggtcga cgctagcgcc                                             500
```

<210> 740
<211> 293

<212> DNA
<213> Ctenocephalides felis

<400> 740

```
accaatattt ttactcaaac ccatgaatga aatcaaagaa attttcaagg acaaagaatc 60
tgacatacga agatcaatca gcccaaatcc acaaactata gaaagttaat tatacaataa 120
tcaaaattta aaaaaaatta taggtataat tgaaacatgt gctcacactt atgatcgtta 180
agcacaatcg accaaagaaa ttatgtgtaa tttgtattta aactaaactg tatttaatat 240
atgttgtaat tacgaaactt attatacttt ctagtcaaat ctaaacatgt tgt        293
```

<210> 741
<211> 124
<212> DNA
<213> Ctenocephalides felis

<400> 741

```
actggtggag ctttcgtttt ggtcttgttt ttactttttt ttataatatc atcgaaagcg 60
ctggacgctc cattctctta aaaactcaaa aactaaattg cccttttttgc tctttttccc 120
ccgt                                                              124
```

<210> 742
<211> 278
<212> DNA
<213> Ctenocephalides felis

<400> 742

```
accagatcta agttatttac agtgtaatct ctgtaaaaaa tgcacccatg aagtttatat 60
cttgaatgta agttgaataa tgtagctatt atttgtaaaa tccctttaaa attaaaaata 120
agacttggtt ttccatgtct tttatcaaaa atgtttcaat attgataaaa ataatgttaa 180
ataatcagag tcatataaaa tgagtttcta gttttcttct taagatcttg aaggttttaa 240
taaaattttt aaaaaattat gcgcttttta atctgtgt                         278
```

<210> 743
<211> 500
<212> DNA
<213> Ctenocephalides felis

<400> 743

```
accatcactc catgagttcg acaaccaact agaaaagacc catcaaccaa ttgaaaataa 60
attctaacaa ataaaaaaat attaatttat aaataaaata ttacctgccg tttttccata 120
ggctaaactt atttcgttta aaaaatcttg cactgttttc ttaacaccat cacttgtaaa 180
aggtgttatg agcctttgca gatatgagcc atcctaaaaa taagatcatt tatttaacaa 240
attttattta gggagcattc tagtctagga gtcaattttc aactttctga aggagttgtt 300
tgttttctcg aaaaaatccg atttgccaat tttatttttt ctcaacgttt caagtcattc 360
tgaatcaatt gagaccaaat ttgaaaaaat atgtgtatgt tttcatgtat gtgagccgat 420
ttttcgtttc gttttctcaa aaacggttga accgatttta aactagtggt gcatggcatc 480
ggccttatgg caaattgctt                                             500
```

<210> 744
<211> 500
<212> DNA
<213> Ctenocephalides felis

<400> 744

```
accagtntgt gagatnaata aactctagcg tntttcgaga atatnngcta tgatctttgn 60
ggggtaatnn actngtttaa taatnnctta tgcattacaa atgttaaaat gaaattatta 120
tgtntattta ataagataca tanaagcaat ttatcttana aatttataan ctantctact 180
aaatactgaa atatgaattt cctantctta agattttact aaatcactga tntctgaatt 240
gaaatattcg ctgaaaagtn attataatgg acattttcac gtancaaang tatttaatca 300
gtaattcttg aacattctca tttngcgtat atatttcatc tataatantt acaaaaaaat 360
atttacacat gccataaaat gaatgctcca atantcattt ttatatcncc tatntaattc 420

ntgtgtaact ttatggccct tcatttgtat tatttttttaa catgcaattt naaatcataa 480
nacttgtgtt nataattnat                                             500
```

<210> 745
<211> 464
<212> DNA
<213> Ctenocephalides felis

<400> 745

```
acattattgt atgaaaagca aatttatttt tgaactgttt gagttgatcc acagatttca 60
ataaaacttt gtaatcaaat taaattagtc agcaaactta gcaattaaaa aattaaaata 120
aataaattta ttaatactaa attttatgaa atttcacata atttttggga tttatataat 180
gtggtatatt catatgaaat gattaacaga catgaccaac gattatttga tcacagaaaa 240
gcccagttta gaatttcatg tatggacaat taaaaaagtt tttttttaat tatttatctg 300
ttatttattt taagattagt aatatgtaat gcaatcattt gaaattaata ttctttatat 360
tactaataag agataactta tatgctcata caaaacaatt tatctgttta acattttatt 420
caaaaaacgt aattaatgta aaattttaaa caaattgaga cttg                  464
```

<210> 746
<211> 500
<212> DNA
<213> Ctenocephalides felis

<400> 746

```
acagttgacg ttaatgaaat acctgcatat gaaaaaatga tccagctatt gtaggtaata 60
ttacattagg taaaaaacta gacaatttac caaaggttct caactttaac ttctgtctgt 120
aatgattatt caaataaaaa cctgcaaggc cacaacaaaa tcctgaaact gctgcaccgt 180
atcgcagagg taatctggaa aatataattt gatgttgaac atcatatcat ttattagcat 240
tagatattat cattacactt ttgactgttc ttcccaattc catattaact tccactgata 300
ttggagagct tcgtcctcag ttagtcgaat agcatctttt ggtggttcac ctttagacct 360
tactaatgcc attatattat atgaaattta ctgctttatt cataaatcat ttgtgatttt 420
acattagttt cccagttaaa agttaagcaa acaaatatat ttataccata agttttatgg 480
tactactttc aattttgata                                             500
```

<210> 747
<211> 500
<212> DNA
<213> Ctenocephalides felis

<400> 747

```
acttttaaat tttttgttat ctaatttttc acattgtaat tcatgttgcc tgagtctctt 60
ttctaagttc tggcaggtct ggccaatata agagcaatta caatccaaac aatttacttt 120
ataaactata tctttctttt ttgttaatgg ggtttcatct ttaattttgg aatataaaaa 180
ttttaattgt tttaatggag aaaaagtaat acgaatattg tgtttttga aaagtctttt 240
tgataattgg ttaaaattat tgtcaagata tgggaaatta atgaaaaaag ttttttcagg 300

aacacttgtt ttgccaggct cttccggttt cttatcaaaa ggttcaagta attttgcgac 360
tctattttta ataattggac aaattaactt atatggaaaa ttattgccaa gtaggatcgt 420
tttaactaat tttatgtttg tatcataaaa cagaggatca gataataaaa tagcgtgatc 480
tcaagattag aatggtagaa                                             500
```

<210> 748
<211> 500
<212> DNA
<213> Ctenocephalides felis

<400> 748

actggttgtg attgcttgaa tcgagaagtt aagaaaagca acttttgtat gaatttcagg 60
actggttctc ccgtcagcag ctggtcatgc tggtcctgtt catcaacatc tcgctagcca 120
taatgttctt caagttgctg acgtagcggc ggcctatcca caacggccct ttgtggccgc 180
catgatcaca ggcataggtt cattacagtc gtaagggaat aaatcaaata gactttatta 240
acgaccagca catgtgacgc acagccgcgg cactgttggc aataatataa tcacaacaaa 300
aaataaatgc aagaaaatca caaaaaagat tctagaatcg atcgaattct tcaaatcgga 360
agaaacgaat tcgccacgaa accgcgtgac tttggttttt catttttttg gcacgcaatg 420
gctgaaaggt atcggatgcg tccagataaa tttgtcagtg ggcgttttca gtttctagtc 480
aatcgcctgc aggaaaagct 500

<210> 749
<211> 500
<212> DNA
<213> Ctenocephalides felis

<400> 749

acacccaggt ttgaaccggg gatcactaga tctgcagtcg aatattctac cattaaatta 60
tgtccccttg atcggtgaca ctcggcaaat agcaaatact ggcaccacat cacatttact 120
cagcaaatag ttaaaaaatt tgtaatactc ttaaagcaaa tagcaaacgg tagcggtaaa 180
tcaaattata tcagctacca atgtttttaa taaattcaaa gtctcattta ctctacaaac 240
atcgttaata gcgaacccct gggtttgaac cggttaata gtgcacacgc gggattgaac 300
cggggacctc tcgatctgca gtcggatgct ctaacactga gctatgtcct cacaatgtgt 360
gttactcagc acatagcaaa tacctacgcc acaccacatt ttctcagcaa gtagcgaaaa 420
ttcagcaata atcttaaagc aattagcaaa cggcagcggt atatcaatgg caagttgacg 480
ttatttacac tgatttcagt 500

<210> 750
<211> 500
<212> DNA
<213> Ctenocephalides felis

<400> 750

acacggcttt gtagtagtcg taaaagaatt ccgggtcgaa gcgacagaag ttcctttcat 60
tgttccccgt aatttctcaa aacgcccttt tcctatctct cctgtatact ttttgaatgc 120

gtgcagcagc agcagggtcg aaggttattt ctgcattcct cctacggcgt cgtctctgat 180
tggtattggg tctgcatgtg cgactagtct cgttccgagt ttcaacgttt tctttcggct 240
tgattgattt ggttaggtct tcgatctctt tcggccaggt gtattaatag cctcaattct 300
ccggcaggac ttgacctgga attttgtcag ttttggagca agcagtgaag gcgtagggat 360
agttcttttg attgttgttt gttcgtgcac gtcttctcct cgcgatggag tcttgcttgg 420
ccggaagttg gtttcttctg tatggttctt aaatcgcgca ggcgttctgc aatttccctc 480
tcatttcatt cacattcaac 500

<210> 751
<211> 423
<212> DNA
<213> Ctenocephalides felis

<400> 751

```
acgtatttca gaccgttcaa tccgggcgtt gtctcgtctt acagcaaatg aatgagcttc 60
tgtcccaatt tgcattacca taatggtatt catttttaaa atcggaaaga agaatattca 120
agaatccttc attgtatatg caaacaacaa taaatgttgc tatttcaatt acccgagatc 180
gggaatgaat atgtttcggc gcgaaaatcc aaatcaacga gtttagagac tcattattgt 240
tctgagtttc tgagcctaaa catcgaatta ataatttatt tgacgataaa ctgtcataaa 300
ttggtctaat gacttcctaa tgccatttgt tctattagac cttgcaaaac cagtttttgg 360
agaccgcgtc agtaaagtgc ctatatcttt gaagataatt caaattttca aaaatcttct 420
cgt                                                               423
```

<210> 752
<211> 500
<212> DNA
<213> Ctenocephalides felis

<400> 752

```
acttatatat ctatttaagc acggaactta tatacgactg aaattcgagt tcaaatagaa 60
atctcgctct acatatttat atttatttaa ataattaata tgtttcagtg aaatgttttt 120
gatttttctc tagttgtgtg aacacgaaaa ttttaaattt tatgctttat tgtgtgtaga 180
tgctctgtca ctttcaattt gcaattttga agatttagaa atatagaaga tggaacatgg 240
tttttgtagt tggtggtttt taaatataaa agcattgtta actttccgtt atagtaatga 300
accaaaaatt aaaattgaaa atgtttgcat ttctagatgt tttgaaggtt tctgaacatt 360
ttggcacacc tcagaaaaaa ggatgtgtgt gtgtgttttg tatgtgaatt ttttcccacg 420
ttttcgggcg catggatcaa cctatttaaa tggtaaaaaa aattcgattc ctattgatag 480
gacatgtgct gattcttttg                                             500
```

<210> 753
<211> 185
<212> DNA
<213> Ctenocephalides felis

<400> 753

```
actataggga ataaataaat ggcagctttt tccttaggtc gccttttcaa cgtccggatt 60
ttcatgtttc gaattataca tggcgctatg tatattccat actgcatgct cgcgagctct 120
ttaataaaat attagtgttg gcacgttgca caagattgga ttcgatttga cacagcggca 180
gttgt                                                             185
```

<210> 754
<211> 376
<212> DNA
<213> Ctenocephalides felis

<400> 754

actagatgtt catgaggaat atttagcgta aaggctggtt cggatatatt tgaataatca 60
tccatatcta tcatattagt atagtcggtg cattcgaaat ttatttcggg tgttttgtaa 120
tctctcaatt gcgtcaggtc ttccacatta ttacggtaat ataagatttt tcttatagct 180
gaatctcgta tttccttttt attatcaaac aacattgcta ataaaatatt ttccgagtga 240
gcatagtatg cgttttcttt agcaacattg ttcacaattt gtcttaaatt cgagtctaaa 300
tattgcgtcc agctaataaa tttataaaat aatatactac catacaccac agagttgtaa 360
tatttaatgt taaagt 376

<210> 755
<211> 492
<212> DNA
<213> Ctenocephalides felis

<400> 755

actttacta atattatgat cataagctcc actttcacga tcttctgcaa gggtnattcg 60
actgcaaggg tcgaattaca taatgggaag agcttaggta aaaaaataaa tataagtctg 120
ttacttatgt ataaagtata aaatttttat atttctatga agttaacaat ttagaaatta 180
acttactttt tatgaaataa gctatttctt tcaattaaac taaggctttt tgtgcaatac 240
ttttaataca atgttatttt ttttaatatt tatgcagcca gttttgtaaa ttctcctaat 300
caaaagaaac cgcctcttgc tatggatatc gaaaacttta attgagaaac taaaaactaa 360
agaaactaaa atatgcttca aagtaattaa agaagttttt tggtttatga aaaagtttat 420
aattatttat ttagaagttt atgaaaattc atataagcta ataatgaaag caaactaaat 480
attattcgaa tg 492

<210> 756
<211> 360
<212> DNA
<213> Ctenocephalides felis

<400> 756

accaggtgag aaatggtcga cttacttaag aaagccttag agtaattagg ttaatgnata 60
ccaattgaaa agtagcttac cataacattg ntgcattttt tgaaataata agcgcgtttt 120
atttaatatt gttttttaa aagaaatgct aaaagcttta tgtaaggaat gtattgccaa 180
aaaataccaa atgaaatcta gtcaacattt tattatatca aatcattcat aattaactta 240

gtattcagac atgacaattg agctttgatt attgtaagct tttctacagg ctaatatatt 300
attttaatat aattattttt gttgcttgat tatcttattg gtgatggatt aagtttttgt 360

<210> 757
<211> 207
<212> DNA
<213> Ctenocephalides felis

<400> 757

```
acaaatgcaa cggaagtgcg ttacggccga actgtaagtc attcgctctc gatgaactca 60
ttttaatacg ggtcgattgt gattagtcgt cgtgtatgtg aatagagatc tgtcgttgta 120
tgcgtaccgg cgtcaacgtt gtatgcgatc cgatatcaac aagaaaatcg gcatggataa 180
ataaatattt taaagttaca tcgtagt                                     207
```

<210> 758
<211> 500
<212> DNA
<213> Ctenocephalides felis

<400> 758

```
caaaaattga aaaaaatatc tacaaatggn tacatatttt tgaaattgtc tagaaangng 60
taaaatatgt agaaatctaa aaatatctaa aaatccatag aagagtctac gtataaaaat 120
cattctcacg aatacaattt acctgccaat gttgtcgtgt atcgtatatt tttctgatcc 180
aatcttgaca aatgtttcaa gattgaggtc agattttta taaaaaatca ttataaatta 240
tttcttttcc acccctattt atgacatttt tgaagaactg acaaaactca attttaaagc 300
ttctatgttt tttattttt gagaggagca aaggaaattc ttcatcttcc tacaaaaaaa 360
aacaataaaa ataatagaag tgatactctg caaaagaaga aaaactttgt tatgtgaaaa 420
tgagattgca gtgcttgcgg gcagtctgca agacctttaa agactatgca acactttcaa 480
acagtctaca atgttctaat                                             500
```

<210> 759
<211> 500
<212> DNA
<213> Ctenocephalides felis

<400> 759

```
acgtctattt agcaggctag agtctcgttc gttatcagaa ttaaccagac aaatcactcc 60
accaactaag aacagccatg caccaccacc caccgaatca agaaagagct ctcaatctgt 120
caatccttcc ggtgtctgga cctggtgagg tttcccgtgt tgagtcaaat taagccgcag 180
gctccactcc ttgtagtgcc cttccgtaaa ttcctttaag tttcagcttt gcaaccatac 240
ttcccccgga acccaaaagc tttggtttcc cggaagctgc ccgccgagtc atcggaggaa 300
cttcggcgga tcgctagctg gcatcgttta tggttagaac tagggcggta tctgatcgcc 360
ttcgaacctc taactttcgt tcttgattga tgaaaacaca tttggcaaat gctttcgctt 420
ctgtccgtct tgcgacgatc caagaatttc acctctaacg tcgcaatacg aatgccccca 480
gttgtcctat taatcattac                                             500
```

<210> 760
<211> 338
<212> DNA
<213> Ctenocephalides felis

<400> 760

acaatatttt ttcaaaaggg tcataaaagt gttcgtttta aataggtatt ttattatgtg 60
tnatataact ttgataaaat ttcaaacgcg tatttataca tcagcgtttg cgtatccacc 120
ttaatttcaa tttaattttc atcggttgat tatactgaat ataataaaga ttttgtgttt 180
attataattt aagtataaaa ttgtagacaa taatattcta cgattcaaaa atctataaca 240
cataattgat ttaattttag tttaaactat aaaaaagggg aaaaactcac gatttatatt 300
tcttctagta agtgtaagct tagatatgtt gcttttgt 338

<210> 761
<211> 348
<212> DNA
<213> Ctenocephalides felis

<400> 761

accaaaaatc ttattgcccc aaatttatnn tatctaacac tagcctattt ttatatattt 60
ttacaaagaa aatgcgaaag ttatatatat tatagatatt tttacgtcta gtcgttttta 120
tataaatact caatatatca tgaaataaat caaataaaat ataattataa ttctatatat 180
aatgaatcaa atttaattta atttttgtga catttattgc tatttctgag atcgtgtcat 240
aaatgatctc aaggaaattt tcgttggcga tttgccatga tttaaattaa tgttgcttaa 300
atatttgcaa cgcattttcc ttataaatag tctaaattag aatcaagt 348

<210> 762
<211> 372
<212> DNA
<213> Ctenocephalides felis

<400> 762

accttctaca ttactaaaac ctctgatttt ttataaagta gatttattac aaatatttca 60
accatcgaca accaaatatc gtgtcaaaat cgatattagc ttttgaagat attctgaaag 120
taaacagcga ttccacgtat tttttattag tttcgggaag atttttattt ggctataaaa 180
gtgttttgat tgctcgagaa caagatgtta tctcttcaca acaatcttta cagataaaca 240
gataaactga gtcttcgatt aatggcagta ttgaaatgag gttttatata ataattagac 300
acgaaggcag gtccagcagt ctgagcatca gtttcgtcta tgattgcaag tatatatagg 360
gtaaggcgag gt 372

<210> 763
<211> 500
<212> DNA
<213> Ctenocephalides felis

<400> 763

```
acggagtgta aaatattgtt gaagtatttt gaaatttatt aatttattcg aaaaggngat 60
ttcattaaat aaaaatggtt tacgaaagtg acttttacac gacccgtcgg ccctacagtc 120
gtccggcttt gtcttcatac tccgtaacga cgccgtcccg tcattacgtg gtgacagaca 180
ctccatccag accaagggta gcggaagagc aatattctta ctcctaccgc agccagcagg 240
aaagatcttc tgcagatccc tacggaagga actattcgac aacttccacc accgaaagca 300
caagacgtgc aggcggttat ccaggatctg actattctta cacgagcgaa cgctcatcca 360
gaactggaga tggaccaggt agctacagat ccagctacag ctccactact tctggacgtc 420
ttcctggagg aaccacttac cgtcacttct cataccgtgt gtaaacacga aaattgaaaa 480
attggtttga taaattggtt                                             500
```

<210> 764
<211> 302
<212> DNA
<213> Ctenocephalides felis

<400> 764

```
acgccaccac ctcctgggca ttcaccttca ctgatctttt tcaattcttt gtgttgttca 60
cngttataat tcctcataac gcattcattt ccaaatgata tcggtttttc accatcaaca 120
ccagcgcata caggagtgta gtcgtctgtg cagattttaa tgcatggtgg atttctgttt 180
tttgcggggg tagcttcgat taggctagcc aatgccacaa ataaaacgac agccaccaaa 240
taaagcttca tccttattta tcttctttcc aaaaattcga tgttgtctct cagatatttc 300
ct                                                                302
```

<210> 765
<211> 500
<212> DNA
<213> Ctenocephalides felis

<400> 765

```
acggaaggta tttatattaa tgtcaactct caaatgcata agaaaagctt atggtctaat 60
attgccactg gaaaattgtc taatttaata ttaatttctt ctatgtttgt aaattcgtta 120
ttacatattc ctgtagtaag attaatatga gtatgggttt aatttagtag tgaatactat 180
agtaacccta taaacataga cattaagttg atcaacgcgt ttatatcatt agatctttca 240
atgaaattag cattaaaaat tttcaaataa aaagcgtctt atattatcgc agtaaagttt 300
ccttaactct ccaagaatga ataaactgac gcattaggtg atctatacat caatattaca 360
gcgcgttcat tgatatttcg atgttatttg ctcacattca acatttttaa agtttttca 420
ttctcaagag tttccggcga tgttcccggt gctagtcgaa acacatgcag gtgggctttc 480
ctttctactc ccgtgatatt                                             500
```

<210> 766
<211> 500
<212> DNA
<213> Ctenocephalides felis

<400> 766

accagagatg aaatttgcgt gcatcacccg gaagttttgg aagacattgc caaatnngaa 60
tgactacaaa gcggtttcag gaattgaaaa cgaaaacata gaacaaataa taaatcaagc 120
tgacctgcaa aagtattatc cagaaattat aaatttatac aatacaatgc acgtctttgc 180
gttacctcta gcacatttgc agtcttgttt cacgtttaat attgaaaata ttcttaaaaa 240
cgaaaacaat atcagttatg ccaataaaat taatggattt atcgaagaat tacaaaactt 300
tgtagtaata gctcaaacgc agtctaaatt ggggccaaat gaggaactca caactttaaa 360
agatgtaagc gatcttacca cacttgaaga tcttagtcat aaaataatta gtgaattaga 420
aaaaagcagt aacagcagtg atgaagtaag ttcatttgta aaatcattga agaccctaat 480
atgcttgtga attctataaa 500

<210> 767
<211> 479
<212> DNA
<213> Ctenocephalides felis

<400> 767

acattttccg caaacatgtt tgttgcaact ttgacacgtg ttattagttt tattattatt 60
acaaagtctt atttggcaaa attttctttt attactattg gtatttgcaa gatgtctgtg 120
ggtcatcatt ttcctttgat tttttttgtg ttgatgtcgt gtagtccgcg gaaagctgtt 180
ctgccaattc aaacaaaaac tgttctctaa ggatttgttt tctttggatt tttattcctg 240
tcgcttcctt gtaaagaatc caggcattta tcgcagccaa atccaaaata ttgaaaaata 300
tttgcagggg tcatctattg gattggaact ggatttcacg gtatattttc tttacctttg 360
atctgccatg tcttctttgc tgaccataaa taagtcattt tcaatttttt tgctcatagg 420
ccccacgagc atataaaatt ccaataaatg cacgtatttc ggtaacgatt tgctaatcg 479

<210> 768
<211> 500
<212> DNA
<213> Ctenocephalides felis

<400> 768

actcaagatt tgttacaata ctttataaga cttaacntaa taatacgata gataacctat 60
tacctatcat actatattat attattgtgt atcatcgata gatatctatt atctatcata 120
ccgtgagata catacaccgt cattaacttg taatatacct acagaccttt tacttcttat 180
aaatactact gtctaattta tatattcacc tatattatat aggtttacct tacgccctat 240
gtattatctc tacctatcta ttgtctatac caggaattga acccgggacc tccgcgtgga 300
agtcgcgcac cttaaccact accctatcgg ctacccccat taataatata atatgggacg 360
cactgttgtt tccgcgggac attttctaat taattagata attaatacta aggtggcgcg 420
acaacacgcg aaaaaagacg gttgattgta gcgttacggt ggccgagtgg taaggtcgtc 480
gcgtgccacg cggcggaccc 500

<210> 769
<211> 500
<212> DNA
<213> Ctenocephalides felis

<400> 769

acaattatgt tcttatttaa gctttgcaat aattctntag actgtcctct tattctcaat 60
caactatcct ttaatgttcc aaggctatct tcgcgttcac aattattttt caattatccc 120
acccctaaat ctaattctct cttaaattcg ccaattttac taatgtcctc taactttaac 180
ttaattaata atgaaataga cattcacaac acatcaccta atcaaataat ctccatctgc 240
acaagttaat ctttttattt tctttcaaat atcttattat tttctgttta taattattat 300
tgttacattc tgcattatat gaaattttat cttagcattt tatgttacta actatatatt 360
gtaattttgt gtatatttta attgttattc attatgtcaa ataattgtat ttttgagtcc 420
tcttggagaa atctgtgggc tatatattta aataaataaa taaataaata aataaatata 480
tgtacaaata tacttataag 500

<210> 770
<211> 106
<212> DNA
<213> Ctenocephalides felis

<400> 770

acaaattatt gttgtgtggt agtttactan ttnagagtaa agtaaacgta acacacaatt 60
gcatatgcat atattattat tcttatatac atataaacaa tagtgt 106

<210> 771
<211> 453
<212> DNA
<213> Ctenocephalides felis

<400> 771

acataataaa tcgtgataaa attaattcaa agtagcaccc aaaaaccacc gaactttgca 60
cgaaattacg ttttgttctc aaaaaagaaa aaggaaataa atcaagatgg tccaaggcga 120
gaaatacctg gcaaccaagt ccttgtctat tgacaactct cacaagaaga gtggattcga 180
cgctgtgcaa aaacgcgagg aggagaaaag gcagcaggcc aggaaggagc aggagcagaa 240
agctaaatat ggagcctggg gtcctgtttt caaagacagg gaaacttttg ccagcatgca 300
cttctgttga aaacataaaa ttttatatgg aataataaat ttgttttata aaatactagc 360
atactaacaa aaggactaac atgattctac cttggaccta aatacaatac tcaaataaat 420
tggaacaaaa cagagatggc aacgctttaa tgt 453

<210> 772
<211> 182
<212> DNA
<213> Ctenocephalides felis

<400> 772

acaaagccat cactccacca attacgccag tttcaggcca catcaacccc gcttttttc 60
ggccttatga cttgttagtt tgttatatct aaatattaaa tttatgttat tcaacaataa 120
gtaaacccca caacgtgaac aacactgctg ttctttgtcc tgaggtagga ttcgaaatcg 180
gt 182

<210> 773
<211> 500

<212> DNA
<213> Ctenocephalides felis

<400> 773

```
acaaaggtcc aattgaactt caagtaatta ctntatttaa accaaatgaa ctacatcata 60
catgtaacat gtgtaaatat acaactacag ttttttttct aaaatttgtc atatggccca 120
ttttacccca taaacataga taaaatggga cagtcagggg acaacagttc ttctaatgtc 180
tgattttaca aaataatagc taaatattta atcaatgaaa aatttacaaa gattatattg 240
tatatgactt agatctggta ttttatcaga agtatttatg agcagagttg gtataattat 300
taattttttt ccttgtaaaa ttgctttttt ttaactgagt ttacattttt atttacattt 360
tttattatat actttttgc cagggtaaag gttcagttgg ttctttttgc cccatccaca 420
tgtaagccat tatagaggtt atatagaaac atctgttgaa tctaatttga aattaacaat 480
agattttata aaacaattca                                              500
```

<210> 774
<211> 500
<212> DNA
<213> Ctenocephalides felis

<400> 774

```
aacatttctg tgataaataa actctagttg attcgagaat atttgttatg atctttgttt 60
ctaatgcact tgttaataat aattatgcat tacaaatgtt aaaatgaaat tattatgttt 120
atttaataag atacataaaa gtaatttatt ttagaaattt ataatctatt ttactaaata 180
tgaaatatga atttctagtc ttaagatttt actaaattac tgatatttga attgaaatat 240
tgctgaaaag taattataat ggacattttc acgtagcaaa ggtatttaat cagtaatttt 300
tgaacatttt cattttgcgt atatatttca tctataattt ttacaaaaaa atatttacac 360
atgccataaa atgaatgctc caataatcat ttttatattt acctatctaa ttcgnngtaa 420
tttttatggg cccttcattt gtattatttt ttaatatgca attttaaatt ataagatctt 480
gtgtttataa ttaatttatt                                              500
```

<210> 775
<211> 473
<212> DNA
<213> Ctenocephalides felis

<400> 775

```
actacgatca atggtgtgat agcttcgaat taaaatgttg atactatcga gtatccaaat 60
taaatttgga aattaaaaaa tgttgtgtgt taattgacag aatctttact tcttctaaaa 120
gcaaatgaat aagttatatg ttatgttact tataaaataa ttaatatttt taaatacaac 180
aataatgtct gcattattat cttaacgatg ggaaaatgta gtaaaagtca acagatctag 240
ttgaccgtat cactcctgat tattaaccat agggttaata aaagtattat aaattcatca 300
aaatttaata ttaaaaatga tcaaacaata ctgatcttat tatgatcata cctaattata 360
catgtgataa tataaaattt caaatcaata cactagcgaa tactattatt agtttatcat 420
agactaatgc attgattaaa attcagggcc atccttaaat taattaaaaa ctg        473
```

<210> 776

<211> 499
<212> DNA
<213> Ctenocephalides felis

<400> 776

```
acctttncac agcctctctt angtctgatg ttagacgttc ngctngcaac ttttgttggg 60
ngtntcctcg gcncncaacg ngagntagcc tttgaagatc tctacttgna gctgatatta 120
gctgnattgc actgaanngt ggcgtatgac tgnnaanann acnnnnnnnc ccttnccagt 180
ttnntatang catntanata attntaagnc atttttataa nctattatan anattatcta 240
tnggggagnc agncataatt gaaaccagtt ttttctatca atcatagatg atgtaatggt 300
ctaactntca atagaactat tgaaagttac nccatngcat antgaaaatt anactattca 360
annatnaaaa taatacaata attatgnngn agcagntacn ttttaactat tgatactaat 420
gctactttaa tatcttaanc aactaagtna ctcatnttgc tagaaaatat ctaatnaaat 480
tatataaact nacatcttn                                               499
```

<210> 777
<211> 500
<212> DNA
<213> Ctenocephalides felis

<400> 777

```
acgttcacct gccgcgcaat cacatcggct ggtcaagtgg aaacctcagc aaaacttgtt 60
gttaaaagta agaaataaga catggactcg ctctaataat tgttctaaca tttaaattac 120
tattatcact agtcttgcta tatttatttg ctttgttttc aatttagtat atcaatcaaa 180
gttagattaa tagtagtttt tcgagctatt taaaaatgat tttaaagcag tataaaaata 240
taactctaat aatattattg cgattgatat atttatataa attatttatt ctatagttca 300
atttaatagc tattaaaagt atattgtgaa tataaataaa attgcttgcc atagatatat 360
taaaatatag cttaagcaac tgtctttata tcatattagt aaggtcctaa tcggtcatga 420
tattttgtgg tgttgattat tattctgtgc tgtaatcatt gtccaaatga tatctgattg 480
tttaacatga tacaaaaatt                                              500
```

<210> 778
<211> 188
<212> DNA
<213> Ctenocephalides felis

<400> 778

```
acatgcgtaa agtgcaattt aaataagaat taaagaccta ttaagaatgt tttatagcac 60
cttttatgta atttgcataa tcatggatac tagagctttt atgtataaaa ccttattcgg 120
ccatttattt atatccagaa aagtaaaaaa ataagctggc tctaaaatga ctattattta 180
taaaaagt                                                           188
```

<210> 779
<211> 500
<212> DNA
<213> Ctenocephalides felis

<400> 779

accagataga aaaagttaga agtatttgat gataagttga tgttctcatt atttatttta 60
taatgacata attccagtta atcattccac cgttattttg atctgactca ttcctaattt 120
actaaaatac ctgaaacaaa attccgtcca ttaatagaac agataaaaaa tcaatccatt 180
actaacaggc ggctttcgat tttcgttaca aaatgtttta atgttacttt caaaagatta 240
ttatttgttg ctttgttcta aatatggttt ccgttttatc ggattaaaat tggaattaat 300
ttctaataaa tgtctataat ttatgatcat tttacaaatt gatctttctc tcgaatcgaa 360
tggacaatgt gtgtcaagaa caacaatagt caagatctat tgattggatt cgattggatc 420
tttatcgatt attatcagta tcacaccttt ggataaagtt cagaaatgtt gtaattattg 480
tattaatatt tatatgttac 500

<210> 780
<211> 434
<212> DNA
<213> Ctenocephalides felis

<400> 780

acgcacgtgt cagaattggt tgaaaaaatc agtttacaga aattttactt aatcacgctc 60
attgaacagt atgcacaatt ttctcgtcag aatcgaggta aaattttgct gtgaaaagtt 120
gaaatgcaac tattttttat actggcttaa tcggtaatat attttaaaca tattgtttga 180
gaatatgtgg ttgcaaccta aatgtaatat tcaaagattt atatgcaaaa attctgccaa 240
ttctccatga ggagtatatt cgtcaatcac agttttaagt aaaggtgatc atcgttctcg 300
tcgagctatt aaagttgatg atcatattgt agaaatttta tacgtattgc acacaataat 360
tatcttggaa ttgttattag acttaaacct tttttgaagg ccatccttag tgatggtaaa 420
aaatagagtc atga 434

<210> 781
<211> 500
<212> DNA
<213> Ctenocephalides felis

<400> 781

acgggaagtt ccagcaattt gccttaagtg atgctataca ccactagttt ttaatattga 60
tgaatatacg tatttaattg aaatgttgaa acttgtatat ccaatagtaa ataattcgta 120
tttgcatttc tatttgaatt acagggcttg aattgagaaa tattaaatgt tttttgaaat 180

aatgctttga aatattttga ttcgaaatat tanatccgaa ttaaaatatt attagaatta 240
ttatttttaa tcgttgaata tgctttttaa ttatttattc attgaactta aaagcttctt 300
ttcacaaaga tttattaaag agcaaatatg ttttataagt gagtctactt cctgaaaaat 360
gtcagaatac ttgcatttta atatatattt cagctaaatg acaagacatt ggcaaaactt 420
gtattcactt attttaaatt ctgaagaaga cacaacattt aaaaatattg tcgatttctt 480
gnataaatta tcgcccttaa 500

<210> 782
<211> 500
<212> DNA
<213> Ctenocephalides felis

<400> 782

```
caaactttaa tagatgcaag aagcaaatta ggatcaaaac ccaatttatc tattttttag 60
tgcagaagat ttttttctga tgcattaatc ttcatacagt tttatgttat ataaaattat 120
aagtaaatat tttacctcaa catcacttta cagaatttca gtaaaaattg agcaattaat 180
acaattcaat attgnaaata ctcttactgg ttggagagcg gatagtttca aaaatttct 240
aaagaaaaat tgagtgtcaa cccgatgttc tatttaaaac ggattcgtta tatgccattt 300
cattaagatc gaaacccaat ttatctattt ttaatgcaaa gattttttc tataattaat 360
gtattgacct tcatacagtt ttatgttata taaaatttta agtagaacat tctacctcaa 420
cattacttaa caaaatttct ataaaaattg agcagttaat acaattcaat attgaaatat 480
tcttactggt ggagagagga 500
```

<210> 783
<211> 500
<212> DNA
<213> Ctenocephalides felis

<400> 783

```
acttcttttg taagaattaa tttgtttata tttatatgta tgtatatgct aaaattacaa 60
ctaggtattt ataaatcaga agagcttgtt ttaaaacaat tcatatttcg ccgactcccc 120
tattgaaata taaattaaaa ttttgctacg actagaacgc gtggtttctt gcaaagtgag 180
atcattttat aatgataaaa ttctgaccga aaattatatg tcgaaatgtc agaccagaat 240
tagttgagtg taaacggttt attcgtcttt caatgtccgg cgtaagtcac aatacaatat 300
acgttcactg aagcgatacg ttaaaaggtg ttatagaata agaatgcatt tcaataatga 360
ataatacagc taaagattga attatatccc actttgcaac gcatatttga gttaagagca 420
gagaccgacg gcagacggca gagagcgatg tccatgccgt cgtcgtgcct gaaatattat 480
tattccactt aacatctgaa 500
```

<210> 784
<211> 422
<212> DNA
<213> Ctenocephalides felis

<400> 784

```
actttcttcc caaaactcga ctttttattt gaaaacataa ttactcggtt gtagtaaact 60
tttntaaaat atgtaacaaa ttacttagca tctttctaaa atttattcaa ataagtattt 120
aaattaaatc ttcctctata aaatcgtttt atttctacta atttccaata aatgacttct 180
cgctttgctg ttttttatgc tgngggacaa acaacaaatg acatatgacc aagcgttgat 240
atatgcttga gcatgatatg tcatgaatgt ctttattgta attatgccta atgccttaat 300
aattttcttg aaaatatttt ataccactat tccgtttcca aattcatcta ccacgagtaa 360
ggtatacatt tgaaagccat aaacaatagt tccatttgta tcatcaatgc aatccctgtc 420
gg 422
```

<210> 785
<211> 500
<212> DNA
<213> Ctenocephalides felis

<400> 785

```
actataccgg tgcntaccgt ggcaagatga acgtcccaaa acgtcgctga tcaaattaat 60
agtcctcgtg gctaatttat ttattctgga aaatcgtgcg atttgtttgt gttcacaaaa 120
tttccgttta ccaaattttc ctctataatg ttcggagtgt tggccgccac ctcgatttgg 180
ttactattgg catgggcagc tatgctaata tttttccttc cgttgatgtt cgttgtgtta 240
gcagttttgc cgggactgcc attactgatt attcgaaggg tgtgctacgt gcccttcgat 300
tcgatttaaa tttgatttct tcaattaaaa aatcaatttt aaataaggca gtgttctttg 360
aaatagttat ttaatcgtgt catttcatag tagttgtgat atttatattt tttaaacata 420
tatcttctaa tcattgataa gtatgatatt tatacatagt cttacatatt aaggtataat 480
atataataga ttagtcgtat                                             500
```

<210> 786
<211> 500
<212> DNA
<213> Ctenocephalides felis

<400> 786

```
acaattgaaa tatatcacag tcaacaataa aactgatgca attttagcac aattgaagaa 60
tgaaatccaa ataccataaa caacgcttaa caaccaccta gctaatgatg agctaggtgg 120
ttgaatattg gtaagtaata taaataatga ctttatcata caataatatt actttcactc 180
atcaatttga cacaatttcc caaataatta taccaataac tgagaaaatt tttgaacaaa 240
tttcgacagt tggttatttg atatatgtta tgtgctgata tgattataag tgtatgatat 300
atattgggat gcttaatgga acatttcact caaagatgca tctaatgcag ctcaacaatc 360
aatcatgaaa gatcgaccgc aattttccat cttgataact agatgtaaca aaatcgcatt 420
tttgtttaga tatttattta tatgtaataa cactgcaatg cgtgattgga tatcaaaatc 480
ccataagcgt tttttataat                                             500
```

<210> 787
<211> 500
<212> DNA
<213> Ctenocephalides felis

<400> 787

```
actgaatatt gttgtggtaa ttatttctgg tacgaatgat tcctttgtgg atacggtagt 60
aacttcttgt agcacccgat ctgttatgaa gtcggggata tctttgtctg cattcatatc 120
tggtggtggt gcagtatggc ctggatttgt ttctcttggg cctgttgtat gaatttcttt 180
atcatgctcc gttggcaaaa tcggaatatc gctactttta tttttaaaaa tctcttcatc 240
gtgaccagca atattattat tgttgttacc attaatattt atatcgattc cttctgtgat 300
atctgaatat tcatcaataa aatcatcgga gcttaacgtg tctggagttg gagtcacctt 360
cggtttaaat gtggtcagac gtggcaaagg aggaaaatcg ctttcttcat tcattttttc 420
tgtcacattc atgattttca caatcatatt accgtaagtt gtttcttcga ttcctgaact 480
gcttgataat gcctcggccg                                             500
```

<210> 788
<211> 436
<212> DNA
<213> Ctenocephalides felis

<400> 788

ncatttcaac tgagttcatt aattgagttc attaatncgt gaatgccaag gcatttacct 60
atatcctatt atgtaaaata acgatggctg catttctttg aattacaatc gttattaaaa 120
ctaggcaaac tgaaattttt gcccttggat tgcagtgctt gagtgaatcg cttttggcaa 180
atgggataat tataatcata tcagagtcca tattatgtgg tccaatattt agttaagatt 240
tgtattatac acacagcaaa aaatataaaa tataataaat atatcctgga aagattagtg 300
aaaactatta taaactaaga cttctcgata tactttcagt gaatccaatt acttcgaaaa 360
aacctttgga gttagaaact aaattcctag tagtttaagg ataatatttt gatttgaatt 420
gtaggctgct gtatgt 436

<210> 789
<211> 277
<212> DNA
<213> Ctenocephalides felis

<400> 789

nctccctcca acgcaggtgc atcgtcgttg gcgtcgtcgc tgatctccgt gggggtataa 60
taaancgaca tcttactctg agattcattc aaatttggaa cttctaatgc agccaactta 120
tctattttcg ttttaatata catatctatg cctgcaatat ctttgggatc gaacacaaca 180
gagtcgctac gaccgcaaga accgctttta caattctcga aatagaaact gggcaaattt 240
tctaaaacct tattaagagt ctgttgctgg tatttgt 277

<210> 790
<211> 500
<212> DNA
<213> Ctenocephalides felis

<400> 790

caattttgtt gatggcatga taaatataac ttcttataat taatattgca gattaaactn 60
gtggtatact ctttttgcca tcagcatttt ttattgattg aatacaacag attgttaaat 120
aatatttcca atcaataatt gtgatggttt atttctacta gatttgaaaa tcatttgatt 180
atgatttaaa catataaaac cgttaaatct cgttttaatt atttgataat agtgctgaat 240
tacacattgt agccattata ttaaacaaat attcaaatac ttattagtca aaattagttg 300
ttactataat ttaatttaaa tattgtgcaa ttttatgtag tataatttag aaatttatta 360
ttaaacacgt gttttattgt gaataaaata taagagattt atgggaactt tttcaataac 420
tgaaattgag ttgctttgca ttttttaat atgatgaatc gtttttgaaa tccatattta 480
tattttatta attgacctgc 500

<210> 791
<211> 326
<212> DNA
<213> Ctenocephalides felis

<400> 791

wcmdkccadg nhtastcrys ktwbkhtnta hdvdacsagd mhacrnvcwr tbwwyrrwyk 60
vnwmtmsnwr manrgarcyr chsncnamnb tydnachcks mcratndats trandsncnc 120
ttacaccctc catgctgatg ttgaagaatc ctttcgtgtg tatttggaac aggttgacgc 180
ctncatcaaa tttataattt ccttcgaaaa tcaaattgtc gaacatcagg tcaatttcca 240
tttctaaact gtcatcggtc acagttgctc tgacttcttt gatttcgcaa ttgctgctcc 300
cgaagacttt gttgtctttt ccctgt 326

<210> 792
<211> 500
<212> DNA
<213> Ctenocephalides felis

<400> 792

accgttgttc ctagggggat aaaataagca ggttataatt tttgtaaata acaacaatat 60
ttataatcat actggaattt ttacagaatt tgtggagcaa gcctagataa aaattttgcc 120
ttatttctca agatgttcat atcttattat ttatgttcaa tattaaataa aaacattacg 180
aattatgatt ataaacatta ggaaataaat tacaatatga ctaaattaat tttcaatctc 240
ctatcatttc agttaatttt tactggtgct attatagact tgcttcatta agtttcagaa 300
ttaccttcaa tagttgtaat tataaaatat atttataaag tctataaatt agtaaaaatt 360
gtatccgagt aagcaatttt aaacagatta tttactcact taatacattc actaattata 420
tggaatacta ttagttagat tagaagtagt ttatgaacaa atgatttgtt catataggag 480
tggcttgtga caacaaaatg 500

<210> 793
<211> 219
<212> DNA
<213> Ctenocephalides felis

<400> 793

aatatgggca acaataaaat gaacattaac aacgtgaagg cgttaatgtc gcaatactgg 60
aagttatacg tttgaaatta ccatgtggct catatttagt atgtttaata catttagaac 120
ccacaaccac gctaactgat aattaatatt tctaaacggc aatacgtgta tcattccgtg 180
tctgctatct tcgccataat caaataaaat tgtttatgt 219

<210> 794
<211> 252
<212> DNA
<213> Ctenocephalides felis

<400> 794

acataagaat aagttattat tatgaggtta acgacgcgcg aactaaaatt atcacgctga 60
aatttattgc ttgaatacat attaaagtaa cataaattca aacttacaca tttattcatt 120
tatattaaat gaataaatgt ataaagatta ttaccattca cgttcttcac atattgcgtg 180
cttacattat tactttgatt gtgcaaatat tataacattt acattatgtt ctaatgagtg 240
attttttcat gt 252

<210> 795
<211> 500
<212> DNA
<213> Ctenocephalides felis

<400> 795

```
acttagctgc aagctttttt atattctgac cttgttgcat gagttctcta aagtattcgg 60
gacactcatc acgttttaaa aatcttaaaa atccgggcat agcagcaaaa tatgttgact 120
gcagctgtgc ttgagaccaa ggtccagagg cttttgcttt atcattttct agaccagctc 180
ctataattaa tgcggctgtt tttgttcgca tagtgtttgt tgctactggc cagtaatttt 240
tcttagaatc ccatttcttg atattaaatt gtcctttata tactgtatct aaaagttttc 300
cgagctcgaa agcttctttt tttccacctt ctgttaattt actagaactc tctgataagt 360
tagtcaactt gggtcctcca ggataattgc aagcttcatg gctggacctc ttncatacaa 420
aaacaaactg aagttatcat tgctaaaatg tattgactga ctagcaaatc aaaagaatca 480
ataactctca tgtactataa                                             500
```

<210> 796
<211> 500
<212> DNA
<213> Ctenocephalides felis

<400> 796

```
cgggggccna cncttatccg atanacccat agaccttata ctntcataga ccttatncaa 60
tacatggacn gggagntnan cgcataaaaa gcgtcaaaag atgtcctacg gnatgagggc 120
gtacgagggt aattttgcgg ccgagatatg tcaaatacga acacgagctt taccganatt 180
agtcgaatgc actcgatcgc ttaaatttta cagttacttg ggttcacgtg atgtttgatc 240
gttcggnttt ttaaactaaa agatgataaa aatatacctg ttgtaaaatn ngttaaangt 300
aaaatgtctt ctaaaatgtg taaggnaagg tgcctgattg cagtaaaacc tgttttgtgc 360
tgttctgtat gcttcgcaag nattccggga aatncgacat tgatcgttta aaanatcgag 420
gattctttna tatccttcga ngaatgttta tttatgttat atatagantg ctantcaaag 480
tctttntttta tgtacaaagt                                            500
```

<210> 797
<211> 324
<212> DNA
<213> Ctenocephalides felis

<400> 797

```
gctgaaggca tgaacgagca attgagcaat ttaacttaag aagttttgac ccaagcccaa 60
gcaggggccg ccgacgtgcg ttccagtgtc gacaaattcg ccaaagaaca cgaagccacc 120
cacaccggcc actaattata aacagaaaac cttctgtgat catcatttat catatcaaat 180
atatatataa attcattaat taattaaaat ttgttttata gttgtttaaa taatcttcgg 240
atatttgtaa aaattatttg gatgctcatt tgatttatat tataaataan atacttcata 300
aaaaannnaa aaatanaaaa aann                                        324
```

<210> 798

<211> 500
<212> DNA
<213> Ctenocephalides felis

<400> 798

```
gcctggcctt tgtggtcacg ggcaggtaaa acccttgaca acttcgattg catttcacct 60
tacattttca aatatgtatt tggtgtgtca ctagtaattt gcataatgtg tttagaatgt 120
tccggtgaaa ctttgctgct atttataaac gttaagcgga attagtgcat tacgttcaag 180
gtattatacg agcataattt tacataattt aattgattgt cgcaagctac acatttcatt 240
aattattctg attctattaa ctaccgcaga ttatcctgaa actaaaaaaa atatagattt 300
ttaactatca aatatctctt tacctactgc tgttcagtat tcttttaaca gtgtttgaat 360
tgataaatgc ttttcactat tttatttgcg aaaattatgg aatattcgca aacactttat 420
caaataaaat gataaatttt cgtattctca aaagtaaatg cttgtagnaa tctaaaataa 480
tctaatcatc gcccaaagtg                                             500
```

<210> 799
<211> 500
<212> DNA
<213> Ctenocephalides felis

<400> 799

```
acaataaaaa tacgaagata taaataaaaa aatgagaatt aactttttta acaatatggn 60
aagaaattta ttataagact aaaatatttt gttgtatata aaggtaagac acaactaaaa 120
taattgaaaa atatgcaata actcactata caaataattt gaagaaaaaa caaaaaacac 180
ggaaaatata atgcaccaat tactatacaa ttgtcaaagc acgaattttg aaaactatta 240
aattaaaaca aatattaaat taaaataaaa taaatgaaaa ggttttaaaa aataaactct 300
tttcatcgag acgtgttata tgtttggaaa aaaaattact agtgaattgt gttgtgtgtt 360
tctatgtaaa gataaggaaa ctcgaagagc tgtttcgtta ttggtgaaag actgagatag 420
gaaagttttt gtggcgcttt gccggcgaaa aaatacgaag taaggantgg aaaatatagc 480
tttggattaa gctgtaatag                                             500
```

<210> 800
<211> 500
<212> DNA
<213> Ctenocephalides felis

<400> 800

acggaatata cgtttccgtg acccttgagt gccaaatcaa cagatggttc aggactgtgg 60
gnaaccttan atcttaattc aacacttcct ggtgattttt tctttgagtc gttaagcgtg 120
aaaactgact cagtattgaa accaacaaca gtcttctcgt ccaatccgct agtttgaagg 180
tcaagtggtg caatcaggtt atatgtgata ccttggtatt cagcgaattt gctggccaaa 240
tagaatttga tatcttggaa tggtaatttg gcggctttaa catttgttcc gtgaattcca 300
ataattgttt tggtagcacc agcgcggaat ggatattcaa cagcatcatc atatgcagca 360
taagcagaag tcaatccgaa ttcatacttc acgtcattca cangcttagc aatagttcga 420
taagtcatca agttttcatg tccagttttc aatggtgtag ctgttgttat cattacggnt 480
tacggtccag nttaccacng 500

<210> 801
<211> 166
<212> DNA
<213> Ctenocephalides felis

<400> 801

acacttttaa cgactaatgg agcggagtag tgacttatca caggggccgt ataagccaaa 60
ggcgctacgt atcctgggcg agcaaaagct acagctacga gagcaaataa aacgaccagt 120
ttgaacattt tgatttagtt ttgaataatt ctcaggaggt ctgaat 166

<210> 802
<211> 266
<212> DNA
<213> Ctenocephalides felis

<400> 802

acttaataaa atatttaaaa tttgaactct acttctcttc ttaaaacttt tcaaattaaa 60
ancatttatt ttagtaaact atgctcatta ctattaaata cacatatcta ataaaatctg 120
cacatgaaaa gaagagcggt taaccgctgg gttgctccct tgagaaatat ctaggctata 180
tattcaaaga aaaaaaatta tagaacacgt agttctgatc gctattgata taaatatata 240
aaataattgt aaaatattaa caatgt 266

<210> 803
<211> 499
<212> DNA
<213> Ctenocephalides felis

<400> 803

catataatac taattataaa ttaaagtgta tggggttgcg ttgcttctgg gaaaaacatc 60
gcaaggaccc atacaccgaa tgaaaaaaaa taatttatta acgaaacgcg aattactaga 120
acctgccgtg gggggtaggc gacaggaaca catttaaacg cggaaatcgt tttaaacgat 180
attaaaatta cgaacaagtc ctcgcacgaa caaaaaaaaa attaaaatgt ggacgcaaat 240
aactcaaaat tataatttaa caacattata aaaaataatt aaaaattgtc aatgtgattg 300
aataaaaaaa attcatgcac aataaatccc taaagcaatc cgccactgcg caaaacactg 360
tatgaaatct gaaacaaaac aaatgtagtc aatcgtaaat atttgaaaca taagatttat 420
attaaaattt aatattattt aattatattt aaatttaata atctgaagat tgattaaatt 480
atttatatta actcaaagg 499

<210> 804
<211> 500
<212> DNA
<213> Ctenocephalides felis

<400> 804

```
gaacaagagg tttaggnatg agatctgcaa aatttgggca agatctaaaa tcgaaggaaa 60
ttattttata tgaatatttt gaatgtaaaa aaatgtgttg aaaaatttat attgtaatgg 120
atggtttcat aaaggcggtg ctagaaacaa aattattata gttggtttta acatatattg 180
caagatattt caaagatatt tgtttagatt atttttctac atttattttt catgctctta 240
tttatagaat cacgattacg ataaaatgtc taagtttaaa tattgagtct gctgatcttg 300
agtcctaact tcaatatcag aattgttggt ggcattgttg tagaagcact tctgtgaaag 360
atcttttgcc catatgaatc tgtggtcaat caatttgcat gtgggttgct tataattttt 420
cctatttggc caattattct ttggctacga ttttgagaca natcttttcg gtatcaaata 480
actcttgatt cgaataatca                                              500
```

<210> 805
<211> 216

<212> DNA
<213> Ctenocephalides felis

<400> 805

```
actaaagatt ctttatattt ttaatatgtt ctcattgcac ccgtttaatc tagttcaagt 60
tttataatac acaaacatct tataaattta agtataaaat ctaaaaatta tcacacaata 120
aacgctagtc tttcagagta aataatataa cccttaagac tttgattctt aaattaatgg 180
taatatcaac cacaaaatat tggaaaagaa aatcgt                            216
```

<210> 806
<211> 500
<212> DNA

<213> Ctenocephalides felis

<400> 806

```
actgtaaatt tttacacaaa atgcaaatat ttaatatctc ctataattta tggattaatt 60
ctgccttatg tcggaagtca accggatcat aaaaagtgat ctaattgcgt aaaaaaatta 120
gtcacacttc tgccaacgta tatcttggag catcgtttta ttcacagata tggtgcaatc 180
atttcaatta tacgttggta gatcttctag tcttagtctt gtttttataa tcatagagga 240
gactggagaa aagaatactg gaaaacctta ctgggtgatg gatatccaca atatcgttag 300
cagatagtat ctgggataga tttttggagt ttttagtcat ctagtaaaat attttttcac 360
taatattaac aacatccaga ataagaattg atcaaaaatt tttacttcat tttataataa 420
gtttgttagt aattcgtgat aggatatatt tttattactt tgattatttt ttaataactt 480
tccttccatt ttaacaacgg                                              500
```

<210> 807
<211> 355
<212> DNA
<213> Ctenocephalides felis

<400> 807

acaagaatgc aattattgtt cttcaaagga aaatgattga ttttctacac caacagataa 60
aacagtctct aaaagaattt gtaaaacttg aatatttttt tcggtgatac ttgcaggaac 120
cgtaaaatac aatcaccacc accaccttta gcttcagcat tcttggtttt taccttttc 180
tgtttttaac acctgtgaac tgccttttcc tcgacttcaa ctggctcaac gatattttta 240
gataccaaaa aatatcgtat ctggataaat ttgttgtgtc tgcttttaat attttagact 300
ttcattttgg tattaacatt ttttgtgaga acttttactt ttactaagtc ggggt 355

<210> 808
<211> 424
<212> DNA
<213> Ctenocephalides felis

<400> 808

acaagcgatg gaccaccngc tcncccgcct nngttggtcn gtagtaaaag aaatctaaac 60
ctnnngttct tatttattaa gnncattgng taattcaact tacacctagg taatttatta 120
ataattatcc aattattaat aaaaaaaatt aatattgact acttaattac atttatattt 180
gctaaaatag tatatttaca tcaatttttt ttttcaaagg caatataaca gaaaaggctc 240
ctgataccat gatcaggcag attctttcag cttgtggtcc agttgtttct tggaaacggg 300
tttctgcatt tggattttgt gaatttaggt gtgtattgta atgatttatt aataagacaa 360
aaatgtctag gttattaaat tacgaaattc attccagttg tccagaagct ggtttaagag 420
cagt 424

<210> 809
<211> 500
<212> DNA
<213> Ctenocephalides felis

<400> 809

acatcacaga tttttttttt tcaatatata tttcagtgga gagaaattta tccaacangg 60
gggtaatttt gctcatacca gtgaaatatg tttattagcc caacttggct tcaaataata 120
taattttttt aaaccttatt cacatattta ttatcactgt acgacttttt caatgcgcgc 180
tcgaacgaaa ttatgaacga gttttaaaaa tatttttagc agctcttaca gtgttggtat 240
ttgtaaatga tgatatttat aaatttcaac agatacttga agtacacagt caataaaatt 300
tccaattttt cacaacnggt taaatttgca ccaccggnaa ctggcnccct tgcaaaagnn 360
tccntntngg aaantaattg gccgganncn aanttanctt atnttttttt cnggtccana 420
ccgccctttn aaaanccctt ttttttnggg aaaaaatttt ccngggtttt ttttttnccn 480
aaaaanncnn ngggnnaaac 500

<210> 810
<211> 298
<212> DNA
<213> Ctenocephalides felis

<400> 810

```
ggggacatat ctaaataaca nccaaaacaa ctctccnttt tgtttgtgct gggttgcaag 60
caagcgggga ctttagtcaa acgtctntca aatgcttnta aatctttaca cgcagcttgt 120
tcaagtgaca tgtttactat tttttgtgtt acatgatcac ataagatnat natgccacag 180
ctaaaatcnt tttgaaaaaa taaacctggt atttattaca cactacatca atttacactt 240
caaactaacg tngcattttt attgaatata taacatgata agctaaactg aaaaaatg   298
```

<210> 811
<211> 243
<212> DNA
<213> Ctenocephalides felis

<400> 811

```
actgtatctt tttgnctatt cacaaaaagt atttgtcaga agtgggattc gaacccnggc 60
cctcatagag gaccagaatg ctcagccagt tgttaaccgg caaggaaacc ttgagtatgg 120
cgccttagac cgctcggcca tcctgacata cagcaaagct ttgcaattgt ctcatcagaa 180
acatatttgc tgngtgtaat ttacgctaat acaatgaact ttgtttgaga tttttttaca 240
agt                                                               243
```

<210> 812
<211> 500
<212> DNA
<213> Ctenocephalides felis

<400> 812

```
acttcaagta taacccaacg attcacttnn ntctgtgcac tttggcataa atatccngna 60
aaatatgtaa aacaggaacc agggcaccgt gataatgtaa tcaaacgccg gaaaatgatt 120
tttgatcgta tccatacctt tatagcgaag tcgggaatcc ttgtgattat attttcctaa 180
aaaaggaaag aagttctcgc atactgaaaa gaaaagtttt gtctggtggt ccgatttcga 240

actttcaaac ttattcattc ggttaccgcg accgcgaatg aaatattatg atttttcttg 300
ctcttgtccg gaataagata gattgacttc gaatattacg gaatataagc tgggctgcga 360
gtaaattgtt tgatttgggg ctttttatta tcgataaaat atgtgctttc tttgaagatt 420
gtttgaaact tctgtgttat cttcaagttc acaatgataa cattaagctt aataataaat 480
atgttagagc aaacaaaaca                                             500
```

<210> 813
<211> 500
<212> DNA
<213> Ctenocephalides felis

<400> 813

```
actttttcaaa ctatactgga gagtttttt cttntatttt ttattgtaat tactatcaat 60
acgatcacct cgttacggtt tgacgttgag ttctgggaca ccctgggtaa attatgttaa 120
ttatatgtat ttgagcataa atacatgtaa agcaaatgta ttactgatag acttttgact 180
ttaactttac ttgaattagt aataaaacca catgatagaa aattattgag ttttttata 240
gaaaagttta ctgttgattt tttttattaa atcattgcaa tgtgctaatt cacattcttc 300
attactcaat tttatgccca actcattata atattttgtt tttcttaagt tttcatacct 360
cgtatcagaa ctttctcaaa gctcatccct agctttactt taacttcctt attatttcac 420
tgataataag aacttttgat ttttttaaac atatatctat caagttaaag tttaaaatat 480
tcatgaaatg aactttgatg                                             500
```

<210> 814
<211> 285
<212> DNA
<213> Ctenocephalides felis

<400> 814

```
tctgantatt ttatnaatat ttaagacatg tgcnaanntg angtnnnttn natanaanaa 60
tngtgcnnta tcaaaacgtn actttttttt tctctccaaa ggcaattttt ttaaaaaaaa 120
ctccgattat ccgaatattt gattatccga atgggtcccg gtccccatta attcggataa 180
ttggagttct actgtataaa actttgtata tttttgaaaa ttttgaaaaa actattgata 240
tttgtttcag ccatacaagt tagtttaaaa attaataatc tcggt                 285
```

<210> 815
<211> 500
<212> DNA
<213> Ctenocephalides felis

<400> 815

```
actcttcttc gttcaccaat gaagtttgat tgtgcatatc aaacttcctg tgtccaccat 60
agccctgaat tctttgccat ctatggtgac acgtttcata tatttttctc ctgtctgatc 120
ctttttatca gcaatccaaa ttatttcacc actaggattt ttagtgtcgg tttcaatttt 180
gatcttatta cgttctgggc aatcctgaat aaagtgtcct accttcctgc atcggtagca 240
tatcacttct ctttttcgag attgttcact atcttcttta acctcacact tcactttcgt 300
```

```
ggtagttgca tttcgtgtct ggctgcactc atccgtttca agctttaagt catttaaggc 360
actttcatga gccaaaatgc ttgtgccgag ttcgtcataa tcggcgtgct tctctgcagc 420
taaaatgaca tacagctgtt gtctagcagc cgtaagtact tgccgttcgt tctcgaaact 480
catgcgtaac gcttggcaca                                             500
```

<210> 816
<211> 346
<212> DNA
<213> Ctenocephalides felis

<400> 816

actgcatgcg tagtttgcgg aatgcttctt tccttntaga tatgaatacg tcgcattctt 60
gatcccacca cacggagggt ttgaataacg ttctttttgt gatcttaatt ttttttata 120
ttcttttctg ccgctatttc tataattcgg attacatgtt cgtatttaaa attctccttt 180
tctctagata taggcgattg taacaatcgt gttatgtctt cagcacatcc ttgccaattt 240
gctttcttga tgttccactt tttgtgggga taatatttat gtaagttaag aattccacac 300
ctatgtttat aatgattaga agatggtcag aacccaatgc ttctgt 346

<210> 817
<211> 500
<212> DNA
<213> Ctenocephalides felis

<400> 817

accattatta aaattattgg attgttttat ttgtgccact cttaatataa atttgcatga 60
tngtagtatg gtgtggatag tatattttat gtagaatttg agggtaacta tgtatatatg 120
catcgataat ttttcacgtc gacctccatt gcatgtcaag ttgcagacgt tctgtttatg 180
tcaacattat caaggtaatc gcactcaact caacaatatt tccatatccc ctttttgtta 240
tagctaaaaa caatttgttt attgtattta agcatttaat aatgtgttac aacaatagaa 300
attgatttga tgtcttgggc ttgccatcac taacagtgct acgagctttg gcttggcaag 360
caagttctat actcatctgg gcgaacttga tatcaagcag tagtgatatc cgttttcact 420
tgaaaacatg aattataaat aaatttgcag ttgaaatagc acttctggga attctgcaga 480
acgtaaaaat ataatttcaa 500

<210> 818
<211> 500
<212> DNA
<213> Ctenocephalides felis

<400> 818

acaatagcca taaagcatga tgaaataatg tncacaatag tctctaagaa ttccattgtt 60
ngaaggaaaa gttgaaatat tattatgtaa gttaaacaga gttatatgtg aagtattatt 120
ttctggtgtt aacattgtga gcacttcaat attggaatta ccccatatac ttgttatata 180
ttgtgcgcgt agaaaatgct cgtgtaattc ggaccgacac ggtggtgaat gtgatgcgtt 240
atgatttaaa tattttttat taaatggctt gttcactttc tttggtttat aagtgataat 300

gatcttatta catctcgtat cattaacagt tctttaattt ttcaataatc gggaacattt 360
cactgctgct ccctgttata taagcactaa gtttttgaat acttcttggt attctaaatt 420
tgtttaacaa ttgtaaaagg tcgttcttac cttttaggaa aaatgatggg tatagcacaa 480
ccattagagt atgaaatttg 500

<210> 819
<211> 431
<212> DNA
<213> Ctenocephalides felis

<400> 819

accgataatc tctcaagaaa tgctaatctt ttaagtgaaa ttctcaaccc tttagttagc 60
caaggngttg aagtattatt ttttgggcaa acggtttttt taatgagagg aaaagtttga 120
ttgaagttga ataaaaatgt agaaataaag ttgtcgaaca ttgtatttga gtctttggac 180
ttgttaagca aggtccatga ttccttttca agaagacatt tgaaaatcaa tttatttgat 240
tcatcaaaat aacgtttgtg taagataaaa gtagatttag aagttgaaaa attacaaaaa 300
tttaatatta tgcctcgatg gtctgaaaga gtggtattta taacacgcga atcaaagtct 360
tttaaattgg tgaagaaatt atcaatgcaa gaagaagatt gatgcgaaaa acgagtaggt 420
tcatgtatag t 431

<210> 820
<211> 500
<212> DNA
<213> Ctenocephalides felis

<400> 820

actgaaaata acaaattttg aattattata gttttacagg tttattttat ctaataaaaa 60
angntagttg acattttcga aatatgtgaa atatatttca acttacggaa aattgcaaat 120
aaatctgtca tgggcatgac agtatatgta tatttagcag tatatgtaat tgatttgtcc 180
acttttatcc attgggtaac atcatattta ccaatatctc tgctattctc acatctgtca 240
atagctgctc cacatctagc agttctgcct ctgtccgaac aggaaattta ttataaactg 300
ccagtgtata atgaggcaaa tcatccttca tatcacactg gcatcactgc ttattaagcc 360
tattcattaa aacttggtgc cgtaatgaat cagccttttt attaatattg agtgcaaaat 420
ctgaacaata tacaacctta tactgaggtt taccacaata ttctatcaaa ggtccttcga 480
aatgcataga catctcacaa 500

<210> 821
<211> 500
<212> DNA
<213> Ctenocephalides felis

<400> 821

acaaatagaa aaaatactta ttttttttata acttatatac catatgaaaa ataataaaaa 60
ctttatttgt ttacgtcact atgctctatc attatttacg aaaaattttt atatgcttaa 120
agctttttgt taataatttt caataaaaga ttcaactaaa taattatata atataaataa 180

aggatttaaa gtttatttga tattatataa gttattagaa tgaaatctca ctacataaga 240
taaaattcaa ttttatctat gtctcatcga aatagttgta taattttgaa ttttgtgacg 300
tttttttattt tatttaaatt tagttatatt tgaattgnta gtatcatatt ccattaacca 360
tatttcatag gtatcaatac aacttttata ttcatattat atatttaaaa aaaatgttta 420
tgaaattttt aaataaatgc aatagcagaa tgtattttca ttagttatac cgaatatagg 480
aaagccagga gcttgtgnaa 500

<210> 822
<211> 500
<212> DNA
<213> Ctenocephalides felis

<400> 822

acattaaaaa tatttgttag agttaaattt gaacttgatg acagtgcaaa aaaataaata 60
taaannattt aaaatttgta tgaattcaag ttaaaatatg tatgaatctt aataattaaa 120
ttttgtgagt aattatatat atatagataa atagattttt aattaataga taacaagttt 180
ttagaactac aacaattgta ggagaaggtg aacatcttaa ggaccaagag cgaaatcttt 240
gaatatcaga aagaaatgtt tcaaaatgat acgaaaaatg ttttaaagtt ataagaagaa 300
attaatcact tgaatttatt tgaataaaat cttatttaaa ctccatttat taatactcat 360
ttccaattga acttctcacg aaatttaaaa aaattgaatt tattttattt taagttagtt 420
aagagttcat attactaaaa gttaaatttc attatgtatc ctttaatatg ttcaccgccc 480
tccccataat tttattaaat 500

<210> 823
<211> 240
<212> DNA
<213> Ctenocephalides felis

<400> 823

actccatgtg tcataattct attattcaat atgtgcaaat tacgttctaa tataaatcgt 60
gacaatatca taatatattt atcgtggaaa taatatagca attgcaagct aactaatgtc 120
tcctagcgta ggagtatttg taaccgcgcg gtatagcgtt tccacttggg ccatcatcgg 180
ctgccaaatc ccctccaaga cctgctccgg ccctgcccca tggagatcgt gcttcggcgt 240

<210> 824
<211> 500
<212> DNA
<213> Ctenocephalides felis

<400> 824

acctaaattt caaaattgat ttatataatt ctatatcctg cgtgtcaaat taaatctata 60
tttaatacct aatatttaaa actaactacg aatgaattca gtgtttcata catacataac 120
atctctggtt tctagtatac tattgttagt ataatcagaa ccactataaa aaaagtcact 180
ataaaagttg aaaacctaat ttctaatgac tattatacta gagcacatga taagaatgca 240
gaagcaatat aaatctatct ttttgtaata aatttctata ctattactat tacttttctt 300

agtggctcgt aatatgcaaa caatcactgc caccgcagta acttcagccg taatttatgg 360
cacatcactg ccgatatgat aattttctat gcttttatta ctctgaaaag cgtttcgttt 420
tggccacggt gtcggttgga aaattttcat cggtggaaac ttgccgtaga cgtcctcctg 480
ctgctccccc cttccacacg 500

<210> 825
<211> 370
<212> DNA
<213> Ctenocephalides felis

<400> 825

```
acttgaaaat tactaccctt gtagagagct agcaaagagt ttggtgggac taattgttta 60
cttccaaaac tccgcataag aacatttgca ataaatgatc aataaatata tcttcttcaa 120
ttagatctta gtgataaatt aatagtgtaa ttagaacagc accaaataca taaattatgc 180
caataaatca gtgtttcaca cagcactgct acaaaggtta aactaaccta gcgtcttgag 240
ttaatcggag gagagactca acacttaatc ttgcaataat cttgatatga tattgtgatc 300
taagcaataa ttttatctta agataagaac tttttcacca tattatggtt agcttaactg 360
tattgtcagt                                                        370
```

<210> 826
<211> 166
<212> DNA
<213> Ctenocephalides felis

<400> 826

```
acaaaaaagt taaaatttta agaattagca gttgtttaag tgtggcatca aaaagttgtc 60
acgtgaccac gcccacgatt gcaaaagaaa gggtgaagaa gagggttata tcatggccga 120
acagcgataa ctcagaaaat aattaaatct ttaaaaatcc tcgcgt                166
```

<210> 827
<211> 304
<212> DNA
<213> Ctenocephalides felis

<400> 827

```
accggtagaa aaattgttga taatgtgcgc attactnatt cgattttcaa tatttgcgat 60
gaccantaag tttggtaggt tttcaaggaa ctctcgtcgt gaaaatacta acattcatgt 120
tacaacacat agcaaataat atataaaatg aagtttgaat agcaaatact agaaaaaaga 180
caaaatactg gtttacatgt atcaaaaagc gtatatttca tacaaaatgt atgggtggtc 240
tagacagacc actccgaccg tttggagtat aaaaaagtta atatctttat aattatactt 300
ttgt                                                              304
```

<210> 828
<211> 352
<212> DNA
<213> Ctenocephalides felis

<400> 828

```
acgggaagtt ccagcaattt gccttcaagc acgatnccat acatcactag tttttttgt 60
ttatggggat atattacttt cgattattac taaaacaaca gntataaaac tttactattg 120
ctaatagaac gaatntttct gaaaaacgtg atatgggtta aacataggaa attatagtaa 180
attattcatg agagtatatc aataagtcaa taaatatttg tcttctgaat ctttttacat 240
tnggctctgg ttaaatttgc ttactaaata tagaattttt agctttataa tttatctata 300
ttcattanat ccggtccaaa ccataataaa tcagatttga tacaagatac gt         352
```

<210> 829
<211> 500
<212> DNA

<213> Ctenocephalides felis

<400> 829

```
acgttgcaaa aattaagcaa atcaagacta ccgtatttac gaatatgtcc ccaattcgct 60
gcatacatac acacataccc gcacactagt gattcgtgct ctataaatat cgtgcgtcat 120
tgatagaaaa caagtgtatc tataagcttt gacattatat tacacaaatg atttatcgta 180
gatgaaataa aagaaacatc tgattctctc attactacca tattttatgg atatgggaat 240
aatggaaata agtaaaaaaa atcatatatt ttcttgctaa agagtagtca tttcaaactc 300
aattattagg gttaaaaatt aaaaagaaca tataccgtaa ttaggtcaca atattctgtg 360
gtcatcgcca aacttagtaa caccaaaaac accactaaat ttaagcataa ttgagaataa 420
aagactaaaa taattcaggt ctacttttcc taatgcgtga cactcacgga anacgttcaa 480
tgcctcaatg tgtgacttaa                                             500
```

<210> 830
<211> 500
<212> DNA
<213> Ctenocephalides felis

<400> 830

```
ccttaaatct gtatcttaaa aacatttaag taatgcattt ttaatatttt gacataaatt 60
attaaagaat caatagtttt aatatataat atttgaccta gccaaaaacc agatattgtt 120
tatctgaatc caggtattgt agttggcttt ttcttgtgat gtagtttatt acaagtcgtt 180
taaaaattgt attggttagc tattttcaac aatcttattt gaaaatctgc tttgttagcg 240
aattacagag ttaaaactat tatattttca cttgaaggca tattttagat gaaaaaaatt 300
ttaggtgaaa gccaatatca gtaggggaga caatagatca cttttcttct tataattaat 360
gagtcatcat cacgagtagt tttgttataa attttttttt gaaatcagtc aaaaatgtta 420
actactaatc atcagtttac taatcagcaa aatattcggt aatttaaatt atatactgag 480
actagtcgaa tcatcattat                                             500
```

<210> 831
<211> 550
<212> DNA
<213> Ctenocephalides felis

<400> 831

```
aagaatatga acaaatagta gtagcaccag ttcaccctgg tttagatgta caggaagcac 60
aactaaatga agataatgag gatttcgcat caaggcgtcg ataccatcaa tcagctactg 120
tgcatggaca ttacgtaaac attgacggat agttgtttta attaatgatc acctaataca 180
tatttgacca gtattgcaaa tttttgagtc acaaagctat tgatttagat ttttatatat 240
ccttataaaa gctatttcta tggtataatt tatttaattt aacaaaaatt tgcaatatta 300
gcttgtattt taaaaagctg attaaaattt attgtgaagt atctaattta ttaaaaaaaa 360
tctaatataa tgaataatat agaaatgaat gaaaaccgac tcgagtgcag tcaacattac 420
tgataatgtg atttgatgca ttttgcttta ttaaaggcta aattagttca aaaaggccag 480
tgtttaattt aatatttatc acttatttaa ttcaaataat taatcactcc agttgtatta 540
taaataatgt                                                        550
```

<210> 832
<211> 550

<212> DNA
<213> Ctenocephalides felis

<400> 832

```
gngtgctntt ggtgactgtg actgtactcg tactgtcaac atacttgaag tatgcagttt 60
catgcaaact tctacattgt cattctcgaa cccaaatgac ttacctattg ttttctttaa 120
gtaaaacaat gattgtgcta aattagcaaa agcgttcaaa ttttattatt ataattagga 180
actgttgttt ttagtgactg ttattatcca attctaatca aaaatgacag acacacctga 240
taatgcccga attactacca tggttgaaga tgtttttgca ataacattaa acccagaaaa 300
atttgtggac tgtgaacaaa aaccagatta tattcaaatg atttatctag aagaactagc 360
agaaagtttg aagccgcaaa aacacattga cattgagact ttagaacaag ctctttttga 420
aaggttgatg ctaacaaata tcacagaatt tgtntaccaa aatccagtaa gcccccatat 480
atagattatg tagttcagaa taaagcaatt tctattaaat ctgttatgaa agacttagaa 540
gtacatgctc                                                        550
```

<210> 833
<211> 550
<212> DNA
<213> Ctenocephalides felis

<400> 833

```
gnngctnttg aaacagatcc gaacaatcga tcgatcggag cagatttttt tttgataata 60
gtaataaatg tgtattgttg tggctgaagt ggattaaaat aaaattaata ggttgataat 120
aataataagt tttaaatttg aaggttatcc attacaatgt gtaaaatttt tttgctaaac 180
aaataattct agaccaaaat ctaagtgcct atcatatagg cacggactat agaccaaaaa 240
atacaaaatg tttccaaaac attatgattg ttcttaaaaa caaattgtgg acttttagtt 300
gaaaatgaaa tgtgatttaa aaattgtcgt ccaaaaatat tggtagtagt gaatgttgat 360
tgccaaaaac agtagctgga aaatagtgac aaaaacaaag gttttggtag tgtttttatt 420
tgcaaaatat tggcgtccag attgtgatga taaaattgat agttgagttg acatagttgt 480

gtacaattaa taaaacaaag tngtntaact tcaagacttg ctgctcttca caaatctgat 540
tgaatataat                                                        550
```

<210> 834
<211> 550
<212> DNA
<213> Ctenocephalides felis

<400> 834

atcgtacgcg cgatacggcg acgtccgaca atattacgca tattcaacta atttattcat 60
atcataacca accgttcagt tcaaactaaa ctaaactaaa ataaactaaa ccaaagcgaa 120
agctaacgac taacggggag gataagcggg gggccacgag gcgaccccag tttcatttta 180
catgtgtgca cgtatgcgaa ataacagttc gtcgacaaga cgttgtacgt ccgccctata 240
gtgaaacacg aaaaacctca tatccgatgg gataaaatcg accgaattca gcggacgact 300
gaagatcgcc gcattcaacg tcctaaagtt aacaacttag catcagttgc ggcgcggtga 360
gagtgcccgc gataggacag cgaagcgatg atcgatccca gttcgtctga agaagagagc 420
gaggaagagc atggaggagg cggcggaggc gtcaagcagc agcaccacca tcacagccgg 480
cagcgcgccg agcggagtgc ttcgtcacag cgaaggcaac catacgctca ccggcggcag 540
tccggcacta 550

<210> 835
<211> 550
<212> DNA
<213> Ctenocephalides felis

<400> 835

gacagtttgg ttgtgtcagt caacacttca aaagtcagcc aatgaaattt gttttatatt 60
tgattggcgt tcattcgaat tttctttgcc gaaaagatta gtttataaat taaacctatt 120
tgtaattgct taatgcttca ttgattgttc tgttcatctt aaggttattt taaacatcta 180
acaatctggt ttttggtaat aaaaatgcct agattgtcta gattaacttg tgctgtggag 240
gattgcgagt ttagtacata tgatagaaca ataaatgaat tacgcagagt tcagtttttt 300
gcattaccat ctggattctc taacagagat cgtaggtcga agtggattaa atggctcagt 360
gatatcaatg gtgaaaattg ggaggtatgt aagtattcac atgtgtgctc tctacacttt 420
attggtggga aaccttcaaa catgctttta catcctgata tgctccatca ataaagactc 480
gagatgatta tacccattag aagaaaagat gcctggcaag tttgaattat aaaagcaaaa 540
atgcaatnta 550

<210> 836
<211> 550
<212> DNA
<213> Ctenocephalides felis

<400> 836

tgcacgccat caacgaccac cacgaagggg tgtcatgcgt cggcacggat ggctcaacgg 60
caaactctcg cttgctgtcg agcactgtaa gatcgacacc ggtcgttctc ctcagcacgg 120

acggcggacg agctcgtcaa tgtaatgacg tagcagcgga cgctgcagtc cagatacaaa 180
cagcgacgga ggatgccagt catcccggcc aaaaggacga atggttcgtc taacaagtag 240
gatcaaacat tggtcgcagg gcgatgctca tgaattggaa atctcgctac ctatgacaat 300
tccatgaatg aaaatcagtt gcacatttta ttcataaata aatatagata aacaataaat 360
aaaacgattt aaattagtat acactgtttg tgttttactt tgatatgata ttgcattata 420
taatatattt agcaacccaa tttgataggt gtattttgtt gaatattttt tcagatggtg 480
aaagatatta gataaataaa ataaaactaa attattttct caataaatta ttacttcaca 540
caaatatttc 550

<210> 837
<211> 550
<212> DNA
<213> Ctenocephalides felis

<400> 837

```
gcaaaagcaa tgactgtatc aaatggcaat ttagtagatg ttagattttt tggagcacat 60
gataaagcat ggatacctat gaaggattgc ttgctttata gtgagaaaga tccaaatttc 120
agtgataaag gaaaacgctc tgatttcatc gaatcactta gggagttagc tatatatgtg 180
aaaaatcttg agcaaaaatt tggaaaattt tgtcatgcac cattcaaaac tccatatcct 240
aatgatcaag cagctattta tagtataatg ttaccttcat ataaattcaa atcagatatt 300
gcaaataaaa aaataataac aaaacaaaaa gtttgtgaca taacggataa actactagag 360
gacacaaaag gtaacatgaa aataaataat tcgttagatg aggatagtta tattgaagga 420
tatgatactg aagatgagga agcactaaaa gatgtatcaa atgaatctgt gatatgtaat 480
gatatgaaaa ttaaacaaac ccttgcgtgc cgtgttagaa tgagagnggt aaataattca 540
caggaaaata                                                        550
```

<210> 838
<211> 550
<212> DNA
<213> Ctenocephalides felis

<400> 838

```
atgacagcca tataagctat cataattaaa agtgctttta ccgtttagta ttcttttaga 60
atattaacta aacaatcatc atgaaattct acggtttaat gtttgtgttg tgttttgcaa 120
ctctttccac atgtctacca gtggacaaat ccgtagaagt tgaggttttc agcagtgaaa 180
gcgaaattct gaagaagtac ccgcaagcca agcccgctga caagacccac attgatgatg 240
gtaaagctac ttatgaattt ggggagagag tacaaggcga caaattgatg gtcggcgcaa 300
ctgatgtata caacgcccca acccgcaaga tgttgccctg aaattcaact ttcccggaaa 360
agtaccctat tccgtcacct acgcctctgt cgaagtccta caagattctg aggtcggaca 420
ggctttcatc gtctcggagg aatcggccaa tctgaaatcc atttggctgt tgtagctgca 480
aaattaagca agtcaatatc ctacgaaatc tacgccatgt ctcgttaaat cttaatgact 540
gaatcgtcgc                                                        550
```

<210> 839
<211> 549
<212> DNA
<213> Ctenocephalides felis

<400> 839

aaattgacga gtcaagaaga actactgcca ttaaactata agaaatgtga aaattgttga 60
aaagcagtga cttttataaa atatcaacaa caattctgtg tgccatatgt gccaatataa 120
acttattttt gtatgtttgt atttaatatt tatttaaaaa tttagcgaca cgaaaaaata 180
agaagcaata ttattgatct aataaacagg ctgcttaaaa atacctctaa ctacattagt 240
gattaaatta taaattagca attgcgaaag ctttttataa tactaacaaa gatattaatg 300
tggcagctga agtaaatgat acatctggta gctaatatac tgtgatgatt atattttgct 360
tattacaaat gttattaaat tgtatattat tattaatgag taccaatttg taaataggaa 420
atatctttat tcagcaattg tgtggaaagc atagtcaatt ataaatatct ataaaattat 480
tgtattgtaa atctaatgac taattataat aatgtatgaa tatgaagcaa tctataaatt 540
tgngcctcg 549

<210> 840
<211> 549
<212> DNA
<213> Ctenocephalides felis

<400> 840

aatcggcacg aggtttaaag gtagtttatt aaatgtgagt ggtgtttaaa tttattaaaa 60
ataacgatca aatctaagtt ttacaactat ggctgctgat gcaagaactt attcgatatg 120
tttgggtgta acttgtatgt ttgcttcgat tttgtcggcg tacgcacttc ttgtggaact 180
ttcggctgaa cttcatcctg atcaaccagc catgtgcgat attggtgaac atatgagttg 240
cagtagagtg ttgacatcca ggtatggcaa aggctttgga attgttggtc taatattagg 300
agaaaattca aaattcaacc aacccaacgg atttactggc attatcttct actccttcat 360
ttctacttta gctctcatag agaaacgttg gacagcaaaa attcaattag ctttaagttt 420
catatcgatt cttctctcaa tttatttggc atgtattcta tattttgtct tcacgatttg 480
nggtagtttg cgtaccattt acttttaaat taattaattc atacttccta taaaagacac 540
agttgtagc 549

<210> 841
<211> 549
<212> DNA
<213> Ctenocephalides felis

<400> 841

ngtncngcac cagatttgga tntncnnctg tgagaaccat ggangggcaa ttgatcangt 60
caacttggtg ggttantata nggantacng catctaccat nttcgccngt ttaanaagaa 120
cntggcttga gtgantantn aataacagnc taacctgccc ctacgangat gannaangcc 180
ntttcaantt cantgggcgg aaggtancag acatatncnc ttttaannng aaagctggan 240
ntgaatgatn ngcatgaaat atgtaactgg cttacgtgac ctgttntact ancntaattg 300
ttatgcaaaa agcgtctttn ttatnaacng ncaacaagac ctttccntta tnnatntcac 360
tatagancaa ttttnngacn angacnatgt tgatttttct nnncanatcc tanannnncn 420
ttngganaca ttntgtagnn tttnnnnnat gangncanaa ttcnngagtc gatgttctcc 480

antatgtttc aacgatttnt cctntacnta tncnnngnaa catttncagg nacaacctca 540
gnnangnnc 549

<210> 842
<211> 549

<212> DNA
<213> Ctenocephalides felis

<400> 842

```
aaaaggatag cacagattgt gcaccagaga aggacagaag atcaggacaa cttggtggta 60
aagcagagtt aggaaaagat ttaccatgat ggttaatggt tgatgaaagt ggtttgagtt 120
gttctgagaa caaggataaa ggataacagg gttggagcaa tttttttgc gaggacaatg 180
atggttattc aagtcatatg ctacatcatt gttattcttt tttttgtctt cagccagaaa 240
aatgtaaaca aatcacgtga ccaatttact accaaataat ttttacaatt tctgccttcg 300
atttaaactt ttagccagga cacaaacctt tagccaggac actaaagagc aatttttttg 360
cgaggacaat gatgattatt caattcacat cctacatcat cattgttatt catttttttg 420
ctttagcaaa aaatgtaaac aaatccgtgc gattttacta ccaatttatt tttacaattt 480
ctgcctctac tgcgagatta acttttagca ggcacaacct ttaccagtca caaccggttg 540
aattttgaa                                                         549
```

<210> 843
<211> 549

<212> DNA
<213> Ctenocephalides felis

<400> 843

```
gcgaagagag ttagatcact gtagaagaca gatttcagaa cagtccggtc aaatatctcg 60
tttacaatct gagttaagta ttagccaaaa aaatgaagga caatatacaa caaaattagc 120
tacagctttg gaaacagttg aaaaaaatat ggcacggagc aataaaaggg caatagatgc 180
tgaaagtaca gttgcaaagc ttaagaaaca gatttcacaa atgacgtcag agatgatggt 240
tcttcgaaat gaaaatacat cactgcgcta tggtccagct gcaaatgatt ccaatagcat 300
gatgagatta tcaaatgagt tgcgaactgc agctagtact gcagagtcgt cactgaggca 360
actattaacg ggtgttgata atttaaggac tcttgtagtt ctttagaaag ctctaaccga 420
atatttgaac cttctgatga caatttctgc gaaaatgaag atgaagatgc cggcctgact 480
ataatgtgta gtgaataaat ttntcattca aatgtcttgt attaaaataa atattctagt 540
ttatatgct                                                         549
```

<210> 844
<211> 548
<212> DNA
<213> Ctenocephalides felis

<400> 844

```
aagcacgtga tataaaaatg aatatttgaa gttatgtgtt atactcatac ctcaatttgt 60
atcgtgctta cgtccaagtg aattattgac tgatatgtct atagtggctt ctatcatatt 120
```

tagagtattt tgaagctttg gcggtatgaa gtaagtagca gtatgcggca ttctggtcta 180
aagcaacttt ttacctcaaa acaacatttt acttcgactc tgactttgca gaactgaatg 240
gtgagtcaat gagcgcacca cgcttaacta gttaaggaac gatgcaactt cgaataaacg 300
caaaacgatg caaagtgagg tatgggtgtc tacatttgat gcgatataat ataatatttt 360
gtacgtattc agaccgcacg tgaagagcta accgccaggc tctgcgtctg gtgacgtcca 420
gcaaattgta gtgatatcaa tgtccgattc atcattaact ggctaccaga catctcgcag 480
cagtctgacg gattaccagc atcactgact cacccaatcc ttgcgtcaca ccactctcat 540
tcaaccgt 548

<210> 845
<211> 549
<212> DNA
<213> Ctenocephalides felis

<400> 845

acgaactgaa atcaactatt aataaaatat acctgtatct tttacctgtg acggttctat 60
ttgtgttgta ttttgacaaa tacagtttct gtttgcgatc gaattatata tattataacg 120
agaattgtgt cataagtaga agaagttagc ggccaagatg tggcgattgt tgggagccgt 180
tcttgttctt tctgttgtca acagtcaagc ccaattcgag gaccagttct tgagttggag 240
gcgagatgta ggtgccaatc gtggaagcgt ttggcctgga ggctaccaaa atgtttaccc 300
tcataaccag aggcatgttc ctaaaaaaac acacaaggaa gaagaggctg tcactgaaag 360
ggaccgtgaa ccacttgagc ctcattcaaa ttgggcaggt cgcagaacag ccgatacatc 420
cgacatcgaa caaatcgaga ctgttcctga attgctgaaa ccagtgacga agatagtcgt 480
ctgatgtcga ttcctggtgc cttctctcct tggaggactc ggaggatttc tgtgacaatg 540
gcatactct 549

<210> 846
<211> 481
<212> DNA
<213> Ctenocephalides felis

<400> 846

ataaagctta attatttttt gggaaaataa gtctctacgg ccacagtagt tttatttgtt 60
gctgtgagca gtttatcttt atactattga gtgtttagaa aaatcacaac taagtcataa 120
aagccataga ttctactttg atagtgttga tgtagcttga taattttcat taatcaaaat 180
cagattaaat tttgcgaacg cggtgtgtta gtaaattgac ttcatattga tatggaatcc 240
aaagaatata aaaattaaac atagaaaagc aaaatttggg tagtctttaa attatctgaa 300
acaatagtta gtgttttgtt gtttaataga aatgttagat aaaatgaaat acgaaattag 360
tattataaaa ttgatgaaaa tatttatgtt gatatatttg ggaactataa atgtgctaaa 420
ggtgtaaact attgtatgta catgtgcaag atgttaaata aatagtatca ttgtnaaaaa 480
a 481

<210> 847
<211> 548

<212> DNA
<213> Ctenocephalides felis

<400> 847

```
gaagatgtcg tggaagacaa agctacattg cgcgaagctg tactgaaaac ttgggcacaa 60
ctagttggcg gttgtctgat atttcgttat gtacaattat tttggtattt ggagctttct 120
ccaacgcata caggaagagc atttgaaaac tgcacggctg atttacaggt atctcctatg 180
ctaggaacgg caatagaagg aattgccacg tgcctatgcc ggctgacgtc gaaagtgatc 240
tctcatcacg aacccagatt tgccgcggcc ttagattcct ttgtaggaac ggcacttgtt 300
gtggctgctt ttaattactc gggtggatat ttcaatccag ttctagccac atctttaaaa 360
ttcggctgca tgggacattc cgcttgggaa cacgtgattg tgtactggtt cggtgcttgc 420
gccggacact tgcagcagtt gcgttgtgga ggattcccaa attagaaata gactaatcgg 480
tcgaaactaa aatcagcgta atcaaagcat tagtcatatc gatcaaacaa atatacaaaa 540
ataagaaa                                                          548
```

<210> 848
<211> 549
<212> DNA
<213> Ctenocephalides felis

<400> 848

```
gaaactacaa aatagtgctc aacacaatga actccttcgt tacaactgtt gccatcgtcc 60
tccttgcagg aacttgcgcc caaggtgcaa caactgtgct ctcctctacc aatggcaatt 120
accacggcaa ctatggatac ccatctgtct tatcccaagg tttcaacaat ttcaactcat 180
tcccaactac ttacgatcac tacaacactg gagtagtcga tactgttgtt tcttctcccg 240
tcgttaaatc ttcagtcaca acaactcctg ttgttgatac cgttgtttct actccagtcg 300
tcaaatccac tccagttgtt gcaactccag ttgttgaaac tgttgccaca ccagttgttg 360
caaccaccgg ttataccacc ccagttgtcg caaccaccgg ttacaccact actggctaca 420
ccactccagc tgtatcgact ggatacacca ccactggata taccactcca gcagttgctt 480
ccactggata tactggttgg gaagtatcgt ntggtttggg aggatacgat ttacacatct 540
attcaagga                                                         549
```

<210> 849
<211> 549
<212> DNA
<213> Ctenocephalides felis

<400> 849

```
ggtaaatcta gtaattattc gaagaattta gcaaaataat aatttttgac aaaatggcgg 60
cttcttaaaa aaatcgtttt ttggcgaaat tttaacctta aattgtttat aaaaataaaa 120
gtatttatcg gatcgcgaaa atctttgatt aaccactaaa aaatatattc tatgcaaatc 180
ctattattat aaattattat aagactgttc aaaatgttta taatatgaaa atataagaga 240
ctctccaccg attcggaaac ttctaataac tccaaccggt gttgtctgca tcccttcttc 300
tatctcaact tctttttcgg catatgaatt aagaaaacac tgttataaaa tttagaaagc 360
aatactaaac agtcgaatca catgcaacta aaatttcctg ttgtttatta agtttttcg 420
cggatacttc ttccatctca gccgtntttt cttttcaaat tgcgaacttg aaatattccc 480
agaatgaggg aaatttctgt gtgcatttta aatatatgac atcagaaatc ttttttggaa 540

actactgtt                                                         549
```

<210> 850
<211> 549

<212> DNA
<213> Ctenocephalides felis

<400> 850

```
cncgcttatc gcatctctaa caaattcgca aataataatc aatttagtaa ttaaaaaaaa 60
acaattgcac taaaatcaat tcaataaaca tttacgaaac gcgaattctt ttaatcctta 120
gtgtgtgtgt ctgtgtacaa taattcttgt taacaattac ttattattgt gaacaataaa 180
ataataagag ttaatataaa cattttgtta caaatagttt aaaacaattc taattaaata 240
attcgtctcg tcctatcggg taacactatc aaggaatgct ttgcacaaac ttcacgatgg 300
tgctggtgct ggtggtcgct atggtgacgg tgttgccaga tggtggtgac ggttcgccgt 360
ttcttgatcg attgttttcg ggtacggat tggcacgagc ctcttcgaaa caaatcacag 420
gcccacaaac atccggaaca gctacgcccg gagctcggca accggagatc gtcgaacggc 480
ccggaggcgg cgaacttaca aacagatttg caatgctata aatccaagcc cctacgcagc 540
cctggagcg                                                        549
```

<210> 851
<211> 549
<212> DNA
<213> Ctenocephalides felis

<400> 851

```
gaacaggaga tnttggaagc ancactnatc atatggacat gtgggtccag gagaagtctg 60
cngngtgatt ttaaaagtgg gctccanaga atttgtanga gctggcnaca cgccccangc 120
tgcccggcac gatgctgctt ctaaagctct tgaggaattg aagaatcttc ccatgccaga 180
tggaacatgt cctgctgtgc aaggcgatca tgtaactgnt ccaatggagg atgatccana 240
ttctgaatta aaatcaccta tatctcttgt tcatgagatc gctttgaaga gaagtntagc 300
tgnacacttc gcagtatcca gtgaanaggg tccaccacat atgaaagttt ttgtcacaat 360
ttgtaaagta ggtgatnngc aaactgangg agaaggcccg gacnaaaggt atcaaaganc 420
gtgctgcaga ataaatgctt gaggaactca caaactngtc cgattatact aaacagacct 480
gtggagcccc tntgtgcgta ctagaatnaa acgcnacctc tgtccaagan nagnaagaaa 540
tctgtaaag                                                        549
```

<210> 852
<211> 549
<212> DNA
<213> Ctenocephalides felis

<400> 852

```
aatacatttg atactcaacc cataacttcc ataaaattta tacatttttc tatatcgacc 60
taaaacagtt gagtagacgt ggaaaaaaca tcttttagta cttatgagta gatattgttg 120
gtaacaacta aaatctaacc aatgccatta tcaattgaat ttatacgaat gactttggat 180
```

tttgaaaaca gtgaataaaa atttatagtt ataaaatcaa cacctttaaa attttgatat 240
ttgtcaataa aataggcgat aaggtatttt tatctgtctt atgagtatct atatcaaaaa 300
atattccaaa aaccactttt ggaattttga tatttgtcaa taaaattatt attaagttta 360
ctatttactg gcatttttat taagttatta ttttatgcag acatgtaaat taaaacgatt 420
ttagcaaaat attgttgtgc attacatatc ataagattaa tattgttaaa aatgagtttt 480
actgagaata aataactttg aagcataatg tttattatct caaacattat ctgggtaatt 540
atatttctc 549

<210> 853
<211> 548
<212> DNA
<213> Ctenocephalides felis

<400> 853

ctggatacca tcattttagt tatgtattca taaaaaatca tatttattga cgaactgtat 60
tgttgatagt aacgttagct agcacgatat caagcaaaat tgaggtaatg aagcagattt 120
tcctttggtt tttcatattg taggattgag ttcacccaaa tctatatcaa attatttatt 180
agtatttttc gtagtctttg aaagacgaat ttggaataga tttatattta aatgaagtga 240
agagaagttt tttgattgcc ttgattgttg aaatcaaaag ctttctccaa ctagtgagtc 300
ctatattaaa tctgtttaat tgtccaatat ttatattttg aactgtcata caaccttcat 360
atttttctac ttcacttgaa atttatggaa aatatgattt taaatgaaaa ttacttttta 420
ggatgccgcg attaaaaatc gattgaaatc tactctctca atttttttatt atgtatatat 480
ttacttcttg acggagttct atattcaatt cctatgaatc aaaaagttta tatcaagctt 540
tttataga 548

<210> 854
<211> 549
<212> DNA
<213> Ctenocephalides felis

<400> 854

gcataatgat aatccacatg tagaaaaaca aattgttgag ttaatatatg atgccgtact 60
atttgaaaac ttgtcaaaaa attataaaaa taccgcattg tggcaagaaa tttttgtttt 120
gccattctac tccctatata aggagaattt gataaaccta atagacattt tacatataga 180
tacaagatat gcaattgaaa ataaattaca ccaagcagcc agggcaaagg cactgtattg 240
gactcatgca ctttattatt ttgagaagga gttaaagtac tcgtcaaatt ctccaacgtc 300
actaagtttt gatgtagaaa aattatataa aagggttctt tgtaaagaga attcagatac 360
aaccgatctt ggctggatac atatcacaca gatattcaaa aatttacccg cagaatgcat 420
atctgtaaaa tttgatggca aaatgattca tggataagtt ccgctggaac caactgtgcc 480
gccagatgta tgtcagagtc cgaattacgc gatgattctg tactcnccgt cgagataatg 540
atgtctctg 549

<210> 855
<211> 549
<212> DNA
<213> Ctenocephalides felis

<400> 855

gctatcagtc cacctaaaag caaaaccagc ccattaagtg tgtcttcaaa aggaagggcc 60
atagattttt caaatcagtt tgacgttggc gaaaagcaga aaacaaaaat agacgacatg 120
aatgacatga tgtcgacaaa aaacatcatc gccgataagg ataaaacgaa aatcgacagc 180
aaaggtcttg atgatgtaag catggatgat gacgatgacg acgatgtgat atcagcaggc 240
gacgtttcga aaagtaaatc agaacaatca ctggctcgaa aaccaatact gaccacaaat 300
gattcgccaa atatgcaagt gcattgtatc ttcaatggaa caacatataa gccaggacat 360
tcgttagata aacactgtga aggcatgtgc aaatgttccg aagaaggtct ttggagatgt 420
gagcccaggt gtgaagctct tatgtcacaa gactcctgat ggcaccctaa atgatgtcac 480
caccaaaaat gaaaggggtg ccgcgaaatg gccacccaac aannatgctg cctgtctggt 540
tggcancgt 549

<210> 856
<211> 549
<212> DNA
<213> Ctenocephalides felis

<400> 856

acaaccagaa atattgcttt cgattaaaat taaatatata ataaaaaaag aaattgattt 60
tcttgtattt taattactaa aaatgaattt tagcatggat tttaaaagaa atgaattaca 120
aatttgtaat ggatgtattt aagcagctaa ttcgttgaac taaaaatgaa aaaccaattt 180
ttgtcttctt catttatata tgtcttgact cttgatattt gtcatcattc tcaatctaaa 240
ttataatttg ttttgcacaa tgaatacaat tataaacata ttataacatt tagttgaaac 300
tacttactac tacaaacata actattattt taaaataaaa tatgaatatt ttaaaatagt 360
aattgttgct ctgtttttcg ttaattaaat ttttcacatt gaaaaaatat ttgccattta 420
tatttaaatt atgatttttt aaaagtaatg tgtaatttta ttgtattatt tgaatataaa 480
tttattgaat gatgcacata tcagngtcaa ancgcgtaag agatcaaaat tatctgcatt 540
taaatctga 549

<210> 857
<211> 549
<212> DNA
<213> Ctenocephalides felis

<400> 857

attagcagaa agctgcaaga tgttcataac taacatggaa ataaaatatc gtatagagag 60
tattgtcatg gatatgtata tatgtcttac acaatgtata gataatttaa catgcaaaga 120
ctcacagaca cctattcaac tgtgtcacgc gttcaaaaat ggaatgaaag aaattgcaat 180
agcttgtgct cattttctag aactctgttt agatttaaaa atcaactgca acaatttgca 240
tacattggac agacatgcaa tattacgata tgcttttaat ttatttccta catttgtcaa 300
attattatac aagggcttaa aatgtgcatg ggactcgttc aaaacattat caccttctca 360
taaacaagag ctcgctcagt atgttattcc tgaggtcagt taaaatatag ttgaagcaga 420
gtcaacatct gaaggccaag ccggtcgaaa gttatttctc gaaataaatg tgattatgga 480
tataatgagt tgctatattg aaatactcac agcaaaattc ttattaatgc tctatggaat 540

gataaangg 549

<210> 858

<211> 549
<212> DNA
<213> Ctenocephalides felis

<400> 858

```
gttgcgtgta aattgagtat gtcatagacc ttattataat atacattatt ttgtaattta 60
catattatta aaatattcat atgtattaca taagatcttt tgtggtgagt gcgtatgttg 120
tatgtatgaa tatcaagagc ttggttcaac cgatttgaat gattctaaaa tagaaaatgt 180
agaaatatgt agatagtggt gtgtgggtat ctatgtacgt atgtgcggat acaatgtttt 240
gtcgactttt tcaagagctt ggctcaaccg atttgaatca ttctagaaac attttacttg 300
atagaaagat gtaaatagtg gtgtgtgtat atttatgtat gtatgtgcgg atacaatctt 360
ttgtcgacga tttcaagagc ttggctcaac cgatttggat cattctaaaa acattttact 420
tgatagaaag atgtaaatag tgatgtggta tatctatgta tgtatgccgg attaatcttt 480
tgtcgcggtt caagagcttg gcgcaccgtt tggatgatct aaaacattta cttgtagaaa 540
gatgtcata                                                         549
```

<210> 859
<211> 549
<212> DNA
<213> Ctenocephalides felis

<400> 859

```
gagaccactt tcattgtatt cttcagacta ttcttcagac ctacctctca cctactctac 60
ggttcactca gtgcctctct atggagcccc cctggtacac agaagtgtca tcgttcaacg 120
acctgtgtcg gtgtacacac catccccgat gtccgtggtt attcgcacca ggccatcagt 180
cctggaccgt gaattcgacc gcatccaaag gcgcgtcagg cccaccacct acaaaccagt 240
agaagatttc ttaaacagca gcagcacatt ggactttgac gatgaaacaa gaaaaatccg 300
atctcaagca aactcacttt tgactagaat acacactccc gtacatagac cattgaaaac 360
gatattcttc agtactgctg gaggatacgg atcaataact ccttcttacc ctcatttgga 420
ggaggaatta tcccacatct tccaacctct gagcaactac cgcaaaaata tcggcctggt 480
catttggcat gcgtacgatc cgcagcgaca agcctcaaca agangaatct actaccagaa 540
gnggaaaat                                                         549
```

<210> 860
<211> 549
<212> DNA
<213> Ctenocephalides felis

<400> 860

```
aatctacgta ctttcactaa attggaaaac atgaaagtaa aatattaagc gagttttgta 60
aaaatctaga attaaaattt ttctaatgta atttagtaat aagtataaac atgaagagaa 120
actaaaaaat gccccttaat tttaggaatt ataaaatgta attaatgtga ttgaataatt 180
```

```
tttatataaa aaagctaact aaataactgt agtagatatt attagaactt taagatgtca 240
atcactcgaa attatgatgc aaatgtatgt aacaataaga atgatatccg acgttttgtt 300
ttctaaaaat ttgcatcatg atttcaacat ttgacgtaag tgataaaaaa ttcctaatta 360
ttcaattact gctaattaga aaagagaatt ctcaaacatt gctttttccg ataggcaaaa 420
catttttctt ttgcaaagct tagatgattt tttaatggac agatgttaat catttctagt 480
cgtaaacgat aaacgaaata tttcaaataa tttgtattcn ttaagaaaag tcaatataat 540
gnaattcaa                                                         549
```

<210> 861
<211> 549
<212> DNA
<213> Ctenocephalides felis

<400> 861

```
atagaagcaa aagacatcaa gatgaaattg gcgattgtat ttttggtttt ggcagtagtt 60
gccttggctt tagctggtga agcaaagcaa aatcagcgta aacatgggca gaggtcactt 120
caaaatgctg gacacaaagg agctgcaagg aagcaaaata atcatagtgc atcagccaaa 180
tttttgtcca ggaatcgacg agccaatcga aatgaagagg caacagcaaa tgaggctgtt 240
catgatatag atcgtgaacc agaacatgaa ggaggtgctg aacctgtaga agatcccgat 300
catgaagtag tcattgatgg tgatgtagaa gatcatgaat ctggtgtcga cggatattct 360
acaaataatg attatagtac ttacaacgca gaaagtacga atgatgctat tgaagcagca 420
aaaaaagctc tgaacaaagc cattgcctct tcaaacaagc tcgtaaagct ttggaccagt 480
tggcgaagaa ccttgactta gacgtgcagt tttgaccatg taagttctgg aaaatcaaaa 540
gatcaacaa                                                         549
```

<210> 862
<211> 549
<212> DNA
<213> Ctenocephalides felis

<400> 862

```
atactattta tcattagatc atatggttat aatagaaaat ttaatttttt atactaattc 60
ttgtttttca caataatttg aagaattctc ttcctttaag tatgtattta actttatata 120
caactttgat gatataagaa aatcatgctc tttttattta aatcttgtag tataacgcgt 180
tttttttatt accttaatta tatttaagag ttttacattt attagatgtt ttttggaact 240
aggacttgcc aaaataaaaa tcttattctg cattttccaa gatttcagaa taaatttttc 300
tgaaagctga taaatgttca ataatcaata taccaagcag tccttttccc aaataattta 360
gtaaatatat atttgttgta aataataatt gtgttaaact ataatctttt tttctattga 420
ggttgtgcaa tgtcaaggac atccaattcc agcaaattcg taaagattct ggcgcatttg 480
atttgtattt tatntattgt attatattct attgaattaa taataaatgt ttatggtatc 540
gatngggcg                                                         549
```

<210> 863
<211> 87
<212> DNA
<213> Ctenocephalides felis

<400> 863

cacctcggtg gtgaaagggc agagcaccac gctgaccgtg gccttccagc cggagggcgt 60
aaccgacaag agagagagag aactagt 87

<210> 864
<211> 549
<212> DNA
<213> Ctenocephalides felis

<400> 864

cattactcca gtgccaattt taaaacaaat aaatagacac aacgaagatg gaagttattc 60
atacggatac gaagccgccg atggttcctt caaaatagaa tccaaatatc ccacaggaga 120
agtatatgga aaatacggtt atattgatga ccaaggacaa ctgcgagagg tggagtatgg 180
agcgaccagt ggacgaggtt tcgaaccggc aggaaccggt ataaaagtcc caccaccaac 240
tgtcaacact aaaaacgaat actacccacc cttgaaacca ggcgaagaag atgacggcca 300
atacagagaa gaccccagca tttattacaa agattctcgt tacaacagcg atgtgagcaa 360
cagtttgcaa caacaacaac cccaacctca atacattcaa cctcaaccta aaccaagtgc 420
tgcaccatcg tacgctgtcc tcaaagaaag aacttctact ttatcaacaa tgcctccctg 480
ctccagtttc ggaagaccaa aagaagattc taccagctaa gtctcagcta acagcaatat 540
taccacctc 549

<210> 865
<211> 549
<212> DNA
<213> Ctenocephalides felis

<400> 865

agtgtacgat gtggtgctca acatgatttg aatacggata actgatcatc ttatggatct 60
tatggatatc tcatacatga acatatatca atgataatgg caactaattc atcggaacct 120
acggttcgac ggaatcgtta ttcacggtcg tctacgacca gtgtgacgca actgttatct 180
gaaagttgtt cgagccttct tcatcggttg acacggcgtg gaccatcgga aaagcccacc 240
taccgcgtgc ggccaagcaa caggcccgaa gaaaaaccga tagatactgt gaaagtttct 300
agtaaaatat caaccgccgt tcctgtgaca attacctctg ccttgggaag taccagaagc 360
cggcttgaaa gcaaatattc agcagtgtta gaccgtgtaa aacgtcggga agttatagat 420
catgataaga cattagaacc cagtccattc gtacccacgg cgcaaaaaaa gtgcatcgag 480
tgnattttag gtgaaaaggc ttatcctatg gagtgcatta gaaccaaagt nttattggat 540
cgcgatgat 549

<210> 866
<211> 549
<212> DNA
<213> Ctenocephalides felis

<400> 866

```
agttgctatc agttaatagc tgtaactcaa acatcacaca acgtaaataa aacaaatcct 60
tatcttgtcg tgatatcaca attcattttt catttttcac atatcttaac agaatataat 120
agtacgttcg gatgttccag tttatttgca cgtggaccgg tgaccgttat agataagaga 180
gataattgac agtgaacagt ttttatgaag tgtttaggat attgagtgca cttttcgttt 240
tgttttggat tttgatggac gttggattat ctgtgattta gtcagagaat gatatatgtg 300
cttgaagtca cagaagatta ttagcacaca tgtatttgga tactctgcag atagaatagt 360
cttcagtatt ttgataacta aagagccata tatttcaaat gtaaaatatt atctatacat 420
tcttttcaag ttgtgtctta gttttcttgc agtagtgctc tttttatgaa gatcttttaa 480
tttagccctg tcgtctatca gtttgtggat ttgatgccga acaagggttg ctgaagagag 540
tgcagacnt                                                         549
```

<210> 867
<211> 549
<212> DNA
<213> Ctenocephalides felis

<400> 867

```
gaggagtcgg tcctttagcc tggggtacag aaacacatcg aaaaccgcag acgcttccac 60
caaatctgag tccaaaattc ttccatagat cgccaagaga agcattgaga agagtcacta 120
gtctattgat caggaaaggg gcagctggtg gaggcacgcc ccgtgactcg cgtaaagaac 180
gcgagggcag cgtgttcccg atgccgcttg gacaaacggt gcatagggaa gctttagagg 240
aagtggtgcc taaacaaaga agaggatttt tgaaaaactt ttttaagaaa tctaaacatt 300
actcactgga ccagtaaata atcgaaaagt tgacaattta ccgagttcta tgttttttag 360
gcataagaaa atatgtacac tgccctcaac tttagtccta accataatat tagcattgaa 420
tactactgta gttacccgtt tagttgtagg attatttatt attctaataa tgaattacaa 480
ttaaccgtct gatatggacg aangggaagt aagacagttg caataaatag tgcctgagaa 540
atacatttt                                                         549
```

<210> 868
<211> 549
<212> DNA
<213> Ctenocephalides felis

<400> 868

```
gtacgaaagg accacgccct ttgttataac atgacattaa gactcaggta gcttaaataa 60
taattttta attctataag tatcatatta taggccagaa acagcatgtt atatatttat 120
atccgaaata ataagtagat ttatatacga acagacaagt gtatccaagg aaataaattt 180
aatttattta tattattaat ggtaatttat caaaattcag atatatatat atatatatat 240
atatatatat atatatttta aattaaaaca gcatatgtaa gtatatatat atgccatatg 300
cccattaaat tcagagctat ttaattgatt gatgagtact taatcatttt tttgtcgaaa 360
ttgttttaga ttttttatat ttattagtat aattggtata ttatcaaaat attatactta 420
taccaatctg ttttatattt ttttcatgaa atattatatt ttattatgaa attattttaa 480
gcgttaaaat tatttaaata tttcatacag aggcagcgtt attgaaaact gctttttttgt 540
atcttgtac                                                         549
```

<210> 869
<211> 549
<212> DNA
<213> Ctenocephalides felis

<400> 869

```
cgcggtggcg gccgctctag aactagtgga tcccccgggc tgcagggtga gattaaatcg 60
tggacatcag atcaaccacc ggacactttt acagaactag ccgtaattga ttcaacgacc 120
aataaaggag caagaaggca aataaaccaa aaagatggca acctatccaa agcttgtcaa 180
taagcaattc aattcaccaa ttaaactgta ttctccgcag aacgttcagg aaacgctcaa 240
caagcaaact cagttgttag ccaatggagc agttggaatc gacttcatgc acaacaagaa 300
cgtggataag ccaggcaact tagcaaattc agctgtcttg agaatgcttg aagaagaaga 360
agaaagacaa cgaaaaggac aaccaccaag tttgaaaaga gttgcctggc caccaccagc 420
tgaacctgac catcaatcag gaactccaat tttggaacaa gagcctgtct tcgctcagac 480
gcaacctccg aattattcga cttcaccgca cagcaacagt atcagccgaa cccacgcaag 540
ccttccgcc                                                         549
```

<210> 870
<211> 549
<212> DNA
<213> Ctenocephalides felis

<400> 870

```
gctcggttaa cttatatctg aaggctcctt gacagttgtt agtaattaaa tctaatatat 60
tatagaataa ggtgggttgt gcggttcagc aatggtatca cgctttaccg acatccttat 120
acaataaaaa ggggccatct cacaattgca attatataac tagttactaa gaatttgaat 180
attatatatc atagttttat tatgaaattg gataacttta tacaaagatt ataagattac 240
tttataaata tacatatatt taggataaag ttttttatga aatgaagggc tagttgattt 300
ctttggtttg agatatgagc catttgtgat ttctggtagt aggctaggcc agtttgtctg 360
tatgccaggc agataattat aatgaaatgt aacttcaaag gacttccata ctctgaagaa 420
tcacgagttt aatttttttaa tattttttgt acgtgtttgc aataacttct ttaaaattga 480
cattcaatgt aaaaacagag aatagtctta tataagtatg atttgagtgg ctccttattt 540
atatgaatt                                                         549
```

<210> 871
<211> 549
<212> DNA
<213> Ctenocephalides felis

<400> 871

```
gctcatttga ctgaacgaga aaagctatat atttatacca tccattatac ggtggatgcg 60
tagtggagct ttacaaagac ttaaacaatt attagtgaac aaacaatgtg aaattaaaga 120
ctttgcgata tacgaatatt taatgccttt tcttacagaa cattttaaac aattgaattt 180
tattgaatga atcactctga ttatttgata ttgtgagatt tggaaaacat aaattcacta 240
aatgtatata tgaatgggac cgtcactttg atagattaac gagaagttat taattatacc 300

gaaaagaaat aattgtgtgt tttcttagta acatttgttt ttcgttctcg tgcttcactc 360
gaaataaatc atctagaaat tttcatgaag cgaactgtgg tgaattgttt tcgtgatatt 420
tgtgatgaac ttctcgtaag gacatatcac gttgtatttt ctagtgcatt ttgaatctat 480
ttaaagttat tatatttata gcaagtaggc acatgacgat agcaatgttt attattatac 540
tcttagaaa                                                         549
```

<210> 872
<211> 549
<212> DNA
<213> Ctenocephalides felis

<400> 872

```
gtgttcgata gatcacatag gcaattttgt tattagtgga atagtttgat agttttccat 60
atggtttagt gtgtgataaa tcaatattta aatttcaagt gtttgtgtga tatatgtgat 120
tagaatacat cgatttgata aagtgatttg ggatcgtttg ggtgcatctc atatgcatta 180
attaaatata aggattgaaa gattttgtat agctttagta gtaaaacagc ttagtttgga 240
ggcagattat gaattctcat gagaagtttt aatgaggtga cgagtaatca tatagcaagt 300
attgacatta ttacatatct ctcaaaagga ctaataaggt aatcatctaa ttgataccta 360
agtgtgaaat aaatttagta tgttgtggtt tagaatttta aaggatccta cttgaaaatt 420
atgatacaaa ggtgaatgtt gccagtttgt tatatgccgn nttaaatcac gtaatcccac 480
tccccactct ttaagaacaa tacctcaata ttcattgtta ccgacgataa atagcagcag 540
ctantcacc                                                         549
```

<210> 873
<211> 549
<212> DNA
<213> Ctenocephalides felis

<400> 873

```
gagaaatgtc aaagtggtta atggaattat taaagttaag ttcgaagata aatttagtaa 60
aaaggtcagt gttagcatta tatcctatca aacaataaac gaattagtaa atatcgaagc 120
atacttatta ttatacactt agacttctca atttttaaag aactcatcat ctgtttgttt 180
attataacct acaaacctca aacatgtctg aagaaacaga ttttaacaaa acaattaaaa 240
cggaaccgat cgatgattat cctcaagtga aacaggaatt cgatgattgt gaaaatacat 300
cagatttgta tatggatgat gatatatgtc tgcagcaatt tgaaaaatct gaggttacaa 360
ttgacgagga aataaaacaa gagacgattg atgaccaaga tcatgtgact agtacaaaca 420
aactgttcag atgaaaattc ttatattgtg atgaagaggc agtaattcaa gnaattagtg 480
gtagtacgaa caaattattg gaaaagttca ggcaaaangg aaccactaat aattggaacc 540
gnttctgtn                                                         549
```

<210> 874
<211> 549
<212> DNA
<213> Ctenocephalides felis

<400> 874

agtacgggat gtttcaataa tttgtcttaa ggcacaatgc catgagtcac tagtttaaaa 60
ttttttcgac gctataagca caaatattgt gcacttccac acaaattcgt cattttaaga 120
gggctcgaga actgaagtaa atatttttct taaactttca gcttgtttac ggaaatttat 180
attagccaaa ttgccaaatt taataactta gtaaccaatt tgataattta cgttcggaga 240
gggaggcaag gcaaaataat aaattttaat aatcggttat ttcaacccaa attttgcctt 300
gcaatcgtat tatgttttgt taattatatt tcttgaaata taaaaatgtt ggtaaaaaaa 360
tttgattaca ttgtatagaa tttttttctc actatccaaa ttttgccgcc ccaaaaattt 420
gccgcctggc agtagccccg attgccctct gaaatccagc ctggatgngg atatgnatat 480
atatatatat atatatatat atatatatat gaaatgcggn gcccatatat natngggttc 540
attacaaaa 549

<210> 875
<211> 549
<212> DNA
<213> Ctenocephalides felis

<400> 875

gcgttgcgtc atacgttaga aatgacaaag ttttacaaat tcataagatt taaatgaaac 60
cgtttctcat ttctgtttct tttacaaacc gtgtgctcga tttatgtagt ttttgcccaa 120
gtactactgt ctaagacgca cgccggatat atttcatctc ataattttaa tttgcatcat 180
ttggtgccca ctgttatata aaactaaata atattatttc tttgactaag caagagttca 240
caattgaatg gctggccgtc gctgtgttat tttatttcga gacttattgt gcaggaaatc 300
aagatgaaat cacatggagg ccacaatata tcatatcaag ttgttgagta tcgaatggcc 360
aaatatcgag tgaaaattgc acaccgctcc ttcaccggga attgctctga aaataattta 420
tgaagtcttt cactactgct gtctgacact gctcttcacc aggaatcttt ccagaacatt 480
ttaccagctt ttnactactg nttnttttca ctgncccttc ccagaatcgt ctgaaatatt 540
taaaaaagt 549

<210> 876
<211> 549
<212> DNA
<213> Ctenocephalides felis

<400> 876

gatgaagtga atacacaact tgataatagc aagaagttat ggggtataga gatattgcca 60
aaaacaaaaa ataatgtcag aaagaatttt gaaaatgcct taggaattgt aacactgaca 120
tttttatctt gtatagtatt aagaaaggtg tttaagattt tgtaagctaa tatagccata 180
tatttccata ccttgaaggg tatttattat aatataattt atatcgttag tcaaacttat 240
gtatacagat aaatttcaca ttatctatta cattttgttt tgttttgtat tatagtcaca 300
tgtgaaaaat atataatatt aaatttgatt tcattttgcc tgtttattgg ttttgcaaaa 360
aaatcctgta cctaaaatca taataggttt cgaaatcaca tacttagctt agagtaatga 420
acttattaaa gaaagaaaaa agctgngtta cttatgggct aatatacgng ngcaaatgta 480
aanccccaaa agntttgctg gaacaattgg ggtgatgnat catgtataaa natatagttt 540
tgaaaancn 549

<210> 877
<211> 549
<212> DNA

<213> Ctenocephalides felis

<400> 877

```
gtccgtctgt caaagtacat cagttaacct caaagatata cttcaaaaag acattaaatt 60
gttatgaaca attatatgtt taagttaaac atctgatatg tataagcaat tttttgaact 120
cgattaaata caataagctg gtacacaatt cagtacttca aaaataagat gaataaatga 180
actggttaaa aatgagaagt aaattgtgac tctttgaata tgtttacgac cgagtgctca 240
ccccgttcag tcgatgtgct catcttaaaa tctgctaatt gctataaatt aagttaacta 300
tagtatgaaa gatctaaaga gttacaaata agagataatg tctgtcgtgg gagaaagacc 360
atcatcaatg atggtgtcat gacatcatct ggtattggaa atccaagtgt ttaccatgcc 420
ctatagtgat atttttagaa tttttttgctt gggggctcct acgatgccaa taatatctgt 480
ctaaatgaaa cattcccagt catcattttn atgaatggtt aattaatggt ataaangatt 540
tatcttcta                                                         549
```

<210> 878
<211> 549
<212> DNA
<213> Ctenocephalides felis

<400> 878

```
aaaaaataga gatatttata ataatagtaa caatataata aattctaaaa atgttcagtg 60
taatgttatc agtatgtaca agcatagctc gagcaaatgc aatatcaacg aatttaaata 120
aattaagtaa taagagatct ttccatgaat catgtgttac ttttgctgct agaaaaaggt 180
accagagaaa aggcacgaaa gaagaaagga gttaaagtcg aagaaaaaaa agttggtttc 240
atcccacata atattcgtgc caaaaaataag ttagccgtga gcacaataga caagcatttc 300
aatgatagct acaaacatgt ttcaagcgac aatgttttca tctctaagtt ttacaaaatg 360
aaagtttacg attttgcaga agcaatagct gctcataggc aaacacatca tcctacgtgt 420
acaatgttcc agatgcaaat ctaaatgtta aaattgacta aacatggctg gtggaaaagt 480
acccgattat ggataatttc acgantgcat gtacccctgc ttcgtccaat gtgaaagact 540
ntcntgatc                                                         549
```

<210> 879
<211> 549
<212> DNA
<213> Ctenocephalides felis

<400> 879

```
atctttaagt catatttgtt gccagtttaa ctatagttat atataaatat ataaccaaaa 60
taattgtgat acttcatgcc tttcattaca actgatataa aaattaattt gcgtctaatt 120
tgaagtcaac attaagttaa atagttatta gtctaaaact catgaaaaag tatagggttc 180
ccattatctg catcaattct ataaatggta tggaaaatga agctttaaaa tgattgagtc 240
aaatttgcat tgtaaaaaag aaatgtcgtc ttacatccat atcttcatat ttgacaaaac 300
```

gatattttt aaagtaaact ttaagcttat aaaaatctta aatacatgca tatttatata 360
tatatatgca caggcaaatc ctattttgct atgtcttctc tttggtgctt ttgggaaatc 420
tgntgaaatt ttatttaata acaatgagag tatgcaatta atttccttgc catttatgtt 480
ttacatggat gagtttgaga ctaattaact tcatttttta atagtgatat agtcgcaata 540
tatttggat 549

<210> 880
<211> 549
<212> DNA
<213> Ctenocephalides felis

<400> 880

acattatcaa tcataataga tagtagtaat gagtatagta atatgatgtt ttctcagcat 60
tgaatgagtt ttgtcacaat ccaaaatacg caatatttct aaatcgatta aaaacacgtc 120
acactgttga gaaaataata tattgactta ctcatctttt ggaacaagtt ttaactctaa 180
ttctgtgcag ttttctacct cagaactgaa ctaattttcg agtattaata gatatacgtt 240
aaattatcac aatataaacg tattagaaat atgttcatgt ttttaatttt ctttaagaat 300
tagttcacaa ttaattagga agatcgcctg ccataaccta atttatttag ctgacaaatt 360
taaagtttct tgcactccct tgaaatttgt atatgtttac aattttgcaa aacggagatn 420
tcgtacacaa tttgtttaga aataagattn tagtatggcg cgtcctaccg agaattcctn 480
gggcagtgag ggaaaaagng tttnaanggt aaactatttt agggtaaaag acgaggtctc 540
nttaancca 549

<210> 881
<211> 549
<212> DNA
<213> Ctenocephalides felis

<400> 881

aatcggcacg aggacgattt aaaaagagag caatttctga caaagaacgg gcagagtcta 60
atcgaaaacg atcaaaaata atagatccta tccctacact gtctactaat tctcaatgcg 120
aggaaaatgc tgatatattt ccagatactt catataaggt aatttcaatg cctatgctat 180
cccaattgac gcctattgag gctgcagaaa tggacgtgga cgatccactc ccattgttgc 240
cattagattc cctgttagcc tgcacttctg atacacatcc tggtccggcc gtagaagtgc 300
aagatgctca tcagggtatg cagggattag gaatggcaga agatgaatta gctggattat 360
tactatcaag tggatgggat gaatcacaac tagaattact agattcatta ctggattcac 420
tttaaagaag aattagaaat tactcatact taaaggtaag caatagtctt gntggtaagc 480
ctacaagaaa aagactttat ttggtnaatt ttatctaaga tgacgagccc aaaaatttgg 540
cattttttg 549

<210> 882
<211> 549
<212> DNA
<213> Ctenocephalides felis

<400> 882

```
gttnaaatgg cgcctattct aaaagaagta aagtctcgta aaccttacat actcttgtca 60
gctcgtagga agcgtgaaat agcacatgaa gctaaaaagg aatttttaca ataccttaat 120
gccaagcatc cactgtttac tgagaaggat ttggatgtta ccatttgtgg ctccagtaca 180
agtgttgaag acgtcgagaa gaatttatat cacacaacac gtgtaaacgt caaacatcaa 240
agacctttta atagcattaa tgaagaacaa agctttgtgc tttgtgataa tgaagaaaat 300
gtagaagaac cttcttacac acgtgtaaac gttaaacgtc aaagaccttt taatagcatt 360
aatgaagaac aaagctttgt gctttgtgat aatgaagaaa atgtgggaga accttcttcc 420
ctctcacctt tgcaatgcga actacagaat ctgttaatac ccaaactatg acgccagagt 480
gtcaactaaa ttgtggaaat tctgaggcgc atggcataag aatgaactcc agtagatcta 540
cacatatgc                                                         549
```

<210> 883
<211> 549
<212> DNA
<213> Ctenocephalides felis

<400> 883

```
gttggccgtt gtgaggttat cgtgtgcctt gaatcgctcg aatctaagct ccatcgtgct 60
aattatttcg ataactccca ccccgactgg gacatctcga gagtcgggaa gcccctggca 120
aaggctacgg cgattttgtt taaatcagtg tctacagttg aacgggtcca cttggacggg 180
aaacagtgtt catacgagat tattatatat ttaaaattga ttgaaacagt gacaaagtga 240
tatagtaaaa tattttacta actgttctta aggattcgaa tcataagatt tctttacatg 300
atggctgaaa tgaccgccag cacgcgcttc aaacaataac accaatcatt ctccaccaca 360
agtgcaacaa ttgtgaaata aaacttgatt ttattccaat catataaact ttaaatacag 420
tgcaactaat caaataatt tgtcggcaat tgtaaaatac ctaagtgtcg ataagtctat 480
atgtgatcag gctaaagcct tgaaaaagaa tcttagtagg aatattagta ttcgtattaa 540
ttaattaat                                                         549
```

<210> 884
<211> 549
<212> DNA
<213> Ctenocephalides felis

<400> 884

```
gctactccta accaaaaaac acatttttga taagttcgtt agtggcttct gtcggatttt 60
aatttttaat tcaatcttac atttgcaggg tcaaataatt taactatttt tatttaatac 120
agattctaac gataatcttt tattttgtaa tatgtctgaa gaaggaacaa cgagaagaac 180
tacgaggtcg cttgccaggc ggctgagtac tgattcaata tcgcctccag ctgcagggac 240
tcctggcaaa aaagcgaggg cttcaagagt tacggggttg ccgtctattg cagaaactaa 300
accgaaagca gttagcactc gtaaatcccg aagattaagt actgacttaa atttagaaga 360
acctggaagc agaccatcaa cacctatatc aactgaaagg cgtcgttctc gccgactaag 420
tattgcttag atgaacaacg cccacaatct gtatcaactc tcccattggt ggagttatac 480
aagaagagga agacatcaat attttagcaa tgaaagatga tataaataat aaatccggta 540
tgngtgtgn                                                         549
```

<210> 885
<211> 496
<212> DNA

<213> Ctenocephalides felis

<400> 885

```
agttacagct cctgttaatg ctttagctga aacaagtcaa acttcatcaa tatttggtgg 60
tgctaaacca cgggaagaac ccactgagaa ataagttatc aagttttaac attaattatt 120
aaccaccata tagaatacca tcttgaatca tgtaataatt tttcgattaa aaattctgca 180
aaactcataa caggcgtcga taatcttact cttgcaataa attctttaac tgatatataa 240
aatgtatcaa gttttgtaaa agaaaaatca ataattattg atttacagaa taaaatattt 300
attttgttta aaaattgact aatactttgt aataatatgt aattcttata tatatagatt 360
aaagagttgt tgttagtgtc ttttgttttc aaatagtttt acactaatat atttaataca 420
aaacgcttta caaattttac aataattgat gaaaactatt tgagatttta ttctcgaagt 480
acaacttatg tattaa                                                 496
```

<210> 886
<211> 549
<212> DNA
<213> Ctenocephalides felis

<400> 886

```
agcntaaata acaagactga cagtttagtt ttaacaattc atcatgggcg atggaagaga 60
tattgaacga cagccattga ttcaaaatga tggaactgga agccttagga atcaaggatc 120
ctatacggaa ggttctcaaa ccacgacggt ttcccctata ggtcctgatg agttgccacc 180
gtcttaccag ggaagttcgg ccagtggcgt gcccatggtc acttgcaggg tgtgtcaggc 240
catggtcgat atttcaggca aacgcgaaca gcatgtcgtc aaatgcaatc agtgcaatga 300
agccacacct atccgcaatg caccaccagg caagaagtac gttcgatgtc catgcaactg 360
tttattgatt tgcaaaagtt catctcaaag gatagcttgt ccgagaccaa attgcaaacg 420
cataataaat ttagcaccta gtcctgtgac accacctgtc ctcacggtgg aaattttcgt 480
gccaggaatg tgcagggttt gtgtgctatt gtggggancg ttttttcaa caccctaaca 540
atgcctcgc                                                         549
```

<210> 887
<211> 549
<212> DNA
<213> Ctenocephalides felis

<400> 887

```
gctaataatg tgacagccat aaatattgta gattaatgta gaataatttg tattgagatt 60
tagattttgt agcatactag aatgttatgt gtgcttgaat aatgcaagtg agggaaccaa 120
taatttggtc tgtttttata atacatttta gataataatt attggtgaac tcaatcttgc 180
atatacgccg ctaatgaatt aaaccagcag gcatataatt tttgtactta aatatttata 240
taactaaaac tgatacgggt tacgaaaaac acataactat attatttatg tttctagacc 300
cgcatgaatt aaaacgaaaa cggcaaaaaa ttgacgggga tccaaaacat cttttatggc 360
```

aactgcaggg tcaaaatcct tctacgagta agtcattttc aacttttatt ttttttatca 420
gcaaatcaaa cagggttcaa tgtcaggtga cggtgaatca cggggtggat gaatataaaa 480
actcatatca tacgcttatt acatataaca ctaccatttt catattatca gtaattttct 540
aggagnata 549

<210> 888
<211> 549
<212> DNA
<213> Ctenocephalides felis

<400> 888

atggcatcct aaacaatata ataatataat aataatgaat ggcatttctc gctaacagat 60
attttaatat agcgatttga atagttttaa tttatttgaa tattgttcag agtgatactt 120
tttatatttg ctgtaataaa aatgattatt atgataactt atattatgaa agggaaaata 180
tatttaaact tttaattgat tacccaagag gatattgatt tgtatatatc tacttgaata 240
tgaatttgaa cagttaacat tatcttcaaa tttttaatat aatttaaaat tattggttac 300
tagcaaaaac gtcaagatgt ctaattacgt gttgaaagtc aaatcaaaag aaggacagca 360
tattttaaga gatctcaaat cttccatgac tctgggcgat cttttactga aactttcatg 420
ttgacatcga tatctaaacc aatttgcaaa ttttatcggg ttttccgcct aaagcattag 480
atttatctga tnagagtaag actttaaagg ngagtgattt nattcaggag atctgtattg 540
tgaaaaaat 549

<210> 889
<211> 549
<212> DNA
<213> Ctenocephalides felis

<400> 889

gagaagggaa actgatcacc atgaaattcg cagtagcaat tttgggcctg gccctgtgtg 60
gtttggcatc agctcagttc cagaatggac gcatcttaga accaccagta cctgcactct 120
gcgcccaaag gacgatacac gaacgtagcc cagacggcaa aggatacttc ttctcgtggc 180
gtgacccaca attggctggt gttgaggaag attggttggg cgtccgcaac ttctgtcgcc 240
aacgttgcat ggacagtgtc agtttagaaa ccagtgccga aaatgaatgg atcaagcaaa 300
gaattgtcaa tggaaatgtc aaatacatct ggaccagcgg tcgtctatgt gacttcaagg 360
gttgtgaccg accagattta caacctgttt ccgtaaatgg tggttctgga ccgctgaatt 420
gcaaaaactt gcccaaccac agacagacaa caaaacgact ggtctgaagg agtggtattg 480
tcttcctcac cagatacaag aattgaacaa ggtggacaac cgaaactgtt tgcagtttga 540
cactttaca 549

<210> 890
<211> 549
<212> DNA
<213> Ctenocephalides felis

<400> 890

cgtgggataa aaaatgaaat tatcaattat tttacttttt gtggcttgta tagccttggc 60
ttcggctgca aatttaaaac aaggaaggaa agctaaaaga tcgggtaatt tggtgatgaa 120
aaaatcacca agaggttctg aaaataatat ttttaacact ggttttgtga aggattccat 180
cgatttcgga gcaaagactt taattcgtgc cataaattta gcagaaccct tagttcgtga 240
tagttttgac ctaggaaaag atgtaaccct acgtctgatc cgaggagcac caattgacca 300
acatggaaga gcttctgatt ctgaactttt tgaaagattg gatgaacttg tccagaatat 360
aggaaaagct tttgaagccc tatttgataa ttctgaaaca aatgcgagga aaggacctcc 420
aacggaggct agggttccat actaataata atagtaaagg actaagggtg agaaaaaatt 480
ttttgagggc aaaatggagg agaaggatgt gtgaatttga tgtctcaata taaaaaaatg 540
catnaatag 549

<210> 891
<211> 548
<212> DNA
<213> Ctenocephalides felis
<400> 891

cccttccccc cttccccaca aacatcacgt gtatttttag ttatgaaaaa aaattgtaat 60
tattttgatt tttggatttt tttaaaaaaa aattctctct ccctaaaaac atcacctgat 120
taatggacgc atcctatttg tattccttat ataaaaataa aaaatgtgaa atttagatat 180
actgaatgat tactcgtacc aagagtcgaa acatataaat aaataatata aaaatcaatt 240
taccattttc aaatttgatg aattggttgc aaactatacc aaattcctca attccaatca 300
tattcatata caaaaatacc tatcaaattt tccgattcat ttaaaaccga ttacaatcaa 360
ttccattagg caccagtatt tataaataaa attttgcgca tatgtgtgaa catatattca 420
ttattttatt agtctgagaa atagatgttg actattcgag agagcagcga aatgtcgata 480
tttgccacct catcagatgt tggataacca accgntaata accgattttt agggggatgt 540
taangcct 548

<210> 892
<211> 549
<212> DNA
<213> Ctenocephalides felis

<400> 892

tatttttggg tcttttatgt tttacaaaaa ggatttatcg ttttaagttt aaataatgac 60
aatcttacaa attaatctaa acaaaaatgt aacaatattg ctttaattaa ataattcgtt 120
tattgttata ttgaatccac aaatatgaat tgttgattat tagcatcgtt tatttttgtt 180
gtaaataact tatgcaaagc agagttgatc ttataaaaca tctaatactt attttattat 240
acatagtgtg atacttgttt ttattttaaa tacattacaa actaaatgta gttcatctat 300
gatttacatg aaaaaaaatt agttattatt tgttagtgtt taagacattt tacgcaatat 360
ggcagtaaat aaatgcgcac actaaaaatt attattaata tttttatagg aacgaaagtc 420
tataattcta tacactacat cgctttgtgc aattgaaata atatttttca ttatattgna 480
tgaatttagt atatcaaaac atttaaaatg gttatatgta tacaggtnca ttattgtaat 540
aatgagaag 549

<210> 893
<211> 549
<212> DNA

<213> Ctenocephalides felis

<400> 893

```
gaacagttgg ataaaataaa aaattgtgaa ctaaaattta ttttcaaatt tcattaattt 60
ttaacaaacc cttttccgta atattgacgg aacctgccta agtgaagcaa aggacgatat 120
cggttgtcgt tggtagtgta gtgaaactat aaaattgtga ataaaactta aaacatcacc 180
aaaaataatc cacacacttg cagtaaacaa attattattt gtctctatag acagaaccaa 240
atagaagaaa aactcagctg ccaaatcaag attgacataa ctgtcaatta tttttatgtt 300
gatcatttat taatatatca attttgactt tcatcgattc tatctcggcc cccttccca 360
ccatgacgct ttcggccaac agcaatgcta ccaatcgcct cgggtcgcag cagggacgta 420
gcctcaatta gccgccgtgc gaatacaggc cggcncagag gatatgcgtg cgcaagcagt 480
caaacgttgc aaaatcaaag tgacggtgca agagacagct gnagaatcta ttgacagaga 540
tacgcgcat                                                         549
```

<210> 894
<211> 549
<212> DNA
<213> Ctenocephalides felis

<400> 894

```
attagacttg ccacatctaa ataatataaa aattagtaaa ataatttgta aatatttatt 60
taaaaaataa aattaggatt ttttattaat atgaaataat aaataatcaa ataaatttag 120
tgtaaataat aaaagtgtgc atgtgtcatt cttgattaat attcaacgca attaaagatc 180
aattttgaat gtatcctctc aaaaattgtt gtatgaggag atgactagca aatttttttat 240
aaatgtcgtg gataaatgag tttcgaaaat ttattttgga ttttataaga actgttttcg 300
aattataata gagggaagag ctagaaatcc acatatcaaa attttaaatg gacctgttat 360
agaagaaaat gaaactcaac aaaggaacca tgaaaaaacc ngagttctct acaattgccc 420
ctgtgtcgaa gaagacgatg atcgagtata tctaatttga atcagatcta cacaaatgag 480
tggccatcct gcccatttta tagactcaga caagcggagg gcccttggan ggcagatgaa 540
attcccant                                                         549
```

<210> 895
<211> 92
<212> DNA
<213> Ctenocephalides felis

<400> 895

```
taaaatcatc tccttttgat aaatcaaagc tcttgattaa catttcttgt attaattttc 60
gcttcttgta ttcagctttc atatttgccg gt                               92
```

<210> 896
<211> 386
<212> DNA
<213> Ctenocephalides felis

<400> 896

atatatctta catcgaatct aataacaata aagagttatt aattttcatg tcgatgtttt 60
agaaaatgat tcagtagcaa cttctgaatt ttctttgtaa ttgataatac tttctcaaag 120
tataattgca tttcacaaca cacgacggaa atatggttat ggtccatatg aagcaatgct 180
cgttcttcta tccacttgct tctgataaat gttcggtata aagaatgcaa tcactccaca 240
tgctatcaga gatgttcctg atattaaaaa tgttaaatcg caattataat ctagaattat 300
tcctacaaca ttgcttccaa caacacttcc taatcgaccc atcattaaag atatacatac 360
tgccattgcc ctgagttgtg taaggt 386

<210> 897
<211> 105
<212> DNA
<213> Ctenocephalides felis

<400> 897

caggcagaaa cgagaccaat tactcactcg aagagtggaa ggcattgaca ttgccaagag 60
aatataattc caaaggacaa atcgactaca gctcttgcaa tatgt 105

<210> 898
<211> 498
<212> DNA
<213> Ctenocephalides felis

<400> 898

aagttttcgc agtttacaaa tccagaagag ataccannng caacttcaan atcagatcaa 60
tgagtgcagt tcanaattaa gaatgtctag ttcagagcta acaaagctgc tancgtcatt 120
atcaagcata ttgagcgatc aatcatatat ccaacagntt cncgatatca acgataccat 180
cactgagatg tcagaagttg aaaattggaa gtcatcaaca aatgccangt catcaangaa 240
tggcacgtca naaataantn tattaaattc tttttaaaat ttatgtatta ntaaattgtn 300
atgtctaatg cttcatatta ntattaagtt ttcaaatttt tatgttnttt tttgtactgt 360
atnaagagtt tacttanttt tcntagtttg nnatacctgg gtaatttgta cctattnang 420
aagcttaagc tcgnngaaaa ctanncctta ccnntatcag tgctangtat tttnacnnca 480
annataacan gcttngag 498

<210> 899
<211> 359
<212> DNA
<213> Ctenocephalides felis

<400> 899

cgtcaagtcc aatccaagag tggaaacttt ttcaggaaag ctactagaga ggtgtcattt 60
ccaccagtgg gatcgagaaa taatcgcaca attagctgtg ttcgtgccct ggaccaatat 120

gtaaatggcc aaggaggcta tgccactctt gtcaatggtg gagttggctg gaaaaatgta 180
actttactcc tatcttctca gactggcaaa ggatttaact tcttagttga aatttgggga 240
tattagatta gaaatataat gaaaatgtga atatagaaaa aaaattaaat atacaatagt 300
attttaaatt tggcacacat ctatctatgc atccataaat aaaggttata tacagcatt 359

<210> 900
<211> 353
<212> DNA
<213> Ctenocephalides felis

<400> 900

tgataaattg cctgcaagaa tgagtttcag ggaacgaaaa gaatcggcca aatgcatgca 60
aaaggttgca ttaaatatac aactagcagc agccagagtt tcaactgatg ttggcattaa 120
agtaggagaa gccctcagga atgtagtgga acttcgttta gatttgggaa aatgttcttc 180
agacaatttg aataaatggg aatccagatt ggaagaaatt aatgatgttg tggaaaattg 240
catcgtgtga aattgtaact tgaaataatt tttcttaact attagtttta tagatgaaca 300
aatacataat ctaataaacc agcaagtgaa aaaaaaaaaa aaaaaaaaaa aaa 353

<210> 901
<211> 498
<212> DNA
<213> Ctenocephalides felis

<400> 901

aagttttcgc agtttacaaa tccagaagag ataccagagc aacttcaaca tcagatcaat 60
gagtgcagtt cacaattaag aatgtctagt tcagagctaa caaagctgct atcgtcatta 120
tcaagcatat tgagcgatca atcatatatc caacagcttc tcgatatcaa cgataccatc 180
actgagatgt cagaagttga aaattgaaag tcatcaacaa ntgccatgtc atcaaagaat 240
ggcacgtcat aaataatttt attaaattct ttttaaaatt tatgtattat taaattgtaa 300
tgtctaatgc ttcatattag tattaagttt tcaaattttt atgttttttt ttttgtattg 360
tattaatagt ttacttattt ttcttatttt gatatacctg gtaatttgtn cttanaaaga 420
agcttaagct cgtncnaaac tatctttata tttatcaatg cctangtatt ttaagnncan 480
nantanaatg ctttgann 498

<210> 902
<211> 498
<212> DNA
<213> Ctenocephalides felis

<400> 902

tttttttttt tttttttttt tttttttttg gacggcccaa acnatttatt tttattaaaa 60
agntanatgt aatttcataa agcttctata tttacanant ttanataatc agaaattact 120
atagttaatg acantttgtt tgtgcnccnt acataaaata caattttata cattttaatt 180
tttatacata tttttttaaan ntaantgcac agatcatttg ngtatttatt atattaattg 240
actactttat ganaatttga atcctaatta gtctattcaa gattgttttc ctggtttaag 300

ccttacttan caatctcact tttgcattct tcgccgggtg gcgtgggtan gcancgcaac 360
tccgttttat caaagtgcaa gccanttgga caatcanaag ntttatttgc canttggttg 420
ttaaccaagn nnccattgnt aacanttttt acantctctt cgatnngaaa aaaacgtttt 480
cacctttgca tgcagttc 498

<210> 903
<211> 500
<212> DNA
<213> Ctenocephalides felis

<400> 903

ttttttttt tgtcattttt aatttatttc atcaactaca ttgaaattaa acaaataaag 60
aactattatt cttaaattaa agggaaattt tacatcagta cattcaacat gaatcaattc 120
taaaattatg tgaataacca aaattttaaa ccattaacac aaatattttt tattcccact 180
taacttatta cttattaaaa tattacttgg caagtatttt tatattttttg tattttttta 240
ggtatattat aaagacgcct ttagttgttg ccttatgaat aaaatacata tttataatac 300
attcacatat tacatactaa actattgaga agacttagac gatttaatgg aattaatatg 360
aaggaattta ttaatttaaa ttttataaat ataaaaaatt ttagtgcttt ttttangata 420
tcctangtta tattanaatt cgttttcatt tacagatgtg gcatatangt atantttttg 480
tnanttaatt tttagttatt 500

<210> 904
<211> 440
<212> DNA
<213> Ctenocephalides felis

<400> 904

antaatttta aaattaatct ttcaagttat tctgaatcgt ttgacagaaa aaaatttaaa 60
aacatgtttt cttgttttac taaaaaatgt cgttattttt tcaatgaata ttgaaagtgt 120
ttccaaaaat ttctaagtta atttaattaa ttttttaatt atttttattc agatgttgtc 180
acacaaaact atttatatat catcatattt antttgtata tattttctat gataaacctg 240
taaattattt ataaaaagaa gccaaaaaaa agaatttgta gtgaaaaaat atttcaaatt 300
tgaaacacaa actgaatgtt ctgaatgtta attatatgtg attaaaataa taaacattta 360
ttgaaatatt aaaaaannnc aatacaaatt atggtagata gtagaancca anngcataan 420
aannancnca aaaaagcttg 440

<210> 905
<211> 494
<212> DNA
<213> Ctenocephalides felis

<400> 905

tttttttttt ttttnntttt ttttttgaac ggcccaaacg atttattttt antaaaaaga 60
tacatgtaat ttcataaagc ntctatattt ananatttta aataatcagn cattacnatn 120
gttnacgaca ttttgtntgt gctccttaca tngaatacaa ttttanacat tttaattttt 180

```
atncatattt tttatattta attgcacaga tcatttgtgt atttantata ttaattgact 240
actttatgat aatttgaatc cnaattaatc tattcaagat tgnnttcctg gnttaagcct 300
tacttagcaa ncncactttt gcattcttcg ccgggtggcg tgggtatgca tcgcancncc 360
gttttatcaa antgcanccc gatttggnca atcataatgt nttatttncc ncntgtngnn 420
nnccccagaa ncattgntca nacttttnac aancccctcn atngggaaaa acctttcact 480
tntgcatgca cttc                                                   494
```

<210> 906
<211> 498
<212> DNA
<213> Ctenocephalides felis

<400> 906

```
ggaagcacct taggctgtgc aggacatgta actgtatagc attttgtgcc cagagccata 60
ggtccgcaat tatgacgtaa cggatcatat gcaaaatttg cggggcaata atattgggtt 120
cctatactat tttcgtcaca ataataatag ctttgacaat catttatatt tggataaaat 180
ctcgatggtg acggacattt gaaaccttgt actttgcana ctgtactttc tttagtggtg 240
cactgtagat atccactttg atcagcgcac acttgatctg ggctacagga ttccttaacc 300
ggatctgctc ctatttccgg acaatactgc atttcgggtg aactgcacga attcacgcaa 360
ctaaatttta ctttttttaca tttttgctcg ggtttcggta cangtgtacg ttcangagta 420
gtactactcc tggtgcgggc tcatccaatg atgcaaatac ttctttattt actgttaaan 480
gcgttccgtc gacattaa                                               498
```

<210> 907
<211> 215
<212> DNA
<213> Ctenocephalides felis

<400> 907

```
agactgaccc tgggcccagg cgtttgctta acagtgacgt taaagacgac caaccacagt 60
caacgaatga tgaaataagc tcatccacaa ctgctccaag ttcaaacgtg aatgaaagca 120
atggaaacct caccgaatca caacccttaa gccaaaatac ttcaacaaat actccattag 180
aaacatcaaa tacctcacta gcagaaagca gcagt                            215
```

<210> 908
<211> 498
<212> DNA
<213> Ctenocephalides felis

<400> 908

```
taccttttgc taccgatgct caagggcgat aacggaccca cagcctggcg tccaaaatgc 60
ttcctcgtcc gcaacgacat agtcgttttg caagatttat ccatgtataa ttttcgccat 120
tcgtatccaa aattctcttt ggatcttgat catgttaaag ttgctttaac ggcattggct 180
cgtatgcatg caagttgcat tatctacgag aagaaaaaaa attgttgcat ggggagagtt 240
tacaaagatc ttatgttcga gactatggcc aacaataatc aatggtggag aactggtgag 300
```

gatactgctt tagcaattgc tgaggaatct gagaaattcg gcaaatatac cgaataccat 360
tccatggtcc aggagaagtt aattgatttc ctgagattgg catggtctat ggtaaaacca 420
tcgcgcattt ataagaatgt ggtatgccac cgcgatacgc gcaatcacaa tttgatgttc 480
aaatataatt caactggt 498

<210> 909
<211> 245
<212> DNA
<213> Ctenocephalides felis

<400> 909

tagacatgca atataccaaa gtttctcagg actatacttg ataaattttt ttgcatggca 60
aacataagct ggtctttcag gttcctcaga tttagtcggt ggaaagatcg tgttattgta 120
agacaaaaac ttttgtggac cttcagatct atctgtgcgt tgcaaggaac tagatgtatg 180
aaataaactt cccctaaata atgaagttat tttgctattg attagcataa gttaagcact 240
ctcgt 245

<210> 910
<211> 386
<212> DNA
<213> Ctenocephalides felis

<400> 910

cctacacaac tcagggcaat ggcagtatgt atatctttaa tgatgggtcg attaggaagt 60
gttgttggaa gcaatgttgt aggaataatt ctagattata attgcgattt aacattttta 120
atatcaggaa catctctgat agcatgtgga gtgattgcat tctttatacc gaacatttat 180
cagaagcaag tggatagaag aacgagcatt gcttcatatg gaccataacc atatttccgt 240
cgtgtgttgt gaaatgcaat tatactttga gaaagtatta tcgattacaa agaaaattca 300
gaagttgcta ctgaatcatt ttctaaaaca tcgacatgaa aattaataac tcnttantgt 360
tattagattc natgtaagat atatgt 386

<210> 911
<211> 66
<212> DNA
<213> Ctenocephalides felis

<400> 911

ttattgcatt tggtccaaaa atctcaggct tataatattg gcccacagaa gaatatcctg 60
gtctgt 66

<210> 912
<211> 498
<212> DNA
<213> Ctenocephalides felis

<400> 912

```
tgaaatgtga agacgacgtc aacgaatggt ttgacccaaa atctttctcg tgcagaactg 60
catgcaaaag tgaaaacgtt ttttccgatc gaagagattg taaaaaatat tatcaatgtt 120
tcttggttaa caacaaatgg caaataaaac attatgattg tccaaatggc ttgcactttg 180
ataaaacgga gttgcgatgc atacccacgc cacccggcga agaatgcaaa agtgagattg 240
ctaagtaagg cttaaaccag gaaaacaatc ttgaatagac taattaggat tcaaattatc 300
ataaagtagt caattaatat aataaataca caaatgatct gtgcaattaa atataaaaaa 360
tatgtataaa aattaaaatg tataaaattg tattttatgt aaggagcaca aacaaaatgt 420
cattaactat agtaatttct gattatttaa aatatataaa tatagaagct ttatgaaact 480
aaaaaaaann nannnann                                              498
```

<210> 913
<211> 498
<212> DNA
<213> Ctenocephalides felis

<400> 913

```
caagttatct ctgaattcct tcacggctcc tcttgcgcat ttgccgactt cacgaaccaa 60
gttgcccaat tgggaccagg tttctttgaa agccttggcc aagtccttgg ctccttcacg 120
ggcagcttga aggacatctt tcaagcaagc tctggcttca tcgacatcgg ctgcggtaac 180
aacgcactcc ttggcttgtg ctctgagggc ttcggcgttc tccctggctt ctctccaggc 240
ggccaagcca ttttggcgaa cttggttgaa ttcctcacgt ttatcatccc tgcaagcctt 300
gaccttagca cgagcttctt gtgcaaaacc tagagctttt tggtgttggc ctttaaggca 360
ttcctttgcg gcttcactca ctttttttgct aagtttcctt tcaacttctt tgttgaacct 420
ttccaaatct ctatcgactt cangggcaaa tccttgtttt tcgaattcag cagcaatcat 480
ttaattttgc tatcntgg                                              498
```

<210> 914
<211> 123
<212> DNA
<213> Ctenocephalides felis

<400> 914

```
tcttatgatg tagtcttaaa taaatgacat tatttctatt tcataaaatg ttactaagat 60
tgctcatatt ggttagaaga tttaaaaata aatcagcagc aagataaatg aattctgtaa 120
tgc                                                              123
```

<210> 915
<211> 190
<212> DNA
<213> Ctenocephalides felis

<400> 915

```
aatgtcattt gttttcaaat ccgtagctat tttagctgct tcagctgatg attttgccac 60
```

accaaatgga ggtgtcggta tgccaccttc ctgcaaaatg ctgaaggata tatgctccaa 120
gacattgagc tttcgtgttt gggtgttttg ccatggtgct acggataaaa tcttaattcc 180
atttttcgt 190

<210> 916
<211> 500
<212> DNA
<213> Ctenocephalides felis

<400> 916

ttttttttt ttttttttt tttgctgaac tataacttgc tcttttattg ctcatcatat 60
aaggctcaac agaaaatcat tattaatatt acatattttt atagtgattt cattgatata 120
accttacatc tatttttaa ttcatttaat acctagtgcc aaataaaaaa gttatgcata 180
taagtgttaa caatttaaat gcttacatat aggacattta cttgaggcca agttatattg 240
acaatattca gttgatgcac gaataaaata aaatgattta attataatca ttctattcgt 300
acaacgattt tatacaatca atctgaagct acaagctaga attatataaa tacattttaa 360
tactatacaa atatncagaa atatttcatc ctcatcgtcc tacacaaatn cancctaaaa 420
tgtcttacaa ggacngtcat atatcacgaa aggaatnaaa attangaaag ggattaatta 480
anactacatt aataatacat 500

<210> 917
<211> 95
<212> DNA
<213> Ctenocephalides felis

<400> 917

tacgagtgca cattaaacgc tcaaggtgta ttcgacgtgg cccgatatgc ttgtgcagct 60
gagttatatt tcaacagcgt tctgcagcag tgcgt 95

<210> 918
<211> 68
<212> DNA
<213> Ctenocephalides felis

<400> 918

gaaaggacca ttaatttata ataattattt taatttgaat aaaattaaaa aaaaaaaaaa 60
aaaaaaaa 68

<210> 919
<211> 498
<212> DNA
<213> Ctenocephalides felis

<400> 919

ttttctactt tcgccgtcaa gttttataac gactgaaaat tcaataatcc aagtttaatt 60
ggttatattt ctttttacag ttatagataa ttctataata agtattgttt gagtgctaag 120
tcagtttcta ttcttgtttt tgtgtgctgt cgatgtcgat atcgatagag ccgcagacct 180
tatcaatttc acttatcgtt atgtgaaact actttgattg agttagaatt tgtcactgag 240
ctttatcaag gtcttgtatc atgaaaacag atcggttgcg aaataactgg catagcattc 300
tgacacaaaa atactagaat acctactcaa aacgtctttt atgaccgtgc gatgaccttt 360
gaaacgaacg ttcattagta tccgtatatc cgattgattt acttttaaca cgtttaatta 420
ctacagaagt aatatcaaac gaacggctnt cgctnaagtt tatatgaagt aagaattgta 480
acaagcaaag aagtataa 498

<210> 920
<211> 500
<212> DNA
<213> Ctenocephalides felis

<400> 920

ttttttttta attnttttt ttttttttt ttactttacn anatatgtnn gtattacaca 60
acattttagt tgcatataca agcttaaaat ataaatatat attatnaatt ttagttaaaa 120
aatctaaatg ctaggtaaag aacctantac cgaagtantt atggattcga atataatcna 180
agaaatanat ctaaaggcat ttgtatcatt cctgcnataa tttgtantga aataggnaat 240
aaatattcca caacananct aatgtattaa naaacattta cgtttggtca nccattcata 300
acaatctana tanttcaaaa taataaattt tacaggacca aatacatnaa gcggtcattt 360
ntgtngnant gcacnaggaa acacacatat tcaattattg cgccntgtnn ctataataat 420
ttttgngnnc nttntgcnac ttattaanca anatttgnga gatantattt ttcgtttctt 480
ttttganaca ttttttacag 500

<210> 921
<211> 173
<212> DNA
<213> Ctenocephalides felis

<400> 921

tttctttagt ggtgcactgt agatatccac tttgatcagc gcacacttga tctgggctac 60
aggattcctt aaccggatct gctcctattt ccggacaata ctgcatttcg ggtgaactgc 120
acgaattcac gcaactaaat tttacttttt tacatttttg ctcgggtttc ggt 173

<210> 922
<211> 217
<212> DNA
<213> Ctenocephalides felis

<400> 922

cccttcagta tttcttttat tttcaattac gaaaatcaga aaacagttat tccaagatca 60
tggttcttgt tcccttcttt aagtttattt cctatcctaa agtgttaact attactacct 120
aaactgcgat gatatttgtg atgaatgatg ttgacatgat gtccgaagac gttggctaag 180

atgcggtaaa attctgttcc tggtcatcat cgttccc 217

<210> 923
<211> 269
<212> DNA
<213> Ctenocephalides felis

<400> 923

agatctacta atgccgcgtt taccactgtc acagcaattc cgcaaaccag caaccacaaa 60
tataaatatg atgctattgt taaatgttca ataaatgcac aaattattcc acaaatcccg 120
catgagacca tcacgaatac aagaattgga agctttccca cagagttaat aatagcacct 180
attatgggaa atccaatcgc atatccagct tccaacatta aagaatgcat aaatgcctgt 240
tcttccattg tgtctttaca ctcggtcgt 269

<210> 924
<211> 303
<212> DNA
<213> Ctenocephalides felis

<400> 924

attananata cgctgagact ttaacctant tatagacata gaanatataa ttcttanata 60
ttaaaacctt ananacttac tntataaatt gtnatttaaa aacaggttga atataaagta 120
taaataatta tagaagattg atttccntaa cagtgagtgt tatgttaaaa atatatattt 180
aatacatgaa atggacaatg tttcanttat tganacgaac ttcttgacta tgtctgcaat 240
cgatgactgt gagttatata taaaagtttt cttanatact tacgtgtata tatataagta 300
tgt 303

<210> 925
<211> 145
<212> DNA
<213> Ctenocephalides felis

<400> 925

cnttacgcct tcagtgtgcc ggaattcggc tnancgtggt cgcggccgcg gtacgaaagg 60
accattaatt tataataatt attttaattt gaataaaatt aaaaaaaaaa aaaaaagaaa 120
aaaaaaaaaa aaaaaaaaaa aaaaa 145

<210> 926
<211> 428
<212> DNA
<213> Ctenocephalides felis

<400> 926

taagttgtcc ctaaattctt tcaaggctcc ccttgcgcat tttccgactt cacggactaa 60

gttgcccaat tgggaccagg tttctttgag tgccttggcc aagtcttttg ctccttcgtg 120
ggcagcttgg agaacccctt tcaagcaaac tctggcttca tcgacatcgg ctgcggtcac 180
aacgcactcc ttggcttgtg ctctgagggc ttcggcgttc tccctggctt ctctccaggc 240
ggccaagcca ttttggcgaa cttggttgaa ttcatcacgt ttgtcatctc tgcaagcctt 300
gaccttggca cgggcttctt gtgcgtagcc tagagctttt tggtgttggc ctttaaggca 360
ttcctttgcg gctttttgc taagtttcct ttcaacttct ttgttgaact ctccaaatct 420
ctatcgac 428

<210> 927
<211> 118
<212> DNA
<213> Ctenocephalides felis

<400> 927

catcaccgag aagaactctt cggctacgga atatcttcat tggaggcgcg ttgttggtgc 60
acgcagcatt aacaaggttc ttgcaattca gcataatgac cagaagaaaa atacaggt 118

<210> 928
<211> 489
<212> DNA
<213> Ctenocephalides felis

<400> 928

catttgcaag aattatcagt gtaatccaaa aacaatcctg tccgcacgca agcaaagggt 60
tgcgatggtc ctggtcctgg tggcggtggt ggaggtgcag ctgtcgccaa gcactcggat 120
aaaggcgctt gcacgcattg tgggtaaagc gcgctgaagt atgtctgtcc ggcgcatgta 180
aaacgaccca cgttgaaatt tcctccagag agtctcacac attcaaaata tactttgcat 240
gttttgtctg ctggatctgc aaatgagccg ctctgcacgc agctgtatat gggcggcggc 300
gtaggtggtt gccaaggcgc tggaggattt cttaggcact ccgaaaggga agcgaccaca 360
cattgctgat agaagtaagt agaaccaagg acaattgtaa cgcgctacac tgaatcctcc 420
gcctgctttc aaagcacatt tgtagtatcc tttgcaagtg ctgtcatagg gatctataaa 480
catcccttc 489

<210> 929
<211> 409
<212> DNA
<213> Ctenocephalides felis

<400> 929

gactcgtaat tatatattaa gtcagtttgg agaacatact aaaaacacta atgatataat 60
cgtaagtgaa aggaaattct caataaatgg tgaaattcta gctagtgcaa aggttaattt 120
aggtttggct atgtcacagg aaaagttttt aagtattaat aatttcattt tgatattaca 180
agaaatatta ttatctgtgt tgtttaatga tgataaaggg aatcacattg tatagatttt 240
caggaactgt caaatgacag gcaaagtata atcatgatta atgataaaat tctaaaaagc 300
aaagggataa aacaccctta atgtgcactt gcttattgca tttgactatt ctgaaagaga 360

tgtcaatagc taatgtctta ttgtagatgc tataagaaaa ctattaagt 409

<210> 930
<211> 349
<212> DNA
<213> Ctenocephalides felis

<400> 930

taaaacgaac tctagaatat tcaaacttag ctggaggtga tacttccaac atgaaatata 60
ttgagctaaa attaatccaa agtaaaaaag atatagaaga tccacaatta tgctgtaaag 120
ctgaggtagt atcttgatat gtgaagttct tgaacataat tgtgttctta aaaatatgaa 180
aatttgtgta tttatattta aagtcaactt attttctaaa aattacgata gcttataatt 240
atgtgttatg tgaatatgat tatcaattgt taaatcaatg tattctttat atgttactat 300
tatatattat taaattaana aaaanaaaan naaaanaaaa aaaaaaaaa 349

<210> 931
<211> 382
<212> DNA
<213> Ctenocephalides felis

<400> 931

taaaatcttg tggagaagaa tgtgtcattg cgatcaaaac caacacagcc cttgataaat 60
aatgtatttt tcaacacatt caaaattaaa atactatgag aaccaattca aactgtttaa 120
acaagtttga caaagttgtg tcgttgataa tcctagttga tgtattgtct attaaaatcc 180
tgacacttca agcattgttt atgaatatat tgatcaagaa tacaaggttt ttaagttctc 240
tctggaatat tttttgttct tccagagatg tttaattgac agttgtcaat caattgggtt 300
tttcatagac atacattgta acagtatgct tgttttaata aaatcaatca aattaataaa 360
tatccatgat tatgaattaa aa 382

<210> 932
<211> 313
<212> DNA
<213> Ctenocephalides felis

<400> 932

aattcttggc tataagttcg ttgcatgttt tgcaagtgat ttgtttattg catttattcc 60
cgtggcatat tttacatgaa tcagagtttg cattacaatc gttaccctga tctgatttgc 120
atcctcgagt aatgacacct ttttcaattt ttgaatagca gtcgtccttt ggattcatac 180
aaactttggt cttcgtcttt cctgcatctt tgaaacattc gtttcttagt tctggaagtt 240
tttgaacgtt acaacccgat ttctcgcaac acatttttc tcggttagtc gagtcacaac 300
tttctttaac cgt 313

<210> 933
<211> 85
<212> DNA
<213> Ctenocephalides felis

<400> 933

```
tttgatcaat aatcttattg atcgcaaggt ttgtgctgcc tcgagtaaag tgcacactac 60
gagaaaagtt gccaaaggaa tttgt                                       85
```

<210> 934
<211> 446
<212> DNA
<213> Ctenocephalides felis

<400> 934

```
gaggagactt tgtttaccgt aatggacaac cattcgaaat tccaaaagga aatttactat 60
tgaatgatta aatgtaatag attaatcaaa ttttagatta ttaaaattgt tctattacta 120
cagtagcaac ctcagcctga aaattaaccg aacaaatttc taacccttat caatgtatag 180
attttgaaaa ataacataga aatactattt ttttgatgac tgtaataaaa aaatgtataa 240
atggccatac ctgaaaagat ttctatgtgt attttttatt accttttatt gctgaatgga 300
taaaagataa atacaatttc ataagctctt ggattaaatt aattttgaat aaatccataa 360
ttataaaata tcaaattgaa atatggaact acaaaatgta tacgaaatat aacttatata 420
ataaatgana acnaatnntg ccgncc                                      446
```

<210> 935
<211> 491
<212> DNA
<213> Ctenocephalides felis

<400> 935

```
ttttttttt tttnttttt tttttntttg tacancatat aacgttatat ttcgtataca 60
ttttgtggtt tgtggtccat attttaattt gctacttaac atattctatg attatggntt 120
tattcaaaat taatttatcc aaacagctta taaaattgta tttanctttt atccagttca 180
acaataaaat gttaacanaa aaatacacat aganatcttt tcanacatgg ccatttaaac 240
attttttcat taagggtatc aaaaatatan caggcatggn catttataca ttttttttag 300
tatgagccan canaaaaata ntanaatttc natgttanta ntcaanntct anacantnan 360
anngatgtta aaagttaatt ttcangcaga agttgctact gnactnatnn aacaattttt 420
tcatccanaa ntngntnaac ccacnncatt taatcatcac nantaaattc cgnttggnat 480
tncgantggg t                                                      491
```

<210> 936
<211> 323
<212> DNA
<213> Ctenocephalides felis

<400> 936

```
aggtcaagag atatagataa atatttttta taaatttctt ccttgttatc aattanatta 60
```

ttataannta tattagcaag tgtattaata attatctcga aatattatat tanaattaaa 120
gcagctcgac tcacaatcag tcttganaca aatgtcggnc gacatattgc ttttacacaa 180
atcgattcac ttttacacgt ggtgacttgt tcatttgatt cgtcatctcg cgcattgatt 240
caaatctatc ccgcanactt ttggcacttt ctaattgtga gtcattattt tcagtttggg 300
cactcttcaa gaacatttat cgt 323

<210> 937
<211> 386
<212> DNA
<213> Ctenocephalides felis

<400> 937

aggnaatggc aaaaactgtg tctagaactt tgaaaaccaa aatttcattt tacttattaa 60
cgttttcaaa actttaaatt ttgtttttat ttatcaatcg atcaattttg tctttctttt 120
ttgtcttgaa acaatttaga gacactgtaa tttcacctag cacctgattc tgtgattaca 180
ttataattag aatattatca cgcggattat taacacaatt tcatcgtgaa taattaatat 240
aacactagaa tacagaagac gcacgtgcat gtattttaaa atcatacacg cgacaatggt 300
gggtggttta caccatctga atccggttca acaccggatc aggtgagcgt ctttgctaac 360
cattaaaggg tggaacaagg cggggt 386

<210> 938
<211> 385
<212> DNA
<213> Ctenocephalides felis

<400> 938

tttttttttt tttttttgcc ttagcatatt tgcgagcctg ngttcaacca nantattttt 60
ttatataaca cataaagnan ancaaagtan atattttgta tagtttcttt tgtngagnac 120
ttcatcaata tantaatata atttangntt cctcncgcan gttcccgtgt tgcancnttg 180
ngtatcttgg ntaaataatg ttncaatcgg acattgntca ggancnggnt cccctttagg 240
gcgtaaacac gtgaaaaact tccggcatgt tncgtnggtc tnnaaancac attttccacg 300
tnctcngnna nangtttctc tccttgggga aaactcggcg ggttcttcgc attgcaatac 360
ggctccnctt ccagcaatnc atacc 385

<210> 939
<211> 489
<212> DNA
<213> Ctenocephalides felis

<400> 939

ttgatagaca gatcagtcgc atcaattgaa tcttgagcta ttttgctcaa ttgactattt 60
tgctcaattg gaactgactg aatagacgga attccttcaa tactttgact tccaattcca 120
tctccaagaa ttttctctaa tttttgggtg aataaggaac ttctaattga cgctgagtca 180
gaatttttat ctctgagctt tacagaacta gatcctgtat tagttttatc agtttcgggt 240
ttataacaat catcacattg agaattcacc agtcctgcag gatcaactat ttgaatattg 300

tcattacagt gagctttatc atcgttgtct ttgaattctt catttttgat atcgatttca 360
tttttagttt cgggaaaatc tttcagcgct ccgtcacgta gtaagctaga ttcagtggaa 420
gatattgaac tacttctaat tgatttaatg gatgagttat tgctttttga agagcttttt 480
cgaagttca 489

<210> 940
<211> 478
<212> DNA
<213> Ctenocephalides felis

<400> 940

gttcattttg tatgaaactt tcagtaagaa gtaaggtatc cttttgtgaa tttgatggtc 60
tttctttcac tgatccaaat attggttctt cgcgattaat atcaacattt aattcttttt 120
gttcattact tgtcacggtt gttgaagtag tgattactgt attaatatct tgcaccgtgt 180
taatatcttg caccgatgat gctatctctg gaacattgct aatatcttga ggttctgaaa 240
cgtcttttga tcccactgtt ggttcacatt tagtaaattc caaagtaaac tgtgtgacgg 300
gtttcattcg tataacttcg tgggttatga gacaaatctt ttgagttttg ttttcatctt 360
taggaaaatc gtgtgaaatt gatacgtcat aaatatcacc aacttttcct gcaacttgcg 420
atgtgtttga agtcgttata gcnttcgagg tcctcatttc attgggatca atagaagt 478

<210> 941
<211> 139
<212> DNA
<213> Ctenocephalides felis

<400> 941

ttaaagagga tcaacaaaac atggaagacg aatcggtagt aactaatgtt cgatctccgc 60
cgactggtat ttggcaaccg attcctaatg ttcctcaaat gccatggatg ccacaacaga 120
tgatcagacc gactttggt 139

<210> 942
<211> 390
<212> DNA
<213> Ctenocephalides felis

<400> 942

cgattttttt gtttaatttt ttgcaaaaac aaaagtcagc cactttcggc gtgcatcatt 60
tataaatgat tcgacaaata accaaattcc tcgaagacgt ctccgcgagt gcgttaaagt 120
tccaacggga attctacaag gcaaaatcga aagagttcag gatgctcctt cagcagatct 180
cgaggagcag gcaggggacg atttttaaga aacaaccggc tctgtggtgt ctggtcggat 240
gtctagtagg tggttttagt ctggccactc tcagcctggc ccgcaccgtc aagaatcacc 300
cggacgtcta cctgatccgc agccactcgg gcgaggagcg ctactggcgc cgatacacca 360
aactaatcga cgtcagcgga tcgcgcaagt 390

<210> 943
<211> 274

<212> DNA
<213> Ctenocephalides felis

<400> 943

```
acatanacta atgccgcgtt taccactgtc acagcaattc cgcataacca ncaaccacaa 60
atatanatnt gatgctattg ttaaatgttc aananntgca canattattc cacanatctc 120
gcatgatacc atcacganta caacgaattg gaagctttcc cacagnagtt aataanagca 180
cctattatgg gaaatncaat cgcatatcca gcttccaaca ttaanggaat gcatagnatg 240
cctgttcttc cattgtgtct ttanactcgg tcgt                            274
```

<210> 944
<211> 598
<212> DNA
<213> Ctenocephalides felis

<400> 944

```
tatttttgca ataaaaacca gtaaccagtg gcacacaaat ttgtaatggg catgtttcat 60
tatgatcgat cgtattttat attaagagca attacattat taattaagat attacattct 120
cttgaattac ttcttattct ttggaacctc taaaagttct tccagtagtt catccatagc 180
ttctatcaag ctagtacgat aattttcatt gttctccatg agatccaaaa cagtctgctt 240
tctcattcca ttaacagtac gatcaaactc ctcagaatcg tggntcaaaa ctgccagtat 300
ttcaggaggc atttgagtaa gaggcaatgt catcaccgcc aaaattttag cgcacaaacg 360
caaatcatcc gtgctancat caaactcttg ttcaggaatt atatcgttta catccaaatt 420
ttccntcgta atgttttcca caaatgctgc tctgtagaac ctcttcaatc ctggtagngc 480
tggtctctaa agctccanat gttgtcgata tanaagatga ttgnatccan agctggtgga 540
ttgttgtgca tcttggantc gncaagatgc attgtnaagt anacanttgn ataganct   598
```

<210> 945
<211> 167
<212> DNA
<213> Ctenocephalides felis

<400> 945

```
ctgcatacca gtcagactgt ttaggtgcaa gtgtcacttc ttccccatcc ataccatcct 60
taccatcctt accatctttc ccaaccttgc caacatcttc cccaacagca ggatttcctt 120
ttggccgaaa atctcttgat atgcaaacaa aaactggaac taaatgt               167
```

<210> 946
<211> 160
<212> DNA
<213> Ctenocephalides felis

<400> 946

ttatttccca atgcanatgc actgctggtg atttgactcg tatggctaca attgttgccg 60
acaaactagg tgcccaacct ggttgtccac caattacagc tttgaccttc ttgcgtccat 120
tacatgccat gtttangcaa agggctctgg aattgggggt 160

<210> 947
<211> 193
<212> DNA
<213> Ctenocephalides felis

<400> 947

cctattccgt cacctacgcc tctgtcgaag tcctacaaga ttctgaggtt ggacaggctt 60
tcatcgtctc cggaggaatc ggccaatctg aaatccattt ggctgttgta gctgacaaaa 120
ttaagcaagt ccaatatacc tacgaaatct acgccattgt ctacgtttaa aatccttaaa 180
tgactgaaat tgt 193

<210> 948
<211> 413
<212> DNA
<213> Ctenocephalides felis

<400> 948

acacaattag caataggaaa atctagccac aaatattttt aacaatttat ttaatactta 60
tgttctagaa atacaaagtg taaagttcaa ggtttgtata gcttaataaa gtaaacattt 120
taaaatgtaa tgaatttatc tgcaaatagt caaaatcaaa attactatat aatgattatt 180
gaaataatgc ttttatagat tgcatttcaa tctgtattaa attgtatgta aatcacagca 240
caagatctaa cacacaattc atctaacaga aagaacaaaa taattttaat gttattaaaa 300
attacatata aaataacttt tacaataaag taaccttaca tgagatgtgt tgatttcaga 360
tacttttcat acaacagtgc tttctgtata aacaatataa tatanataac ggt 413

<210> 949
<211> 237
<212> DNA
<213> Ctenocephalides felis

<400> 949

aaacctatta attacaacaa ttacactaaa ttaaattaca attaattcct aaactaacaa 60
attcacttcc acgtgtctgt caaaaataaa atcactcaca aatctttaag caaacaccat 120
gacaagttca ctcttgatga caaagttctt atcttttatt tacaattgtc tttgcacaat 180
aatggnttat ttaatttaaa tcactttgat tgatttatgt gatgttcttc atctggt 237

<210> 950
<211> 131
<212> DNA
<213> Ctenocephalides felis

<400> 950

attgtctttg gagtctcaag aactaattcc agttgaaaca aattccagca gcgttcgtat 60
ttggcagcaa ccattcattt gtccaagagt tggactattt cgcgatccat tggatcctac 120
atgcaaaagg t 131

<210> 951
<211> 204
<212> DNA
<213> Ctenocephalides felis

<400> 951

agaaagctga aaaattagct aaggaagcat ctaattaaat ttataaataa gtgttcaatc 60
actgaccatt caaaatttta acctttttt acattatata accacacttt attcattata 120
gtttatataa tttaaaacaa gtagaaaatc cacaaacagc tttgtgatca gtgattttac 180
taaataaaaa aatatnaaan atca 204

<210> 952
<211> 598
<212> DNA
<213> Ctenocephalides felis

<400> 952

tatgatgtgc taaagtgctt tgtaattaaa tctaaatgaa aatgggagat tcaaaagcat 60
aaatgaaaaa ttaccctact tcccttgcaa tttgcatcaa aactcattct ttagagattt 120
cgtttattaa gttgtaaatt atatataatt aagagctaat gatatttata aaaaatatta 180
cacaaaataa ttcgtcaaac atttaaacaa aaatttgaac tttccaatgt atatgtataa 240
taaagggaac attaaaaagg accaattcaa aggaacggga tgaggctgac atggattaag 300
tagaaaaaaa gaatataatt gcaacaaccg gagatcttca atttgggtcc aagtctcaat 360
tcaacaaagg aatgttagtg aaattttgca atagctatgt gaagatatgt ttagctatga 420
aataaagcat aacanactaa gaaaaagttt tcttacaaca cagtgcagca attcttattg 480
cattagtcaa ggaaaaaaaa cataaatcga agttgtccac tgaagtggtt tgaaactggn 540
tccattcata aatatttgat ttatacttta ataatcatag tgtnntagtn ngtaatac 598

<210> 953
<211> 138
<212> DNA
<213> Ctenocephalides felis

<400> 953

taattaattt taaaaaatta aggatagaaa ccaacctggc ttaaaccggt ttgaactcag 60
atcatgtaag aattattggt cgaacagacc aaattttaaa acttctgcat tttaaaatta 120
tcttaatcca acatcgag 138

<210> 954
<211> 429
<212> DNA
<213> Ctenocephalides felis

<400> 954

aagagtatct ttcgaatgca atcaaggatt tatcctgacg ggagatccaa ggagaatctg 60
tctcgatgat ggctcttggg acgttcctgt ttatggatat actgaatgtt tacgtgaaat 120
attttacact actcgctcag catggttaac tgccggaatc atctttgctg ttatggtacc 180
tcggccgcga ccacgctcga gcggccgccc gggcaggtac ggaagcacct taggctgtgc 240
aggacatgta actgtatagc attttgtgcc cagagccata ggtccgcaat tatgacgtaa 300
cggatcatat gcaaaatttg cggggcaata atattgggtt cctatactat tttcgtcaca 360
ataataatag ctttgacaat catttatatt tggataaaat ctcgatggtg acggacattt 420
gaaaccttg 429

<210> 955
<211> 296
<212> DNA
<213> Ctenocephalides felis

<400> 955

caaantataa gataccggaa acatttanat aaacgatata tgaatattta atacatcatn 60
aaaaagaatt tattaaatta anagaaantg gngatctacg tcaatttatc taaaatagtt 120
tttaggtgta anaacnatta gaactaattc tatttgttta ctacttttgt tnctcgtatt 180
taggttttgn tttaaattan gtatgttatt ttagaaagca ctgataagta taaggnattg 240
ttganctgta ttttanaaga ctgccangca antcacgttt atttannnaa naangt 296

<210> 956
<211> 221
<212> DNA
<213> Ctenocephalides felis

<400> 956

gaacaatata gataccaaaa tcattttgaa cgagatggaa cttgcgcaag agctcacatg 60
gaatctttag ttgatggtaa aataaaattc agacatgtca tggaagaaaa tggaaaaaaa 120
gttgaattta gtggacaact cagacgtaat gatgaacatt ccggtaatgg atatctgaga 180
atcagttatg aagatacaaa tcgagaatca gattatatag t 221

<210> 957
<211> 126
<212> DNA
<213> Ctenocephalides felis

<400> 957

tggcactgct tttcatcata atacagtcat tagcgatgac atggtattct ttatcatata 60

taccctatgc tagggatgct gttaagaaaa ctgtctctag ttgtataaca tagtctgctt 120
agcagt 126

<210> 958

<211> 337
<212> DNA
<213> Ctenocephalides felis

<400> 958

```
cgaggggctt atgaagctca ctggccatct ttcgaatgtt ttcagtcggt ccgcttcgat 60
tttcatgtca agattttcat cgtcgccatt gtcgggcaca tcaatgtccg cggactgtcc 120
tgtttcatcg cccgtttttg taactgtttc agactttaga ttgattatat cgtatacttc 180
gggtctcgta tcgattgttg tattgtttgg aggcatttgt aatgcgttta gagactgttt 240
tgatgcactt ttattgttaa aaacatcgaa cactgggcgt gacgcgataa ctttgttaca 300
ctttagccca ccgttaacat tgaacgacac gaacagt                         337
```

<210> 959
<211> 498
<212> DNA
<213> Ctenocephalides felis

<400> 959

```
acatacacca aaatatctaa agctaaatat ttggctctta caattgataa tttgtattan 60
aagcatccaa tgaagcatct tgtaaacaac tatgcatctg tgaaatctat gttgattttg 120
aacatcagat tggggatgtc attgaaacaa aagttaaaga cagattcatt actgaaaatc 180
tgaaaaacaa actgaaggaa tttgttgcac atttatctga cctcccaaca tctttaaata 240
tgatctagta ttaagcataa gaattcaana tatacaacct ttccgttacc aatctaatgt 300
agacaaaaag caattactgg caatatttaa acttaatttt gcatatggat aagtcctatt 360
gtttagccaa caaacannaa tgctgacaat ctantatgtc tgaaaaacat caaaagctgt 420
tcttttggna acacatcana cacagtatta nncanacctt tctataaagc tctannnata 480
tnnnanagtt agannact                                              498
```

<210> 960
<211> 498
<212> DNA
<213> Ctenocephalides felis

<400> 960

```
agcatttttg gacccttttc aataagagaa aattttggta gtagttttat gacacaactc 60
tctggtgtgc cccaatattg atgactttcc ttccattcgc aaggagatcc aatacaaaat 120
acatgcccct cttcactgcg cattaagact agagtaggac ccttatatga taaaacatga 180
tgcaaaaatc tattcattcc caaaccgtgt tcccttgaat cataaagtaa tgcccaatga 240
gaaggcacaa cagatagtaa tttagccatg aatgcttgtg aggctaaatt aactccagaa 300
tcgctaggac tagaagcttt gactggctta gaatatagag gcggcaatgc acctgctagt 360
aaccatgcaa gcgatacagg taatagtgga ggttcaacca tttttccaaa tggatttggt 420
ttatccaaaa taggtgtgga taattccaaa cccaagccct cctcatgtgt gtcaatacct 480
ctgtatgatg tgcttaaa                                              498
```

<210> 961

<211> 414
<212> DNA
<213> Ctenocephalides felis

<400> 961

```
aaatggaata acactataat ttctgttaca ttatagttaa agtgtaaaaa aaatgagtga 60
aaatatggcg tcccatattc cttacaataa ttggntcact tacaatcagt tataacatct 120
gcatacaaaa tgtnggtatt tcaattttat acatacaana caaatttcat ttacaatttt 180
acgtagttat tttattaact aattgataat aaaaaccctt ttaagacaca aaaatgtgtg 240
tgaaatttan ttaatacnta tgaaaatttt taaatcttta ntgttaacan tgattttttt 300
ttaaatagga acancttaan gcaacattga gaaaatcttc gacngctatt gttatggttc 360
ctgtttgttg ggaatctcgt tgtctgaatg cttcctgtga gcctctgtat ttgn       414
```

<210> 962
<211> 234
<212> DNA
<213> Ctenocephalides felis

<400> 962

```
aaaagtgntt taccatagan gtttgtgaaa gacctgngtg tcctcaacgc tttatcagac 60
aaagtcttag aactaacccc tgatgaacgt cctagcttat tcttcttccg cttancanta 120
aatttcaaat cccccgnaga aancgnnaaa nccaanactg aactcaccaa nggcatcaan 180
cancttgagc gaagccttcg ataaaaccta nanaggcaaa gtgctgttca ccgt       234
```

<210> 963
<211> 379
<212> DNA
<213> Ctenocephalides felis

<400> 963

```
taacctccaa tgacgtctta acacgtaaaa caaggcaagc agatgatgcc ccaaaagaac 60
ccgatttcaa cctagctaaa gaatacaacg actcctaccc ggtggttttt aacatcatct 120
tatggtttgg agtagccttt ttcttctcgc tactagctat ctgtatctcg atctccacaa 180
tggaccccgg aagggactcc attatttaca gaatgacatc cacacgtatc aagaaggaga 240
attaagtttg tgaaggagtg tttttaattg taaatagatt gtttagtatt attacaatgc 300
aacgtgatca atattattta aaaatacaat atctttgttg ttacatattt gtttaacatt 360
cctcatatta ccgaattgt                                              379
```

<210> 964
<211> 462
<212> DNA
<213> Ctenocephalides felis

<400> 964

atggtgcatg gatgcatttc gctgatcggt aaaatacgcg atacaaaaaa ttaacaacgt 60
tgcgcgtctc gcgcataatt acngttcnan aattctcaaa nctttantct tctcntanta 120
naattatgtg tatttagtcn cgcgacnata nttatacgtt tcgcgctata tatanaataa 180
taaagattca ttacatactc aaattactat tacnatatac attttgcant gacaaccata 240
tcnttgnnca tgnaattata ttgtnactgt anattcaata catttctctt tatttaantt 300
tgnaactann ancnaactaa ctattantac tcgcattana tccannntta tanctatncc 360
tatnntcgng ttttttactt ttgtcataca ctttnctttt nttttacttg antannatcn 420
cttaaaacgn tnanaatnan nccnacatct atnttagnaa nc 462

<210> 965
<211> 258
<212> DNA
<213> Ctenocephalides felis

<400> 965

gtatatacaa taatcaattg ctaagataca tcaagcatat gtcatacata ctgtccccaa 60
ttttggaggt tttctaaaac aattatgaat ataatgtatt tgttaccaaa taatttgcat 120
atggcaacca tatatgaaga tctgtatatt gttcatggtt tcattccaat tctgcacgac 180
tcatattcag acaatcatcc tcgagctaat cctctccacc gactaattta agagatttaa 240
gaagtgaagt tatgtcgt 258

<210> 966
<211> 134
<212> DNA
<213> Ctenocephalides felis

<400> 966

aanaccanga acaanactta ggaacacaag gttttattac ccaccacaat tttgctggac 60
aaaaccctag ccaaagtggt ttcaactatc acaccagcaa tttccangca agggctaata 120
nacactgnna atgt 134

<210> 967
<211> 462
<212> DNA
<213> Ctenocephalides felis

<400> 967

aaatatgaaa tgtttccgac antcattaaa atttctttta ttcaaataca aaatgtagca 60
nttaanaaaa taatacaaca attaactgtc agccatttta tcttctgagc aaaataacct 120
ttttaccatg tttatcnaaa tttataaaat tgacctttaa acctttcccg atttattaaa 180
aatggatttg tatataaaat taaatgcgca agcaggaggc agggataaaa taattagtat 240

atgagcagaa ctttgtggga cttcctgcag aaacatggac aacatcagga agtaattgac 300
aagttgaaaa gtttggagtt tacattcagc tcattcagga aattgctgcg tttgggaaaa 360
tgcatanatg ttttcnactc ttcattgcca actatacatt acccanaccc acaattnnaa 420
ctacattngn nattgnncac nccttntttg nnttannttt an 462

<210> 968
<211> 470
<212> DNA
<213> Ctenocephalides felis

<400> 968

```
tactttcacc aattacaang taaatctttc aggaaatcaa tcangaaatt attantttat 60
gagtataccg agttgctcta tggacttaag atgttagaca aagttgtaaa ttatttaatt 120
tcaaacaaat ttgccaaata tttcattcan attaaacaaa ccattcaaaa gttacaagaa 180
ttttaaagtt agnaaacatt gcgacaacgt antgtcnaca tcaatttaaa attantaatt 240
tttcagtata ccgagttgct ctatggactt aggatgttag aaaaagttat agactattta 300
attttgaaca aatttgccaa atatttcatt taaattaanc aaacagttta nangttacaa 360
gaatttgaan gttagtaaac attgcgacaa cgtagggtcg acatcaagta nnnattagta 420
anttatgagt ataccgagtt gantagggac ttttggaanc gatntngtgt            470
```

<210> 969
<211> 397
<212> DNA
<213> Ctenocephalides felis

<400> 969

```
atcatgctga gctttcaatt cccaaaattg gcgcatgttt cctaaaggct gagatttttt 60
atctttagga attgatattc cagataggtc acgatctgca gttttaaccc aattctctag 120
tctgtcactt gtgatgtcgt atgtagacca tgtatcacgg aacgccatta atctgcgtaa 180
atattcacca acctcatctt gcaggtgtag caatacatcc attgcctcat tgtgtgcttg 240
ctcaactcct ggacaatcaa ctaactcttg aagtttttca gtcatcaaaa ccaattgttg 300
caatttagcg tgatgatgag ctatatctga actcanagat tgatataatg tgatgtattg 360
atcataatcg actgtcgtaa ctgcagacaa atcaggt                          397
```

<210> 970
<211> 340
<212> DNA
<213> Ctenocephalides felis

<400> 970

```
gaatactggt tcataaaatt tagaagccct aatttaccgc ctacggcgat atgatgatca 60
gacatatgtc cgggcttgtc caaaaatgta aaatgcacat gggaattttc caatggcgtc 120
cacttatgaa taatattggg aagtcctagc aattgcgtta tcaaattttc ctgcacgggt 180
tgaaaacaat ccttangcat cttcaaatca tcccttacaa ttttaccctg gttaattaat 240
ttggtaaact cgtcaccgga tttgtcgagt gtcttaactt tatcngcgat gctggttctg 300

cacgcgtcca attttcggcg tgttttgtcc aaatcttggt                       340
```

<210> 971
<211> 135
<212> DNA
<213> Ctenocephalides felis

<400> 971

```
tttttttttt tttttttttt ttgtgccttc agattttatt taaatatata acataagana 60
ataaaaacat tgatggctta tanaattcca atcttattan caatgtccaa tgcatcgtaa 120
tccctggcta aacgt                                                  135
```

<210> 972
<211> 498
<212> DNA
<213> Ctenocephalides felis

<400> 972

```
tttagctgca tcntccccaa cgntccaaaa tactcccccc antantaatc caacaagaat 60
gtgacanagt acacgcaatt gtgcaaagtn aaagtctcgt gacgtgcaca atagcgatcg 120
ttttgacaat actgcnaatt gtttganaaa tgatgtcggn tacaaagatt ccgatcttgc 180
tttcataact ttctttaagc tgcaagagag aggctccgtt tcgccaaatt ctattcgnat 240
tgcancantc aagagcngga tttttgccac gagcgattga tctgtatgtn tgttattatc 300
acatttcana ctgttgattc ctacttcggc gtgatttcta ctccatttan tttctgtnan 360
ctctaccaga ngatcgaggn tttcgcccgt tncaaacttg cnangntcta angcacaatc 420
ggctctatta caaaantgtg gnattganng gccgaatcnt ctatngttna aatcangtca 480
caagtgngcc tangtata                                               498
```

<210> 973
<211> 305
<212> DNA
<213> Ctenocephalides felis

<400> 973

```
tgtctgcttc tatatatttc tccacaatta ggacaatcaa taacagatcc agaccaagca 60
tatgacatta aaccagcaac ccacgaccct gcagcaagtg gcccattatt ctgactagta 120
tcagctgact gataatgcgg tgttacaata acttccattc cagacatatg gcaggccttg 180
caaacataaa ctttattgcc aaattgatgc tgaaatctgc atttttttact gttataatgc 240
gattctcctt gctgttcatg gcccatgctc tgtgcacatc tttcgccaca agncaagcaa 300
atggt                                                             305
```

<210> 974
<211> 171
<212> DNA
<213> Ctenocephalides felis

<400> 974

```
agaaaaaatg agccctgacc ataagcaaag accgaatcac ttgagtttta cctctgaacc 60
tgtaaatgtc cataacaata gtcctactca tagcccaaaa tcagcaactt tatcgaattt 120
accgagacga ccgccagtag atgtggaatt tatagatatt tcatattcag t          171
```

<210> 975
<211> 498

<212> DNA
<213> Ctenocephalides felis

<400> 975

```
tgaggtagaa gaaatataat aaatccaata caggagatac gaatcttgct atttgccaat 60
cgatgaagca aatttcaacg ggtttaccat cctgatattt aaacatcata ttattattcc 120
aacaatctcc atgtgaaaga actctataat tttcagaacc cgttgcattg aaacaacttt 180
ccatatattc ttcacaatta tctttgattt tcttcacatt ttcatgaaat tctgtgatac 240
ctccgtcatc tattgcttcc aaggcttgtt tcagtccaaa atcgcataaa tcgtaaaagg 300
cttctctacc ggtcatatca tacatggctg agtcgatttg agtaatttta tggaattttt 360
caggctgttg atctctcatg acaaaactta atgcgtgata tttagctaat ccttttgcaa 420
ctaacgttat gtgagaaaaa tccatttttt ttcttttgtc aaacattaca aaatttctgg 480
cgtgcaaatc ttccaaaa                                                498
```

<210> 976
<211> 255
<212> DNA
<213> Ctenocephalides felis

<400> 976

```
tgaagaagct aatcagcaag caaagcaaaa tgatatacaa aagaatcata ataaaaatat 60
ctataaagga ggacattata aaaattatca aagaggtggc tatagaggac gtggaggaca 120
tcattccagg cgaatgcaga atcgaacaaa tagtgatact actaacgggg atagatgaag 180
atggcttact cttttagtaa agtatatgta tcaatgtgaa gcgataaaga aatatatcaa 240
gtcattataa taagt                                                   255
```

<210> 977
<211> 498
<212> DNA
<213> Ctenocephalides felis

<400> 977

```
aagttttcgc agtttacaaa tccagaagag ataccagagc aacttcaaca tcagatcaat 60
gagtgcagtt cacaattaag aatgtctagt tcagagctaa caaagctgct atcgtcatta 120
tcaagcgtat tgagcgatca atcatatatc caacagcttc tcgatatcaa cgataccatc 180
actgagatgt cagaagttga aaattgaaag tcatcaacaa atgccatgtc atcaaagaat 240

ggcacgtcat aaataatttt attaaattct ttttaaaatt tatgtattat taaattgtaa 300
tgtctaatgc ttcatattag tattaagttt tcaaattttt atgttttttt tttgtattgt 360
attaatagtt tacttatttt tcttattttg atatacctgg taatttgtaa cttataaaga 420
agcttaagct cgtataaaac tatctttata tttatcaatg cttatgtatt ttaaaacana 480
aataaaatgc ttganagg                                                498
```

<210> 978
<211> 288
<212> DNA
<213> Ctenocephalides felis

<400> 978

```
agaaacacac acacacacac atgctatata tattacaatt ataatagatg ccccaaaccc 60
acatgcaatt gactgttatt tactaataga ttttcatact tatttattta acaaaacgct 120
cttttccaat tcctcctcat ggttaatgta tcagcatgac attaaacatc aatggatttt 180
gaaatagtaa ataattaata tttcagtcat ttcataaata atcaacaggc aaactataaa 240
tcacacaaag ggttgcgata atttaatagt tttgcttgaa tataatgt             288
```

<210> 979
<211> 498
<212> DNA
<213> Ctenocephalides felis

<400> 979

```
atttattttt gcttaacaag aaactcatgg acaaataaaa ttataggtaa catccggacg 60
atataaaaat aatttaaaaa atgtcacttt ctaacaatgt tctagattta ctaaaatatt 120
agttatgctt tttatgtgct ggattggcat gcaaaactct accaaataaa aataagaaat 180
ttgaaaagaa acaattattt aaatttaata tatttaatta aattaaatta cagggcagta 240
aatatctttc agcttatgtt cttaattcaa aaactttatt ttcttttaat tcaagatgta 300
taatttgatg ttgcctctta caattctagt attctactaa tatttttgga ttttgagttc 360
tctacatggt gcactactgg taacaacctg gtgatacttt aatggcaagt attaccttga 420
taggatttcc attaaaacat tgtggcataa gagtattgtt tcactcatcc tcagttgaat 480
tttcgacaaa taaagttt                                               498
```

<210> 980
<211> 251
<212> DNA
<213> Ctenocephalides felis

<400> 980

```
tttccgtaat gatgatggtg ggcgcgactt ccggaatgtt cttgccggaa actttacacc 60
aaaagttacc ggaaacgcta actgaagctc acgtattcgg cagggatcag gtgttctgga 120
gcttaccgaa aaaaggtgta tcaaagtcta cagatacggc cgtcaagaaa ttatcacaag 180
aagactgtta aatagttata atttgtattg ttatacatat gtttcttata taataaaatt 240
atatttattg t                                                      251
```

<210> 981
<211> 351
<212> DNA
<213> Ctenocephalides felis

<400> 981

```
tttttttttt tttttttttt tttgttgata aaacacaaat ttattttaca tgttaaaaat 60
tcttataaag taaatacatc atccagttac acacgtattt atatatccat ataactcgac 120
aaagtagctt tacacaattt ggaaattgca atgtgttaaa actaattgtt ttttagaact 180
atattatatt atattttggg gaatattaaa gttacggacc tacaaaatta agactatgta 240
atgtattctt ctttagccac tgacaaattt aaataataaa tcttgcttcg ataagtaatt 300
tgaactaata tcgatgactt cctttcgttt ttgaaaacta aaaactcttg t          351
```

<210> 982
<211> 224
<212> DNA
<213> Ctenocephalides felis

<400> 982

```
aatgattttg tttgacaaat ggtttttaaa taaacaggta ttcgaaattg atacgattga 60
tgatgaagta gaaaaattct ggaacagcca tataataata gtccaaaatg tatttcatag 120
taaattacaa aaataattga ttaaaattat aaatctatga ctgttcttga tatgaacctc 180
tttcataaaa catattaaga tttgattcaa aattgaggtt taag                  224
```

<210> 983
<211> 498
<212> DNA
<213> Ctenocephalides felis

<400> 983

```
tacgagtgca cattaaacgc tcgaggtgta ttcgacgtgg cccgatatgc ttgtgcagct 60
gagttatatt tcaacagcgt tctgcagcag tgcgtacctg cataccagtc agactgttta 120
ggtgcaagtg tcacttcttc cccatccata ccatccttac catccttacc atctttccca 180
accttgccaa catcttcccc aacagcagga tttccttttg gccgaaaatc tcttgatatg 240
caaacaaaaa ctggaactaa atgtacaaaa ggagaagttt ctaaagacga tttaaattct 300
acatgtacct agtgagtgtg ttatatgaaa ttgctattgc atattacata caagcttaat 360
aaaggttatg gtgatttatt tcatttaaag gcaatgtatc ggtttaatgg ttttaaattt 420
atttttattt aatattaaat agattaatta aaaatctata aagtgataag aggctgatat 480
ccataatatt attgaata                                               498
```

<210> 984
<211> 102
<212> DNA
<213> Ctenocephalides felis

<400> 984

```
agaccaagta aaaatctcac tcacagcttt taccttatta ggatcattta aaaatctatc 60
tagaatgcca ggttttgcca gagattgctg cactttctta gt                    102
```

<210> 985
<211> 204
<212> DNA

<213> Ctenocephalides felis

<400> 985

```
ggacggccga ctcgtccgaa ttatgctcgg cacgtgttcg attcaaatga tcccgctacg 60
ttcgacatcc aaatgataaa cactatctaa tcaagtgatg ttgtttaatt aagcaagtgt 120
tagttcgaat tcatttttgt gttttgtgta tattataata aatggacccc gaaattcctc 180
tgaaaggtgt gactccgggt ttgt                                        204
```

<210> 986
<211> 324
<212> DNA
<213> Ctenocephalides felis

<400> 986

```
gcgccggacg cgtcaaataa actaaattan atttaattta ctaacaaaca tttggtcatt 60
agtttaatgt caataatgtg cattttacat ctaataattt gaaatcatta atggtcataa 120
atcttcgaaa agttgatggc acttcatgct tcaaatcgcc aacgacatan caagctcaag 180
atgcaatatt aaactatgtt gtaaacatan natatattat tgaaatttta taaagaggac 240
ttttataatc gctcaaagtt tgacttaata caanataaat tatttctcnt tantatattt 300
ttatatttta aagaaaatgt ntgt                                        324
```

<210> 987
<211> 498
<212> DNA
<213> Ctenocephalides felis

<400> 987

```
gtgagttagc acacaaatta gtctcagatt cgatcccaac ctctgtcgat cctatatcag 60
acagcacgag aaaattttca caaattttat agatctcaat ttaatttcaa gtgaaaatcg 120
ccttgatgcc gataacaagc tgcatataaa aatattaagt taaaacatat tattagatat 180
aatcataata tgtcgcaact agtatagagc aactactgaa taacattcca gaaatccaga 240
aacaagccat gcaataaaat ttcaatacaa ttactgaatg tcctcataat atgaaaaaaa 300
aatanannan atantataat atataatcga agtaaatatt caattaacga aataaaatcg 360
taacanagaa tttgagagaa taatttgcac tatttatatg gcgatatggc gttgtcanta 420
gtattcnctg tatagaatga tcaatctgat aagaatgtca catattaaat tatttanttg 480
aaaatttata tgaatccc                                               498
```

<210> 988
<211> 420
<212> DNA
<213> Ctenocephalides felis

<400> 988

aatctgaatt tcatgaattt tggaaaaatt agtcatcata atttcaaaat ttgtctttga 60
caactttaaa ggtagctaat tctcacttcc tattaacttg ttttaggaca aaactccact 120
tacacctagt ttgaactaca aagactacta tatatacaaa ttgaatttta tgatttttgt 180
ataatttata aattcaaaat tcaaaatttt actctgccaa cttcaaaagt agctgaatct 240
cacttcctat taacatgtct taggaagaca aaatttcact tacacctagt ttgatcttac 300
aactactata cataaaggct aaatttcatg aattttggaa aaaatgactc tcatcattat 360
ttcaaaattt tacttcgaca actttagaag caattccact tactggtttg acctacaagt 420

<210> 989
<211> 500
<212> DNA
<213> Ctenocephalides felis

<400> 989

ttttttttt ttttttttt ttttctggnc catacaactc taagctttcc aacatttgta 60
ttttattcaa ataaatataa ataaaaatca taattcgaca attttacttg tatttatctt 120
aaacatattt acagtatttc ctcaaaaact gttaaataca ataagttaac ttaaatacat 180
ggatgttctg agaatgtgga tgagctcaag attgtagaac tgagtttgat ctacacaatt 240
tttgcctaaa aggttcgact actcagaaag cagccacccg tgattccata ccgcctcaca 300
tttgacaata aaatatatgt ataatgttaa ataggatttt aatggaaaaa agccaattta 360
atactttagc attatgaata tgtatttcca aattagtcta taaccgaatt taacctaaaa 420
tacaggaaat acatattcat anatatacat atattttaat acaaaaaggt aacttgtgca 480
aaaatgaaat tcaaatacac 500

<210> 990
<211> 498
<212> DNA
<213> Ctenocephalides felis

<400> 990

tctttttcga tcattacgcc agtagtatct tgcataatta tgttaacaat attgttacat 60
tttataaaat aatcttacta ttatctatag aacaatttat aattttatat atatcaacat 120
tattgtttat tgctagatat tagaatttgt gtatttgata aatgaatgtt ggtttgtgta 180
aatgatatat aattgcacga ttttagctca caaaagtttg cttagatatt tgaactaaat 240
tatggacaga tcgataccta gatccataac aaaaatttat ttaaatttca aatataattt 300
ttttttgctg catgctgaat aacatctcgc attatctttt gaaatcgttt ctttgttgct 360
aaatctttaa attctaaaca cttttgtaaa gatccatttc catcgaatgc attagtcatg 420
tcttgaaaat ctggagctgt ttcagtattc gcggaaatga aatttagagc aaaactacat 480

aaaactactc caaagtgt 498

<210> 991
<211> 498
<212> DNA
<213> Ctenocephalides felis

<400> 991

tttgctcttc atagttcagg tctttataag accagtatgc aaaatcagct tgtgcaactg 60
caaaatcaac atcctgtgaa aatactgcca gagcgttatt ttcttttgca tatgttatga 120
tttctgagtc acattcttca aatgtggatc taataacatt acatttattt tggaagctac 180
acattatgac ctcaccgatg ttacaagctg gctttatatt atattcatta acatattcca 240
aaccattatc atctaaagta tcgaagattt cataaatagc catgatatca gacctttttc 300
gagctaccca tctatcctgc atatcatcta tgttgcatcc atcaaaaaat gcaactaatt 360
ccacatcaaa cctttgcaaa aattctatga attcaccagc tttttgctta tacaatttgt 420
attgacctcc tgtcaaaaat ttttttggt cagtctctct aaatatttgt ctagagcaac 480
ttccatcgat cactatga 498

<210> 992
<211> 498
<212> DNA
<213> Ctenocephalides felis

<400> 992

acactgtcag taatattgat aaaagtggtg agcaagacgg aaataatgtg cagcctactg 60
acattgcaga tgacttaaac tgtgttgatg tggatagtgt tgacactgaa acttactcga 120
aatgcgacaa aaacattaaa cttattgata aaccactcaa gaagcaaatt gtagttttat 180
cagaaaatga ttttgatgac ggctgtccaa aatcgaatac taataataat aacgacccca 240
aagaaatttc ttctcatatt tgctaccttc aagatagcga ttttaatacc agatcagatt 300
taagtcggat catgactccg aaacatattt caacaccaga aataccaaag tctaacgcaa 360
ataattatgc gactttagat cagagtttcc atttaggtca aaacgttcaa aatgcgcaaa 420
ttaacaaaaa taaatacatt tatatcgatc ctaataaaat tgaacaagat tttaacagca 480
atttaaataa tttaaaca 498

<210> 993
<211> 235
<212> DNA
<213> Ctenocephalides felis

<400> 993

aattgatcca taaattatga ttaaaagatt aagttactta agggataaca gcgtaattat 60
ttttaagaga acatatcgac aaaatagatt gcgacctcga tgttggatta agataatttt 120
aaaatgcaga agttttaaaa tttggtctgt tcgaccatta attcttacat gatctgagtt 180
caaaccggtt taagccaggt tggtttctat ccttaatttt ttaaaattaa ttagt 235

<210> 994
<211> 72
<212> DNA
<213> Ctenocephalides felis

<400> 994

atcgatcttt ggcggctaaa catatcttgc tgagcgaagc cggccgtata gaaatatgtg 60
gctttaagga aa 72

<210> 995

<211> 206
<212> DNA
<213> Ctenocephalides felis

<400> 995

aacagacatt agtcaccacg cgctcgacga actttgattt tttttccgaa cgacaatcaa 60
gtaaactgca cataaaagag gagatgagat ttaattttg cgtttaatgt atgtaaatac 120
ttttaatctt aattaatttt tgtcattcgg aattaaagca gtcacttgat ggtctattgc 180
acggaccgta gtaaactgcg ccgcgt 206

<210> 996
<211> 260
<212> DNA
<213> Ctenocephalides felis

<400> 996

aatggtgtaa aaactgtaaa tatcccattt gttgtcacca gccattattg atgatttaat 60
tacagttatg aaactgctga tgctatgaga attaaaattg accaaattat tgttttattt 120
tatttaaaga taaatacata tatttaaatg gaaattcgtg gctctatgtc cctaatggga 180
cttctattat ccataggaac tgctacagga gtgatattca cgttagaacc agaggatagg 240
atttcattaa aaatgggcgt 260

<210> 997
<211> 310
<212> DNA
<213> Ctenocephalides felis

<400> 997

ttcggccgtg ttcttcatcc taagatgagc tcgggccaag gatgcgcaat cgccttggga 60
aacgacgacc tttaacgaac tgtcgtccaa gcgcatcacg ctgaaactat gactgtcgtg 120
ggttgatggc gaactcattg ttgaacctta caaaaactct taacttaaca ataaatactg 180
ttcattaaaa tcaataaatt atattaaaac taaaagcgat cgaaaaaact attttttttt 240
aatttggtaa aacaatctta aaagattgtt tagaccccgg aagaaacagt ggtttgttac 300
tgttgtcaca 310

<210> 998
<211> 59
<212> DNA
<213> Ctenocephalides felis

<400> 998
tcctggatga gcgttttgaa attctgtcat tttacttatg atatcgggaa accacatgt 59

<210> 999
<211> 498
<212> DNA
<213> Ctenocephalides felis

<400> 999

ttgaaactat tcattttaaa ataactgtga caatctcata ttatattatt cacatatttg 60
tatgctaata tagatcttat atattatatt catttttaca angatgcttc tttagaaaca 120
gatttttttt caaacagatt ttttaattgt ctttgaacat ttttcaaatt ttcttcttta 180
attgtaacag gtagtccaca ttctttctga tccttcatga tttgttccat aatanaaatt 240
tttcttttta gaaagccttc ttgctctgct tttaataagt tttcataggc tgccaatctt 300
ttagctttca actcatcaca atatatatta gttagttttt gcaatgattc tttcctcgtt 360
atccagtgaa aatgatttgg tggtggntta tatacaatat tcaantttttc naatatcnac 420
tcanatcttt tgtagtccca tctcctaang tatttcaaaa atttcttacg ttatcaatna 480
antctttang taaagccg 498

<210> 1000
<211> 231
<212> DNA
<213> Ctenocephalides felis

<400> 1000

gcaatcatgc aacaataata aagtatctat ataatatctt acaaattgta caaagttaat 60
catgaactaa atgtaatcct actaatataa atacaaaact tgtataaaat gttcgaatta 120
gaattttggt aaagtgaaat aaattctcta taaaactaaa aaaaatattt ttgcactgta 180
tcatatattc cattgactca gaactcaaag actataattg ccgaatcacg t 231

<210> 1001
<211> 247
<212> DNA
<213> Ctenocephalides felis

<400> 1001

agaactactt gcatcttttt taggatgtct tttgccacac attctgcgga acatcacttg 60
agcaataaat attcataatt ttgcgaattc cttgatttta aacaagcgca gacataatgt 120

atgtatatat gtatatatat atatatatat attgaatggt agagtcttgg atttgtgtat 180
attttgtaga gtttcgaaca agaatccaaa aataatacaa aaaaaaaaaa aaaaaaaaaa 240
aaaaaaa 247

<210> 1002
<211> 297
<212> DNA
<213> Ctenocephalides felis

<400> 1002

cacctatgta taattttata tatatatata taaagaaaat taaatctcac tcgattttaa 60
agataataaa ataaaataaa gttacattta taataatgta agattaaatt gtaaattcta 120
atttaattat atttcaaatt aaatatgtta atgtcgaact ttaaatgcaa tttcatttat 180
attgcacgat ttattttgta ggtcttgagt aaggggagct acgataacgc gtatcgcggg 240
tgctcacgtg acggctagtt agagacggtt cctcaacacg gcccttagag gagctgt 297

<210> 1003
<211> 498
<212> DNA
<213> Ctenocephalides felis

<400> 1003

```
acgcgttgat taattttgcg aggcagcagg ccaagccatt tctttttaat tagtagctaa 60
ttagacaaaa ggttttgtca aaaatcctga gccgatttcg aaaattttca actttttcaa 120
aaatatctgt ttggtattgc ttccccgtag ttacttgtta aaataaatta aaatagtttg 180
acaattttgt agaaaaaata tttcagattt gtaaaaaata ttttaataaa gtattgaatg 240
gtcgtggacg atgacaaatt ttcaaacttt tatgttcaca attttaaaat ttgctgcatc 300
tcaattcttg tagattaaat acacatatct gaaagttttt gcaacttgtc ttaagccact 360
aaaatgccac ttacacctag ttaatataag ccaaacccaa tttgtaaata agtatacaga 420
aaaacattcc aaattttata gataatacat aatctcagat tatttaagtg gtttagaatg 480
gttgccaggn agaactaa                                               498
```

<210> 1004
<211> 498
<212> DNA
<213> Ctenocephalides felis

<400> 1004

```
tcgttgattt agagttattg tattacatta caataaattt taatttttag ataaattgac 60
gtctttttac atatgtttgg aaaatatctg tattagaatt ttaattacat actatcacaa 120
attaataagg tgtatataaa tatacagaga aactataatt tgtttaaatt ttatttcgtg 180
tcgcttcgtt tcaacaaaca agcaaatttc catgaacaat ttattggcaa catttataca 240
aatagctaaa atttattcta tttttgaata tcatgaatat ttaaaactta aattatttta 300
ttataacgat atacaattat taactttttt tatatacctt tacgtttcaa atgtttttga 360
atattttggc ataccttaaa aaaattgatg tatgtgtgtg agtggatgcg gatacaaatt 420
ttcgggaaca ccgatcaacc gatttaaata aatcaaaata cattcgaata gtattaggca 480
ggtctagtcg ttccaaaa                                               498
```

<210> 1005
<211> 498
<212> DNA
<213> Ctenocephalides felis

<400> 1005

```
aaatctcgat agttacgcga ctcataaata attatttaaa caatgaatgt taaaaaaaat 60
taaagtttgt aaatataata tatgtattgg attaacttag ttaattctcc ctcgcaattt 120
tatactatag taaccagaaa caaaaatcag agtgtgtaaa atgtaaattc atatattttt 180
ggtaaaaata caatcaagat ataacagtgc cacaatatta ttaaaatacg tatttgtgac 240
gaaacctaaa gtgtagtcag aatataaaaa acaataatat ttataccttc aaatgtatgc 300
tttgaaaaaa agcattcgaa gattttgttt aaatattttg ttaagtccac atttatgttt 360
gttcgttcaa ttaaattatg tttcttttgc aagtaagatt atgttgtaga tattgtcaca 420
aaattagtat attttatgta attttaagca tattgntatt ttttggaaaa tcggactatt 480
gtcaccaaaa ttaaataa                                                498
```

<210> 1006
<211> 67
<212> DNA
<213> Ctenocephalides felis

<400> 1006

```
tattcgtagt atcaatgaag tcgtccgccc taagcattga tgaattttgg acaacgtagc 60
atattgt                                                            67
```

<210> 1007
<211> 498
<212> DNA
<213> Ctenocephalides felis

<400> 1007

```
catttgattt gattatattc ttgagtttat ttacttcatc ataacagcaa cataaaggat 60
attccaaaaa atatgtgcga atgaatttga tagttttcat cacctgtgga tgtccaatat 120
cgtttttaa atttttctca catagtgtag taagtccttt aagaaagtca ttcgacaacg 180
ttttggattt taattccaat aacagataat tctctggata gttactcgga aattgtatgc 240
acaaaataat tgtattaaat gatgtcgtgc taatttctac acgaaccatt tctccgacac 300
aagtaataag ctttgtatta tccaaagttt tctcacatag ttgtcgaact gttttaagtt 360
catcgtctaa tgcagccata cttcgttttc tctgtataat gtgttatata attatacagc 420
aacaaatgca gctgtttaga aatttttagt gaatatgagt atgatatatt ctttagaaca 480
tgtaagctaa ttagatta                                                498
```

<210> 1008
<211> 95
<212> DNA
<213> Ctenocephalides felis

<400> 1008

```
tacgagtgca cattaaacgc tcaaggtgta ttcgacgtgg cccgatatgc ttgtgcagct 60
gagttatatt tcaacagcgt tctgcagcag tgcgt                             95
```

<210> 1009
<211> 406

<212> DNA
<213> Ctenocephalides felis

<400> 1009

```
gtttgctata ttagttcggc ggtaagaaat attttttaa tagcttgatg aaaacaagaa 60
aatatttaat cgagtgattt taaatataaa aatatatata tagaattata tgacaacaaa 120
aaatgagtaa tgtagacgtc gtcgtgtttt cgtttcttgt cattttattt aacatttctt 180
acgcttatta catattgttg tatatacata catatatata aattaataga agttttatcg 240
aaacgatatt gatgaaatgt gtcttgtaag gtttttttta attacgaaaa aaacaaaaaa 300
tgtgtgaaac tgtgattcct gcctttctcg tgtaaaacgg cgatgctaga aaatagaaac 360
attaagcgtt ctttccattt aattaaaaat aattactaaa aagctg 406
```

<210> 1010
<211> 84
<212> DNA
<213> Ctenocephalides felis

<400> 1010

```
taattaattt taaaaaatta aggatagaaa ccaacctggc ttaaaccggt ttgaactcag 60
atcatgtaag aattaatggt cgaa 84
```

<210> 1011
<211> 228
<212> DNA
<213> Ctenocephalides felis

<400> 1011

```
ctcgtaaatc acttggtcac cagtttgatt tgtggtagtc tcattttcta taggnataaa 60
catcagntgg ttcattgaaa gggttgtttg ggtgcttgtc ggcttcgtcg aagggattgg 120
tgcttgtttc gggttctggt tcgtcgaaag gatttgctgg aacgtcttgc ttggcgtaac 180
tggtgtcccc gtagacttgt ttcgaagatt caccaattgt tttgacgt 228
```

<210> 1012

<211> 245
<212> DNA
<213> Ctenocephalides felis

<400> 1012

```
gggtggatca ttccaacctg gtggtggtgt agggtttgct ctacttggca tttgagtagg 60
aggtggtgct tgcggttgga attgattgac aaaacctgct ccttgtggtg aattcaacat 120
agggttaagt agggcatttg ttggttgagg ttgagaacta ataggattca gaagacttgc 180
tggttgttgg aattgaccag acattgtgct catcgtaact gtattggcat ttacaggcga 240
attgt 245
```

<210> 1013
<211> 351
<212> DNA
<213> Ctenocephalides felis

<400> 1013

```
atggaataaa taatgttggc ccatatatat ccaatttatt attaaaataa attcatgatc 60
cacggcagac cttaaattta atttgaggtc atttaaaaca tttaaaattt attcgattaa 120
caacaatttt tagtagtagg ttagtttaat tatgaatcac aatatagaat tagcacatgt 180
atttttgtga gtatgaactc acaccaataa acctgaattt taataatgtt taaggatata 240
actaggttta tgattacatt gctacagcaa tcatttgcgg gcagtttgga attgtgcatg 300
gttcagcact cttgtatttg ttgcctcttg ttctagcttc ataatgatcg t          351
```

<210> 1014
<211> 496
<212> DNA
<213> Ctenocephalides felis

<400> 1014

```
agcactgcca gaaacctgtg gctcatattc aacagccccg caagtagata caggtctttt 60
taacaagtct caagttataa ttcattgttg ttattgcaat aaactttacc tttgatctct 120
ttccaattat ctataaaaat tcattccaaa attactgcaa ttatttagag tcacagatta 180
ataatcttaa aaggtttcga gttagtgtgt tacattaaga ttttctcatg cttgacagaa 240
tcttagtttt tctttggttg ttttataatt tagaaaaaag gtgaaggtgt gatttgagag 300
attgtaaatg cttggcacta ttctatgaga tgtataaaat atatttttaa tattttaatg 360
atttatattt aaataaagtg ttacatttaa taataatgta tataattgca atacgtttga 420
ttacattttt tagttttcca tatgtaactt tatatttgaa tatttaatac attgcaatta 480
aaaaaaaaaa aaaaaa                                                 496
```

<210> 1015
<211> 194
<212> DNA
<213> Ctenocephalides felis

<400> 1015

```
atccttttttt tctatgaacg gttttaggta acggagaata atgtaaggca tcactaaagt 60
cgtttttatt tgtgatttca tgattaggct cgggtcttct gacccgcata aattgacccc 120
gcaattttgc ttcctttgcc tcgtttgatg atacagcccg ccaatccaaa aataataatt 180
ttggtaaatg atgt                                                   194
```

<210> 1016
<211> 401
<212> DNA
<213> Ctenocephalides felis

<400> 1016

```
tatcatcaac agcaacactg tgaacctttt ttcttctcac gacaccgttt atcgcggcat 60
cattggtcat atccctatca atagatgtcg aatctaatgg caggtgagta ggtctgggag 120
gagcagacat agacctttcc aaaagttctt ctgtcggttc aacgttcatt ttcattaaat 180
ccctcatccg tggaggtttt actggaggcg tgctattacc ttctgaaggt attgtctcat 240
aaatggcttc cgatgatgca cttgatgcca ctgtttttga caataattca ttgtcattaa 300
tgactgcagg ttccttttgg gcagctatat atgactcagg gcgtattcga tgtgatcgcc 360
tcgattctgt cgtcgtctga gattcagctg aatgggaacg t                     401
```

<210> 1017
<211> 422
<212> DNA
<213> Ctenocephalides felis

<400> 1017

```
tactgatttc aaaaacaaaa tcnaanagaa atatactatg aagttcaact gtaatgggac 60
ttagngcaaa taaatgcgag ggagtgagag aaagagaaag agaaagagag agtttgaata 120
aattatatta actattgcna gaaaaaaaga ntttaaaaca attatgttaa ctaatgacaa 180
tatatgntcc attcatcana tccgaaagac tgcacaanat acttattctg naaagatggt 240
aaaggtnagg ttttcgaatg cccacctaac tatgtatatg atcattctaa aaatatgtgt 300
aaaaagaaat cagtcagaag ntgattgcac cgtnatgaaa tgcacaaatc ccaattcttt 360
ataacctatg caccggancc atcaatntat gctttgggca atgacaaatt gcaaccgatc 420
gt                                                                422
```

<210> 1018
<211> 233
<212> DNA
<213> Ctenocephalides felis

<400> 1018

```
taattaattt taaaaaatta aggatagaaa ccaacctggc ttaaaccggt ttgaactcag 60
atcatgtaag aattaatggt cgaacagacc aaattttaaa acttctgcat tttaaaatta 120
tcttaatcca acatcgaggt cgcaatctat tttgtcgata tgttctctta aaaataatta 180
cgctgttatc ccttaagtaa cttaatcttt taatcataat ttatggatca att        233
```

<210> 1019
<211> 459
<212> DNA
<213> Ctenocephalides felis

<400> 1019

```
cctaaaaagc ggatttggat aaaatttgct ggttgtatgg gagctgccta ttcatcctcg 60
aggaaaaata gaattagctt ggattctcac acgtgagcaa aacccatcgt cggaattaat 120
gaaagaaatt tggacatatg tgaaaaataa tacacccata gatgttgaca aactaaaaca 180
caccgagcaa aattgtggaa aggatttctg aagataatta ctggatataa agncctttta 240
agatcagctt tatcaaaaca actgatgttt aagaataact aaatatatta tacttaatac 300
ttagcttttc ctggcaatac tatattaaaa tattgacata agtgcattaa tatttttgca 360
atagatttgc tttttatatt caatatataa tatgnattaa taataaaatc ttctaacatc 420
aagtgtataa atgcattaat tttctcaaaa aggcttatg                        459
```

<210> 1020
<211> 477
<212> DNA
<213> Ctenocephalides felis

<400> 1020

```
cttgaatttg ataagtgaag caattagaat aaagtccgaa cttttgctta gatttagaga 60
tgcattgatg agaccagcct tgtctaaagc aaagctttcc ttggaacgac ttgaaaaggc 120
tgtttcaatg gctattgatg atgctagact taaatttgaa aatgccatcc aagaaatgaa 180
tgacgacgca gtaggcattc ttaattccat cactgaaaaa atccaagagg aaaccgagga 240
gatcaaaaca aatattggag gattactatt acaacaaatg ttcgatgatt ctgcccgtca 300
atgcttgagg gatcaattgg ctgcagttaa agttttggcc aattcaacaa tcaacgatgc 360
aaattcatgt attcgtcgtc aattatttgg agctaatgat ttaagtgata atcttggtgc 420
aatatttgat gaagctttcg aaggtgttca agaattagtt gatgagctaa atgattg    477
```

<210> 1021
<211> 433
<212> DNA
<213> Ctenocephalides felis

<400> 1021

```
ccatgttggt aagatttatt attttatatc ccttncaaaa atctgcacgt tgttcacaaa 60
aaaaaatcat aaaatcttaa atttatttgc aaaaaaaaaa atagtgcggg aggagtgaaa 120
cttcgtaaat aaaactgcaa aagttacatt gataatttac aataaattca gacagtttgc 180
aacgtttcta gaaaagttca gaagttttga aacaaatttc atcattttca accaaatgct 240
ttcttttcta tttaccttta aagnttcact tcaatatctc cctataatat tttgntacaa 300
gagtgttgnt ttaagattta tagcatatta caaactgnta gttatattat tttctactta 360
tcggtaatgn ttcctttttt tggtaatcag tttacaaaac tttctgcaaa aagttgtgcc 420
atcaacaatt tgn                                                    433
```

<210> 1022
<211> 319
<212> DNA
<213> Ctenocephalides felis

<400> 1022

```
atttatttgt tgaatatttt atatcaattt atttttgata attattttga taagctcaat 60
ttgttctttg tgacaataat ttaggcatat aaaagaaaac cagtaaaaca atacaataaa 120
atcaccaaca attagtttgt ttttcttctg tgttattaat catcagaaca taaataaatt 180
ctataagtta ttttgatata cattattgac tattagatac aatttattga atcaagttaa 240
ttggtcgtaa ttttttacta tttttatatt tgtaaataat aaattaagat cttgctttta 300
gaagtcttca atgtatagt                                              319
```

<210> 1023
<211> 518
<212> DNA
<213> Ctenocephalides felis

<400> 1023

```
aattttggta atactttgaa atatattctt aaattaaatt ctttaacata tacataccac 60
tttattaatg gagttttatt tggcatgcta attcttccat taccttggtg attcattatg 120
tgttagattg gcgcctgtat ttattaggac gaagaaaatt gaacatgcca ttccttctat 180
tattttttat ggcatttggt gtggtattgt cactaccatt tggtgtatct gaagtttctc 240
ttgtgtcaga caaatgcaac aaagtcgcac gacctccatc taaacgacta ggcccccaac 300
ctgaagtgta tgcatattca ccagtatgcc ttcgcagact gtcattagca tgtgatccat 360
ttgctgaaag gctgtttggt tgagacctat gccctgcatc catttcatca tgagaaattg 420
tattccgatg ataatcaggg tttgttgatg ttctaattgg cacaggtgga ggcggtggtc 480
taacgctgac agtgacagta tttgaatact ggggtggt                         518
```

<210> 1024
<211> 112
<212> DNA
<213> Ctenocephalides felis

<400> 1024

```
ctggtttgca agtcattcca ctcggacatg tttccaatgt tgtttctaag aaatcagngc 60
caatcgcaat gcaaaggttt gctgtctcac aatcctggca ctttatttct cc         112
```

<210> 1025
<211> 304
<212> DNA
<213> Ctenocephalides felis

<400> 1025

```
atatctagcc aatattgcaa aattcttatc agtgcatgtg aaagaaaaac tattattntc 60
gaaaatatat caatattatt ataagtaaag aaattgtcat tccgaatgaa cttctgaggt 120
ctaaagaccc attacacaag tcagtgtcgc acatggtgca atttagatcc cgggctcctt 180
tatttgttaa atctttcttt atttcttcac atgtgactcg ggcagggaca cattctctaa 240
tcacaccttt ttcaatcatg gatttataga aaactctttc tccgatgtct gttgtacact 300
agcg                                                              304
```

<210> 1026

<211> 498
<212> DNA
<213> Ctenocephalides felis

<400> 1026

```
ccggtatgaa aaggaaagtg cttttacgtc caaaccgtgg agtttccaat atgccacatg 60
tcattattcg tttactattc ttatccttaa tatttatagc actgtcgtaa taattctttg 120
taaaatgagc catatctctg ttgagagtca taccgtaatc atatcgacat tgatatgatt 180
ctccacataa atcttcagca cgattgatgt cgctacttct gtttgatgga agaaaatctt 240
taggttcttt aacccagttt ggtataaatg aggaatttgc ataataactg gctgtncgac 300
caaattcacg agtgaagagt gccgctccga ggtgttcatt ttcacgatcc gcagcatcca 360
gtgacgtgcg anatctttat gaacagattc aaaactattg agattaacag gcacaaaagt 420
tccatcangg tngacaanag cgantgccat atcccaactc caatttccga gtagaccagc 480
agtttattaa taaatgcc                                               498
```

<210> 1027
<211> 305
<212> DNA
<213> Ctenocephalides felis

<400> 1027

```
ggaatattaa tgcattatcg tttcggtgct aatatattct tatatatgta ttaaatttgc 60
cccgttttct taatctgaca ataagagata ataatgtcaa caaaaacatg cgattttaat 120
cttgacataa aaagtgctaa ggtaatgtaa ataaaccgta ggcagctgat tataagacaa 180
cttttatata attactaaac aatttttatg gcataaatta atttaattaa ttaaaaaata 240
taattaataa aattgatacc attctaatca atataaaatt atcattttat ttattcaaat 300
cnaag                                                             305
```

<210> 1028
<211> 127
<212> DNA
<213> Ctenocephalides felis

<400> 1028

```
aaaggcactt tttgtcttca agcacacacg aggctttaaa ctaccaatgt aatatttgac 60
tagaataatt ttaattgttt cagcgtccac tgaaggcttt aagcaacttc gtgccaaaca 120
gactctc                                                           127
```

<210> 1029
<211> 498
<212> DNA
<213> Ctenocephalides felis

<400> 1029

aaaattccaa attttagttt ataaatcatc ttatatggaa actgtctgca acaaaccaga 60
ttttaaaata gaatccaata aatacggata taaaatttta ttaaaataca caaacataac 120
aaatgtcgtt aattactaat tcaaagtttc atacgatatg tcatatgcta attttagatt 180
tattatcata aataaattta ttatttaaat catattagag gaaaaaaaaa tattttctca 240
attctctcct tcaaaacttc tttgcacttc tccggctcca tacagtttta ataccacttt 300
tttcacaaaa ttcaacattt ttgtaaaatc cattgtgaaa attcatttgg ctgtttatta 360
aaagacatca tcccttcgga atctgttata taccccaaac cagcactctt caaatacaca 420
aaatcctttt tcaagttaac ctcgaacgtt acccatattt ggaagttcct tttggattca 480
acattgaatt tgttttcg 498

<210> 1030
<211> 454
<212> DNA
<213> Ctenocephalides felis

<400> 1030

acaagtanta attgtttata caatatgata catagttgaa aatagcatga aatatcatta 60
taattttgaa aggataaatt ctgttgacat ttttcttttt ttttaatatt tttcatatat 120
atttgatttt tgctgaacat tataaaattc atgaattata gggaaataga atttgtaaat 180
ttaaccaaac ttaccaataa gtcaacatta tatgataagg aaatagtaaa tatgacttct 240
taaaatatca gttattaaaa tttggagcaa tatgcattat caataaattt actggaattt 300
ttatgcctga aatcaaaatg ccagaatagg ctgtgaatgn tttaaatact ttttgatagt 360
tggatatgga atagctgatt tgtgaatgat tctactgatg natatttact aatactaata 420
taaaacacga ananannngn anaaaaaaag cttg 454

<210> 1031
<211> 154
<212> DNA
<213> Ctenocephalides felis

<400> 1031

tgatagatgt attgcaataa gttatttttt tatgaaagat aatgttcaac actaattgaa 60
atagattata agattttcac tacattttca tcccattttt tctacatcat gaaattctgt 120
attgtatgta attcattatt tttaagttaa atgt 154

<210> 1032
<211> 285
<212> DNA
<213> Ctenocephalides felis

<400> 1032

atatttatat tattatgata tatattgtta aattataaca atatgattat ttacatacat 60
attttattat ttatcttcta ggaactcttt atccatatta ttgaattttt ctactaaatc 120
tgaaggtatt tctagagatt ttaggtcatc tgtcccaatt tcttcctcag ttcttattag 180
aatgtcgaca acattctcac aagcaagcaa actggccttg tcaccaagtt ttaatgaaac 240
ttgttttgtt tcccatttgt ggtattctct taatatttca taagt 285

<210> 1033
<211> 498
<212> DNA
<213> Ctenocephalides felis

<400> 1033

atcgaaaaca aattcaatgt tgaatccaaa aggaacttcc aaatatgggt aacgttcgag 60
gttaacttga aaaaggattt tgtgtatttg aagagtgctg gtttggggta tataacagat 120
tccgaaggga tgatgtcttt taataaacag ccaaatgaat tttcacaatg gattttacaa 180
aaatgttgaa ttttgtgaaa aaagtggtat taaaactgta tggagccgga gaagtgcaaa 240
gaagttttga aggagagaat tgagaaaata tttttttttc ctctaatatg atttaaataa 300
taaatttatt tatgataata aatctaaaat tagcatatga catatcgtat gaaactttga 360
attagtaatt aacgacattt gttatgtttg tgtattttaa taaaatttta tatccgtatt 420
tattggattc tattttaaaa tctggtttgt tgcagacagt ttccatataa gatgatttat 480
aaactaaaat ttggaatt 498

<210> 1034
<211> 88
<212> DNA
<213> Ctenocephalides felis

<400> 1034

acaggataca agtgttctgt ctgcccataa taaaaaacga cataaacgta gtaaatacga 60
taagtaatat gggcatcatn tatcagcg 88

<210> 1035
<211> 394
<212> DNA
<213> Ctenocephalides felis

<400> 1035

ttagcccaag tttcacagca ggtttggcag tcactataat agtatgctcc ctttcatttt 60
cccctgaacc aactttgnat gtgtaagagc cagcatcatc ttgttcagtt ttttcaataa 120
tgaattgatg ttcttccttt attaattgat aacggtcctt gagatcattg atttcttcta 180

cttttttctc atctttaaac cactgatcaa caggaccatc tttcaatgga caggttagaa 240
ccaatggact gcgaatatca aaaagttttt gcgacgtggc ttcaccttct cgggcataaa 300
cactgctttg ggccaataat aataaaattg cactacacaa aaactgcttc atatttattg 360
aattttcttc ccctcgattg aaaaagttca taag 394

<210> 1036
<211> 498
<212> DNA
<213> Ctenocephalides felis

<400> 1036

```
taggaaaaat tatacttgat aacacttcaa attcactcaa aaatataaaa gattttattt 60
aacgatgtta ctctggtgaa cgaggctttt tttgaaataa tatacataca ctaagtatcg 120
agaactgttg cataaaactc aaaataatta ctgaagcatt aaaaaatttt gtcgccttca 180
aaatagacct tattcgaagt aatgaaaaca tgccaacaat taatccaatc ttcaaagcat 240
cttttaaacg cctttataag gatattcttc agctcctttg gcgttttttt cttacaaaca 300
ttgttagtgg aatctatcga tccaaagagg tatctaacaa tagtattact tgnaattcta 360
tacttaagta tccatgacaa taaataattt acagagggat agatctagtg aatacgttgg 420
ttgcccatga tatttcatga gtttatggcc nnaaaagcgt tcacaatata ggcgttgtgt 480
gaagagagtc aacaatga                                                498
```

<210> 1037
<211> 415
<212> DNA
<213> Ctenocephalides felis

<400> 1037

```
ccacatgaaa ttttaaatgg aaagtatgaa atcagtcgca ttcaagtatt taacaacatg 60
ctatgatagn nttattaaaa caaccaaatt taccatgtag caagtcttcg caaatcatca 120
cattttttt caacaatgga ttttcagtga cacttagaac aagtggaaca gaaccaaagt 180
taaaatatta tagtgaaatg tgtgcaaaac cagagatgaa ggatctagtc acactgaagc 240
aaactgttaa agaaatgata gaagctgttt gtcaagaatt tcttcaacct gaggagaata 300
gattaatatc aagagaaaaa tagatcttaa ttaaatagac tttaaataat tagcacaatt 360
tatttttagc tgaagagatt tcagttttca cttagctgaa ataaaacaac tattg      415
```

<210> 1038
<211> 109
<212> DNA
<213> Ctenocephalides felis

<400> 1038

```
aagctggctt gccaccgttt ggtcctccac catttgaagc aaatgttgga aatagaacag 60
taacattcgt tgaaatgtgc agcgacctag gatctgccat catagttgt            109
```

<210> 1039
<211> 440
<212> DNA
<213> Ctenocephalides felis

<400> 1039

```
gggnaagtag cgtgtgttgc ncttctgaaa tnantnggaa accctcggaa cttntngggg 60
nantngccna ggacatncta tgcaaaataa attagtaaaa caatataatt naantatttg 120
caatctttgc tgtgtataaa actangggnt tatnggntga aactgattta aattcatttt 180
tgaactttgt tcttatatgt tgntcgtcat tagctaggaa tgttcctaca tatactttaa 240
cattcaccta aatatatata tatgtaatct tatatgtata taaagttttt gatctgtgtt 300
ggcgaggtaa tattggcttg ataaacagtg tttttaacat gatgaatgtt caattgttaa 360
atcttgtgaa aatgtaaatt tgcaatttca gctgtgaatg ttttctggct gcctagcatg 420
tgtattgcat catatggngt                                             440
```

<210> 1040
<211> 500
<212> DNA
<213> Ctenocephalides felis

<400> 1040

```
ttttttttt ttttttttt tttttttat aaacaatttt tatttgtcaa aaagctacat 60
aagctagaac cattngacca caaataatat atcactagac atttttagga agtttacagt 120
ttgttataca attcttaaac taatttagtt atcagtaagt tttaagcagt cggcacagtc 180
tttacggact tgtactaagt tgnccctaaa ttctttcaag gctccccttg cgcattttcc 240
gacttcacgg actaagttgc ccaattggga ccaggtttct ttgagtgcct tggccaagtc 300
ttttgctcct tcgtgggcag cttggagaac ccctttcaag caaactctgg cttcatcgac 360
atcggctgcg gcacaacgca ctccttggct tgngctctga gggcttcggc gtctccctgg 420
cttntnttca gcggccaacc attttggcga acttgggtga attcatcacg tttggcatct 480
ntgnaacctt gaccttacac                                             500
```

<210> 1041
<211> 498
<212> DNA
<213> Ctenocephalides felis

<400> 1041

```
attagatatg gtctgaccat gattgatata ttttgataca ttttaggctt ataatatann 60
aaaatacagt caatttaaca tttgatatta catatattaa atcaacaata tctaaacatt 120
gaaattagag aaactgaacc tgaagatagt gatttaagta taaattctaa gctagtaaag 180
aagatccgaa aaaatctgat aatggtaatt ttaccgctcc aactaaatat ccaaaattcc 240
agcagaattt gtaatgtaaa tattgattct gataaactga aatcaccagt aaaaaaaatc 300
caagaagttc taataatggt aattttacca ctaaggaata taaatgctgc tgcaagagaa 360
gatttgacta atttcaaaca gaataccccca tcgaaataag cgatcagcaa ttgaagaaga 420
taattgtgct aattggtgat ggtcttaatt ttatgatcgt ttatatatgg taataataag 480
tcttataagc ttttgatt                                               498
```

<210> 1042
<211> 498
<212> DNA
<213> Ctenocephalides felis

<400> 1042

```
gattgctttg aaaaaagttc agaatttcca aaatatgtca aaataaaaaa acattttttt 60
ttcaaaagng gtattttctt gtcaaaatag tttctcaaaa atgttaatcg tttttttaaa 120
aaataataat ttttattacg ggaagttcca gcaatttgcc ttaagacacg atgccatgcg 180
cccactagtt ataaaattga ttttagataa tgactttcaa tacgtcgcag gaacatttag 240
atgcggaatg cgttgcagtg gttaaatcat ccatataatt tgtattagct acctaattga 300
cttatacccc aaatacctcc acattttcag ttatttttaa ctttctgtag ggtaaattat 360
agtaattgtc caaaaatcga aaataaaaat ggttgcatat cttcacgctt tggcatacct 420
tagaaagaat ggatttatgt gtgtgtatgt agtatgtatg tatgtataga gacaattttt 480
ccccgacgtt ttcgggag                                               498
```

<210> 1043
<211> 498
<212> DNA
<213> Ctenocephalides felis

<400> 1043

```
aacattgttg taaataatat gttctcgatc caatatacaa tactgtagta aataatatgt 60
gctcgaattt aaagaaatgt ccgggctcgt gtaaactaca tatatgatga ataatataat 120
catcaaatac tattgggtat aaacaatatg cattgaatgg tccagagtat aaataataac 180
tactttacgc ccccgggtgg gctcgaacca ctaacctttc ggttaacagc cgaaagtgct 240
agccaattgc gccacggggg ctcttatcgc ttcttgataa tagtaacaca tctcaataaa 300
cacattttac acatgatttg ccattttagt aaataatagg cactcgattt tatagaaatg 360
tcagggctcg tcccgggatt tgaacccgaa acctccgcac ccaaagcggg aatcataccc 420
ctagaccaac gaaacacatg ttctatccag tgcgtgttgg ggtgcatcca atgtcaacat 480
tgtagtaata atatggcc                                               498
```

<210> 1044
<211> 437
<212> DNA
<213> Ctenocephalides felis

<400> 1044

```
gattttcaat acaaagtgga agatccgcca attcaacttt catttggtgc caatgaagca 60
ggagatgcat caggaaaggt gaccggcagc tactacgttt tactccccga tggaagagtg 120
atgactgtcg actacgttgt agatggcgaa agcggttttc aaccgaaaat ttctttcaac 180
aaatagatca agctgacaaa ggaatcgaat cacgtaaact tcattaacaa catcatttca 240
tagctaaaaa tttaaacaac gaaacatata tactcatgaa ttactgccta tattattgtt 300
tatactgcct aataaacaat ttgtgattaa tactaattaa tttactactg ctaaacagaa 360

gaaatatatg acgaagctgt gaaacgcaat aaagttttgt aaatattttg cagattcatt 420
taaactaaca attatgt                                                437
```

<210> 1045
<211> 498
<212> DNA
<213> Ctenocephalides felis

<400> 1045

```
taattaattt taaaaaatta aggatagaaa ccaacctggc ttaaaccggt ttgaactcag 60
gatcatgtac gcaattaatg gtcgaacaga ccaaanttta aaacttctgc attttaaaat 120
tatcttaatc caacatngag gtcgcaatct attntgncga tatgttctnt taaaaatant 180
tacgctgtta tcccttaagn aacttaatct tttaatcata atttatggat caantattca 240
attatttatg ttttaataaa aaaaangttt tataaattnt cctatcaccc caataaaatg 300
tattaatata aataaantnt aataatattc ttaaaattaa tctatattna tatataaaac 360
tttataaggg tcttctcgcc tttaataata tttacgctnt ttaacataaa aattanattc 420
tataacaatt ntattaagac agttaatatt tcattcaatc attcattcca gctntcantt 480
aaaaaactat tgatatgc                                               498
```

<210> 1046
<211> 582
<212> DNA
<213> Ctenocephalides felis

<400> 1046

```
cctacacaac tcganatact cataaattat taanttctaa tcgattncct gaaaaagggc 60
gttgggattg gtgaaagtgc atttattcgt tacatttctg ataacttaaa aactatttgc 120
ggaaaactaa aaatatgttt ggagaaaata tagaaaattg aatagtctaa aacattttcc 180
taacatagga agaccctaca gcaactcggn atactcaaaa aagtttgttg aaaagttgat 240
aaatattcac agcgcttcac tgntttgttc ttgagtttag gaaaagtgca tttatttata 300
acttttgaac gggttatttg attggaatga aatattcggc aaatttatag aaaattaaat 360
aatctacaaa nttttctttt accgcaacag aaaccaacga aatattccct tcggctaata 420
cgggtaaaca tcacaaaatg natcgaatca atattttttt aaaattgaaa attgcaaatg 480
ataaatggaa ttggatttat attttttttct aaaaangaat ggggaactat tccaaggaat 540
gggagaatgn aagaaaaata atattattcg atgngaaaat gg                    582
```

<210> 1047
<211> 472
<212> DNA
<213> Ctenocephalides felis

<400> 1047

```
cccggnttgg gtgatcaaca ccaaagacac gctcagaatt ggcgttccat taggtaattc 60
caggttcacg agagtcaaat catcaaaggg gtagcgttct ttaaagcgtt tatccaaaga 120
gtcccaagta ttatccaatt cctcatccac aaccaaagga tatgtattct tcgaatccaa 180
actaagttta tcagtgcctt ctacaactac agtgactaca gctttaggta attgaaaagg 240
attcaattta ctcactccgt cccattcagc agaactctgc tccgatgtca atcctaaggc 300
tacggaatac aattccttta acaaagattg gggtaaagga gtctcgcctt ggaattggac 360
tccatttgga gaatttaata cactcagttc gccgttgcaa tatatggatg ccacaagggg 420
cagtaaataa attagaacct tttgcacatt ttattactta cggataaaac gt         472
```

<210> 1048
<211> 221
<212> DNA
<213> Ctenocephalides felis

<400> 1048

```
aagcagaaga tgagattaat ctgcggaggt tgttgacaaa ttaccagaag atctggncgg 60
aaaattgaac atttcagcac ttccttcgac agaggaagca gaaaaactgg ccagagaaaa 120
atgcagaaag gaaagtggaa gcgacgatgc ttacgataaa gcttttgccg ccaaagatga 180
gctgaagact tgtttcacgt ctttgctgaa tatggaggaa c                     221
```

<210> 1049
<211> 427

<212> DNA
<213> Ctenocephalides felis

<400> 1049

```
ccaataatta catttaattg aaagtgtcat taaaatattg accatgcatt atacaaatta 60
tatttataat tatngcgtta acaaaataaa ataattttgt gctaaagcct ctgcaataaa 120
aagaactgac tcatatgtat aaagatttaa ttgtataaat gcaatacaaa ttaattacag 180
ttaaaattta agaacatatt gcatgacagt ttgttataaa gcataaatga ctgcttagtt 240
tttttttta atgaaagttt aagacatcta ttataggaag atatatggcg gaaacagaat 300
cattacatat acatcaacat caatgtgcat tactttttt tttaatttct cttcagatag 360
aatacgatga atataaacat aagtatgaat tataaaattg tattgtatac tgtataaaat 420
taaatgt                                                           427
```

<210> 1050
<211> 570
<212> DNA
<213> Ctenocephalides felis

<400> 1050

```
aaatacaagg ggttgtatga aaccaactga tgactgtgat gatatgaaat gccaggagtg 60
caaaggtaac ctttgtaatg tcgatgtttt cccaagaaat cgcagggttt gcaacgctaa 120
agatgatgcc aaggaagttt gtctcaagcc acaagatact tgtttgagta ttttaaatgc 180
acaaggtgat gccataaaat tagggtgcac cagcaacctg ttcaagaacc agaatctgaa 240
aaaaatgtgc gagaaaaaac cagaacgttg cccaacttgt gacaaaaacg aatgcaatgg 300
tgatgccaaa aaacacgaat gtgtttcttg tgatgaaaat gatgaaaatt gccttgacga 360
ccctgaaaaa gtcgagacga agaccaaatg catgggcaaa tgctacttag atattgacgg 420

agataaagtg aagagaggct gcaccgacac ctacaaatgc gataaggaaa cttgtctgga 480
atgcgaggaa gagatttgca cgagcgaaat gtgctatgcg taaattgttt aaataatttt 540
tgcaagtttt aaataaaatt tatcaacttn                                  570
```

<210> 1051
<211> 386
<212> DNA
<213> Ctenocephalides felis

<400> 1051

```
atagatgnac annctnactn ncgaaaaccc cgncgncggg gcagaggcgc gntcaagcat 60
anggtctttg gtatttcgtt atggcggcga aaatatatcg ataatatatn gggattgggg 120
tataggaant ganttaatat tttattggaa acaaaagttt aaaatagttt tgcttaacta 180
ttatttatta aataattatt atgcatcatt ttaaaatcag tcatcattca tcataaatat 240
cgatattcgt atttcaatat atcgcacacc tcaattaatt tatgtatcaa ttatattgat 300
tganttattt gcgaatttct cagttatgaa aatgcagcaa tgcctgtgat agctcttcct 360
agaataagtg cgtggatatc atgtgt                                      386
```

<210> 1052
<211> 537

<212> DNA
<213> Ctenocephalides felis

<400> 1052

```
cctctctcag ggtaaagtga tatgagctga tttggtttca tttatgcaaa tcttccattt 60
ggcaagccac atttgtagtt tattgatatg ttcttgcgtt tgtcgggaag cttgatcagc 120
actgtgatta tttttaaagg tttaacttat cgaaatacgg aaaagtcaaa attcgatttt 180
ctgaaaattt cacactaggt gggcttctta atataaggtt gtagtgaccg aggtcgctaa 240
cgcgcggacc tatgtggatg gtgtcaagtt ggacggacct gtccaagggc agtagtgcta 300
cacattgnat atatgtatta ttttgtatta attttattca attacaattt aaaacaagct 360
gtttgaaatg aaacctttgc ttccatattg tttactccag aatctaaaat tatgaataaa 420
tgaatattct agtgaattat ttgtaaaagt tcttcttcaa cttagtatct aaaatacaac 480
aaacaaactg cgcataaatc gagaatatta ttagacatca tgcagagtcc ctgccgg    537
```

<210> 1053
<211> 331
<212> DNA
<213> Ctenocephalides felis

<400> 1053

```
ggtctgaaac actttcaaac aaactcagaa ttatcgatga tattttggaa atgcaaaaaa 60
tggctgatga gctaatatta ttggcacctc ttaaatcagt cgtaaccatt gaaacacttc 120
gcaaaatgtc aaaaaaagga ttgacttatg agatacttaa aaaagcttac gaaggaaagg 180
gagaagaggg ccttgaacaa attttgaaaa ctaaagtaac aaaggcaaag cttactataa 240
acaatgttgt ggtgtttttt caaaaacatg caaatgatgt ttaatgaccc ttatttatca 300

gaaatatatt ataaatattt aacattttag t                                331
```

<210> 1054
<211> 344
<212> DNA
<213> Ctenocephalides felis

<400> 1054

```
cagngattca tttcatattg gtatacatat ttctcaatat gncatttttc taaactttgt 60
gggacagnaa tataccaagt tccttccgta attgntcatc tccaatattt ccattatgag 120
gaatgcttct aagcattttt aaataactgn ctggnaatcc agattctaac gctccttgaa 180
taattgnctc aaganatatc aaactaggct tcctatcttc tggaaaaggn tcacccggtt 240
gcaacggagc aggcagttta ctttgctcat atattcggca cgatacattt tcgccactct 300
tagttttgat gnttacatct ttagcaaagt atgtgtttaa atgt                  344
```

<210> 1055
<211> 264
<212> DNA
<213> Ctenocephalides felis

<400> 1055

cccatattga acatttattt ttagcgacta cattagaaat gtaatagata aaaatgttat 60
aaatctgcga atataaaata ttgtattttc ataaagaata ttattattct ttcttatttt 120
ttgttcatag cagaatattt ttttcgcccc aaaaagccga cataatacac cactaaagac 180
ttgtcgaaag ttgtggcgtc cgggactcgc aaattcaatt tgtgcacaat cgcgcggtga 240
aatgcacaat ttccatttct atgt 264

<210> 1056
<211> 647
<212> DNA
<213> Ctenocephalides felis

<400> 1056

tatacatatt tcagtgagan atcagcaata gtttatgatt ttattgacgt tgtgaacgga 60
catgtgntct gggngtttat aaatttgcgt atagaaacaa taataaatta cagtacataa 120
tactgatttg attttactac cgnaacgatt tgcttcagat atttccagaa aaatgttagt 180
tttcatacat tttatttaat ttaatatctc cgacttgtta ttactaagcg aaattttctc 240
tcttttaaat atagaattct ttatagtatt cataatagtg aagctttaaa gtatttctct 300
cactatcacc ctttttttata caaacgcatt ttgagaagta acgtgaaagc ttactttcga 360
attggtgatg nttctatgaa tacttatatg tattataata tgnattaaat tgaattttttg 420
cgtttcaata gaacttcaat cataaatttg aattggggtt aatattggtg ggggttggat 480
tattaaaaga cttcgatatg aaaaaaatca atgggncatc aaatcctagn aggatatttt 540
atgnggatac ctagcacccg caatggnggn aaatggcaaa aactggtatg accccgattt 600
ttgacgaatt tttggtgggc gatggtgnaa actaaaaatt tntcgct 647

<210> 1057
<211> 499
<212> DNA
<213> Ctenocephalides felis

<400> 1057

ctcaatgaaa tncctattnt gntctttttc tgctttacag gatttaatgc aaactgnggg 60
catannnaat tggaataact caaatcangt taaatgatct atttcagata aattattaca 120
gtatcttttc atnctaaaaa ttntatnccg ctntacgcan ttttaaaacg gtctctgatt 180
attttaatca tnatttgtaa taattaattt aatgcttatg caagcattct nataatactg 240
taatttaaat attgnaanaa ttaaatgttt ntaggtttng gactagtgtn tataacagat 300
aaattaatat attattgtng gattgtattt aatnntttaa agaatattaa natcagtgtt 360
tggnttatat agttnngcaa tnctgtctaa agtacctggt tattttnatt tattttagga 420
tcattatgaa gcaattgcaa ananttattt ncaaatttaa atttatatnt tttttgacca 480
ngtgccaatt tacttgncc 499

<210> 1058
<211> 310
<212> DNA
<213> Ctenocephalides felis

<400> 1058

ttttttttt ttttttttt ttttttttt ttaatgattt aattaattta ttntaagcca 60
ataattgata ttaattatgc attaatcatt gnatttatac tttcctagaa aactatacat 120
cacatgttga aacaaattaa ggttcatggc ttctcgcctt tccttagcgc ttgttttaaa 180
tttgatccat tctttccgaa tcttgttact ntttggtccc catttntcat tgggagtgaa 240
cacgtttttc aatgtatcat taatggaatg ttttctattt ttcaaatata tccaaattgc 300
ccaaagnggt 310

<210> 1059
<211> 215
<212> DNA
<213> Ctenocephalides felis

<400> 1059

atctacaaca gcaccgataa cggcaacaac tttnccttgg gcacctgctg ctgctttggc 60
agcataactc ctgctgttcg ataaaatcga tgcgatttta cccgattctg ttttgctcag 120
ggttcgcaaa gtggaattga ttacggagtg catcttgtaa taaaatgtat agccagttga 180
gttgaattaa attgatctca cctgtaagct actgt 215

<210> 1060
<211> 275
<212> DNA
<213> Ctenocephalides felis

<400> 1060

agcaaaacgt ttccataact aggatatcct acttgaaagt gatggctatt gttggtactt 60
attgcatttg gtccaaaaat ctcaggctta taatattggc ccacagaaga atatcctggc 120
ctgtactgag aaaatgcgta aggtggtgga tggtaatcac agttcgccca ttggacaatc 180
gagatggcca ccaaaaatat tacagctttc attttagtca cttggtgaaa tatcacgaat 240
tgaaatattt caaccttcgc aaaaggagct tgtga 275

<210> 1061
<211> 330
<212> DNA
<213> Ctenocephalides felis

<400> 1061

nnnaagccct ttngaaaccc tnggaangan tctggcgccc tttcccgtac atatccnagt 60
taatatatat tatagcttct aaaataattc nntcacgatg tttattttgt ctcaacggtc 120
aaaataaatn ctgatttttt tttttaaatg taaaagatca ataatcaatg aagcaaagat 180
tttataaaca tttttgctaa atttttatgt aattgaatta tttttcatgc tatatataat 240
ttatattggt agttaattat tagagatttt ctaaaaaaaa atgatctaaa atttggaata 300
gaacaaattt ttcatggcgg cctaaattgt 330

<210> 1062
<211> 126
<212> DNA
<213> Ctenocephalides felis

<400> 1062

```
tttactactt cagaattatt tatttctagt aaacaacgaa tttaaacttt tagaatattc 60
aaaattaatt gcgacaagca atattaccat gcactcttga agattttaaa aaaaagtagt 120
taaggt                                                            126
```

<210> 1063
<211> 116
<212> DNA
<213> Ctenocephalides felis

<400> 1063

```
ccgtttgtta tatattggca tacaaatgca ttaagattag gctctgagac agtgattgaa 60
tgttattttt gttattttaa ggaaatttaa taaaataatt attaaatcga aaaaaa     116
```

<210> 1064
<211> 333
<212> DNA
<213> Ctenocephalides felis

<400> 1064

```
aattaaaatt ttctaacatt cacaaaattg ntnntcaaan tccgaacgat attttcaaac 60
aataataaat taacaacgga cttagcaatt ataaatatta cagctcacta ctacaaatta 120
catattttta tcaatcatat atatatatat atagttttgg tttaagatat ataacaacaa 180
tgtttctatc gcacatatca ttaaatatac tttcatatgc gacaagacaa gttttgcatt 240
aaaaaaatat atgttactaa tgacaatatt tagaatcaca cttgaagact cctcctcgga 300
gcctncgggg ggcgccgagt aaaacaacaa tgt                               333
```

<210> 1065
<211> 498
<212> DNA
<213> Ctenocephalides felis

<400> 1065

```
agtagagaat tgacagcagc nattgctgcg gaaaccgcac tcactgaact tcagcagcaa 60
ggaaatgatg ttctcatggt tccatttata acaaaatgtc agctgccatt ggaaagtttt 120
cgattgttaa aaaccagcaa agccatagtc aataaaagaa gtttgaaata tcagattttt 180
ctgttcgaga atttaatact gtttactgaa cctactattg tgaacaatga agagtttttt 240
acctgcaaag actatatatc tggatagaat tttaccacct gtcgaatgtc agaagtcttt 300
atatgcattt accctgacga gttcagaaaa taaatctaga attttccata taaaatgctc 360
aagtggcgat gaaaggatcg tccctggatg actattatta ggaatatttt gtttcagcaa 420
caacaggcat gggaaaggaa gccatngaaa aataaattag ctttattctn ccgacaggag 480
ggcttccacc caanaacc                                                498
```

<210> 1066
<211> 264
<212> DNA
<213> Ctenocephalides felis

<400> 1066

```
aaccacgagc attcctattg ttaatgtcgt gactgacgta gcatcgatct tcgtttatct 60
tgcagactgc cactttagct gccaattccg cagcagtgtt gcttgtgcag gtgtcatcat 120
tttcgttgca ttttacacat ttcaggggat catttttatt acataggtta ctattgcact 180
ttagacaaga cccagatgtt gcattttcgc attcttttct aatcgaatca gcttcaggta 240
aatcgaccaa acaacctttt tttg                                        264
```

<210> 1067
<211> 498
<212> DNA
<213> Ctenocephalides felis

<400> 1067

```
tgtgactcat ttggagttct tgaaggtggt tcttctaccc aagctttatt agatccctgc 60
atgctcatgt tcaagctatg accaggtgga ggatgttcaa ttatgtcgaa ttgagagtta 120
```

```
cattgcgcac tccttacacc tagtgagttc agaactgtta tattctcagc tcctgatttt 180
gtgaaaagca tatattcttc taaaggntnc atagtatgat tgttgacttc aatatatata 240
tttgagaggc atcctgaata gctttttgaa tgtagcaaat ctgggatcat ctgncgataa 300
accaccgatt tgtatttcat tgattccttc ttaggagatt ttacaaaatt gacttcgggt 360
aatatttcta aagatcttna agttctgtct attaacaata tgggagcctg ttgngatacc 420
cttttggnat nagacgggaa tgcttagcac cgttttagaa aannttctn gttaactttt 480
ngcttccaaa cacaagac                                               498
```

<210> 1068
<211> 422
<212> DNA
<213> Ctenocephalides felis

<400> 1068

```
taacctctaa tgacgtctta acacgtaaga caaggcaagc agatgatgct ccaaaagaac 60
ccgatttcaa cctagctaaa gaatacaacg actcctaccc ggtggttttt aacatcattt 120
tatggttcgg agtagccttt ttcttctcgt tacttgctat ctgtatctcg atctccacaa 180
tggaccctgg cagggactcc attatttaca gaatgacatc cacacgcatc aagaaggaga 240
attaaagttt gcgaaaggag tgtttttaat tgtaaataga ttgtttagta ttattacaat 300
gtaaccgagt tattaatttg tgatcaatat aaattaaaaa ttaaaataca atatctttgt 360
tgaaaatcga attattgntc tattatattt tttgttttac attcctagta ttaccgaatt 420
gt                                                                422
```

<210> 1069
<211> 288
<212> DNA
<213> Ctenocephalides felis

<400> 1069

```
catagaaata taatggaatt ttacttttaa cattgtttat aaactatact ttacaaatta 60
attgtaatat ataaatataa ttatattatt tcataaaata tttgttctct gactagtgta 120
gatgttttcg tgctttctaa ataagggaag ttatataggc ctgtatgcct ttctgtgaac 180
aactctatgt atgttctaat tttgttagga tttattttta taaaaatgaa tatataaata 240
tataaccgca ttaaaaaaaa aannannaaa aaaaanaaan aaaaaaaa             288
```

<210> 1070
<211> 498
<212> DNA
<213> Ctenocephalides felis

<400> 1070

```
aatttacaat cagaatcaat cttacaatta gtgtagttgt ctatgagaga aaacatcggg 60
cgttggtcac aaaatgatta acagttatag aatgccatgt gtttaatacg cagtttacaa 120
tataatgtta ctattgttag ttggcatact gtaccgagtt ttattttttt tatgaaaaag 180
aagatagagt ggccaaatta ttttggtcat gtgatatttg ttccattaaa gattatatga 240
gggcatgtgc attggtaggt gcatgattta atatttttgt accgttgcgg aattgatttg 300
tggtggagat actgtattaa agngtttcaa atagttttga aactgctctt gtgtgaaatt 360
tagtatagtc cccaattcaa tctaagangn cttgggtctt ccctttatgg tgtcagcact 420
tggataaact ttattctacc ttatccantt attttagtta nnatgctccg ccccttnggc 480
cagnatantt tgaaccga                                               498
```

<210> 1071
<211> 269
<212> DNA
<213> Ctenocephalides felis

<400> 1071

```
caggattact aatgnccgcg tttccactgt tncagnnatt ccgnaaacca ncnaccncgn 60
atatnaatat gaagctattg gtnaatggtc aataaatgca caaattattt cacaaatccc 120
gcatgagacc atnacgaata caagaattgg aagctttccc acagaggtaa taatagcacc 180
tattatggga aatcaatcgc atatncagct tncaacatta aaagaatgca taaatgcctg 240
ntctttcatt gngtctttac actcggncg                                   269
```

<210> 1072
<211> 498
<212> DNA
<213> Ctenocephalides felis

<400> 1072

aagatttaaa caatatgtaa aatatttatg tcttttaatt taaaatgaat ttatangttt 60
tttactataa ctagttaatg aattatataa atattttaaa tattgttaag cactggttaa 120
ttttcgtata actgctatgc aaaatactgt tagatttgaa ttaattttac tgaatcaaat 180
gaaaaaaagc aaagaaaacc agatacttca agtaaactac acgtaaataa agaatgtttg 240
cataatatga tattattata ctcacaatgt tcttttgtgc gaagtaattg acaagttttt 300
tgaatactaa acaggtatgt aaaataatat gtatataata attatatgca catttcttaa 360
ataactgatt agttgncaaa atcacacgaa atggtagctn cccaatattt taaaagctac 420
ctaaatgnct aaatccctct ggtcggaagc taagtagcat caaaccatgt ttncttattt 480
acggtaaaat taacatta 498

<210> 1073
<211> 498
<212> DNA
<213> Ctenocephalides felis

<400> 1073

aatgnttnca gatccagccn ccnaagaagc ggnttgnttg cttaaaccna tgngccaaat 60
ttgagatacn agctttgagc gantcagagt tttcttggaa aatgttaatg gtaaaatgaa 120
aatatgaaaa atttcgcaat tgngacaaag attttcctaa catagaaata ttttgctgnt 180
ttatgatagt aaattatttc ataattagna atttaattat attttaatat taattggtta 240
ataacatgcn aattaatcga gaattgnttt aatgnttgat attttaacat taattgnggn 300

tgggcagata tttattaatt atatgttaag ttcgcgtntt tgctttttat ttaattaaaa 360
ttaagattat tatattcctn atcatgggtt aaaaaaacca tgntntcatt naagggcnaa 420
gtaccccaag ttttngntta acaagggttt ttaaggtnta ctgcagctan gcctcngtat 480
tacatattcn acacttct 498

<210> 1074
<211> 437
<212> DNA
<213> Ctenocephalides felis

<400> 1074

caagaaacaa gcataagatg gccatggcaa tgctatttta tgaggcagaa ttgccatgtg 60
aagatttttt tccccgaaca ttatacaaga ttttccactt agattttttt tattacgata 120
gttattaagt agtcggcatt tctgttgtag tagtagtaag tagtagaaaa atatgaaaaa 180
aatatttgca atttattatc aaaaactcta atataagtcc cagatttttt tatctaattt 240
tgtttattga tcaataatta ttattattat tattgttgtt gttgttgacg atgaaatatg 300
atttatatac aacaaattta tatttaaaat agatctttat aacttactga ctgnagatta 360
ttggtaattg gnctggtgac caattcntgg tncccntggt tccattaaat gaaaaaaaaa 420
aaaaaaaaaa aaaaaaa 437

<210> 1075
<211> 324
<212> DNA
<213> Ctenocephalides felis

<400> 1075

```
ttttaaccct ttntaaccnt tngaantccc cnngntttcg gcnaaggccc acaccttgtg 60
tangccngct taaacttana ncctttcatt ttaaatagac aagaagtgcc attgggggga 120
nctattgatt ttgaanaacc accattcnaa anctcttatg gaggaggaga tagttattca 180
tattctgcac caccccncccc ttcttntaaa ntgaganaca agtgctcctg cacatccagc 240
tgattatgaa ccaacggagt atggngaata tgcttatcgt cgtggtatgg attatgaaga 300
tacaggaagt tccgctggaa gtng                                        324
```

<210> 1076
<211> 497
<212> DNA
<213> Ctenocephalides felis

<400> 1076

```
gaaatatgat aatatgttca aaataaaaat gataaaaata aatgagtgga ttatcaannc 60
aggatattaa acataataga aatttttttt gaaagttatg gaaatttttt aagaaaaggt 120
taatgacaaa gttgaattaa ttacctgtat gtttgaatat ttgttagcac ttttgcacta 180
aacaagcttt tttaacaaaa atgattttgt gagttaattt tccgccgaat aattagattt 240
tgtaaataca aaattactaa aaaatttgtc ttttttaaaa ttattatttg aaaacacgaa 300
taatattgtt ctctggatgt tgttcgattg gttttcgaaa attaactaaa tgaaactggt 360

gtgccctttg tacacgtgta acacttgacg agagatggga gatggntggg gngngttttt 420
atatgtcagg ggggaatact taaattgtgg atcttaaaag gaagtcgtat taattgtttg 480
tcaaaaagtg aaaaaat                                                497
```

<210> 1077
<211> 354
<212> DNA
<213> Ctenocephalides felis

<400> 1077

```
ttttaaaccc tttttnaacc cttttaagtt ccgcgnaggt ttccgcccga ggnccccttna 60
attggtaaat atttataaac tacntttttta attttgttca aactaatttg gatanngggnt 120
tacgnttaaa gctatatcaa nttggataat aaattattgt aagaatttac tactcattta 180
cacgataata tataaagcac accataatat aatattattt tatctataat acaccatggc 240
tgttaaaaac accaaattta tattctcaaa tactaaccac atacaatcct gatttaaatt 300
tgtataatat acgtttcaac tcaactaagt ctatcacaag aaattggacg tagt       354
```

<210> 1078
<211> 387
<212> DNA
<213> Ctenocephalides felis

<400> 1078

ttgntaaacc cntntnaacc ctcntggaan tccccaannn tttccccngc agccccnaat 60
aatnntactt cnagttaacc tancacgatt attnttataa tttttttgnt aattnnangg 120
tctgggnctn gatgtcacct cactgnncat atgaaacaaa natgtgacgc tctttataaa 180
tttatcacta ccttatctat aatcatatca cctcacataa atttcatacc atggttgtat 240
ttacaaaatt cagacagtag ttgcagagat gtattcgaaa tttgaatcat taaacaaaaa 300
tattttgata aaattgatat gtctctcctt aaaaaaaatt gaatttctca tttataatta 360
tttcttgntc tgggcatatg taatagt 387

<210> 1079
<211> 467
<212> DNA
<213> Ctenocephalides felis

<400> 1079

cgncaccaat gggttttccc naaatttttn ttcggnccgg accnggnggc aaaagtgnaa 60
ccgtttttttc cgnccgagga gaatggnaaa aattttnntc atggtttttt gggtnaccac 120
caatnggcaa taaaaccttt tggttggccc aatnggctng ccctttgtta aaccggggtg 180
cgngcatccc cccccccccg cgaagnatgc aaagtggaga atggttaagt agggcttaac 240
ccaggaaacc atcttgatag actaatnngg ttcaattatc ataagtagtc attatttata 300
atccacaatg actggcatta atataaaaat tgttaaaatt aaatgttaaa tgttttatgt 360
angggccaac aaangcatac ntgtattctg gtattaaatt tantttgagc ttttgatctg 420
ttttttataa ataacgttgc cgcttgaaaa aaaaaaaaaa aaaaaaa 467

<210> 1080
<211> 489
<212> DNA
<213> Ctenocephalides felis

<400> 1080

annccnccnt caccgaangg gtttgcccca aaattttttt tggngcngaa ccngctngca 60
aagggggnaa ccgtttttttc cgnncnagga gntggtaaaa atttttttcca tggtttttng 120
gttaccanca aangncaant aaaccnttat ggntngtcca aanggcttgg cnctttgnta 180
aaccggagtt gcgatgcatn cccncncccc ccggcggaaa aatgcaaaag tngagatgct 240
aagtagggct taacccagga aaccatcttg atagctaatt nggatccaaa tatcataagt 300
gtccatttaa tataataatn cacaatgact gngcaattaa tataaaaaat tgtttaaaaa 360
ttaaaatgat aaaatgtntt ttatgtangn gcncaaccaa aatggcatta ctttagtaat 420
ttccggtttt taaaattttt aattttgaag cctttggaat tncntgttct tttataaaaa 480
taaatcgtg 489

<210> 1081
<211> 386
<212> DNA
<213> Ctenocephalides felis

<400> 1081

nacctcnttg gatttgcccc aannggtttt gggccaattn anccttttcnt accnttnnca 60
aattggnncg gtanttnccg acggtttttt taaaccaata attaaggggc caccgggntn 120
ncanttntna antnttccag ctcntancnc naaataccat attttnttca atcatntatn 180
tntntaattt aggtttgggt ttnangaatt ataccaccaa tngtttctat cggcncattt 240
aatnaaatat cctttcatta tgncgancaa ggacaagttt tgnattaaaa aaantttntg 300
ttacttaatg gacaatattt agaaatcaca ctttgaagac tccttcctcg ganccttccg 360
gggggcgccg agnaaaacca acaatg 386

<210> 1082
<211> 436
<212> DNA
<213> Ctenocephalides felis

<400> 1082

gaattngnca ggcagcantt ggcttgcggg aaacccgtcc tcnctgganc ttcaaccagg 60
nanggaaaan ganggttcct catgggtccc atttttaaca aaaatgtcag ctggccattg 120
ggaaaggttt ccgattggtt naaaaaacca gcaaagccat tagtcaaata aaaagaagtt 180
tgaaaatatc agatttttct gttccgagaa tttaatactg tttactgaac ctactattgt 240
gaacaatgaa gagtttttta cctgcaaaga ctatatatct ggatagaatt ttaccacctg 300
tcgaatgtca gaagtcttta tatgcattta ccctgacgag ttcagaaaat aaatctagaa 360
ttttccatat aaaatgctca agtggcgatg aaagatcgtc ctggatgcta ttattaggaa 420
tatttgttca gcacaa 436

<210> 1083
<211> 497
<212> DNA
<213> Ctenocephalides felis

<400> 1083

atntttngnt nccatngctc aanggcgnta ttggccccnc agccgggcgt ccaaaangct 60
ncntngtccg caccgnctta ntcgtttggc angattatcc ntgtttaatt ttcgcctttc 120
gtccccaaaa tcttttggga tcttgatcat gtaaaagtgn tttaacggct ttgcttcgta 180
tgcatgcagg tgcattatct acgagaagaa aaaaattggt tgctggggga gagttacaag 240
atcttatgtc gagactatgg gccatataat caatgggtgg gggctggaga ggatctgntt 300
taagcattgc tgaggatctg agaaatcgta aatatncgat ccattcatgg acaggagaag 360
taatgattnc tgagatgcat ggctatggta aaccatccgc attataaaaa gnggttgccn 420
ccgcgtncnc ccatcncatt gntgtcaatt attnactggc tnctgacaat gctntggcgt 480
tccaagatnn cctcatc 497

<210> 1084
<211> 281
<212> DNA
<213> Ctenocephalides felis

<400> 1084

aagtgtaact gtaattgatc cnttttattt taaattacag ntanaaattc cgnnaaaatc 60
ttnacccgat gatatcacaa aaacgactta taaaaaatta ttaaagcaaa tattgntttt 120
actcttagac cgcggacaat gtgccatatt gttgtaatgg atttttgata gaaatgtagc 180
tttaaaaaaa aacatcttga ctcggnattt attattatta aatcatcaat ttgtgtgctt 240
tacaactaag ctatatttct gatccaaaat gcaatactcg t 281

<210> 1085
<211> 489
<212> DNA
<213> Ctenocephalides felis

<400> 1085

tttttttttt tttttttttt cgntttccgc atntccacaa gccaaaacca agcctgngcc 60
agcattaaaa acccaatcct tntcgngttn tttcagngct atttcanata ttaactntgt 120
ttntaaccgn ntnaatacct tttcaggatc gcgtatggcc tcatttttgn gtgtnaacct 180
gnggncaaca tcgccaatcc atgttaccaa tcgatcctta cgattagcaa acatttcacc 240
gagtcccaaa cgttnttnta acctatggna ctgnatagct aattganggc aanancgcca 300
tgctgttgcn cagaatccaa accattngat ttattggttt catatctgnt ggactaacgc 360
attcttttag ttggtcttga gaaactctaa atcttcaaag ttgggngnga gaacaactaa 420
acgagngccc tgaatcgnaa attntccaaa nattncncnt cccggtttta naagangga 480
actggngaa 489

<210> 1086
<211> 389
<212> DNA
<213> Ctenocephalides felis

<400> 1086

ccnggccagg tccattccag gangtgggaa cctttttaag gaagnctcct nangggggc 60
atttnccccn ngggggaccg ggaaaaatcg cccattagct tgggtcggnc cctggccnat 120
atgtaatngn ccaaggggt atccccctctt tgcaatgggt gggaggttgg ctgggaaaaa 180
ggtactttac tcctatcttc tcaagactgg caaagggatt taaacttctt tagttggaat 240
ttgggggata ttagattaga aatataatgg aaatgngaat atagaaaaaa aattaaatat 300
acaatagtat tttaaattgg ccacatctat ctatgcatnc ataaataaag gtatatacag 360
cattcaaaaa aaaaaaaaaa aaaaaaaaa 389

<210> 1087
<211> 499
<212> DNA
<213> Ctenocephalides felis

<400> 1087

```
tttttttttt ttttttttgg taaaaaattt ttcttttatt aaantgnttt accacccana 60
taatacctct ggcntaaaat tggaaattat tctaacaaaa gtttggggnt atggcatgaa 120
tgccacttaa cactaccagc aattttgtct cagcataaaa tttcataaat agaggagctc 180
aaaaagacaa aataaccata atatagtcat taacattaac aaaaatataa tattatctat 240
taaaaagnaa agcaaatata atctttgaaa aatattctta cacacactca catatatatg 300
cctttgcata gactccgaat ggagcgagat caaaataata acatgggcat catcncactc 360
aaagacaaat agtataataa taatatgcgg gtaaatatca ttcattcatt tttttttttt 420
attgccggtt atccgnggcc caataataaa antnaatttt catattaaat ttaaagncaa 480
tttggttacc gnngaagga                                              499
```

<210> 1088
<211> 303
<212> DNA
<213> Ctenocephalides felis

<400> 1088

```
tttttgggca aaaagactaa caattaataa ccaacacata ataaaaataa attgntggga 60
tagaaaattg attcaatttt ctactcgatt aacaaaattg ncctttatct gattatgngn 120
tgggntttat tatatattat tattaatatt gatcgatgga tttaattaca ctgattttgc 180
atttaagtga gtttatccta ttattggttg gttttggtat ttaattaatt gaccagcgtg 240
agaagaactt tcttgnagtc tcctgatgta tcgncggaga tcagctctcc aaactctttc 300
cgt                                                               303
```

<210> 1089

<211> 498
<212> DNA
<213> Ctenocephalides felis

<400> 1089

```
acctaatatt ataatattaa aaacaaatca ataaaataaa tagcaaagta aattttagtg 60
ttaactttat aaataccaat catcattttt atatgatttg tataaaaact tatattccat 120
aaaataattt atatatcatg aacatagaaa gtgtttatgt atcatttcat tcttatatca 180
atctaaaaat tataatatta tgaaatataa attttagttg taaaattgaa atatgtcgag 240
gttataaatc ataaaaaatt ataatataac tatacctcct aaaatagcat tgccaatagt 300
ttgtttatat aaacttctgg cgatcgaact tcttttcgtt catttcccga gattaaacat 360
gaaaagccca cggttttcga atgcattgaa taaaatgatt tgcttaatcg cagcctaata 420
taatattcat caggctatta tgaaaatatt ggatacaatt tttatataaa accattctnt 480
agaaccatta gaaaatat                                               498
```

<210> 1090
<211> 499
<212> DNA
<213> Ctenocephalides felis

<400> 1090

cacccccgag ctttcnaatt ggtaatggtc gggactggac gtngcatcga tctttcgntt 60
tatcttggag actgggactt ttagctgcca attcccgcag cagnggttgc tttgtgcagg 120
nggtcatcat tttcgttgca ttttacacat ttcaggggat catttttatt acataggtta 180
ctattgcact ttagacaaga cccagatgtt gcattttcgc attcttttct aatcgaatca 240
gcttcaggta aatcgaccaa acaacctttt tttgtttcag tgccgntaac atagatgtaa 300
cacaatcctt cacatttttt ttctcccccg catccgttga catgacgggc gctcgaaatc 360
ttattattgc atgtagcacc gcacaagttt tgcaaagagt attgncatcg cagatatcct 420
cgtgcgcacc ctcgattact ttaccttttc tagttagcgn agcataatct ccattcatat 480
gctaacaatc ttgcttcga 499

<210> 1091
<211> 500
<212> DNA
<213> Ctenocephalides felis

<400> 1091

cctggctaan ntttaagtgc ttgggattct ataaaanttg gnttttaaaa aagctggatt 60
tataatacaa agcagggctt cnttttcgat ttgtcacata tatattaact tttttcaaat 120
caatttatct atgcatttag tttatccatt ttctaactta caaaatataa ttctaaatag 180
ttcaataagt aacagaatct aataataaca taaaataata atatataacc tctggacagt 240
aaacatggta gagattttaa aatgacaaaa cacattcttg aaaatgaagt tcaaaacaat 300
atctagtttt gatcaagagt ttctttggtt atgactaaag ttttcaaaca tcacgtaaga 360
atggnaataa ttgacttgaa taatgcttcc tatcaatcaa tttttatgta aatcataaaa 420
ggtaaaatta aacaggantg ctgntataaa attactgnca cttccccaaa atattatttc 480
atctctaatt accatagata 500

<210> 1092
<211> 308
<212> DNA
<213> Ctenocephalides felis

<400> 1092

ccccttcagt aatttctttt tatttttaaa ttancgaaaa atcaganaac agttattcca 60
aagatcatgg gncttgntgn ncttctttaa gcttatttcc tatcctaaag tgttaactat 120
tactacctaa actgcgatga tatttgtgat gaatgatgtt gacatgatgt ccgaagacgt 180
tggctaagat gcggnataat tctgttcctg gtcatcatcg gttccctttc ctggtaatct 240
ttacttgttc ccaatcttca gcacggccgt tctaacgtgg agcaagcttc gagccagttt 300
cttcaaag 308

<210> 1093
<211> 498
<212> DNA
<213> Ctenocephalides felis

<400> 1093

tatcagtatc acccttacgt gaatactccg aaaaaatcat tgaagcacga agaacagaag 60
caccttactt actaccaccg cttttaagcc actacgatag ttcccaaaca agacctttat 120
ccagcttgca gcatctcatg atcgatcaaa acactttaat cgaaggcttg aaattggttg 180
gtgtcgatgg atcttcatta aaacaactag ctcactttgc acattgcccg ataactatga 240
acatgggtca cagacatagt tgggtcgagg ctggcacttc tttaggaagt gcttcggntt 300
tgagctcttt aaacaacgat gtggatagcg cacaagntca ccaggattat ccaggaaaat 360
taacacccga agaatattct ttttgtgtca tggaacgagc agtaaatgga ccaactgngt 420
ctggacaatt actgnagang gaccatntaa cgctttgcca agcgtttana aatgancatt 480
tntgaatgtg nnccnaac 498

<210> 1094
<211> 228
<212> DNA
<213> Ctenocephalides felis

<400> 1094

cgttttnaca gcaaaaaacc aagccttgat tggccgaagt ggtcgggtgg cttctcatgt 60
ggattcatat aaatcaagct tttgtggttg ctttgtgatg aatgggtgct ttggttgaaa 120
ttggntcttc atggttcatt taagattcaa cgatcctggt gaattgggnt gaaccaactc 180
ggctccaaaa tcaggcgacg aagaagagct ttcctgaatc ctctccgt 228

<210> 1095
<211> 308
<212> DNA
<213> Ctenocephalides felis

<400> 1095

cctaatggng gttttangct aaccangaaa aataagggtt aaaaaaggnc naaccttnaa 60
atgggataaa anccattaaa atcantttct ttancacatt ctagtcacac actttgatcc 120
gaaaggttgc cccgaatcgg cagaatttat gaattttatt ataaattcac aatgatcgca 180
tcacataaaa ttgnctttaa atctgnttat cttgcaggaa aaatatcgca aacacgtttt 240
tttntggctt ctaactcaat atttaacaca cgtaaattat cacaccgtta ggcaagctga 300
tggnctgg 308

<210> 1096
<211> 335
<212> DNA
<213> Ctenocephalides felis

<400> 1096

ctttctttaa aattgtaaca tctatatcat gaaatcatga tcggctccct ggtttatttc 60
taccgattac tacagtatta tatttataat atgttcaata tagcactgng gtccaatttt 120
attttgacca aaggtttata taaaaatcca ggttttcttg atcatgtaaa atgntggtcc 180
attatcttaa attattatat tcagaatgat atataatcca ttcaactttc taaaacaaac 240
aagaaatctg tgcaacgtct gatatctgng aatatgtatt ggatttaata tatatgttaa 300
gngnttaaaa atatatttct atttcatata cttgn 335

<210> 1097
<211> 500
<212> DNA
<213> Ctenocephalides felis

<400> 1097

```
ccttagacct ccaaatcttt ttttaaggaa aaatcccccg gaaaaatagc ccttncatat 60
tccgcctgnt tatacttgtg acaagnttaa aggatttaat ttaagcgact tctgtattta 120
tgtaattcac ctactctaaa ttaagactgn aatttatcat tatctgacat agtttttgg 180
ttttacgtat tttttattaa aattcttgtg aagttcaaca agtagaatat tgnttttta 240
attgggttat ttggacctta gaatattcta aaatactcta tttacataga gagagcaaga 300
tgcttaaatt tatacaatat gtcgagtaaa accaaaaaaa acgtaaactt gcatgctttc 360
tagacatgct gctataatca taaaaaacag ttttgccatc ttgaaataag gcagacttat 420
attatataca ttaataatgg ntcgacacnt attgtgntca ttggaatgaa ataatcaggg 480
nggaatataa ttttcttntg 500
```

<210> 1098
<211> 392
<212> DNA
<213> Ctenocephalides felis

<400> 1098

```
cacnntnttt ntntaagttt tttccctgta aatngtaaat atgantttgg ttngngaaaa 60
tagtggcngg ggtncatgtt aaccaaaact atgggacctc ttggataatt taatcactta 120
tttatatttg cccgcgtggt aatatttaag atatgaaatc tatagaagcg atcctttgta 180
tgacaaagtt gacattgtta atcatatatc caatgatttg cgacatatgt tatggtgcta 240
tttgccctta aaaatctaca tgtgtagtga atttgttctg cttctacgtg ataaatgctt 300
ggatatagng tttgatcttt ttataaatat catatatatt ggattaaaca atcaaaagaa 360
ataaaaatgt aaaaaaaaaa aaaaaaaaaa aa 392
```

<210> 1099
<211> 362
<212> DNA
<213> Ctenocephalides felis

<400> 1099

```
tttttttttt tttttttttt ttttttcant tttctatctt ttattattta agccataaat 60
atatatatag tataagnata ggctaactac cttttattat tcccgaaacc atttcaataa 120
tttcaactat tcttagattt tctttnattt ctcagacaaa accacatatg cttgttaatc 180
tcaaaacgaa taattatcat ttttgtttat gactatataa ttatacatat gcgggcctac 240
ttagtattca naaatacttt atacaggnag cttatcattt tagataatat cattcaattt 300
tgttatttgn attacttcaa taaaaataac acctaatata ttttttcaat tgatgaaata 360
tg 362
```

<210> 1100
<211> 498
<212> DNA
<213> Ctenocephalides felis

<400> 1100

```
caangaatat angggttttt antggcacca taatggcttt taaacttggg gttggttgga 60
aaaacaaatt tgggttggag gggataaccg ccanttttag gattcaaaat acctccgcca 120
atcacacaaa aacactttta aaattccttc taccagaaaa aaaaatggca aaaagaaaaa 180
cacgttttta accagttaca ggagtgntac tcctcaatcc aatgaaattt tatgcagttc 240
tgttcaaaat tatatctgnt gnaatattta aaaaagttaa acttttgctt aaccaatgaa 300
aataagatct caatagaaga cttaaattaa atatgatagt gatcatgagc atatgttcat 360
aaatcaactt tctgattgat tttctatgat aaatgcattg nctacactat ttacttacta 420
ctacacggtc cttacaggaa tgnatataaa ttaaaatatg catatattta tattcattgn 480
gggaaaccgg atttggaa                                               498
```

<210> 1101
<211> 319
<212> DNA
<213> Ctenocephalides felis

<400> 1101

```
ctacntnaaa gggggtggct tttcttaagc agntggcata ncccaaggca gcacccactg 60
caaanggctc ttataaaaaa naggtatttc tttggcaatt taaattcttt ctttcanctg 120
taagacccgt gatacccatt ttttttggct tttcttttga tttttcttta agcaattccc 180
tctgctttgt caattttcct atcaacaaat ctctcaattt tatccattaa tttaggagtt 240
tctccagtaa caagcctctt caattttatc tgtcacctta tcagcctttt tagttacttt 300
atcccagtca atagaaatg                                              319
```

<210> 1102
<211> 283
<212> DNA
<213> Ctenocephalides felis

<400> 1102

```
ttgggcnttt ggtnggtatt ttaanaacaa gaagcatttt nttgggactt ggacttttca 60
ctactcataa gaaagcatca aaagaaaact gggnctgggt attttggtgg agggttggga 120
ctaaatatca tcgatcnggg gttttactta aaacttttcc ctcatatcct taaaaatctc 180
ttgatgggct tccaaaactt gtccgagaat ttcacaaaag taacctccgt tccaaaagaa 240
ctttttgtca aaacacgtgg ggttaaaagt gcaggtcaca agt                   283
```

<210> 1103
<211> 287
<212> DNA
<213> Ctenocephalides felis

<400> 1103

```
tatcaagcaa actgaatttg atctgaggta aaactgaatt caaganataa aagcatcacc 60
nantatgtat agataaaaga taaagattgt aaaaagtttg agaaacactg tttggatggt 120
gaaacgaaat atagaaaaca caaaagttaa aatatatata tactcagccc caaataatat 180
aatttaaaat tatggttaag tttgtttagt agctcagtgg taatagcatt agccttgcaa 240
ccagaattgc agattccatt ccctcattaa acctgatatc aagcagt             287
```

<210> 1104
<211> 434
<212> DNA
<213> Ctenocephalides felis

<400> 1104

```
accatatcnt atttttggan taantggaat gncatttaat ttggctacta atacttctat 60
ggaaattcat tttttaaata aggtggntta aataaataat ttcattgnaa agncacatca 120
aaacttttct ccaaatatag ntctataata tttagttttt agcctgntat cactattaga 180
ttaaattata aattgnttgn aattggctaa ttgnaaagng nttataaatg aacttttgnt 240
gncatttgnt atgaagatat atgtattttg aagcatattt tttgtaacat tgtgcaaatg 300
cttatttgtt tggttgnaat aggaaaataa ttatgattct tattactcat taaactataa 360
taaattttag cccatggtaa acattacaat taagtatcta tttagcaaaa aaaaaaaaaa 420
aaaaaaaaaa aaan                                                   434
```

<210> 1105
<211> 498
<212> DNA
<213> Ctenocephalides felis

<400> 1105

```
ntttgagccc ctcggngtct tgaaagcccn tnaangggggc cccgggcggg ggaccaagcc 60
caaaggnnta tttggttctt tcnacnggta atcaaaaatt ggccttttat attttaaaan 120
tggataantt aagntttant anttggcccc cgganaatcg ggccataaaa aaaaaaaaat 180
ggatggatgg tttttaacnc ccnaattatt attataccat ttggtctttg nggggggang 240
aatgcnccct gntattattt tggaccccgc ccccttcggg ggctatgcca agggntatat 300
tgggaggtgg ggggnaagaa tatttttcaa anantatatt ttgctttacc ttttaataga 360
aaatattata tttttggtaa tggtaaatgg ctatattaat gggtattttg gtctttttga 420
gcccnctatt tatgaaattt atgctgggac caaattgctg gnagnggtaa gtggngcatt 480
catgccntaa ctnccaac                                               498
```

<210> 1106
<211> 500
<212> DNA
<213> Ctenocephalides felis

<400> 1106

```
ggnnnttttg aagcccttnn nnnaaangaa gctcttngnn agctcnaatn ncgttnggcc 60
gaccggcccg gtncaacagt ggcatggacc ttggccttta acggtcantt ctaaggatgg 120
gggttnttta ntcttggggt gggatatggc tttangggcc ttaaattctt nctnanganc 180
ccaaattact aaatcannat ncaagccttn tttggnttgg ccttttangn catccaagcc 240
agntaatttc gcanggcccg cagataataa acgagatcat catgagcttt aaagnccttt 300
ttaaaancac cgggccataa taaanatatn caaactggat accaacaatt ncaccccaag 360
cttttaagga atcaaggngg acaccattgn acaggccttn antgcacaag tggaggngaa 420
tgacactgct tncatatcat atctttgttt tcaaagcttg cataattttc tggatacctt 480
cttctctaat gggggacgca                                             500
```

<210> 1107
<211> 370
<212> DNA
<213> Ctenocephalides felis

<400> 1107

```
cctaccttta ccttctcact tacccttacc tacgctttac cnacccttnc cnacccttnn 60
accccgggcc tttttactgg ttactnacta ttggctaggg gntttggngc tnaatcnccn 120
ncgaagagct nctnnaagaa agaacaggca aattttggnt nacaacctnc attctttgnt 180
accnatcatt antatggcna ctactctatg gncaccnatc gnttaacaat caggcaatct 240
gntcttggtt ttgggggncg gggattgaaa cccgaatcgg gnttcgaaaa tgaacannaa 300
tatttggccn tattaaatgg tggtaaaata tnccaattaa tggattattt catcactttc 360
gnaannnaaa                                                        370
```

<210> 1108
<211> 149
<212> DNA
<213> Ctenocephalides felis

<400> 1108

```
tttttggtgn tatatcactt tcctttgcag gtgcctntga accgggcgtc ttatttaaat 60
catcgctttc ttgattatta tcctcagcgc tttcctgatt ggtatcgtct ttatgcgtca 120
aagttgccaa gatcgttgca ttatttggt                                   149
```

<210> 1109
<211> 498
<212> DNA
<213> Ctenocephalides felis

<400> 1109

atattagtaa taagtggcca tcaaagatat taaaattaat ttgccagtcc tgtttattgg 60
gggangaact tcaaaatcat ctattacaag gattatttca agtgccattg gtagataaaa 120
cataaagtag atatacctta atgccaggag tatatatata actaatgaaa aatgaactaa 180
atttgaactg gatttcaaat tttgactgga tctgtaactt tttgtagttc ccttctatta 240
aataggatat ttgtattcac cgcattttat gacctgaagg aggtcaaata ttccactgtt 300
tcttttgcat aaattttatt aagtgatagt aagaatttta tacttagggc taatcaaaag 360
agccggcaca cttatttttt ctaatttcat aattaaaaat tggctatatt tgtcctaagt 420
tgnaaaggtc ttagttattg naaaattata ttatagcgat agattcattt tcattgtggg 480
atgacttatt gattgaca 498

<210> 1110
<211> 400
<212> DNA
<213> Ctenocephalides felis

<400> 1110

tttattctat aatgaaatga ataatgntac tccaaatata atagtatgtc tttcatggna 60
aatttatatg tatatatatt atatttatat atttgnatat atgcacaatt gtgtatgctt 120
atatctttac atatgtgcaa atatagtttt atggatgtat atagatgtgt aagaatattt 180
tatgtaattt ttattaactc gtttaaaatt gagtttatta cttctaaaac cacttgcaat 240
ttgattgact ttttggatac tgntctaatt aaagtatgat gcattttaaa tatgaaaata 300
gaaatagtta acagtaaact aaacaagctt tgttgctagt gtaaaatgga aaaatcattt 360
atgtttttat agcttttaaa catgtttcaa tttgataagg 400

<210> 1111
<211> 379
<212> DNA
<213> Ctenocephalides felis

<400> 1111

caaatattgg tgcatcacag gctcatggga tatttataaa acatagaatg nggggaatat 60
atcaatcata taaaatttac atgcttcata ctagacaaac aattaaagat caaacattat 120
tttttataca tttatatata aaattgaatg atcaaattct ttctttgcaa aaaccacata 180
ttattgaagc ataaatttag aaatcaaata aataaaatag cacaccaaac attatgttat 240
tagtttatgt gtcaaatcac atattgttat aaattttgag taatatacaa ttataggcgt 300
gtttttaca tttttcattt cagaggtctt taaaacttat acaatatatt atttactatt 360
attgttaaaa cggtttagt 379

<210> 1112
<211> 486
<212> DNA
<213> Ctenocephalides felis

<400> 1112

```
tttttaagnc cccttnttga aagccctttg gggnttancc gtgggtccnn gggcgggggt 60
ccccccaagg tggnattggg ggaaancnta cccnccaaat tttttttaac caatttantt 120
aataccttan tgggcctaag aaaaggccga aggtggtaaa ggttcaaggg gttggtatta 180
gcttaantaa aaggtaaaac tttttaaaaa nggtgatgga atttatctgg caaaataggt 240
ccaaaattca aaaaattact attattaatg gnntattgga aataatgctt tttattagga 300
attggcattt tcaantctgg tatttaaaat tggtntggta aaatcaccag caccaaggat 360
cccaaccaca cantttcatc tnaccagaaa gaacnaaaat taattttaat ggttattaaa 420
aattacatta ttaaaataac tttttacnaa taaaggtaac cctacnttga gaatggtgtt 480
ggtttc                                                            486
```

<210> 1113
<211> 500
<212> DNA
<213> Ctenocephalides felis

<400> 1113

```
ccgacnggtg gaaattggca ntgganccag ccnaaantgg nngggcccgg gcaaaggtan 60
taaaagaant ggggattggg cantttattg acgggggcaa naacttttgg cgaatttttt 120
ttaacacata tttttagaaa tggacatatc atagttaggg tggggattcg aaaaactgnc 180
tttancatct tttgaaaata aggtatnttg ggcagncttt cggatctggc tgatatccta 240
caacccgcgc catgtagttt ctgccgctta caagttttcc caaggttttg atgagccata 300
tcatctacac aaaatgggac atcggaattg aagctctgca atngattggt ggaattgggg 360
gttctttgtc acgccacttc aaagtanaca atcgtgacaa atgttccant ggtattatac 420
ctnggccgga cccgctaagc caaatttgag attcatnaan tggnggccgt ngagctgctt 480
taaggccaat cgcctatagn                                             500
```

<210> 1114
<211> 500
<212> DNA
<213> Ctenocephalides felis

<400> 1114

```
gctagctaca agttgttgaa atattattaa attttattat tatatttgtt tttttctgn 60
atggggttta aattacattc catgataatt ttcagtttta tataatctaa taaattcaat 120
ttaactgttg aaatttgatc atattgtgta tatacatagc aatataaaat ataaagttac 180
acattgtctg tttcattata actgtactgc aaaattaatt attttgattg tttgttactg 240
tattattaaa gttattcaga ttcagtcaaa atttaaatgt ttggcctaaa cttattattg 300
aaatattgcc aatagtgtat tattacttag caattatgta taaagatttt atttattaaa 360
gcaatattcg aatgtctgta atatcataaa ttatgtatat aaataaatat atcaatgtga 420
acaataacag agaacataag ctcaaaatca tcatactatg tctggaaaat caaatataaa 480
tngtttttag ngtaaataaa                                             500
```

<210> 1115
<211> 500
<212> DNA
<213> Ctenocephalides felis

<400> 1115

```
ttttntngtt ttttttttt tttttttgg ggattagaaa attaatattt catttaaaaa 60
aataatgggg tattacagga ttattgtttc tataaacaaa ctacataata tggcatgatt 120
tttttcaata atcaatttga ttcgcaaatg aatcttctgg ctggatgcag attttgtcgt 180
tccatccttc cttgctggat ttgnatactg cttctacaca attttctttt cctctgtaat 240
tatttgggtc accacgaacc catttttcaa aagtcatggg ctttgaattt tcaatatgcc 300
cacaaatttt gatcgatctg ctagattgtt accagctgtc caaaacattt gtttggcacg 360
gtaattgctg ttaacaaatt tctcaaattt tcatattgna atggagntca atggntgcca 420
ccctgcgtcc aagctttttt accaaaagca ttttggtcaa ccagttcact tangaggncg 480
aaaatgaaaa ctttttccgt                                             500
```

<210> 1116
<211> 317
<212> DNA
<213> Ctenocephalides felis

<400> 1116

```
aggggaaaat tctttttttt gaaaactggg attctgnagt ttgcncgccg ggtggctttg 60
cgacgttttt agttccttga acgacataat cattattcat ttcttgagag tctatcaaac 120
tgattataat tgcttcgttt tcgttcaccg cagttttggc ttgttttatt tccggtttct 180
tttgtagttc aagttctaca tttgtctgac tcattatatt ttcggttaca ataagaggtt 240
cttgcggttt ttgtattaat tttgcattat ctaatctctt agtatcttca acaaactcac 300
tttctttttc acgaagt                                                317
```

<210> 1117
<211> 307
<212> DNA
<213> Ctenocephalides felis

<400> 1117

```
taccgtattc aggtcccaaa ccatcattaa aatggtagca ttaatcaaaa atcaaagaat 60
cattattcaa attccaagga agtaaaataa aaaataattt ctttattcac ttattgggag 120
tgagtataaa tctaattttg gattcccgat gattgttgca aagctccatt agtctttatt 180
aatacatttt tattttttta tttatttgca ttgtaattgt tgattcagac accgcacacg 240
accgccgaca tggcgctgta gtcttgctcc tctgccggcc gtaacgtcaa agtaatttgg 300
ttgttgt                                                           307
```

<210> 1118
<211> 374
<212> DNA
<213> Ctenocephalides felis

<400> 1118

```
ctgggggaaa ggnctccaac cnagngnccg gttttgcaaa aaaccactct tggctggttt 60
ggaattctca tctattaaat taagaantct tttgcttcat ttcaattatt ttcaaaatat 120
gattgatctc ttcttcagtt ggnggntccc ggccgtctat ttcgcaacca tgcttccatt 180
ctgcagggaa ttcctgatca aacttttctt ttattgntgg ctcaaaatat ctattcgctt 240
tgcgtttacc aatgctagga tttcctggag tttcataata cttattgcct aggtgatctt 300
ttccaataaa gtttgcttta atatttgctt ttccaatctt gaatgagtta taaaggtttt 360
aaagacttga aggn                                                   374
```

<210> 1119
<211> 498
<212> DNA
<213> Ctenocephalides felis

<400> 1119

```
tttatcaaaa atcctagttt atgagtagtg tgaagccaga aggccgtaag gtcgtgaaag 60
atctgaacgt ccatcaacat cttcaatgga tgaaatcgtc gcctaagtca agaaatggtg 120
ttggtaaact accatttagg ttcagtcaag ttagtttttg accttaacat atctaacaaa 180
tcaattatac catcaaattg ggcatgagac gctttgctgt tcatctctac catcattgtt 240
gaactctctt cacacaacgc ctatattgtg aacgcttttt tggccataaa ctcatgaaat 300
atcatgggac aaccaacgta ttcactagat ctatccttct gtaaattatt tattgtcatg 360
gatacttaag tatagaatta caagtaatac tattgttaga tacctctttg gatcgataga 420
ttccactaca atggttggta agaaaaaaac gccaaagggc tgaagaatat ccttataaag 480
gcgtttaaaa gatgcttt                                               498
```

<210> 1120
<211> 455
<212> DNA
<213> Ctenocephalides felis

<400> 1120

```
gcgtnacata tctggaattt ttttggtgaa ttatttttaa tgtaataaat catagtaata 60
tttatttcca angtgcaaat atggaaacta ccatccctat attatatata attgtatata 120
ttcctgaatt gttttatttg aatttatttt aaacacaaac tttaattata ttcaaataat 180
gatggatgtg atttcatata tccctaaact ttggatcaga gtttgagaga agcctagcca 240
tagtgtatag aaaattatgt gtaggtgtat aattaagatt gcaggagtca aatatataaa 300
gcattgatac aaattataca ttccaatttt ctgtaaaccg tgttctttgg ttgattatgg 360
tttatatcac aatttatgaa tcatgtaaat taattgtaaa tttgaaatgt atctncgatg 420
tnaatgtatt gaaaaaaaaa aaaaaaaaaa aaaaa                            455
```

<210> 1121
<211> 500
<212> DNA
<213> Ctenocephalides felis

<400> 1121

```
cttgaagccc tngggagnac nnaagccctt cggcgnccgn caggggcnna cctcccggca 60
ggggctcgtg gggtagttct tggcaaatta attattattc attaatatgg atttatatng 120
atatggaata aaaatcccag caatcacatt ctatcaaaaa taaacttaat tcaattcagt 180
ttggaaagtt ttaatcacaa agtctaaatt atcataaata gccattatca actgcatgcc 240
gaacattatc cactaatctt aaacgacctt ctttatgagc aattggacgt actttccata 300
ctgttgttat aggcacagca tcatcgctct ctatcatatc caagtctggg catcatcatt 360
tttcataaga gtgaatggca ctgattctaa gctgaatcct aaaccaaatt ttgaatatat 420
ttttaactca tcatgaaatg catttttttgg atacattttt tctgggctgc tcccaattct 480
ttaattgagt agcataagcn                                              500
```

<210> 1122
<211> 500
<212> DNA
<213> Ctenocephalides felis

<400> 1122

```
ttggattatt gnaagacaat tgcaattata tttcaacaac tggnttagtt accgaatata 60
atttatagga ttcataaaaa ctaattgngt tcaattatca ttcacaatat tcataaataa 120
gcaaataaat tattcaataa aatatataaa attgatgttt ctgtaacata tatactataa 180
gataataata ggattgntag aaaactttaa attaaagtga agtctatttc tttagttctt 240
taaggggctt gcatttagat caatttcaag actattctca ggactagaat tcaattggac 300
attattgatc tctgcacgat gttttccatc agagacagtc gttacgcttc tgtaaaatga 360
ggaccaagcg ggcatttgat tggtggtagt gncgcattct gaacttccaa attgtggcng 420
ngnatcgcta ctcacgaagn atttcggtaa attcctgggg cattatctaa atcttctatt 480
ggtttagcat ggtacagttn                                              500
```

<210> 1123
<211> 415
<212> DNA
<213> Ctenocephalides felis

<400> 1123

```
tttttcnttt cttcaanaaa natnttttaa anaatatggg ggttttgttt tatggggggc 60
cttacatatt aacttttgat agcaatataa agtantcnna nttatttcgn ggatcctacc 120
caattccttt attaaactgg natacttctt atactatccc attattttta ttaaatatac 180
acacaacttt aaaaactncc ttntggtttt atacaaactt taaatatcac tcctttttta 240
ccattaggat cgtaattaat ttntacctaa attatntgac ctggtaaatg tttacataca 300
tttacctttn nggcatataa attttttttag tttaaataaa tgctccagaa atatttttta 360
ttctnttnta catatcaact taattcctag tttttcaaaa tnaatattac acagt       415
```

<210> 1124
<211> 382
<212> DNA
<213> Ctenocephalides felis

<400> 1124

```
tttttttttt ttttggnttt ttgganggct gggnaaaacc nttatttatg ggngcataga 60
nagaaggtgg tgccaaaatt aaaaaaccat tgganattta anttttttc tatattcaca 120
ttttcattat atttctaaac taatatcccc aaatttcaac taagaaatta aatcctttgc 180
cagtctgaga agataggagt aaagttacat ttttccagcc aactccccat tgacaagagt 240
ggcatagcct ccttggccat ttacatattg gccagggcac gaacacagct aattgngcga 300
ttatttctcg atcccactgg nggaaatgac acctctctag tagctttccg aaaaagtttc 360
cactcttgga ttggcttgac gg                                          382
```

<210> 1125
<211> 500
<212> DNA
<213> Ctenocephalides felis

<400> 1125

```
ggcgggggct ctggaggacc caaaaccntt ttcctttttt aggggttcat ccttcttggg 60
ggctggtttt ggncggggga tttttccgcc ttggccggcg ggnttttgcg aattgggaat 120
cgttgggtga agnctggagt accgaaggga acagccccac cacctcgtct ggttgntggg 180
aaatggatct tgatcctgga tctttcgctg aaagaattgg ggtctattat ggncttattc 240
ggtancccat ctacctctac acccaaataa aacaaaatta tttatgggat ttcaaagaat 300
acaacaagtc gccctggctc ccaaatcccc ttaatatata tgcaagcagt gcagtctgtc 360
tattattttt gccactcaca cttaaaaata taatggctgg cccgtatttt atattatatt 420
ggttattcca gaaagcactg ttgtattgaa aaagatctgn aatcaacaca tctcatgtan 480
ggtactttnt ttgaaaaggt                                             500
```

<210> 1126
<211> 301
<212> DNA
<213> Ctenocephalides felis

<400> 1126

```
tgtgaaccgg caaacattgt cagtaaagct gcactcatga atttgaggta ttgaccccag 60
gatactccgg caggcattgt aatagttata gttttaaatt tggattgtcg agggcaagtt 120
tttcctcaac tatggttttg gcttggcgac gcttgtaaac tttatagaag ttcgtttccc 180
cgacttgcca ctttatcata gagaattcta gcgcagcacc caatacaaag aatattggta 240
aaaaactata gaatccaaat ttacgtcgtc ctggccactt actcaaaaaa gagcgtaatg 300
t                                                                 301
```

<210> 1127
<211> 500
<212> DNA
<213> Ctenocephalides felis

<400> 1127

cgagaaaaaa acttttcttg gccccgattt aacccctggc gcctcanacc cctggagaan 60
ctggtggntc aantctttta aagaaaaaaa attgncaaaa actgggttga ctggcgttga 120
aactggtaaa aacaacgacn aattaaatat aaaaaattgn gagaagaagc atatcgaaga 180
ggaaaatgaa agggacgatg ccccattgat atctgtgcaa aggacgtgct ggaagaaagt 240
tataagccta atggacctgg atcttctcaa ggatttgact tttcttaaca tagtggtcgg 300
agtcgcatta cctatccgcc agtatcaatt tcagcatgtt atttccattt tttttgcaag 360
aatcaactgg actaaaccga gctgatacag cgatgtgcat gtcagtctng ctggggcggg 420
cattggttct cggtaactct tccacaatta cacaaatctg ggatttcttg cagatggcta 480
cctatcggac tgccgtctaa 500

<210> 1128
<211> 500
<212> DNA
<213> Ctenocephalides felis

<400> 1128

ttctcaataa ttaanacttc atggttttat tttttaatat gatgaagact gcagacgact 60
atcacatttt aattcacaat agtgaaaaaa aaatacgact cacaattata tgtatcttta 120
acactagttg atgtaaaaaa aagatcacat aatttatatt tctggaaatg caacttgcat 180
aacataaggc cacaatttca tatgaaattg aaagtaaaaa atacttaaca gttgaaaaca 240
actaaataat gaacatgaac aataggaact atattcgaca acaaaaaata ttctcaacaa 300
ttatttcaaa tttctaaaca gcaaaattac taggcaaatt acaaaaattg gntccattac 360
atattggatt taagtaatta agactaccgc tgcatctgcc gatggtttct ttcttgcatg 420
ccataagttt tcttgaggca gcattagcat tgntttaagg ncttggtaca ctttgcctaa 480
gggggaaggt cttgggcctt 500

<210> 1129
<211> 500
<212> DNA
<213> Ctenocephalides felis

<400> 1129

aantaaancn cctttggaaa ataantgaaa actctttggc agccccngca aggtccaatt 60
ncggtgggtg cttaagggct tgggaatcaa aaattggncc caaaagantt tacttcttga 120
accccgaaat gggaaaaaga acaccaaaga acctttcacc tttggaaatg ggcaaaagaa 180
agggattatt tgggncccgg gtggtanggt ggtccaaccc cttgacatgg ggaaactggg 240
tgaaacgtga aaatggtcat gggatttggc ttgatccata tgcctaaaga accatttggg 300
cattttaaga tatcaaatgc cccacgcttt gttgttactt gggcaaaacc aaatcaaccc 360
aaagggaggg tatttcacgg nccgtgtctt cttgcnactt ggacgtggtg gttttcaccg 420
gctttggaga acttcatcaa ttgaaactta ctacatgagc atgantggga caacaccttg 480
gatnggggtg ggnaaacttt 500

<210> 1130
<211> 121
<212> DNA
<213> Ctenocephalides felis

<400> 1130

aacgcaatct aatgcctaag ttctcaagtg gcgcaaaacc acgtacattc ttttttgtaa 60
gancacatgt atgttatatt ataaataaaa gagggaaatc taaccnnnnn aaaatnnnaa 120
a 121

<210> 1131
<211> 500
<212> DNA
<213> Ctenocephalides felis

<400> 1131

aattcaccnc tntttaangg gcctaataat tatctccttt tttagagcct ggggataatt 60
tacctcttaa ttttggggcc cctttagctt taattggctc aaagctcctg ccatgtcaga 120
aaatcttcct ggttaaaaaa atatggaaac tggtaatttt atgtcgattt gnttggggtt 180
tggtatcata tattacacgt naaacagaca aactctgnga tattatgaag gggtattttt 240
ttataatatg cttagataaa tctttattac tcccattata aaagtttggt tgttcaacaa 300
aactttcaaa ccagcttgcc gcttttaggt ttaccnttta aaaaaaatat accccnttta 360
ttaatcactg gggtatttta actttaatgg attattaatc tncatataca tttttagact 420
ttgcttcact ttagtagggg tttatcccct tccatcnctn tnttgcgggg gntgaacttt 480
tatcatttnt tccttcgggg 500

<210> 1132
<211> 129
<212> DNA
<213> Ctenocephalides felis

<400> 1132

aatttggttt ttnggagcct ctttttanag cccatttcca gaacattttt tagtaacctg 60
gtgggaaaac tgatcgacac cacttntgaa tttggtaaaa aacatttgga aatccctctt 120
acttccggg 129

<210> 1133
<211> 398
<212> DNA
<213> Ctenocephalides felis

<400> 1133

taatcttaaa ccttaatatt ggtttattac cgaaaatttc tcaaatataa taaccattca 60
acttattaac cgtcacttat taaacttttg ntcatcaatt ggaatgtcta cagaacacca 120
gcgtgtttta taatgaatac aaaaacgcgg aacagacaaa aataaatcta aattgcatta 180
ttatgtttat aatgtatctt agaacatcaa agatataaaa cctttctaaa tattaaattg 240
aaaaaatatg aatagccatg atataagggt aaatagatgt ccaaaaaaaa cgtttcattt 300
aagaaagtca tatgagtaga ttagatttta aatttgtgca cagaatttat gactcatgat 360
ctttttaatc gctattcata aaataaacgg naaaatgt 398

<210> 1134
<211> 327
<212> DNA
<213> Ctenocephalides felis

<400> 1134

```
attgttctct tacaaatcta aaaattagaa ctaatctatg atataaataa aaaataggca 60
gcatacttat acacaatgta tctaatacaa tatgtattac ttcacgattg ttgtcatata 120
aacttcaata cccagaattt tttttgcagt tgttcaaaat tacaaattgt ctgcacaatc 180
catagatcgg agtgttttcg gaatggctct tttcccactt agattccttt acgtaaacac 240
aacacaaagc gtgtggagca aaagttttcg ataatactgc attatattga atatatctgn 300
taatgttcca aatcatgtag gttgcag                                     327
```

<210> 1135
<211> 357
<212> DNA
<213> Ctenocephalides felis

<400> 1135

```
cacacagtca gtgatagagt tacgcgttcg ttccgcactt aaccttacga atacttagat 60
tttctccaag aaaatctttt atctaagaag aaaccaaatt caagttgatg cattattctc 120
cggaattgtt agagaatctg aacaacggtt cgttgttctt cagctacgaa tacaaccggc 180
actgtaaagg gtctttgaag aaactggctc gaaagctgct ccacgttagg acggccgttc 240
tgaagattgg gaacaagtna agattaccag gaaaggggaa cgatgatgac cangaacaga 300
atttaccga tnttaccaac gnnttnngac atatgtnnac atattataca aatatat     357
```

<210> 1136
<211> 500
<212> DNA
<213> Ctenocephalides felis

<400> 1136

```
tttttttttt ttttttttgn tttggaatac ataatttggt ttacatttat tcttttataa 60
aattacataa aatatcttta ggaataacaa aaagggattt tcatatgcac atgtgcatat 120
aaataccatg catatatgta tatgcatgtt aacagaaaaa ttggnaatta gttaattagt 180
tcactgggac attatttttt ccccacgttt gaaagaggca cttcatttcc taaaaccgga 240
aagngatttt caaatgagtg ggcaacacat gatgctttaa atcggcacgc aaaagtcttg 300
atgacatgtg gcacacatct caatcttgca tttgattttt cactttggat aaaaatatag 360
tataattcct gntgcttgca gatatcgggc agtaagcaag ccactctggg aagatttcat 420
caaaatcttg cccaagcttt atgccatgac ataaatgatg cctgataaaa tccttcctaa 480
tattggtcct cagaagctgc                                             500
```

<210> 1137
<211> 378
<212> DNA
<213> Ctenocephalides felis

<400> 1137

ttgaaattta taaatatata ttttcataat ctatcatttt ataattctta aaattattga 60
gctacattat caaataattt ctctgcaggt aacttccaat gttcaaacac ataaggtaaa 120
cgatcttttt gttcagtgct ccaatgatgt attctgcatt taacatctac tttttcttta 180
ttattaattg ttacaacaac attgaactct ttattttgca agttcatatc cggacctttg 240
ataggctgtt cgggtatttt tattgcttct aatgggcact caattccacc tttacgcaga 300
gctatttgaa tgtgccatgg ctcgataacc caaggctctt ctttgttcat tactacggca 360
aatacatgtt tggagagt 378

<210> 1138
<211> 264
<212> DNA
<213> Ctenocephalides felis

<400> 1138

acgtggtaga aattctagaa gttgccaatt tcttctgtcg actagttcga atggtgtgtt 60
ggttacccat tcgtgatcat actggttcag aagataacaa ccgacagtca tgcctcccaa 120
aaagatcaat ccaatggctc cgatggagaa aaatcttcct ttacatttgc agctggtttc 180
agtatacaaa tattgacctt ctatatcttc tgattttttt aaaagtggtt gttcttggtt 240
gccttctgat ggtggttgtg ttgt 264

<210> 1139
<211> 498
<212> DNA
<213> Ctenocephalides felis

<400> 1139

gtttttgtta ttttcggaaa cttggattaa taatgaagaa cgaattaata ttcagaacta 60
ttactgcgtt actcaataca aacgatcgga ctcaagagca ggtggtgtag ctgtctaatg 120
tctattattc atgttcagat actcttcata atgcgactcc tatgcagttt cttgcgccaa 180
cgatattact ggatatttct aatgaaatta cacaagtagg tgatattaca cacacagaat 240
caaacattga ttatagtcgt cctttacatt tctcccaata aaaacatatc aaatattatt 300
aaatttcttc atcagcagtt acttcatttt tcttatgagg gtgcaaaact tttaaacact 360
aatcacaacg agataccatt aattcttgct ggagatttca atgtcaattt tcgttcagaa 420
gaatctcaac cattaattga ttttttaaat aataaattta atttaacaat gaataattct 480
ccatttgaat caaccaca 498

<210> 1140
<211> 498
<212> DNA
<213> Ctenocephalides felis

<400> 1140

```
taccgtcggt gtttattgaa tttttagtta tttaatcaga agtaatttaa gaaatatata 60
caaaaattga attttatcag aaaacatata ccatttcata acaagtttta tcgcgttcga 120
caattttcat ataatgaatt aataaatatt ttcaaaatga catcagaaat ttttctctta 180
tcacctgtcg atcgcaatta aaaaaacact gttagccata atattgcaac agaggaattt 240
atccagcaca tgcttattta taaagatcag cttatcaata acatgatata tgaaaatata 300
cttacctaaa tgataatcta ttgtttaaat gcatttttat atttcaaaat tcttatttaa 360
tattttcgct ttcgtatagc agataaaatc ttctggaaat ttgcaaaatg gaatcgatca 420
aaaaatcgtt gntttacagg gaatgcacag caattaatgc cattattaat ggaaatgatc 480
ctatttaagc aggaacac                                               498
```

<210> 1141
<211> 289
<212> DNA
<213> Ctenocephalides felis

<400> 1141

```
catancaatt atgaaatgga ttcatgatat atatatatat ttatcttcaa aacatagaca 60
ttcaaaacta tttgttctca ttaataagat tacattcagt caagaaaaac atgccaccga 120
gttttagctt tttcaacaaa atataatttt tttttattgt taaaaaacaa ctgccataca 180
aatacaaatt caaacacatt cattacaaca ttatgagaat aagtttagga tttaaccaga 240
aataaattaa taatggcgaa caaatagtaa taaataactt aaaatcagt             289
```

<210> 1142
<211> 484
<212> DNA
<213> Ctenocephalides felis

<400> 1142

```
tttttttttt tttttttttt cggtttacaa aatttattta ttcgtcagaa aagaaattta 60
taaatttact tttttgataa aatctgngtt tgtaaaaaag ngttagtgta tattcaatca 120
tacaggnaat taattctgtt ttttttcagt aaccttttct taaaactttc taaaactttt 180
caatttttaa cattttcaat attttgntac tcatttattt ttatcatttc attttgatta 240
ttggcatatt tctatatatt attttttcaa caatgttatt aatttaaaaa atccatcatc 300
ttataatttt cctaaacata ttgntngctt aaatcttatc aaaatcatgt ttcttgacag 360
atgtcctgaa gttggaacat acctagttcc gaaacgcgat aataaataaa taaagaaatg 420
ggtnttgatt attttattac tttttggatt ctttggcgaa gctcaacaat atatatatat 480
atct                                                              484
```

<210> 1143
<211> 498
<212> DNA
<213> Ctenocephalides felis

<400> 1143

aaaatattcg aaacatttac agttacttaa gaataaattt acttatgagt aaacatagtt 60
caaaatattt aatgttattt ataattataa aataattgat ttttagccaa tcgtcgaaat 120
aaaaaaagac aaataaataa aaatatnctt tataaaatac gttttgacac ggattgtgtg 180
tcatctatca gcaatttttg ttaaatagta tctaaaataa taattacaaa gtttaaaaaa 240
taaatataaa attttatatt taaaataagg natacatacc acactagaaa aaaacatttt 300
ttaacttccc gtaaggnagt tatgtnatga accgaaaatc gaaaatgaaa ttttttgcat 360
ttctcgacgt cttgaaggtt tctggacatt ttggcatacc tcagaaaaaa tggatgtgtg 420
tgtgtgtgtg tgttttgtat gccgcatttt tagtcaacgt tttcgggcgc acagatcaac 480
cgatttgaat gggttaaa 498

<210> 1144
<211> 500
<212> DNA
<213> Ctenocephalides felis

<400> 1144

ttttnttnnn tttttntttn nttttgnann nttaaccacg ccatntcggc aagccatggn 60
ntacagcaaa tgagtaaaca cacagncaag gcatggaaac atattatgtn gctgngtgtn 120
gcatctcgaa taaacccaac gattggaccc aaagccaatg tcattattcc ttgcaagaac 180
atgaacagcc cgtatgccga aggaaatctc tcctgtgaaa aatattcggc aaatactaaa 240
ggtaaaggca catgaatcca tgtgcggaag aatcccaaaa aacttgatat tataagcatc 300
attgtaaagt cagtcacgta caagaacact atacgtgcga aaactgagaa taaagctccc 360
gctaaaaata ctgttctact gctgacttta acacaagcag tcattcctgc caaaaacatc 420
cttgatgaca aatctgcagc tgctgcagca gacacacaaa ttgctgtatc agttttgcca 480
gcctaatgca aacagataca 500

<210> 1145
<211> 280
<212> DNA
<213> Ctenocephalides felis

<400> 1145

gtttaagccn ttaggtttaa gnccctngtt agcgcggtcg cgggccgggg tacatttaat 60
gntacacctt gagggaatga cgcttgcaac agccttagaa gcacccggat attttgtaga 120
aggtaaatca caaatctgaa catttctttc atagacgcaa agctggtatt cggtttcaat 180
agccgaactc tttggtttta atttttgaat cttaaagtat tgcccaggcg tagcccaccc 240
atcattaata tctaattcta atgattttgc aatacgatgt 280

<210> 1146
<211> 287
<212> DNA
<213> Ctenocephalides felis

<400> 1146

```
tttcactggc ctaccaaatt tactacgcca cctagtggcg gccaattgtc cttcatattc 60
ctctttgatg gtttttgact caaattcttg caaagcacac atcaaagntt ttctttnatc 120
ggatgttagt ctcaagggga gctcgattgc ttgttcctta cttatttttc catttgaagg 180
gnatgatgag ngattaacaa gactgggagg tattcgnctc tcacantttg gtgntataat 240
agcagcatgt aaaaatttgg aacgactgtg ctgggaaaga acacaat               287
```

<210> 1147
<211> 484
<212> DNA
<213> Ctenocephalides felis

<400> 1147

```
ttgctagttg accatccatc agcattattg agattgtttt gaaaaaaaaa aaaaagtcaa 60
tttaggttaa tttataattt ttgctcttta acaaattgtt tatttatatt gtagtatttg 120
ttttaaatat aacttgtgcc agattcttct tttctaaaac gaattgatat aattttatag 180
atatatttag aaatattgnt tttgggcatg gagggtaaaa gtaattgcgt ttgaatggaa 240
aaaatgggtc tttgtatata tggaacttga tttttatgtt ttgtgaccct aaaaagaaat 300
cttcctgaga gaatcaattt cttctttgct ccacaaatgt aacacacatc acacagggtc 360
tgtacaaaca tcgggaaata tatgactata tatatttcta tcattttcat actttttata 420
ctaaacattt atatttggnn aagaatgnat gatttattat tatataaatg ctaaaatatt 480
tatg                                                              484
```

<210> 1148
<211> 498
<212> DNA
<213> Ctenocephalides felis

<400> 1148

```
atattttgta tagtttcttt tgttgaatac ttcatcaata tattaatata atttaggttt 60
cctctcgcaa gttcctgtgt cgcagctttg agtagcttgg ttaaatactg ttccaatcgg 120
acattgatca ggaactggct ctcctttagg acgtaaacac gtgaagaact tccggcatgt 180
tgcgtcgttc ttgaaagcaa attttccacg tgctcggcat tgcccgacac aggtttcgct 240
ccttggggaa aactcggcgg gttcttcgca ttgcaatacg gttcctcttc cggccataca 300
tacgacgtac aatgatttat caccaatgta cggaagcacc ttaggctgtg caggacatgt 360
aactgtatag cattttgtgc ccagagccat aggtccgcaa ttatgacgta acggatcata 420
tgcaaaattt gcggggcaat aatattgggt tcctatacta ttttcgtcac aataataata 480
gctttgacaa tcatttat                                               498
```

<210> 1149
<211> 306
<212> DNA
<213> Ctenocephalides felis

<400> 1149

taaaattgtt gagcataaaa tacttccaaa gtctactctc caatgtaatc tacaggatca 60
acattcattt ataaagattt aatgtttcga gtgcttcgta taatagaaca cctataaaaa 120
cttttttttg atattcgtgt tgatactata taaattttta aaatcttatt ttattaattc 180
actcatactt ttagcacaga tttaatactg caaaaatttc aatacaaatt tattatatac 240
agatacaaca atccagcttg tcactactct cactctaaat aactacatga ctatcaacct 300
ctctgt 306

<210> 1150
<211> 500
<212> DNA
<213> Ctenocephalides felis

<400> 1150

ctcaattaaa acaaattttg tgctcataac taaagatttt tctggagttg cggnccatta 60
gctcatagaa ataaattatt gacagcaatt ttgttcaatt atatgtcaaa aaaattatgt 120
ttctatctaa tcattattga atgttaatgt aaatggntta attttttaat gcatgaatat 180
ttccaataaa acatattttt gaatgtgaag atagnttatg gcatataaat tggaccatta 240
ttnctatggn caatatttga aaatattaaa ataattttaa tgcttattat nctgnaaact 300
attaagaacn ttnctgggcc cngaatatnc ttttnaaaaa catttggtta ttcnaaatta 360
ttaagaatna aaacgtgttg ntttcagacn tgaaaggtac natttctaac ntctggnttg 420
gcacngaacn ataatttgnt taaggcccac atttaaaatc ntnacaaaac ntctcacncn 480
nanttttta ttattcatca 500

<210> 1151
<211> 349
<212> DNA
<213> Ctenocephalides felis

<400> 1151

agaaacagat agtagtcaaa tgagtttccc cctgcaatag tcgccagaac gaggggaaat 60
atatgcttag ctattaaaat attcattata attaaaaggg tttgttaact ttattttaag 120
agttaaatta gaagtggctt cagaccttcg taattttata aacactatga tctgtaagca 180
taaatcagta gtaaatggtt tactaaactt cctaaatgca caaacaattg ctcaaaaata 240
tcattgttaa agcattgaac tatgtctaaa atattgcaaa agaaattaac atattttaaa 300
tattactaag aacaccatgt attctgcatc agatgtttat ctttttttgt 349

<210> 1152
<211> 396
<212> DNA
<213> Ctenocephalides felis

<400> 1152

atggattcat gtgcctttac ctttagtatt tgccgaatat ttttcacagg agagatttcc 60
ttcagcatac ggactgttca tgttcttgca aggaataatg acattggctt tgggtccaat 120
tgttggattt attcgagatg caacacacag ctacataata tgtttccatg ccttgactgt 180
gtgtttactc atttgctgta taccatggct tgccgagatg gcgtggttaa aaatgaaaaa 240
taagaaataa atttaagaat taagttaata ttaatggaaa aattatatat agtttatgtg 300
aattttatca cacgtgttat atatctttat aaaagtaatt tataaaggat tgtcacagaa 360
aatataaatg acaaaaaaaa tgttttnnaa aaaaaa 396

<210> 1153
<211> 500
<212> DNA
<213> Ctenocephalides felis

<400> 1153

tntttttttt tttttttttt ttttttcaag cangtcatca aaaaatttta ttaaatttcg 60
gnnatngnca tantaaatat gtaatatcac ctcaatttca gntataaaaa taatagccaa 120
ctgttatcat tcanaagnat tatttngcct tctgactcct ttaaatcttg ttcttgactt 180
aaacgttgtt ctgnagcccg ttcccatgca tattnaaatg gaaanactaa ngggttattt 240
tcctctgggc cgttngcgcc atctcgacct tatgggcngg agntcccttc atatgcaatc 300
gntctaatat ggnggggnat gaacaacgga atcaaaagaa gttatntacg agaatntttc 360
cttatcagat ttcacatccg ggcgcccttt tgntgaaaaa agaacttatg tttgccaatc 420
tctnttaaaa actcaaagca cttggatntt ctgggccgat tgntgggcac atctattaac 480
caacatttat ttttgtcctc 500

<210> 1154
<211> 314
<212> DNA
<213> Ctenocephalides felis

<400> 1154

aaatatttgg tttctgttta taaaagacta atacaaacca tttaatcatt cattnnaaaa 60
taaaacattc ataatattta agttgncagg tgaaaactaa ttgcataatt atttaactaa 120

attattgact gttttatttt tcgtttgatt ttgtgctgaa atgatgcaaa aagaataatt 180
ttgagcaaaa cctacctctt tttaatttgg aaactataga gggnattatt aatgnttcca 240
attccataga atgtatattt gtagattagt aaaggtaatt aatgccnaaa aaaaaaaaaa 300
aaaaaaaaaa aaaa 314

<210> 1155
<211> 352
<212> DNA
<213> Ctenocephalides felis

<400> 1155

atggctacat taaaaatcaa acgattcaat gtttttatg aattgagaaa acaatttata 60
taaaaacgat taactttcat tcagaagttt ttgttaatat aacaatagta tctgaaaaat 120
attaaaaaca tctgtcaaaa atatgcaaag taatttcatt caattataaa aagattttc 180
aataatttac aaaaattaaa aacattttg aaaagatatc aatatcagca catgttaaat 240
agtaagagac aagaaacatg acctttaaaa tttaatccgt gttgcatata caacaatgta 300
ttttaaactg tgattttggc aaccatcact tcatttattg catacgtaat gt 352

<210> 1156
<211> 500
<212> DNA
<213> Ctenocephalides felis

<400> 1156

ctantnagcc ccttactnac ncttggannn ccgcggncgt ccggcggtcc tggncaaatt 60
ggagttattc gatgtcttat gtagagnatt ttgaagnggg taaataaata atacatcggc 120
tcgcgagntt gttaaagttt gattaaataa cttagtgatc attttcaagt tattgaatta 180
caacactttg aaagcattaa gtgcctttgt tagattttaa caatatcaac ataataaata 240
attatattaa aaaatttatt ttaattcact cattttaaac aaaataaatt tcatatacaa 300
tatttaataa atcctgacaa aattgtattt aatttacttg atattttaaa taataaatgt 360
tttagttaga atattaagga aacgtataca ttaaaataaa atgatatgtt cttcaccttt 420
atcttttcaa taatacgaaa ttcgaattag tatggcttcc gcttaaatta aatatgtttg 480
ctaaactgcc aatcaagaaa 500

<210> 1157
<211> 92
<212> DNA
<213> Ctenocephalides felis

<400> 1157

caaacgaatt acaacaagtg gaaaccactg taataacaaa cgaaaagtgc tacgaattgn 60
ctcaattcgn tgaaccaact tcgcaaatat gt 92

<210> 1158
<211> 495
<212> DNA
<213> Ctenocephalides felis

<400> 1158

tttttttttt ttttttnttt ttcaagggaa aagactttat naaaatangg ngattttata 60
ntcngtatnc aacnctgatt acnantgttc cattactttt ataaaatntn agaaacaatt 120
nacgctngna tnaacaattt aacttcaact ttgggagcag tttcaanagc agccaacaaa 180
tngtctacat tacagcaata tgngtatcct ctagttaaat tagcaaaata ggaatcttca 240
aatccagctt ntttgcagan gtccttgcag agtanatctt tatgaggaac tttntntgca 300
taaggattat tgagggtaan aaaagcaang ccttctccag tttttggcga tngtactatt 360
ttccaattcc atttgggtat ccaaatgttt ccgtntttgg caataaacat ttttacttcg 420
ttgttattgc atcaggcaga gttaggatat natggacacc tgaatagacg gncaaatctt 480
cagctntgga agaan 495

<210> 1159
<211> 148
<212> DNA
<213> Ctenocephalides felis

<400> 1159

```
atgaaattat gtgttttgaa atttttccga ttttgtaaat aagacatgct tttgttttga 60
tattttaaat gtgttcattg ggattactgg ctttttttac aaactcatat gcttgatacg 120
catatacata cacataattt gcacatgt                                    148
```

<210> 1160
<211> 339
<212> DNA
<213> Ctenocephalides felis

<400> 1160

```
cattctagag tatttgggga aagaagattg ttcgataaat caacaatttt agacaaacga 60
gaaattaagt aatattataa aaatagatat caatctttca tcttactaat ttgatattga 120
taaaaaacga agcaatacat agggtgtgtg gttgaggcgt ttcttttttt gttttatagt 180
agatgaattc taaaacatcc agaaaaataa tatatataaa tcagttaaaa aaatgttttt 240
tttcgcctat tcagtatcac aatatcccaa caatgcaata tatggttggc acccattcaa 300
aactaaagta tgacaagacg catagccaat tgaaaatgt                        339
```

<210> 1161
<211> 212
<212> DNA
<213> Ctenocephalides felis

<400> 1161

```
aagctcttta caccatcatc aacttttgtt ttgcagatgt aatagaaatg atcatctcct 60
ggccatgggc cgttttctcc agctctttgg cagtctactg cgagattctt gcaaatctcg 120
gaatccaagc ttgaagtgca cgtaccagtt acgacatcga atgcattttt accaacgcat 180
tcttttaata ttccatcagc tcctttttca gt                               212
```

<210> 1162
<211> 498
<212> DNA
<213> Ctenocephalides felis

<400> 1162

```
ttaaaacaaa gaaaattagt tcaaatattt ttcaattctt agtttgtgtg tgcatatatt 60
gattatttag aaatctttat aaacaatagg acacttacat ttcaaaaata ggtgttaccg 120
aataaattat gatctctttc actacatata ttacaatcaa catttattaa ataagtgntg 180
aatttattta ttgngcaaat aataatatgc tntncatgtt tgnaatcaat ttattaattt 240
aattgnataa tttgntggtt taccncacat tttagctact ggtgagctag gcataatgng 300
aaanggatat cttaattttt ttgngatcaa agctttcatt ttattaaaaa aggaanttat 360
taataaatat gngccanttt tcantattgg gatgctantt atgccnatga tatggtggna 420
tcatnatatt tgaataatcg attttttgat cctnttgngc gatatttacc aacnntaatg 480
ttttaagctn taaagaat                                               498
```

<210> 1163
<211> 360
<212> DNA
<213> Ctenocephalides felis

<400> 1163

```
tccnaagccc ttgtnggctn catttngntt aacgggggcn ncggcggggg actaaaattg 60
ntgagcataa aatacttcca aaggctactc tncnatggaa tctacaggat caacattcat 120
ttataaagat ttaatggttc gagggnttng tatnatanaa cncctatnaa aacttttttt 180
tgntantcgg ggtgntacta tataaanttt taaaaactta ttttattaat tcactcatac 240
ttttagcacc anaatttaat actgcgaaaa tttcaataca natttatttt atacagatac 300
ancaatccag cttgnngcta ctctcactct naataactac atgantatta ancnctctgt 360
```

<210> 1164
<211> 500
<212> DNA
<213> Ctenocephalides felis

<400> 1164

```
cgagtatgaa tatgattcaa agggaatcaa aaataacaaa gataaagttt cattaaaaga 60
agctatttta ctcatggaga aaatgcaaaa taaaatggat gattccagca ctgtgctcag 120
cgatctggac gatgatcttt tggaagctga aattttgcag tctgcaaatg caggaagtca 180
tgaaagcatt ttcaaaggtc gctatcacta cgtgtggcct atgttgcttc tccttttaat 240
tgttcttacc gttttgtttg tcgttggaaa aatagttcac atttgcaccg ataaccgaca 300
acgttcactc aaatatcaga atataataac tgctgtgaat cacaagattg taaagaagaa 360
ggattgtgga ttagtatatc aacctctatc agaagaaatc agatgtcccc aacgccaata 420
gtgacacgct atcaagctat ttgagcagcc gtttcatcat gaaaaaatac ccccgacaag 480
tgtactttaa acgttaattc                                             500
```

<210> 1165
<211> 495
<212> DNA
<213> Ctenocephalides felis

<400> 1165

ttttttttn ctnttttttn gatgaattaa cattagtcaa tttttttatt nnaaaaaata 60
taatatgaga tttccgatta caaattattt ttgtttttttc tttgatgttg atggtttatt 120
ggctttaaat ttagatatta ttgnagttat caaaacaaga ctgaatccta tagttgtaaa 180
tgcgataaca ccaaatatta cnatagatca acgatggngt attcatttat gagtgnggta 240
aatttatcaa cagcatcacg aatcatttcc actacccagg aaactagatc atcaaatggt 300
gcttttggta gtggcacagg ttcagcttta gtatttttgt caagcccnnt tttttttttt 360
tttttttttt ttgcttgnng ggttattaga ntatggattt gtcatctata aaacnaatag 420
ttaanaaaaa ttttttaagg tcaatttcca cgangcaatt ttncacaaca tcattaattt 480
ttccaatctg gattc 495

<210> 1166
<211> 419
<212> DNA
<213> Ctenocephalides felis

<400> 1166

cagcaccatt agcacaaaca gacaaccggc atgagttatc caatcgcttg cgattctatg 60
ctctggtaaa aaatataagt cgagaagttg ccatatgctt ctccaaacat tgacagttcc 120
aatgaaacta aaaaatagaa ataagtcagc aactaaaact ctccaaaatc cttccaaacg 180
agcacatgtn catcgcatga taggctgaag actgaatgtg agtattacaa tcccgtagcc 240
aagtgcaagt gaagccagag cactaagcga tggatcatct ggaaatataa tattgtccaa 300
tatcacccaa gctcccctnc acacgacaac caccaaggat ccaacgatga atacggaaaa 360
taagcaatcg attaccgtaa agccatttct tntgatgctc gattctttaa aatatgtag 419

<210> 1167
<211> 293
<212> DNA
<213> Ctenocephalides felis

<400> 1167

tagcccttca atcattattt ttatttaata attgggcaga tttgntagaa caaaaataat 60
gattgttagg gctataaaat ctgcacaatt attaaataaa aaaatattgg gaaaattttc 120
tatatcatgg gcaatttatt acagaagagc gaagttaagc atgagggntt tatcaattat 180
tggtctataa attataatag aaatgaaaaa aattgttaga tataaagaaa tgtttactgt 240
aagattttag caagtgttga gttgaaaatg aagaatatgc ttacaaaatt ggt 293

<210> 1168
<211> 109
<212> DNA
<213> Ctenocephalides felis

<400> 1168

caatcaagac ctttggaaag atttttgggg cannttaggt tggcccaaat ccaagaggaa 60
attcaacgtt ggccaagcgc ctttncctac atcagcgggc aaatctggt 109

<210> 1169
<211> 438

<212> DNA
<213> Ctenocephalides felis

<400> 1169

```
tgagaataaa gctcccgcta aaaatactgt tctactgctg actttaacac aagcagtcat 60
tcctgccaaa aacatccttg atgacaaatc tgcagcagct gcggcagata cacaaattgc 120
tgtatcagtc tttgaccagc ctaatgcaaa cagatacatt gtttgaatag cagaaaaatg 180
ctatgtcgga gtatagtgca aatgtcactc caataaccat atttacataa atccagtctt 240
ttaacaaagt caaatccaaa aagtcaacga taacttgcaa ttttcccctg gatttcttct 300
ctaccggtgc agtagtgcta tcactgacaa tgactgctcc agtccaatta cctaaactag 360
aaatggaaga tccacgtctt ctcattctat gttcttcata ttgaagcatc ttaactggag 420
ttacgccttc tatatncc                                               438
```

<210> 1170
<211> 500
<212> DNA
<213> Ctenocephalides felis

<400> 1170

```
tttttataa atngtttaaa aattacatat tattgaacaa aaaataaact gtagtaaatt 60
aaaaacaaga tccaaatttc caaagcaatt tgttaatcaa taactgagaa acgactcgtc 120
gattattgac aaaatttaat cagcacgatt ttcaaataga aaactttttt aatggttttt 180
gaattttcaa aatcgattca caaatgtaag ctataacgtg aacaaaaaat ttgagatttt 240
tttgaaaatt ctaaagaaac ggctcggccg atcatgaggc aaaactaacc agcacgagtg 300
cttatcgaat ttgcgaattt caacgacttt tcagatagga ttttgcatta ttaagaaaac 360
gtcgactaaa gatgaaattt agactcacgt aaaaaagtgc tgctcatttt tttggaaatt 420
tttatatttt tctgttgatt agaaacagct tggccgtcgc gcacaaaact aaaagacgct 480
agtaattatc tagtacatca                                             500
```

<210> 1171
<211> 220
<212> DNA
<213> Ctenocephalides felis

<400> 1171

```
tctcaaggtc tggtgagacg ttccacaaaa attctagaaa tcagaacaat gcttccaaaa 60
tacaaataca cgtcctacaa tatcaccatt caacacctcc aacaatttta ctatatcatt 120
tactcctcaa aattcgtcct agtaggctta taaacaccga catccttgtc ctccgaatgg 180
tagcccaagg aggccaattc gtccataacc tcaacattgt                       220
```

<210> 1172
<211> 284
<212> DNA
<213> Ctenocephalides felis

<400> 1172

```
aataatttta actatgtctt attatatccc tttgtgcata taggggntaa actgactaac 60
aagtatcact ttttttata aatatccaag tatcatacga cacacgttta taacgatttc 120
ataaattaaa tatcactgtt tgtttttgta caaaggcact ttttgtcttc aagcacacac 180
cgaggcttta aactaccaat gtaatatttg actagaataa ttttaattgt ttcagcgtcc 240
actgaaggct ttaagcaact tcgtgccaaa cagactctct gtcg                 284
```

<210> 1173
<211> 500
<212> DNA
<213> Ctenocephalides felis

<400> 1173

```
ctaagatgta tatattctac acatatgtgc atataatatt gtcaagtaat gtcatgtgat 60
atatctccaa aaggcgtaaa cgctggaaga aaactgagta aactagaaaa ccattgatgt 120
gtggaagatc tagtccaaga agcttttta tggtataaaa tggaaacaaa aatgtaaaaa 180
tatagttgca attataataa tcatatcatt agatatgtag cgaatatgag atagattctt 240
tttaaaactt ttataaattt atttatatat aaaaattctc aatataaata gtatactcaa 300
atatatttat atgatttaat aatattgaag tttttataac caatattttt cagtaaaatt 360
ttcagtaaat gatgattttt tttataaaag tgcgtattaa ttaaaataat ataaaaaaaa 420
aagattttaa ataatcaata tactattaaa tatatgatca tttttatgaa gtattaattt 480
tcttataaat tattagataa                                             500
```

<210> 1174
<211> 353
<212> DNA
<213> Ctenocephalides felis

<400> 1174

```
cttcattttt tcaatatgca nttcgatttt aatctaaggg aaaaaatntn cnggtgtngg 60
ttaanattta attnggaata aaattctgtg tattatattt agttttttt aaattatcat 120
ttaatnttaa ttattgntaa aaatatnatn cgattaatta aaataatatt aaacataaat 180

ttcattgntt tttttangaa actaaattgg gaaccttttt atatcttatt atcnacgcta 240
aaaaaaatac atttttttta gtattttacc gtttcatatt atattaanaa aaacttttag 300
ttaatactaa ttattataaa aataactaaa agttnnacaa aaatactgga agt       353
```

<210> 1175
<211> 500
<212> DNA
<213> Ctenocephalides felis

<400> 1175

tttgtnanna aagatacagg ctgtttgttt atattgcata ggtatacaag tgaagcaata 60
ttttgaacat tgaaaaaatc acaaattggg tatataattt acagnaaaaa tacaatatag 120
ttcagaattc ataaattaat attttntaca agaaacaatt actttaggng cttaataata 180
agtttcttaa tgaggcatga agatataaaa ttaaataaaa atgatcatag ngnaaaatac 240
aaattaaata gttcacacaa ataaatagtt agcaaagtca tctacgctat atataccctt 300
aatactcgta tattcttcta gtagtaattt tttcactaat ttcacagnct tcaagattat 360
tccaaaagat ttacgacgaa ccatctgatg tgagcgaatt tgatgtagag ctcagtcgat 420
ctccgtattt attaagaagg ctaatatgac accattcgtn cagccaaaac caagctggcc 480
tcatattntc cgcatttctg 500

<210> 1176
<211> 299
<212> DNA
<213> Ctenocephalides felis

<400> 1176

ttggcaatga aatacagttg ggaaaggact aaatcggctt cggtgctaag gcttaagctc 60
tanaaattct ttcccatttt gggatttttc ccggggacaa aggcagaaaa acttggtaaa 120
ttcaccgggg aattttaaat ggtagaagca aaacatccaa ctcttggaac ccggagatac 180
caccacctcc agaaaatacg tctgcaggta acgtcattgt tccacaaacg gaaactgatt 240
taaatattca aaaatcataa aagcggtcat agntttatta atgagttgga aataattgt 299

<210> 1177
<211> 230
<212> DNA
<213> Ctenocephalides felis

<400> 1177

aggtgagcgt ttggtgaatc ctgaagcatn ctgttcatag caacagctga ttgggatatc 60
agcctgcgct gtccggactg gaccatggtg ctcttgacga gcaatcctac acatctcatn 120
gccataacga aatacgaaaa ttattagtga accttcgaaa aatcaggaac cccgttgtaa 180
atgatagatg gacacacaca acgcgcgttt gaaatgaccg acactgtngt 230

<210> 1178
<211> 318
<212> DNA
<213> Ctenocephalides felis

<400> 1178

ggcaacanct ttgccttggg caccggctgc tgctttggca gcataactcc tgctgttcga 60
taaaatcgat gcgattttac ccgattctgt tttgctcagg gttcgcaaag tggaattgat 120
tacggagtgc atcttgtaat aaaatgtata gccagttgag ttgaattaaa ttgatctcac 180
ctgtaagcta ctgaacgaag aatattaaat cctttatgag cactgatagt atttttgga 240
gtgtgcttac gtaacttctg cacttttcag tttcattgct tgnaacattt tcttgaggag 300
cgggtaattc ggcatggt 318

<210> 1179

<211> 329
<212> DNA
<213> Ctenocephalides felis

<400> 1179

tgtagccacc gcgatacgaa tcggaacgcc catgcttatc atccatgttt ctggatagct 60
gactcacaaa ctaacttcaa tggaacccca actcacacgt caaggatttt gattaaatac 120
acgtgcaaat ttgaataatc aaaattaaac ttgtcttata ataaatgttc cacacatttg 180
ncctnctgca tntgnaggta tgatgatatt gaactggcac gagtgcactt aaaatagttg 240
cattataata ttaattcgtt gcacttgaaa atttaatgct gaaatgncga aatagtccac 300
acagcctaca agcacgtaac acacgtnaa 329

<210> 1180
<211> 190
<212> DNA
<213> Ctenocephalides felis

<400> 1180

ttatattana gggncgtgtt gaggtaactg ncggaaaaga aaatttantc tttgagagtg 60
gtccatttac ttattttggt cttcaagcga ttacacaaaa cattggagtt gccgattctg 120
ngaaaggatc tatgcaatcg ttaaatatag atggtatatt aaaaaacagt tttataccag 180
actatacagt 190

<210> 1181
<211> 305
<212> DNA
<213> Ctenocephalides felis

<400> 1181

cgttacttcc aaccattttt gcaataattt taactatgtc ttattatatc cctttgtgca 60
tatagggttt aaactgacta acaagtatca cttttttttta taaatatcca agtatcatac 120
gacacacgtt tataacgatt tcataaatta aatatcactg nttgtttttg tacaaaggca 180

cttttgtct tcaagcacac acgaggcttt aaactaccaa tgtaatattt gactagaata 240
attttaattg tttcagcgtc cactgaaggc tttaagcaac ttcgtgccaa acagactctt 300
tgtcg 305

<210> 1182
<211> 58
<212> DNA
<213> Ctenocephalides felis

<400> 1182
tacgatttag ccgaggcaaa taaaaattct attgnaaacc canagtggaa gcaaatgt 58

<210> 1183

<211> 383
<212> DNA
<213> Ctenocephalides felis

<400> 1183

```
agngggctaa tataggatct ttataattac cgnagggcca aattagnnga caaaatnnaa 60
ctgagatttg taataaagat tgcaaacatt ttcaaaaaaa aaaaaaaaat tgaaaatttt 120
acgatttttt tcggaggagg gtaattttca atatacggtt tcacagaggg gaaaggggat 180
ttcaaaaatt ttcaaaaaaa tcgattacgt gattaagtga cgtcccgagc acaaaactta 240
tgggtccaaa tatatttaaa atnttaaaag tccaaatgtc caaaatctgt attacaaatc 300
tgtcagtctt tttgcaatcc tttatcaaat attattttcc atcaaagcac acactttttt 360
atacatgcat tttccaaata cgt                                         383
```

<210> 1184
<211> 500
<212> DNA
<213> Ctenocephalides felis

<400> 1184

```
tttttttttt tttttttttt tcacataata tacatttatt ttcaatttaa ttaacnggga 60
aatgctattn cataaatata acgtattnta acatacaaaa atcaaaacat atttatncta 120
ctaagtttat nctgttngca acagttgtct taccttaaag taaaaactgg gggccctgcc 180
ctatatacac tgngtctatt tactgattac aatcacatct gacaatccca atcacaattn 240
antntaatcn ccanctcatt tatttaattt ataatcaata tgcctggatt tgaatttaaa 300
ttcaatngga aaaccaatca gatnggtngc acacataaat atactatttt gtattttatc 360
gacatgaaga gataagttat atttttagat caccactgac ttttttactt ttgattaagt 420
ctcaaattta tattatatat ttaaatattg gtttataata ctgaatattt gatgtgngaa 480
tattattaag ccaaaagatt                                             500
```

<210> 1185
<211> 327
<212> DNA
<213> Ctenocephalides felis

<400> 1185

```
cttctactct atcgcctctg aagaaagacc aatctttaat aggctcgatg aagatttttt 60
tcttggcatc aagaggtgaa ttatcttgtc ggaattcttg agaccatggt cggtgagctc 120
cgaagtagaa cttccttttt ttgatttctt tcctcgaata attcggtttg ttaggagttt 180
tccaatatac ttgttccatg cttcgcttaa tgtagttttc tggcaaattg gaatattgtt 240
ttgttaaatc gccaaccttt tggcataatt gcaaagatct cctcatgttt gaaattattt 300
acacaaaatc aatgacagaa taatagt                                     327
```

<210> 1186
<211> 498
<212> DNA
<213> Ctenocephalides felis

<400> 1186

```
atataagtaa acaccaaaat aatccattgg ggttccaata gattatattc ataattaata 60
ttacttacac gtagttatat ttttattggt aggaagtagt gatcattaaa gttcaatttg 120
aaaaaatatc aatttttttt tatcaaatgc tgngtatgga attatatata caatttcatt 180
tataaatttg ccaaatacta atttataggt gattatattt tctattctgc ccatcacaaa 240
taaatgaatt ctttttata ttccctccat ttgcttttag attcagtagt aatcactact 300
tctatcaata atcaatgcat atccctaatt aagagagnag attcaaatat cattttatta 360
attctaaaga ggattcttat gttgatcgat attttaacat gntgaaagga aaactgaacg 420
acttgctcct ttatattctc attttaatag cagantgntt tttgaattag gtataatatc 480
tatatttatg cnaaataa                                                498
```

<210> 1187
<211> 496
<212> DNA
<213> Ctenocephalides felis

<400> 1187

```
aagcngaaaa aatatgaaaa atatgtaaac tgnaataata ataaatatta taattattgg 60
gcaattaata ttaattataa aactattatt atatttctag aagcaaaatt ttttttttcct 120
taaagatgtt tttttgtagc atgtgtattt cattcaattt acaattatta tgaaggagaa 180
aaactgtcga atatggagtc acgttttact ttaaaatttc aataaagtat tttaataaat 240
aacatgttta atgaatttaa tatttttcca tttattatac acagagagaa tgctttatat 300
tcaaagattt atgttttaaa atcatttttc ataaagggtt tcatatacga cttttatatt 360
ctaaatcatt aatttttggg atttttataa taattaataa aacaaattta ttcagtaccc 420
nacntcagta tttaaaaatt attaacaagg atatgcttat attaatcaaa tgngagtggg 480
naattatttg cncaaa                                                  496
```

<210> 1188
<211> 448
<212> DNA
<213> Ctenocephalides felis

<400> 1188

```
ccgggcgggg gaccagaagt ttacttacat aacttgngng ggnaacttat tccaaaaaga 60
tgngnttacg ggngnatata gnagaataag atttaatatg tnatattaca tggnacttaa 120
tttttaattg natatcaatc tttagtataa gcaagactac gagggccgta ttttaatgna 180
tattaaaaat gatgngnata atcattattt ctatttaatt caaaagagta tatatttgna 240
atatctatat ttaaacaaat attattaaaa taatcatata taaattatac agcattctat 300
tttattcaat ggcatgagca ttcaggacgt aaagngatta acagggttcg atcaaaatag 360
aattctatag ttttggtcta aaagaaacga aattttaaaa aaattatgtt attcngnggg 420
tttattacag ctattaaatt ttttacct                                     448
```

<210> 1189
<211> 117
<212> DNA
<213> Ctenocephalides felis

<400> 1189

```
cgnacgccct cttaancncc ttaaaangcc ctcgagcggg ccgnccgggc acggactttn 60
aaantacaaa ttnacggnat tgttataaac taacagaatt gacagtttta taattgt    117
```

<210> 1190
<211> 213
<212> DNA
<213> Ctenocephalides felis

<400> 1190

```
caacaacaat ttcnaaacaa aatggctgga nggataaaac ggntaaaaaa nggtattcaa 60
tnggattggn ttttggtacc ggtaatnccc taaagggagg caggggcggt actttttctt 120
tntgggcggt gggtggactt nggcccaatg gtcaatttcg gttatttncc aaacttcaag 180
gcttttgaaa ctttggcatt caaaggacaa agt                              213
```

<210> 1191
<211> 207
<212> DNA
<213> Ctenocephalides felis

<400> 1191

```
cgctatcaca gcaaaagaac cagccatgat tgccgaagtg tcggtgactc tcatgtgatt 60
catataatca gctttgtgtt gcttgtgatg atggtgcttg ttgaattgtt cttcatgttc 120
attagattca cgatcctggt gaattggttg aaccaactcg gctccaaaat caggcgacga 180
agaagagctt tcctgaatcc tctccgt                                     207
```

<210> 1192
<211> 330
<212> DNA
<213> Ctenocephalides felis

<400> 1192

```
agggantagt aaagggaaat tagngcccat tgataccaaa acaaaagaat tnggtctggc 60
tttaatttca acaaaaaaat ggttggggtt antggaggga atgaaaaaaa ttcactggaa 120
ttaaatggcg gtctanttcc ctcacccatt tcaacaaggg gatcaacgaa aggggtgggc 180
aatngttttc aagccgcatn ccaagttnat tttagtagga atcaagcccc tcaagcggat 240
nccctacctt cttaattttg cttgcaaggg tattgaaacc gggttttaag cttggcccat 300
tgnaccgggt ctttggctaa ccaaaaaagt                                  330
```

<210> 1193
<211> 149
<212> DNA
<213> Ctenocephalides felis

<400> 1193

ataaaacaaa caccccgatt ataaaatatn aaaatatgta ccatatcatt aggttaagna 60
tgtaaatagc gctcgaaaat tatttattaa taaaacattc ttaaaataat tgattgcaat 120
tatgaattca ttaacgatat ttgttatgt 149

<210> 1194
<211> 342
<212> DNA
<213> Ctenocephalides felis

<400> 1194

aaaatggcat tatatgcnna ttataggctg agcctttata aatctgtgna tgaaccanac 60
cactctatct tctaacctaa agtgtaattt tgttttacag aatttcttta ataggtaata 120
tttttaattg cagngtatca tattatagtt tgtaggtgtt atattttatt tgtaaattgn 180
tgttcatatt tctcaaacga ttgtttatgt attacacagt attaaaataa accttggatg 240
caataattgt tataacaaca ttgttgttag taataaaatt ttatcaatgg attattaaaa 300
aaaaaaaaaa aaaanannaa anaaaaaaaa aaaaaaaaaa aa 342

<210> 1195
<211> 131
<212> DNA
<213> Ctenocephalides felis

<400> 1195

atgatggcat tcatgacgac ttctgtcgtn naacaggcat tttttgttca caaatcctgn 60
ncagtaaatc ataaatataa cgaaactatt tgtagaaacc tttctcagta taaagacatc 120
aacaccaaag t 131

<210> 1196
<211> 463
<212> DNA
<213> Ctenocephalides felis

<400> 1196

gtgcagtttt taattgngaa ttaaatatat gttgatttaa tttttgngna aactgangag 60
cgtttattta aaatcaatat taaactaaca ttgctaaggt gatcttaaat ggtgaaatgt 120
tcattatcat attaaatttc aattcaacgt atctacttca tcaaaataaa taattttatt 180
ggtgacttca aattatcaat atacctcctt caaattataa atatacctca atttttttc 240
acatgccgtg cagtgccgtt tgaaaattgt aagttacatg cttttgttga tttgtaattg 300
aatcttttga tgtcttggaa aacttttatt gntacacata aaggatgaat tataaatcaa 360
tggaagcatg taatgattgc attacacata ttcgcctcat atatattaag tatattttgg 420
tggtaattct tgcacttatt ggttaatatt ttattcaaat atg 463

<210> 1197
<211> 252
<212> DNA
<213> Ctenocephalides felis

<400> 1197

```
ngntcttcaa cnttgctctc caangccctn nagcggncgn ncggggacgt tttttttaa  60
ctgntaaaat atttattata aaaatacaaa acaaatttca taaaatatnc attacaattt 120
gngaccaaaa aaattaataa ttccctgttg catcaatatt gccttacaaa aaatttccac 180
gaatgaaaat ttatattata ttctatttaa acacttatta actattattt ttagttcaag 240
ctaatgtaac gg                                                     252
```

<210> 1198
<211> 444
<212> DNA
<213> Ctenocephalides felis

<400> 1198

```
aacaaaaaat aaaaaataac ccattttact ggattcattt gaataataaa tatgttngtc  60
tgnatgggtt ttttcttaaa aaaaataacc aatatctaca attgtctttg acacaatata 120
ttaataacag acactaacat agtatttgaa ataacatagt attaatattt gtattaaggc 180
actcaatact ataggaatac ttggaacgcg cattgctaaa ttcctcgctt atccaaaata 240
cgtataaata cttattatca attatagggg catattgcta aatgacttaa aatcaataaa 300
aagcgaatct cgcgtataaa aaaatgccat catcatatct cactgtgtgn aaaaatatta 360
aaaatttgat cacgatgatt ttgctgatca caacttatgc tagactcaca atatgtgaat 420
taaacatgag ctctcttata tttg                                        444
```

<210> 1199
<211> 498
<212> DNA
<213> Ctenocephalides felis

<400> 1199

```
attattcata agacatcaaa gactttatta cataagggac aacatagtaa ttaaagttac  60
agaaacaatg cagagtcaat acttcgattg cttataggaa ttcatggagg tcgaagaaat 120
ttctagtata tttttgattt caaattttaa tactcagtta atggtatgaa tgctaagtct 180
ctctctactt tattcatata tgacaatatt gcacatttgt gcacgaaacc ttgagttgaa 240
tttttcagag atagttatgc atgaaatgtt gaataccagt atttgctcca acggccttcc 300
tgcatgcgtc tatatggaag ctatgaactc attttaaagt tgaagatttt atggaattaa 360
ttataagagc ataatgactc tgagtttgag gaacttgctt atgtttaact aaatcatttg 420
caacaacatt taaagtcgga actgttcaca actggaaaca ggatatctgc atttataata 480
ctttaataat aattgcat                                               498
```

<210> 1200
<211> 226
<212> DNA
<213> Ctenocephalides felis

<400> 1200

ttaaatattt atatgatatt tctctacgat gtttaataaa aaaaaaattg attgttttga 60
acttgaaatg tagatttgga atatctgcac gactgttatt aatttaaaaa gttcaaagat 120
tgtttattca ataaggaaca tattaaatat catattgtta aatttttata tattgatcta 180
ttgtaatcta tctctgtgat taacatttac caattaaaat tggagt 226

<210> 1201
<211> 228
<212> DNA
<213> Ctenocephalides felis

<400> 1201

agtatataga tagatagaaa ataaatatag atattaaact gtgtttaatt aaatattnac 60
aacatataat ttataaatat atgcttagaa aaatacttgt agcagcatta aaattaacat 120
atcaagcatt tgttttcata aaaattaaaa actgcttcat ttatatgatg aaactatgat 180
cgtaaggctt gcaggcgact ctgcatttct tctaaatctt cttctggt 228

<210> 1202
<211> 70
<212> DNA
<213> Ctenocephalides felis

<400> 1202

acttagcggg cgccgtgcaa gtgattcgac caaagacgga caccgaagta gacgtggaac 60
tataactggt 70

<210> 1203
<211> 114
<212> DNA
<213> Ctenocephalides felis

<400> 1203

ggcgcaaccc ttttggcgac tgcctcacct gccttcttag tgagtttctt aagattattt 60
ctctttttga tgttaagaat agtcttaact ctcctcttgt tttcaagagt acgt 114

<210> 1204
<211> 337
<212> DNA
<213> Ctenocephalides felis

<400> 1204

gaatactggt tcataaaatt tagaagccct nntttttcgc ctacggcggt atggngttcc 60
gattngtgtc cgggcttgtc caaaaatgta aaatccacac gcgcatcttc caaaggcgtc 120
aattcatgta aaacattcgg taaccttaac anttgtgtta ctaaattctc ctgcacgggt 180
tgaaaacaat ccttaagcat tttaaatcat cccttacant tttaccctgg ntgattaatt 240
tagtaaattc cgtcaccgga tttgtcgagt gtttaacttt atcggcgatg ctggttctgc 300
acgcgttcaa ttttcggcgt gttttgtcaa atcttgg 337

<210> 1205
<211> 445
<212> DNA
<213> Ctenocephalides felis

<400> 1205

aaatatggcc caaacaaaat attgcttttt ctctaattgn gctttttaat gaagaatcat 60
acataagtat gaataatttc ctaactaata ttgctttatc attgatttct ttgaaatcag 120
tgtctttaaa gnttcctatt tcacttccat ttggcatagg aagttcactg caacgagaaa 180
tcaaacttat acctacacaa gctgcttcaa taatcattgg tttagaggac ataacatgtt 240
gtaacaatgc atttactgca attgtataag tattgctttc aatcagntta ctagaatccc 300
tttttgaaat ttgtattcta cgttcacatg cattgctaaa tgctaatatc agcccatgtt 360
tgttttcatc agatatgtta gctgattttg attgcgttat catagactga atttcttttt 420
caaattcaaa tgctttcata gtggt 445

<210> 1206
<211> 383
<212> DNA
<213> Ctenocephalides felis

<400> 1206

catataactg tatgaacagt actatataaa tgatgcgtgt attataattt cgagttaaaa 60
cattttattt gtgattatac ttgtgattta atatacaata tctattttaa gttatgacat 120
gttaccgact caaaaaagtt gatatattga atttttatat aatttaaact atttgtgaag 180
agatttaata cttctattta tgtataattg taaataataa atacagtatt aatttgttgt 240
ataatagaag atattataag tatatttaga agcattgatt attaattctc ttaagcgttc 300
ttatctttta aactgttatc acatttctaa atgtttaata aataaaccag ttgaaaaaaa 360
aaaaaanaaa naaaaaaaaa aaa 383

<210> 1207
<211> 350
<212> DNA
<213> Ctenocephalides felis

<400> 1207

caacgacgac gacggagatg ccaaccacgg aaatactcga ctacaaccac agaagaacca 60
accaccacca cgaagtcaac aacgacaacg agcaatacac caactccagg atttccaaat 120
tgggtgctat aattggcaag tgataagata atatccacta ttcaaataac tattaatgtt 180
aaaaaaaaat tcagtattat aaagaacaca aacaatatta ataataagtt attctttata 240
ttcagtaaca gtattaattc tgnttgttta tgtcgttgta aaaattatac aaataaatta 300
atttggaaat atgaacaaaa aaaaaaaaaa aaaaaaaaaa aaaaaaaaaa 350

<210> 1208
<211> 147
<212> DNA
<213> Ctenocephalides felis

<400> 1208

attaactact tttgatgcat aaaaagctgc agaagagtgc actaatcgat tatgttgagg 60
ccacttcatt aacacctttg ttctaggtat gttatatacc tctggtttgg ctagtcttag 120
aacgcattgt ttgtgtgagg ctttagt 147

<210> 1209
<211> 304
<212> DNA
<213> Ctenocephalides felis

<400> 1209

caacttttaa tgatattcaa cttcaaactc acgtggctaa tattttccaa gttgatagaa 60
gtaaaattaa aataactcat acacctgaaa cttcagtaac tattctctct tgatttcagt 120
attaaaatat aataacaaaa agtgataatg taattttgtt catgagtttt tgctgaaatg 180
atctgaatat ttacctgttt tcgaattacc aaacattgaa aagttaaaaa taaaaaatat 240
ttattacttt aatttatttt agcatatgaa caattcatat tgttaactta taataaataa 300
tagt 304

<210> 1210
<211> 498
<212> DNA
<213> Ctenocephalides felis

<400> 1210

aaaaggagaa gtttctaaag acnattttn ttntacatgt acctagtgag tgtgnggggg 60
ggaaattgct attgcatatt acatacaagc ttaataaagg ttatggtgat ttatttcatt 120
taaaggcaan gtatcggntt aatggcnnta aatntatntt ttatttaata ttaaatagat 180
taattaaaaa tctataaagt gataagaggc ctgatatcca taatattatt gaatattaag 240
aagtgacgga tagatctgat aatgtagtag ttgttataga caatttactt aataattatg 300
agttgcatca ttatgcaatg atttatcact attatttatt caacatttta tttaactgct 360
tgcaactttt aataaaacgc atnttttatt gttttaagta taaatcttat tagggcacaa 420
tatgaaaata aaaataaaga actttataca aaagctnttt tatcaatatg cttcttgcgc 480
tattaagtta agaaattt 498

<210> 1211
<211> 342
<212> DNA
<213> Ctenocephalides felis

<400> 1211

```
caatgacaga aaaacctcgt attgntatca ctaaaaattc aattccaaca agtgatctgg 60
cttatactag gcgccaagaa aaaaaggcga acaatgtaga tattattcgt aagaacagcg 120
acacttcgaa taaaactgac tcgtaagaat tttgtgaaaa gtgtttgtga aagtattatt 180
ttgtcaaaag gacgatttag gctatgtagt gactgtatgg ttaagaacta tattagaact 240
tattgctcat aaatccaaac atacaataat ctttataaaa atcttaataa tttattaaat 300
atttttacat aaaaaataaa aatagtaaaa acgcaaaaaa aa                   342
```

<210> 1212
<211> 487
<212> DNA
<213> Ctenocephalides felis

<400> 1212

```
tgaacttcat tatttaaatt taatcanagn ntatttttta aacaaaacat agtagcggng 60
taatataaaa aatacattta tataacacat taagtttaaa aaataaaaga aagcaaacta 120
caaaacaaag tttaaatttt tcaaaatcaa aaaccgtaga ataagtatca attataagaa 180
tttttttatt gctttttttaa tatttttata taacttacat gtaataatac atttctttat 240
catagaaact ttttatacat gctattatta tctatatgta aaagattgtg caaaaatatt 300
tgagtaaatt ttcaaatcaa aataatacga aacttataat caagcggtta atgtaatttt 360
tttttgtctt tttcgacatt attatcttaa ctgtttttaa gacttagttt gtgtttctca 420
atctccaaaa taatttatca ccatgaaata tctcttatca tatattttta aacttaattc 480
tataggt                                                          487
```

<210> 1213
<211> 236
<212> DNA
<213> Ctenocephalides felis

<400> 1213

```
atctgagata cagcgattag taattgntng ccagctactt aaagagtatg ctgcatantt 60
atttatacgc tatataatta tatcttttat attaaatttt gataaagaag tgaatataaa 120
aaagattaaa tttaattaca agcgtataat tgtatattgt ttgatttaca ccaataaaag 180
canatcagta agttgagttn ctttaaaaaa aaaaaannaa aaaaaaaaaa aaaann    236
```

<210> 1214
<211> 379
<212> DNA
<213> Ctenocephalides felis

<400> 1214

```
gggggttnta agaaacncnn ntttgaccca ccnnttttt anggggcccc ccggccggga 60
cnagttgtat aaaacataaa ttgtaagtnn tcttacaggc acatgctatt gncttattgn 120
atntatttgt tctaccagga aagngcttta tagattttac taaatatata ttaagaaaag 180
cgttctctgt tgaattgtaa ttaatccttt ttgtaagatt tacagcangt atgaagaaat 240
gttaaatttt gttaaatttc atgtattgna tatgatattc gcacgtactt atgaaatgta 300
tgtcagtcaa atgctgaatt tattttaata tacaatcttt gnaatacaac nnanaaaaaa 360
aaaaanaaaa aaaaaaaaa                                              379
```

<210> 1215
<211> 498
<212> DNA
<213> Ctenocephalides felis

<400> 1215

```
ataaacaagc agaaataaat atagctgcta tacatatttc tgcttgttta tgtaccganc 60
agagtggtaa cagaagatac tcanatattt taaatgtagg ttttccaagc ccatccaatg 120
ttcccagcaa atcaaatatt gacccaatca atttaagcta cagtggaaat caaatactta 180
ctacctcaac cagcccccga agcacattta gctatagcaa tgcactccgt ggtcagacta 240
ctcaacgccc ctttaataga aacacaaata taccaaataa tgaccgattt tggcctcaac 300
taccccaaag aggaccacaa tccggtgaca atactcaatt tagttctagc ggtgtttcaa 360
atataaatcc aagtcaaata aatactcaca atcaaaatgt aagaccacag agtgcaaata 420
ataatagacc attcagttac agtagtattg ngggtgcatc caataataat ctgccaatct 480
gtactcctca acaactaa                                               498
```

<210> 1216
<211> 343
<212> DNA
<213> Ctenocephalides felis

<400> 1216

```
caatgacaga aaaacctcgt attgntatca ctaaaaattc aattccaaca agtgatctgg 60
cttatactag gcgccaagaa aaaaaggcga acaatgtaga tattattcgt aagaacagcg 120
acacttcgaa taaaactgac tcgtaagaat tttgtgaaaa gtgtttgtga aagtattatt 180
ttgtcaaaag gacgatttan gctatgtagt gactgtatgg ttaagaacta tattagaact 240
tattgctcat aaatccaaac atacaataat ctttataaaa atacttaata atttattaaa 300
tatttttaca taaaaaataa aaatagtaaa aacgcaaaaa aaa                   343
```

<210> 1217
<211> 498
<212> DNA
<213> Ctenocephalides felis

<400> 1217

catatgtaga aataccaatc aattngntga ttacatcaaa aatatcaaat gttnaattga 60
acttttcttt gaaaaatatc gatgaactaa ctattgattt gccacaaatc atatcgcaaa 120
catttggaaa tgtgcagaaa actgcaaatc ttataccaga aaaagacgaa acaacanttt 180
cagataaaca acaaaatttt gctaatgaag atttgtatga tccagagaag tttgaccaaa 240
tgccaggtcc ttggcccgtt gacctaccat tgcctatgcc ttgttgggga agagaaactg 300
atgcaaagtt agatttttca tcagtaatag aatatgaggc atttgccagc agagtagaaa 360
tgacggaaca gagattattg gaagagacca actatgatac tgtagataac tttttacaat 420
tcagccagga atatagaaaa atgagggatg cttccgaaca aattatgacc tggataaatt 480
cttncgtgct tatcacca 498

<210> 1218
<211> 420
<212> DNA
<213> Ctenocephalides felis

<400> 1218

ccnaanntta atccctgttg atatagcatc acgaaatgct ccccattgga aactgcccac 60
tgngtatact ggctttgctt ctttggttat ctcttcttca gaatttactt ggtctgcaag 120
tagttttatt tgcattacgt ctctgtaagt ttcttttcct tgtaaaacag ccatggcaga 180
ttttgctaaa gcagaaactc gttttattcc actttcagac gctccaggaa acatttcgga 240
tcctacatta ctacatctgc ctaacggcaa aaccccaatg ggacaagttg caccatctgg 300
tcggcgtaac attcctgtaa cagtttcagc taatgttcca tcacctccag caacaagtaa 360
agcgtctggt aatgtaggca tttcctctat aatttttcta gcgcttcctt ctgcagttgt 420

<210> 1219
<211> 183
<212> DNA
<213> Ctenocephalides felis

<400> 1219

caattatcta gtgaaaaaat gtctcgattt tttttgcacg tgactcaata atctagagtc 60
aataggtaaa tagctatttc cactgaccag aaatttcatt tcaattgact tagctcttat 120
ttccgtattt taaacaagtt tcaataaact taagacagtt ttaatatttc tgttttgccg 180
ggt 183

<210> 1220
<211> 500
<212> DNA
<213> Ctenocephalides felis

<400> 1220

```
cctgcaaaat ggtaggactg ctcaatattg ttttattagc ttttgcatct gttaactttt 60
ctttgtaatt ctcaattctc cataccatgt gccccgttcc attaaccggt tcactcaaag 120
ccttatggaa ggcccttgca tcttctgcat cttttatctg ntgtgacaat tgcgccacca 180
atctctcttg tttaaccaat tgccgactta atttatccaa aatatctgta ttatcttgaa 240
ctgtgctttt catagatttc aattcgtata aaaaagaagc tattcgagct ttttgtttgt 300
ccaactcgtc tgccgttttc gtatgatctt cataaaagtt ggaagccact tctcgtctcg 360
tttttgggc atctttttgt aattgtatta ctgattgcgt atgagancca tgtcttgtct 420
aactcattta ctcttgcgaa gtttttatcc tgccgttaat acgacctcga cttgggttct 480
tggtttcttc atctagtatc                                             500
```

<210> 1221
<211> 498
<212> DNA
<213> Ctenocephalides felis

<400> 1221

```
aaactacaaa ctacaaaata attaagatca ttatatgaat cacataattg tataactaaa 60
aactgcattt tatcataaaa attgntataa tttttttta taaataataa ttattattat 120
tatcctgaaa aattttgaga attgacaatt tccatacatt atgccactga atcataccga 180
attctgtcaa aatatcatgt atttgctgtg cattcaggcg ttttacacat aaactgcaac 240
cgtcaatcgt atgttatgaa gctgagtcag aaagaccatg ttatataaca tcgtttttg 300
acgtttgtgt ggtttctggg tttttatgtg ataacttgag aaatcgcctg actgcagagc 360
aaccgcatgg cttttgcaat attcagtatg gaaagntgtc gtatcatagc cttgcataac 420
ataagtctgt tatgcggccg aatcggccat taatattaaa ttttgatgtg atttttagaa 480
taaaatatgc tcagtgtt                                               498
```

<210> 1222
<211> 402
<212> DNA
<213> Ctenocephalides felis

<400> 1222

```
ccgnaagggt acanaaattg ataaagctaa aaaggactta ttcaagaaaa ttactagtca 60
gttaattggg ccgtagtatt agtgagttat tcagaaagga agtaaaaatt gcaaatctcc 120
ctttactcat tgtgccaaag ccagaacaac aagctctcgt tgactcaaca aatgatgtng 180
gattaataaa actgtttgat cgaaagtaaa ttgttataaa aacatctgaa cacataatca 240
atatctattg attaaatttt gtatgaatga gacattagca tttaaagtat ttatctctaa 300
cgcattttct acttaatgta gataactatt ctttgggaac caaatctatt ggttacaata 360
aatatttcta agtgttaaaa aaaaaaaaaa aaaaaaaaaa aa                     402
```

<210> 1223
<211> 207
<212> DNA
<213> Ctenocephalides felis

<400> 1223

aaatgatatt aactttataa ttttttttta taaaaagcca aaattttctc acgtgcgcca 60
aaaattgtgc tacaaaagta aaacatctgg tgcttaacat atttttgttc aagcactaaa 120
aatagtgttt aatttgacct taaccacttt ggtttatatt tagcaaaaat ttattaaaag 180
cgatataaat aatattgata attaagt 207

<210> 1224
<211> 427
<212> DNA
<213> Ctenocephalides felis

<400> 1224

tcatacattg caactgatgt agacatatat tcaagcatac ataaatactc cttcacaaaa 60
taaatacaag atcgatctac taataattgt tacattttaa tatatatcgc tatttactgt 120
ttgtaacaac ttaagataat tcattttctt ctatttgaga aagaaaatga tcaaacattt 180
tgcaaagaat attaaaatat aattatatca cgaaaaagtt ttattcatgg aatcaaaagt 240
tattcaaaat gtcaactatt taaaaactaa attaacaatt acattcaatt ttgatacagt 300
tattaaagta acatcaataa attttgaata cgcatttaca tgaatagttt cttaaatttt 360
gaaatgaaaa ctgacaatta tttaattcag gctaatctta catttgcaat ataataatca 420
ctacaag 427

<210> 1225
<211> 263
<212> DNA
<213> Ctenocephalides felis

<400> 1225

ttcatttcag gcataaattc tgttgttttg tgaccattaa taacatcaac ccctggtaac 60
atatgaccag gcccagtgta gtgccctggt tcatattcaa atttcacagg tgtgctcaca 120
tgtggagcta aaggaggagc cagagatctg agatgatttc cattagagga tggtggactg 180
ttttgttctt ggaagtatct tctggcaaac atatggtgtt tcttctgagc aacaggaggc 240
tggcgcgagg atatcgaggc ggt 263

<210> 1226
<211> 295
<212> DNA
<213> Ctenocephalides felis

<400> 1226

tggttagggc ccncccccgg acaaaccaca antattaaat tagaggagag gtttttttag 60
taaaaaaaaa aaaaantaaa gggcaagggt gaaaggtggg atgcactttc ttaaataaan 120
nnactgntaa ataaagntta tatttatatt aaattacaat taggactaca taatgcactt 180
taaaaattat ggcatagtta agacttcatt agaaatacaa taagctacaa ttataaattg 240
gattatattt nggattttta tcataaaaga ataaaatcat tcagaatgca aaaaa 295

<210> 1227
<211> 335
<212> DNA

<213> Ctenocephalides felis

<400> 1227

```
ctggcacttc cccaataaaa aacgccannn aaacctaaaa ataggnaatt aaataagact 60
gggnagcagt gatgatagcg gagctgtgtc tcctgataca actacaacaa tttctccttt 120
aaagaatgct gcagataaaa ctgngaagac taattaattt taatgccaga taaacttgat 180
ttttgatata catatgttgg tatgtagtta tgaatttaat atactttaat taaacccatt 240
ccaaaaatca taattcatat tttttaaatt tgatttaaga aatcttaatg tgtaacttta 300
ttttgttttt atttagagct gnaaagtttt attgn                            335
```

<210> 1228
<211> 225
<212> DNA
<213> Ctenocephalides felis

<400> 1228

```
caaagggttn aaaaggtccg gnaacaatcg tttttttttc naaaataaaa ggantgtnaa 60
ngggnggggn tattgggang aaaaaaagta naagaacccc aanntattgg ccccgcaaat 120
aatgcatgtg aataccttat ntcataaatg gcgggnctat gggctttggg cacaaaaatg 180
gctatcaagn tacatgtcct gnaacaagnc taaggngctt tcgna                 225
```

<210> 1229
<211> 435
<212> DNA
<213> Ctenocephalides felis

<400> 1229

```
tctttaaaan agagttnggg ttaaattcat tggactnttc aagaaatgca caggcttgng 60
ggggnggtga aaaccagcgg taaacttgga aaaaaatatt ttggatacaa agttaaacgt 120
gctgatcctg aattaaaaaa aggntgngat aaaacctgtc ttaaaaatca tatgtgtgca 180

atagttacca cagntgtatc agatctcatc cagtgcaaaa atatcatcaa atcatcatca 240
agcattcttc agcatttgaa tatatatgtc ctaggagcta ttgtaatttc caattattta 300
ttattataaa gtcatttttg ttaagttatt ataaatttag aataaatcat tatgttggaa 360
atatgtaaac gctttgaaaa agctaggatc aaatcaaata ttttttgaaa aaaaaaaann 420
naaaaaaaaa aaaaa                                                  435
```

<210> 1230
<211> 282
<212> DNA
<213> Ctenocephalides felis

<400> 1230

ttttatatta catagatntt ttcttttatg tttttgtaag ctttgaaaaa acaattngtt 60
tttcatatat attttataca caagaatgtc ctgtatgctt attttttaa tccaacgaaa 120
taaaagaaca aataaacgta aaaaataact acattatgaa ccccaataaa atataatact 180
cctgtgtaac gcgggatgtc gngttcccat tgtaatttta tgtaaatatg tataatttga 240
aagctcgtat attgcttgtt ctattttctt gtatataaat gt 282

<210> 1231
<211> 101
<212> DNA
<213> Ctenocephalides felis

<400> 1231

cattcaaaag atatcattta aatttattgg gaaattaact ataataataa taattgattt 60
atttcttaaa gctattaacc cattattttc aaattgcatg t 101

<210> 1232
<211> 224
<212> DNA
<213> Ctenocephalides felis

<400> 1232

ggttgaaccg ttcaagtatt ttataagtga tgtctctgat tgtgtatttt tcaaggcact 60
tgattttatg ttcattgttt taatatcaat gtgccttctc ggtagtatat aaattttaca 120
tagtaaatcc tgtttttgat tttatcgcac attttttgtg ttgttttaaa ttaattttac 180
aagagaccat cacttataag ctgtaatttt actaaaacta aagt 224

<210> 1233
<211> 347
<212> DNA
<213> Ctenocephalides felis

<400> 1233

caactgttga agatgagaag aaaccagtta ccaacggaac tttaccaaag attgaagaaa 60
agtgtgacag cgttgcagac cttaatggat ccataaataa atctttagat catctaaagc 120
caccagaagt agcagttgct gatatcaaca cctgtattcc agaaaagaat aaagactttt 180
tatgtggtat cagagaaagt cagctcttan atcatgaact gcagtgggac tccatggaag 240
acaaggaatc caggcagaac gatgatgcag agtcggacca tagcgaagaa gacgacttgg 300
gtgaattgcc ggtaagaacc gtattggatc agtatgcacc tctaggt 347

<210> 1234
<211> 498
<212> DNA
<213> Ctenocephalides felis

<400> 1234

tataactata ataatggggc aattctgctt cataacacat agttgtcatg gcaacgcgta 60
gtgaagactt cgaaaacgtt aagctatttg ccgtgcgatc aggcgtnttt tacatataaa 120
catagcaaga aagaatgtta tgaaaacagc atcataagat tcttctttgc ttgtttatat 180
gtaaaataaa tgtagacatt ttnatattgc catgacaacg ttatattatg agctctgtta 240
cgccagaatt gccccataag tttaaatatt cttcaatnat ctttctccat tggtngaaaa 300
atattttatt tacagtnnta gcacagtctt gtgaatctat ttgcgaattc tacattttaa 360
taataaatat aaaaacanct gaccgccatc cgcatactag aagaaacatt ccaatctgat 420
acctcattcc atttgntcaa aatnaaaaat aaaattacag ncgactgatt tattcctggg 480
tgcttaaaaa aaatccnt 498

<210> 1235
<211> 337
<212> DNA
<213> Ctenocephalides felis

<400> 1235

ttttataagc agaaccacca ggtgaaagat aaacgttcca ggatcctgca ttattgaaaa 60
ctttcagaaa attaccagat aatttatttt gtatacaaaa cgngtctgta tatgcagcag 120
cctggatgga agatggtaac ttttgtatta gtttggnaac ttntttcttt tcatntggnt 180
gaaaattgga tcgatnatac acttttttga cgcgttgcac aaaagtgaaa aaatcatcgt 240
aattattggt tngcaataaa tcattcacta caaataaccc atgaatgctc agtggtnagt 300
tactgntgna actaacaaaa tctatgagag aaagtgt 337

<210> 1236
<211> 351
<212> DNA
<213> Ctenocephalides felis

<400> 1236

taacccctta nggattccct tccgngccng aannnggtta acgnggncgg cgggccgagg 60
gtccatccta gggcgaaaat ttttaatata gaaataaaat gaggtcgcct tctttttgaa 120
aacccatttt gttctttgnt aagcagnatc ggcaggtgaa acataaacct gncaggatcc 180

tttataatta actttcaaat aattatcaga ctctttattt cgtatacaaa atgtgtnccg 240
tataagcagc agcctgaaca gaagaaggna aacttttgta ttagtttggt aactttttgc 300
ttttcatcag ggtgaagatt tgaatattna tatgttcctc tgacatgtcg n 351

<210> 1237
<211> 156
<212> DNA
<213> Ctenocephalides felis

<400> 1237

```
aaagccagcg atagnataga actgctatgt ttncctccaa ataccactgt tgttttatca 60
tntaaaaatg ccataagtat ggagctaatg catccaatca ccatcaatat atatatatat 120
gaagagtctt ttatccttcc tgggtaaagn ttctgt                           156
```

<210> 1238
<211> 347
<212> DNA
<213> Ctenocephalides felis

<400> 1238

```
ttncttnttt tttntntttc tttttnttta ttcngnataa ggggggnata attcatagnt 60
tatcttacaa attacaaaca tgaagtcaag ccgnttgatc caatatgaan attggtggat 120
ataaatatcg atgatccaaa tacaagtctg ntgntcaaac cttcaagnga tataatacaa 180
aaattatgct ggaatgcttt gggtaggaac tcttatcccg attaaaatga cgagataaaa 240
ataaaaacag caaatatgcn taaaatgagg atgatgtaga tgaaataaag atgnnnatat 300
taattaatga tgttaatgaa cacttttctt agaagcactt cctgtgt              347
```

<210> 1239
<211> 598
<212> DNA
<213> Ctenocephalides felis

<400> 1239

```
tataatataa ngtntttagt attaaatggn ttgagaaact aatttagtta agttgtatac 60
antaaantat aatcgngnga nantatgttt atatttacaa ancctcgaga tggagnttaa 120
ngttagatga atatattaaa atgtatgntc taacaatngt aatattaatg tnaattttaa 180
cccggaaatt aaggaaaaca tngctttgat gactacttag cttccgatcc agaggtgatt 240
tagacattta gttagcattt taaaatattg tgtagctaac atttcgngtg attttgcaac 300
taatcagtta tttaagaaat gtgcatataa ttattatata catattatnt tacatacctg 360
gntagnattc aaaaaacttg ncaattactt cgccaaaaga acattggggg gataataata 420
tcatattatg caacattctt tattacgggn agtttacttg aaggatctgg tttcttgctn 480
tttttcattt gatcagaaaa taatcaaatc tacagtattt gggtagcagt ntncgaaaat 540
taacnggntt accatattaa aaaatttnta taattcanna cccggtttgg gaaaaaan   598
```

<210> 1240
<211> 446
<212> DNA
<213> Ctenocephalides felis

<400> 1240

ttgaaatttt atgtgtnatt atatttatta tgttagtaaa caagtgaaac cgctgctata 60
aagnattgtt taatctgaat gtttatatat attgtatttt caagattatc ggcactatgg 120
tatttcttac aacattcata ctatacaatt ataaaaatat atgcantttt tgnntgnnta 180
catattggng gataacccac aaataatatg aaatggaaaa aaaaatgtaa accggngaca 240
caaaatactg gagacaatta ttataacang ggatattcaa cttttaaant ttcatcaata 300
gntttttatg anctcgcttt tgaaggattc attcatttat tcaatccaat gncnaatcat 360
aaatttattg gnntttaggg gcactactat attggattaa taaganaaac aacttngtta 420
tgaaaaaatt caantaaaac catgnn 446

<210> 1241
<211> 274
<212> DNA
<213> Ctenocephalides felis

<400> 1241

tttaagtcta aaagaaatcc aagctttttt atctcaaagt atcaattttg attttaaggt 60
gatttaatta ttttatataa ttatatataa agaaactgtt ttacaatcag tgaatatttc 120
cacaaaatta ttaaaactat ataaatataa tacaagtcca tcataggtat gactactttt 180
gacgaaatat tcataatttt gaccaactac cctctttcat aattttgacc atattatcaa 240
gttaacccaa acttatctta aatatgcgat atgt 274

<210> 1242
<211> 102
<212> DNA
<213> Ctenocephalides felis

<400> 1242

tttggatcca ttcattttat ttacaactta ctatgtaatg taaattaaat gtgaatgnga 60
ttattataat aagtgaaaat gtttgttatt caatcacatt gt 102

<210> 1243
<211> 193
<212> DNA
<213> Ctenocephalides felis

<400> 1243

ttcaaaatat acaaattcgt atatgttatt tattntatat caccaattta tacataagca 60
tgggnaaaat tcttacagaa ttactgaaac ataagataaa atatgttcaa aaatatgatt 120
gacgtatcac tggcagcttt gtaatattaa tattgaacga tatgccaatt gcaattgcga 180

aatatattat agt 193

<210> 1244
<211> 498
<212> DNA
<213> Ctenocephalides felis

<400> 1244

```
atcgaaatag tcttcagcat catcgactgt naccacacaa ggtggtgctg atatgttngt 60
cagcgttttg agtaatccca aaagtaaaac cgctgataat gtaactgcta tgatcaattg 120
gagagatcta tcttgatata agtttcctac taaccaagca cctcgccatg aattcacaca 180
tataaatcca taaacagcgg tgtagaccca cgaagatagt gcaaaacatg tgttagattt 240
accttctaaa tatcttttga tcggtccttg tgccaagcaa aatataaaca tcccaataaa 300
gcctatggcc aaagacacga aactgctata tatttcatta tctgtataaa cataatgtcc 360
cattagattc cacgtgcctc tccaataccc gactacggcg ggcgcaacca gtgttagagc 420
aaatagagca tccaaaacat caagaataac tgatgatttc aaaggattca tagtcatcaa 480
tgatcaccgg aaggtctc                                               498
```

<210> 1245
<211> 112
<212> DNA
<213> Ctenocephalides felis

<400> 1245

```
tttttttttt tttttttat aaaataatct tttattccaa acgtttcgac attcaatttn 60
ggngtcatca tcagtggata ttgaacatag tattttatat ctaaaaatgt gt         112
```

<210> 1246
<211> 379
<212> DNA
<213> Ctenocephalides felis

<400> 1246

```
aaccgttggc aaatatcaac ttccacatca tcancaaatg ttttatccca atcaggcgat 60
gaatccaaat gattgaattc attacactgt tcaggctgag tgtaaaataa tgatgttgga 120
ctttgaggcg gagtaaaatg atttagttcc actttatcat aaatgcgttc aaattctcgt 180
aaaagactct cagtgtcttg ttgaataaaa tctgtggtat caaattgctg aggaaatttg 240
gtttctccgc ttagagtttt gttcatacca gttgtagtag acaaatcctc cagcagaggc 300
aaatccactt tctcctccag ccattgagaa aatgcttcat tagtaaagtt atcttccagc 360
aagcacgaat cagcaaagt                                               379
```

<210> 1247
<211> 230
<212> DNA
<213> Ctenocephalides felis

<400> 1247

```
ccttgctgaa actnctcctg atatgggcga tttttttatt aaagcaagcg aaaaaaaatn 60
nggnaatgaa tgnctcagta atcttaatgc cgaagcacaa gaaaaatttg ccaatagagt 120
caacattata atcaaacaag ccaaaagtaa agggtttgtt taacataata caaaatagca 180
attagaaggt ttagaaaggn ttaataataa ctaattttgt acctcggacg           230
```

<210> 1248

<211> 498
<212> DNA
<213> Ctenocephalides felis

<400> 1248

```
catctattaa ttccagtatg gtgcttnctt gcaacaacat cctggatagg aagtcttctg 60
attcaacctc cgcgtctaaa ttcataagtc cactttttcc atctttagga ttagttaaat 120
tcgataacaa gatttttaaa aacatctgta attcaagtaa atcactaata aaaaaatctc 180
tatgtgctgg ctcgtcaaga atacttaagg cttcaaaccc aacagcaacc gatgattggt 240
caatttcatt agcatcatct ccagcaataa natcaatctt gaactccttt tcttctattt 300
tatcattaaa atatgcaaac ataatttctt cttcagtttg actatcatca aatgccactt 360
catagggcag cttatctaaa acaacacgct tcggttcctt gcagtgtatg aattgataaa 420
cggtcgtgtt accaaattca agaatatgtc taacaaccgt ctagtcctac ctttacattt 480
cttttatttt tgaatcct                                               498
```

<210> 1249
<211> 290
<212> DNA
<213> Ctenocephalides felis

<400> 1249

```
agtatcagta ggagtgttat gtgcaaattt ntaaatcgng tatataaaat atatatggta 60
tagattaaat gaatcagaac aatcttaaga gccacaaggt gtatggcatc tagaggatac 120
cgagaggata ctaaaacgaa atgatagatt taaaaagtgt ttccacctta tatgtcttca 180
aaatatgtaa ataaaaacat cttgaataaa ataatttctc aatgttngtc tgaaattgaa 240
agttgtgatt aaagttatag ttactagatg aaaaaaaaaa aaaaaaaaaa          290
```

<210> 1250
<211> 345
<212> DNA
<213> Ctenocephalides felis

<400> 1250

```
ttaaataaga caaagaaatt cactccacca nttccctata ctatcccttt aaagcccttt 60
ngncacttca acgtccacat agntttccac agatggcgcc acatagaaat cattgctttt 120
ttataatcgn gttcgaaaaa atcttatctt attgttagtc tattaattaa gagttaagtt 180

tttgttaact tgtcaacttg ttttcggttg ttaatattta aataaacaaa tgaatgtaaa 240
agtgactgac ttgtcgattc aaattgttat tacaaacaaa tcagtcattt aactttgaac 300
ccagtcaatt gttaaaatac atattataaa cacatacacg taagt                345
```

<210> 1251
<211> 498
<212> DNA
<213> Ctenocephalides felis

<400> 1251

ttaaaacaaa gaaaattacn ttcanatatt tttttatnct tagttcagng tgtgcatata 60
ttggttattt agaaatcttt ataaacaata ggacacttac atttcaaaan taggtgttac 120
gaataaatta tnatctcttt cactacatat attacaatca acatttatta aatangngtt 180
gantttattt attgtgcaaa taataatatg ctttccatgn tctgtaatca actgattaan 240
ttacttgtat nangcagtng ttttacctac acattttagc tactggtaag ctaggcataa 300
cgtgaaangt atatcttanc tcgttttgac atcaaaagcc ttttcanctt aatgnaaaga 360
atggaaattt atgtaataaa ntangagcac cctttccaat cattggtgat gccctangnt 420
atgccctatg anaatgttgt catcataacn nttntganat aatccgantt tatatgaatc 480
ttntatatgt gcgaatat 498

<210> 1252
<211> 258
<212> DNA
<213> Ctenocephalides felis

<400> 1252

tcttccatca tggagaacat ttggggtaac cntaatctta aagtattcaa ccccaataaa 60
ggcagaagtt gtatagcaca cacnatgatg cantttttca aatgtggtgc ataaaataaa 120
ggcttgaatt gtttgaaacc agctttaaga gcttccattc cttcaggttt gggagaacca 180
tcttgtncat tggctttggt ttctttcacc agtngtttta ttggatattc agtcttgtna 240
ttccaatgat taatcctg 258

<210> 1253
<211> 364
<212> DNA
<213> Ctenocephalides felis

<400> 1253

tttttttttt ttgttcattt tgtntgaact taagnatata tnttnaggga aatttcaatt 60
taaatnatcc gcnatatatc atacatgtaa ggcaataata cataatttaa ttaacgcatt 120
aaatttaact atgtaaatta tgtanngntg atttatctac aatgttttta acataatata 180
tttaaaaatt catggaaaaa tntatctaaa nagctaaata tantaaaatg gaaagcctgc 240
gctntgcttt agcatattta taaatagcat ataataattn ataatanatn ctntanttag 300
ganatcattg ataatcattt attcttgtat tacgnnaata taatagtaaa ttacaaatgt 360
tngg 364

<210> 1254
<211> 318
<212> DNA
<213> Ctenocephalides felis

<400> 1254

cttaaaaaat cgccccacaa tagttaaagg canttggacg atagcaacta aataacataa 60
anggtattat ataaagattc caatcaatta tcatcgtcac cgtcgtcgtc agccacaaag 120
aagaaaacca accagtgact gatgatccaa cacgatgatg atgatgatga tgatgatatg 180
tatatatgta atgttttacg gtgttaattt actattatcg gacgaatcaa gattttattt 240
attaagatat gggaaggtag tcacaaatat tttttttntt aaatngatng tnncccattc 300
ccaaaccaaa ccaaacca 318

<210> 1255
<211> 312
<212> DNA
<213> Ctenocephalides felis

<400> 1255

gtatttgtta aataaaaata agtcacatct tanttctgcc aataatatta aaaatgcaac 60
atntatccag atgcaactaa ctgaatatca ctttcaacaa ctcaagacgt acagatgtta 120
cgataaaaaa acagcccaca ataaatcctt gttgcatatt ataaaataga tgcttccaca 180
ttggtaataa ggctaaataa aatccaacat cacccagacc aggatatgat ttgaatatag 240
caattaaagc tgcgagactc gttgctaata acataggctc atgtttgaga cgaagagaga 300
gcggaagcaa gt 312

<210> 1256
<211> 135
<212> DNA
<213> Ctenocephalides felis

<400> 1256

aaaaaaagtt atactaaaaa aaatttactt gactgagcac ttagtacaaa aaagttatac 60
taaaaaaatt tactcgactg agcacttagt acaaaaaagt tatactaaaa aaatttactc 120
gactgagcac ttagt 135

<210> 1257
<211> 500
<212> DNA
<213> Ctenocephalides felis

<400> 1257

tcaaaaagcc cttatacgat tcgtatacac nttctttaaa tgttcgctat atatttatnt 60

```
tccagtaatg gttttaataa aattatgccg atctacattt tctaaacatc ggcaaaatta 120
aatcacatta taatgttaac ctttcaaaaa aatactgtcc aaaaagtatc acactttgat 180
ctacaaatgc aaaatggttg aaatcaagtg aagcaatgtc atttatacat caaaagccaa 240
tttagagtca cttgtgttca cccactttgt ttataacatc gtcaatcaag gcggataaca 300
acagtgttta ttgtaaataa attatttcta ataaattgac aaaccatcgg gtaacaaaac 360
aaaaaagtta atactgcttt tcagctttgt gatggttaat ggttatgctc gaataaagca 420
ttgactgtgc catcaagaaa gtatactngt ttccattagt atttcgattt attccaattc 480
caatcatatt atcacaaata                                             500
```

<210> 1258
<211> 485
<212> DNA
<213> Ctenocephalides felis

<400> 1258

```
tacttttact ttgtaaaatg taaataatag gantcactga gacttaaatg atccattggt 60
attcatacaa gttaatgata tctgtgaggt atttaacgag caaaattatt ttgattctta 120
agaagtatga tgtttatagc cttcttaaga agttatgata ctgttatagc cttcacactt 180
tgagaaagat aatagaaatt gttctcacat aaatattact cattagcagt ttaacttaaa 240
aatgaaattc ttattcaaat ttgaataaaa aaaagttgca tcaaagtatc acagtataca 300
acaatattag aatcgatatc ataaattgtg ccaacattct gtgttgccac tgatatcatt 360
attcatttga aatatcaatg gaattatcaa ctgaatagag aagtatttgg ctgagcttaa 420
tataatacta atagcgagat ctgaaattaa caaagttgaa aactaaatag tattatttta 480
ttagt                                                             485
```

<210> 1259
<211> 232
<212> DNA
<213> Ctenocephalides felis

<400> 1259

```
tgtaaacagc actttgcctt tgtaggtttt atcgaaggct tcgctcaagg ttttgatggc 60
tttggtgagt tcagttttgg ctttgtcggt ttcttcgggg gatttgaaat ttgttgctaa 120
gcggaagaag aataagctag gacgttcatc aggggttagt tctaagactt tgtcagataa 180
aagcgttgag gacaccaggt ctttcacaaa cttctctggt aaatcacttt gt         232
```

<210> 1260
<211> 371
<212> DNA
<213> Ctenocephalides felis

<400> 1260

```
tactttataa aaaaaaaaat caaatctaga ccaaccaatg atgagagtat agcgcagcac 60
cattcaatgg taaaatttat attttagaaa aaattactta aagaataata ttgtattgct 120
cttcagattt atcttcagaa atctaaatat attttgaaaa tagcattgcc aaaacattac 180
```

tagccaacta tttcaatagt cagaaatgct attttcaaat atttaagcaa tatgcaagtt 240
atttttttgg aactgataag ttcactcagt caatttcccg atttataagg tttcaatatc 300
atttgttaac ttcacttaac ttttaatctg tatatgaata ttctgcatat ctttctaatc 360
atctttgaag t 371

<210> 1261
<211> 401
<212> DNA
<213> Ctenocephalides felis

<400> 1261

tatctgtttg atgaattact agttctgctt tgttctctac tggttccttc aaatttgaat 60
atgtcaaatt tggtgtanat cctagtgtgt tattacttat tccttgattg tttgaaatct 120
caataatttc gattttatcc aaacttttgt cacagtcaaa atctggtaaa atctcttcat 180
aacgtttgtg aacataagaa tcatgatcat cttttggttt ataattaata atcgttcttt 240
ggtttatttc cttgtaagca aaattatttt ttacaggcgg cgtttcgacg tcgttatatt 300
ttgcattgct taaaaagctt gtatcgacaa aatccggaat tgaatgagaa tttaacgttg 360
cgttgtgaga tgaatttaca aaaggttgat acacactttg t 401

<210> 1262
<211> 498
<212> DNA
<213> Ctenocephalides felis

<400> 1262

catcaccgag aagaactctt cggctacgga ntntcttcat tggaggcgcg ttgttggtgc 60
ncgcagcatt acttttggat ttagaaataa attaattttg gattaagaaa aaaaaaatta 120
aacataaata taacgatctc tctacaaacc catataagtn ttcattttga ttcttttagc 180
ctttaacttt attttatatg aaaaactcat atttcttcct ttgcatcgga ggctttacat 240
ttagataaaa gaaaacagat tatttgactt atttaaagaa ataattatan ttcagaaaaa 300
gaatatttat tacttttatt atattcatta ccgtttatta tgattggttt taccgagcca 360
aactgataat aacatgcatg tatatgctta tatagtcgca cagtttttta acattatcta 420
ttatttagtc gttaaatttt aataacattt atttatttat ataatttagc gccgcaatct 480
ctaatcaaaa ttacgtga 498

<210> 1263
<211> 452
<212> DNA
<213> Ctenocephalides felis

<400> 1263

ggcacgaata tttgtcttca taatcattac ccgctcttgt atattgagtt ttagggatca 60
acttcttcac ataattctca tttataaaac gttggaacaa aactccaaat gttgttttcg 120
ctttcgaatg tcgaaatagt gcataaaatt catcgaagtc caaaaaccgt gaatgattag 180
agtcacccaa actcataaga tactgagtcg cagattctgg aaactcatta ttatattcac 240

ttctctctac aattctttgt aattctctga cagatatcaa gttatcatta tctgtgtcat 300
actttcaaaa tacaattaat tattgttaac gtgcatacat aaactttgat ttattataat 360
tttaccttcc cgaataggta tctgatatat tgatcagttt ccctcaacgg tatattatat 420
tccatctcta attgtgatat ccttctctga gt 452

<210> 1264
<211> 490
<212> DNA
<213> Ctenocephalides felis

<400> 1264

tttttttttt tttttttttt ttttttttctt tactagatgt ctttattcct aatcccaaaa 60
tattatatgt cgagngtctt cttttatcag aaggatcgac atccttcaat gcacttttct 120
ttccaaaaat gcttaatctt ttagttctta atttttcaga gttatgagaa tcatttaagt 180
cactgttgat tttttggttg atttcattaa tactggctaa agatctgcga cgcatagaat 240
ccttttcggt atcatttagc aaatgaatgc catttggaga ttcgttaata tacaaagtag 300
ccattgtttt ggctttgaaa tatcacttat tgttatatat cacttattag tttacaataa 360
cactggtaaa acatagcatt ataagttttt ggttcacatt ttcactcaca aacacgcggt 420
cagtatccgc gcgagtattg tgaaagcaac agacgaaaca ctgcggttaa atgtcacacg 480
gtgttttcgt 490

<210> 1265
<211> 498
<212> DNA
<213> Ctenocephalides felis

<400> 1265

tctagagata ttttagttgc agttctgact gtgattattt taatatgtct tactttacct 60
gctgcattta taatttacaa atatggcata tacgactgga tttgtagaaa agttaatggt 120
aacagaatgc tacctcgata tgaggatgtg atgatagggc aagaagatac tgacgatgat 180
cccttgccat aagtgaatat tataatttta tattttcttg atctccttac aatgatgtag 240
aatattgcct tcaagtcata tgttgtaaat agttttacaa aatgataatg ttatttatct 300
attgaatata tgtatgttta tgtaatttat ttaaatatta atttattaac agatgatgac 360
tagaagtcac aattgtgtta ttgaagttgt tgcatgtaaa tgaattattt ttaaatagat 420
atattcaaat aacatgaaat aaattttgta tttgtgattg gtgaaagtga tggaaatatt 480
ttgttgaata caagcata 498

<210> 1266
<211> 311
<212> DNA
<213> Ctenocephalides felis

<400> 1266

aacaattctt tgttttaata tcgatatata atcacgattt tagtttatga tatctttatc 60
tgggctcatt gtgaagattt cataccaaaa tcgatattaa aatattgata acttcaataa 120

taactgtaat ttccatagat atttgcaaga taatcgataa atctatcaat attatcgata 180
aatatatttt cccccttta gtcgaaagta ttatatagat tctattaaca caaaaacaca 240
cagacacaca aattagttta agtttagacg acattccggt gaaaaaaaaa aaaaaaaaaa 300
aaaaaaaaaa n 311

<210> 1267
<211> 453
<212> DNA
<213> Ctenocephalides felis

<400> 1267

ggccccgtac cggaagtgtg agctggcaca tttgacttaa cttcattttt atcttcatac 60
tcaattatat tttcntttac catttctcca gcagaaggtg tcacattctg tgtttctaaa 120
tttgtatttt gataatcatc actaaatcta tttcgggatc gagaccgcga tctgcttctt 180
ctacctctta attctttcct tgtaacaaac tccaaaaatc cttcagaatc tggcatcaca 240
acaggttctt caggttgtga atccgcttct tgcacgcata attggaaaat tggttgtgtt 300
ttgggtgttc cagctgaatg tttttcaaaa atattcgatt ccgtgatttg tggcacttga 360
gaaggcaatt cgctattagc tttgatacca acaatagacg cccatgatgt cagttttggt 420
tggtcaagta gactcacata gcggttgttc agt 453

<210> 1268
<211> 498
<212> DNA
<213> Ctenocephalides felis

<400> 1268

caaaggggca agcaccacct gggaaatttn naaatgcaga attgatgaaa attccaaatt 60
tcttgcactt aacaccgcct gttataaaaa gacagtgtga ggcgttaaaa cagttttgca 120
cacagtggcc aaaaggttta gaatctgaag aaaaacaaaa taaacatttt cctgttacag 180
taattagttc tgattattgt cacagcggtc cgactataag aaatccatta ggtagaattg 240
ttactttgaa ggtcaaatta tcggatttac cactagacaa gcatgctcgg gataagtttt 300
tacggttagt tggtgacaga cacgatcctg atacagatat attaacatta gtagttgata 360
gatgtccact gaggaaacaa aattatgact atggcatgta tttgctgaca gctttgttcc 420
atgaatcttg ggtcactgag ccttgggaag cagataaatc tgaggcggat atggaatatt 480
acgattggca aaataatg 498

<210> 1269
<211> 285
<212> DNA
<213> Ctenocephalides felis

<400> 1269

tgctgctttc tgctagtgag gtatttgctg tttctaatgg agtatttgtt gaagtattnt 60
ggcttaaggg ttgtgattcg gtgaggtttc cattgctttc attcacgttt gaacttggag 120
cagttgtgga tgggcttatt tcatcattcg ttgactgtgg ttggtcgtct ttaacgtcac 180

```
tgtctgggaa agcaatagta aaagtatgat tatgagtgaa tatttatttg ctcgactatt 240
attattatct tactaagcaa acgcctgggc ccagggtcag tctgt                 285
```

<210> 1270
<211> 498
<212> DNA
<213> Ctenocephalides felis

<400> 1270

```
tagagccttt ccaaaaagtt gttactgagg aagatttgat aatgcgccta gaaatagtag 60
cttgtgattc aacttgtgca aatgtaagac catgtgagct ggctcttgtt ttattatgca 120
cccaacttga tagctgtgtg tctcaattgg aatctcatac tgctcaaacg atgcttaaat 180
tggttgattt tgctattcac atgcagaaac agtgcaggat tcctgactcc agcttcttca 240
gttgtcacgg ttgtgttgtc aacatactgt cgaggtataa caatcaagat aaaagtccac 300
acaggcaaag acttgtttgg aaattatctt cgcgcacatt gaaacttctt cgaccaacag 360
atcgtcttac atcacttctg cccactattg atgaaaatgg atgtctacca aggcttagaa 420
ctggcagtgt gagttctgtg ggcagtgaag atactgaaga ttggcccacc agtcctcttg 480
gtcccgttgt gagcaatg                                               498
```

<210> 1271
<211> 500
<212> DNA
<213> Ctenocephalides felis

<400> 1271

```
ccaancggtt ncanttggnn ntnnnccnat ttnnaattgn angggcccgg gncntagggg 60
gtaantttaa ttggnatttg ggcnttncat gacngngcan tnagcttcnt gacnanttct 120
ttttacacat atttttanaa tgatcatata catanttagg tgggcattcn aaaacctgac 180
ctttaccatc tttgcagaan aagnatnttn tgcnntcttt tcgnatctgg ctgatatcct 240
acaaccgccg catgtanntt ctgccgtctt acagctttcc ccaggttttt gatgagcaca 300
tattattcta cacaaaatgg gaaccatttg gaaatttgan tccttcttca aattgattgt 360
angaaatttg ggggtttctt tgcncnaccc cacaaacttt aaaagnaaaa acaanctnna 420
naaaaaagnt cccnacntgg ttattnaaat ncnttgggcc cgtaccnccc ttaaacccaa 480
ttttgtnata ntcnnnanaa                                             500
```

<210> 1272
<211> 157
<212> DNA
<213> Ctenocephalides felis

<400> 1272

```
tttttttttt tttttttttt tttttttnga tnggtngaac tttgtcactg antataatgt 60
cttttgtcac ttttattana ccaatatcag catagttgac agtaattttt ggngtatatt 120
tatgatgcat cacaaaacgt tcgacgtcgt agtatgt                          157
```

<210> 1273
<211> 235

<212> DNA
<213> Ctenocephalides felis

<400> 1273

```
tttttttttt ttagttttaa taattattgt agatcaacat gttatattac tttgcatagn 60
gngatatcgg ttactaaata ccagtatgtt tcttcacaaa ttctcttatt tctggatcag 120
ctagtcttgt aaatacattt ggatatggaa aacttgtgca atttctagtt gaaaaagctg 180
tcaagcctac taaaacccca ttttcgtcaa cgacgggtcc accaaaatca cctgt      235
```

<210> 1274
<211> 400
<212> DNA
<213> Ctenocephalides felis

<400> 1274

```
acatcaagca taattttcaa aaatggtcca agaaaccgaa tgcaaattaa atggatcctc 60
aaaccccgag gaaactaatg tgattcacgg tccagccgcg cctgccaacg aaggtttgag 120
gggctttgga gaggaaattt atgacagaat agttttacat ggcgatagag tatccatgat 180
cgacggagac accgatcgct ccaccacctt cgacgccctt cgggcacgca gtgcagcaat 240
cgctctagcc ctaagggctc gtggagtgac caacaaagac gtcataactt tggtagaact 300
caccacagaa gactcttttg ccattgtttt ggggataatt ttctcaggag ccactttagg 360
gatgttggat ggagcctgga cagtcccaaa cactgctagt                       400
```

<210> 1275
<211> 343
<212> DNA
<213> Ctenocephalides felis

<400> 1275

```
ccaggatata atttgtgaga taattataaa ctattgtaaa taaaacataa tttaattatt 60
ggaaaaaata tttgttaaat atttaggata agattgctca agtcataccc aggatataat 120
ttgtgagata attataaatt atattatctg ctaccattaa acttaagtta ttgttaaaca 180
aaactaataa attgtgtaag caaacgtttt taattggtag actggtcaat tttatgaaac 240
aaaaataatt aaaagtttgg taaagtttct gctaattttt tttgctcaat tgtaagcatg 300
ttaagtaagg taggtgtctg ttttaataaa aaaatgaaat tgt                   343
```

<210> 1276
<211> 204
<212> DNA
<213> Ctenocephalides felis

<400> 1276

```
ttacgaattg attttagagc attcaaatcg gatgctctgt tctctcaaaa acttaatttt 60
tattttctgc actctttgct tcgtatcttc gaaatagctg gatcggcctt tgccaaaaac 120
taatcagcac atctccctat caataggaat cgaatttttt tttgaaccat tcaaattggt 180
tgatccgtgc gtccgaaaac gtgt                                        204
```

<210> 1277
<211> 150
<212> DNA
<213> Ctenocephalides felis

<400> 1277

```
ttctatttgt tgggttattt tatttcagct atgaattata tatctttatt atttaattaa 60
gttttttaat aatctctttg aaaatattga taaaatgtaa tagaaaatac aaaaattttt 120
tgaaatattt agcttcataa aaaattccgt                                  150
```

<210> 1278
<211> 337
<212> DNA
<213> Ctenocephalides felis

<400> 1278

```
ccnnttcgaa agtgttaact tatcggatga gcnnaagctt tactaattga attgattgct 60
agagcttgna aaaatattgc agcagaaata aattctctta aagcgttagc ttacatgttg 120
aagttcanta cctctcgata acaaaagaaa atgcnnacta cgttaaaant attcaatttt 180
tttaaaaaaa nntgggttgt ttgctttctg anntgtgctc gttgggggcc cccctggcgt 240
gggggccccg tatttttgat acngctgata cggnggtaag ttacgcccct gatctgcatt 300
natatacaaa ttttaattta aaaaaaaaaa aaaaaaa                          337
```

<210> 1279
<211> 549
<212> DNA
<213> Ctenocephalides felis

<400> 1279

```
aatttatagt tgtgtaaaat attttttaa caatcggttc aggaattaat gtaacgttca 60
ataactaata cgctgacaag cggatcgatt taaaaaacgc ttgggcacat atgatttttg 120
gattctagga atcaaaataa gatactttgc tcaagaaacc atacgtntaa agtgaattct 180
ggttaatgaa acgttccgtt ttcacccatt tcagctgatg gtaaattgga caatgacact 240
actagctctg aacaaaatac caaattaaag accgtagaaa aatgcaagtt acctgagaaa 300
ctggaggcat taaccatcaa attgtaacag gagtagttaa aaattaaaaa aaaatgttat 360
atctatctaa gatttttgt gaaacgaaaa tcgattatga tgaaactcag ttgctcttgt 420
atacttatgt cgattgatat acaattctgt acatacataa tatgaaatta tccaaatatt 480
aaacaagtgc aatttcaatt aaaaataata acgcaatatt catgtcgctt caagtcaatt 540
cattactga                                                         549
```

<210> 1280
<211> 549
<212> DNA
<213> Ctenocephalides felis

<400> 1280

cgctgtttta atgagcggcg cgataaaata aatntgttga atacatggct attttaaaaa 60
tcgtttttat atgaaaatta aataaacact atatattttg cgcttatatt gaaacagaaa 120
atactatagt tcaatgagtt ttattaaaaa tattgaatta ttttacattt ggagataaat 180
aaaatggttt ataattatat gatatacgca tgagtaatta tgagtttgcg ctaaatctca 240
atcaccactg tgtagattca gctttaacat agccaatcca actgtaaaga gagaaggaat 300
aaatgatagg ataaatggca ggaaaggatc aacaaatgcg accatacata tattccatca 360
taataatgag acatacgtaa ataataaggn gagggaaatc ataatgggat tgagaacatt 420
aataaaattc aagaaaactt attaaaaatt ttcagtgtgg tgtcttctgt cactctctac 480
aaaggtagta aatctgagga ggtcgtatca aggataagaa atcactaaga tgaggatttt 540
attgcaaat 549

<210> 1281
<211> 549
<212> DNA
<213> Ctenocephalides felis

<400> 1281

cgggttccga agaagatgat caaatgattt tcgactctga tagtgatgat ggccaaaaaa 60
cctcacagga aaatatatta acaaaaaata ctaacaaatg gatggacagt ttgccagctt 120
ttgtttttga ccctagcata gacgctaaac aaagatccaa attgatagaa aaggaaatga 180
atgaacaagc aaaaatcatc catgacttga gaacattggg tgcatctcaa ggaactatag 240
aacatgaaat gagaagactc catgaattgg aaaccttaat ttttaaagat tttcgaagag 300
atatgataaa gaaactgcgt cgacaaagta tgcgcacaac agcaatcaag gataagttgg 360
gttttggtca acacagtact cttagaagtc gttacccgac tattgaacaa aatattcaaa 420
tgaatgggtc ggttctcgag acgattgcga gtggcgatat cggcggagat ccagcccagg 480
tggccatccc gatctgtttc ttaaagtgcc gggcagcacg tgttcatttg gagatcccta 540
cgttcttag 549

<210> 1282
<211> 549
<212> DNA
<213> Ctenocephalides felis

<400> 1282

ctgtaggtct gtgttcctcg attctaataa aataacagtg tcatttttta attaaaatga 60
tttaatttaa acaatcagtt atgttaattg ttagttaaat ggatagtttt aaacagtgag 120
caagtgttga agtgttaagt gaatcagtgc atttaagtgt gtatgaatga aatagtgttt 180
ccatttggat taactggata cagattttac tgaagacagc tggggaaaat atggcgttct 240
gtcagtttgc aaatgatgga taattattaa actaaaggac ccggcataat gaaacctgaa 300

aatccacaaa actaccacat gaaaatctaa aaattttagt caatcttaac gaaaccaacc 360
aaagcaacaa aactatgaaa aatccaacaa catttccgct tgaactgtca tctaaaccga 420
atctttagat ttcgtagttc aacatcaaac cacttcaaga catctttcta atatttataa 480
ttcacttata cgcttagaca agacaacttt ctatgacggc tcgattgtgt ncaaatggtt 540
gtaaggcat 549

<210> 1283
<211> 383

<212> DNA
<213> Ctenocephalides felis

<400> 1283

```
agcnttaaat aacaatttca aattcaatat gaggaattta gtggttttcg ggttagtgtt 60
ggtagtttta tttgttgtta caatggcaga agacacacca gatgaaaatg agaaattcga 120
agtgggaatg tcagaggttt ctttgaatga tgtagagcca gcaccacgtg tagtatgcca 180
acttggagga aacagattat gcaatgctcg gtgcatatct ctaggaaaaa gaggaggctc 240
gtgcaaaaaa ggaacttgtt actgcagaaa ttgaagaaat ttaatatagc ataatatatt 300
agataaactt tgaataaaac cgtgttaaaa attttgcgca aaatatataa tatacctaca 360
aattaaaaaa aaaaaaaaaa aaa                                         383
```

<210> 1284
<211> 549
<212> DNA
<213> Ctenocephalides felis

<400> 1284

```
gcgcatcgga gcggttccct aagaaacatc tatccgtcaa tctatcaaac ttgtaaccgt 60
taaaaataat tataagagaa taccttaaaa taaaaggtag ttttcgttta aaatatcctt 120
aattgtgaac tatgtgataa taagtgtttt aagatagtgc ctatgttaat atttttgatg 180
acgagaacag aataagagaa caaaacggga tactaaaaga caagagcttt gtttgacacg 240
acagttgacg gagactagaa tgcaattgtg attttgaata tattaaattt taaaataatt 300
agaaacaaaa atgtcgacca aagacataca agctcagaca aatggggatg ctgacatacc 360
tcagcggcga ccccaacata ggggatgttg tagcaaaatg ttatattttt ttaaagttta 420
ttggagaagt tttgtaatag tattagcacc aatattatta accccggctt tcttgtaata 480
atgaaccgaa attccggtga tgatgtagtt atgtaatgct gatattggtt accgaagttt 540
acctctcca                                                         549
```

<210> 1285
<211> 541
<212> DNA
<213> Ctenocephalides felis

<400> 1285

```
gttgctgtgg gtcttttact tttgggtctg gtgatcgctg gacttgtcaa ggccggattt 60
ttcaagagga cgactaaaga agaattagaa gcacttaaag aggctgatca gagtgcacca 120

ggaataagtg aagaggaggc tttagcgcac agccatgatg ttgaaaactc atctgaaaaa 180
gaagaagctt aaactgtgtt gaatgcaatt tcaagttgta aaatttttgt gctttttgta 240
tatagatatc tacaaatatt tcgcagaaga agtataagaa tgtttgagaa tatttgaaat 300
caagagaaag ttgtgtcata atcataaacg tatttagatc ataagtagta aacattcaaa 360
tatcacttaa gtagtgtaaa tatgagactg aaagtgaacg attaagcatt tttatattta 420
aagtcatata atttatttct aagattagat atagaatatc tgtgatgaat aaattccaaa 480
cgatatatta taaattatta ataaatcaat tctatataaa aagaaaaaaa aaaaaaaaaa 540
a                                                                 541
```

<210> 1286
<211> 549
<212> DNA
<213> Ctenocephalides felis

<400> 1286

```
ccttattgca ggtgtgatag tgaaaaatat tattttataa tacgagtttt attcttgtca 60
attttaaatt tccggtatgc aatatgtatg cattcataca acaaataaat tacccggtgc 120
acaattatgc agtgctgtta tttaaaagat aatgaaaatt gcataataat tagaaaatat 180
cataaacata ttcattagtc attttgtttt tatgtgaaaa atatgtgcat aaagttgctg 240
ctgaatagaa gtcggatttc ctcaataata tagaaagaaa tggttcaggc tgacccaacg 300
tgcagcctct ggctggacaa aaacattgac agcagacgag gatctgattt ttcccgcctg 360
gacttcgaca cgagttcaat gtttgaatcg aatgtccagt tgtcaagttc attagatgtc 420
atcgactggc aaaaactaaa attccaaacg gctagtatca acagaggatc gctgaggagc 480
aaatcatttg tataattaga ccaaaagata cacattaatg gaaattcgga ggataaggga 540
aagattctt                                                         549
```

<210> 1287
<211> 549
<212> DNA
<213> Ctenocephalides felis

<400> 1287

```
gggccaagga accaagccaa aggccaagac ctacaggtgc agctggtgaa acctttcctt 60
ttcctacaac atccatacag aggtccgggc attctatgga gcgaccgcga ggggaaacaa 120
ttaggagata tccaggtaca actggtgaac gccccgccac gccaatgaca agaacaccac 180
agagatcccc gggccgcgcc cccattagga ttataataac tccggtcagt aaaccctcgg 240
ggccgcgatc gcccccaaca cgtggcagaa tattaagtgc tggccatctt gcgcccccta 300
caccggtgta tcggcgcagc gcctctaagg gttcttcgcc cagaagaaga ccagaaaggc 360
ccagtgaagt tatgcaaccc aaaaggagac gtctagaatg aaacatccca tttaattgct 420
gaagagcttt ccatttattt tgagagtcaa ccaagaattg cagcagcagc ttcgcagcag 480
agataatgtg gctccaaaac tctgcctaga caatgacacc ttcgaattca tttgcctact 540
gaataatga                                                         549
```

<210> 1288
<211> 384
<212> DNA
<213> Ctenocephalides felis

<400> 1288

```
atttatttat attaatatat tttattgggg tgataggaaa atttataaaa cttttttttt 60
attaaaacat aaataattga ataattgatc cataaattat gattaaaaga ttaagttact 120
taagggataa cagcgtaatt atttttaaga gaacatatcg acaaaataga ttgcgacctc 180
gatgttggat taagataatt ttaaaatgca gaagttttaa aatttggtct gttcgaccat 240
taattcttac atgatctgag ttcaaaccgg tttaagccag gttggtttct atccttaatt 300
ttttaaaatt aattagtacg aaaggaccat taatttataa taattatttt aatttgaata 360
aaattaaaaa aaaaaaaaaa aaaa                                        384
```

<210> 1289
<211> 548
<212> DNA
<213> Ctenocephalides felis

<400> 1289

```
gaccactggg cttgttctcg aacaggactg gcagtcgaac agcaatgatt ggtttgtagt 60
gtatcgattt ttatatctta acatatgtgt aaataaagtg atatttaatt ttttatatgt 120
acatatcaaa caaagaataa taaatgatgg aaattgtatt tttgtttttc aattcatatt 180
tttttaaact tctgtaatct gccctcgtat gttttatttt acatgtacga ttctgccaat 240
gcttggtggt caccacttgt ctgcaatgaa taaaaaggaa tacctaagac cgtgcgtgtt 300
tcgacttgat ccagtaacca tcactaatat aaacctgcca ctgcatttta aacaatcgag 360
cggaatcaca taattacggt accatttgca agcgttttta catatgaaag gaaactgttt 420
atttaccata ggcatgttag acataaggta gtaggattaa tcgtgtatcc ccagagttta 480
ctgcaaataa acttttggca ccacctcaat gagaagcaat tgacttcact tgcgactagt 540
ttggtttc                                                          548
```

<210> 1290
<211> 549
<212> DNA
<213> Ctenocephalides felis

<400> 1290

```
ctggttttgg gtctaacagt attaatcgcc atcgtcagtg gacttgtaac aggtgtggtc 60
ctgaaatatg cttgtgctca agttgaagaa gaacataggc acgacgattc tccagcatgg 120
gaattacctc atgatgaaga aaaggcggaa aatcatcatc aaaatgttgc tacaattagc 180
tgaatatttg ttctaactat attgtatgag actgttcgaa aaatgaagaa agaaagtgaa 240
gcacaattta aataatttta aaaatactaa aatatctatt aatgtagttt tagtgtaatt 300
actgtgcttt aaacacaata tttgatttta tattgattta taaaaaatat gttttaatat 360
aatgtcataa gaaacaacta atttgccaac caatttgtca attggttgaa tagaatttaa 420
taaaaattaa tgtattttta ttcaaaaaaa aaaaaaaaaa aaactcggaa atatataggc 480
tttcgatgtt gatgccaaaa cagaccggtc caatctacga gcccttttaa ggagggagta 540
tgggccaag                                                         549
```

<210> 1291
<211> 549
<212> DNA
<213> Ctenocephalides felis

<400> 1291

gctttttcga taactcaatt tttgcttcaa gtaagcttaa gaatttttga aaagcttgtt 60
tgagtcccaa gtaagcttaa gaatttttga aaagcttgtt tgagtcccaa gtaagcttaa 120
gaatttttga aaagcttgtt tgagtcccaa gtaagctttc gaaattttag aaagctaaag 180
ttaaaatttt tgtttcaatg tttcaatgtt tttgaatcat tcttatcgga tgctcctttc 240
tcttaaaaac tgcaattttt tcacttgttg ctccgtatct tcgaaactgc taaacctgcc 300
tttggcaaaa agtaatcagc aagccagtac aaatcgaatg tttttagaat cacgctcgga 360
atcgtagacg aaaaatttta tccaaacaca cacacataca catacattcg atttttcttc 420
agtatgccat aaatttataa agaagacctt gactcactat attaaaaatg ncatgtgaga 480
tttttgcctt ccctaaagaa ggtgtaaaat taggaattta tcatattctt ancagagcgc 540
ccttactga 549

<210> 1292
<211> 549
<212> DNA
<213> Ctenocephalides felis

<400> 1292

atacagcaag caatcggtgc cagtttgtct aaatacccag gacctgaaga aactttttgag 60
gctcttcgaa tattttgcag aggagacttc ttaaaattga ctctaggatc attgacaagt 120
gtgtgcttca aaacaaatca aacaaaagct ttgaaattcc tcaacgacaa ctttgatcaa 180
cctgtgtctg caaagaaaca cataataaga ctcatcaacg ccatcggtac caaaaatgtg 240
attttgaaaa tgctgaaaca actctgggat tgtgagaagc atccttctat ccgtaatgtt 300
ttaataacgt ctgtcttcaa ttggtttgcg agtgatcccg atgaggaaat atggaatttg 360
ttgaaatcta tatttttaga tctgacgacc aaggataaag atatattcac catgcttctg 420
agtttcgcca agtcgacaag gattatttaa aggaacatat tgaactttgt ggaagacgca 480
acaatttgga gtccaggata aaatcaattt ggatgatgag aagtctaaaa tctgcacgcg 540
taacgaaga 549

<210> 1293
<211> 549
<212> DNA
<213> Ctenocephalides felis

<400> 1293

gtgaagtgat gtaaagaacg tatcattttt ttattacttt tattgattaa caagtgtttg 60
atttcggttt acattaaact acaatttatt gacggtcgtg gcggctcgcc ctagtgaaat 120
ttttgagttg aacccttat atgtaacact gatactgcac ttatatttat tgatattgtc 180
tgtattttta cattttgtgt tcattattaa aacttaaaaa atgaatgtcg aagttacgcc 240
gaactattct tacgtctttg atttcgaaaa tgaatttatt catcaagaaa cgagaaattg 300
gatgactaaa aattggacat ggggctttta ttattgtgga atctacatgc ttgtaatttt 360
tgggggacaa cattacatgc aatcacggcc aagatttgag ctccggggcc ttctgacagt 420
atggaatgct gnctagcaat gttttcgatc gtggtgcctg ccggacagct ctgaacttct 480
cacgtgctgc gcactacgga ttgtccatag cgctgcgacc aagcttcatc gacaagacgg 540
tagcggttt 549

<210> 1294
<211> 549

<212> DNA
<213> Ctenocephalides felis

<400> 1294

```
gtaaaatatt tcaagaaacc tgagccaatt ccaaattatt gtgacacaag tgttaagagt 60
tcttctcttt catcgagtcg agaaagtggt cagaatggat ctaaaacttc agcaggcagc 120
agaagatcat cattaaaaaa gaaggaagta aaaaataaaa aagattccaa acggacgagt 180
ttgtcttctt accaaaccag ctctgaaatt ttgagaactg aaagggaaat attgtctcaa 240
aacttggaag atgttacctc tccgctggaa actttgatcg aagagccgct aaataacgat 300
ttaaaaggct ctaatcaaaa tgatgcacca caacacgtgt ttccggattc tcttgaccaa 360
tctcatatgt cgtctttaga acctttaccc gattggaaac ctgttatttc agatgaagat 420
tgtcgatcac tttcaaaaat ggtggatgta tattggacga gtttcagaaa gggtatggaa 480
ggcaaggttg tcagatggac aatggaacat atatcaaatt atttagggag aattttgcaa 540
tcttcaccc                                                         549
```

<210> 1295
<211> 547
<212> DNA
<213> Ctenocephalides felis

<400> 1295

```
naaatnnatg tggcaaatga aatgagtgag aatcgttgnt taaagcatta aatcgtntaa 60
ttagttattt ttttgtatat tgggcttgcg ttttaacgnc ccgangatct natttnnttg 120
nacaataaaa ntgttnattg nttaacatta ncaanttaaa antatctgan tcttgncttt 180
aaaatgacan tntgtatngg tgctggtacg tggggccaaa gtgtttatgg tgcaatgcgc 240
acgttaatcc attntattgc ggcaacacaa tattggtntg cttgttacta tgattggaat 300
tttgnacacg tncngnagag cgttcatgtn atgggtaatc cgttcggtgg aaaattcaaa 360
tatctgacat ttattgatgc cgatctgcaa gccttatatt tcactgtttg tgtattaaat 420
gactttgctg gatcaaatga aactcgnnna ngctaaatcc ttctgcagtc tgaaagacnc 480
atgatgcagc attcgcnttn ctgntgnatg aatgnggant cattttggac cntnngcttt 540
gacaggc                                                           547
```

<210> 1296
<211> 549
<212> DNA
<213> Ctenocephalides felis

<400> 1296

attntaatta accataactt attgttatgt taaatcttta aataaatctt atttaattat 60
atttatcaat tatttataag aaacaatttt aaaacattcc ttcattatta taactgggat 120
gaccggaatg aaaatgttag cacctacttt gcatttttca catgataact agcaatcttg 180
tgaggaattg tattaggcct cagtaaccca tgaagcgccc ttttgtaaca caataataat 240
gattcatcct tttctactgg ttctagataa gcttgcagag gataacacaa cttttcttca 300
gggcaattta aaactcttaa taatccagaa tgttctgacc aaatttgctt tctccaacaa 360
atgtcgtggc aatgtgctaa cggaactagt acacaagctg aaaacatttc atccccataa 420
ctaacagctt gataatgtcc agaagttttg tatatagatc agtaaaactg cttagacctt 480
gtatggttgg taaagcaaac ggtgagtagg ccaatttgaa attcttcgaa atgctttagc 540
aacagaggt 549

<210> 1297
<211> 464
<212> DNA
<213> Ctenocephalides felis

<400> 1297

tgnaacacca cattagcaaa ttcacgaata ttngagttgt ggacaaacta ttaagaaaat 60
aatgattaat tatttttatc aagattcttt aatttgatac taaaatgttt tgctccacaa 120
aatgactaaa tatactttaa aaaaacaact gactagaata tattgaaatt tacattcgga 180
tatatttaaa tacttactag accacaaaaa tatttgtcaa agtttgtcaa aaatattttg 240
tatatatttt cttgaatatc gacagtgata acgctggatt tgtgctgcct ggtagatttt 300
ttcttgaaaa ggtgatatta ctcctatgat ttgtatttat atgaaagtgc tttatattat 360
tttaatgatt ataaataata gtatgtatgg gatattatat aattgtgtat atcgatataa 420
ttgttgcctc aataaaaaat atttttaaaa aaaaaaaaaa aaaa 464

<210> 1298
<211> 547
<212> DNA
<213> Ctenocephalides felis

<400> 1298

atgtgtttat tattctaacg aacatgcaca gtgattctaa tttatatggt cataaaatgt 60
agaataaata aacaataaat tctttgataa atatagtatt tagctttagt atccacgtgt 120
cactaataat tttacaatgc gcatgagtcc gaaactgata aaaggatgcg gtataaacaa 180
aattccttca gtcacgcggt aacaattgtc agaataacac tttaatctca ttttttgatg 240
tgaattactt tgtaattaat cgcgtattct acaaatcacg atgaaagtta aggacttgac 300
attcattaga gaaagactta cattcccatt agttttatta tggcttatcg catttcctgt 360
tcgagctatc gaaccaaatg aaattctttt tatcatcctg agtcaaccaa atacttatag 420
ttcaagttta gctgaaagtc tgcgaaagga tattttaaga caagccatcg atctgaataa 480
gacgccgcaa taatccacct ttcacacctg gattatccgc agcatcttca tggccgtctc 540
cttattg 547

<210> 1299
<211> 549
<212> DNA
<213> Ctenocephalides felis

<400> 1299

```
atataaagca gacacctccc gaaaaaccgc ccgcctatga actgtttgcg ccccctgcct 60
atgaaacggt tgtgaaaggg gcagacttaa atggaaagaa caaaggccct gaatgcactg 120
tttttactat aagttagtaa aaaacgagtg aagtaaggct acctgtttat ggccaaatca 180
aattaagata gaagaccaag agtcttttgc actttggagt tacttgtggt gttcagcatc 240
tggtggactg atttggccat ttgcagggcg ctgagccgag agataaaata aaaaatgtgt 300
gactgcaatg ataaatgttt atcaataact tatccagaaa gattatttag gatctttatt 360
gctgcaaact ttttcaaaag cgaaagaaac tctggaaaat cattgctaat ttatctctgt 420
gatataataa attattattc aataattatg cttgtagttt tttcttataa atatgaatta 480
gataattaat aaatataagt aactgtagta ttattaaagt cttntttatt agcaaaaaaa 540
aaaaaaaaa                                                         549
```

<210> 1300
<211> 549
<212> DNA
<213> Ctenocephalides felis

<400> 1300

```
gaanaatgaa tctctatcta gtagaagtat tactactgac gacttggaag ctgctcgaaa 60
atacatgtct agcgaatcgc caataaggca gggtattgag ggacgaatca cgttcatcaa 120
tcttggcgat cctaattcat cagttaacct gcactcatca attggaatga tatcatttat 180
attctttatt gtcttcaaac atttttgtac cttgtaataa aaacatgtat atttagatta 240
agcaacaaaa tgtaaatcta tttttgtcac aaaagacatt aaccagtaat taatatgaat 300
atttaaatat ttttattagt actccaaatt ataaatttgt gacttgaagg tttaaaagta 360
aaacaagaac tttgtgtgtg atcaatcact ttattaagaa aatatttgtt tttttgttgt 420
catatggtgc tcgctatatt gtaatcaata tgtatttatt gattagtata tttatcactt 480
atttgtgcta attattgatc ttatgataca tataatagaa atgaaaatgg ttcacaaatc 540
tcattaatt                                                         549
```

<210> 1301
<211> 320
<212> DNA
<213> Ctenocephalides felis

<400> 1301

```
gtgcatacgc gttacatatc aaatgaatat gaattttaat tactatacat tattatttgt 60
tttaataagc gcgccttaca taaatagcgc caaattcaca tgtctcacgg atggcgtgaa 120
aaaggaaaca tcttgtgcag cagattcatg ttttctttta tacaataaat ataaaggtgt 180
gcgggagtat ggttgcataa taaaaatgac gcagagccaa agaaaatact gnttccgtca 240
tccagaatta tgctttanct nccaccccga aaaacctgta attcaatgaa actgatgaaa 300
ctntgccgcg aatgcctcac                                             320
```

<210> 1302
<211> 549
<212> DNA
<213> Ctenocephalides felis

<400> 1302

```
actgggaaga caatcaatca aaattcataa aaaagttata aaaagttaga aaaatggtta 60
caaaagtagc actgttagtt cttgctgttg ctgtcgccca agtttcctgc gatggattaa 120
catggaagta ccatccatct ctttcagcat ttatgctgaa aggtaaagac gaaatgggta 180
aagattgttc agctcctgga gaaataaagt gccaggattg tgagacagca aacctttgca 240
ttgcgattgg tgctgatttc ttagaaacaa cattggaaac atgtccgagt ggaatgactt 300
gcaaaccagg tacaggttgc gttagagcct ctgaaaatac attaaactgt cctgatcaaa 360
ctcctcccgt agacaacagt tttgtatgcg aatctattgg tattttccct gatctggaag 420
actgtaagaa attccatttc tgtttccaaa tacagaaggt gttaaagctt ccacagaagc 480
tttacctcat tctgaaatta aaatatgtcc atagaagcaa tcaaaagtca gaccagagtt 540
gcaaaacaa                                                         549
```

<210> 1303
<211> 417
<212> DNA
<213> Ctenocephalides felis

<400> 1303

```
gttaagagct gtcaaattat tgtacaaata tttctaaatt aaatacgtat atataattta 60
ataaataata ttttactact atgaaaagag ccgcaaaaaa ccttctacta gaccgtctgc 120
acaaaggcgc cgtcatggcc tgcatgggca tcaccgtttt gggaacactc agtcttggat 180
tccgagttta tcaatacttt actgatataa aacctgaaat acaaagaaaa caaatattgg 240
caaagaacga gctgttaaaa gaaggagcct cggacatatc attatacgag agcaatatca 300
cgttaaagga ataactccta ggatagtaga tatatttagt actgattact ccaaaaatgt 360
atgttatata atgtaaataa gacttataat ttatttcaaa aaaaaaaaaa aaaaaaa    417
```

<210> 1304
<211> 549
<212> DNA
<213> Ctenocephalides felis

<400> 1304

```
gagccatctt atcaaacata tttaatataa atttataata aatcgtgcaa agttttcatg 60
ttttaattaa aataccgatt gaggtaatac gaagtacaat ggtggataat aaattagcag 120
gattaacaga ggaaaagcta cgtgttttag taaaacaatg taacaaatgc ccgaaatgta 180
atgaaatttg tctagaggat tttcccgttg ttcaatgtag tttgaaccac agactttgca 240
agacgtgctt tttggcttcc ataaatgatc cttgcttcca gtgcactaag ggcagcaaac 300
catccgctaa taaaaaagat cggccaaagc agccaaatgc cccagacaat tctttcccga 360
aggtaaactg caaatatgcc agtgacggat gcaaaatctc aaaaaagaag gacaaaatta 420
gatttcacga atcggaatgt gtgtttcaac cacaagaatg tctggaaaat tcactgttta 480

tttaattgta ctggccggta tttcaacgca tgctaccttt gtgagaacat cattaaatgg 540
aacacatga                                                         549
```

<210> 1305
<211> 549
<212> DNA
<213> Ctenocephalides felis

<400> 1305

```
atanaatcag tgttgacaaa tggtttgagg aaaatgagaa aaatacctgt cacaaaattt 60
tatattgcaa aaaaccaggt gtagaacaca tgtaatacta gaagaatggt taaaatgcag 120
gtgaccattt caaaggaatt tgaatatgat aaaatatgca tagatggaac tcatggtaca 180
aatgcttatg gatttaccct gcatactctt cttgtaattc atggggcagg ttatcaggta 240
gaattttgtt tcacaaatcg gcaagatgaa actttattta aattattttt tgagaaaatt 300
tttgggcaaa gtaggaaaaa ttgccacctc aacttttatg tctgaggatg cacctgcata 360
ctacaattca tggtcatctg tcatgacttc gacaaataac catttacttt gttcatggca 420
catatcaaga agctggaaaa gggtacttaa tacaaaagta gacaaagaag tagatcacaa 480
aagcagacaa acgttttcac atcttattaa gaatcgagtg agatgatttt tggctaagtn 540
aatcttatt                                                         549
```

<210> 1306
<211> 549
<212> DNA
<213> Ctenocephalides felis

<400> 1306

```
gcttatgttt gtttttattt tgacgttgct aaaattttag ctttaatgtt tgttttcttg 60
tgataaatta gttttatttt aaaggctagc gcataataat aatgatgcat ctgagcgctg 120
acatatctag tgcacttcag caacttgaga gcatcaagac agcaatagat gactcccatg 180
atccaaaact tcagctcagt actaatgaag atttggatat gataataagc ctattgcaag 240
atccagtttt tcgaagcatt gttactactc aagattcact aggtgaattg aattcccaaa 300
taacacaaca tccatcaata ttaccaggag attttgatat aactacttca ggtgatctaa 360
ttctgcggtg ccccccttctc ttgatttata tgataatgag tacactgatg aacaaagagt 420
accctctgac aattaagtcc aggtagccct cagaggttag gtatagcatc ggtnggggca 480
gtcangggaa cattacattc atgaagggat caatntngca atgaggcaac cntgatggat 540
attcccaca                                                         549
```

<210> 1307
<211> 549
<212> DNA
<213> Ctenocephalides felis

<400> 1307

```
atttatttca atacgtgtga aatatattat atggttaaca tatttattca tcttgttttt 60
aaaataacta atttgctatg aaactaatag taaacaaaaa agtttttat gccggcatta 120
```

aaccagacaa atttttgtga aattgaatta tttatttatt ttaaacaatt aaacttaata 180
aaaataattt catttttttt aaataattgg atgaataaaa aactttttcg tttactgtta 240
tatctgtcgc catctgaaat actattgctt taggtgtata taaatatcta tttacatatt 300
ttgcaggtat ctgattaata ctaatttttg aatcactggt ttatttaaaa aatatttcac 360
atttttaggt tctaacagca cnacactttg ctacatttat tagtaaatgt tattaattaa 420
ttatgtataa tactttgatt tcaactaaaa ttttgacgca aattgaaatg tgcttaagtt 480
tacaagcagt ggccggacgt tggcacccat cctgacttgg ggaggaagac attgtgtaca 540
nctcatgag 549

<210> 1308
<211> 549
<212> DNA
<213> Ctenocephalides felis

<400> 1308

aataaatcat tttcaaaatg gtgtccaaag tgtttgttgc tcttgccatc attggcttta 60
taggggctgc acaagccttt ttcgaacaag atgccgtctt agataagatg gtagatgaca 120
tctctgctga atatgagaac agcattgcaa gaattagcca agatagcaaa attgatatga 180
ttgccgctga attcgaaaaa caaggatttg cccctgaagt cgatagagat ttggagaggt 240
tcaacaaaga agttgaaagg aaacttagca aaaaagtgag tgaagccgca aaggaatgcc 300
ttaaaggcca acaccaaaaa gctctaggct acgcacaaga agcccgtgct aaggtcaagg 360
cttgcagaga tgacaaacgt gatgaattca accaagttcg caaaatggct tgccgctgga 420
gagaagccag ggagaacgcc gaagccctca gagcacaagc caaggagtgc gttgtgaccg 480
cagccgtgtc gatgaagcca gagtttgctt gaaaggggtt ctccaagctg cccgaagagc 540
aaagacttg 549

<210> 1309
<211> 445
<212> DNA
<213> Ctenocephalides felis

<400> 1309

attgcatgga agctagactg caagtatcat cggaaatgta tggtgaagtg ttggagaaat 60
tcaatataaa aatagagatt tttcaaaaaa gattagatta ctatcgtaaa agagcattat 120
tttctatgca attagttcat gatctaacaa tatcagatga caagaactta gaaaatttgt 180
ttcttcaatc tcgcttatta tatttgcaag gcaataacaa atctcatgaa gaacttatta 240
agtttattag atctccaagc aaagatgcat attttgctaa tggaactagt atattaaaag 300
gatccaacaa tgaaaatctt gccatattag ctcttaataa cattggagtt attgaatttt 360
ctcttggtca ctttcatcta gcaactcatt cacttcaaca gtcattaagt agagatattc 420
aactggtaaa aaaaaaaaaa aaaaa 445

<210> 1310
<211> 549
<212> DNA
<213> Ctenocephalides felis

<400> 1310

atgtatcaag ttatattttc ttttgtataa taattacatt cgtttagtta agcaacttgt 60
ttctagtgat aattgtagaa aagtgtagaa aaatgacacc tgcattatat cctgggatga 120
agaaacgtta ttttatgtcg atttctttag atgacaaagg aggtaaccaa atagcatatc 180
ttacaaaatc tgaggacaat aataaggatc gtttagcaag tcggttgaga agcggatcga 240
gaatgattgt ttgaatataa gtcgtacaaa attggaatga aacgatgaac aatatacaga 300
tctttagaca ggctcaaata atgtatgttt aggagcaact tgaagataat ggagatttat 360
cttcttaata taaaacctct aaaattggga acagtatgtc atatatgcat gaacttgatt 420
gatgtattga tgtataatga actgaagata gaatgaattc aatcaggtta aaaaaaatgt 480
tggcaaaagc gattcgattt agatttgctc ctgatataaa acttccagaa gtcatcatat 540
atgaatatc 549

<210> 1311
<211> 549
<212> DNA
<213> Ctenocephalides felis

<400> 1311

ttgacaagtt tcgatttggt tagttcttgt acttaatatg gaatcaatct catctgaact 60
gactgaacca caaacaaatt ctaattcatt taccgttgat caaatagaaa ttgacatatt 120
gcccactata tatgatatca tacgaagtgt tgaaagagat ccacatgata gcgctggcaa 180
aaccagagaa tcacaagatt gcagtgtgaa ggtattagac ttacaaaaga agttagaaaa 240
aattcgaagt caagttactc agctacctgg aattgattat aataaagagg aacaacttca 300
atatttagaa acacttagga aacaattaaa acttaagcaa gagcttttgc acaaatacag 360
gactatgtac acatttgatt caatgaaaat ataaattgtt taaaatgcct ctgcgatctc 420
tcatgaatta tttgctgaat aacgacgttt agttcagaag ttgctgaatc ttatccagtc 480
gaagagctgg cagttagcca tttcgctatg atagatcaaa atcaatttag ggacacactg 540
agaagctgg 549

<210> 1312
<211> 423
<212> DNA
<213> Ctenocephalides felis

<400> 1312

aattnattta aagtcgaaat tccaatttgt tgtactttat aagagtaaag caatttcact 60
cacatctata atgtgtatac atgtattaca tttttatttt attaccttgg aatattgaca 120
ttttgaatga aacattagta attttgttgt ttaaatgctc acctattaca gtaataactt 180
gtatgtatgt tctaaacctt tttaaaattt agaaatgtaa cgagttatgt ggatatttgt 240
gatctatgct gaatgatact atttagtgga caaaattgca gttctgcatt aacctttttgg 300
tgagaatctt gcaatccaat attgtgtagt tttagtttat aatgtacata taagcaacct 360
taatgtaagc attaatttaa tcaataaaat cttttctata tcaaaaaaaa aaaaaaaaaa 420
aaa 423

<210> 1313
<211> 549
<212> DNA
<213> Ctenocephalides felis

<400> 1313

taatatttta ttgtaacaga ggcaaaatct agaaattatg ggatgatttg tgataagtgt 60
gaccacatta atggaaccag agttttaata aacgcatttt gaaccacaag tacaatggga 120
tgaacttaat aacttctgga attataatca tttataaagg tggactcatt tatttgaagc 180
tctatgtaaa atcctgctct gattagtata actactgcat ttcagaatgg aaaatggtgc 240
taagaagctt gatataaaca agtataagct ggatgtgtca gatttagcaa agcaaaacta 300
ccgacaaata attgaaaata aatatagcag tagtattcca aataaccaag aggtgcaaaa 360
ctcatcaaat gcgggttcct tgatgtcttt gacaacaata tctatgtcat cttcagacaa 420
tagttatcaa ttatctcaaa attgcgaaaa agagctagtg caagaaaatt ggatatatac 480
tattttccag ntggtntcct ttatgatgct ggatnggaca taggtgcagt gcatactcac 540
ctcgccaca 549

<210> 1314
<211> 549
<212> DNA
<213> Ctenocephalides felis

<400> 1314

gtcacaatgt gatatttatt tttatatttt aaaataagga acatgctgga gcaagaacat 60
gtatcattaa atttacccga tattaaggag tccgttactc atagaaaaag catatggcgt 120
tggtggaatc aactgtccag atttcaacga agtctatttt atatgatagt attagttcta 180
ttttttacac tcttatattt attaccaagt caacataatg gagatggaaa aactatagag 240
catatacaaa taacaccaat tgaagcaaat aacttaccat ttttacaaaa tgatcaagtg 300
gtatcaccta ttaatttaaa tcaaaattca gaaactaatg gcttagattt agaaaaatca 360
attaaagaag tgaggaaaaa tgtgattgat gaaccaaaac accacactga ggcaatctat 420
ttaaaaagta gtcttgggac tagtcatggc aaagtttttg tggccaaaaa cagaaagaca 480
gaaagcagtg tgaagccttc aagctgcttg gaagggctta aaacattgct ggggcacgcc 540
attaaccat 549

<210> 1315
<211> 549
<212> DNA
<213> Ctenocephalides felis

<400> 1315

attaaattga aaacttcatc tcctaaatga tggtcaatgt tacataaaat tgtataagat 60
agtttgtgtt aaattttacg acgctttgat aatttattaa taaacattaa ttgatatcag 120
agtagagtca aatcgatata taatattatt ttacctactt ctctggaata caaatttatt 180
aataaatatc gatttcagtt acaagctgat cagtagtata ttttctgtgg tcaaggattg 240
tcatcatgtc aactaaaata atattattat ccgcattgct gttgctaata tcttccactg 300
ccgtccaggc ccaagtatgc agttgtgtat gcatcacaat atgctgcacc cccgaacagc 360

tttcaaccct cgcctgtccc caagcgcagc agcagcagac ggcctgactg tccaaactca 420
ccctcctaaa aataatgctg atgcttacct cgaatacata caggcacaaa atattttaaa 480
tcagtactta catcaacagc agtgcgacaa tccaaaaagt naggaacaaa aattcttggc 540
acagcagca 549

<210> 1316

<211> 549
<212> DNA
<213> Ctenocephalides felis

<400> 1316

```
gtttgcattt tcattgtttt tgacgcgaaa aataaaacta caatgctgtg acatatatcg 60
ttcaagtgtc aaatagcaca taaaaccgta tttaaatgaa aaacctctgt taacaatagt 120
tggcttgtgg ttgcctatgt cgtttaccta ttccccttgt gcaaaccccg ggttgtggga 180
atgtgtaaga aaattagtaa aatgctcgga acgataaaaa ttgtgcaagt gaaaaatatg 240
ttgattaatg tttaattatt actgtgaggt gaaattgata attctgtatg aatttaaaat 300
taacggattt caatatggaa gtgactgata gtgatacgaa ttccagcaat tataaggaga 360
cgaataaaag tgaaccatcc aacacgtctc ctgaagacga tgcaactggc tgcgaagatg 420
aggacgagga tgagctctac cttcaacttc atcttcatgt aaatctgcca actaaagcct 480
cacttgattc agcatgctca agtatccagc agtcctcaac gttgaaatag gacttccgtc 540
ccgaatgct                                                         549
```

<210> 1317
<211> 549
<212> DNA
<213> Ctenocephalides felis

<400> 1317

```
gtcatattat tattgaaata gaaaaactaa ttgttactaa ttaactaact gattaattca 60
ttgattgttg gaataactat ctgaaagaac aacgctatgg ctgacgaagc actaaaattc 120
ggccaagatg gacaaccttt gccaagtggt gaagaattat taaaaatgtt agaaggaatg 180
gacatgtccg aggaagacaa acagagcctg agggattctt tgcttcaaca ggcaaatagg 240
gctgcttctc aggaccccac gggggctact ggggtcacat tccaacaagt cttgttcatg 300
ttggccatgg tggcgattat agtatcagtt ttcgcatttt ttgcaaataa attatacaaa 360
tctctgacgt acaaagacag aatgcgtgaa gaaaaaagga aagccaagga ggagagaaag 420
aacaaggaaa agaagaaagt caagtagtca ttttgaaaac gacaagactt ttaaatccat 480
aattattata cttacccata gttcagtcag tcagatcaaa acagtntgat attaaaataa 540
ttttccgta                                                         549
```

<210> 1318
<211> 549
<212> DNA
<213> Ctenocephalides felis

<400> 1318

aacaaacctt cgtggaggtt ttgtttaata tcatccagcg cgcagcaacc tcaaagaaca 60
tgcacagaat taaagtctga cactctggac atccaatgcc aaaggggatt tcgctcagtg 120
ccctgcacca gccgtatgca gcccgggacc agggcaaact tggcatgcaa accaggattc 180
cagttgctca aagaaccaga gttctcgcaa attaattgcg gaaatgatgg gatttgggat 240
aattgtttgt tttcttgcga accagaatgt ggaaatccaa caccaattga aactgttttt 300
aattcggacc cacctgtaac gtacttagca ggtcaatatc catggtatgc aatgttgttt 360
acccgaaggg aagatttatt caaaggacaa tttctattca gttgtggggg gtcaataatc 420
aactcacgaa tgatagttac aactgcttat gcgctcataa gccagaaatc gattggatga 480
tcagagagtg tgtggatcta gtgtattcgt ttaataaaca gagatnttat gctagcntac 540
gaatagaaa 549

<210> 1319
<211> 549
<212> DNA
<213> Ctenocephalides felis

<400> 1319

gaaccacctt caagaactcc atatgagtca cagtacgctc gagcatctga agaagaagat 60
agatcgcaat tagtgtttat catagttacc tctttattct tctttatcat aatatgttgt 120
atcattgaag tattgcgaac aaattatcaa cataaaaagc gaatagaaag agaaactgac 180
gaaagtatca taattgcgaa agaacatgca actaagctgc acgaatcacc agctgttggc 240
atgaaatttg gtggatataa agcggtacca actgttgaag atgagaagaa accagttacc 300
aacggaactt taccaaagat tgaagaaaag tgtgacagcg ttgcagacct taatggatcc 360
ataaataaat ctttagatca tctaaagccc cagaagtagc agttgctgat atcaacacct 420
gtattccaga aaagaataaa gacttttatg tggatcagag aaagcagctc ttagatcatg 480
aactgcagtg ggactcatgg aagacaagga tccagcagac gtgatgcaga gcggncatac 540
gaagaaacg 549

<210> 1320
<211> 549
<212> DNA
<213> Ctenocephalides felis

<400> 1320

atgatctata aaccctcaaa ttcttaagta aaactgtaat agtattttaa cttataatta 60
ttagagaata ttttatatga aaaaacatgg aatttattat ggcaataaag cgtgatgcaa 120
tagtgaaaaa aacatctaag tgaaatcaga aaacaacaac aggaataaca ttttgaatca 180
gtaaaaataa cggatgacac ttcaagggtg cccagatgaa agggtgacaa ccccgtgtcc 240
gccgtctccg cccccgattc cgctgcagac ttatctttgg gaggacgtta gaagggaacg 300
aaaaaaaggg ggctaccctt ggacgcactt cgttaagagg cctttcgatc ctgatgctcc 360
acaggaaatt ttggagtacg accggtcccc aggcagtaaa cgtaaatacg gaagcctaga 420
tgttcaggaa ctagaagagt ctccgagtgt cactagaaga agaagagcgg atccgtggag 480
agtctttggg aaacgaacca gacttacggt gatccaaaca caggagtaaa tcagtantat 540
agaagacaa 549

<210> 1321
<211> 384

<212> DNA
<213> Ctenocephalides felis

<400> 1321

```
tactaaaaat aattttatca ttttcaaaat tattgtgcgc atgtatttaa gggtaaaatg 60
aaatttttga gttcgattgc gtcacatgta tataagtatt attgatgcgc cccctagagg 120
ggttcagtgc aggtgaacga tgttatttat ttaatataag ttgcaatgaa tgacgttaaa 180
ggacttgtgg aaagaaacta ctacttataa ttagtgaata ttatttatta ggacgaagta 240
gtttttattg tctccctaat ttatctttgc gagtttagtt aattcaaaat atatgatttt 300
agaattattt gttacttaaa aaaacatgaa agaaatctgt atttgaataa aaagatacac 360
aaacatcaaa aaaaaaaaaa aaaa                                        384
```

<210> 1322
<211> 549
<212> DNA
<213> Ctenocephalides felis

<400> 1322

```
aaagtgatgc aacaattatt ttcaaaatac aaaagtgtta aaaaatgggc gttaaaaata 60
tatatttata ctgcattctg atatgcctgc tacattatgc atcttatacc aaaactgaat 120
ctattaccaa caattctttg gaagaattgt acacaaacac ttctgccaaa acagattcca 180
ttactctttt atcaaaaacc agtctaccgc ctgatcaaaa tgccacgatt gaaaatcctg 240
atccagtgct tcctgaaaag ggctccgctg aacaagaaca acacagctcg atgtctatat 300
tcttcgtgct ttgtgtgctg gctttaggga ttcttttaat tcatttcatg ttacaaacag 360
ggtttcagta tttacctgaa agtattgttg tagttttctt aggtgcttta atcggcttga 420
taattaattt aatgtcgtct aaaaatattg caaattggag aatgaagaac cttttcaccc 480
acagcgtttt cttagtgctc tccgctataa tattgaatcc ggtatattgc ataaggnatt 540
ttttcaaat                                                         549
```

<210> 1323
<211> 29
<212> DNA
<213> Ctenocephalides felis

<400> 1323
tgtcanggct tatcggatac ccgtcgacc 29

<210> 1324
<211> 550
<212> DNA
<213> Ctenocephalides felis

<400> 1324

```
ttttaatttt caattcaata ctttgcagga tagattttgc gataatgttt tattatatag 60
ctgtaatggg aaatagcaat cgtagtcact tcatcctatc atcatgtacc atggaatatg 120
agaatatttt atgtacttct gcaaacagtt tcctgggtgc tcctcagctt atattaagta 180
gtaaaatgca gtagatgagg tattgtgacc aaaatttaaa aaaaatatat tgcctattta 240
ggcaatatga tatggtaccg gcaaagtgtg tttagtacat ttcaggtatt tttatataaa 300
atttttaaac gtgtacctca aagagctgcg ctatctataa tgttccaatg atgtgccgta 360
ttgcatacaa tgctggtgtt tccaaatctt tgagaacaat tgcattaaat agtgatgttt 420
gaatggatac ttttgcggcc ttatgaacaa atgtgggaat tgcaagcaat aatacttcta 480
gagtagaaat tatgctctat ttttgtgngc acatcttaga ctattgagat ctagtagatt 540
gctgaatgag                                                        550
```

<210> 1325
<211> 491
<212> DNA
<213> Ctenocephalides felis

<400> 1325

```
atcttaatgn attaagtgna aattaatata agggaattta atgaattaca ttttgaaaat 60
gagagcaaaa ctactttagg atacattttt tgactaaatc atgtaataaa tttaccatgg 120
cccattggaa aatttttctg atactatcgt ggcgattaac gtgttaataa ttttatagtc 180
cctagttcgg gttaaaataa aaaaacgtgt ctcatattta ctattcacct atattnacca 240
cttcatttac ttcttgattc ttcttctctt ccatatccga tcttcttgat tcttcttctc 300
ttccatatcc gatcttcctg attctttttc tcttccatac ccgatcttct tcattcttct 360
tctcttccat atccgatctt ctttattctt attctctttc atatccgatt ttcttcattc 420
ttctttnctt ctaaatctga tctccaattc tgctcttgct tttcttttca tttattcttc 480
gggcatttgt g                                                      491
```

<210> 1326
<211> 550
<212> DNA
<213> Ctenocephalides felis

<400> 1326

```
ccacgggata gcagtattat tgatgcattg ttgagcagtg tacgcgagtg caagtgaagt 60
attagttgaa caatgtgaaa tcatttctag aaattttatg tgatattaat aatattgttg 120
tcgtaatgga ttcagaataa tgtaataaat gatctttatt tgatgagatt attaaaaatg 180
aatattacaa cattcacgca atcaagtaat ttagaatcaa tccaatcagc caattaacaa 240
aatcgacgaa tatttttcaa gttttattaa tcaaaacctt aaaaagttga taacaagaca 300
aatgatacct acgagcaatc aacaagtatg cctagaagag accttccggt gatcattgat 360
gactatgaat cctttgaaat catcagttat tcttgatgtt ttggatgctc tatttgctct 420
aacactggtt gcgcccgcgt agtcgggtat tggagaggna cgtggaatct aatgggacat 480
ttgtttatac agataatgaa atatatagca gttcgtnctt ggcataggct tattggatgt 540
tatattttgt                                                        550
```

<210> 1327
<211> 550
<212> DNA
<213> Ctenocephalides felis

<400> 1327

```
gttcnnncgc ccacanacat tttcaaaatg ctaaaagcan caacaataat ttttatcgct 60
ttcaattttg tgtctggtgg cgtttatgat ggttacaaac tttacgaaat aagaccccaa 120
acaaaatccg aggcttacga tttaatggaa tggcaagtaa aaccaggagt cgatttctgg 180
tccgaagcca ggatgctcaa tcaggctagc caggttatga tctcacctga acttcaggag 240
gaattcgaag gatatctggt caatggtaat tatacttgga aagttgctga ggataacata 300
gagagacttt tacaagattt tgaaagaagc agaaaaaagt caagtgcccc acgtgacgat 360
ggatttgatt tcaatgatta tcaaagatcg caaacgatca acttatacgt aaacaaattg 420
ccaaaacgta tccaaaatat gtgactgtta aggatgaagg aagaagtttt gacagcgaat 480
catcaaatct gtccaattac agatggatca attccaaaaa caagcgcgat ggtgatcgct 540
gtggtgccat                                                        550
```

<210> 1328
<211> 550
<212> DNA
<213> Ctenocephalides felis

<400> 1328

```
ggcaaaagct cttgaaaagt tcttctgcga caagtgctag tgttagaaag ggcaaagttg 60
gcaggccgag aaggagtaga gattaactag tgaaaaaatg atattaagag aacattaaat 120
aatatcagac agtgattttt tccataaaac aatcgactaa tgatttgtta ttctgaatta 180
cttttcagaa ctgtacattt tgtttaaagt ttggtgaagt ggtgaggact tgataaaatt 240
ttatttaaca atgtttttta ttcataggtt agtttaaatc catatatgtg taattaaaat 300
attatttaac ataagatttt tacattatac aatattatat aggatactag cattatttca 360
gtaaatgaag gcatactgct tttgtgattt tttaatttta tggtcatcat ttacatttta 420
tatcaagtta caatcgtatg taaatattta atttaatata taagattatt atcattagta 480
ttagaatttg taattgagta gacaatcagc aaagctgtgt gattcgataa acattttgaa 540
tgnaagtaat                                                        550
```

<210> 1329
<211> 550
<212> DNA
<213> Ctenocephalides felis

<400> 1329

```
ggaaactctt aaattctctg atttaaaata tgaagaaaaa gatcttcata taccatctct 60
tcaggaggtc aaagaagttc tatctggaaa gttacctaac aatttcactc atgtctctgt 120
tgatatagtt gagtgccctg atttaaccgc aaaaaccatt ttgtctcgct gcaccaggtt 180
tgagtggaaa tcctaattta ctcgaactag gtggggctcc ttatctctta ccacttgttc 240
aaagagaaaa aatatatgat ataaaaaaca ttgctaagtc tttggacttg agtccggttt 300
tagctatagg agctggtgca ggaccatggc catatgctgg tgtatgttgt gagggtattt 360
tcaatatgca cctagcatct gacggtacct tgaacaacaa aacacacata gctactgtta 420

acatggaaaa tagtgcatgc gtacttggaa cggtccaaat gatgaacacg ttgtgcatta 480
ttaggaaatc tcttctgctg aagacatgcc ggatctgttt gcgtgtcatt gngcactagc 540
atggnctaaa                                                        550
```

<210> 1330
<211> 550
<212> DNA
<213> Ctenocephalides felis

<400> 1330

```
gcgcttcaga attatcgtaa ttgtcgaata aaatatatac tttataatac attcacatat 60
tgnatattaa tagttttaat tttaaattta ttactaaata gttttaattt ttaataagtg 120
gtgatacaag atttagataa gagcccgatc gtcccggccg gtgataacag cgcaacgccc 180
agcagagact tagcatcgag cgccctcgtc ggttctcaac ccctgccaaa tcgaattcat 240
tccgaacgca ngagacagcc actctcaacc ctattcggca taggatggtc agactgccct 300
gggcccangc gtttgnttaa caggggccgn taaagacgac caaccncagn caacgaatgn 360
tgaaataagc catccccact gnttcagtta aacgtgaatg gaagncatng gaacctnacc 420
cgatcccacc ttaagccaaa tcttcaccaa tcttcttnga acatcaattc ctcctngcga 480
agcngnngtc ccagtagtgn cgacgttata tgtaacaaac ncggtcttaa ggaagangcc 540
gntctttcta                                                        550
```

<210> 1331
<211> 550
<212> DNA
<213> Ctenocephalides felis

<400> 1331

```
atgaagcgna gcgntatcac taataattgn tatatcatca tctaagngat aattaaaact 60
aataaaatta agattttttc acttttaatg cnaccnncaa cgncatagtt tcaatggtgg 120
tgactgnatc tggctttgnt acnattgagt gcnaataatt gatgacacat cgttttgata 180
aataagaaga tcaaagtatg ncatacaata ttttctctag tatccgggtt ggaaacaatt 240
taatttatca aaattataat caacttttg aataaactat gatgacatga tgataagaaa 300
ctaaaatgat attacataat tgcatcaaat catttttcaa cccaatattg nttttatcct 360
gaaattactt gatattgaca tcatgctcaa attttatttc agcattttca tttgcatcat 420
gnaatcgtac ttttattacg tattcaaatt tacccgtcat ccgtagacca tagtctacga 480
tagtgatgnc agaagctgct tacactgncn atagtttctg aaaaagcgat aaatctntaa 540
atncgattaa                                                        550
```

<210> 1332
<211> 550
<212> DNA
<213> Ctenocephalides felis

<400> 1332

```
cgcacgtata attttcataa cataacctta aaacaaagaa cattttaata ggacttaaaa 60
```

aaggataaag gatactgaaa attagtgttt aataggtcga tagagactgn gcggttcaca 120
agtttttcca ggacattagg taaaggaata gttctcggtg gattttggat tataacaaag 180
ttaattttta tttttcgaca tgtgtatcgg ttaattatcc tttatcctga ttgngattgn 240
ggttttgttg tagttatatt cttaaaggca gtgtactgtg actgtgaccg cctgtgcctt 300
tgcatgtgtg tgtgtgttgt aagttgtaag agtgctttaa ttggtggaac cagttttgat 360
caagagtcaa aaggaatgac tttgaaggtg cgtcattgaa aacaaaatgc ctggtgcatt 420
tctacatatc cctaatcctt attagaacct aattaaaant aaattaaatc tcagcatcta 480
gatcaaatat gagnacnggg atttcttgta tacaggttcc tcattattac atacattcct 540
ccatactcat 550

<210> 1333
<211> 550
<212> DNA
<213> Ctenocephalides felis

<400> 1333

gtgttatcaa aaattttcaa catatgatta gaaggngaaa agttggtgtg aatgatgtta 60
aaatattctt atgataaatg nttttgaaaa gaagcaatat ggatttatca gatatagcgc 120
ggcgtctgaa gaggaacttt gatagctatt cctcatatcg tcgtttcctc attgttttaa 180
tactgtttat tctaatgctt ttatatatgg caccttcagc cttcagatgg ttgctatcga 240
gttctaagcc tctagaaaat tatgaatatc gttgtatatc agacagatta gcagcataca 300
gtttcaaaag tgccgaatat gatgtgaata ttagacataa acctctgcaa ataaatgaaa 360
aagattttat accatatgct ggtaacggtt tctttggttt ggagatatct gacataggtc 420
atataaatat aaaattgggt agatcactaa atcttcctat attttatcac ccattggtta 480
tgcatctgct gcaatggaaa tagcttcgaa gctncgttgt tgaatataaa aaaggatcat 540
tcacaaatcc 550

<210> 1334
<211> 550
<212> DNA
<213> Ctenocephalides felis

<400> 1334

gtataatgca ataaataaat ctaaacatat aaatcactaa attatcgcgt gtcggtcctc 60
aattgcatta gtgaaaacaa attgtgacca tacagctata aaaaaagagc gcgcgaatca 120
ttaattaaag tttggctaaa ttttattttg ttaaataaaa gtatgctctt tattttttat 180
gtgtagttat ctgtattata caatgagggg agaatattaa catttctagt aaaaaaaagt 240
taccggcata aatgtttgaa aatgagtgag cagatgagac aattatccaa tagtaaaaat 300
ctttcccttt ctgtcaatta taaatctcgt gacaaaccca taatagagga agctattgaa 360
acaaatcaag taaaatgtca gttgtcgaag agtttaatat ggggatgctt gaatgcagcc 420
attctcgcta taattggttt gatttaattg acgatgcttt atccgtggca gtgaccacag 480
atgaatgagt ttagcagttt atactattga ctatttcccc tgtgctgtat attatattca 540
actggtagac 550

<210> 1335
<211> 466
<212> DNA

<213> Ctenocephalides felis

<400> 1335

```
gtaacttgga aaaagagctg tctcatgcaa agaaagataa agaggctatg gtttcacaag 60
tggaacattt gcataacgaa tatgataata tggctgataa attagtgaag tatgaaagag 120
aacatgtttt taaggccagt ggagaaccag ataaaaaaga tgactagatt aataatccca 180
ttcattttaa ttttagattt tttggaattt tttgattcat ttattctcac atatgtttta 240
attttgttta attattatta caaagtacat attcaaacat atttaactgt taaaccatac 300
taatattgct tgtttcctaa attatttaca ttaaatttaa tatatactct atgctaattg 360
tttgttaaga atttgtattt gtgcttaagc attgaatatt tatgtagttt ataaaactat 420
ttatataata aaatttaatc caaaaaaaaa aaaaaaaaaa aaaaaa 466
```

<210> 1336
<211> 550
<212> DNA
<213> Ctenocephalides felis

<400> 1336

```
attattatgg gggattacag cactcacacc aggctcacac atttacaaaa agatttgcgt 60
ctagctttgg gtatggccga ggacctaacc ttacccatgc ctgtcacagc gactgctaat 120
gaagtgttga aacatgcaaa acgcttgggg tttggggaag atgatgtatc agccttgtat 180
ttcagagcac gtttttaaat ataaatttat atatggtttt ataatatata aaattttaga 240
ctaatttata tatttccaca acagaagcat ataatttaga aatttatttg tttataatta 300
ttgactaagc taaaattttg tacttttgga aaattatttc tattaggaga atgtagttaa 360
gttcacgtgt tggataattt gtgttttaaa tgttttacat gtaggtgcaa tgacatgtat 420
tgaagaatag taatttaaat ttctgtcata attttacaat aagtaaatgt tagttattga 480
tagatgctat gttgcatact atatggagtg nactcatttt atttagttac aaaaatttac 540
cgtatgaatt 550
```

<210> 1337
<211> 550
<212> DNA
<213> Ctenocephalides felis

<400> 1337

```
cgctaaggac cccaggaccg gccctgcatg tgtgtatata tatagatata tatatatata 60
tatatatata tatatataat agggtggatg ttttcgaatt ttcttgctca caggagctca 120
ggtgcttccg gattgataaa ggacaattct ctgaaagttt tagctctctt aatgggaccg 180
gccgtggtcc cgtggtaaga acgtgggcta gcaaatccta gtcccgggtt cgaatccaac 240
ctcggtcggt tcggtatcaa aacagcttga taccaaattt tcacttgaaa tcatagattt 300
caaatatgat ttcaagtgaa aatggccttt cgtgggcaaa gctgtagtgg caacacgcca 360
cttggaagta aagaggtact aagatggtat cgctttgaaa tcaaaaagga agtcgaattt 420
gtatctctat acaacagaaa aatgtaaagg ctctgacatc gtgcaccatg attgagtgca 480
tttcaactca cccacggcct gccttggaga atattcgtaa agaaaatgta taacagtaaa 540

atatgtaaga 550
```

<210> 1338
<211> 549
<212> DNA
<213> Ctenocephalides felis

<400> 1338

```
catcaagcaa gttgaactca ataaatagga aataaattct ttctttgttt ttgattaaat 60
agtgcttacg aggatataag gataatttca agcagatatt ttgtgtgaaa gttgtgcttt 120
tcaaattcaa gaacatttta gtaataaatt aaactaataa aaaagttgct aatatctact 180
tcggttttac tgcaatttct tggatgctac aaaatgaatt ctaaaatatt gtgtttcatt 240
atattttcca cctttttttct ggctaaaagt caacaaataa attactacgg caactcaaga 300
ctaataaatc ctggcccata caattaccag tatcctcctt taccaccaag gttgccgcca 360
attaattgtg aaccagtata tgccacagtt gatttctcat acttaagatt catgctggat 420
aaacttggtt caaagtagtt cctaaggata tcgtgaatcc tcaaaacttc ggncaattgt 480
gcaagcttcc anaggcggtt tgattcagga agtgctaatg tagtgaagac gacagatagg 540
tttctggga                                                         549
```

<210> 1339
<211> 413
<212> DNA
<213> Ctenocephalides felis

<400> 1339

```
caatgattga ctgctatctg atactagtga actaaaatat gttcaagtgc ttattctata 60
aactatcaat ttattcaaag aaaaatcact ttgaacataa attgcaactt attgtgcgaa 120
tcattggtga tatattgttt tccaggcatt aataactatt tgattaaata aataattaga 180
aatgttatga attgtaattt gctctttact attattattt gtaaatttgt gatgcattta 240
aaaatttaaa aatattcatt tgttgaataa tatttgaaat gttttatgtt ggaccattgc 300
aaagtgagca aactcaaaaa tgtattaatt tttttttttt gtatatgtac tgccaataga 360
aacaattttt tatttaaata aaggcacctt gaattaaaaa aaaaaaaaaa aaa        413
```

<210> 1340
<211> 549
<212> DNA
<213> Ctenocephalides felis

<400> 1340

```
attattttaa gatgatatga tgctataatg gtggaatcca aaattaatat ttggaatgat 60
attttgagag ttttcattaa ttgtgtgatg tagaggtgaa aagtataaaa aaggcatgta 120
ttaatcaatg aagcatattt aattttaaca attcattttg ccaaatatga cttttaatta 180
aaatttatat gattaatatt attttaaaaa tcaatacaat ttcaactgct tattcagatt 240
tacatgcaat atgtttcatt taattttatt taatatatta atagtccagt tgaatcaatg 300
ggaaggcaaa tactacatca tgtaattgtt gaatactctc ctatgtactt ttagaatgat 360
```

```
atggattgaa cgaatgtatg aatgagagtt aaaacaatca tattataaaa ccaatttaaa 420
tcaaaacagt catgaacctc atattatgcg aaaaactgcc aaaaggtttt tttaagttgt 480
tgagactggc cttgtgacat ttcaaaatga tatttgagta taattttata ttatgagtct 540
tccaaaatt                                                         549
```

<210> 1341
<211> 549
<212> DNA
<213> Ctenocephalides felis

<400> 1341

```
cgaaaccaac tgaagacgtc acaaactggt caataaaagc ttattaattt ctcgataaat 60
gctattttat caagcgtttc cttgttaaat acaaaattat agtgattatg gatcgatttc 120
ctaaatctct tgaaaatgca attatcgaag cgaaaaggcg ctttgaagaa caaagtgctt 180
tgacgtcaga gttaggacca gcaatatttg acacccgtga tgctgctcca tttgctctac 240
caagagcata tgtaacaggt ccatgtttaa gctctgacga aagttcaaac aatagtagaa 300
cttctgaatc agcagttgtt gctgtcagtc accatacacc gaatgcatta acatcatttt 360
taaatgatcg ttattatatg ggtcacaaaa aatcaaggaa atgttatagt attcaatcat 420
tttcaattcg atgactttga aaatcgcgat ggacacatga agatgtagaa gaattgaaaa 480
tcttttggga taaggtagga tcaagngaag ttatgatgac ttacaggtcg caatactgna 540
gtgtcatng                                                         549
```

<210> 1342
<211> 549
<212> DNA
<213> Ctenocephalides felis

<400> 1342

```
aattaatggt cctttcgtac taattaattt taaaaaatta aggatagaaa ccaacctggc 60
ttaaaccggt ttgaactcag atcatgtaag aattaatggt cgaacagacc aaattttaaa 120
acttctgcat tttaaaatta tcttaatcca acatcgaggt cgcaatctat tttgtcgata 180
tgttctctta aaaataatta cgctgttatc ccttaagtaa cttaatcttt taatcataat 240
ttatggatca attattcaat tatttatgtt ttaataaaaa aaaagtttta taaattttcc 300
tatcacccca ataaaatata ttaatataaa taaatttaat aatattctta aaattaatct 360
atatttatat ataaaacttt aaagggtctt ctcgccttta ataatattta cgctttttaa 420
cataaaaatt aaattctata caattttatt aagacagtaa tatttcattc aatcattcat 480
tccagctttc aattaaaaaa ctatgnttat gctcctttga cagcaaatac tgcggctttt 540
aatctcatg                                                         549
```

<210> 1343
<211> 549
<212> DNA
<213> Ctenocephalides felis

<400> 1343

```
aataaactta ataacagacg tttattcaaa gattaaaatg cgtataacta atttacaatt 60
aatgatgtgt ttgatcaatt aaattaaaat tcttgggcct ttgcagatta ttagcctatg 120
aaaaataact tttgatagtg acgtatactg cacaaataac tctaaatttc ttgtaaatat 180
tcttgacaag atggaaatgg aaacagatgc taaaaaggac aataaaaaca tggctcatgt 240
tatgctaaat gaagtcactg atatgctaga agatgatttg cagccgatcg aacaatatga 300
ttacattgcc ttagatgaat tgcaaccaat ggaacaaggt cttgaacaat atcaagaaac 360
aatggaaagt gaagaaaatc aaagtgaaga acagttgcaa cagcaggaac ctgaagttga 420
agaagtttag tgccagaaat agaggaaaaa ccaattttgt caaactgtcc tctgtagaac 480
aatagctcca acctgagtct caaattaatt gggggctca ggcagccaat tgtttatcaa 540
ggcctattc                                                        549
```

<210> 1344
<211> 549
<212> DNA
<213> Ctenocephalides felis

<400> 1344

```
atcacataga tcaataggaa tttatccata tcttagagtt ttgaaggtat ctgattatgc 60
tgacattgtt ctaagagaag ctcgtagaat tgccgaaggc tctgaaactt acagtccaac 120
agtaaatcaa ttatatagag atttaggcat aagagtacaa tccaggtatc atattgaagt 180
caaacaaaca aatggtgttc tggaaaaagt aaatgaaata tatgatcaat attgtttaca 240
aataagtgaa acttataaaa aactgcaaga tattaattat ttatctgaag agctggattc 300
attagataag ttaagtgatt ttaacacaag gcagaaatgg caactgctgg ttcatcaaga 360
aaatcatgga gcaagcacag atttagaaga agtaaattgc cttattcagt gtgctagctg 420
tggaaaattt tgtacaatat attgatgcgt gatttaaaaa tagatgtaaa ttctatgaag 480
atgatagtaa acaaaagaan tgcttcngat tttacactta ttagaaatca aggaaattgt 540
aaagaagag                                                        549
```

<210> 1345
<211> 549
<212> DNA
<213> Ctenocephalides felis

<400> 1345

```
cagcgatcag atttaccaga cacatctcat tggcaaagtt caatttccaa agatgaagct 60
gcntttgaaa tagactactg caatcctcaa tcaagcaatc aacattcgaa gncaactaaa 120
gataataaca atgacggtac tactgntcca gacgaagatt ttttttcgct cattatgaaa 180
atacaaagtg gaaggatgga tgaccagcga gcaagtataa atataaaacg agtaatatag 240
aactctactt taataattgt aataatattg tatatggatt attagattac ttttaatact 300
agaatatttc caatttttta atatcatttt ttgtggatta catacataga atagtctggc 360
tatcgattgg tactttgact atgaattgtt gtacctttga accgcaacaa tttctaatat 420
aaaatgagta gaaggtttat tagcgacata atagtgacat tgctataata tagcatttaa 480
atcaaacaaa ttaaaaatgt gattttatta ataggtacta tcataaagtc acaaaagccc 540
ttccggtac                                                        549
```

<210> 1346
<211> 549
<212> DNA

<213> Ctenocephalides felis

<400> 1346

```
gtggattagt atatcaacct ctatcagaag aaatcagatg tccaccaacg ccaatagtga 60
cacgctatcc aagctatttg agcagtccgt tccatcatga aaaaattacc cccgaacaag 120
tgtagcttta aaatatttaa ttctgtcgca aaatttgctt ggttagtctc catcgtaaat 180
tgatttgcaa ataacaatat aggaatttta acagcagcgc tgtttgtaca aactgtagtc 240
aaagagaaca aacctttagg atcagaatta atttatgtta ctttggctta tacattcctc 300
gatcaattta cttaagtaaa atagtacact gtggatactt caatgagata ttgtcatatg 360
taacggcatg ggtaataaat aatttagttg tttatactgt ctacttaaaa taaaattggt 420
taaatatgac tttataatta aacatattta accacaggaa attagtataa ttagtaatga 480
ttttaacatt acattgcttg aagaagttat gccagctcat gataatatct tattgtagtt 540
tatgataag                                                         549
```

<210> 1347
<211> 549
<212> DNA
<213> Ctenocephalides felis

<400> 1347

```
gcaaaatatg atcaatactg tatatttaac atacacgcgc acatatgcat atatatatgt 60
atatatatat atatatatat atatacttat atgcatataa agcaatatca acatctattt 120
agttttcgat attctgcaaa taataatgat cttaaattac aaatagaata ttacatttaa 180
cagctaagaa tttgcagttt cacaaatact gccagtcacc aaatggtatt cagaatatca 240
ttcaaaacaa tttagccaat aatgtaagat gaaatacaga tgattataga aacattcaga 300
atttatacac tataaatatg aacattaata ctatgtacca cttaaaatgt gaaatctgaa 360
tacgtcattt gatgactgcg tgtaacaaga acttgttata gattataaca attataattt 420
aatatatctt tttcattttg taaccccaaa agagcattcc tcgcgtcttg cacgacttgn 480
ggggtgtggt agtgtgatga tgatcatgta gatggtgatc cttatatcac ggttgggtga 540
cgagattat                                                         549
```

<210> 1348
<211> 377
<212> DNA
<213> Ctenocephalides felis

<400> 1348

```
attttatgtg ccagtgctcc taataaatag ttaatattag ttcatatttt agaatcaatt 60
taagtttact tgtaaaatag atgcagtata taatataaat aggggtgtgc attttaaagt 120
ttgcattgta aaaccaaata cttctcatat tctgatgcaa atacttcaat aatttgttat 180
tgttgcaata agcaccccaa actgttgtaa atgaagttgc atgatgtgtt aaaaatatca 240
acgaatattc aacaacataa gtcaagaatg aaatgtaatt tctgtatata taggtctgca 300
tatgatttgt ttgttacgat gtgaaatttc aataaattgt tgacttcgtt gaaaaaaaaa 360

anaaaatnaa aaaantg                                                377
```

<210> 1349
<211> 349
<212> DNA
<213> Ctenocephalides felis

<400> 1349

```
cattcggttt tcacaaataa tacaaaaacc agattattaa aatgattgga gtccgcgcca 60
tcacaaagtc gtccccagtg gtcagaactc ttttgcaaca gaccaggaac ttcaatgatg 120
cttatccagt agtatcaggg cccccaagga ctaagatttc tactgctgag aaaattgtcc 180
atggagccgt catcacagtc ggttgcttgg ccatccccgc ttgggtgctc ctccatttgc 240
aagaatacaa aggagaacaa taaacaatga aaattcttag taaatgtgtg ataagtgtaa 300
atttaactaa atacaaagga ataaagtaaa aaaaaaaaaa aaaaaaaaa             349
```

<210> 1350
<211> 549
<212> DNA
<213> Ctenocephalides felis

<400> 1350

```
cttcaaacct ttaatgtttt gaaacacaaa ctgttcataa tcaattgtag ttttgtgata 60
aaactcaata tgatcgtata aacctgtgta cctagaaaaa acagaggagc agggagggag 120
tggacatttt attctggatt tccttgatgg tgtacctata aaattttcct tagtactgat 180
tggggaactg ggaatgggag gcgtgtgaat ttgaacttta gtagaggaag tacaaggatg 240
tggcgataaa cttacgtggg aatgtttgtg aagacgatct gatatggaag gagcagaggt 300
aaaagatgag caagcagaaa gtggatatct tagtttctct tttgttgatg gtgtacctgt 360
aaaatttcc ttagtactga ttggggaact gggaatggga ggcgtgtgaa tttgaacttt 420
agtagaggaa gtcaaggatg tggcgataaa cttacgtggg aatgttgtga agacgatctg 480
tatggaagga gcagagtaaa agatagcagc agaagtggat atctagttct ctttgtgtgn 540
gtcctgtaa                                                         549
```

<210> 1351
<211> 549
<212> DNA
<213> Ctenocephalides felis

<400> 1351

```
atatgaagaa aaatcctaat aatccagtat taggatgcaa aacaagaaca catttatcga 60
agactgccaa agtatttggt gatgaacagt cagattcttt aaattttaaa tcattgcttt 120
ctagctgtcc tgaatttgca tctaaaccca aagctgcaaa caagcagtac tcacaaaaag 180
tatttaaaca agttagatgc aaatctaaaa ccttagaaaa tcaattagaa gttcatcgta 240
gtttaatgtt aaaatcaacc gaagtgcata gttcttgctc tcagcaagcc agaatgaatg 300
aaccatgtga tataacaatt gatgaattag catcatattt tgagactttt gttcacatac 360
ccaaaaagat gtcttcgatg gcagaaatga tgtatattta atgatatttt tttcatattt 420

gtaaattgtt acttataagt tctctatatt tatgtaaatg aattagtgta atattgataa 480
agtccattag ttcatgtttt gatacatata aatcaaatag atatacatca tctaagctta 540
ataatagag                                                         549
```

<210> 1352
<211> 363
<212> DNA
<213> Ctenocephalides felis

<400> 1352

```
gcngttgtat cagtacctca tgatgaagtt tgcgatttga tggtcggatt tgttgaactt 60
ttaccaggat acgaggacaa ggtcactgct gaagaactgg aggaatatac caatgaacac 120
gttcacgacc acgaaaaact ccgaggaggc ctttacatag tgcaggagct gcccgtacta 180
acaaatggca aaaaagataa accaacagtc cgaaaaatgg caaaagaaat gtctcaacag 240
atctatgaaa aatacgacaa ctcgactaga aaataaaaga caaaacacct ttgttaaaat 300
agtttgtaat gtagatttat gataaataaa attgataaaa attaaaaaaa aaaaaaaaaa 360
aaa                                                               363
```

<210> 1353
<211> 549
<212> DNA
<213> Ctenocephalides felis

<400> 1353

```
aaaatggctg actccggggg tgaaaaagga attatagctg ataataaagc tggtgccgcc 60
cctaccggtg atgccttaag acacagtcga ccttcatccg ttaataaagg ccgtaggcga 120
gtcaggccca tgtcgatgga tgctacgccg atcaacgcgc acacgatgca ctggttcgcc 180
tatctcggcc cggaaccagt cgccgagcag caggcccaat tgtcggccga acaacagggc 240
cagcctgacg ctgcggcctc tgccgatggc caattgtcgg atggagagaa cgaagatgtt 300
agacgttcaa cgaacttcga tgccgaaaac aacgacccag gtggtgcttc ggcggccggg 360
tctcgcgacc gtcccgcgac ggacgagcga ctgcggcacg ttcgcgacag gcagcaggaa 420
gaacgccagc gtcggctcga ggagctgagg cccaacgttg gccggcagag gttagggaac 480
agaagaggaa gagaggagga aacggatcga ggagatcngc tgaggatatg acagcgctct 540
agtcgaaga                                                         549
```

<210> 1354
<211> 384
<212> DNA
<213> Ctenocephalides felis

<400> 1354

```
attttgataa cgattacaga tatctacact ttgtcggaag gttcgcattc aatagtttta 60
aaaattaaac gatatgctta attagttcag aacctaaaaa atatcataaa caactttaat 120
ataaattaat gtaatcacat cattatgctt tatagtaaat attttaaatg ttataaatta 180
tatattttga aaacatagtt attctatgca aattacgcaa atatgaacaa atttttatca 240
acataatatt atttatgata taatagtaat gtccgaattt aaaatatacg ttctcgtatg 300
aaaatattat acaaatcaaa caatgcctat tcgaagcgaa atatctaatt aagaaattta 360
tttaaattat aaaactgcca aaaa                                        384
```

<210> 1355

<211> 288
<212> DNA
<213> Ctenocephalides felis

<400> 1355

```
gttaagcatg acccagagga attgaggaat ttggctggaa agccagatat aaaggttact 60
aatgcataat aagaacttaa attagtaatg cagatatcaa attcattata ttgttatagg 120
atacatttga agaactgaag aagagactac attcttggca gcaacaaaca aatgatccct 180
ggcgttgtgc tccacattct gtgcttatgg acactcctgg tataactgca gaatgtttgc 240
cactatataa ttgaaattgg aacaaagctt aaaaaaaaaa aaaaaaa             288
```

<210> 1356
<211>549
<212> DNA
<213> Ctenocephalides felis

<400> 1356

```
acaagtctga gcaggtgcgt catttatgtt aggtaatata aaatttatta tgatatgtta 60
attttaaatt ttataaatcc tattcttttt taaatttatc aatgtataat aatacatttt 120
tgtaattgct actgaaaatg attaatttcc acttcaattt caatttttac gtgattcggt 180
agaggaatag ttacagtata aatataatca atggatttca agatatgaat ttagaaaaaa 240
aatgtttaaa ccagccccta gctctcctac cataaattta tttctaatat ataaagatgt 300
atcagattaa caactcagtc tttccataat tcttccaaga tattataaca gcatgttgcc 360
atgtctgatt tttttttcat taaaatattt aaaaaattag ttatctacgc gtgctccatg 420
cgtcttacac gtccttcttg nagaaacaga ttatcattat atatatttat atcataataa 480
taatcgaatt tatagattta tcgtttttca gaaacttgtg caggtgataa ncagactctg 540
nctccctat                                                         549
```

<210> 1357
<211> 549
<212> DNA
<213> Ctenocephalides felis

<400> 1357

```
ctctcgcagt atggtccttg gggctttttg ntaataggcg ttttaatttg cggggtcatg 60
ctactgattt gcggactttg ggagtgttac tgccgccgac ctcaacagcc tagtccgcct 120
ctggtagatc cttgtgcctc aatctctcct gataatcatg tggtagcaag taatttagaa 180
aatattagtc cgcctctcta tgatgaattg gacactccac cagcttataa tatcttattt 240
ccgccggaac aaaaaagtat acctgtagca acaaccagtg accagggaac gagttctggt 300
aatattccag tgtaaatgaa gattactatt agtttttatt ttgttttaat gtgtatcaca 360

aaaaaacgag tttatggatc ttaagtctta ttaattgctg aactaaatga atgagatgcc 420
ctcctggtgc atctcagtga atgtgaagat gatgaactta aataatgacg cttgcttagg 480
ttcaggactg cttaactgtg attcttctnt cttattcang aagttaaact atcttgaaac 540
aacaagtaa                                                         549
```

<210> 1358
<211> 497
<212> DNA
<213> Ctenocephalides felis

<400> 1358

```
gctnatgtca tttatacgct acatttatac gcgctaaaaa tttacaaaca aattttctgg 60
gtcgttaatg ggacatttaa cagatagcca gtaactgtca aatagtttat ttattaaatt 120
gacatttttt aattattgta tttttaaaca tgttataaag cacataacct atatgttata 180
gtatttttta tcgcgttaca tttacaatta tgtatactac aaaaatagag aaaaacatgt 240
gctctatttg tgagaagcaa tatgatcgtg attgggaaat tgaggaaatg caaacaaata 300
ttgctcttga acctacagaa gaggaattct cgagacgtac aagttcccgg tcaaacaacc 360
aaataagtac tattccacaa aacgaaagca atatgatgat tttagaaatg ttagccaaaa 420
tgagtcatga aataaagcaa atatctcaag aacaaaaaga actttccaaa cttctgaaaa 480
aagaaaagac aaaaaaa                                                497
```

<210> 1359
<211> 549
<212> DNA
<213> Ctenocephalides felis

<400> 1359

```
gaactttatt aaaattcaat agttacaatt taagtcattt acaatacata attattctaa 60
aaattcattt ataaaataat agggattatt tagtaaataa tttttaacaa tatttttaaa 120
ggtgaatctt gttttgcatc tccatccatc aggtagctta ttatagatct tggcacccaa 180
tgtagttctt agcgatgatt tgagtttgat ttttggacat atcgctgcat ttttattccg 240
cgtattgtgg tcgtggacat cacttctcaa taaatgatta gttttatccg catgcaccgc 300
aatagcttgt tgccaaatat aaatgtttgt gacagtcaaa attttgtgtt ttatgaatag 360
cggcctgcaa gactcccgag gtaagactcc ttctattgct ctaaccgctt cttttgtaga 420
tccataactt tagaagtggt gcactatatc cccaagctaa tattccgcgg acgagtcact 480
attaaagaac gtgactccaa cgcaaaggcg aaaaccgntt naggcatggc ccctccggac 540
catcccccta                                                        549
```

<210> 1360
<211> 549
<212> DNA
<213> Ctenocephalides felis

<400> 1360

```
cataaaaata atatatattt caaagccctg ngttatgtaa tgcttataaa attcttaaca 60
```

```
attatatgta tatatgtaca tctctttcta actcaaacga ctactaatac tgctctctcg 120
ctctgttacc atagttgtac ggctgtgcag tcacaggtct caaatgagcc cttggaagac 180
cttgagcatc gtaatcaggc tgcggaataa ctggcatacg gcttccatac cgcgtagcag 240
gattatcgta atccgtcgtt tgagtcggcg taggttttct tgcaaatctg gtgtttccta 300
cgactgcagg ttttggcaac actggtggtg gagttccgta tcctggtgaa tttggattgc 360
taactgggta attcaagttg gagccacggt cgtctggtga cttcggggcg agaatggagg 420
gtttaaggga gatggtttga ttgctcattt ggtcgatcgg accagcgtag cattgtgttg 480
taaccttgtg gacttgatat atatgcgtca aatgtntccg ataatctgtg anggtagact 540
ctgtttctt                                                         549
```

<210> 1361
<211> 549
<212> DNA
<213> Ctenocephalides felis

<400> 1361

```
ctgtgattat ttactatgtt tgtcgtcgaa ttataaattc ataaaattat ttagttttat 60
agtgaaattg aaatattcta aaaatgttga gtaattctga tttcttaaga agaccggata 120
gatctcacat aagatcatca agaagatatg actgtgacga acgcgcctct aaacgacgaa 180
aggattcatt ttatgaatct tttatatctc atagaactat actagtagtt gaaaacaaag 240
aagagcctac atgcaaagtt tcaagatgcc gatggtctgg atttgatgat gaattattat 300
cgcattgtat acacaaacac aacgttttcg aatcaaatga acaaaatata gtcgagtttc 360
agaattggca accaagtgat atctacaaag gagttctact taaatttcgc agtttgctgt 420
tgtggttatt catggaaaat gacggtctaa tattaatgta gtgncagaac gcagctgcga 480
tgtcacgagc tgtggattag gtcaatgtag ctangtcaaa agatgtcaag aatgccagaa 540
tctgtaaat                                                         549
```

<210> 1362
<211> 549
<212> DNA
<213> Ctenocephalides felis

<400> 1362

```
atttaaaaca ttccatataa atgtagtttt ttagctattg tttttctgtg ataaattccc 60
aaaaaaggac atgacattat agtttgaaca agctgatttt atttataaaa tataatttat 120
tgtatatgga tgtgtgattg tgttaggtat tgcatgggta aattttgttt ttaaatatct 180
tcaaaacagt ttgtggtaaa atatttaatt taggaaatat tgttatatca tgaatatgcc 240
aactgaagta aatcaaatat aaaggcactt tgtttagata tttaaatatt taaaatgttt 300
gttaaatata tatatttgtt atttaaatgg cctgcaatat tactgatatt gcctgttcaa 360
atctctaaat tctagtaata tatgtcacca ttcatgtgca tttattcaac gattgtttta 420
tgaagctctt tacagcttcg cttatagtta aaatatttag cctaacatta ttaatgatat 480
tatcacatta gaaatctgtg aaaatattgc tgntaaaatg tatgtgagtt taaattgcaa 540
tatccactc                                                         549
```

<210> 1363
<211> 549
<212> DNA
<213> Ctenocephalides felis

<400> 1363

```
ctttgacaca tctaaaaatt attttaattt acaaaaaaga acttcataaa tttaaaagag 60
agttaaataa aaatttaaaa aaataacact atttaaaatg caatatttaa acatattgaa 120
taaaataaac aatgttgtta gagttaaata tttatacgcc acataggttt taaataaata 180
aataattatt ttaaaaaata atttatataa attactttga acaaaatcta gcaataagta 240
aaaatgggtt ccgctattaa acaatactat gcagcttttg tagccaacat cgccacaatc 300
tgctacggca ccacaatagg ctggtcaagt ccagccctgt cagccctttc aacatcgaac 360
ccttcaccag gcaaagacat catcttccaa ttaaccgacg aagaggcctc ctggataggc 420
ggtttgattt gcataggagc cctttttgga ggcctttgtc cgcctggttg gcaggagtca 480
ggggcagaaa gttgtggata tatgacgtcg nccatcataa tcagtggctt tgctctgttg 540
cgaattgtc                                                         549
```

<210> 1364
<211> 549
<212> DNA
<213> Ctenocephalides felis

<400> 1364

```
ccattcgccg tattttctta acaaaacaat atttatacaa aatttatgtt caataacact 60
gtgtaaaccc ataacttatt cataactatg acagcaagta gaaaaagcaa gagttgtgct 120
aattatacga tgaagaaagt agcagccgat catggaaagt tcctaaatag gatcaccaaa 180
acgctttatt atggaaaact accgaatact gatcgtttta gtatacccat gtcagaattg 240
gcagtggaga tgttctccga agcccatcgt ggccacactc tgcaaagact acaaatgaac 300
tcggcttgca acatatctcg aaatgcctgt gtcaacccct gcgctctcgt attggctatg 360
ttatatttgg acagactacg tgattgcaac cctgaatatg tgcgacaagt agcccccagt 420
gaactatttc ttgatctttg atggtctcta gaagtttttg catgacgatg gagctgaaga 480
tgaagtattt ttacacgaat ggctgctcag gggtattca gtacagctta aacactagaa 540
aaagattct                                                         549
```

<210> 1365
<211> 549
<212> DNA
<213> Ctenocephalides felis

<400> 1365

```
ataaattcat ataattaaat ttgtaaattt tgaagacatt tataaaacca aagtatttat 60
ttcaatttaa tacttgaaca ttaattttaa aattgattaa attataaagt aaattaagat 120
aagataataa gtatagagtt tcgtgattaa atataatagt ttttatattt ttatataata 180
attttatcga gataaaaatt gataaaggtt ttttctttgg atcttaaata ctatgaatac 240
ctaatacaag ttatgtatta tatatttacc attgaggtaa attttaagca gttttgtcat 300
ttataatatg ttgaagttga tttaacaaaa aattaatcgt ccttatgtaa aacaatatat 360
aaaatatttc tctagaacca acctcaatga tttttacagg tatataacaa aataaatata 420
aatacaaaat ttatattaag ttgtaacatc aacatatact tatattaata tctgaaaaat 480
tatatattca taattattac cctatctaca tattatatcc tgtctataac ttcattcaat 540
ccatattgg                                                         549
```

<210> 1366
<211> 549
<212> DNA
<213> Ctenocephalides felis

<400> 1366

```
atttatgcg acattttggg ttatatgatt tttaaacatc tgaactgtaa gtcttttttc 60
atatagattt attaaaaaga atattttaaa aattgaattc ttcctaaact attgctaagg 120
aataaaaaaa acatcgtcat acatttatat acttagctaa ttactgtaaa attgtaagaa 180
ttcattttat accttaatga ttaaccatat tccaattaaa acacttagaa gacattaaaa 240
aaatcaaaag cactaaaaac tgttatggaa aatcgaaata tatgtctttg ctcttcagag 300
caccggttaa acaacaatca attcattttt ttttaaagat tattcatata ttttgggtgt 360
caatgatctt attagtttaa atatttttgc atctgtagaa ttaactgcag tagtattttg 420
caatgcattt tcaaagttta taatgcgttt tgaattggaa atgattgnca ttaaggtgna 480
ggtgaatctt catgtataat attaaataag tatctaaatg ttttttttggt tattatcaat 540
agttttggn                                                         549
```

<210> 1367
<211> 371
<212> DNA
<213> Ctenocephalides felis

<400> 1367

```
gttatgaatc aatggcatca cctcaaagtg aaacttctat tacaagcagt gaaaattatt 60
gggatgatag actttatgaa ttgtttccag atttggaatt ctaacagttt aaatatttac 120
ttaatatgaa atcattccta tttttatatt gatattttta ttatgaataa gttcataatg 180
gaagtaccaa ttgtttaaga atgctatgcc atcattcgcc taatattagt aatatatgaa 240
atcaaattta attatgacaa ataatatata atgtttagca tgtcatattt atttaaagta 300
ttttgtatat tattattaca aattaaaaat agcttatcaa ataaaatata tataaaaaaa 360
aaaaaaaaaa a                                                      371
```

<210> 1368
<211> 549
<212> DNA
<213> Ctenocephalides felis

<400> 1368

```
gtcactggtt actggtcact ggtcattaac ggcgacacgc acgctcgtta atttacaatt 60
gtcctgaatt tgattttttt atttggcggt tccatgagtg attaattcta ttaaattagt 120
ttgtgtatac ataatatgtg tttgtaatgt acattggatt ctgattttaa cacgtgcttg 180
aaccagtatc atccgagatg ttgacaataa ccagtactta gaattatcag tgccattaaa 240
```

gattcgtttg tgcagaaata gacggaaaaa gtaattttaa tattgttagt gatttttttg 300
taacttattt gacaatgacg agcacaattg gagtgggttg tgacattcaa gttgtggaac 360
ctaaatcttg ttccttaccg gtaccacaag aacctttaag ggagccttta ccatcagaca 420
atggttcgtt ttatagtcgg caaggtccca aagtgaggcg ctcacggatt gccgaagacc 480
ctgtggatat acagtcaaag acatcaagat gccgctcgct gggttcagga aagggataaa 540
gaaatttac 549

<210> 1369
<211> 549
<212> DNA
<213> Ctenocephalides felis

<400> 1369

aacgcgctaa caagcgatca cgaacccaat cactaatagt acgatcagta agtgaatcac 60
ttcactagtg atataaatgg tcaccgatta aacgatttac cggtaaagtg ttcgtgtgag 120
ttattaaagt gtcggataac aaaatcacag gacgaagcaa gatgaatttg tgcgtgccac 180
tcttaaattt attgatcctg gcgctggttt tgatagcgca atgtcatgct tcgaaaattc 240
cggagggaat gcctttaatt cttccaaaag atgccacacc ttcaccctta ttgagagatc 300
cacccacacc accctgctt ctcccaaggg atgcgactac tccaccaccg ccaccactgg 360
tctttccaga agatgtcaaa aactacaaca tcgtaccaaa taatgcaacg atcttggcaa 420
ctttgacgca taaagacgat accaatcagg aaagcgctga ggataataat caagaaagcg 480
atgatttaaa taagacgccc ggtcagaggn cctgcaagga agtgtatagc acagaagtac 540
agagaggtt 549

<210> 1370
<211> 549
<212> DNA
<213> Ctenocephalides felis

<400> 1370

gaagaaccag ggatattaca ccattcataa taaattatat tgcgatattc acgccaagct 60
cgttgctcgc caaaatccac cagctggtac cgaaggatgc attccattca ccgtgccacc 120
cggcggaaaa attccggtga gcgctatctc cgcagctctg gctgcccact cgtctcatcc 180
acttaatgga ggtatggcac cacctaagga ccaagcaccc tctaccccgg gatatgcacc 240
agtttcagca ccaacatgca ccaatctgtg tgttcaagtg tctccttcgg ccgtcaatga 300
tactaaccag cctaaccagg ttacatacga atccaacctt ctagtacaga ttgctcccaa 360
cgatcccaca tatactaatg acgaaacttt agagaaacaa cggaaattga attcaccaaa 420
aagttcactt gtgaactatg tgaaaattac tccgtataat aacaaccatg aaactagaaa 480
ttcacctacc tcgattgagc acacacatct ctaaatcaga aactaatcat cccgaatgga 540
ctgtattaa 549

<210> 1371
<211> 550
<212> DNA
<213> Ctenocephalides felis

<400> 1371

```
caaacttatt gttaacataa accttaccac ttttacgacc tgttcaacac ctccaatctg 60
aaagtaatgt cgatttaact cgggtcgtat aacacgttgg gtatgacggt ggcagagaca 120
gaaattgtgc aatcagaaag ataattcgga agaaaaaata tatttatata tagatttgaa 180
actgtcctga ctacgaaatg taacaaatat tatgatttac aagtatagaa acatggagtg 240
cctagcttca ccaagaaatc taagattcaa aataacttta tggacaaccc tatatgtagc 300
agaagtgatt atgtgcaagg agaagaaaac tgtgactaaa aatattgtaa acatttagta 360
ttaacaattg atatttctat tgcaacttta ttggtaaaaa accaaaattt tcactactat 420
taatttacat taatagctac taaataattc aatcactcta aagaatgact gttttcgatc 480
ctactccatt catctataaa ctggtctata tgcaaagtat gagacaaaga agtttactgt 540
ccggtgtcgg                                                        550
```

<210> 1372
<211> 550
<212> DNA
<213> Ctenocephalides felis

<400> 1372

```
attttgtttt acattaaatt tttcaaattc gatatgaaat ttttactggc aatttgcgtg 60
ttgtgtgttt tattaaatca agtatctatg tcaaaaatgg tcactgaaaa gtgtaaatcg 120
ggaggaaata atccaagtac aaaagaggtg tcaataccat ctgggaagct tactattgaa 180
gatttttgta ttggaaatca tcaaagttgc aaaatatttt gcaaaagtca atgtggattt 240
ggaggtggtg cttgtggaaa cggtggttca acacgaccaa atcaaaaaca ctgttattgc 300
gaataaccat attccggatg aaagaccaaa ttgatataaa ttactaaaat tatgctagat 360
agcaatcata aaattttgaa gttttcaatg atcctaacat gttttgcctc aatttatttt 420
aacagcaaat tgtggaacta ccgtccgtac aaatgtcaag aaatctgatg ttacaataga 480
tattataata tgtacattgc tatattatag aatatatact gattgcaagt tgaaaaaaaa 540
aaaaaactgn                                                        550
```

<210> 1373
<211> 550
<212> DNA
<213> Ctenocephalides felis

<400> 1373

```
gcaaataatt ctgtgttgat atatcttgtc atattaaaaa caagtgaact tgtgaaaatt 60
tgcaattaat ttgggtgaca ataatgagac ttattatatt tatatcgtgc ttggcatcaa 120
gtacggttta aatgtaccta tagtcaaagg cataataaaa acgctagtgg aatttgcacc 180
aagtgttatt gtataaaact gcaatatgat caatataaaa agtatatgtc cttatttttt 240
tgtttatatt ttactttcat cttatataaa cactatttta tgtatcccgg taccaatgag 300
gcgctgtgca aagttataca actcaataag tcctcaagcc tcggcagagt acgatgagaa 360
tggcgatata tcaaaagatc tggtcgaaaa tctcttacac aggtgtagaa caatgcacaa 420
gtaattttgt actcttgtgn cggaaaatga aacggtctct catgtatggg canggtgtgg 480
ggaactcaaa taaanaantn gtntcccaaa naatgnttac atgncctttt ntaagcnttt 540
atccaagatt                                                        550
```

<210> 1374
<211> 550
<212> DNA

<213> Ctenocephalides felis

<400> 1374

```
taancaaatt cgttagtttt gaaacaaaac attnttttaa gctttaaacg gatttttttat 60
cacctgagga aatcgattta tacaattttt ggaactatgc gctggtgttc tcacttttct 120
gatttttttaa acagttntgt gtgagatatn aggntcgtag gcctttgaca tatatgccgn 180
acnagatcct cccaatttta ttattggagn nggtcctaat ttanttaccc cattccaaaa 240
attttaaatt tttgccaaaa atgcntttct accatggtta aagctcaaat atccggactt 300
ttttattgcc ttattattng atgaataatt tcgattattt gatttcgatn caaaatctaa 360
atgaaccagt ttaaatccaa atcaaataat tgaaattatt tgtaacaaaa aaaaaattta 420
taatcccaaa aatataaaaa atatttattt ctactcattg aacacaaaat tcacatagaa 480
aatgngcctg tttgctatac aatatagatt aaaatntacg tncgtatttt aataattttt 540
gcatatttat                                                        550
```

<210> 1375
<211> 348
<212> DNA
<213> Ctenocephalides felis

<400> 1375

```
caatagaaat taatgaagga atgaaaattt attagttatg tttgacctca tctagtccgt 60
tgactttggt ttaaaaatat aacgtaattg agttaccagt atgttatatg aagaaaccag 120
tttttctca ggttagtaag ttatattctt gtttgttttg ttaatcaatc aaacaacttt 180
gttttactga tagttctcta aaactgatta ataataagcc tccccagtga gttgatgtct 240
tcatagtaat aaagtgctct taatactcca agttttattt acaaaactag tttttttttgt 300
aaattgtgca aataataaat gttactgttc aaaaaaaaaa aaaaaaaa             348
```

<210> 1376
<211> 155
<212> DNA
<213> Ctenocephalides felis

<400> 1376

```
gcattgttta aatatttttc gattgtataa aattgaatta taatgccatg tgtaaaaata 60
taaaagttat gtatttttaa cattatattt atagatatat aattgtaaat aaaaataaat 120
tctgacacta ataaaaaaaa aaaaaaaaaa aaaaa                           155
```

<210> 1377
<211> 550
<212> DNA
<213> Ctenocephalides felis

<400> 1377

ctcnntttca gttgaattct tctattcgtc atattgtttt acatacaaat aaatgcaatg 60
tgcgttttgt aacgggatct gtgatacttc atacacacat atgtatgaat gatagaaaat 120
aacatttcca acgttcaagt agatactgta tgtagtttag tgtttgtgac ggaattcctg 180
cagatcggaa attactaata attacctgta cagttggaca ttttcaatca aaaaatgtac 240
tggttagctg cattattgct tttgtctacg tgctcagtgt ttgcccaatt taatcgcaac 300
atttcccaat gctgcgaaga aagttgctac agtactgatg accaatccca aaatacaaga 360
tttgcgacta aaactgctta tgaattagtt aaaggagtca gaggagatct gacagggttc 420
cacattgtga acctgtacag ttctggttgc tgccagacat ggtctagctg caactgccaa 480
gaaatagcag gatgccacat ttggaagttt ggagatgaga tatcgaaatt atcaagacag 540
aaatctggct 550

<210> 1378
<211> 550
<212> DNA
<213> Ctenocephalides felis

<400> 1378

gctgctggtt aaatattgtc aaggcacttt aatttattgt tatattacat ttgtttttat 60
ttgataagtt aatatttgat tttattatta tttaaatgtg tggcaaaatg attagaataa 120
tttattcaaa cctgttcgaa aattctgccg aagtaagatg cctacgtatt cgtcatcatt 180
ccgtcatctt tgctctcatt tgctgtgcaa acaaatattg tgaaagcaga aatgaaatga 240
agtaaggtgt tctggcgcgg ttgttgttta tgttagtttt gaacaaatat taagcgaagt 300
gaaatatcac tgttgatgaa tatcagtcat gttttgatat atagttaatt aataatgatt 360
tactttgata tcaagatatt gatatttaat taatgacact tttattttgc gtgcacgttc 420
tatgtttcaa agtttataca cattttgatt tgcttgatta ttgataaatc atgacgaaaa 480
gaatcggcgg cacttacgtn gacgggcata ctagcaaggg caggaaatca tggaaactgt 540
taggtccttc 550

<210> 1379
<211> 550
<212> DNA
<213> Ctenocephalides felis

<400> 1379

ganntttcta aacaagatgc ctncaaacct gatggagctg aagcgaaacc ggatctagag 60
gagaaatact gtgaaataaa acttgcacaa ccagaaattc caacttcatc tcagaccagc 120
gaactaggtc cagcagtgct tgtggaaaag actccagaaa tcgggccagc cgtgttggtc 180
aagaagggta aggctgaaac agagaaaact ctcgagttgg gtcctggtac aatctcgacg 240
gttgccattt gtcctaattt aacagtcgct gaaatttaca atgccccttt attagcagcg 300
agcgaaccag caaaaccaaa tgttcaagct gatgtcgctc cagtcgattc caacaagcca 360
tcgactcaac cgacagaact tgtagccgcg gaagctaaac ctgcagcgag cgaaccagca 420
aaaccaagtg ttcaagctga tgtcgctcca gccgattcca acaagccatc gactcaacca 480
acagaagttg tacggcggaa gccaaacctg tgcacaccta agaggttcta aacaccagtg 540

tatcgaggca 550

<210> 1380

<211> 550
<212> DNA
<213> Ctenocephalides felis

<400> 1380

```
gaatttttgg ttattctaaa taaatatatt taaatacgaa atgctgagca aatagtaata 60
taataacaat ttattttatg ttttattaat aaattacaca atggaacaac taaataacgc 120
tttaaaagcc ataaaagtgc tccgttcgag tgtcggacat gtttttgaga ctttgtcaga 180
aggtttaaga actgaacatg gacaagatac taaagacaca aagtttttga ttgaattaca 240
agaattgctg agtgctgtta atgttaactt gagagaggta gagacttcag tgaatagttt 300
aaatgcgccc cccgggccat tcaatttagc aaacactact tatttaagtc aagagactac 360
acaggagaga caggctttgt ctcgcagctt gtaatagtat agtggccgat aagattcatg 420
agtcnaggnc ctacacagct ttttaagtca aatgctttga aaggcatnnt taatcaagca 480
tgcgaaaggc ggnggaccca actttcttta catgtgccnc cgaaacgngg ttctttatac 540
aagttttcag                                                        550
```

<210> 1381
<211> 550
<212> DNA
<213> Ctenocephalides felis

<400> 1381

```
aatctgtgga tagttcattt catcgtcctc cagtaaaatc agcaaaggct gaatagtgct 60
gaagttgctg cttgtaatac tgattttgcc attcaattgg aataggcata ttattaagca 120
aatgaaaaaa gttacaaata attctaattg aactcgaaat attgtgtcta ccatatggaa 180
atcaattgca tattgtagtt attcagcagt tcaatgcatt tttgaaatga catttcgtga 240
aaatttagta ttagtagtat ttcccagtga gtgtctataa aactaaatag ttcgaatatt 300
taaataggac ttcctaaagc cattttttaat agcttttatt ccgcatagta tttattaact 360
tatacctcca gatgcaaata ttggtcctta tggtaatcct cttatcctta ttattcctat 420
gtaaatatgt aaaaattggt tataaggaag natgtttatt atccaagttt agatattatc 480
atacctatta tattagactg tttgtggcat gctttganaa ataataatat ggattatcta 540
ttaatctgtc                                                        550
```

<210> 1382
<211> 550
<212> DNA
<213> Ctenocephalides felis

<400> 1382

```
catctgggaa attaggcctg gagcaaggat tatctcctaa tgtttgtcca aattcaacat 60
aaaaatatat attttaaaat ataccatgat tatttagtag ctggagagtt tgaggtaaat 120
tttaatgatc agttcattgt ttgaaattag ttttgaacac agacaaaaaa acaagcagtt 180
```

tgttgatata tttgaattca aaacaaactt gcaattctcg attgcacatt aaattctgtt 240
ttttttttctg atcacggatg aagagattta aaaatttggg ttttgggagt gttacaaaat 300
tcgctctgag aatataccgc gcgaattcta agttgtttac tctaatttat ataaattata 360
ttagaatgct aagaatgaaa gcagtcaatc cctaaatata aaatgagaaa tagtttagtt 420
ttaaaggcag cgtactcaat ataattataa taaacaaaac aaatttatct caatacaacc 480
cgtaacgaaa cttttatgtg gatttggata attctttggg atatcggaaa ttaaaattcg 540
atagatattt 550

<210> 1383
<211> 550
<212> DNA
<213> Ctenocephalides felis

<400> 1383

ggactaggag catgaatatg tatgaataat ccaaatatat tgggttttat tatttttttta 60
ttgatattaa ttttgactat ttatatttgc cataaagtga acttatcaaa attagtgtca 120
cgagaaaata atttagaaat catggggtcc attaaacaac ctggcaggtt tatgaataca 180
caacaaaatg cacagaggac aacaaaagct caaggtggtt ttccacaaag cttatctgga 240
agtgagactg atgtgtccac atccaatgag aatttatcac atgaagagcg atatgttatc 300
cgtcacactg cacgtgttga accacaaggt caagagacct tgcaaaaccc atcgccaagt 360
cccactcaaa gcccagtgat taatcgattg aaatcaccaa atcaaaatgt acaaagaaaa 420
ttggaatcca acatccgaga ccattgatat gagtagaaaa atggaagtat taccagtcca 480
ataaacatga ttcaaattat cgaaataaag agctccatgc cagaatcatt gcatatcaga 540
atcgagaatn 550

<210> 1384
<211> 550
<212> DNA
<213> Ctenocephalides felis

<400> 1384

aaaactggaa cacaagcaat tatttcttaa gattaaattg attgggttca catgcaacat 60
tttaaatact cgaaacatcg atatatctat tgttgcaaat taaatactgc gaacgatgtt 120
gttctctttt cattattttg gttattgtca taacaacata agaataaacc ttttacttaa 180
ttgaacagga atggaaagta tgaaaccgtg gttttaataa aagtaagatt ataatttaat 240
gattagaacc acttttgatt aaatatcagt aaatgtaaaa gcctcgtata aaacgatgat 300
atgataacag ataaggccaa tatagtgatt aaagtcaatc tttagacagt gaaaagttta 360
attaattgtt tattctaaaa ttaaaattat tattcaatac tatttaaata aatagatact 420
cataatacaa atattaaaca taaaataatt cttgtaatca tgagtataat gaatacttaa 480
tataaatggg ttattaataa ctaatatcat tatataatta tatataattg gattcgagtg 540
aaacncatac 550

<210> 1385
<211> 550
<212> DNA
<213> Ctenocephalides felis

<400> 1385

```
ctttngttag ttagttgtgc aagaaaaatt agcatttggt catttccaaa gttacatatg 60
gtttgtgcaa acttgatgca agttgtcgac gcgtgtagaa atggaaaaat ggagccccct 120
tagcagtccc gagcagctaa tcgagtgaca tttaaaaggg cgcaaacgtt acatcggagc 180
atgaatagca aggtggaccc tttgccagac gagcacctga ttattctgaa taaattatcc 240
gctactgaat caacattcct catcgctttt aatccatcaa acaattcaat tatccacttc 300
tgaatcaaca gccctcatcg cctttaatgc atcaaacaat tcaattatcc acttatgaat 360
caccagccct catcaccttt aatgaatcaa acaattcaat tatccacttc tgaatcaaca 420
gcctcatcgc tttaatgcat caaacagtaa gacttatcaa ctatctatca ggcaattcaa 480
ttatccactt atgaatcaca gcctcatcac cttatgatca acaatcaata tcactataat 540
cacagcccta                                                        550
```

<210> 1386
<211> 550
<212> DNA
<213> Ctenocephalides felis

<400> 1386

```
cttcntggta cgggttggtc gttggagttc ctttggccat agtgtttgcg attgtgattg 60
gcatattagg ttatatattc tacaaaagaa gaagaaacgg gtgcgactac agggccgcag 120
ctacacattg atatagaatc aaaaaaaaat attcagaaga aaaatataat ttcatacagt 180
atagtaaatg tataagtagt taattgtagt aatatgtttg tttatggaac ttgcatttat 240
agatttttta aattgaaaat cgtttaaaat caataataag gctaatgtac ttgcttacca 300
aaataaaaat atcagtaagt attataaaat ttgatttaac aatttggaca aattgcttaa 360
aaataaaagt cttcgaatat ttttggctct tattttcatt tatttcattt tattttttgg 420
tactaataan gctggtatct ctaagtttat ttcttatcca gagattttaa attaaatttt 480
taanccaaaa ttatgaatgg ttttaatcnc taaaataaaa accaaaattt atggtagaat 540
aaattttttt                                                        550
```

<210> 1387
<211> 550
<212> DNA
<213> Ctenocephalides felis

<400> 1387

```
tnatnnngtn cgagattntg tattaantat atcgatnntt ttccagtcgc caaaatggng 60
ttgttgagta aatttaaacc tattgatact acttgtattg aatatgtgca cccttggaca 120
aatagttgtt ctgatgcctg tgctgattta acactnnagg ctttcgagag gnccatnaaa 180
atatatacag ttgcatatac agncggacta ttgatgcgag gcaaagtacc aaataaatcg 240
gacttaagaa agacttttct ggggattcta caatctgcag cattttttaac gacaaatggt 300
tttacattcc ctatgttttt atgttttctc agaaaagttt gtggcnatta caatatattg 360
acagtctcat acgtgccttc gtttcttgca tcgtttgctg caatattact agaacggcat 420
ctagacgaaa tttattatgt ttatatgtat caaatgaagc aacaganact gttggaatat 480
gttgaagtct aggggatatg tccgtctata aaatatggag aagtgggaat cnnttcatga 540
gcatcgtatt                                                        550
```

<210> 1388

<211> 542
<212> DNA
<213> Ctenocephalides felis

<400> 1388

```
ganttgnatt catgtttact ttcccatctg anncagaata tcagattgtt gttttaaagt 60
taaattttta acgtattcta ttaaaagatg caaagaaata tttccttaac tatattgaaa 120
acaaaacaca attatatttg tatgtgaatt atctctgctt agttaataat taaatttagg 180
tatagacacc actaggtata aaaaaagctc aagatttttg tttctagtac attttacttc 240
aatttctcat atttgctcca tttccataaa tcgtatgtct tcaacgtaaa atttcatcag 300
atgcgaaaca ttttcaacaa gaacaaaagt tactattcag tttctcggtc ttaatttaat 360
aagccatttt ctatgcactg acttccaatt ttccatgttt ttatattata atttgtatct 420
atttttttga acaagtttcg ttcaaactaa atcagctcgt agtttttcgc aataatgccc 480
aataatttaa actttcgagg ttgtgcttat gggtttgaca ctagacaatt ctttttgagt 540
cc                                                                542
```

<210> 1389
<211> 542
<212> DNA
<213> Ctenocephalides felis

<400> 1389

```
gccgtatgca cgtcaggtcg gtaggcagga ataaaaacat aaacactggc aaattgaaac 60
gtacatttgc tacatttgtc attatgtaat cgagccagtg cgatcgcacg gccgtacggg 120
tctgcagcat tcacgatcag tcattgatga ggtgtcgtat tgtgttttgc tgtaatatta 180
gttatatatg cgaagcgttc tttacgccgc cgtcatgatt attgcgaagt gatttcgctg 240
agtgtaccgc cgccgtacgc acggccgcgg gccggtgtgc ctctggacac gctaagtgcg 300
ccgatcatct ccgccggtta cctggtaata gtaataacaa tttttatgca gaactcgata 360
acgaatcgaa gtgggttatt aagtgattta ttagtgtgtg cagtgtttaa attgaaaaat 420
tgcagttttc ttatggtaaa gtgcagtttt aatgttaatg tgtgttcaat ctgtgggtaa 480
attatggtac cggtaactgc tctattcatt tatataaaca ttttaatcaa agcctaagac 540
gn                                                                542
```

<210> 1390
<211> 542
<212> DNA
<213> Ctenocephalides felis

<400> 1390

```
actctattag cgatactatt ataatgtaaa aaagttacaa aactttgaaa acgaaataga 60
tttttttatg ttcgttatgg atatatccaa tgtcacagca catgtaaaaa ttacacacga 120
caatgtaatg ataatattta atcttaattt gaaaagatat tgatttatga atatgaggaa 180
```

```
gctgtcacaa gcagtaaatc gcgttgtcta atactaaaat atattatatc actttatatt 240
aaatt   itt ctcatttctt ataatcctta aatgttttta tagagactct tcacaactgg 300
atttggagca gaaacatcag aagtacatat tattaagtct ggtgttccac aaggaagtac 360
tctaaagcca attttgtact ctgtatacac acaaaatatt taagtaacta ttgaaacttt 420
tgccgtatat gcatttgtca tgaggattca cgaaagtgca tcggtggcga cggcaaatgt 480
ttaacaataa tatgaattag tacttaaaaa gtagcttata agtagaaaat aaaaanaatg 540
aa                                                                542
```

<210> 1391
<211> 542
<212> DNA
<213> Ctenocephalides felis

<400> 1391

```
gccgactcat gtctcagttt gaggttagaa tttattaagt gaaaacatgg tcccacggtc 60
gcagtgtttt acttctattt tggcggttgc gcccaggctg ttgccgttcc tgataacagc 120
ggtttcatat ttcgtacttg ctcttccgaa ggatcagccg tctgttctgg cagtgatagt 180
aaaatgcact cctatttatt gtcttattta ttttgtgttg aaagctggag tatccactaa 240
taaaaacaaa ttggcattgg cacttgtttt ttcgagtatt ggtgatgctt ttctggtgtg 300
gaaggagttt tttccacacg gaatggccgc tttcggtgtg gcgcaggtga tctattttac 360
tacttttggg ttcaaaccat taaaacctgt tttaggtgct atttggtact tagtaggaac 420
tgctttggtt gccctagttt tttcaaacct caaaggcatt tactgtatgg actgccatct 480
accagttctt ttagttacaa tgctttggag agctccgcca gacacagttg atgataaggc 540
tg                                                                542
```

<210> 1392
<211> 542
<212> DNA
<213> Ctenocephalides felis

<400> 1392

```
cngctatgat gatgaattgt gaaccagctt acagctccct tttgcctgat tatgaagagg 60
ctgtcaaaca atcccaggat attccaccaa gttatcaagc tgctgttgct agcgctgggt 120
taattgaaaa agaggctgct gcttcttctg ctgatgcaac tgaggaaacg aataagccag 180
agactgagcc cgccaagaca cctgaaccgg aaactaccac aaaaagtgaa ccccataatg 240
tgtaaagtaa aaaattaatt ttaaatcaac tgaaataaat tgcataaccg aattgaaatt 300
ttcatctgca tatgcataga tggcaattag ctgacagaca tattttgaaa atataactac 360
atatatgtat taaacaatat ataagggcat atatataaat atatatatat atatatatat 420
atatatatat atatatatat atatatagaa tatctgaatt agttttaggg ggagatatat 480
atgatgttat atnaaannaa agtgtntcta tatgaanaan ggggggngcg atatttttta 540
aa                                                                542
```

<210> 1393
<211> 542
<212> DNA
<213> Ctenocephalides felis

<400> 1393

gantagaatg tattcgtgaa tatcaactat acanaagatt ttgaggttag aaattgtaaa 60
aaaggggttt tgccctatat ttaaatataa aacgatatag ctaattatta gtggaacacg 120
actattgaac aaaatcaaat gcaaaaagta aataataacg atttgtgatg cttgaactgt 180
gattcaaaat gtgaaatagg aaggttgtaa aacgaacgtg acgtcttgtg agtgtgtttc 240
taaaactgtg tcagtagtgt tttaagtccg atataaaatg gatgaagagg agaaaaagaa 300
taatattggt acacctaaac ataattcctt aataaatagt accggaaaac acacttctgt 360
aacacataat ggcgcgaaac ataattcacc tatgatcatc aacaatggcg taattatttc 420
gcacaacgat tacggcagta tcgttactgg aaggagatcg cgaaggatta catcaatgga 480
aggatggcct atcttctcaa aacattgtcc tggacttcgg cgagttatca tcattgatga 540
ta 542

<210> 1394
<211> 542
<212> DNA
<213> Ctenocephalides felis

<400> 1394

ctataatttt attattacta actatgccta ttataatcaa gaaccgttta attcaataaa 60
ttaaattata taagtagcgt aaaatatttt tcatttgaaa catttgtgtt cgaccaagat 120
atttatgttg attataggta agagaatact gcaatgatga tggtgtcctt ttaatatgct 180
tctttatatt ttgtaataac ttttaacttc ccgtagggta agttatagtt atcgcaaaaa 240
atataaaacg aatttttttt tatatcttca cgtttcaggg tttctgaaca ttttggccat 300
agataaattg gcacacctta gaaaaaatgg atgagggtgt gtgtatgtat tggatacaat 360
ttagtccacg attttgggcg cacggatcaa ccgatttgaa taattcaaaa acattatgtt 420
cttttacata aggagacact tgctgattag ttttttccaa aggcaggtcc agcggtttcg 480
gagatccaga acagaatgtt tttaaatatg ccatttgacg gtcgaaaatc agtattgact 540
gt 542

<210> 1395
<211> 542
<212> DNA
<213> Ctenocephalides felis

<400> 1395

ctttattagt caacggcact gtccatccat ngatcatcac tacattttat tctcgtttct 60
cccgttgttg agttgttttt gtttagttat ttttagaatt aagtttgtgc aactttttatt 120
tcaagtgaag aagcagtatt atattatggt tataaatggc attgcatgta tgactcacaa 180
aaattaaaga agtgagtgta gcgattgatg atgaattaga aaacgggtgg aagtattctg 240
aaggtgcaca agatttgagt gattcagtaa ataaatcatc caaaaataag ttgaatgact 300
acatgatggc gtcccaattg aatccagaag ctgccgagtt tgtaccggtt gaagcgggta 360
gtcctgcacg atcagtatta tttttgagag aaggcttgct tgatgatgta atggctcgaa 420
gtccaagaca atttagtgct gctgaacaag ttcttaatgt accatcggat actgaatttg 480
catcagaaat taaatcanga ccaggagact aagtgcaaat ggttcttttg aaagcgataa 540

ga 542

<210> 1396
<211> 542

<212> DNA
<213> Ctenocephalides felis

<400> 1396

```
cttactttac ccacacaacc tgcagctcct atatttatgt acaatggaaa cattgatttc 60
aagcctgata cattactaaa ccaaccacaa gtgatatcct tgcaaaaccc tatagtaaac 120
aaccaaaaca cacttatttt ccatccggta caaaacaata cgttttatga tatcaaaaca 180
ttctcacatg ccccgacaag aaatgtacca attgcaaaac agaaacctgg tcgaaaacaa 240
ctggcagctc gtcagactac aaataatgtg caaccacaaa aaatctttgt gcctaatatg 300
aatacaatgc aggacaagca acatgtatta ttgcaagcaa agttaatcaa atcggaacaa 360
catataaaca aaactgttat gtatactact acaccaattg ctgtaatcga tgacaaattg 420
gctatcaaag atttgtccca taaagaacca aaagtgaaag aagtcaaacg cagtgccaca 480
atgctataga acgcagatcc ggacaagtat caatgataaa atattgactg aaaatatgat 540
gg                                                                542
```

<210> 1397
<211> 542
<212> DNA
<213> Ctenocephalides felis

<400> 1397

```
gtgcctttgt cgccatatta tttctggtca tngtattttt ggtaggattc gcctatgtga 60
tgtaccatca gaaaattttg gccaaatcct attttgaaaa agtgcaattt aataaaatga 120
ggagatcttt gaagatttat gatgataatg gtgatactat cattagtgga gagttgggta 180
caaccctaag gtctgatagg gtgtttcctt gtcatgccga ggatatgaac ttttctgaaa 240
ggaagagtcc cctatgccta gaatggctgc acagtgcccg tctctatctg gatcgcgaag 300
aagacgcttc cataatatcc tcgcacgtcc attccatatc cacatccagt ggttcttccc 360
aaaattccaa caacccaaac cccacacatt ccactttgaa atgctatgac gtctcctggg 420
ttgcaatttc gccctaccat tacccgactg actgttacca gtggactggg cctgccattg 480
cttgcaaatg atacaagagc ttcaaaccat ttattactgg gaaaaactgg ggaaatgtct 540
ga                                                                542
```

<210> 1398
<211> 542
<212> DNA
<213> Ctenocephalides felis

<400> 1398

```
aangtgcaaa atgatgatat cgattttact atttacattt ttgagtgtat ttcatttagg 60
atcatcccta caatgttacc aatgcgacct ggcaaataac ccggcctgca cggcgtctct 120
aaattccact aaagacattg cagccgtaga atgcggaaaa cttcctcaac aagttagtga 180
```

aaatgatcgc tccttaatac gatttttgcc accaaatttc gatggacact tgtcaaccac 240
cagggaagga gtcggatttc aatgcgcaaa aattgttgcc acgaataaca ttcagggtgg 300
ttataatatc acgagaattc ttcgaacatg cattgtcgat accttaaatt gcgagaaaat 360
taatgaagac ttaaaacaag aaggattttc ttcaatgatg tgcgcaactt gcaacacaaa 420
tttatgcaat agttctactt ctttaagcat cacaatttta ttaccattta tattattttt 480
aatcacaaaa gcccgtctta gggaaataat ttgagttaga tgaaaatttt caaagtcttt 540
ca 542

<210> 1399
<211> 542
<212> DNA
<213> Ctenocephalides felis

<400> 1399

atnaatgtta atgtatttta attaattaca tgtcattaaa atattgaaca gttttagaag 60
gttgtaataa ataaatcaac tataatttta gtgtgcgcaa tgacatctgt caagttattt 120
tctaatattc tgtctaaata aatgagaaaa atccaaaatg ttcaaaatgg agggatgcta 180
tttgtcagga gggggtatgt aataacagct atctgaatcg tcacatttct catagcattc 240
tggtggtata gtgatgggtt ctcggacagg agcggggcaa ataggataat tatcttcaag 300
ttctgaatct tcagaagatt gttccttttc tataatttca tcattttaat ttaatttgtt 360
tgcacacaat aaatggttta caaattgaaa actaatgtaa aaaaattatg agcaagtttt 420
ctgaaaatta aaaataaaag atgacttaat ttagtatcag tgtcatcggt ttatggagta 480
acatccaatc atgcaaataa atgattaatt atgaccatgc cagagacaga tttcacatga 540
at 542

<210> 1400
<211> 542
<212> DNA
<213> Ctenocephalides felis

<400> 1400

attaccaagg gagagggggt tccagaaatc cttaaaaaat ttatcatgtg accaatggac 60
gcccccttag gattaatatg cagtaaaagt ttttttattc ttattttatt ttaaaaaaat 120
gacaatgtat gtggttccac aataaaaaac gtaacgattc tattttatta ccttattttt 180
ttgtggaatt ttgtgaaact atttcacaaa atttttgtac gatttatctt cttgctttct 240
gattgtatac ttttgatctt agttttattc cacaatttaa cagcctcata caaatgagca 300
tatggacgtg atgtcactat tttatccact taaaaaaaaa tggacctgaa aatagatcga 360
gttctatatt gttcccattt tatccaaatt gacttcccat caaagtcggt ggtaagttct 420
tggttaaaga tttaatggaa gctttttgta tttttttttc ttgtggaatc gacatttaca 480
aattgttgac taaattcctc ttaatttctt aaattggaat cagctcataa atcaatgtaa 540
aa 542

<210> 1401
<211> 542
<212> DNA
<213> Ctenocephalides felis

<400> 1401

```
anttcggttg aattgtagct tcttacttga aatatattat cttgtgtgcc ttactacatt 60
ttgatataca aaacattcga ttcatgaaag atcattccat aaaaaccata atgtatcagt 120
attatcadat aataaaattt ttaataattt taactaccgc tgattatatt tcattgcttt 180
tttgtactgt gcagcataat ttaataaaat ataagcacct aaagcaagtc atatacagta 240
ctacatatgt tgaagaattt gatgaaagca atcaattttt gatttgtaat tgatttaaat 300
atattatgtt aattaatttg aatatatgta ttagattata gattgttcat tcgtattgca 360
gaaatacgaa ttgaaaaatt aattatttaa ttaaagatct ataaatttcg tccaaaactt 420
aatcactgat tcaaaatttg tgcagtagtt gcacatattt attttatatg taatttagat 480
gttatttttt gcatattcaa taggatagct tttttacaaa aaatattatg taatcaatat 540
aa                                                                542
```

<210> 1402
<211> 542
<212> DNA
<213> Ctenocephalides felis

<400> 1402

```
aanaaacgag cccactggct gtctatcaaa aaaaaaaaaa aaaacgagcc cacccatcaa 60
aaatttggtt ccgacgccac tgcatagatt ctaactaact aaatacataa aatacaataa 120
gtactactta ctttatatat tattaagttt acttacaaca atattttttt atttattact 180
tttttttatat tcgacttctc agattatata tattattaaa atttattatt cgatgtatta 240
ttacaatcgt gccataaatt aaaaaatatg agtatcgctt gtaaatatat tagttaccag 300
ttagatgtaa tttttgtact ctcaaatttt aatcgaaacc aaatttttga ttattattaa 360
taattgataa gtagttttaa ttaaaatagt tggacgtttg catctcagat aaattatatt 420
aattatttta taagttatgt atgcattgta ataagtcaca cttctataaa ttaattttta 480
atcaaattct tggtataatg ngaaaattta ataagctcac tatatattta tgcttatata 540
ta                                                                542
```

<210> 1403
<211> 542
<212> DNA
<213> Ctenocephalides felis

<400> 1403

```
atgtcgtcga aatgggcaaa gtcggctatc aaatcagagt tcgttggttg gtgccgtcgc 60
gcttcgactt acttttattt catttattaa acgcacgttc attcattgaa tcggaaaaca 120
tggatatgga gagacagtcg cttcatagtt acactaaaca taacctaaaa tattggtgta 180
taactaaaag ataacgatat tataatgttt cgagtgaaat ttttgttata agttctgtgt 240
attaaattaa ataatagttt agaccgaaga tatcacattt gtgtcaaaat gccaaatgtc 300
aatgtgacaa acggtgcatt aaaatgaatc tcataacctc tgtgtatatt ttggttctta 360
gcaaaaattt agtgtacgct gatagtggat atgtgtaaat tagacgttac tagaaatttt 420
tgattttaat aatgagtatt ggtaaaatgt gtacgcaaac gagcgtctac tagttggacg 480
tatgtgataa tggctggtgt cggacataga tctagcaacg ctcacaaaag acgtcttaac 540
ga                                                                542
```

<210> 1404
<211> 550

<212> DNA
<213> Ctenocephalides felis

<400> 1404

```
acaacatccc ataataaaag cattgtacag cctagtggta atgatcatat tcgagggggt 60
aatgttgctc cacctagaat gcaagatgtt gtccgtccaa aaagatattc ttgtcaaaga 120
cctggtggta ttgtaccaga aacaaacatg caaggccaac cgcagcaaca acaacctgta 180
tatcaacaga actactatgc aactgaatat actccgcctg tagcaaatga acaaaataat 240
tcacatcaag gacaacatat accacaagca ctaaatggga tgccccaacc aggggggtcag 300
gttgtacctc ctaatatttc tgtgccaccc ccgcaaacaa tgccttatgt accagaacca 360
gttcctacac aggtcataac cactagtaat caagtgatgc cgcaacaagt tcttgccctc 420
aatatcctca agttctatgc agtttaattc gggaggaccc tacatggctc aaataatact 480
tttatccaca atctttaggt atctgtcctc tctaacatgc accccacaca atatctccca 540
atccctcata                                                        550
```

<210> 1405
<211> 550
<212> DNA
<213> Ctenocephalides felis

<400> 1405

```
cacatatttt tcaacatgga aggacgtgtg tgtcgggctc gattaaaaaa aacgtggaaa 60
ttaattatct cataatggtg ttagtttcga aaggctatat ttatatatgt agacgccagc 120
ctaccatttt ggttgatggc gatgatctga tattacgata aggaaaaatc tggaccacga 180
gataactgta accgcgccag ttaactcgtt caggatgtgg tgtaagtact gccttcatat 240
attcctgata aataattttc atgatgttta tcgtgatccg agggctgatt gtttgtgaat 300
gttaactctt tgactgaatt aatgaacacc ctctaattta ttatggaaat ctctacaatc 360
ttgtaatatt tacatgattg tattacagtt tatgctgtct gagcatagag aagcacctgg 420
aaccataaat ggcttcttga aaacatgcat cacatctcat gtctacacat gtatcctgca 480
ttgatgagaa gggtgtcaaa tcacatccat acataatgat gtacatatat cctgcattag 540
tgaagagaga                                                        550
```

<210> 1406
<211> 550
<212> DNA
<213> Ctenocephalides felis

<400> 1406

```
cnacattgca tggnatactg tnntcttnng cgggnttntt ggtnngggtn ngtngatngg 60
naggnttgnn ggctcgtnac nagacncntt gnnnncgntc aantagccca ngattngttt 120
ctcacannat tgntggngtg ttnttctctg cctnaaagac ngatacacat ggatntatgt 180
```

cgnngnnant ncatgtactg ncggccagnn ttgtaatttg ngacaaggan gntgctnttn 240
caaacctgan cntgcnattc tncacgcacc atgcaatgcg acancanatg aattgacctg 300
anccctacna ntgcanaagg ngtnanttct gtctgnaaac gganntgatg gantnttnna 360
agaatgcgnc ngtaanannn natnctntgn cnganctgga cgtgcncttc tgcttggata 420
ccaactttaa cnagacctcg ccnatatgtc atnaantgag aacccgcttg ccangacata 480
tcnntntttt ctctgaaaac agaacattta tngtnnaann gctatncctc aggtntaaan 540
cccatcgang 550

<210> 1407
<211> 550
<212> DNA
<213> Ctenocephalides felis

<400> 1407

gtcaacagcg aacagcgagt ctcggccgcg gnnctctaca atcattaatg atacgcacgc 60
acgccacgta actgggcgca gatatcgcaa aaatacataa caggagtaat agtatagtaa 120
tagtatattc atttgaatag acttgagatt gagacaaata gcgcgcgatc tgcaagtcat 180
tgtgaccaac ctttgacgtt tagcgaattc atcgtgtatg tgttttggaa tcctattatt 240
gtgtgtgttt gtgaatttcg tgttttatta ccgtgtgaag ttgtattcag gtgctctaac 300
actaagtgat tttcaacaaa gattttttaa gaaataaatt ttaatcaaaa tcgaatctgc 360
tacaataatc cggctgaacc agtagatgac cttgacaacg agtttgattc gtagcgatta 420
acgaacgctg ccggaccttc cgaccaatac tcacggattt attttgatta aatgagattc 480
tgttataagg tgacgtctag aaatagatgc agatgtgtaa ccttgtttgc gnggcagaga 540
aactgtgtcg 550

<210> 1408
<211> 428
<212> DNA
<213> Ctenocephalides felis

<400> 1408

aaaaaactat tttatactgt tacgtaaaac atgtttcaaa gtacagttat agtttgcagt 60
gcatggtaaa ctactttggt tcttatttaa tcggatgttt aattaacaga tatccaaaga 120
tgtaacttca actttccaat ctagtacaaa gaattttttt gaacaaattt atggaatgtt 180
cggatttcga ttcccaacca ccaccggatg ggattttgca aatcccacca ccaccgccac 240
cgccagaacc aaaattttat gaattgtgcg aaaaattgga agaagatcgc cgaatatcga 300
tgtatgctct tgaaagcacc ccaggcttca ccatgtacac agaccactgg tatataatat 360
tgattgtgat tttagtagta attgtatcga taataattat aatattcctc aaaaaaaaaa 420
aaaaaaaa 428

<210> 1409
<211> 455
<212> DNA
<213> Ctenocephalides felis

<400> 1409

tgaacgtcca gaaccaaatg atgaagattt ctgaaaatct gcgcgcgtaa aacaaataac 60
taaggttaga catggaaatt gtgaagtatg tcacaggcac gccgcacggg acacctcaca 120
ttcagccatt cagggatcat acaagtggcg accgtgcgaa gcgcgagccc tgcgtgaacg 180
cgcgaacctt ccgccataat ataggtttca gtttaaattt atagtatcat ttcaaattta 240
acttttagaa cagaacaatg ttcaaatact atatctgagt attatctgac caatactgct 300
agatctatta cacttatgca gtcatcaatt agtacataaa agaaaacaat ataagaaaaa 360
aatattattt tcttgcatta ttcaaaagaa gctatgtaaa tattaataaa gcataaatag 420
aaagattcac ataattcaaa aaaaaaaaaa aaaaa 455

<210> 1410
<211> 550
<212> DNA
<213> Ctenocephalides felis

<400> 1410

gaagcatttc atctgtcgat cataatctag tcnaggtgga aaaatgaaaa tcgttctctt 60
aacaattggc tttttggtcg cggtcgtgtc ttcaaacacg atctcattgg atgaaagttt 120
tgagtcaagt tttaatgatt taaaatcact cgagagcaga ggggcattag aaaataaact 180
attagcagct gctgaagatc tgagaaaaac tttaagaaaa ggtggtaatt tgactaatgg 240
cgaagttatg gacccatggt tccttgaaaa tgttgatgct gacattgaac taccaaacat 300
tgccaagcta aacggagctc ttgccaacgt aacagttgat ggtttatcta gtttcaatat 360
aaatcacata aaagtgaatg tgctcttcat gaaagctact ttcaatatca cttttgacca 420
tctcatggca aagggtctat acgacattga aggcaagttg gcagatttgg ctaagttgtt 480
ggcaaaggtt cctttgatat catgnacgtg attaaacgct gtggacatgt caactatggt 540
tgaaaggtta 550

<210> 1411
<211> 550
<212> DNA
<213> Ctenocephalides felis

<400> 1411

gtcggtcaat tctacttaat cgctgtgcaa ttatattgta acttgtaaat aatcgttatg 60
ctatagctca gtgtcgtaga gttattttga tatgaataag tcataagttc tatcaagata 120
taaaattatt gcctaatatc aatgatcacc aaacaaatag tttttaattc gtttttttatt 180
taattttaag agaatattat tatgaatatg ttaagattat ataaatagtt atgaacgttt 240
ttcttaagaa atactcaaag acataatata tacaaacttc atataaatct gttcttaata 300
taaaacttgt taagagattt tgtcatatct ataaatttta tttacttcga aatactatta 360
aattgtgcat aaaattattt gatatacagt gttcttttta atatccactt ttattatttt 420
aaattcatat agtattttca tattttatgt taataaaata tatttaatca gacactaact 480
cttatttata ataatatgta catttggtct catgatagaa atactgtgat gctaaaaaaa 540
atggtgtcgg 550

<210> 1412
<211> 550
<212> DNA
<213> Ctenocephalides felis

<400> 1412

cgccaacgtc tatttaaaat tttgatatgc aatttgtgtt aaatgcgttg taaagaaaac 60
tatttatacc tatttactgt tgtctttatt ttgcttcata ttttattttt cggtatccat 120
aaatatcgtt gtttgtgaaa atttgtgtga aaaagtctat taagcatctt caagtttggt 180
atggctaaac ctcacctgaa gaaagtagcg ttcctgagga cccgctatgt cacagcacta 240
aaattaagat ttttcttctt tgtgacaata ccagtttgtt atttggtatt tacagcgtta 300
acaaaacaat ccgtttcttc tgaagatatt gaatattacc cgcagacacc agaaattact 360
ggatcgcgga aattgcttgg ctatctttca ccgatgccaa gaattcgagc ctggcagatg 420
atcatggtca taactgcacg cccgcagcta tcttagattt tccctctgat ggattcacca 480
gggagcaaag aagacagggc tgggcctggt ccatgccgca tcgatctact gttctggctt 540
tagcatagtt 550

<210> 1413
<211> 550
<212> DNA
<213> Ctenocephalides felis

<400> 1413

ggaagttacc agtatggcac tcctacatta tatccacaaa tggctacaac agcaaatgca 60
catggttcaa caactaatag tggataccc aagccttctt attctggtta taatgcaggt 120
tatgaatcac taagccaaag ccaagattat acaaaaccca gtggttctta tggtccagga 180
ggaagtgtta gcggtactac agtggtgcct caggtatctg gcaaatccaa cacaactaca 240
tcggggagtg ttggtccaaa cagtcagggt gctaatgtgc agacaggtgc aaataacaca 300
gatatagcaa gcgctatgta taataaaact catacagcat taaataaagt taattcttat 360
gagaagcaaa catttcattc gggcacacca ccaccgttca acatgccagg aagtggttcc 420
agtggtacta cttatggggc tccgcattta tttataccaa caatggcacc tcaccaacaa 480
gctcatcata atacacagat gctgatcagc ctttgataga ttatgggggc cttcacaagt 540
agtcacangg 550

<210> 1414
<211> 550
<212> DNA
<213> Ctenocephalides felis

<400> 1414

gttattctta aaaagctaag tagaaataat ttatctttat cagttcaatt gatttacatg 60
tgaacatgat taacatatta tcttttatca attttaatgt tcaatgtgat gtttagtgta 120
aattttaagt tattttttta attatgatca tatcttttta ttcctacatg tctttcattt 180
tagtattgca tataattttt agtaaaaacc ccttttcagt tacattaatg ttttctgttg 240
catgctacta gagaaagtag ttcattctaa tttaatgaat gttagtaatt gaatgcttgt 300
ttagtgttag tttttgagat tgaccaaaat aacatctttt ctatctgatc atattctgtg 360
tatttttata taatttatac taacagcttg cttttaataa ccgtttgaat ttagtttgtt 420

attgttgtga tcaaaacgat aatttgcatg tcttcaaaat ttctttgcca gaaatttgga 480
aaaaagtntt cttatttatg accaatttat gntgagtcta attgggttta ataatattta 540
attctggaca 550

<210> 1415

<211> 550
<212> DNA
<213> Ctenocephalides felis

<400> 1415

```
cagagatgaa tacaaatttt taactcaata tggtgtaatc ataattttct aaagttaatc 60
gaattcgaac gaagtgataa gtaacattgt gccaaaatgt caatcttagc cattgatttt 120
agtggtctgg ggcctcgaaa atctgttttt gttgtcatta taatagtagg atgtttttct 180
attttatggc caaaaatatt tcatcccatg tttatgggat ttccagatca acaaattatg 240
ccaaatgcta tggacagata cgcaggttgc tgcgatgtga tattcagcag cgatatgaac 300
gctctgatga cagtaactaa tttatgtgcc caagtgctta aatttcaaga tttcaatgaa 360
acaaagttat tgggctcaaa cgtgaataat agatgtaggg ccgaaatatt atcacgctgc 420
ggcctggcat attaccggta ttttcatccg gaaaaggtgg tccagatgtt aaaggagtga 480
aacgccttct tacgagatng ttcgcttaat ggctcatctt gcctnagtga gttcggaatc 540
ctccatggct                                                        550
```

<210> 1416
<211> 549
<212> DNA
<213> Ctenocephalides felis

<400> 1416

```
attaaaaaaa aatctcaaat cgatcataaa agttttaata ttaatttcag taattaatac 60
aacttctgga tcagatgcta acaaatacga agccacctgg gagagtttgg atagcagacc 120
atctcccagt tggtatgatg atgccaaagt tggaatattt ttacattggg gagtttatgc 180
agtaccaagt tttggcacag aatggttttg gcaaaattgg caaggatcta atgtgtcctc 240
ctacgtggat tttatgaatc agaactatcg accgggattt acatatcaag attttggtac 300
agaatttact acggaactgt ttgatccaaa ccactgggct gagctattcc aagcttctgg 360
ggctaaatat gttgtgttaa caagcaaaca tcacgaagga tacaccctgt ggcatcaaag 420
tactctttca gttggaactc ccaggacgtt ggtgctcata aggatttaat aggtttacta 480
ccaacgcaat tcgcagcaaa accaactacg tttcgtctct atcactcttg tcgatggtca 540
cagacttat                                                         549
```

<210> 1417
<211> 549
<212> DNA
<213> Ctenocephalides felis

<400> 1417

```
gtgtattcat attaaaaact gctgaatcca cgtgtgtata atgtcagacg cagtggtggc 60
```

```
aggaagtggc ggacaaccta attctggaca aatcaagaaa gaattatcgg aaggcattat 120
ataccagacc gcagaaagtc caggatcaac atgctgcaca gcgctgtatt ccgatgtaga 180
agtgataaag atggaaattt cggatcattt tgaagttgtt gacccgaaat ctctacagat 240
agcaaatagt ccgggtagtc cagatagaca attctgcagc tcaaccacgg cgtcaattgg 300
ggaaattacg acaaacgatg atattaaaga agacagtcca aggcgtcttt gccttgtttg 360
tggtgatata gcctctggat tccattacgg cgttgcctca tgtgaggcat gcaaagcatt 420
ttttaagaga acaatacagg gtaacataga gtataattgt ccggcagttg gagattgtga 480
aatcaataaa cgaaggcaaa agcatgtcaa gcctgtcgat tcaaaaatgt tagcattgga 540
tgtaaaaga                                                         549
```

<210> 1418
<211> 549
<212> DNA
<213> Ctenocephalides felis

<400> 1418

```
gccgtcaaaa atggctggag tattatttcg tgttaatttg gctaaaacac aattgggcat 60
aatcagccca gtttttacaa ttagacataa cagcggtgga ccaccaccac caggcactcc 120
acctccacaa acacgtacta aatttggtcc tcttaaagat gaagacagaa tttttacaaa 180
tctttatgga cgtcacgatt ggagattgaa aggatctttg aaaagaggag attggtataa 240
aactaaagaa atcattctta agggagctga ttggatcata aatgaaatta aaacttctgg 300
actcagaggt cgtggaggtg ctggttttcc ttcaggtttg aaatggtctt tcatgaacaa 360
accaggagat ggaaggccaa aataccttgt ggttaatgct gatgaaggag agccaggaac 420
atgtaaagat cgtgaaataa tgcgacatga ccctcataaa ttggtggagg ctgttaattg 480
caggaagagc atggcgctcg actgctcatt acatagagga gaatttatat gaacatcaat 540
ntcagttgc                                                         549
```

<210> 1419
<211> 406
<212> DNA
<213> Ctenocephalides felis

<400> 1419

```
gttaatttaa aataacaaaa tgaaaggaac attattaata ttatcatgtc ttgtgatcat 60
gataagtgcc gaatatgctg acgtagatgt gtgccaagat ttggacgatg gaacttttct 120
tgctgattca aacaattgcc aaaatttctt catttgtgat ggaggccgag cttggaaaat 180
gtattgtcca ggatcacttt tatggaatga tcacgaagga acatgtgatt acgcacaaaa 240
tgtagaatgt taccaaccag aataaaacat tttaatatct gacagcgatt ttctgaaact 300
atatttcata ctactgttat aataaattta tcttcattgc tctcctccta taaatttatt 360
ccgttttaat aaaatcaata taaagacaaa aaaaaaaaaa aaaaaa                406
```

<210> 1420
<211> 549
<212> DNA
<213> Ctenocephalides felis

<400> 1420

agaacagaag tatatatgat gagtatcagt gcaatttaat aatgagagaa cgttgtttca 60
attttattag taccatatac catataatga attagtagaa aaaaaattgt tgtagtgttt 120
tagtattttc ggacatcaac tcattaaatt cataatgtct tcaggtagac ctataaaatg 180
tgttgtcgtc ggcgacggaa cagtgggaaa gacatgcatg ttaatatcat acacaactga 240
tagtttccca ggagaatatg tccctacagt tttgacaat tattcagcac ctatggttgt 300
tgatggaatc cctgtttcat tggggctttg ggatacagct ggacaagaag attatgatcg 360
attaaggccg ctgtcttatc cccaaactga tgtatttctc atatgtttta gtgttgcaag 420
tccgtcatct tttgaaaatg tcacttaaag tggtatcctg aaataaaaca tcactgcctg 480
atgcacctat aatcttgtgg accaaaatag attaagagac gatagagaaa ccttaagttc 540
tctacagac 549

<210> 1421
<211> 549
<212> DNA
<213> Ctenocephalides felis

<400> 1421

ctgataccat cgaagccagt gctgattcat gacttgggtc gtgtttatat acaaactggt 60
cctgatgtta agaggtgcat tcttagatta ttagagggac cagttagaca actcggcatg 120
gaaaatactg aattgatgaa attagttgag gcctgtgcaa agggatctga aaccttggtt 180
acaagggtga tacatatttt gaccgaaaga agtttgccta gcatggaact agtttctaga 240
gttcgagatc tttatcacac taaagtatct gatgtccgat tcttaatacc agtattaaat 300
ggtcttaata aagacgaggt tatagattct ttgcccaaat tcattaaact aaatccagta 360
gttttaaagg aagtttttaa taagctgctg aataatcaag caggtcctac atcatatcct 420
agtccagtta cacctataga gttgttgtag cgtacataca atagacacaa ctagtgcaga 480
tttaaaattc gagtgaaagc aacaagttgt gttagcagag aagnaattac acccagaang 540
atgcagagt 549

<210> 1422
<211> 534
<212> DNA
<213> Ctenocephalides felis

<400> 1422

tgangatcgg cttgtgcgcg tacagtggat acaaaattta tcccggccat ggcaaaacca 60
tggtcaaaat tgatggaaag acattcactt tcctcaattc aaaatgtgag gctgctcatt 120
taatgaagag gaacccacgt aaagtaacat ggactgtctt gtacagacgc aagcacaaga 180
aaggtcaaga agaagagttg actaagaaac gtactcgtcg tacacagaaa ttccaacgtg 240
ctatcgtagg agcttctctt aatgacatcc ttgccaagag gaacatgaaa cccgaagtac 300
gaaaggcaca aagagaccaa gctatcaggg ctgcaaaaga gcagaagaaa tccacgaaag 360
cagcaaagaa ggctgcagct ccacctaaag tgaaagcccc accaaaagct aaggccgcta 420
aagtgagcca gaaagcaccc ccagggttgg cggtaaacga taagtggtgt tggagattat 480
tctaatgtat tatatgaaaa ataatttgta aaatgctaaa aaaaaaaaaa aaaa 534

<210> 1423
<211> 549
<212> DNA
<213> Ctenocephalides felis

<400> 1423

tgaaaaacta cgatccccag aaggacaagc gtttcagcgg caccgtaaaa ttgaagcaca 60
ttccacgacc aaaaatgcaa gtatgtattt tgggagatca acagcattgt gatgaagcta 120
aggccaacaa tgtcccatgg atggatgccg aagctctgaa aaaacttaac aagaacaaaa 180
agcttgtgaa gaaactagct aaaaaatatg atgctttctt agcttctgaa tctttaatca 240
agcagatccc ccgtttgttg ggtccaggtt tgaacaaggc aggtaaattc cctggtctct 300
tgtctcatca ggaatccatg gtgcagaaga ttgatgaagt taaaggaact atcaaattcc 360
agatgaagaa ggtgttgtgt ttatctgtag ctgtggtcat gttgatatgt ctcctgacga 420
gttggccaaa acgttcactt gtcaattaac ttcttggtgt cacttttgaa gaagcattgc 480
agaatgttag gtctctcatg tcaaatccac tatgggcccc caaagatata taatacataa 540
tgatatgtt 549

<210> 1424
<211> 549
<212> DNA
<213> Ctenocephalides felis

<400> 1424

aaaattcaca ttttaatcac aattaatgtc tagtttctaa accacaagga atatacttat 60
aaatatattt atataaatat aaggaactga ataatctttg atatttggtt gtttgggtat 120
ttatgttgta caattaatat atcctttacc ctaaagtcta catatattat caaccatggg 180
taaagcagaa gtaggtaccc ccaagtacat agccaacaaa atgaaggcca aaggcctcca 240
gaagctccga tggtactgcc aaatgtgtca gaaacaatgc agagatgaaa acggtttcaa 300
atgccacaca atgtccgaat cccatcaaag acaactttta atctttgctg ataactcaca 360
catgtaccta gatcaatttt caaaagaatt ctctgcggct tcttagaact tctgagaaga 420
caatttggaa ctaaacgagt ggtgctaata aagtgatcag gattatatat ctgataggaa 480
tcatttgcac atgaatgctc aaagtggtgc attgcggggt tgcaagtggt tggggaggaa 540
ggcatgcgt 549

<210> 1425
<211> 549
<212> DNA
<213> Ctenocephalides felis

<400> 1425

gcaagttagg gatcttgata ttaacactac agaaagactt aaaggtgtta tggatttagt 60
atttgaaaag gctgttgatg aacctagttt ttctgtagct tatgcatata tgtgtaaaga 120
gttagctctt atccaagttc ctgctgacca acaaaacggt caatcttcaa ctgttgaata 180
cgtcaatttc cgtaaattgc tcttatcccg ttgtcaaaat gaatttgaga aaaatacagt 240
agatgaaacc gcaagagaag ttaaactgaa agaaattgaa gcgtgtacag accctgagaa 300

gaagaaggaa ctacagttta atttagaaga agaagagcga aagattcgtg ttaaatctgt 360
tggaaatgtt agatttattg gagagttatt caaacaaggt atgttgccac aaaaattatg 420
atgcagtgcc ttaggatgct ctgcagtcaa ttgaagaaga aagcctcgag tnctggcaaa 480
ttgtgcgaca ttgccggatt tagaaattac aaaatcaaga tttnaacctc tttcttaaaa 540
tgcaagaat 549

<210> 1426
<211> 549

<212> DNA
<213> Ctenocephalides felis

<400> 1426

```
cgccgtcagc agcacccagg ccgaatcttt ctgtatctct ctgggtgtcg ggcctctgtg 60
gtggtcctga tgatttccac cacgagatag gacatagccc accaccttac catgaagacg 120
acgaccagct gcggcacacc cgcaaaagca gcataagtga tagcgattca gaagacgatt 180
ctattgatac ttcgccggat tattcccaga acgcggatgt cgatgagtta cttcaccttg 240
gcaaaccagt acgcattaca ttcgaactac ggctcacgaa gcgcaacagc acgctatggg 300
agacagtttt gaaggagaac gtgctctatg taaatattcc taatgtattg ccttctgagg 360
ggtctcgtga aagtttcacg gcgctcctgg aactgaccga ggagaagttg ggatgccatt 420
cgatggtgtt gtgcatgcga cgcgatcgtt cagacagaca gcagctcatg cggcattcat 480
gttcctcggg ttccaactgt gcaccaaaca acaagctaat gcaccgaagg tcacgatgat 540
atttgatct                                                          549
```

<210> 1427
<211> 549
<212> DNA
<213> Ctenocephalides felis

<400> 1427

```
atttgtattt atgatttaga tttatagtgc attaagattt gtgcttcaaa aatgttatca 60
cgtgcctcat atacttcatc tgtattctta aattcgctgc agcaatttcg tttacaaagc 120
acattagtaa ttgctgaaca ttcaaatgca aaattaaacc cattgactca aaatgcttta 180
acagctgcat caaaaattgg aggtgaagta acagttttaa ttgctggcaa aaattgcaaa 240
ccgcttgcta atgaagttgc caaagcagcg ggtttgaaac aagtcttatt agcggaaaat 300
gctgcatttg aaaactgttc tgctgaagcc ttgacaaaac ttgttgttgc agttcaagag 360
caatataaat ttacgcatat tattggtggt gcaagttccc ttggcaaggc tgtattgcaa 420
gaatagcggc aagttagatg tttccctatt tctgaaataa ttgatgttaa agatctgaac 480
attcgtcgac aatttatgcc ggnacgcttt cagacactga ggtgaaagat ccctaaagct 540
atcttcgtg                                                          549
```

<210> 1428
<211> 555
<212> DNA
<213> Ctenocephalides felis

<400> 1428

```
rchsncggaa atatgagcat ttacgtcagt tttgtatgga attgaatggt ttagcagtgc 60
gcctacaagg acaatgcttt cctgaacaat gtacacaaat gactgcaact gaacaatgga 120
tatttctatg tgctgcccac aaaactccaa aagaatgccc agcgatagat tatacaaggc 180
acactttaga tggtgcagct tgtttattaa acagtaataa atattttccc agtcgtgtga 240
gtataaaaga gtcatcagtt gctaaactag gttcagtgtg cagaagagtg tatagaatat 300
tttcacatgc ttattttcac catcgcagaa tttttgatga atttgaagct gaaacatatc 360
tatgtcatag atttacacag tttgtaacaa agtacaactt aatgtcaaaa gacaatctga 420
ttgtgcccat tttagaggag gatgcaacta tcggagaatc agaggcttaa gcatttatgt 480
tatttattta aaatatctgt gcatgcattt tttatttttc ttgtgagtga ncgtttatta 540
aatcattatg tnnat                                                   555
```

<210> 1429
<211> 549
<212> DNA
<213> Ctenocephalides felis

<400> 1429

```
gtcaactggg tcactagagt agatctacga gacaagaaaa tcgaatatat taatggccta 60
ggatacttta aagatcaaaa tactattata gctgtattaa agaataaaag tgaaaaaata 120
ttgtctgcca aaaatattgt tatcgcagtt ggaggacggc ctaactatcc agatattcct 180
ggagcactag agtatggaat tactagtgat gatattttca gtttagatag agaacctgga 240
aaaactgtag ttgttggtgc tggttacatt ggacttgaat gtgcaggttt tctgaaagga 300
ttaggatatg atgcaacagt tatggtccgc tcagtcttat tgcgtggatt tgatcaacaa 360
atggctaata tcattaagga atctatgatt gaaaaaggag tcaaattttt agatacatgc 420
attccaaaat cagtagaaaa atgctcagat ggcaaacttc ttgtacttgg taaatcttct 480
gataatccca cattcagatg tatttgatcc atttgttgta tcggaaggaa gcttaacaaa 540
gattaaaat                                                         549
```

<210> 1430
<211> 549
<212> DNA
<213> Ctenocephalides felis

<400> 1430

```
attgccttaa cgctggcatt ttaaaatccg tctatttggg atcaaactca ttagatggcg 60
atggtacata ctacgacgtc gaacgttttg tgatgcatga taaatataca ccaagaatca 120
ctgtcaacta tgctgatatt ggtctaataa aagtggcaaa agacattgta ttcggtgaca 180
aagtccaacc gatcaaaatt agcaagagaa acatcaaggg tggtgaaatt tgcaaggcaa 240
ctggttgggg tctattaggt tctgtggact cagtaccaaa cgaattacaa caagtagaaa 300
ccactgcaat aacaaacgaa aagtgctttg aattgactca attcattgac ccaacttcgc 360
aaatatgtac attcagggaa tttggtagag gcatttgctt tggtgattct ggtggaccac 420
tagtttacaa aaatgaactt gtggcattac atcgatgcac ttatctcctg cagaggtggc 480
aggcagatat tttgtgaagt gcgagatttc caatcctgga ttaattctga aattgaaaaa 540
ataaataga                                                         549
```

<210> 1431
<211> 549
<212> DNA
<213> Ctenocephalides felis

<400> 1431

```
gcccagtgca cgaggagcat gaggaaccag atccaagaag aactgagcgc ttcgatgcaa 60
tacttggcca tgggggcgca tttctcaaga gacactgtca acaggccagg atttgctgag 120
atgttcttca aatcggcaag cgaagagagg gaacatgcca tgaaactcat gtcttacttg 180
atgatgagag gagaactgac cgagaggctg caggacttga tcagaacacc aactgttcca 240
atcacgactt gggctgatgg tttgagtgct ttgaaagatg ctctgaaatt ggaggcttcc 300
gttaccaaga agattaaaca tgtgatcaaa gcttgcgaga acgataatgg agctaatgat 360
tatcatttgg ttgactacct gaccggtgaa ttcttagaag agcaatactc tggtcaacgt 420
gacctgccgg aaagatctcc accctgggca agatgatgaa ccaacaaggt gttctcggga 480
gttcttgttg acaagaaact ctgggttaaa tgaaacatac atcccatcaa atatcacagt 540
aataaaata                                                         549
```

<210> 1432
<211> 549
<212> DNA
<213> Ctenocephalides felis

<400> 1432

```
gcaattttca gtgataaaag tgaattttaa tttgtaaatt aaaatgtcgc tgtgcaaccg 60
agctgctcaa cgcgagcacg tattagccgt aactcgggat tttatttctc agccccgatt 120
aacatacaaa actgtctctg gtgtcaatgg acctctggtt attttggatg aagttaaatt 180
ccccaaattt gcagaaattg tacaactcag actttctgat ggaactttac gttcaggaca 240
ggttttggaa gtcagcggct ccaaagctgt tgtacaagtt tttgagggta cctcaggaat 300
tgacgctaag aacacacttt gtgaatttac tggtgacatt ttgaggactc cagtatcaga 360
ggatatgtta ggtcgtgtgt tcaacggatc aggaaagcca attgacaaag gacccccaat 420
tttggctgaa gatttcttgg catccaaggt caacccatca atccctggtc tcgtatctat 480
cctgaggaaa tgatccaaac tggatctctg tattgatgtg atgaactcat tgtcgtggac 540
agaaatccc                                                         549
```

<210> 1433
<211> 549
<212> DNA
<213> Ctenocephalides felis

<400> 1433

```
gaaaaacaaa attatttgat acattttaat ctagaatcat ctgtgtggct aaatattatt 60
tgtgtttcaa tttcgactat ttgacgaaaa tagataatag aaatggcacc taaagcattt 120
ggtgatgtga aaatgcaagg acaagcatat aaagataaaa gtaaaccggc tgatattcgt 180
agcagcaata tatgtgctgc gaaagctgtt tctgatgcag tacgtacaag tttgggaccc 240
aggggtatgg ataagatgat ccaagcctca aacggtgaag tgacaattac caatgatggt 300
gccacaattt tgaaagaaat gaatgttact catccagcag ctaagatgtt ggttgaattg 360
tctcgggcgc aagatattga agctggcgat ggcacaacat cagttgtagt agttgcaggt 420
gctttgctgg aggctgctga aaagttactt cacagaggac ttcatccaac tgcatttctg 480
atgcatttca aagatgtgct ccaaagctgt ggaaattctt acacctgtca caccaattga 540
cttactgtc                                                         549
```

<210> 1434
<211> 549

<212> DNA
<213> Ctenocephalides felis

<400> 1434

```
tgtgtattat tcgcacacgt gaaataaatc atatcggtaa acttgtaata tctattttat 60
taactaataa gcgtagtttg tgtaataaca agtaatttct attagtagtc ataagtaatt 120
gaagagaaaa ctattttcca tataagaatt gaaagcataa aatgattctc tccgtgctga 180
aaaaatctcg catcgtgcac ttgtgttttg ccatttcatt cttcacatca ggcctgatca 240
taaacattgc acaattcata ttatacacat gtctcaagcc tttcaacaag aggctttaca 300
gaaaacttgg ttattatttg tgctacacat tttacagtca gatagtgttt ttagctgact 360
ggtggtccaa atctaatctg accttacaca tatcaaagaa agattatgaa caatgtggga 420
aggaacatgg tcttttaatt atgaatcata cgtacgaaac agattggtat taggatggat 480
gtttacggaa aaaattggtg tcttggaatt gtaggatatg caaaaagact attcaataca 540
tccgcatta                                                         549
```

<210> 1435
<211> 509
<212> DNA
<213> Ctenocephalides felis

<400> 1435

```
ttaatattag aagcaatgac tgagagcaaa cccatgttgg tagagcattg cccattactt 60
gtggaaggtg cacctattcc tccagagcct gcaattggtc actggagacc tgaggctaca 120
ttttatcaag atggcgcaag aattgaagct ggttttagaa aatattttca ccgagcagat 180
cctgatcaga aggaagacag ttacacaatg attgtatgtc acgccaatgt aattcgttat 240
tttgtgtgca gggctctgca atttcctgct gaagcttggc tgagactttc attaaatcat 300
gcatctatta cttggattag tatacagccc agtggtagag taattctacg agtttttggg 360
gaatcaggtc atataccagc aatacaagtt accagtcagt aaaattttaa taacataaga 420
taatttcaat ttatttttat tcgttttctt gaaatttata tattttgtat caaattatgt 480
ttatatacct gcaaaaaaaa aaaaaaaaa                                   509
```

<210> 1436
<211> 549
<212> DNA
<213> Ctenocephalides felis

<400> 1436

```
aaaaaatatt attctaacat tatggcgcag atacctgcac gaccgaaaat tttgttaact 60
catccggata taccgaagga agctattgac atcatgtctc ccaattgcga aataattatg 120
tgcaccggac gtccgtctcc atcacgacaa ggaattttgg aaaaaattgt gggagttcat 180
ggtttaatgt ggtgtactaa agaacgtttg gacaaagaga tattagatgc tgcaggaccc 240
aacattatgg ccatatctac tatgtcggct gggctggaca atgtagattt acctgaaatc 300
cgcaaaagaa atattccaat tggttatact gcaggagttc tgaataatgc tgttgctgat 360
ttaactgttg ggcttatgat agctactgct agaagatttg gagaagctag gaaacacata 420
gagaatggaa catggggaag cggacctgcg tggtttttag gcagaaatgt atctgaaact 480
cagctggtat tgtcggctag gagaaattgg caaactgtgc aagaagatng aaagcttcaa 540
tatgactat                                                         549
```

<210> 1437
<211> 251
<212> DNA
<213> Ctenocephalides felis

<400> 1437

```
gaatttggta gaggcatttg ctttggtgat tctggtggac cactagttta caaaaatgaa 60
cttgttggca ttacatcgat gcacttatac tcctgcagag gtggcaggcc agatattttt 120
gtgaaagtgc gagatttcca atcctggatt aattctgaaa ttgaaaaaaa ttaaatagat 180
tccaatcatg atttgttata atgaaaaatg gtttaataaa ggcagcataa tttaaaaaaa 240
aaaaaaaaaa a                                                      251
```

<210> 1438
<211> 549
<212> DNA
<213> Ctenocephalides felis

<400> 1438

```
gttttattga agaaactaaa aatgcatttt tattttaaaa ctggccctgg tgttcatgtt 60
atatgcatac agaagagaag taatttgggg aatacacaaa aatggaagat gatgccgata 120
taagagaaca aatttttcac aacaatgtca gagagcagat tatatttcta ttactctttc 180
ttctgctgta tttctcttcg tactggctga ttggatggtt tcgtcgcaaa gagcgcgatg 240
atttttacac ttgcgcctac gacgacgatg gcgaggcgac ggtgtacaga atcagcctat 300
ggctctgcac cttcgcgctt gcagtcagcg tgggagcagc acttttacta cctatgtcta 360
ttgcaagtaa cgaagtgctg atactgtatc cgaacagcta ttatgtcaag tggctgaaca 420
gctcactgat tcaaggattg tggaaccatg tattcttgtt tcaaatttat cactattcga 480
cttctgcatt tgcatacctg ttgcggagtc ttccggattc gaaggacgcg aaaggagttt 540
ggcagactt                                                         549
```

<210> 1439
<211> 549
<212> DNA
<213> Ctenocephalides felis

<400> 1439

```
gtcaggcgca ctttacttaa ctgaaggtgg agaacatcat gctgttgctt ccatcaaata 60
tcacgaaaag tacagcccaa acactttgga taatgatgtg gcagttttga agttgaaaaa 120
tccattgact ttcaatgcta accagaaacc tgtcgccttg gcctcaaagg atacacctgg 180
agacctcaaa tgcaaattct ctggttgggg attagacgca tatccaagtg atgttttacc 240
aaatcattta caaaaaatgg acgttctgac ctacaataat gctgattgcc aaaagttcca 300
taatgctgga cctaaatcta acacaatcta cccaggaatg ttatgcggat tcaacaaact 360
taatgttggt gcttgcaggg gtgattctgg tggccattgg tatacgaaag tgcaaatggt 420
ttggaacaag tcggtgtagt ttcctgggtt tatgaatatt gtgctgtggg tgtgccagat 480
gtctacgttc gcgtatatta ctattggact ggattcacga aaatccgcct gttttgcata 540
aatatataa                                                         549
```

<210> 1440
<211> 549

<212> DNA
<213> Ctenocephalides felis

<400> 1440

```
gaattggcta gtgaatagtg tttgtgttta ttaataaaaa atatatgatt tatttactat 60
ttacttcaaa atattaaaat gtttcccagt tcaaggtcta tagtagtgtt aagtaacaag 120
gcatctcggg aatttttgaa acaaaaactt atacaaaatg ttatcgtgaa caagtactct 180
acaaatatag ctgaggctgt atccgtcaaa gatgctttac catacaacaa gatacctgga 240
ccttcaacat taccaattat tggtgtagcc catcattttg cacctggagg taaatacaaa 300
ggtctcgatt tagcccaact aacagaaaag ctctatgaag aatatggaga tattgttgcc 360
atcagaggat tgcctggtaa acccgatatg gtttccttta taacttcgat catatggaaa 420
aagtatatcg tctgaaggcc ttgcctgata gaccagcttt tgaatcacga gaatattctt 480
gagagaaaaa agaccagaag tttcaaattg ttatggctaa tcactccaag ggaagaatgg 540
caaaaatcg                                                         549
```

<210> 1441
<211> 549
<212> DNA
<213> Ctenocephalides felis

<400> 1441

```
attttgacca catacacaat ggttagcagt agtcctgagg agcggaaaat gtttcgtgtt 60
acacaaatgc attatgttat atttgatgaa gcacacatgt tgaaaaacat gaatacacag 120
agatatgaca atttgattaa aataaaagct tcaagacgaa tattgctaac tgggacacct 180
ttgcagaata atttgttaga gttaatgtca cttttatgtt ttgttatgcc ttctttattt 240
gattgccaaa gggaggattt gaaaagttta ttccagaaaa attcaaaaac taactcaaca 300
gtgaagaaag gtgaagatga tgatgattta ccactttttg aacaaacaca aataacacaa 360
gctaaaaaga ttatgaagcc gtttgttctg agaagactaa aaagagatgt tcttaaagat 420
ttgcctaaaa aaactgatta cactgacaag ttccaatgca ccttctcaaa aacttcaata 480
tgagcaatta attcaaacct tttttcagaa ccggagaaat catgcaaata aagaaagagt 540
ggaatgcaa                                                         549
```

<210> 1442
<211> 549
<212> DNA
<213> Ctenocephalides felis

<400> 1442

gtggtgcagg atttgtagat ggtatgaatc ttggagataa caccaaggca gcagttattc 60
gtcttggttt aatggaaatg attcgttttg ttgatgtttt ctatccagga agtaaacttt 120
ctactttctt tgaatcttgc ggtgtggctg atttagttac tacatgttat ggtggtagaa 180
atcgtagagt ttctgaagca tttgtgaaaa caggtaaatc attgaaacaa ttagaagacg 240
aaatgttaaa tggtcaaaaa ttgcaaggac caataacagc agaagaagtt aattttatgc 300
tgagaaatcg tggcatgcaa gacaaatttc ctttgttcac tgctgttcat aaaatttgca 360
caggagatat ggatgttaaa gaatttctta attgtataag gagccacccg gaacacatgt 420
aagtgcctgc tttataaaat ataaatcaga tttaaaatgc ttctcagatg gtaatattag 480
taatgtcttt acatgaacat aagtgagagt tgctagttgt ttgatatttt gntcttcata 540
tagtatttg 549

<210> 1443
<211> 546
<212> DNA
<213> Ctenocephalides felis

<400> 1443

atttgtttt acattaaatt tttcaaattc gatatgaaat ttttactggc aatttgcgtg 60
ttgtgtgttt tattaaatca agtatctatg tcaaaaatgg tcactgaaaa gtgtaaatcg 120
ggaggaaata atccaagtac aaaagaggtg tcaataccat ctgggaagct tactattgaa 180
gatttttgta ttggaaatca tcaaagttgc aaaatatttt gcaaaagtca atgtggattt 240
ggaggtggtg cttgtggaaa cggtggttca acacgaccaa atcaaaaaca ctgttattgc 300
gaataaccat attccggatg aaagaccaaa ttgatataaa ttactaaaat tatgctagat 360
agcaatcata aaattttgaa gttttcaatg atcctaacat gttttgcctc aatttatttt 420
aacagcaaat tgtggaacta ccgtccgtac aaatgtcaag aaatctgatg ttacaataga 480
tattataata tgtacattgc tatattatag aatatatact gattgcaagt tgaaaaaaaa 540
aaaaaa 546

<210> 1444
<211> 550
<212> DNA
<213> Ctenocephalides felis

<400> 1444

gcatnatgct gaagtgtgga tttatcgttg ttttncttgt agcctccgcc ttgggcgagt 60
tttcgctcga cgaccgcatc gtaggcggca ccagtgttaa tatcgagaac ttcggatggc 120
aagtgtcctt gttcgatcgt atgggacact tctgcggtgg ttccatcatt agcgacgaat 180
gggtcttgac agcagcacat tgcgtatttg acctattctc gccaaagcaa tatgcagtgc 240
gtgtcggaag tagtttacat aacaaaggtg gattnatcca caaaattgcc aaagtatata 300

tccatccaga ctacgatgaa gtaagctacg acaatgacgt cgcagtcctg aaagttgaaa 360
agagatttag actgaacggc atgagcgttc gcacagttaa attggttgac gaagatcacg 420
agttgatgat ggtgcccact tactgtcact ggatggggca aattaatgaa taggcccacc 480
cataaattac angagtgaaa gtgcctttgt cgaccatgat catntccgca gtactctttc 540
cggaaacaat 550

<210> 1445

<211> 550
<212> DNA
<213> Ctenocephalides felis

<400> 1445

```
gttatcaaca ggattttgga gatcgtgcaa ggtgtttcca acctagtcta acttcttcat 60
cttcaatatg tgatgaacga ttcacttgga accctgataa tgagcaaaga atgattttac 120
aagaagctct gtccaaagca cacactcatt taggcactgg agcatcaata ggttccggtt 180
ccatttctgg tagctccatt ggttcttctt ctacgtcgtc ttcctcatct agttcttcct 240
caagttcttc ttcatggtct ggcagcggaa tgggagttag ttcaactcaa ggacaaaagg 300
aaattgttca aatcagccca caaaaagtag ggcttaaatt aagaattaat gaagttcata 360
aactaaatgt ttattatgca caagctgaga ttatccagta gattatatta tttgatggac 420
ttgctaagtc tatggaagat gataagagag atgncagcat tggagattac tctcagaacc 480
tgagaaatat actcaaattc cggtgggttt ggactttgng gataagtgng atgcntatgt 540
gcaacatgcc                                                        550
```

<210> 1446
<211> 550
<212> DNA
<213> Ctenocephalides felis

<400> 1446

```
cttgtgccgg tccgagagca ccctaaattc aactttgttg gaaaactgtt gggacctaag 60
ggtaattcca tgaaaagatt acaagaagat actatgtgta agatggccgt cttaggtaga 120
ggctcaatga aagacagaca aaaggaagaa gaacttcgta attccttaga tcccaaatat 180
gctcatttgg ctgatgatct acatgttgaa atatcggctt tgggtcctcc acagaagctc 240
acgctcgagt tgcttatgct ttggctgaag tccgtaatat ttggtccctg ataataatga 300
tacaattcgc caagaacaat gcgtgaaatg atgactgacc caatgcccgc cagaagaaat 360
gagangagca cccacattag acgaggtggt tggctgtggt gcttctcgng aggaggatac 420
taatcgngca gcttcgacat ntggggnatg cttctgngtt ctaatgtgat ccggcctcac 480
ccctcctcac tcgtgtatcc tgtaactaaa tcttcattaa taacaaagtg ctgagaactt 540
gtntaaaccc                                                        550
```

<210> 1447
<211> 550
<212> DNA
<213> Ctenocephalides felis

<400> 1447

```
tcttattcat attaattata agaacattta ttngtatttc ttcaaattct tgattaggag 60
catgaattgg attagaaata aatttattat catttatccc tatactaaat gataataaaa 120
atttactatc taatgaatct tcattaaaat attttctagt tcaagttttt gcttcaattt 180
tattattatt ttttatttct ttgaattttt tatttaaaaa ctttttttaga ataatatatt 240
ttaatgaaat ttatttaatt ttattaaatt catccctatt tttaaaaata ggggctgccc 300
catttcactt ttgatttcct agaataatag aaggaataaa ttgaataaat aattttattt 360
taataacatg acaaaaaatt aaccctataa tttgttaagt tattgnatta atataaatta 420
ttttatttaa ttcattatta agaattataa ttggagctta ggtggattaa ataatcctct 480
tacaaaaatt atacttattc tcaatacact atggctgaat aatattgttt aataaataat 540
gaataatttt                                                        550
```

<210> 1448
<211> 550
<212> DNA
<213> Ctenocephalides felis

<400> 1448

```
attttttaaa gttttttttt tttgaaatta atgtatttgc acagtgctca aatatataat 60
attgcttcta acatataaaa aatgagtttg aaaagacagc gcgatgataa tcttaatgaa 120
gaacctgata agaagcttat caaccgttca tgtccatact tagatactat caatcgtcac 180
gttttagact ttgattttga aaaattgtgc tcagtctcgc taactagaat taatgtttat 240
gcatgtttag tttgtggaaa atattttcaa ggcagaggaa acaacactca tgcttacacc 300
cattctgtca gcgaatctca tcacgtctac cttaatctga caacattgaa attttattgt 360
ttgccagaca attatgagat aattgactct tccttagatg atataaaatt gtattaaatc 420
cagtgtttca cctgaaagca tagctgcatt agatgtaatg tgaagctatc tagagctatg 480
atggtctatg atatgcccgg aatggtggtc ttataatatt aagcaatgct atgcatgtat 540
ctgcagcctt                                                        550
```

<210> 1449
<211> 550
<212> DNA
<213> Ctenocephalides felis

<400> 1449

```
gtcagatcaa gaatgtgcca gtcttcccaa ttcaagatgc gatagtggca tttgtgtttg 60
caaattcaac tttgtgcctc atccagaaga cccaatatgt ctagaacgta aacaattggg 120
tgaacactgc aacgtaggcg aacaatgtca cacaaaattc aacttcaatg ctcattgtgt 180
ccgagaagtc tgtgtgtgca gggaagatca ccacgaatcg aggaacagaa cttgcatcca 240
atctaaaggc tataaccaga attgcgttga tgatatcgaa tgtttcatcg gagaagaata 300
cagagacagg atcagatgtt tccagaacaa gtgtctttgc aaacctgaat ttccagtcac 360
tgaggacgga aaatgcggat ccggagctcc aggcacacaa gtgtggtcct cggttatgac 420
attagcagtt atggcatcag tagcaaaaat catccgatgt aaaagtttaa aaatagaatg 480
tccaagaaaa taagaaaaga cttaccaaaa cttacttcga ttacttaaga acatcttttc 540
nagcatagng                                                        550
```

<210> 1450
<211> 550
<212> DNA
<213> Ctenocephalides felis

<400> 1450

```
gtgnaacttc atgtactata atattatatt tgtattatat gtattataaa ttcatacatg 60
cacacacaca catatgtata tatatatata tatatatata tatatatata gttcaaaaat 120
aatagcagct aattattcaa ccaacatgat aatataactg ttacggattc ttcacaatta 180
ttcaatattg aataattcag aatatatata tatatatata tatatatata tatatatnca 240
tgtgtacgaa catgtatatg tgtatatata tatatatata tatatatttn tatatatata 300
tatgtatatt cgcacatgtg tntgtacatg tatataggta tagatgtttt ttttgacana 360
catantgtnt attagtatat atataaacta tagtataaaa atataaatga gtggttcatt 420
tactttntgc acacctgact catngaatct tgctttctaa gacataaaat agaataatta 480
aaattngngc tcatgtattt naacaatata ctatctatgg gggantgtat atgccngctt 540
atctatttnt                                                        550
```

<210> 1451
<211> 550
<212> DNA
<213> Ctenocephalides felis

<400> 1451

```
gaaatatgca tcggtagaat attttaagtt tttgtgaata ataagttatc gagaatcttc 60
agaaacaatg ggagcaggca gcacgatatt tagtgcaaga caagtgctgt ggtgtatggt 120
gttctgcgga ttcgccgtta actatatgat aagaattaat ttaaacatag caatagtgtc 180
tatggtcagg catcgatctt taattgtggt taacgaaaca attaacgaaa aattgcaatt 240
agttttggat tctggggcgg aaaatgtttc ggaaagttca tccagcaccc aagcgcctgg 300
accaagcgag atctatcagg aggaagatgg tttcatctgg gacgaatacc agcaagggtt 360
aattttaggg gcatttttct ggctccactg gatcactcaa gtaccaggag gaatttggct 420
agaaaatatg gtctaaactg gtgtttggtt tgcgaatttc taggtgctta ttttgctcat 480
cacccctatc gcgtatatga atatcaacac tatatnctcg agtaatcagg cattgttgcg 540
gtttcttggc                                                        550
```

<210> 1452
<211> 550
<212> DNA
<213> Ctenocephalides felis

<400> 1452

```
aattgttgct ctgatcattg ccggagttga gcttctggga gccatatttg ccctgtgtct 60
tgcaaattcc atccgcaacg aagataggag gtacgcataa gatctacttc aatactgtgt 120
gtgaatatgt aattcgtttt tgcaaaatat ctattattat gcaaaaacaa tcaacaatta 180
taccttcaaa tttgaaaata ataattcgat gtatgtatac aaaagccaaa ttttactaaa 240
ggcttctaat ttaagaaata ttatataatt gagaacattt ttatgaacta tttaaacgtt 300
aggaattatt tggagtgttg tcaaatgctt ttaaatgtat taatatttac aatttgatta 360
ttctcaaata acaaatattt ttaatatttt tgtaatgtta ttttatatat tatcgaagta 420
aacacaaaga caaaaaatat atctacacat gtatgtatat atatatatat atatatatat 480
atatatatat atatatatat atatanacac angnnnaccn cacacacata natttctatg 540
tgtgtnnatg                                                        550
```

<210> 1453
<211> 550
<212> DNA
<213> Ctenocephalides felis

<400> 1453

```
atttttcgag tgccactaag atggttaaaa ttnntaatga accgcgaaac tgggaaagtt 60
atggaatagt tgatgagcaa caacaacctt caacctcctc tgcaaacctc aattgcctaa 120
atccaactac tacttctact tcagaccgtc ccgcagcgac ggtttccaaa agaaaactgc 180
cttcatatcg agaaaaaatg ccacgttctc gcacaagatc gcgatcccgc agtcgtcggc 240
ataacaatca tacaaatcat cggagggact attctgatga gcgccattct ataagagtga 300
ggtcttcttc tagaaggagg agacatcata gaagtaggag caggaataga gacagacata 360
cacgtaggan aaacgagagc ggagatcatc atcgcagtta caggcgcagt tcaggagaaa 420
ggagctatcg aagacaccac agacatggag atgattcagt cccgccgaac aagtacgcgt 480
gaaaggcatc aggaaggtcg attnagatga taggaggaca tctcatgtca aacctgngat 540
ataattggag                                                        550
```

<210> 1454
<211> 474
<212> DNA
<213> Ctenocephalides felis

<400> 1454

```
gtcncattgg tgccttccaa acccatgtgt gtcgaatcct tccaggagtt ccctccattg 60
ggtcgtttcg ctgtgcgtga catgagacag accgttgccg tcggtgtcat caagtctgtg 120
aacttcaagg atgcctccgg tggtaaggtc accaaggctg ccgaaaaagc caccaaggga 180
aagaagtagc tagatctacc gatctgctta ctgcagatgt tcaacactgt aatgaaacac 240
tacttccatc gcaaagcgtt tcgaagaaaa aaggcctcat tcattccttt actatatgtt 300
ttttgactca gctttatatt ttatatgact attttataga ataataatta ttttatgttc 360
tgctctatat taaaatgaat ttatattaat gtatggatat tatgtgctaa gaaagaactt 420
gaagagattt gcagctgatg tgtattatag tatggnataa atggaancca aagt       474
```

<210> 1455
<211> 347
<212> DNA
<213> Ctenocephalides felis

<400> 1455

```
gtttngtctc atgcaataat taattaaact tgtttgttag aggtgcaaaa taaaattaaa 60

ttaaaatgac tgcctggaga caagctggtt taaactacat taactttca acaatcgctg 120
cccgaatggt ccgccaagct ttgaaatctg atctaaaaaa tgaggctttg aaacgggacg 180
tatctagcat taaattcaca ccctggaagg acggaaaagc gatcaaaagt gaaaccaagt 240
aatcacaaga tggaataata gacaattcac tcaaattaat aatgtgtacg taaccgataa 300
caagaataaa tgttagttng atnaaaaaaa aaaaaaaaaa aaaaaaa              347
```

<210> 1456
<211> 356
<212> DNA
<213> Ctenocephalides felis

<400> 1456

```
agtttgctac acacagcacg tgtaaatttt cataaaataa attaaaaatg gataaacctg 60
tagttcttgc tcgcgttatg aaagtcttgg gccgtacagg atcccaagga caatgtacac 120
aagttaaagt tgaatttatt ggtgaacaaa accgacaaat catccgaaat gtcaaaggac 180
cagtacgaga gggtgatatt ttaacacttt tggaatctga acgtgaagct agaagactaa 240
gatagattaa ttctaaaatg tgagaggtga tatcctgaac tataatatct gggttcaatt 300
atttgtatgg aattataaat atacgactta tcactcccaa aaaaaaaaaa aaaaaa     356
```

<210> 1457
<211> 550
<212> DNA
<213> Ctenocephalides felis

<400> 1457

```
cttaaccacc gtcacccact cgccaaatta caccatggct gaaccagaaa aggctcctga 60
acaaccaaag gtgcccccac agaagcaaat catcgcttca aaggtgatgg gaactgtgaa 120
atggttcaat gtcaaaagcg gatatggatt tataaataga actgacacca aggaagatgt 180
gtttgtacat caatctgcca tagttaaaaa caacccgaag aaggctgtcc gcagtgtagg 240
ggatggagaa actgtggagt ttgatgtggt aattggagag aagggaaatg aggcagctaa 300
tgtgaccggc ccagaaggag agccagtcaa gggaagccca tatgcggccg acaaacgccg 360
cagctatcgc caatggagct ccctcgtgga ggacccaggg gacccagaag aaattacgaa 420
ggaggcgaag gtaaagaagg gtcgggttcg ggaggcgaga acggtgatgg caaggcccgt 480
caaggaggcg gccccgcgtg gccgattccg cggtcgccac gatacacact attacggcgt 540
ggcggacggc                                                        550
```

<210> 1458
<211> 550
<212> DNA
<213> Ctenocephalides felis

<400> 1458

```
aaataatctc tgaaaatgtt gaagggcgtt gtaggtcttg ttactggagg tgcttctggt 60
ttgggaaagg ccactgtaga gcgaattgtg caacaaggtg gccgagttgt tttatgtgat 120
ttacctaaat caagtggatc tcaagtagca aaagatattg gagataattg tttatatgca 180
```

```
ccagttgatg tcacatctga aaaagatgtt gaggaagcaa tagcattaac taaagaaaaa 240
tttggtcgtc ttgatgttgc tgtaaactgt gctggtatcg gtgtagcatt taaaacatat 300
aatttcaata agcagttgcc tcataaatta gaggatttca caaaagtttt aatggttaat 360
actgttggaa ccttcaatgt aattcgattg ctgtggattg atgggagtaa atgaacctaa 420
taaggatggt caaccaggag tgattgtaac acacaagtgt gctgcttatg atggcagatg 480
ggacaagctc ttattctgcg tccaagggct ttgtggaatg cactgctttt gctaganatt 540
accaggcaag                                                        550
```

<210> 1459
<211> 546
<212> DNA
<213> Ctenocephalides felis

<400> 1459

```
atttaattaa aatgggtaaa tcctaggcaa tattagaata tgatgtactc atgagatggc 60
aaatcaaaat tttacaagtt cagatgcaaa tgaagaaaat acaaatgaac ctctaccaga 120
atttatttgc tcgtgttgtt cacttaaaca accttatgac tataaaggat gtaatcctcc 180
atttgcaaaa aatatagcta caattgatga atcctatata atgaaagatc cttttagtcc 240
tgagaacaaa aatgaaattc ttatattggg agccgattgt agtatttgtc aagaatctgt 300
atgcgtatca accacttgca gtatattttt catgaaaact ctatgcaaga aatgtgcatt 360
gaaacagaaa aacatgtacc ctgaacgatt taaacaaatt attgacatgt ttttaaataa 420
atgcttaggt agttgaataa atgttgtatt taaatttata tttaaaatta actgttaaat 480
attgngaaaa agaaaatata gaaaataatt gataaaatgt gggtgatatg aaaaaaaaaa 540
aaaaaa                                                            546
```

<210> 1460
<211> 550
<212> DNA
<213> Ctenocephalides felis

<400> 1460

```
gattcagctt tgcatagaat tgtagatgca actgatgcaa ttgttagcac tgcatattcc 60
cataaaagaa catttattat ggaagtaatg ggacgaaact gcggatattt agctgttgtt 120
gcaggcttgt gtgttgaagc tgattttata tttgcgcccg aagatcctcc agatgccaac 180
tggccaagtg ttttatgcgc actattaagt caggaacgat tagctggaag aaggcaaaat 240
attatcatgg tatcagaagg ggcaattgat agaaatggag aacctataac agcagaaaaa 300
attaaagaag tcattattgc aggtttaaat caggatacaa gaattacagt gcttgccatg 360
tacagagagg tggaagtcct tctgcttttg atcgattact gggatgtcgc atgggggcag 420
aagcagtatt agcccttatg gaagctcatg atgagtctga ccttgtgttg tgaccttgcc 480
cgaaccaaca gtcgtcttca atgatggaat gtgttctcac caaactgtac ttacgcttgg 540
aaaataaaat                                                        550
```

<210> 1461
<211> 550
<212> DNA
<213> Ctenocephalides felis

<400> 1461

```
caaactcatc ctttgtagaa cgtgtcaaga agcgtggatt cgaagtaatc tacatgacag 60
aaccaattga tgagtatgtt atgcagcaaa tgaaggatta catgggtaaa actttagttt 120
ctgtcacgaa ggaaggtttg gagttgcctg aagatgaaga tgaaaagaag aagcgtgaag 180
aggataagac taaattcgaa agcctttgca aggttatgaa gaatatcttg gataacaaag 240
tagagaaagt agttgtgagc aaccgattgg tagattcacc ctgttgtatt gtaacatcac 300
aatatggctg gacagccaac atggaaagaa tcatgaaggc ccaagctctt cgcgattcct 360
cgaccatggg ttacatggct gcaagaaaca cttagaaatt aacccccgatc attctgtgat 420
tgatacttta agacaaaagg ctgatgctga tccaaaggat aaggccgtaa agatttagtt 480
atcttacttt tcgagacagc tttgctgcat ctggtttact ttagatgaac cccaagtccg 540
ctnaagaatt                                                        550
```

<210> 1462
<211> 312
<212> DNA
<213> Ctenocephalides felis

<400> 1462

```
attgnaactt caagaattgt acgtaaaggc tttgactaat gaggagtgca aagctaaatc 60
accaattcca ccaacgaccc aagtctgcac acttttggaa aagaatcacg gagtatgctc 120
gggagattct ggtggtccat tgcttttgga tggcgagcaa gttggcattg cctcatttgt 180
tatcttcaaa tgtgcaatgg gataccctga ctatttcaca agattgtctc tatatgtaga 240
ttggattgaa caacacatgg attaaaaata ataaaataat aatttaatgt aaaaaaaaaa 300
aaaaaaaaaa aa                                                     312
```

<210> 1463
<211> 550
<212> DNA
<213> Ctenocephalides felis

<400> 1463

```
gtcaaacatc acaaaaatat atagctttat gtgtcacaaa tgattacact tttatcgtta 60
tgatataata caaaataaat acaatttctt acatgctatt attataattc aatattaaaa 120
catttgtact ataatataac aatgaatttc ttatatttat taatatgctc tctttgtgcg 180
gtagtaaatg ccggtgggga tacattagtt ttattagaca atcttgcaat taaagaaact 240
cattccatat tttttaaaag cttacaagat agaggttatg ttctaacatt caaattggct 300
gatgaagcca atttagtttt atctaagtat ggagaatatt tatacaagca tttaattcta 360
ttttcacctg ctgtggaaga gtttggagga tcattaagtg tcgaggcaat tcagaattca 420
tagatgaagg tggcaatgtt ttggtagcag gaagtgaatc ctggtgatgc aattcgtgaa 480
tagctctgaa tgcggtttga ggagatgagg aaggacactg tatggacatc ttaatatgat 540
ggtctgataa                                                        550
```

<210> 1464
<211> 550
<212> DNA
<213> Ctenocephalides felis

<400> 1464

```
gtcgttttac atgcgccgga cagacatact tcagcgcgct ttaccaacaa tgcgtgcaag 60
cgcctttatc cgagtgcttg gcgacagctg cacctccacc accgccacca ggaccaggac 120
catcgcaacc ctttgcttgc gtgcggacag gattgttttt ggattacact gataattctt 180
gcaaatggta ctacgagtgc acattaaacg ctcaaggtgt attcgacgtg gcccgatatg 240
cttgtgcagc tgagttatat ttcaacagcg ttctgcagca gtgcgtacct gcataccagt 300
cagactgttt aggtgcaagt gtcacttctt ccccatccat accatcctta ccatccttac 360
catctttccc aaccttgcca acatcttccc caacagcagg atttcctttt ggccgaaaat 420
ctcttgatat gcaaacaaaa actggaacta aatgtacaaa aggagaagtt tctaaagacg 480
attaaattct acatgtccta gtgagtgtgt atatgaaatg ctattgatat acatcaagct 540
tataaggtat                                                        550
```

<210> 1465
<211> 550
<212> DNA
<213> Ctenocephalides felis

<400> 1465

```
cgttaacgtt ggtttttcgt aacgtattat atatacaagt gtgtggatta attaaatatt 60
tacaatggca actgaagtgg taaacaacgt gcaagtcacc gagaaggatg tcaaagagga 120
gttgcccgag aaaaaaattg aagaagaagc cccggccaca gagaaatctg aaaaggccga 180
tgaggctccg gcttcgaaga ccgagcctgc accaccgaag gtgctcgtgc ataaaacaaa 240
cttcgaaaag gataccgtgt atctttacca attctccaga actcctcttt tgccatcgat 300
gtcgccgtac tgcttgaaag tggagacctg gttgcgtttg gcaggaatca aatacgagaa 360
tgtcgaccac aagatgaaat tccgcagcaa gaaaggacaa ttgcctttcg tcgaattgaa 420
tggtgaagaa attgctgcag tgccatcatc atgaaggagt tgtcaaacct acggaaagga 480
tctggatgct gtttgacacc agacacgcat gtctccatgc tatggatcca tgattgaaaa 540
ccacttgtat                                                        550
```

<210> 1466
<211> 546
<212> DNA
<213> Ctenocephalides felis

<400> 1466

```
tttttttttt ttttttttatt tgcattgnga tagccggccg attgcattca gttctaaaat 60
gccgatcaaa agtatcaaag ctcgtcaaat tttcgactct aggggcaatc ctaccgtcga 120
agtcgattta gtgaccgaat taggattatt tagagcagcg gttccttcag gtgcttccac 180
aggagcttat gaggctcttg aattacgtga taatgataag actcaatata tgggtaaagg 240
agtatccaaa gcattgcaca atattaatca attgattgcc cccgagttaa ttaaacaatc 300
ttttgaggtt actcaacagg aagagatcga caaatttatg ttgaaactgg atggtactga 360
gaacaaatct aaatttggag ccaatgctat attaggagtt tcattagctg tgtgcaaagc 420
tggtgctgct aaaaagggtg ttcctctgat caacatattg cagatttggc tggaaataaa 480
aaaaatgngc ttcctgtcca ctttcaatgt atcaatggcg gagcatgctg gaataactgt 540
ttcaga                                                            546
```

<210> 1467
<211> 550
<212> DNA
<213> Ctenocephalides felis

<400> 1467

```
gtaaaataat taatttattg ttaattacat tataaaataa gctaatatgt aactcatcac 60
aaaaatagca tttaataaat tacaatggcc cacctaaaca agagtttgaa accaataaat 120
tactccaaca cgtcattcag acactttttt cttgtattat ttttcgtatt ggttttcgcc 180
ctcagtattg ctttattagt tttatttcca tatagtggaa aaagacatga aatctataag 240
gatataagtt tggaagatgt cataaacgtt ccagaacaaa tcattcatga tcaagctccc 300
tcagcaaatc ctcgcaatcc aaaatgcagc cactgggatt gttttaatgt gtatcgctgt 360
ggcaataaag ggcacaatca aatatctatt tatatttatc caattaaaaa atatcttgat 420
gctgatgata tacctgcact ggtatcatgt gcaaagaatt tattttattt taaaaactat 480
aaaagatnca aatttacaca tcaaatctga tgaggctgga tattggtcca agattgtact 540
taaatcaaac                                                        550
```

<210> 1468
<211> 550
<212> DNA
<213> Ctenocephalides felis

<400> 1468

```
gaggaaagng gcaaagatga tgacgaggat anggaagcaa ctgaaaatga cacaacaaaa 60
gcagtgcctg gaatttcttt tgtcaaattg acatgcgtgc attgctctac aaaatgtgct 120
acattgaagg agtatgttac ccatttgagg agcaaaatac acaactcata tatgagccgt 180
ttagctgcac gtcacaaatc tcgattggcc aaaatgcgcg cacaacagag gaatgctcag 240
cgagagatcg atgaaaagaa tcaggaggac tattatttaa agacaaagtt ttgtcctact 300
tgcaaactta attacaggca actcagagct gaacatcaag cttctgaagc acataacgat 360
attaagaagt atttgaagcc ttattgtagg acttgtcgta tgactttttc aagtcctatg 420
cgttatgaag tacatatttg ttcaatgcac acataaaaca taaagctcat ctggagtatg 480
taaaacacaa aaaacaagat atgaagaagt tggtagtgat gaagctgaaa gaatggattg 540
gatatttatg                                                        550
```

<210> 1469
<211> 550
<212> DNA
<213> Ctenocephalides felis

<400> 1469

```
cagaagctca agccagagcc gaagaagtcg aggaaatcag gaggaaatac agtgccagaa 60
```

tccaagaaca agaggagcac atcgagacct tgttggtcaa gatcagcaat ctggagaaac 120
agaagagcag actgcaaagc gaagttgagg tcctcatcat cgatctggag aaagcaaaca 180
gcgctgcccg cgatttgcag aaacgttgcg agcaattgga acgtgtcaac atcgagatca 240
aatcacgtct tgaggagacc attcagttgt atgaaggagc ccagagagat ttgagaacca 300
agcaacagga gttgcagagg gtcaaccacg aattggacaa gaccagggaa cagaaagatc 360
agttggccag ggaaaacaag aaattgggag acgaattggg agatgctcgc aaccagttgg 420
ccgaatacaa cagacgtttg cacgaattgg aactcgaatt gagaagactc gagaatgagc 480
cgaagagttg ctgctgcatc aagaggccga actgtcgcaa ggccaagaca cgttccacgt 540
tggcnacgat 550

<210> 1470
<211> 550
<212> DNA
<213> Ctenocephalides felis

<400> 1470

gcaantcgca aacgagtttc agagcacagc ggctgcgcat ctgttaagct caacggctat 60
agtttttcag tattataatt tgtattaagt cgaagtaaat aacaaacatg agggaaattg 120
tgcacattca aaccggacag tgcggaaacc aaattggagc taagttctgg gagatcattt 180
ctgatgaaca tggaattgac ccaactggag cttatcttgg agatcatgaa cttcaattag 240
aacgtatcaa tgtttattac aatgaggctt ctggtggaaa atatgttcca cgtgccattc 300
ttgtggattt ggaacctgga accatggact ccgtgcgttc tggaccatac ggtcaaattt 360
ttaggccaga caattttgtt ttcggacaaa gtggtgctgg taacaactgg gctaagggac 420
attatactga aggtgctgaa ctagtcgact ctgtttggat gttgtgcgta aagaagctga 480
atcttgtgat tgctgcaagg ttccactacc cattcttggg angtggnctg cttgnatggg 540
acactattga 550

<210> 1471
<211> 550
<212> DNA
<213> Ctenocephalides felis

<400> 1471

ctgtgccaga catccaaaaa ccagctcctg aatttgcagg tactgctgta gttaatggtg 60
aatttaaaga cattaaactc agccagtaca aaggaaagta cttggtacta ttcttctatc 120
cattggactt cactttcgtg tgtccaactg aaatcattgc cttctctgac cgcattgatg 180
aattccgcaa gattggatgt gaagttgtag cagcctcttg tgattctcac tacagccact 240
tggcttggat taataccgca cgtaaagaag gtggcctggg acaaatgaat atcccacttc 300
ttgctgataa atccatgaaa attgctcgtg attatggagt tttagacgaa tcatctggag 360
ttccattcag aggcttgttt atcattgatc ccaaacaaaa tgttagacag gtcactgtta 420
atgacttacc agtaggcaga tctgtagatg aaaccttgag attggtcagc cttccaatta 480
ctgatgaaca cggagaagtt tgccactggc tggagacctg tagtaaaact atgaaagcgg 540
accaaacttc 550

<210> 1472
<211> 550
<212> DNA
<213> Ctenocephalides felis

<400> 1472

```
cagcaactca tttaatattt gtggatcctg atgcgaaaaa tgaaacatgg atattacaca 60
cacatattgc agcagtagaa cgattaccgt tgagtactac aggatgccca cttcaaattc 120
gatgcaaaac attccaatca gtaatatttc ttataagaaa agaaaacgat agccatgacg 180
tttataatac tttgttgcaa ttagcccaac ctgtttctat cgatgatctg tattgcttcc 240
agtacaccat aaataaaaat gatatgcata agagcgaagg ttggaattat tttaatttag 300
aggatgaatt taaaagaatg aatgttccaa atgatgaatg gatttacaca gatttaaatg 360
aaaattatga gctatgtgat acttatccta attgttgtat gtgcctgcta acagcacaat 420
aaatatgtta caaggaagtg caaaatttcg atccaaagga agataccggt ctcacatatt 480
acacaagaac aagcttcaat atgcagatca gcaacctttc tggttcagtg cacgatgtct 540
gagatgacaa                                                        550
```

<210> 1473
<211> 550
<212> DNA
<213> Ctenocephalides felis

<400> 1473

```
aaaattttaa ttttaattat tatgtgttcc tttatcattg atgcgagaag taaaagtttc 60
gaaataaagt taagacggtg gaaaagtcct cgacttcaat tgattgaaat ggatcgaaat 120
atgcgtatag cggtttggaa agatattcaa gaaaacgaaa gctattattc ggatccatct 180
aaacctaaac ctcctccagg tcctaaggac aatgatacta tagcaattta taaattttta 240
gatactgaat tttatgctga ggttggaata ggccatcctg taaagtattt caaacttgtg 300
gttgacactg catgggcaga aacatgggtg gcctcgaaac aatgtgggtt aaagtgtgtt 360
ggatgttgga atcttaataa atatgactct ttggcatcat caacatttca agaaaacggt 420
aaagaatttt cttttggctc agcaaagaac cataacaggg tcttctcaat agaaagtttt 480
atattgccac ataaatgtta aaatcagact ttggggaagt acatgtttgc cttgcactct 540
gtttcaaaca                                                        550
```

<210> 1474
<211> 550
<212> DNA
<213> Ctenocephalides felis

<400> 1474

```
ccntaccgcc gatttgttcg aaagaagaaa catcgtccaa gtcacacttt gtttatatgc 60
tttgggcaga cttacacaaa aacatcccga atacacagga ccatcattag gccccaaaat 120
ggcagaaaag aatgaaagga cgtttactga agagcaactt agagcccatg aaggagaatt 180
gaacttgcaa atgggataca acaaaggtgc atcccaatct ggccatggtg gatttggtaa 240
cactaggcat atgtaatttg ctgattttat acgtatttct gaaaatattc ctcacaagtc 300
tgacgacgat acagattagt agattcgagc acactttgtt caatgttatt ctgatacaga 360
ctcaaaatta acacaaacta acagaataaa cacgacattc acaaacactc ataactaata 420
gatatagaca caacactaat tttaacacaa tccaatacat aatgacatat aaaaataaat 480
taacatctct cgcattaata cgatacaaaa atttaattac taacaaacat aatttacata 540
ttgaaatctt                                                        550
```

<210> 1475
<211> 542
<212> DNA
<213> Ctenocephalides felis

<400> 1475

```
agctcacaga attcagggct agctttaacc acttcgacaa gaaccgcaca ggacgtttgg 60
cgcctgatga gttcaagtca tgcctggttt ccctcggcta ctcgataggc aaggatcgtc 120
aaggcgattt ggacttccaa aggattttgg cagtagtcga tcccaatgga actggatacg 180
tccacttcga tgcattctta gatttcatga cccgcgaaag tacagacacc gacactgccg 240
agcaagtcat cgacagcttc agaatcttgg cctccgacaa gccatacatt ctgcccgatg 300
agttacgcag agaattgcca ccagaccagg ccgaatactg cattcaacgt atgccacctt 360
acaagggacc caatggtgta cccggtgctc tcgattacat gtccttcagc acagccttgt 420
acggtgaaag cgatttgtaa ggtagaataa attttcataa acatttaagc aaacatttga 480
caagtacaca taaaggtaaa taatgaagtt atgagagtta cgatcaaaat tagcattgng 540
ct                                                                542
```

<210> 1476
<211> 542
<212> DNA
<213> Ctenocephalides felis

<400> 1476

```
gtttatcggt gtcttgggtc atgggaccag ngnggcatca ttaatcatga atttaaatag 60
tgtcgttact aatttctcag gtcttgtgac gagtccgatg ttgaaaaagt attccgttcg 120
gaaagtggcg gtggccggat cgctcctgac cgccacgggt ctcatgatca gcagtcaggc 180
acgctccctg tggctgattc tgtttggata cagtttttttg acaggtctag gtttgggttt 240
tataatgcct tcggtgttct tggcagtgac ctcttatttt aaggtgctcc gaggccgggc 300
ggtgggcctg gcagcagccg gtaccggcct gggacaaatg gtaatgcctc atgccgntcg 360
agcactgttg gacgaatata gtttcanagg agccactctt attatggccg ctatggcttt 420
acaaggggtg gtaggcgctt ctttattcaa cccgtaaaga atacatgaac cngtcnatga 480
cgnccgaccg agaaaaaatn tttactngac cngatttacc cctgacgctc agacctgaag 540
aa                                                                542
```

<210> 1477
<211> 542
<212> DNA
<213> Ctenocephalides felis

<400> 1477

```
ctccattctt tcttaggttt cattctttta agctgaccat cccaaaccaa atcaaccaac 60
```

cctccttgtt tggctatcac tgctttcaca cgatttgcaa agtctattga actctctttt 120
tcttctctaa acattggagg taagtaccaa acatcacata caatcgccca tgaagacatc 180
atcatgtata aataatgcat cattgaatac tttgaactgt tccaaaaggc atctccaaac 240
tttggatcat atttgatggc tacaggataa attactcctc cgacttcaaa tgagcctttt 300
ttaaactgca ttaccgatgt attattgata catgttcctt ctggaaatat taatattggt 360
ggattattag gatcagagat atgctgccga agtctgtagt gacggcagtt cgatctttga 420
cttcagagcg ttcaaaccaa atgtgaggtg atgccctagc cagcgccctt tgcagtatac 480
ctaggaaacc atcatgtctc tgccaattag agaatagcaa ttatcacaca tgagaacaag 540
ag 542

<210> 1478
<211> 542
<212> DNA
<213> Ctenocephalides felis

<400> 1478

cacnaaggca aaagtttgta catttccaaa gngatggatt ccaaaccctt tgatcccgtg 60
gaaaatgggc tcgataaaga cttcaggctt actaaattta ctgaattagg agggtgaggt 120
tgtaaggtcc ctcaagatgt tttaaacaac ttacttgagg gactctgcga tatggagaag 180
gccaagtcca gtgatagtaa ggaagttgga attggattgg actgctccgt tactcctctc 240
gaagaaaact ggtacctttt acaaacaaca gattttttct atcctttaat tgaagatcca 300
tatttaatgg gtcgaattgc ttgtgcaaat gtggtcagcg atttgtatgc aatgggtgtt 360
accaaaatta caaacatgat gatgttatta ggcattagta ataaaatgac tccgaaggaa 420
cgagattgtg ttattccttt aatgatgaaa ggatttaaag atggtgctaa agaatcagat 480
acaactatcc aggtggacaa acagtcttga acccttgtgt attattggtg ggtcncacat 540
ca 542

<210> 1479
<211> 542
<212> DNA
<213> Ctenocephalides felis

<400> 1479

gntatggaaa aagccgaaaa gctgggccat cgcgttcaaa aatgtgtggt tgtcgctcat 60
ctgaaacgtg tcactcctgg aaccagcgag gagatcaatg gagtcgagac tcctatgaca 120
gatggccgcg accattggtg gcatgaggaa atggacgaag ttgaaccagc atgttaccca 180
gaatggatgg ctgctgagga tccattgttc atgctttata caagcggctc cactggtaaa 240
ccgaaagggg ttctacatac tacaggtggt tatctattgt atgctgccac cacatttaaa 300
atggtttttg actacaagcc cgatgacatc tactggtgca ctgctgatgt aggatggatt 360
actggacatt cgtatgttgt ctatggacca ttagctaatg gcgccacctc cgttatgttt 420
gaagggacgc cattttatcc ggataacgat agatattggg cggtattgaa aaatacaaag 480
tgacccagtt ttacacagca ccaaccgcat aagacttgat gaagttcggg aagaccagtc 540
aa 542

<210> 1480
<211> 542
<212> DNA
<213> Ctenocephalides felis

<400> 1480

```
ttgagaaact tctcaaaatt gataaaaaag ntgctgtagt aatggccatg gatatgttgt 60
tagcaggagt tgatactaca tcaaatagta cagcattttt gctgtatttc ctggctacga 120
atcaaaaatg ccaagataaa ctacgtgaag aaatcagaac aattctgcca aataaggatt 180
cacatgtaaa taacgaatca tttcaccatt tgccgtattt gagagcttgt atgaaagagt 240
cgagtagaat attgccaata attggaggca ctgttaggaa agtaccagtc gatattgttt 300
tatcaggata tcaaattccg aaggggaccg aggtggttct aagtcatctt actacatcaa 360
tgaaaagcag tcaattccct gaacctgaaa aattcatgcc agaacgatgg ctgagcagcc 420
aggaatcaga aggatgtcct ttagcaaaaa atgctcatcc attttcgcac atgccttcgg 480
tttgggcctc gcactgcgtg ggaaaagatt gcagatttgg aaatggaact ttatatgaag 540
ta                                                                542
```

<210> 1481
<211> 539
<212> DNA
<213> Ctenocephalides felis

<400> 1481

```
taaaaatttt aatngcttat tcttcaggng ttcatataag aatagtatta ggaggaattt 60
ttactataaa tatattaggt attgtaggta ggtttatttt aataatttct catggattat 120
gttcttctgg gatattttgt ctttctaata ttatttatga acgatcagga agacgaagaa 180
ttttaattaa taagggttta ataagattta taccttcaat aacattattt tgatttttat 240
tatgtagatc taatatagca gctcctcctt cattaaattt attaggtgaa attatattga 300
ttaattctat aataagttga tcaataatat taatagtatt aattataata atttcattta 360
taagagcaag gtatagacta tatttatttg cttatagtca acatggaata agaagaataa 420
gactatattc ttgtcttctg gaagggttcg agaatattta ttattatttt tacattgatt 480
ccattaaatt attaatttta aaaagagatt attataatta tttataaaaa aaaaaaaaa  539
```

<210> 1482
<211> 540
<212> DNA
<213> Ctenocephalides felis

<400> 1482

```
ctaatcgaag tgatcgtaaa atattaaatn gacagctatt tgttaaaaca attgtgcatt 60
atagaaaaac atttaagtga aaattaatta cgtttcagca gaaatacatc ttctttgtaa 120
atttggggtt aataatcttt caaaatgcag atctttgtga aaactctcac agggaaaacc 180
attactttgg aggtagaacc ttcagatacc attgaaaacg ttaaagctaa gattcaagat 240
aaagaaggaa tcccaccaga tcagcaacgt ttgatttttg ctggtaagca attggaagac 300
ggcagaactt tgtctgacta caatatccaa aaggaatcaa cattgcattt agtattacgt 360
cttcgtggag gtatgcaaat ctttgtaaaa acattgactg gaaaaactat tacattagag 420
gttgagccct ctgatccatt gagaatgtaa aagctaaaat ccaagataaa gaaggaattc 480
cccagatcag cagcgtttgt ctttgctggc aaacaattag aagatggaag acttgtctga 540
```

<210> 1483
<211> 542

<212> DNA
<213> Ctenocephalides felis

<400> 1483

```
gtnacattga tcggatctgt tagcaaaggc tnccaaantg ccgaaatcga tgaatgttcg 60
tgtgaccacg atggacgcgg aactggagtt cgcgatccag cagaccacca ctggaaaaca 120
gcttttcgat caggtggtca agacgatcgg tctgcgcgaa gtctggtttt ttggactcca 180
atatactgac agcaagggtg atttgacgtg gattaagctt tacaaaaagg tcatgaatca 240
agatgtcaaa aaggaaaatc cacttcaatt caaattcaga gcgaaatttt atcctgaaga 300
tgtagctgaa gaacttatac aggacattac atctcgtctt ttttatcttc aggttaagaa 360
cgctatactg tctgatgaaa tatattgccc tcctgagaca tcggttcttc tagcatctta 420
tgctgtcaag cccgacatgg agattttcaa aaagaccaac actcttctgg attttggcga 480
acgatagact gtaccacaaa gggtaatgga tcaacacaaa atgtctaaag aagaatggga 540
ac                                                                542
```

<210> 1484
<211> 542
<212> DNA
<213> Ctenocephalides felis

<400> 1484

```
cagacactga caaaggcaaa aatgggtcgt cgaccggcca gatgttatcg ctattgcaaa 60
aacaagccct accccaaatc tcggttctgt cgtggtgtgc cagacgctaa aattcgtatc 120
ttcgatttgg gtaagaagaa ggcaggcgta gaagattttc cactatgtgt gcatcttgta 180
tctgatgaat atgaacaatt gagttctgag gcactggaag caggacgtat ttgctgtaac 240
aaatacctcg ttaagaattg tggtaaagat caattccaca tcagaatgag gctgcatcct 300
ttccatgtta tccgcatcaa taaaatgtta tcgtgtgctg gagctgatag gctccaaact 360
ggaatgcgtg gtgcttttgg aaaaccacaa ggtactgttg ccagagttca catcggtcaa 420
ccaatcatgt ctgtcgttcc agtgacagat acaaggccgc tgttgtagag gctctgcgtc 480
gtgctaagtt caagttccct gcagacaaaa gatctatgtt ccaagaaatg gggattcact 540
aa                                                                542
```

<210> 1485
<211> 542
<212> DNA
<213> Ctenocephalides felis

<400> 1485

```
cacaagttct attcaaccag atagaatctt acttatggat acgtttttcc aaattttgat 60
cttccatgga gagactatag ctcaatggag agctttgcga tatcaagata tgcctgagta 120
tgaaaatttc aaacaacttt tgcaagctcc agtagatgat gctcaggaaa ttttattgac 180
```

acgttttcca atgcctcgtt atattgatac cgagcaaggt ggatcacagg ctcgattttt 240
attatccaaa gtcaatccct cgcagacaca taacaatatg tatgcttatg gaggagacgg 300
aggagcacca gtattgactg atgatgtgtc tcttcaagta tttatggagc atctaaagaa 360
attagcagta tcttcaaatt cataaaatta tataaagaca gaaatagata tacaaatctt 420
attttatctg taatattgtg tgtcagtctt tatatttgca atatagataa gtaataaatt 480
caattgatta tgaacctaaa tacattgata tattaaaaga acatttacac taaaaaaaaa 540
aa 542

<210> 1486
<211> 540
<212> DNA
<213> Ctenocephalides felis

<400> 1486

acagttaaag tgtattcaaa tcaaataatt ccataaaact attctagaaa attatccaac 60
acagttagat aagatgaagt ctttggtgct cttggccgtt ttggctgtag gttttgcaaa 120
cgctgaatat tgttacgatg aaagtgttac tgcttgttca tcgactgcca ataaggagga 180
tctcccccat tgtaacgctc tctactccgg tttccacact gtggctgccg atttatcatc 240
atacgttaaa agagaagtcc tatactccta cgactacttg ctgatgtcca cacactttgg 300
aaactatgaa aagaatcgtg ttggctttga aaaactcttc aagggcctct ccgacaagtc 360
ttgggaaaac gcaatcaatg tcatcaaata catcaccaag cgtgggggca ctgtcgactt 420
ccagactact cacaacgtca acactacccg tgtagccgaa acatcagaat tggaaagttt 480
ggctagggct ttggagaatg agaaagtgct cgctaaggat gccaggctat tcacaaacgt 540

<210> 1487
<211> 542
<212> DNA
<213> Ctenocephalides felis

<400> 1487

caggagaaag cttcagttcg tgcacactaa atgtgttagt accaggcgag aagaaaaaga 60
caccagcttt cagcactttc ccagagtccg ccagcgtgca agagggcgag agtgcatcgt 120
tcttagttcg cacagaagac gaagttcttg gacttcaatg gataaaagat ggtaaaccta 180
ttgacgagaa gagctctcgc tatcgattca caatggaggg caaaacgacc ttccgattag 240
agatagtatc ctgcgccagc atagacgtgg gccaatacca agccaaagcc atcggtaaga 300
caggagaaac attcgctgcg ttctcagtga atgtcgcagc cgagcattga gccgaacaac 360
taaattaata accgatcctg cacacggctt aggccaaaaa acgagacgac tcgcgttgaa 420
tcgcgagata tcgtgtaaac ttaagtttat taaattatta tgtttatatg aataaattat 480
tagtgtgatc taagcgtgtt ttcattataa ttcgccttgc cacttcgctt atatatangc 540
cg 542

<210> 1488
<211> 542
<212> DNA
<213> Ctenocephalides felis

<400> 1488

atcagctacg cagagccact tccatttcac tcctggacga gtcctttgtt gtgtccggtg 60
aacgtagaga tcagggcgtg gtagtggcct tgaaggatgg ttttggcttt cttcgttgtg 120
cagaaaggga gccaagactg tttttccact ttaccgaggt tttggatgtg acgagagaga 180
tctccatggg agacgaagtg gaattcacag cagtacaaga tcctaatagc tcttttgcta 240
attttagaca cagcgctata aggattaagc atttacctcc gggtactgta aaattcgaaa 300
ccctaatcga atcaaatcta acgggagtcg tgactaggga agcctctcca agaagcccta 360
gcaaatctca aaacggtggt ccaacacaaa acggcgggcc tccagttcca gaagggggca 420
tgatttctta tcaaagcaac ggtcaaaaga aatcagttcc tttctttgca aaagattgtg 480
atcggcaacc tagaatgggt gataaagtga tttcaacatt agtcaggtta agcgtaataa 540
ag 542

<210> 1489
<211> 542
<212> DNA
<213> Ctenocephalides felis

<400> 1489

gttttggtca gcctgaaatt gcaattggaa caatccctgg agctggtggc acccaacgtt 60
tgaccagatc tgttggcaaa tctaaggcta tggaaatatg tcttactggc aacatggtca 120
cagctgaaga ggccaaaaaa atgggcttgg taagcaaggt attccctgct gataaattgg 180
tcgacgaaac tgtaaaatta gcagacaaaa tttcatcaca ttctccactt attgtctcat 240
tgtgcaaaga agctgttaac actgctttgg aaacttcttt gcaagaaggg ttacactttg 300
agaagagagc attccatgct acatttgcca ctaaagacag attggaaggt atgactgctt 360
tcgtagagaa acgagcacct aactttaaga atgaataaaa agactactcg aattttataa 420
ctctatatgt atatatttta cattaccttt gtaataggta atatgaataa cctctttatt 480
tacatgcatt tgatatactt aatgatattt gatatattcc ttatatgatg attccattat 540
tg 542

<210> 1490
<211> 542
<212> DNA
<213> Ctenocephalides felis

<400> 1490

cgaaaccata aaaaaatacg gcaaactcaa tgncctcatc aacaatgccg gcatgttgga 60
aacaggcacc atcgagacaa ccagtctaga ccagtacgac agaatcatga acgttaacat 120
gagatctgtg taccatttga caatgttagc agtgcctcat ttgattgaga cgaagggaaa 180
tattgtgaat gtctcgagtg ttaatggtat acgttctttt cctggtgttc tggcttataa 240
tatatccaaa gcggctttgg atcagtttac aagatgcgtt gctttggaac ttgctcttaa 300
gcaagttcgt gttaattctg ttaatccggg ggtcattatt actgagatac ataaacgggg 360
gggaatggat gatgaaactt ataaaaaatt cttagaacat tgcaaaacta ctcacgcctt 420
aggacgtcca ggacaagtaa gtgaagtatc caatgccatt gcgtttcttg caagcgagca 480
cagcagcttt attactggtg cttcgttacc ggtgatggag gcagacatgc ctgtgcccac 540
ga 542

<210> 1491
<211> 542
<212> DNA
<213> Ctenocephalides felis

<400> 1491

```
gnnaattatt ttattaagcg cattatttgc gngtgtnatt tgctccttta acgcggaagt 60
acaaaatcga atcgttggtg gcaatgatgt aagtatttca aaaattgggt ggcaagtatc 120
tattcaaagt aataaccaac atttctgtgg tggttcaatc attgctaaag attgggtact 180
gacttcttct caatgcgtcg tggacaaaca aagtccaccg aaggatttaa ctgttcgtgt 240
tggaactagc actcacaatg atggaggaaa agtgtatgat gttattgaaa ttataaaaca 300
tccgaaatat aataaagcag tgccagatga ttttgatgtt gcacttttac ggatcaaaga 360
gccaatatca tttactccat gcacagtaac tcctgtaaaa ttaatacaat cgggaaaaga 420
agtccgaagg gaacaacttt gagtgtaact ggatggggcc cacgaangaa tgggggccaa 480
tttcgcaaag ttcaagaagt taaagttaaa gcttactcaa gtcaagaatg cangaacagc 540
at                                                                542
```

<210> 1492
<211> 542
<212> DNA
<213> Ctenocephalides felis

<400> 1492

```
aaaaaaacaa gctttttaa cgaaatattt ngcaaaaaga gaaatatata aacaaactta 60
atgtctgaaa caatataaaa taatattttc aacgctaacg cttaaataat tccaaaacaa 120
atcatgagtg actattttct tttatgttaa cctctttacg caaagaaaat gtttataaac 180
ttcaaacaac gttaaggtga taaatgttta cctaataata ataataataa gtttattttg 240
attttgtgta taaaaacatg tcttggaggc tgctgttgaa gttttaattt gcaagtgtta 300
gatacaactt aataaaaaat gtctcaatga tgacaataac aaaaaattct tttacccaag 360
tgcctcttgt atttataata tgatatacaa aatgcccccg tttagacgta aaaaatctgg 420
aaagtctttt ccagttaaag tttgcacttt ggacgctgag ttagaattta atttggagtg 480
gaaagcgaca ggcaaagatt tattcgaatt agtttgccgg acgattggtt acgagagacc 540
tg                                                                542
```

<210> 1493
<211> 542
<212> DNA
<213> Ctenocephalides felis

<400> 1493

```
agttcatctt atgttgtaat tatattatca gnttgttgta aacacttctt attttatatc 60
ataaatatcg ctgtatttga tttacacgat ggctgcaaga ttgaattcat tgtttaaaac 120
agatttcaca aattacataa aacgtttaaa cagcgttcaa atacgttgct taaatttgca 180
agaatatcaa agtaaaactc tacttcaaaa aagtggcgtt gccgttcaag cctttagatt 240

attagataac accgaaaaca caagtgtctt aaatgatttc aaagtccccg aatatgttat 300
caaggcccaa gttttagcag gaggtagagg caaaggccat tttgataatg gctttaaagg 360
tggtgttcat ataacaaagg accctaaaga aatattgcca attgctaaaa atatgatggg 420
tcacagactt attacaaaac aaacaaaagc tgaaggtata cttgtcaaaa agttatggtg 480
cacaaagcgt tgacatttgc gggaaacata ttatgtatca tcatggcaga gctcatatgg 540
cc                                                                542
```

<210> 1494
<211> 542
<212> DNA
<213> Ctenocephalides felis

<400> 1494

```
gtncgattga tttggtgcta attcgattca gntttgcatc agtcacagtc aaagtcggta 60
ataaaatatg gttactaaag cagtttgcgt tttaaacggg gaagttaagg gaaccattta 120
cttcgatcaa agcggtccag aagcacctgt cacactaaca ggatgcgtta gtggtttaag 180
caagggtgat cacggtttcc acatccacga attcggtgac agcacaaatg gatgtatttc 240
agctgggcca cattttaatc cccacggtaa agaccatgga ggacctgatt ctgctatcag 300
acatgtcggc gacttgggaa atcttgtagc tgatgccgat ggaaacgcta aagtgaaaat 360
aaccgacagt caaatttcct tacaaggtcc tatgagcgtt ataggcagaa cattggttgt 420
acatgctgat cccgatgatc ttggattagg tggtcatgaa cttagcaaga ccactggtaa 480
tgctggagct cgattggctt gtggtgtatt ggaatctgca aaccttaatt taaaattgta 540
tg                                                                542
```

<210> 1495
<211> 542
<212> DNA
<213> Ctenocephalides felis

<400> 1495

```
attcggtgcc agtgttccag aatcatggaa aagattagat ggtagaatcg taggaggaca 60
cgataccagc atcgataaac accctcatca agtatcttta ttgtactcca gccacaattg 120
tggtggttcc ttgattgcca aaaactgggt tttgactgca gctcattgca ttggagttaa 180
caaatacaat gtccgtgtag gaagttccat cgtaaacagc ggtggtatct tgcataaagt 240
taaaaaccat tacagacatc caaaatacaa cgcagctgct attgactttg attacgcact 300
cttagaactc gaaactcctg ttcaactcac aaatgatgtg tccatcataa aattggtcga 360
tgaaggagta gatcttaaac ctggtacctt gttaactgtt actggatggg gatcaactgg 420
aaatggacct tcaaccaatg ttttgcaaga agttcaagta ccacatgtcg accaaaccac 480
ttgctccaaa tcttacccag gaagtttgac tgatcgatgt tctgcgctgg ttatttggga 540
ca                                                                542
```

<210> 1496
<211> 542
<212> DNA
<213> Ctenocephalides felis

<400> 1496

```
gttnccttgt ctgcagctat tcctcactcc agcagagtcg ttggaggact ggaagctgca 60
gagggttctg caccttatca agtatccttg caagttggca acttccactt ctgtggtggt 120
tcaattctga acgaatattg ggttttgact gctgctcact gtttgggtta tgacttcgac 180
gtggtagttg gaacaaacaa acttgatcaa ccaggtgaaa gatacctcgt agaacaaact 240
tttgttcacc aattcgacca ggaatcttta agacacgatc ttgctttggn gaaagtgcca 300
gccctatcng aattcaatga ttatgttcaa ccaattccat ttgggcgaaa cttatgttgg 360
aggcggtgaa ntgctcgctt actggatggg ggaagactgg aactacttga atggaccaat 420
gaactccaag acttacactg tacataaanc acaacnatgt gtaagnaaca attntccagt 480
tacncagcac tttgannntt ggtgcagnga cnagccctnc accgtgactn tggtgncctt 540
gg                                                                542
```

<210> 1497
<211> 542
<212> DNA
<213> Ctenocephalides felis

<400> 1497

```
gcccgagtta gacgcgtttg tgaaatctag agttttttat ttagtaaatt cgtgcttcgt 60
gttgaatata ttcgtgatgt ctggacgacg atcgggtact ggaagaactg gcccggccta 120
taactcccga gcgagtactg gggtcctaa taggcactac aacgatggca atcgttataa 180
taatactagg agtaactcaa actacaacca gcaggaacag caacaacaga acaatcagca 240
gcaatatcca cttgctcaag cacaagctcg caatcaaaat acatacaaca aaccggttga 300
agtaaaagaa gaaataaaac cggaagtaat gccatcacct caaccacaac gtcaagctcc 360
aactccagtt ccagcgccag ctcctgttgc tacaactccc accaaagtaa aggcagatga 420
accccccgcg caagagcccg ttcagaaacc agaagcaatg caagaagatg gtggagatgg 480
tgataacgat aaaaagcctg gaagaaaaac aaattgataa gtgaaaaact agcaacagcg 540
aa                                                                542
```

<210> 1498
<211> 542
<212> DNA
<213> Ctenocephalides felis

<400> 1498

```
gnntttgcaa gctttgaaga gtgttatacg agaccagttg ataaaatatg attttcagta 60
ttatcatgtt gattttcaat gtgatattcc tgtattagta tttagcgagg gcaaaagtat 120
tctggatatt aaaaaccaaa ttcctttgga gatagatgaa aacatcaggg acaactttga 180
aaatatttta ccagctgtta aacagttttt aagtaatgaa actatggatc tcattagatg 240
ttatttaaca atcatgaaat actcagaatt tgaagttaat caggaattgc atgagattat 300
agaaaatgat tttgtgaact tactgcaaga accaggaatg actccagaag atctacactc 360
atatttaaca ctggccagat tgtatagttt atcccgaggt ctaaggcatt taacaaaaga 420
ctcctgcaag cagttaaaga tcttgagact aaaagaagat ctagaatcaa agccccaaac 480
acactgcgaa atgtgaattg atataaagtn tttcattctt gngatatatt aacttgtttt 540
ga                                                                542
```

<210> 1499
<211> 542
<212> DNA
<213> Ctenocephalides felis

<400> 1499

```
atnacttcaa gttttgtctg caatatataa aaatttaaga tggctaaagc accagcagtt 60
ggtatagatt tgggtactac gtactcctgc gtgggtgttt tccaacacgg aaaagtagaa 120
attattgcaa atgaccaagg aaacaggact actccttcat atgtcgcgtt taccgataca 180
gagcgtctca tcggagacgc cgccaagaat caagtggcca tgaaccccaa taacacaatt 240
tttgatgcca aacgtcttat tggcgtaaa ttcgaggacc aaacagtcca agctgatatg 300
aaacattggc ccttcgaggt tgtcagcgat ggaggtaaac caaaaattag agtatcgtac 360
aaaggagaat ccaaaacctt cttccctgaa gaagtcagtt ccatggtgtt gactaaaatg 420
aaggaaaccg ctgaagctta cttaggcaaa actgtgacca atgctgtcgt tactgncctg 480
ctacttcaat gactcacacg tcaagccacc aaggattcgg gactatctcg gtctaaatgt 540
gt                                                                542
```

<210> 1500
<211> 542
<212> DNA
<213> Ctenocephalides felis

<400> 1500

```
ccaatttata taaaagaatt taaaaaaata taaacatgaa gccgctgata cttcaagggc 60
acgaacgttc cataacccaa atcaaataca acagagaagg cgatttatta ttttcggctt 120
ctaaggatca caaacctaat gtttggttct ccttgaacgg tgaaagactc ggtactttta 180
atggccataa tggtgtggtt tggtgtatag atgtggattg gcaaagtact agatttatgt 240
caggaagtgg tgaccggtct ctgaaattgt gggatttgga actaggcaaa gaaattggtt 300
caatacctgc ccaagcatct gtgaggactt gcaatttctc attttcgggc aatcaagcag 360
catattcaac cgatagtagt aaatcctcat cttgtgaatt atatattatt gatgtgcgaa 420
atgcagatag cagtatgtcc aagctgatcc tattttaagg ataccaattc cagaatctaa 480
agttacagca atgttatggg gtctttagat gaaacagtat taacggacat gaaaatgggt 540
ca                                                                542
```

<210> 1501
<211> 542
<212> DNA
<213> Ctenocephalides felis

<400> 1501

```
gagaaaagag tggaagaatt taaattaaag aaaatgtgga aaagtcccaa tggaactatc 60
agaaatattc tcggtggcac agtcttcaga gaagcaatca tctgcaagaa catacccgga 120
ttggtaacag gatggaatga gcccatagtc atcggcagac acgctcatgc tgatcaatac 180
aaagccaccg acttcgtggt accaggaaag ggtaaattag aattgacatt tacccccgaa 240

tcgggaagtc caatgagctt caccgtccac acttaccaag gacctggagt cgccatggga 300
atgttcaaca ccgatagttc aattgtagat ttcgctaatg cctcattcca atatgcctta 360
aaccgaaaat tgcccttgta cctatctacc aaaaatacca ttctcaagaa atacgatggt 420
agatttaagg atatattcca ggaaatttat gacaagcaat acaagaaaga atatgaagct 480
gtggaatctg gtacgaacat cgactgatcg atgacatggt cgctacgcaa tgaaatcatc 540
tg                                                                542
```

<210> 1502
<211> 537
<212> DNA
<213> Ctenocephalides felis

<400> 1502

```
tttttttttt tttaaatatt agtgtaactt ttaataaaat tatttactca atatcttaaa 60
attgtttgtg atggcacatg ttttataact gttttgaaaa tttcgagtca cttcttaaag 120
ttaaaattta attaaagtca gtgtagtggt aataaataaa ttttggcatc aaattaagta 180
ttggatatca tggcgaataa taatgtgata aattttgggg caggacccgc caaactccca 240
gaagaggtga tgcttgaagt gcaagaacaa ttggtgcatt atggtgaaac aaaaatcagt 300
gtaatggaaa tgagccatcg atcaaaagac tacatgaaaa tcaacgatga tacacaaaat 360
gcagttaaag aattattcaa ataccggaca attcaaaata ctatttctgc aaggaggtgg 420
cacaggatgt ttgcagccat accactgaat attttgaaga ctggagttgc agattatgtt 480
gttacaggtt cttggtctgc aaaagctgca aagaagcacc aaattggnaa gtaacat    537
```

<210> 1503
<211> 542
<212> DNA
<213> Ctenocephalides felis

<400> 1503

```
cnttaatata ctatgaatgg tggagaagat tgngctcang atgtgaaaga gcaagaacaa 60
gttaacggtg gcggtgaaga aggagccggc gatgcgtctc gtgaaaatgg aagcgccgaa 120
gctcctggcc gtgacgacga taggaaactc tttgttggtg gacttagttg ggaaacaact 180
gataaggaat tgcgtgaaca ctttggtgca tatggagata tcgatagcat taatgttaaa 240
actgacccca gtactggtcg atcacgagga tttgcattca tcgtgtattc atctcctgaa 300
tccattgata aagttgttgc tgtatctgaa catataatta acaacaagaa agtagatcca 360
aagaaagcca aggcccgcca tggaaaaatt tttgttggag gtttgacgac tgaagtcagc 420
gatgatgata ttaaaaacta tttcaatcag tttgggacga tcatagaagt tgagatgcca 480
tttgacaaga cgaagaatca acggaaaggt ttctgtttat acttttgaat ctgagcaagt 540
tg                                                                542
```

<210> 1504
<211> 542
<212> DNA
<213> Ctenocephalides felis

<400> 1504

cgcagtcacc ctaacccttc tagtcatatg cgttttctac acctctttcg gaggggatgag 60
ggccgtcgtc tggacggaca cgcttcagtc catcgtcacc tgcggcgcta tgttcgccgt 120
cgtctggata ggagtagcag acgtcggagg aatagcagaa gtcttcagga gggctgacga 180
aggaggcagg atcatatttt tcaatatgaa tccaagtatt taccaacgaa catcattctg 240
gagtgtcagt cttggactga caaccatgtg gttgtctaat cttggtgtca gtcaaagttg 300
tatacaaagg ttcctgtctg tgccaacttt aaaggacgct agatggtcca ttatctactt 360
tacgataggt ttagtcttaa caaaatcaat atcctgcttc accggactat tgatgtatgc 420
tcattacaaa gactgtgatc ccttaagtac tggtgatgtc aaaaaagcag atcagatgtt 480
gcctattatg tccacgacgt ggcaggtata taccaggact ataagtttat tcgtactgng 540
tc 542

<210> 1505
<211> 542
<212> DNA
<213> Ctenocephalides felis

<400> 1505

ctaataaaga gatggctgga aaacgacgag ttataatcga cgtagatgcc ggttcggacg 60
atgccatagc tttgctcatg ctgatagcag cgcacaaacg gggtgatgtg gaactaatgg 120
gtatcacttg tgtggcgggt aatacaaatg tggataatgt tgcgataaat gttctacgtg 180
ttctgggtgc tgtaaaggct ttagatatcc ccatttacaa aggtgcatcg gaaggtctga 240
ttcctctaga tattccaaat tccacagaat ctgagttcca tggtggtgat ggatttggtg 300
atttagagca ctatggaaat gatcctgatt tgagtttaat caaaccagag catgcagtaa 360
attacctaat ttctgcagct aaacagtatg aaaatgaaat tacttttata tttgtgggcc 420
tctgacaaat gcagcacttg caattaaaat gtatccaggt tttctagaca aaactaagga 480
tgtgtantaa tgggtggcaa ctataaagcg tgggtaataa aacaagaccg cagaattaat 540
tt 542

<210> 1506
<211> 542
<212> DNA
<213> Ctenocephalides felis

<400> 1506

aaaatactag ttttattgcc attaatattt gaaaaatatc attgagaaaa tgtcaatatt 60
attatgcgcg ctatttttttg cttcgacgct tagcaatgaa actttgtcga aacaccatca 120
agttaagaat cgtcgatttt acatagatta tgataaaaat acattcatga tggatgacaa 180
accatttcga tacatagcag gttcattaca ttattttcgg gtacatccgc aacaatggaa 240
agaccgtttg gaaaagctaa aagcagcagg tttaaatgcc gttgatacat atgttgagtg 300
gtcacttcac aattttgatg aaggaaaata ttggtggggc agcaacgccg atttggagca 360
atttattaaa actgcacaag aagtaggatt gtatgtaatt ttgagaccag ggccatacat 420
ttgcgctgaa cgtgattttg gaggccaacc ttattggctc ttatccaccc agaaaaagat 480
attaaaccaa ggacactgtt tcgtatatgg aagcagcacg agatggatac aagaatatta 540
aa 542

<210> 1507
<211> 550
<212> DNA

<213> Ctenocephalides felis

<400> 1507

```
cttgaccgcc accgagtctg aagtggctgc actcaacagg aaggtgcaac aaattgaaga 60
agatttggaa aaatctgaag aacgtgccgg aagcgctacc accaaattgc ttgaagccac 120
acaagctgcc gatgaaaaca accgtatgtg caaagtattg gagaacccgc tcacaacaag 180
atgaagagcg aatggaccaa ttgaccaacc aattgaagga agcacgtctc ttggctgaag 240
atgctgatgg taaatctgat gaagtatcac gcaagttggc cttcgttgaa gatgaacttg 300
aagtagctga agaccgtgtt aagggaggtg acagcaagat catggagttg gaagaagaat 360
tgaaggttgt aggaaactcc ttgaaatctt tagaagtatc cgaagaaaag gccaaccaaa 420
gagtagaaga attcaaacgc caattgaaga ctttgccgtc aaacacaagg aaccgaactc 480
gtgccgagtt cgccgaaaag accgtcaaga actgaaaagg agtcgcaggc tgaagacnat 540
tggcataaca                                                        550
```

<210> 1508
<211> 548
<212> DNA
<213> Ctenocephalides felis

<400> 1508

```
aacatcccat aataaaagca ttgtacagcc tagtggtaat gatcatattc gaggggtaa 60
tgttgctcca cctagaatgc aagatgttgt ccgtccaaaa agatattctt gtcaaagacc 120
tggtggtatt gtaccagaaa caaacatgca aggccaaccg cagcaacaac aacctgtata 180
tcaacagaac tactatgcaa ctgaatatac tccgcctgta gcaaatgaac aaaataattc 240
acatcaagga caacatatac cacaagcact aaatgggatg ccccaaccag gggtcaggt 300
tgtacctcct aatatttctg tgccaccccc gcaaacaatg ccttatgtac cagaaccagt 360
tcctacacag gtcataacca ctagtaatca agtgatgccg caacaagttc ttgccctcaa 420
tatcctcaag ttctatgcag tttaattcgg gaggacccta catggctcaa ataatacttt 480
tatccacaat ctttaggtat ctgtcctctc taacatgcac cccacacaat atctcccaat 540
ccctcata                                                          548
```

<210> 1509
<211> 405
<212> DNA
<213> Ctenocephalides felis

<400> 1509

```
gattattcaa aagaaagaca atttagagga ttgcaaagta aaattgaagg cgagcataaa 60
ttttcagtta ttagacaagg agaagtgaaa cagatatcgg ttggtgatat tgtcgtcggc 120
gatatttgtc aaatcaaata tggagatctt ctaccagcgg acggtttatt gatccagagt 180
aatgatctga aggtggacga atcctccctg acgggagagt ccgaccatgt gaagaaaggc 240
gagtccttcg acccgatggt cctgtcgggg acgcacgtca tggagggcag cggcaaaatg 300
ctggtgacgg ccgtcggcgt caactcgcag gccggcatca tcttcacact gctgggtgct 360
gcagtcgacc aacaggaaca agagatcaaa aaaaaaaaaa aaaaa                 405
```

<210> 1510

<211> 482
<212> DNA
<213> Ctenocephalides felis

<400> 1510

```
caagctgtca aagtctggca aatatatttt aatttaataa tttttgtctg ttttaaataa 60
aaaatgtttc ggagagcgat ttctagtgct actaaattgc acaaacaaac tttaggtaat 120
caaattgtgc aagtcaggaa ccatggtgga actgccaatt accgctgtgc ttcacgtcct 180
tcaaaagaaa ttaaacttct cggtgaactt actggggctt taatgtggta ctggtgcttg 240
taccatattt ggactgaacc ggatcatatt ttaggagagt ttccttatcc tgatcctagt 300
aaatggacgg atgaagagct tggtatccca gccgagtaat gatggatagt taattgatag 360
aaataattaa atattgaaat ttaatgaagt atctggattg tatatggtac tgcaatttgt 420
atatagaaag tgaataagat gtgttaaacg gtagtgaaat ggaaaaaaaa aaaaaaaaaa 480
aa                                                                482
```

<210> 1511
<211> 550
<212> DNA
<213> Ctenocephalides felis

<400> 1511

```
gctactcatt ttacgtaacg ttttagaagt gtgtgggtgt ggtgctaatt ttagcaccgt 60
agcgatcgtt tttattaagt ttttttattt ttgagtaata gttttaatat attttaccaa 120
aatgttgcat cttaagtcaa tcaccaaaag tgcactgaaa cataacccct ccgaagtgtc 180
cacattggtc aaagctctgc caacggccat ccaaacgcgc tcttactccg agcaccaaat 240
tcccgatagg ctgaaggatg tcccaacaca tcctaatcca agatttttcg acatggtgga 300
gtacttcttc caccgtgcct gccagatcgt cgaatctaaa ttggtcgagg acatgaaagg 360
aagcaaaatg acagttgaag ataaaactaa gaaagtcaaa ggtattctta tgttaatgca 420
gccatgcgat cacattttgg aaattgcttt cccattgcgc agagattcag gaaactacga 480
aatgattcag ggttaccgtg ctcaacacag cacacatcga caccaccaaa ggaggttccg 540
tttcaatggt                                                        550
```

<210> 1512
<211> 550
<212> DNA
<213> Ctenocephalides felis

<400> 1512

```
gtcacttgta gcggtttgtt cgattataat atttttacga aacaatgaaa gtaatattga 60
gaaacaaata atacaaatgt agttgatttc gtacatttta tagcaatctt cacgatggcc 120
gccggtccga tagcagaaag aaatcaagat gccactattt atgttggtgg tttggacgat 180
```

aaagtctcag aaagtcttat gtgggagcta tttgttcaat ctgggcctgt tgtgaatgtc 240
catatgccaa aagatcgagt aactcaaatg catcaaggat atggatttgt tgaatttctg 300
ggagaagaag atgcagacta tgcaatcaaa attatgaaca tgataaaact ttatggaaaa 360
ccaattaggg tcaataaagc ttcagcacat cagaaaaatc ttgatgtcgg tgccaatgtg 420
tttataggaa atctagacac agaagttgat gaaaaattat tgatgacaca tttctgtttt 480
ggagtaattc ttcaaacccg aagatatgag ggatccaacc tgnaatcaaa gggtttgcat 540
cataaatttg 550

<210> 1513
<211> 550
<212> DNA
<213> Ctenocephalides felis

<400> 1513

ggcagttaac ctaccgtgtg gaataaattc gagggttttc tgctgaagtt gttattaagt 60
taatgatatc aagtctatca catatattta cgtactattt ttaagtgtga cagaattata 120
agtgtgcttt gtttataatt taagaagtga aaatagtaat taaagcagta aaatatggta 180
aatttaggtg acattttccc aaattactgt ttaaaaacat ctattggtga tattaagttt 240
catgattacc tacaagattc atggggaata ttattttctc acccggccga ttttaccccg 300
gtttgcacca ccgaattagc cagggttgta aaactgatgc cagaatttga gaaacgcaat 360
gtcaaggtaa ttgccttgtc ttgcgataca gtagcttctc atctggaatg gtccaaagat 420
attttgagct atgcaggtga aatgaccaag acattcccat acccaatcat cgacgatagt 480
tcccgcgaat tagcagtaaa cttcgtatga ttgcccggcg agaaggataa ggatggatgc 540
gtgccgccag 550

<210> 1514
<211> 550
<212> DNA
<213> Ctenocephalides felis

<400> 1514

tcaaagtgga cgttagttaa atgataattg atttgtagta attgaaagtt ttgtatttta 60
ttttgttaaa atattttgtt aatttcgttg ataataaatt aataaaaagt gcccgaaaat 120
gtctgcatct cctctggaac gattgtcaaa aacagagacc atcgagttga gggacaaaca 180
tattggaaaa tcctgccaat tgttcttccg agaggatcct ctgaagattg taagaggcga 240
gggtcagtac atgtacgacg agactggcac aaagtacttg gactgcatca ataatgtagc 300
acatgttggt cactgccacc caaaagcagt agcagccgga gcccgcaaat gtccctgctc 360
tacactaaca accgtttcct ccatgacgag ctggtgatcc tggcccgcag gatttcctct 420
cttttgcaga acccctgagt gtctgctacc tcgtcaattc aggatcggag gctaacgatt 480
tggccttgag gtggcaagga ttcacacagg aaacaagatg ttatcacgct gcacagtgag 540
tagtagatgg 550

<210> 1515
<211> 550
<212> DNA
<213> Ctenocephalides felis

<400> 1515

```
agagaactag cctgtgaaaa gatcgtcggc tttggggcag ccagcacgta ggtttctgac 60
tcatttcagt ttggtcattt ctgattggca tacatcattg ttctgcaact gtttttataa 120
gtttttacac atcttccaaa tcctgtgcag tttttttat ttgggaccac agtatataac 180
aagttattgt atgagtcaga aaaccgaaaa accagtacta tcaggtcagc gcatcaagac 240
cagaaaaaga gatgaaaaag agaagtatga tccaagtgga ttccgtgacg cagttatctc 300
aggtctcgaa cgtgctggca acgatctcga cgcagttaac aaattccttg acacagcagg 360
ctctaagctt gattatcgca gatacggaga agcactgttc gatattctta tagctggtgg 420
attgtagtgc cgggtggttc aatagcacaa gatggagaaa agccccaaac cagtagttgt 480
gtctcacagc ttctgaggat atggagtcaa tgcgaaacca ggagcaggtt tcgtgaatta 540
tgctcgctta                                                        550
```

<210> 1516
<211> 550
<212> DNA
<213> Ctenocephalides felis

<400> 1516

```
agaaagatat tccantgaaa agtgctattc tatggtttca attantnttg gatcacaaga 60
natacctact cgttnatnta aaatgaaaat tctaatttta ttcttaatca ttctttctat 120
gttttacctt gcntcctgtg atgagcgcat ggtgaatgga aacgaagtat acattctaac 180
attttattgg caattgtcct ttncaagtaa ttttcaacat ttttgcggtg cancaatgat 240
cagtaagcga tgggcaataa caaccgcttt ttgtgctnaa aaaacgtcta tccatgcagc 300
tagagtacgt gctggaacca gcaaatataa tagnggtggt actcattacg gtgtggaatg 360
gntcgtccct catcctcgtt atgatagccg cgatcaaaat tcaatgtagg tttaattntg 420
ataacanagg atttcaatga aactagaaga accgcctgcn agctcgtana ggcaaatgtc 480
gcttnctgta gctcctttgc cattctggat ggngatctga ncgatccang agcacctatg 540
taagaaatct                                                        550
```

<210> 1517
<211> 550
<212> DNA
<213> Ctenocephalides felis

<400> 1517

```
ttaatgtcga cggaacgcct ttaacagtaa ataaagaagt atttgcatca ttggatgagc 60
ccgcaccagg agtagtacct actcctgaac ctacacctgt accgaaaccc gagcaaaaat 120
gtaaaaaagt aaaatttagt tgcgtgaatt cgtgcagttc acccgaaatg cagtattgtc 180
cggaaatagg agcagatccg gttaaggaat cctgtagccc agatcaagtg tgcgctgatc 240
aaagtggata tctacagtgc accactaaag aaagtacagt ctgcaaagta caaggtttca 300
aatgtccgtc accatcgaga ttttatccaa atataaatga ttgtcaaagc tattattatt 360
gtgacgaaaa tagtatagga acccaatatt attgcccgca aattttgcat atgatccgtt 420
acgtcataat tgcggcctat ggctctgggc acaaaatgct atacagttac atgtctgcca 480
gcctaaggtg cttccgtaca ttggtgataa atattgacgt cgatgtatgg ccggaagagg 540
accgtangca                                                        550
```

<210> 1518
<211> 550
<212> DNA
<213> Ctenocephalides felis

<400> 1518

```
cgagtttata ttttattttt aataatttaa tttatattat tgaatgggag attattagaa 60
ttaattctat aataattatt tttagaatat tatttgattg aatatcttta atatttataa 120
gatttgtatt aataatttct tcaatagtag ttaagtatag agaaagatat ataatagaag 180
attttaataa aaatcgattt ttattattag tattaatatt tgtattttct ataataataa 240
taattattag tccaaattta attagaattt tattaggttg agatggatta ggtttagttt 300
cttattgttt agttatttat tatcaaaata ttaaatctta taatgctggt atattaactg 360
ttttaataaa tcgaattggg gatgtgcttt attaattaga atttcttgga taataaatta 420
tggtagttga aattatttat tttatataaa atatataata aataatttga aataatttaa 480
ttatattttt gatttaattt ctgcaataac taaaagagca caaatccttt tcttcttggt 540
accagctgca                                                        550
```

<210> 1519
<211> 550
<212> DNA
<213> Ctenocephalides felis

<400> 1519

```
ggtaaataaa taaaaaaaat acttttatga taacagaaaa aaaactacag gtttaatgaa 60
atcagttaaa atcagagtga attttaacat ataaatttaa atattataaa aatcatctta 120
gcttttacaa acttaaattg caatgagtaa ttgttgtcga atattatgga ttgcgatagt 180
gatcggttta ggtgtgttgt attacgaaat aactaaagaa tttccaaagc caaatatacc 240
tctggataca tggtggggaa ctggaaaatc acaaaaaatt gatacatcaa tgaggccgtt 300
taaaattgcc ataaacgatg aggtccttaa taccttgaaa gtgaaactaa gtgatgtgtc 360
ctttactcca cctcttgagg gcatcgattt ccaatatggt ttcaatacaa ataccctgaa 420
aaaacttgta gacttttggc gaactcaata caattggcgc gaacgtgaag cattattgaa 480
taaattccca cttcaaaaca aatattcaag gcctggtatt cactatgtcc ataaaccaca 540
gtctccaaaa                                                        550
```

<210> 1520
<211> 550
<212> DNA
<213> Ctenocephalides felis

<400> 1520

```
ctggtcttcc cgccttatcg tacatcataa ttcgaactca aaatcccaat atcgtacaaa 60
gactagtcct cggcgctgct ttgctatatt tatcatttgt tcacatgcac agacagtatt 120
atgattacgg atcatacacc ttagacatta caggtcccct tatggtaatt actcagaagg 180
```

```
tgaccagtct tgcgttcagc attcacgacg gcctggcacg cgacgagaaa gatttaacga 240
aggaccagaa ttatcatgct gttcgcagaa tacctactgc tctagaattc ttcagttaca 300
ccttccattt ccagggtctt atggctggac cagttgtttt ttatagagat tatattgatt 360
ttattgatgg aaaccatgtt ctgaaattta caccgaaatc aacggcaagt ctagataata 420
actcaaacag cagaaaagtt gtgcttgaac cttctctata atcattgctt caaaaaagtg 480
atagtcagca caatatgcgc ctgtatttat tagttcttcc attgttccaa ttaaagcttg 540
aaagaagatg                                                        550
```

<210> 1521
<211> 550
<212> DNA
<213> Ctenocephalides felis

<400> 1521

```
ggaaaacata aattcctcgg atacaaaacc aactcattgg atgaataaag atggaaaacc 60
tttgactatt gaagtaggaa ataaagatgg atgggttatt ataaataaac agaattcagg 120
ttactatcgc gtgaactacg ataaggacaa ctggaaaaag cttgcagatg tcttgaaaag 180
tcctgaattt gaaaaaatcc acgtactcaa cagagcccaa attttagacg attctctaaa 240
cttggccaaa actggaaaac ttgattatga attggcctta gacattttag attacttgca 300
ccacgaattg gattacgtgg cttggaaagc agctgaagaa gatctcaatt ttctcgataa 360
tatgctgagt ggaacaaaag tctaccccaa atttaagaaa tttgtattgc atttagtgaa 420
caaagtttat aataaaatgg gatttgagca acaagatgct atgggcacat tgngtttcac 480
tcgcataaat gctctaacat gggcttgnaa atggccttnc agatgcttgc agtctcatgt 540
cgcttntgct                                                        550
```

<210> 1522
<211> 172
<212> DNA
<213> Ctenocephalides felis

<400> 1522

```
caggatttca tggtcttcat gttttaattg gaacttcttt tcttattatt tgtttactac 60
cacttagatt atttcatttt aatcctaaac atcattttgg atttgaagca gcagcttgat 120
attgacattt tgttgatgta gtatgattat ttttatatat ttctatttac tg         172
```

<210> 1523 <211>
673
<212> DNA
<213> Ctenocephalides felis

<400> 1523

```
gttaattccc attaacacgc ggcgtttttc gtttagcaat tcaataaatt acacacttca 60
caatggctga tatggaagat actcatttcg aaactggaga ctctggagct tcagcgacct 120
atcccatgca atgttctgca cttcgaaaaa atggttttgt aatgttaaaa tcccgccccg 180
tgtaaaattg tagaaatgtc cacttccaaa actggtaaac atggtcatgc taaagttcac 240
```

atggtcggaa ttgatatttt caacggcaag aaatatgaag atatatgccc atctactcac 300
aacatggatg tcccacatgt aaaacgtgaa gattatcagc tcactgacat tgatgacggt 360
tacttaacat tgatggctga caatggagac cttcgagaag atcttaaaat tccagatggc 420
gaattgggac agcaacttcg taatgatttc gaatctggaa aggagctttt gtgcctgact 480
aaaatcttgt ggagaagaat gtgtcattgc gatcaaaacc acacagcctt ntaatatgat 540
tttcacacat tcaaataaaa tctatgagac cattcaactg ttaacagttg caagtggcgt 600
gtatctagtg tgttgctata aatctgcctc agcttgttan attatgcaaa atcaggttag 660
tctttgatat ttg 673

<210> 1524
<211> 681
<212> DNA
<213> Ctenocephalides felis

<400> 1524

gaaactgtta ctcttttggt tgcattaaaa gttaggtatc gtgaaagaat aactatactg 60
cgaggaaatc atgagtcacg tcaaattaca caagtttatg gtttttatga tgaatgttta 120
cgaaaatatg gaaatgcaaa tgtttggaaa ttcttcacag acctatttga ttatttacca 180
cttaccgctt tagttgatgg acaaatattc tgtttgcatg gtggtttgag cccttcaatt 240
gatactttag atcatattag ggctttagac cgtttgcagg aagttccaca tgaaggtcct 300
atgtgtgatc ttttatggtc agatcctgat gaccggggtg gttggggaat ctcgccacgt 360
ggtgctggat acacttttgg ccaagatatt tcagaaacat ttaaccattc aaatggcttg 420
acattagtat caagagcaca tcagctagtt atggaaggct ataattggtg tcatgatcga 480
atgttgtgac gatttctcgg ctcctaatta ttgctatcgt gtggcaccag cacattatgg 540
aattagatga tgcattaaaa tttattctca atttgaccac tctaacgggt gaacctatgt 600
ctagaagact cnatactctt gagatatcta tcatgtgtaa ttatcatcca gntagtcttc 660
tttctacacg ncataacnnc t 681

<210> 1525
<211> 676
<212> DNA
<213> Ctenocephalides felis

<400> 1525

gccgntgaag ccgcaccagc tccagctgca gcccccgccg ccagcgaacg ccaatcatcc 60
aggggatccc gcaaagccgt caagcgcagt ggatctaacg ttttctccat gttctcacaa 120
aagcaggtag ctgaattcaa agaagccttc caactaattg accacgacaa agatggtatt 180
cattggaaag aacgatctcc gtgccacttt cgacgaattg ggccgtttgg tacaagagaa 240
agaactcgac gacatgatcg gtgaggcttc aggaccaatc aacttcaccc aattgttgac 300
cttgttcgcc aaccgcatgt ctggatcagg tggtactgat gatgatgatg ttgtcatcaa 360
cgccttcaag accttcgaca acgacggcaa aatcgacagt gacaggttac gtcatgccct 420
catgacctgg ggagataaat tcactgccaa gaagttgatg acgcctacga ccaaatggca 480
ttgacgataa aggcttcatt gtacccagaa gctcatcaaa tgtgactgcg tgcgaagaag 540
acaaaaagtg gtgaatataa gaaattgaat acttctacat atacatttaa acactgttac 600
ccattgtggg atatgttaaa atgaactaca tattttgcaa ctataataaa agtatgaaga 660
tgacanacaa atatgn 676

<210> 1526
<211> 647
<212> DNA
<213> Ctenocephalides felis

<400> 1526

```
gagacatcga aaaagtaagt tttatagaac ggccgcttac actttacaca accaaaatta 60
atctgctgaa gaattttacg aagactaatt agttttaaat ctacagtatg gatgtgttga 120
ataggcccgc tcacgaattt ggaaatgatg aaacagttga aactttatgg gctatgaaag 180
ccatggatca tgttattgtc tattttaata tactttgctc tgtagaccca aaatttctca 240
aaatgtgccc ccaagatgaa attatctacc attgttttcg tcaagaattt ccagatatgg 300
atgttaaagt actagatgaa aattcattaa aaggctatac aggaaaatgc cgatggagag 360
aattttgtga acgattcaaa catatagaag attacagttt tggtccttaa tacgcttaga 420
ttgcacattg gattatagtc cagaaaatac tattttagtt ccaagagttc agttttatgc 480
ctagaatctg tcgaaataaa gaaggcttat gctgatcaga aaaaaatatg ctaatgaagt 540
gatgttgcga gnatgaaaat gaaatccata gaaatatagc atggtatgtt aagagtcatg 600
agaaaagggg gtanttattt tatttataat nttatcaaaa aaaaaaa               647
```

<210> 1527
<211> 540
<212> DNA
<213> Ctenocephalides felis

<400> 1527

```
gaggatgcca aaacagaacc gtgtcgcaat ctacgagcac ctctttaagg agggagttat 60
ggtggccaag aaggactacc atgcaccaaa acacccagaa ttagagcaaa ttccaaactt 120
gcaagttatt aaggctttgc agtcgttgaa atctagagga tatgttactg aacaatttgc 180
atggaggcat ttctactggt atctgacaaa tgaaggtatt gaatacttga ggacatactt 240
gcacttgccc ccagagatcg tgccctccac tctcaaacgt caaaccaggc ccgaattggc 300
aaggccaaga ccagctgccg gcccaaggac tgaaggatct cgtccagctg aagacagatc 360
tgcctaccgt agggcacctg gtgcacctgg tggcgctgac aagaaggctg atgtcggtgc 420
tggcactgga gacttggaat tcgtggtgga tatggacgtg gcagacctgc cctcaataaa 480
tttatataag taatttataa taaattcaat aaaacattta tgataaaaaa aaaaaaaaaa 540
```

<210> 1528
<211> 671
<212> DNA
<213> Ctenocephalides felis

<400> 1528

```
catnaacaga tatcccgatg atccaaagca tcaaaatgat ttagcagtaa cactacttat 60
gggaaacaga ccagctgaag ctttacgcgt tttgcacaaa gttttacaag tttggagaaa 120
taatggtttt gcattggtcc actatggatt tatttataaa actactatga acgacttgga 180
aagaggtgta aaatatttgc aagaaggtat tgacaccaaa gataatggca caatggatgg 240
```

aagattcttg tttcacctcg gtgatggatt gcaaagactg ggcagaaatg atgaagctat 300
gaaggtttat gaaaccggag taaaaaataa aatcttccta tctcgatatc aacgttcact 360
ctataatatt gatagattag tgtccagacc ctggtggact attgaacaaa ctggttatac 420
taaacttttt aatacattaa cttctaattg gaaagctatt cgagatgaag ctttaaccgt 480
gcttgcatgc aaaagcgcaa gagtaaggaa aaattgcaga atcaaaatct gggcctgtta 540
gagccaggca gaaaattaag agatctgggn atggagcagt tgacttatcg gagnatanga 600
taccggttgt ttacccatac agccgctatt tgcntcagan ctactgcctn ggcaataatc 660
actatgaccg n 671

<210> 1529
<211> 667
<212> DNA
<213> Ctenocephalides felis

<400> 1529

aggnnncacg cctatgctca aaagaacttg cgcagatctg ntcgtcgcat taaggaactt 60
nccttccaag ctgaagaaga ccgcaagaac cacgaacgta tgcaagacct tgtagacaaa 120
ttacaacaaa agatcaagac ttacaagagg cagatcgaag aagccgaaga aatcgccgnc 180
ctcaatcttg ccaaattcgc aaggctcaac aagaattgga agaggctgaa gaacgcgctg 240
acttggctga acaagccgtc agcaaattcc gcgcgaaggg acgtggtgga tccatggcac 300
gaggtggcag cccagtgccc gcaagaacag cagctcgccc acaatttgac ggaatggctt 360
tcccgctagg ttcgacttga accctgacag cgagttctaa attatttata attaatttta 420
aaaaagcgaa cgtggctgct tgacagtaaa cattaatatt ttttaaaaat tattaaaagt 480
aaaaaatatc agctcgttca attgtgctta gtatgacatt tgatctaatc taacactgtc 540
agnctaacac attatactat ttatcgccca tacatcaacg tcgctgcata aaatatacag 600
ctccattagc tcctaattct tanttttgna atactttttt gcgatttatc aanggaatta 660
ttatgtt 667

<210> 1530
<211> 670
<212> DNA
<213> Ctenocephalides felis

<400> 1530

agcgtgttcg ccgaggagga acgcgcacgt tttgacacat ttctttagtt tttaagataa 60
aatccaatcc ttcccaacaa aaaattagtt ttaagcgcac atttatgttt agtgacacga 120
aggcggttgt tataaaatta gtgtttacgt gatgtctaaa caacagtaca cagttgacgg 180
tgatctattc ggtacgcctc acaggaaaaa accagtgcct tggaatgagt tcttgtacaa 240
cagcgaagag ggaacagtct taggaaggac aggactgagt tgggcgaaaa tcggtatatt 300
ctacacaata ttctacggag tattggcagc attagtggca atatgcatgt gggtgttctt 360
ccagacgtta gatcctcgta taccaaaatg gcaattagac gaaagtatca taggaacgaa 420
tccaggcctt ggcttcaggc ctttgccgcc gatgagaaca tagaaagcac gctcatctgg 480
tacaagggta ccgatttaga caactacagt cgatggncaa atcctttaga gttcttcaat 540
ttacaaaact ccggatacct caccgagtaa acattcagtg tgctccactc gccgcccaaa 600
gttgggtngc gngacgcaag ttggtcctgt cganaaacat ttatatacag acngcgggta 660
tttgagtgan 670

<210> 1531
<211> 558
<212> DNA
<213> Ctenocephalides felis

<400> 1531

aantngtcga ggtcacgaat ctgaaaatga gtngncaccn attcagctga tctctgccac 60
agcttaccag ctcaaaaaac ccaatcaaca aatgttgatc ccaataactg nttggattgg 120
tatggagcaa gcattcattg gtgctgattt cacacaggcc tatgtatcct gngcttttgg 180
gtattaagtc aaattggata ccgtcatgat ctgcttcgga gtagtcaacg caatttgntc 240
agtcgttttt ggttctataa tgaaattcat cggtcgccaa attatcatca ctttcggttt 300
cttcctgcac atgggcctga tgattggatt attattctgg agaccaagcc ctgatgataa 360
aatgatgttc tttgtcatgg ctggactttg gggagttggc gatgcagntt ggcagacaca 420
aatcaatggt ctatacggaa ctctgtcagg cggacnaaag aggcagcttt cttcaactac 480
cgctgtggga aagcttaagg ttgtcatcgt tacgctacag cacacacttg tgcccggcat 540
gaanttacgt caattgac 558

<210> 1532
<211> 660
<212> DNA
<213> Ctenocephalides felis

<400> 1532

gtaaaaacaa aataaaatca ttttagtttg agttagaaca tgtgttatta taagttagcg 60
taacacacaa aaattaatca taaatgtctg ataataattg gtagtatact tgtacagtaa 120
attctttatt ttccttttcc caatcttttt ctaaaagcga gtgtggtcat actgctgtgg 180
taattcgtca ccgagaagcg gatgaagttt atcctccatc attagtgaag gctttgcttg 240
tcagaagaga gaggagtgtt ggttgccggt gtttattgta gttttaattt aattttaaac 300
taagttatgc gtatgaccca agacatggac gatgatgaaa agggaaagct tttcgtcgga 360
gggctatcat gggagactac tcaggagaac ctgcagaggt tcttctcgag gtacggcgaa 420
gtgatcgatt gcgtcgcatg aagaacagcg agtcggcagg tcgcgcggct tcggcttcgt 480
cacatttgnc gatccctcga acgtcacgtc gtctgcagaa tggccgactc cctggcggaa 540
gacttagatc aaacctgcac cccgcccctc agaccaaacg ggcgagctcc caagttcttg 600
cggctgcatc acggnggaac cgctagtgtc tcgcntacga aagttgagtg ctntgcnacg 660

<210> 1533
<211> 669
<212> DNA
<213> Ctenocephalides felis

<400> 1533

cacatattca agttgattca attcagcaaa gattattaca aaaagttgat ggtgtattag 60
cagtacatga atttcatgtg tggcagttag ctggagatcg tatcattgcc tctgctcaca 120
taaggtgtag aaatttatca gagtacatga aaattgcaga aagagttaag gaattctttc 180

```
ataatgaggg aattcattct actacaatac aaccagaatt tgtagaacta agaagtttat 240
tagagccttc acgagatatg gagcaaccat gtgctctaga ttgcccaatt actgatgtac 300
catgtgctca agctacttgt tgtggaactt ccaaaccaga tagagatacc ccatcaccag 360
cggcctcacc ttttatgtgc aggcaacgag gagcaggtca acgagttcaa ggtggttctt 420
caggtccaaa cactggtgat ctagaaagtg gtcattgtta ggtggacatc caaccactca 480
gttgttgcaa ttgcctctgg tctaattgtg ctgtccaaat caaataaaga atcttgtgct 540
gcacacatta tagcgcaaag tccctgccat cagttgctga tattctatat gcaaattttn 600
ataagaatag atggaattat gcagggagga nangtcatat gataattatt atcattatgt 660
ggactggat                                                         669
```

<210> 1534
<211> 546
<212> DNA
<213> Ctenocephalides felis

<400> 1534

```
cttgtnttct gaagcaataa tcttgttaaa tgctttgggt tgtagcaaag atcctgatat 60
tttaaggagc tacttggaaa aaactgtaga acctgattcg aaaatcagag atcaagataa 120
attccgtgct atgtattctg taatcagaca aggaagtgat ggagtgacaa ttgcattaga 180
atttatgcgt aataaattgc caaaaatgat tgaacaatat acaagcttga atgcgctcaa 240
aaaagttttc gaaactgtag gcgcagcaat ttcaaacgaa aaacaagaag aactgcttcg 300
tgaaattatt gctaaataca attctacatt ttcggattcc ctaatgcaag gtgcaaaaac 360
tgctttagat gccatggaag ataataaaac ttggagagat aaaaatttag ctactgtgac 420
aaggtggttt gaaaaacagc ctgaagttta tcctaataat gcaacaaaac tagtccaaag 480
tatttattat tacttgattg tattcaatat tgattatatt taatatatac atcagtntcn 540
gaaaaa                                                            546
```

<210> 1535
<211> 662
<212> DNA
<213> Ctenocephalides felis

<400> 1535

```
aagccgtggt agtttcgctg tatgaggaac ctattcagcc tcaacaagct gcttctgtcg 60
tggagaaact tggtgactac ctcataactt gcggctacta aatgtgaaac actggagtgg 120
ccccttatta atttttaaga aaactataat aattataatt acgattaatg aataaattta 180
aacaatgaag accggccagc aaaaagggac agaatttttt tctcatgcct cctgcaatgc 240
agtaaaatat tttgtgccat ttttgtgcct ttgttcccgg tgaccttttt ttcctcagaa 300
gggacacctg gagtgtactg gcaaaatcga ggacattagt ttaaacattt gtattataaa 360
accgtgcgga cggcaaaatg aataaaaaat cttactacga ttaaatttgt attaatttcg 420
atgtgaggtt atcgacttgt ggatttaaat atactttctc ctttaataaa actcgtgctt 480
ttgcaattta tgatttgcta ttattagcta ttgatgnata aatctgtatg aattttgatc 540
cctcaataaa gaatttatat agtgactagc tctcagtttg aatttattat atcgtattgt 600
gcaaagccga ccatagttgc gactgactcn cgttctnttg tgctgacttg tattgctatn 660
ct                                                                662
```

<210> 1536
<211> 668

<212> DNA
<213> Ctenocephalides felis

<400> 1536

```
gaaatattta cctacattag ctttgccaga tggatcccat aattttacgg aagattctgt 60
atttttttcat ttaccaggat tacaaaagga tgaacagact atttttggag tatcatgtta 120
tagacaatta ccagtggaga aattagttaa tataccttct gatgtaactc gcagcactgt 180
tcaaaaatca gtttgtgttc taagcacctt acctttatat ggtcacattg agataaaact 240
tgcattaata gcacatgcat tttttgaaca aggcgacttc agccaaacca agatattgca 300
ggatgcatat cataatatga accaatgttt tggctcaaat gaaattttag aaaaaataac 360
tataggatta agtgtcagag atttagtgtt gaggtggcgt cataaaatat tagttttatt 420
taaattaata ttattggaaa aaaaggttgc atatttggtt caccagtaag gccattatgt 480
ctagccctat taacttatta tctcttcatc caatattata gacaaaggat tattgagtcg 540
gcaatcagga cacttntata aaagaaaatt ctagtatatg caatcagata tatcatgcgg 600
aaacagaatt aatatctctg atctaataca aatatgaatg tggaaagttt tgagatatct 660
aaatgctg                                                          668
```

<210> 1537
<211> 620
<212> DNA
<213> Ctenocephalides felis

<400> 1537

```
atcaacttgg attttgtcgg ttaattccga aatcgaaaaa gtttttaagt gcaaaacatt 60
taactgtgac atcagtttgt aatgtatctg ggaaggaaat gagatctgta aatccgattg 120
aaaggtttcc tccttatgac tataagaaga aaggatacgg atttatcaat gcgtttttttg 180
attacacaac taagagattc aatgataaca caaaggttat tacagtggaa ggtccaccag 240
ctgttggcaa aactgcattt gctaaagctt tggccgaaga tttagatatg aaatactttc 300
cagctgttac tatggatcac tactatatta atgaatatgg gtatgatctc agacaacttg 360
atgataaatt acctgaatca tgtaaaagca tcgattttga taaatttcat aaagatccgc 420
ataatagaaa tgtagcaaca atgcaaattg tgttatatat gaaaaaatat gaacaatatc 480
ttgcggctct tgccatttat taaatctgga caaggagtta ttttagagag acttgtactc 540
tgtttgtttt ctagaacctg gctagctggt tgttcgcagg actcgtcgat atttgaatag 600
agcacacatc cagatgagca                                             620
```

<210> 1538
<211> 557
<212> DNA
<213> Ctenocephalides felis

<400> 1538

```
gtcattcact ttttacgagt tctttagtgt cctaatttat taataagtat cagaatggtg 60
ccgaaaacaa aagtatttgt tggaagcttg ccgccaggct ccaagcctga agaattacgt 120
```

cgtttgttcg aagcttacgg tgttgtaaca gaatgtgata ttatgaatcg ttgtggcttt 180
gtacatatgc agaccgaaga gatggctttt agcgcgattc aagcgttgaa taatactaca 240
tttaatgggg ccacgataag tgtcgaaagg ggccgcatca aggaacgcgg atcaggtggt 300
ggtcgtggag gcggtggccg cggcggtcga ggatttggag gacgaggagg tggtatgaat 360
cgtagtggtg gaggacctgg gggtatgcgt aatggtggtg gccccatggg aggtggcatg 420
cgtcgtggag gcggtggccc aggacccatg cgtggtggtg gccgtgacat gaatcgtgga 480
caccctattc tngtgatggt ggcgtggagg agatttggtg gcgagggctg gacccatgcc 540
aatggttgnc gcgcgga 557

<210> 1539
<211> 556
<212> DNA
<213> Ctenocephalides felis

<400> 1539

gcgtgcctta cctccgagct gttgttactt gttcattttc gccggtttgc gttcgaggaa 60
attttgtttt cggacgcacg tgagcggtaa cgagtcctgc aaagatgtat ataaaattct 120
gtttattggc attcgtggcg gctgaactaa tgttaatgtt agttgaggcc aatgttattc 180
ctggtgaaga agaaaagagc gatggattcg agctgctcat tctgcataac aacgacatgc 240
acgctcggtt cgagcaaacg gcggcacgca gcggcccctg cgtagacgga gaacgatgtt 300
atgggggctt cgcccgggtt gcacatttag tacgggaagc tcgcaagaat gaatctaacg 360
gcggccctcc tgtgctgtac ttgaacgctg gcgatcatat caaggaactc cttggtacac 420
gctttacaag tggaacatat cttatatgat gtaaacgaac tcgctccaga tgccatgtcg 480
tgggaaatac gagttcgaca cggtgtccca ggttgctcca tttctggaaa atgtgaaatt 540
tcagtgtagc tctacc 556

<210> 1540
<211> 620
<212> DNA
<213> Ctenocephalides felis

<400> 1540

cctacttaca aattaacgta cttcgacctc aaaggaatag gagagccttt gagacttcta 60
ctaagctatg ggaacataaa attcgaagat gtccgagtgt catttgaaga atggcctgca 120
cttaaaccaa agatgccatt tggtgtaatg ccggtattgg aggtcgacgg aaaggaactg 180
caccagagtt tggcactttc gcgctatttg ggaaaacaat tcgggctcgg gggcaaaaat 240
ctaatggagg agttggaaat tgactcaata gtcgataccc tgaacgattt cagatataaa 300
ttgacgatgg cctattggga acaagatgcg gctgtaaagg agaaaaaaat gaaagaagta 360
cacgatacac tcatcccatt ttatacagaa aaacttgata aaatagcgaa ggctaataat 420
ggcatttagc tcttggaaga ctaacttggg ccgctttgtt tcgcggagtt atcgaatata 480
tgagtttcat atctggaaca gatttccttg gaaattgcag gattcaagag tgctttaata 540
tgtgcaattg ccgacgtgaa ggaatggtcg caagaggcaa aactgttgta tgtcatttgg 600
gaatttaaat aacattatac 620

<210> 1541

<211>
620
<212> DNA

<213> Ctenocephalides felis

<400> 1541

```
atttgaatcc tcttatcgat tactaatctt ttgtacagtt taatatttat tatcaaggcc 60
ttgtacgcca gtgtgtgtaa aggtctcttc agtcgacgac agaggataga agtcagagga 120
catttataat acgcagtttt taagtttttt ctggtgaatt tgaatatatt tgaagaaaat 180
gaagaggttt ttggttgctt tggcggtttt ggtggtcgtt gctgaggcta agctgtcatg 240
ttcgagtaca aaagcctcga tccccgaaga atggatcgac atgacagccc agtgcacgag 300
gagcatgagg aaccagatcc aagaagaact gagcgcttcg atgcaatact tggccatggg 360
ggcgcatttc tcaagagaca ctgtcaacag gccaggattt gctgagatgt tcttcaaatc 420
ggcaagcgaa gagagggaac atgccatgaa actcatgtct tacttgatga tgagaggaga 480
actgccgaga ggctgcagga cttgacagaa caccactgtc caatcacact tggctgtggt 540
tgagtgcttt gaagatctct gaatggagct tcgtccagaa ataacatgtg acaaagcttg 600
cagacatatg gactatgata                                             620
```

<210> 1542
<211> 591
<212> DNA
<213> Ctenocephalides felis

<400> 1542

```
aagaagatca agaagaagaa ggcaaaagaa gagtctggag atgccccagc cgctgaagcc 60
gcaccagctc cagctgcagc cccgccgcc agcgaacgcc aatcatccag ggatcccgc 120
aaagccgtca agcgcagcgg atctaacgtt ttctccatgt tctcacaaaa gcaggtagct 180
gaattcaaag aagccttcca gctaattgac cacgacaaag atggtatcat tggaaagaac 240
gatcttcgtg ccactttcga cgaattgggc cgtttggtac aagagaaaga actcgacgac 300
atgatcggtg aggcctcagg accaatcaac ttcacccaat tgttgacctt gttcgccaac 360
cgcatgtctg gatcaggtgg tactgatgat gatgatgttg tcatcaacgc cttcaagacc 420
ttcgacaacg acggcaaaat cgacagtgac aggttacgtc atgccctcat gactggggag 480
ataaattcac tgccaagaag tgtgacgctc gaccaatggt catgccataa gcttcattgt 540
cccagaacta tccaatgtgc tgcgtgcgag aaacaanagn gngatataga a          591
```

<210> 1543
<211> 554
<212> DNA
<213> Ctenocephalides felis

<400> 1543

```
gcaactttgg gtctcatggt tttccaacag ttgtctggaa tcaatgctgt gatcttctac 60
agcgtgtcca tatttaaatt agcaggaagt gacctggacc ctgcggtgtc ttcgatcatc 120
atcgcagcag tgcaagtggt gatgagtcta gctgctattg gattggtaga gaaatttggt 180
cggaaaactt tgctaatgat cagttccacg gttatgggaa tctgtttggc agctttgggg 240
tattacttca gggtgcaaac atcaggcgaa gacgtcacct ctctgggctg gcttcctctg 300
tctagcctgg ttttgttcat cgtggctttt tgcatagcct atgggcccat tccctggatg 360
```

gtcatgggtg agattttctc tgcgacgtta aaggagctgc ttgcagccta acagtcatcg 420
cagctggtcc ctcgtctttc tggtcactaa agtattcccc acatgaggga gactttagga 480
ggagatgtac cttttggatc ttcactttca tgatatcgta cactgtttcg tgtcttctgg 540
tgccgaacaa agat 554

<210> 1544
<211> 604
<212> DNA
<213> Ctenocephalides felis

<400> 1544

aagattacct agacttggaa caagaattcg acctaataaa tctaaacagc aaggtaataa 60
ggaatctacg gaagtgcaat ctcaagttga tgctgataaa gtaggcgaac aaccagcaga 120
gaatgtggtt gagcaaagtg ccgaaacaaa agctattgcc gaagaagatg atgttaagga 180
tgcatgggga tgctgattca agcagtgaag aggaaagcac aactgaaaca ccagcagtaa 240
ctgcaaagtc tgaaacaaaa gtggatcaat ctgcagaaag taaaaaggaa gaaacttctt 300
cagaagaaga gtctgaagaa gaaagtgatt ctgagtcaga atctgaaagt agcgaagaca 360
gtgataatag aactgatgca gaaaagaaac gagaaaaggc tctccagaga atacaaaaac 420
gcagaattga tgcagaacag aataaatcat tagacaaact gagagctgcg gtggttgtgt 480
gttgggacat gtagatctgg aaaacaaaaa ttttgcaata ctcnacaaat gtcaagatgg 540
tgaantgtgg tttctcacca aatgtgctca aatgtcatag aaacataagg acagtaaatt 600
gtaa 604

<210> 1545
<211> 608
<212> DNA
<213> Ctenocephalides felis

<400> 1545

gccntttttc gatcaattcg tattagactt tttgataaaa aatgctgtct aaagcttcgc 60
ttttggccaa ggtatcccgg ccactgactg tggcagtgcg aacaacatcc caggctgcaa 120
catgccctgc tcctacaaag gtagaagaag ccgatagtgc tgaaagagat ttggtcaact 180
tcccaaggcc aacacgtttg gaacattcac ctaaagttcg ctttggattc attccagact 240
catggtttga atttttctat gagaagaccg gtgttactgg accttacatg tttggaactg 300
gtttaattac ttacttatgt tcaaaggaaa tttacgttat ggagcatgaa ttctatactg 360
gtatttcatt gggtattatc tgtctctatg ccactaaaaa gttgggtcca catattgcaa 420
aatacttgga caaagaagtt gatgcctatg ccgatgaatg gaattcaggt cgtgtagaag 480
aagttaaaag ttaccaagat gccattgaag gagaaaagtt ggacaatgga gactgaaggc 540
aacttatgtt gatggatgca aacgtgaaat gtgcttgcac ttgaacacta caggaccgcc 600
tgaaggta 608

<210> 1546
<211> 595
<212> DNA
<213> Ctenocephalides felis

<400> 1546

agacntacga gcaccaagtg gacaagacac acagtggaca aacagtgaaa ttgttgattt 60
gattgtatca gtgatagcca gtgatataac aatattgtgt taaaaaatga tgacgccgac 120
cgaaattagc aatttgaaaa aagtgaagtt ggagaatttt tgggacgagg tcgatcccaa 180
aaacatatcg cctgagtatc caaaaaatga aatccaggat tttttcagcg gcggaagcgt 240
cttcataacc ggcggtaccg gatttttggg aaaattattg atagaaaaaa tactgaggac 300
atgtcccgat ttgtcaagaa tctacatctt ggtccgagac aaaaaaggga aagatgcaaa 360
aaatagatta aaagatatgt taaatgatgt ggtattccag cgattaaaga aagagagtcc 420
gtggcccaac aaaaattgga ggttgttatg gagatgttgg caagcccgat ttagggttgc 480
ggaagtgata gaaaaatata caggatatgc aacagttata ttcacgtaca ncctgcaatt 540
tcacgaccat aaaaactcag ttgcataatg taagggcant agagtgttga actgc 595

<210> 1547
<211> 595
<212> DNA
<213> Ctenocephalides felis

<400> 1547

atcntatcgg tgtgtttata tccagaagct ccgncaactt gttacaattg aaacaagtat 60
ggatcatgtc ggtgttccaa ttcatcaacg ttttggtgct tctattccaa gttttgtatg 120
ggtacattcc taatatctgg attgtttttg ccatcgtttt atgggaagga cttctaggag 180
gtggtgctta tgtcaacacc ttctacagga tgagccatga gattccaatg gaaaaacaga 240
agttctctat gtcaattact gctattgctg atagtctggg aatagccttg gctggatgga 300
tagctatgcc aacacataat gccttgtgtg ctttgcctaa accaacttaa gtgaataaaa 360
tttgaccaaa tccaacttta ggtggatgaa attaaagttt agttgtttgc gagtgcaagt 420
gtagataatt gaaccagtga aatagttttt aacgtaaaat ttctatcaga aaattatcat 480
tttaaggaac attgtggata ataaaacctt agcttaaatt aaatacccac ttataaacta 540
atatcgagta ttctctatta aatcaatcga accgtcatgt gaatcgaata tttga 595

<210> 1548
<211> 653
<212> DNA
<213> Ctenocephalides felis

<400> 1548

gaanncaata gaagacctct agtaatgggg tcagttggac cttatggagc acatctgcac 60
gacgggtcag aatacagtgg ctcttatgca aaaacgatca cgaaagagga aatccaagaa 120
tggcatagac caagaataga agctttaata aacgcaggtg tagacggatt agcaattgaa 180
actattccat gtcagttgga agctgaagcc ctagttgaac taatagttaa tgagtatccc 240
ggcacaaaag catggcttag ttttcaatgt caggatgaat ctcgtctcgc ccacggcgag 300
ttattccgcg acgcggcctt gagctgctgg gagctggcaa gggagtccca atgtttactc 360
gctgttgggg tcaattgcgt tcatcccaaa tacgccgtcg gtctctgcaa atccctcaat 420
agggatcaga tgccccaaat accgtcgtgt tatcccacag tggcgaaaat tacacccgcc 480
gaagggtgga aagataagac cgtgtgttct gtccaagata cgtcagttct ggttggatca 540
ggaccctttt anggaggatg tgcgtctgtg caggcataga atntagaaat tggatcgtgg 600

atataacgga ttaataattt aataaataat tnttacgaat ttntataatg tgg 653

<210> 1549

<211> 553
<212> DNA
<213> Ctenocephalides felis

<400> 1549

```
ctcgttcact gcatttatca gcaatggctc agattaaggt aggcgataaa attccgtcgg 60
tagacttatt tgaagacact ccagctaaca aagtgaatat tgcaaatctc gctgcaggaa 120
aaaaagttgt cttattcgct gttcctggcg ccttcactcc aggatgttct aagactcatg 180
tacctggcta tgttgcaaag gctgaagaat taaaaaagag tggaattgct gaaattcttt 240
gcgtctctgt taatgatcct tttgttatga gtgcttgggg taaggatcag cagagcaatg 300
gaaaggtaag aatgcttgca gatccaagtg gtacattcac aaaagaactt ggattgggag 360
ttgaattgcg cccttaggag gtttacgttc taagagatct ctatggtgat agacaatggt 420
gtcgtatcag aattgaatgt agaaccagat ggtctggact ttcctgttcc ttagctgaca 480
aacttaaagt ttagaatata gtaattattg aaataagagt aataaatata atgtaatatt 540
aaaaaaaaaa aaa                                                    553
```

<210> 1550
<211> 661
<212> DNA
<213> Ctenocephalides felis

<400> 1550

```
gaacntcgtg ctgatagcgc gcgcgttccg tcaccaattc ataattccgg acttccaagg 60
attcgcgaag gacttggagg aagtgtactg gaagtgcaag ggcaataacg atggcaaggt 120
gggcagaccg tcgcgcgttg tgaacccgga cgacgatccc gccggcgaaa atgttgctag 180
ctacattcca cagcttggct cgcatgaacc ctgatttttg ggtgtgagc gtctgcacca 240
ttgacggaca aaggttgtcg attggagatt gcaatgtgcc atttacttta caatcttgca 300
gtaaaccgtt gacatatgcc atagcattgg aaaagctagg ccaggcgacg gttcatcaat 360
atgtcggtca agaaccaagt ggtagaaact ttaatgaact cgtactagat tataataaaa 420
gaccgcataa tcctatgata aacgctggag cgatattggt tgtcgtattg aaaacttagt 480
caaacctgag atgactctac tgagaagttc gactatatga ccacatattt aagagattgg 540
tggtggaaga gtctcggctc aacaaccggt gtcttgcgga cccgaagccg cgatagaata 600
cctttggttt actgaggaac cagtctnccg gtaaactaat acggatctgc ttatttatgt 660
g                                                                 661
```

<210> 1551
<211> 671
<212> DNA
<213> Ctenocephalides felis

<400> 1551

```
gcattattag ctaacgatct tcataatata tttgtcaaca gtaatctaac gcatttgaga 60
```

aaacttcact tggaacaaaa tgaaatatta aattttggag acaaaagagt tttctgcgat 120
ttacctagtc tgcgagattt gcatttgggt gataattatt tatcagagat agactttaac 180
tttttgtgtc ttaagaattt acggtttctt gatttggaac ggaataaaat cgaatacttc 240
aagaagcgtg acctgctcac cctggaccag gtgaatgctg ctggccgaga agagcagttg 300
gtcatcgatg ttggcggcaa ccctttccga tgtgattgca ttgttagcga gttttattca 360
tggctgttcc gaactaatgt taccgtcagg aacaaggagt ctttacggtg tcatcgcgcc 420
caaaagcatg gcggggaagc attgatcagc ctatccgtcg ataaatgtcg aaaagctcaa 480
tctgtagtgc acgatcgagt aaaatatcta ccgtgacttt atgctgatta ttttaatagt 540
catttttctg gactgtcgcg tctggctaca tgagcagaga taagttgaaa tagcattact 600
ctgtgataga accgtttaaa aagtcaattn ccgatccgaa catgacagaa gttatatagg 660
aattgtgcaa t 671

<210> 1552
<211> 611
<212> DNA
<213> Ctenocephalides felis

<400> 1552

cacntttacc atcgtcgaaa gttgaagtca ccgtncagaa gttaaaggat gatcagaaac 60
tgaagagtca gcaggagagt gcggagaatg tttccacggt tacggctctc aaacctgcag 120
atagtgccga gataacagtt gtgaagaaaa cgctgaaaca gaaaataata catgaacttc 180
tacattacta ccatggtttt cgacttcttt tcatcgattt aaatgtatcc agaaaattaa 240
tatggcgagt tctgaatgga aaccagttga ccaggaggga acatagattg ctagtgagaa 300
ccactgctga tcttttcaga cttctgccat tttctgtgtt cattattgtg ccttttatgg 360
aattactgct gcagttgcca ttaaactatt cccaggaatg ttgcatcgac cttcaaactg 420
cccagagaga gaggataaat tgaaacaaag tttgaaggta aaactagaga tggctaaatt 480
tctgacggaa acattagacg acatgactct acacacaaag atcacagatc agaattggct 540
aaagagttca attgggttac gaaatgagac agctgcctca gcnntacgaa gaatcatgaa 600
attcaaaatg t 611

<210> 1553
<211> 566
<212> DNA
<213> Ctenocephalides felis

<400> 1553

ctgntngaca agaattgatt gacagattta atgangatga agaaattttt atatttttat 60
tatctacaag agcaggtggg ctaggaatta atttaactgc agctgatact gtgattatac 120
atgatattga ctttaatcca tacaatgata aacaagccga agacagatgc cacagaatgg 180
gccaaaagcg gccagtaact atttatagat taattagtaa aggaactatt gaagagggta 240
tgcttcaagt tgctagagaa aaacttaatt tggagagaga agtcaccact aacacggaga 300
atgatcctca agaagtaaaa aatgttgtgc gattactcac actagcgctg ggcgtcgatt 360
caaataaagc tgccaatctt ttaactccat cgcgagaaaa tcggaatcct aattgcaaaa 420
gccgctatta tttagcacac attatgaatt ttgatctttt aaatttatta gttaattgat 480
gattatttaa aaatataggt atttttatat aatttatgaa ttactgcagc gccaataaaa 540
tccgtttact ctaaaaaaaa aaaaaa 566

<210> 1554
<211> 585

<212> DNA
<213> Ctenocephalides felis

<400> 1554

```
acttnnctcc atgcaagcct ctcttgaagc cgaagccaag ggcaaggctg aggctttacg 60
catgaagaag aagttggaag ccgacatcaa cgagcttgaa attgctctgg accatgctaa 120
caaggctaac gccgaggccc agaagaacat caagcgttac caacaacaac ttaaggatgt 180
acaaaccgcc ttgaggaaga acaacgtgca cgtgatgatg cccgtgaaca acttggcatt 240
tctgaacgcc gtgccaatgc tctccaaaac gaattggaag aatcccgtac cttattggag 300
caagccgaca gaggccgccg ccaagccgaa caagaattgg gagatgctca cgagcaattg 360
aacgaacttt ccgcacaaaa cgcttcagta tctgctgcaa gaggaaattg gaatccgaat 420
tgcaaacctt gcattctgac ttggacgaac tctcaatgaa gccaagactc agaagaaaaa 480
gcaagaaggc atggttgacg cagctaggtt ggtgtgaact ccgcctgaca agacntgccc 540
aaacccaaga gaactcagaa aggtcttgaa cacaaataag aattg                585
```

<210> 1555
<211> 612
<212> DNA
<213> Ctenocephalides felis

<400> 1555

```
agacngctgt ttttgtgttc aaaaagcagt aaantataga aaaaaaaaac ttattactag 60
tgcacattaa caaaaataag cagaacaact attaaaaata tgttttccat gtcaaacctt 120
ttctaaaaac cattcaatgc atagaaatgt aatttttaaa atcaatagta tgattttta 180
aaatatttgg ttttatacca ggagtggact actgaatttc tgcagtgttg ctatggctct 240
caatgatcta gggtacaaag ctgttggtat acgaatagac tctggtgatt tagcatatct 300
ctcagttctg gccagagaaa catttgagag gatagcagag aaatacaaca ttccttggtt 360
tgcaagactc atgataattg catcaaatga tatcaatgaa gacacaatac tcagtctcaa 420
tgaacaggga cacaaaatag attgttttgg aattgggacg catttagtta catgtcaaag 480
gcaccagcac ttgttgtgtc tacaaaatgg tagaaataaa tggacacctc gaataaactc 540
agccagatgt gctaagtaca tgccagtcgn agatgcctat cgttatatgg nagatggcat 600
gccctatgct gc                                                    612
```

<210> 1556
<211> 613
<212> DNA
<213> Ctenocephalides felis

<400> 1556

```
gtggttcaaa gataatccaa gatgggtaag ggaaataata tgatccctaa tgggcacttc 60
cacaaagatt ggcaacgttt tgtgaaaact tggtttaacc agcccgcaag gaaaattcga 120
agacgtcaaa acaggattaa aaaagctcgt gccttgttcc ctcgtcctgc tgctgggccc 180
```

ctcagaccaa ttgtgcattg ccccacagtg cgataccaca ctaaggttcg agctggcaga 240
ggttttaccc tcgaagaaat caggggagca ggtttgaatg caggatttgc tcgctcgatt 300
ggcattgcat tagatgttag acgtcgtaat aaatctgtag aatctttaca acagaatata 360
caaagattaa aggaatacag atctaaattg atcttgttcc caagaggtgg aaagaaattg 420
cataagggtg aagcaactga agaggaatgc aaggttgcat ctcaattaga aggcgtcgtg 480
atgccaatta aacaaacttc agttaaatct aaagtcgtgt cattctgaag atgaaaagaa 540
attctagctt tcccacttaa gaaaggtcgt ctgtcagcgc tggtggtttc gtgaaaagga 600
gtaaagatca ntg 613

<210> 1557
<211> 659
<212> DNA
<213> Ctenocephalides felis

<400> 1557

aatnntattc tgaatactat gttcgatttt agcgttttcc cggcgagctt gatataatat 60
taattgtttg gagtaaaaac aacaaatttt cgaggattaa tttatacaaa aaataattaa 120
aatgaaatta gttaggtttc ttatgaaatt atcacacgaa acagtgtcga tagaattgaa 180
aaatggaact caagtaaatg gaacaataac tggcgtggat gttgccatga atacgcattt 240
gaaagctgtc aaaatgacta ttaaagatcg agatccagta tttcttgata ctataagttt 300
gagagggaat aatattaggt actatattct cccagatagt ttaccattgg agaccctttt 360
gatagatgat acacccaaag ccaaagctag aaagaaggaa gcagctcgag gtggaatacg 420
aggaaggggt cgtggtcgtg gtggcccaga ggcggtcgtg gaggtggtag gggacgtgga 480
cgaagataat aatattttg attgtaagct attataatca ttgaacattg gctcatagga 540
aggctatcaa tttgatgtat aatgtatttg atcaacaaga atttangttg atccattttt 600
aaccaatttt nttgtggcta atcagtttga gtcgatcata ttctgatgtt atatgaaga 659

<210> 1558
<211> 564
<212> DNA
<213> Ctenocephalides felis

<400> 1558

gtactttgct ttcatttttt tcattatttt caagagcaat ggctgaagca cgcgaaagaa 60
ctttcattat ggtgaaaccc gatggcgtcc aaaggggttt ggtaggaaaa atcatcaaac 120
gattcgaaag ctaaagggat tcaaacttgt agcaatgaaa ttcatgtggc catcagagga 180
gctcttgaag aaacactatg ccgatntttc ttgccagacc ttntttnctt ggactagtta 240
aatacatgag ctctggccct gttgtcccca tggtctggga aggcactaat gttgnaaaaa 300
ctggtccgtg ttatgttagg agctacaaat ccagctgact cgaccccagg aaccatccga 360
ggagatctct gtgtggaagt tgccgcaata ttctccatgg atcagatggt gtagaaagtg 420
ccaagaagga aattgcttat ggtcactgaa aaggaagtaa tctctgaccc cagctcatga 480
atcatggttt atgaataatg gtgataattt ngattttgnn taanctgatt aaaaaaanta 540
tgtgaataaa aaaanaaaaa aaaa 564

<210> 1559
<211> 617
<212> DNA

<213> Ctenocephalides felis

<400> 1559

```
gtggntttca taggatgctc ttggggtttc cccagtgggt ccaccaatgt aaataatcga 60
atcgttggag gcaaagacac cacaattcaa gaacatcctt accaagtatc aattttgtac 120
aatgatgaat atcacagctg tggaggttct ttgatttctg aaaaatgggt tttgacagcc 180
gggcattgca tcgattcttt caaattctac atccgtgtgg gaagttctct tgaaggcgaa 240
ggtggagctg tgcatcgagc tctaaaacaa tatcgacatg aaaagtttga tgcaaaaact 300
gtagattatg attatggatt aattgagtta gacacaccgg tacaacttag tgaaaatgta 360
aaattagtca aattggctga acctggtgtt gaacttgaag aaggaactct actaaatgtc 420
acgggatggg gtagccgcga tccagcgcac tctcaaatag taactgacca tatgtatccc 480
aagaagttgc aaaaatacat tcagacaggt gatctcccat atatgttctg tntggtaaat 540
gcangannga agactgcatg gnacttggtg gcngtgatca atggatccat tggatggttc 600
tgagcttgtg gcctacc                                               617
```

<210> 1560
<211> 659
<212> DNA
<213> Ctenocephalides felis

<400> 1560

```
cttanatgtg tggaatttgt gactgtgatc ctgcacactt tggaagacat tgcgaatgtt 60
ctgctacaga tgtaacatca cacttagatt tggcgatggg atgtagaagg gataatacca 120
ccacagttga ttgctcagga aaaggaactt gtgtatgtgg tgtttgtgaa tgcgaacagc 180
gtgccaatat cgaagaacaa atctccggca aatattgcga atgcgataac ttctcctgcg 240
accgtcataa tggtatttta tgctctggtc cagaacatgg tgtttgtgtc tgcggtcagt 300
gcgactgtct gcccggctgg accggccctg cttgcgattg tagagatacc aatgctacat 360
gtatcgctcc aggatccaca ggcgaagaaa tgtgctctgg acacggagtt tgtgaatgcg 420
gagtttgcaa gtgtgatgtt gctgaggatg gcagatattc aggaagatct gcgagaagtg 480
tcctacttgt cagccgtgca ggagttcaaa gaatgtgtta tgtgtcaaat gtataaaact 540
ggccctcaca gaagagaatg cggaaattga catctccatc gtcaagatna gtgaactgtg 600
aagtaagata cattatgtct ctccacgaaa catgccattn atctttntca caaaagcaa  659
```

<210> 1561
<211> 662
<212> DNA
<213> Ctenocephalides felis

<400> 1561

```
cggaancttt aaaaaatgag tgctcctaca gtaacaattg cccagggcac tttatcagga 60
aaggttctgg ttaatgaaaa tggaaaagag taccatggtt tttgtggaat tccatatgct 120
gctgctccag ttggcaaatt acgtttcagg cctccacaaa aaccagaatc atggagtggt 180
gttcgtcaag ccactgaaca aggcagtgaa tgttcatcga aacatatgct tttgcaacac 240
cctataggaa ccgaagattg tctctttgca aatgtctata ttccccaaac tgatgccaaa 300
```

aagcctcttc ctgtcatgtt ttgggttcat ggaggaggtt ttgtcatggg atcaggaaat 360
actgacatgt atggtcctga ttatctcatg gactacgatg ttatcctggc accttcaact 420
atcgtctcgg agttctggga tttttgaatt tagatttgga agaatgtctg gaaatgtcgg 480
actaatggat caggttgctg ctctcaaatg gcaaaacaaa acattgcaag tttggtggtg 540
atcaaacaca ttctattttg gagaatctgt gtggtgcagc tacattatta gttgctgatc 600
tacagaggtt gtccaaaagc atgncaagtg aggcttaaat catgcttcac gcncactaag 660
an 662

<210> 1562
<211> 655
<212> DNA
<213> Ctenocephalides felis

<400> 1562

gaatntgcta ctttacaagc cgctattgaa gcaatcagtt caatgaacat gtttgaccta 60
ggaggccaat ttttaagagt gggacgtgct ataacgccgc cgaacgctct tatgggaccg 120
acaactggat ctatgatgcc gactgctgct gctgttgccg cagctgccgc cacagctaaa 180
attcaagcga tggatgcagt tgctagtaat gctgtcgctt tggggctgcc gagtttgagt 240
tcttcaccta caattccgtt atcagtgccg acgatagcgg tgccgcccat cgcaacgata 300
acgccatcag ctcccgttgc tatatccggt gctgttaagt taccaggtgt cgtcataccg 360
cgcaggtgtc gtcgtacctc aagtcataca gcctccagga attgtgacgc cgacatcgca 420
accagttata attccagtat cagtattacc taattgtgaa tcttctccga cgatgatatc 480
aacaagctca cctgcacaca gccgatcaca atactacaca gaacaatgaa gccagaatgg 540
ccaagaacnc acaagagact tnaaaaaact atagatgaac tgacccaact tacagacaag 600
aacatgtgct aaggnaaagn nncgcctatn tcaagntatg aagangatca ctnta 655

<210> 1563
<211> 651
<212> DNA
<213> Ctenocephalides felis

<400> 1563

ctnttcagat aattcttttt caccaaaaaa taatatctgt aatttacctc gttaataatg 60
gctgcttcag caattccctt tattattttc accgtccttt ggggtatagt gggtgtcgtt 120
ttacctttta tagtgccaaa aggaccaaac agggggattg tgcaagtcgt tttgatattg 180
acagcagctt gttgctggct cttctggctc tgctgctaca tggcccaaat gaacccccta 240
ataggaccca aactacacca gaacacgatt ttattgatgg caagagaatg ggaaaatcca 300
atcagcgatt tgtaatttag tcaaccaaca ttcctgaata tcttctgata taaaatttca 360
tactgtgtga tgatgataaa tgaagataat atgatttaca tttcacataa atgtgtacat 420
ataattttaa gaacaagtta ttaatttgcg tgattgtata tagatatttt atgagaacta 480
ttcgttgtag caaatatatg tgtcctggga gaaaaatnnn nnnnnnnnnn nntnnttnnt 540
ntnnntnnnn tggggggggc cggccccatc ccntaagggg ggtttaaata ncggcgggtt 600
tnnccgggga tgggaaacct ggttnccaat antgcttgan antcccnttn c 651

<210> 1564
<211> 664
<212> DNA
<213> Ctenocephalides felis

<400> 1564

```
cgaataggtc cgttttaaat tttataaatt aaaaaatata tcaaaatgaa aagttttgtt 60
ccctgtgatc caggatgtga ttttcccatt caaaatttac catacggcgt attttcgaca 120
ccggcaaatc ctgtgcacag aattggagtt gccattggag ctctcgtatt agatttgagt 180
ggagtattgc atttttcaa accagaatat cagaatgcgc ttagtgccac aactctcaac 240
ccattgatgg gcctagaaaa atctgattgg aaacaaatta gggagaccat acaaaaattg 300
ctgttggaag gttcggaact tcacagaaat gaagaactcc aacaaaaagt attgcttcca 360
caagcgtcat gcatcatgca cttaccagct actattggag actataccga tttctattct 420
agcatacatc atgccaccaa tgttggaaca atgtttaggg gaaaagataa tgctttaatg 480
cccaactgga aatatttncg gtggatatca cggtcgcgca agctcagttg tgctctggac 540
tgtatccgaa gaccatggga caactttaca gtgtggactg acccatttgg ccttnagnct 600
atgatttgac tgaagngant tttgagtgca agcacaaatg gtcaagagcn gtgcaatcta 660
nann                                                              664
```

<210> 1565
<211> 664
<212> DNA
<213> Ctenocephalides felis

<400> 1565

```
gtantttta cggccacttc agcttcaccc attaaaaatg aagatttggt ttggatggaa 60
agcggaaagt tcgaaggtga catggtctta aatcaggaac aaatgttatc agttttagga 120
cttgggtcta aaaatggcct tatcgacaaa aaatatcgct ggcccaaaaa cgaagtgcct 180
tacgttattg taggaggata cttcaatcga agtcaaatta attacattca taaggctgtt 240
gcagaattta gaaacatttc ttgcgttaaa gttagaccca aaacagttac ggacacaaaa 300
tatgttcaaa tcacgggtct tccaggcggt tgttattcta gtgtcggatt ccaagatgga 360
gtccagaccc ttaatttagc accatacgaa attgagaaag ggtgcttccg taaagcgact 420
attcagcacg aatttctgca cgctttaggg tctatcacca gcaatcgact cacgacaggg 480
acgaatacgt gaccatcatg tgggacaata ttttgcaaac actgaccact taacaaatat 540
acggtagtcc gtcaggattc gaaccggtat gctattggcg tatgcntacg ggcttcggtc 600
tcgaaacgng aagacttcgc acaagancac gtgnagatnc atggcaaggt gagatatgan 660
tcat                                                              664
```

<210> 1566
<211> 662
<212> DNA
<213> Ctenocephalides felis

<400> 1566

```
atttttgcca aacacgatca atctcaagct attntcaaca gacacaagga tgttttagcc 60
agatggcagc gtctcctggg agactcccgt gctcgcaaga cccgtttgct ggacatgcaa 120
gaacagttca gacagatcga ggaattatat ttgacattcg caaagaaggc atcagctttc 180
```

```
aattcctggt tcgagaatgc cgaagaagat ctcacagatc ctgtcaggtg caactcgatt 240
gaggaaatcc gtgccctgag ggaggctcat gcacagttcc aggcttcttt gtcgtctgca 300
caggctgatt ttgaagccct ggcagccctt gatcgccaaa tcaagacgtt caatgttggc 360
cgaatcctta cacctggttc accatggagg ctctcgaaga cacctggagg aacttgcaga 420
agataattgc tgaacgtgat actgaacttg ctaaagaagc tcaacgacaa gacgaaaatg 480
ataaattgag gaaagagttt gcaggaccca tgctttcata atggtgccga gactagaact 540
caatgatgga aggtccggtt tntcgacaga attggagncc ttcnagaagg tacaaagttc 600
ngccaaagag tgttgagcga atgaaaatgg tntnctggag acttnttgna caattcnact 660
tc                                                                662
```

<210> 1567
<211> 648
<212> DNA
<213> Ctenocephalides felis

<400> 1567

```
atttttttca tagttatgca tcctttatca ttaggattaa tactaattat ccaaacaatt 60
tgaacaagaa tatttattgg tttaatttca aaaacattct gattntcata tattttattt 120
atttcattta ttggaggaat attaattcta tttatttata taacaagaat tccaaatact 180
gaatattttt ctttaaatat tnataaaact atttttatta ttttattatt aacattaaga 240
attattattt attataataa aaattttcta ttaattttta acaataattc catatttaat 300
gattttaatt caaacttact tatatataat tttaatctaa ataaattata taattttcca 360
aataatttat taactattat attaattatt tatttattaa tttcattaat ttgngaggta 420
aaattcagat atttttttatg gcctttacga aaaaatttta aaaaaaaaaa aaaaaaaaaa 480
tcgngggggg gccgggccca atcgcctaag gggtcggttc aatcctgggc gggttacacg 540
cggctgggaa cccgggttnc cacttatcgg ctgggncatc ccttngcagt gggtatagcg 600
aaggcccccg cnccttcaaa gtgccgctga tggaagnaat ggagcgtn             648
```

<210> 1568
<211> 661
<212> DNA
<213> Ctenocephalides felis

<400> 1568

```
caggnttcca caacttgtca ttaatatctc ggtctcttca ctgagacggt aaaagaattt 60
caaaatgggt tacttttatt gtatatcact tttatgcctg ttcactatag ttagctgtaa 120
taccggatta gatcctaaag gtccaaaagt tactcatgag gtatactttg acatcagcat 180
tggtggtgaa cctgcaggcc cgtgttttga tcggcctttt cgggggcact gtacccaaaa 240
cagttgaaaa tttcgtagaa ttgtccaaga aacctaaagg tgaaggatac aaaggcagta 300
aattccacag ggtcatcaag gacttcatga ttcaaggtgg tgacttcacc aggggagatg 360
gaactggagg ccgtcaattt atggagaacg ttttgctgat gaaaacttta agttgaagca 420
ttatggtgct ggatggttgc catggcaaat gctggcaaag acctatggat ctnaattttt 480
atcctacaaa ccctgctggt agatgacgtc cgttgattcg gaaagtatca aggaatggat 540
gtgngaggaa atgatcacat cacagattca aaacaacccc actgtgtgaa tttagacttg 600
ngtnactgtt ntaacctcgg gggtaagaat cactaataaa gattntcttt gtatttttat 660
n                                                                 661
```

<210> 1569
<211> 941
<212> DNA
<213> Ctenocephalides felis

<400> 1569

```
gtcntttgcg ttgaaagctg aaacatttct atttttatct cgaattgcac aagcatttat 60
ttagtaactt taattagtgt acagtattag caaacaaaat atgatgaaca ttcaaaaaat 120
tgttaggagc ttaaaccgag gtggagttgc aagaatgtca actggacagt acggtgatgg 180
tgctggcaaa ggaggtggtg gtggtggatc catccgtgaa gcaggtggat ctttcggcag 240
aatggaagct gctagagaag aagaattctt ttataaacag caacaagccc aattgaagaa 300
acttaaggaa caagcaatag atcagaaaac tttccatgag gaacaattaa aacttcacaa 360
ggaggctttg gagagacatg aaaaacattt ggctgagctt aagaagtaaa ttgaagtttc 420
atatatagca ttatgaaaat gttagacagt gatnaaaatt caaatatttg ntctattact 480
gaattaataa acttgcatta agcttctata agttattagt tttctagcat cttaatatac 540
tcattgncac tacatatagt tttaaattca ttggngctat gcatgtattg gactaaatat 600
tggaaataat ccataacaat ttttttgnga aaaaaaaaaa aaaaaaactc ggngggggggg 660
gcccggaccc caattcnccc tntaggggng tcnattacna atcactgggc cgcggtttta 720
caacgtnngn gactgggnaa accctggggg tancccaact tantcccttg gaaaacantc 780
cccttttnnc aagtnggggt aaaaccnaaa aggcccnncc cattnccctt tccaacaatt 840
tgcccccctg aantggggaa tggcaattng aacctttttt tttttgtaaa attccgttaa 900
nttttgtaaa ancntntttt ttaaccanan gcccnaaang g                     941
```

<210> 1570
<211> 931
<212> DNA
<213> Ctenocephalides felis

<400> 1570

```
aagatttaga cctagacgat gtaatcctca taggaaagac acaaacacaa tacattttaa 60
taatttacat cgaaaaagta agttttatag aacggccgct tacactttac acaaccaaaa 120
ttaatctgct gaagaatttt acgaagacta attagtttta aatctacagt atggatgtgt 180
tgaataggcc cgctcacgaa tttggaaatg atgaaacagt tgaaacttta tgggctatga 240
aagccatgga tcatgttatt gtctatttta atgtacattt attaaaatat ttatcttcaa 300
cttataggca atattcagta cagacgtgcc ttatttcaga tactttgctc tgtagaccca 360
aaatttctca aaatgtgccc ccaagatgaa attatctacc attgttttcg tcaagaattt 420
ccagatatgg atgttaaagt ctagatgaaa attcattaaa aggctataca ggaaaatgcc 480
gatggagaga atttgtgaa cgattcaaac atatagaaga ttcagttttg gtccttaata 540
cgcttagatt gcacattgga ttatagtcca gaaaatacta ttttagttnc aagagtcagt 600
ttatgccata gaatctgctc gaaataaaga aggccttaat gactgnatca gaaaaaaata 660
tgctaatgta agtgatgtgc gagtaatgaa aatgtaaatc catagaaaat aattagcatg 720
ggtatggtta aaaagtncat gaaaaaaaag nggggtttta tntttttttn tntttaataa 780
angatttttt caaaaaaaaa aannnnnnnn nnnnnncctt gggggggggc ccgggcccaa 840
ttccccctnt anggngnggn tttnnaaatc actgggcggg gttttnaacg gngggaangg 900
gaaaaccctg gggtacccaa ttaatncctg n                                931
```

<210> 1571
<211> 942
<212> DNA
<213> Ctenocephalides felis

<400> 1571

```
ttttaaaaaa aatgacgcgt ataatacata cgtcagccgt tttattatta gcattatttt 60
acctctcgag ttgcaataat gcagaggcta cagaaaaaca aataccgatg acgaaaatca 120
gtcaaaacct nggcggaccg acaatgaagt tcctttactg ttattcctgt ggttacagga 180
aagtctttga agactacatc ggcatcatcc aacaaaagta ccccgaaatc aggatagacg 240
gtgccaacta cgacccccca ggcctgtaca tgtacctagc aaagttccta agtctagcca 300
aaatggcttt aatcatctgc gtcttgacca gagtcaatct attccgctac ctcggcttag 360
aagagcccac gtggtggacc tggtgcctcg agaacaaaat gtactcctgc atgatgttct 420
tcttcttggc aaacacgata gagggacagc tggtgtcttc aggtgcgttt gagatttctt 480
taaacgatgt ccggttggtc ccaagttgga aacgggaaga ataccttnac ccccagagtt 540
gttcagatta tagataatca tatgaatatg gttggctgnt aaagtcgant ttaaaccgga 600
gttttgngaa atgaataatt atgggttaat aacaccgttt gntattttga atttttgggt 660
aaaccaaacn aaatngnaat tttttctagg gttggtttcc attccangga ttntaaatta 720
aaacctgnna ccttcnntan tatatatatt tgaagangng nttgatgnag aaattatttn 780
attaccttta atggatattt aanaaaaaan gggggtataa attttanttt ttntttggga 840
aaancnanat annanantnt ttatngnggg gggggncccg nccccatttn cctnaggnng 900
cnatttaaat ttatnggcnn ngnnttaaac ctgggngtng gg                    942
```

<210> 1572
<211> 918
<212> DNA
<213> Ctenocephalides felis

<400> 1572

```
atgtnttcgg gcatttagtg ttttgaagta tcgtggcata aagcagattt aagttaatct 60
gcttcattca atacacaaca tggtagggaa acccaacaag atgtatgttt ttaagcgaga 120
tggccgcaag gaagaaattc attttgataa aatcacttca aggattcaaa agctatgtta 180
cggattgaac atggattttg tagaccctgt ctcaataaca ttaaaagtca taaatggatt 240
atatactgga gttacaactc aagaacttga taatctggcc gcagaaactg ctgctaccat 300
gacaacaaat catgctgatt atgccgttct tgcagcacga atagctgttt caaacttgca 360
caaagaaaca aagaaacaat tttcagatgt catggatgat ttgtcaatat gactaatgag 420
tatactaaaa agagatcacc aatgatagca gattatcatc ataaaataat aatggataat 480
gcagatcgca ttaactctgc tattatttat gatagagaat tcagntacaa ttactttggg 540
ttcagacatt ggagcgtctt atttantaaa aattatggga aaagtagttg gacnacccca 600
catatgctaa tgcggtagct ntttggaatc attggggaaa acnntggtgc ngcaattgat 660
ccctttactt antttctgga aaaaatttta ctcatgccna gccacatttt ttgttgtncc 720
ccnccnanct caattatnaa gtggnttttc ttgctttcca naanaangtt ttganggtt 780
ttgntcttta aaccaagngg nttttattc naaaatgggg ggngggtttg gngaccccctt 840
tttggttttn gggaaaaacc ttctttgggg gacccaaggg atttttaagg gnttngtcnt 900
tcnccggttt naaanaan                                               918
```

<210> 1573
<211> 922
<212> DNA
<213> Ctenocephalides felis

<400> 1573

ttatttgtga aataaattta ttataaaatg ttagatctat ttacaatatt tagtaaaggg 60
ggcatagtgc tctggtgctt tcagagcact agccacatat ttgcaccatc tgtaaatgca 120
cttattagaa gtgtcatatt gcaggagcga tctggaaaaa atacctacga ccacgattcc 180
ttaacattgc aatataaatt agacaatgaa tttgagctgg tgtttgtggt tgcctttcag 240
aaaattttgc aactgtcata cgttgataaa tttttaaatg atgtacattt agagtttaga 300
gataagtata agaatgactt acaaaataaa aggtattttc aagaatttga ctttggtgct 360
tgttataaca gtattctgcg ggcggctgaa gaatggggtc ggactcaggc taagttacct 420
aaacaaatgc gttcttttga agattctatg aaatctaaaa agacagttgc ttcaatgatt 480
gaaagaaagg gcgagggaaa aaaagacccc agaagaatgg taaacaaaaa ngcaaaaatg 540
gttggtttga tgaagaaaaa attaatactt aaactggtat tggacccgng ccaaccaata 600
attccaaaag nggtgatcca atgattaatc atgccaaatc gatgggaact tgcncaaaaa 660
atgggggcct nttaaaaaaa aaaaaaaaac ttgngggggg gcccggnccc aattcccctn 720
tagggaggcg antacaaata actggccgcg gtttanacgc gggaatggga aaacctggng 780
ttncccannt aatngcttgg aganattccc ttttncaagt ggggtaaaaa caaaaggccc 840
caccnatgcc ctttcnaaaa ttggccncnt gaangggaaa ggnaaaatnt aacggtaant 900
tttttaaaaa tccggtnaan tt 922

<210> 1574
<211> 943
<212> DNA
<213> Ctenocephalides felis

<400> 1574

attgtntgtt atttagtttt taatgtcatc gcaaaatgat gattcaacgc ggtctgccgc 60
tgcaaacaat gtggagccta atgaaacaga tgggttgtct tcagagcgcc cagaacaagg 120
tcaatctgag agtagtatat caaacttgat gaaacatttt gctaacaaat taggtttaaa 180
tgaaaaagat gaaagtgatg aagatgaacc acaggtttta tcagaagtta caattgaggg 240
tgtggttgaa tatattaaaa gtggaaaatg taaaaatata ataacaatgg ttggagcagg 300
aatttctacg tcagccggta taccagattt tcgatcacca aactctggcc tttatgataa 360
tttacaaaag tataacctgc ctgatccaca agctattttt gatattgatt actttagtga 420
aaatcccaag ccattttttt ccttagcaaa agagttatac cctggtaatt ttcggcctac 480
aatttgccat tattttatta aactattgaa tgaaaagaaa ttggtattaa gacattatac 540
tcagaatata gatactttgg agagagtatc tggattagat gaagataaat ggtagangct 600
catggttcat ctacaaatca catgtatcgg tgnaggaaag aatatnccct gggtggatga 660
aaaaaaaatn ttttntgatg agaattccac ttgnacatgc cggagaaagt ggtcaagcct 720
ggatataccg tctttggnga aaatttgcca naaaggttca ttggctaata aaanaagact 780
tcacccaaag ngccttggtn aanaatcatg gganccgctt tggtggtcaa acctttgctt 840
ccttggatgg antgggattc ggaacntggc ctaaactttt gattaaatta aaaaaaangg 900
ggggaanggg gntaaaaggt tccaatggta ggtttnttaa ang 943

<210> 1575
<211> 931
<212> DNA
<213> Ctenocephalides felis

<400> 1575

ccgacgttct aagcacggtc ggggacatgt gaaccctgta agatgcacaa attgtgctcg 60
atgtgtccct aaagacaagg ctatcaagaa gttcgtcatt aggaacatcg tcgaagcagc 120
tgctgtacgt gatatcacgg aggcttctgt ttattccgct tacgtcttac ctaagttgta 180
cgctaagtta cactactgcg tttcctgcgc tattcactct aaggtagttc gcaatcgctc 240
taaggaagat cgtcgcatta ggactccacc agtgagatca ttcccaagag ataatcaaag 300
gcaacaaaat gctccaagaa agtaaatgtt catattttaa ataaaaaaaa cgacaactta 360
aaaaaaaaaa aaaaaaaaaa aaaaaaannc ctngggggg ggcccggccc caattcnccc 420
tatagngagt cgtnttacaa ttcactggcc gccgttttac aacgtcggga ctgggaaaac 480
cctggcgtta cccaacttaa tcgccttgna gcacatcccn tttcgccagn tggcgtatag 540
cgaaaaggnc cgcccgatcg cccttccaac agttgcncag ccttgaatgg ngaatggcaa 600
attggaagcg ttatatttng tnaaaaatcn cgtnaaattt tgtnaaacaa cttntttttt 660
taaccaatng gccngaantc gggaaaatcc ttttaaatna aaanaaaaan ccggataggg 720
ttggggttnt tccctttngg aanaaanttc cntttttaaa naacgggggn ctccacctna 780
aaggggnaaa acctttttta nggngaangc ccctcgggna ccntnccccct aatnaatttt 840
ttngggncng gggcccnaaa nccttaaatg gnancctaan gggncccccct ttaaaatttg 900
cngggnaaac ccgcaaccgg ngaaaaaagg a 931

<210> 1576
<211> 907
<212> DNA
<213> Ctenocephalides felis

<400> 1576

attgnttaaa ttaaactgtt gtcatttaag ctttaaatta cacactgaca acggagaagt 60
acagtttatt tcgcaatcgt tatgttaacg ttttctctta catgtatgaa agttatttag 120
ttataattaa aaatggctct gcaaagcttg aaaggttttt ctaaattgtt aaacaaatgt 180
tcgaatcgtg tttcatattt aactgtaaga tgtgcatctc aatattatga catagatgat 240
aacatatttg gattaaatga ggatcaggtt cagttgagga agaccatttt cgattttgcc 300
cagaaagaat tagcacctaa agcagctgaa attgataaaa ataatacctt taaagaactg 360
agagagttct ggagaaaatt aggagacttg ggaacattag gaataactgc tccagcagaa 420
tacggtggaa cagaaggcac atatttagat catgttatta ttatggaaga attatcaaga 480
gcttcgggag ctattgccct ctcatatgga gctcattcta atctagcaat aaatcaaatt 540
cgaaggaatg gaactgatga gcaaaaagcg aaatatttgn ctaactttgg tctggtgaac 600
atatcgggct ctagcatgtc tgacctggtc tggacagact agttctntga actaagagcn 660
gagaggaagg ngattttttg tttcaaggga ataatttggg atactaaggn ccaannctga 720
acttaanggt tatgccctnc catccaatgt aaagcctcac atggaattcc gcntttttgg 780
anaaaaaggt ttganggatt aatnctggcc aaaattggtt aacttggaan ggaggtcaat 840
tccngtgacc tgntttcaaa actgaaggtn cnccccnaan tttttngnga aaaggnaang 900
ggttttt 907

<210> 1577
<211> 917
<212> DNA
<213> Ctenocephalides felis

<400> 1577

```
tttattgcat caggcgacaa ccatcttttc atagtgacag agttcatgga aaacggttcc 60
ttgagaagct ttctacacaa aaatagaaac agattgacca aaaccgatct tttagacttc 120
tccaaacagg ttgcaagtgg tatgaagttt ttggaagagc gtatgtatgt ccatcgagat 180
ttggctgcca ggaatattct ggtggacaag aaattcggtg ccaagatttg cgattttggg 240
ctcagcagga ttataaagga cgatatgtac aaatcgactg gttcagcatt tgctgtcaag 300
tgggctgctc ccgaatctat agagtatagg aattttacgt ccaaatcgga cgtttggagt 360
tttggaattg ttttgtatga aatctatacg ctgggcaagg aaccttaccc ggaattgcag 420
aagaacggcg aacttataga catgctgaga aatggttccg gatgcggaaa ccggaattgg 480
ccggagttga aatttacgaa ctcatgaccg atgttgggaa ttcgccaaga atccagcccg 540
acttttgccg aagtctacga actgatcaac gaagttacga aagtcgatta tccgaagccg 600
aagtcgtcga agaactaaat taatgaatag ttgcnagttt tggccatntc ttacctntaa 660
aaactaatta ttttttattt tagtttggaa aaaaaaaaaa aaaaaaactn nngggggggc 720
ccgnacccaa ttccncctta nggggccgtn ntncattccc tggccggtgn ttnaaacgtc 780
gggnttgggn aaaacctggn gttcccaact tantcgcttg gagaaatccc nttttgncan 840
ttngggtana aacnaaaagg ccccnccgtc ccttcccaaa tttggccnct naatgggnaa 900
ngcaaattgt accttnt                                                917
```

<210> 1578
<211> 939
<212> DNA
<213> Ctenocephalides felis

<400> 1578

```
ttttaacgct cttataaaag aaattgttaa atctattgaa atccttattc atcggtgcct 60
attttcggct taaagatggt tcaaaagaag cctaagaaga aggtcggaaa gaaagtggca 120
gccgcccctt tggctgtgaa gaagtctgaa cccaaaaagt tggttaatcc tttgttcgag 180
aagaggacaa ggaactttgg aatcggtcag gatatccagc caaccagaga cttatccaga 240
tgcgtgagat ggcccaaata catcagaatc caacgccaaa agagcgtttt gcagaagcgt 300
ttgaaggtcc ctccaccaat caaccagttc acgcagactt tggacaaaca aacagctaca 360
cagttgttca agattttaga aaagtacagg ccggagactg ctattgctaa aaaggcacgt 420
ttgcaagcac gtgctgaagc taaagcccaa gggaaggaag acacacctac aaaacgtcca 480
aatgttgtac gttctggcac aaacactgtc acaaaacttg ttgaacaaaa gaaggccact 540
tgttgtgatt gcccatgatg tggacccccct tgagttggtg ttgttcatgc cagccctatg 600
ccgcaaaatg ggtggtccct actgcatagt gaaaggcaag gttcgtcttg gagcctggtc 660
cgcaggaaga catgcncatg tgtagcacta accaatgttg acctggcgac aggagtgggt 720
ttgaataagt tggttgaggc cgcaagacca cttcaccgat nggcccgatg agatcaaaag 780
gcnttggggc ggnggnggtn tgggatctaa aacccttgcc anaattgcca agttgganaa 840
aatcaaggcc cgggaaatgg cncaaaaacc gggttgatna atattgattg ganaaataaa 900
gntttttttgg tggnaaaann nnnnnnnnnn nnnnnnncn                      939
```

<210> 1579
<211> 919
<212> DNA
<213> Ctenocephalides felis

<400> 1579

```
atntttattg catttgttgc gctcttcact ctgcccaaag tttatgaaac taacaaaaca 60
caaatcgatg ccaatctcga tgtcgttcga agcaagttac aagaaattac ttccaaggta 120
aaagcagctg taccaattgg aaagaaagct gaaagtgata aagataaata agcacaaaaa 180
attatattta tataataatg tggatattta attgtatagc gatatacata actcttcaaa 240
cataaccaca ccctttttaa caataattaa atcaactaaa tatgaaatat atattatata 300
tgttcgattg tttggacata tcgaaagcta tatgtgaata ttgcaaatcg aattgatttg 360
tagtatatat ggaatcaatt tttttcatg atgaaccaat tcgatagatt tatagaaagc 420
ctttttattt taaatattta catttgacac taaaaatagg tatcttataa aaaaatttca 480
tttaatacat ataatatgtc tcatatataa tatttattat acattctttt aaagaaatat 540
gttaaaggtt tgnaaaatat tactttttag tattaggttc tattcaaaaa ttggttgggt 600
ggaattattt ttggnattgg cggtaaattt tatttttaaa taatatgcaa ttctcatcct 660
tgnctttaag aataatnttt taaatatgga ttggtnaaaa gtaangtatg aattttactt 720
ntcgcatcaa tncaggtatc attaaatttn ttttaggagt ttaaaaaana aaangcncnn 780
gnnnnngnnn nnngnntnnc ctgggggggg ggcccggncc caattnccct nagngggggn 840
ggttaanann cnggccgggt ttnnaacggg ggnngggnaa accnggnttc cccaattaan 900
ngctgtggaa aaaccccct                                              919
```

<210> 1580
<211> 935
<212> DNA
<213> Ctenocephalides felis

<400> 1580

```
ctttnctagt tattaagata acatnattaa aannacttat taacaataat gtcngctaat 60
ttaaaaacca tatacgcttt tgcgcgagag atgcattcca aaaatataaa taaacctata 120
caatatggta cagctggatt tcggaccaaa gctacagact tggactatgt catgtttcgt 180
atgggggttc ttgcagctct tcgttcccgt gttaaaagca cagcagtaat tggattaatg 240
ataactgcat cacataatcc tgaagaagat aatggtataa aactcgtaga cccccatggt 300
gagatgcttg aacaaagttg ggaagagtta gcaaccactt tagttaacgt tagtgattct 360
gagttagagg ctacagttga aaaaattgta aaagacttga atatcgatct taatttgaag 420
gctaatgtgt ttattggtat ggatacaang tatagtacgt ncccagtctt tgcaaagctg 480
ctgttgatgg agtattatct atttcggggg atctcccaga gaattttggt attggtaccg 540
actcccaatg cttcactatt ttgttttntn tgcaaacctg atcaagccta ttgggaaaac 600
ctactggaag aangntttta tacccaattg gataacttgc ttcaanaacc ccttcgggcg 660
angttncatt tcaatgggaa agnattncnc caaaattttn ntttgatngg ngccnatgga 720
ntgggggca aaaaangntt caatttnaaa aagacnggng gangtttgat ttgtttttta 780
acaancggcn aaggaaaata aatnttaggn gnggggctnt ttttggaang acaccattcn 840
cnccccaagg antncnntta aaaaantttt canngngntt tttgttgaaa cccaancgna 900
tgnttccnnt tnnggananaa aaaaggtttt antgc                              935
```

<210> 1581
<211> 920
<212> DNA
<213> Ctenocephalides felis

<400> 1581

```
tttnatagtg atgcatcctt tatcattagg agntaatact aattatccaa acaatttgaa 60
caagaatatt tattggttta atttcaaaaa cattttgatt ttcatatatt ttatttattt 120
catttattgg aggaatatta attctattta tttatataac aagaatttca aatactgaat 180
atttttcttt aaatatttat aaaactattt ttattatttt attattaaca ttaagaatta 240
ttatttatta taataaaaat tttctattaa tttttaacaa taattccata tttaatgatt 300
ttaattcaaa cttacttata tataatttta atctaaataa attatataat tttccaaata 360
atttattaac tattatatta attatttatt tattaatttc attaatttgt gtagttaaaa 420
ttacagatat tttttatggt cctttacgaa aaaatttttta aaaaaaaaaa aaaaaaaaac 480
tcgagggggg gccccggncc caatcgccct atagtgagtc gtattacaat ccctggccgn 540
cggtttacac ggccgngact gggaaaaccc tggcgttacc caacctaatc cgccttgnca 600
gcacatcccc tttcgcagct ggcgtaatag cgaanaaggc ccgcnccgtc gnccttccna 660
caagttggcg ccaccggaan ggggaanggc aaatgnnagc gttnatnttt tggtaaaatc 720
cggggtaaaa tttgntaaaa ncgnccattt ttaaccaatn ggccggaatn gggaaaccct 780
tnntaatnca aagnattgnc cgggntgggg tggggngtgg tcngttngga acaaagtccc 840
ttttaaggaa cggggncccc cgnnaagggc aaaaaccngt tttagggggg nggcccctcc 900
ggnacccccc ctaanaggtn 920
```

<210> 1582
<211> 904
<212> DNA
<213> Ctenocephalides felis

<400> 1582

```
acagatacgt agctgttaaa tgtcattgcg ttgctatttt tgattatttc atcagtattt 60
caataaatcc aacatatgtc tcggcacaca tcattataat taaattatta gtttcatttc 120
cattaattta ttttactggc gtcaatatga cggtatcaaa cgacgaatta gttgaatctt 180
ttaaagcttt aggtttaagt gaacaaaagg cgaaagaaac gttaaaaaat acagttgtta 240
cgaaaaattt aacattagca ttacatgagg tgaggggcat tattttgccc caaggagctg 300
gttctttaat ctattatgtg gcaacaaaaa tcaaaccaca gattatagat caattgcctg 360
tacttgtaaa atatatatca acatcaaaat tagacacaac agttagagtt gatgcggcct 420
tgcaatttat gttgtctcat ttaaatggat atcaaatcga tgaatttgag aaggcttgtg 480
gaattggtgt tgttgtacac ctgaacaaat tgaaaaagca gtaaatgagg ctatgttgga 540
acataaagag gcaattcttg agaaacgata cagatacaac actggtcctt tgatgcaaag 600
tgtaagagtt ctttaccctg ggcagatggt cggctataaa tgtgaagtgg acttacaggt 660
tttgacttat tggggnccaa aactggtgcc gattancacc ttggcaaaag tggaaagaaa 720
gtnaagntca cnccagtgcg gataaaaatc ngaccgaaaa gaagcccgag gaagtggatc 780
aagcagcttg gggccatgtn tttntgaatg atgaaaaaaa agnccttta tgcctctggg 840
naacctttaa aacnggggga tgagtacccc cctcccatga ctttnaaaaa cntttnaana 900
aang 904
```

<210> 1583
<211> 907
<212> DNA
<213> Ctenocephalides felis

<400> 1583

gggantgtta aatcgccgta ggtgctcgca tagacaaata gttccttttt ataatatatt 60
gtaatgaaac atttctcact caaaataaaa atactaagta ttgctgcgta aaaatatttt 120
ataagggcgg tcaaactagt ctctcataat ttgatttatc tcagaagcca attatttcgt 180
atatgatttg gcgccaattc cattccccag accgcccgtc aaggaatacg tcagtgcaca 240
tttgctgtgt cgtatttgtt gtttacttgt gttgtgtccg gataaataaa gtgcttgtga 300
tttaagtaaa atggctttaa atgatgataa tgaggaaaat atagaaaaaa gtcataaaga 360
tttgtgttta gaattaaata tggacacgtc tgcagctact gcttcgtggg attcttacaa 420
ttctattaga gaaaattgtc attagaggga aacccgaagc actggttatg ttgctcatta 480
tatgtagcct gtcgacaaga tatgactccc acagtgggtc aagtgaggcc ttagtagaag 540
gaaattgtgt cagtttaact agattattga gattgtgcaa tattagtctg gggtgagttt 600
ttaaaaaagt aagacatggg ctgaattgac aaatatgcct gaatcattat gagacgttta 660
gatggattag aaaaagttng ctgggctgng atttattcaa aagttcaagc ttattcgacc 720
actggttgng aaacccacaa gaaggttgag gatcaccagt ctcccaacna acaaaaattt 780
aaatctntca caagatatcc cagtctgtgg gtcttttggt gcatnaaagg aaacnccctn 840
ccaaagngan gattaattgc ctggccctct ttgntgggcc ggcttgattg gtatatcnaa 900
agccntg 907

<210> 1584
<211> 898
<212> DNA
<213> Ctenocephalides felis

<400> 1584

gttttntnct agactgatat atgcaaaaag tgtaaaaaaa tatagatgaa atgcgagagc 60
gtgattttgc agacatgtgc tgaacagtat actattgtgt ttagatctag aaagtattgc 120
gataagccaa tgttactaaa aaggcaattt ctttattttg aatattatct tatcgagtag 180
gtaataacaa aataatcaac aaacgtgaat taatgaagtg ataaaaaata ctgacaataa 240
tagtggctca aacggagatg cttagaagtt ctgtgaaaaa tatgtgatcg ttaatttgtg 300
catggacaaa ttttctatca aaatgaagac taaagatatg gaaatgaatt tagaggaact 360
gaaaaatgat aacatcaaga aacagcagga tgaaatcaat ttttcatttc gaagtattcg 420
ttatacaaga tgcgacagaa aagaaaaaaa atcagtgtgc atactgaaag gagtctcggg 480
atgctttaca tcaggaaaac tcgtggccat actaggtcca tcaggtgcag ggaaatcttc 540
tctgctcaat atactatcgg ggntcagaga aacaggccgt gcaaggntca atccgtctca 600
atggagccaa tgtgacatta gataaacggg tttaggaaag cctgttggta tatacccaag 660
atcngcatgc tggccactac caccgagaga actntctgtt gcagcagact gaaatgaaac 720
agaaatcggg taaaactgtg gagatgcatg caaaatgctt acttaccaat gttanaccnc 780
gattantgcn ttttggnggc aaaaaaanat ttcaatggng tcaatgggga aaatccccaa 840
tntgttttgg tgancccttn ggccttgaag ggttcgcctt tnactaaggc cctntttn 898

<210> 1585
<211> 912
<212> DNA
<213> Ctenocephalides felis

<400> 1585

tgatttagat gtcggcgaac tgtttagcgt caacattgta caattacagt aaatattgtt 60
taaaacctaa tctactatcg gccggaaagt cccatttaat attgactctt aatttttcta 120
ctacaatgta cagaaaagga aaatttgact ttcctaagag atttacagga caagaaaaaa 180
gtgtatgggt tgagtatata gaattagcta tcaaatataa gccattaaat ttgggacaag 240
gttttcctga ttatccttgt ccagaacatg ttataaaggc tttggcagat gttgcaactg 300
gacccgattc attattgcat caatatacaa ggggttttgg acacccaaga ttagtaaatg 360
cattagccac tttatatagt aaagtcttga atagatcaat tgatcctatg aaagaaatat 420
tagttacttc gggtgcttat gaagcacttt actcaacaat tcaaggacat atcgatagag 480
gagatgaagc tataattata gaacctttct tcgattgcta tgagccaatg gtaaaaggag 540
ctgaaggtgt ttgcagatac attgcattaa aaccatcttc tgataaatgc tctgatgctc 600
aaagttctgc tggattgggt tcttgataaa aaagaattgg aatcattatt caatggaaaa 660
aaccaagctt attattttna ataccctcac acccaacagg aangtattcc cttggaagaa 720
cttgaattta tgntgactat gtaaaaaagg atgtctttgt tatcggtgaa gttatgaatg 780
gatggngtta agctcatgac ctataaaatg gtaccttccg gatgggggaa aaactttact 840
ntggactgcc gnaaaacttt ntggtcaggg tggaantggg ngggcctttg gncctttgga 900
ttactaaaaa aa 912

<210> 1586
<211> 941
<212> DNA
<213> Ctenocephalides felis

<400> 1586

antcgggcgc ggccgcggtc gcgggcgtgg ccgaggcaga ggaagaggtc gcggccgagg 60
ttcctcaact cctgagtaca ccgtgtcctc cacaccaccc ggtggaggaa cgattccttg 120
agtgtggcct taccaatctc catatcacac cggcgaacat catggtcaaa cagactctcc 180
aatgggacct tcttgcagcg ttagtaatcc ggaagctggc tgctcgtgct cgcaccagtg 240
ccatcaacca gatgacgtgg atgatactgt ttggttttaa gctggtgtat atggatattg 300
gttaggtatt gaaatcttga ttcttatgtg aactcgagtt tatttaatta gaatattata 360
ttgtaatcgc attttatagg ctgtggagat aaacttcttg aataatatat taaatattgc 420
ccacatttgc tcataatttg taggagtcaa taacgcaaca gaatgacttt tgaataatat 480
attagtcatt gacatgagcc ggtgttttaa aaatgcttag tataatgggt aaattcttaa 540
tagtgaataa aaatattaga aaaaattggt ttctggatgt gattcaattg gatatattac 600
tattgaatag gtaaacaatg aaccttattg gatcaatgtg cgatatataa aatacacgtt 660
gttggaaata ttcatctatc tcttgataaa ctaacttttt tgctcacttc ttcgtacctn 720
aaaatcttgg ggngattttt taaaggnaag ttttgngctn tatggatttt taaacttgac 780
ttaaaaaaaa anaagtcnaa atggtttcct aatattcacc gnnccannaa anatagttct 840
aattattgng ttancatttt tttttaactt ggaggcgtat nttggggntn gggntnaana 900
nnttggaaat tnattttnaa acttttagtt ttaaaanant n 941

<210> 1587
<211> 925
<212> DNA
<213> Ctenocephalides felis

<400> 1587

tgtgacagca ctcaaacatg tgttttgtca atactccatc atcaaatatc aaggctataa 60
ttggaagctt tttcccggaa tcgtttgctg gcacgggaaa tgcgcataaa atgtatccca 120
tgagtgaaca ctttttcaca cttttggaag aaatgggtta tttgcatcta caagctacca 180
aaccagatac agtgggcgtt gctttaagag attcaccagc tggtttagca gcttatattt 240
tggagaaatt ttcaacatgg actaacagat cttggaggtc agttaaagat ggaaacttgc 300
tgttaaaata caatattcct gaacttttag acaatgtcat gatatactac gttactgatt 360
ccattactac ttcaatgaga ttatatgcag aatcattcac aaaagcacac cttgctttga 420
acttagatag ggtgcgcaat catgtcccag cagcctgcgc aaaatttcca aacgagttgg 480
cttatgtgac ccgattgcca acttgctgag aaatataaaa ctttattgca gtccaatgac 540
atgccaagtg gtggccattt ttgcagcatt tgaggaacct ggtctttaac agaagacatt 600
ttcactgcgg tgaaaaagtt taaaagaatt ttattccaaa aaagctgaag aaagccaaaa 660
gaaagctgat ttgggataat tttggtggtg gatatataat tatgctaata atatttggag 720
ataaatttaa cccattcatg gtcacatatn ttttttcctt cctccctttt tttaaataaa 780
aaaaaaaaaa actngngggg gggcccgncc caattcncct tntgngagtc gattacaatn 840
actggccgng ttttanacgg tnggactggg aaaaccctgg ggtanccact taatggcttg 900
gnaanaatnc cctttggcng tgggg 925

<210> 1588
<211> 892
<212> DNA
<213> Ctenocephalides felis

<400> 1588

tatattcgga tataatttct atgttttatc aaatatattg ttaaaaatgt tatagtgtta 60
ttgtaaataa ttagtcatta ataaaaaatg gctacatatg aagaatttat tcaacaaaat 120
gaagatcgag acgggattag gcttacatgg aacgtttggc cttctagtag gatagaagcc 180
actagattag tagtcccgct agcatgtctt taccagcctt tgaaagagag gcctgattta 240
ccaccaattc aatatgatcc tgtacaatgt acgagaaata catgccgagc aattttaaat 300
cctttatgcc aagttgacta tagagccaaa ttatgggttt gcaacttctg ctttcagagg 360
aatccgtttc ctccacaata tgctgcaatt tctgagcagc atcaaccagc agagttgatt 420
gcaagtttct ctacaataga gtacaccatc acaagggcac catgtatgcc tccaatattt 480
tatatgtcat gggatacatg catggatgat gaagaactgg gtgctttaaa agctctttac 540
aatgtcttta agttatggcc ccaaatctct tggtgggcta gtacattngg aaaatggtca 600
ggtcacgagt aggactgang tgtcaaagac ttgtttagag gcctaagatt aacagctaan 660
caatcaagaa agttaggcat tgtcggaagt gatncccaac acagaaaggc ccaatgccca 720
caataagcgg gccagccaat anatcatcca cctttccata aangcaaatg gntttaccgg 780
ccttttggga naacggnacc agaaccttgg cctgtncctn aagggaaggg ggcttacgtt 840
aactgggnct gatttttcga agcccgnggg ccttttgngn gctttttgnc cn 892

<210> 1589
<211> 928
<212> DNA
<213> Ctenocephalides felis

<400> 1589

```
tattcgtttt acaaatcagt ctcaaacgat aaacacaaag tgaacgtttt aaaacaaaca 60
taaaactgtg cctaatccca ttcagtgatc gaaaaaacta tataaaacca gttcgatagt 120
aataatacat aatttaccca gcaacgtgca ctgtataacc tacaaatcac gatttcgact 180
gaatgaaatt atggacgctg aagatttgaa tcagttctac aacgggcggg agatgggtgc 240
ttggcctccc aatgttggca tccttgccat cgaactgacg tttccgtcgc aatatgtcga 300
ccaggccgaa ctcgaggcat tcgatggagt gtcagcagga aaatacacaa ttggactcgg 360
ccaagcaaga atgggcttct gtggtgaccg agaagatatt aattcattgt gtttaacagt 420
tgttaaaaat ttgatggaaa gacatcaagt gccttatgat agaataggtt atttgggagt 480
aggcacagaa acccttctcg acaaatcaaa aagtgtaaaa tctgtcctta tgcaattatt 540
cgaacccact gcaaangtgt actgatatcg agggtgtaga tctacaaatg cttgttatgg 600
gggcactgct gcctttcaat gctgnttcct gggtcgaaag cagtgcttgg gatggcagaa 660
tggctttggg ttgttgctgg aaacattgca gtatacccaa nggngctgct cgtccaacag 720
gaggngcaag tgcttgaacc atgctggtgg gtcccggact tccnttgcga tgggacaaag 780
gcttanggct ttatgtntga nacatgctta ttgatttttc caacctggat tgggttcaaa 840
gnttccccgg tgganggnaa aataagtttt aatgntnttt ngggagcnct ggacccttgg 900
tttgctntta ctgggaaaaa agggcaag 928
```

<210> 1590
<211> 922
<212> DNA
<213> Ctenocephalides felis

<400> 1590

```
tggtggcagc agggaaattc aactgactct cgacggagaa ccgggggtca agtataacgg 60
agtctcggac tgggtttacg aagaggaaat gctgggttct ggcgatgcac tttggttttc 120
tccggacggt gcatatatcg cgatcggctg ctttgacgac tccaacgtcg acgaaatgat 180
gtatttccgt tacggagagt ctggtactat tgaaagccaa tatccggaat tggtagatct 240
gcgatatcca aagcccggta gagtaaatcc gacagccaaa gtaaaagttc tccgtctgaa 300
cgaagctcgt ggtcgtaata tgccctggac ggaactgaca gctcccgaac aggatgttgg 360
caaagatcat ctgctagccg ctgtcacctg ggcctctaat aacgaggtgg cagtcacctg 420
ctaaacagac gccaaaacta ttctgttatg caaatctgca atgcagggac cggagaatgt 480
aaaacggaac tagaaaccag catgcctggt tggatcgacg tacgtaaatt ctggtttact 540
cctgatggcg atcgttatat aactatcaga tctgtgcaac acacttgatg gattacatat 600
cctcatatag taggagcccg aaagaaggac ggaaccgaaa ccaatcccca gtgggaaatc 660
gtctggtacc aatattatag gttcgatgcc gaacggggat cttttntntt actcagcaac 720
attggctgga aacngtcccc ccaagaaatt ttggtngncc ggttccaaag cttgtccttg 780
nccgtcactt ntgacccaaa atgganataa tgcngatttg gtaaagngat ttaacnaana 840
gttnttttg cttgcttgat tggcttggac caacccnccc tttgcgtnct ttttgaccgg 900
ggaagatnaa ancttgcaac ng 922
```

<210> 1591
<211> 926
<212> DNA
<213> Ctenocephalides felis

<400> 1591

```
ctatttaaaa ttttgatatg caatttgtgt taaatgcgtt gtaaagaaaa ctatttatac 60
ctatttactg ttgtctttat tttgcttcat attgtatttt tcggtatcca taaatatcgt 120
tgtttgtgaa aatttgtgtg aaaaagtcta ttaagcatct tcaagtttgg tatggctaaa 180
cctcacctga agaaagtagc gttcctgagg acccgctatg tcacagcact aaaattaaga 240
tttttcttct ttgtgacaat accagtttgt tatttggtat ttacagcgtt aacaaaacaa 300
tccgtttctt ctgaagatat tgaatattac ccgcagacac cagaaattac tggatcgcgg 360
aaattgcttg gcctatcttt caccgatgcc aagaattcga gcctggcaga tgatcatggt 420
cataactgca cgcccgcagc tatcttagat tttccctctg atggattcac cagggagcaa 480
agaagacagg gctgggcgct ggtccatgcc gcatcgcatt ctactgcttc ttggctatta 540
gcattagttt gcgatgacta cttcgttccg gcaattgaga tgctgtgcaa aaagctagat 600
atgaaggaag atgtcgccgg actacatcat gggcgctgcc agttctagtc ctgaattatt 660
attaattcng naggacttca tacagaagga gacctcgggg tcggcncggg cgngggttca 720
tctgnattta atatactggc cgccctgctg tgnggggctc tttgtgggaa ggtagtanaa 780
tttaaatggn ggccagaacc tcgggatntg gcttgnttgg atttgccgaa ttggccctnt 840
tcctacnttt ntaanatggg aaaagtttgt gggttgaanc cctaacccta aanctggctt 900
atatnttttt ttngcgcttt gtnctn 926
```

<210> 1592
<211> 943
<212> DNA
<213> Ctenocephalides felis

<400> 1592

```
tttntataaa ttaatatatc gttctcgttg ttaccaaatt atattattta aattaagtga 60
aaaatatggc cagtgttgtg aagcagttgc caagaatatc cggctccaaa gtagcgaaat 120
gcatttcgag ccgtaattat tattcgtatg taaatgagcc tgcccaacaa atcaaagaca 180
aagaaccgaa atgggttact gccgaagaag ccgtaaaaat tattaaatca gatgactggg 240
tgtacgctca gggtgcggct gctaccccaa tcaacttatt aggtgcgatg actgcacatg 300
ggaagaagca aggcctgaag aatgtgcgcg tctgccacat gcacactgaa ggacctgcat 360
tatacgctca gcctgattgc gaagggatat ttagatctgt atcattcttc atgggaggca 420
acgtacgtgc agctgtgaat gaaggacgag gcgatgcaat tccaatcttc ctatcagaaa 480
tacctcttct attccaaaag aaaattattc aacctgatgt agccatagtt catgtttctc 540
ctccagacgc tcatggattc tgcagtttan gtaccagtgt tgactgtgcc agagcttgcg 600
atgcaagcat caaaagtgat aattgcccaa gttaatccta aaatgcccgt catttggtga 660
ctcattggtg cacaaaaagt cacatagata tgctgtanaa attggtgaaa ccgtggtacc 720
catggnggna aaccacctaa tganggaaga aactgcaatt ggngcacata ttgnnaataa 780
cttggnggaa aaaggngccc actttaaatg ggtntnggaa ccattcctga tgcngttntt 840
tgccaacttc cttaatcnca aaaatnttgg aattcatttt tgaaagtttg gccaanggnt 900
aatngcntta aaggaaagga nggngttgtt ccaaaaccta aat 943
```

<210> 1593
<211> 929
<212> DNA
<213> Ctenocephalides felis

<400> 1593

tcaaagaacg tgacaagaaa aatatgtgaa ttttgtatgt tacataataa tttttttatt 60
tcctcgagta tttgttaatt tgcgtgggct gaattttcgg ggcgaaactt gtgatatgaa 120
caaaattgag atggctgaag ctgcatcacc tagtacagaa atgccacact caggtggaga 180
accagggaca ccaatgggtt cagttgttgg tgcttcaggt gaacttgcgg ggctgagccc 240
agaagaagct gaattgaaaa gagctgagtg gagccaagag cttgcaagag tggaagaaga 300
aattggtact ctaagaactg ttttggcaag caaagttcga agaagtggag aactgaaagc 360
taagttaggt atcactgttt ggaaagaact aactggggat gtcaatcaaa gtctacgtac 420
tgttaaagag agtcaagtct atggtaatat tgaaaattgt attggacaaa taagtaaagc 480
ttgtgactag tgcacctatt taccagaaaa cagaattggt actgaaatct actgccgaaa 540
aaactacttc tttgttaggt ggaatactgg tggtttgaca tccaaaattg tcagatgaga 600
aatctgaatc tttcaaatca ctagaagaaa aaatgggttc agcctatgaa aacgttaaaa 660
caaaagttca tcatctcgtt ctaattctat tcaaaatttt gatgaagcat tgcgtgaagc 720
ttaacaagat tctgttcaac ttctactntt cctgaaaant caatttcaac cagtaccntt 780
tgnttcaaag ctcctcttgg ttntttatna aattttnaaa aattagtaac canggaaaag 840
tccggnccac ccgaagatgt tttttcctac caaacttggt tcacatactt tnttggaaat 900
cttttccatc caattcatta tttttnaag 929

<210> 1594
<211> 938
<212> DNA
<213> Ctenocephalides felis

<400> 1594

cgnttggctc atcgctactg ncgaatatna caacagtcaa cttaggttac aacttgtgng 60
accttcatca tcttgtacaa caatctcatc cccatatctt tgcaagtgac tttagaagta 120
gtcagattta tgcaggcgat ttttataaac atggacgtcc taatgtacca ttcagagtct 180
gatacaccag ccatggccag gacttcaaat ctaaacgagg aattggggat ggttaaatac 240
gttttcagtg ataaaacagg aacgttgacc aggaatgtta tgaagttgtc taagtgttct 300
attggtggaa ttatttattc ttgtcctgat tgcccggaag gcagctttag agatagtccg 360
gttgatggac ttgatattga ggatgctcag aaaagcgatc tggttcagaa tttactcaac 420
aatcatccaa cagtcgactt gcttaaagaa tttatgagtt tgctatcggt ttgccatact 480
gtgatacctg aaaagagcga ggatggnaaa atcattatca tgcatcatca ccagatgaaa 540
agactttagt nagtggtgca aagaaatacc gnttcttcat ttgaaacccg gncaccacat 600
caccgtaaaa antaaacgct tggggtgaaa ttttaaaaat tgagatattg gatggtctcg 660
aatttacttc aacaggaaaa gaatgtttnt tgnttggtag aacncccagg gccaattaaa 720
ntnttttggt aaaggaccag attcctggna tnttttgaaa ggttngccaa agaggacncc 780
aatttcgnga ngtaacnttg gaaanctttn gnccaatttg ntnagggggg ggcccggtcc 840
ccaattnccn ttaagggngg nggattacaa ttnntggccg cggtttcnac cncgggactn 900
ggaaaacctg gnntttccca cttantcctt ggangcct 938

<210> 1595
<211> 929
<212> DNA
<213> Ctenocephalides felis

<400> 1595

```
cttttcgatg ttgatgccaa aacagaaccg tgtcgcaatc tacgagcacc tctttaagga 60
gggagttatg gtggccaaga aggactacca tgcaccaaaa cacccagaat tagagcaaat 120
tccaaacttg caagttatta aggctttgca gtcgttgaaa tctagaggat atgttactga 180
acaatttgca tggaggcatt tctactggta tctgacaaat gaaggtattg aatacttgag 240
gacatacttg cacttgcccc cagagatcgt gccctccact ctcaaacgtc aaaccaggcc 300
cgaattggca aggccaagac cagctgccgg cccaaggact gaaggatctc gtccagctga 360
agacagatct gctaccgtag ggcacctggt gcacctggtg gcgctgacaa gaaggctgat 420
gtcggtgctg gcactggaga cttggaattc cgtggtggat atggacgtgg cagacctgcc 480
ctcaataaat ttatataaag taatttataa taaaatatca ataaaacatc ttattgataa 540
accnnnnnnn nnannnnnnn nnnnnnnnnn nnaacttgng gggggccccc gggncccaaa 600
tccnccttta gggagtctan tanaattcnc tggccggngt tttanaacgt ngggatggga 660
aaaacctggn gttnccaaat taattccctt ggaaaanatc ccntttncca gttgggtnaa 720
aacnaaaaag gccccaaccg atcccctttc caaaaatttg cccaccttaa tggnnaaagg 780
gaaatttgaa ncttnatttt ttgtaaaaat ccggtnaaat tttggnaaaa aactnntttt 840
taaccaanag gccnnaaatn nggaaaatcc nttttaanna aaaaaanaan cccgaaaagg 900
ggtnggggtt ttccaattgg naaaaaant 929
```

<210> 1596
<211> 935
<212> DNA
<213> Ctenocephalides felis

<400> 1596

```
ttcctgttat tgcattcacc aacactgatt ctccattgaa atttgttgat attgcaattc 60
catgcaacac taagtccaac cattccgttg gtttgatgtg gtggctgttg tccagagaag 120
tcttgagact tcgtggatca attccccgtg agaagacttg ggatgtcgta gttgatttgt 180
tcttctaccg tgatccagag gaagtagaga aagaggatct tgttcacaag gaagttgtgg 240
ccaaagttga gactgctgtt gctcatgata ctgctgaagt atgggcagga gatgagccag 300
ccactcaatt atggactgat gatgcacctg ttgctgcagt tccggctgtt taccctgctg 360
ctgccagcca agattgggcc gagcaagtgc aagaagaatg ggctgccaac cctaccccag 420
ctgctggtca aactacttgg ggaagctcca cacaagaatg gtcataaatc aaatgatact 480
ttttgtattg gataaatgaa ataaatgaaa attaaaatta aaaaaaaaaa aaaaaaaaaa 540
aaactcgagg gggggnccgg tcccaattcg ccttatagga gtcgtattac aattcctggc 600
cgncgtttta caaccgtcgn gactgggaaa accctggcgg tanccaactt aaatcgcctt 660
gnagaanaat cccctttcgc aactggngta aaaaacnaaa aggccccacc cgatcgcctt 720
tccaanaatt ggncaancct gantngngaa tnggnaaatt tgaagngtta ttttttggtn 780
aaaatcccgg ttaaattttt gtnaaaaang ntnnttttt aaccaaangg ccgnanttng 840
naaatncntt ttaatnaaaa aaatancccg gaatggggtt gggttttttt cnatttggna 900
caaaaatccc tttttaaaaa acnnggccnc canct 935
```

<210> 1597
<211> 941
<212> DNA
<213> Ctenocephalides felis

<400> 1597

```
tgctttgtgg aaggaaagtc taaaataaga atataaacta tgccgggtac cgggacgttc 60
aaattattca tcggaaatct tgacgaaaaa actcaagcgt cagatatacg tcctctgttc 120
gagaaatacg ggacggtcgt cgaatgcgat gttgtcaaga actttggttt tgttcatatg 180
gagaccgaac agcaaggtcg ggatgcaatt cagaacctaa acggctacgt aataaacggc 240
gaggccataa aatgcgaagc cgccaagagc cggagggcgc cgtcaacccc gacgacaaag 300
atattcgtcg gaaatttgac agataaaact cgcgcgccgg aggttcgcga actgtttcaa 360
aaattcggta cagtcgtcga atgcgatatc gttcgtaact acggtttcgt gcacttggac 420
gcgagcggtg acgtgaacga ggcgattcgg gagctgaacg gaatgatggt cgacgggcaa 480
cccatgaaag tgcaagtgtc cacgagtcgc gtgcgccaga agccgggcat tgggtgatcc 540
cgacaatgct accgctgcgg gcgtggggga cactggtcca aggagtgtcc ccgtgccatg 600
ggggccgatc gtaacggctt tccgggagan gatgttcggt tgngaccggt accttcgccg 660
gcggcaccgg cnttctgngc caaccggatg atgggccgga ttcccgggat tctattgacc 720
nttcttcgat agangttttg atggttcaag ggatttattt gganaggcgt tnttctggng 780
ggaatgcccg gtntgcgtgg gggggccaaa atttattgcc gcccaaggaa aaaanctttt 840
gcctcctttc cccctttagn aataaaaaan gggattggga tttttanggc ctccaacttt 900
tattccattt tttaccggng tttccccaag gngggttgcn g                     941
```

<210> 1598
<211> 937
<212> DNA
<213> Ctenocephalides felis

<400> 1598

```
aacnttttta agtgatttat ttattttata ttattgttgt aaaattacaa ttttcaaaat 60
gagattagga gccacagtac ccaatttcaa ggctgacacc acagaaggac ccatccagtt 120
ttacgactgg tctggcgata gttgggttgt gattttctcc catccagctg acttcacacc 180
cgtctgcacc acggagttgg gtcgcatggc cgtccaccag ccgcacttcg tcaagcgcaa 240
caccaaattg ctcgccctga gcgtcgacga cttgcagagc cacaaggact gggtcaatga 300
catcaagtct tactgccagg acattcccgg aaaattcccg tacccaatca tttccgatcc 360
gaaacgcgag ttggctgttg ccttggacat gatcgacgaa gagcacaagg acgaccctgc 420
tcacgccatg accgtcagat ctttgtcgtg atcgatccga accacaaatt gagattggcc 480
atggtttacc cattcagcac tggacgtaat gtcgacgaaa gtattacgtg tgatcgactc 540
tatgcaattg actgatcgtc tgaaagtcgt cgcaacgctg ccaactgggt tccttggggg 600
aaaaaagtaa tgatcctgcc ttccgttcag aatgaaggaa gcagccaact gtttccaaaa 660
ggtggtgaca cttgttctat gccttcaggt aaaggttttg gtgcccncac cgactggttt 720
ttnaaaaaaa acaagaactt tttttgnttc cttaaaaatt tttaatccnt ttccaaatnt 780
ttgcnaaaan ttttaatttt tttttatga acaanttttt gaaccctaag cnttttaaan 840
ttttntttcc gggatcaatt anctttaagg atgggngttt ttaaaaaaga ttttttccan 900
ggttttntaa atntgggaaa cccttttttg naatntt                          937
```

<210> 1599
<211> 648
<212> DNA
<213> Ctenocephalides felis

<400> 1599

```
gtcttccggc caggggggcct ttggcgggac atacacttgt gagagcaagg gcaccgccgt 60
ctccggagcc gttttgcccg gagcaccagg acgagaggct aggattttac tgtttggcgt 120
gcaaggaacc tgcctgtgcg agctgcctgc agacggagcg acacgccagt catgacgtgc 180
aggcgatcac tgcaatgtgc aaggcgcaaa agactgaatt atcccaaaat ctgcaacagc 240
tttctgaaaa agcgaggtct acgacagaat tcatacaacg gctaaaaggg atgagtgaca 300
aggttatgga atcatgtaca gaatttgaac acttagtaac agcacaatgt gatgcattga 360
tcacagctat tttaaatagg cgagattatc tcctagaggc aatacgatgt gatagagaag 420
ctaaactcag agcactcaag gaccagcatc aactgctaca ggaaaattac aacacacgac 480
aggttaatac aatttgatag aggcttcaaa gaaacgatag tgtgatttta caggtggttc 540
atgtatagcc ganggccaac gacatcgctg catcggaggn agagcncgcg gtgtttnaca 600
gtcatctacc tgacgacagg cgtctaggca tgncattact tntcaang             648
```

<210> 1600
<211> 650
<212> DNA
<213> Ctenocephalides felis

<400> 1600

```
gatgctcttg ggtttcccc agtgggtcca ccaatgtaaa taatcgaatc gttggaggca 60
aagacaccac aattcaagaa catccttacc aagtatcaat tttgtacaat gatgaatatc 120
acagctgtgg aggttctttg atttctgaaa aatgggtttt gacagccggg cattgcatcg 180
attctttcaa attctacatc cgtgtgggaa gttctcttga aggcgaaggt ggagctgtgc 240
atcgagctct aaaacaatat cgacatgaaa agtttgatgc aaaaactgta gattatgatt 300
atggattaat tgagttagac acaccggtac aacttagtga aaatgtaaaa ttagtcaaat 360
tggctgaacc tggtgttgaa cttgaagaag gaactctact aaatgtcacg ggatggggta 420
gccgtcgatc cagcgcagct ctacaaatag taactgtcca tatgtatccg aagaagtctg 480
caaaaaatca cattcagaca ggtgatctcc catatatgtc tgtgtggtaa aatgcaggag 540
gagaagncct tgcatggtga cttgnggcca gtgatcaatg gntcaattgg attgttctgg 600
acttgatggn ctccactttc tcgttttgta atncgntntc agatggtaag           650
```

<210> 1601
<211> 649
<212> DNA
<213> Ctenocephalides felis

<400> 1601

```
atttnagcca accagcaagc agaaacattg cagactcaat taaatctaat gtcccagcaa 60
cgagatgaac ttttggccaa gatgagtgac actgaagaca aatataatag acaagttgct 120
gctctaacca atttgcaatg tgccttagaa caattccaaa gagataaaga tcangaaatc 180
caccaatgca cagaaaggat aagaaaccaa ttggaattgg aacgacagga acaaactgct 240
```

```
ttaaggaacg aaatccaatc cctaaaaacg caacttagtg aacaacaaca aggcctgatg 300
gctgcaggaa gacttgccag tcaacttgaa tcctcacaaa caactgtaca aaatctcaga 360
caagaattga aagagagcca agacaaatac gctgctctga ccgccaaagt ggagtcttca 420
aaaaacaacc aagccgacaa aatcgagaag agcttgtaaa aacctaatgc tcggtacatc 480
cagctggtca acaaaacgac angcacaaat ctaaaatata tagcagtctc gacttcacca 540
gtcggatgcg accgatagct ttgagcacca aaatatntgg ttgagatctg tctggatgga 600
gagaggctgt aancttgcna ttttgggaag atttaccaac gntnggagg            649
```

<210> 1602
<211> 646
<212> DNA
<213> Ctenocephalides felis

<400> 1602

```
gttttgtgga gctactggta gagctagcaa ctttttatta tccgtcaggt gtgttaagga 60
aaaagataaa cctgtgtcga tgggttttgg attgatgata atgtccttgt ttgccttcgt 120
accgtcacca atattttttg gagcaattct agatcaaaca tgcatagttt ggggaaaaac 180
atgttccgga actggaaact gctggctata tgatgttgaa tctttgcgtt acatcatgaa 240
tttaacggca gcttcttttg tcacaattgg agtattattt gatgtcggag tttggtattt 300
cgtcaaaaat ttaaaaattt acgatgaacc tgatgatgac gaccaagaaa tgaattcttt 360
gaaggcgcaa aaggcggatg gagctgctgc aacttattcc aatcgaatct ctctatacaa 420
agtcatataa agaatagtac ttgaggaatt taaaattaat ttaagttgta atgcaaaaaa 480
tgtaactgat taaaatatat aaataaataa gtngaactat aaaaaaaaaa aaaaaaaact 540
cgngggggcc cggtcccaat cgcctatagg agtcgataca tcnctggcgc gttacacgcg 600
gctggaaacc ctgcgtncac taatccttga gacatccctt cgcagt               646
```

<210> 1603
<211> 643
<212> DNA
<213> Ctenocephalides felis

<400> 1603

```
caancatcca tgttttattc tgtatatatg tttgtgatgg caattgttta ttgatatgaa 60
attaaattaa tatttcgata aaacattcgt tattattaga atatttgttc tagataagtt 120
aaattatttt aaggggtctt aagtatttgt tttatttgtt cgtttaattg tgatgtagaa 180
ttgtgagaat tcaatataat atattaaaaa tgtttcggca agacaattca aaagctttta 240
tgaaagaccc tcatactgcg aattgtagga tttatattgg aaatataaac gagcatgtga 300
attctcaaga aatagaaaca catttcgcaa aatatggaaa aattctcggc gttttgctac 360
acaagggatt tggctttatc caattcgaaa aggaacaatc tgtcaatgaa gccatcaaaa 420
tggaacatca aaatatgttt catggcgaaa catgattgtg agacgagcca aagcaatgta 480
ggaggagcag gtggtactct gcaggcctgt acgcaggtct ctccgaatcg tgatcgggcc 540
gttccagnac tcacacatga gggcagacag gtctggtccg gcctgtcnct tgtgcctcgc 600
attgcagcgg agtggttggg gctcaaagcn ggcgtgtgtg aan                  643
```

<210> 1604
<211> 651
<212> DNA
<213> Ctenocephalides felis

<400> 1604

```
tgtttatatt ccttcaaaat gtccgtacgt gtgcaattcg aaaacaacaa tgaaatcggt 60
gtttttagca aactaacaaa cgcttattgt ctcgtagcga tcggcggatc tgaaaacttt 120
tatagtgtct tcgaggctga attggcagag actattccgg ttgtccatgc tagcattgca 180
gggtgtagga tcattggcag gttgactgtg gggaataaaa atggtttatt ggtgccagct 240
tcgacaactg acacagagtt gcaacatata aggaattctt taccagaaag tgtaaaaata 300
caaagggttg aagaaagatt atcagctttg ggtaatgtga tagcttgtaa tgattatgta 360
gccctagtac atccagatct tgataaggaa actgaagaaa tcctaacaga tgtctaaatg 420
ttgaagtatt ccgcaaacag ttgcagtaat gtcctagtag gctcatacac agttttaagt 480
aaccaagggg gcttagtgcc cccaaaacat ncatacaaga caagacgagc ttcgtcatac 540
tcaagtccgt agtggccgga cagtnccgag caggagnttt agcagaggtt gnagcaacat 600
tgngctctct ggggatggcc nacncactga ctagtggatg aagcggtcac n          651
```

<210> 1605
<211> 637
<212> DNA
<213> Ctenocephalides felis

<400> 1605

```
gttttaaaga atatcaagtg taaattaaac gcgatttaaa tttattgtaa catcaaaatg 60
gtgactttga catcttttat aaatacattc aacaataaac tggaagcgcc agtccagcaa 120
catctcaaag gcgtctacgg gtgtctggca gctacaacat cattagctag tataggggct 180
tacagtttcc ttgcaggatg gctctcagca ggattgttgc cggctttagg agccctagga 240
ttaggcttgg gcttgatgat gacctctcca gatgccaaaa attttaatat gagactagga 300
atgctgttag gattaggatt cttgtcaggt ttaggattag gacccttact agctcatgtg 360
gcccaaatca atccaagtat aataacaact gcattatttg gcactacttt agtattcgtg 420
gcattcaact tatcagcaat atttgctgaa cgtggaaaat ggtattcctg ggtggaatta 480
ttgcagcgtt tgaatatgat gtcttctcct cttggcaatt ttctgcaaag actttgnttc 540
nagctccctt ntgagatatt gtntggggct tggccttatg atccagcttt nttgaaaatc 600
anactggacc caccagtggc tctttgctgt cttgatt                         637
```

<210> 1606
<211> 644
<212> DNA
<213> Ctenocephalides felis

<400> 1606

```
gtttttcagc ctatgcatat ttaggagaat ttnntggaga taaaacaaga gccagggcaa 60
tcagttgggg agcttctttt atttctatgg gaatggtatt tttgccagca attgcttggg 120
gaataattcc cctcaacttc agatatccca taccaggttt gggcatagac tggacggcct 180
ggaggttgta cgtcctgatc tgctcgcttc taatcttttg ggcctgatta tgattataac 240
atttccagaa acaccaaaat ttttgttggc cactggaaaa actgaagaag cactcgatgt 300
```

tcttgctagg atgtacgcca gcaataaagg ccgtaagcct tctgactttc caataaaatc 360
cctggacatt tctggtgttt ctggtaacct ggcaaaagct ggaaatgctc gtgaaattgc 420
ttctctcatg atacancaaa cagttccatt attcaagaaa ccacttgtaa atcacacttt 480
tgattgtatt gtgcagttgg aatgttgcat cttcagtggc atgtcatgtg gtccagtgat 540
agctacgatt tgatgcatat atgacaattn gcactatatg atgctgtcag ttttgataag 600
gccaatcctc catccttnga caatntgctc aangatccgg gttc 644

<210> 1607
<211> 650
<212> DNA
<213> Ctenocephalides felis

<400> 1607

gttgggccca atggtgtagg caaatccaca ttcttgaagt tattgactgg tgacatcaca 60
ccaatcagag gagaagtaaa acgcaaccat agactgcgca tcggtcgttt tgaccagcat 120
tctggtgagc acctgactgc ggatgaaacg ccggccgagt acctgcaacg gctgtttgat 180
ttgcaacatg aacgtgcaag gcgagccctc ggatctttcg gtttgatttc tcgtgctcat 240
actgtatgca tgaaggatct ctcaggagga cagaaggcta gagttgcatt agctgaatta 300
tgtcttaatg cgcccgatgt tctcattttg gatgaaccta cgaataacct tgatattgaa 360
tcaattgatg cattggcaga agccataaat ggatataaag gcggagttat tgtagttacg 420
cacgacgaaa gacttattag agaaaccgat tggccttata tgtgatagaa gatagaacga 480
taaatgaggt ggatggagat tcgatgacta tagaaaggat tattagaaag ctgggtgaag 540
tagtgaataa tcaagtattg ccgtaatgca nagtatcata aaagatatac atatacataa 600
tgctgattta cttcaatcta tatgatatgc nttgnaataa angataagaa 650

<210> 1608
<211> 637
<212> DNA
<213> Ctenocephalides felis

<400> 1608

ttgntcgtcg ttttggccac tttggccaca ttagtggcag ctgatggagg atacaaaggt 60
tacaaaatct acgacgtgac tgtaacaaat tcaatccaag aagcagccct tagatcaata 120
ggtaacagtg gcgaattcga tttctggagt ccctcaaggg tccttgtaaa acctgaacaa 180
atcgcaaaat tcgaaggact tttgaaaact ggaggaatcg acttccaagt ttttgttgac 240
gacgttgacg aatttgcacg taaggaaaaa gccgaaaatg aagtcgccga atctagggcc 300
gaagggcgct tgtccttcac tgcttatcat cgttacgatg tgatccaaca atacttgagc 360
gaaatggcat ccaaacaccc agatttagcc aaggtcgaaa ccatcggcac ttccagcgaa 420
ggaagaccga tcaaagcgct gcgcttatcc agcggaggaa acggtacaaa ccggttgtgg 480
tatggcctgc atccacgccc gagaatggtg gcaccaacca ccgcttgact tgtagaccaa 540
atctggaacg gccgacatat tattgcgaat ggatgggtca tcattctgtt taatctnngg 600
tacaatctct acctgggacg tttgagaaac cgacaga 637

<210> 1609
<211> 642
<212> DNA
<213> Ctenocephalides felis

<400> 1609

```
gacnncggtg atgacagagg ccgggatggc gattggacat gtcctagttg ttccaacacc 60
aactttgctt ggagaaatgc ctgcaacagg tgtagtgaag aaagacctga tggtgcaggc 120
ggcggtgact ccggccgagg tggtggccgt ggaggcggcc gcggcggtgg tggtggaggt 180
ggtagttacg gtaaccgcgg aggtggtgat agagattctg gtcgcggtgg tgatagaggt 240
tttcgcggag gtggtggccg cggtggcggt ggcggttatg gaggtggccg cggaggcggt 300
ggtgatcgta attctggagg aggtggctca atgcgtggag gagatagagg acgagatagg 360
caacgacctt attaaataat tagcttgtaa ttttattcac cctcattttc acatcattcg 420
tactcatatt agatttattg atttttagaa ttatttatat gatttgtatg attcaatgta 480
acacaatatt gtaaatctta tattaagatt taataatata aaataataga aaaaaaaaaa 540
aaaaactcgn gggggcccgg acccattcgc ctatagtngc tatacaatta ctgccgcctt 600
tacacgtcng actggaaaac ctgcttncca cttatcgctt na                    642
```

<210> 1610
<211> 634
<212> DNA
<213> Ctenocephalides felis

<400> 1610

```
acttttcgat caattcgtat tagacttttt gataaaaaat gctgtctaaa gcttcgcttt 60
tggccaaggt atcccggcca ctgactgtgg cagtgcgaac aacatcccag gctgcaacat 120
gccctgctcc tacaaaggta gaagaagccg atagtgctga aagagatttg gtcaacttcc 180
caaggccaac acgtttggaa cattcaccta aagttcgctt tggattcatt ccagactcat 240
ggtttgaatt tttctatgag aagaccggtg ttactggacc ttacatgttt ggaactggtt 300
taattactta cttatgttca aaggaaattt acgttatgga gcatgaattc tatactggta 360
tttcattggg tattatctgt ctctatgcca ctaaaaagtt gggtccacat attgcaaaat 420
acttggacaa agaagtgatg cctatgccga tgaatggaat tcagtcgtgt agaagaagta 480
aaagtaccaa gatgccattg aaggagaaag tggacaatgg agactgaagn cacttatgtg 540
tggtgccaac gtgaaatgtg cctgcacttg acactanagg accncntgaa gtnntagagg 600
tagaaacata ttccagttgg aaangaantt gacn                             634
```

<210> 1611
<211> 639
<212> DNA
<213> Ctenocephalides felis

<400> 1611

```
cannatgcca cgaggaaaat ataccaatca caaagggcgt aacaggcatt tcacaaatcc 60
agaagagtta gaggaacaga gaaagcaaga agaacaaaaa aggcaatggc gtagggatca 120
tggaaacgaa tcaagttctg aggaagagga agtagccaaa aaagctgcag gtgataagaa 180
gaaagcccca gggctggaaa gctctgattc agagagtgaa tcggagactg aatcttcaga 240
agatgagaag gataagaaga aaggtgtatc tggattgata gaagtggaaa atcctaatcg 300
tgttcaaaaa aaaaccaaga aactttctac tttaaatgaa acattaactg atagcaaacc 360
```

```
acaattgtca agacgagaaa gagaagaagt tgagaagcag agagcgcaag cacattacca 420
aaagcttcat gcagaagcaa aactgtcaag caagatctgt ttagcaagac ttgcatcatt 480
aaacacagcg tgaagaacag cttaaagacc gagttggaaa aaaaaaaaaa aactcgnggg 540
gggccggacc cattcnctnt gnngtcgtnt acatcatgcc gcgtttacac gtcggntgga 600
aacctgcgta cccacttatc cttgagactc ccttgccgt                        639
```

<210> 1612
<211> 640
<212> DNA
<213> Ctenocephalides felis

<400> 1612

```
atttntgttg gttaactcga gcagtaagaa tatataaaaa tggctgcagt tggcaaagat 60
ttagaaaaac caacagctga ggtgcctcag gtccatcgca tccgtataac tttaacttca 120
agaaatgtac gttcattgga gaaagtatgt accgatttaa taaatggagc caagaaacaa 180
aagctccgtg tgaagggacc agttcgcatg ccaaccaaaa tcttgcgtat taccacacgt 240
aaaacacctt gtggtgaagg ttcaaaaact tgggatcgct tccaaatgag aatccacaaa 300
agagtcatcg atctgcactc tccatctgaa attgtgaagc aaatcacttc catcagtatt 360
gagccaggtg tagaggttga agtaacaatc gctgatgctt aaattttgta aattgatgga 420
aataaatgac aaaacctaaa aaaaaaaaaa aaaactcgag gggggccgg tcccaattcg 480
cctatagtga gtcgtataca attcactggc gcgtttacac gtcgtgctgg gaaaccctgc 540
gtacccactt atcgcttgna cacatcccct tcncantgcg tatangaaag ccccncgatc 600
gncttncaca gtgccanctg atgggaangc aattgnagct                       640
```

<210> 1613
<211> 644
<212> DNA
<213> Ctenocephalides felis

<400> 1613

```
cactttgagg ttttgatcct taaccatggt gaacggacgc atcccgtccg tcttttcgaa 60
gacatatgta acccctcgtc gtccttatga aaaggctcgt ttggaccaag aattgaaaat 120
cattggagaa tatggtctcc gtaacaagag ggaagtatgg cgtgtcaaat acactttggc 180
taaaatccgt aaagctgccc gtgagttgct cacattggac gaaaaggatg gcaaacgtct 240
cttcgaaggt aatgctttat tgcgtcgttt agtacgtatt ggagtgttgg atgaatccag 300
aatgaagctc gattacgtgt tgggtttgaa aatagaagat ttcttggaac gtcgtctgca 360
aacccaagtt ttcaaactgg gattagccaa atcattcacc acgctcgtgt tttgatccgc 420
agagacacat tcgtgttcgc aagcaagttg taatattccc tcttcattgn gcgttggatt 480
acaaaaacac attgacttct ccctcaaatc gccttcggtg gtggtcgtca ggacgttaaa 540
gaggaagact tgagaaagga tcagcgtgnt cactgccgag gaaaagagat aactttcaca 600
tttaatgtta tttttcataa taaacaaatc gcaaaaaaaa aaaa                  644
```

<210> 1614
<211> 635
<212> DNA
<213> Ctenocephalides felis

<400> 1614

gtttggacaa gatgcgaaag caggctttct catctgtatg tctgttcggt gaagacaata 60
acagcagcat ttctggtgtt tgggtatgga ggggtcagga attagcattc aatctttccc 120
ctgactggca aattgactac gaaacctaca actgggtcaa attggacccc aaatcagaag 180
agaccaagaa attagtcaag caatacttct cctgggaagg tgccgataag aacggacgca 240
agttcaacca aggaaaggtc ttcaaataaa attcaactaa agaattgcca cttttgttaa 300
tatttcaagt tattgtaatc ttatcctgcc acaatattgt tattttata gcaaaatgtt 360
tgctgatgat ggcatgttat attaaaattt cttgaaataa attatttaaa atctaanaaa 420
aaaaaaaaaa anaaaaaaaa aaaaaaaaaa ntgggggggg gccgggcccc aatcccctta 480
tagggagtgg ntaacattcn ctgccgcgtt tanaacgngg gatgggaaac cctgngttcc 540
caattatgcc ttgaacnatc cctttccnan tggnnnanag aaaaggcccn ccntncttcc 600
aaattgccac ntnatgggaa ngaanttgag cttat 635

<210> 1615
<211> 641
<212> DNA
<213> Ctenocephalides felis

<400> 1615

gtttnagtat tttttgttat ttcttgactt gatcatctgt tcgataagtt aattctgtaa 60
taatgaaaat tactagatat aagaaagtac atcgaaattt gaacttttat attaacaatt 120
ttggttttca tcagccattt caaatactta ttgacggaac tttctgtttc gatgcgttaa 180
agaatcaatt caatattcaa gatcagttaa agaaatattt tcaagctgag cttaaacttt 240
taactacgca atgtgtaatt gtggagacag aaaatttagg acccaaattg gttggtgcca 300
tgaaaattgt aaagcaattt ggtattcaca aatgcggaca tgaaaaggct ccaattggtg 360
ctagtgattg cttactatcg atggtaggca aaagtaatag agacagatat gtcatagcta 420
cccaagatcg tgatctacaa gaaaaaattc gggaaaaacc tggtgtgcct ctattgtatc 480
tgcatcgtaa agcaccatgt tagagcgcct cacaactagc cgtgataagc tctggaatgc 540
tctggagctc aagtccaaca agggagactt gnataanaat aaagagagtg gttatcagaa 600
acacatgaca gaaaggagag aagaaaagaa ggnctatctt t 641

<210> 1616
<211> 636
<212> DNA
<213> Ctenocephalides felis

<400> 1616

gcattatacc gccaattgtt gttcactgct ccagactctt tgggtgacca catatctggt 60
gttattttgt tccacgaaac cttataccaa aagactgatg atggcactcc attcgtagaa 120
cttttgaaac gcaaaaacat catcccagga atcaaagtcg acaaaggtgt agttgacttg 180
atgggcagtg agaatgaatg cactactcaa ggattggatg atttggctgc ccgctgtgca 240
caatacaaaa aggatggatg ccactttgcc aaatggcgtt gtgtattgaa aattggcaag 300
aatacaccaa gctaccaagc tattttagaa aatgctaatg tcttggccag atatgcctca 360
atctgccaat ctcaacgttt ggttcctatt gttgaaccag aggttcttct gatggagatc 420

atgacttgga acgccacaaa aagttactga aaccgtttgg ctgtgctaca aagcttgaat 480
gaccaccacg ttatttggaa ggacttattg aacccaatat ggtactgtgg caaagtgccc 540
acaaaaccac ccagcagtgt aggagttgcc tgggctgntt gagganactg tccactcagt 600
ccggattctt ctnntgagac attgaaaaaa gctatc 636

<210> 1617
<211> 650
<212> DNA
<213> Ctenocephalides felis

<400> 1617

aacnncttaa ggaaaagcaa gctaagcgca aggtatcccg cgctgaagaa gaaaagcgta 60
tggcccaaag gaaaaaggaa gaagaagaac gcagaataag agaaattgaa gaaaagaaac 120
aaagagacat tgaagaaaag cgtcaacgtc tcgaagaagc cgaaaagaaa cgccaggcta 180
tgcttcaagc caccaaggat gccaacaaga agggccccaa cttcaccatc accaaaaagg 240
acagcaactt caacatgtct tctgcccaaa ttgaaagaaa caagaccaag gaacaattgg 300
aagaggaaaa gaaaatttct ctgtccttcc gtatcaagcc tctggatatc gaatctctta 360
gcgttgaaaa actcagacaa aaggccaatg aactttggga atgcatcgtc aagttggaaa 420
ctgagaaata cgatctggag gaacgccaaa aacgccagga ctacgatctt aaagaattga 480
aagaaaggca gaaacacaac tcagacacaa agcattgaga aaggattgat cagagcccta 540
caggaaaatc cccccaaatc aagtcgctca atatgagagc ngtcgcacag acattgagat 600
agaaaaattn ttgaggggct aaactaaaca agagncttga cgttttgccn 650

<210> 1618
<211> 642
<212> DNA
<213> Ctenocephalides felis

<400> 1618

gccntgtagc accaacaaac ttttcaaaat ctacacgtca ttatgaaatt ttcaaattaa 60
aaagtgttca ttaataatta tagtgtatta tataaatatt agttctaatc tattgttatt 120
aaaatggttc tcttaggcaa tgatgaattt ttatcaaaac ttacaaaaat gtttcaaaat 180
gccagaagtt cttcatcggt taccttaact atgaaacgat atgatggaag aactacacct 240
taccctagag aaggcaggcc cccttttgccc aaaccagatg agcatttatg tctgttaaga 300
gccgtacaca aatcaaaaag atttcgactg taattaaatc gaaagacgca gtgaggtttc 360
acatagcata ttgcagttta ttaaaaggta atatggatgg tttgaaaaag atgaaaaaag 420
tcaaaccaaa agtgaaagcg catagaccta tttatttaat ggtgggtttg taagtgagca 480
agaagatttc tttcttaaat ttatatgtga tgcgagatga atgctggtca gaaccttatc 540
attttcgatc tgtcacttaa tcgctaagta ttaaccctta ttaattcntt gatattggat 600
ggatattttg aaatagatnt gtttatttaa aaaaaaaaaa cc 642

<210> 1619
<211> 646
<212> DNA
<213> Ctenocephalides felis

<400> 1619

```
gtttcgtaat aattacgtat ttaaaaaaaa atattttaat aaacatgtac tcattaagaa 60
tattttcaaa acccgttaat ggtcttgcaa gatttagtag ttcctataac gctacaagac 120
caaatctaaa attaaatgag aatactaaag taatttgtca aggatttact ggtaaacaag 180
gaacttttca ttgtaaacaa gctatagaat atggcactaa aattgttggt ggagtatcac 240
caaaaaaaag ctggaacaat tcatctcgat ttaccagtat tcaaaacagt aaaagaagcc 300
aaagatgcaa ctgaggctac tgcttctgtt atttatgtac cacctccagg agccgctgat 360
gcaattctgg aagcaattga agctgaaatg cctctaattg tttgcataac agaaggtgtt 420
ccacaacatg acatggttaa agtaaagcat agactattaa ggcaaaacaa aagccgattg 480
attggcctaa ttncctggaa tattgacctg actttgcaaa ttggattatg cctggcatgt 540
cacaacgagg aaaatggaat gttctcggct ggactttgca tatgagnagt aataacacct 600
agtgggctcg ccaaccttgg tggaataggg agacatttat gnctgt                646
```

<210> 1620
<211> 643
<212> DNA
<213> Ctenocephalides felis

<400> 1620

```
attgtctgta caatcgccaa attgtggttg atataagaag aattacaatt cttcgccatg 60
acgtcgaagg tttcccgtga cactctatac gagtgtgtca atgctgtatt ggattattca 120
aaggaaaaga aaaagaagtt cttggaaact gttgaaatcc aaatcggttt gaaaaactac 180
gatccccaga aggacaagcg tttcagcggc accgtaaagt atgtttacct gacatgaatt 240
taatttgagg tttttaaagt caagttatac tggtaacctc aagttttatt ctgataaatc 300
aggaagccaa agttatcttt ggcggacctg cacccagggt cttaaataaa ttggggaggc 360
atttgccgaa ccttttagct ttctattcaa agtcgactac ttgctgcatg aactacttag 420
tagtaggtaa gccaagtaac agtcataatt tagaaagtaa agcaatgagg caaatgcctt 480
ttgatcctgc gggaataagt ttcaccgatt aaattgaact atcaattaac tttctttgtt 540
gtagatgagc acattcncga caaaatgcag ttgtntttgg gagatcacag catgngatga 600
ctaggcacat gtcatgntga tgcgacttga aactaacaga caa                   643
```

<210> 1621
<211> 639
<212> DNA
<213> Ctenocephalides felis

<400> 1621

```
gtnnttttgc ctggattatt taattagaag actgttaagt tttagaagat atgactgtag 60
tttaacagtt tatacgtttt ataaatcagc ttctgaaact ttaatttcat aatgatttct 120
ggcgattttc ctgaggaccc agaaaaggag ctgcagagtt tagaagatga tgttgttcaa 180
gaaattctca aaactggcac tgatctgaga caatactcga aacaaataga aaaagaactg 240
aaagatgtag aaaataaatc tatacaggat tatattaaag aaagccaaaa tatagctagc 300
ttgcacaatc aaattggggc ttgcgatgac atccttgaaa gaatggaaga tatgttaatg 360
agttttcaga gtgttttagg taatatcagt tctgaaataa cgtctctaca aaaaaaatct 420
gttcaatgtc attcaattat caaataggca ggctgtccga ggagatctct cacagttatc 480
```

gaagatattt ctgtactcaa agctgtaccg gatttggatc ccagtactga gaaagaatta 540
tactcagtac aatctcatcc caganagttg taaagaccac attcaagagc caacttgcat 600
gtgtaggtgt cngagaactg aaataagcat gcaaaataa 639

<210> 1622
<211> 635
<212> DNA
<213> Ctenocephalides felis

<400> 1622

aacnatacaa caatcgatac gagacccgaa gtatacgata taatcaatct aaagtctgaa 60
acagttacaa aaacgggcga tgaaacagga cagtccgcgg acattgatgt gcccgacaat 120
ggcgacgatg aaaatcttga catgaaaatc gaagcggacc gactgaaaac attcgaaaga 180
tggccagtga gcttcataag cccctcggta ctagccaaat cgggattcta ttatatgaaa 240
gtcgacgaca gagtcagatg cgagttctgc aaagttgaaa ttggcagatg ggaacaaggt 300
gacgacccct ccgtcgacca ccaacgttgg gctcctaatt gtccattcct acgcaacagg 360
cctgtaggaa acgtgccaat agaccctccg agcacgtccc gagacccgga ccgagctacg 420
acgtatgcgg cctttgacca tacgtccaac gcgtccagaa aaccaaaacc tagcctacct 480
gtcaccaagg gcctgaatcc cccagtacgc atgagcagca cgcttagatc tactccatgg 540
ccggcagtta aagacgtcag gagaactagt gaagagcttt ttaccaggca cgaacgacct 600
tgttcntggg ngnggtaaag atggaccgga tntct 635

<210> 1623
<211> 641
<212> DNA
<213> Ctenocephalides felis

<400> 1623

gaattatttc tttgtggctg taaaagactg cacagctgct ctacttcaat caccggctga 60
ttaaaaaaaa cacgtgctga aaatggcttc gaaagtagca gcaaagaaag gcccagtcca 120
aactggcaaa aaacaacaac ttcgcggaaa gggattgaag aaaaagaagg tatctcttaa 180
atttaccgtc gactgcacca cccccgtcga agacaacatt atggatgtac agaacttcaa 240
aaaatacttg caagagagga taaaggtcaa cggaaagacc aacaactttg gtaacaacgt 300
ctcgttggag tgccaaaaaa tgaaagtgtc tgtcatctct gatatcccct tctcgaaaag 360
gtgccttaaa tacttgacaa agaagtattt gaaaaagaac aacttacgtg attggattcg 420
tgttgtcgat ggtggcaagg actcctatga attgaggtct tncagatctc atccaagacg 480
atgatgatga tgaagatgtt gnataatgta aatatcttnt gatataaatn taatattaaa 540
aaaaaaaaaa aaaaactcag ggggcccgnc ccattcgcct ttaggagcga tcaatactgg 600
ccgcgtttac acgcggntgg aaacctgcta ccacttatcn n 641

<210> 1624
<211> 407
<212> DNA
<213> Ctenocephalides felis

<400> 1624

```
tctccatata aaaccttaaa tctttcaaca aaatgatgaa gtagctgctg agcataatct 60
aaaagatcat ttaataacgt gttgctacat aaaattttaa tagcaatatg taaagtaaga 120
aaatttttat agaaagtcat ttgaaatatg acatgagatt tcaaaactac tggaccacta 180
tacaataata attgtctaaa ttctgtagct ttccatggtc tcacatattt caatgatcga 240
ggattcctag caaagtcctt ggatatataa atctttaagc tgcataaaaa attggaaaag 300
tcattagttt tagcacatgg caaacgaatt ggaagctcac cttcagtcca caaaagcatc 360
aatctcttaa ctgctcctaa acaaatcaaa ttcatatagt caagtgg               407
```

<210> 1625
<211> 345
<212> DNA
<213> Ctenocephalides felis

<400> 1625

```
tcatngngca tganttatgt gctttcagcg tgaatttatt cagtgccaat tatnttcact 60
tacagaaaag attctcaatc ttgattattc taaatttccg attangaaaa ttactcangt 120
agaacttatt acgggngcca caaaatnttc cggttattta agggatgatt gtatattaat 180
aaacgcattn gtcaaagcag agctctcatt catgataagt cgataaaaca taaaaaaatg 240
tgagtcgatn tcaaccctca aacttacatc ttttagtgng ggtatggagc tatgcaaagg 300
aaaatctttg caattagatg ttcaaataaa tctcgtttac tggtg                 345
```

<210> 1626
<211> 77
<212> DNA
<213> Ctenocephalides felis

<400> 1626

```
cgaacaactt tcgacaatct tattgctccg cttcgcaaca tgattaatgg cagtttttac 60
tttacggtaa gtgtgac                                                77
```

<210> 1627
<211> 285
<212> DNA
<213> Ctenocephalides felis

<400> 1627

```
gtattgaatt tttctttctc attctgaaag tattctattt cggatggaga acgaatgctg 60
cttggcaaaa tcattgatta tattataaat catacgaaca aatttatctc gaatatttat 120
tatagcgtca tattgatcta ataaaagaaa aaaagcagtt caacataaaa atattggaaa 180
aagtgctatc attgcaaaat atccaagtat tgcttataat atcgcaatta tatgtataaa 240
cttcgcaagt cgagtgcaac gtttaacgta agcgcggttt gtggt                 285
```

<210> 1628
<211> 436
<212> DNA
<213> Ctenocephalides felis

<400> 1628

```
tcgtaattaa taaaattaaa cttaaaaacg ttcaaacgct atcaaataat atatattttg 60
ttctcaagtg aaaattgcct ttttccagca tatttacata aagattcatt aatattagag 120
aaattttaat aacaaaaaat atatcaaata tttgtaggcc aaccgttgag ttacttcctg 180
aaaaagctgc gttgttaaaa agaatcatta attcatcaca acagctcata cgactattag 240
gctctgatta ctggtcaaag aaagcaaata ccttgatttt tttttaaata ttttgtcttt 300
ttaccttttg ttaaaaataa agaaatatat tataaaatta tattaataat tttccggcaa 360
agaattgtta ttttgctgta attttacatt ttgtaaggca tttttggcta atattccgat 420
catgtcagag agcaga                                                 436
```

<210> 1629
<211> 103
<212> DNA
<213> Ctenocephalides felis

<400> 1629

```
gagtgctacg atatatactt tcattgtatt tatttatctg gaatctcggc acgttaatat 60
cgtctcaaat tgagtagatt tccgttgcta tctttgataa atg                   103
```

<210> 1630
<211> 436
<212> DNA
<213> Ctenocephalides felis

<400> 1630

```
ggcttttcaa agcgctttga atatcttgat atcaaaaata atatctgtag gtgtatctac 60
ttgtgcatca gcaaaatatt ttctagagta ttttagtaaa caaatgtggg cgacgtaatt 120
ttcttggatt attaattcag atctgtttca tattttagat tttttattat tacaatgaga 180
taagtgattt gaatactttg ttataaattt ctataacctc attaaattta cgaatatttc 240
ctcaatcaac atgataaaac tcaccactgg atatctaatc tttttgttct aactttatgc 300
atatttgtca tagtaattac taactgttta ttattaaaga aatagatagt aatttcatct 360
tgagaaatga aaaataactt cgtgataaat aattctccag atatttccac ttggaaacat 420
tttggctctt tcaagt                                                 436
```

<210> 1631
<211> 281
<212> DNA
<213> Ctenocephalides felis

<400> 1631

```
aatngngact ttnttgnttc taaaaatctg ataaaatttt aaataacttg aagattaaaa 60
```

```
agcttttnaa aattcaacat acaaaaaaat acatgcaaaa gcaaatatac aattttaaaa 120
aacctgcaan tgtgggncaa cancactnat tcaacattca ccctgatctt tcattctttg 180
atttttata agctacataa antgccaatt ctttcttttc tttgntgata ttacgagtat 240
cgtattccaa tctgtgcatt attatgcgtt caacaagtcg t                    281
```

<210> 1632
<211> 549
<212> DNA
<213> Ctenocephalides felis

<400> 1632

```
gttggccgtt gtgaggttat cgtgtgcctt gaatcgctcg aatctaagct ccatcgtgct 60
aattatttcg ataactccca ccccgactgg gacatctcga gagtcgggaa gcccctggca 120
aaggctacgg cgattttgtt taaatcagtg tctacagttg aacgggtcca cttggacggg 180
aaacagtgtt catacgagat tattatatat ttaaaattga ttgaaacagt gacaaagtga 240
tatagtaaaa tattttacta actgttctta aggattcgaa tcataagatt tctttacatg 300
atggctgaaa tgaccgccag cacgcgcttc aaacaataac accaatcatt ctccaccaca 360
agtgcaacaa ttgtgaaata aaacttgatt ttattccaat catataaact ttaaatacag 420
tgcaactaat caaaataatt tgtcggcaat tgtaaaatac ctaagtgtcg ataagtctat 480
atgtgatcag gctaaagcct tgaaaaagaa tcttagtagg aatattagta ttcgtattaa 540
ttaattaat                                                         549
```

<210> 1633
<211> 549
<212> DNA
<213> Ctenocephalides felis

<400> 1633

```
gctactccta accaaaaaac acatttttga taagttcgtt agtggcttct gtcggatttt 60
aatttttaat tcaatcttac atttgcaggg tcaaataatt taactatttt tatttaatac 120
agattctaac gataatcttt tattttgtaa tatgtctgaa gaaggaacaa cgagaagaac 180
tacgaggtcg cttgccaggc ggctgagtac tgattcaata tcgcctccag ctgcagggac 240
tcctggcaaa aaagcgaggg cttcaagagt tacggggttg ccgtctattg cagaaactaa 300
accgaaagca gttagcactc gtaaatcccg aagattaagt actgacttaa atttagaaga 360
acctggaagc agaccatcaa cacctatatc aactgaaagg cgtcgttctc gccgactaag 420
tattgcttag atgaacaacg cccacaatct gtatcaactc tcccattggt ggagttatac 480
aagaagagga agacatcaat attttagcaa tgaaagatga tataaataat aaatccggta 540
tgngtgtgn                                                         549
```

<210> 1634
<211> 549
<212> DNA
<213> Ctenocephalides felis

<400> 1634

agttacagct cctgttaatg ctttagctga aacaagtcaa acttcatcaa tatttggtgg 60
tgctaaacca cgggaagaac ccactgagaa ataagttatc aagttttaac attaattatt 120
aaccaccata tagaatacca tcttgaatca tgtaataatt tttcgattaa aaattctgca 180
aaactcataa caggcgtcga taatcttact cttgcaataa attctttaac tgatatataa 240
aatgtatcaa gttttgtaaa agaaaaatca ataattattg atttacagaa taaaatattt 300
attttgttta aaaattgact aatactttgt aataatatgt aattcttata tatatagatt 360
aaagagttgt tgttagtgtc ttttgttttc aaatagtttt acactaatat atttaataca 420
aaacgcttta caaattttac aataattgat gaaaactatt tgagatttta ttctcgaagt 480
acaacttatg tattaaanaa ngngcnnnnt gnnnnnntnn cctngggggg gggccggccc 540
cattcnccc 549

<210> 1635
<211> 549
<212> DNA
<213> Ctenocephalides felis

<400> 1635

agcntaaata acaagactga cagtttagtt ttaacaattc atcatgggcg atggaagaga 60
tattgaacga cagccattga ttcaaaatga tggaactgga agccttagga atcaaggatc 120
ctatacggaa ggttctcaaa ccacgacggt ttcccctata ggtcctgatg agttgccacc 180
gtcttaccag ggaagttcgg ccagtggcgt gcccatggtc acttgcaggg tgtgtcaggc 240
catggtcgat atttcaggca aacgcgaaca gcatgtcgtc aaatgcaatc agtgcaatga 300
agccacacct atccgcaatg caccaccagg caagaagtac gttcgatgtc catgcaactg 360
tttattgatt tgcaaaagtt catctcaaag gatagcttgt ccgagaccaa attgcaaacg 420
cataataaat ttagcaccta gtcctgtgac accacctgtc ctcacggtgg aaattttcgt 480
gccaggaatg tgcagggttt gtgtgctatt gtggggancg ttttttcaa caccctaaca 540
atgcctcgc 549

<210> 1636
<211> 549
<212> DNA
<213> Ctenocephalides felis

<400> 1636

gctaataatg tgacagccat aaatattgta gattaatgta gaataatttg tattgagatt 60
tagattttgt agcatactag aatgttatgt gtgcttgaat aatgcaagtg agggaaccaa 120
taatttggtc tgtttttata atacatttta gataataatt attggtgaac tcaatcttgc 180
atatacgccg ctaatgaatt aaaccagcag gcatataatt tttgtactta aatatttata 240
taactaaaac tgatacgggt tacgaaaaac acataactat attatttatg tttctagacc 300
cgcatgaatt aaaacgaaaa cggcaaaaaa ttgacgggga tccaaaacat cttttatggc 360
aactgcaggg tcaaaatcct tctacgagta agtcattttc aacttttatt ttttttatca 420
gcaaatcaaa cagggttcaa tgtcaggtga cggtgaatca cggggtggat gaatataaaa 480
actcatatca tacgcttatt acatataaca ctaccatttt catattatca gtaattttct 540
aggagnata 549

<210> 1637
<211> 549
<212> DNA
<213> Ctenocephalides felis

<400> 1637

```
atggcatcct aaacaatata ataatataat aataatgaat ggcatttctc gctaacagat 60
attttaatat agcgatttga atagttttaa tttatttgaa tattgttcag agtgatactt 120
tttatatttg ctgtaataaa aatgattatt atgataactt atattatgaa agggaaaata 180
tatttaaact tttaattgat tacccaagag gatattgatt tgtatatatc tacttgaata 240
tgaatttgaa cagttaacat tatcttcaaa tttttaatat aatttaaaat tattggttac 300
tagcaaaaac gtcaagatgt ctaattacgt gttgaaagtc aaatcaaaag aaggacagca 360
tattttaaga gatctcaaat cttccatgac tctgggcgat cttttactga aactttcatg 420
ttgacatcga tatctaaacc aatttgcaaa ttttatcggg ttttccgcct aaagcattag 480
atttatctga tnagagtaag actttaaagg ngagtgattt nattcaggag atctgtattg 540
tgaaaaaat                                                         549
```

<210> 1638
<211> 549
<212> DNA
<213> Ctenocephalides felis

<400> 1638

```
gagaagggaa actgatcacc atgaaattcg cagtagcaat tttgggcctg gccctgtgtg 60
gtttggcatc agctcagttc cagaatggac gcatcttaga accaccagta cctgcactct 120
gcgcccaaag gacgatacac gaacgtagcc cagacggcaa aggatacttc ttctcgtggc 180
gtgacccaca attggctggt gttgaggaag attggttggg cgtccgcaac ttctgtcgcc 240
aacgttgcat ggacagtgtc agtttagaaa ccagtgccga aaatgaatgg atcaagcaaa 300
gaattgtcaa tggaaatgtc aaatacatct ggaccagcgg tcgtctatgt gacttcaagg 360
gttgtgaccg accagattta caacctgttt ccgtaaatgg tggttctgga ccgctgaatt 420
gcaaaaactt gcccaaccac agacagacaa caaaacgact ggtctgaagg agtggtattg 480
tcttcctcac cagatacaag aattgaacaa ggtggacaac cgaaactgtt tgcagtttga 540
cactttaca                                                         549
```

<210> 1639
<211> 549
<212> DNA
<213> Ctenocephalides felis

<400> 1639

```
ccccttcccc ccttccccac aaacatcacg tgtattttta gttatgaaaa aaaattgtaa 60
ttattttgat ttttggattt ttttaaaaaa aaattctctc tccctaaaaa catcacctga 120
ttaatggacg catcctattt gtattcctta tataaaaata aaaaatgtga aatttagata 180
tactgaatga ttactcgtac caagagtcga aacatataaa taaataatat aaaaatcaat 240
ttaccatttt caaatttgat gaattggttg caaactatac caaattcctc aattccaatc 300
atattcatat acaaaaatac ctatcaaatt ttccgattca tttaaaaccg attacaatca 360

attccattag gcaccagtat ttataaataa aattttgcgc atatgtgtga acatatattc 420
attattttat tagtctgaga aatagatgtt gactattcga gagagcagcg aaatgtcgat 480
atttgccacc tcatcagatg ttggataacc aaccgntaat aaccgatttt taggggggatg 540
ttaangcct                                                         549
```

<210> 1640
<211> 549
<212> DNA
<213> Ctenocephalides felis

<400> 1640

```
tatttttggg tcttttatgt tttacaaaaa ggatttatcg ttttaagttt aaataatgac 60
aatcttacaa attaatctaa acaaaaatgt aacaatattg ctttaattaa ataattcgtt 120
tattgttata ttgaatccac aaatatgaat tgttgattat tagcatcgtt tatttttgtt 180
gtaaataact tatgcaaagc agagttgatc ttataaaaca tctaatactt attttattat 240
acatagtgtg atacttgttt ttattttaaa tacattacaa actaaatgta gttcatctat 300
gatttacatg aaaaaaaatt agttattatt tgttagtgtt taagacattt tacgcaatat 360
ggcagtaaat aaatgcgcac actaaaaatt attattaata tttttatagg aacgaaagtc 420
tataattcta tacactacat cgctttgtgc aattgaaata atatttttca ttatattgna 480
tgaatttagt atatcaaaac atttaaaatg gttatatgta tacaggtnca ttattgtaat 540
aatgagaag                                                         549
```

<210> 1641
<211> 549
<212> DNA
<213> Ctenocephalides felis

<400> 1641

```
gaacagttgg ataaaataaa aaattgtgaa ctaaaattta ttttcaaatt tcattaattt 60
ttaacaaacc cttttccgta atattgacgg aacctgccta agtgaagcaa aggacgatat 120
cggttgtcgt tggtagtgta gtgaaactat aaaattgtga ataaaactta aaacatcacc 180
aaaaataatc cacacacttg cagtaaacaa attattattt gtctctatag acagaaccaa 240
atagaagaaa aactcagctg ccaaatcaag attgacataa ctgtcaatta tttttatgtt 300
gatcatttat taatatatca attttgactt tcatcgattc tatctcggcc cccctttccca 360
ccatgacgct ttcggccaac agcaatgcta ccaatcgcct cgggtcgcag cagggacgta 420
gcctcaatta gccgccgtgc gaatacaggc cggcncagag gatatgcgtg cgcaagcagt 480
caaacgttgc aaaatcaaag tgacggtgca agagacagct gnagaatcta ttgacagaga 540
tacgcgcat                                                         549
```

<210> 1642
<211> 549
<212> DNA
<213> Ctenocephalides felis

<400> 1642

```
attagacttg ccacatctaa ataatataaa aattagtaaa ataatttgta aatatttatt 60
taaaaaataa aattaggatt ttttattaat atgaaataat aaataatcaa ataaatttag 120
tgtaaataat aaaagtgtgc atgtgtcatt cttgattaat attcaacgca attaaagatc 180
aattttgaat gtatcctctc aaaaattgtt gtatgaggag atgactagca aattttttat 240
aaatgtcgtg gataaatgag tttcgaaaat ttattttgga ttttataaga actgttttcg 300
aattataata gagggaagag ctagaaatcc acatatcaaa attttaaatg gacctgttat 360
agaagaaaat gaaactcaac aaaggaacca tgaaaaaacc ngagttctct acaattgccc 420
ctgtgtcgaa gaagacgatg atcgagtata tctaatttga atcagatcta cacaaatgag 480
tggccatcct gcccatttta tagactcaga caagcggagg gcccttggan ggcagatgaa 540
attcccant                                                         549
```

<210> 1643
<211> 500
<212> DNA
<213> Ctenocephalides felis

<400> 1643

```
acatcaatgc aagagaagac accaacaaaa aataacgaat ataaatcgaa atttgcgaaa 60
ttaaaaattc gtgatgatca gataagttac aatattttgc aattctttta aagtaaatat 120
ccaaagtttt aacattataa aattaaaaaa aaaaaccaat ggggaatttg gnttaaccaa 180
tttttgnacc tnnaaagnaa ttnttttttt tgnaagnacc nnntnnaaat tttgnttggg 240
nccaantaaa aaaaccgnng ncaggnatnt nttttttcnt acctnntgga tnnnttttac 300
ctnttacnnt ggtttttaac ntattttaaa annnntttgn tttcngaggg gttannttta 360
nnaaatgnct taaaaatttt aatnttttcn tttnnaggnt tttttggggg aacnttttttt 420
aaaacctcnt tnttananta ttcnttggaa cccgtggnaa ncatttgggt ttantantta 480
aaggatatng gnngggtttt                                             500
```

<210> 1644
<211> 500
<212> DNA
<213> Ctenocephalides felis

<400> 1644

```
acacatagca aattaagtct tcgcttggat ttacatgaaa attgccaaaa aaagaataca 60
ttcattatag gaagtattta ttcaagtatt ttttgatgac tgttctagct gttcatcaat 120
tcatacttaa aatagttacc ctttttaatt tttacagtat ttaaatttaa tagttggtat 180
agtattttat aaattttaat ttgaagcatt cctttatagg tttggaaatt atatttttac 240
aaaaaaaaat aacccgtcca attgatttta aacaaaacca taaaaaacta ggtgctatag 300
taaacagcat ttgatttgat gtatacattg cattgaaatg agtggagaag caagatgaaa 360
gcctatccat taatgactta agacttttta gaaatgtttt atatatacat atgtatgcta 420
gatatttaga tggtgtcttg taattgaatt agatttacaa ggaaccaaat gttctacatt 480
aaaactaaat ttatctctat                                             500
```

<210> 1645
<211> 500
<212> DNA
<213> Ctenocephalides felis

<400> 1645

```
acagtttttt ttttcctcaa acacgagcta ttggaaaaca aaattaatta agtttagttg 60
aaagtcaaat tgacccatat atatatatat ctagttttga tatttgtctt acagattatg 120
gttgtcgatt tatatatgtt tttattgaat acttgtgatg tttattattg atttcttttt 180
gtttgttgtt catcaacgtt ctttgttaac aaatggggga atagctagtt aggaacccaa 240
gacgtcatat atctggccga aagcacaatt actgaaggca cacacccaca actacagtct 300
acgctcaaat tcatattaag taaaccaggc tctggacgtg cggccaacaa ttatggatac 360
cgctacgggg acctcaaaca tcgttggaaa gtagcaacaa tttttagagc cgagggcggc 420
tctggtgtga caggggggcg tgtgacgata atcataatta tcgcgtattg ataattattg 480
tcgctgatgg tcgcaacccc                                              500
```

<210> 1646
<211> 277
<212> DNA
<213> Ctenocephalides felis

<400> 1646

```
actgaatgtt acaaattttt acttggatag aaggagcaat tttcgttgcc ctcgaccggc 60
gtttcgccag aatttaataa ttgtttattt tattgcattt aatcctcttt ttttaaaatg 120
atgcatatta taataatata cttttaaatt attattgata caactttttt gcaaaccaaa 180
ataggtatga tcttttaata atttttatcc catttaaaaa aaattctatt caacaaatat 240
ctttttttca atgcaactag gattttatat gttaagt                           277
```

<210> 1647
<211> 500
<212> DNA
<213> Ctenocephalides felis

<400> 1647

```
acttaagtaa tatgttagta aatttatatt aggaattatg acaaattttg ttatattaga 60
acttatatat attaggcttt acaaaaatgt tatttggatg aactatttta tctattagat 120
ttacttaaac attatagaga taggaaaaaa tctcttaaac cattttgcta aaactttgag 180
aacgaaaaaa taaaacatct acaagacatc aagtcttttc ttttaaaaga gtaacactcg 240
aaaagggagc gttcattctt agaccaaaag aactacaaac atattatatt ttataatata 300
gaattattaa tagtagtatt tttttactaa tataaatatg tttaatttca ggttattttg 360
actcgtcatc ctatcaaatt agtcaagaaa ataacgtcaa cagaagtcag aaaagaacca 420
aaaaattttt ctgctcaagt gcaaaaaggg ctacacttat tcacgtggtt gcaacaccac 480
ataaaatacg aatgtggcaa                                              500
```

<210> 1648
<211> 331
<212> DNA
<213> Ctenocephalides felis

<400> 1648

acagtagatt gttttgaaat cggtgccgtc ccgtaagtgt taaatatatg taagcaaaat 60
gatttactc accaatacat cataccccat gcaaaaatac ttttatagag taatgcaaca 120
cgcttacctg aaacaaaata aaaattatta aatacattgc aaagaaatat ataaggtcat 180
gaagaaaaat tttatgtaac gcaataatta tatgtagtag cagtcttaca aaaatatgta 240
ttaactaaca atatcaatag acaattattt aggggccaat tctgctgtaa aatagaccaa 300
tgttaagtaa gggcttctca tacaaaattg t 331

<210> 1649
<211> 113
<212> DNA
<213> Ctenocephalides felis

<400> 1649

acgagttaat aaacnttnaa atataataag taacatttta aagntgcatc ttagtgaaaa 60
tctttaccat gttccttgaa aataataaaa caaantaaac atttgtgcca tgt 113

<210> 1650
<211> 474
<212> DNA
<213> Ctenocephalides felis

<400> 1650

acaggaagtt ccagcaattt gccttaaggc acgatgccat acgccactag tttttgttag 60
ttgtatgttt aattatgcat taatcaaata attttttcat agttcaaaac cagacggttt 120
ttttttaaaa aaaaaaaaga attataaaaa aatatgttca atgtgacagc cagttgtgtt 180
catgcaaggc gacggaaaga gcactcgtgt cgagcaagcg atttgcccgc tacacatatc 240
agtttggtct gacgagtatt tttacngngc ccgcctatag tgtgttaata tgatgcaatt 300
cagtcattat caacacgaga cagtctaata aaatttattt aattttagct attatatcaa 360
ttaaaaataa ctatacgtct ctatttattt ttgttccggt tactagaaac tggatgaaat 420
atttagtcat acttttaaga agtaacactt ccaatatgga cgaatttttg gggt 474

<210> 1651
<211> 500
<212> DNA
<213> Ctenocephalides felis

<400> 1651

acatacacac agatatacat acatacctac acnaacacac acacatacat catttttttc 60
ttaggtatgc caaaatgttc agagggcttt gaaacgtaaa gatatgtaaa aaatacattt 120
tcattttgtt gcgaacatac cctactggaa gtaaataaaa atgcntgaat caagcacagt 180
aatgggaaaa aattaactaa aaggttagtt atcgtataac taaattaaat caaagggctg 240
ntttattatt tacagttnct caactacaaa ctatataagt caaactcaaa aaactataaa 300

tacttattcc aagaaattaa taaactttta attcaatgaa taataaaagc atttccctat 360
tattngcttc aataattatt ataaactatt aaaaccattt atacattgtt tataacttac 420
aagaaagcan cttaataaac ttgaaatctc taataaacaa cattngcaag actttntaat 480
ccatcaaaaa atacttgaca 500

<210> 1652
<211> 500
<212> DNA
<213> Ctenocephalides felis

<400> 1652

acatgctaag tatcaaggat gacgctgtat gtgaaaaaag tgagtgtttt ccgctttcct 60
gttgaaatct ttcttcaatt ttctttgcta caaacttcac ggagttcgag acctttccaa 120
cgaatgcaaa agattcgtgc gttctggagt tatatcgtca ggaaggaaaa cccgtcttat 180
ttttatataa tagataatat atactatatt attatagtaa tcgggcgtaa catcaataaa 240
aaacatgaat tttagtgtta aattagaata ttaactaatt tttcgactga atgagggcta 300
ngaatggagg caacatatgg atgtgaaaaa actagtatga gagtttttag aattttttta 360
aaattcatat gcttccacta gcatagcctt cattcattgg caatcaattt aactgtaaaa 420
attaaaaata aataattttt tattaacatt attgtttttt tatgaaactc cttacatatt 480
atttatttag ttttaatttt 500

<210> 1653
<211> 226
<212> DNA
<213> Ctenocephalides felis

<400> 1653

acttaaaaat aatttctata aactgtttta gattggaaac ttatgcacta aattaaaaat 60
attgnattga taataaggtg tgattgcgat tttaatataa aacctcactt aacctagaat 120
taagtttata cattatacac atcagacttg cttaacagca actattaaaa ataaatcaaa 180
tatggtatat caaaaatgat atcaaagtaa attggcatat caaagt 226

<210> 1654
<211> 500
<212> DNA
<213> Ctenocephalides felis

<400> 1654

acattttaaa ttaaatctct aaatgtatat tttttattaa tattatttgn tatctatcat 60
atgaatgaat tattcgtaat tgtcatatac gcttaactat ttctgtttga tcaagcttga 120
ttttaaaatt ttgagttatt atatttctgt gttcattcac tacatatgta ttatagtttc 180
tctacacttg tctgcaaaat cattcattaa attataactc atatccaata caaatgatat 240
gtttgtattt aaagaactga catctcgaaa cgcaaaaatt taccaagatg accaataata 300
tttctttcat tttcttatta tgaagaatca ttatttgtgg taatttaatt ttgtatgata 360
accattgtga tcaatgctgt aacatttgtt aacaattaaa ttatatagtt tatgttgtat 420

acatttagac ctgtaagaag cgatatttaa tattattttt tataatttgt taatcttaca 480
tatatagata aatattatgt 500

<210> 1655
<211> 500
<212> DNA
<213> Ctenocephalides felis

<400> 1655

acattctgaa caaactagat aaaatgaaaa tcatgatatc agttttattc ttgttcttag 60
ttttttacgt aggatttaac atatcaactt ctatttaact tttccaagtt tcgtctttaa 120
ataaagtttg ttatcggcaa acacaagttt taaagagaat tattccgttt gtctattttt 180
actagaatac ttttttagtg taatttgaag tagtaagtag tatcttggaa aaagataaat 240
ttaacgtcct ttgtaatcga catattaata tcgaataaat ttgatactga tgaagtatta 300
tttgttagca gaattttcca ataattagtt taccataatt tattaactga taatgcacac 360
aatttttttaa aaatatactt tttaaaatta aagtcttcaa ttatttcaaa atagttcaat 420
actcaatcta ataataaatt taactgttat tcactatcct gccacgattt ttaaattagg 480
cttcgttaaa ttcaattttg 500

<210> 1656
<211> 343
<212> DNA
<213> Ctenocephalides felis

<400> 1656

actaattttt ttaaacattt ttttaccctc tggcaaatat ttccctaact gtaatgtaaa 60
aattgttcac aaatggnaat tngcctgtca ttcatgttcg aacgttttaa tngcatgttt 120
gcatttgcaa cacgagaatc ataattctct tcttgataat ttatttntat gtcattttta 180
gtcatgaaaa tgaaatatat aatttgtaaa taaatactca gttcatacac gataatgtaa 240
ttataggtag tgtttaaaaa aattagcgac tgtgaacaag atatcaaaaa aatacacatt 300
tatttatttc actatccaaa gttttcttac agtcctctcc tgt 343

<210> 1657
<211> 500
<212> DNA
<213> Ctenocephalides felis

<400> 1657

tcaantntga actgcantng ntaactgnnt ttnatgatcn antcnattga aaanaaangt 60
nnctaanntn aaangggcnt gacgncnnct gtcttcncat tatgtnctat gctcatcagt 120
ttataagtta aagttgnnca cctctcntnt atagnaaata natgatgact attgcgaaag 180
aagtctagac tataaaaatt ctaacatata tatttaggta nttgatccat anactaggga 240
gngaaagacg tttaanatac atggnttcca naatagtcaa catgatttaa ccacattttg 300
tatgttataa gtatgcaagc ntgttgtgcn tgagatcgcg accacgacct angtgatang 360
canctcngtn gcgaccactc taancngaat tttgcngatn tacatcacac tggcggcgct 420

cgagcatgca tntagagggc caattncaac tatagtgagt gggaaaacna tgcagttgcc 480
cnacttntna cttgcatgac 500

<210> 1658
<211> 500
<212> DNA
<213> Ctenocephalides felis

<400> 1658

actataccgg tgcttaccgt ggcaagatga acgtcccaaa acgtcgctga tcaaattaat 60
agtcctcgtg gctaatttat ttattctgga aaatcgtgcg atttgtttgt gttcacaaaa 120
tttccgttta ccaaattttc ctctataatg ttcggagtgt tggccgccac ctcgatttgg 180
ttactattgg catgggcagc tatgctaata tttttccttc cgttgatgtt cgttgtgtta 240
gcagttttgc cgggactgcc attactgatt attcgaaggg tgtgctacgt gcccttcgat 300
tcgatttaaa tttgatttct tcaattaaaa aatcaatttt aaataaggca gtgttctttg 360
aaatagttat ttaatcgtgt catttcatag tagttgtgat attatatttt taaacatata 420
tcttctaatc attgataagt atgatattta tacatagtct tacatattaa ggtataatat 480
ataatagata tagtcgtatt 500

<210> 1659
<211> 500
<212> DNA
<213> Ctenocephalides felis

<400> 1659

actataattt caattcaatg catttttggt nagccaaaaa ttatatgaac catttgtatg 60
tttgttaatt caacagttat tcgctctgga ttctttaaat tcgaagtgtt actatactga 120
tgatattaca aatctactgc aattaaagat tatttattca ttgcaatatt agtatatagt 180
aatatttatt agctctttga caaatttgtc tatcaaaata attaaacctt ttcaataaat 240
ttttataaga tacatagttt ttatctcctc tacttaataa aattacatca ttatttgaat 300
taagtggttt atataagtgc aattttactt atttacaata tttttctttg aactcattaa 360
aataaactca atctctaatt ttatgagttg tccatttagt atgaataaat atgaacagtt 420
tcttgaaggt ttttgatctc ggccatacca ttttttcata tattgtctat atacattttt 480
tacaatgact atatatcata 500

<210> 1660
<211> 343
<212> DNA
<213> Ctenocephalides felis

<400> 1660

actagattat cctgatactt ttgcgccatt cctttcgcca tagaactagc aactaatttc 60
tcaccgggaa tagcaaactg atgtgaagca ccacgaagtt tagattcttt aacaacttgc 120
tcattttcta gttcctgcag tttattataa tattcttcgc ccttcttact tttgactgtt 180
tgctgccatt ccgtttccgc ttgcttaatt actttggtc tcaaatgttc tggatttaat 240

```
tcaattcctc cttgttgagc caataatgct tgtctttctt cgaaggtctt ctctaattca 300
gttcctacac gttctttttg atattgtgtt aggtcgtagt cgt                   343
```

<210> 1661
<211> 500
<212> DNA
<213> Ctenocephalides felis

<400> 1661

```
ncttgtgcaa aagcaacatt ttgacattat angtnnggan attgaactaa attnaattat 60
aaaantatna ntnantggta aactnacant attgaacatg ttaatnttat attccatgtt 120
tagcaaagaa gctcctaatt taaaacaata annnnatncn tgcgcnatca anctttcttg 180
gttgngcatg aagtgtgatc attaaattat aagtattctc aagtaagatc tgaaaactat 240
atcaaaaatg tataattaaa ctaaaaanat atatttatta tataaatntt cctatngcga 300
cantanncan tttttaatnt naatgagnaa tgtatnaagg tgagnntntg ctcgtgcgaa 360
tgntatnggn cnttnagana attnaagaat tatggtaant attntngaat ggattgcntt 420
atngnttcct aaatgncctc tggttagttn aaancttgta tgantngaga cacagcaaca 480
actgttgctg nntttatatt                                             500
```

<210> 1662
<211> 334
<212> DNA
<213> Ctenocephalides felis

<400> 1662

```
acactgcaaa atttcaaatg cgaatatctc gaaaactaat agaccaagtg tcataaaatt 60
ttcacagaat tttactaaca ctattgtgca taaacactat gagtttaatc gaaatccgag 120
atgataagac tttttcatca aaaaattttt agngtatttg atcagattca tttntnantt 180
tccggcggtt ttaaccagga aagcatacga gtattaaaat aattattata tggcgttata 240
tctcgctata tgacctacaa ttaatttcca attnggaccc tntatctgcc ccacacgccg 300
agcaatcgtc aaaaaagtga gaattttttt tggt                             334
```

<210> 1663
<211> 500
<212> DNA
<213> Ctenocephalides felis

<400> 1663

```
acttaaacca agaaaaaaaa aaagctttta gcctggcgtt gacatataga gaccataaga 60
tcgcaacacg agaacgatgt ctggacattt tcgaactcac atatactagc cacccgcgtt 120
gtggcgcctt aaaaacgtag ggatgaaatg tttacacaaa aaaagagaca taaatattat 180
cttgctgcgt tacttaaatg tctatgattt gaaatcgaat tctaagagaa aaggacttca 240
acgatatctt acccttgcaa tatgatattt taaaataact ttctttaaaa tatttacgac 300
tatatataaa aaaaaaaaaa aataaactct actcacaaga acgatttcca tgaaacaaca 360
cttaactgta taacataact taatgtatct ggcaatcatc atagattttt aaaaaaaaaa 420
```

aaaaacaaat tattttatgt attggtgttt acgaaaatga ttatgaataa cacaatacac 480
taaacaattt caataatgtc 500

<210> 1664
<211> 500
<212> DNA
<213> Ctenocephalides felis

<400> 1664

accgtcaatc actttataag tcctttatat acttgaatat aaaaatgaag caaattgcta 60
ataaaccttt cctcaaaaac atttgatgaa gagatttcga cttctcaatt tagcttcaca 120
attcttcaaa tagagagtga aattacactg tagagactaa aacagaaacg aggcttttca 180
tattttcacg aaacaatgat tacatattgt agactcatgt tcggtaacat ctattctgca 240
taatcatgcg attagttgtt ggacacagac tgtagatgtc ctatactttc taagaatttt 300
tatcgaagtg cacaaactag ttacattctt gaccaacaaa tatattacgg gcaggaacgt 360
gaactaatta ttgatctgat caatagcttt tccctaatac ggtgaacgtg ccctattaac 420
tttatactgt aatcttgtaa tgctcttact cggccataaa ataatgatgt ggaaacccaa 480
gactaaagcc taagcaggtg 500

<210> 1665
<211> 433
<212> DNA
<213> Ctenocephalides felis

<400> 1665

acacaacata ttaagctaat gaactcattg nnnaattgac ntatntatat tattnataac 60
taaaagtctg ggagtttaca tatattaatt attttataac agcttgctta aanttgactc 120
gttngctatg caataantac ctaattgcaa tttaaagttt agatctcgaa cttcacanat 180
cntcctatta aaagntgttt acagnanttg gttnnggatc nantgnntgg caaantaata 240
ntnnaatatn tgtatgnnag atttaatctc cantcatnat cttcatgnat tcatcgaaan 300
cgacagttcc tgatccnnna gtatngattt ncgcgatgat nccntccang tcggacgagc 360
tgagtntgcg ncgagcgctc ccaagatctc cttcagccgt gctggntgtg angnaaccgc 420
nccttcgtgg cgt 433

<210> 1666
<211> 500
<212> DNA
<213> Ctenocephalides felis

<400> 1666

acttgtgcaa aagcaacatt ttgacattat aggtttggaa attgaactaa attcaattat 60
aaaaatatta ttaattggta aacttacaat attgaacatg ttaattttat attccatgtt 120
tagcaaagaa gctactaatt taaaacaata aataaataca tgcgcaatca atctcttttg 180
gttgtgcatg aagtgtgatc attaaattat aagtattctc aagtaagatc tgaaaactat 240
atcaaaaatg tataattaaa ctaaaaatat atatttatta tataaatatt tactatagcg 300

acaataaaca atttttaata taaatgagta atgtattaag ttgagttttg ctcgtgcgaa 360
tgtttattgt ttatagagat aattaaagaa ttatgttaat gaaaatgaaa tgtaattgaa 420
tattgcatta aataaattaa ctctgggtta gtttaaaatt atgtatgatt tgagacacag 480
caacaactgt gctgcattat 500

<210> 1667
<211> 500
<212> DNA
<213> Ctenocephalides felis

<400> 1667

actatacttt gacaaatagt ttatcatgta atntactaga ttaggatttc aaataattta 60
taaactggat atacttctac tnccaatatt tgaatatgat atgaatgcat gtgtgtatgt 120
atacttattg catattaaga tgcattaaca tagaattaaa tctcttggtn acgtatatnt 180
aggatatcat acatactcaa aatagtgata tttacaataa aaatataatt ttcttaaacc 240
tatattaaaa tataaataaa tagtaacnac nttaaaataa cctggccatn attnnnagan 300
ttgntnttcc ttttatnggc ccntattann nnatncnttt caaanttggg nnngnnaatn 360
cnngcncnna cttttatnaa nanantaann ttatgggcnn aanttanncc nnntggcnta 420
tnngtcnant naatattcca nggngatagc atgttgggcn ngcctantca gcaaattngc 480
ctanatgggc atgttntaga 500

<210> 1668
<211> 500
<212> DNA
<213> Ctenocephalides felis

<400> 1668

acatacacta agtcatatga aatctcggca agcgttaaaa gcacaatact gtcagttctt 60
ttgaatttct aaattttaaa actttacttc gagatggacg aaaaatgttc accatccaat 120
gcgcctaggc aataagaaaa attccaactc ttttctaact tgtttgccat catctctctt 180
cctttcaata agaatggaaa agttataaat ttagcgtgtt tttgaatatg gaatagtgat 240
aaaaagtctg cattttggtg atatagtgtc tttgcgtata actgcgtttt gcttttgaaa 300
aagtagttct acaagttaat agtttacatt ttagattgaa ccggttcatc agcgtataac 360
tagtaaatct tgaataattt tctgggctga aaattttaat gtaacaaatc gacaattttt 420
tagctaacca tgaattttga attttgaata attaaagtta ttttaatcgt cacgtncgcg 480
cccactcgct tttttggcca 500

<210> 1669
<211> 500
<212> DNA
<213> Ctenocephalides felis

<400> 1669

catgtcaata gttagcatat gtattaagaa tttaacataa taattnaaac atcaatnaaa 60
acaatataaa taaataaata attttngtat atcaataaca aatttcttaa ataactaatc 120

acatttataa ccagtccatt gttccttata tttatttttc aatatatcac tcgtctccaa 180
ttataaaact ttgactagac ctgataaaat attttataca taaaaatact ttgnttatat 240
caccaatagt taaactttaa aataatttca taatcacaag tgctatgata ttaaattaga 300
agcatttaat ttatataaac atattaatag taaatttatt atgaaacttg gtaaagttaa 360
taaatacaca ttaataaaat tcttttccaa tatatgcttt tttcttcgaa atgcctttcg 420
atgaatatac attctaagct tctttccaat tcgtatccgt ccattctatc ctctgacaca 480
aaattaattt cattcccntc 500

<210> 1670
<211> 500
<212> DNA
<213> Ctenocephalides felis

<400> 1670

ncaaacgtga ccatttttca cgtgacggtg cattgtggtt ttttgttatt aattttgttc 60
ataaataaca tttcttggat ttgtcaaaaa acaattcggt gtaattgttc ttatatgcaa 120
gtttcaatta ttttatattt tgggttatat accaattaag taaaatccac tatttttttc 180
atgtatctct accatttatt attttatatc ccttgtagta attacgcaga gcaatgtaaa 240
taattcagat attatttatt cattaaaaca tgacagattt attctcaaga taaattctcg 300
acggagttgt atattttcca ctataaaagc tgtaaacaat ttgtaaaaca ttcattattt 360
acctagaact actgctcata ttctatttaa aaataaatcg taagattatg ttcatttgca 420
taataaatat tagcaaagtt tatagtatta taatngtaaa ttatttgcat aaaggngttt 480
ataagtaatt agttttgtga 500

<210> 1671
<211> 500
<212> DNA
<213> Ctenocephalides felis

<400> 1671

acaataagga aaattttgca agcgaaagtt tttggataaa ctgaaatatc attatgttaa 60
tgtttcatac actctacaac ttattgattt tttttttctt tttttgaaaa tgatttttag 120
agtttgagtt ctgaatattt cgaaaaattt agaacacttc aaaagttttg aaatattaat 180
gatgaatgtg gcatgttctg taaataattc aacaaactaa atattttata ctagattcta 240
gttggataaa aaataattcc tgatttataa tatcattacc aaaatttctc taaacaattt 300
accgtaggca aaattttgtt agaatattgg tgttttttaa gnaatctcaa aanggnataa 360
attcccaaaa attcccattn ttggggantt nttntcttaa tnttggaana aaaatggngg 420
ttttnttcca anatctttna aaaggggtta attanttccc aanngggttt tgngaataaa 480
nattcaaaat tttgnccttt 500

<210> 1672
<211> 500
<212> DNA
<213> Ctenocephalides felis

<400> 1672

actaattgat tattgttatt ctgcaagata tgatgttccg aattattact gttccaatta 60
attcttttac gaagtataac tcatatttat ttttacacta acatacttgt tactctctag 120
acatgttttt gtttgcgttt tgcgtattaa ttacttgtgt gttttgtttc cactgaagaa 180
gagtcaaatt ggaactcgaa ccgtatggat ctaataaatg tattttaaga aacatctctt 240
agtttctttt ctattcatta tctggataga tgactgttaa aaagtttgtg ttatctataa 300
attaatttct tgaagggcgt aacatcaagc ntagagcggg ggttaggtat tcnaanncan 360
agggggaaac attatttgaa acatattggt aattttcgta tagaaaggga gtatcagttc 420
gcttattaat actaagtttg gctaagaaaa taaaaatcga ttccgcatag cgctttatga 480
gactttcgct catgataaaa 500

<210> 1673
<211> 441
<212> DNA
<213> Ctenocephalides felis

<400> 1673

aaaaaactga ggtcgaagca taagtcgcgc cttccactat cgcttattaa ttatactttt 60
actatgtatt ataaatgaat tgaaaatatt ttttatctca aatatctaaa ttgttacatc 120
tatacaggcg tttatcaaca tcaacagtag aatgcctttc tgatcttttg taaaacaact 180
tatattatca actgattttt caaaactttt gtttttttct atctgatatg ttttttgata 240
taacatgtta atgattgata taacttcaac aataaatgac atcatatttc agagaatcag 300
ttgccacaaa aaataattta ttaatattgt atgtttgtaa atttgttaag aaaagaaaag 360
caatatcttg aaaacatata tgtatataat attttaaaaa aaaaaagnnn nnnncnnnna 420
nntntnnnaa anannaaaaa a 441

<210> 1674
<211> 262
<212> DNA
<213> Ctenocephalides felis

<400> 1674

actaggcaaa tcaatattat ccttttcgca ttcagagttc tgcaaaacac tttccgaacg 60
taatttctgc atttcccaaa aaagtcttct ataaaaatgt ctatctgaca cagtcgcaat 120
gtgtttgcga atttttttat cagacatatt tatttataca atttttttg acaaattaca 180
gagtcaaaca acaaacaaat tacgaatata ttatattcta atattgttta gaattttagt 240
agcataagta tacgtataca gt 262

<210> 1675
<211> 500
<212> DNA
<213> Ctenocephalides felis

<400> 1675

acatttaata aaatttattg taaggttctg taggagaatg gaaaacgata ttgttaatac 60

aataaaatac aaaatcaatt ttttaaattt atcatcatac aactgcaaat actaacatat 120
attttttcaa ataaaataaa gttgcttttt tggtctccct taattaccct tcatatgaac 180
tttatatcaa atgaacattt tatatattga agccttttat ctgataatag aaaaaaacta 240
tagttcaaaa gctttaggag aaagaaaata tccaatgcta tgtcaaaatc tttttcggaa 300
tcttactttt caaacacttt ttatgcttgc atatgcactt tgtgaggaac tatagcagga 360
gttgaattat aaggattgta atgcaaaatt tataatttca aatggccata gctaacacta 420
aaatcaaatg ttgatatttt ctaaatattt tccaaagaaa ttcaatgaca aattttgatg 480
gttgtgtaga atatagatca 500

<210> 1676
<211> 500
<212> DNA
<213> Ctenocephalides felis

<400> 1676

acttaagaac aaaaatctgg taatcatttg tgtgaaagtg taataggaca aatagaaatt 60
tatatcattc taagtcattc aataaatgct gctaatgaat aattttcatg aataactcat 120
caaaactaat tcagatttta atgattgaac cgttattcat taaagcaatc gttttctact 180
aatatgaaat ttgcctttca actaaatttc ctttataagt tcctttgact gcaagatata 240
aagagaaaat aaatgacatt ataatgtttt atgtattcct atgtgagcca tttgcagtat 300
tcgatataat actcataaat cagtatttaa atctgagacg cctgctccat gtcataaatt 360
atgaacagtt attttcctca aatcctacac agtaattatt tgatatttat atagatgata 420
taaataaaat atctttaatt tcgttttggt taatatttta tggaagctga aatcaaaaac 480
atctgctaaa ataattaatt 500

<210> 1677
<211> 500
<212> DNA
<213> Ctenocephalides felis

<400> 1677

ctggtaacga ggaaacacta tttttaagag cagaagaacg cgattattca aatttaaaag 60
agcacaaatt tataccaaag caagatggca aaatcatgca agtgaagcat aatggagcta 120
gtcatatata tttatatttg ccagccgata ctgatttgaa agatttagat tcattatttt 180
ctcatggaga aaagacgctt ggagactggg aatcagctac cgatggatat ttctgtgatt 240
tggatgaaac caatgaagcc aaatcagttt gtatttctcc ttcagaagat acaacaaaag 300
tgaatcaatc tgagctatta gacgaattcg aaactaaata tgtcaatgaa tatgtagata 360
aaagaaagga aaaatgtccc aagtaacata aattatattc ttacaataac ttaatacata 420
tattaaaatt tttgtaacag tttatgtgta tgagtttaaa caataaatta ttaaaaataa 480
gattttnaaa aaaaaaannn 500

<210> 1678
<211> 330
<212> DNA
<213> Ctenocephalides felis

<400> 1678

acacgttttt ttctaatatt ttgagacttt gcaaattttt taaaatatcc gattttttt 60
aaattaacga aatattctct attttttgta agcaatcttt ttataacatg tcaaaattta 120
gtttttttgc tgaaaataat gaaaaatttc ggaaatttga attttttaa ctttgatttt 180
ttttgaaaat tttgattttt ttttgaaaag ttttaaaatt attaaatttt ctgtaaatgc 240
atttttgcac actgtcttct caagcaaaat gtaaaatgaa atttatctaa tcggatgagc 300
cgttctctca aaaactgcat aaccgtttgt 330

<210> 1679
<211> 500
<212> DNA
<213> Ctenocephalides felis

<400> 1679

actcatttta tatattctcg gtggataaaa tataattaat tatgtcgcca tctaacggtg 60
tgctttacaa ataaccaaaa tctggtaggg gggcttttga cccgacattt tgagctttta 120
attttattcc ttattattat gtaaaatata gttgttaaaa atacatggct aagacagcat 180
cgacgtttca tttaagttta atatacattt tggtctatcg acacggatcc agtcattaaa 240
aattacagtc cacgctttac aattaaatat ttgcgagttc gtctaaaagt gaagccaaat 300
aaagctctac gtgaatgaaa acccgggaac ctgagaagcc gccattaaag cttggaagcg 360
tcgccaggag ttcactggaa ttggaaaact ataactaaag atcacaattg gaaatacaag 420
taaatccaca attagtctaa aaccatttca aaattgctta gtattattgt tgtaagttta 480
gtctatgtct cggccgcgac 500

<210> 1680
<211> 187
<212> DNA
<213> Ctenocephalides felis

<400> 1680

aaattcctac aattattttc gaatgacata aaaaggatta aacagcctcg aaatgtcgct 60
gtttgtaatt cagcacactg taaaagcctc cagaaatcag acaggatgga tcggaattgc 120
ttgtctgcag caggcctctt acagtacaaa gccccagaaa accaatctgg ctgctatgaa 180
aagaggt 187

<210> 1681
<211> 412
<212> DNA
<213> Ctenocephalides felis

<400> 1681

acctccaaat aatgaatttg tcgttcctcc tagatttaaa ccagttcctg aatatcaaat 60
tcattaagca tttatgtatg actatgtaaa ataaatgaat ttatatttta tcatttttct 120
aattcaatta taatgatgaa gaataaaaaa aaattcatct tgtcaaagat aacttaaaaa 180

ataattaaat ataaatagat ttatctaaaa gcagttcgtc ttatgagaat ttatattgaa 240
atgctatgtt aacttgtatg gccgtgttgt attttacaaa ataaaacttc ttacctaaag 300
tggtgttggt agaagtgttt ccaaaaccaa atgtgctagt tgctggtttg ttaaatgtat 360
tattaaataa agagtttgaa gtagttgttt gaccaaatcc tccaaaacca gt 412

<210> 1682
<211> 380
<212> DNA
<213> Ctenocephalides felis

<400> 1682

acaacaggca ccaccagaga acatggaatc cgaacgccca catttcgtcc aaccaagatc 60
tgtgcgcagc atcaaggaga atccccttcg ccgcgaaaaa cgcgtcaaga gatgcgcctg 120
caactgcgaa tgttaaacaa aaaccccaag aaattattta cgaaagcatt gataaaatca 180
ccgctaaacg gaaataattt ttaattcaat aaaaaatatt ttgttaaaac atattcggat 240
attaacaaca tgtttatttc tttaatttta aactaatgca gttattaatt gtataaataa 300
aattagagat ttttattaaa gaatgacgaa aagtttagat caaagtcttg tatatatgtt 360
cttttaaaac tgttacacgt 380

<210> 1683
<211> 182
<212> DNA
<213> Ctenocephalides felis

<400> 1683

acaaaacaaa aattcggccc agtaagggct gaaataaaaa tttacattta tccgaaatta 60
aaatatttct aaatttaaca cttctgcata aagttagtgt tttagtttat gattatatgc 120
agttattttt cccggcaatt ttaaattaca tgtgaaaacg cctagctaaa tctaactaca 180
gc 182

<210> 1684
<211> 500
<212> DNA
<213> Ctenocephalides felis

<400> 1684

gacgtgcnan tttancacna nnaantgggg gttgaanccn acctgncnca aaccggttag 60
agctcagatc atggnngant gangggganga acagaccana taaagcaagt tctggattng 120
anaantantt tacaccaaca ncnacggcgc gancaatttt ggcggnangt nctactaana 180
gtaangacgc tgccatccct tangcggcgt aacntnntan gcacacacag nggccaattt 240
tcaattatat atgcttaatn aaaagangct tgntaagctc atccggtcnn cccaatgaaa 300
tatangnagc tanaanaaca tantcctant ccaaancnat ntctatacnt ctntaannct 360
tnaangggtn ntctcgtctn ntnnangtcg acgctcatnn catnanaggg nccttctatn 420
cnattgtgng aagacagcaa tatttgntcn atnattantc ngcttncaan ggaaaaacct 480
ntgttatgct cctttggcag 500

<210> 1685
<211> 377
<212> DNA
<213> Ctenocephalides felis

<400> 1685

accaaacgtt aagttactaa cagaactttg gaaataccta cagttaaaat caaatttgtt 60
tcacgcgcac ccatgctcag aatatcaaaa ataataaatg aatttgcttc tgagattaat 120
atctttggtc tgtcgatgac aaatataaaa aatcaagcca gaaaaattat ccttaaaaga 180
ctgtcttaaa tagtgattta tagtaatttg attaaatgtt taattttatt agtttttatt 240
taatgttata tatttgtagt ttatattaag tgttatgtat atatacaaag ctatctatga 300
actcttaata ctctaatttt ctcacatatt atctctttaa tttataattt gtagacttca 360
aaatgtaact gtattgt 377

<210> 1686
<211> 333
<212> DNA
<213> Ctenocephalides felis

<400> 1686

gnncntttat caagtagatc cttattgttt ttaataaata ancataaaat tattctacat 60
aatgtcttca nngcgcccct ttgttagaag caccgtaaat cgcatgaatg ctgttcgtgg 120
acaatgtaga aacagctcct acggtcatca tggacctcca gcagattatg ttcctccatc 180
catgaatgag ctaccaactc cacaaggatc ctggcaagct aaacacgatg cccgccaaag 240
gaaatataat gcaacccttc ttgctggcgt tggtgtcttt actggaactc tcatttttat 300
gaaagcaaca gggttcgact tccactacta tcc 333

<210> 1687
<211> 500
<212> DNA
<213> Ctenocephalides felis

<400> 1687

acatttactt cttacactgt atctggaaaa aatactgatt tacgctaact aattttaagc 60
atagtggttt tgttttgact tatagtataa atacatgaag cattactcaa aagggatcta 120
gtccacaggc aagattttaa tttaacagat tatagtaatg cgaaaagatt tagatctgcc 180
ataaagtaac tgaaatggcg aatcaatctc gcaccaaaca gtatttttc agaccgttat 240
aagtgccatc aatattatgt gcttcgtaat cgcataataa atctcgagat agaccagatg 300
gctccactta gacgaattta tacgagattc taaataattt attgtctatc aacctaatca 360
tatataaagt taataaccca attcagtttt ctgttaactg tggcaaatgt ttgttttagg 420
gaccacacct aaccaaggaa ttcggttgag aaatccacac gttcttggta tatctgtggc 480
atcaaaaatg aaatatttta 500

<210> 1688
<211> 500
<212> DNA
<213> Ctenocephalides felis

<400> 1688

actaacattg ttgttgattg tttctatatg tgactaacta ttggttgaga ttaaaaacta 60
atgaactgac ttctctcctt ttaacttccc gtagggtaag ttatagtaat cgcaaaaaaa 120
atttttata tttcacgttt cagaggtttc taaacatttt gaaacgtaaa gatatataaa 180
aattttcatt ttcgattttt ttttttgcga ttactataac ttgccctatg ggaagttgga 240
aaaattaagc tatgtatata catttttgaa attgattcac gatttttgga caattttaca 300
cttttctgat aattataaaa aaaaccgctc gcctgatcgt tgcgcaaaac taataaacgc 360
taactcacta agttaaatag aatggtttta gttttttca gtagaaagga gaaaacttgc 420
ttaactacac tattttctaa atttggactt attcgattca gaatgacttg aacgttgatg 480
aaacctaata ttagcccatc 500

<210> 1689
<211> 500
<212> DNA
<213> Ctenocephalides felis

<400> 1689

accgccacat gagcactaag ggagcgccaa ttaatcacgt gatcgatttt tttaaggatt 60
tttaaacctt tgcccttggt gatacgtggt gatatccaaa aattttctga tatttttcac 120
ttgtgaattt agactgntga aaaatttaaa agtttactat tgtttcatca tatcataaaa 180
aattattatc tcgctcttca ataaaacatg gatttaaaaa ttgaaataga agatccaagt 240
taaatgttgc attatcatta tttaatccag caataaatat atctgcacca aaatttgcct 300
caatacaatt tgtattagtt gcaataaaaa ccatccttat aaatttcaaa aatcacttta 360
ataatatcga ttactaacga ttattatttt taatctctta atcataggat taaaaggnca 420
cgtagccgga gtcacatgca tcgccaacta ccggattagt aatagatcac atagaattag 480
aaagaacggc catttagaag 500

<210> 1690
<211> 110
<212> DNA
<213> Ctenocephalides felis

<400> 1690

acaaactttg taaaaattta gttaataagt tacttgtaag ccatttattt ataacattca 60
aaacatctcg taagcaacat tttaatggga aaattatttt accctcacgg 110

<210> 1691
<211> 500
<212> DNA
<213> Ctenocephalides felis

<400> 1691

acgagtatta taatattttg aatttattaa cgcagcaatc ttgacccaat tatctggtaa 60
aattatactc ttttctcttt gaaaatatac tgtttgtatt tcaaaaaagt atgcatttca 120
cacattttat gagtagttgt gtgaaacaat tggatttcaa attattttaa cttaaaaggc 180
taataattat atattatttg atatcaatat taagctgtag tgatatccat tttcacttga 240
tagattacaa tgtaatttca agtaaaaata ttctaaaaaa ggacccattt attataatat 300
gttattagtg ttagaaaaaa actgaaaaat agatcaaata tagataaaac aaccggattg 360
cctcctaaaa agtcacgttg ttaagtcact tgcttacatt tttagctcat agaattctca 420
tgttctgatt gatgttcaaa aaattcaacg gtttctccgt agatcagtgg tagagctttg 480
ggattcaatc taaatccaag 500

<210> 1692
<211> 351
<212> DNA
<213> Ctenocephalides felis

<400> 1692

ncgcgttgta tttcacacta gatcttgcct gccgagcatg tttttatttt ttacaaccaa 60
cagtagntta tatgatagat gcaaatattg aattaaacaa aaatcaaagc catagttagg 120
ttacatccta aaaaaaattt tggtaaaaga taatacaaaa tatcttagaa ttcaatccga 180
gtcattgaag catttattat tgattagcaa aaaaatatga aataattaat aaatattcca 240
aatcaaatat tagaatctat ctatatttga tatcaaacac aataatactt tacaagtatg 300
ttcatatcag ttttaactta tagaattttg ataggaaaaa tcttaagaag t 351

<210> 1693
<211> 500
<212> DNA
<213> Ctenocephalides felis

<400> 1693

acnnagtagt attgaagatc ttttgncctc tggtgattnt tcattgacta tacagngtnc 60
anncttgttt aatatctgaa nnataaanat anatatatat aattatcaga ggtatttata 120
ttaataatat atacctntga aattatttca gttcccatag aactccctaa tttattataa 180
agctcgccga gatttgaaaa tgctgatgat cgaaccaaaa aatcctcatc atgtgtgcca 240
ctcagaaaag tattaagaaa tactgtttta tacttttgga caattgattt ttctggaaaa 300
taagataatt ataaaaatcc atctctaata ttttagcaac tgaattaagc acctgcatta 360
aatattaatt ttactaatgc ttcaccaact tttaatctta gctcttcttt tccatccttc 420
tgaaatttat catgtcgatt atttaaatat tcttgtgtca gcaattcaat tcatcttctg 480
gcatactttg gaaacttctg 500

<210> 1694
<211> 549
<212> DNA
<213> Ctenocephalides felis

<400> 1694

```
attagctgta gcagcattgc ttgagttttg agttgataac ctgcaaccta gcctgcgact 60
tctgttctat tgataaagga gaaataatta ttttatgtcc aaattacagg gtgataaaac 120
ccattgacgt caatttgaaa aaaaattcaa taaaaaaaca atggacagga ggatgatata 180
ttaatagaat gtgcctttat atcaataatt tcgaccacaa acactgcctt ttatgaatta 240
aaatgtcttt aaaagaatgt gaaagtgata taaaacattt aaaatcagaa tctatgttat 300
ttgcatgtga agaaaattcc aaagaaaaca gtgcaaacta tttaaatgga aaacaaccaa 360
tcaagaatgt gagtaataaa atattaaaaa actgtcaaaa tgtgacagct ccattgaggg 420
aaatataaaa aatagcagcg atattgcacg aaaacgatgg aagatattag caaaagccct 480
aaataaaccg gcacangcat caatggaaga tatttctgtg cgtagattca caccttcgtg 540
tattaaatc                                                         549
```

<210> 1695
<211> 549
<212> DNA
<213> Ctenocephalides felis

<400> 1695

```
gcgcgaacaa caacagttca gtgcgaattg ataaagttat cagcaaaaaa taataaaata 60
acagtaataa tacttgtcga taattttaaa cttgtgcatt tattactagt ttatttccag 120
aagtagaaat aagttggctc gactattgga ggaggtttat ttactcataa aacgtaaaga 180
aattaacatg taattacagg tcacaaatat ataattagca aatacgtaat ttagctagag 240
tcattcgtgc gtaaactaca gcaccgatca tttcaaagga aatcattggg aaattttttac 300
aatgtggata ccgttgtttt ttattttaaa tatcgcgttg gtatacgcag gaaaattcga 360
agaaatctat gcttggaaag acgtagattt tgtgtggcct tctaatgaaa taaaagaaga 420
atatattaaa aatggtcata catcaaggaa aataatctta tcttgggaat ggccaggtgg 480
caagataagt ttttctcaca attccagatg gaaaagtgga gtacctcaca ttggctatat 540
ccacttaat                                                         549
```

<210> 1696
<211> 549
<212> DNA
<213> Ctenocephalides felis

<400> 1696

gaggagtcgg tcctttagcc tggggtacag aaacacatcg aaaaccgcag acgcttccac 60
caaatctgag tccaaaattc ttccatagat cgccaagaga agcattgaga agagtcacta 120
gtctattgat caggaaaggg gcagctggtg gaggcacgcc ccgtgactcg cgtaaagaac 180
gcgagggcag cgtgttcccg atgccgcttg gacaaacggt gcatagggaa gctttagagg 240
aagtggtgcc taaacaaaga agaggatttt tgaaaaactt ttttaagaaa tctaaacatt 300
actcactgga ccagtaaata atcgaaaagt tgacaattta ccgagttcta tgttttttag 360
gcataagata atatgtacac tgccctcaac tttagtccta accataatat tagcattgaa 420
tactactgta gttacccgtt ttaggttgta ggattattta tttattctaa taatgaatta 480
caattaaccg tctgcatatg gaacgaaggg gaagtaagac agtttgcaat aaaatagtgc 540
ctgagaaat 549

<210> 1697
<211> 549
<212> DNA
<213> Ctenocephalides felis

<400> 1697

aattgatgaa gcacatgaca actctagctc ttctggtgat tgcattttga tagaggatga 60
tcctattgca gatactggaa ataaccaata tcttaagttt gggaaagata ttgaattaat 120
acgtatgggt gaatcaatgg cacagaatgt cgtgtctcct actgttacaa ctcctaacng 180
aggaaccatg ccaaggaaac gaggaagacc aagaaaagat gctaacttaa ttgcgcaaaa 240
gaaagaacaa cttgctcaag aaatgctaca aaacagtctc tttccagcac agcatatgct 300
tgaaggtcta atgactggat ctggtgaaag cccagttaga acaagtcgta gaagtaccag 360
aggacgatca tcagtgggta aaggaggtat gatttctacc acaccaaggg ggcgtggcag 420
aggccgtggc tctaaacagc aagctcttca ggtttcaatg gaacagcaga ggattcaaca 480
natgctgaat atccaggaca aatcattcag cagcaagctt cgggtgataa tgccaggtgc 540
atgaaaagt 549

<210> 1698
<211> 549
<212> DNA
<213> Ctenocephalides felis

<400> 1698

ccgtaaattc atattctaaa cgcacttgcg aatactttta ttcgacattg taacgttgac 60
tatgcttatt ctgtgagaat tttcatgttt tccaaaaatt ttcacaaaac tgaaagagtg 120
ctcccgtaaa ttcacattct aaacacattt gcgaatactt ttatgcgact ttgtcactta 180
gactgtgctt atcctgggcg aattttcatg ttttccaaaa atttgcacaa aagtgaaaaa 240
gtgcttcagt tcagtcacat tctaaacgca tgtgtgaaca catttgtacg acgttttcaa 300
aaaattttca caaaattgaa aaaagtgttc ccgtaaattc acattctaaa cgcatttgct 360
aatactttta tgctactttg tcacttagac tgtgcttatc ctgggtattt tttcacattt 420
tcaccagaaa aaaatgtttc gcttcacaca tacttggaaa aatgctcttg taaatcagat 480
tggtatttgg tcttttggag gtgggattgg tgnaaacgct gacctacaca atcgtgctac 540
aactcatat 549

<210> 1699
<211> 549
<212> DNA
<213> Ctenocephalides felis

<400> 1699

```
atttggtcgg tgtacgatgc ctccgaagcg tctttgaagt ggtcaatgtt catggggctc 60
aactgttcct ataggctcac cggccagttt gccttacgat cagtaatgaa ctagaatcac 120
tttaaatacc ttcgcatcaa acaattttta aatagtacgt aatttacaat tgtaaacaaa 180
attgtcattg tatatactta tatgtgtata tgtatacaca tatatttata tagttattta 240
tacatattat atataatata tatgaatatg tatatatggt gttatcgata aaattaattt 300
tcactaacac attgttaggt tttttaatat gttttaaact tcatttcgca attttatata 360
aatatgatag ataaaatgtt gtcacgataa tagttgattt gaatatatat gtagatagag 420
aatgcaatca tttggatgat atatttccca aagtgcaatg tttattgcta agatatattt 480
acataggtca tgtatctagt catgtattca agttactatt cgtgnaatga ataatcagag 540
tatttggta                                                         549
```

<210> 1700
<211> 549
<212> DNA
<213> Ctenocephalides felis

<400> 1700

```
atcgacttaa taagatncct acgaaagtgg aattgattaa gaaacgnagg tccttaataa 60
aagacatagg accttaataa gagatacagg cccttaataa gagatataga ccctttgatg 120
agacgcagtc gacgcagtac ctctgccaac ccttaggaaa gacggtggag tcgctggtgt 180
gggatatagt gtgttcgtga attttgtgca aaggaatagt gaaataagag atttgtttag 240
tgaatattgt aacactggtt gttctactgt tgctgtttta ttatttagtg aagaggaaag 300
ccaaaacaat ggtttcgtcc cagaaagtcc ctttgggcag ttgctgccgt tgctacactc 360
tacggacggg caccatcttc agtggtgtaa tgggaatatt attggcggtg gtcgcttaat 420
attgatgttt gcacttcggc gagttcaaga caataaccat agtcagtctg catcgtggat 480
cgtaaagata tattaccatt aatctacctg actgtctcat atcggtatgt tgatatagga 540
gccgtaagc                                                         549
```

<210> 1701
<211> 549
<212> DNA
<213> Ctenocephalides felis

<400> 1701

```
gcggtaacgt cattcattgc aacattaata tgggtgttcg tttacctgct gggaattcga 60
gaggtgctac aacttcccat caactggctg ttgacggagc tggttaacac tggcatcatc 120
accgtgctat atacgatcgc tttcattgtg cagttagcga aatgggtccc gggttggctg 180
gttcattcgt cgcatcaaac attacggcgg gagtttttgg aattttcaat gcattggcgt 240
atgctgccgg agtttacttt ttgcacttgg aatggaaaag cagcggcggt gcatctacga 300
actagtcctg gttattatag ttcatttact caacaccggc tgttgtattc atatcaacta 360
tttaggataa aattcttatt atatttatcg ccaaacagtt tgtatgtata agtacaaaca 420
attcgttgaa gaattatgtc gaaataacga atataaaatt tatatttttc taaatgtttc 480
tatatgaatg gttttatata aaaattgata caatatttca taatacctga tgaattatat 540
agctggata                                                         549
```

<210> 1702
<211> 549
<212> DNA
<213> Ctenocephalides felis

<400> 1702

```
aataacgctg ttgcnnttnc tggaactgta ctggcagcaa agcganccgg cgcggcggac 60
agtgggttgg ctgcgaatgt taaaacgtct gcgcaaagcg cgggaaccac ggcctttacg 120
cttaagtcca ttgcatatgg atgtccacgc cggaaattta gtgcatagcg cgtcagggtt 180
aaaactcatc gactgggagt atgccggaga tggtgatatc gcgctggaac tggcggcggt 240
gtgggtggaa aatactgaac agcaccggca attggtcaat gactatgcca ctcgcgcgaa 300
gatttatccg gcgcaattat ggcgtcaggt caggcgatgg tttccctggc tgctgatgct 360
caaagcaggg tggtttgagt accgctggcg acaaaccggc gatcaacaat ttatcaggct 420
ggccgatgac acctggcggc agctattaat ttcccgcaga catgaccctt ttagcactcg 480
gtatcaacca taaaacggac ctgtatcgtg gagggggganc cggtcccaat tcgcctatag 540
tgagtcgat                                                         549
```

<210> 1703
<211> 549
<212> DNA
<213> Ctenocephalides felis

<400> 1703

```
gctataccca ccactagaac ctgatcctgt ctttagacgg agagtttttg ggcctgattc 60
tattttaaac ggaacaccca aagataaagt agatagagta cggggaaatg attcaaaatt 120
agttataacg gcaatccaag gtttggtagc agatgttgat caaacaagag ctgctgtaca 180
aaaccaacat attgaagatt caacacgaaa tactagtgta gaggaccatg tgcatttaaa 240
tgggaatatt aagaccagtg acttagttaa atcgaaaaca tcagacccga ctattaattc 300
aaaagttcat ggagaagtag aacataataa aaatttcaca caattggatc aggaaattga 360
agacaaacct acagttaaga ttttgggcat ggagatgact acgcagtgcg aaggtggcag 420
aaatttagtc aaccaagttc accaattctt tatcagctcc aagaccagaa caattgtgcc 480
atcaatatat ttggaggact tcccataatc cagttttggt cagaattgac cacacagaat 540
tctccgttc                                                         549
```

<210> 1704
<211> 549
<212> DNA
<213> Ctenocephalides felis

<400> 1704

```
agaatcagct gtcacttatc ttggttacat tattctgtat attttcaaga tgcttcaata 60
taaatattat gatacgccag atggccaaga tttaataaaa aaattattta taactggaaa 120
atatgctact ttaactggct taggcatctc atcattagat gtgcttcttt attcaaagcc 180
taaagggtat ttggaagcat taggaagata tgcaacagtt acggggcctc ttgtgggaat 240
ggctgctgtt tttacaattg gtacctatgt agctaccaat gttcgtgaaa aagatgacta 300
taagaattat gtagttgggg ccacttcagc tggttgtcta tggggcgctt tacgcagaag 360
ctatattaca gcagtattca gttctttagc gttttcacta gctggtgtta tcaagaaaaa 420
ggcaatagag aatgattata cattatttcc tcacctaaga gacattttgt ggtcattgtg 480
gagccaaaag ccgatttact atcttaaaag acgccaaggg tggcccaggg gaaaatgata 540
acataaatt                                                         549
```

<210> 1705
<211> 549
<212> DNA
<213> Ctenocephalides felis

<400> 1705

```
agattcttag tgcgatatag gatataacat cacaattagt gaaattgatt tgaagggaaa 60
aatgtcggaa acgagcgagg gcgtccatcc gttggtatgg tccgcgggtt tcattgcctt 120
taccctcttg ttggcgcaag tatgccgcat ggccgcctcc aggacgcagc gcggaatgat 180
ccgatccttg attttggaag gaatcgccgc cgctgaactt tgtgcttcct gcttcgaatt 240
gatcatagtt gccgacaact acggtgtatc tatgtacgct attttcctgt tcgtcctgac 300
gatatggtgg tccatggttt ggggcgatgc cactgcctgc ccgtatacgc ttctggaaga 360
tgtcgtggaa gacaaagcta cattgcgcga agctgactga aaacttggc acaactagtt 420
ggcggtgtct gatatttcgt atgtcaatta ttttggtatt tggagcttct caacgcatac 480
aggaagagca tttgaaaact gacggtgatt tacaggtatc tctatgctag gaacgnaata 540
gaagattgc                                                         549
```

<210> 1706
<211> 549
<212> DNA
<213> Ctenocephalides felis

<400> 1706

```
gagagagaga gagactgttc gaggaagcaa tgaaaagtcc acttaacgcc gaacctgtaa 60
ataaatcatt ccatcagtat ttgaagccgt ttttgcaatc caatctgtga aaagaaaata 120
ttctacattt ttctagataa agtggaatga attgaaaata ttaaggagtg cacgtgtttt 180
gttttgcgaa atgctatatt atctgtccat ttatacacaa gtattcagag attaaaaaat 240
caattactta aatcttattt tgaatgccac atatttatta caaagggctt caactataaa 300
tttattttag atatatactt atcaggatat acctgataat tatgtctctt ttctgagcat 360
ataatgatgc attcacaaaa caattttgaa caaaattaga aaaatgatat aaaatgtaaa 420
tgacctattc ctaaatcatt gcttcatatt atttcgcctc catttttttc cagcggttta 480
ttatagtaat atgatttgtg aggaatttat cttaaggagc atttgtaatg aattagaagt 540
gttagatca                                                         549
```

<210> 1707
<211> 549

<212> DNA
<213> Ctenocephalides felis

<400> 1707

```
gagagagaga gagagagaac tagtctcgag ttttttatgt cgcgtttttg tatgtgctat 60
aatttacatg ttgattattg aatcttggtc ttccatttat ttgaaaataa aattcatatc 120
ttagttattt aataataata acctcaaagt ttatgacagc cagtttcata acattgctag 180
tggtagagat tattagttag gttttatcag atagtgcacc tcttaataat ctgacagcag 240

aaaaaataac aaaatatgtc agattcgatg tcacttcatt ccacatcggc aaaattgatt 300
gtggaaaatc ctttatggga gcaaaatacg ttcgttggtc ggttccgtca cttcttatgg 360
atgaccgatt ggcgatcctg ttttgcttca gaacgtgatc tcgataaagc agaagagttg 420
ataaaaaaat gcaaacgcgg tgaaggatgc ggtgatgcgc aaggtgcgac ttgattaccc 480
aagagctata tgaatcacct tccccggcac tggagaaaac aaatctttcg accatgtatt 540
cagtgcccg                                                         549
```

<210> 1708
<211> 549
<212> DNA
<213> Ctenocephalides felis

<400> 1708

```
ataaaatgac aactctcttg ataatcgcgt cgcaaaaaaa catttatcct ttcaaaccga 60
tcacaaaaac acctaaatta accagcattg tgaataaagt gtaagaaccg acacaagact 120
attaatacaa tgaccagtgt caatctgaca agaagatgtg tcctgtttgg gtggaagcat 180
tttgccaaaa caagaaccaa cagcctcata tactcttccc agagacaact tattacacct 240
gtgccccaga tatctgtgtt gtcttcaagt tttatcaaaa gcagattcta ttcgacacag 300
aaaaatgaac agagtgctat gaaaccagac gatgatgatt ctacggataa agacaaggat 360
aaggaagctg ttaagaagga ggaggatgtt aagaaaatgg gtttgttaag aagtttaaac 420
aaatgtacag ggattctgga tgtgctgatc cggtgcatgt tgcncttcta cgtgctgggc 480
tgnctattta ctcccgcaaa agtggtgtgg tgtgatagca tcctggatcg tcaagcagtc 540
tgactatcg                                                         549
```

<210> 1709
<211> 549
<212> DNA
<213> Ctenocephalides felis

<400> 1709

cttgaataac acatcacaca aaaataaaat tatctagtac taaatattct attttgttta 60
tcaaaatgtt taacataact catataaaca ctataacaac ccaagaattt tgcgaaaaat 120
tcaacaatgt catagaacac tacccggggg ctgctgaaga catggctaag caaagacctt 180
tcggaaacac cgaagatttg atacagaaat ttagtgatta tttggaaaat ttaccaaaaa 240
cagaaaaaga gctgatttta aaactgcatc cagatttagc tggaagattg cttgatactg 300
gcaatctgac acctgagtcg cagaaggagc aggaggcggc aggtttgcac aaattgtctc 360
aagaggaaaa acagttgatg accgacttga atttagagta caaaaagaaa tttggtttcc 420
ctttgtcatt gtagctcgtg aaaacaaagc tgcagcaatc ttaaacggct aaaacacgtt 480
tactaaatac aagagaccaa gactctctgc cggataataa gtcaaagcta tcagattgga 540
atcttactt 549

<210> 1710
<211> 549
<212> DNA
<213> Ctenocephalides felis

<400> 1710

aatnnaaagt gaacattata tacattgttg gttacttaga tccaagttat aaataaatat 60
taaaattcga caaatattat tcaccaaaat atattgattg caaatacaaa cgcagattta 120
ttatcaaatt ataatagcat ctcttacgta ctgtgtgttt cccagttcta atcagtgttt 180
tcgtgtttca agtttggata aaataaaaat ggccgataaa gtaccagaca ccaaccaatg 240
cgccttgtca cagaaaaaag tcgaaggggc caaaagcaac ccttctttgc tgccccattt 300
ggagtctttg gaacgcatga tgaaactgcc tgtggtagaa gcggcctggg cccaatctca 360
gggcgtttat gataaagtta aaggttacaa cccgatgctg acatgggcat ttggaacagc 420
gaaagcacag tcaattggcc tggcaacagc ttcccttaca tccagaaaat tncaaaccaa 480
tccactatgt cgatgagacc tcgcaaagga tcacaattag aagctagttc atcgcangac 540
acctagnga 549

<210> 1711
<211> 549
<212> DNA
<213> Ctenocephalides felis

<400> 1711

aagaactcac tattgtttat cattccatgt gtattttcat atattctaga agtntaaatt 60
attctaaagc aaatgcttaa atatttggaa aatatattgc tatgtgtttt tatatgagtt 120
catttgatat atgaagtatc ggagcatgtt ttatttgtgt tttatttatc atcgtatatc 180
taattgttta tttaactgaa atgtgtttaa aaaatattat ataattctta cttatttatg 240
ataaaaattc aatttccatt taattataag attctttgac aggttttaag actgttgatt 300
cgttttaacc acatttagag caagagttaa ggttaacata atcagtataa tatttcaata 360
atgtgacaat gagtaatatt tgtactattg taattgtact gataacacaa atctctaaca 420
tataaaacat attcattttt cttcaaacat aactaaatcg catttttgat tatttgacat 480
atatatatat acatcatcat cntacatata tatatatata tatatatata tattatatat 540
aaaaatatg 549

<210> 1712
<211> 549
<212> DNA

<213> Ctenocephalides felis

<400> 1712

```
gttttagtgc tcaagtgctc caaggtaatt agccagcngg tgaagaaata gtgagtaaat 60
atcaatcaaa gtgtttttta ttaatactgt tgaacatcaa aatgagtgta aaaattgaaa 120
atattgaatc ggaaaatcca aaaataatac aaccgcagta cagcgtccaa aatgtctgcc 180
cgcttcaaac aagcgctact gaagcgcctc tatcaggaga aaaaggaaat gatagtgata 240
gaaaagaagc aaaccaagtg ggtgcaacaa atgggaagaa gaaaaaacat agacgaggta 300
aatgtaagag aaaacctaat aaaccgtaca ataaaacagc ctggacccaa cgtaagaatg 360
tccaaaaaga aatgatgaga gttcgtagtg cccgtgctaa aatattagcc atggggcaca 420
cattagttcc ttgtaacacg aaccaatttc tcatggaaga tcatgatgtc ctcaccaagg 480
attcatctgc agactcggac tctacttagt gtcgttctga agacactctn atgtcttcct 540
gagatgagn                                                         549
```

<210> 1713
<211> 506
<212> DNA
<213> Ctenocephalides felis

<400> 1713

```
gtnttagaga ggttatttaa gattgttata ttttcataaa tatctcggac atggctacta 60
agtttattgt aaaatgtggt attgccgggg gcatagttta tcaatcagtt gaccaaggac 120
tatggggtag cagttctaga acaattgaac tttatgaaga tttatctaaa ctcgtggaac 180
cagtaaccaa agaagttaag caaaagattg agcttccaga tttgcctaca tctggtgaag 240
ttggtttcat tgcaacttat tattggaatg caggtgtgaa agctacattt gcatttttaa 300
aagaatttcc aacgaatact gctcgattgg gatgcaaatc ttataattat attgtaaatc 360
atcctgaaat aaaaaaattg caggagcaaa cagaaacaaa aacagaaaag aattgatatg 420
tatatagaaa aaaatcatga ttatggatta tgtagataat atttaataat aaatgctata 480
catcttaaaa aaaaaaaaaa aaaaaa                                      506
```

<210> 1714
<211> 549
<212> DNA
<213> Ctenocephalides felis

<400> 1714

```
gagttttttt tttttttttt tgaaaaatat acactaagta attacattta tattcaattt 60
acatttgtac atcattgtaa ttgactacga attaacagca atgataaaat actttatata 120
tgtatatata tatatatttg aaaatctgtg taaaacccgt tacaaaaata ntatacanga 180
caaaaaaaat attacatcat ggcaacctcg actgtccatc tntcaattaa aatctacact 240
atcttttgtt tgttattaac attttcctat tggatttaat tattaattta aatgctttca 300
atgattcaat tagcggaatc attttgcttt ggatatcttt tcacatatct cgtttgtgaa 360
ttctgaacac ttagcgttgc tcctaaatca cctgttaggt atttggcttc acggattgct 420
cgaaggctgc tgtttaatta agtctgcgtg tttgaaggtc atgtgctcag catcattcgg 480
aggatacaag agggcagtgg ggttgctttg cttgccgctt gctgggtgnc gtgccagatc 540
aaangcgct                                                         549
```

<210> 1715
<211> 549
<212> DNA
<213> Ctenocephalides felis

<400> 1715

```
aantntcgtc tgtgaatatt aggaaaaagg cgggttttaa agaatttgca attgatatta 60
agtggttttt tatttaatta gtttaagtgt tatataagtg caaaatcatg acgttaatag 120
tgaaatggct cagttctttt ctgctgatcg catccttggc attagcacaa gtgaccccca 180
aggaccccgc ctgcatanaa ggcttcgatg gcaacatcca cgccctggac gcggctgaat 240
tggacttctc tttacgaatg ttagctgctg cagttcgatc tgcacctggt caaagtgtat 300
tttttttcacc ttacagcatt tatcaagctt tgctcttagc gtatttctct agtgctaacc 360
ataccgaagc taacttgaag aaaacattag ctatcgagga acatgtgcca aaactacaag 420
ttctgcatgg atataacttt gtgagaaaaa tgcttggata tagaacgaat caatcatatg 480
aatcaatntg ctgtcgcttt tgtactaaga tgaccanttn ggattgattc aagctgcatg 540
agagacaaa                                                          549
```

<210> 1716
<211> 549
<212> DNA
<213> Ctenocephalides felis

<400> 1716

```
attcnatgat acaattttgt ataaattaga atggccagga agaactgacg aatttttaga 60
acctccaggt agtgagtcta tgttcatcac aacagctgat aaagaacgat atcaatgttt 120
tgttcctaaa atctcaacac cagagtctga aaaaaattta ccatatagtg gaccaactgc 180
gttagaatta ttggcacctt tattcacaca aactgcttgt tcatatcgtg ttgaaagcta 240
ttggacatat aagctttgcc atgggcggta tgtgcaacaa tatcatgaag aacgagaagg 300
gaaaaaggtg aaaactcaag aatacttttt gggaaagtgg tctgctgaaa ggcatgatga 360
attattggct gaaatttcaa aagcagaaaa aagctcagaa cctttgcgaa ccacaaaaat 420
tgaatcagca gtttgctttt gtagaagtag ttatgacaga aggtctttat gtgatttaaa 480
taataaaaaa cgagttacaa gagttctgat gttgtatgca atggaaacat gaaattattc 540
ctaaagaaa                                                          549
```

<210> 1717
<211> 549
<212> DNA
<213> Ctenocephalides felis

<400> 1717

```
aataccggtg gcgaaggcgg ccccctggac gaagactgac gctcaggtgc gaaagcgtgg 60
ggagcaaaca ggattagata ccctggtagt ccacgccgta aacgatgtcg acttggaggt 120
tgtgcccttg aggcgtggct tccggagcta acgcgttaag tcgaccgcct ggggagtacg 180
gccgcaaggt taaaactcaa atgaattgac gggggcccgc acaagcggtg gagcatgtgg 240
tttaattcga tgcaacgcga agaaccttac ctggtcttga catccacaga actttccaga 300
gatggattgg tgccttcggg aactgtgaga caggtgctgc atggctgtcg tcagctcgtg 360
ttgtgaaatg ttgggttaag tcccgcaacg agcgcaaccc ttatcttttg ttgccagcgg 420
tccggcggga actcaaagga gactgcagtg ataaactgga ggaaggtggg gatgacgtca 480
agtcatcatg gccttacacc agggctcaca ctgctacaat ggcgcatcaa agagggggnc 540
cgtcccaat                                                         549
```

<210> 1718
<211> 549
<212> DNA
<213> Ctenocephalides felis

<400> 1718

```
taacgttact aacaaatcgt catctacttg taattattaa gtaatacttc ataattaagt 60
cctgaaaaca accagggcag cgggtttaaa taaattgtcc tcaacatttt ttcttaaaat 120
agaggcgttt tgtatgccaa atattcatga atatgtactt atttcaagtt tcgtttttag 180
aaatactatt taggtattgt aatagcatgg actatcattc caaaatccat atctattttt 240
ataattgttt ttgtagaatg atattaggag agacttatat gaatgatttt gaacaataat 300
ggttgaaata aactattcaa tcttattact ttttacatat atgtatttat ttacatataa 360
atacatattt gatttcaatt tgattcaatt gtttgttgaa ttttttgttg atagatcttt 420
ttgcagtgtt ttctcgtaag ttaattcaaa tatatgcggg cataagtaat tcaatagaat 480
atttttctgt atacttctat cgtattcatt gaatatatta attgnaaagn gctatttata 540
tcggtaaaa                                                         549
```

<210> 1719
<211> 549
<212> DNA
<213> Ctenocephalides felis

<400> 1719

```
agaacagatt ttcaaagacg ccaatgacag tattacactt ttggctccca atgatgaagt 60
ctttgaatca ttgactgaag aagatcttaa tgtattgttg gaggataaag acgccgctag 120
tgagacattg aaaatgcatg tacttccaga gattttatgc tgcaccggaa attggccata 180
acgtatggcc attcttggaa tctgtccgat ctttgacaag gccattaaat atcaaccgtg 240
atccaaatac cgatgaagtg tatattggaa ccacaactgt cgcccaatgt gatgtaatga 300
atattaatgg agtcatacac agagtcgaca aggtaatgtt acctcaacga cctaaagtaa 360
gaatgccttt cttccgtcaa ttcatgtttt actaagcact gattaagcgt gaatatatat 420
tttcatatac caaataaagc cattagtttg aattttcgat atttgtaaaa tgagatgatt 480
gaaatatttc attaggacgc taaatctaat tgagtattgt attaattaaa aatttataat 540
taattatag                                                         549
```

<210> 1720
<211> 549
<212> DNA

<213> Ctenocephalides felis

<400> 1720

```
ataaaaaata agagaaaatg gtcgtttgta gagttatatc attatcttgt cgccgtttac 60
atgcacctgc caaatttttg aacccacttt tgaattcaca aaatcaattg gcaaggacga 120
gtattaaata tatttctaca tcacagatta actttgcaga acgtaagtac acagataaac 180
atgaatgggt tgttgtcgaa ggcaatgtag ggactgtagg aatctcaaaa tatgctcagg 240
aagccctcgg agatgttgtt tatgcccagc tccctgatgc aggtacagat ctttctcaaa 300
aggatgaatg cggagcatta gaaagtgtaa aggcagcatc agaattatat tctcctgttt 360
cgggcaaagt tactgaaaaa aactcagctg tagaaaattc accagctttg attaacacat 420
catgctatga tcaggttttg ttacaggntg gtattcaaag taaatctgcg aagcctgaaa 480
ggtgaataaa ttgatgagtg agaaaagtat gagagttttg aagannaccc attaaattgg 540
ncattaaan                                                         549
```

<210> 1721
<211> 549
<212> DNA
<213> Ctenocephalides felis

<400> 1721

```
aaaccatacc tagtccaatt tggcatttag aaacaattgc aaaatataaa aactaaataa 60
aaaatgggtg tttacgattt gttattcagg aggacttcca catttgtagc cactattatg 120
gcctcgacgt tctttttcga aagaacgttc gaattaggtt ctgaatacat gttcaacaaa 180
gttaatgaag ggaaactgtg ggcgcacatc aaacataaat atgaataaac aacaactcaa 240
attggataaa gtagagttta ttatataagc tagacaaata aatttttatt ctgttacttc 300
ccaaggcttt tctggtctgt cagcgtccca tggttgagcc ttcttgaatc ttgctggttc 360
attgagtgca acaccacctg aaaaatcaat tatgtaataa aaagtgtaaa atataatata 420
gtagtaacag aaaaaaaaaa aaaaaaaaaa aaaactcgag gggggcccgg acccaattcc 480
cctataggag ccgattacaa tcactgggcc gcgtttacac gcggactgga aaccctgcgt 540
acccactaa                                                         549
```

<210> 1722
<211> 549
<212> DNA
<213> Ctenocephalides felis

<400> 1722

```
atttatttag taactttaat tagtgtacag tattagcaaa caaaatatga tgaacattca 60
aaaaatcgtt aggagcttaa accgagctgg agttgcaaga atgtcaactg gacagtacgg 120
tgatggtgct ggcaaaggag gtggtggtgg tggatccatc cgtgaagcag gtggatcttt 180
cggcagaatg gaagctgcta gagaagaaga attcttttat aaacagcaac aagcccaatt 240
gaagaaactt aaggaacaag caatagatca gaaaactttc catgaggaac aattaaaact 300
tcacaaggag gctttggaga gacatgaaaa acatttggct gagcttaaga agtaaattga 360
agtttcatat atagcattat gaaaatgtta gacagtgata aaaattcaaa tatttgttac 420
tatttactga attaataaac ttgcattaaa gcttctataa gttattagtt ttctagcatt 480
cttaatatac tcattgcact acattagttt taaattctgg tntatnatgt atgnactaga 540
tttgnaata                                                         549
```

<210> 1723
<211> 549
<212> DNA
<213> Ctenocephalides felis

<400> 1723

```
aatctgctta atgcgcgcaa acacctctgt cagctcgctg acattccccg ccagacgctg 60
atttccagc tcagcagtcg ctttacttct gctaacataa ccgggatcgc ggcgagacag 120
ggtaaactcc gccagaccaa tcggatttga acgataagaa gaggtttcac cggaaggaat 180
cagttcgtcg gtggtggtca cttcgtcgag gatcttcgag cacactttca ggacgatatt 240
gtcagtcagc gcacccaatt ccggccagtc tttaatgttc ggcccgtaaa tcagcggttg 300

ctgagttgcc cctttcacaa agccctgata aacacggttt ttatacggcg ttacatcgaa 360
ggcgtactcc ggcacgttgt cccagcaatc aagttcgctg gcagaggtta aatagccacc 420
gtttgcgcag tcgcagcgat agaacgagcg tcatcaacgc caccgctgac atctgccatt 480
agctggctta gagccttcgc ggtcggaagt tncgcgtggg tggcggatct cacgcagcgg 540
acagcgttg                                                         549
```

<210> 1724
<211> 549
<212> DNA
<213> Ctenocephalides felis

<400> 1724

```
gtcagacgga gacgtgctcc aaacgctctt ataaaagaaa ttgttaaatc tattgaaatc 60
cttattcatc ggtgcctatt ttcggcttaa atatggttca aaagaagcct aagaagaagg 120
tcggaaagaa agtggcagcc gcccctttgg ctgtgaagaa gtctgaaccc aaaaagttgg 180
tgaatccttt gttcgagaag agaacaagga actttggaat cggtcaggat atccagccaa 240
ccagagactt atccagatgc gtgagatggc ccaaatacat cagaatccaa cgccaaaaga 300
gcgttttgca gaagcgtttg aaggtccctc caccaatcaa ccagttcacg cagactttgg 360
ataaacaaac agctacacgg ttgttcaaga ttttagaaaa gtacaggccg gagactgtat 420
tgctaagaag gnacgtttgc aacacgtgct gaagctaaag ccagggcaaa gaagacacac 480
ccacgaacgt ncaatgttgc gttccggcac aacactgcac aaantgtgac aaaagaagna 540
cagnttgcg                                                         549
```

<210> 1725
<211> 549
<212> DNA
<213> Ctenocephalides felis

<400> 1725

```
ctttgggggt aagcggggtt cctgtaccaa aagattcatc agtccgcatc aagtgtccgg 60
gttgccaagt tcgacccgtg ttccattcca atctttctag ccagactcac aaaaaactac 120
aaccatggct gacgatgagg agaagaagaa gaagcaggcg gaaatcgacc gcaagcgtgc 180
tgaggtgcgc aagcgtatgg aggaggcctc caaggctaac aaggccaaga aaggtttcat 240
gaccccagac aggaagaaga aacttaggtt gcttctgcgt aaaaaggctg ctgaggaatt 300
gaagaaagaa caagaacgta aagccgccga gaggaggcgc atcattgaag aacgttgcgg 360
taaaccaaag aacgtcgatg atgccaacga agaggcggtt aagaaagttc tgcgcgatta 420
tcaccaacgt atctgtcctt ggaagattcc aaatatgata ttggatctcg taaacgcaag 480
gccttcgaga ttgccgtctc aactcacagg tgaacgacct agangaaaat tatgaacccc 540
cctcaagaa                                                         549
```

<210> 1726
<211> 549
<212> DNA
<213> Ctenocephalides felis

<400> 1726

```
aattcggctt cctgctngac gctctgaaat acggtactcc gccgcacgca ggtctggcat 60
tcggtcttga ccgtctgacc atgctgctga ccggcaccga caatatccgt gacgttatcg 120
ccttcccgaa aaccacggcg gcagcgtgtc tgatgactga agcaccgagc tttgctaacc 180
cgactgcact ggctgagctg agcattcagg ttgtgaagaa ggctgagaat aactgatatg 240
actcaaatac acgaaatcat tcgcgttgca tcgaggcggc aactgagtga actcccatga 300
gcatagataa ctatgtgaat gggatgagcg aaggcagtca acgaagaggc agcgtgaagg 360
ataaagtgta taagcgtccc gtttcgatct tagtggtcat ctacgcacaa gatacgaaac 420
gggtgctgat gttgcagcgg cgtgacgatc ccgatttctg gcancggtaa ccggcagcgt 480
ggaaaagggt gaaaccgcgc cgcaagctgc atgcgcgaag taaaggaaga gtaccantgt 540
gttgcgctg                                                         549
```

<210> 1727
<211> 549
<212> DNA
<213> Ctenocephalides felis

<400> 1727

```
acgagtcatg tgcttccgga tggctgacga cctacagctt ccgatgtcag ccagtcgatt 60
attcgaggtc gcccgaagcc atgcggatgg tgcatggctg ttggtggtac tacttctcga 120
agttcacgga gtttttcgat acgattttct tcgttttgcg taagaaaaac aaacatgtgt 180
cgacactcca cgttattcat cacggatgta tgccaatgtc cgtctggttt ggcgtcaaat 240
tcactccagg tggtcacagc accttttttg gcttcttgaa tactttcgtc cacattatca 300
tgtattctta ctacttgctt gccgcacttg gacctcagta ccagaaatac ttgtggtgga 360
agaaatacct gactggtctt caaatggtac aattcgtcct ggttatgatc cacgccttcc 420
agctgtgtca ttgaatgtaa ttacccacgt gccttcgttg ggatcggca tgcatgcagt 480
catgttctac ttctattctc tgtttctaca aacagactta cacaagaaag aaagaagaga 540
agcgatgaa                                                         549
```

<210> 1728
<211> 549
<212> DNA
<213> Ctenocephalides felis

<400> 1728

```
aatttcctgt gaggtgatta ccctttcaag caatattcaa acgtaactat cctttaattt 60
tcggatccag cgcatcgcgt aaaccatcgc ccaacaaatt gaacgccagt acggtcagaa 120
aaatagccag ggccggaaaa acagcgacat gcggcgcgat aaccatatcc gctcgagcct 180
cattgagcat tgctccccac tctggtgtcg gcggctgcgc accgaggccg agaaatgaga 240
ggctggcggc agagataatc gaggtaccaa tgcgcatggt gaaaaacacc acgatagaag 300
agacggtccc aggcaggata tgacgcaaca aaacggtcat atcgctggca ccaatactgc 360
gtgctgactc aataaaggtt tgctgtttca acaccagcgt gttgccgcgc accaggcggc 420
aaacgcgggg cccggtaccc aattcgcctt agtgagtcgt attacaatta ctgccgtcgn 480
tttacaacgc gtgactggga aaccctgcgt tcccaactta atcgcttgca nacatcccct 540
ttngcagct                                                         549
```

<210> 1729
<211> 549
<212> DNA
<213> Ctenocephalides felis

<400> 1729

```
aatactctgc gcttcgtcat aggcttcaat acgcgccgcc agctgctgcg aagagagcgc 60
cttacgcaat acgctgtcga atttcggatc gcaccagtgg gcgaggttgg tctgcgaatg 120
aattgccgcg cagctcagta acggacggaa gaaactgtcc gggtcgttac tgtccgtcgc 180
ccaaccggat aacgtcagat catggctcat atccatcaac cgcgcctcct gaaagcgacc 240
ttctaccggc acaatcacca cttttacgcc aacctgcgcc atatccgcct gaatcagttc 300
ggcagttttc agtggactgg ggttccacgc ctgcgaacgt gtgggggccc ggtacccaat 360
tcgcctatag tgagtcgtat tacaattcac tggccgtcgt tttacaacgt cgtgactggg 420
aaaaccctgg cgttacccaa cttaatcgct tgcagcacat ccccctttcg cagctggcgt 480
aatagcgaag agcccgaccg atcgccttcc aacagttgcg cagctgaatg ggaatggcaa 540
ttgtagcgt                                                         549
```

<210> 1730
<211> 549
<212> DNA
<213> Ctenocephalides felis

<400> 1730

```
gaaagggatg gcgtcttatg ccctaatatg gcctgtttcc agtattatac aacagacaat 60
ggaaggaaaa agattagaaa attacgattg gggtcgttgt ttacgattca gtttatacgg 120
atgcctattt actgctccca ctctgtatgg ttgggttcga ttatcaagtt caatttggcc 180
acacaataat tttaggacag ctgtaactaa agccctcatt gaacaagtta catatggacc 240
attcgctctt gccagtttct tctttggaat gagcttgatg gaaacacata gtgtggacga 300
agcagttaaa gaagttaaag ccaaattttt gcctacttat aaagttggtg tatgcgtatg 360
gccgtactcc aaacaatcaa tttttctcta gtaagtgaat caaacagggt accttttgca 420
gttgtgcagt ttgatgtgga catcattctt ggcatatatg aaatataaaa atgcccgatg 480
gaatcgccag caaacacggc aacagcctct tgggaaaaca atcaacgcct ctaaccatct 540
ttgataagt                                                         549
```

<210> 1731

<211> 549
<212> DNA
<213> Ctenocephalides felis

<400> 1731

```
aaacgcctgt aacgctggaa gccttagccg accaggtggc gatgagtcca tttcatctac 60
atcggttgtt taaagcgact accggaatga cgcctaaagc ctggcaacag gcctggcgcg 120
ctcgccgttt gcgcgantcg ctggcgaaag gggagagcgt gacnacgtnt nttnttaacg 180
ccgantnccn gcagggngca gttctnntcn aaaattncan aannctnngc atgnnaggta 240
nanannttcc nnncgggggg gaaaanatgg gggggngctt tcgctctgtn ngnttgngat 300
cnggctcttn ccctggnggc aaaangngag cggatntttg cgntatnttg gtggcccgnc 360
cncantccnc cttatngnga gtcatatnac aatacagngg ccgttttcna cagcgtggan 420
agngaaaaac cggtgtnncc cctattagcn tgtngaaant cccnttttcc gntngggata 480
ataaaagggc cccccactgn ccttttccna attgcccccc natggggaan gggaatttga 540
gggttattt 549
```

<210> 1732
<211> 549
<212> DNA
<213> Ctenocephalides felis

<400> 1732

```
caacgcttgt ggaantgcaa tccaccaaat ttgaatgcca acttgctcca gttggagatc 60
cgaacatgaa ggttgaatgg ttcctaaatg gcaagccttt acctcacaaa aatcgtttca 120
cgccgatata tgactttgga tatgttgcta tgaattttgg ctgggtctac cccgaggata 180
gtggagagta tttatgcaga gcaacaaatc tttatggtat ggatgaaact agagcgatta 240
taaaaactgc tggtagacca ggaatcatat atgattcaca acttcccaaa catatgaaga 300
gcattgaaag aattagggag atggaagcag cttggcaaat tgtgccggac gaacctgatg 360
aagaatctaa acctaatgtg cacctgtgtt tgttagcaaa ccagaccctg ccaacggaag 420
aaggcgaatg gctagattct gctgtagagt acaggtcatc cacgtcctan agttatgtgg 480
gtagggtaat ggtacactga gtcaacggtc aagattaaat tacatatgat ggnatgttca 540
catggnttn 549
```

<210> 1733
<211> 549
<212> DNA
<213> Ctenocephalides felis

<400> 1733

aagttgtcga catgcttgca ctggatgccg atgttgtatg cagatgtgcg ggtggaaata 60
atgctgggca cactgtagtg gttgaaggca ctgaattcga tttccattta ctgcccagcg 120
ggataatcaa caaagactgt atttctatta ttgggaatgg tgttgtcatt cacttgcctg 180
ttttattgag gaattggaaa aaaatgagaa gaaaggtcta agtggttggg aatctcgtct 240
tgtaatatca gatcgggcac atcttgtatt cgacatgcat cagcaggtcg acggcatgca 300
ggaatcagaa aagggtggat taaccattgg cacaaccaaa aaaggcattg gtccgacata 360
ctcttctaag gcaacccgta atggattgag agtgggtgat ttattgggag attatgattt 420
gttcagccga aaatttaggc agtggctgct ttgtccaaaa atgtttcaag tcttgaattg 480
ccgtggaagg gaattgaaag natcgcnatt tgcagcaatt ggcaccnttg taaggatctn 540
atcgatttn 549

<210> 1734
<211> 549
<212> DNA
<213> Ctenocephalides felis

<400> 1734

atgatatttc acaagcttta attccatatt ggagaaaaga ctgcatgaga aatgaactga 60
aatcccttaa gaaaacatta gatgacaaag atagaatggc aaaagctgct gttatgaatg 120
acattgttga gaaggtaaaa gaattgtgcc tttcaagtga atcagatgta tttgttaaac 180
aactgcaagc agattcaaac actaaagcct tagatgctgc tttaaaacaa ataaaattat 240
tgaaaccaga tgcatcagcc atgtttttct ctgttgacca agacagtggt aaaatattct 300
gtttagcttc tgcttccaaa caagcaattc aaaaaggatt aaaagcaaat gaatgggtta 360
atcatataac aaaaatcata aatggtaaag gtggtggtaa acctgaatca gcacaagcat 420
tggtagcaat atcgcaaaaa ttgatgagtg ttaatagctg caaaagaatt tgcagatttg 480
aaatactgna aaatgntttt acttctgcat tgttgaaaca ttnactatat cttagcattt 540
atccgtatt 549

<210> 1735
<211> 549
<212> DNA
<213> Ctenocephalides felis

<400> 1735

gcaacaacaa cctcagaatt tacatcacca caatactagt cagcaacatc accatcaaca 60
acacagtagt aacaacaata tatccagtgc tggcaacatg aatccgcaac atgtgaatag 120
tcatctttct caccacagtc cattatcttc atccatgagc catcatcata caatgcacac 180
accttcgtcg atcaattcgg ggtatcagca tgtgaaaaat gaacctagtg aatacgatta 240
catgaataat tgtcttcctg gaagttattt cacaggaacc agttttggta ctcccctgag 300
tcattcacag agcagtgctg tagattccct gagtgggtac catcatcaac acaatgttat 360
ccaagccgct aaattgatgg caacttcttg aaattttggt ttgaaataag taaaaatact 420
aaagactaat aaaaaatgta aatgatatag aagtcaacat atttgactat caaaatataa 480
tgcaaaaaaa tgttgtgtga caacattgga tggcaagtag caaggcaata tcaagcataa 540
ttacataat 549

<210> 1736
<211> 549
<212> DNA
<213> Ctenocephalides felis

<400> 1736

```
atngaatgat ggcagttact gatcctgcat tatgtagaag agttgcttta agacatacct 60
gtttttttagc agctttgtca ttatcagctc cttttctaga tgttactaat tattggtttg 120
ctgctgaaac gttaccattg aacggatact ttatgtactt agcttggaaa ttttataaag 180
aatcggacag taaaagttct agaaaactat ttagattttc actaattcat ttacctgcat 240
tgatgttatt atttttatta aataaaaagg aatggttttt tacgaaagac tcaacattgg 300
aaacaccttt ggatatcgat gaacacaata aaactcaaac aataagcaat aaacctgcaa 360
aacaaacagt tgttgtacct tcagctattt tagataaaat gtaaataata tataacaaaa 420
aatgttttag taaaaaatag aacaattaaa gatattatga gaacaaaaaa aaaaaaaaaa 480
aacctcgngg ggggcccgga cccaatcgcc tataggagcg atacattcct ggncgngttt 540
acaccgcgg                                                          549
```

<210> 1737
<211> 549
<212> DNA
<213> Ctenocephalides felis

<400> 1737

```
aaattttctt ttaataagaa aaatttatta ttaagtttat taattttaga atataaagct 60
ttaattatat ttttaatttt atatttttgt tttaattata taatatttga gaattatttt 120
tgtaattttt atattgtatt tacagtatgt gaaggggttt taggcctttc tattttagtt 180
gcaataattc cgtactcatg gtaatgatta ttttaaaaga tttaatataa tacaatgtta 240
aaatttttaa taataatatt ttttataatc cctattattt tattaaaaaa ttgttattga 300
ttggttcaaa caatattatt tataatatta tttatttata taattttagg aataaaaaga 360
attataattg ttaatattag attaaatttt ggttatgata ttatatctta tagattgaat 420
ttattaagga tttgaattat tattttaata ttaatagcat caattttaat ttataaaaat 480
aattataatt taaattattt ttattattat taatttttta ttattaatat tatttttacc 540
tttagtcat                                                          549
```

<210> 1738
<211> 549
<212> DNA
<213> Ctenocephalides felis

<400> 1738

```
aatgctacac tggctaccac taaccctatg tatgtccaga tgcgaagagg gaatgtggaa 60
aagcttgtaa taccctcaag tgctaaattc caaagtttcc atccattaaa ttttgtatct 120
ccagcaattc tttccgctcg cacgtattca acaatatctg tctttcctcc tacccagctc 180
agaatacctt tcatgaaaag gtttcgttct ggcataagtt taatattttc gacaacatca 240
cggctcatca gcctgaaatc accaacattc tcttcaattt taggattgct tattttattg 300
tggagcttat agaaccactc agccgttttt cgcttcaggc gtccatcagt tgagcggtca 360
gatcttttag caagaaccat atcagcacct gcttgccatt tttcaataag atgaggaata 420
acctcaatcg ggtcttgcag gtcaacatca attgggatta tcgcatcccg gtacccaatt 480
cgcccttagg agtcgtatta caattcactg ccgcgtttac aacgcgtgac tggaaaaccc 540
tgcgtaccc                                                          549
```

<210> 1739
<211> 549
<212> DNA
<213> Ctenocephalides felis

<400> 1739

```
gccagacgga gggctaacac tgcctttaac ttttcattac tccagataac tttcaaaatc 60
aactgcaatc atgtccgacg aggaagaggt ctacagtgaa gaagaggagg aggaggagga 120
ggtcgtggag caaaaacaca cgaccaaagt agaaggcgat gcagctgctg gagacccaga 180
attcatcaag cgtcaagacc aaaagagatc agatctcgat gatcaactca aggaatacat 240
cttggaatgg cgcaaacagc gtgccaagga agaagaagat cttaaacgcc ttaaggaaaa 300

gcaagctaag cgcaaggtat cccgcgctga agaagaaaag cgtatggccc aaaggaaaaa 360
ggaagaagaa gaacgcagaa taagagaaat tgaagaaaag aaacaaagag acattgaaga 420
aaagcgtcaa cgtctcgaag aagccgaaaa gaacgccagg ctatgcttca agccccaagg 480
atgccacaag aagggcccac ttccctccca aaagacagac ttcactgttt ctgccaattg 540
aagaacaag                                                         549
```

<210> 1740
<211> 549
<212> DNA
<213> Ctenocephalides felis

<400> 1740

```
aaggaaaagc tgtttttgaa ggtgaagcaa cttcagaaga ggctcttaag aaattcgttc 60
aatcacaatc gttgcccctt atggtagaat tcaaccacga gactgcacaa aagatctttg 120
gtggagatat taagagccat ttattattgt tcctgtcaaa ggaaggtggg cattttgaca 180
gctatgttga gggagcgcgg gaggttgcaa aagaattccg cgatcaagta ctgtttgtta 240
caatcaacgc tgatgaagaa gatcaccaaa gaattttgga attctttggc atgaagaaag 300
aggaggtgcc tgctatgaga ttaatcaagt tggaagagga tatggcaaaa tacaaaccag 360
ctacaccaga cctttcagca gaaaatatta aagaattcgt aggaagcttt attgaaggca 420
aattgaagca acatttatta tctcaagatc ttccagaaga ttgggataag aatccagtta 480
ggtttagtca gctccatttg atgaagtaca ttcaattcag aaaaggtggt ttgnggaatc 540
tatgctcca                                                         549
```

<210> 1741
<211> 549
<212> DNA
<213> Ctenocephalides felis

<400> 1741

aattgtccat caaacacgta atacgttttc aactaaagaa cactggctgt ggtgttattg 60
ggttattttt ctcggctcaa tcacggtact gcaggggata tacgtcttag tcagttccga 120
tgcaagcgcc cgactggctc ccggcattat tcttatttgc ctcggaatga tctgttacag 180
catattctca aaagtctggc tactggcact ggtatggaga cgtacctgtt cgttagccaa 240
cagaataccg atgattcccg tcttcacctg cctgttttgc cttttcctgg catcgtttct 300
tgcggaaatg gcgcagaccg acatgggata ttttattcct tcgcgagttc tggtcggttt 360
gggagcggta tgctttacgt tgttctcaat cgtttcaata ttagaagcgg gttctgctaa 420
aaaataattg caacgtaccg gataaaacca gcgttgacca tttgcgtaac gctggttttt 480
cttaggcatc atgaaataac gcacattaat gcatagtggt aagtataaaa aacagcaagt 540
actgttttt 549

<210> 1742
<211> 233
<212> DNA
<213> Ctenocephalides felis

<400> 1742

tcacggtttt agaaatggaa gtaggatcaa tatcgatatg cagaacagtg gcatttgggc 60
agtactttgc cagattgttc gtcgttcggt catcaaatcg taccccgacg gcgaaaatca 120
catccgcgtt atgcatcgtc atattggctt cgtaggtacc gtgcattcat caccatttgc 180
tgcacccgca gagcgctaag gcgcagttca ttcatctggt taacatactc aag 233

<210> 1743
<211> 549
<212> DNA
<213> Ctenocephalides felis

<400> 1743

aagtcgctga acataccaac ctcgaaacgc actttatcga ttccagcggt ttaatctcct 60
gggacctgtt caagcaggat gccgattatc cgtttgtgga ctggaatttc tccggcacca 120
cggaagaaga gttcgccaca ctgatggcta tcagaaaaag gccatctctc agcgcctctg 180
aacaaggtca ccaatgctga gatagctgaa gagatggcgg cccgttttgc ccgttgctgc 240
tgtgcgaatt tatccgcagt gtgaacaccg ttggcaaatc ggcattcgtg gaggccgtcg 300
gtcgggcgag cgatgatgcg gaaggttacc tggatttttt caaaggcaag ataaccgaat 360
ccgttgaatt acatcacacc ccgcgagagt cgaatcaacc aattttgttt tttttacatt 420
ttatacaaaa tttataacta agtatataag tgttgtattt ggtttgaata ttaaatgcag 480
tataccttttt tattaggaaa ctacaaacat tcttgttata gataaaatat aatttaaata 540
tntgttgaa 549

<210> 1744
<211> 549
<212> DNA
<213> Ctenocephalides felis

<400> 1744

```
aacacgctgg ctgacaaccg cacgggcagt cagagcaccg ttgctgttga agacttcaat 60
ccagtcgtta tcggcgatac ccagatcttt ggcatcggct tcactcaacc agaccaccgg 120
accaccgcga cctaaagtca gcatcagcag gttgtcgctg taggtggagt ggataccca 180
cttctggtgc ggcgtcagga agttgagcgc tttttcctgg ttgccgttgg atttctggcc 240
tatcacttct ttcaccgaac gggtgtcgat cggcggacga taaaccagca ggctttcacc 300
gaaatcacgc atccactggt gatcctgata cagttgctga cgaccagaga gcgtacgcca 360
tgggatcagc tcgtgaacgt tggtgtaacc ggcgttgtaa gaaacgtgtt catcttcaga 420
ccagaccagg tcgggctgga gataattttg cgcggctgtg cctgaatatc gcggaacgga 480
tcttctcgtc ttnttattca gcgccagatg cgtatggcac gaccggnaat tcgttnaggc 540
agccaagct                                                         549
```

<210> 1745
<211> 549
<212> DNA
<213> Ctenocephalides felis

<400> 1745

```
ccgcggtggc ggccgctcta gaactagtgg atcccccggg ctggtacttt gccaactatg 60
acccgcgtat gaagagatcg cgcataaagc cgagcttttt cgccaacaaa cgggaattgc 120
accgtttatt gtggttttac ccgacattaa taatgaagcc agtctcagac aaaatggtaa 180
agcgatgctg gcgcatgcgt catcttcatt gagtgatgta aaaggaagtg ttctgttact 240
atttactacc cgcgaaccac ggttaattat gatcaccaac ggccaggttg aaagtggtct 300
ggacgataaa catctcggcc ttctgataga aaatcacacg ctggcttatt taaatgcaga 360
tctctggtat cagggaatca ataatgcatt ggctgttcta caagcacaga tattaaaaca 420
atcgacgccg cactaacgta ttatccgcat ccaggcgcgg gctgcacgac ttgttcagcg 480
gcatggtgcc gtatttatcc agcgccagcg agaaacctgc tccgtacccg ngtgccggaa 540
gccagatgc                                                         549
```

<210> 1746
<211> 549
<212> DNA
<213> Ctenocephalides felis

<400> 1746

```
aatttctatc gttatacgaa aagataatca attccgctag ccttaacgcg tgaattattg 60
tcttatcaca acttatcggc aggcgctttc ccgcctttcg ggtcaaggcc cgcatcactc 120
ctcaagaata gtttttatcg cttcccccgc cattgcagct gttgcacaga gttgttctat 180
agagtgcccc tctcgtgcct tcgcagaaag cccagacatc acggtactca taaaatcagt 240
cacacattgt gcgcgttgtg gatgccgcct ggcgatgtag tcataaatgg tcgtttctgc 300
ggcgtgataa tattgaacgg caatatcacg cgcttgtgga tcatgactat gaataccttc 360
aagaaccata cagccagcgc agccgccgtt ttggctatat ctgcgcgccg cttcttttaa 420
tacctcaacc aggcactcgc ctaccggacg atcatcacga agaatatcgg caagcggaat 480
agcttccgta ccgcgtattc attgagtcac ggttaataac cagcttactg caaaaccggt 540
agaggtcgg                                                         549
```

<210> 1747
<211> 549
<212> DNA
<213> Ctenocephalides felis

<400> 1747

```
gaaataatga gagtatttta atatcatcac aattacaaat acgtttggca aaatgattaa 60
taggtttaaa tatatttatt ctaatacaaa taagttaata aaaaatacaa gttttcaaca 120
aacaaaacga agttatacga tgcgaagagg tgcatacgat ggcgaaggta aaactgtttt 180
gcgtttatta aatcaagatg ccgaaatggg cctgatgatt aattcatata gtcaagttgg 240
tttttccctg aacaatggtg tttcagtaat gggtcctgta gcgatatttc ccaggactgt 300
tctatcatgg aatgtaggaa atagtgaaga cataaatgag gcttcattat ctttatttta 360
taccttagat ccaaaattgg atgttcttgt tattggtata gaacaagaaa gtgttaatcc 420
caagttaagg cagaggatta tggaaatttt acgccaaaaa gaattaatgn tgaagtttta 480
cctactgtgt tgcatgcact acattaactt tcttatgcaa aagcccagcg tgctgggcat 540
attcccctt                                                         549
```

<210> 1748
<211> 137
<212> DNA
<213> Ctenocephalides felis

<400> 1748

```
accaaccatg acgacacncg atgcatcctt tcttctcctg atcgaaggca tcgaaggcat 60
tcttgagtag tttgatttga tccttgtcga gctcctccat tgttactgta gatcacagac 120
gacgctttcg tncttac                                                137
```

<210> 1749
<211> 193
<212> DNA
<213> Ctenocephalides felis

<400> 1749

```
actgatttta tacgaaattt atagattaaa atcaacgtgt attaattttc tgtcaattat 60
tgatcaatac ataatattat aaaattgttg taaatcatta tgttacataa tatatataaa 120
gtcttgtttt aacttattta taataattat aataaaaaca taaacatcaa aaaaaaaaaa 180
aaaaaaaaaa aaa                                                    193
```

<210> 1750
<211> 500
<212> DNA
<213> Ctenocephalides felis

<400> 1750

```
accaagtgtg gccaaagaca atctttattg aaatggtaac aatcataggc gcacaaaaaa 60
tccaactnca gataattatc taattatttc ttactagggg gtttgaattc aaatttacgt 120
gatttaagtg ctgtccattt gtgtcttttc agataaaagg ccaatgcaaa aagcattgaa 180
aagattccaa tagccttaat ggtcattttc ttacgatcat catgttcagg ctcagatgcc 240
catttcaaga atgtagaaac atctttggcg agttgactgg cagtggctgg cgttccatct 300
gaatattcca taacctcatt atacaatgct tgagccatag aaatggctcc ccctgggaaa 360
tatggattgt aatactgcct tcccttagaa ttacacctgc tggtgggtct gtgtatccag 420
tcaggagtgc aaacaagtag tcttctcctc catgacgagc caatgtaata aaactcaaat 480
cangtggaaa agcccattat                                             500
```

<210> 1751
<211> 500
<212> DNA
<213> Ctenocephalides felis

<400> 1751

```
acatataagc gaatttttt atatatatct tcaaaaagaa aatatccata tttttgaaac 60
aatttaataa atttctgttc ctttaatatt tttatcaatt catatgactt agttatacaa 120
aataatcata tgttacttat aaacatgtat catagaagtt ttacatctga ctctgattgt 180
tataattaat aaaataaaaa aaaaaaaaac atacatacag aagttttaca ttttattaca 240
aaattaaata cataaatgta tataactttt caaaatttta ttataaattc atagatgttt 300
ttcatgttta attatgtgtt tctaaccaat cggttttaat aaaaattata agaatgtaga 360
tattgtagta aatcgaacat ttctgttctt catctacaag atacaataaa tagaaattat 420
aaatagttat ataataattt tgcattttta atgaaaatca cactgtgtat catagtagca 480
cttccaattt tgcaacaata 500
```

<210> 1752
<211> 252
<212> DNA
<213> Ctenocephalides felis

<400> 1752

```
acttgaacaa ttcgatgatc ttctagttaa aattttactg ttagctgcta ttatatcttt 60
cgttctcgcc ctttttgaag agcacgaaga ttctttcact gccttcgttg aacctttcgt 120
cattttactg attttaatcg ctaacgctat tgtcggtgtt tggcaggaaa gaaacgctga 180
atcggctatt gaagctctta aggaatatga accagaaatg ggaaaagtcg tccgtggcga 240
taaggccggt gt 252
```

<210> 1753
<211> 265
<212> DNA
<213> Ctenocephalides felis

<400> 1753

```
cttcttataa ccatgtaatt ntctacacat aatgattatn ttatttattc acgatataca 60
acanntgctt ttttcttgag tgttaccgtc tgctttttct atctcgacta acaaagcatg 120
gaatatttca gctactgact cattttgttt ggcagatgtt tctaaaaatg cggctttcca 180
actatcagct aatcgtttac cttcatctgc acttatcatt cgntccatat gtaaatcagt 240
tttgttacct actaacacta caggt 265
```

<210> 1754
<211> 305
<212> DNA
<213> Ctenocephalides felis

<400> 1754

actacatcaa aatgtgtgct tttcctaagc agctgcaata cccaaagcag caccagctgc 60
aaatgctcct ataaaaatat gtatttcttg caatttaaat tcttcttcac ctgtaagacc 120
gtgataccat tttttggctt ttctttgatt tttctttagc aattcctctg ctttgtcaat 180
tttcctatca acaaatctct caattttatc cattaatttg ggagtttctc cagtaacagc 240
ctcttcaatt ttatctgtca ccttatcagc ctttttagtt actttatccc agtcaataga 300
aatgt 305

<210> 1755
<211> 500
<212> DNA
<213> Ctenocephalides felis

<400> 1755

accattatat aagtcaggaa atcgtcaatc aacagcaaac tacagaccca tagccatact 60
gccaatattt tcaataatta tagaaatgtt gttagcatta agaattacaa gtttcttcga 120
tgctaataat tattttgtaa aaaatcaatt tgaattcaaa aagaaaatta tcacaattga 180
tgctgtgcta gactttttga actttatatc aaattcactg gaagatcata aacactgtgg 240
ggcactattt tgcgatctca gcaaagcatt cgattgtgtt ccacatgaca tatttctcag 300
gaaactggaa tattatggat ttaggggcgt agcccttaaa ctgatgcaat catacctatg 360
taacaggtat caagtttgtg gaatggaaca ataaacgaag tgagatagca agattaaatg 420
caggcaatgc ccaagggagt attcttggac cacttctttt tatcatattt gtgatgatct 480
accaccgaac attagctgca 500

<210> 1756
<211> 486
<212> DNA
<213> Ctenocephalides felis

<400> 1756

acggtctatt aacaaatatt ctgaaatcaa aatcaaacta atagcggcaa gatctcgagt 60
agcaccatta aaaaatatta cgctcccccg tctcgaactt tgtgcggcac aattgctagc 120
aaatcttgcg caaataacta aacgtgcatt aaatatcgcg tttgataaag aattctattg 180
gagcgattcc acaatcactc tttcgtggat aagatctcca tcttataaat ggaaaacctt 240
cgttgccaat agagtttctg atattcaaac aaaaaccgac gcaaataact ggttacatgt 300
ccgatcggaa gacaatccag cggacctcat atcacgcggg tgctatacac atgatttatt 360
gaattcatcc ttgtggtggg caggcccatc ttggctacaa aatccaactg aaacacagcg 420
acgtgcaact gacaatattt caattcctga aactgatgtc gaaagtcgaa tcacaagttt 480
gacttg 486

<210> 1757
<211> 500
<212> DNA
<213> Ctenocephalides felis

<400> 1757

accactgact cctgaatgtt aaaaattaag tcgtttattg atttatcgat atcttcttca 60
tttttcaatc gcatttcaag atttgttcgt tcatttagtg tgttgcgaaa ttttttccca 120
atttgtgaat ttattataaa gtattctgtt gttttttata ggaattggtg caattcctat 180
tgntagaatg attggagaat ggtcggatga taaatcaaaa gatggtttta tttcaatgtt 240
cttatcatca atatcctttg taatgaagaa attgagcaga tccggtattt tattagtatc 300
gctcggccag tatgttgggc ttccagttga tatgtaagag agattgtttt tatctattgc 360

atttaataat tccttgcctc tagatgtaat taatcgagaa ccccatcttg tatgtttgca 420
attgtaatct cctcctacaa taaattttgg gcctagttgt gaaagaaaat tttcgaaatc 480
aatctgttaa tttatgtcgt 500

<210> 1758
<211> 270
<212> DNA
<213> Ctenocephalides felis

<400> 1758

acatgaactt aatcctgatt ttaaaccacc aaaaagacca tttaaaagga tggattatgg 60
tgctgccata aaatacttga gagaaaataa tatcactaaa gatgatggaa cattttatga 120
atttggagag gacataccag aagctccaga gcgcagaatg acggatgcaa tcaatgaacc 180
tataatgctt tgtcgttttc cagcaggaat taaatcattc tatatgtcca aatgccctga 240
gaacaaagaa cttactgaaa gtgttgatgt 270

<210> 1759
<211> 500
<212> DNA
<213> Ctenocephalides felis

<400> 1759

acatttttac atttttaaat ataatacaac cttatataag tatcacacca tcttgtattt 60
atgaaaatag tctcgcaaac tcttcgaagt tgatgcgtcc atccttgtcc gtatcggcca 120
acgtcatcaa ctcggtgacc tgctgctccg tgacattttc gccgatcatc tccatggcgg 180
ttcgcaattc gtctcgggaa atgtatccat taccatctcg gtcgaacacc ttgaaggcag 240
atcgtagatc tttattgaca tcatcgttgg agtctgaatc tccccccttgt ctttgggttc 300
cgccttgcat tgcctgaact gcttgaatct ttgcgatcca ttgtaaaaac tcggtctcgt 360
ctattaatcc gctaccagac tgactagcgt ctttcatcag atcttgaata agttcatccc 420
tcacgtggat tcctagattt tttaacataa attgcaaccc tgaagccgta catgtccatc 480
ctggtgcgat ccagcaaccc 500

<210> 1760
<211> 500
<212> DNA
<213> Ctenocephalides felis

<400> 1760

accaccgtct ggaccgctag gatccttgat ttctagggta agttcaaata cttcttcttc 60
tacatctacc gttcgtattt taattttact cgattcttta acgactgtgg ttccgcgata 120
ccaaacgaca tcaggcttgg gcttagcttt acatttacat ttcattgtta ttaacgagcc 180
tgtttcatta ggaataattc ttggtttttc gatgaacgtt ggcgcgtatc ctgcagcatc 240
atcaccgctg tcgaagttca agctgatagt cgcattgctt tctccaagtt cattctttgc 300
agtcacacga tatttgccag catcctcaac agtgacattt ttaatctcga gtgaagcaaa 360
gtatgaatga ccatctttgt caaccatcaa cttgtgtctt ggtgagtcct taacagggtt 420

ggtattgtga aaccaagcga ctgtaggttt aggatcagct tgaattcggc attcaaagag 480
caaacgtttg catcatctct 500

<210> 1761
<211> 500
<212> DNA
<213> Ctenocephalides felis

<400> 1761

acctaacgat caattcatta cagattactt gaatgacggt aatttcatag actagcccat 60
tccaaaaatt aagtattctg aagtgctcta cgagattagt aacactaaat tggtaaaaac 120
acttggtaat gatgcgatca catttggact aataaaacac cttcctgata aaggtaagag 180
atttttaacg atattattga atgccataat acggctgaag catttttctt catcgtggaa 240
attggcaaaa ataatcttaa ttctcaagcc tggaaaaaat cctactgatg cagtttccta 300
ccggcccatt agtcttctat cttgcctctc acattttttt gaaaaagtta ttcacaaaag 360
aatcattaat attttagaag ataataattt tatgccaaaa catcaatttg ggttcantta 420
gacaacactg agcaaaagaa caaatacaca gagtagtgga ttcatttata aagcctttca 480
cttaaaaaaa atggtcagcc 500

<210> 1762
<211> 500
<212> DNA
<213> Ctenocephalides felis

<400> 1762

acgggttgat cttagcagcc tcagcatatc ctttttttcca catcctgttt ttagcttcaa 60
aataagatat gttatgtagt gccatcattt cttttatttc tttatttgct ttgaagaatt 120
tacattcttt gtcgtttgct tgatggtttc catggcatac tgcacatata aagtctgaca 180
cgatatggca attttcatga gagtgttctt ctgaacaata tcggcatctt atcttgcttt 240
tgcattagtt ttttatatgt ccataacgcc agcatttgtg gcattgcatt acagattgaa 300
tgtaaggacc tacagcacac ctaaccgaat acattgaaac gtattctggc agatagttac 360
ccctgaacac tatttttacc atttgcgatt tcatatttgt ttattctaag ttttttttaat 420
gcatttgacc tctaatattt tgatatccga tctaataaca tccatgattt cttgctcgtc 480
atgtcgggtt caacgttaaa 500

<210> 1763
<211> 500
<212> DNA
<213> Ctenocephalides felis

<400> 1763

```
acctgggcaa aggtggagaa gttgccaaat taaccaaaga agcaatgaaa ggcagcatgt 60
ccttccaaga agctctcaca aaacgattag atataatcag accttcacaa cagaatattt 120
cagacttcat caaagatcat ccatcaacac tcacacctgg aataaaaaac ttaattgcat 180
ccttacatcg gaaaagaata ccagtatatt tggtgagtgg aggcttccga tcactcatag 240
aacctgtggc caaagaatta ggaattccat atgaaaatat atttgccaat agaatcatat 300
tttattataa cggagattac gctggatttg atgcaactca accgacatct cggtctggcg 360
gcaaggggca agttctagcg ttgctgcggc agcaaaaggg ttacagacac attgcaatga 420
ttggtgacgg tgctacagat ttagaagcac aggctgatac tttcataggt tttggcggaa 480
atattgtcga gaagaagtca                                             500
```

<210> 1764
<211> 315
<212> DNA
<213> Ctenocephalides felis

<400> 1764

```
ctcgtgttat ttttttaagt tgtatttttt tgtttagttt taattttata aaatgtgatc 60
ccccgactgt aactttgccc cagggcgaat tggttggaaa agctttgacg aacgaaaatg 120
gaaaagagta ttttagctac acaggtgtgc cttatgctaa acctccagtt ggagaactta 180
gatttaagcc tccacagaaa gctgagccat ggaatggtgt tttcaacgcc acattatacg 240
gaaatgtgtg taaatcttta aatttcttct tgaagaaaat tgaaggagac gaagactgct 300
tggtagtaaa cgtgt                                                  315
```

<210> 1765
<211> 500
<212> DNA
<213> Ctenocephalides felis

<400> 1765

```
acccctttaa gtgaaagatt atgtatttgg gagaaggctg cagatttaat ggccggtgtt 60
tatagacaag aattaaatgc tgcaaccatg ttaggacaat caaaaacagt cattcaagct 120
gaaattgatt ctgcagctga attgattgat ttcattagaa tgaatgcctt cttcctgaag 180
gaattaacta aataccaacc aattagcgaa gatttgagtg ttacaaggaa ttctatgcgt 240
ttccgaggta ttgatggatt tattgctgct gttagtcctt tcaactttac cgctattggt 300
ggtaatttgg cctatacacc agctttaatg ggcaatggtg tattatggaa accatcagac 360
acagctttac tgtcaaattg gatcatattc aagatcatgc gtgaggctgg tcttccacca 420
ggtgttgtca actttatccc ttgtgatggt cctgtatttg gtgacactgc actgcctctc 480
tcatttagct ggcattaact                                             500
```

<210> 1766
<211> 456
<212> DNA
<213> Ctenocephalides felis

<400> 1766

```
accacgtatc taatataatt taaaacttat ttgagttatg taaatcaata atctcaagta 60
taactggcaa attaagattt gtagaaaaat aaccaaagta atgcgacaaa cctttacatc 120
acaatgcttc taggttggaa tttgtttcaa tggtcaactt tttctcctga ctcatttcat 180
atgcaaccgc ccttccagct tgatataatt gtgttagtcc aacaaacata ttaacagcaa 240
acaaactcca ggtttttgga attatgacta aggagtatct ggcccatacg aatccagtta 300
cagccaatga tcctgattgt ctaactgata gttgctcagc tggcctggat aaatctccta 360
tgccagctat aaccagaccc catttaaaaa gaggtgccca aaagaagaca gtttttggac 420
ctgtggtgaa ttccataacg gtaaagccga ttttgg                            456
```

<210> 1767
<211> 500
<212> DNA
<213> Ctenocephalides felis

<400> 1767

```
acgcaactaa tgcaatataa atttgtttca agtcaacaat ttaacaccaa aaacaataat 60
agatcaaaat ggtcctttca aatagctgat ataatgtata atgtttgaga ttaggttata 120
taaaagttga atgtttttttg attctttaat aaaaatatcc atcactgata taactaaatt 180
acgcagtaat cataattaga attatgtctc tagcaacagt tattctttct cagcaatagc 240
ggtcaagtga acatctaatt ctgcctctgt caaaacccta aatttaagat tttctttcgt 300
aaccaccgca acctcaattt ctgaaggttt gaagtcaaca cataacactg ttgataaaca 360
tgaaattgcc aactggacag cttcatcaaa tgtataatcc tgtttcttct tcagtttttt 420
ctcaagatat ccattggctt cagtttgctt tacacccaca ctaatggctc taaaaccaca 480
gcagtaacca gcaggatctg                                              500
```

<210> 1768
<211> 549
<212> DNA
<213> Ctenocephalides felis

<400> 1768

```
gataaacgat cacctaaaaa atacggtggt aacttagata caactggaag tccttccaga 60
tctcgtagcg ctaccaaaga attaatattg cccccagatg attctctaat gtgcaaacca 120
gattttacaa ctcctttaaa agatagatct gtaaaagatg gtgatagttt aacattaaca 180
tgtaccgtta agggtgaccc agaaccacaa gtttcgtggt ttaaaggcgg caaggcattg 240
agttcttccg aaattatgga cttgaaatat aaaaatggcg ttgccacatt atcgataaat 300
gaagtattcc cagaagatga aggtgtttat gtttgtaaag ctaccaattc aattggagtt 360
agtgaaacaa gttgtaaatt aacggttaaa ccaatggcaa acggcaaggt gtctaagaaa 420
gctcaggaga caaaccacct aaaatagtga gtcacttaga atctaaatac gtcgaagatg 480
gacacccgca cgttatcttg tagatataga tgctgagaaa tcatgtcgct ggtgnacata 540
ccaagaaat                                                          549
```

<210> 1769
<211> 549

<212> DNA
<213> Ctenocephalides felis

<400> 1769

```
aatcaccgct gtaaccaatg cgcgagttaa tctcaaaagg cgtttcacct tttgccatat 60
caaacagtgg tttgcttact tcgttattaa ccagcgtggt ttgaattgat gccatcgacg 120
ggatcaggtt cagtttttta agctgggcaa gcgggaaggg accatgatca accgattcgt 180
tgaagatgac gctctgaccg cttttaatcc acggattttc tttcccggca atgggtttca 240
ccaacagttg caactggctg ctgaatacgc cgcgatgata gttttgataa ctcacttcca 300
ggttggattc aggagctgtc agtttgagtt gcgcgttcgc ctgcgcgacc atgtcttcga 360
gatgggtttc aatcttcttg cctgtatacc atgcgccgct gtccagacta cgcctagcgc 420
aacaatgacg cctaccgcta ccagcgattt attcataatg attatcataa aatgaaatca 480
ggcggactgc cgctgaaggt gtataagcct ttaataagct tacaagagat gtaatttttc 540
agtagctct                                                         549
```

<210> 1770
<211> 549
<212> DNA
<213> Ctenocephalides felis

<400> 1770

```
aaggatggag tcactgtagc gaagggtatt gatctcaagg acaaatttca gaatattggg 60
gccaaattag tccaagatgt agctaataac accaatgaag aagctggtga tggtactact 120
actgctactg tgcttgccag agctattgca aaggaaggct ttgaaaaaat atctaaagga 180
gcaaatccca ttgaaatcag acgaggtgtt atgctggctg ttgatgcagt caaagaatct 240
ttgaaaggaa tgtcgaagcc tgttactacc ccggaggaaa ttgcacaagt tgctaccatt 300
tctgccaatg gagacaaagc tattggaaaa ttgatctcag atgcaatgaa gcgtgttgga 360
aaggaaggtg taataacggt taaagatgga aaaactttac atgatgaact tgaggtcatt 420
gagggcatga agtttgatag aggatcattt caccttactt tatgaattcc agcaaaggtg 480
ctaaagtaga attccaagat ccttattttg tcagtgacaa aaaatactca gtcaagcata 540
ttctgcttg                                                         549
```

<210> 1771
<211> 549
<212> DNA
<213> Ctenocephalides felis

<400> 1771

gctcaacgag taataatggt cagagcgaaa tattgttttt aaaaaattac gtcatacaaa 60
tatcgaatta cttttgaatc taaaaatatc cctggtgaac atttttgata ttaagacgtt 120
ttacatcgaa atttttaagt ttgcattttg gcgaaaatat ttgttacgtg ttcgttacgg 180
aagtttgctt aaaaactctg gtgaattttg aactcaaaat agtgtctgtt ggttttttta 240
attgttgtgt tgccgtgcgg gtcgcagaaa taaattggtg atatggcaca attaatatca 300
gttcgattaa atcgaggaga tgcttgtccg tggggattca gactccaggg tggcaaagat 360
ttcggcactc ctttggttat tcagaaggtg aacagcggaa gcccggccga gcgagctggc 420
tgcaagcagg cgacgctgca tcaaggtgaa taacacagac gtctataatc tgagacacaa 480
ggacgcgcaa gacgcctcgg cgcccggaca tcttgaggtg cagtcaaaga ggtggatcac 540
atggcaccc 549

<210> 1772
<211> 549
<212> DNA
<213> Ctenocephalides felis

<400> 1772

taagaaagtt gctgaagaat ttaaagaagt tttagacacc ttattgngga atgatgaata 60
tggtgtttgg atggactacg attcagaaaa tagaatatcg agaccttatt tctacccttc 120
gaatcttgct cctttgtgga ctcaaagtta taaggacgtt gagtgtggga gtggtaaaag 180
tttgggagta gacaagaatg tgcaggatag gattgagagg gttttgcggt atttaaatag 240
tccagatgta gcaataaaaa gctacccagg tggtgtacca accactttat tgaatacagg 300
agagcaatgg gacttcccaa atgcctgggc acctcttcag catatggtga tcctgggatt 360
ggacagcaca gataatcagg aagctaagga tttgtcattt gatctttgcc agaaatgggt 420
ccgatcaaat cataaagctt acttggaaac gaatcatatg tatgaaaagt taatgcacac 480
atgctggggc accangaaat ggcggagaat tgaggccact tggtttggtg gcgaatggtn 540
ctattagac 549

<210> 1773
<211> 549
<212> DNA
<213> Ctenocephalides felis

<400> 1773

gccgtattcg cagacttata aattagtcgt cgtgggcgga ggaggcgtcg gaaaatctgc 60
aataactatt cagtttatac aaagttattt tgtgacagat tatgatccca ccattgaaga 120
ttcatacaca aagcaatgtg tcatagacga catcccagct aaattggaca tcctggatac 180
tgccggtcaa gaagaattca gtgcaatgag agagcaatac atgagaagtg gtgaaggatt 240
tttgctcgtg tacgcagtga cggatagggc tagtttcgac gagatgtaca aatttcacag 300
acaaatctta cgtgtcaaag acagagacga atttccaatg ctgatggtcg gcaacaaagc 360
agatctcgag acgtctcgcg tggtgtctgt tgaagaggcg caaaatttgt caagacaatt 420
aaaaatacct tacatcgaat gtagcgctaa attgagaatg aatgttgacc aatccttcct 480
gactagttag aattgcagaa gatttcaatt atcagaaagg cagatccaat taaatcaatt 540
ataggataa 549

<210> 1774
<211> 549
<212> DNA
<213> Ctenocephalides felis

<400> 1774

```
aattattgcc ggatgtctgc tggtgatgct gccagcgatg ggcctgttct atgtatccga 60
cctgatgggc ggtgcgaaaa acctgctgat cggtaacgtc atcaaggtcc agttccttaa 120
tattcgtgac tggccgtttg gtgcagctac cagcattacg ctgactatcg taatgggcct 180
gatgttgctg gttactggcg cgcttctcgt ttgctgaata agaaggtgga actcgaatga 240
tcggtcgact gcttcgcggc ggttttatga ccgctatcta cgcgtacctg tatatccacc 300
atcagttcaa gacgacgcag cacctccggc cgggcatcat cctccgtcat ggcacagaga 360
aaatcattca gcgtccccgg ttgtgaatct tcatacacgg tgatggtccc ggcgtgcgat 420
ggtggaaaac cgtcaacctg caggatgaca ctgtctgacc gtactccaca tcatgctgta 480
acgcccggct tatccggatc ttgacccacg tgttcaccac caccgtggtg ctgtacgtct 540
ggtttactg                                                         549
```

<210> 1775
<211> 549
<212> DNA
<213> Ctenocephalides felis

<400> 1775

```
gaaacgagtc gcatagtgca cttctgaatg tgacataatc ccaaagtcta aataacaaaa 60
atactattta gatggagagt gctatccctg acgtatacgt gaattgatac gtgcaccttt 120
tactatcaag gtgatacact cgtgcatcaa gtgataaaga gtgcctgcaa tttattttta 180
ttttagtttg tccgtgaatt gtgtttttcg ttgtgtaacc caagcgccaa agtaacgccg 240
aaacagacat ggaggaaata ttacaagaaa ttaggactat aaaattcaca cgggaggaag 300
tggaatcgtt gatttactgg cgttgtccgc gtaagtctgg catcgtattc ggcgtgtccc 360
tcgccctgct gctggcgtta tcatgcttct ccctaatcag cgtgttagcg tacgcttccc 420
tcacagccgt ctgcggctgc atggccttcg tatctatcgc aacgtattgc aagctgtcag 480
aagacgtccg atggcatcct ttaaggagct gtggaaacag atgtcagtgt gtctaagaaa 540
acccacagn                                                         549
```

<210> 1776
<211> 549
<212> DNA
<213> Ctenocephalides felis

<400> 1776

```
gctgatggtt gcggatccgc tgagagcgga tatagtgacg aacgcagtga tgaaagcgat 60
tgcgagacta aaaatttagc taatgattgt actgataatt tagataatga tgttgaacat 120
gtaggtagtt cgaatgatat tgtagaagaa gatgagcacg tactgagtca caataatgat 180
gtagtagaag acgaaattgt gaatgtaata aattcgattg aaatagaaga actctctggt 240
aatgacatta atgaaataga cggcgtggaa gtagaagacg agactgatat tcatattaac 300
aaagaaaaca gttgtaaatc tgattttaac acaacgcaaa ttggagatgg ctcaaataat 360
gtgacagtcg ttgcaatcca gataaagaag gagcagatag cacacttgaa cttgaagcgc 420
aatcgtgttc aagttctgga tcttttaata aaatctctaa tattgtaatg atgaaaaatc 480
tcagaattta gacaaattga cacatgcaaa gacgataagt tgtgcgtgtc agacctctca 540
tcgcgagtt                                                         549
```

<210> 1777
<211> 549
<212> DNA
<213> Ctenocephalides felis

<400> 1777

```
aacgaagaaa aacaagatta aaattcaaca cggatcagct gtgtattgta ttatatattt 60
ggataacaaa gtatttgtat cgtttgcaaa tggcgatatt agtgtttaca caagagatca 120
aacgggttgg aatacaaacg acccaacgac ggtgtgcgtt ggttcaaatg tggccgcccg 180
tactaagttg cttcccgtcg ctagtcgatt gtggtgttcg gcgcataatc atatcaaaat 240
tatcaataca gaatcattac aagttgaaca aacgtttcaa gtcaacaccg atatcaataa 300
gccaataaca aatatggtta ctggaaattc gggggtttgg atatcgttgc aaaattccgc 360
agttttaaaa tgttatcatg caaatactta cgaatgtgtc tggaagtaaa tattgctccg 420
agtgttacaa agatgttagc ggcttgcgat gatattattc gacaacataa agcagcgtgt 480
ttaagagtgc agcctgtagc ttgcaaagat tatatggata ggaacagtgc tggagtatat 540
tgcctccat                                                         549
```

<210> 1778
<211> 549
<212> DNA
<213> Ctenocephalides felis

<400> 1778

```
atacgtgata aggatgacta cccatatata acattttgcc atgttattat atatttttc 60
atgctagctt catagaaatt tttcaaaatt aacattaaaa cattgttcat acatttcgtt 120
taataatttt tatgtttaaa actcattggt caccttaata aatgtacata acttattatt 180
tctttgcaaa tccagaatta ggacattttt ttcaagtatg tattatggct ctcaaaattt 240
agtttattct tatatcgggt tttattttcc tggacggttt tcaaccgcaa tcacagatat 300
gatggacgtg gagccctagc ggatctccaa gcccatgaag ctatatcaca ttgggactac 360
aatgaaggac ttagtgatga agaaagaaga gcagaacagc tttgcgatga agagaggtca 420
gagcgttgta tgagcggcgg aagaagaaga aatatataaa gaagaggtaa tgaagagggc 480
acaacaacag atcatgtgaa agtnatggca agtgcattca attatcagat gttggtggtg 540
ggatgatga                                                         549
```

<210> 1779
<211> 549
<212> DNA
<213> Ctenocephalides felis

<400> 1779

aatttattta atgatgagct ttttactcag taatataaaa tattgaattg ttatttttgt 60
gtgttgttta agataaaaag ccgtatttat tattacggct ttaattaata aaaggcaggc 120
tgtattaaaa ttaatattca aagcataaac cgatagccaa taccggtttc agtaatgaaa 180
tggcgtgggc ggcgggatcc tgttccagtt tttgtcgcag atgtcccata taaatacgca 240
aatagtgact gtgttcgacc gcgtttggcc cccacacctg gttaaggagc tggcgctggg 300
tgagtacttt tccggcattg ttgagcagca ccgccagcag gcggaactca attggtgtga 360
gatgcacctc ttcctcaccc cggtgaatca cgcgggcggc taaatcgacg gtaacatcgg 420
aaaattttac cagcggatcg ggcgcggtgg tggagagtgg cggcgtaatg cgactgctgt 480
acttcggcgt gcacaacatt gccatgctgg cgtattatcg cgtatgggat gctgcgtgat 540
ggtgaggtg 549

<210> 1780
<211> 549
<212> DNA
<213> Ctenocephalides felis

<400> 1780

ggtacacaat gtacggtccc gatttatcca ttgattctgc aatatattca aatcttgaaa 60
gtgacgaact aaaagaaatt attcaggatg atgacaaatt tgaagaaata ttcaaggaat 120
tggcccaggt taaaaactgg gaaaatcaaa aggaagcaat gatagaaaga aataangctc 180
ttgccgaagc aaatctttta cgcaatcctg acttagctga aataaaagaa aaattacaag 240
aactttctga agaaggcaaa caattatgta ccagtatcca agaaatgctt gctgaaataa 300
aagaaaaatc tggaagtatc agtttggata cagctttagc tctgttacaa acagcagctg 360
caacaagcga agaagaatct gaaaatatag cagatcaatt catttcacgt gatattgata 420
tagatgcatt tttagaacag tttgcatcat caagaaaagt tatgcatttg agaaaagtca 480
aagctgataa aatgaaagac ttcttnccaa agaaatagta gtatcaaata atcttatgtg 540
cctagtgta 549

<210> 1781
<211> 549
<212> DNA
<213> Ctenocephalides felis

<400> 1781

gatagttcat gctcgccggt ccgtgaaact accaaaaaca aaaaaaaaga ataataaaaa 60
aaaatcgtct caattgaatt taatcggacc gtactaaaaa aaaagaaact tattactgtt 120
attgttgcga taatttcgaa ccgaagaaaa ttaaattaaa tccaaaccta ttgtgtaatg 180
gcgtttttga gatcgtctat tcagtgtaat aatttactaa gaaagttcgg cataatacca 240
aacagtttgc aatcgttgtc gacaccgtgt gcgtctcata tacatggaat aaatacagat 300
tcttcaaaaa gtcaacatgt tgcaagccca gctcaagccc atgtacagaa agaccctttg 360
gacatatcat tcaacgatca cgttgcagcc ttcaaaagta aaaagacaag tgagctcgtt 420
cgggcttcat cgtctatgca ctatgcacct cagaatatct tgtggaaaat aatatgaagc 480
taatgaaaat atccaaggcg atcttaggcg agaagctgtc acagcctaat gaagcttcgt 540
ctatggcnt 549

<210> 1782
<211> 326
<212> DNA

<213> Ctenocephalides felis

<400> 1782

```
aattgctcac cgtgtaaatc ttccagcacc gtcaggttga tgcgcgagcg acgttccgct 60
acttcctcgc gcagaccatc aagccctttt tccagcagca gcgggaaatt caccgccagg 120
tgcgcatcgc cggaggtcat attgccttcc gctttaatga ttccggtcgc cagcagacct 180
ttttgctcat cggtaaacat gccgtagcag cgatcctgtn cggtctgacc gcgccaccac 240
gggcacactt catgcagaac gcgtttgttc tcttcgctca ccgcaaagcc agcaccggga 300
cgatctgcca gatcatcaat ctcttt                                      326
```

<210> 1783
<211> 549
<212> DNA
<213> Ctenocephalides felis

<400> 1783

```
ggaacatctc ctgaattaca tatgctaata ttgttttgtg attgtatgac acattatgtc 60
acaatacttg atgatttgga aatgtatgag cagcaaaatc catttaaatt atctgatttc 120
gttgtaattt caaatttttt gaatatgttt ttatataaag cgatactggg aaatctattt 180
gatttgaaaa acctatcagg gtgtccatta tttgtgtcaa ctcatacttt gttattagct 240
ttgtatagac gagattgtcg aagaccattt acaccaaact cacattggct tgctaaagat 300
atcaggccat ctacttttat gaatgatctt gacaaaggaa aaaaaactac tcaggtgctt 360
ttacaaaaaa tgccgcacat aataccacat gaagaaaggg tgcaattatt tagaaagtgt 420
gtgtcaaatg aaaaggctgt gttaggttta actgaatcag catgtgtatc acccaaagca 480
ctttgatact gtcatagaga cagaattgtg aagatggata tcacagctgc tgcgtgcaac 540
catgcttaa                                                         549
```

<210> 1784
<211> 327
<212> DNA
<213> Ctenocephalides felis

<400> 1784

```
agttgattga aaaggtctga tgtgcatgcc attgcgcacc tgaccggngg cggcttctgg 60
gaaaacattc cgcgcgtatt gccagataat actcaggcag tgattgatga atcttcctgg 120
caggggccgg aagtgttcaa ctggctgcaa acggcaggta acgttgagca ccatgaaatg 180
tatcgcacct tcaactgcgg cgttnggatg attnttgccc tgcctgctcc ggaagtggac 240
aaagccctcg ccctgctcaa tgccaacggt gaaaacgcgt ggaaaatcgg tatcatcaaa 300
gcctctgatt ccnaacaacg cgtggtt                                     327
```

<210> 1785
<211> 549
<212> DNA
<213> Ctenocephalides felis

<400> 1785

aatgttccac cagcagggga aataatgttg ttctggctat cggtcaccac cacgcccacg 60
ccaggacgtg acgtcttaat cgcctgggga tagctggggt ctgtggttgc cgtcagactc 120
aggttgacat taactaatcc cgcgtttccc gtgttacacc tgactggaat tgcaagttct 180
gccgtctggc cccaagaggt ttatgcccta caacacggaa attggcagcg aagacatcac 240
ctaaatcaac ttcaattgtc tctccggcat tgacggaaca tgagccaagt gaattgatcg 300
taccagacag gtaatacact aaaaccggtg tacctgtagt cgtgcaggaa tcacctgcgg 360
gtatgttgta gcattcatat aatcgcgcca gcgccatagg aggaatgacg accgagccaa 420
cgaacggctg caatatttta gggaaaagct accgctatta ccgctgcccc ctgccactaa 480
acgttgaccg cagggtcgaa attgcctcgg ttcccccctg tgcccacgtt ttcaaggagc 540

ccggtccca 549

<210> 1786
<211> 549
<212> DNA
<213> Ctenocephalides felis

<400> 1786

cgtttacgtg gtggctctaa tcaggctttt gttctaaaat tttgctcagc tatatttctt 60
ttattggatt catttggcct gcaacttatt tagttttcaa agtatagtgg caggatagaa 120
cgtaatcata gaatttatga aatttttcat acttcttggt caaatctgtg gtgatgtgtt 180
aaatattttc aatacggatt agtgtatctg aaaatatata tttggcaaat ttgttattaa 240
atatttagtt atcgcagacg cacagtaata cgaagtagta tttaatgaat actcggtact 300
cttatcacag gatgctattg tatgaaatgg ggttttagag tagtcacctc cgtctcgcat 360
cagaatgctc actaggactg tctacaaaga ttctggttta taatgatctc acttgtttaa 420
tcacacgtta attacccact attgttgaat atatatatat atatatatat atatatattt 480
atatatatat atatatattt atatatgtat atatatatta tatatatata tatatattta 540
tatatatat 549

<210> 1787
<211> 549
<212> DNA
<213> Ctenocephalides felis

<400> 1787

ttccaaaata gnganancag ttaattttag acaaatataa aataataaat aaatcgaaat 60
aaagtttgat gaacaatatt tattattaaa ttgcgattgc acaatttaag cagtataacg 120
aaatggcgtt acggattttg aagcctttaa gaaactctct tggaatttgt caagatatca 180
aaaaggcttc gacgctatct gcatttgata ttcaacataa aacccctctt attaagcaat 240
cagatgaaat tccaaaagca caatatgggg gtcgtcacgc agtcacaatg ctgcctggtg 300
gcggtattgg tccggaactt atgggatatg taaaagaagt tttcagatat gctggtgtgc 360
cagtagattt tgaagaaatt acaatagatc cttctgtaca ttcagatgct gatttagaat 420
atgccatcac ttcaattaaa agaaatggag ttgctattaa aggtaatatt gaaaccaaaa 480
gtgaatctgc acangtatta tctcgtaatg taccctgaga aacgaataga ttatttgnaa 540
tgtttaaac 549

<210> 1788
<211> 549

<212> DNA
<213> Ctenocephalides felis

<400> 1788

```
gtnagccacc attacatccg gtgagcagtc aggtgcggtg atacgtggtg tttttgatga 60
ccctgaaaat atcagctatg ccggacaggg cgtgcgcgtt gaaggctcca gcccgtccct 120
gtttgtccgg actgatgagg tgcggcagct gcggcgtgga gacacgctga ccatcggtga 180

ggaaaatttc tgggtagatc gggtttcgcc ggatgatggc ggaagttgtc atctccgcct 240
ctttcactca ttaagactgt aaataaacca cctgggtctg cagatattca tgcaagccat 300
gtttaccatc tgcgccgcca ataccggatt tacgccatcc ggcgtggaag ccttgcatag 360
cttcgaagtt ttcacggttg atgtaagttt caccaaactt caccctttaa tggctttcat 420
cgcgacgttc agattttggg tatagattga tgaggtcagg ccgtaatcac tgcattagcc 480
attgagatag catcttccag cgtgtaaatg cgcaactggc agcaccggcc aaagtttcta 540
tgctatcga                                                         549
```

<210> 1789
<211> 549
<212> DNA
<213> Ctenocephalides felis

<400> 1789

```
aatgcccata cctaccnttc cgcgctgccc cgtcgcctgc tccgccatat caaccaacgt 60
ggcgatcgtt tcaatagtct gccggtaatc atcacgcggc gtgggcagac gatggcggta 120
caactgctcc cctgcatcgc ccagtgcaat cacttcagtt ttggtgccgc ctaaatcgat 180
acctatacgc acggtactct ccttattttt ttcaatatca atagcgtaga gacggacaac 240
cggattggca atgcaaggcc gccgacaatt cgttatcatg cccgctaaat ttaacgacaa 300
ggccgtggaa attatcatgc tgtggttcaa aaatttaatg gtttaccgtc ttagccgcga 360
gatttcgctg cgtgcagaag agatggaaaa acagctagcc tcgatggcat ttaccccatg 420
cggcagccag gacatggcga agatggctg ggttcctcga tgggatcgca cagcgatgcg 480
ttaacgcacg ttncaatggc aaattgtatc tggcgccaaa gaagaaaaat ctccgtttcg 540
gtgataaac                                                         549
```

<210> 1790
<211> 549
<212> DNA
<213> Ctenocephalides felis

<400> 1790

gcgagaccaa tgtcaccggc ttaacaatct atcaaatttg ccgggccgtc gactggacaa 60
ttgtttcaca aatgatgtta ttttgtccaa caataaataa tagtcccagt gttttaaaac 120
gtcagcgata cgctgcaaag tgtacaagtg tcatatatgt tctgtagtcg tgtctgcata 180
tcgatccgtt ttgccattgc cttgaaaacg ccatattgta tgtactttac gtagttatat 240
ccaacttttg aattaaatgt gttttaagtg tttaatgtta ttttcctaaa tcttgtattt 300
gattagtaac ccttaatcat ggatagaagg aagctttcca cttctggtga tactttatac 360
caaatattag ccattccaaa aactgccact caagatgaag ttaagaaaag ttataggaaa 420
ttggcgttga agtaccatcc tgataagacc caaataatcc agaggcgtct gaaaaattaa 480
agaagtaacc gacccatatg atactagcga tgcagcaaaa gaacatatat gacaattatg 540
gtcttagga 549

<210> 1791
<211> 549
<212> DNA
<213> Ctenocephalides felis

<400> 1791

aatcggcggt gcgccncaac gtattacctg ggaaggttcg cagaaccagg atgcggatgt 60
cagcagcgac ggtaaattta tggtaatggt cagctccaat ggtgggcagc agcacattgc 120
caaacaagat ctggcaacgg gaggcgtaca agttctgtcg tccacgttcc tggatgaaac 180
gccaagtctg gcacctaacg gcactatggt aatctacagc tcttctcagg ggatgggatc 240
cgtgctgaat ttggtttcta cagatgggcg tttcaaagcg cgtcttccgg caactgatgg 300
acaggtcaaa ttccctgcct ggtcgccgta tctgtgataa taattaattg aatagtaaag 360
gaatcattga aatgcaactg aacaaagtgc tgaaagggct gatgattgct ctgctgttat 420
ggcaattgcg gcatgttctt caacaagaac gccagcaatg acggcagctg ctggaaaaag 480
tcgagctgcg gaggataacc cagcagactg gaggagtttc gaaagatgga aggatccagt 540
cccaattcc 549

<210> 1792
<211> 248
<212> DNA
<213> Ctenocephalides felis

<400> 1792

cagagacttc atatgctttg atggtaataa ctttctgagt cctgatgcat ttgataacga 60
aaaaattaaa gaacgaaaat taacttacaa ggcaaggaaa aacgatagag aaactatgtg 120
tcccaaaata taggaaaaac aagaattatc aaaataatct aaatatattt acaatgtaat 180
aaaatattat atataaaaat aaaaaaacgt catnnttcaa aaaaaaaaaa aaaaaaaaaa 240
aaaaaaac 248

<210> 1793
<211> 549
<212> DNA
<213> Ctenocephalides felis

<400> 1793

aatccacaat tgttacncgt aacaaatcga atcattgaac gttcgcgcga gactcgctct 60
gcttatctcg cccggataga acaagcgaaa acttcgaccg ttcatcgttc gcagttggca 120
tgcggtaacc tggcacacgg tttcgctgcc tgccagccag aagacaaagc ctctttgaaa 180
agcatgttgc gtaacaatat cgccatcatc acctcctata acgacatgct ctccgcgcac 240
cagccttatg aacactatcc agaaatcatt cgtaaagccc tgcatgaagc gaatgcggtt 300
ggtcaggttg cgggcggtgt tccggcgatg tgtgatggtg tcacccaggg gcaggatgga 360
atggaattgt cgctgctaag ccgcgaagtg atagcgatgt ctgcggcggt ggggctgtcc 420
cataacatgt ttgatggtgc tctgttcctc ggtgtgtgcg acaagattgt ccggtctgac 480
gatggcagcc tgtcgttggt catttnctgc ggtgttgtcc gctggacgat ggcagcggtt 540
gcaaataaa 549

<210> 1794
<211> 446
<212> DNA
<213> Ctenocephalides felis

<400> 1794

aatggctcca gcgcttcgaa aagtttatga tcaaatgcct gaaccacgtt gggtaatctc 60
tatgggtagt tgtgctaatg gaggaggtta ttaccattat tcatattctg tagtgagggg 120
ttgtgataga atagtaccag ttgacatata tgtaccagga tgtcctccaa ctgcagaagc 180
tttgttatat ggttgtttac agcttcaaaa gaaagttaaa cgaatgaaaa ccctgcaaat 240
gtggtataga aaataaatta ttgaaaagga gaatattaat tataaaaaat atgctatatc 300
aaaagtgtag aattatgttt acagtaatgt aaacaaattt tatttttttct tcactaatta 360
ttgaaaataa tatgttcata atcctatagc agaatttaat aaataaaaac acattttaaa 420
aatgaaaaaa aaaaaaaaaa aaaaan 446

<210> 1795
<211> 549
<212> DNA
<213> Ctenocephalides felis

<400> 1795

attcatttca atattgggtg aaatcagtat gaattcatta ttaagttcaa tcattattgt 60
ggtattttcg gtatggttaa gtgtaatttt tgcagttaac gtaaagccca aaccaaatca 120
agatgattac tgtaatctaa attgtacaaa tggaccaaat gtaggatgca caaaaccgga 180
tgtacctaga gactgccaaa actttaaact tgtgaatata acagaacgta tgaaaaaggc 240
atttttaaat gcacacaata gaaagagaag acttgttgca gccggaaaag gtcttctgaa 300
agatggtgta cacactccaa ttgctgcaaa gatgcccaac ttaacgtgga atatagcgct 360
cgccaagtta gcagaatata acgtgaagca atgcgaaatg aagcacgatt gtgctaaaac 420
tagacatggt cacactggtc aaaacctatt ttttatggca ctactctcag cccataaaaa 480
actcaactat agcaaaatgg cagttgatgg ttggatgtga agcaaagata cangattgga 540
gatataaga 549

<210> 1796
<211> 549
<212> DNA
<213> Ctenocephalides felis

<400> 1796

```
aaatgtcatt gcgttnctat ttttgattat ttcatcagta tttcaataaa tccaacatat 60
gtctcggcac acatcattat aattaaatta ttagtatcat ttccattaat ttattttact 120
ggcgtcaata tgacggtatc aaacgacgaa ttagttgaat cttttaaagc tttaggttta 180
agtgaacaaa aggcgaaaga aacgttaaaa aatacagttg ttacgaaaaa tttaacatta 240
gcattacatg aggtgagggg cattattttg ccccaaggag ctggttcttt aatctattat 300
gtggcaacaa aaatcaaacc acagattata gatcaattgc ctgtacttgt aaaatatata 360
tcaacatcaa aattagacac aacagttaga gttgatgcgg ccttgcaatt tatgttgtct 420
catttaaatg gatatcaaat cgatgaattt gagaaggctt gtggaattgg gttgtgtaca 480
cctgaacaaa ttgaaaaagc agaaatgagg cttgtggaac ataaagaggc aattttgaga 540
acgatacag                                                         549
```

<210> 1797
<211> 549
<212> DNA
<213> Ctenocephalides felis

<400> 1797

```
antccatata atcggttcgc gaactggttc gaacacatat ctgtgtacac gtgttagtat 60
caaattaatt attaaaagat ataacgatcg aatcatataa gatattttgt gattatccgc 120
gagagaaaga ataaccgctt cgctgacgta gttttggtta tcagttcatt tgattcggac 180
gttccggcaa cttgttagta aacatgagtt tctgtgagaa caataactac acggtggatg 240
aatgttccta tgaaaggatt aaagagacga cagaatttct aattcagaaa accaaatatc 300
gaccaaaaat tggtattatc tgtggatccg gaataggacc cctggctgac aatttacaaa 360
atgcagattc cttcgattac agtcaaattc ccaactttcc cgtaagcaca gttccgggtc 420
ataaaggtcg acttgtattt ggcaccttgg gtggaatcga ggtcctttgc atgcaaggac 480
gttttcatta ttatgaaggc tattcgtgaa tagtgtgttt cggtngagta tgaaatactg 540
gcataccat                                                         549
```

<210> 1798
<211> 549
<212> DNA
<213> Ctenocephalides felis

<400> 1798

```
aattttattt tgcattacta ccttatataa cgaaaacaca aatgtaaaac tcatcccaca 60
gatgaattac ctgatggttg ttgtggcttt gtttttcctt aacgccgtca tttttctttt 120
catgttaatg aaatatttca ctaacaaaca aattttacca acactcattt taagccttgc 180
atttttaagt ggccttatct atttagttga aaccattgta attatccata aaccaattaa 240
cggcagtaca ctgatccaga caaagtcgaa tgatgtttct attttctata ttttccgcca 300
actcagtttt atttgtttaa cctcgctggc gctcttttgt tatggaaaag acaacatcct 360
tgacaacaat aagaaaaaaa cgggaatcct gttgctggcg ctgatccctt ttttagtttt 420
tccccttctg gcacacaatc tgagcagtta taacgctgac tattctttgt atgtcgcgat 480
actgtcggac aaccatactg cgacctgggg aataacttca aaatattggt ttgctgnggc 540
ttttactgt                                                         549
```

<210> 1799
<211> 549
<212> DNA

<213> Ctenocephalides felis

<400> 1799

```
gtaacaaaca tgacgaaaaa ggtgtcacca atttgagcat agcagcacca cctcaaccat 60
tttctgcatc tcaaccttca ccatcggtca tcacacgtat gctgcataat ccaggtggaa 120
atcctccagt gtctattaat ccaactgctc cagtcacttg tgattccaat atgcctactg 180
gatctgtggg attgaccacc tccccttcca gctttcaagg aaaatattac ccgcattctg 240
aaaattattt acaaaggcct aggggaccaa tgggagcagc tatgggaatt tatagacctg 300
caggccctat gggtaattat ccaccacgtg gtatgtatca ttctcctcat catccattag 360
acccctcacc ttctggtgga ggacctatca atgtgcagca aatattctcc cgacgtcagc 420
gcctggacaa attggagctc accacctttg agactgtact cccccaaagc gcgtatgccc 480
tatgaatcat gcatccagca tanggcccag cattctaatt ttnaaatcac ttatggctat 540
nanggcacc                                                         549
```

<210> 1800
<211> 549
<212> DNA
<213> Ctenocephalides felis

<400> 1800

```
aatnaatgga ctgcgttatg aaaggactca ataaaatcac ctgctgcttg ctggcagcac 60
tactcatgcc ttgtgcagga cacgctgaga acgaacaata cggcgcgaac ttcaataacg 120
ccgatatccg ccagttcgtg gaaatagtgg gtcagcatct tggcaaaacg atcctgatcg 180
acccttcggt ncagggaacc atttccgtac gcagtaatga tacgtttagc caacaggagt 240
actaccagtt ctttttaagt attcttgatc tttacggtta ttccgtgatc acgctggaca 300
atggttttct gaaagtggtt cgctcagcta atgtaaaaac atcgccaggg atgattgctg 360
acagttctcg tccaggcgta ggtgatgagt tggtcacccg aattgtaccg cttgagaacg 420
ttcctgctcg tgacctgccc ccctgctccg cagatgatgg atgcgggtag cgtcgtaatg 480
ttgtgcatta tgaaccctcc acgtcttatt ctgccggcgt gcctcaccat taataaactg 540
nttgagcat                                                         549
```

<210> 1801
<211> 549
<212> DNA
<213> Ctenocephalides felis

<400> 1801

gctnagcttt tcaatatact ttaagctaaa acagacgttg taatgaatgg agaaatgtga 60
aggatgaatt acattctaat aaaaatgatt ttaaaatatc atgctacgca tatcatctat 120
tatatgtatg tgattaatgc cgaaatctgc acagcaaaac aaanaccaaa acatttatgt 180
aaactagaat caatatttgc ttgtgtaatt gcatatttat tttgatagcc caatatagag 240
tagatcgact tcgaaattac gaattacata cacacatgta tatatatata tatatatata 300
tatatatata tatataatat gcataaatat acataaatat atgcntgtat atacacatgt 360
ataaatatag aattttacat atgtgtatat gagcatatat atatatatat attcttattt 420
aacctcatat ttataattaa ttatatggta atcgcnaaaa aaaatgataa cacaatgttt 480
tgtaaatcat aaatcacata cgtactaaat atttagtatc tggttttcat acanccctag 540
ncgtattat 549

<210> 1802
<211> 549
<212> DNA
<213> Ctenocephalides felis

<400> 1802

gctaacagtt acaattaatt tttaagtatt gggcgtttta ttattgtatt ttaatatata 60
attctatatt taaatcaatt atgagtttgc ctttagaaat tagaagactt gacgaagcag 120
taattaacag aattgctgca ggagaaatca tccaaaggcc tgcaaatgcc ttaaaagaat 180
tacttgaaaa cagtctcgat gcaaaatcca gtagtattca aatttcagtt aaatccggag 240
gcttaaagta cttacaaatc caagacaatg ggacaggaat tcgcaaagaa gatttggcca 300
tcgtttgtga gcgatttact acgtctaagc tccaaaaatt tgaagattta caaagtatag 360
ccacctatgg attcagaggt gaggccttag ctagtataag ccatatagca agactttcca 420
tacaaacaaa aacagcagac tccgttgtgc tttcaaagca tcttacgaag atggaaattg 480
aaatttcccc aaccatgtgc tggaaataag gacccaatac agtagaagac ttttctatat 540
actacagnt 549

<210> 1803
<211> 549
<212> DNA
<213> Ctenocephalides felis

<400> 1803

gatctaaatc ccagcttaat accggcatcc acgcatagat gtttacacct gagcgggtac 60
gtaattgcca ggcaacccga ctaaaaatat ctgctttcat tggtagcaaa cgatttggaa 120
accagacctc tttgaccagc ccatcaccat cgggatcagc aaatgcctgc aaatacacgg 180
ttgatatttg catatctttc acccgctgaa ttagcacatc aatattgcga tccatttgct 240
ggaggttttc gtcataaacg taatcaagat cgatatgcat tatccgttgt ggtgattttt 300
cctgtacggt aataatttgc tgggcaaact cttttaatga gggattattg gcgattaata 360
cccgcggaat ggaatccaat tgcgacgcat ttgccaaacc tgattcaagg gtgagcccgg 420
tacccaattc gcctatagtg agtcgtatta caattcactg gcgtcgttta caacgtcgtg 480
actgggaaaa ccctgcgtac ccaacttaat cgcttgagca catccccttt cgcagctggc 540
gtatacgaa 549

<210> 1804
<211> 280
<212> DNA

<213> Ctenocephalides felis

<400> 1804

```
aattctgcga tctgnnaggt gtgcgtaatt gtcgcgatct gactgatttt ggcagagaaa 60
ttcgcgcaac ggtgctacaa cgtacccatc ttactgttgg tgtggggatc gcccagacca 120
aaacgctggc taagcttgcc aatcatgcgg caaaaaaatg gcagcggcag acgggtgggg 180
tggtggattt atcaaatctg gaacgccagc gtaaattaat gtctgctctc cccgtggatg 240
acgtctgggg gattggacgg cggatcagca aaaaactgga                       280
```

<210> 1805
<211> 528
<212> DNA
<213> Ctenocephalides felis

<400> 1805

```
gcgcccctat gagtgcagta tatgcaagaa gacattcaca caatcgcggg ctctgaaatc 60
tcacatgctc attcacaatg gtgagcgccc ccttgagtgc aatgtatgca agaagacatt 120
cacacatttt gttgttctga aaagacacat gctcgttcac agtgattagc gtccccatga 180
atgcagtata tgcaataaga cattcaaagg attaagttct ctgaaagaac acttgcggat 240
tcatacagga gagcgtcctt ataaatgtga aatatgcaat aaggaattta ctctattaag 300
agttttgaag aaacacatgg ccattcatag taggaagcgt gatgaaaatc agtgaaatat 360
ctgttaaatt gtatttaaat atttggaaac atgttataaa atattcttac atataataga 420
atgtataatt agataaaata tattttataa atatgtaata ataaacatta aacatgcatt 480
attgtaaata aaggcaattg ttcatattaa aaaaaaaaaa aaaaaaa               528
```

<210> 1806
<211> 549
<212> DNA
<213> Ctenocephalides felis

<400> 1806

```
aataacctta ttgctctact aaaaatatat ataataatct ctgattttgg gacagaaata 60
atttaaatat tttagatagc tttcaataaa gaataactaa tatagtatcc aattaattag 120
tctcctgtca gcatgtctac acgatggtac cctctttatc aaagaggcaa tccacagctg 180
agagtattct taccaaattt ctggttgaaa ttaataaggc ctgttcatga acaaccacca 240
aatgttgtgc aattcgcctg ttccatgcaa atgacaaaat acgatataaa aaactattta 300
gaaaaaatat acaatgttcc aatcatagat gtgagaacaa gaattcaatt aggtaaaact 360
aaacgagatt tgaaaggata tattgtaaaa gaagaagata ctaaattagc ttatgttaca 420
ttgctaaaga agaagtattg aatttctaat atttcaaaaa agatgaagag agcagttcaa 480
gaagattgaa aagntgaatg aatcaaagaa cttataaaat acctggaaga ataaaatcac 540
cgncaccan                                                         549
```

<210> 1807
<211> 549
<212> DNA
<213> Ctenocephalides felis

<400> 1807

```
aattatgatg ttgattncca tgaagatgct gataaagcca atcaggcact gaaagatgcg 60
gtagcggaat taatggaaaa cgaagaaatt cgcgggctga ttattggtga accgaatttt 120
gccgggattg tcggnttaag caataccgng tttacactgc gtgtttcgtt caccacgctg 180
ccactcaaac agtggacggt acgctttgcc ctcgacagcc aggtgaaaaa acatttcgac 240
ctggcgggcg ttcgcgcgcc agtgcagact tatcaggtgc tgtctgctcc gggcgcgacc 300
ccggntgaac cgttaccgcc gggggaacca acgctttaac gctggcgatt gacaaaaacg 360
ggcggcgctg tcgnngttca taaaggtcaa ggcaataaag cactttgctt tgccctgggc 420
ctctcttttt acaccttcac cgcccancgt ccgtaaggca cgnncaccgg ggntagtttn 480
acaacgagat ccagnatgta aaccctaacc tgcttcgnga nncttggttt ctcatatttt 540
ttgnaaaag                                                         549
```

<210> 1808
<211> 549
<212> DNA
<213> Ctenocephalides felis

<400> 1808

```
aatcaattaa atcacttaaa cctgcgagtc atttcgtaat tacattaatt aaaaaactta 60
aaatcattaa ataaataaaa aaccactaac tctatgtgaa ataaatcaaa atttcacgcc 120
gaaatactcc ttaggatgta tagcgaaagg agaaaaagat atacctcgat caccccccttt 180
ctcccaagtg aaaataaaag gttatcagtt tgcaacattg aacaacattc gttgcaaatc 240
gataacaaca tgcaccttca ggatactatt tattatgttc ggcaatgata ttttcacccg 300
cgtaaaacgt tcagaaaata aaaaaatggc ggaaatcgcc caattcctgc atgaaaatga 360
tttgagcgtt gacaccacag tcgaagtatt tattaccgta acccgcgatg aaaagcttat 420
cgcgtgcggt ggaattgcgg aaatattatt aaatgcgttg ctatcagtga atccgccgcg 480
gtgaaggact ggcgctgcat tagccactga attgataaac ctcgctatga cggcacagca 540
cgcttgtta                                                         549
```

<210> 1809
<211> 549
<212> DNA
<213> Ctenocephalides felis

<400> 1809

```
ttgaaagcaa aatattattt tggcactgac taaccctgac ttttgctatg tcaattacct 60
cagcagataa aaataattca taggaaagtg taatttataa caaacgtcgc aatatatatt 120
tcacattttc aagatggaac aaaataattc agttgactgg caaatatcaa gaaaagaagt 180
aagacagagg gctcaatatc ttttagaaac agggcaatgg tcagattgca aatttttagt 240
tggttcagaa tctaatcaac aagttgtgga ggctcataaa ttatttttgg ctattgcctc 300
tccagtattc gaagctatgt tttttggaaa tatggctgag aaaaatgata caattccaat 360
attggatgta cagccagatg ctttcaaggc attgttagaa tatatctaca cagacaacat 420
taatataaac tcttttgcaa agcttgtgaa ctgtgttatt gtgcaaaaaa gttatgttac 480
ccatttggta gagaaatgtc aaaattttat ggctgactca tgctcgaaat gtgtaagcat 540
atgaatttg                                                         549
```

<210> 1810
<211> 549
<212> DNA
<213> Ctenocephalides felis

<400> 1810

```
gtttgaagaa aaaggaaata ttgccgngcg agcagggcag gaagatcgag gcaaaggtca 60
cggacgagct gataaggagg cttataaacg cggataaaat taaggaggcg aatgcggnca 120
gacccaaggc agaaagggcc aagaaggaag cggctgcagc gaaggaaggg gatgaatttg 180
atcaaatggt tgacggttcc aagaagccac tgaagcaacg aatcaaaaag gaagataaag 240
ataaagacaa ggataaaatg aaacagacta aattagattt tagtaaaaag ggaaagaaga 300
acaaaaagaa aggcgccaga aaggattctt ttggcagcga atcatcggat gaagatgatc 360
cggattcaga ttttgaagct catgcatctt cagaatctcc taaaagacaa ttagcaagtc 420
ggggaactaa aaanccggtg aaatacagtc tgcggagttc agatgaagaa gaagagctgt 480
ttgacaataa acaaatggac agtgagccgt gtgtgtaccg tcttcagatc tgattccgtg 540
tgaacctct                                                         549
```

<210> 1811
<211> 549
<212> DNA
<213> Ctenocephalides felis

<400> 1811

```
aatgaaatca gtctttatgc cggttatcag gcaggaatat ggtcaacatc cccaatagtg 60
gcaggaaagc acagatttta tagactaact cgatgctggt gtgatcggcg ataagcccca 120
gaactgccgc tcccagacct cccatgccaa aagcaaaacc gaaaaagagt ccagaaacca 180
taccgatacg tcctggaagc agctcctgag cgtagaccag aatggcagag aatgccgaag 240
cgaggataaa tccaataatc accgttaaaa cccccgtcca gtgcaggctg gcgtagggta 300
aaatcagcgt aaacggcgca acgccgagga tagagcccca aatcacatat ttccgcccaa 360
ttttatcccc tacaggcccg ccgatcaccg tacctgccgc acggcaaaca ggaaggcaaa 420
cagatgaagc tgagcattct ggatagataa tccgaatttt tgcatcagat aaaaggtgta 480
atagctgtgt gctcgcatat agaaatattt cgagaaaatg aggattaaca gatgctgccg 540
cagtacaac                                                         549
```

<210> 1812
<211> 549
<212> DNA
<213> Ctenocephalides felis

<400> 1812

```
atggctctcg cctctntatg ccgacaagct cgctctcttt ctgtctatca gaacaaattc 60
gctactcgta cgacgcgtca gtcttcgatt gttcttgatt cagttcgcga attttcaaag 120
aaaatgggtc tgccacgagt tttcttcgac atgaccgccg acggcgagcc ggttggaaga 180
atcgttatgg agcttcgtaa cgatgtgacc cccaagacct gtgagaactt ccgcgccctc 240
tgcaccggcg aaaagggctt cggctacaaa ggctcctcat tccaccgagt catccccaac 300
ttcatgtgcc aaggggggcga cttcacaaac cacaacggca ccggcggaaa gtccatctac 360
ggaaacaaat tccccgatga gaacttcacc ttgaaacaca ccggcccagg catcatgtcc 420
atggcaacgc aggccccaac accaacggat cccagttctt catacgactg tcaagacacc 480
tggtggacaa ccgcacgttg tctttggatc ggttgagaag gatggatgtc gtgaagaagt 540
ggagagtat                                                         549
```

<210> 1813
<211> 549
<212> DNA
<213> Ctenocephalides felis

<400> 1813

```
aatacgcttt ccacggttng caaccattta aatcggtaag taaaacatca acatattgcg 60
tattcgggta gcgctccagg tagcgtttca cttcctgcgt aaaggcgcta ccccgcctct 120
cttctgactg ctgaacaaag ttctctactt caacgatatt ggtttccatg attcttcgcc 180
tttggtttgt ttttccgctc gttatcaaag cgtaaaatat aatgaccacc attcgaatct 240
gtatgcaaac taaatgtttg tcaaatgtta aattgagttt gcaaaaatga aaacccactg 300
ctagattgaa aaaatattga acataaaggt catttaaagc gcngtaatgg cgatnattta 360
gtccactttg tgagattgag catggaaaat ataatgaaca atncggttat cgggtcgtaa 420
tgtgcangaa caggcttaag ggtcatgcga cccagactct gaagaaangt acctgatgcn 480
tctccatgca ggcggttgct attngctnca catgcgctng cggaccgtna tacttgacaa 540
ctttgccga                                                         549
```

<210> 1814
<211> 549
<212> DNA
<213> Ctenocephalides felis

<400> 1814

```
ggcgcgcagt gacactgcgc tggatcgtct gatgcagggg gcaccggcac cgctggctgc 60
aggtaacacg gcactggtcg gcgtgcaggt ggactcggag cagttcggca gccagcaggt 120
gagccgtaat tatcatctgc gcgggcgtat tctgcaggtg ccgtcgaact ataacccgca 180
gacgcggcaa tacagcggta tctgggtaga tcgggtttcg ccggatgatg gcggaagttg 240
tcatctctgg cttggacggg gcgtaccgcc tgccgttaac cgtcgccgct gaaaggggga 300
tgtatggcca taaaaggtct tgagcaggcc gttgaaaacc tcagccgtat cagcaaaacg 360
gcggtgcctg gtgccgccgc aatggccatt aaccgcgttg cttcatccgc gatatcgcag 420
tcggcgtcac aggttgccgt gagacaaagg tacccggaaa ctgtaaagga aaggccaggc 480
tgaaaaggcc acggtcaaaa atcgnaggcc agaataaagt taaccggggg gatttgccna 540
taactggta                                                         549
```

<210> 1815
<211> 549
<212> DNA
<213> Ctenocephalides felis

<400> 1815

```
aattctgcgc ggctttgatt ttgacggcca ggaggcgctg aaagattctc gcgtgctgat 60
agtgggcctg ggcggcctcg gctgtgcagc ctcgcagtat ctggcaagcg ccggtgtcgg 120
taacctgacg ctgctcgact tcgacacggt ttcgctctcg aatctgcaac gccagacact 180
gcacagtgat gccacggtcg ggcaaccgaa ggtggaatcc gcccgtgacg ccctgacgcg 240
gatcaaccca catatcgcga ttacgccagt caatgcactg ctggatgacg cagaacttgc 300
agcattgatt gctgaacacg atctggtgct cgactgtacg gataacgttg cggtacgtaa 360
tcaactgaac gcaggctgtt ttgccgcgaa ggtaccgctg gtttccggcg cggcaattcg 420
tatggaaggt caaatcaccg tctttactta tcaggacggt gaaccgtgct atcgtgctta 480
ccgttgttgg tgaaaatgat taacctgcgt ggaagcagcg taatgcaccg tgatcgggta 540
ttggtcgtg                                                         549
```

<210> 1816
<211> 549
<212> DNA
<213> Ctenocephalides felis

<400> 1816

```
atcaattcgt attagacttt ttgataaaaa atgctgtcta aagcttcgct tttggccaag 60
gtatcccggc cactgactgt ggcagtgcga acaacatccc aggctgcaac atgccctgct 120
cctacaaagg tagaagaagc cgatagtgct gaaagagatt tggtcaactt cccaaggcca 180
acacgtttgg aacattcacc taaagttcgc tttggattca ttccagactc atggtttgaa 240
tttttctatg agaagaccgg tgttactgga ccttacatgt ttggaactgg tttaattact 300
tacttatgtt caaaggaaat ttacgttatg gagcatgaat tctatactgg tatttcattg 360
ggtattatct gtctctatgc cactaaaaag ttgggtccac atattgcaaa atacttggac 420
aaagaagttg atgcctatgc cgatgaatgg aattcaggtc gtgtagaaga agttaaaagt 480
tccaagatgc cattgaagga gaaaagttgg acaatggaga gctgaagtac ttatgttgat 540
ggatgcaaa                                                         549
```

<210> 1817
<211> 87
<212> DNA
<213> Ctenocephalides felis

<400> 1817

```
aatttccagc gcgctcagta ccaccacgtc gagacgatca accgatgcgc ctttcgaacc 60
ccagttagcg tactggttgg cgctgat                                     87
```

<210> 1818
<211> 549
<212> DNA
<213> Ctenocephalides felis

<400> 1818

```
aaaaaacaaa cgcagtttgc taccatttgc ggtaaaaagt taattacaaa cactacaaaa 60
atgaaagcgt tcatcgtagc agctttactg atcgccatgg tggcagctcg tccccagaaa 120
gaagtggaga tcctgcgtta cgacagcgat aacattggcg tcgacggtta caaattcgcc 180
tacgagctga gcgacggaac caaccgccaa gaagaagctc aattgcagaa cgccggaacc 240
gaaaacgagg caatctccgt ccgcggctct tacacctggg tggcacctga tggacagcaa 300
tacaccgtca actttgtcgc cgacgaaaac ggttttcgac cagaaggagc acacattccg 360
aaataaaacc accaaattaa attagactac tatgtatgat actcaaatga tacctgcatt 420
gaatatgtca tgtatgccaa tatattaact gaattgataa cttaatagat caaaagcaat 480
atatatatat atatatatat atatatatat atatcgcnca atgtntgtat cattataana 540
nttttatca                                                         549
```

<210> 1819
<211> 549
<212> DNA
<213> Ctenocephalides felis

<400> 1819

```
aataccattt tcaaaatcac catattgctt caaaaaatcg ggatgtgttt ccccaaaatc 60
ctctatatct tcccagcctt ctgcaccaga aataacggca caaatagtca acagtagaat 120
atccgataat ttatgttcca ctttccaggc ttgtctgtaa tcggggataa tagaaatatg 180
ttccatcaat tttttaagtt ccattttgtt ctccttaatt atgtaagaag tatttgatca 240
tgtataagca ataaaaaaca gcttcaggta ataaggaata tctcaatttt taaacataaa 300
atgcgaatta tttagtacaa aaagcaggga aagattacga aagcccgctc cccgcaagga 360
ctgacgcgag gggggcccg gtcccaattc gcctatagtg agtcgtatta caattcactg 420
gcgtcgttta caacgtcgtg actgggaaaa ccctgcgtta cccaacttaa tcgcttnagc 480
acatcccctt tcgcagctgg cgtaatagcg aagaggccga ccgtcgcctt ccacagttgc 540
cagctgatg                                                         549
```

<210> 1820
<211> 549
<212> DNA
<213> Ctenocephalides felis

<400> 1820

```
agtnnatcta taaagtgaca tatcaataaa tatttattta gcactaagtg tttttgttta 60
taaaatctta aaaacaacaa ccatggctcg ttttatgatt gctttatccc tggcggtttt 120
aatcgtcgcc gtaaccgcca ctccctacgg tgccggagga cacggaagca gtggcggcgg 180
acacggctcc gggcttgact ccggttttgg ggggcacgga agccgcggca gtggacttgg 240
aggcagtagt ttttccagtg gtagccacgg tggcggactt ggaggtggaa gcagaggcca 300
tggaggccta ggcggatctg gaggttttgg aggccaagga ggccttggtg gtggacacgg 360
aggcgttgga ggcggacatg gaggtcacag tggcggagga cgtggaggac atggcggcag 420
tcattctaga tgaaatataa aagagaactc tctgaaaatt tgttggaatc tgtgattccg 480
tctttatcca ccagaaatta attaaaaatt tgaattaaca atgaataata tgtatttttg 540
ataatttga                                                         549
```

<210> 1821
<211> 549
<212> DNA

<213> Ctenocephalides felis

<400> 1821

```
cccatggtat ggagggantg ggacctgaac attcttctgg acgtttggaa agatttcttc 60
agctctcttc agatgatccc gattatttcc ctccagaatg cgaagaattt gctgttcgac 120
aattgcatga tatcaactgg attgtagcca actgcacaac acctgctaat tattttcata 180
ttttacgtag acaaattgct ttacctttcc gtaagccatt gatcattatg actccaaaat 240
cgttgttgag acatccagaa gctaagagtt cttttgatca aatgacagag aacactgaat 300
ttatcagaat gattccagaa gaaggaccag cagcatcaga tcccagctct gtcaagaagt 360
taattttctg ctctggtaaa atctactacg acttgaccaa tgcgcgccgt gaaaagaaat 420
tggataattc tattgcaatt gctagagtag agcaaatttc tcctttccca tatgatttga 480
taaagaaaga atgtgcgaaa tatctaatgc aaatcttgat ggctcaagaa gagcaaaaga 540
catgggtgc                                                         549
```

<210> 1822
<211> 549
<212> DNA
<213> Ctenocephalides felis

<400> 1822

```
gctatcttgc ggacaacacc gttgcgagag caactgtcac aagggacgtt gtgacccatg 60
ctggcgttca agttttgatg agctgacatg tgaatgtggc tacagcataa ggtatccacc 120
cattccatgt ggcgcccgca aaccaacttg tgatcaagtt tgctcaagac aacactcatg 180
tagtcatccc gttcttcata catgtcatag tgaagccgaa tgtccaccat gtaccgttct 240
atgttccaag tggtgttttg gtaaacatga gatgcgcaaa actatcccct gccaccaaaa 300
ggatttttca tgcggccgtc catgcggaaa agaattacca tgtggtaagc attcatgtct 360
attgccctgt cacaaaggtg catgtctaca agacgggaag acctgtagtc aaccatgtgc 420
tacgcctcgt tcttcttgca atcatccttg tcgtaccctt gcatgaaggt atttgccaga 480
cacaccttga aagaaaagga ttggtcatgt aatgtaaact cgtcccacca gacatgtcag 540
aaacgtcgc                                                         549
```

<210> 1823
<211> 549
<212> DNA
<213> Ctenocephalides felis

<400> 1823

```
aaaacccgac gaactgnaaa aaccgccgcg ccagatatcc tcttcgcgta ccacttgttt 60
cagtcggcca tattccgcaa aggcgacctc acgaatatcc gccttgccag gctcatcttc 120
accgccaata aaaccaatac gattaacgcc ctggttgata tagaagtcga tgatttcttt 180
actgatgcgt gccagatcga tatccaccgc atcgtaaccg ctgccgggtt cgtgaaagtc 240
gataaaacag atattgtcgg tcaacgcgct ggcagcggcg cgcagggcgg gcgtgggttt 300
gccgacaatt aaaataccgg tgacgttttt aatgtctggt aagccgctgt gttcataaca 360
gttggtgagc tcgatgccca gcttttcgca ctgggtttca atgccgtggc ggatcgcaga 420
tagtaaggat cgtgatctcc agctcctgtg gagctgtaga tagccagaat atgggttggt 480
gactgacctg ttggagttac ggcactactg gcttgactca nttttcggga tctcgnaatg 540
cgatgttcn                                                         549
```

<210> 1824
<211> 549
<212> DNA
<213> Ctenocephalides felis

<400> 1824

```
gtcgaactac acgttttggc gacagatttt tcgtacattg tcaataatta atattttaac 60
aaaagcgaat acagtgaacg gtcagtgatt tttatgttat ccgttaacgt aattcacttg 120
tgatacaaag caataggccg tcaaaacaac cgtgcgggta taatggcagc actccctcgg 180
cgtataataa aagaaacact tcgactaatt caggagccag tgccgggaat cagtgctatt 240
ccggatgaca gtaatgcacg ctattttcat gtcgtcgtag ccgggcccga ggactctcca 300
ttcgaggggg gctcatttaa actagaatta tttttacccg aagactaccc catgtctgcg 360
cccaaggtca gatttatcac gaaaatatat catccaaata ttgacagact aggtcgtatc 420
tgtttggaca tactgaaaga taaatggagc cccgctctta aatcagaacg gtactcttat 480
caatacaagc cttactgagc gccctaatcc agatgatcct ttggcaatga tgtggtgatg 540
tggaagtaa                                                         549
```

<210> 1825
<211> 549
<212> DNA
<213> Ctenocephalides felis

<400> 1825

```
aattcggtaa ttaattctta acatgctttt actaataatc tcaattgctg gccctataat 60
attgcgctag cattgctttc tggttgtatc agcgatactc aaaaattctt aatacaatac 120
tcattcgact ggtacttatt tgtaactcag ttatattttt tcgcccggtg attcagaaga 180
atgcaaaaaa cggctaccac tccatcaaaa atacttgatc tcactgccgc ggcattttta 240
cttgtcgcct ttctgacggg tattgcgggc gctcttcaga ctcctaccct aagtatattc 300
ctcgcagatg aactgaaagc ccgtcctata atggtaggtt ttttcttcac cggtagcgct 360
attatgggaa ttctggtcag tcaatttctg gcaaggcact ccgataaaca aggcgaccgt 420
aaattactga ttctgctatg ttgcttattt ggagtgctgg ctgccgcttt ttgcgtggaa 480
tcgcaactac ttcattctcc tctcaacggg cgtcttctga gtagtttgtt caccgaaacc 540
cgaaatgtc                                                         549
```

<210> 1826
<211> 541
<212> DNA
<213> Ctenocephalides felis

<400> 1826

```
aaggnggttt agtaatatta gccaatatgg caaagtattt agggctagat aaagtcgctc 60
gcttatttag gattgtgtct gctaatggcg gaattagagg aagtcttgca aaactggcaa 120
gaacagatga tttaaaactg ggcactctag taggagaaga caaatacggt aataaatatt 180
atgaaaataa tgaatacttc tacggacgta atagatgggt cgattatgct ccacatgttg 240
gattaaatta tgatgcctcc caagtgtgtc ctgagtggtt tggttggctc cattacaaaa 300
cggatcttcc tccaacaaaa gatccagcca gagcgcatca taaatggatg tcgaatcata 360
gtgaaaactt atctggaaca gatcgtcaat atgttccata ttccactact gttcccaaaa 420
tcaggcatgg aatcccaatg caaaataagt catgaaaatt ttggatttac ttagtctaat 480
tgtaataatg taattctatt taaaattatg gacaagatca agttaaaaaa aaaaaaaaaa 540
a                                                                 541
```

<210> 1827
<211> 549
<212> DNA
<213> Ctenocephalides felis

<400> 1827

```
ggaagtcgga ctcggcacaa agaccgactt ttgccgaagc ccggcacgat ctgaccgccc 60
tgcaccgtgc agagtctgcc gccctggacg ccatggacga tgcctcaacg acggactgtt 120
ttgatgtcag tggattcagc gaggactacg aaaatggagt tgtgtatttt gataggcgga 180
tttcggaatt tgaatgtgat atttgattta ggctcaaatt gcacatcggg aaaatcaaat 240
tattatctag ctttgtgcaa ttgggaggga acatcgattt gcagatactt tcttgagcaa 300
atatttttat atttatcaaa tgcatagtat ggaaaagtct ctagatactt tggtctagta 360
cctggattat agcgaaatta gccaaatttt caattatata aacagaaaat tatttaggct 420
caaattgcac attggcattg aatttgccga aattgatttt ctagcttcgt ataattgtat 480
tagaagcatt gatttttttt tggaatacaa aaaggcgtca agtgaaggaa aaattgattt 540
ggagatata                                                         549
```

<210> 1828
<211> 549
<212> DNA
<213> Ctenocephalides felis

<400> 1828

```
cacaagacat gtcagctgag tcttagtcct tcgtctgctg ctaaaagtat attagaaaat 60
cttgtatttt taatatttgt gtttgatttt ccagtcggtt acgttatata cattatcaca 120
gaatcatggg gcaagattcg ggagtaaagg attttccttc tttgcctgaa gacaaaatag 180
atatgattgc tgctaccagc ttattacaac aacatgctgc ggatatccgt cagcaaaaaa 240
ttaactggac atcatactta cagtctcaaa tgatcactca agacgacttt aatttcataa 300
gtgcttatga ctctactgat tctaagggcc gcataaggct tctcacagat cgcactcaag 360
cagctaaaac atttttgaac atcctaacac atgtcagcaa agatcaaaca attcaatatg 420
ttttaatttt gattgatgac atgctgcagg aggatagatc cgtgtcgata ttttccatga 480
gtatgctgta aaaccaagaa agtgatgggc ccttcatgaa ttattgatcg cagacagttc 540
attgtacat                                                         549
```

<210> 1829
<211> 549

<212> DNA
<213> Ctenocephalides felis

<400> 1829

```
ggcctcgttc gccgcctgca atgccccggc ttcatcgccg gaacgctgca actgagcaac 60
atacgcaatc tgctccgccg acacgttatg gaactggcga gccatcgccg tcagccccga 120
cgtcgggtct gtggtcagct tcccgaaggc ttcagcgacc ttgtccacct ccacgccgga 180
tgcagaggag aaacgcgcca cactctggct gatggacgca atctgagcct caccgcttac 240
ccccgcctta accagtgcgc tgagtgactc gctggtctgg ttaaacgtca gccctgccgc 300
ctgcccggct ctggacagga ccagcatacg atctgccgtc agtcccgctg attgccggaa 360
```

```
aggaccagcg ttttgttgaa atcggacagg gttgagttgc ctgataccag gcatacgcca 420
gcgcaccggt cgcaccgcag cgaggtggcc ccacatcggc agggtgatcg accggcaagc 480
cccctgaaca tggggatatc cgccgaagga gtcttacctg ccccctgtgc anaggatagc 540
acggatttg                                                         549
```

<210> 1830
<211> 549
<212> DNA
<213> Ctenocephalides felis

<400> 1830

```
aagtgtcagg gcgtcttaag gatcatttgg acttcttaat taatttccaa ataatggctg 60
atgcaaccga aggaaacatt gaaaaactat ctaaaaatga gctcaaaagg aggttgaaag 120
cagagcaaaa ggcaaaagaa aaagctgaaa aggcagcagc tgtaccagaa aagcctgtaa 180
aagaagccaa aaaggaagta acaaaagttg atgaagaaat tagcccaaat gaatatttta 240
aactccgaac tgctgcagta actgcattaa aaaattcaaa tgatcctgat cagcatcctt 300
atcctcataa atttcatgtt agcattggtt tgactgagtt tatagagaaa tacaaggatc 360
ttcaggatgc gcaaatattg gaagatgtta ctttgtcagt tgncggaaga gtgccgccat 420
cagggagtct ggagctaaac ttgtatttta tgattgaggg gagaaggtgt caaaattcaa 480
gttatggcaa atgcaaatat tagttctgag gaaaaatcca gaagacacat cgaaatccgc 540
gtggtgtat                                                         549
```

<210> 1831
<211> 549
<212> DNA
<213> Ctenocephalides felis

<400> 1831

aatcgcggcg gtgatcattg agccgattgt ccagggcgca ggcgggatgc gcatgtacca 60
tccggaatgg ttaaaacgaa tccgcaaaat atgcgatcgc gaaggtatct tgctgattgc 120
cgacgagatc gccactggat ttggtcgtac cgggaaactg tttgcctgtg aacatgcaga 180
aatcgcgccg gacattttgt gcctcggtaa agccttaacc ggcggcacaa tgaccctttc 240
cgccacactc accacgcgcg aggttgcaga aaccatcagt aacggtgaag ccggttgctt 300
tatgcatggg ccaactttta tgggcaatcc gctggcctgc gcggcagcaa acgccagcct 360
ggcgattctc gaatctggcg actggcagca acaggtgggc ccggtaccca attcgcctat 420
agtgagtcgt attacaattc actggcgtcg ttttacaacg tctgactggg aaaaccctgc 480
gttacccaac ttaatcgctt nacacatccc ctttcgcagt ggcgtatagc aaaggccgac 540
cgatcgcct 549

<210> 1832
<211> 549
<212> DNA
<213> Ctenocephalides felis

<400> 1832

aggacaacat cagagtttga aattgtacgc agcattaaag aaaaagcctg ctacttagct 60
agtaatcctc aaaaggaaga aagtgtagac acagaaaaaa ttcaatatgt tcttcctgat 120
ggttacccat tagatattgg tccagctaga tttagagctc cagaagtttt attcagacct 180
gatctaatcg gtgaagaaag tgaaggcttg catgaagttc ttttatattc tatcgaaaaa 240
gctgaacgag atttgaggaa agtattgttt caaaacattg ttttatcagg tggttctact 300
ctatttaaag gctttggtga cagactgttg tcagaaattc gaaaacaagt accaaaagat 360
atgaagatta agatttctgc tccacaagag cgtttatatt ccacctggat aggaggttcc 420
atttagcttc attagataca ttaagaaaat gtgggtttct aagaaagagt atgatgaaga 480
tggcaaagag ctgtcataga aaacttctaa taatagcatt gcttctcact cantaatatt 540
aaggtattt 549

<210> 1833
<211> 549
<212> DNA
<213> Ctenocephalides felis

<400> 1833

tgccattccg ccacggcgat attttacacg tgacgaacgc cagcgacgac gaatggtggc 60
aggctcgccg ggtacctcct ccaggaggng ctgatgaagg tacggntggc atagttcctt 120
ctcgcaaacg ttgggagagg aaacagaagg ccagagatcg cagcgtcaag ttccagggac 180
aacagcctgg acaagttggg gataagcaaa gcacgctcga taggaagaag aaaaataact 240
ttgcattcag cagaaagttt ccatttatga agtctaaaga tgataagagc gaggatggat 300
ctgatcagga acgcgaagaa aacgttcttt cttatgaagc tgtgcaacaa ttgactataa 360
attcaccaga ccagtcataa tattaggacc gctcaaggat cgcgtcaatg atgatttgat 420
ttccgaattt cccgaaaaat tcggcagctg cgtcctcata ctacaagacc gaagagaggt 480
atgaagtggt ggcgagatat cacttctggc atccgcgaac aaatggacgt gatatcaaat 540
attgttatt 549

<210> 1834
<211> 549
<212> DNA

<213> Ctenocephalides felis

<400> 1834

```
aatttcgtgc agcagangta aataaatctg gcggagcctg ggagctccgc cagagccgtt 60
aaacagctgg catattgcgc ccgtaataaa tctcgcgcat ttctttccac agcgcagcgg 120
taatttcctg gcgctcgctg tcggttaagt cttccggttt ggtgtggaac atgtagtgct 180
taaggtcgaa ctctttaagc aacatcttgg tatggaagat attttcctga tagacgttca 240
catccaccat gtcatacagc gccttcatat cgtcagacat aaagttctga atcgaattaa 300
tctcatggtc gataaagtgc ttcataccgt taatgtcgcg ggtaaaaccg cgcacgcgat 360
aatcaatggt tacgatatcg gactcaagct ggtggatcag gtaattcagc gccttcagcg 420
gagaaatcac gccgcaggta gagacttaat atcgggcgga aggacataaa ccgcttagga 480
tgactttcng gtaggatgtc gcaaatatga ctttataaga tggcacgacc gtttgcagtg 540
gccgggtgt                                                         549
```

<210> 1835
<211> 549
<212> DNA
<213> Ctenocephalides felis

<400> 1835

```
gtggcttcgg ctttgataac ttcctcaaga ttattcattt tgattgtggt aaaaaatgcc 60
ttctttaaac ttgagcgaaa atgatcgtgc tgtgttaaat agtatattta atccgtctca 120
accattagga gaaaatgcat tcgaggaaga tattccagaa ttattagaag atcaagaaga 180
cccggaaaca ccatttttga acgacgcacg agccttggaa gctgaagctg tccgtttggc 240
cgaagccggg gatttggcag ccgcgctgac cgtggtggac cgcgcgatag acctgctgcc 300
cggtcgtcct tcaggttaca acaatcgcgc acaagttcac aggctggcgg gacgagacca 360
agatgctttg tcggatttaa ctatggctgt gaacctcagc tccggaaaag gaagatctgg 420
tgtgcaggct ttatgtcaac gggcctctgt cagaaaaact ggtgaagacg attagctcgg 480
gaagatttaa caaagcgaag cttagctcac ttgcaaaaat cagtagtgga atgaatctta 540
tntgnttat                                                         549
```

<210> 1836
<211> 549
<212> DNA
<213> Ctenocephalides felis

<400> 1836

```
ctttactccg agcaagtngt gggacgactc attattattt attttgtgat aatcttttaa 60
atctcctaaa atgaggatct acaaagacat cattactggt gatgagatgt tctcagacac 120
atataaaata aagttggtcg atgaagtttt gtacgaagtg accggcaaat tggtttcaag 180
gtctcaaggg gatatccaaa ttgaaggttt caacccatct gctgaagagg ctgatgaagg 240
aactgaaaca gccacggaat ctggtgttga tgtggtctta aatcaccgcc tttgtgaaac 300
ttttgccttc tcagataaaa aatcatacac tctttattta aaagattata tgaaaaaatt 360
ggtggcgaaa ttagaggaga aatcaccaga acaagttgag gtattcaaaa caaatatgaa 420
caaagtcatg aaagaaattt aagccgttta aagaaatgca aatgttcact ggtgaatcaa 480
tggttgtgat ggcatggttg ctcttatgga atatcgtgaa atagatggtg aatctgtcaa 540
ttctgtgtc                                                         549
```

<210> 1837
<211> 549
<212> DNA
<213> Ctenocephalides felis

<400> 1837

```
gttcaaataa ttctttatgt tttaaacaat cgttatagtt taatagatgg tcagttataa 60
atttagcgtt tatctattgt tatttgttac tcgttagtta gtattatttt aagtaggatt 120
cagtgagtga tatttgtgaa ttactcgaaa cacatacata tataaactca gaataatggg 180
aactcgagat gacgaatacg attatttgtt caaagtggtg ctaataggag actcgggtgt 240
aggaaaaagt aatttgcttt ctcgcttcac tagaaatgaa tttaatcttg agagtaaatc 300
cacaattgga gttgagtttg caacgaggag tatacaggtt gatggtaaaa caattaaggc 360
gcaaatatgg gatactgctg gccaagaaag gtatagagct ataacatccg ctattataga 420
ggagctgtgg tgctctctta gtttatgaca ttgcaaagca cccaacttat gaaaatgtag 480
aacgtggtnc gagagctaag agatcatctg acagaattag tgtatgctgt gggaaacaaa 540
ttgtctgaa                                                         549
```

<210> 1838
<211> 549
<212> DNA
<213> Ctenocephalides felis

<400> 1838

```
atttttttg ccgggtcgta gtaaccaatt taaggataat ggctcctcca ccatacgcag 60
acttgggaaa acaagctagg gaggtattta acagtggcta tcattttggt cttttcaaat 120
tgaatttgaa aactaaaact gcctctgggg ttgaattcac ttcaggagga acttntgaac 180
atgaaactgg caaggtattt ggatctttgg agacaaaata caaagtaagt gattacggtc 240
tcacttttc tgaaaaatgg aacacagaca atactttagc tacagaagtt tccatacaag 300
atcaaatagc taaaggtttg aaagtatcat tcgactgctc tttcgcacca caaacgggaa 360
gcaaaactgg tgttttgaaa actgccttct tacatgatag tgttgcagta aatgctgatg 420
taaatttgaa tttatcagga cctttgatca atgccagcgc agtagttggt atcaaggatg 480
gtggcggtat aaactggatt tgntctgaaa ttcaaggcac aaagacactt tgccttggat 540
ctcacagga                                                         549
```

<210> 1839
<211> 549
<212> DNA
<213> Ctenocephalides felis

<400> 1839

```
attaaaatat tattgttnta attgaatttt gagttggttt tgagaagcta gtgccatgtt 60
tgattcaagg attactgtcg gtttgctgct gacaataagc tgttacttag tgtcgtccct 120
gccccaaaga ggaggattcc aacctcaggc tccaggaaac caaataccaa ttttacggta 180
ttcattcgaa cctaatccag atggttccta caattacaac tatgaaactg gaaatggaat 240
tcaagtagaa gagcaaggtt acttgaaaaa tgctggaaat ccacaaacag aagctcaggt 300
gatgcaaggt tcctactcct acacgggccc cgacggagtc gtctacacgg tgaaatacat 360
agccgacgaa aacggtttcc gggccgaagg cgcccacata ccctcggcag gaggacccgc 420
aaggcggcgc ccggcggtag attttctagg aaggaaaaca tctagnccca ccacaacatt 480
ttaattatga aaaacacagc gccatgatgc gagtcgagga acaatggact ataataatta 540
atctncgng                                                         549
```

<210> 1840
<211> 549
<212> DNA
<213> Ctenocephalides felis

<400> 1840

```
gccaacttgt atgccacgtc gaaaacacta aaaacaaatc gaattaaata aaattataaa 60
ataaaacaaa tataataaca aagtattaaa acgtgtgata tattatttat attattatta 120
ttatcaaaag tgtgagaacg tacaacgctc ttgttaattt tccagacagg aatttttagt 180
gttgccatat atataagcgc gaggaaagat ttacatacaa gatgggttgc ggaatatcgt 240
tcgttaaata cgttctgttc gtgttcaatt taatatttgc gctatgcggt ctcgcagtgc 300
tcgccgttgg cgtggtcttc aaattgaagt tctcagagat ccagcaaatg ctccaggact 360
taaacgtcca ggccgcacca atactcttca tcaccgttgg aagcatagtc ttcataatcg 420
cttcttcggg tgctgcggag cgattaggga aagtcattgt atgacagtca cttcgcagtc 480
ttttaatcgt ttgctgacgc caagtcgtga tcgcgctgtg tcttcgctat gcgtgcatca 540
acaagatct                                                         549
```

<210> 1841
<211> 549
<212> DNA
<213> Ctenocephalides felis

<400> 1841

```
aattaaacgt gcactggaag gcggcataaa tttctttgat accgccaaca gttattctga 60
cggcagcagc gaagagatcg tcggtcgcgc actgcgggat ttcgcccgtc gtgaagacgt 120
ggtcgttgcg accaaagtgt tccatcgcgt tggtgattta ccggaaggat tatcccgtgc 180
gcaaattttg cgctctatcg acgacagcct gcgacgtctc ggcatggatt atgtcgatat 240
cctgcaaatt catcgctggg attacaacac gccgatcgaa gagacgctgg aagccctcaa 300
cgacgtggta aaagccggga aagcgcgtta tatcggcgcg tcatcaatgc acgcttcgca 360
gtttgctcag gcactggaac tccaaaaaca gcacggctgg gcgcagtttg tcagtatgca 420
ggatcactac aatctgattt atcgtgaaga agagcgcgag atgctccact gtgtatcagg 480
aggcgtggcg gtattcatgg acccgctgca agggccgttg accgtcgtgg gagaactccg 540
acgntgngt                                                         549
```

<210> 1842
<211> 549
<212> DNA

<213> Ctenocephalides felis

<400> 1842

```
aatatcgccc tgaaccntcg aaacctgaat ggttttctcc ggttgngggg taaaccactg 60
gatgtaacgc agcgggaagg gaagggcaaa cagcacgacg gccaccacca gcggacgcca 120
gttgcgtttg accaacgcca gtgccagcag gccactaacc atcatcagca ggaagttaat 180
ggcttccacg cccattatcg gtgccagccc ttttaacggg ccatcaatct ggctatagcc 240
gaactgtaac cacgggaagc cggtcagtac ccaaccgcgc agaaactcgg tcacttgcca 300
gagggcaggg gcggcaatcg ctacgcgcag ccaggtggtt ttcggccaca gacgcgacag 360
cacgccagca aacagtccgg tatacagcga caaatacgcc gcagcagcac caccaggaag 420
atgttaaccg ggccaggcat tccgcaaagg tcgcgatgct gacatagacc cagttatccg 480
ctgcaaagag gcnaatncca gcaaaagcca atagcggcag atggagtgac ggcggtaaag 540
gcaacgctg                                                         549
```

<210> 1843
<211> 549
<212> DNA
<213> Ctenocephalides felis

<400> 1843

```
acttacttca agtcaagcgt catcgccgtt cgtacccaaa acccaaacaa gacttattgt 60
gttcatttgt gttgtttagt gctgcatttg aggtcctcaa tactatcttg aaataataaa 120
atggctcgta ctaaacaaac tgcccgtaag tcgaccggtg gaaaagctcc acggaaacaa 180
ttagctacga aggccgcgcg taaaagtgcg ccatccactg gaggcgtcaa gaaaccccat 240
cgttatcgtc caggtactgt tgctcttcgt gaaatccgtc gttatcagaa atccactgaa 300
ttgttgatcc gaaaattgcc attccaacgt ttggtgagag aaattgccca ggatttcaag 360
actgatctac gtttccagtc agctgctatt ggtgctctac aggaagccag tgaggcttat 420
ctcgtggctt atttgaagat acaaatttgt gcgccattca tgccaagagg gtaacaatta 480
tgcctaaaga tatccagtta gcgcggcgaa ttcgtggtga cggctaaaat cggttataag 540
aancaattt                                                         549
```

<210> 1844
<211> 549
<212> DNA
<213> Ctenocephalides felis

<400> 1844

```
aatttctggt cagactttga ataccgactc aataaacacg gctctgtatt aaatgctgta 60
ttaatcatgc tggcgcaaca tgctctgctt atagcaattt caagcgactt aaatgcatat 120
ggtgttgtgt gtgagttcga ctggaatgat ggaaatggtc aggaaggatg gcctccaatg 180
gatggcagcg aaggaataag aattaccgat atcgatacat caggaatatt tgattcanat 240
gatatgacta tcaaggccgc ctgagtgcgg ttttaccgca taccaataac gcttcactcg 300
aggcgttttt cgttatgtat aaataaggag cacaccatgc aatatgccat tgcagggtgg 360
cctgttgctg gctgcccttc cgaatcttta cttgaacgaa tcacccgtna attacgtgac 420
ggatggaaac gccttatcgc atacttaatc agcaggagcc caaagaatgg atcaaacact 480
atggtatcca gactaaatca ctatcgcctt tatggcgata aaagatgttc gtnaaccgca 540
ccttataaa                                                         549
```

<210> 1845
<211> 549
<212> DNA
<213> Ctenocephalides felis

<400> 1845

```
aattcatccg ttccgcatat attttgagga gctacaacca ggcgacagcc tgttgactcc 60
ccgccgcaca atgacagagg ccgatattgt taactttgct tgcctcagcg gcgatcattt 120
ctatgcacat atggataaga ttgctgctgc cgaatctatt ttcggtgagc gggtggtgca 180
tgggtatttt gtgctttctg cggctgcggg tctgtttgtc gatgccggtg tcggtccggt 240
cattgctaac tacgggctgg aaagcttgcg ttttatcgaa cccgtaaagc caggcgatac 300
catccaggtg cgtctcacct gtaagcgcaa gacgctgaaa aaacagcgta gcgcagaaga 360
aaaaccaaca ggtgtggtgg aatgggctgt agaggtattc aatcagcatc aaaccccggt 420
ggcgctgtat tcaattctga cgctggtggc aggcagcacg gtgatttgtc gattaatcgg 480
tgaatgaagg naacggcgaa tagttgcctt tatttcacta agtttggacg ttgcacatta 540
tgcatgatg                                                         549
```

<210> 1846
<211> 549
<212> DNA
<213> Ctenocephalides felis

<400> 1846

```
aggaagaaga gatggangcc taccgcaaac agaccagttt ggaaatcgag caattgaaca 60
tgcgcgttgc cgaggctgaa accaaattga agaccgaagt tcaacgcatc aagaagaagc 120
tccagatcca aatcaccgaa ttggaattgt ctttggacgt tgccaataag actaacatcg 180
atttgcagaa gaccatcaag aagcaatctt tgcagttgac cgagatccag gccgcctacg 240
atgatgtcca acgccaattg caagtgacct tggaccaatt gtccgtctcc cagagacgcg 300
tccaatcttt gactgctgaa gtcgaggaag tgcgcagcaa ctacgaatct ttgtcgcgcg 360
ccaaacgcca agtcgaacag cagtacgagg agagcgtcgc cgcatcaacg agttgacggt 420
aatcaacgtc aatttggcaa gctcaagagc aagatcgagc aggaattgtc gctttgagcg 480
gagactcgag caagtcccca ggaattgaga gtcagcgacg agagatccan gcgtcagtcg 540
actgaatca                                                         549
```

<210> 1847
<211> 549
<212> DNA
<213> Ctenocephalides felis

<400> 1847

gtcttntaaa tacatcatga aacctttttgn gggattanna ttgtgcctgg cggtggtgan 60
ctgcggtgat gtatccgaaa agaaacaaga gaaacgcgga ctggccggnc tcggcacgca 120
cggaggcggt gccttactgt natccgntgg aggacacggn ggnctcggtg gaggatacgg 180
tggcggatat ggaggtggtt ntggaggcgg tctcggcggt ggactcggcg gtggatttgg 240
cggcggagcn nnattcggcg gaggtgctgg attnggcgga ggattnggag gcggcngctg 300
gangtggnng cggtggcggt ggtggcggng ctggtcttgg tggtccagtc acccttggca 360
cacgcagtna cgaactacta ctgtgacaca nggtattcca tacgccgtcc acaaccttac 420
ccantgactg tnacacgcac cgttggagta ccancentca accnntnccn ttgtncttcc 480
aagactgtnc agnctctgtc ctcaagtnac cagtgcagtc ccctnaccng ccatggttgn 540
ccnctgcnc 549

<210> 1848
<211> 508
<212> DNA
<213> Ctenocephalides felis

<400> 1848

gaancctaaa ggtgaaggat acaaaggcag taaattccac cgtgtcatca aggacttcat 60
gattcaaggt ggtgacttca ccaggggaga tggaactgga ggccgctcaa tttatggaga 120
acgttttgct gatgaaaact tcaagttgaa gcattatggt gctggatggt tgtccatggc 180
aaatgctggc aaagacacta acggatctca attctttatc actacaaaag ccactggctg 240
gttagatgga cgtcatgttg tattcggaaa agtgattaag ggaatggatg tggtgaggaa 300
aattgaatca acatcaacag attccagaga caaaccacaa cgtgatgttg aaattgtaga 360
ctctggtgct gaagctgttt ctgaacccta cggtgtggct aaagaagatg caactaatta 420
aatgatttat taattttgat aatttttaat cacttcaaga caaattgttc tgataaataa 480
aacatttttt aaaaaaaaaa aaaaaaaa 508

<210> 1849
<211> 549
<212> DNA
<213> Ctenocephalides felis

<400> 1849

aattattggc tgggtctntt aaactacaac aagggtaaaa aagatgatgc ggcgtactat 60
tttgcttcgg tagtgaaaaa ctatccgaag tcaccaaagg ctgcagatgc gatgtttaaa 120
gtcggcgtca tcatgcagga caaaggtgac aatgcggctg gctaagtcgc gtttttcgtg 180
ttcatacaca tacgacagca ccgcgaaacc ttgcgtgccg tgctgctggg tggcctcttg 240
cgccagggcg ctacgcagaa tgtcgcggat agcttcggaa cggttgttat aaccacgacg 300
ctggctcagg ctgtccagcg tctccagtaa atcgtcatca agcgtgatgg tgactcgttg 360
catttgcgtt aaaccttttc tgtggtgcga cggcgcacgg ngaatgcggg taataccgcg 420
ttttgtagca cacgtccggc gtcagaggaa aaggttaatt tctctccacc actgggtttn 480
gacgattgtc gtgtcataac attacccgtg gaaaagcgtt ctacagcgta gtcgtggtga 540
tgacagnan 549

<210> 1850
<211> 348
<212> DNA
<213> Ctenocephalides felis

<400> 1850

```
taagcattta tgtatgacta tgtaaaataa atgaatttat attttatcat ttttctaatt 60
caattataat gatgaagaat aaaaaaaaat tcatcttgtc aaagataact taaaaaataa 120
ttaaatataa atagatttat ctaaaagcag ttcgtcttat gagaatttat attgaaatgc 180
tatgttaact tgtatggccg tgttgtattt tacaaaataa aacttcttac ctaaagtggt 240
gttggtagaa gtgtttccaa aaccaaatgt gctagttgct ggtttgttaa atgtattatt 300
aaataaagag tttgaagtag ttgtttgacc aaatcctcca aaaccagt             348
```

<210> 1851
<211> 436
<212> DNA
<213> Ctenocephalides felis

<400> 1851

```
agcttgtcaa acttctnaat aatatttcca ttcccaataa ttataaacta attagcctag 60
acgttatatc cctatttaca aatgtcccac tagacttaat actagacgcc atcaccagca 120
gatggagtga aaattaggcc ttcacaccac attatcttta cattggttta gagaaatcat 180
caaattcatt gtctctaaca cctattttga atttaatgga tcattttatt accaaatttt 240
tggcacccca atgggatctc ccgcatctcc gtctttagta gaaatttcct tacaattagt 300
agaaaacaaa atttttaata attataacaa acaaatcttt tattaccgat atgtagacga 360
tatcattctt gtattccgga agaagacatt aacatattta ctgataattt taacaattta 420
ataaaaaact acagtt                                                 436
```

<210> 1852
<211> 147
<212> DNA
<213> Ctenocephalides felis

<400> 1852

```
ngtaagaacc ttcaatagcc tcacggggtg ttttgaatcc taatcctaca ttcctgtgaa 60
gacgtaagga tttctttttt aaaccgatct tgcggttgag gtttacaccc aactgcttct 120
ggaaggctcg ttcaagtctg atcagcc                                     147
```

<210> 1853
<211> 285
<212> DNA
<213> Ctenocephalides felis

<400> 1853

```
gtattgaatt tttctttctc attctgaaag tattctattt cggatggaga acgaatgctg 60
cttggcaaaa tcattgatta tattataaat catacgaaca aatttatctc gaatatttat 120
tatagcgtca tattgatcta ataaaagaaa aaaagcagtt caacataaaa atattggaaa 180
aagtgctatc attgcaaaat atccaagtat tgcttataat atcgcaatta tatgtataaa 240
cttcgcaagt cgagtgcaac gtttaacgta agcgcggttt gtggt                 285
```

<210> 1854
<211> 261
<212> DNA
<213> Ctenocephalides felis

<400> 1854

```
ctaaatgaaa ttgtaatttt aaatatgtaa gaattataaa aaatagaagt aatatgatat 60
attatcaaat aataaaatga ttaaaattaa agtattgatg ataagaaaat ttaataataa 120
ttaataaaaa aaaatataac tttaataaat gaatatttta tcatcaacaa attacacagg 180
aagaagttag aagtaaataa taaaaaaatc ttcagtaaac tttccaagta ttcgctcagg 240
cgtattttag taactttttg t                                          261
```

<210> 1855
<211> 332
<212> DNA
<213> Ctenocephalides felis

<400> 1855

```
atccttcagc acatttgcaa ccaatataac attctctaga acaaggtcca ggtaaatcgt 60
tcatgtagtt agcacaagtt cttggacaag aagtgccaca ggaagtaaat tcttcatttt 120
ctgggcaagt tgtgttcaca gcaggcaaag gatcaacaag gtcttctgga agaatttcac 180
ctggaagagt ttcttctgga agcgtttcat cacagtagac aaaagcagcc aacacaaggc 240
aactcaatac agtaactaat ataactttca ttttgtaaca aacttttctt cactctaaaa 300
tagatctaaa aattcaaatc gatcgatcgc ag                               332
```

<210> 1856
<211> 341
<212> DNA
<213> Ctenocephalides felis

<400> 1856

```
gagcatggtt tggacgcaga aataagtctc cacaagctat ggctgcttgt gtcagtcgtg 60
cttggtggcg ttggcagcac aaatatattc acccaagaaa agctggaatt gcaggattct 120
atcaattgac tgttggatca atgattttgt tctatgcttt gaactacggc aagatctctg 180
ctcataagaa ctacaaatac cactaaaacc aaacatctta cgatggtagt gtgtctgttg 240
atggcagaat aaatttctaa atgtaaaatc tgtatcaata tacaattcaa tatattaaat 300
tacacgagaa aaaaaaaaaa aaaaaaaaaa aaaaaaaaaa a                     341
```

<210> 1857
<211> 238
<212> DNA
<213> Ctenocephalides felis

<400> 1857

ttctcatcca atcaaatttt tctaatcttt ccataataat cggaagaacg agcattcccg 60
gtgcggccat tactattctt gataaaacaa cttgactgat tccctttgca gcagctaacc 120
gtgactttcc tataatgcgg ccttcactat ctacaacgtc tattcctgct aataattctg 180
tttgacgcat catcggaata ttaacgcaat tagcagctgc tactgcggca aaaggaac 238

<210> 1858
<211> 263
<212> DNA
<213> Ctenocephalides felis

<400> 1858

ccaaatttta ttgtgatatn ngtacacaan ntgtganann ttttgtggaa ncanaanang 60
tantggtntt gcngttaant attataagga tactaatagt gatgttgtaa gagttaggac 120

taatagatga gtcttcttca attttgataa acatattttt ataatctgtg ttaaattttg 180
cacttgtaat gctgtgcttt ttaatgaata aacatgcaat attaaacttc gaaaaaaaaa 240
aaaaaagtta attncttgct tgt 263

<210> 1859
<211> 613
<212> DNA
<213> Ctenocephalides felis

<400> 1859

agctgcccac tatagggcta aagcggccgc cngggcaggt gttggacaaa aatatttaaa 60
attaagaaga aataaatgaa atatttataa ttattaaaga atcttgtatc gtgcaaaaca 120
taaatgatat ataaattaat ttcgatctct taataaaagt attatttcaa ttaccttatt 180
tgatgagaaa atgcacaaca aaatcctggt cctggttgtt taccatgtcg tcctgatgtt 240
taccatggta gtcgccaaga ctacacatga agacaccaat gataattcta cagacgtctt 300
actggagttg cccaaatcga tgaacaatga tgaaaagttg ttcctgacaa ctggtcaaat 360
tttggaaggg actactgtat atagtgacga attggcagat ttcaatataa cagaaaatac 420
agtaaacgta acagccgaca aagtagccct aaaagaaatc acacctgacc atcaccatcc 480
agtagtgact tctacacaaa aaacaatttt aaacgcatcc acgaccgttg aaaaaaatcc 540
tggacatcaa accagtattt cagaagaatc taccacaaaa ttggtaaaaa caaccactga 600
agacaaccac ctc 613

<210> 1860
<211> 613
<212> DNA
<213> Ctenocephalides felis

<400> 1860

```
gaggtggttg tcttcagtgg ttgtttttac caattttgtg gtagattctt ctgaaatact 60
ggtttgatgt ccaggatttt tttcaacggt cgtggatgcg tttaaaattg ttttttgtgt 120
agaagtcact actggatggt gatggtcagg tgtgatttct tttagggcta ctttgtcggc 180
tgttacgttt actgtatttt ctgttatatt gaaatctgcc aattcgtcac tatatacagt 240
agtcccttcc aaaatttgac cagttgtcag gaacaacttt tcatcattgt tcatcgattt 300
gggcaactcc agtaagacgt ctgtagaatt atcattggtg tcttcatgtg tagtcttggc 360
gactaccatg gtaaacatca ggacgacatg gtaaacaacc aggaccagga ttttgttgtg 420
cattttctca tcaaataagg taattgaaat aatactttta ttaagagatc gaaattaatt 480
tatatatcat ttatgttttg cacgatacaa gattctttaa taattataaa tatttcattt 540
atttcttctt aattttaaat atttttgtcc aacacctgcc cnggcggccg ctttagccct 600
atagtgggca gct                                                    613
```

<210> 1861
<211> 2739
<212> DNA
<213> Ctenocephalides felis

<220>
<221> CDS
<222> (191)..(2206)

<400> 1861

```
agctgcccac tatagggcta aagcggccgc cngggcaggt gttggacaaa aatatttaaa 60

attaagaaga aataaatgaa atatttataa ttattaaaga atcttgtatc gtgcaaaaca 120

taaatgatat ataaattaat ttcgatctct taataaaagt attatttcaa ttaccttatt 180

tgatgagaaa atg cac aac aaa atc ctg gtc ctg gtt gtt tac cat gtc    229
           Met His Asn Lys Ile Leu Val Leu Val Val Tyr His Val
            1               5                  10

gtc ctg atg ttt acc atg gta gtc gcc aag act aca cat gaa gac acc    277
Val Leu Met Phe Thr Met Val Val Ala Lys Thr Thr His Glu Asp Thr
     15                  20                  25

aat gat aat tct aca gac gtc tta ctg gag ttg ccc aaa tcg atg aac    325
Asn Asp Asn Ser Thr Asp Val Leu Leu Glu Leu Pro Lys Ser Met Asn
 30                  35                  40                  45

aat gat gaa aag ttg ttc ctg aca act ggt caa att ttg gaa ggg act    373
Asn Asp Glu Lys Leu Phe Leu Thr Thr Gly Gln Ile Leu Glu Gly Thr
                 50                  55                  60

act gta tat agt gac gaa ttg gca gat ttc aat ata aca gaa aat aca    421
Thr Val Tyr Ser Asp Glu Leu Ala Asp Phe Asn Ile Thr Glu Asn Thr
             65                  70                  75

gta aac gta aca gcc gac aaa gta gcc cta aaa gaa atc aca cct gac    469
Val Asn Val Thr Ala Asp Lys Val Ala Leu Lys Glu Ile Thr Pro Asp
         80                  85                  90

cat cac cat cca gta gtg act tct aca caa aaa aca att tta aac gca    517
His His His Pro Val Val Thr Ser Thr Gln Lys Thr Ile Leu Asn Ala
     95                 100                 105

tcc acg acc gtt gaa aaa aat cct gga cat caa acc agt att tca gaa    565
Ser Thr Thr Val Glu Lys Asn Pro Gly His Gln Thr Ser Ile Ser Glu
110                 115                 120                 125

gaa tct acc aca aaa ttg gta aaa aca acc act gaa gac aac cac ctc    613
Glu Ser Thr Thr Lys Leu Val Lys Thr Thr Thr Glu Asp Asn His Leu
                130                 135                 140

ggt gta aag agc ctg aat gaa cct ggt gat gaa caa gaa tta aaa aaa    661
```

```
Gly Val Lys Ser Leu Asn Glu Pro Gly Asp Glu Gln Glu Leu Lys Lys
                145                 150                 155

cca tca tca cat ggt aag gag cat att tct tta cca gtg gct tca cca      709
Pro Ser Ser His Gly Lys Glu His Ile Ser Leu Pro Val Ala Ser Pro
            160                 165                 170

gta cca cca gta tcg cat atc ttc cag gct aca cca gga gac ctt tgt      757
Val Pro Pro Val Ser His Ile Phe Gln Ala Thr Pro Gly Asp Leu Cys
        175                 180                 185

cca gcc ttc gac gat gca gat cgc ttc acc cag aca gaa ctt ttg tcc      805
Pro Ala Phe Asp Asp Ala Asp Arg Phe Thr Gln Thr Glu Leu Leu Ser
190                 195                 200                 205

agg ctg aca aac gat tgc agg tac gat aag ctg gag cgc cct ttg ggg      853
Arg Leu Thr Asn Asp Cys Arg Tyr Asp Lys Leu Glu Arg Pro Leu Gly
                    210                 215                 220

cct cac aat ggt gca ggg ccg ctc ccg gtg gcc gcc aga att tac gtg      901
Pro His Asn Gly Ala Gly Pro Leu Pro Val Ala Ala Arg Ile Tyr Val
                225                 230                 235

tat ttt ata caa aat acg gac gcg cac gaa ttg tca ttt tcc gtg acc      949
Tyr Phe Ile Gln Asn Thr Asp Ala His Glu Leu Ser Phe Ser Val Thr
            240                 245                 250

gtc ctc ctc caa ttt cgt tac cag gac gcc aga ttg gcc tac aaa aaa      997
Val Leu Leu Gln Phe Arg Tyr Gln Asp Ala Arg Leu Ala Tyr Lys Lys
        255                 260                 265

gtg gca ccc acc agg acg gtc atc atg ggc gaa tcg cag ctc agg gac      1045
Val Ala Pro Thr Arg Thr Val Ile Met Gly Glu Ser Gln Leu Arg Asp
270                 275                 280                 285

aaa atc tgg gta cca cat gta ttc gtt gcc aac gag aga tct tcc cag      1093
Lys Ile Trp Val Pro His Val Phe Val Ala Asn Glu Arg Ser Ser Gln
                    290                 295                 300

gtt atg ggc aca gat gcc caa tct aag gac atg ttg gtg tca gta gct      1141
Val Met Gly Thr Asp Ala Gln Ser Lys Asp Met Leu Val Ser Val Ala
                305                 310                 315

cct gat ggt aca gtc gtc ttt tcg gtc agg atg aag gca act ttg tac      1189
Pro Asp Gly Thr Val Val Phe Ser Val Arg Met Lys Ala Thr Leu Tyr
            320                 325                 330

tgt tgg atg aat tta agg aaa ttt cct ttt gat gaa caa cag tgt cag      1237
```

Cys Trp Met Asn Leu Arg Lys Phe Pro Phe Asp Glu Gln Gln Cys Gln
335 340 345

atg atg ttg gaa agt tgg aag tac aat aca agt gaa ctc cta ttg act 1285
Met Met Leu Glu Ser Trp Lys Tyr Asn Thr Ser Glu Leu Leu Leu Thr
350 355 360 365

tgg gaa cca act gca cca gta act tta gca cca gaa cta cat ttg acc 1333
Trp Glu Pro Thr Ala Pro Val Thr Leu Ala Pro Glu Leu His Leu Thr
370 375 380

gaa tat gtc ctt act gac atg tgg gta aat gaa aca gtt gtc aag gct 1381
Glu Tyr Val Leu Thr Asp Met Trp Val Asn Glu Thr Val Val Lys Ala
385 390 395

gat ttg gat gac ctg aga cac gga gca ttt ggt ggg aca tac agt gcc 1429
Asp Leu Asp Asp Leu Arg His Gly Ala Phe Gly Gly Thr Tyr Ser Ala
400 405 410

tta agt ttc acg att caa ata agt cgt gaa atg ggt tac tat tta atg 1477
Leu Ser Phe Thr Ile Gln Ile Ser Arg Glu Met Gly Tyr Tyr Leu Met
415 420 425

gat tac ttt ttg cca tca gta atg atc gtg tcg tgt tcc tgg gta agt 1525
Asp Tyr Phe Leu Pro Ser Val Met Ile Val Ser Cys Ser Trp Val Ser
430 435 440 445

ttt tgg ctg gca gca gac caa tca gca ccc aga gtc acc tta ggt aca 1573
Phe Trp Leu Ala Ala Asp Gln Ser Ala Pro Arg Val Thr Leu Gly Thr
450 455 460

agc acc atg tta tca ttt atc act tta gca agt gcc caa gga aaa act 1621
Ser Thr Met Leu Ser Phe Ile Thr Leu Ala Ser Ala Gln Gly Lys Thr
465 470 475

tta ccc aaa gta tcg tac atc aaa gct tca gaa atc tgg ttt tta ggt 1669
Leu Pro Lys Val Ser Tyr Ile Lys Ala Ser Glu Ile Trp Phe Leu Gly
480 485 490

tgc acc ggg ttt att ttt ggg agt tta gtg gaa ttc gcg ttt gtc aac 1717
Cys Thr Gly Phe Ile Phe Gly Ser Leu Val Glu Phe Ala Phe Val Asn
495 500 505

aca att tgg aga cga agg aaa aat gtg gaa ttg aaa aaa gtc aac agc 1765
Thr Ile Trp Arg Arg Arg Lys Asn Val Glu Leu Lys Lys Val Asn Ser
510 515 520 525

aag tat att ttg aag tca act ttg acg ccg agg ttg gcc cgg aag gag 1813

```
Lys Tyr Ile Leu Lys Ser Thr Leu Thr Pro Arg Leu Ala Arg Lys Glu
                530                 535                 540

ttt cat gct tcg ttt aat tcg aat cct gga ggt ggt aat aag gat gat    1861
Phe His Ala Ser Phe Asn Ser Asn Pro Gly Gly Gly Asn Lys Asp Asp
            545                 550                 555

cag gat ttg gga aga ggg att agg gtc ttt ccg ccg cct ttg gtc aag    1909
Gln Asp Leu Gly Arg Gly Ile Arg Val Phe Pro Pro Pro Leu Val Lys
        560                 565                 570

gct agg tct tgt tcc agt ctg gat agg agt aat gga tcc ggg aat ttt    1957
Ala Arg Ser Cys Ser Ser Leu Asp Arg Ser Asn Gly Ser Gly Asn Phe
    575                 580                 585

ttg agc gtc cat gga aat gat cac aaa gtt cca aca ata aca gca caa    2005
Leu Ser Val His Gly Asn Asp His Lys Val Pro Thr Ile Thr Ala Gln
590                 595                 600                 605

tgt gca gac gat gcc gca agt gac cag att tca gtt tgt gtc gat ggg    2053
Cys Ala Asp Asp Ala Ala Ser Asp Gln Ile Ser Val Cys Val Asp Gly
                610                 615                 620

gaa aac gaa gaa cct gca caa att gtt cac cac acc tgg acg acg atg    2101
Glu Asn Glu Glu Pro Ala Gln Ile Val His His Thr Trp Thr Thr Met
            625                 630                 635

aca cct caa gaa att tcc atg tgg att gac aaa agg tcc aga att tgt    2149
Thr Pro Gln Glu Ile Ser Met Trp Ile Asp Lys Arg Ser Arg Ile Cys
        640                 645                 650

ttc ccg ata gct ttt gct ata ttt aac ttt ttt tat tgg ata ttt gtt    2197
Phe Pro Ile Ala Phe Ala Ile Phe Asn Phe Phe Tyr Trp Ile Phe Val
    655                 660                 665

tat tat tta taaacacact taatatactt atagttttaa taattaataa            2246
Tyr Tyr Leu
670

atttataaaa taattaaaaa taaatatatg taaaatttaa aggaaacgtg aatagaatca 2306

aaagagattc ttattggatt attccattat taataggatt cttactagac aatattaatg 2366

attttatatt atatatcact tataactttt gaacggtttg ttaaaaatga atacaatatt 2426

tgacaaattt atataaaatt aaacaattta taatattgtc gaacatctta ccaccctaca 2486

gcgactcagt atactcgaaa atcgctattg aaatatctta cacaatttag tcattcctat 2546
```

ttcacatata atagttaata attaaaattg aaattttaaa ttaaaaaata atgatactgg 2606

aaattttaat tttaattatt aattattata tgaataatta attttactgc atagttataa 2666

ttataattat aaatattaaa tttttagaat aaatactcag ctggtctgaa aaaaaaaaaa 2726

aaaaaaaaaa aaa 2739

<210> 1862
<211> 672
<212> PRT
<213> Ctenocephalides felis

<400> 1862

Met His Asn Lys Ile Leu Val Leu Val Val Tyr His Val Val Leu Met
1 5 10 15

Phe Thr Met Val Val Ala Lys Thr Thr His Glu Asp Thr Asn Asp Asn
20 25 30

Ser Thr Asp Val Leu Leu Glu Leu Pro Lys Ser Met Asn Asn Asp Glu
35 40 45

Lys Leu Phe Leu Thr Thr Gly Gln Ile Leu Glu Gly Thr Thr Val Tyr
50 55 60

Ser Asp Glu Leu Ala Asp Phe Asn Ile Thr Glu Asn Thr Val Asn Val
65 70 75 80

Thr Ala Asp Lys Val Ala Leu Lys Glu Ile Thr Pro Asp His His His
85 90 95

Pro Val Val Thr Ser Thr Gln Lys Thr Ile Leu Asn Ala Ser Thr Thr
100 105 110

Val Glu Lys Asn Pro Gly His Gln Thr Ser Ile Ser Glu Glu Ser Thr
115 120 125

Thr Lys Leu Val Lys Thr Thr Thr Glu Asp Asn His Leu Gly Val Lys
130 135 140

Ser Leu Asn Glu Pro Gly Asp Glu Gln Glu Leu Lys Lys Pro Ser Ser
145 150 155 160

His Gly Lys Glu His Ile Ser Leu Pro Val Ala Ser Pro Val Pro Pro
165 170 175

```
Val Ser His Ile Phe Gln Ala Thr Pro Gly Asp Leu Cys Pro Ala Phe
            180                 185                 190

Asp Asp Ala Asp Arg Phe Thr Gln Thr Glu Leu Leu Ser Arg Leu Thr
        195                 200                 205

Asn Asp Cys Arg Tyr Asp Lys Leu Glu Arg Pro Leu Gly Pro His Asn
    210                 215                 220

Gly Ala Gly Pro Leu Pro Val Ala Ala Arg Ile Tyr Val Tyr Phe Ile
225                 230                 235                     240

Gln Asn Thr Asp Ala His Glu Leu Ser Phe Ser Val Thr Val Leu Leu
                245                 250                 255

Gln Phe Arg Tyr Gln Asp Ala Arg Leu Ala Tyr Lys Lys Val Ala Pro
            260                 265                 270

Thr Arg Thr Val Ile Met Gly Glu Ser Gln Leu Arg Asp Lys Ile Trp
        275                 280                 285

Val Pro His Val Phe Val Ala Asn Glu Arg Ser Ser Gln Val Met Gly
    290                 295                 300

Thr Asp Ala Gln Ser Lys Asp Met Leu Val Ser Val Ala Pro Asp Gly
305                 310                 315                     320

Thr Val Val Phe Ser Val Arg Met Lys Ala Thr Leu Tyr Cys Trp Met
                325                 330                 335

Asn Leu Arg Lys Phe Pro Phe Asp Glu Gln Gln Cys Gln Met Met Leu
            340                 345                 350

Glu Ser Trp Lys Tyr Asn Thr Ser Glu Leu Leu Leu Thr Trp Glu Pro
        355                 360                 365

Thr Ala Pro Val Thr Leu Ala Pro Glu Leu His Leu Thr Glu Tyr Val
    370                 375                 380

Leu Thr Asp Met Trp Val Asn Glu Thr Val Val Lys Ala Asp Leu Asp
385                 390                 395                     400

Asp Leu Arg His Gly Ala Phe Gly Gly Thr Tyr Ser Ala Leu Ser Phe
                405                 410                 415

Thr Ile Gln Ile Ser Arg Glu Met Gly Tyr Tyr Leu Met Asp Tyr Phe
            420                 425                 430
```

Leu Pro Ser Val Met Ile Val Ser Cys Ser Trp Val Ser Phe Trp Leu
435 440 445

Ala Ala Asp Gln Ser Ala Pro Arg Val Thr Leu Gly Thr Ser Thr Met
450 455 460

Leu Ser Phe Ile Thr Leu Ala Ser Ala Gln Gly Lys Thr Leu Pro Lys
465 470 475 480

Val Ser Tyr Ile Lys Ala Ser Glu Ile Trp Phe Leu Gly Cys Thr Gly
485 490 495

Phe Ile Phe Gly Ser Leu Val Glu Phe Ala Phe Val Asn Thr Ile Trp
500 505 510

Arg Arg Arg Lys Asn Val Glu Leu Lys Lys Val Asn Ser Lys Tyr Ile
515 520 525

Leu Lys Ser Thr Leu Thr Pro Arg Leu Ala Arg Lys Glu Phe His Ala
530 535 540

Ser Phe Asn Ser Asn Pro Gly Gly Gly Asn Lys Asp Asp Gln Asp Leu
545 550 555 560

Gly Arg Gly Ile Arg Val Phe Pro Pro Pro Leu Val Lys Ala Arg Ser
565 570 575

Cys Ser Ser Leu Asp Arg Ser Asn Gly Ser Gly Asn Phe Leu Ser Val
580 585 590

His Gly Asn Asp His Lys Val Pro Thr Ile Thr Ala Gln Cys Ala Asp
595 600 605

Asp Ala Ala Ser Asp Gln Ile Ser Val Cys Val Asp Gly Glu Asn Glu
610 615 620

Glu Pro Ala Gln Ile Val His His Thr Trp Thr Thr Met Thr Pro Gln
625 630 635 640

Glu Ile Ser Met Trp Ile Asp Lys Arg Ser Arg Ile Cys Phe Pro Ile
645 650 655

Ala Phe Ala Ile Phe Asn Phe Phe Tyr Trp Ile Phe Val Tyr Tyr Leu
660 665 670

<210> 1863
<211> 2739

<212> DNA
<213> Ctenocephalides felis

<400> 1863

```
tttttttttt tttttttttt tttttcagac cagctgagta tttattctaa aaatttaata 60
tttataatta taattataac tatgcagtaa aattaattat tcatataata attaataatt 120
aaaattaaaa tttccagtat cattattttt taatttaaaa tttcaatttt aattattaac 180
tattatatgt gaaataggaa tgactaaatt gtgtaagata tttcaatagc gattttcgag 240
tatactgagt cgctgtaggg tggtaagatg ttcgacaata ttataaattg tttaatttta 300
tataaatttg tcaaatattg tattcatttt taacaaaccg ttcaaaagtt ataagtgata 360
tataatataa aatcattaat attgtctagt aagaatccta ttaataatgg aataatccaa 420
taagaatctc ttttgattct attcacgttt cctttaaatt ttacatatat ttatttttaa 480
ttattttata aatttattaa ttattaaaac tataagtata ttaagtgtgt ttataaataa 540
taaacaaata tccaataaaa aaagttaaat atagcaaaag ctatcgggaa acaaattctg 600
gaccttttgt caatccacat ggaaatttct tgaggtgtca tcgtcgtcca ggtgtggtga 660
acaatttgtg caggttcttc gttttcccca tcgacacaaa ctgaaatctg gtcacttgcg 720
gcatcgtctg cacattgtgc tgttattgtt ggaactttgt gatcatttcc atggacgctc 780
aaaaaattcc cggatccatt actcctatcc agactggaac aagacctagc cttgaccaaa 840
ggcggcggaa agaccctaat ccctcttccc aaatcctgat catccttatt accacctcca 900
ggattcgaat taaacgaagc atgaaactcc ttccgggcca acctcggcgt caaagttgac 960
ttcaaaatat acttgctgtt gacttttttc aattccacat ttttccttcg tctccaaatt 1020
gtgttgacaa acgcgaattc cactaaactc ccaaaaataa acccggtgca acctaaaaac 1080
cagatttctg aagctttgat gtacgatact ttgggtaaag tttttccttg ggcacttgct 1140
aaagtgataa atgataacat ggtgcttgta cctaaggtga ctctgggtgc tgattggtct 1200
gctgccagcc aaaaacttac ccaggaacac gacacgatca ttactgatgg caaaaagtaa 1260
tccattaaat agtaacccat ttcacgactt atttgaatcg tgaaacttaa ggcactgtat 1320
gtcccaccaa atgctccgtg tctcaggtca tccaaatcag ccttgacaac tgtttcattt 1380
acccacatgt cagtaaggac atattcggtc aaatgtagtt ctggtgctaa agttactggt 1440
gcagttggtt cccaagtcaa taggagttca cttgtattgt acttccaact ttccaacatc 1500
atctgacact gttgttcatc aaaaggaaat ttccttaaat tcatccaaca gtacaaagtt 1560
gccttcatcc tgaccgaaaa gacgactgta ccatcaggag ctactgacac caacatgtcc 1620
ttagattggg catctgtgcc cataacctgg gaagatctct cgttggcaac gaatacatgt 1680
ggtacccaga ttttgtccct gagctgcgat tcgcccatga tgaccgtcct ggtgggtgcc 1740
actttttgt aggccaatct ggcgtcctgg taacgaaatt ggaggaggac ggtcacggaa 1800
aatgacaatt cgtgcgcgtc cgtattttgt ataaaataca cgtaaattct ggcggccacc 1860
gggagcggcc ctgcaccatt gtgaggcccc aaagggcgct ccagcttatc gtacctgcaa 1920
tcgtttgtca gcctggacaa aagttctgtc tgggtgaagc gatctgcatc gtcgaaggct 1980
ggacaaaggt ctcctggtgt agcctggaag atatgcgata ctggtggtac tggtgaagcc 2040
actggtaaag aaatatgctc cttaccatgt gatgatggtt tttttaattc ttgttcatca 2100
ccaggttcat tcaggctctt tacaccgagg tggttgtctt cagtggttgt ttttaccaat 2160
tttgtggtag attcttctga aatactggtt tgatgtccag gattttttc aacggtcgtg 2220
gatgcgttta aaattgtttt ttgtgtagaa gtcactactg gatggtgatg gtcaggtgtg 2280
atttctttta gggctacttt gtcggctgtt acgtttactg tattttctgt tatattgaaa 2340
tctgccaatt cgtcactata tacagtagtc ccttccaaaa tttgaccagt tgtcaggaac 2400
aacttttcat cattgttcat cgatttgggc aactccagta agacgtctgt agaattatca 2460
ttggtgtctt catgtgtagt cttggcgact accatggtaa acatcaggac gacatggtaa 2520
```

```
acaaccagga ccaggatttt gttgtgcatt ttctcatcaa ataaggtaat tgaaataata 2580
cttttattaa gagatcgaaa ttaatttata tatcatttat gttttgcacg atacaagatt 2640
ctttaataat tataaatatt tcatttattt cttcttaatt ttaaatattt ttgtccaaca 2700
cctgcccngg cggccgcttt agccctatag tgggcagct                        2739
```

<210> 1864
<211> 2016
<212> DNA
<213> Ctenocephalides felis

<220>
<221> CDS
<222> (1)..(2016)

<400> 1864

```
atg cac aac aaa atc ctg gtc ctg gtt gtt tac cat gtc gtc ctg atg   48
Met His Asn Lys Ile Leu Val Leu Val Val Tyr His Val Val Leu Met
 1               5                  10                  15

ttt acc atg gta gtc gcc aag act aca cat gaa gac acc aat gat aat   96
Phe Thr Met Val Val Ala Lys Thr Thr His Glu Asp Thr Asn Asp Asn
            20                  25                  30

tct aca gac gtc tta ctg gag ttg ccc aaa tcg atg aac aat gat gaa   144
Ser Thr Asp Val Leu Leu Glu Leu Pro Lys Ser Met Asn Asn Asp Glu
        35                  40                  45

aag ttg ttc ctg aca act ggt caa att ttg gaa ggg act act gta tat   192
Lys Leu Phe Leu Thr Thr Gly Gln Ile Leu Glu Gly Thr Thr Val Tyr
    50                  55                  60

agt gac gaa ttg gca gat ttc aat ata aca gaa aat aca gta aac gta   240
Ser Asp Glu Leu Ala Asp Phe Asn Ile Thr Glu Asn Thr Val Asn Val
65                  70                  75                  80

aca gcc gac aaa gta gcc cta aaa gaa atc aca cct gac cat cac cat   288
Thr Ala Asp Lys Val Ala Leu Lys Glu Ile Thr Pro Asp His His His
                85                  90                  95

cca gta gtg act tct aca caa aaa aca att tta aac gca tcc acg acc   336
Pro Val Val Thr Ser Thr Gln Lys Thr Ile Leu Asn Ala Ser Thr Thr
            100                 105                 110

gtt gaa aaa aat cct gga cat caa acc agt att tca gaa gaa tct acc   384
Val Glu Lys Asn Pro Gly His Gln Thr Ser Ile Ser Glu Glu Ser Thr
        115                 120                 125
```

```
aca aaa ttg gta aaa aca acc act gaa gac aac cac ctc ggt gta aag     432
Thr Lys Leu Val Lys Thr Thr Thr Glu Asp Asn His Leu Gly Val Lys
    130                 135                 140

agc ctg aat gaa cct ggt gat gaa caa gaa tta aaa aaa cca tca tca     480
Ser Leu Asn Glu Pro Gly Asp Glu Gln Glu Leu Lys Lys Pro Ser Ser
145                 150                 155                 160

cat ggt aag gag cat att tct tta cca gtg gct tca cca gta cca cca     528
His Gly Lys Glu His Ile Ser Leu Pro Val Ala Ser Pro Val Pro Pro
                165                 170                 175

gta tcg cat atc ttc cag gct aca cca gga gac ctt tgt cca gcc ttc     576
Val Ser His Ile Phe Gln Ala Thr Pro Gly Asp Leu Cys Pro Ala Phe
            180                 185                 190

gac gat gca gat cgc ttc acc cag aca gaa ctt ttg tcc agg ctg aca     624
Asp Asp Ala Asp Arg Phe Thr Gln Thr Glu Leu Leu Ser Arg Leu Thr
        195                 200                 205

aac gat tgc agg tac gat aag ctg gag cgc cct ttg ggg cct cac aat     672
Asn Asp Cys Arg Tyr Asp Lys Leu Glu Arg Pro Leu Gly Pro His Asn
    210                 215                 220

ggt gca ggg ccg ctc ccg gtg gcc gcc aga att tac gtg tat ttt ata     720
Gly Ala Gly Pro Leu Pro Val Ala Ala Arg Ile Tyr Val Tyr Phe Ile
225                 230                 235                 240

caa aat acg gac gcg cac gaa ttg tca ttt tcc gtg acc gtc ctc ctc     768
Gln Asn Thr Asp Ala His Glu Leu Ser Phe Ser Val Thr Val Leu Leu
                245                 250                 255

caa ttt cgt tac cag gac gcc aga ttg gcc tac aaa aaa gtg gca ccc     816
Gln Phe Arg Tyr Gln Asp Ala Arg Leu Ala Tyr Lys Lys Val Ala Pro
            260                 265                 270

acc agg acg gtc atc atg ggc gaa tcg cag ctc agg gac aaa atc tgg     864
Thr Arg Thr Val Ile Met Gly Glu Ser Gln Leu Arg Asp Lys Ile Trp
        275                 280                 285

gta cca cat gta ttc gtt gcc aac gag aga tct tcc cag gtt atg ggc     912
Val Pro His Val Phe Val Ala Asn Glu Arg Ser Ser Gln Val Met Gly
    290                 295                 300

aca gat gcc caa tct aag gac atg ttg gtg tca gta gct cct gat ggt     960
Thr Asp Ala Gln Ser Lys Asp Met Leu Val Ser Val Ala Pro Asp Gly
305                 310                 315                 320
```

```
aca gtc gtc ttt tcg gtc agg atg aag gca act ttg tac tgt tgg atg    1008
Thr Val Val Phe Ser Val Arg Met Lys Ala Thr Leu Tyr Cys Trp Met
                325                 330                 335

aat tta agg aaa ttt cct ttt gat gaa caa cag tgt cag atg atg ttg    1056
Asn Leu Arg Lys Phe Pro Phe Asp Glu Gln Gln Cys Gln Met Met Leu
            340                 345                 350

gaa agt tgg aag tac aat aca agt gaa ctc cta ttg act tgg gaa cca    1104
Glu Ser Trp Lys Tyr Asn Thr Ser Glu Leu Leu Leu Thr Trp Glu Pro
        355                 360                 365

act gca cca gta act tta gca cca gaa cta cat ttg acc gaa tat gtc    1152
Thr Ala Pro Val Thr Leu Ala Pro Glu Leu His Leu Thr Glu Tyr Val
    370                 375                 380

ctt act gac atg tgg gta aat gaa aca gtt gtc aag gct gat ttg gat    1200
Leu Thr Asp Met Trp Val Asn Glu Thr Val Val Lys Ala Asp Leu Asp
385                 390                 395                 400

gac ctg aga cac gga gca ttt ggt ggg aca tac agt gcc tta agt ttc    1248
Asp Leu Arg His Gly Ala Phe Gly Gly Thr Tyr Ser Ala Leu Ser Phe
                405                 410                 415

acg att caa ata agt cgt gaa atg ggt tac tat tta atg gat tac ttt    1296
Thr Ile Gln Ile Ser Arg Glu Met Gly Tyr Tyr Leu Met Asp Tyr Phe
            420                 425                 430

ttg cca tca gta atg atc gtg tcg tgt tcc tgg gta agt ttt tgg ctg    1344
Leu Pro Ser Val Met Ile Val Ser Cys Ser Trp Val Ser Phe Trp Leu
        435                 440                 445

gca gca gac caa tca gca ccc aga gtc acc tta ggt aca agc acc atg    1392
Ala Ala Asp Gln Ser Ala Pro Arg Val Thr Leu Gly Thr Ser Thr Met
    450                 455                 460

tta tca ttt atc act tta gca agt gcc caa gga aaa act tta ccc aaa    1440
Leu Ser Phe Ile Thr Leu Ala Ser Ala Gln Gly Lys Thr Leu Pro Lys
465                 470                 475                 480

gta tcg tac atc aaa gct tca gaa atc tgg ttt tta ggt tgc acc ggg    1488
Val Ser Tyr Ile Lys Ala Ser Glu Ile Trp Phe Leu Gly Cys Thr Gly
                485                 490                 495

ttt att ttt ggg agt tta gtg gaa ttc gcg ttt gtc aac aca att tgg    1536
Phe Ile Phe Gly Ser Leu Val Glu Phe Ala Phe Val Asn Thr Ile Trp
            500                 505                 510
```

```
aga cga agg aaa aat gtg gaa ttg aaa aaa gtc aac agc aag tat att    1584
Arg Arg Arg Lys Asn Val Glu Leu Lys Lys Val Asn Ser Lys Tyr Ile
        515                 520                 525

ttg aag tca act ttg acg ccg agg ttg gcc cgg aag gag ttt cat gct    1632
Leu Lys Ser Thr Leu Thr Pro Arg Leu Ala Arg Lys Glu Phe His Ala
    530                 535                 540

tcg ttt aat tcg aat cct gga ggt ggt aat aag gat gat cag gat ttg    1680
Ser Phe Asn Ser Asn Pro Gly Gly Gly Asn Lys Asp Asp Gln Asp Leu
545                 550                 555                 560

gga aga ggg att agg gtc ttt ccg ccg cct ttg gtc aag gct agg tct    1728
Gly Arg Gly Ile Arg Val Phe Pro Pro Pro Leu Val Lys Ala Arg Ser
                565                 570                 575

tgt tcc agt ctg gat agg agt aat gga tcc ggg aat ttt ttg agc gtc    1776
Cys Ser Ser Leu Asp Arg Ser Asn Gly Ser Gly Asn Phe Leu Ser Val
            580                 585                 590

cat gga aat gat cac aaa gtt cca aca ata aca gca caa tgt gca gac    1824
His Gly Asn Asp His Lys Val Pro Thr Ile Thr Ala Gln Cys Ala Asp
        595                 600                 605

gat gcc gca agt gac cag att tca gtt tgt gtc gat ggg gaa aac gaa    1872
Asp Ala Ala Ser Asp Gln Ile Ser Val Cys Val Asp Gly Glu Asn Glu
    610                 615                 620

gaa cct gca caa att gtt cac cac acc tgg acg acg atg aca cct caa    1920
Glu Pro Ala Gln Ile Val His His Thr Trp Thr Thr Met Thr Pro Gln
625                 630                 635                 640

gaa att tcc atg tgg att gac aaa agg tcc aga att tgt ttc ccg ata    1968
Glu Ile Ser Met Trp Ile Asp Lys Arg Ser Arg Ile Cys Phe Pro Ile
                645                 650                 655

gct ttt gct ata ttt aac ttt ttt tat tgg ata ttt gtt tat tat tta    2016
Ala Phe Ala Ile Phe Asn Phe Phe Tyr Trp Ile Phe Val Tyr Tyr Leu
            660                 665                 670
```

<210> 1865
<211> 672
<212> PRT
<213> Ctenocephalides felis

<400> 1865

```
Met His Asn Lys Ile Leu Val Leu Val Val Tyr His Val Val Leu Met
```

1 5 10 15

Phe Thr Met Val Val Ala Lys Thr Thr His Glu Asp Thr Asn Asp Asn
20 25 30

Ser Thr Asp Val Leu Leu Glu Leu Pro Lys Ser Met Asn Asn Asp Glu
35 40 45

Lys Leu Phe Leu Thr Thr Gly Gln Ile Leu Glu Gly Thr Thr Val Tyr
50 55 60

Ser Asp Glu Leu Ala Asp Phe Asn Ile Thr Glu Asn Thr Val Asn Val
65 70 75 80

Thr Ala Asp Lys Val Ala Leu Lys Glu Ile Thr Pro Asp His His His
85 90 95

Pro Val Val Thr Ser Thr Gln Lys Thr Ile Leu Asn Ala Ser Thr Thr
100 105 110

Val Glu Lys Asn Pro Gly His Gln Thr Ser Ile Ser Glu Glu Ser Thr
115 120 125

Thr Lys Leu Val Lys Thr Thr Thr Glu Asp Asn His Leu Gly Val Lys
130 135 140

Ser Leu Asn Glu Pro Gly Asp Glu Gln Glu Leu Lys Lys Pro Ser Ser
145 150 155 160

His Gly Lys Glu His Ile Ser Leu Pro Val Ala Ser Pro Val Pro Pro
165 170 175

Val Ser His Ile Phe Gln Ala Thr Pro Gly Asp Leu Cys Pro Ala Phe
180 185 190

Asp Asp Ala Asp Arg Phe Thr Gln Thr Glu Leu Leu Ser Arg Leu Thr
195 200 205

Asn Asp Cys Arg Tyr Asp Lys Leu Glu Arg Pro Leu Gly Pro His Asn
210 215 220

Gly Ala Gly Pro Leu Pro Val Ala Ala Arg Ile Tyr Val Tyr Phe Ile
225 230 235 240

Gln Asn Thr Asp Ala His Glu Leu Ser Phe Ser Val Thr Val Leu Leu
245 250 255

Gln Phe Arg Tyr Gln Asp Ala Arg Leu Ala Tyr Lys Lys Val Ala Pro

```
                260                 265                 270

Thr Arg Thr Val Ile Met Gly Glu Ser Gln Leu Arg Asp Lys Ile Trp
        275                 280                 285

Val Pro His Val Phe Val Ala Asn Glu Arg Ser Ser Gln Val Met Gly
    290                 295                 300

Thr Asp Ala Gln Ser Lys Asp Met Leu Val Ser Val Ala Pro Asp Gly
305                 310                 315                 320

Thr Val Val Phe Ser Val Arg Met Lys Ala Thr Leu Tyr Cys Trp Met
                325                 330                 335

Asn Leu Arg Lys Phe Pro Phe Asp Glu Gln Gln Cys Gln Met Met Leu
            340                 345                 350

Glu Ser Trp Lys Tyr Asn Thr Ser Glu Leu Leu Leu Thr Trp Glu Pro
        355                 360                 365

Thr Ala Pro Val Thr Leu Ala Pro Glu Leu His Leu Thr Glu Tyr Val
    370                 375                 380

Leu Thr Asp Met Trp Val Asn Glu Thr Val Val Lys Ala Asp Leu Asp
385                 390                 395                 400

Asp Leu Arg His Gly Ala Phe Gly Gly Thr Tyr Ser Ala Leu Ser Phe
                405                 410                 415

Thr Ile Gln Ile Ser Arg Glu Met Gly Tyr Tyr Leu Met Asp Tyr Phe
            420                 425                 430

Leu Pro Ser Val Met Ile Val Ser Cys Ser Trp Val Ser Phe Trp Leu
        435                 440                 445

Ala Ala Asp Gln Ser Ala Pro Arg Val Thr Leu Gly Thr Ser Thr Met
    450                 455                 460

Leu Ser Phe Ile Thr Leu Ala Ser Ala Gln Gly Lys Thr Leu Pro Lys
465                 470                 475                 480

Val Ser Tyr Ile Lys Ala Ser Glu Ile Trp Phe Leu Gly Cys Thr Gly
                485                 490                 495

Phe Ile Phe Gly Ser Leu Val Glu Phe Ala Phe Val Asn Thr Ile Trp
            500                 505                 510

Arg Arg Arg Lys Asn Val Glu Leu Lys Lys Val Asn Ser Lys Tyr Ile
```

515 520 525

Leu Lys Ser Thr Leu Thr Pro Arg Leu Ala Arg Lys Glu Phe His Ala
530 535 540

Ser Phe Asn Ser Asn Pro Gly Gly Gly Asn Lys Asp Asp Gln Asp Leu
545 550 555 560

Gly Arg Gly Ile Arg Val Phe Pro Pro Pro Leu Val Lys Ala Arg Ser
565 570 575

Cys Ser Ser Leu Asp Arg Ser Asn Gly Ser Gly Asn Phe Leu Ser Val
580 585 590

His Gly Asn Asp His Lys Val Pro Thr Ile Thr Ala Gln Cys Ala Asp
595 600 605

Asp Ala Ala Ser Asp Gln Ile Ser Val Cys Val Asp Gly Glu Asn Glu
610 615 620

Glu Pro Ala Gln Ile Val His His Thr Trp Thr Thr Met Thr Pro Gln
625 630 635 640

Glu Ile Ser Met Trp Ile Asp Lys Arg Ser Arg Ile Cys Phe Pro Ile
645 650 655

Ala Phe Ala Ile Phe Asn Phe Phe Tyr Trp Ile Phe Val Tyr Tyr Leu
660 665 670

<210> 1866
<211> 2016
<212> DNA
<213> Ctenocephalides felis

<400> 1866

atgcacaaca aaatcctggt cctggttgtt taccatgtcg tcctgatgtt taccatggta 60
gtcgccaaga ctacacatga agacaccaat gataattcta cagacgtctt actggagttg 120
cccaaatcga tgaacaatga tgaaaagttg ttcctgacaa ctggtcaaat tttggaaggg 180
actactgtat atagtgacga attggcagat ttcaatataa cagaaaatac agtaaacgta 240
acagccgaca aagtagccct aaaagaaatc acacctgacc atcaccatcc agtagtgact 300
tctacacaaa aaacaatttt aaacgcatcc acgaccgttg aaaaaaatcc tggacatcaa 360
accagtattt cagaagaatc taccacaaaa ttggtaaaaa caaccactga agacaaccac 420
ctcggtgtaa agagcctgaa tgaacctggt gatgaacaag aattaaaaaa accatcatca 480
catggtaagg agcatatttc tttaccagtg gcttcaccag taccaccagt atcgcatatc 540
ttccaggcta caccaggaga cctttgtcca gccttcgacg atgcagatcg cttcacccag 600
acagaacttt tgtccaggct gacaaacgat tgcaggtacg ataagctgga gcgccctttg 660

```
gggcctcaca atggtgcagg gccgctcccg gtggccgcca gaatttacgt gtattttata 720
caaaatacgg acgcgcacga attgtcattt tccgtgaccg tcctcctcca atttcgttac 780
caggacgcca gattggccta caaaaaagtg gcacccacca ggacggtcat catgggcgaa 840
tcgcagctca gggacaaaat ctgggtacca catgtattcg ttgccaacga gagatcttcc 900
caggttatgg gcacagatgc ccaatctaag gacatgttgg tgtcagtagc tcctgatggt 960
acagtcgtct tttcggtcag gatgaaggca actttgtact gttggatgaa tttaaggaaa 1020
tttccttttg atgaacaaca gtgtcagatg atgttggaaa gttggaagta caatacaagt 1080
gaactcctat tgacttggga accaactgca ccagtaactt tagcaccaga actacatttg 1140
accgaatatg tccttactga catgtgggta aatgaaacag ttgtcaaggc tgatttggat 1200
gacctgagac acggagcatt tggtgggaca tacagtgcct taagtttcac gattcaaata 1260
agtcgtgaaa tgggttacta tttaatggat tactttttgc catcagtaat gatcgtgtcg 1320
tgttcctggg taagtttttg gctggcagca gaccaatcag cacccagagt caccttaggt 1380
acaagcacca tgttatcatt tatcacttta gcaagtgccc aaggaaaaac tttacccaaa 1440
gtatcgtaca tcaaagcttc agaaatctgg tttttaggtt gcaccgggtt tatttttggg 1500
agtttagtgg aattcgcgtt tgtcaacaca atttggagac gaaggaaaaa tgtggaattg 1560
aaaaaagtca acagcaagta tattttgaag tcaactttga cgccgaggtt ggcccggaag 1620
gagtttcatg cttcgtttaa ttcgaatcct ggaggtggta ataaggatga tcaggatttg 1680
ggaagaggga ttagggtctt tccgccgcct ttggtcaagg ctaggtcttg ttccagtctg 1740
gataggagta atggatccgg gaattttttg agcgtccatg gaaatgatca caaagttcca 1800
acaataacag cacaatgtgc agacgatgcc gcaagtgacc agatttcagt ttgtgtcgat 1860
ggggaaaacg aagaacctgc acaaattgtt caccacacct ggacgacgat gacacctcaa 1920
gaaatttcca tgtggattga caaaaggtcc agaatttgtt tcccgatagc ttttgctata 1980
tttaactttt tttattggat atttgtttat tattta                          2016
```

<210> 1867
<211> 2080
<212> DNA
<213> Ctenocephalides felis

<220>
<221> CDS
<222> (45)..(1868)

<400> 1867

```
aaagtgatgc aacaattatt ttcaaaatac aaaagtgtta aaaa atg ggc gtt aaa  56
                                                 Met Gly Val Lys
                                                 1

aat ata tat tta tac tgc att ctg ata tgc ctg cta cat tat gca tct  104
Asn Ile Tyr Leu Tyr Cys Ile Leu Ile Cys Leu Leu His Tyr Ala Ser
5                   10                  15                  20

tat acc aaa act gaa tct att acc aac aat tct ttg gaa gaa ttg tac  152
Tyr Thr Lys Thr Glu Ser Ile Thr Asn Asn Ser Leu Glu Glu Leu Tyr
                25                  30                  35

aca aac act tct gcc aaa aca gat tcc att act ctt tta tca aaa acc  200
```

```
Thr Asn Thr Ser Ala Lys Thr Asp Ser Ile Thr Leu Leu Ser Lys Thr
            40                  45                  50

agt cta ccg cct gat caa aat gcc acg att gaa aat cct gat cca gtg    248
Ser Leu Pro Pro Asp Gln Asn Ala Thr Ile Glu Asn Pro Asp Pro Val
        55                  60                  65

ctt cct gaa aag ggc tcc gct gaa caa gaa caa cac agc tcg atg tct    296
Leu Pro Glu Lys Gly Ser Ala Glu Gln Glu Gln His Ser Ser Met Ser
    70                  75                  80

ata ttc ttc gtg ctt tgt gtg ctg gct tta ggg att ctt tta att cat    344
Ile Phe Phe Val Leu Cys Val Leu Ala Leu Gly Ile Leu Leu Ile His
85                  90                  95                  100

ttc atg tta caa aca ggg ttt cag tat tta cct gaa agt att gtt gta    392
Phe Met Leu Gln Thr Gly Phe Gln Tyr Leu Pro Glu Ser Ile Val Val
                105                 110                 115

gtt ttc tta ggt gct tta atc ggc ttg ata att aat tta atg tcg tct    440
Val Phe Leu Gly Ala Leu Ile Gly Leu Ile Ile Asn Leu Met Ser Ser
            120                 125                 130

aaa aat att gca aat tgg aag aat gaa gaa gcc ttt tca ccc aca gcg    488
Lys Asn Ile Ala Asn Trp Lys Asn Glu Glu Ala Phe Ser Pro Thr Ala
        135                 140                 145

ttt ttc tta gtg ctt cta ccg cct ata ata ttt gaa tcc ggg tat aat    536
Phe Phe Leu Val Leu Leu Pro Pro Ile Ile Phe Glu Ser Gly Tyr Asn
    150                 155                 160

ttg cat aaa ggt aat ttt ttt caa aat att ggt tcc atc ctg gtg ttt    584
Leu His Lys Gly Asn Phe Phe Gln Asn Ile Gly Ser Ile Leu Val Phe
165                 170                 175                 180

gct ata ttt gga aca gcc ata tca gcc ttt gtt gtc ggt gct ggt gtg    632
Ala Ile Phe Gly Thr Ala Ile Ser Ala Phe Val Val Gly Ala Gly Val
                185                 190                 195

tat tta cta gga atg gca gat gtt gct tat aac tta agc ttt gtt gaa    680
Tyr Leu Leu Gly Met Ala Asp Val Ala Tyr Asn Leu Ser Phe Val Glu
            200                 205                 210

tcc ttt gct ttc ggt tca tta att tct gca gta gac cct gta gct acc    728
Ser Phe Ala Phe Gly Ser Leu Ile Ser Ala Val Asp Pro Val Ala Thr
        215                 220                 225

gta gct att ttc cat gct tta gac gtg gac cca gtt tta aac atg ttg    776
```

```
Val Ala Ile Phe His Ala Leu Asp Val Asp Pro Val Leu Asn Met Leu
    230                 235                 240

gtg ttc gga gaa agt att tta aat gat gct att tca att gtt tta aca    824
Val Phe Gly Glu Ser Ile Leu Asn Asp Ala Ile Ser Ile Val Leu Thr
245                 250                 255                 260

act gca gtt ttg gaa tcc aac aat cct tta atg acg act gct gaa gct    872
Thr Ala Val Leu Glu Ser Asn Asn Pro Leu Met Thr Thr Ala Glu Ala
                265                 270                 275

gta gtc tcc ggt tta aat agg ttt tgt tta atg ttc ttt gct tcg gct    920
Val Val Ser Gly Leu Asn Arg Phe Cys Leu Met Phe Phe Ala Ser Ala
            280                 285                 290

ggt atc ggt gta gtc ttt gcc tta att agt gct ctt ttg ttg aaa cat    968
Gly Ile Gly Val Val Phe Ala Leu Ile Ser Ala Leu Leu Leu Lys His
        295                 300                 305

gtt gat ctt aga aag tat ccg tcc tta gag tta ggt atg atg ttg gtg    1016
Val Asp Leu Arg Lys Tyr Pro Ser Leu Glu Leu Gly Met Met Leu Val
    310                 315                 320

ttt act tat gca cct tat gtt ttg gca gaa gga att cat tta tca ggt    1064
Phe Thr Tyr Ala Pro Tyr Val Leu Ala Glu Gly Ile His Leu Ser Gly
325                 330                 335                 340

ata atg gcg ata tta ttc tgt ggc att gtg atg tcc cat tac aca cat    1112
Ile Met Ala Ile Leu Phe Cys Gly Ile Val Met Ser His Tyr Thr His
                345                 350                 355

ttc aat tta tca acg gtt aca caa ata act atg cag cag acg atg aga    1160
Phe Asn Leu Ser Thr Val Thr Gln Ile Thr Met Gln Gln Thr Met Arg
            360                 365                 370

act ttg gct ttt att gca gaa act tgt gtg ttt gct tat tta gga atg    1208
Thr Leu Ala Phe Ile Ala Glu Thr Cys Val Phe Ala Tyr Leu Gly Met
        375                 380                 385

gct ata ttt agt ttt cgt cac aga gtg gaa cct gcc tta gtt att tgg    1256
Ala Ile Phe Ser Phe Arg His Arg Val Glu Pro Ala Leu Val Ile Trp
    390                 395                 400

agc att gta ctt tgc tta att gga aga gct gca aat ata ttt cct tta    1304
Ser Ile Val Leu Cys Leu Ile Gly Arg Ala Ala Asn Ile Phe Pro Leu
405                 410                 415                 420

tcc tgg ctt gtg aat caa ttt agg gag cac aaa atc act aaa aag atg    1352
```

```
Ser Trp Leu Val Asn Gln Phe Arg Glu His Lys Ile Thr Lys Lys Met
                425                 430                 435

gca ttt atc atg tgg ttc agt ggt ttg cga ggt gcc ata tca tat gca    1400
Ala Phe Ile Met Trp Phe Ser Gly Leu Arg Gly Ala Ile Ser Tyr Ala
            440                 445                 450

ctt tcc tta cat tta gaa ttt tct gat gaa aca cgt cat gta ata att    1448
Leu Ser Leu His Leu Glu Phe Ser Asp Glu Thr Arg His Val Ile Ile
        455                 460                 465

aca acg aca ctt ata att gta ctt tgc aca aca ctt ata ttc ggg ggt    1496
Thr Thr Thr Leu Ile Ile Val Leu Cys Thr Thr Leu Ile Phe Gly Gly
    470                 475                 480

gct acg atg cct ttg ctg aaa ttt ttg cag gcg aac aag aag acc cgt    1544
Ala Thr Met Pro Leu Leu Lys Phe Leu Gln Ala Asn Lys Lys Thr Arg
485                 490                 495                 500

tca gcc aca aga cgt aca aga cgt cag caa aaa gca ata aca tta agc    1592
Ser Ala Thr Arg Arg Thr Arg Arg Gln Gln Lys Ala Ile Thr Leu Ser
                505                 510                 515

aaa acc cga gaa tgg gga tca gca atc gat tca gaa cta cta agt gaa    1640
Lys Thr Arg Glu Trp Gly Ser Ala Ile Asp Ser Glu Leu Leu Ser Glu
            520                 525                 530

tta aca acc gaa gaa gaa cgt gac gtc aca ttc acc caa gta aga cga    1688
Leu Thr Thr Glu Glu Glu Arg Asp Val Thr Phe Thr Gln Val Arg Arg
        535                 540                 545

ggc cta gaa ttc ata cga ctg gac cac aaa tac ttg aga ccg ttt ttc    1736
Gly Leu Glu Phe Ile Arg Leu Asp His Lys Tyr Leu Arg Pro Phe Phe
    550                 555                 560

act cga aga ttc acc cac cag gaa ttg aag gat tgc aaa agt caa atg    1784
Thr Arg Arg Phe Thr His Gln Glu Leu Lys Asp Cys Lys Ser Gln Met
565                 570                 575                 580

acg gat ctt acg aat aaa tgg tat caa act ata aga gtg agt cct cag    1832
Thr Asp Leu Thr Asn Lys Trp Tyr Gln Thr Ile Arg Val Ser Pro Gln
                585                 590                 595

atg agt gat gat gat gac gtt agt acg tgc agt act taatttaaat         1878
Met Ser Asp Asp Asp Asp Val Ser Thr Cys Ser Thr
            600                 605

ttaaatttaa tgctaattga ccaaaagtgt tatgtgatat atataacaga gttacctgta 1938
```

ttttaattta tgttttaagt tttaagataa gtgcagattt gtcagtattt tttctacaag 1998

ggtagaatac tgttgtatag ctaatgtgta aataaaaagt aataaatttg atttattgca 2058

ctctaaaaaa aaaaaaaaaa aa 2080

<210> 1868
<211> 608
<212> PRT
<213> Ctenocephalides felis

<400> 1868

Met Gly Val Lys Asn Ile Tyr Leu Tyr Cys Ile Leu Ile Cys Leu Leu
1 5 10 15

His Tyr Ala Ser Tyr Thr Lys Thr Glu Ser Ile Thr Asn Asn Ser Leu
20 25 30

Glu Glu Leu Tyr Thr Asn Thr Ser Ala Lys Thr Asp Ser Ile Thr Leu
35 40 45

Leu Ser Lys Thr Ser Leu Pro Pro Asp Gln Asn Ala Thr Ile Glu Asn
50 55 60

Pro Asp Pro Val Leu Pro Glu Lys Gly Ser Ala Glu Gln Glu Gln His
65 70 75 80

Ser Ser Met Ser Ile Phe Phe Val Leu Cys Val Leu Ala Leu Gly Ile
85 90 95

Leu Leu Ile His Phe Met Leu Gln Thr Gly Phe Gln Tyr Leu Pro Glu
100 105 110

Ser Ile Val Val Val Phe Leu Gly Ala Leu Ile Gly Leu Ile Ile Asn
115 120 125

Leu Met Ser Ser Lys Asn Ile Ala Asn Trp Lys Asn Glu Glu Ala Phe
130 135 140

Ser Pro Thr Ala Phe Phe Leu Val Leu Leu Pro Pro Ile Ile Phe Glu
145 150 155 160

Ser Gly Tyr Asn Leu His Lys Gly Asn Phe Phe Gln Asn Ile Gly Ser
165 170 175

Ile Leu Val Phe Ala Ile Phe Gly Thr Ala Ile Ser Ala Phe Val Val

180 185 190

Gly Ala Gly Val Tyr Leu Leu Gly Met Ala Asp Val Ala Tyr Asn Leu
195 200 205

Ser Phe Val Glu Ser Phe Ala Phe Gly Ser Leu Ile Ser Ala Val Asp
210 215 220

Pro Val Ala Thr Val Ala Ile Phe His Ala Leu Asp Val Asp Pro Val
225 230 235 240

Leu Asn Met Leu Val Phe Gly Glu Ser Ile Leu Asn Asp Ala Ile Ser
245 250 255

Ile Val Leu Thr Thr Ala Val Leu Glu Ser Asn Asn Pro Leu Met Thr
260 265 270

Thr Ala Glu Ala Val Val Ser Gly Leu Asn Arg Phe Cys Leu Met Phe
275 280 285

Phe Ala Ser Ala Gly Ile Gly Val Val Phe Ala Leu Ile Ser Ala Leu
290 295 300

Leu Leu Lys His Val Asp Leu Arg Lys Tyr Pro Ser Leu Glu Leu Gly
305 310 315 320

Met Met Leu Val Phe Thr Tyr Ala Pro Tyr Val Leu Ala Glu Gly Ile
325 330 335

His Leu Ser Gly Ile Met Ala Ile Leu Phe Cys Gly Ile Val Met Ser
340 345 350

His Tyr Thr His Phe Asn Leu Ser Thr Val Thr Gln Ile Thr Met Gln
355 360 365

Gln Thr Met Arg Thr Leu Ala Phe Ile Ala Glu Thr Cys Val Phe Ala
370 375 380

Tyr Leu Gly Met Ala Ile Phe Ser Phe Arg His Arg Val Glu Pro Ala
385 390 395 400

Leu Val Ile Trp Ser Ile Val Leu Cys Leu Ile Gly Arg Ala Ala Asn
405 410 415

Ile Phe Pro Leu Ser Trp Leu Val Asn Gln Phe Arg Glu His Lys Ile
420 425 430

Thr Lys Lys Met Ala Phe Ile Met Trp Phe Ser Gly Leu Arg Gly Ala

435 440 445

Ile Ser Tyr Ala Leu Ser Leu His Leu Glu Phe Ser Asp Glu Thr Arg
450 455 460

His Val Ile Ile Thr Thr Thr Leu Ile Ile Val Leu Cys Thr Thr Leu
465 470 475 480

Ile Phe Gly Gly Ala Thr Met Pro Leu Leu Lys Phe Leu Gln Ala Asn
485 490 495

Lys Lys Thr Arg Ser Ala Thr Arg Arg Thr Arg Arg Gln Gln Lys Ala
500 505 510

Ile Thr Leu Ser Lys Thr Arg Glu Trp Gly Ser Ala Ile Asp Ser Glu
515 520 525

Leu Leu Ser Glu Leu Thr Thr Glu Glu Glu Arg Asp Val Thr Phe Thr
530 535 540

Gln Val Arg Arg Gly Leu Glu Phe Ile Arg Leu Asp His Lys Tyr Leu
545 550 555 560

Arg Pro Phe Phe Thr Arg Arg Phe Thr His Gln Glu Leu Lys Asp Cys
565 570 575

Lys Ser Gln Met Thr Asp Leu Thr Asn Lys Trp Tyr Gln Thr Ile Arg
580 585 590

Val Ser Pro Gln Met Ser Asp Asp Asp Asp Val Ser Thr Cys Ser Thr
595 600 605

<210> 1869
<211> 2080
<212> DNA
<213> Ctenocephalides felis

<400> 1869

```
ttttttttt tttttttag agtgcaataa atcaaattta ttacttttta tttacacatt 60
agctatacaa cagtattcta cccttgtaga aaaaatactg acaaatctgc acttatctta 120
aaacttaaaa cataaattaa aatacaggta actctgttat atatatcaca taacactttt 180
ggtcaattag cattaaattt aaatttaaat taagtactgc acgtactaac gtcatcatca 240
tcactcatct gaggactcac tcttatagtt tgataccatt tattcgtaag atccgtcatt 300
tgacttttgc aatccttcaa ttcctggtgg gtgaatcttc gagtgaaaaa cggtctcaag 360
tatttgtggt ccagtcgtat gaattctagg cctcgtctta cttgggtgaa tgtgacgtca 420
cgttcttctt cggttgttaa ttcacttagt agttctgaat cgattgctga tcccattct 480
```

cggggttttgc ttaatgttat tgctttttgc tgacgtcttg tacgtcttgt ggctgaacgg 540
gtcttcttgt tcgcctgcaa aaatttcagc aaaggcatcg tagcaccccc gaatataagt 600
gttgtgcaaa gtacaattat aagtgtcgtt gtaattatta catgacgtgt ttcatcagaa 660
aattctaaat gtaaggaaag tgcatatgat atggcacctc gcaaaccact gaaccacatg 720
ataaatgcca tctttttagt gattttgtgc tccctaaatt gattcacaag ccaggataaa 780
ggaaatatat ttgcagctct tccaattaag caaagtacaa tgctccaaat aactaaggca 840
ggttccactc tgtgacgaaa actaaatata gccattccta aataagcaaa cacacaagtt 900
tctgcaataa aagccaaagt tctcatcgtc tgctgcatag ttatttgtgt aaccgttgat 960
aaattgaaat gtgtgtaatg ggacatcaca atgccacaga ataatatcgc cattatacct 1020
gataaatgaa ttccttctgc caaaacataa ggtgcataag taaacaccaa catcatacct 1080
aactctaagg acggatactt tctaagatca acatgtttca acaaaagagc actaattaag 1140
gcaaagacta caccgatacc agccgaagca aagaacatta aacaaaacct atttaaaccg 1200
gagactacag cttcagcagt cgtcattaaa ggattgttgg attccaaaac tgcagttgtt 1260
aaaacaattg aaatagcatc atttaaaata ctttctccga acaccaacat gtttaaaact 1320
gggtccacgt ctaaagcatg gaaaatagct acggtagcta cagggtctac tgcagaaatt 1380
aatgaaccga aagcaaagga ttcaacaaag cttaagttat aagcaacatc tgccattcct 1440
agtaaataca caccagcacc gacaacaaag gctgatatgg ctgttccaaa tatagcaaac 1500
accaggatgg aaccaatatt ttgaaaaaaa ttacctttat gcaaattata cccggattca 1560
aatattatag gcggtagaag cactaagaaa aacgctgtgg gtgaaaaggc ttcttcattc 1620
ttccaatttg caatattttt agacgacatt aaattaatta tcaagccgat taaagcacct 1680
aagaaaacta caacaatact ttcaggtaaa tactgaaacc ctgtttgtaa catgaaatga 1740
attaaaagaa tccctaaagc cagcacacaa agcacgaaga atatagacat cgagctgtgt 1800
tgttcttgtt cagcggagcc cttttcagga agcactggat caggattttc aatcgtggca 1860
ttttgatcag gcggtagact ggtttttgat aaaagagtaa tggaatctgt tttggcagaa 1920
gtgtttgtgt acaattcttc caaagaattg ttggtaatag attcagtttt ggtataagat 1980
gcataatgta gcaggcatat cagaatgcag tataaatata tatttttaac gcccattttt 2040
taacactttt gtattttgaa aataattgtt gcatcacttt 2080

<210> 1870
<211> 1824
<212> DNA
<213> Ctenocephalides felis

<400> 1870

atgggcgtta aaaatatata tttatactgc attctgatat gcctgctaca ttatgcatct 60
tataccaaaa ctgaatctat taccaacaat tctttggaag aattgtacac aaacacttct 120
gccaaaacag attccattac tcttttatca aaaaccagtc taccgcctga tcaaaatgcc 180
acgattgaaa atcctgatcc agtgcttcct gaaaagggct ccgctgaaca agaacaacac 240
agctcgatgt ctatattctt cgtgctttgt gtgctggctt tagggattct tttaattcat 300
ttcatgttac aaacagggtt tcagtattta cctgaaagta ttgttgtagt tttcttaggt 360
gctttaatcg gcttgataat taatttaatg tcgtctaaaa atattgcaaa ttggaagaat 420
gaagaagcct tttcacccac agcgtttttc ttagtgcttc taccgcctat aatatttgaa 480
tccgggtata atttgcataa aggtaatttt tttcaaaata ttggttccat cctggtgttt 540
gctatatttg gaacagccat atcagccttt gttgtcggtg ctggtgtgta tttactagga 600
atggcagatg ttgcttataa cttaagcttt gttgaatcct ttgctttcgg ttcattaatt 660
tctgcagtag accctgtagc taccgtagct attttccatg ctttagacgt ggacccagtt 720
ttaaacatgt tggtgttcgg agaaagtatt ttaaatgatg ctatttcaat tgttttaaca 780

```
actgcagttt tggaatccaa caatccttta atgacgactg ctgaagctgt agtctccggt 840
ttaaataggt tttgtttaat gttctttgct tcggctggta tcggtgtagt ctttgcctta 900
attagtgctc ttttgttgaa acatgttgat cttagaaagt atccgtcctt agagttaggt 960
atgatgttgg tgtttactta tgcaccttat gttttggcag aaggaattca tttatcaggt 1020
ataatggcga tattattctg tggcattgtg atgtcccatt acacacattt caatttatca 1080
acggttacac aaataactat gcagcagacg atgagaactt tggcttttat tgcagaaact 1140
tgtgtgtttg cttatttagg aatggctata tttagttttc gtcacagagt ggaacctgcc 1200
ttagttattt ggagcattgt actttgctta attggaagag ctgcaaatat atttccttta 1260
tcctggcttg tgaatcaatt tagggagcac aaaatcacta aaaagatggc atttatcatg 1320
tggttcagtg gtttgcgagg tgccatatca tatgcacttt ccttacattt agaattttct 1380
gatgaaacac gtcatgtaat aattacaacg acacttataa ttgtactttg cacaacactt 1440
atattcgggg gtgctacgat gcctttgctg aaatttttgc aggcgaacaa gaagacccgt 1500
tcagccacaa gacgtacaag acgtcagcaa aaagcaataa cattaagcaa aacccgagaa 1560
tggggatcag caatcgattc agaactacta agtgaattaa caaccgaaga agaacgtgac 1620
gtcacattca cccaagtaag acgaggccta gaattcatac gactggacca caaatacttg 1680
agaccgtttt tcactcgaag attcacccac caggaattga aggattgcaa aagtcaaatg 1740
acggatctta cgaataaatg gtatcaaact ataagagtga gtcctcagat gagtgatgat 1800
gatgacgtta gtacgtgcag tact                                        1824
```

<210> 1871
<211> 1824
<212> DNA
<213> Ctenocephalides felis

<400> 1871

```
agtactgcac gtactaacgt catcatcatc actcatctga ggactcactc ttatagtttg 60
ataccattta ttcgtaagat ccgtcatttg acttttgcaa tccttcaatt cctggtgggt 120
gaatcttcga gtgaaaaacg gtctcaagta tttgtggtcc agtcgtatga attctaggcc 180
tcgtcttact tgggtgaatg tgacgtcacg ttcttcttcg gttgttaatt cacttagtag 240
ttctgaatcg attgctgatc cccattctcg ggttttgctt aatgttattg ctttttgctg 300
acgtcttgta cgtcttgtgg ctgaacgggt cttcttgttc gcctgcaaaa atttcagcaa 360
aggcatcgta gcacccccga atataagtgt tgtgcaaagt acaattataa gtgtcgttgt 420
aattattaca tgacgtgttt catcagaaaa ttctaaatgt aaggaaagtg catatgatat 480
ggcacctcgc aaaccactga accacatgat aaatgccatc tttttagtga ttttgtgctc 540
cctaaattga ttcacaagcc aggataaagg aaatatattt gcagctcttc caattaagca 600
aagtacaatg ctccaaataa ctaaggcagg ttccactctg tgacgaaaac taaatatagc 660
cattcctaaa taagcaaaca cacaagtttc tgcaataaaa gccaaagttc tcatcgtctg 720
ctgcatagtt atttgtgtaa ccgttgataa attgaaatgt gtgtaatggg acatcacaat 780
gccacagaat aatatcgcca ttatacctga taaatgaatt ccttctgcca aaacataagg 840
tgcataagta aacaccaaca tcatacctaa ctctaaggac ggatactttc taagatcaac 900
atgtttcaac aaaagagcac taattaaggc aaagactaca ccgataccag ccgaagcaaa 960
gaacattaaa caaaacctat ttaaaccgga gactacagct tcagcagtcg tcattaaagg 1020
attgttggat tccaaaactg cagttgttaa aacaattgaa atagcatcat ttaaaatact 1080
ttctccgaac accaacatgt ttaaaactgg gtccacgtct aaagcatgga aatagctac 1140
ggtagctaca gggtctactg cagaaattaa tgaaccgaaa gcaaaggatt caacaaagct 1200
taagttataa gcaacatctg ccattcctag taaatacaca ccagcaccga caacaaaggc 1260
tgatatggct gttccaaata tagcaaacac caggatggaa ccaatatttt gaaaaaaatt 1320
```

```
acctttatgc aaattatacc cggattcaaa tattataggc ggtagaagca ctaagaaaaa 1380
cgctgtgggt gaaaaggctt cttcattctt ccaatttgca atatttttag acgacattaa 1440
attaattatc aagccgatta aagcacctaa gaaaactaca acaatacttt caggtaaata 1500
ctgaaaccct gtttgtaaca tgaaatgaat taaaagaatc cctaaagcca gcacacaaag 1560
cacgaagaat atagacatcg agctgtgttg ttcttgttca gcggagccct tttcaggaag 1620
cactggatca ggattttcaa tcgtggcatt ttgatcaggc ggtagactgg tttttgataa 1680
aagagtaatg gaatctgttt tggcagaagt gtttgtgtac aattcttcca aagaattgtt 1740
ggtaatagat tcagttttgg tataagatgc ataatgtagc aggcatatca gaatgcagta 1800
taaatatata tttttaacgc ccat                                        1824
```

<210> 1872
<211> 2383
<212> DNA
<213> Ctenocephalides felis

<220>
<221> CDS
<222> (60)..(845)

<400> 1872

```
aattcttgtg tgtagaataa aaaacatatt tgaaacgttc atataataaa aagtgcaac  59

atg tca gat gaa atg caa gaa aac gga act att aat ggt gaa gtg cca   107
Met Ser Asp Glu Met Gln Glu Asn Gly Thr Ile Asn Gly Glu Val Pro
1               5                   10                  15

gaa att gaa ctt atc att aag gca tcc acc ata gat ggt cga cgt aaa   155
Glu Ile Glu Leu Ile Ile Lys Ala Ser Thr Ile Asp Gly Arg Arg Lys
            20                  25                  30

gga gct tgt tta ttt tgt caa gaa tat ttt atg gat ttg tac ctg cta   203
Gly Ala Cys Leu Phe Cys Gln Glu Tyr Phe Met Asp Leu Tyr Leu Leu
        35                  40                  45

gca gaa ctt aaa act atc agt tta aag gtt aca aca gta gac atg caa   251
Ala Glu Leu Lys Thr Ile Ser Leu Lys Val Thr Thr Val Asp Met Gln
    50                  55                  60

aaa cct cca ccg gat ttc cgt aca aat ttt gaa gcg acg ccg ccg cca   299
Lys Pro Pro Pro Asp Phe Arg Thr Asn Phe Glu Ala Thr Pro Pro Pro
65                  70                  75                  80

att cta atc gac aat ggc ctg gcc gtg cta gaa aac gac aaa atc gaa   347
Ile Leu Ile Asp Asn Gly Leu Ala Val Leu Glu Asn Asp Lys Ile Glu
                85                  90                  95

cgt cac atc atg aag agt gtc cct gga gga cac aat ctt ttt gtt cag   395
```

```
Arg His Ile Met Lys Ser Val Pro Gly Gly His Asn Leu Phe Val Gln
            100                 105                 110

gat aaa gaa gtg gca aca ctc atc gag aat ttg tac tct aaa ttg aag     443
Asp Lys Glu Val Ala Thr Leu Ile Glu Asn Leu Tyr Ser Lys Leu Lys
        115                 120                 125

ttg gtt tta gtc aaa aag gat gac gta aaa agc aat agt ctg ctg agc     491
Leu Val Leu Val Lys Lys Asp Asp Val Lys Ser Asn Ser Leu Leu Ser
    130                 135                 140

cac ctg agg aaa atc aac gac cat ttg gcg cgg cgc ggc acg aga ttc     539
His Leu Arg Lys Ile Asn Asp His Leu Ala Arg Arg Gly Thr Arg Phe
145                 150                 155                 160

cta acg ggc gac acc atg tgc tgc ttc gac tgc gaa ctg atg ccc agg     587
Leu Thr Gly Asp Thr Met Cys Cys Phe Asp Cys Glu Leu Met Pro Arg
                165                 170                 175

tta caa cac atc agg gtc gcc gcc aag tat ttc gtc gaa ttt gaa att     635
Leu Gln His Ile Arg Val Ala Ala Lys Tyr Phe Val Glu Phe Glu Ile
            180                 185                 190

ccg agc aat cta acc gcc tta tgg cgt tat atg tat cac atg tac cag     683
Pro Ser Asn Leu Thr Ala Leu Trp Arg Tyr Met Tyr His Met Tyr Gln
        195                 200                 205

ttg gac gca ttc acc cag tcg tgc cca gcc gac caa gat atc atc aac     731
Leu Asp Ala Phe Thr Gln Ser Cys Pro Ala Asp Gln Asp Ile Ile Asn
    210                 215                 220

cac tat aaa ctg caa cag cag agg atc agc aat aac cag atg atg aaa     779
His Tyr Lys Leu Gln Gln Gln Arg Ile Ser Asn Asn Gln Met Met Lys
225                 230                 235                 240

atg aag aag cac gag gag cta gaa acg cca acg ttc acc aca tcg att     827
Met Lys Lys His Glu Glu Leu Glu Thr Pro Thr Phe Thr Thr Ser Ile
                245                 250                 255

cca gtc gac gtt tca ggc tgataggaaa gaagtattta aaacaaaatt           875
Pro Val Asp Val Ser Gly
            260

agtaatttgg ataatactaa acatgattat ctgtatatgt taccgttcca aataaattat 935

tcatattaaa atacgattag taagcgttat gatgtatcca aagttcgatt accattttta 995

caaatgccat cagttaggaa tcttttcttt gcttaataca ccaagagttt cattataatt 1055
```

ataacgtact ataatatatt tcgcaattgc aattggcata tcgttcaata ttaatattac 1115

aaagctgcca gtgatacgtc aatcatattt ttgaacatat tttatcttat gtttcagtaa 1175

ttctgtaaga atttttagat gcttatgtat aaattggtga tatagcataa ataacatata 1235

cgaatttgta tattttgaag tactatctgt gtaaactatc caaaactaac attcaaaatt 1295

tgtttttttga aaaaaattat cttacaatat gtgtactata atataatgta tttagtatta 1355

aatgtttgag aaactaattt agttaagttg tatacaataa attataatcg tgttatatta 1415

tgtttatatt tacaaatttt cgtgatggag tttaatgtta gatgaatata ttaaaatgta 1475

tgttctaaca attgtaatat taatgttaat tttaaccgta aattaaggaa aacattgttt 1535

tgatgactac ttagcttccg atccagaggt gatttagaca tttagttagc attttaaaat 1595

attgtgtagc taacatttcg tgtgattttg acaactaatc agttatttaa gaaatgtgca 1655

tataattatt atatacatat tattttacat acctgtttag tattcaaaaa acttgtcaat 1715

tacttcgcac aaaagaacat tgtgagtata ataatatcat attatgcaaa cattctttat 1775

ttacgtgtag tttacttgaa gtatcttggt tttctttgct ttttttcatt tgattcagta 1835

aaattaattc aaatctaaca gtattttgca tagcagttat acgaaaatta accagtgctt 1895

aacaatattt aaaatattta tataattcat taactagtta tagtaaaaaa atatataaat 1955

tcattttaaa ttaaaagaca taatatattt tacatattgt ttaaatcttg tactttgcca 2015

ttttctctgt ttgtttcatg taatcttcgg aagcgtagca aatgttgtgt atattagtaa 2075

tgtacagttg tataaaacat aaattgtaag tccacttaca ggcacatgct attgtcttat 2135

tgaatttatt tgttctacca ggaaagtgct ttatagattt tactaaatat atattaagaa 2195

aagcgttctc tgttgaattg taattaatcc nttttgtaag atttacngca agtatgaaga 2255

aatgttaaat tttgttaaat ttcatgtatt gtatatgata ttcgcacgta cttatgaaat 2315

gtatgncagt caaatgctga atttntttta atanacnatc tttgnantac ctaaaaaaaa 2375

aaaaaaaa 2383

<210> 1873

<211> 262
<212> PRT
<213> Ctenocephalides felis

<400> 1873

```
Met Ser Asp Glu Met Gln Glu Asn Gly Thr Ile Asn Gly Glu Val Pro
 1               5                  10                  15

Glu Ile Glu Leu Ile Ile Lys Ala Ser Thr Ile Asp Gly Arg Arg Lys
            20                  25                  30

Gly Ala Cys Leu Phe Cys Gln Glu Tyr Phe Met Asp Leu Tyr Leu Leu
        35                  40                  45

Ala Glu Leu Lys Thr Ile Ser Leu Lys Val Thr Thr Val Asp Met Gln
    50                  55                  60

Lys Pro Pro Pro Asp Phe Arg Thr Asn Phe Glu Ala Thr Pro Pro Pro
65                  70                  75                  80

Ile Leu Ile Asp Asn Gly Leu Ala Val Leu Glu Asn Asp Lys Ile Glu
                85                  90                  95

Arg His Ile Met Lys Ser Val Pro Gly Gly His Asn Leu Phe Val Gln
            100                 105                 110

Asp Lys Glu Val Ala Thr Leu Ile Glu Asn Leu Tyr Ser Lys Leu Lys
        115                 120                 125

Leu Val Leu Val Lys Lys Asp Asp Val Lys Ser Asn Ser Leu Leu Ser
    130                 135                 140

His Leu Arg Lys Ile Asn Asp His Leu Ala Arg Arg Gly Thr Arg Phe
145                 150                 155                 160

Leu Thr Gly Asp Thr Met Cys Cys Phe Asp Cys Glu Leu Met Pro Arg
                165                 170                 175

Leu Gln His Ile Arg Val Ala Ala Lys Tyr Phe Val Glu Phe Glu Ile
            180                 185                 190

Pro Ser Asn Leu Thr Ala Leu Trp Arg Tyr Met Tyr His Met Tyr Gln
        195                 200                 205

Leu Asp Ala Phe Thr Gln Ser Cys Pro Ala Asp Gln Asp Ile Ile Asn
    210                 215                 220
```

```
His Tyr Lys Leu Gln Gln Gln Arg Ile Ser Asn Asn Gln Met Met Lys
225                 230                 235                 240

Met Lys Lys His Glu Glu Leu Glu Thr Pro Thr Phe Thr Thr Ser Ile
                245                 250                 255

Pro Val Asp Val Ser Gly
            260
```

<210> 1874
<211> 2383
<212> DNA
<213> Ctenocephalides felis

<400> 1874

```
tttttttttt ttttttaggt antncaaaga tngtntatta aaanaaattc agcatttgac 60
tgncatacat ttcataagta cgtgcgaata tcatatacaa tacatgaaat ttaacaaaat 120
ttaacatttc ttcatacttg cngtaaatct tacaaaangg attaattaca attcaacaga 180
gaacgctttt cttaatatat atttagtaaa atctataaag cactttcctg gtagaacaaa 240
taaattcaat aagacaatag catgtgcctg taagtggact tacaatttat gttttataca 300
actgtacatt actaatatac acaacatttg ctacgcttcc gaagattaca tgaaacaaac 360
agagaaaatg gcaaagtaca agatttaaac aatatgtaaa atatattatg tcttttaatt 420
taaaatgaat ttatatattt ttttactata actagttaat gaattatata aatattttaa 480
atattgttaa gcactggtta attttcgtat aactgctatg caaaatactg ttagatttga 540
attaatttta ctgaatcaaa tgaaaaaaag caaagaaaac caagatactt caagtaaact 600
acacgtaaat aaagaatgtt tgcataatat gatattatta tactcacaat gttcttttgt 660
gcgaagtaat tgacaagttt tttgaatact aaacaggtat gtaaaataat atgtatataa 720
taattatatg cacatttctt aaataactga ttagttgtca aaatcacacg aaatgttagc 780
tacacaatat tttaaaatgc taactaaatg tctaaatcac ctctggatcg gaagctaagt 840
agtcatcaaa acaatgtttt ccttaattta cggttaaaat taacattaat attacaattg 900
ttagaacata cattttaata tattcatcta acattaaact ccatcacgaa aatttgtaaa 960
tataaacata atataacacg attataattt attgtataca acttaactaa attagtttct 1020
caaacattta atactaaata cattatatta tagtacacat attgtaagat aatttttttc 1080
aaaaaacaaa ttttgaatgt tagttttgga tagtttacac agatagtact tcaaaatata 1140
caaattcgta tatgttattt atgctatatc accaatttat acataagcat ctaaaaattc 1200
ttacagaatt actgaaacat aagataaaat atgttcaaaa atatgattga cgtatcactg 1260
gcagctttgt aatattaata ttgaacgata tgccaattgc aattgcgaaa tatattatag 1320
tacgttataa ttataatgaa actcttggtg tattaagcaa agaaaagatt cctaactgat 1380
ggcatttgta aaaatggtaa tcgaactttg gatacatcat aacgcttact aatcgtattt 1440
taatatgaat aatttatttg gaacggtaac atatacagat aatcatgttt agtattatcc 1500
aaattactaa ttttgtttta aatacttctt tcctatcagc ctgaaacgtc gactggaatc 1560
gatgtggtga acgttggcgt ttctagctcc tcgtgcttct tcattttcat catctggtta 1620
ttgctgatcc tctgctgttg cagtttatag tggttgatga tatcttggtc ggctgggcac 1680
gactgggtga atgcgtccaa ctggtacatg tgatacatat aacgccataa ggcggttaga 1740
ttgctcggaa tttcaaattc gacgaaatac ttggcggcga ccctgatgtg ttgtaacctg 1800
ggcatcagtt cgcagtcgaa gcagcacatg gtgtcgcccg ttaggaatct cgtgccgcgc 1860
```

```
cgcgccaaat ggtcgttgat tttcctcagg tggctcagca gactattgct ttttacgtca 1920
tcctttttga ctaaaaccaa cttcaattta gagtacaaat tctcgatgag tgttgccact 1980
tctttatcct gaacaaaaag attgtgtcct ccagggacac tcttcatgat gtgacgttcg 2040
attttgtcgt tttctagcac ggccaggcca ttgtcgatta gaattggcgg cggcgtcgct 2100
tcaaaatttg tacggaaatc cggtggaggt tttgcatgt ctactgttgt aacctttaaa 2160
ctgatagttt taagttctgc tagcaggtac aaatccataa aatattcttg acaaaataaa 2220
caagctcctt tacgtcgacc atctatggtg gatgccttaa tgataagttc aatttctggc 2280
acttcaccat taatagttcc gtttcttgc atttcatctg acatgttgca ctttttatta 2340
tatgaacgtt tcaaatatgt tttttattct acacacaaga att                   2383
```

<210> 1875
<211> 786
<212> DNA
<213> Ctenocephalides felis

<400> 1875

```
atgtcagatg aaatgcaaga aaacggaact attaatggtg aagtgccaga aattgaactt 60
atcattaagg catccaccat agatggtcga cgtaaaggag cttgtttatt ttgtcaagaa 120
tattttatgg atttgtacct gctagcagaa cttaaaacta tcagtttaaa ggttacaaca 180
gtagacatgc aaaaacctcc accggatttc cgtacaaatt ttgaagcgac gccgccgcca 240
attctaatcg acaatggcct ggccgtgcta gaaaacgaca aaatcgaacg tcacatcatg 300
aagagtgtcc ctggaggaca caatcttttt gttcaggata aagaagtggc aacactcatc 360
gagaatttgt actctaaatt gaagttggtt ttagtcaaaa aggatgacgt aaaaagcaat 420
agtctgctga gccacctgag gaaaatcaac gaccatttgg cgcggcgcgg cacgagattc 480
ctaacgggcg acaccatgtg ctgcttcgac tgcgaactga tgcccaggtt acaacacatc 540
agggtcgccg ccaagtattt cgtcgaattt gaaattccga gcaatctaac cgccttatgg 600
cgttatatgt atcacatgta ccagttggac gcattcaccc agtcgtgccc agccgaccaa 660
gatatcatca accactataa actgcaacag cagaggatca gcaataacca gatgatgaaa 720
atgaagaagc acgaggagct agaaacgcca acgttcacca catcgattcc agtcgacgtt 780
tcaggc                                                            786
```

<210> 1876
<211> 786
<212> DNA
<213> Ctenocephalides felis

<400> 1876

```
gcctgaaacg tcgactggaa tcgatgtggt gaacgttggc gtttctagct cctcgtgctt 60
cttcattttc atcatctggt tattgctgat cctctgctgt tgcagtttat agtggttgat 120
gatatcttgg tcggctgggc acgactgggt gaatgcgtcc aactggtaca tgtgatacat 180
ataacgccat aaggcggtta gattgctcgg aatttcaaat tcgacgaaat acttggcggc 240
gaccctgatg tgttgtaacc tgggcatcag ttcgcagtcg aagcagcaca tggtgtcgcc 300
cgttaggaat ctcgtgccgc gccgcgccaa atggtcgttg attttcctca ggtggctcag 360
cagactattg ctttttacgt catccttttt gactaaaacc aacttcaatt tagagtacaa 420
attctcgatg agtgttgcca cttctttatc ctgaacaaaa agattgtgtc ctccagggac 480
actcttcatg atgtgacgtt cgattttgtc gttttctagc acggccaggc cattgtcgat 540
```

tagaattggc ggcggcgtcg cttcaaaatt tgtacggaaa tccggtggag gtttttgcat 600
gtctactgtt gtaaccttta aactgatagt tttaagttct gctagcaggt acaaatccat 660
aaaatattct tgacaaaata aacaagctcc tttacgtcga ccatctatgg tggatgcctt 720
aatgataagt tcaatttctg gcacttcacc attaatagtt ccgttttctt gcatttcatc 780
tgacat 786

<210> 1877
<211> 457
<212> DNA
<213> Ctenocephalides felis

<400> 1877

gcaaacaaca aggtccgctg atgcaagatc cacacgattg tcacgcatat tacacatgtc 60
tagaaattgg atcattaccg aaacatttta attgtaataa aggtgcttat ttcaatacag 120
tcaaattaaa atgcgtgaaa ggaaattgcg aaaatagcac agaaattcct cttcctgagc 180
ttccagacat ttgcgatgaa gtaggacctt tggtgcaaga tccaaacgat tgccgcaagt 240
attattcatg cgtcacgatt ggaaaagaac ctgaacattt tacgtgcaat aaaggggcgt 300
attttgatcg agaaagatta cggtgtgtca gaggatcttg ttaacaaata ttgttatata 360
acaaagttca atctttaatt attatttaga agaatttgaa aatgtatatt taatgttttt 420
taataaaata gtttattggc aatttnaaaa aaaaaaa 457

<210> 1878
<211> 1291
<212> DNA
<213> Ctenocephalides felis

<400> 1878

gattagctgc ccactatagg gctaaagcgg ccgccgggtg gtgttctgca gatccaagcc 60
catgtttctc ggaaggttct ataacttgca ccggtccagg agtttttcct gatccgtatg 120
attgtcagcg ttatcatgaa tgtaaaactg caaatgaatc atctaagcct gtcgagtgtg 180
ggggttacaa ggcttataat gttatagaaa ataattgtag cctgaacatg aatcatcaat 240
cgtgtaaacg cttacaattt cattgtgata ctataggaga tgaaaatgct tggccgagca 300
atagaaatat atattatagg tgcaccgaaa aaccttgtg gttcaatagc aacaaaatat 360
tatatccttt attatatcgg tgtgatgaga gtgagatata tgatgcagtg cagagagttt 420
gcgtaagaga tgaaaccacc acgacgcctg ccacaacgcc aaccgaatct tccacgtcta 480
gtgaaacaac cacgacgtct gccacaacat caaccgaatc ttccacgtct agtgaaacaa 540
ccacgacgtc tgccacaaca ccaaccgaat cttccacgtc tagtgaaaca accacgacgt 600
ctgccacaac accaaccgaa tcttccacgt ctagtgaaac aaccacgacg tctgccacaa 660
caccaaccga atcttccacg tctagtgaaa caaccacgac gtctgccaca acaccaaccg 720
aatcttccac gtctggtgaa acaaccacga cgtctgccac aacaccaacc gaaccttcca 780
caaagcctac ttctacggaa actcccgcaa caaaaccacc gcaagaaata ccatgcaaac 840
aacaaggtcc gctgatgcaa gatccacacg attgtcacgc atattacaca tgtctagaaa 900
ttggatcatt accgaaacat tttaattgta ataaaggtgc ttatttcaat acagtcaaat 960
taaaatgcgt gaaaggaaat tgcgaaaata gcacagaaat tcctcttcct gagcttccag 1020
acatttgcga tgaagtagga cctttggtgc aagatccaaa cgattgccgc aagtattatt 1080
catgcgtcac gattggaaaa gaacctgaac attttacgtg caataaaggg gcgtattttg 1140

atcgagaaag attacggtgt gtcagaggat cttgttaaca aatattgtta tataacaaag 1200
ttcaatcttt aattattatt tagaagaatt tgaaaatgta tatttaatgt tttttaataa 1260
aatagtttat tggcaatttn aaaaaaaaaa a 1291

<210> 1879
<211> 1291
<212> DNA
<213> Ctenocephalides felis

<400> 1879

tttttttttt tnaaattgcc aataaactat tttattaaaa aacattaaat atacattttc 60
aaattcttct aaataataat taaagattga actttgttat ataacaatat ttgttaacaa 120
gatcctctga cacaccgtaa tctttctcga tcaaaatacg cccctttatt gcacgtaaaa 180
tgttcaggtt cttttccaat cgtgacgcat gaataatact tgcggcaatc gtttggatct 240
tgcaccaaag gtcctacttc atcgcaaatg tctggaagct caggaagagg aatttctgtg 300
ctattttcgc aatttccttt cacgcatttt aatttgactg tattgaaata agcaccttta 360
ttacaattaa aatgtttcgg taatgatcca atttctagac atgtgtaata tgcgtgacaa 420
tcgtgtggat cttgcatcag cggaccttgt tgtttgcatg gtatttcttg cggtggtttt 480
gttgcgggag tttccgtaga agtaggcttt gtggaaggtt cggttggtgt tgtggcagac 540
gtcgtggttg tttcaccaga cgtggaagat tcggttggtg ttgtggcaga cgtcgtggtt 600
gtttcactag acgtggaaga ttcggttggt gttgtggcag acgtcgtggt tgtttcacta 660
gacgtggaag attcggttgg tgttgtggca gacgtcgtgg ttgtttcact agacgtggaa 720
gattcggttg gtgttgtggc agacgtcgtg gttgtttcac tagacgtgga agattcggtt 780
gatgttgtgg cagacgtcgt ggttgtttca ctagacgtgg aagattcggt tggcgttgtg 840
gcaggcgtcg tggtggtttc atctcttacg caaactctct gcactgcatc atatatctca 900
ctctcatcac accgatataa taaaggatat aatattttgt tgctattgaa ccacaaggtt 960
ttttcggtgc acctataata tatatttcta ttgctcggcc aagcattttc atctcctata 1020
gtatcacaat gaaattgtaa gcgtttacac gattgatgat tcatgttcag gctacaatta 1080
ttttctataa cattataagc cttgtaaccc ccacactcga caggcttaga tgattcattt 1140
gcagttttac attcatgata acgctgacaa tcatacggat caggaaaaac tcctggaccg 1200
gtgcaagtta tagaaccttc cgagaaacat gggcttggat ctgcagaaca ccacccggcg 1260
gccgctttag ccctatagtg ggcagctaat c 1291

<210> 1880
<211> 279
<212> DNA
<213> Ctenocephalides felis

<400> 1880

caatgatcaa gtcagttata attgtggcca acttttggct attcgtagcc ttgcaatcga 60
ttgataatcg gcagactaac aactgctctg agattggtgt tgatggtttt ttttgtctca 120
actgcagcgt cacagctttt tgtggtagag gacctacggg tgaattcaac accgtgtcta 180
caagtccatg tagttctggt gaagtttgca gtacctgggc gggtagatgt tctgcagatc 240
caagcccatg tttctcggaa ggttctataa cttgcaccg 279

<210> 1881
<211> 279
<212> DNA

<213> Ctenocephalides felis

<400> 1881

```
cggtgcaagt tatagaacct tccgagaaac atgggcttgg atctgcagaa catctacccg 60
cccaggtact gcaaacttca ccagaactac atggacttgt agacacggtg ttgaattcac 120
ccgtaggtcc tctaccacaa aaagctgtga cgctgcagtt gagacaaaaa aaaccatcaa 180
caccaatctc agagcagttg ttagtctgcc gattatcaat cgattgcaag gctacgaata 240
gccaaaagtt ggccacaatt ataactgact tgatcattg                        279
```

<210> 1882
<211> 1477
<212> DNA
<213> Ctenocephalides felis

<220>
<221> CDS
<222> (3)..(1361)

<400> 1882

```
ca atg atc aag tca gtt ata att gtg gcc aac ttt tgg cta ttc gta     47
   Met Ile Lys Ser Val Ile Ile Val Ala Asn Phe Trp Leu Phe Val
   1               5                   10                  15

gcc ttg caa tcg att gat aat cgg cag act aac aac tgc tct gag att    95
Ala Leu Gln Ser Ile Asp Asn Arg Gln Thr Asn Asn Cys Ser Glu Ile
                20                  25                  30

ggt gtt gat ggt ttt ttt tgt ctc aac tgc agc gtc aca gct ttt tgt    143
Gly Val Asp Gly Phe Phe Cys Leu Asn Cys Ser Val Thr Ala Phe Cys
            35                  40                  45

ggt aga gga cct acg ggt gaa ttc aac acc gtg tct aca agt cca tgt    191
Gly Arg Gly Pro Thr Gly Glu Phe Asn Thr Val Ser Thr Ser Pro Cys
        50                  55                  60

agt tct ggt gaa gtt tgc agt acc tgg gcg ggt aga tgt tct gca gat    239
Ser Ser Gly Glu Val Cys Ser Thr Trp Ala Gly Arg Cys Ser Ala Asp
    65                  70                  75

cca agc cca tgt ttc tcg gaa ggt tct ata act tgc acc ggt cca gga    287
Pro Ser Pro Cys Phe Ser Glu Gly Ser Ile Thr Cys Thr Gly Pro Gly
80                  85                  90                  95

gtt ttt cct gat ccg tat gat tgt cag cgt tat cat gaa tgt aaa act    335
```

```
Val Phe Pro Asp Pro Tyr Asp Cys Gln Arg Tyr His Glu Cys Lys Thr
                100                 105                 110

gca aat gaa tca tct aag cct gtc gag tgt ggg ggt tac aag gct tat      383
Ala Asn Glu Ser Ser Lys Pro Val Glu Cys Gly Gly Tyr Lys Ala Tyr
            115                 120                 125

aat gtt ata gaa aat aat tgt agc ctg aac atg aat cat caa tcg tgt      431
Asn Val Ile Glu Asn Asn Cys Ser Leu Asn Met Asn His Gln Ser Cys
        130                 135                 140

aaa cgc tta caa ttt cat tgt gat act ata gga gat gaa aat gct tgg      479
Lys Arg Leu Gln Phe His Cys Asp Thr Ile Gly Asp Glu Asn Ala Trp
    145                 150                 155

ccg agc aat aga aat ata tat tat agg tgc acc gaa aaa acc ttg tgg      527
Pro Ser Asn Arg Asn Ile Tyr Tyr Arg Cys Thr Glu Lys Thr Leu Trp
160                 165                 170                 175

ttc aat agc aac aaa ata tta tat cct tta tta tat cgg tgt gat gag      575
Phe Asn Ser Asn Lys Ile Leu Tyr Pro Leu Leu Tyr Arg Cys Asp Glu
                180                 185                 190

agt gag ata tat gat gca gtg cag aga gtt tgc gta aga gat gaa acc      623
Ser Glu Ile Tyr Asp Ala Val Gln Arg Val Cys Val Arg Asp Glu Thr
            195                 200                 205

acc acg acg cct gcc aca acg cca acc gaa tct tcc acg tct agt gaa      671
Thr Thr Thr Pro Ala Thr Thr Pro Thr Glu Ser Ser Thr Ser Ser Glu
        210                 215                 220

aca acc acg acg tct gcc aca aca tca acc gaa tct tcc acg tct agt      719
Thr Thr Thr Thr Ser Ala Thr Thr Ser Thr Glu Ser Ser Thr Ser Ser
    225                 230                 235

gaa aca acc acg acg tct gcc aca aca cca acc gaa tct tcc acg tct      767
Glu Thr Thr Thr Thr Ser Ala Thr Thr Pro Thr Glu Ser Ser Thr Ser
240                 245                 250                 255

agt gaa aca acc acg acg tct gcc aca aca cca acc gaa tct tcc acg      815
Ser Glu Thr Thr Thr Thr Ser Ala Thr Thr Pro Thr Glu Ser Ser Thr
                260                 265                 270

tct agt gaa aca acc acg acg tct gcc aca aca cca acc gaa tct tcc      863
Ser Ser Glu Thr Thr Thr Thr Ser Ala Thr Thr Pro Thr Glu Ser Ser
            275                 280                 285

acg tct agt gaa aca acc acg acg tct gcc aca aca cca acc gaa tct      911
```

```
Thr Ser Ser Glu Thr Thr Thr Thr Ser Ala Thr Thr Pro Thr Glu Ser
        290                 295                 300

tcc acg tct ggt gaa aca acc acg acg tct gcc aca aca cca acc gaa    959
Ser Thr Ser Gly Glu Thr Thr Thr Thr Ser Ala Thr Thr Pro Thr Glu
    305                 310                 315

cct tcc aca aag cct act tct acg gaa act ccc gca aca aaa cca ccg   1007
Pro Ser Thr Lys Pro Thr Ser Thr Glu Thr Pro Ala Thr Lys Pro Pro
320                 325                 330                 335

caa gaa ata cca tgc aaa caa caa ggt ccg ctg atg caa gat cca cac   1055
Gln Glu Ile Pro Cys Lys Gln Gln Gly Pro Leu Met Gln Asp Pro His
                340                 345                 350

gat tgt cac gca tat tac aca tgt cta gaa att gga tca tta ccg aaa   1103
Asp Cys His Ala Tyr Tyr Thr Cys Leu Glu Ile Gly Ser Leu Pro Lys
            355                 360                 365

cat ttt aat tgt aat aaa ggt gct tat ttc aat aca gtc aaa tta aaa   1151
His Phe Asn Cys Asn Lys Gly Ala Tyr Phe Asn Thr Val Lys Leu Lys
        370                 375                 380

tgc gtg aaa gga aat tgc gaa aat agc aca gaa att cct ctt cct gag   1199
Cys Val Lys Gly Asn Cys Glu Asn Ser Thr Glu Ile Pro Leu Pro Glu
    385                 390                 395

ctt cca gac att tgc gat gaa gta gga cct ttg gtg caa gat cca aac   1247
Leu Pro Asp Ile Cys Asp Glu Val Gly Pro Leu Val Gln Asp Pro Asn
400                 405                 410                 415

gat tgc cgc aag tat tat tca tgc gtc acg att gga aaa gaa cct gaa   1295
Asp Cys Arg Lys Tyr Tyr Ser Cys Val Thr Ile Gly Lys Glu Pro Glu
                420                 425                 430

cat ttt acg tgc aat aaa ggg gcg tat ttt gat cga gaa aga tta cgg   1343
His Phe Thr Cys Asn Lys Gly Ala Tyr Phe Asp Arg Glu Arg Leu Arg
            435                 440                 445

tgt gtc aga gga tct tgt taacaaatat tgttatataa caaagttcaa         1391
Cys Val Arg Gly Ser Cys
        450

tctttaatta ttatttagaa gaatttgaaa atgtatattt aatgtttttt aataaaatag 1451

tttattggca atttnaaaaa aaaaaa                                      1477
```

<210> 1883
<211> 453

<212> PRT
<213> Ctenocephalides felis

<400> 1883

```
Met Ile Lys Ser Val Ile Ile Val Ala Asn Phe Trp Leu Phe Val Ala
1               5                   10                  15

Leu Gln Ser Ile Asp Asn Arg Gln Thr Asn Asn Cys Ser Glu Ile Gly
            20                  25                  30

Val Asp Gly Phe Phe Cys Leu Asn Cys Ser Val Thr Ala Phe Cys Gly
        35                  40                  45

Arg Gly Pro Thr Gly Glu Phe Asn Thr Val Ser Thr Ser Pro Cys Ser
    50                  55                  60

Ser Gly Glu Val Cys Ser Thr Trp Ala Gly Arg Cys Ser Ala Asp Pro
65                  70                  75                  80

Ser Pro Cys Phe Ser Glu Gly Ser Ile Thr Cys Thr Gly Pro Gly Val
                85                  90                  95

Phe Pro Asp Pro Tyr Asp Cys Gln Arg Tyr His Glu Cys Lys Thr Ala
            100                 105                 110

Asn Glu Ser Ser Lys Pro Val Glu Cys Gly Gly Tyr Lys Ala Tyr Asn
        115                 120                 125

Val Ile Glu Asn Asn Cys Ser Leu Asn Met Asn His Gln Ser Cys Lys
    130                 135                 140

Arg Leu Gln Phe His Cys Asp Thr Ile Gly Asp Glu Asn Ala Trp Pro
145                 150                 155                 160

Ser Asn Arg Asn Ile Tyr Tyr Arg Cys Thr Glu Lys Thr Leu Trp Phe
                165                 170                 175

Asn Ser Asn Lys Ile Leu Tyr Pro Leu Leu Tyr Arg Cys Asp Glu Ser
            180                 185                 190

Glu Ile Tyr Asp Ala Val Gln Arg Val Cys Val Arg Asp Glu Thr Thr
        195                 200                 205

Thr Thr Pro Ala Thr Thr Pro Thr Glu Ser Ser Thr Ser Ser Glu Thr
    210                 215                 220
```

```
Thr Thr Thr Ser Ala Thr Thr Ser Thr Glu Ser Ser Thr Ser Ser Glu
225                 230                 235                 240

Thr Thr Thr Thr Ser Ala Thr Thr Pro Thr Glu Ser Ser Thr Ser Ser
                245                 250                 255

Glu Thr Thr Thr Thr Ser Ala Thr Thr Pro Thr Glu Ser Ser Thr Ser
            260                 265                 270

Ser Glu Thr Thr Thr Thr Ser Ala Thr Thr Pro Thr Glu Ser Ser Thr
        275                 280                 285

Ser Ser Glu Thr Thr Thr Thr Ser Ala Thr Thr Pro Thr Glu Ser Ser
    290                 295                 300

Thr Ser Gly Glu Thr Thr Thr Thr Ser Ala Thr Thr Pro Thr Glu Pro
305                 310                 315                 320

Ser Thr Lys Pro Thr Ser Thr Glu Thr Pro Ala Thr Lys Pro Pro Gln
                325                 330                 335

Glu Ile Pro Cys Lys Gln Gln Gly Pro Leu Met Gln Asp Pro His Asp
            340                 345                 350

Cys His Ala Tyr Tyr Thr Cys Leu Glu Ile Gly Ser Leu Pro Lys His
        355                 360                 365

Phe Asn Cys Asn Lys Gly Ala Tyr Phe Asn Thr Val Lys Leu Lys Cys
    370                 375                 380

Val Lys Gly Asn Cys Glu Asn Ser Thr Glu Ile Pro Leu Pro Glu Leu
385                 390                 395                 400

Pro Asp Ile Cys Asp Glu Val Gly Pro Leu Val Gln Asp Pro Asn Asp
                405                 410                 415

Cys Arg Lys Tyr Tyr Ser Cys Val Thr Ile Gly Lys Glu Pro Glu His
            420                 425                 430

Phe Thr Cys Asn Lys Gly Ala Tyr Phe Asp Arg Glu Arg Leu Arg Cys
        435                 440                 445

Val Arg Gly Ser Cys
    450
```

<210> 1884
<211> 1477
<212> DNA
<213> Ctenocephalides felis

<400> 1884

ttttttttt tnaaattgcc aataaactat tttattaaaa aacattaaat atacattttc 60
aaattcttct aaataataat taaagattga actttgttat ataacaatat ttgttaacaa 120
gatcctctga cacaccgtaa tctttctcga tcaaaatacg cccctttatt gcacgtaaaa 180
tgttcaggtt cttttccaat cgtgacgcat gaataatact tgcggcaatc gtttggatct 240
tgcaccaaag gtcctacttc atcgcaaatg tctggaagct caggaagagg aatttctgtg 300
ctattttcgc aatttccttt cacgcatttt aatttgactg tattgaaata agcaccttta 360
ttacaattaa aatgtttcgg taatgatcca atttctagac atgtgtaata tgcgtgacaa 420
tcgtgtggat cttgcatcag cggaccttgt tgtttgcatg gtatttcttg cggtggtttt 480
gttgcgggag tttccgtaga agtaggcttt gtggaaggtt cggttggtgt tgtggcagac 540
gtcgtggttg tttcaccaga cgtggaagat tcggttggtg ttgtggcaga cgtcgtggtt 600
gtttcactag acgtggaaga ttcggttggt gttgtggcag acgtcgtggt tgtttcacta 660
gacgtggaag attcggttgg tgttgtggca gacgtcgtgg ttgtttcact agacgtggaa 720
gattcggttg gtgttgtggc agacgtcgtg gttgtttcac tagacgtgga agattcggtt 780
gatgttgtgg cagacgtcgt ggttgtttca ctagacgtgg aagattcggt tggcgttgtg 840
gcaggcgtcg tggtggtttc atctcttacg caaactctct gcactgcatc atatatctca 900
ctctcatcac accgatataa taaaggatat aatatttgt tgctattgaa ccacaaggtt 960
ttttcggtgc acctataata tatatttcta ttgctcggcc aagcattttc atctcctata 1020
gtatcacaat gaaattgtaa gcgtttacac gattgatgat tcatgttcag gctacaatta 1080
ttttctataa cattataagc cttgtaaccc ccacactcga caggcttaga tgattcattt 1140
gcagttttac attcatgata acgctgacaa tcatacggat caggaaaaac tcctggaccg 1200
gtgcaagtta tagaaccttc cgagaaacat gggcttggat ctgcagaaca tctacccgcc 1260
caggtactgc aaacttcacc agaactacat ggacttgtag acacggtgtt gaattcaccc 1320
gtaggtcctc taccacaaaa agctgtgacg ctgcagttga gacaaaaaaa accatcaaca 1380
ccaatctcag agcagttgtt agtctgccga ttatcaatcg attgcaaggc tacgaatagc 1440
caaaagttgg ccacaattat aactgacttg atcattg 1477

<210> 1885
<211> 1359
<212> DNA
<213> Ctenocephalides felis

<400> 1885

atgatcaagt cagttataat tgtggccaac ttttggctat tcgtagcctt gcaatcgatt 60
gataatcggc agactaacaa ctgctctgag attggtgttg atggtttttt ttgtctcaac 120
tgcagcgtca cagctttttg tggtagagga cctacgggtg aattcaacac cgtgtctaca 180
agtccatgta gttctggtga agtttgcagt acctgggcgg gtagatgttc tgcagatcca 240
agcccatgtt tctcggaagg ttctataact tgcaccggtc caggagtttt tcctgatccg 300
tatgattgtc agcgttatca tgaatgtaaa actgcaaatg aatcatctaa gcctgtcgag 360
tgtgggggtt acaaggctta taatgttata gaaaataatt gtagcctgaa catgaatcat 420
caatcgtgta aacgcttaca atttcattgt gatactatag gagatgaaaa tgcttggccg 480
agcaatagaa atatatatta taggtgcacc gaaaaaacct tgtggttcaa tagcaacaaa 540
atattatatc ctttattata tcggtgtgat gagagtgaga tatatgatgc agtgcagaga 600

```
gtttgcgtaa gagatgaaac caccacgacg cctgccacaa cgccaaccga atcttccacg 660
tctagtgaaa caaccacgac gtctgccaca acatcaaccg aatcttccac gtctagtgaa 720
acaaccacga cgtctgccac aacaccaacc gaatcttcca cgtctagtga aacaaccacg 780
acgtctgcca caacaccaac cgaatcttcc acgtctagtg aaacaaccac gacgtctgcc 840
acaacaccaa ccgaatcttc cacgtctagt gaaacaacca cgacgtctgc cacaacacca 900
accgaatctt ccacgtctgg tgaaacaacc acgacgtctg ccacaacacc aaccgaacct 960
tccacaaagc ctacttctac ggaaactccc gcaacaaaac caccgcaaga aataccatgc 1020
aaacaacaag gtccgctgat gcaagatcca cacgattgtc acgcatatta cacatgtcta 1080
gaaattggat cattaccgaa acattttaat tgtaataaag gtgcttattt caatacagtc 1140
aaattaaaat gcgtgaaagg aaattgcgaa aatagcacag aaattcctct tcctgagctt 1200
ccagacattt gcgatgaagt aggacctttg gtgcaagatc caaacgattg ccgcaagtat 1260
tattcatgcg tcacgattgg aaaagaacct gaacatttta cgtgcaataa aggggcgtat 1320
tttgatcgag aaagattacg gtgtgtcaga ggatcttgt                       1359
```

<210> 1886
<211> 1359
<212> DNA
<213> Ctenocephalides felis

<400> 1886

```
acaagatcct ctgacacacc gtaatctttc tcgatcaaaa tacgcccctt tattgcacgt 60
aaaatgttca ggttcttttc caatcgtgac gcatgaataa tacttgcggc aatcgtttgg 120
atcttgcacc aaaggtccta cttcatcgca aatgtctgga agctcaggaa gaggaatttc 180
tgtgctattt tcgcaatttc ctttcacgca ttttaatttg actgtattga aataagcacc 240
tttattacaa ttaaaatgtt tcggtaatga tccaatttct agacatgtgt aatatgcgtg 300
acaatcgtgt ggatcttgca tcagcggacc ttgttgtttg catggtattt cttgcggtgg 360
ttttgttgcg ggagtttccg tagaagtagg ctttgtggaa ggttcggttg gtgttgtggc 420
agacgtcgtg gttgtttcac cagacgtgga agattcggtt ggtgttgtgg cagacgtcgt 480
ggttgtttca ctagacgtgg aagattcggt tggtgttgtg gcagacgtcg tggttgtttc 540
actagacgtg gaagattcgg ttggtgttgt ggcagacgtc gtggttgttt cactagacgt 600
ggaagattcg gttggtgttg tggcagacgt cgtggttgtt tcactagacg tggaagattc 660
ggttgatgtt gtggcagacg tcgtggttgt ttcactagac gtggaagatt cggttggcgt 720
tgtggcaggc gtcgtggtgg tttcatctct tacgcaaact ctctgcactg catcatatat 780
ctcactctca tcacaccgat ataataaagg atataatatt ttgttgctat tgaaccacaa 840
ggtttttttcg gtgcacctat aatatatatt tctattgctc ggccaagcat tttcatctcc 900
tatagtatca caatgaaatt gtaagcgttt acacgattga tgattcatgt tcaggctaca 960
attattttct ataacattat aagccttgta acccccacac tcgacaggct tagatgattc 1020
atttgcagtt ttacattcat gataacgctg acaatcatac ggatcaggaa aaactcctgg 1080
accggtgcaa gttatagaac cttccgagaa acatgggctt ggatctgcag aacatctacc 1140
cgcccaggta ctgcaaactt caccagaact acatggactt gtagacacgg tgttgaattc 1200
acccgtaggt cctctaccac aaaaagctgt gacgctgcag ttgagacaaa aaaaaccatc 1260
aacaccaatc tcagagcagt tgttagtctg ccgattatca atcgattgca aggctacgaa 1320
tagccaaaag ttggccacaa ttataactga cttgatcat                        1359
```

<210> 1887
<211> 406

<212> DNA
<213> Ctenocephalides felis

<220>
<221> CDS
<222> (20)..(262)

<400> 1887

```
gttaatttaa aataacaaa atg aaa gga aca tta tta ata tta tca tgt ctt  52
                     Met Lys Gly Thr Leu Leu Ile Leu Ser Cys Leu
                     1               5                   10

gtg atc atg ata agt gcc gaa tat gct gac gta gat gtg tgc caa gat   100
Val Ile Met Ile Ser Ala Glu Tyr Ala Asp Val Asp Val Cys Gln Asp
            15                  20                  25

ttg gac gat gga act ttt ctt gct gat tca aac aat tgc caa aat ttc   148
Leu Asp Asp Gly Thr Phe Leu Ala Asp Ser Asn Asn Cys Gln Asn Phe
        30                  35                  40

ttc att tgt gat gga ggc cga gct tgg aaa atg tat tgt cca gga tca   196
Phe Ile Cys Asp Gly Gly Arg Ala Trp Lys Met Tyr Cys Pro Gly Ser
    45                  50                  55

ctt tta tgg aat gat cac gaa gga aca tgt gat tac gca caa aat gta   244
Leu Leu Trp Asn Asp His Glu Gly Thr Cys Asp Tyr Ala Gln Asn Val
60                  65                  70                  75

gaa tgt tac caa cca gaa taaaacattt taatatctga cagcgatttt          292
Glu Cys Tyr Gln Pro Glu
                80

ctgaaactat atttcatact actgttataa taaatttatc ttcattgctc tcctcctata 352

aatttattcc gttttaataa aatcaatata aagacaaaaa aaaaaaaaaa aaaa       406
```

<210> 1888
<211> 81
<212> PRT
<213> Ctenocephalides felis

<400> 1888

```
Met Lys Gly Thr Leu Leu Ile Leu Ser Cys Leu Val Ile Met Ile Ser
1               5                   10                  15

Ala Glu Tyr Ala Asp Val Asp Val Cys Gln Asp Leu Asp Asp Gly Thr
            20                  25                  30
```

Phe Leu Ala Asp Ser Asn Asn Cys Gln Asn Phe Phe Ile Cys Asp Gly
35 40 45

Gly Arg Ala Trp Lys Met Tyr Cys Pro Gly Ser Leu Leu Trp Asn Asp
50 55 60

His Glu Gly Thr Cys Asp Tyr Ala Gln Asn Val Glu Cys Tyr Gln Pro
65 70 75 80

Glu

<210> 1889
<211> 406
<212> DNA
<213> Ctenocephalides felis

<400> 1889

```
ttttttttt ttttttttg tctttatatt gattttatta aaacggaata aatttatagg 60
aggagagcaa tgaagataaa tttattataa cagtagtatg aaatatagtt tcagaaaatc 120
gctgtcagat attaaaatgt tttattctgg ttggtaacat tctacatttt gtgcgtaatc 180
acatgttcct tcgtgatcat tccataaaag tgatcctgga caatacattt tccaagctcg 240
gcctccatca caaatgaaga aattttggca attgtttgaa tcagcaagaa aagttccatc 300
gtccaaatct tggcacacat ctacgtcagc atattcggca cttatcatga tcacaagaca 360
tgataatatt aataatgttc ctttcatttt gttattttaa attaac 406
```

<210> 1890
<211> 243
<212> DNA
<213> Ctenocephalides felis

<400> 1890

```
atgaaaggaa cattattaat attatcatgt cttgtgatca tgataagtgc cgaatatgct 60
gacgtagatg tgtgccaaga tttggacgat ggaacttttc ttgctgattc aaacaattgc 120
caaaatttct tcatttgtga tggaggccga gcttggaaaa tgtattgtcc aggatcactt 180
ttatggaatg atcacgaagg aacatgtgat tacgcacaaa atgtagaatg ttaccaacca 240
gaa 243
```

<210> 1891
<211> 243
<212> DNA
<213> Ctenocephalides felis

<400> 1891

ttctggttgg taacattcta cattttgtgc gtaatcacat gttccttcgt gatcattcca 60
taaaagtgat cctggacaat acattttcca agctcggcct ccatcacaaa tgaagaaatt 120
ttggcaattg tttgaatcag caagaaaagt tccatcgtcc aaatcttggc acacatctac 180
gtcagcatat tcggcactta tcatgatcac aagacatgat aatattaata atgttccttt 240
cat 243

<210> 1892
<211> 974
<212> DNA
<213> Ctenocephalides felis

<400> 1892

ttaatgtcga cggaacgcct ttaacagtaa ataaagaagt atttgcatca ttggatgagc 60
ccgcaccagg agtagtacct actcctgaac ctacacctgt accgaaaccc gagcaaaaat 120
gtaaaaaagt aaaatttagt tgcgtgaatt cgtgcagttc acccgaaatg cagtattgtc 180
cggaaatagg agcagatccg gttaaggaat cctgtagccc agatcaagtg tgcgctgatc 240
aaagtggata tctacagtgc accactaaag aaagtacagt ctgcaaagta caaggtttca 300
aatgtccgtc accatcgaga ttttatccaa atataaatga ttgtcaaagc tattattatt 360
gtgacgaaaa tagtatagga acccaatatt attgccccgc aaattttgca tatgatccgt 420
tacgtcataa ttgcggacct atggctctgg gcacaaaatg ctatacagtt acatgtcctg 480
cacagcctaa ggtgcttccg tacattggtg ataaatcatt gtacgtcgta tgtatggccg 540
gaagaggaac cgtattgcaa tgcgaagaac ccgccgagtt ttccccaagg agcgaaacct 600
gtgtcgggca atgccgagca cgtggaaaat ttgctttcaa gaacgacgca acatgccgga 660
agttcttcac gtgtttacgt cctaaaggag agccagttcc tgatcaatgt ccgattggaa 720
cagtatttaa ccaagctact caaagctgca acacaggaac ttgcgagagg aaacctaaat 780
tatattaata tattgatgaa gtattcaaca aaagaaacta tacaaaatat gtactttgtt 840
ttactttatg tgttatataa aaaaatatta tggttgaaca caggctcgca aatatgataa 900
ggcatttaag aattttacaa tttagatttt tttaaatcca tgaatatatt tgttctaatc 960
aaaaaaaaaa aaaa 974

<210> 1893
<211> 974
<212> DNA
<213> Ctenocephalides felis

<400> 1893

ttaatgtcga cggaacgcct ttaacagtaa ataaagaagt atttgcatca ttggatgagc 60
ccgcaccagg agtagtacct actcctgaac ctacacctgt accgaaaccc gagcaaaaat 120
gtaaaaaagt aaaatttagt tgcgtgaatt cgtgcagttc acccgaaatg cagtattgtc 180
cggaaatagg agcagatccg gttaaggaat cctgtagccc agatcaagtg tgcgctgatc 240
aaagtggata tctacagtgc accactaaag aaagtacagt ctgcaaagta caaggtttca 300
aatgtccgtc accatcgaga ttttatccaa atataaatga ttgtcaaagc tattattatt 360
gtgacgaaaa tagtatagga acccaatatt attgccccgc aaattttgca tatgatccgt 420
tacgtcataa ttgcggacct atggctctgg gcacaaaatg ctatacagtt acatgtcctg 480
cacagcctaa ggtgcttccg tacattggtg ataaatcatt gtacgtcgta tgtatggccg 540

gaagaggaac cgtattgcaa tgcgaagaac ccgccgagtt ttccccaagg agcgaaacct 600
gtgtcgggca atgccgagca cgtggaaaat ttgctttcaa gaacgacgca acatgccgga 660
agttcttcac gtgtttacgt cctaaaggag agccagttcc tgatcaatgt ccgattggaa 720
cagtatttaa ccaagctact caaagctgca acacaggaac ttgcgagagg aaacctaaat 780
tatattaata tattgatgaa gtattcaaca aaagaaacta tacaaaatat gtactttgtt 840
ttactttatg tgttatataa aaaaatatta tggttgaaca caggctcgca aatatgataa 900
ggcatttaag aattttacaa tttagatttt tttaaatcca tgaatatatt tgttctaatc 960
aaaaaaaaaa aaaa 974

<210> 1894
<211> 1043
<212> DNA
<213> Ctenocephalides felis

<400> 1894

tgttttatat cacattggtt tttattagtt ttgtggcgtt atctgtcgtt accgcttatg 60
atggtgagtt taatgtcgac ggaacgcctt taacagtaaa taaagaagta tttgcatcat 120
tggatgagcc cgcaccagga gtagtaccta ctcctgaacc tacacctgta ccgaaacccg 180
agcaaaaatg taaaaaagta aaatttagtt gcgtgaattc gtgcagttca cccgaaatgc 240
agtattgtcc ggaaatagga gcagatccgg ttaaggaatc ctgtagccca gatcaagtgt 300
gcgctgatca aagtggatat ctacagtgca ccactaaaga aagtacagtc tgcaaagtac 360
aaggtttcaa atgtccgtca ccatcgagat tttatccaaa tataaatgat tgtcaaagct 420
attattattg tgacgaaaat agtataggaa cccaatatta ttgccccgca aattttgcat 480
atgatccgtt acgtcataat tgcggaccta tggctctggg cacaaaatgc tatacagtta 540
catgtcctgc acagcctaag gtgcttccgt acattggtga taaatcattg tacgtcgtat 600
gtatggccgg aagaggaacc gtattgcaat gcgaagaacc cgccgagttt tccccaagga 660
gcgaaacctg tgtcgggcaa tgccgagcac gtggaaaatt tgctttcaag aacgacgcaa 720
catgccggaa gttcttcacg tgtttacgtc ctaaaggaga gccagttcct gatcaatgtc 780
cgattggaac agtatttaac caagctactc aaagctgcaa cacaggaact tgcgagagga 840
aacctaaatt atattaatat attgatgaag tattcaacaa aagaaactat acaaaatatg 900
tactttgttt tactttatgt gttatataaa aaaatattat ggttgaacac aggctcgcaa 960
atatgataag gcatttaaga attttacaat ttagattttt ttaaatccat gaatatattt 1020
gttctaatca aaaaaaaaaa aaa 1043

<210> 1895
<211> 1043
<212> DNA
<213> Ctenocephalides felis

<400> 1895

tttttttttt ttttgattag aacaaatata ttcatggatt taaaaaaatc taaattgtaa 60
aattcttaaa tgccttatca tatttgcgag cctgtgttca accataatat ttttttatat 120
aacacataaa gtaaaacaaa gtacatattt tgtatagttt cttttgttga atacttcatc 180
aatatattaa tataatttag gtttcctctc gcaagttcct gtgttgcagc tttgagtagc 240
ttggttaaat actgttccaa tcggacattg atcaggaact ggctctcctt taggacgtaa 300
acacgtgaag aacttccggc atgttgcgtc gttcttgaaa gcaaattttc cacgtgctcg 360

```
gcattgcccg acacaggttt cgctccttgg ggaaaactcg gcgggttctt cgcattgcaa 420
tacggttcct cttccggcca tacatacgac gtacaatgat ttatcaccaa tgtacggaag 480
caccttaggc tgtgcaggac atgtaactgt atagcatttt gtgcccagag ccataggtcc 540
gcaattatga cgtaacggat catatgcaaa atttgcgggg caataatatt gggttcctat 600
actattttcg tcacaataat aatagctttg acaatcattt atatttggat aaaatctcga 660
tggtgacgga catttgaaac cttgtacttt gcagactgta ctttctttag tggtgcactg 720
tagatatcca ctttgatcag cgcacacttg atctgggcta caggattcct taaccggatc 780
tgctcctatt tccggacaat actgcatttc gggtgaactg cacgaattca cgcaactaaa 840
ttttactttt ttacattttt gctcgggttt cggtacaggt gtaggttcag gagtaggtac 900
tactcctggt gcgggctcat ccaatgatgc aaatacttct ttatttactg ttaaaggcgt 960
tccgtcgaca ttaaactcac catcataagc ggtaacgaca gataacgcca caaaactaat 1020
aaaaaccaat gtgatataaa aca                                        1043
```

<210> 1896
<211> 1062
<212> DNA
<213> Ctenocephalides felis

<220>
<221> CDS
<222> (19)..(873)

<400> 1896

```
gtaacatatt tattaaga atg ttt tat atc aca ttg gtt ttt att agt ttt    51
                    Met Phe Tyr Ile Thr Leu Val Phe Ile Ser Phe
                     1               5                  10

gtg gcg tta tct gtc gtt acc gct tat gat ggt gag ttt aat gtc gac    99
Val Ala Leu Ser Val Val Thr Ala Tyr Asp Gly Glu Phe Asn Val Asp
            15                  20                  25

gga acg cct tta aca gta aat aaa gaa gta ttt gca tca ttg gat gag   147
Gly Thr Pro Leu Thr Val Asn Lys Glu Val Phe Ala Ser Leu Asp Glu
        30                  35                  40

ccc gca cca gga gta gta cct act cct gaa cct aca cct gta ccg aaa   195
Pro Ala Pro Gly Val Val Pro Thr Pro Glu Pro Thr Pro Val Pro Lys
    45                  50                  55

ccc gag caa aaa tgt aaa aaa gta aaa ttt agt tgc gtg aat tcg tgc   243
Pro Glu Gln Lys Cys Lys Lys Val Lys Phe Ser Cys Val Asn Ser Cys
60                  65                  70                  75

agt tca ccc gaa atg cag tat tgt ccg gaa ata gga gca gat ccg gtt   291
Ser Ser Pro Glu Met Gln Tyr Cys Pro Glu Ile Gly Ala Asp Pro Val
                80                  85                  90
```

```
aag gaa tcc tgt agc cca gat caa gtg tgc gct gat caa agt gga tat     339
Lys Glu Ser Cys Ser Pro Asp Gln Val Cys Ala Asp Gln Ser Gly Tyr
            95                  100                 105

cta cag tgc acc act aaa gaa agt aca gtc tgc aaa gta caa ggt ttc     387
Leu Gln Cys Thr Thr Lys Glu Ser Thr Val Cys Lys Val Gln Gly Phe
        110                 115                 120

aaa tgt ccg tca cca tcg aga ttt tat cca aat ata aat gat tgt caa     435
Lys Cys Pro Ser Pro Ser Arg Phe Tyr Pro Asn Ile Asn Asp Cys Gln
    125                 130                 135

agc tat tat tat tgt gac gaa aat agt ata gga acc caa tat tat tgc     483
Ser Tyr Tyr Tyr Cys Asp Glu Asn Ser Ile Gly Thr Gln Tyr Tyr Cys
140                 145                 150                 155

ccc gca aat ttt gca tat gat ccg tta cgt cat aat tgc gga cct atg     531
Pro Ala Asn Phe Ala Tyr Asp Pro Leu Arg His Asn Cys Gly Pro Met
                160                 165                 170

gct ctg ggc aca aaa tgc tat aca gtt aca tgt cct gca cag cct aag     579
Ala Leu Gly Thr Lys Cys Tyr Thr Val Thr Cys Pro Ala Gln Pro Lys
            175                 180                 185

gtg ctt ccg tac att ggt gat aaa tca ttg tac gtc gta tgt atg gcc     627
Val Leu Pro Tyr Ile Gly Asp Lys Ser Leu Tyr Val Val Cys Met Ala
        190                 195                 200

gga aga gga acc gta ttg caa tgc gaa gaa ccc gcc gag ttt tcc cca     675
Gly Arg Gly Thr Val Leu Gln Cys Glu Glu Pro Ala Glu Phe Ser Pro
    205                 210                 215

agg agc gaa acc tgt gtc ggg caa tgc cga gca cgt gga aaa ttt gct     723
Arg Ser Glu Thr Cys Val Gly Gln Cys Arg Ala Arg Gly Lys Phe Ala
220                 225                 230                 235

ttc aag aac gac gca aca tgc cgg aag ttc ttc acg tgt tta cgt cct     771
Phe Lys Asn Asp Ala Thr Cys Arg Lys Phe Phe Thr Cys Leu Arg Pro
                240                 245                 250

aaa gga gag cca gtt cct gat caa tgt ccg att gga aca gta ttt aac     819
Lys Gly Glu Pro Val Pro Asp Gln Cys Pro Ile Gly Thr Val Phe Asn
            255                 260                 265

caa gct act caa agc tgc aac aca gga act tgc gag agg aaa cct aaa     867
Gln Ala Thr Gln Ser Cys Asn Thr Gly Thr Cys Glu Arg Lys Pro Lys
        270                 275                 280
```

```
tta tat taatatattg atgaagtatt caacaaaaga aactatacaa aatatgtact    923
Leu Tyr
    285

ttgttttact ttatgtgtta tataaaaaaa tattatggtt gaacacaggc tcgcaaatat 983

gataaggcat ttaagaattt tacaatttag atttttttaa atccatgaat atatttgttc 1043

taatcaaaaa aaaaaaaaa                                              1062
```

<210> 1897
<211> 285
<212> PRT
<213> Ctenocephalides felis

<400> 1897

```
Met Phe Tyr Ile Thr Leu Val Phe Ile Ser Phe Val Ala Leu Ser Val
1               5                   10                  15

Val Thr Ala Tyr Asp Gly Glu Phe Asn Val Asp Gly Thr Pro Leu Thr
            20                  25                  30

Val Asn Lys Glu Val Phe Ala Ser Leu Asp Glu Pro Ala Pro Gly Val
        35                  40                  45

Val Pro Thr Pro Glu Pro Thr Pro Val Pro Lys Pro Glu Gln Lys Cys
    50                  55                  60

Lys Lys Val Lys Phe Ser Cys Val Asn Ser Cys Ser Ser Pro Glu Met
65                  70                  75                  80

Gln Tyr Cys Pro Glu Ile Gly Ala Asp Pro Val Lys Glu Ser Cys Ser
                85                  90                  95

Pro Asp Gln Val Cys Ala Asp Gln Ser Gly Tyr Leu Gln Cys Thr Thr
            100                 105                 110

Lys Glu Ser Thr Val Cys Lys Val Gln Gly Phe Lys Cys Pro Ser Pro
        115                 120                 125

Ser Arg Phe Tyr Pro Asn Ile Asn Asp Cys Gln Ser Tyr Tyr Tyr Cys
    130                 135                 140

Asp Glu Asn Ser Ile Gly Thr Gln Tyr Tyr Cys Pro Ala Asn Phe Ala
145                 150                 155                 160

Tyr Asp Pro Leu Arg His Asn Cys Gly Pro Met Ala Leu Gly Thr Lys
```

```
                165                 170                 175

Cys Tyr Thr Val Thr Cys Pro Ala Gln Pro Lys Val Leu Pro Tyr Ile
            180                 185                 190

Gly Asp Lys Ser Leu Tyr Val Val Cys Met Ala Gly Arg Gly Thr Val
        195                 200                 205

Leu Gln Cys Glu Glu Pro Ala Glu Phe Ser Pro Arg Ser Glu Thr Cys
    210                 215                 220

Val Gly Gln Cys Arg Ala Arg Gly Lys Phe Ala Phe Lys Asn Asp Ala
225                 230                 235                 240

Thr Cys Arg Lys Phe Phe Thr Cys Leu Arg Pro Lys Gly Glu Pro Val
                245                 250                 255

Pro Asp Gln Cys Pro Ile Gly Thr Val Phe Asn Gln Ala Thr Gln Ser
            260                 265                 270

Cys Asn Thr Gly Thr Cys Glu Arg Lys Pro Lys Leu Tyr
        275                 280                 285
```

<210> 1898
<211> 1062
<212> DNA
<213> Ctenocephalides felis

<400> 1898

```
gtaacatatt tattaagaat gttttatatc acattggttt ttattagttt tgtggcgtta 60
tctgtcgtta ccgcttatga tggtgagttt aatgtcgacg gaacgccttt aacagtaaat 120
aaagaagtat ttgcatcatt ggatgagccc gcaccaggag tagtacctac tcctgaacct 180
acacctgtac cgaaacccga gcaaaaatgt aaaaaagtaa aatttagttg cgtgaattcg 240
tgcagttcac ccgaaatgca gtattgtccg gaaataggag cagatccggt taaggaatcc 300
tgtagcccag atcaagtgtg cgctgatcaa agtggatatc tacagtgcac cactaaagaa 360
agtacagtct gcaaagtaca aggtttcaaa tgtccgtcac catcgagatt ttatccaaat 420
ataaatgatt gtcaaagcta ttattattgt gacgaaaata gtataggaac ccaatattat 480
tgccccgcaa attttgcata tgatccgtta cgtcataatt gcggacctat ggctctgggc 540
acaaaatgct atacagttac atgtcctgca cagcctaagg tgcttccgta cattggtgat 600
aaatcattgt acgtcgtatg tatggccgga agaggaaccg tattgcaatg cgaagaaccc 660
gccgagtttt ccccaaggag cgaaacctgt gtcgggcaat gccgagcacg tggaaaattt 720
gctttcaaga acgacgcaac atgccggaag ttcttcacgt gtttacgtcc taaaggagag 780
ccagttcctg atcaatgtcc gattggaaca gtatttaacc aagctactca aagctgcaac 840
acaggaactt gcgagaggaa acctaaatta tattaatata ttgatgaagt attcaacaaa 900
agaaactata caaaatatgt actttgtttt actttatgtg ttatataaaa aatattatg 960
gttgaacaca ggctcgcaaa tatgataagg catttaagaa ttttacaatt tagatttttt 1020

taaatccatg aatatatttg ttctaatcaa aaaaaaaaa aa                    1062
```

<210> 1899
<211> 855
<212> DNA
<213> Ctenocephalides felis

<400> 1899

```
atgttttata tcacattggt ttttattagt tttgtggcgt tatctgtcgt taccgcttat 60
gatggtgagt ttaatgtcga cggaacgcct ttaacagtaa ataaagaagt atttgcatca 120
ttggatgagc ccgcaccagg agtagtacct actcctgaac ctacacctgt accgaaaccc 180
gagcaaaaat gtaaaaaagt aaaatttagt tgcgtgaatt cgtgcagttc acccgaaatg 240
cagtattgtc cggaaatagg agcagatccg gttaaggaat cctgtagccc agatcaagtg 300
tgcgctgatc aaagtggata tctacagtgc accactaaag aaagtacagt ctgcaaagta 360
caaggtttca aatgtccgtc accatcgaga ttttatccaa atataaatga ttgtcaaagc 420
tattattatt gtgacgaaaa tagtatagga acccaatatt attgccccgc aaattttgca 480
tatgatccgt tacgtcataa ttgcggacct atggctctgg gcacaaaatg ctatacagtt 540
acatgtcctg cacagcctaa ggtgcttccg tacattggtg ataaatcatt gtacgtcgta 600
tgtatggccg gaagaggaac cgtattgcaa tgcgaagaac ccgccgagtt ttccccaagg 660
agcgaaacct gtgtcgggca atgccgagca cgtggaaaat ttgctttcaa gaacgacgca 720
acatgccgga agttcttcac gtgtttacgt cctaaaggag agccagttcc tgatcaatgt 780
ccgattggaa cagtatttaa ccaagctact caaagctgca acacaggaac ttgcgagagg 840
aaacctaaat tatat                                                  855
```

<210> 1900
<211> 855
<212> DNA
<213> Ctenocephalides felis

<400> 1900

```
atataattta ggtttcctct cgcaagttcc tgtgttgcag ctttgagtag cttggttaaa 60
tactgttcca atcggacatt gatcaggaac tggctctcct ttaggacgta aacacgtgaa 120
gaacttccgg catgttgcgt cgttcttgaa agcaaatttt ccacgtgctc ggcattgccc 180
gacacaggtt tcgctccttg gggaaaactc ggcgggttct tcgcattgca atacggttcc 240
tcttccggcc atacatacga cgtacaatga tttatcacca atgtacggaa gcaccttagg 300
ctgtgcagga catgtaactg tatagcattt tgtgcccaga gccataggtc cgcaattatg 360
acgtaacgga tcatatgcaa aatttgcggg gcaataatat tgggttccta tactattttc 420
gtcacaataa taatagcttt gacaatcatt tatatttgga taaaatctcg atggtgacgg 480
acatttgaaa ccttgtactt tgcagactgt actttcttta gtggtgcact gtagatatcc 540
actttgatca gcgcacactt gatctgggct acaggattcc ttaaccggat ctgctcctat 600
ttccggacaa tactgcattt cgggtgaact gcacgaattc acgcaactaa attttacttt 660
tttacatttt tgctcgggtt tcggtacagg tgtaggttca ggagtaggta ctactcctgg 720
tgcgggctca tccaatgatg caaatacttc tttatttact gttaaaggcg ttccgtcgac 780
attaaactca ccatcataag cggtaacgac agataacgcc acaaaactaa taaaaaccaa 840
tgtgatataa aacat                                                  855
```

<210> 1901
<211> 1875
<212> DNA
<213> Ctenocephalides felis

<220>

<221> CDS
<222> (44)..(1633)

<400> 1901

```
acaaaacggt ttaattgaat ctgtagtttg gaaattaata aat atg gac agt aac     55
                                                Met Asp Ser Asn
                                                1

acg ggg att caa ata ata gct tcc aaa gaa cca aaa cca agg cag ttt    103
Thr Gly Ile Gln Ile Ile Ala Ser Lys Glu Pro Lys Pro Arg Gln Phe
5                   10                  15                  20

gaa gat gcg ttg gca ctc aca ggt ttt gga aaa ttc aat tac ctt ctt    151
Glu Asp Ala Leu Ala Leu Thr Gly Phe Gly Lys Phe Asn Tyr Leu Leu
                25                  30                  35

ctg gtc gtg agt gga tgc gta tta gta tgt gtt ttg atg gaa act ctt    199
Leu Val Val Ser Gly Cys Val Leu Val Cys Val Leu Met Glu Thr Leu
            40                  45                  50

gga atg agt ttt gtc gtt cct tca gca caa tgt gat ctg gaa tta aca    247
Gly Met Ser Phe Val Val Pro Ser Ala Gln Cys Asp Leu Glu Leu Thr
        55                  60                  65

aca aaa caa aaa gga ata tta agc gct ata gct ttt ata ggt att ata    295
Thr Lys Gln Lys Gly Ile Leu Ser Ala Ile Ala Phe Ile Gly Ile Ile
    70                  75                  80

agc agt tca cat tta tgg gga ttt tta gcc gat acg aga ggg agg cgg    343
Ser Ser Ser His Leu Trp Gly Phe Leu Ala Asp Thr Arg Gly Arg Arg
85                  90                  95                  100

aaa gtg att atg cct aca ctt ctt ctt gca ttt ttt tgt acc ttg gca    391
Lys Val Ile Met Pro Thr Leu Leu Leu Ala Phe Phe Cys Thr Leu Ala
                105                 110                 115

tct agt ttt gta aat tca gtt tgg ctg ttt att ttg ctg cga tat ttc    439
Ser Ser Phe Val Asn Ser Val Trp Leu Phe Ile Leu Leu Arg Tyr Phe
            120                 125                 130

aat gga ttt ttc gta tct gga gga agt gca aca ata tat gca tat tta    487
```

```
Asn Gly Phe Phe Val Ser Gly Gly Ser Ala Thr Ile Tyr Ala Tyr Leu
        135                 140                 145

gga gaa ttt cat aat cct agg cat cgc agc agg gct att atg gga gcg    535
Gly Glu Phe His Asn Pro Arg His Arg Ser Arg Ala Ile Met Gly Ala
    150                 155                 160

tca agc atc ttc gga ttt gcg tgt ctt gca tta ccg acg gtt gca tgg    583
Ser Ser Ile Phe Gly Phe Ala Cys Leu Ala Leu Pro Thr Val Ala Trp
165                 170                 175                 180

tta att ata aat cag aaa tgg tca ttc tat att gac ttt ttg gga tat    631
Leu Ile Ile Asn Gln Lys Trp Ser Phe Tyr Ile Asp Phe Leu Gly Tyr
                185                 190                 195

aca tac aag ccc tgg agg ttg tat atg gtt gca tgt ggt ttg cca tca    679
Thr Tyr Lys Pro Trp Arg Leu Tyr Met Val Ala Cys Gly Leu Pro Ser
            200                 205                 210

ctg ctt tgt tgt ttt gct ttg tgg aaa tta cca gaa agt ccc aaa ttt    727
Leu Leu Cys Cys Phe Ala Leu Trp Lys Leu Pro Glu Ser Pro Lys Phe
        215                 220                 225

ttg atg aat cag gga aga aac gaa gaa gct cgt caa att att gcc aaa    775
Leu Met Asn Gln Gly Arg Asn Glu Glu Ala Arg Gln Ile Ile Ala Lys
    230                 235                 240

atg tat aga att aat act ggt aaa cca gaa agt gaa ttc ccc gta tca    823
Met Tyr Arg Ile Asn Thr Gly Lys Pro Glu Ser Glu Phe Pro Val Ser
245                 250                 255                 260

tca atc tta gat gaa tat cca gga gtg gat ggt gaa aat aca aat aaa    871
Ser Ile Leu Asp Glu Tyr Pro Gly Val Asp Gly Glu Asn Thr Asn Lys
                265                 270                 275

aca aag aaa tca ttt tta aga act gta tgg gat caa act gct ccg ctg    919
Thr Lys Lys Ser Phe Leu Arg Thr Val Trp Asp Gln Thr Ala Pro Leu
            280                 285                 290

ttt atg ggt gag cac atg aaa aaa aca ctc att gca tgt act ctg caa    967
Phe Met Gly Glu His Met Lys Lys Thr Leu Ile Ala Cys Thr Leu Gln
        295                 300                 305

ttc gga ata ttt gcc aca tct aac ggc atg tac atg tgg ttt ccc gat    1015
Phe Gly Ile Phe Ala Thr Ser Asn Gly Met Tyr Met Trp Phe Pro Asp
    310                 315                 320

atc ata agt aaa atg aca gaa ttt caa aac gct cat cca gga gta cca    1063
```

```
Ile Ile Ser Lys Met Thr Glu Phe Gln Asn Ala His Pro Gly Val Pro
325                 330                 335                 340

agt aca ata tgc tac gtt gtc caa aat tca tca atg ctt agg gcg gac     1111
Ser Thr Ile Cys Tyr Val Val Gln Asn Ser Ser Met Leu Arg Ala Asp
                345                 350                 355

gac ttc att gat act acg aat agt acg acc gag tgt aaa gac aca atg     1159
Asp Phe Ile Asp Thr Thr Asn Ser Thr Thr Glu Cys Lys Asp Thr Met
            360                 365                 370

gaa gaa cag gca ttt atg cat tct tta atg ttg gaa gct gga tat gcg     1207
Glu Glu Gln Ala Phe Met His Ser Leu Met Leu Glu Ala Gly Tyr Ala
        375                 380                 385

att gga ttt ccc ata ata ggt gct att att aac tct gtg gga aag ctt     1255
Ile Gly Phe Pro Ile Ile Gly Ala Ile Ile Asn Ser Val Gly Lys Leu
    390                 395                 400

cca att ctt gta ttc gtg atg gtc tca tgc ggg att tgt gga ata att     1303
Pro Ile Leu Val Phe Val Met Val Ser Cys Gly Ile Cys Gly Ile Ile
405                 410                 415                 420

tgt gca ttt att gaa cat tta aca ata gca tca tat tta tat ttg tgg     1351
Cys Ala Phe Ile Glu His Leu Thr Ile Ala Ser Tyr Leu Tyr Leu Trp
                425                 430                 435

ttg ctg gtt tgc gga att gct gtg aca gtg gta aac gcg gca tta gta     1399
Leu Leu Val Cys Gly Ile Ala Val Thr Val Val Asn Ala Ala Leu Val
            440                 445                 450

gat ctg tac cct aca caa ctc agg gca atg gca gta tgt ata tct tta     1447
Asp Leu Tyr Pro Thr Gln Leu Arg Ala Met Ala Val Cys Ile Ser Leu
        455                 460                 465

atg atg ggt cga tta gga agt gtt gtt gga agc aat gtt gta gga ata     1495
Met Met Gly Arg Leu Gly Ser Val Val Gly Ser Asn Val Val Gly Ile
    470                 475                 480

att cta gat tat aat tgc gat tta aca ttt tta ata tca gga aca tct     1543
Ile Leu Asp Tyr Asn Cys Asp Leu Thr Phe Leu Ile Ser Gly Thr Ser
485                 490                 495                 500

ctg ata gca tgt gga gtg att gca ttc ttt ata ccg aac att tat cag     1591
Leu Ile Ala Cys Gly Val Ile Ala Phe Phe Ile Pro Asn Ile Tyr Gln
                505                 510                 515

aag caa gtg gat aga aga acg agc att gct tca tat gga cca             1633
```

```
Lys Gln Val Asp Arg Arg Thr Ser Ile Ala Ser Tyr Gly Pro
            520                 525                 530

taaccatatt tccgtcgtgt gttgtgaaat gcaattatac tttgagaaag tattatcaat 1693

tacaaagaaa attcagagtt gctactgaat cattttctaa aacatcgaca tgaaaattaa 1753

taactcttta ttgttattag attcgatgta agatatatgt acatactcat aggtaaaaaa 1813

tgttaatgcc gtcaatgttc aaaattataa agagaataaa taaatacgtt aaaaaaaaaa 1873

aa                                                                1875
```

<210> 1902
<211> 530
<212> PRT
<213> Ctenocephalides felis

<400> 1902

```
Met Asp Ser Asn Thr Gly Ile Gln Ile Ile Ala Ser Lys Glu Pro Lys
1               5                   10                  15

Pro Arg Gln Phe Glu Asp Ala Leu Ala Leu Thr Gly Phe Gly Lys Phe
            20                  25                  30

Asn Tyr Leu Leu Leu Val Val Ser Gly Cys Val Leu Val Cys Val Leu
        35                  40                  45

Met Glu Thr Leu Gly Met Ser Phe Val Val Pro Ser Ala Gln Cys Asp
    50                  55                  60

Leu Glu Leu Thr Thr Lys Gln Lys Gly Ile Leu Ser Ala Ile Ala Phe
65                  70                  75                  80

Ile Gly Ile Ile Ser Ser Ser His Leu Trp Gly Phe Leu Ala Asp Thr
                85                  90                  95

Arg Gly Arg Arg Lys Val Ile Met Pro Thr Leu Leu Leu Ala Phe Phe
            100                 105                 110

Cys Thr Leu Ala Ser Ser Phe Val Asn Ser Val Trp Leu Phe Ile Leu
        115                 120                 125

Leu Arg Tyr Phe Asn Gly Phe Phe Val Ser Gly Gly Ser Ala Thr Ile
    130                 135                 140

Tyr Ala Tyr Leu Gly Glu Phe His Asn Pro Arg His Arg Ser Arg Ala
```

145 150 155 160

Ile Met Gly Ala Ser Ser Ile Phe Gly Phe Ala Cys Leu Ala Leu Pro
165 170 175

Thr Val Ala Trp Leu Ile Ile Asn Gln Lys Trp Ser Phe Tyr Ile Asp
180 185 190

Phe Leu Gly Tyr Thr Tyr Lys Pro Trp Arg Leu Tyr Met Val Ala Cys
195 200 205

Gly Leu Pro Ser Leu Leu Cys Cys Phe Ala Leu Trp Lys Leu Pro Glu
210 215 220

Ser Pro Lys Phe Leu Met Asn Gln Gly Arg Asn Glu Glu Ala Arg Gln
225 230 235 240

Ile Ile Ala Lys Met Tyr Arg Ile Asn Thr Gly Lys Pro Glu Ser Glu
245 250 255

Phe Pro Val Ser Ser Ile Leu Asp Glu Tyr Pro Gly Val Asp Gly Glu
260 265 270

Asn Thr Asn Lys Thr Lys Lys Ser Phe Leu Arg Thr Val Trp Asp Gln
275 280 285

Thr Ala Pro Leu Phe Met Gly Glu His Met Lys Lys Thr Leu Ile Ala
290 295 300

Cys Thr Leu Gln Phe Gly Ile Phe Ala Thr Ser Asn Gly Met Tyr Met
305 310 315 320

Trp Phe Pro Asp Ile Ile Ser Lys Met Thr Glu Phe Gln Asn Ala His
325 330 335

Pro Gly Val Pro Ser Thr Ile Cys Tyr Val Val Gln Asn Ser Ser Met
340 345 350

Leu Arg Ala Asp Asp Phe Ile Asp Thr Thr Asn Ser Thr Thr Glu Cys
355 360 365

Lys Asp Thr Met Glu Glu Gln Ala Phe Met His Ser Leu Met Leu Glu
370 375 380

Ala Gly Tyr Ala Ile Gly Phe Pro Ile Ile Gly Ala Ile Ile Asn Ser
385 390 395 400

Val Gly Lys Leu Pro Ile Leu Val Phe Val Met Val Ser Cys Gly Ile

405 410 415

Cys Gly Ile Ile Cys Ala Phe Ile Glu His Leu Thr Ile Ala Ser Tyr
420 425 430

Leu Tyr Leu Trp Leu Leu Val Cys Gly Ile Ala Val Thr Val Val Asn
435 440 445

Ala Ala Leu Val Asp Leu Tyr Pro Thr Gln Leu Arg Ala Met Ala Val
450 455 460

Cys Ile Ser Leu Met Met Gly Arg Leu Gly Ser Val Val Gly Ser Asn
465 470 475 480

Val Val Gly Ile Ile Leu Asp Tyr Asn Cys Asp Leu Thr Phe Leu Ile
485 490 495

Ser Gly Thr Ser Leu Ile Ala Cys Gly Val Ile Ala Phe Phe Ile Pro
500 505 510

Asn Ile Tyr Gln Lys Gln Val Asp Arg Arg Thr Ser Ile Ala Ser Tyr
515 520 525

Gly Pro
530

<210> 1903
<211> 1875
<212> DNA
<213> Ctenocephalides felis

<400> 1903

tttttttttt ttaacgtatt tatttattct ctttataatt ttgaacattg acggcattaa 60
catttttttac ctatgagtat gtacatatat cttacatcga atctaataac aataaagagt 120
tattaatttt catgtcgatg ttttagaaaa tgattcagta gcaactctga attttctttg 180
taattgataa tactttctca aagtataatt gcatttcaca acacacgacg gaaatatggt 240
tatggtccat atgaagcaat gctcgttctt ctatccactt gcttctgata aatgttcggt 300
ataaagaatg caatcactcc acatgctatc agagatgttc ctgatattaa aaatgttaaa 360
tcgcaattat aatctagaat tattcctaca acattgcttc caacaacact tcctaatcga 420
cccatcatta aagatataca tactgccatt gccctgagtt gtgtagggta cagatctact 480
aatgccgcgt ttaccactgt cacagcaatt ccgcaaacca gcaaccacaa atataaatat 540
gatgctattg ttaaatgttc aataaatgca caaattattc cacaaatccc gcatgagacc 600
atcacgaata caagaattgg aagctttccc acagagttaa taatagcacc tattatggga 660
aatccaatcg catatccagc ttccaacatt aaagaatgca taaatgcctg ttcttccatt 720
gtgtctttac actcggtcgt actattcgta gtatcaatga agtcgtccgc cctaagcatt 780
gatgaatttt ggacaacgta gcatattgta cttggtactc ctggatgagc gttttgaaat 840

tctgtcattt tacttatgat atcgggaaac cacatgtaca tgccgttaga tgtggcaaat 900
attccgaatt gcagagtaca tgcaatgagt gtttttttca tgtgctcacc cataaacagc 960
ggagcagttt gatcccatac agttcttaaa aatgatttct ttgttttatt tgtattttca 1020
ccatccactc ctggatattc atctaagatt gatgatacgg ggaattcact ttctggttta 1080
ccagtattaa ttctatacat tttggcaata atttgacgag cttcttcgtt tcttccctga 1140
ttcatcaaaa atttgggact ttctggtaat ttccacaaag caaaacaaca aagcagtgat 1200
ggcaaaccac atgcaaccat atacaacctc cagggcttgt atgtatatcc caaaaagtca 1260
atatagaatg accatttctg atttataatt aaccatgcaa ccgtcggtaa tgcaagacac 1320
gcaaatccga agatgcttga cgctcccata atagccctgc tgcgatgcct aggattatga 1380
aattctccta aatatgcata tattgttgca cttcctccag atacgaaaaa tccattgaaa 1440
tatcgcagca aaataaacag ccaaactgaa tttacaaaac tagatgccaa ggtacaaaaa 1500
aatgcaagaa gaagtgtagg cataatcact ttccgcctcc ctctcgtatc ggctaaaaat 1560
ccccataaat gtgaactgct tataatacct ataaaagcta tagcgcttaa tattcctttt 1620
tgttttgttg ttaattccag atcacattgt gctgaaggaa cgacaaaact cattccaaga 1680
gtttccatca aaacacatac taatacgcat ccactcacga ccagaagaag gtaattgaat 1740
tttccaaaac ctgtgagtgc caacgcatct tcaaactgcc ttggttttgg ttctttggaa 1800
gctattattt gaatccccgt gttactgtcc atatttatta atttccaaac tacagattca 1860
attaaaccgt tttgt 1875

<210> 1904
<211> 1590
<212> DNA
<213> Ctenocephalides felis

<400> 1904

atggacagta acacggggat tcaaataata gcttccaaag aaccaaaacc aaggcagttt 60
gaagatgcgt tggcactcac aggttttgga aaattcaatt accttcttct ggtcgtgagt 120
ggatgcgtat tagtatgtgt tttgatggaa actcttggaa tgagttttgt cgttccttca 180
gcacaatgtg atctggaatt aacaacaaaa caaaaaggaa tattaagcgc tatagctttt 240
ataggtatta taagcagttc acatttatgg ggatttttag ccgatacgag agggaggcgg 300
aaagtgatta tgcctacact tcttcttgca ttttttttgta ccttggcatc tagttttgta 360
aattcagttt ggctgtttat tttgctgcga tatttcaatg gatttttcgt atctggagga 420
agtgcaacaa tatatgcata tttaggagaa tttcataatc ctaggcatcg cagcagggct 480
attatgggag cgtcaagcat cttcggattt gcgtgtcttg cattaccgac ggttgcatgg 540
ttaattataa atcagaaatg gtcattctat attgactttt tgggatatac atacaagccc 600
tggaggttgt atatggttgc atgtggtttg ccatcactgc tttgttgttt tgctttgtgg 660
aaattaccag aaagtcccaa atttttgatg aatcagggaa gaaacgaaga agctcgtcaa 720
attattgcca aaatgtatag aattaatact ggtaaaccag aaagtgaatt ccccgtatca 780
tcaatcttag atgaatatcc aggagtggat ggtgaaaata caaataaaac aaagaaatca 840
tttttaagaa ctgtatggga tcaaactgct ccgctgttta tgggtgagca catgaaaaaa 900
acactcattg catgtactct gcaattcgga atatttgcca catctaacgg catgtacatg 960
tggtttcccg atatcataag taaaatgaca gaatttcaaa acgctcatcc aggagtacca 1020
agtacaatat gctacgttgt ccaaaattca tcaatgctta gggcggacga cttcattgat 1080
actacgaata gtacgaccga gtgtaaagac acaatggaag aacaggcatt tatgcattct 1140
ttaatgttgg aagctggata tgcgattgga tttcccataa taggtgctat tattaactct 1200
gtgggaaagc ttccaattct tgtattcgtg atggtctcat gcgggatttg tggaataatt 1260
tgtgcattta ttgaacattt aacaatagca tcatatttat atttgtggtt gctggtttgc 1320

ggaattgctg tgacagtggt aaacgcggca ttagtagatc tgtaccctac acaactcagg 1380
gcaatggcag tatgtatatc tttaatgatg ggtcgattag gaagtgttgt tggaagcaat 1440
gttgtaggaa taattctaga ttataattgc gatttaacat ttttaatatc aggaacatct 1500
ctgatagcat gtggagtgat tgcattcttt ataccgaaca tttatcagaa gcaagtggat 1560
agaagaacga gcattgcttc atatggacca 1590

<210> 1905
<211> 1590
<212> DNA
<213> Ctenocephalides felis

<400> 1905

tggtccatat gaagcaatgc tcgttcttct atccacttgc ttctgataaa tgttcggtat 60
aaagaatgca atcactccac atgctatcag agatgttcct gatattaaaa atgttaaatc 120
gcaattataa tctagaatta ttcctacaac attgcttcca acaacacttc ctaatcgacc 180
catcattaaa gatatacata ctgccattgc cctgagttgt gtagggtaca gatctactaa 240
tgccgcgttt accactgtca cagcaattcc gcaaaccagc aaccacaaat ataaatatga 300
tgctattgtt aaatgttcaa taaatgcaca aattattcca caaatcccgc atgagaccat 360
cacgaataca agaattggaa gctttcccac agagttaata atagcaccta ttatgggaaa 420
tccaatcgca tatccagctt ccaacattaa agaatgcata aatgcctgtt cttccattgt 480
gtctttacac tcggtcgtac tattcgtagt atcaatgaag tcgtccgccc taagcattga 540
tgaattttgg acaacgtagc atattgtact tggtactcct ggatgagcgt tttgaaattc 600
tgtcatttta cttatgatat cgggaaacca catgtacatg ccgttagatg tggcaaatat 660
tccgaattgc agagtacatg caatgagtgt ttttttcatg tgctcaccca taaacagcgg 720
agcagtttga tcccatacag ttcttaaaaa tgatttcttt gttttatttg tattttcacc 780
atccactcct ggatattcat ctaagattga tgatacgggg aattcacttt ctggtttacc 840
agtattaatt ctatacattt tggcaataat ttgacgagct tcttcgtttc ttccctgatt 900
catcaaaaat ttgggacttt ctggtaattt ccacaaagca aaacaacaaa gcagtgatgg 960
caaaccacat gcaaccatat acaacctcca gggcttgtat gtatatccca aaaagtcaat 1020
atagaatgac catttctgat ttataattaa ccatgcaacc gtcggtaatg caagacacgc 1080
aaatccgaag atgcttgacg ctcccataat agccctgctg cgatgcctag gattatgaaa 1140
ttctcctaaa tatgcatata ttgttgcact tcctccagat acgaaaaatc cattgaaata 1200
tcgcagcaaa ataaacagcc aaactgaatt tacaaaacta gatgccaagg tacaaaaaaa 1260
tgcaagaaga agtgtaggca taatcacttt ccgcctccct ctcgtatcgg ctaaaaatcc 1320
ccataaatgt gaactgctta taatacctat aaaagctata gcgcttaata ttcctttttg 1380
ttttgttgtt aattccagat cacattgtgc tgaaggaacg acaaaactca ttccaagagt 1440
ttccatcaaa acacatacta atacgcatcc actcacgacc agaagaaggt aattgaattt 1500
tccaaaacct gtgagtgcca acgcatcttc aaactgcctt ggttttggtt ctttggaagc 1560
tattatttga atccccgtgt tactgtccat 1590

<210> 1906
<211> 381
<212> DNA
<213> Ctenocephalides felis

<400> 1906

acgttatatc gtgcccttaa tggcagcagc tatggcttcc aaatgggttg gtgatgcttt 60
gggcagacag ggtatatatg atgcccatat acagcttaat ggatatccat tcttggacag 120
taaagatgaa tttgcacata catctttagc tgcagatgtc atgcaaccca agaggaatga 180
aacattaagt gtaatcactc aagactcgat gactgtggat gatgttgaag gtttactgaa 240
agaaactgag cacaatggat atccagttgt tgtttccaga gaatctcagt atcttgttgg 300
atttgttttg aggagggact taaatctagc catagccaat gctagacgca tgatcgatgg 360
gataacagga caaagtttgg t 381

<210> 1907
<211> 381
<212> DNA
<213> Ctenocephalides felis

<400> 1907

accaaacttt gtcctgttat cccatcgatc atgcgtctag cattggctat ggctagattt 60
aagtccctcc tcaaaacaaa tccaacaaga tactgagatt ctctggaaac aacaactgga 120
tatccattgt gctcagtttc tttcagtaaa ccttcaacat catccacagt catcgagtct 180
tgagtgatta cacttaatgt ttcattcctc ttgggttgca tgacatctgc agctaaagat 240
gtatgtgcaa attcatcttt actgtccaag aatggatatc cattaagctg tatatgggca 300
tcatatatac cctgtctgcc caaagcatca ccaacccatt tggaagccat agctgctgcc 360
attaagggca cgatataacg t 381

<210> 1908
<211> 2191
<212> DNA
<213> Ctenocephalides felis

<400> 1908

atttttgctt ctttggcagc ctctttggtg cgcatgtttg caccttatgc ttgtgggtca 60
ggtataccag agattaaaac cattctgagt ggtttcatca tcagaggata tcttggaaaa 120
tggacattga ttattaaaag tgtaggaatc atgttgtctg tatcagctgg attgagtttg 180
ggtaaagaag gtcctatggt acacattgcc agctgtatag gtaatatatt gtcttattta 240
tttcctaaat atggtcggaa tgaagcaaag aaacgagaga ttttatcagc agctgcagca 300
gctggtgtat ctgttgcatt tggagcacct attggaggtg tgcttttcag tttggaagag 360
gtgagctact atttcccatt gaagacctta tggagatcat tcttctgtgc tttgatagca 420
gctttcatat tgcgatccat aaatccattt ggaaatgagc actctgtcct tttctatgtg 480
gaatacaata aaccttggat attttttgaa ctgatacctt tcataggcct tggaataatt 540
ggtggtgttg tagcaacgct gtttataaaa gctaatttgt actggtgtcg ctaccgtaaa 600
ttttctaaac taggacagta ccccgttgca gaagttttag ttgttgctgt tgcaacagca 660
gtgattgctt atcctaatcc ttacaccagg atgaatacta gtcaactgat ttatttacta 720
ttcagccaat gcgggatttc caattctgat cctttgtgtg attacaatcg caatttcact 780
gatgttaaat cagctataga aatagcagca gctggtcctg gtgtctacca ggctgtgtgg 840
ttgctcctga ttgctttggt actgaaattg ggaatgactg tatttacctt tggtatgaaa 900
gtaccatgtg gtctgtttat cccaagttta tgcctaggag ctattatggg tagaattgtg 960
ggcattggaa ttgaacaatt ggcttactat tatccaaaat tatggttctt ttctggtgaa 1020
tgctcaactg gagacaattg catcacaccg ggcctgtatg ctatggtggg cgctgcagct 1080

```
gttttaggtg gtgtcactag aatgacagtt tctctggtgg taataatgtt tgaactgact 1140
ggtggtgtac gttatatcgt gcccttaatg gcagcagcta tggcttccaa atgggttggt 1200
gatgctttgg gcagacaggg tatatatgat gcccatatac agcttaatgg atatccattc 1260
ttggacagta aagatgaatt tgcacataca tctttagctg cagatgtcat gcaacccaag 1320
aggaatgaaa cattaagtgt aatcactcaa gactcgatga ctgtggatga tgttgaaggt 1380
ttactgaaag aaactgagca caatggatat ccagttgttg tttccagaga atctcagtat 1440
cttgttggat ttgttttgag gagggactta aatctagcca tagccaatgc tagacgcatg 1500
atcgatggga taacaggaca aagtttggta cttttcataa atggccctac agtgcaaagt 1560
ttaggacctc cacctttgaa actaaagaaa atattagata tggctccaat aacagtgact 1620
gatcaaacac caatggaaac tgtggtggat atgtttagaa aactaggttt acgtcagaca 1680
ttagtcacac acaatggcg tttgctcggt gttataacta aaaaagatgt tttacgacat 1740
gtaaaacaaa tggataatga agatcctaat agtatacttt ttaattaata tttacatata 1800
tgtattaaat aataaatgtt aagttgcata aaaatccata aaactatgta ggtgtacatt 1860
ttaatggcta ttcttcatat acctaaatga aaagactaaa aagaaaccaa tttttaaaat 1920
atttaggtat ttgatataat acatatttat tttaatatga atagtatttt gtgtaattta 1980
attgaaggca tcacaaatta aattgtgtaa ttgttataac agaatcagag tgtatatatt 2040
agatttatcg attcgaaatt tgtacaaaat gtatctcagt ttttttaat gaggacttag 2100
aaaattttat tgttaattga ttattttttt agtattattt tagtatttgt aactgattat 2160
taaacgattg attataaaaa aaaaaaaaaa a                                2191
```

<210> 1909
<211> 2191
<212> DNA
<213> Ctenocephalides felis

<400> 1909

```
tttttttttt ttttttataa tcaatcgttt aataatcagt tacaaatact aaaataatac 60
taaaaaaata atcaattaac aataaaattt tctaagtcct cattaaaaaa aactgagata 120
cattttgtac aaatttcgaa tcgataaatc taatatatac actctgattc tgttataaca 180
attacacaat ttaatttgtg atgccttcaa ttaaattaca caaaatacta ttcatattaa 240
aataaatatg tattatatca aatacctaaa tattttaaaa attggtttct tttagtctt 300
ttcatttagg tatatgaaga atagccatta aaatgtacac ctacatagtt ttatggattt 360
ttatgcaact taacatttat tatttaatac atatatgtaa atattaatta aaaagtatac 420
tattaggatc ttcattatcc atttgtttta catgtcgtaa aacatctttt ttagttataa 480
caccgagcaa acgcccattg tgtgtgacta atgtctgacg taaacctagt tttctaaaca 540
tatccaccac agtttccatt ggtgtttgat cagtcactgt tattggagcc atatctaata 600
ttttctttag tttcaaaggt ggaggtccta aactttgcac tgtagggcca tttatgaaaa 660
gtaccaaact ttgtcctgtt atcccatcga tcatgcgtct agcattggct atggctagat 720
ttaagtccct cctcaaaaca aatccaacaa gatactgaga ttctctggaa acaacaactg 780
gatatccatt gtgctcagtt tctttcagta aaccttcaac atcatccaca gtcatcgagt 840
cttgagtgat tacacttaat gtttcattcc tcttgggttg catgacatct gcagctaaag 900
atgtatgtgc aaattcatct ttactgtcca agaatggata tccattaagc tgtatatggg 960
catcatatat accctgtctg cccaaagcat caccaaccca tttggaagcc atagctgctg 1020
ccattaaggg cacgatataa cgtacaccac cagtcagttc aaacattatt accaccagag 1080
aaactgtcat tctagtgaca ccacctaaaa cagctgcagc gcccaccata gcatacaggc 1140
ccggtgtgat gcaattgtct ccagttgagc attcaccaga aaagaaccat aattttggat 1200
aatagtaagc caattgttca attccaatgc ccacaattct acccataata gctcctaggc 1260
```

```
ataaacttgg gataaacaga ccacatggta ctttcatacc aaaggtaaat acagtcattc 1320
ccaatttcag taccaaagca atcaggagca accacacagc ctggtagaca ccaggaccag 1380
ctgctgctat ttctatagct gatttaacat cagtgaaatt gcgattgtaa tcacacaaag 1440
gatcagaatt ggaaatcccg cattggctga atagtaaata aatcagttga ctagtattca 1500
tcctggtgta aggattagga taagcaatca ctgctgttgc aacagcaaca actaaaactt 1560
ctgcaacggg gtactgtcct agtttagaaa atttacggta gcgacaccag tacaaattag 1620
cttttataaa cagcgttgct acaacaccac caattattcc aaggcctatg aaaggtatca 1680
gttcaaaaaa tatccaaggt ttattgtatt ccacatagaa aaggacagag tgctcatttc 1740
caaatggatt tatggatcgc aatatgaaag ctgctatcaa agcacagaag aatgatctcc 1800
ataaggtctt caatgggaaa tagtagctca cctcttccaa actgaaaagc acacctccaa 1860
taggtgctcc aaatgcaaca gatacaccag ctgctgcagc tgctgataaa atctctcgtt 1920
tctttgcttc attccgacca tatttaggaa ataaataaga caatatatta cctatacagc 1980
tggcaatgtg taccatagga ccttctttac ccaaactcaa tccagctgat acagacaaca 2040
tgattcctac acttttaata atcaatgtcc attttccaag atatcctctg atgatgaaac 2100
cactcagaat ggttttaatc tctggtatac ctgacccaca agcataaggt gcaaacatgc 2160
gcaccaaaga ggctgccaaa gaagcaaaaa t                                2191
```

<210> 1910
<211> 1968
<212> DNA
<213> Ctenocephalides felis

<400> 1910

```
gtggtggcat atcatttgct ggtatgcaag ctgaaagcga tgatattcct gggattgggc 60
aatatgatga tttccatacg atagattggc aacgtgatat agccagagat cgaatgagac 120
atcgatatat tgttaaaaaa cgacaagact ctatactaga cctgataaag ggtgcccatg 180
atgcctggtc aggttgggtg tgtgttctcc tagtcgggct ggtgacagga gctattgcag 240
gcgtcataga tatcggagca agttggatga cggatttaaa gaacggtgtt tgcccacaag 300
cattctggtt gaatagagaa caatgttgtt ggtcattgaa tgaaacaacc tttgatgatg 360
gaaattgctc acaatggctg acttggcctg aggttttcgg acaacctaga actggggcgg 420
gggcttacat aattgcttat ttgttttata ttatttgggc attgattttt gcttctttgg 480
cagcctcttt ggtgcgcatg tttgcacctt atgcttgtgg gtcaggtata ccagagatta 540
aaaccattct gagtggtttc atcatcagag gatatcttgg aaaatggaca ttgattatta 600
aaagtgtagg aatcatgttg tctgtatcag ctggattgag tttgggtaaa gaaggtccta 660
tggtacacat tgccagctgt ataggtaata tattgtctta tttatttcct aaatatggtc 720
ggaatgaagc aaagaaacga gagattttat cagcagctgc agcagctggt gtatctgttg 780
catttggagc acctattgga ggtgtgcttt tcagtttgga agaggtgagc tactatttcc 840
cattgaagac cttatggaga tcattcttct gtgctttgat agcagctttc atattgcgat 900
ccataaatcc atttggaaat gagcactctg tccttttcta tgtggaatac aataaacctt 960
ggatattttt tgaactgata cctttcatag gccttggaat aattggtggt gttgtagcaa 1020
cgctgtttat aaaagctaat ttgtactggt gtcgctaccg taaattttct aaactaggac 1080
agtaccccgt tgcagaagtt ttagttgttg ctgttgcaac agcagtgatt gcttatccta 1140
atccttacac caggatgaat actagtcaac tgatttattt actattcagc caatgcggga 1200
tttccaattc tgatcctttg tgtgattaca atcgcaattt cactgatgtt aaatcagcta 1260
tagaaatagc agcagctggt cctggtgtct accaggctgt gtggttgctc ctgattgctt 1320
tggtactgaa attgggaatg actgtattta cctttggtat gaaagtacca tgtggtctgt 1380
ttatcccaag tttatgccta ggagctatta tgggtagaat tgtgggcatt ggaattgaac 1440
```

```
aattggctta ctattatcca aaattatggt tcttttctgg tgaatgctca actggagaca 1500
attgcatcac accgggcctg tatgctatgg tgggcgctgc agctgtttta ggtggtgtca 1560
ctagaatgac agtttctctg gtggtaataa tgtttgaact gactggtggt gtacgttata 1620
tcgtgccctt aatggcagca gctatggctt ccaaatgggt tggtgatgct ttgggcagac 1680
agggtatata tgatgcccat atacagctta atggatatcc attcttggac agtaaagatg 1740
aatttgcaca tacatcttta gctgcagatg tcatgcaacc caagaggaat gaaacattaa 1800
gtgtaatcac tcaagactcg atgactgtgg atgatgttga aggtttactg aaagaaactg 1860
agcacaatgg atatccagtt gttgtttcca gagaatctca gtatcttgtt ggatttgttt 1920
tgaggaggga cttaaatcta gccatagcca atgctagacg catgatcg             1968
```

<210> 1911
<211> 1968
<212> DNA
<213> Ctenocephalides felis

<400> 1911

```
cgatcatgcg tctagcattg gctatggcta gatttaagtc cctcctcaaa acaaatccaa 60
caagatactg agattctctg gaaacaacaa ctggatatcc attgtgctca gtttctttca 120
gtaaaccttc aacatcatcc acagtcatcg agtcttgagt gattacactt aatgtttcat 180
tcctcttggg ttgcatgaca tctgcagcta aagatgtatg tgcaaattca tctttactgt 240
ccaagaatgg atatccatta agctgtatat gggcatcata tataccctgt ctgcccaaag 300
catcaccaac ccatttggaa gccatagctg ctgccattaa gggcacgata taacgtacac 360
caccagtcag ttcaaacatt attaccacca gagaaactgt cattctagtg acaccaccta 420
aaacagctgc agcgcccacc atagcataca ggcccggtgt gatgcaattg tctccagttg 480
agcattcacc agaaaagaac cataattttg gataatagta agccaattgt tcaattccaa 540
tgcccacaat tctacccata atagctccta ggcataaact tgggataaac agaccacatg 600
gtactttcat accaaaggta aatacagtca ttcccaattt cagtaccaaa gcaatcagga 660
gcaaccacac agcctggtag acaccaggac cagctgctgc tatttctata gctgatttaa 720
catcagtgaa attgcgattg taatcacaca aaggatcaga attggaaatc ccgcattggc 780
tgaatagtaa ataaatcagt tgactagtat tcatcctggt gtaaggatta ggataagcaa 840
tcactgctgt tgcaacagca acaactaaaa cttctgcaac ggggtactgt cctagtttag 900
aaaatttacg gtagcgacac cagtacaaat tagcttttat aaacagcgtt gctacaacac 960
caccaattat tccaaggcct atgaaaggta tcagttcaaa aaatatccaa ggtttattgt 1020
attccacata gaaaaggaca gagtgctcat ttccaaatgg atttatggat cgcaatatga 1080
aagctgctat caaagcacag aagaatgatc tccataaggt cttcaatggg aaatagtagc 1140
tcacctcttc caaactgaaa agcacacctc caataggtgc tccaaatgca acagatacac 1200
cagctgctgc agctgctgat aaaatctctc gtttctttgc ttcattccga ccatatttag 1260
gaaataaata agacaatata ttacctatac agctggcaat gtgtaccata ggaccttctt 1320
tacccaaact caatccagct gatacagaca acatgattcc tacactttta ataatcaatg 1380
tccattttcc aagatatcct ctgatgatga aaccactcag aatggtttta atctctggta 1440
tacctgaccc acaagcataa ggtgcaaaca tgcgcaccaa agaggctgcc aaagaagcaa 1500
aaatcaatgc ccaaataata taaaacaaat aagcaattat gtaagccccc gccccagttc 1560
taggttgtcc gaaaacctca ggccaagtca gccattgtga gcaatttcca tcatcaaagg 1620
ttgtttcatt caatgaccaa caacattgtt ctctattcaa ccagaatgct tgtgggcaaa 1680
caccgttctt taaatccgtc atccaacttg ctccgatatc tatgacgcct gcaatagctc 1740
ctgtcaccag cccgactagg agaacacaca cccaacctga ccaggcatca tgggcaccct 1800
ttatcaggtc tagtatagag tcttgtcgtt ttttaacaat atatcgatgt ctcattcgat 1860
```

```
ctctggctat atcacgttgc caatctatcg tatggaaatc atcatattgc ccaatcccag 1920
gaatatcatc gctttcagct tgcataccag caaatgatat gccaccac              1968
```

<210> 1912
<211> 673
<212> DNA
<213> Ctenocephalides felis

<400> 1912

```
tactcactat atggctcgag cggccgcccg ggcagtcgtt tttgttagaa aaagtacaaa 60
aattanttaa tgtaacagtg cttgtgatat aaaaaataat gattcaagtg catcatattt 120
aaatcaatta attttttttaa aatcttagaa atgaatttaa agcagctatg gagaaatatc 180
cattaaaagg aaattctacg atacgtttaa gtggtcctgc ttcttatcaa tcggttacta 240
aaaaaaaacg cccaggagac tccactaatc acatagtgga acgagtggat catcctttga 300
gcggcacgat gccctcggac ggaccccaac agcataatcc agtgtcggac tcgggcgatt 360
tttcgccaga cgaattcggc aaacgtatat ttcctcccgg atccacacag ttgaccctgg 420
atgatgataa tggatcagca aatttggcaa ttaaatttga gggtctttct agtggtggca 480
tatcatttgc tggtatgcaa gctgaaagcg atgatattcc tgggattggg caatatgatg 540
atttccatac gatagattgg caacgtgata tagccagaga tcgaatgaga catcgatata 600
ttgttaaaaa acgacaagac tctatactag acctgataaa gggtgcccat gatgcctggt 660
caggttgggt gtg                                                     673
```

<210> 1913
<211> 673
<212> DNA
<213> Ctenocephalides felis

<400> 1913

```
cacacccaac ctgaccaggc atcatgggca ccctttatca ggtctagtat agagtcttgt 60
cgttttttaa caatatatcg atgtctcatt cgatctctgg ctatatcacg ttgccaatct 120
atcgtatgga aatcatcata ttgcccaatc ccaggaatat catcgctttc agcttgcata 180
ccagcaaatg atatgccacc actagaaaga ccctcaaatt taattgccaa atttgctgat 240
ccattatcat catccagggt caactgtgtg gatccgggag gaaatatacg tttgccgaat 300
tcgtctggcg aaaaatcgcc cgagtccgac actggattat gctgttgggg tccgtccgag 360
ggcatcgtgc cgctcaaagg atgatccact cgttccacta tgtgattagt ggagtctcct 420
gggcgttttt ttttagtaac cgattgataa gaagcaggac cacttaaacg tatcgtagaa 480
tttcctttta atggatattt ctccatagct gctttaaatt catttctaag attttaaaaa 540
aattaattga tttaaatatg atgcacttga atcattattt tttatatcac aagcactgtt 600
acattaanta atttttgtac tttttctaac aaaaacgact gcccgggcgg ccgctcgagc 660
catatagtga gta                                                     673
```

<210> 1914
<211> 3126
<212> DNA
<213> Ctenocephalides felis

<220>
<221> CDS

<222> (168)..(2720)

<400> 1914

```
tactcactat atggctcgag cggccgcccg ggcagtcgtt tttgttagaa aaagtacaaa 60

aattanttaa tgtaacagtg cttgtgatat aaaaaataat gattcaagtg catcatattt 120

aaatcaatta atttttttaa aatcttagaa atgaatttaa agcagct atg gag aaa   176
                                                    Met Glu Lys
                                                    1

tat cca tta aaa gga aat tct acg ata cgt tta agt ggt cct gct tct   224
Tyr Pro Leu Lys Gly Asn Ser Thr Ile Arg Leu Ser Gly Pro Ala Ser
    5                   10                  15

tat caa tcg gtt act aaa aaa aaa cgc cca gga gac tcc act aat cac   272
Tyr Gln Ser Val Thr Lys Lys Lys Arg Pro Gly Asp Ser Thr Asn His
20                  25                  30                  35

ata gtg gaa cga gtg gat cat cct ttg agc ggc acg atg ccc tcg gac   320
Ile Val Glu Arg Val Asp His Pro Leu Ser Gly Thr Met Pro Ser Asp
                40                  45                  50

gga ccc caa cag cat aat cca gtg tcg gac tcg ggc gat ttt tcg cca   368
Gly Pro Gln Gln His Asn Pro Val Ser Asp Ser Gly Asp Phe Ser Pro
            55                  60                  65

gac gaa ttc ggc aaa cgt ata ttt cct ccc gga tcc aca cag ttg acc   416
Asp Glu Phe Gly Lys Arg Ile Phe Pro Pro Gly Ser Thr Gln Leu Thr
        70                  75                  80

ctg gat gat gat aat gga tca gca aat ttg gca att aaa ttt gag ggt   464
Leu Asp Asp Asp Asn Gly Ser Ala Asn Leu Ala Ile Lys Phe Glu Gly
    85                  90                  95

ctt tct agt ggt ggc ata tca ttt gct ggt atg caa gct gaa agc gat   512
Leu Ser Ser Gly Gly Ile Ser Phe Ala Gly Met Gln Ala Glu Ser Asp
100                 105                 110                 115

gat att cct ggg att ggg caa tat gat gat ttc cat acg ata gat tgg   560
Asp Ile Pro Gly Ile Gly Gln Tyr Asp Asp Phe His Thr Ile Asp Trp
                120                 125                 130

caa cgt gat ata gcc aga gat cga atg aga cat cga tat att gtt aaa   608
Gln Arg Asp Ile Ala Arg Asp Arg Met Arg His Arg Tyr Ile Val Lys
```

```
            135                 140                 145

aaa cga caa gac tct ata cta gac ctg ata aag ggt gcc cat gat gcc     656
Lys Arg Gln Asp Ser Ile Leu Asp Leu Ile Lys Gly Ala His Asp Ala
        150                 155                 160

tgg tca ggt tgg gtg tgt gtt ctc cta gtc ggg ctg gtg aca gga gct     704
Trp Ser Gly Trp Val Cys Val Leu Leu Val Gly Leu Val Thr Gly Ala
    165                 170                 175

att gca ggc gtc ata gat atc gga gca agt tgg atg acg gat tta aag     752
Ile Ala Gly Val Ile Asp Ile Gly Ala Ser Trp Met Thr Asp Leu Lys
180                 185                 190                 195

aac ggt gtt tgc cca caa gca ttc tgg ttg aat aga gaa caa tgt tgt     800
Asn Gly Val Cys Pro Gln Ala Phe Trp Leu Asn Arg Glu Gln Cys Cys
                200                 205                 210

tgg tca ttg aat gaa aca acc ttt gat gat gga aat tgc tca caa tgg     848
Trp Ser Leu Asn Glu Thr Thr Phe Asp Asp Gly Asn Cys Ser Gln Trp
            215                 220                 225

ctg act tgg cct gag gtt ttc gga caa cct aga act ggg gcg ggg gct     896
Leu Thr Trp Pro Glu Val Phe Gly Gln Pro Arg Thr Gly Ala Gly Ala
        230                 235                 240

tac ata att gct tat ttg ttt tat att att tgg gca ttg att ttt gct     944
Tyr Ile Ile Ala Tyr Leu Phe Tyr Ile Ile Trp Ala Leu Ile Phe Ala
    245                 250                 255

tct ttg gca gcc tct ttg gtg cgc atg ttt gca cct tat gct tgt ggg     992
Ser Leu Ala Ala Ser Leu Val Arg Met Phe Ala Pro Tyr Ala Cys Gly
260                 265                 270                 275

tca ggt ata cca gag att aaa acc att ctg agt ggt ttc atc atc aga     1040
Ser Gly Ile Pro Glu Ile Lys Thr Ile Leu Ser Gly Phe Ile Ile Arg
                280                 285                 290

gga tat ctt gga aaa tgg aca ttg att att aaa agt gta gga atc atg     1088
Gly Tyr Leu Gly Lys Trp Thr Leu Ile Ile Lys Ser Val Gly Ile Met
            295                 300                 305

ttg tct gta tca gct gga ttg agt ttg ggt aaa gaa ggt cct atg gta     1136
Leu Ser Val Ser Ala Gly Leu Ser Leu Gly Lys Glu Gly Pro Met Val
        310                 315                 320

cac att gcc agc tgt ata ggt aat ata ttg tct tat tta ttt cct aaa     1184
His Ile Ala Ser Cys Ile Gly Asn Ile Leu Ser Tyr Leu Phe Pro Lys
```

```
        325                 330                 335

tat ggt cgg aat gaa gca aag aaa cga gag att tta tca gca gct gca    1232
Tyr Gly Arg Asn Glu Ala Lys Lys Arg Glu Ile Leu Ser Ala Ala Ala
340                 345                 350                 355

gca gct ggt gta tct gtt gca ttt gga gca cct att gga ggt gtg ctt    1280
Ala Ala Gly Val Ser Val Ala Phe Gly Ala Pro Ile Gly Gly Val Leu
                360                 365                 370

ttc agt ttg gaa gag gtg agc tac tat ttc cca ttg aag acc tta tgg    1328
Phe Ser Leu Glu Glu Val Ser Tyr Tyr Phe Pro Leu Lys Thr Leu Trp
            375                 380                 385

aga tca ttc ttc tgt gct ttg ata gca gct ttc ata ttg cga tcc ata    1376
Arg Ser Phe Phe Cys Ala Leu Ile Ala Ala Phe Ile Leu Arg Ser Ile
        390                 395                 400

aat cca ttt gga aat gag cac tct gtc ctt ttc tat gtg gaa tac aat    1424
Asn Pro Phe Gly Asn Glu His Ser Val Leu Phe Tyr Val Glu Tyr Asn
    405                 410                 415

aaa cct tgg ata ttt ttt gaa ctg ata cct ttc ata ggc ctt gga ata    1472
Lys Pro Trp Ile Phe Phe Glu Leu Ile Pro Phe Ile Gly Leu Gly Ile
420                 425                 430                 435

att ggt ggt gtt gta gca acg ctg ttt ata aaa gct aat ttg tac tgg    1520
Ile Gly Gly Val Val Ala Thr Leu Phe Ile Lys Ala Asn Leu Tyr Trp
                440                 445                 450

tgt cgc tac cgt aaa ttt tct aaa cta gga cag tac ccc gtt gca gaa    1568
Cys Arg Tyr Arg Lys Phe Ser Lys Leu Gly Gln Tyr Pro Val Ala Glu
            455                 460                 465

gtt tta gtt gtt gct gtt gca aca gca gtg att gct tat cct aat cct    1616
Val Leu Val Val Ala Val Ala Thr Ala Val Ile Ala Tyr Pro Asn Pro
        470                 475                 480

tac acc agg atg aat act agt caa ctg att tat tta cta ttc agc caa    1664
Tyr Thr Arg Met Asn Thr Ser Gln Leu Ile Tyr Leu Leu Phe Ser Gln
    485                 490                 495

tgc ggg att tcc aat tct gat cct ttg tgt gat tac aat cgc aat ttc    1712
Cys Gly Ile Ser Asn Ser Asp Pro Leu Cys Asp Tyr Asn Arg Asn Phe
500                 505                 510                 515

act gat gtt aaa tca gct ata gaa ata gca gca gct ggt cct ggt gtc    1760
Thr Asp Val Lys Ser Ala Ile Glu Ile Ala Ala Ala Gly Pro Gly Val
```

```
                520                 525                 530

tac cag gct gtg tgg ttg ctc ctg att gct ttg gta ctg aaa ttg gga    1808
Tyr Gln Ala Val Trp Leu Leu Leu Ile Ala Leu Val Leu Lys Leu Gly
            535                 540                 545

atg act gta ttt acc ttt ggt atg aaa gta cca tgt ggt ctg ttt atc    1856
Met Thr Val Phe Thr Phe Gly Met Lys Val Pro Cys Gly Leu Phe Ile
        550                 555                 560

cca agt tta tgc cta gga gct att atg ggt aga att gtg ggc att gga    1904
Pro Ser Leu Cys Leu Gly Ala Ile Met Gly Arg Ile Val Gly Ile Gly
    565                 570                 575

att gaa caa ttg gct tac tat tat cca aaa tta tgg ttc ttt tct ggt    1952
Ile Glu Gln Leu Ala Tyr Tyr Tyr Pro Lys Leu Trp Phe Phe Ser Gly
580                 585                 590                 595

gaa tgc tca act gga gac aat tgc atc aca ccg ggc ctg tat gct atg    2000
Glu Cys Ser Thr Gly Asp Asn Cys Ile Thr Pro Gly Leu Tyr Ala Met
                600                 605                 610

gtg ggc gct gca gct gtt tta ggt ggt gtc act aga atg aca gtt tct    2048
Val Gly Ala Ala Ala Val Leu Gly Gly Val Thr Arg Met Thr Val Ser
            615                 620                 625

ctg gtg gta ata atg ttt gaa ctg act ggt ggt gta cgt tat atc gtg    2096
Leu Val Val Ile Met Phe Glu Leu Thr Gly Gly Val Arg Tyr Ile Val
        630                 635                 640

ccc tta atg gca gca gct atg gct tcc aaa tgg gtt ggt gat gct ttg    2144
Pro Leu Met Ala Ala Ala Met Ala Ser Lys Trp Val Gly Asp Ala Leu
    645                 650                 655

ggc aga cag ggt ata tat gat gcc cat ata cag ctt aat gga tat cca    2192
Gly Arg Gln Gly Ile Tyr Asp Ala His Ile Gln Leu Asn Gly Tyr Pro
660                 665                 670                 675

ttc ttg gac agt aaa gat gaa ttt gca cat aca tct tta gct gca gat    2240
Phe Leu Asp Ser Lys Asp Glu Phe Ala His Thr Ser Leu Ala Ala Asp
                680                 685                 690

gtc atg caa ccc aag agg aat gaa aca tta agt gta atc act caa gac    2288
Val Met Gln Pro Lys Arg Asn Glu Thr Leu Ser Val Ile Thr Gln Asp
            695                 700                 705

tcg atg act gtg gat gat gtt gaa ggt tta ctg aaa gaa act gag cac    2336
Ser Met Thr Val Asp Asp Val Glu Gly Leu Leu Lys Glu Thr Glu His
```

```
        710                 715                 720

aat gga tat cca gtt gtt gtt tcc aga gaa tct cag tat ctt gtt gga    2384
Asn Gly Tyr Pro Val Val Val Ser Arg Glu Ser Gln Tyr Leu Val Gly
    725                 730                 735

ttt gtt ttg agg agg gac tta aat cta gcc ata gcc aat gct aga cgc    2432
Phe Val Leu Arg Arg Asp Leu Asn Leu Ala Ile Ala Asn Ala Arg Arg
740                 745                 750                 755

atg atc gat ggg ata aca gga caa agt ttg gta ctt ttc ata aat ggc    2480
Met Ile Asp Gly Ile Thr Gly Gln Ser Leu Val Leu Phe Ile Asn Gly
                760                 765                 770

cct aca gtg caa agt tta gga cct cca cct ttg aaa cta aag aaa ata    2528
Pro Thr Val Gln Ser Leu Gly Pro Pro Pro Leu Lys Leu Lys Lys Ile
            775                 780                 785

tta gat atg gct cca ata aca gtg act gat caa aca cca atg gaa act    2576
Leu Asp Met Ala Pro Ile Thr Val Thr Asp Gln Thr Pro Met Glu Thr
        790                 795                 800

gtg gtg gat atg ttt aga aaa cta ggt tta cgt cag aca tta gtc aca    2624
Val Val Asp Met Phe Arg Lys Leu Gly Leu Arg Gln Thr Leu Val Thr
    805                 810                 815

cac aat ggg cgt ttg ctc ggt gtt ata act aaa aaa gat gtt tta cga    2672
His Asn Gly Arg Leu Leu Gly Val Ile Thr Lys Lys Asp Val Leu Arg
820                 825                 830                 835

cat gta aaa caa atg gat aat gaa gat cct aat agt ata ctt ttt aat    2720
His Val Lys Gln Met Asp Asn Glu Asp Pro Asn Ser Ile Leu Phe Asn
                840                 845                 850

taatatttac atatatgtat taaataataa atgttaagtt gcataaaaat ccataaaact 2780

atgtaggtgt acattttaat ggctattctt catataccta aatgaaaaga ctaaaaagaa 2840

accaattttt aaaatattta ggtatttgat ataatacata tttattttaa tatgaatagt 2900

attttgtgta atttaattga aggcatcaca aattaaattg tgtaattgtt ataacagaat 2960

cagagtgtat atattagatt tatcgattcg aaatttgtac aaaatgtatc tcagtttttt 3020

ttaatgagga cttagaaaat tttattgtta attgattatt tttttagtat tattttagta 3080

tttgtaactg attattaaac gattgattat aaaaaaaaaa aaaaaa                 3126
```

<210> 1915
<211> 851

<212> PRT
<213> Ctenocephalides felis

<400> 1915

```
Met Glu Lys Tyr Pro Leu Lys Gly Asn Ser Thr Ile Arg Leu Ser Gly
 1               5                  10                  15

Pro Ala Ser Tyr Gln Ser Val Thr Lys Lys Lys Arg Pro Gly Asp Ser
            20                  25                  30

Thr Asn His Ile Val Glu Arg Val Asp His Pro Leu Ser Gly Thr Met
        35                  40                  45

Pro Ser Asp Gly Pro Gln Gln His Asn Pro Val Ser Asp Ser Gly Asp
    50                  55                  60

Phe Ser Pro Asp Glu Phe Gly Lys Arg Ile Phe Pro Pro Gly Ser Thr
65                  70                  75                  80

Gln Leu Thr Leu Asp Asp Asp Asn Gly Ser Ala Asn Leu Ala Ile Lys
                85                  90                  95

Phe Glu Gly Leu Ser Ser Gly Gly Ile Ser Phe Ala Gly Met Gln Ala
            100                 105                 110

Glu Ser Asp Asp Ile Pro Gly Ile Gly Gln Tyr Asp Asp Phe His Thr
        115                 120                 125

Ile Asp Trp Gln Arg Asp Ile Ala Arg Asp Arg Met Arg His Arg Tyr
    130                 135                 140

Ile Val Lys Lys Arg Gln Asp Ser Ile Leu Asp Leu Ile Lys Gly Ala
145                 150                 155                 160

His Asp Ala Trp Ser Gly Trp Val Cys Val Leu Leu Val Gly Leu Val
                165                 170                 175

Thr Gly Ala Ile Ala Gly Val Ile Asp Ile Gly Ala Ser Trp Met Thr
            180                 185                 190

Asp Leu Lys Asn Gly Val Cys Pro Gln Ala Phe Trp Leu Asn Arg Glu
        195                 200                 205

Gln Cys Cys Trp Ser Leu Asn Glu Thr Thr Phe Asp Asp Gly Asn Cys
    210                 215                 220
```

```
Ser Gln Trp Leu Thr Trp Pro Glu Val Phe Gly Gln Pro Arg Thr Gly
225                 230                 235                 240

Ala Gly Ala Tyr Ile Ile Ala Tyr Leu Phe Tyr Ile Ile Trp Ala Leu
                245                 250                 255

Ile Phe Ala Ser Leu Ala Ala Ser Leu Val Arg Met Phe Ala Pro Tyr
            260                 265                 270

Ala Cys Gly Ser Gly Ile Pro Glu Ile Lys Thr Ile Leu Ser Gly Phe
        275                 280                 285

Ile Ile Arg Gly Tyr Leu Gly Lys Trp Thr Leu Ile Ile Lys Ser Val
    290                 295                 300

Gly Ile Met Leu Ser Val Ser Ala Gly Leu Ser Leu Gly Lys Glu Gly
305                 310                 315                 320

Pro Met Val His Ile Ala Ser Cys Ile Gly Asn Ile Leu Ser Tyr Leu
                325                 330                 335

Phe Pro Lys Tyr Gly Arg Asn Glu Ala Lys Lys Arg Glu Ile Leu Ser
            340                 345                 350

Ala Ala Ala Ala Ala Gly Val Ser Val Ala Phe Gly Ala Pro Ile Gly
        355                 360                 365

Gly Val Leu Phe Ser Leu Glu Glu Val Ser Tyr Tyr Phe Pro Leu Lys
    370                 375                 380

Thr Leu Trp Arg Ser Phe Phe Cys Ala Leu Ile Ala Ala Phe Ile Leu
385                 390                 395                 400

Arg Ser Ile Asn Pro Phe Gly Asn Glu His Ser Val Leu Phe Tyr Val
                405                 410                 415

Glu Tyr Asn Lys Pro Trp Ile Phe Phe Glu Leu Ile Pro Phe Ile Gly
            420                 425                 430

Leu Gly Ile Ile Gly Gly Val Val Ala Thr Leu Phe Ile Lys Ala Asn
        435                 440                 445

Leu Tyr Trp Cys Arg Tyr Arg Lys Phe Ser Lys Leu Gly Gln Tyr Pro
    450                 455                 460

Val Ala Glu Val Leu Val Val Ala Val Ala Thr Ala Val Ile Ala Tyr
465                 470                 475                 480
```

```
Pro Asn Pro Tyr Thr Arg Met Asn Thr Ser Gln Leu Ile Tyr Leu Leu
                485                 490                 495

Phe Ser Gln Cys Gly Ile Ser Asn Ser Asp Pro Leu Cys Asp Tyr Asn
            500                 505                 510

Arg Asn Phe Thr Asp Val Lys Ser Ala Ile Glu Ile Ala Ala Ala Gly
        515                 520                 525

Pro Gly Val Tyr Gln Ala Val Trp Leu Leu Leu Ile Ala Leu Val Leu
    530                 535                 540

Lys Leu Gly Met Thr Val Phe Thr Phe Gly Met Lys Val Pro Cys Gly
545                 550                 555                 560

Leu Phe Ile Pro Ser Leu Cys Leu Gly Ala Ile Met Gly Arg Ile Val
                565                 570                 575

Gly Ile Gly Ile Glu Gln Leu Ala Tyr Tyr Tyr Pro Lys Leu Trp Phe
            580                 585                 590

Phe Ser Gly Glu Cys Ser Thr Gly Asp Asn Cys Ile Thr Pro Gly Leu
        595                 600                 605

Tyr Ala Met Val Gly Ala Ala Ala Val Leu Gly Gly Val Thr Arg Met
    610                 615                 620

Thr Val Ser Leu Val Val Ile Met Phe Glu Leu Thr Gly Gly Val Arg
625                 630                 635                 640

Tyr Ile Val Pro Leu Met Ala Ala Ala Met Ala Ser Lys Trp Val Gly
                645                 650                 655

Asp Ala Leu Gly Arg Gln Gly Ile Tyr Asp Ala His Ile Gln Leu Asn
            660                 665                 670

Gly Tyr Pro Phe Leu Asp Ser Lys Asp Glu Phe Ala His Thr Ser Leu
        675                 680                 685

Ala Ala Asp Val Met Gln Pro Lys Arg Asn Glu Thr Leu Ser Val Ile
    690                 695                 700

Thr Gln Asp Ser Met Thr Val Asp Asp Val Glu Gly Leu Leu Lys Glu
705                 710                 715                 720

Thr Glu His Asn Gly Tyr Pro Val Val Val Ser Arg Glu Ser Gln Tyr
                725                 730                 735
```

Leu Val Gly Phe Val Leu Arg Arg Asp Leu Asn Leu Ala Ile Ala Asn
740 745 750

Ala Arg Arg Met Ile Asp Gly Ile Thr Gly Gln Ser Leu Val Leu Phe
755 760 765

Ile Asn Gly Pro Thr Val Gln Ser Leu Gly Pro Pro Pro Leu Lys Leu
770 775 780

Lys Lys Ile Leu Asp Met Ala Pro Ile Thr Val Thr Asp Gln Thr Pro
785 790 795 800

Met Glu Thr Val Val Asp Met Phe Arg Lys Leu Gly Leu Arg Gln Thr
805 810 815

Leu Val Thr His Asn Gly Arg Leu Leu Gly Val Ile Thr Lys Lys Asp
820 825 830

Val Leu Arg His Val Lys Gln Met Asp Asn Glu Asp Pro Asn Ser Ile
835 840 845

Leu Phe Asn
850

<210> 1916
<211> 3126
<212> DNA
<213> Ctenocephalides felis

<400> 1916

tactcactat atggctcgag cggccgcccg ggcagtcgtt tttgttagaa aaagtacaaa 60
aattanttaa tgtaacagtg cttgtgatat aaaaaataat gattcaagtg catcatattt 120
aaatcaatta attttttaa aatcttagaa atgaatttaa agcagctatg gagaaatatc 180
cattaaaagg aaattctacg atacgtttaa gtggtcctgc ttcttatcaa tcggttacta 240
aaaaaaaacg cccaggagac tccactaatc acatagtgga acgagtggat catcctttga 300
gcggcacgat gccctcggac ggaccccaac agcataatcc agtgtcggac tcgggcgatt 360
tttcgccaga cgaattcggc aaacgtatat ttcctcccgg atccacacag ttgaccctgg 420
atgatgataa tggatcagca aatttggcaa ttaaatttga gggtctttct agtggtggca 480
tatcatttgc tggtatgcaa gctgaaagcg atgatattcc tgggattggg caatatgatg 540
atttccatac gatagattgg caacgtgata tagccagaga tcgaatgaga catcgatata 600
ttgttaaaaa acgacaagac tctatactag acctgataaa gggtgcccat gatgcctggt 660
caggttgggt gtgtgttctc ctagtcgggc tggtgacagg agctattgca ggcgtcatag 720
atatcggagc aagttggatg acggatttaa agaacggtgt ttgcccacaa gcattctggt 780
tgaatagaga acaatgttgt tggtcattga atgaaacaac ctttgatgat ggaaattgct 840
cacaatggct gacttggcct gaggttttcg gacaacctag aactggggcg ggggcttaca 900

```
taattgctta tttgttttat attatttggg cattgatttt tgcttctttg gcagcctctt 960
tggtgcgcat gtttgcacct tatgcttgtg ggtcaggtat accagagatt aaaaccattc 1020
tgagtggttt catcatcaga ggatatcttg gaaaatggac attgattatt aaaagtgtag 1080
gaatcatgtt gtctgtatca gctggattga gtttgggtaa agaaggtcct atggtacaca 1140
ttgccagctg tataggtaat atattgtctt atttatttcc taaatatggt cggaatgaag 1200
caaagaaacg agagatttta tcagcagctg cagcagctgg tgtatctgtt gcatttggag 1260
cacctattgg aggtgtgctt ttcagtttgg aagaggtgag ctactatttc ccattgaaga 1320
ccttatggag atcattcttc tgtgctttga tagcagcttt catattgcga tccataaatc 1380
catttggaaa tgagcactct gtccttttct atgtggaata caataaacct tggatatttt 1440
ttgaactgat acctttcata ggccttggaa taattggtgg tgttgtagca acgctgttta 1500
taaaagctaa tttgtactgg tgtcgctacc gtaaattttc taaactagga cagtaccccg 1560
ttgcagaagt tttagttgtt gctgttgcaa cagcagtgat tgcttatcct aatccttaca 1620
ccaggatgaa tactagtcaa ctgatttatt tactattcag ccaatgcggg atttccaatt 1680
ctgatccttt gtgtgattac aatcgcaatt tcactgatgt taaatcagct atagaaatag 1740
cagcagctgg tcctggtgtc taccaggctg tgtggttgct cctgattgct ttggtactga 1800
aattgggaat gactgtattt acctttggta tgaaagtacc atgtggtctg tttatcccaa 1860
gtttatgcct aggagctatt atgggtagaa ttgtgggcat tggaattgaa caattggctt 1920
actattatcc aaaattatgg ttcttttctg gtgaatgctc aactggagac aattgcatca 1980
caccgggcct gtatgctatg gtgggcgctg cagctgtttt aggtggtgtc actagaatga 2040
cagtttctct ggtggtaata atgtttgaac tgactggtgg tgtacgttat atcgtgccct 2100
taatggcagc agctatggct tccaaatggg ttggtgatgc tttgggcaga cagggtatat 2160
atgatgccca tatacagctt aatggatatc cattcttgga cagtaaagat gaatttgcac 2220
atacatcttt agctgcagat gtcatgcaac ccaagaggaa tgaaacatta agtgtaatca 2280
ctcaagactc gatgactgtg gatgatgttg aaggtttact gaaagaaact gagcacaatg 2340
gatatccagt tgttgtttcc agagaatctc agtatcttgt tggatttgtt ttgaggaggg 2400
acttaaatct agccatagcc aatgctagac gcatgatcga tgggataaca ggacaaagtt 2460
tggtactttt cataaatggc cctacagtgc aaagtttagg acctccacct ttgaaactaa 2520
agaaaatatt agatatggct ccaataacag tgactgatca aacaccaatg gaaactgtgg 2580
tggatatgtt tagaaaacta ggtttacgtc agacattagt cacacacaat gggcgtttgc 2640
tcggtgttat aactaaaaaa gatgttttac gacatgtaaa acaaatggat aatgaagatc 2700
ctaatagtat acttttttaat taatatttac atatatgtat taaataataa atgttaagtt 2760
gcataaaaat ccataaaact atgtaggtgt acattttaat ggctattctt catataccta 2820
aatgaaaaga ctaaaaagaa accaattttt aaaatattta ggtatttgat ataatacata 2880
tttattttaa tatgaatagt attttgtgta atttaattga aggcatcaca aattaaattg 2940
tgtaattgtt ataacagaat cagagtgtat atattagatt tatcgattcg aaatttgtac 3000
aaaatgtatc tcagtttttt ttaatgagga cttagaaaat tttattgtta attgattatt 3060
tttttagtat tattttagta tttgtaactg attattaaac gattgattat aaaaaaaaaa 3120
aaaaaa                                                            3126
```

<210> 1917
<211> 2553
<212> DNA
<213> Ctenocephalides felis

<400> 1917

```
atggagaaat atccattaaa aggaaattct acgatacgtt taagtggtcc tgcttcttat 60
caatcggtta ctaaaaaaaa acgcccagga gactccacta atcacatagt ggaacgagtg 120
```

```
gatcatcctt tgagcggcac gatgccctcg gacggacccc aacagcataa tccagtgtcg 180
gactcgggcg atttttcgcc agacgaattc ggcaaacgta tatttcctcc cggatccaca 240
cagttgaccc tggatgatga taatggatca gcaaatttgg caattaaatt tgagggtctt 300
tctagtggtg gcatatcatt tgctggtatg caagctgaaa gcgatgatat tcctgggatt 360
gggcaatatg atgatttcca tacgatagat tggcaacgtg atatagccag agatcgaatg 420
agacatcgat atattgttaa aaaacgacaa gactctatac tagacctgat aaagggtgcc 480
catgatgcct ggtcaggttg ggtgtgtgtt ctcctagtcg ggctggtgac aggagctatt 540
gcaggcgtca tagatatcgg agcaagttgg atgacggatt taaagaacgg tgtttgccca 600
caagcattct ggttgaatag agaacaatgt tgttggtcat tgaatgaaac aacctttgat 660
gatggaaatt gctcacaatg gctgacttgg cctgaggttt tcggacaacc tagaactggg 720
gcgggggctt acataattgc ttatttgttt tatattattt gggcattgat tttgcttct 780
ttggcagcct ctttggtgcg catgtttgca ccttatgctt gtgggtcagg tataccagag 840
attaaaacca ttctgagtgg tttcatcatc agaggatatc ttggaaaatg gacattgatt 900
attaaaagtg taggaatcat gttgtctgta tcagctggat tgagtttggg taaagaaggt 960
cctatggtac acattgccag ctgtataggt aatatattgt cttatttatt tcctaaatat 1020
ggtcggaatg aagcaaagaa acgagagatt ttatcagcag ctgcagcagc tggtgtatct 1080
gttgcatttg gagcacctat tggaggtgtg cttttcagtt tggaagaggt gagctactat 1140
ttcccattga agaccttatg gagatcattc ttctgtgctt tgatagcagc tttcatattg 1200
cgatccataa atccatttgg aaatgagcac tctgtccttt tctatgtgga atacaataaa 1260
ccttggatat tttttgaact gataccttc ataggccttg gaataattgg tggtgttgta 1320
gcaacgctgt ttataaaagc taatttgtac tggtgtcgct accgtaaatt ttctaaacta 1380
ggacagtacc ccgttgcaga agttttagtt gttgctgttg caacagcagt gattgcttat 1440
cctaatcctt acaccaggat gaatactagt caactgattt atttactatt cagccaatgc 1500
gggatttcca attctgatcc tttgtgtgat tacaatcgca atttcactga tgttaaatca 1560
gctatagaaa tagcagcagc tggtcctggt gtctaccagg ctgtgtggtt gctcctgatt 1620
gctttggtac tgaaattggg aatgactgta tttacctttg gtatgaaagt accatgtggt 1680
ctgtttatcc caagtttatg cctaggagct attatgggta gaattgtggg cattggaatt 1740
gaacaattgg cttactatta tccaaaatta tggttctttt ctggtgaatg ctcaactgga 1800
gacaattgca tcacaccggg cctgtatgct atggtgggcg ctgcagctgt tttaggtggt 1860
gtcactagaa tgacagtttc tctggtggta ataatgtttg aactgactgg tggtgtacgt 1920
tatatcgtgc ccttaatggc agcagctatg gcttccaaat gggttggtga tgctttgggc 1980
agacagggta tatatgatgc ccatatacag cttaatggat atccattctt ggacagtaaa 2040
gatgaatttg cacatacatc tttagctgca gatgtcatgc aacccaagag gaatgaaaca 2100
ttaagtgtaa tcactcaaga ctcgatgact gtggatgatg ttgaaggttt actgaaagaa 2160
actgagcaca atggatatcc agttgttgtt tccagagaat ctcagtatct tgttggattt 2220
gttttgagga gggacttaaa tctagccata gccaatgcta gacgcatgat cgatgggata 2280
acaggacaaa gtttggtact tttcataaat ggccctacag tgcaaagttt aggacctcca 2340
cctttgaaac taaagaaaat attagatatg gctccaataa cagtgactga tcaaacacca 2400
atggaaactg tggtggatat gtttagaaaa ctaggtttac gtcagacatt agtcacacac 2460
aatgggcgtt tgctcggtgt tataactaaa aaagatgttt tacgacatgt aaaacaaatg 2520
gataatgaag atcctaatag tatacttttt aat                              2553
```

<210> 1918
<211> 2553
<212> DNA
<213> Ctenocephalides felis

<400> 1918

```
attaaaaagt atactattag gatcttcatt atccatttgt tttacatgtc gtaaaacatc 60
ttttttagtt ataacaccga gcaaacgccc attgtgtgtg actaatgtct gacgtaaacc 120
tagttttcta aacatatcca ccacagtttc cattggtgtt tgatcagtca ctgttattgg 180
agccatatct aatattttct ttagtttcaa aggtggaggt cctaaacttt gcactgtagg 240
gccatttatg aaaagtacca aactttgtcc tgttatccca tcgatcatgc gtctagcatt 300
ggctatggct agatttaagt ccctcctcaa aacaaatcca acaagatact gagattctct 360
ggaaacaaca actggatatc cattgtgctc agtttctttc agtaaacctt caacatcatc 420
cacagtcatc gagtcttgag tgattacact taatgtttca ttcctcttgg gttgcatgac 480
atctgcagct aaagatgtat gtgcaaattc atctttactg tccaagaatg gatatccatt 540
aagctgtata tgggcatcat atataccctg tctgcccaaa gcatcaccaa cccatttgga 600
agccatagct gctgccatta agggcacgat ataacgtaca ccaccagtca gttcaaacat 660
tattaccacc agagaaactg tcattctagt gacaccacct aaaacagctg cagcgcccac 720
catagcatac aggcccggtg tgatgcaatt gtctccagtt gagcattcac cagaaaagaa 780
ccataatttt ggataatagt aagccaattg ttcaattcca atgcccacaa ttctacccat 840
aatagctcct aggcataaac ttgggataaa cagaccacat ggtactttca taccaaaggt 900
aaatacagtc attcccaatt tcagtaccaa agcaatcagg agcaaccaca cagcctggta 960
gacaccagga ccagctgctg ctatttctat agctgattta acatcagtga aattgcgatt 1020
gtaatcacac aaaggatcag aattggaaat cccgcattgg ctgaatagta aataaatcag 1080
ttgactagta ttcatcctgg tgtaaggatt aggataagca atcactgctg ttgcaacagc 1140
aacaactaaa acttctgcaa cggggtactg tcctagttta gaaaatttac ggtagcgaca 1200
ccagtacaaa ttagctttta taaacagcgt tgctacaaca ccaccaatta ttccaaggcc 1260
tatgaaaggt atcagttcaa aaaatatcca aggtttattg tattccacat agaaaaggac 1320
agagtgctca tttccaaatg gatttatgga tcgcaatatg aaagctgcta tcaaagcaca 1380
gaagaatgat ctccataagg tcttcaatgg gaaatagtag ctcacctctt ccaaactgaa 1440
aagcacacct ccaataggtg ctccaaatgc aacagataca ccagctgctg cagctgctga 1500
taaaatctct cgtttctttg cttcattccg accatattta ggaaataaat aagacaatat 1560
attacctata cagctggcaa tgtgtaccat aggaccttct ttacccaaac tcaatccagc 1620
tgatacagac aacatgattc ctacactttt aataatcaat gtccattttc caagatatcc 1680
tctgatgatg aaaccactca gaatggtttt aatctctggt atacctgacc cacaagcata 1740
aggtgcaaac atgcgcacca aagaggctgc caaagaagca aaaatcaatg cccaaataat 1800
ataaaacaaa taagcaatta tgtaagcccc cgccccagtt ctaggttgtc cgaaaacctc 1860
aggccaagtc agccattgtg agcaatttcc atcatcaaag gttgtttcat tcaatgacca 1920
acaacattgt tctctattca accagaatgc ttgtgggcaa acaccgttct ttaaatccgt 1980
catccaactt gctccgatat ctatgacgcc tgcaatagct cctgtcacca gcccgactag 2040
gagaacacac acccaacctg accaggcatc atgggcaccc tttatcaggt ctagtataga 2100
gtcttgtcgt tttttaacaa tatatcgatg tctcattcga tctctggcta tatcacgttg 2160
ccaatctatc gtatggaaat catcatattg cccaatccca ggaatatcat cgctttcagc 2220
ttgcatacca gcaaatgata tgccaccact agaaagaccc tcaaatttaa ttgccaaatt 2280
tgctgatcca ttatcatcat ccagggtcaa ctgtgtggat ccgggaggaa atatacgttt 2340
gccgaattcg tctggcgaaa aatcgcccga gtccgacact ggattatgct gttggggtcc 2400
gtccgagggc atcgtgccgc tcaaaggatg atccactcgt tccactatgt gattagtgga 2460
gtctcctggg cgtttttttt tagtaaccga ttgataagaa gcaggaccac ttaaacgtat 2520
cgtagaattt ccttttaatg gatatttctc cat                              2553
```

<210> 1919
<211> 1181
<212> DNA
<213> Ctenocephalides canis

<220>
<221> CDS

<222> (127)..(432)

<400> 1919

```
acatcacaga ccgtcgacat ataaacacaa ccgaaatctc ctatcacagt gtacggagtg 60

taaaatattg ttgaagtatt ttgaaattta ttaatttatt cgaaaaggag atttcattaa 120

ataaaa atg gtt tac gaa agt gac ttt tac acg acc cgt cgg ccc tac    168
       Met Val Tyr Glu Ser Asp Phe Tyr Thr Thr Arg Arg Pro Tyr
       1               5                   10

agt cgt ccg gct ttg tct tca tac tcc gta acg acg ccg tcc cgt cat   216
Ser Arg Pro Ala Leu Ser Ser Tyr Ser Val Thr Thr Pro Ser Arg His
15                  20                  25                  30

tac gtg gtg act gac act cca tct aga cca agg gta gcg gaa gag caa   264
Tyr Val Val Thr Asp Thr Pro Ser Arg Pro Arg Val Ala Glu Glu Gln
                35                  40                  45

tat tct tac tcc tac cgc agc cag cag gaa aga tct tct gca gat ccc   312
Tyr Ser Tyr Ser Tyr Arg Ser Gln Gln Glu Arg Ser Ser Ala Asp Pro
            50                  55                  60

tac ggc agg aac tat tcg aca acc tcc acc acc gaa agc aca aga cgt   360
Tyr Gly Arg Asn Tyr Ser Thr Thr Ser Thr Thr Glu Ser Thr Arg Arg
        65                  70                  75

gca ggg ggt tat cca gga tct gac tat tct tac acg agc gaa cgc tca   408
Ala Gly Gly Tyr Pro Gly Ser Asp Tyr Ser Tyr Thr Ser Glu Arg Ser
    80                  85                  90

tcc aag aac tgg aga tgg acc agg tagttacaga tccagctata gctccactac  462
Ser Lys Asn Trp Arg Trp Thr Arg
95                  100

ttctggacgt cttcctggag gaaccactta ccgtcacttc tcataccgtg tgtaaacacg 522

aaaattgaaa aattttgttt tgataaattg gtttttattt gaagtctcat aagaactaaa 582

agaatttttt ttataatttt tatttttttg ctatgaatta ttattactat tatagcactc 642

atattaatat cctgaaaatg taaaacaaaa ctatgatttc tgatcaaaca accaatttta 702
```

```
tcatcactga tttttatcat atcctaggat gatgtaatat tgtagtcctt ggaaagttat 762

accagaagaa gactcaatat gctattctgt gtaattatta atataactta atattatttt 822

tatttattta attaattagc aatgtctact aaaaattgtc taaaattagc ttttaatttt 882

ccctagatat attatcataa aaaccaaaga ttaggcatat ttttgatact gtacaaattt 942

tcttgtgata taagtttaca atttgtttta atttttgttg aaacaaaata cgtaatggta 1002

ttgcaattaa taattataga tactaatagt gatgttgtaa gagttaggac taatagatga 1062

gtcttcttca attttgataa acatattttt ataatctgtg ttaaattttg cacttgtaat 1122

gctgtgcttt ttaatgaata aacatgcaat attaaaactt caaaaaaaaa aaaaaaaaa  1181
```

<210> 1920
<211> 102
<212> PRT
<213> Ctenocephalides canis

<400> 1920

```
Met Val Tyr Glu Ser Asp Phe Tyr Thr Thr Arg Arg Pro Tyr Ser Arg
 1               5                  10                  15

Pro Ala Leu Ser Ser Tyr Ser Val Thr Thr Pro Ser Arg His Tyr Val
            20                  25                  30

Val Thr Asp Thr Pro Ser Arg Pro Arg Val Ala Glu Glu Gln Tyr Ser
        35                  40                  45

Tyr Ser Tyr Arg Ser Gln Gln Glu Arg Ser Ser Ala Asp Pro Tyr Gly
    50                  55                  60

Arg Asn Tyr Ser Thr Thr Ser Thr Thr Glu Ser Thr Arg Arg Ala Gly
65                  70                  75                  80

Gly Tyr Pro Gly Ser Asp Tyr Ser Tyr Thr Ser Glu Arg Ser Ser Lys
                85                  90                  95

Asn Trp Arg Trp Thr Arg
            100
```

<210> 1921
<211> 1181
<212> DNA
<213> Ctenocephalides felis

<400> 1921

```
tttttttttt ttttttttga agttttaata ttgcatgttt attcattaaa aagcacagca 60
ttacaagtgc aaaatttaac acagattata aaaatatgtt tatcaaaatt gaagaagact 120
catctattag tcctaactct tacaacatca ctattagtat ctataattat taattgcaat 180
accattacgt attttgtttc aacaaaaatt aaaacaaatt gtaaacttat atcacaagaa 240
aatttgtaca gtatcaaaaa tatgcctaat ctttggtttt tatgataata tatctaggga 300
aaattaaaag ctaattttag acaattttta gtagacattg ctaattaatt aaataaataa 360
aaataatatt aagttatatt aataattaca cagaatagca tattgagtct tcttctggta 420
taactttcca aggactacaa tattacatca tcctaggata tgataaaaat cagtgatgat 480
aaaattggtt gtttgatcag aaatcatagt tttgttttac attttcagga tattaatatg 540
agtgctataa tagtaataat aattcatagc aaaaaaataa aaattataaa aaaaattctt 600
ttagttctta tgagacttca aataaaaacc aatttatcaa aacaaaattt ttcaattttc 660
gtgtttacac acggtatgag aagtgacggt aagtggttcc tccaggaaga cgtccagaag 720
tagtggagct atagctggat ctgtaactac ctggtccatc tccagttctt ggatgagcgt 780
tcgctcgtgt aagaatagtc agatcctgga taaccccctg cacgtcttgt gctttcggtg 840
gtggaggttg tcgaatagtt cctgccgtag ggatctgcag aagatctttc ctgctggctg 900
cggtaggagt aagaatattg ctcttccgct acccttggtc tagatggagt gtcagtcacc 960
acgtaatgac gggacggcgt cgttacggag tatgaagaca aagccggacg actgtagggc 1020
cgacgggtcg tgtaaaagtc actttcgtaa accattttta tttaatgaaa tctccttttc 1080
gaataaatta ataaatttca aaatacttca acaatatttt acactccgta cactgtgata 1140
ggagatttcg gttgtgttta tatgtcgacg gtctgtgatg t                     1181
```

<210> 1922
<211> 306
<212> DNA
<213> Ctenocephalides felis

<400> 1922

```
atggtttacg aaagtgactt ttacacgacc cgtcggccct acagtcgtcc ggctttgtct 60
tcatactccg taacgacgcc gtcccgtcat tacgtggtga ctgacactcc atctagacca 120
agggtagcgg aagagcaata ttcttactcc taccgcagcc agcaggaaag atcttctgca 180
gatccctacg gcaggaacta ttcgacaacc tccaccaccg aaagcacaag acgtgcaggg 240
ggttatccag gatctgacta ttcttacacg agcgaacgct catccaagaa ctggagatgg 300
accagg                                                            306
```

<210> 1923
<211> 306
<212> DNA
<213> Ctenocephalides felis

<400> 1923

```
cctggtccat ctccagttct tggatgagcg ttcgctcgtg taagaatagt cagatcctgg 60
ataacccccct gcacgtcttg tgctttcggt ggtggaggtt gtcgaatagt tcctgccgta 120
```

```
gggatctgca gaagatcttt cctgctggct gcggtaggag taagaatatt gctcttccgc 180
taccccttggt ctagatggag tgtcagtcac cacgtaatga cgggacggcg tcgttacgga 240
gtatgaagac aaagccggac gactgtaggg ccgacgggtc gtgtaaaagt cactttcgta 300
aaccat                                                            306
```

<210> 1924
<211> 2161
<212> DNA
<213> Ctenocephalides felis

<220>
<221> CDS
<222> (107)..(907)

<400> 1924

```
ggcaccaggt gagtacttgt actctaacac cgtgccaaag cctcaaacac actcgcctgc 60

gctgggagtt agtacacagt gatctccact ggcaagcgat tacacg atg act ttc    115
                                                   Met Thr Phe
                                                   1

gga att tca gta att ctg tta gtg tct att tgg aca aca aat act cat   163
Gly Ile Ser Val Ile Leu Leu Val Ser Ile Trp Thr Thr Asn Thr His
    5                   10                  15

gca tat tta aca tca gtg caa gaa tta gac gat gcc ata aga gca gtg   211
Ala Tyr Leu Thr Ser Val Gln Glu Leu Asp Asp Ala Ile Arg Ala Val
20                  25                  30                  35

gtg tca cgg atg cat cga gta gcc gat att gaa agt ggt ggc gaa tac   259
Val Ser Arg Met His Arg Val Ala Asp Ile Glu Ser Gly Gly Glu Tyr
                40                  45                  50

tca gat ctg gga gta gac ttc cca gta ccc gca att cca cga tct caa   307
Ser Asp Leu Gly Val Asp Phe Pro Val Pro Ala Ile Pro Arg Ser Gln
            55                  60                  65

aaa gct cta gaa tcg gat tcg gaa tat gat tcc ata ttc gat gaa ggc   355
Lys Ala Leu Glu Ser Asp Ser Glu Tyr Asp Ser Ile Phe Asp Glu Gly
        70                  75                  80

cag tta cat cct agc ctc aga gat cag gaa tat ctc cag cat agt cct   403
Gln Leu His Pro Ser Leu Arg Asp Gln Glu Tyr Leu Gln His Ser Pro
    85                  90                  95

cta tgg ggt cag cag tac gta agt gga ggc gct ggt gaa ggc caa caa   451
Leu Trp Gly Gln Gln Tyr Val Ser Gly Gly Ala Gly Glu Gly Gln Gln
```

```
100                 105                 110                 115

agg ctc aaa cca gat gga agt gca atg aat cat cag caa gta aaa acg    499
Arg Leu Lys Pro Asp Gly Ser Ala Met Asn His Gln Gln Val Lys Thr
                120                 125                 130

gat aat ctg ccc gct tat tgt aat cca cct aat ccc tgt cct gtt gga    547
Asp Asn Leu Pro Ala Tyr Cys Asn Pro Pro Asn Pro Cys Pro Val Gly
            135                 140                 145

tta aca gaa gag cat ggt tgc acc gag aac ttc gag aac acc gca gcc    595
Leu Thr Glu Glu His Gly Cys Thr Glu Asn Phe Glu Asn Thr Ala Ala
        150                 155                 160

ttc agc cgc gac tac cag gcg gct cag caa tgc atg tgc gac ggt gag    643
Phe Ser Arg Asp Tyr Gln Ala Ala Gln Gln Cys Met Cys Asp Gly Glu
    165                 170                 175

cat atg ttc cgt tgt ccg tcc tca tta gac gga gac gaa ctg gac gac    691
His Met Phe Arg Cys Pro Ser Ser Leu Asp Gly Asp Glu Leu Asp Asp
180                 185                 190                 195

tca tcc gaa tca gac gaa cag gac gaa cac cag gac ctt ctg gac atg    739
Ser Ser Glu Ser Asp Glu Gln Asp Glu His Gln Asp Leu Leu Asp Met
                200                 205                 210

gaa ggc gga ctt gat act cga act gcg cct gaa atc ttc atg gcg caa    787
Glu Gly Gly Leu Asp Thr Arg Thr Ala Pro Glu Ile Phe Met Ala Gln
            215                 220                 225

agg gag tac cga cga tct ggc ttg agt ggg aat aaa cat cat aaa aga    835
Arg Glu Tyr Arg Arg Ser Gly Leu Ser Gly Asn Lys His His Lys Arg
        230                 235                 240

aag agt act aat ccc tac ttg cat ggt gag aaa ctc cca gtg gct gct    883
Lys Ser Thr Asn Pro Tyr Leu His Gly Glu Lys Leu Pro Val Ala Ala
    245                 250                 255

aag aag ggt atc aac gtt gtc tac tgaaccaatt taaaacgctc accactaatt   937
Lys Lys Gly Ile Asn Val Val Tyr
260                 265

taacaaaata tatttttatt tttttggata aaccaaccca gaaacaaatg ctaattaatt 997

ttcagaaaaa agcatttgta aacgactttg gcaattgata aacggaataa tgatttcaaa 1057

ccgaataaac aaaagaaaaa tctacaaaaa taatttagtt ttcgttagtc attacatatg 1117
```

```
tattaccggt taaaaccctt tattcgaagg agaattaaga actaataaaa caatgtttaa 1177
tattaagtag ccgtgtaact tactttgtgt aatattaaag aggcccatta tataataatt 1237
aaatttaatg tagtaaacta tttataatat attaatatta taaatacatc attaaaacat 1297
caaattacta atgaaatcta atacaatgct attggaatcc gattggggta tgatcaaatt 1357
tcttattgtc tttctctcta ttatttattt gcaagtttgt tccgtacccc aggacgattt 1417
caaatatagt ctccatcaat gcgcgcattt gcgaaattaa aaaaagctca caaaaattta 1477
ataaagtctc aattacatcc cgagacaaat ttgaaaaagg aagagttgca ttaacaacaa 1537
ttcacattag aaaagaattg attggraaac ttacacntgn tttcggtgaa cgtgtttcag 1597
accccattga tgctcttgta tccgtgcaat gttcatttat aatttccata atttaatata 1657
ttatgaaacc attgtaatgt attgtattat ccgatttttg taatagacat tacacgcaat 1717
gagtgcacga tacgtgttta atgaaataag tgtatgtttt aagatttgca ttcactattt 1777
gacatagata taagtaatta tatttatcaa gcaaatcact tcgtatcatt tctattatgg 1837
atatataata atgatatact aatgtgaaca aaacatctca aaatattttt aaaatataaa 1897
tgtataatta aatttagaat acttttcgtg aactgtaata atttactcac agttcatgct 1957
aacgatcatg tgtctaaata caattatgaa gaagattata tattacatac atgtgaaatc 2017
tatataataa tgcatgcttg ttaaatttat attataatga atatataatg aatattttaa 2077
atataatata tatgcgcgtt agttgtcaaa taaaagtcta tattgctccc actattggaa 2137
aaaaaaaaaa aaaaaaaaaa aaaa                                        2161
```

<210> 1925
<211> 267
<212> PRT
<213> Ctenocephalides felis

<400> 1925

```
Met Thr Phe Gly Ile Ser Val Ile Leu Leu Val Ser Ile Trp Thr Thr
1               5                   10                  15

Asn Thr His Ala Tyr Leu Thr Ser Val Gln Glu Leu Asp Asp Ala Ile
            20                  25                  30
```

```
Arg Ala Val Val Ser Arg Met His Arg Val Ala Asp Ile Glu Ser Gly
        35                  40                  45

Gly Glu Tyr Ser Asp Leu Gly Val Asp Phe Pro Val Pro Ala Ile Pro
    50                  55                  60

Arg Ser Gln Lys Ala Leu Glu Ser Asp Ser Glu Tyr Asp Ser Ile Phe
65                  70                  75                  80

Asp Glu Gly Gln Leu His Pro Ser Leu Arg Asp Gln Glu Tyr Leu Gln
                85                  90                  95

His Ser Pro Leu Trp Gly Gln Gln Tyr Val Ser Gly Gly Ala Gly Glu
            100                 105                 110

Gly Gln Gln Arg Leu Lys Pro Asp Gly Ser Ala Met Asn His Gln Gln
        115                 120                 125

Val Lys Thr Asp Asn Leu Pro Ala Tyr Cys Asn Pro Pro Asn Pro Cys
    130                 135                 140

Pro Val Gly Leu Thr Glu Glu His Gly Cys Thr Glu Asn Phe Glu Asn
145                 150                 155                 160

Thr Ala Ala Phe Ser Arg Asp Tyr Gln Ala Ala Gln Gln Cys Met Cys
                165                 170                 175

Asp Gly Glu His Met Phe Arg Cys Pro Ser Ser Leu Asp Gly Asp Glu
            180                 185                 190

Leu Asp Asp Ser Ser Glu Ser Asp Glu Gln Asp Glu His Gln Asp Leu
        195                 200                 205

Leu Asp Met Glu Gly Gly Leu Asp Thr Arg Thr Ala Pro Glu Ile Phe
    210                 215                 220

Met Ala Gln Arg Glu Tyr Arg Arg Ser Gly Leu Ser Gly Asn Lys His
225                 230                 235                 240

His Lys Arg Lys Ser Thr Asn Pro Tyr Leu His Gly Glu Lys Leu Pro
                245                 250                 255

Val Ala Ala Lys Lys Gly Ile Asn Val Val Tyr
            260                 265
```

<210> 1926
<211> 2161
<212> DNA
<213> Ctenocephalides felis

<400> 1926

```
tttttttttt tttttttttt ttttttccaa tagtgggagc aatatagact tttatttgac 60
aactaacgcg catatatatt atatttaaaa tattcattat atattcatta taatataaat 120
ttaacaagca tgcattatta tatagatttc acatgtatgt aatatataat cttcttcata 180
attgtattta gacacatgat cgttagcatg aactgtgagt aaattattac agttcacgaa 240
aagtattcta aatttaatta tacatttata ttttaaaaat attttgagat gttttgttca 300
cattagtata tcattattat atatccataa tagaaatgat acgaagtgat ttgcttgata 360
aatataatta cttatatcta tgtcaaatag tgaatgcaaa tcttaaaaca tacacttatt 420
tcattaaaca cgtatcgtgc actcattgcg tgtaatgtct attacaaaaa tcggataata 480
caatacatta caatggtttc ataatatatt aaattatgga aattataaat gaacattgca 540
cggatacaag agcatcaatg gggtctgaaa cacgttcacc gaaancangt gtaagtttyc 600
caatcaattc ttttctaatg tgaattgttg ttaatgcaac tcttcctttt tcaaatttgt 660
ctcgggatgt aattgagact ttattaaatt tttgtgagct ttttttaatt tcgcaaatgc 720
gcgcattgat ggagactata tttgaaatcg tcctggggta cggaacaaac ttgcaaataa 780
ataatagaga gaaagacaat aagaaatttg atcatacccc aatcggattc caatagcatt 840
gtattagatt tcattagtaa tttgatgttt taatgatgta tttataatat taatatatta 900
taaatagttt actacattaa atttaattat tatataatgg gcctctttaa tattacacaa 960
agtaagttac acggctactt aatattaaac attgttttat tagttcttaa ttctccttcg 1020
aataaagggt tttaaccggt aatacatatg taatgactaa cgaaaactaa attattttg 1080
tagatttttc ttttgtttat tcggtttgaa atcattattc cgtttatcaa ttgccaaagt 1140
cgtttacaaa tgctttttc tgaaaattaa ttagcatttg tttctgggtt ggtttatcca 1200
aaaaaataaa aatatatttt gttaaattag tggtgagcgt tttaaattgg ttcagtagac 1260
aacgttgata cccttcttag cagccactgg gagtttctca ccatgcaagt agggattagt 1320
actctttctt ttatgatgtt tattcccact caagccagat cgtcggtact ccctttgcgc 1380
catgaagatt tcaggcgcag ttcgagtatc aagtccgcct tccatgtcca gaaggtcctg 1440
gtgttcgtcc tgttcgtctg attcggatga gtcgtccagt tcgtctccgt ctaatgagga 1500
cggacaacgg aacatatgct caccgtcgca catgcattgc tgagccgcct ggtagtcgcg 1560
gctgaaggct gcggtgttct cgaagttctc ggtgcaacca tgctcttctg ttaatccaac 1620
aggacaggga ttaggtggat tacaataagc gggcagatta tccgttttta cttgctgatg 1680
attcattgca cttccatctg gtttgagcct ttgttggcct tcaccagcgc ctccacttac 1740
gtactgctga ccccatagag gactatgctg gagatattcc tgatctctga ggctaggatg 1800
taactggcct tcatcgaata tggaatcata ttccgaatcc gattctagag ctttttgaga 1860
tcgtggaatt gcgggtactg ggaagtctac tcccagatct gagtattcgc caccactttc 1920
aatatcggct actcgatgca tccgtgacac cactgctctt atggcatcgt ctaattcttg 1980
cactgatgtt aaatatgcat gagtatttgt tgtccaaata gacactaaca gaattactga 2040
aattccgaaa gtcatcgtgt aatcgcttgc cagtggagat cactgtgtac taactcccag 2100
cgcaggcgag tgtgtttgag gctttggcac ggtgttagag tacaagtact cacctggtgc 2160
c                                                                 2161
```

<210> 1927
<211> 801
<212> DNA
<213> Ctenocephalides felis

<400> 1927

```
atgactttcg gaatttcagt aattctgtta gtgtctattt ggacaacaaa tactcatgca 60
tatttaacat cagtgcaaga attagacgat gccataagag cagtggtgtc acggatgcat 120
cgagtagccg atattgaaag tggtggcgaa tactcagatc tgggagtaga cttcccagta 180
cccgcaattc cacgatctca aaaagctcta gaatcggatt cggaatatga ttccatattc 240
gatgaaggcc agttacatcc tagcctcaga gatcaggaat atctccagca tagtcctcta 300
tggggtcagc agtacgtaag tggaggcgct ggtgaaggcc aacaaaggct caaaccagat 360
ggaagtgcaa tgaatcatca gcaagtaaaa acggataatc tgcccgctta ttgtaatcca 420
cctaatccct gtcctgttgg attaacagaa gagcatggtt gcaccgagaa cttcgagaac 480
accgcagcct tcagccgcga ctaccaggcg gctcagcaat gcatgtgcga cggtgagcat 540
atgttccgtt gtccgtcctc attagacgga gacgaactgg acgactcatc cgaatcagac 600
gaacaggacg aacaccagga ccttctggac atggaaggcg gacttgatac tcgaactgcg 660
cctgaaatct tcatggcgca aagggagtac cgacgatctg gcttgagtgg gaataaacat 720
cataaaagaa agagtactaa tccctacttg catggtgaga aactcccagt ggctgctaag 780
aagggtatca acgttgtcta c                                           801
```

<210> 1928
<211> 801
<212> DNA
<213> Ctenocephalides felis

<400> 1928

```
gtagacaacg ttgataccct tcttagcagc cactgggagt ttctcaccat gcaagtaggg 60
attagtactc tttcttttat gatgtttatt cccactcaag ccagatcgtc ggtactccct 120
ttgcgccatg aagatttcag gcgcagttcg agtatcaagt ccgccttcca tgtccagaag 180
gtcctggtgt tcgtcctgtt cgtctgattc ggatgagtcg tccagttcgt ctccgtctaa 240
tgaggacgga caacggaaca tatgctcacc gtcgcacatg cattgctgag ccgcctggta 300
gtcgcggctg aaggctgcgg tgttctcgaa gttctcggtg caaccatgct cttctgttaa 360
tccaacagga cagggattag gtggattaca ataagcgggc agattatccg tttttacttg 420
ctgatgattc attgcacttc catctggttt gagcctttgt tggccttcac cagcgcctcc 480
acttacgtac tgctgacccc atagaggact atgctggaga tattcctgat ctctgaggct 540
aggatgtaac tggccttcat cgaatatgga atcatattcc gaatccgatt ctagagcttt 600
ttgagatcgt ggaattgcgg gtactgggaa gtctactccc agatctgagt attcgccacc 660
actttcaata tcggctactc gatgcatccg tgacaccact gctcttatgg catcgtctaa 720
ttcttgcact gatgttaaat atgcatgagt atttgttgtc caaatagaca ctaacagaat 780
tactgaaatt ccgaaagtca t                                           801
```

<210> 1929
<211> 741
<212> DNA
<213> Ctenocephalides felis

<220>
<221> CDS
<222> (1)..(741)

<400> 1929

```
tat tta aca tca gtg caa gaa tta gac gat gcc ata aga gca gtg gtg    48
Tyr Leu Thr Ser Val Gln Glu Leu Asp Asp Ala Ile Arg Ala Val Val
 1               5                  10                  15

tca cgg atg cat cga gta gcc gat att gaa agt ggt ggc gaa tac tca    96
Ser Arg Met His Arg Val Ala Asp Ile Glu Ser Gly Gly Glu Tyr Ser
            20                  25                  30

gat ctg gga gta gac ttc cca gta ccc gca att cca cga tct caa aaa    144
Asp Leu Gly Val Asp Phe Pro Val Pro Ala Ile Pro Arg Ser Gln Lys
        35                  40                  45

gct cta gaa tcg gat tcg gaa tat gat tcc ata ttc gat gaa ggc cag    192
Ala Leu Glu Ser Asp Ser Glu Tyr Asp Ser Ile Phe Asp Glu Gly Gln
    50                  55                  60

tta cat cct agc ctc aga gat cag gaa tat ctc cag cat agt cct cta    240
Leu His Pro Ser Leu Arg Asp Gln Glu Tyr Leu Gln His Ser Pro Leu
65                  70                  75                  80

tgg ggt cag cag tac gta agt gga ggc gct ggt gaa ggc caa caa agg    288
Trp Gly Gln Gln Tyr Val Ser Gly Gly Ala Gly Glu Gly Gln Gln Arg
                85                  90                  95

ctc aaa cca gat gga agt gca atg aat cat cag caa gta aaa acg gat    336
Leu Lys Pro Asp Gly Ser Ala Met Asn His Gln Gln Val Lys Thr Asp
            100                 105                 110

aat ctg ccc gct tat tgt aat cca cct aat ccc tgt cct gtt gga tta    384
Asn Leu Pro Ala Tyr Cys Asn Pro Pro Asn Pro Cys Pro Val Gly Leu
        115                 120                 125

aca gaa gag cat ggt tgc acc gag aac ttc gag aac acc gca gcc ttc    432
Thr Glu Glu His Gly Cys Thr Glu Asn Phe Glu Asn Thr Ala Ala Phe
    130                 135                 140

agc cgc gac tac cag gcg gct cag caa tgc atg tgc gac ggt gag cat    480
Ser Arg Asp Tyr Gln Ala Ala Gln Gln Cys Met Cys Asp Gly Glu His
145                 150                 155                 160

atg ttc cgt tgt ccg tcc tca tta gac gga gac gaa ctg gac gac tca    528
Met Phe Arg Cys Pro Ser Ser Leu Asp Gly Asp Glu Leu Asp Asp Ser
                165                 170                 175

tcc gaa tca gac gaa cag gac gaa cac cag gac ctt ctg gac atg gaa    576
```

```
Ser Glu Ser Asp Glu Gln Asp Glu His Gln Asp Leu Leu Asp Met Glu
            180                 185                 190

ggc gga ctt gat act cga act gcg cct gaa atc ttc atg gcg caa agg    624
Gly Gly Leu Asp Thr Arg Thr Ala Pro Glu Ile Phe Met Ala Gln Arg
        195                 200                 205

gag tac cga cga tct ggc ttg agt ggg aat aaa cat cat aaa aga aag    672
Glu Tyr Arg Arg Ser Gly Leu Ser Gly Asn Lys His His Lys Arg Lys
    210                 215                 220

agt act aat ccc tac ttg cat ggt gag aaa ctc cca gtg gct gct aag    720
Ser Thr Asn Pro Tyr Leu His Gly Glu Lys Leu Pro Val Ala Ala Lys
225                 230                 235                 240

aag ggt atc aac gtt gtc tac                                        741
Lys Gly Ile Asn Val Val Tyr
                245
```

<210> 1930
<211> 247
<212> PRT
<213> Ctenocephalides felis

<400> 1930

```
Tyr Leu Thr Ser Val Gln Glu Leu Asp Asp Ala Ile Arg Ala Val Val
1               5                   10                  15

Ser Arg Met His Arg Val Ala Asp Ile Glu Ser Gly Gly Glu Tyr Ser
            20                  25                  30

Asp Leu Gly Val Asp Phe Pro Val Pro Ala Ile Pro Arg Ser Gln Lys
        35                  40                  45

Ala Leu Glu Ser Asp Ser Glu Tyr Asp Ser Ile Phe Asp Glu Gly Gln
    50                  55                  60

Leu His Pro Ser Leu Arg Asp Gln Glu Tyr Leu Gln His Ser Pro Leu
65                  70                  75                  80

Trp Gly Gln Gln Tyr Val Ser Gly Gly Ala Gly Glu Gly Gln Gln Arg
                85                  90                  95

Leu Lys Pro Asp Gly Ser Ala Met Asn His Gln Gln Val Lys Thr Asp
            100                 105                 110

Asn Leu Pro Ala Tyr Cys Asn Pro Pro Asn Pro Cys Pro Val Gly Leu
```

```
        115                 120                 125

Thr Glu Glu His Gly Cys Thr Glu Asn Phe Glu Asn Thr Ala Ala Phe
    130                 135                 140

Ser Arg Asp Tyr Gln Ala Ala Gln Gln Cys Met Cys Asp Gly Glu His
145                 150                 155                 160

Met Phe Arg Cys Pro Ser Ser Leu Asp Gly Asp Glu Leu Asp Asp Ser
                165                 170                 175

Ser Glu Ser Asp Glu Gln Asp Glu His Gln Asp Leu Leu Asp Met Glu
            180                 185                 190

Gly Gly Leu Asp Thr Arg Thr Ala Pro Glu Ile Phe Met Ala Gln Arg
        195                 200                 205

Glu Tyr Arg Arg Ser Gly Leu Ser Gly Asn Lys His His Lys Arg Lys
    210                 215                 220

Ser Thr Asn Pro Tyr Leu His Gly Glu Lys Leu Pro Val Ala Ala Lys
225                 230                 235                 240

Lys Gly Ile Asn Val Val Tyr
                245
```

<210> 1931
<211> 741
<212> DNA
<213> Ctenocephalides felis

<400> 1931

```
gtagacaacg ttgataccct tcttagcagc cactgggagt ttctcaccat gcaagtaggg 60
attagtactc tttcttttat gatgtttatt cccactcaag ccagatcgtc ggtactccct 120
ttgcgccatg aagatttcag gcgcagttcg agtatcaagt ccgccttcca tgtccagaag 180
gtcctggtgt tcgtcctgtt cgtctgattc ggatgagtcg tccagttcgt ctccgtctaa 240
tgaggacgga caacggaaca tatgctcacc gtcgcacatg cattgctgag ccgcctggta 300
gtcgcggctg aaggctgcgg tgttctcgaa gttctcggtg caaccatgct cttctgttaa 360
tccaacagga cagggattag gtggattaca ataagcgggc agattatccg tttttacttg 420
ctgatgattc attgcacttc catctggttt gagcctttgt tggccttcac cagcgcctcc 480
acttacgtac tgctgacccc atagaggact atgctggaga tattcctgat ctctgaggct 540
aggatgtaac tggccttcat cgaatatgga atcatattcc gaatccgatt ctagagcttt 600
ttgagatcgt ggaattgcgg gtactgggaa gtctactccc agatctgagt attcgccacc 660
actttcaata tcggctactc gatgcatccg tgacaccact gctcttatgg catcgtctaa 720
ttcttgcact gatgttaaat a                                           741
```

<210> 1932
<211> 24
<212> DNA
<213> Artificial Sequence

<220>
<223> Description of Artificial Sequence: Synthetic Primer

<400> 1932
gcgatactgg tggtactggt gaag 24

<210> 1933
<211> 27
<212> DNA
<213> Artificial Sequence

<220>
<223> Description of Artificial Sequence: Synthetic Primer

<400> 1933
ccatcctaat acgactcact atagggc 27

<210> 1934
<211> 23
<212> DNA
<213> Artificial Sequence

<220>
<223> Description of Artificial Sequence: Synthetic Primer

<400> 1934
gaggtggttg tcttcagtgg ttg 23

<210> 1935
<211> 23
<212> DNA
<213> Artificial Sequence

<220>
<223> Description of Artificial Sequence: Synthetic Primer

<400> 1935
actcactata gggctcgagc ggc 23

<210> 1936
<211> 24
<212> DNA
<213> Artificial Sequence
<220>
<223> Description of Artificial Sequence: Synthetic Primer

<400> 1936
caattttaaa cgcatccacg accg 24

<210> 1937
<211> 37
<212> DNA
<213> Artificial Sequence
<220>
<223> Description of Artificial Sequence: Synthetic Primer

<400> 1937
ccgctcgagc gacccatttc acgacttatt tgaatcg 37

<210> 1938
<211> 36
<212> DNA
<213> Artificial Sequence

<220>
<223> Description of Artificial Sequence: Synthetic Primer

<400> 1938
ggaattctaa aatgcacaac aaaatcctgg tcctgg 36

<210> 1939
<211> 40
<212> DNA
<213> Artificial Sequence

<220>
<223> Description of Artificial Sequence: Synthetic Primer

<400> 1939
gactagtaaa atgggcgtta aaaatatata tttatactgc 40

<210> 1940
<211> 32
<212> DNA
<213> Artificial Sequence

<220>
<223> Description of Artificial Sequence: Synthetic Primer

<400> 1940
ccgctcgagg tactgcacgt actaacgtca tc 32

<210> 1941
<211> 21
<212> DNA
<213> Artificial Sequence

<220>
<223> Description of Artificial Sequence: Synthetic Primer

<400> 1941
gtctggaagc tcaggaagag g 21

<210> 1942
<211> 24
<212> DNA
<213> Artificial Sequence

<220>
<223> Description of Artificial Sequence: Synthetic Primer

<400> 1942
gtaatatgcg tgacaatcgt gtgg 24

<210> 1943
<211> 24
<212> DNA

<213> Artificial Sequence

<220>
<223> Description of Artificial Sequence: Synthetic Primer

<400> 1943
cggtgcaagt tatagaacct tccg 24

<210> 1944
<211> 32
<212> DNA
<213> Artificial Sequence

<220>
<223> Description of Artificial Sequence: Synthetic Primer

<400> 1944
cgggatcccg aatatgctga cgtagatgtg tg 32

<210> 1945
<211> 32
<212> DNA
<213> Artificial Sequence

<220>
<223> Description of Artificial Sequence: Synthetic Primer

<400> 1945
ggaattctgt tttattctgg ttggtaacat tc 32

<210> 1946
<211> 23
<212> DNA
<213> Artificial Sequence

<220>
<223> Description of Artificial Sequence: Synthetic Primer

<400> 1946
gatatccact ttgatcagcg cac 23

<210> 1947
<211> 21
<212> DNA
<213> Artificial Sequence

<220>
<223> Description of Artificial Sequence: Synthetic Primer

<400> 1947
ggtactactc ctggtgcggg c 21

<210> 1948
<211> 23
<212> DNA
<213> Artificial Sequence

<220>

<223> Description of Artificial Sequence: Synthetic Primer

<400> 1948
ccgtcgacat taaactcacc atc 23

<210> 1949
<211> 22
<212> DNA
<213> Artificial Sequence

<220>
<223> Description of Artificial Sequence: Synthetic Primer

<400> 1949
cgatcatgcg tctagcattg gc 22

<210> 1950
<211> 23
<212> DNA
<213> Artificial Sequence

<220>
<223> Description of Artificial Sequence: Synthetic Primer

<400> 1950
cccgccccag ttctaggttg tcc 23

<210> 1951
<211> 20
<212> DNA
<213> Artificial Sequence

<220>
<223> Description of Artificial Sequence: Synthetic Primer

<400> 1951
cacacccaac ctgaccaggc 20

<210> 1952
<211> 31
<212> DNA
<213> Artificial Sequence

<220>
<223> Description of Artificial Sequence: Synthetic Primer

<400> 1952
atggatccgg caaaatatac caaagaagaa g 31

<210> 1953
<211> 30
<212> DNA
<213> Artificial Sequence

<220>
<223> Description of Artificial Sequence: Synthetic Primer

<400> 1953

atgaattctt atattggtat cgcgtccatt 30

<210> 1954
<211> 24
<212> DNA
<213> Artificial Sequence

<220>
<223> Description of Artificial Sequence: Synthetic Primer

<400> 1954
agtcgcatag tgcacttctg aatg 24

<210> 1955
<211> 22
<212> DNA
<213> Artificial Sequence

<220>
<223> Description of Artificial Sequence: Synthetic Primer

<400> 1955
ctgacatctg tttccacagc tc 22

<210> 1956
<211> 24
<212> DNA
<213> Artificial Sequence

<220>
<223> Description of Artificial Sequence: Synthetic Primer

<400> 1956
aatagtgatg ttgtaagagt tagg 24

<210> 1957
<211> 29
<212> DNA
<213> Artificial Sequence

<220>
<223> Description of Artificial Sequence: Synthetic Primer

<400> 1957
gtttaatatt gcatgtttat tcattaaaa 29

<210> 1958
<211> 21
<212> DNA

<213> Artificial Sequence

<220>
<223> Description of Artificial Sequence: Synthetic Primer

<400> 1958
gcgccatgaa gatttcaggc g 21

<210> 1959
<211> 23
<212> DNA

<213> Artificial Sequence

<220>
<223> Description of Artificial Sequence: Synthetic Primer

<400> 1959
aagtgcaatg aatcatcagc aag 23